# EUROPEAN PATENT SPECIFICATION

(11) **EP 4 051 388 B1**
(45) Date of publication and mention of the grant of the patent: **10.06.2026**
(21) Application number: 20811872.9
(22) Date of filing: 30.10.2020
(51) Int. Cl.: A61P 37/00, C07D 417/14, C07D 471/04, C07D 519/00, A61K 31/4155, A61K 31/4355

(54) **SUBSTITUTED PYRAZOLE COMPOUNDS AS TOLL RECEPTOR INHIBITORS**
SUBSTITUIERTE PYRAZOLVERBINDUNGEN ALS MAUTREZEPTORINHIBITOREN
COMPOSÉS DE PYRAZOLE SUBSTITUÉS UTILISÉS EN TANT QU'INHIBITEURS DU RÉCEPTEUR TOLL

(30) Priority: 01.11.2019 US 201962929299 P
(43) Date of publication of application: 07.09.2022
(73) Proprietor: Bristol-Myers Squibb Company, Princeton, NJ 08543 (US)
(72) Inventor: PASUNOORI, Laxman, Bangalore Karnataka 560 099 (IN); KUMAR, Sreekantha Ratna, Bangalore Karnataka 560 099 (IN); SRINIVAS, Pitani Veera Venkata, Bangalore Karnataka 560 099 (IN); BHOGADI, Vikram, Bangalore Karnataka 560 099 (IN); DURAISAMY, Srinivasan Kunchithapatham, Princeton, New Jersey 08543 (US); RAMACHANDRA REDDY, Anupama Kandhi, Bangalore Karnataka 560 099 (IN); ANUMULA, Rushith Kumar, Bangalore Karnataka 560 099 (IN); DYCKMAN, Alaric J., Princeton, New Jersey 08543 (US)
(74) Representative: Dehns
(86) International application number: PCT/US2020/058081
(87) International publication number: WO 2021/087181

(56) References cited:
- EP-A1- 2 386 546
- WO-A1-2009/146343
- WO-A1-2010/045188
- WO-A1-2019/125977
- WO-A1-2019/126081
- WO-A1-2019/136147
- DATABASE REGISTRY [online] CHEMICAL ABSTRACTS SERVICE, COLUMBUS, OHIO, US; 1985, XP055761718, retrieved from stn accession no. 1635 Database accession no. 103660-46-4
- K.F.TURCHIN: "Syntheesis, structure and properties of pyrazoöo[4,3-b]quinuclidine derivatives", KHIMIYA GETEROTSIKLICHESKIKH SOEDINENII, vol. 9, 1985, pages 1258 - 1256, XP055761718
- COCCONCELLI ET AL: "Aryl azoles with neuroprotective activity-Parallel synthesis and attempts at target identification", BIOORGANIC & MEDICINAL CHEMISTRY, ELSEVIER, NL, vol. 16, no. 4, 4 November 2007 (2007-11-04), pages 2043 - 2052, XP022482500, ISSN: 0968-0896, DOI: 10.1016/J.BMC.2007.10.090

## Description

### CROSS REFERENCE

This application claims priority from U.S. Provisional Application Serial No. 62/929299, filed November 1, 2019.

### DESCRIPTION

The present invention generally relates to substituted pyrazole compounds useful as inhibitors of signaling through Toll-like receptor 7, 8, or 9 (TLR7, TLR8, TLR9) or combinations thereof. Provided herein are substituted indole compounds, compositions comprising such compounds, and methods of their use. The invention further pertains to pharmaceutical compositions containing at least one compound according to the invention that are useful for the treatment of conditions related to TLR modulation, such as inflammatory and autoimmune diseases, and methods of inhibiting the activity of TLRs in a mammal.

Toll/IL-1 receptor family members are important regulators of inflammation and host resistance. The Toll-like receptor family recognizes molecular patterns derived from infectious organisms including bacteria, fungi, parasites, and viruses (reviewed in Kawai, T. et al., Nature Immunol., 11:373-384 (2010)). Ligand binding to the receptor induces dimerization and recruitment of adaptor molecules to a conserved cytoplasmic motif in the receptor termed the Toll/IL-1 receptor (TIR) domain with the exception of TLR3, all TLRs recruit the adaptor molecule MyD88. The IL-1 receptor family also contains a cytoplasmic TIR motif and recruits MyD88 upon ligand binding (reviewed in Sims, J.E. et al., Nature Rev. Immunol., 10:89-102 (2010)).

Toll-like receptors (TLRs) are a family of evolutionarily conserved, transmembrane innate immune receptors that participate in the first-line defense. As pattern recognition receptors, the TLRs protect against foreign molecules, activated by pathogen associated molecular patterns (PAMPs), or from damaged tissue, activated by danger associated molecular patterns (DAMPs). A total of 13 TLR family members have been identified, 10 in human, that span either the cell surface or the endosomal compartment. TLR7/8/9 are among the set that are endosomally located and respond to single-stranded RNA (TLR7and TLR8) or unmethylated single-stranded DNA containing cytosine-phosphate-guanine (CpG) motifs (TLR9).

Activation of TLR7/8/9 can initiate a variety of inflammatory responses (cytokine production, B cell activation and IgG production, Type I interferon response). In the case of autoimmune disorders, the aberrant sustained activation of TLR7/8/9 leads to worsening of disease states. Whereas overexpression of TLR7 in mice has been shown to exacerbate autoimmune disease, knockout of TLR7 in mice was found to be protective against disease in lupus-prone MRL/lpr mice. Dual knockout of TLR7 and 9 showed further enhanced protection.

As numerous conditions may benefit by treatment involving modulation of cytokines, IFN production and B cell activity, it is immediately apparent that new compounds capable of modulating TLR7 and/or TLR8 and/or TLR9 and methods of using these compounds could provide substantial therapeutic benefits to a wide variety of patients.

WO 2010/045188 A1 discloses heteroaryl substituted indole compounds which are inhibitors of MMP-13.

WO 2009/146343 discloses use of amyloid binding compounds as tracers in positron emission tomography.

WO 2019/125977 and WO 2019/126081 disclose compounds that act as inhibitors of signaling through Toll-like receptor 7, or 8, or 9, that are useful in treating inflammatory and autoimmune diseases.

WO 2019/136147 discloses compounds that have Toll-like receptor inhibitory activity, including at TLR2, TLR4, TLR7 and/or TLR9.

### SUMMARY OF THE INVENTION

The present invention relates to a new class of substituted pyrazole compounds found to be effective inhibitors of signaling through TLR7/8/9. These compounds are provided to be useful as pharmaceuticals with desirable stability, bioavailability, therapeutic index, and toxicity values that are important to their drugability.

The present invention provides compounds of Formula (I) that are useful as inhibitors of signaling through Toll-like receptor 7, 8, or 9 and are useful for the treatment of proliferative diseases, allergic diseases, autoimmune diseases and inflammatory diseases, or stereoisomers, N-oxides, tautomers, pharmaceutically acceptable salts, solvates or prodrugs thereof.

The present invention also provides pharmaceutical compositions comprising a pharmaceutically acceptable carrier and at least one of the compounds of the present invention or stereoisomers, tautomers, pharmaceutically acceptable salts or solvates thereof.

The compounds of the present invention may be used in a method for inhibition of Toll-like receptor 7, 8, or 9 comprising administering to a host in need of such treatment a therapeutically effective amount of at least one of the compounds of the present invention or stereoisomers, tautomers, pharmaceutically acceptable salts or solvates thereof.

The compounds of the present invention may also be used in a method for treating proliferative, metabolic, allergic, autoimmune and inflammatory diseases, comprising administering to a host in need of such treatment a therapeutically effective amount of at least one of the compounds of the present invention or stereoisomers, tautomers, pharmaceutically acceptable salts or solvates thereof.

The compounds of the present invention may also be used in a method of treating a disease or disorder associated with Toll-like receptor 7, 8, or 9 activity, the method comprising administering to a mammal in need thereof, at least one of the compounds of Formula (I) or salts or solvates thereof.

The present application also describes processes and intermediates for making the compounds of Formula (I) including salts or solvates thereof.

The present invention also provides at least one of the compounds of Formula (I) or salts or solvates thereof, for use in therapy.

The at least one of the compounds of Formula (I) or salts or solvates thereof, may also be used for the manufacture of a medicament for the treatment of prophylaxis of Toll-like receptor 7, 8, or 9 related conditions, such as allergic disease, autoimmune diseases, inflammatory diseases, and proliferative diseases.

The compound of Formula (I) and compositions comprising the compounds of Formula (I) may be used in treating, preventing, or curing various Toll-like receptor 7, 8, or 9 related conditions. Pharmaceutical compositions comprising these compounds are useful for treating, preventing, or slowing the progression of diseases or disorders in a variety of therapeutic areas, such as allergic disease, autoimmune diseases, inflammatory diseases, and proliferative diseases.

These and other features of the invention will be set forth in expanded form as the disclosure continues.

### DETAILED DESCRIPTION

The first aspect of the present invention provides at least one compound of Formula (I): 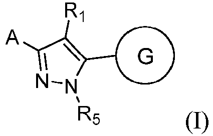 N-oxide, or a salt thereof, wherein:
G is: 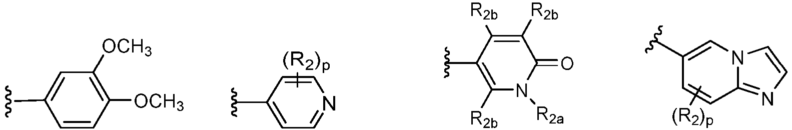 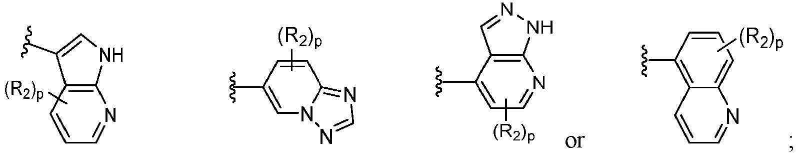
R₁ is, -CH₃, -CH₂CH₃, -CH(CH₃)₂, -CF₃, or -CH₂CF₃;
each R₂ is independently -CN, -CH₃, or -OCH₃;
R₂ₐ is C₁₋₆ alkyl, C₁₋₃ fluoroalkyl, C₁₋₆ hydroxyalkyl, C₁₋₃ aminoalkyl, -(CH₂)₀₋₄O(C₁₋₃ alkyl), C₃₋₆ cycloalkyl, -(CH₂)₁₋₃C(O)NR_{y}R_{y}, -CH₂(C₃₋₆ cycloalkyl), -CH₂(phenyl), tetrahydrofuranyl, tetrahydropyranyl, or phenyl;
each R₂₆ is independently hydrogen, halo, -CN, -NRₓRₓ, C₁₋₆ alkyl, C₁₋₃ fluoroalkyl, C₁₋₃ hydroxyalkyl, C₁₋₃ fluoroalkoxy, -(CH₂)₀₋₂O(C₁₋₃ alkyl), -(CH₂)₀₋₃C(O)NRₓRₓ, -(CH₂)₁₋₃(C₃₋₆ cycloalkyl), -C(O)O(C₁₋₃ alkyl), -C(O)NRₓ(C₁₋₃ alkyl), -CRₓ=CRₓRₓ, or -CRₓ=CH(C₃₋₆ cycloalkyl);
A is:
   (i)-CH₂N(CH₃)Rx, -C(O)NRₓRₓ, -C(O)N(CH₃)(CH₂CH₂CN), -C(O)N(CH₂CH₃)(CH₂CH₂CN), or -C(O)N(CH₃)(CH₂CH₂CH₂N(CH₃)₂);
   (ii) -C(O)A₁, -C(O)NRₓ(CRₓRₓ)₀₋₂A₁, -CH₂NHA₁, or -C(O)C(O)NHA₁;
   (iii) cyclohexyl substituted zero to 1 R_{3b};
   (iv) piperidinyl substituted with zero to 1 R_{3c};
   (v) phenyl substituted with zero to 1 R_{3d} and zero to 1 R₃ₑ;
   (vi) pyridinyl substituted with zero to 1 R_{3f} and zero to 1 R_{3g};
   (vii) pyrazinyl or pyrimidinyl, each substituted with zero to 1 R_{3f};
   (viii) thiazolyl substituted with R₃ₕ and zero to 1 R₃ᵢ;
   (ix) diazabicyclo[2.2.1]heptanyl or diazaspiro[3.3]heptanyl, each substituted with zero to 1 R₃ⱼ; or
   (x) benzo[d]thiazolyl, dihydroisoquinolinyl, tetrahydronaphthyridinyl, tetrahydrobenzo[d]thiazolyl, tetrahydroimidazo[1,2-a]pyrazinyl, tetrahydroisoquinolinonyl, tetrahydroisoquinolinyl, tetrahydronaphthalenyl, tetrahydropyrazolo[1,5-a]pyrazinyl, tetrahydropyrido[4,3-d]pyrimidinyl, tetrahydrothiazolo[4,5-c]pyridinyl, or tetrahydrothiazolo[5,4-c]pyridinyl, each substituted with zero to 1 R₃ₖ;
A₁ is azetidinyl, cyclohexyl, pyrrolidinyl, piperidinyl, piperazinyl, morpholinyl, tetrahydropyranyl, pyridinyl, diazepanyl, hexahydropyrrolo[3,4-c]pyrrolyl, each substituted with zero to 1 R₃ₐ;
R₃ₐ is -OH, -CH₃, -CH₂CH₃, -CH₂CH₂CH₃, -CH(CH₃)₂, -CH₂CH(CH₃)₂, -CH(CH₃)(CH₂CH₃), -CH₂C(CH₃)₃, -CH₂CH₂CH(CH₃)₂, -CH(CH₃)CH(CH₃)₂, -CH₂CH₂C(CH₃)₃, -CH₂C(CH₂CH₃)₂, -CH₂CF₃, -CH₂CH₂CF₃, -CH₂CH₂CH₂CF₃, -CH₂CH₂CN,-CH₂C(CH₃)₂OH, -CH₂CH₂OCH₃, -CH₂CH₂S(O)₂CH₃, -CH(CH₃)CH₂S(O)₂CH₃, -CH₂NH₂, -CH₂CH₂N(CH₃)₂, -CH₂C(O)NH(CH₃), -CH₂C(O)N(CH₃)₂, -NR_{y}R_{y}, -N(CH₃)(CH₂CH₂OCH₃), -NH(CH₂CH₂CH₂CF₃), -NHCH(CH₃)(CH₂OCH₃), -C(O)NH₂, -C(O)OC(CH₃)₃, -CH₂(cyclopropyl), -CH₂(methyloxetanyl), -CH₂(tetrahydrofuranyl), -CH₂(tetrahydropyranyl), -CH₂(dimethylisoxazolyl), -CH₂(methyltriazolyl), -CH₂(methoxypyrimidinyl), -NH(oxetanyl), -NH(methyloxetanyl), -NH(tetrahydropyranyl), -NH(dimethyltetrahydropyranyl), -N(cyclopropyl)₂, -NHCH₂(cyclopentyl), -NHCH₂(dimethylisoxazolyl), -NHCH₂(methyloxetanyl), -NHCH₂(pyridinyl), -NHCH₂(pyrimidinyl), -NHCH₂(methylpyrimidinyl), -NHCH₂(methoxypyrimidinyl), -NHCH₂(tetrahydrofuranyl), -NHCH₂(tetrahydropyranyl), cyclobutyl, oxetanyl, isopropylpiperidinyl, tetrahydropyranyl, dimethyltetrahydropyranyl, or pyridinyl;
R_{3b} is -NH(CH₃), -NH(CH₂CHF₂), -N(CH₃)(CH₂CH₃), -NH(CH₂CH₂N(CH₃)₂), -N(CH₃)C(O)CH₂N(CH₃)₂, -N(CH₃)CH₂C(O)N(CH₃)₂, -NH(isopropylpiperidinyl), -N(CH₃)C(O)(azetidinyl), -N(CH₃)C(O)(isopropylazetidinyl), -N(CH₃)C(O)(ethylazetidinyl), -N(CH₃)C(O)(methylazetidinyl), -NH(CH₂(methyloxetanyl), morpholinyl, methylpiperazinyl, or dimethylaminopiperidinyl;
R₃, is C₁₋₃ alkyl, -CH₂C(O)N(CH₃)Rₓ, -C(O)CH₂N(CH₃)₂, -C(O)CH₂CH₂N(CH₃)₂, or -C(O)CH₂CH₂NH(CH(CH₃)₂);
R_{3d} is:
   (a) -CRₓRₓNRₓRₓ, -CRₓRₓNRₓ(C₂₋₅ alkyl), -CH(CH₃)N(CH₃)(CH₂CF₃), -CH₂CH₂S(O)₂CH₃, -CH(CH₃)N(CH₃)CH₂C(O)N(CH₃)₂, -CH(CH₃)NR,C(O)CH₂N(CH₂CH₃)₂, -CRₓRₓNRₓC(O)CHRₓNRₓR_{y}, -CH(CH₃)N(CH₃)C(O)CH₂NRₓ(C₃₋₄ fluoroalkyl), -NRₓRₓ, -NH(CH(CH₃)₂), -C(O)NH₂, -CRₓRₓQ₁, -CRₓRₓNRₓQ₁, -CRₓRₓNRₓCH₂Q₁, -CRₓRₓNRₓC(O)Q₁, -CRₓRₓNRₓC(O)CRₓRₓQ₁, -CRₓRₓNRₓC(O)CRₓRₓNRₓQ₁, or -CH(CH₃)N(oxetanyl)(C(O)CH₂N(C₁₋₂alkyl)₂);
   (b) azetidinyl substituted with zero to 1 substituent selected form C₁₋₃ alkyl, -CH₂C(CH₃)₂OH, -C(O)CH₂N(CH₃)₂, -N(CH₃)₂, -NHCH(CH₃)₂, oxetanyl, and tetrahydropyranyl;
   (c) cyclopropyl or cyclohexyl, each substituted with -NRₓRₓ, -NRₓ(C₂₋₄ alkyl), -NH(oxetanyl), -N(CH₃)CH₂CH₂OCH, -N(CH₃)CH₂C(O)N(CH₃)₂, -N(CH₃)C(O)CH₂N(CH₃)₂, or -N(CH₃)CH₂(ethyloxetanyl); or
   (d) morpholinyl, piperazinonyl, piperazinyl, piperidinyl, or pyrrolidinyl, each substituted with zero to 1 substituent selected from C₁₋₅ alkyl, -CH₂CF₃, -CH₂C(CH₃)₂OH, -CH₂CH₂OCH₃, -CH₂C(O)CRₓRₓ, -C(O)CH₂N(CH₃)₂, oxetanyl, methyloxetanyl, and tetrahydropyranyl;
Q₁ is azetidinyl, cyclopropyl, morpholinyl, oxetanyl, tetrahydropyranyl, triazolyl, oxaazaspiro[3.3]heptanyl, piperazinonyl, difluoropiperidinyl, or pyrrolidinyl, each substituted with zero to 2 substituents independently selected from F, -CH₃, -CH₂CH₃, -CH₂OH, and oxetanyl;
R₃, is F;
R_{3f} is:
   (a) -OH, -NH₂, -N(CH₃)₂, -NH(CH(CH₃)₂), -NHCH₂C(CH₃)₂OCH₃, -CH₂NH(CH₃), -CH₂N(CH₃)₂, -CH₂NH(CH(CH₃)₂), -CH(CH₃)N(CH₃)₂, or -CH(CH₃)N(CH₃)C(O)CH₂N(CH₃)₂;
   (b) cyclohexyl substituted with -NH₂, -N(CH₃)₂, -NRₓ(CH₂CH₂OCH₃), -NH(cyclobutyl), -NH(methyloxetanyl), -NHCH₂(methylsulfonylcyclopropyl), morpholinyl, methoxyazetidinyl, piperazinonyl, difluoropiperidinyl, methoxypiperidinyl, oxaazaspiro[3.3]heptanyl, or oxaazaspiro[3.5]nonanyl;
   (c) diazaspiro[3.3]heptanyl substituted with zero to 1 substituent selected from C₁₋₄ alkyl, -CH₂CH₂OCH₃, -CH₂CH₂S(O)₂CH₃, -CH₂(C₃₋₄ cycloalkyl), -CH₂(tetrahydropyranyl), cyclobutyl, oxetanyl, and tetrahydropyranyl;
   (d) piperazinyl substituted with zero to 1 substituent selected from C₁₋₆ alkyl, -CH₂CH₂CF₃, -CH₂CH₂OCH₃, -CH₂CH₂S(O)₂CH₃, -CH₂(C₃₋₄ cycloalkyl), -CH₂(ethyloxetanyl), -CH₂C(O)NH(CH₃), -CH₂C(O)N(CH₃)₂, -C(O)CH₂N(CH₃)₂, -C(O)CH₂N(CH₂CH₃)₂, -CH₂(tetrahydropyranyl), cyclobutyl, oxetanyl, tetrahydropyranyl, and dioxothiotetrahydropyranyl; or
   (e) piperidinyl substituted with zero to 1 substituent selected from C₁₋₆ alkyl, -CH₂CN, -CH₂CH₂F, -CH₂CH₂CH₂F, -CH₂CH₂CF₃, -CH₂C(CH₃)₂OH, -CH₂CH₂OCH₃, -CH₂CH₂S(O)₂CH₃, -CH₂C(O)NRₓRₓ, -C(O)CH₂N(CH₃)₂, -C(O)CH₂N(CH₂CH₃)₂, -CH₂(C₃₋₄ cycloalkyl), -CH₂(tetrahydrofuranyl), -CH₂(tetrahydropyranyl), -CH₂(methyltriazolyl), cyclobutyl, ethoxycyclobutyl, oxetanyl, tetrahydrofuranyl, and tetrahydropyranyl;
R_{3g} is F, -CH₃, or -CF₃;
R₃ₕ is:
   (a) -CH(CH₃)N(CH₃)Rₓ, -CH(CH₃)N(CH₃)(C₂₋₃ alkyl), -CH(CH₃)N(CH₃)CH₂C(O)N(CH₃)₂, -CH(CH₃)N(CH₃)C(O)CH₂N(CH₃)₂, or -CH(CH₃)N(CH₃)(tetrahydropyranyl);
   (b) cyclohexyl substituted with -N(CH₃)Rₓ, -N(CH₃)(CH(CH₃)₃), -N(CH₃)(CH₂CH₂CF₃), -NRₓ(CH₂CH₂OCH₃), -NH(CH₂C(CH₃)₂OCH₃), -N(CH₃)C(O)CH₂N(CH₃)₂, -NH(cyclobutyl), -NRₓ(oxetanyl), -NH(methyloxetanyl), -NH(tetrahydropyranyl), -NHCH₂(methylsulfonylcyclopropyl), -NHCH₂(methyloxetanyl), methoxyazetidinyl, (trifluoromethyl)hydroxyazetidinyl, morpholinyl, pyrrolidinyl, piperazinonyl, methylsulfonylpiperazinyl, oxazepanyl, oxaazaspiro[3.3]heptanyl, oxaazaspiro[3.5]nonanyl, or dioxothiaazaspiro[3.3]heptanyl;
   (c) piperazinyl substituted with zero to two -CH₃; and zero or 1 substituent selected from C₁₋₅ alkyl, -CH₂CN, -CH₂CH₂F, -CH₂CH₂CH₂F, -CH₂C(CH₃)₂OH, -CH₂CH₂OCH₃, -CH₂CH₂S(O)₂CH₃, -CH₂C(O)N(CH₃)Rₓ, -NHC(O)CH₂N(CH₃)₂, -NHC(O)CH₂N(CH₂CH₃)₂, -C(O)CH₂N(CH₃)₂, -C(O)CH₂N(CH₂CH₃)₂, -CH₂(C₃₋₄ cycloalkyl), -CH₂(tetrahydropyranyl), -C(O)CH₂(morpholinyl), cyclobutyl, oxetanyl, and tetrahydropyranyl; or
   (d) piperidinyl substituted with zero or one -CH₃ and zero or 1 substituent selected from C₁₋₆ alkyl, -CH₂CN, -CH₂CH₂F, -CH₂CH₂CH₂F, -CH₂CH₂CF₃, -CH₂C(CH₃)₂OH, -CH₂CH₂OCH₃, -CH₂CH₂OC(CH₃)₃, -CH₂CH₂S(O)₂CH₃, -CH₂C(O)NH₂, -CH₂C(O)N(CH₃)₂, -CH₂C(O)N(CH₃)(CH(CH₃)₂), -C(O)CH₂N(CH₃)₂, -C(O)CH₂N(CH₂CH₃)₂, -C(O)CH₂N(CH₃)(CH₂CH₂OCH₃), -NH₂, -NH(CH₂CH₂CH₃), -NH(CH(CH₃)₂), -NHCH₂CH(CH₃)₂, -N(CH₃)₂, -N(CH₃)(CH₂CH₃), -NH(CH₂C(CH₃)₂OCH₃), -CH₂(C₃₋₆ cycloalkyl), -CH₂(methylsulfonylcyclopropyl), -CH₂(oxetanyl), -CH₂(methyloxetanyl), -CH₂(ethyloxetanyl), -CH₂(tetrahydrofuranyl), -CH₂(tetrahydropyranyl), -CH₂(methyltriazolyl), -CH₂C(O)(oxetanyl), -CH₂C(O)(morpholinyl), -CH₂C(O)(oxaazaspiro[3.3]heptanyl), -C(O)CH₂(methoxyazetidinyl), -C(O)CH₂(morpholinyl), -C(O)CH₂(oxaazaspiro[3.5]nonanyl), -C(O)CH₂(piperidinonyl), -N(CH₂(cyclopropyl))₂, -N(CH₂(tetrahydropyranyl))₂, -NH(methyloxetanyl), -NH(tetrahydropyranyl), -NHC(O)CH₂(morpholinyl), -NHCH₂(cyclopropyl), cyclobutyl, ethoxycyclobutyl, ethoxycyclobutyl, oxetanyl, tetrahydrofuranyl, tetrahydropyranyl, dioxotetrahydrothiophenyl, and (oxetanylamino)piperidinyl;
R₃ᵢ is -CH₃ or -CF₃;
R₃ⱼ is C₁₋₆ alkyl, -CH₂CH₂OCH₃, -CH₂CH₂S(O)₂CH₃, -C(O)CH₂N(CH₃)₂, -CH₂(C₃₋₄ cycloalkyl), -CH₂(tetrahydropyranyl), -CH(CH₃)(cyclopropyl), cyclobutyl, oxetanyl, tetrahydropyranyl, or isopropylpiperidinyl;
R₃ₖ is C₁₋₄ alkyl, -CH₂C(CH₃)₂OH, -CH₂CH₂OCH₃, -CH₂C(O)NRₓRₓ, -C(O)CH₃, -C(O)CH₂CH(CH₃)OH, -C(O)CH₂N(CH₃)₂, -NRₓ(C₁₋₃ alkyl), -NRₓ(C₃₋₄ fluoroalkyl), -NRₓ(CH₂CH₂OCH₃), -N(CH₃)(CH₂CH₂S(O)₂CH₃), -N(CH₃)(CH_{2C}(O)N(CH₃)₂), -NRₓ(C(O)CH₂N(CH₃)₂), -N(CH₂CH₂CH₂CF₃)₂, -NRₓ(oxetanyl), -N(CH₃)(methyloxetanyl), -N(CH₃)(tetrahydropyranyl), -NH(ethoxycyclobutyl), oxetanyl, or isopropylpiperidinyl;
R₅ is hydrogen.
each Rₓ is independently H or -CH₃;
each R_{y} is independently H or C₁₋₆ alkyl; and
p is zero, 1 or 2.

One embodiment provides a compound of Formula (I), N-oxide, or a salt thereof wherein G is: 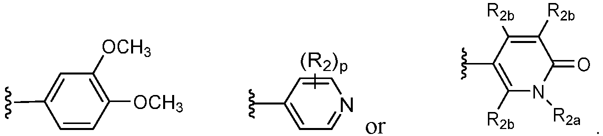 Included in this embodiment are compounds in which R₂ₐ is C₁₋₄ alkyl, C₁₋₂ fluoroalkyl, C₁₋₄ hydroxyalkyl, -(CH₂)₁₋₃OCH₃, C₃₋₆ cycloalkyl, -CH₂C(O)NRₓRₓ, -CH₂(C₃₋₆ cycloalkyl), -CH₂(phenyl), tetrahydrofuranyl, or phenyl; each R₂₆ is independently H, F, Cl, -CN, -NRₓRₓ, C₁₋₆ alkyl, C₁₋₂ fluoroalkyl, C₁₋₃ hydroxyalkyl, -(CH₂)₀₋₂O(C₁₋₂ alkyl), -(CH₂)₀₋₂C(O)NRₓRₓ, -(CH₂)₁₋₃(cyclopropyl), -C(O)O(C₁₋₂ alkyl), or -C(O)NRₓ(C₁₋₃ alkyl); and p is 1 or 2.

One embodiment provides a compound of Formula (I), N-oxide, or a salt thereof wherein G is: 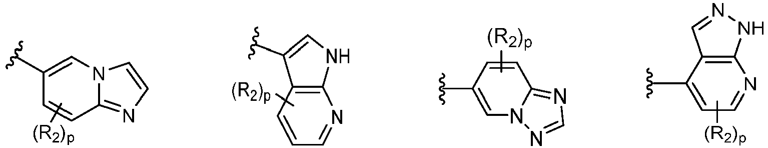 or

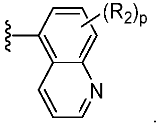

One embodiment provides a compound of Formula (I), N-oxide, or a salt thereof wherein G is:

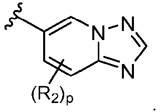

One embodiment provides a compound of Formula (I), N-oxide, or a salt thereof wherein R₁ is -CH(CH₃)₂.

One embodiment provides a compound of Formula (I), N-oxide, or a salt thereof wherein A is -CH₂N(CH₃)Rx, -C(O)NRₓR_{y}, -C(O)N(CH₃)(CH₂CH₂CN), -C(O)N(CH₂CH₃)(CH₂CH₂CN), or -C(O)N(CH₃)(CH₂CH₂CH₂N(CH₃)₂).

One embodiment provides a compound of Formula (I), N-oxide, or a salt thereof wherein A is -C(O)A₁, -C(O)NRₓ(CRₓRₓ)₀₋₂A₁, -CH₂NHA₁, or -C(O)C(O)NHA₁.

One embodiment provides a compound of Formula (I), N-oxide, or a salt thereof wherein A is cyclohexyl substituted with R_{3b}. Also included in this embodiment are compounds in which G is:

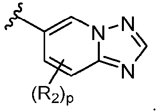

One embodiment provides a compound of Formula (I), N-oxide, or a salt thereof wherein A is piperidinyl substituted with R_{3c}.

One embodiment provides a compound of Formula (I), N-oxide, or a salt thereof wherein A is piperidinyl substituted with zero to 1 R_{3c}. Also included in this embodiment are compounds in which G is:

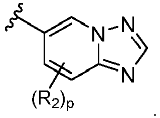

One embodiment provides a compound of Formula (I), N-oxide, or a salt thereof wherein A is phenyl substituted with zero to 1 R_{3d} and zero to 1 R₃ₑ. Included in this embodiment are compounds in which A is phenyl substituted with R_{3d} and zero to 1 R₃ₑ. Also included in this embodiment are compounds in which A is phenyl substituted with R_{3d}. Further, included in this embodiment are compounds in which A is phenyl substituted with R_{3d} and R₃ₑ.

Also included in this embodiment are compounds in which G is:

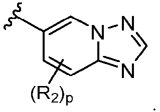

One embodiment provides a compound of Formula (I), N-oxide, or a salt thereof wherein A is pyridinyl substituted with zero to 1 R_{3f} and zero to 1 R_{3g}. Included in this embodiment are compounds in which A is pyridinyl substituted with R_{3f} and zero to 1 R_{3g}. Also included in this embodiment are compounds in which A is pyridinyl substituted with R_{3f}. Further, included in this embodiment are compounds in which A is pyridinyl substituted with R_{3f} and R_{3g}.

Also included in this embodiment are compounds in which G is:

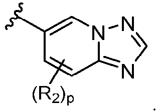

One embodiment provides a compound of Formula (I), N-oxide, or a salt thereof wherein A is pyrazinyl or pyrimidinyl, each substituted with zero to 1 R_{3f}. Also included in this embodiment are compounds in which A is pyrazinyl or pyrimidinyl, each substituted with R_{3f}. Further, included in this embodiment are compounds in which A is pyrimidinyl substituted with zero to 1 R_{3f}.

. Also included in this embodiment are compounds in which G is:

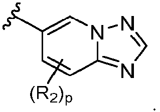

One embodiment provides a compound of Formula (I), N-oxide, or a salt thereof wherein A is thiazolyl substituted with R₃ₕ and zero to 1 R₃ᵢ. Further, included in this embodiment are compounds in which A is thiazolyl substituted with R₃ₕ.

Also included in this embodiment are compounds in which G is:

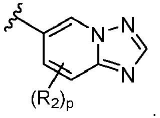

One embodiment provides a compound of Formula (I), N-oxide, or a salt thereof wherein A is diazabicyclo[2.2.1]heptanyl, diazaspiro[3.3]heptanyl, or dioxidothiaazaspiro[3.3]heptanyl, each substituted with zero to 1 R₃ⱼ. Included in this embodiment are compounds in which A is diazabicyclo[2.2.1]heptanyl or diazaspiro[3.3]heptanyl, each substituted with zero to 1 R₃ⱼ.

One embodiment provides a compound of Formula (I), N-oxide, or a salt thereof wherein A is diazabicyclo[2.2.1]heptanyl or diazaspiro[3.3]heptanyl, each substituted with zero to 1 R_{3.j} Also included in this embodiment are compounds in which G is:

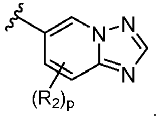

One embodiment provides a compound of Formula (I), N-oxide, or a salt thereof wherein A is benzo[d]thiazolyl, dihydroisoquinolinyl, tetrahydronaphthyridinyl, tetrahydrobenzo[d]thiazolyl, tetrahydroimidazo[1,2-a]pyrazinyl, tetrahydroisoquinolinonyl, tetrahydroisoquinolinyl, tetrahydronaphthalenyl, tetrahydropyrazolo[1,5-a]pyrazinyl, tetrahydropyrido[4,3-d]pyrimidinyl, tetrahydrothiazolo[4,5-c]pyridinyl, or tetrahydrothiazolo[5,4-c]pyridinyl, each substituted with zero to 1 R₃ₖ. Included in this embodiment are compounds in which A is benzo[d]thiazolyl, dihydroisoquinolinyl, tetrahydronaphthyridinyl, tetrahydrobenzo[d]thiazolyl, tetrahydroimidazo[1,2-a]pyrazinyl, tetrahydroisoquinolinonyl, tetrahydroisoquinolinyl, tetrahydronaphthalenyl, tetrahydropyrazolo[1,5-a]pyrazinyl, tetrahydropyrido[4,3-d]pyrimidinyl, tetrahydrothiazolo[4,5-c]pyridinyl, or tetrahydrothiazolo[5,4-c]pyridinyl, each substituted with R₃ₖ. Also included in this embodiment are compounds in which A is benzo[d]thiazolyl, dihydroisoquinolinyl, tetrahydronaphthyridinyl, tetrahydrobenzo[d]thiazolyl, tetrahydroimidazo[1,2-a]pyrazinyl, tetrahydroisoquinolinonyl, tetrahydroisoquinolinyl, tetrahydronaphthalenyl, tetrahydropyrazolo[1,5-a]pyrazinyl, tetrahydropyrido[4,3-d]pyrimidinyl, tetrahydrothiazolo[4,5-c]pyridinyl, or tetrahydrothiazolo[5,4-c]pyridinyl. Also included in this embodiment are compounds in which G is:

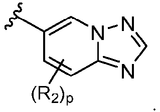

One embodiment provides a compound of Formula (I), N-oxide, or a salt thereof wherein A is: (i) -CH₂N(CH₃)Rx, -C(O)NRₓRₓ, -C(O)N(CH₃)(CH₂CH₂CN), -C(O)N(CH₂CH₃)(CH₂CH₂CN), or -C(O)N(CH₃)(CH₂CH₂CH₂N(CH₃)₂); or (ii) -C(O)A₁, -C(O)NRₓ(CR_{X}R_{X})₀₋₂A₁, -CH₂NHA₁, or -C(O)C(O)NHA₁. Included in this embodiment are compounds in which A is (i) -CH₂N(CH₃)Rₓ or -C(O)NRₓRₓ; or (ii) -C(O)A₁, -C(O)NRₓ(CRₓRₓ)₀₋₂A₁, or -CH₂NHA₁.

One embodiment provides a compound of Formula (I), N-oxide, or a salt thereof wherein A is: (i) cyclohexyl substituted zero to 1 R_{3b}; (ii) piperidinyl substituted with zero to 1 R_{3c}; (iii) phenyl substituted with zero to 1 R_{3d} and zero to 1 R₃ₑ; (iv) pyridinyl substituted with zero to 1 R_{3f} and zero to 1 R_{3g}; (v) pyrazinyl or pyrimidinyl, each substituted with zero to 1 R_{3f}; (vi) thiazolyl substituted with R₃ₕ and zero to 1 R₃ᵢ; (vii) diazabicyclo[2.2.1]heptanyl or diazaspiro[3.3]heptanyl, each substituted with zero to 1 R₃ⱼ; or (viii) benzo[d]thiazolyl, dihydroisoquinolinyl, tetrahydronaphthyridinyl, tetrahydrobenzo[d]thiazolyl, tetrahydroimidazo[1,2-a]pyrazinyl, tetrahydroisoquinolinonyl, tetrahydroisoquinolinyl, tetrahydronaphthalenyl, tetrahydropyrazolo[1,5-a]pyrazinyl, tetrahydropyrido[4,3-d]pyrimidinyl, tetrahydrothiazolo[4,5-c]pyridinyl, or tetrahydrothiazolo[5,4-c]pyridinyl, each substituted with zero to 1 R₃ₖ.

One embodiment provides a compound of Formula (I), N-oxide, or a salt thereof wherein A is: (i) cyclohexyl substituted zero to 1 R_{3b}; (ii) piperidinyl substituted with zero to 1 R_{3c}; (iii) phenyl substituted with zero to 1 R_{3d} and zero to 1 R₃ₑ; (iv) pyridinyl substituted with zero to 1 R_{3f} and zero to 1 R_{3g}; (v) pyrazinyl or pyrimidinyl, each substituted with zero to 1 R_{3f}; or (vi) thiazolyl substituted with R₃ₕ and zero to 1 R₃ᵢ.

One embodiment provides a compound of Formula (I), N-oxide, or a salt thereof wherein A is: (i) piperidinyl substituted with zero to 1 R_{3c}; (ii) phenyl substituted with zero to 1 R_{3d} and zero to 1 R₃ₑ; (iii) pyridinyl substituted with zero to 1 R_{3f} and zero to 1 R_{3g}; or (iv) pyrazinyl or pyrimidinyl, each substituted with zero to 1 R_{3f}; or (vi) thiazolyl substituted with R₃ₕ and zero to 1 R₃ᵢ.

One embodiment provides a compound of Formula (I), N-oxide, or a salt thereof wherein A is: (i) phenyl substituted with zero to 1 R_{3d} and zero to 1 R₃ₑ; (ii) pyridinyl substituted with zero to 1 R_{3f} and zero to 1 R_{3g}; or (iii) pyrazinyl or pyrimidinyl, each substituted with zero to 1 R_{3f}; or (vi) thiazolyl substituted with R₃ₕ and zero to 1 R₃ᵢ.

One embodiment provides a compound of Formula (I), N-oxide, or a salt thereof wherein:
G is: 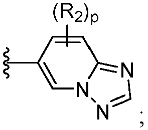
R₁ is -CH(CH₃)₂;
each R₂ is independently -CH₃ or -OCH₃; and
p is 1 or 2.

One embodiment provides a compound of Formula (I), N-oxide, or a salt thereof wherein said compound is a compound of one of examples disclosed herein below.

The invention encompasses all combinations of the aspects and/or embodiments of the invention noted herein. It is understood that any and all embodiments of the present invention may be taken in conjunction with any other embodiment or embodiments to describe additional embodiments. It is also to be understood that each individual element of the embodiments is meant to be combined with any and all other elements from any embodiment to describe an additional embodiment.

### DEFINITIONS

The features and advantages of the invention may be more readily understood by those of ordinary skill in the art upon reading the following detailed description. It is to be appreciated that certain features of the invention that are, for clarity reasons, described above and below in the context of separate embodiments, may also be combined to form a single embodiment. Conversely, various features of the invention that are, for brevity reasons, described in the context of a single embodiment, may also be combined so as to form sub-combinations thereof. Embodiments identified herein as exemplary or preferred are intended to be illustrative and not limiting.

Unless specifically stated otherwise herein, references made in the singular may also include the plural. For example, "a" and "an" may refer to either one, or one or more.

As used herein, the phrase "compounds" refers to at least one compound. For example, a compound of Formula (I) includes a compound of Formula (I) and two or more compounds of Formula (I).

Unless otherwise indicated, any heteroatom with unsatisfied valences is assumed to have hydrogen atoms sufficient to satisfy the valences.

The definitions set forth herein take precedence over definitions set forth in any patent, patent application, and/or patent application publication referenced herein.Listed below are definitions of various terms used to describe the present invention. These definitions apply to the terms as they are used throughout the specification (unless they are otherwise limited in specific instances) either individually or as part of a larger group.

Throughout the specification, groups and substituents thereof may be chosen by one skilled in the field to provide stable moieties and compounds.

In accordance with a convention used in the art, 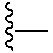 is used in structural formulas herein to depict the bond that is the point of attachment of the moiety or substituent to the core or backbone structure.

The terms "halo" and "halogen," as used herein, refer to F, Cl, Br, and I.

The term "cyano" refers to the group -CN.

The term "amino" refers to the group -NH₂.

The term "oxo" refers to the group =O.

The term "alkyl" as used herein, refers to both branched and straight-chain saturated aliphatic hydrocarbon groups containing, for example, from 1 to 12 carbon atoms, from 1 to 6 carbon atoms, and from 1 to 4 carbon atoms. Examples of alkyl groups include, but are not limited to, methyl (Me), ethyl (Et), propyl (*e.g*., n-propyl and i-propyl), butyl *(e.g.,* n-butyl, i-butyl, sec-butyl, and *t*-butyl), and pentyl *(e.g.,* n-pentyl, isopentyl, neopentyl), n-hexyl, 2-methylpentyl, 2-ethylbutyl, 3-methylpentyl, and 4-methylpentyl. When numbers appear in a subscript after the symbol "C", the subscript defines with more specificity the number of carbon atoms that a particular group may contain. For example, "C₁₋₆ alkyl" denotes straight and branched chain alkyl groups with one to six carbon atoms.

The term "fluoroalkyl" as used herein is intended to include both branched and straight-chain saturated aliphatic hydrocarbon groups substituted with one or more fluorine atoms. For example, "C₁₋₄ fluoroalkyl" is intended to include C₁, C₂, C₃, and C₄ alkyl groups substituted with one or more fluorine atoms. Representative examples of fluoroalkyl groups include, but are not limited to, -CF₃ and -CH₂CF₃.

The term "cyanoalkyl" includes both branched and straight-chain saturated alkyl groups substituted with one or more cyano groups. For example, "cyanoalkyl" includes -CH₂CN, -CH₂CH₂CN, and C₁₋₄ cyanoalkyl.

The term "aminoalkyl" includes both branched and straight-chain saturated alkyl groups substituted with one or more amine groups. For example, "aminoalkyl" includes -CH₂NH₂, -CH₂CH₂NH₂, and C₁₋₄ aminoalkyl.

The term "hydroxyalkyl" includes both branched and straight-chain saturated alkyl groups substituted with one or more hydroxyl groups. For example, "hydroxyalkyl" includes -CH₂OH, -CH₂CH₂OH, and C₁₋₄ hydroxyalkyl.

The term "hydroxy-fluoroalkyl" includes both branched and straight-chain saturated alkyl groups substituted with one or more hydroxyl groups and one or more fluorine atoms. For example, "hydroxy-fluoroalkyl" includes -CHFCH₂OH, -CH₂CHFC(CH₃)₂OH, and C₁₋₄ hydroxy-fluoroalkyl.

The term "cycloalkyl," as used herein, refers to a group derived from a nonaromatic monocyclic or polycyclic hydrocarbon molecule by removal of one hydrogen atom from a saturated ring carbon atom. Representative examples of cycloalkyl groups include, but are not limited to, cyclopropyl, cyclopentyl, and cyclohexyl. When numbers appear in a subscript after the symbol "C", the subscript defines with more specificity the number of carbon atoms that a particular cycloalkyl group may contain. For example, "C₃-C₆ cycloalkyl" denotes cycloalkyl groups with three to six carbon atoms.

The term "alkoxy," as used herein, refers to an alkyl group attached to the parent molecular moiety through an oxygen atom, for example, methoxy group (-OCH₃). For example, "C₁₋₃ alkoxy" denotes alkoxy groups with one to three carbon atoms.

The term "alkoxyalkyl," as used herein, refers to an alkoxy group attached through its oxygen atom to an alkyl group, which is attached to the parent molecular moiety, for example, methoxymethyl group (-CH₂OCH₃). For example, "C₂₋₄ alkoxyalkyl" denotes alkoxyalkyl groups with two to four carbon atoms, such as -CH₂OCH₃, -CH₂CH₂OCH₃, -CH₂OCH₂CH₃, and -CH₂CH₂OCH₂CH₃.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The compounds of Formula (I) can be provided as amorphous solids or crystalline solids. Lyophilization can be employed to provide the compounds of Formula (I) as amorphous solids.

It should further be understood that solvates (e.g., hydrates) of the compounds of Formula (I) are also within the scope of the present invention. The term "solvate" means a physical association of a compound of Formula (I) with one or more solvent molecules, whether organic or inorganic. This physical association includes hydrogen bonding. In certain instances the solvate will be capable of isolation, for example when one or more solvent molecules are incorporated in the crystal lattice of the crystalline solid. "Solvate" encompasses both solution-phase and isolable solvates. Exemplary solvates include hydrates, ethanolates, methanolates, isopropanolates, acetonitrile solvates, and ethyl acetate solvates. Methods of solvation are known in the art.

Various forms of prodrugs are well known in the art and are described in:
a) The Practice of Medicinal Chemistry, Camille G. Wermuth et al., Ch 31, (Academic Press, 1996);
b) Design of Prodrugs, edited by H. Bundgaard, (Elsevier, 1985);
c) *A* Textbook of Drug Design and Development, P. Krogsgaard-Larson and H. Bundgaard, eds. Ch 5, pgs 113 - 191 (Harwood Academic Publishers, 1991); and
d) Hydrolysis in Drug and Prodrug Metabolism, Bernard Testa and Joachim M. Mayer, (Wiley-VCH, 2003).

In addition, compounds of Formula (I), subsequent to their preparation, can be isolated and purified to obtain a composition containing an amount by weight equal to or greater than 99% of a compound of Formula (I) ("substantially pure"), which is then used or formulated as described herein. Such "substantially pure" compounds of Formula (I) are also contemplated herein as part of the present invention.

"Stable compound" and "stable structure" are meant to indicate a compound that is sufficiently robust to survive isolation to a useful degree of purity from a reaction mixture, and formulation into an efficacious therapeutic agent. The present invention is intended to embody stable compounds.

"Therapeutically effective amount" is intended to include an amount of a compound of the present invention alone or an amount of the combination of compounds claimed or an amount of a compound of the present invention in combination with other active ingredients effective to act as an inhibitor to TLR7/8/9, or effective to treat or prevent autoimmune and/or inflammatory disease states, such as SLE, IBD, multiple sclerosis (MS), and Sjögren's syndrome, and rheumatoid arthritis.

As used herein, "treating" or "treatment" cover the treatment of a disease-state in a mammal, particularly in a human, and include: (a) preventing the disease-state from occurring in a mammal, in particular, when such mammal is predisposed to the disease-state but has not yet been diagnosed as having it; (b) inhibiting the disease-state, i.e., arresting its development; and/or (c) relieving the disease-state, i.e., causing regression of the disease state.

The compounds of the present invention are intended to include all isotopes of atoms occurring in the present compounds. Isotopes include those atoms having the same atomic number but different mass numbers. By way of general example and without limitation, isotopes of hydrogen include deuterium (D) and tritium (T). Isotopes of carbon include ¹³C and ¹⁴C. Isotopically-labeled compounds of the invention can generally be prepared by conventional techniques known to those skilled in the art or by processes analogous to those described herein, using an appropriate isotopically-labeled reagent in place of the non-labeled reagent otherwise employed. For example, methyl (-CH₃) also includes deuterated methyl groups such as -CD₃.

### UTILITY

The human immune system has evolved to defend the body from microorganisms, viruses, and parasites that can cause infection, disease or death. Complex regulatory mechanisms ensure that the various cellular components of the immune system target the foreign substances or organisms, while not causing permanent or significant damage to the individual. While the initiating events are not well understood at this time, in autoimmune disease states the immune system directs its inflammatory response to target organs in the afflicted individual. Different autoimmune diseases are typically characterized by the predominate or initial target organ or tissues affected; such as the joint in the case of rheumatoid arthritis, the thyroid gland in the case of Hashimoto's thyroiditis, the central nervous system in the case of multiple sclerosis, the pancreas in the case of type I diabetes, and the bowel in the case of inflammatory bowel disease.

The compounds of the invention inhibit signaling through Toll-like receptor 7, or 8, or 9 (TLR7, TLR8, TLR9) or combinations thereof. Accordingly, compounds of Formula (I) have utility in treating conditions associated with the inhibition of signaling through one or more of TLR7, TLR8, or TLR9. Such conditions include TLR7, TLR8, or TLR9 receptor associated diseases in which cytokine levels are modulated as a consequence of intracellular signaling.

As used herein, the terms "treating" or "treatment" encompass the treatment of a disease state in a mammal, particularly in a human, and include: (a) preventing or delaying the occurrence of the disease state in a mammal, in particular, when such mammal is predisposed to the disease state but has not yet been diagnosed as having it; (b) inhibiting the disease state, *i.e.,* arresting its development; and/or (c) achieving a full or partial reduction of the symptoms or disease state, and/or alleviating, ameliorating, lessening, or curing the disease or disorder and/or its symptoms.

In view of their activity as selective inhibitors of TLR7, TLR8, or TLR9, compounds of Formula (I) are useful in treating TLR7, TLR8, or TLR9 family receptor associated diseases, but not limited to, inflammatory diseases such as Crohn's disease, ulcerative colitis, asthma, graft versus host disease, allograft rejection, chronic obstructive pulmonary disease; autoimmune diseases such as Graves' disease, rheumatoid arthritis, systemic lupus erythematosus, lupus nephritis, cutaneous lupus, psoriasis; auto-inflammatory diseases including Cryopyrin-Associated Periodic Syndromes (CAPS), TNF Receptor Associated Periodic Syndrome (TRAPS), Familial Mediterranean Fever (FMF), adult onset stills, systemic onset juvenile idiopathic arthritis, gout, gouty arthritis; metabolic diseases including type 2 diabetes, atherosclerosis, myocardial infarction; destructive bone disorders such as bone resorption disease, osteoarthritis, osteoporosis, multiple myeloma-related bone disorder; proliferative disorders such as acute myelogenous leukemia, chronic myelogenous leukemia; angiogenic disorders such as angiogenic disorders including solid tumors, ocular neovascularization, and infantile haemangiomas; infectious diseases such as sepsis, septic shock, and Shigellosis; neurodegenerative diseases such as Alzheimer's disease, Parkinson's disease, cerebral ischemias or neurodegenerative disease caused by traumatic injury, oncologic and viral diseases such as metastatic melanoma, Kaposi's sarcoma, multiple myeloma, and HIV infection and CMV retinitis, AIDS, respectively.

More particularly, the specific conditions or diseases that may be treated with the inventive compounds include, without limitation, pancreatitis (acute or chronic), asthma, allergies, adult respiratory distress syndrome, chronic obstructive pulmonary disease, glomerulonephritis, rheumatoid arthritis, systemic lupus erythematosus, scleroderma, chronic thyroiditis, Graves' disease, autoimmune gastritis, diabetes, autoimmune hemolytic anemia, autoimmune neutropenia, thrombocytopenia, atopic dermatitis, chronic active hepatitis, myasthenia gravis, multiple sclerosis, inflammatory bowel disease, ulcerative colitis, Crohn's disease, psoriasis, graft vs. host disease, inflammatory reaction induced by endotoxin, tuberculosis, atherosclerosis, muscle degeneration, cachexia, psoriatic arthritis, Reiter's syndrome, gout, traumatic arthritis, rubella arthritis, acute synovitis, pancreatic β-cell disease; diseases characterized by massive neutrophil infiltration; rheumatoid spondylitis, gouty arthritis and other arthritic conditions, cerebral malaria, chronic pulmonary inflammatory disease, silicosis, pulmonary sarcoidosis, bone resorption disease, allograft rejections, fever and myalgias due to infection, cachexia secondary to infection, keloid formation, scar tissue formation, ulcerative colitis, pyresis, influenza, osteoporosis, osteoarthritis, acute myelogenous leukemia, chronic myelogenous leukemia, metastatic melanoma, Kaposi's sarcoma, multiple myeloma, sepsis, septic shock, and Shigellosis; Alzheimer's disease, Parkinson's disease, cerebral ischemias or neurodegenerative disease caused by traumatic injury; angiogenic disorders including solid tumors, ocular neovascularization, and infantile haemangiomas; viral diseases including acute hepatitis infection (including hepatitis A, hepatitis B and hepatitis C), HIV infection and CMV retinitis, AIDS, ARC or malignancy, and herpes; stroke, myocardial ischemia, ischemia in stroke heart attacks, organ hypoxia, vascular hyperplasia, cardiac and renal reperfusion injury, thrombosis, cardiac hypertrophy, thrombin-induced platelet aggregation, endotoxemia and/or toxic shock syndrome, conditions associated with prostaglandin endoperoxidase syndase-2, and pemphigus vulgaris. Included in this embodiment are compounds of the invention, for use in methods of treatment in which the condition is selected from lupus including lupus nephritis and systemic lupus erythematosus (SLE), Crohn's disease, ulcerative colitis, allograft rejection, rheumatoid arthritis, psoriasis, ankylosing spondylitis, psoriatic arthritis, and pemphigus vulgaris. Also included are compounds of the invention, for use in methods of treatment in which the condition is selected from ischemia reperfusion injury, including cerebral ischemia reperfusions injury arising from stroke and cardiac ischemia reperfusion injury arising from myocardial infarction. Compounds of the invention may also be used in a method of treatment in which the condition is multiple myeloma.

In one embodiment, the compounds of Formula (I) are useful in treating cancer, including Waldenstrom's Macroglobulinemia (WM), diffuse large B cell lymphoma (DLBCL), chronic lymphocytic leukemia (CLL), cutaneous diffuse large B cell lymphoma, and primary CNS lymphoma.

In addition, the TLR7, TLR8, or TLR9 inhibitors of the present invention inhibit the expression of inducible pro-inflammatory proteins such as prostaglandin endoperoxide synthase-2 (PGHS-2), also referred to as cyclooxygenase-2 (COX-2), IL-1, IL-6, IL-18, chemokines. Accordingly, additional TLR7/8/9 associated conditions include edema, analgesia, fever and pain, such as neuromuscular pain, headache, pain caused by cancer, dental pain and arthritis pain. The inventive compounds also may be used to treat veterinary viral infections, such as lentivirus infections, including, but not limited to equine infectious anemia virus; or retrovirus infections, including feline immunodeficiency virus, bovine immunodeficiency virus, and canine immunodeficiency virus.

The present invention thus provides compounds for use in methods of treating such conditions, comprising administering to a subject in need thereof a therapeutically-effective amount of at least one compound of Formula (I) or a salt thereof. "Therapeutically effective amount" is intended to include an amount of a compound of the present invention that is effective when administered alone or in combination to inhibit autoimmune disease or chronic inflammatory disease.

The methods of treating TLR7, TLR8, or TLR9 associated conditions may comprise administering compounds of Formula (I) alone or in combination with each other and/or other suitable therapeutic agents useful in treating such conditions. Accordingly, "therapeutically effective amount" is also intended to include an amount of the combination of compounds claimed that is effective to inhibit TLR7, TLR8, or TLR9 and/or treat diseases associated with TLR7, TLR8, or TLR9.

Exemplary of such other therapeutic agents include corticosteroids, rolipram, calphostin, cytokine-suppressive anti-inflammatory drugs (CSAIDs), Interleukin-10, glucocorticoids, salicylates, nitric oxide, and other immunosuppressants; nuclear translocation inhibitors, such as deoxyspergualin (DSG); non-steroidal anti-inflammatory drugs (NSAIDs) such as ibuprofen, celecoxib and rofecoxib; steroids such as prednisone or dexamethasone; antiviral agents such as abacavir; antiproliferative agents such as methotrexate, leflunomide, FK506 (tacrolimus, PROGRAF^{®}); anti-malarials such as hydroxychloroquine; cytotoxic drugs such as azathiprine and cyclophosphamide; TNF-α inhibitors such as tenidap, anti-TNF antibodies or soluble TNF receptor, and rapamycin (sirolimus or RAPAMUNE^{®}) or derivatives thereof.

The above other therapeutic agents, when employed in combination with the compounds of the present invention, may be used, for example, in those amounts indicated in the *Physicians' Desk Reference* (PDR) or as otherwise determined by one of ordinary skill in the art. Such other therapeutic agent(s) may be administered prior to, simultaneously with, or following the administration of the inventive compounds. The present invention also provides pharmaceutical compositions capable of treating TLR7/8/9 receptor-associated conditions, including IL-1 family receptor-mediated diseases as described above.

The inventive compositions may contain other therapeutic agents as described above and may be formulated, for example, by employing conventional solid or liquid vehicles or diluents, as well as pharmaceutical additives of a type appropriate to the mode of desired administration (*e.g*., excipients, binders, preservatives, stabilizers, flavors, etc.) according to techniques such as those well known in the art of pharmaceutical formulation.

Accordingly, the present invention further includes compositions comprising one or more compounds of Formula (I) and a pharmaceutically acceptable carrier.

A "pharmaceutically acceptable carrier" refers to media generally accepted in the art for the delivery of biologically active agents to animals, in particular, mammals. Pharmaceutically acceptable carriers are formulated according to a number of factors well within the purview of those of ordinary skill in the art. These include without limitation the type and nature of the active agent being formulated; the subject to which the agent-containing composition is to be administered; the intended route of administration of the composition; and, the therapeutic indication being targeted. Pharmaceutically acceptable carriers include both aqueous and non-aqueous liquid media, as well as a variety of solid and semi-solid dosage forms. Such carriers can include a number of different ingredients and additives in addition to the active agent, such additional ingredients being included in the formulation for a variety of reasons, *e.g*., stabilization of the active agent, binders, etc., well known to those of ordinary skill in the art. Descriptions of suitable pharmaceutically acceptable carriers, and factors involved in their selection, are found in a variety of readily available sources such as, for example, Remington's Pharmaceutical Sciences, 17th Edition (1985).

Compounds in accordance with Formula (I) can be administered by any means suitable for the condition to be treated, which can depend on the need for site-specific treatment or quantity of Formula (I) compound to be delivered.

Also embraced within this invention is a class of pharmaceutical compositions comprising a compound of Formula (I) and one or more non-toxic, pharmaceutically-acceptable carriers and/or diluents and/or adjuvants (collectively referred to herein as "carrier" materials) and, if desired, other active ingredients. The compounds of Formula (I) may be administered by any suitable route, preferably in the form of a pharmaceutical composition adapted to such a route, and in a dose effective for the treatment intended. The compounds and compositions of the present invention may, for example, be administered orally, mucosally, or parenterally including intravascularly, intravenously, intraperitoneally, subcutaneously, intramuscularly, and intrasternally in dosage unit formulations containing conventional pharmaceutically acceptable carriers, adjuvants, and vehicles. For example, the pharmaceutical carrier may contain a mixture of mannitol or lactose and microcrystalline cellulose. The mixture may contain additional components such as a lubricating agent, e.g. magnesium stearate and a disintegrating agent such as crospovidone. The carrier mixture may be filled into a gelatin capsule or compressed as a tablet. The pharmaceutical composition may be administered as an oral dosage form or an infusion, for example.

For oral administration, the pharmaceutical composition may be in the form of, for example, a tablet, capsule, liquid capsule, suspension, or liquid. The pharmaceutical composition is preferably made in the form of a dosage unit containing a particular amount of the active ingredient. For example, the pharmaceutical composition may be provided as a tablet or capsule comprising an amount of active ingredient in the range of from about 0.1 to 1000 mg, preferably from about 0.25 to 250 mg, and more preferably from about 0.5 to 100 mg. A suitable daily dose for a human or other mammal may vary widely depending on the condition of the patient and other factors, but, can be determined using routine methods.

Any pharmaceutical composition contemplated herein can, for example, be delivered orally via any acceptable and suitable oral preparations. Exemplary oral preparations, include, but are not limited to, for example, tablets, troches, lozenges, aqueous and oily suspensions, dispersible powders or granules, emulsions, hard and soft capsules, liquid capsules, syrups, and elixirs. Pharmaceutical compositions intended for oral administration can be prepared according to any methods known in the art for manufacturing pharmaceutical compositions intended for oral administration. In order to provide pharmaceutically palatable preparations, a pharmaceutical composition in accordance with the invention can contain at least one agent selected from sweetening agents, flavoring agents, coloring agents, demulcents, antioxidants, and preserving agents.

A tablet can, for example, be prepared by admixing at least one compound of Formula (I) with at least one non-toxic pharmaceutically acceptable excipient suitable for the manufacture of tablets. Exemplary excipients include, but are not limited to, for example, inert diluents, such as, for example, calcium carbonate, sodium carbonate, lactose, calcium phosphate, and sodium phosphate; granulating and disintegrating agents, such as, for example, microcrystalline cellulose, sodium crosscarmellose, corn starch, and alginic acid; binding agents, such as, for example, starch, gelatin, polyvinyl-pyrrolidone, and acacia; and lubricating agents, such as, for example, magnesium stearate, stearic acid, and talc. Additionally, a tablet can either be uncoated, or coated by known techniques to either mask the bad taste of an unpleasant tasting drug, or delay disintegration and absorption of the active ingredient in the gastrointestinal tract thereby sustaining the effects of the active ingredient for a longer period. Exemplary water soluble taste masking materials, include, but are not limited to, hydroxypropyl-methylcellulose and hydroxypropyl-cellulose. Exemplary time delay materials, include, but are not limited to, ethyl cellulose and cellulose acetate butyrate.

Hard gelatin capsules can, for example, be prepared by mixing at least one compound of Formula (I) with at least one inert solid diluent, such as, for example, calcium carbonate; calcium phosphate; and kaolin.

Soft gelatin capsules can, for example, be prepared by mixing at least one compound of Formula (I) with at least one water soluble carrier, such as, for example, polyethylene glycol; and at least one oil medium, such as, for example, peanut oil, liquid paraffin, and olive oil.

An aqueous suspension can be prepared, for example, by admixing at least one compound of Formula (I) with at least one excipient suitable for the manufacture of an aqueous suspension. Exemplary excipients suitable for the manufacture of an aqueous suspension, include, but are not limited to, for example, suspending agents, such as, for example, sodium carboxymethylcellulose, methylcellulose, hydroxypropylmethylcellulose, sodium alginate, alginic acid, polyvinyl-pyrrolidone, gum tragacanth, and gum acacia; dispersing or wetting agents, such as, for example, a naturally-occurring phosphatide, e.g., lecithin; condensation products of alkylene oxide with fatty acids, such as, for example, polyoxyethylene stearate; condensation products of ethylene oxide with long chain aliphatic alcohols, such as, for example heptadecaethylene-oxycetanol; condensation products of ethylene oxide with partial esters derived from fatty acids and hexitol, such as, for example, polyoxyethylene sorbitol monooleate; and condensation products of ethylene oxide with partial esters derived from fatty acids and hexitol anhydrides, such as, for example, polyethylene sorbitan monooleate. An aqueous suspension can also contain at least one preservative, such as, for example, ethyl and n-propyl p-hydroxybenzoate; at least one coloring agent; at least one flavoring agent; and/or at least one sweetening agent, including but not limited to, for example, sucrose, saccharin, and aspartame.

Oily suspensions can, for example, be prepared by suspending at least one compound of Formula (I) in either a vegetable oil, such as, for example, arachis oil; olive oil; sesame oil; and coconut oil; or in mineral oil, such as, for example, liquid paraffin. An oily suspension can also contain at least one thickening agent, such as, for example, beeswax; hard paraffin; and cetyl alcohol. In order to provide a palatable oily suspension, at least one of the sweetening agents already described hereinabove, and/or at least one flavoring agent can be added to the oily suspension. An oily suspension can further contain at least one preservative, including, but not limited to, for example, an antioxidant, such as, for example, butylated hydroxyanisol, and alpha-tocopherol.

Dispersible powders and granules can, for example, be prepared by admixing at least one compound of Formula (I) with at least one dispersing and/or wetting agent; at least one suspending agent; and/or at least one preservative. Suitable dispersing agents, wetting agents, and suspending agents are as already described above. Exemplary preservatives include, but are not limited to, for example, anti-oxidants, e.g., ascorbic acid. In addition, dispersible powders and granules can also contain at least one excipient, including, but not limited to, for example, sweetening agents; flavoring agents; and coloring agents.

An emulsion of at least one compound of Formula (I) thereof can, for example, be prepared as an oil-in-water emulsion. The oily phase of the emulsions comprising compounds of Formula (I) may be constituted from known ingredients in a known manner. The oil phase can be provided by, but is not limited to, for example, a vegetable oil, such as, for example, olive oil and arachis oil; a mineral oil, such as, for example, liquid paraffin; and mixtures thereof. While the phase may comprise merely an emulsifier, it may comprise a mixture of at least one emulsifier with a fat or an oil or with both a fat and an oil. Suitable emulsifying agents include, but are not limited to, for example, naturally-occurring phosphatides, e.g., soy bean lecithin; esters or partial esters derived from fatty acids and hexitol anhydrides, such as, for example, sorbitan monooleate; and condensation products of partial esters with ethylene oxide, such as, for example, polyoxyethylene sorbitan monooleate. Preferably, a hydrophilic emulsifier is included together with a lipophilic emulsifier which acts as a stabilizer. It is also preferred to include both an oil and a fat. Together, the emulsifier(s) with or without stabilizer(s) make-up the so-called emulsifying wax, and the wax together with the oil and fat make up the so-called emulsifying ointment base which forms the oily dispersed phase of the cream formulations. An emulsion can also contain a sweetening agent, a flavoring agent, a preservative, and/or an antioxidant. Emulsifiers and emulsion stabilizers suitable for use in the formulation of the present invention include Tween 60, Span 80, cetostearyl alcohol, myristyl alcohol, glyceryl monostearate, sodium lauryl sulfate, glyceryl distearate alone or with a wax, or other materials well known in the art.

The compounds of Formula (I) can, for example, also be delivered intravenously, subcutaneously, and/or intramuscularly via any pharmaceutically acceptable and suitable injectable form. Exemplary injectable forms include, but are not limited to, for example, sterile aqueous solutions comprising acceptable vehicles and solvents, such as, for example, water, Ringer's solution, and isotonic sodium chloride solution; sterile oil-in-water microemulsions; and aqueous or oleaginous suspensions.

Formulations for parenteral administration may be in the form of aqueous or non-aqueous isotonic sterile injection solutions or suspensions. These solutions and suspensions may be prepared from sterile powders or granules using one or more of the carriers or diluents mentioned for use in the formulations for oral administration or by using other suitable dispersing or wetting agents and suspending agents. The compounds may be dissolved in water, polyethylene glycol, propylene glycol, ethanol, corn oil, cottonseed oil, peanut oil, sesame oil, benzyl alcohol, sodium chloride solution, tragacanth gum, and/or various buffers. Other adjuvants and modes of administration are well and widely known in the pharmaceutical art. The active ingredient may also be administered by injection as a composition with suitable carriers including saline, dextrose, or water, or with cyclodextrin (i.e. Captisol), cosolvent solubilization (i.e. propylene glycol) or micellar solubilization (i.e. Tween 80).

The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed, including synthetic mono- or diglycerides. In addition, fatty acids such as oleic acid find use in the preparation of injectables.

A sterile injectable oil-in-water microemulsion can, for example, be prepared by 1) dissolving at least one compound of Formula (I) in an oily phase, such as, for example, a mixture of soybean oil and lecithin; 2) combining the Formula (I) containing oil phase with a water and glycerol mixture; and 3) processing the combination to form a microemulsion.

A sterile aqueous or oleaginous suspension can be prepared in accordance with methods already known in the art. For example, a sterile aqueous solution or suspension can be prepared with a non-toxic parenterally-acceptable diluent or solvent, such as, for example, 1,3-butane diol; and a sterile oleaginous suspension can be prepared with a sterile non-toxic acceptable solvent or suspending medium, such as, for example, sterile fixed oils, e.g., synthetic mono- or diglycerides; and fatty acids, such as, for example, oleic acid.

Pharmaceutically acceptable carriers, adjuvants, and vehicles that may be used in the pharmaceutical compositions of this invention include, but are not limited to, ion exchangers, alumina, aluminum stearate, lecithin, self-emulsifying drug delivery systems (SEDDS) such as d-alpha-tocopherol polyethyleneglycol 1000 succinate, surfactants used in pharmaceutical dosage forms such as Tweens, polyethoxylated castor oil such as CREMOPHOR surfactant (BASF), or other similar polymeric delivery matrices, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat. Cyclodextrins such as alpha-, beta-, and gamma-cyclodextrin, or chemically modified derivatives such as hydroxyalkylcyclodextrins, including 2- and 3-hydroxypropyl-cyclodextrins, or other solubilized derivatives may also be advantageously used to enhance delivery of compounds of the formulae described herein.

The pharmaceutically active compounds of this invention can be processed in accordance with conventional methods of pharmacy to produce medicinal agents for administration to patients, including humans and other mammals. The pharmaceutical compositions may be subjected to conventional pharmaceutical operations such as sterilization and/or may contain conventional adjuvants, such as preservatives, stabilizers, wetting agents, emulsifiers, buffers etc. Tablets and pills can additionally be prepared with enteric coatings. Such compositions may also comprise adjuvants, such as wetting, sweetening, flavoring, and perfuming agents.

The amounts of compounds that are administered and the dosage regimen for treating a disease condition with the compounds and/or compositions of this invention depends on a variety of factors, including the age, weight, sex, the medical condition of the subject, the type of disease, the severity of the disease, the route and frequency of administration, and the particular compound employed. Thus, the dosage regimen may vary widely, but can be determined routinely using standard methods. A daily dose of about 0.001 to 100 mg/kg body weight, preferably between about 0.0025 and about 50 mg/kg body weight and most preferably between about 0.005 to 10 mg/kg body weight, may be appropriate. The daily dose can be administered in one to four doses per day. Other dosing schedules include one dose per week and one dose per two day cycle.

For therapeutic purposes, the active compounds of this invention are ordinarily combined with one or more adjuvants appropriate to the indicated route of administration. If administered orally, the compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose.

Pharmaceutical compositions of this invention comprise at least one compound of Formula (I) and optionally an additional agent selected from any pharmaceutically acceptable carrier, adjuvant, and vehicle. Alternate compositions of this invention comprise a compound of the Formula (I) described herein, or a prodrug thereof, and a pharmaceutically acceptable carrier, adjuvant, or vehicle.

Also disclosed is an article of manufacture. As used herein, article of manufacture is intended to include, but not be limited to, kits and packages. The article of manufacture may comprise: (a) a first container; (b) a pharmaceutical composition located within the first container, wherein the composition, comprises: a first therapeutic agent, comprising: a compound of the present invention or a pharmaceutically acceptable salt form thereof; and (c) a package insert stating that the pharmaceutical composition can be used for the treatment of an inflammatory disorder and/or an autoimmune disease (as defined previously). Alternatively, the package insert may state that the pharmaceutical composition can be used in combination (as defined previously) with a second therapeutic agent to treat an inflammatory disorder and/or an autoimmune disease. The article of manufacture can further comprise: (d) a second container, wherein components (a) and (b) are located within the second container and component (c) is located within or outside of the second container. Located within the first and second containers means that the respective container holds the item within its boundaries.

The first container is a receptacle used to hold a pharmaceutical composition. This container can be for manufacturing, storing, shipping, and/or individual/bulk selling. First container is intended to cover a bottle, jar, vial, flask, syringe, tube *(e.g.,* for a cream preparation), or any other container used to manufacture, hold, store, or distribute a pharmaceutical product.

The second container is one used to hold the first container and, optionally, the package insert. Examples of the second container include, but are not limited to, boxes *(e.g.,* cardboard or plastic), crates, cartons, bags *(e.g.,* paper or plastic bags), pouches, and sacks. The package insert can be physically attached to the outside of the first container via tape, glue, staple, or another method of attachment, or it can rest inside the second container without any physical means of attachment to the first container. Alternatively, the package insert is located on the outside of the second container. When located on the outside of the second container, it is preferable that the package insert is physically attached via tape, glue, staple, or another method of attachment. Alternatively, it can be adjacent to or touching the outside of the second container without being physically attached.

The package insert is a label, tag, marker, etc. That recites information relating to the pharmaceutical composition located within the first container. The information recited will usually be determined by the regulatory agency governing the area in which the article of manufacture is to be sold *(e.g.,* the United States Food and Drug Administration). The package insert may specifically recite the indications for which the pharmaceutical composition has been approved. The package insert may be made of any material on which a person can read information contained therein or thereon. For example, the package insert is a printable material (*e.g*., paper, plastic, cardboard, foil, adhesive-backed paper or plastic, etc.) on which the desired information has been formed *(e.g.,* printed or applied).

### METHODS OF PREPARATION

The compounds of the present invention can be prepared in a number of ways well known to one skilled in the art of organic synthesis. The compounds of the present invention can be synthesized using the methods described below, together with synthetic methods known in the art of synthetic organic chemistry, or variations thereon as appreciated by those skilled in the art. Preferred methods include, but are not limited to, those described below.

The compounds of this invention may be prepared using the reactions and techniques described in this section. The reactions are performed in solvents appropriate to the reagents and materials employed and are suitable for the transformations being effected. Also, in the description of the synthetic methods described below, it is to be understood that all proposed reaction conditions, including choice of solvent, reaction atmosphere, reaction temperature, duration of the experiment and work up procedures, are chosen to be the conditions standard for that reaction, which should be readily recognized by one skilled in the art. It is understood by one skilled in the art of organic synthesis that the functionality present on various portions of the molecule must be compatible with the reagents and reactions proposed. Such restrictions to the substituents that are compatible with the reaction conditions will be readily apparent to one skilled in the art and alternate methods must then be used. This will sometimes require a judgment to modify the order of the synthetic steps or to select one particular process scheme over another in order to obtain a desired compound of the invention. It will also be recognized that another major consideration in the planning of any synthetic route in this field is the judicious choice of the protecting group used for protection of the reactive functional groups present in the compounds described in this invention. An authoritative account describing the many alternatives to the trained practitioner is Greene and Wuts (Protective Groups In Organic Synthesis, Third Edition, Wiley and Sons, 1999).

### EXAMPLES

Preparation of compounds of Formula (I), and intermediates used in the preparation of compounds of Formula (I), can be prepared using procedures shown in the following Examples and related procedures. The methods and conditions used in these examples, and the actual compounds prepared in these Examples, are not meant to be limiting, but are meant to demonstrate how the compounds of Formula (I) can be prepared. Starting materials and reagents used in these examples, when not prepared by a procedure described herein, are generally either commercially available, or are reported in the chemical literature, or may be prepared by using procedures described in the chemical literature.

### ABBREVIATIONS

- Ac: acetyl
- AcOH: acetic acid
- ACN: acetonitrile
- anhyd.: anhydrous
- aq.: aqueous
- Bn: benzyl
- Boc-anhydride: di-*tert*-butyl dicarbonate
- Bu: butyl
- Boc: *tert*-butoxycarbonyl
- CV: Column Volumes
- DCE: dichloroethane
- DCM: dichloromethane
- DIPEA: diisopropylethylamine
- DMAP: dimethylaminopyridine
- DMF: dimethylformamide
- DMSO: dimethylsulfoxide
- EtOAc: ethyl acetate
- Et: ethyl
- EtOH: ethanol
- Et₃N: triethylamine
- H or H₂: hydrogen
- h, hr or hrs: hour(s)
- HATU: O-(7-azabenzotriazol-1-yl)-N,N,N',N'-tetramethyluronium hexafluorophosphate
- hex: hexane
- i: iso
- HCl: hydrochloric acid
- HPLC: high pressure liquid chromatography
- IPA: isopropyl alcohol
- LC: liquid chromatography
- LCMS: liquid chromatography-mass spectroscopy
- LiAlH₄: lithium aluminum hydride
- M: molar
- mM: millimolar
- Me: methyl
- MeOH: methanol
- MHz: megahertz
- min.: minute(s)
- mins: minute(s)
- M⁺¹: (M+H)⁺
- MS: mass spectrometry
- n or N: normal
- NBS: n-bromosuccinimide
- NCS: n-chlorosuccinimide
- NIS: n-iodosuccinimide
- nm: nanometer
- nM: nanomolar
- NMP: *N*-methylpyrrolidinone
- Pd/C: palladium on carbon
- PdCl₂(dppf): [1,1'-*bis*(diphenylphosphino)ferrocene]dichloropalladium(II)
- Pet ether: petroleum ether
- Ph: phenyl
- Pr: propyl
- PSI: pounds per square inch
- Ret Time: retention time
- sat.: saturated
- SFC: supercritical fluid chromatography
- TEA: triethylamine
- TFA: trifluoroacetic acid
- THF: tetrahydrofuran
- XPhos Precatalyst: chloro(2-dicyclohexylphosphino-2',4',6'-triisopropyl-1,1'-biphenyl)[2-(2'-amino-1,1'-biphenyl)]palladium(II)

### Analytical and preparative HPLC/LCMS conditions

Method A: Column: Acquity UPLC BEH C18, 3.0 x 50 mm, 1.7 µm particles; Mobile Phase A: 5:95 acetonitrile: water with 5 mM ammonium acetate; Mobile Phase B: 95:5 acetonitrile: water with 5 mM ammonium acetate; Method: %B: O min-20%: 1.1min -90%:1.7 min-90%; Flow: 0.7 mL/min.

Method B: Kinetex XB-C18 (75 x 3 mm) 2.6 micron; Solvent A: 10 mM ammonium formate in water: Acetonitrile (98:2); Mobile Phase B: 10 mM ammonium formate in water: acetonitrile (2:98); Temperature: 50 °C; Gradient: 0-100% B over 3 minutes; Flow rate: 1.1 mL/min; Detection: UV at 220 nm.

Method C: Waters XBridge BEH C18 XP(50 x 2.1 mm) 2.5 µm; Mobile Phase A: 5:95 acetonitrile:water with 10 mM NH₄OAc; Mobile Phase B: 95:5 acetonitrile: water with 10 mM NH₄OAc; Temperature: 50 °C; Gradient: 0-100% B over 3 minutes; Flow: 1.1 mL/min.

Method D: Waters XBridge BEH C18 XP(50 x 2.1 mm) 2.5 µm; Mobile Phase A: 5:95 acetonitrile: water with 0.1% TFA; Mobile Phase B: 95:5 acetonitrile: water with 0.1% TFA; Temperature: 50 °C; Gradient: 0-100% B over 3 minutes; Flow: 1.1 mL/min.

Method E: Ascentis Express C18(50 x 2.1 mm) 2.7 µm; Mobile Phase A: 5:95 acetonitrile:water with 10 mM NH₄OAc; Mobile Phase B: 95:5 acetonitrile: water with 10 mM NH₄OAc; Temperature: 50 °C; Gradient: 0-100% B over 3 minutes; Flow: 1.1 mL/min.

Method F: Ascentis Express C18(50 x 2.1 mm) 2.7 µm; Mobile Phase A: 5:95 acetonitrile:water with 0.1% TFA; Mobile Phase B: 95:5 acetonitrile: water with 0.1% TFA; Temperature: 50 °C; Gradient: 0-100% B over 3 minutes; Flow: 1.1 mL/min.

Method G: Column: Waters Acquity UPLC BEH C18 (2.1 x 50 mm), 1.7 micron; Solvent A = 100% water with 0.05% TFA; Solvent B = 100% acetonitrile with 0.05% TFA; gradient = 2-98% B over 1 minute, then a 0.5-minute hold at 98% B; Flow rate: 0.8 mL/min; Detection: UV at 220 nm.

Method H: Column: ZORBAX SB AQ (4.6 x 50) mm, 3.5micron, Solvent A: acetonitrile (98:2); Mobile Phase B: 10 mM ammonium formate in water: acetonitrile (2:98); Temperature: 50 °C; Gradient: 0-100% B over 3 minutes; Flow rate: 1.1 mL/min; Detection: UV at 220 nm.

Method AA: Waters XBridge C18, 19 x 150 mm, 5 µm particles; Mobile Phase A: 10 mM ammonium acetate; Mobile Phase B: methanol; Gradient: 10-45% B over 20 minutes, then a 5-minutehold at 100% B; Flow: 20 mL/min.

Method AB: Waters XBridge C18, 19 x 150 mm, 5 µm particles; Mobile Phase A: 0.1% trifluoroacetic acid; Mobile Phase B: acetonitrile; Gradient: 5-25% B over 25 minutes, then a 5-minutehold at 100% B; Flow: 15 mL/min.

Method AC: Column: X bridge C18 (250 X19, 5µm), Mobile Phase-A: 0.1% TFA in water, Mobile Phase-B: ACN, isocratic 0/10,10/60, Flow: 17 mL/min

Method AD: Chiral SFC: Column: Lux Cellulose-4 (250 x 4.6) mm; 5 µm, solvent, ACN (1:1), Co-Solvent; 0.2% NH₄OH in MeOH, 20 4 g-50%-100 bar, 30 min, Flow 20 ml/min.

Method AF: Chiral SFC Conditions: Column: Luxcellulose-4 (250 X 21.5) mm, 5 µm, % CO₂: 50%, % Co solvent: 50% of 0.2% NH₄OH in MeOH + ACN (1:1), Total Flow: 70.0 g/min, Back Pressure: 100 bar, Temperature: 30 °C, UV: 290 nm.

Examples 175, 178, 188-189 and 876 are provided for reference purposes only, and do not form part of the present invention.

### EXAMPLE 1

### tert-butyl 4-(4-isopropyl-5-(8-methyl-[1,2,4]triazolo[1,5-a]pyridin-6-yl)-1H-pyrazole-3-carboxamido)piperidine-1-carboxylate

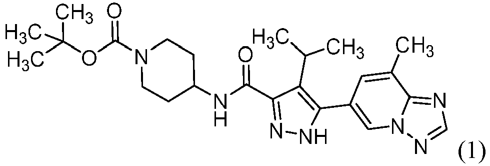

### Intermediate 1A: Methyl 4-bromo-1-((2-(trimethylsilyl)ethoxy)methyl)-1H-pyrazole-3-carboxylate

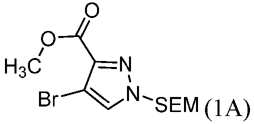

To a solution of methyl 4-bromo-1H-pyrazole-3-carboxylate (10.0 g, 48.8 mmol) in THF (150.00 mL) was added NaH (2.341 g, 58.5 mmol) portion wise at 0 °C. The reaction mixture was stirred for 30 min, then SEM-Cl (10.38 mL, 58.5 mmol) was added. The reaction mixture was stirred at 0 °C for 5 h. The reaction was quenched with water (30 mL). The reaction mixture separated into two layers, and the aqueous layer was extracted with EtOAc (2 X 50 mL). The combined organic extracts were dried (Na₂SO₄) and concentrated to yield crude compound. The crude compound was purified by ISCO using 120 g silica column, the compound was eluted in 20% EA in hexanes, the fractions were collected and concentrated to afford methyl 4-bromo-1-((2-(trimethylsilyl)ethoxy)methyl)-1H-pyrazole-3-carboxylate (13.95 g, 41.6 mmol, 85 % yield) as an oil. LCMS Retention time: 1.63 min [A], MS (E⁺) m/z: 337.1 [M+2H].

### Intermediate 1B: Methyl 4-(prop-1-en-2-yl)-1-((2-(trimethylsilyl)ethoxy)methyl)-1H-pyrazole-3-carboxylate

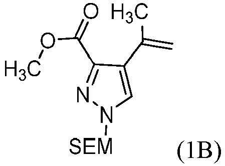

A mixture of methyl 4-bromo-1-((2-(trimethylsilyl)ethoxy)methyl)-1H-pyrazole-3-carboxylate (13.9 g, 41.5 mmol), 4,4,5,5-tetramethyl-2-(prop-1-en-2-yl)-1,3,2-dioxaborolane (23.38 mL, 124 mmol) and potassium phosphate tribasic (26.4 g, 124 mmol) in dioxane (150.00 mL) and water (50.0 mL) was degassed with nitrogen for 10 min. Next, PdCl₂(dppf)-CH₂Cl₂ adduct (3.39 g, 4.15 mmol) was added and the reaction mixture was stirred at 90 °C for 16 h. The reaction mixture was cooled to room temperature, separated into two layers. The aqueous layer was extracted with DCM (2 X 50 mL) and the combined organic extracts were dried (Na₂SO₄) and concentrated to yield crude compound. The crude compound was purified by ISCO using 40 g silica column, the compound was eluted in 15-20% EA in hexane, the fractions were collected and concentrated to afford methyl 4-(prop-1-en-2-yl)-1-((2-(trimethylsilyl)ethoxy)methyl)-1H-pyrazole-3-carboxylate (11.550 g, 39.0 mmol, 94 % yield) as an oil. LCMS Retention time: 1.46 min [A], MS (E⁺) m/z: m/z= 297.5 [M+H].

### Intermediate 1C: Methyl 4-isopropyl-1-((2-(trimethylsilyl)ethoxy)methyl)-1H-pyrazole-3-carboxylate

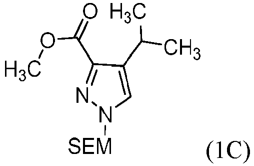

To a solution of methyl 4-(prop-1-en-2-yl)-1-((2-(trimethylsilyl)ethoxy)methyl)-1H-pyrazole-3-carboxylate (11.55 g, 39.0 mmol) in MeOH (120.00 mL) was added Pd-C (4.15 g, 39.0 mmol) at room temperature. The mixture was stirred at room temperature under a hydrogen bladder for 3 h. The reaction mass was filtered through celite, the filtrate was collected, concentrated and dried under vacuum to afford methyl 4-isopropyl-1-((2-(trimethylsilyl)ethoxy)methyl)-1H-pyrazole-3-carboxylate (11.5 g, 38.5 mmol, 99 % yield) as an oil. LCMS Retention time: 1.56 min [A], MS (E⁺) m/z = 299.5 [M+H].

### Intermediate 1D: methyl 4-isopropyl-1H-pyrazole-3-carboxylate

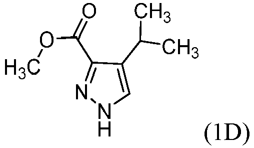

Methyl 4-isopropyl-1-((2-(trimethylsilyl)ethoxy)methyl)-1H-pyrazole-3-carboxylate (11.50 g, 38.5 mmol) in 4 M hydrochloric acid in dioxane (50.00 mL, 200 mmol) was stirred at 70 °C for 16 h. The reaction mass was concentrated, the residue was dissolved in DCM (50 mL), brought to a basic pH with 0.5 M NaOH, separated both the layers, the aqueous layer was extracted with DCM (2 X 50 mL), the combined organic extracts were washed with water (50 mL), brine (10 mL), dried (Na₂SO₄), the combined organic extracts were concentrated and dried under vacuum to yield methyl 4-isopropyl-1H-pyrazole-3-carboxylate (6.12 g, 36.4 mmol, 94 % yield) as an off-white solid. LCMS Retention time: 0.89 min [A], MS (E⁺) m/z = 169.4 [M+H].

### Intermediate 1E: Methyl 5-iodo-4-isopropyl-1H-pyrazole-3-carboxylate

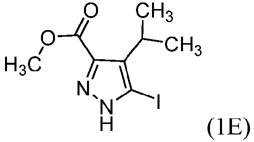

To a solution of methyl 4-isopropyl-1H-pyrazole-3-carboxylate (3.75 g, 22.30 mmol) in acetonitrile (150 mL) was added NIS (12.54 g, 55.7 mmol) at room temperature. The reaction mixture was stirred at 70 °C for 2 days. The reaction mass was concentrated, the residue was diluted with DCM, the precipitated out solid was filtered and washed with DCM, the filtrates were collected and concentrated to get crude compound. The crude compound was purified by ISCO using 40 g silica column, the compound was eluted in 25% EA in hexane, the fractions were collected and concentrated to get methyl 5-iodo-4-isopropyl-1H-pyrazole-3-carboxylate (4.11 g, 13.98 mmol, 63 % yield) as an off-white solid. LCMS Retention time: 1.68 min [A], MS (E⁺) m/z= 293.1 [M-H].

### Intermediate 1F: Methyl 4-isopropyl-5-(8-methyl-[1,2,4]triazolo[1,5-a]pyridin-6-yl)-1H-pyrazole-3-carboxylate

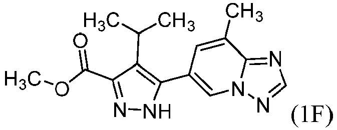

A mixture of methyl 5-iodo-4-isopropyl-1H-pyrazole-3-carboxylate (2.00 g, 6.80 mmol), 8-methyl-6-(4,4,5,5-tetramethyl-1,3,2-dioxaborolan-2-yl)-[1,2,4]triazolo[1,5-a]pyridine (2.64 g, 10.20 mmol) and potassium phosphate tribasic (4.33 g, 20.40 mmol) in dioxane (30.0 mL) and water (10.0 mL) was degassed for 10 min. Next, PdCl₂(dppf)-CH₂Cl₂ adduct (0.555 g, 0.680 mmol) was added and the reaction mixture was again degassed for 2 min. The reaction mixture was stirred at 90 °C for 16 h. The reaction mixture was diluted with DCM (20 mL) and water (10 mL). The two layers were separated, the aqueous layer was extracted with 10% MeOH in DCM (2 X 30 mL), the combined organic extracts were dried (Na₂SO₄) and concentrated to get crude compound. The crude compound was purified by ISCO using 24 g silica column, the compound was eluted in EA, the fractions were collected and concentrated to get methyl 4-isopropyl-5-(8-methyl-[1,2,4]triazolo[1,5-a]pyridin-6-yl)-1H-pyrazole-3-carboxylate (1.12 g, 3.74 mmol, 55 % yield) as an off-white solid. LCMS Retention time: 0.92 min [G], MS (ES): m/z= 300.5 [M+H].

The following Intermediates were prepared according to the general procedure described in Intermediate 1F.

**TABLE 1**

| Intermediate | Structure | Mol Wt. | LCMS MH⁺ | RT (min) | HPLC Method |
|---|---|---|---|---|---|
| 1G | 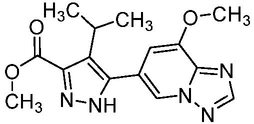 | 315.33 | 316.4 | 0.92 | A |
| 1H | 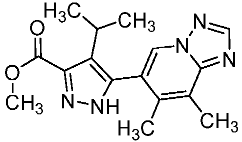 | 313.15 | 314.1 | 1.80 | B |
| 1I | 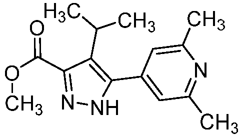 | 273.2 | 274.2 | 1.03 | A |
| 1J | 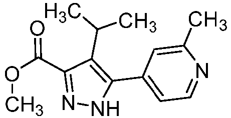 | 259.3 | 260.1 | 1.84 | B |
| 1K | 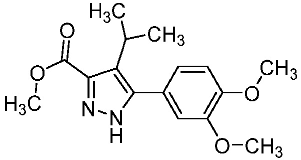 | 304.1 | 305.1 | 2.26 | A |
| 1L | 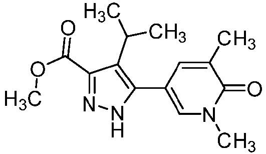 | 289.2 | 290.3 | 1.61 | B |
| 1M | 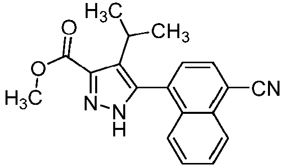 | 320.2 | 321.2 | 2.33 | B |
| 1N | 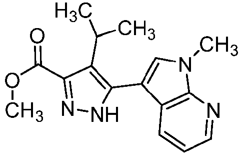 | 298.35 | 299.5 | 1.07 | A |
| 1O | 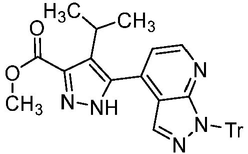 | 527.2 | 528.2 | 3.94 | B |
| 1P | 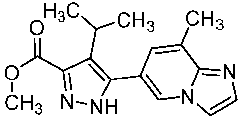 | 298.2 | 299.4 | 0.93 | A |

### Intermediate 1Q: 4-isopropyl-5-(8-methyl-[1,2,4]triazolo[1,5-a]pyridin-6-yl)-1H-pyrazole-3-carboxylic acid

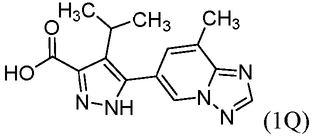

To a solution of methyl 4-isopropyl-5-(8-methyl-[1,2,4]triazolo[1,5-a]pyridin-6-yl)-1H-pyrazole-3-carboxylate (1.00 g, 3.34 mmol) in THF (10.00 mL) and MeOH (10.00 mL) was added 1 M NaOH (10.02 mL, 10.02 mmol) at room temperature. The reaction mixture was stirred at 80 °C for 16 h. The reaction mass was concentrated to remove THF and MeOH, brought to acidic pH using 0.5 M HCl, the precipitated out solid was filtered, washed with water and then dried under vacuum to get 4-isopropyl-5-(8-methyl-[1,2,4]triazolo[1,5-a]pyridin-6-yl)-1H-pyrazole-3-carboxylic acid (0.930 g, 3.26 mmol, 98 % yield) as an off-white solid. LCMS Retention time: 0.46 min [G], MS (ES): m/z= 286.4 [M+H].

### Example 1: tert-butyl 4-(4-isopropyl-5-(8-methyl-[1,2,4]triazolo[1,5-a]pyridin-6-yl)-1H-pyrazole-3-carboxamido)piperidine-1-carboxylate

To a solution of 4-isopropyl-5-(8-methyl-[1,2,4]triazolo[1,5-a]pyridin-6-yl)-1H-pyrazole-3-carboxylic acid (0.500 g, 1.752 mmol) and tert-butyl 4-aminopiperidine-1-carboxylate (0.526 g, 2.63 mmol) in DCM (20.00 mL) were added TEA (3.00 mL, 21.52 mmol) and 1-propanephosphonic anhydride (5.00 mL, 8.40 mmol) at room temperature. The mixture was stirred at room temperature for 16 h. The reaction was quenched with the addition of water (10 mL). The two layers were separated, the aqueous layer was extracted with DCM (2 X 30 mL), the combined organic extracts were dried (Na₂SO₄) and concentrated to afford the crude compound. The crude compound was purified by ISCO using a 24 g silica column, the compound was eluted in EA, the fractions were collected and concentrated to yield tert-butyl 4-(4-isopropyl-5-(8-methyl-[1,2,4]triazolo[1,5-a]pyridin-6-yl)-1H-pyrazole-3-carboxamido)piperidine-1-carboxylate (0.610 g, 1.305 mmol, 74% yield) as an off-white solid. LCMS Retention time: 0.92 min [A], MS (ES): m/z= 468.6 [M+H]; ¹H NMR (400 MHz, CD₃OD) δ ppm 8.72 (s, 1 H), 8.49 (s, 1 H), 7.54 (s, 1 H), 4.18-4.04 (m, 3 H), 3.02 - 2.88 (m, 2 H), 2.68 (s, 3 H), 1.99 - 1.91 (m, 2 H), 1.61-1.50 (m, 3 H), 1.48 (s, 9H), 1.34 (d, J = 7.2 Hz, 6 H).

The following Examples were prepared according to the general procedure described in Example 1.

**TABLE 2**

| Ex. No. | Structure | Mol Wt. | LCMS MH⁺ | RT (min) | HPLC Method |
|---|---|---|---|---|---|
| 2 | 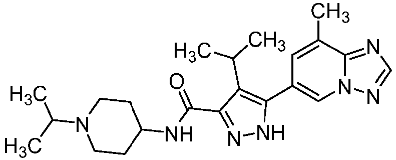 | 409.5 | 410.2 | 1.199 | E |
| 3 | 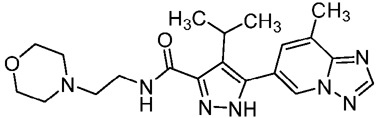 | 397.4 | 398.2 | 1.047 | E |
| 4 | 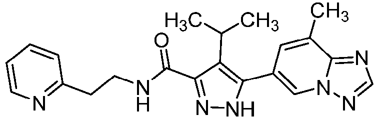 | 389.4 | 390.2 | 0.808 | F |
| 5 | 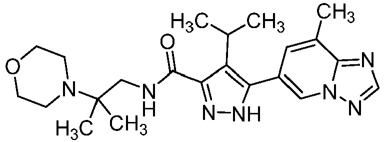 | 425.5 | 426.3 | 0.904 | F |
| 6 | 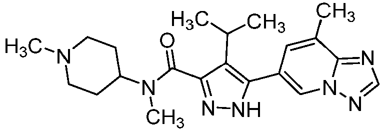 | 395.5 | 396.3 | 0.721 | F |
| 7 | 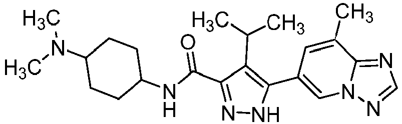 | 409.5 | 410.3 | 0.809 | E |
| 8 | 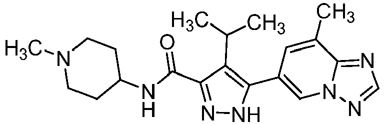 | 381.4 | 382.3 | 0.811 | E |
| 9 | 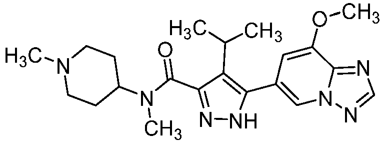 | 411.5 | 412.1 | 0.98 | E |
| 10 | 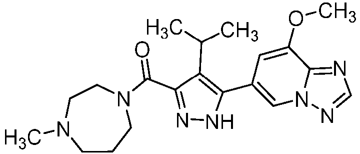 | 397.4 | 398.1 | 1.04 | E |
| 11 | 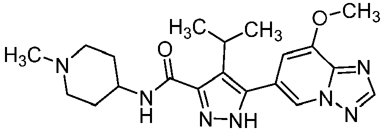 | 397.4 | 398.1 | 1.023 | E |
| 12 | 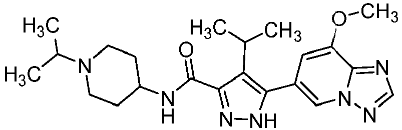 | 425.5 | 426.1 | 1.142 | E |
| 13 | 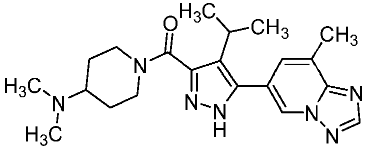 | 395.5 | 396.3 | 0.813 | E |
| 14 | 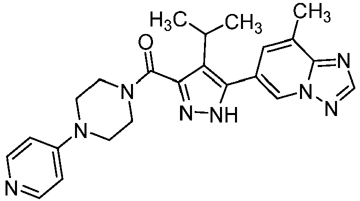 | 430.5 | 431.3 | 0.911 | E |
| 15 | 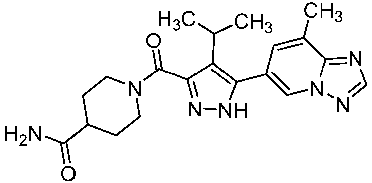 | 395.4 | 396.3 | 0.834 | F |
| 16 | 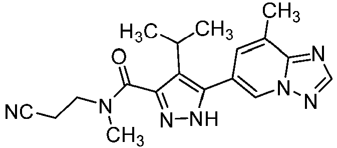 | 351.4 | 352.3 | 0.994 | F |
| 17 | 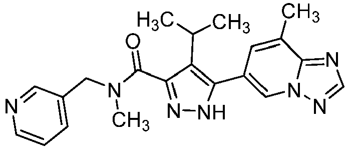 | 389.4 | 390.3 | 0.801 | F |
| 18 | 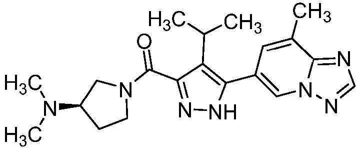 | 381.4 | 382.2 | 0.987 | E |
| 19 | 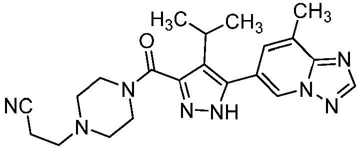 | 406.4 | 407.3 | 0.772 | F |
| 20 | 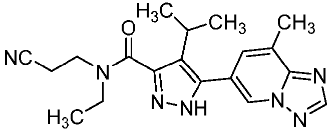 | 365.4 | 366.2 | 1.138 | A |
| 21 | 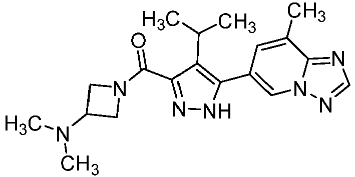 | 367.4 | 368.3 | 1.034 | E |
| 22 | 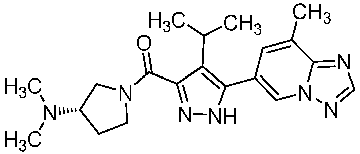 | 381.4 | 382.3 | 0.991 | E |
| 23 | 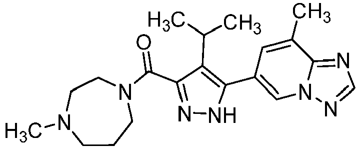 | 381.4 | 382.3 | 0.895 | E |
| 24 | 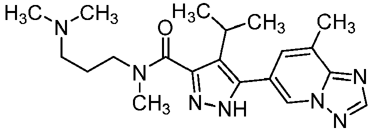 | 383.4 | 384.3 | 0.803 | E |
| 25 | 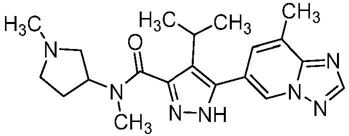 | 381.4 | 382.3 | 0.777 | F |
| 26 | 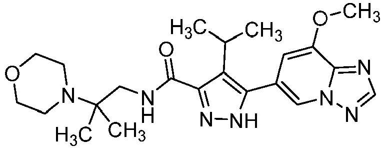 | 441.5 | 442.1 | 1.48 | E |
| 27 | 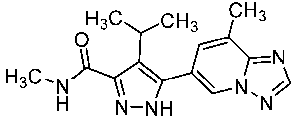 | 298.3 | 299.1 | 1.29 | E |
| 28 | 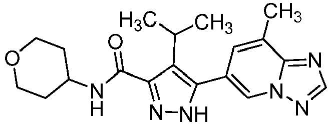 | 368.4 | 369.2 | 1.43 | E |
| 29 | 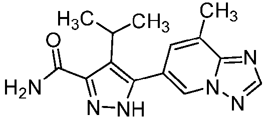 | 284.3 | 285.2 | 1.16 | F |
| 30 | 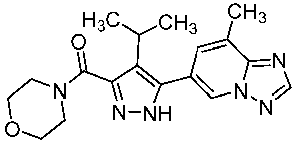 | 354.4 | 355.2 | 1 | E |
| 31 | 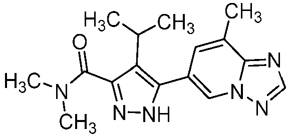 | 312.3 | 313.2 | 0.98 | E |
| 32 | 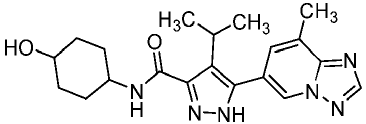 | 382.4 | 383.2 | 1 | E |
| 33 | 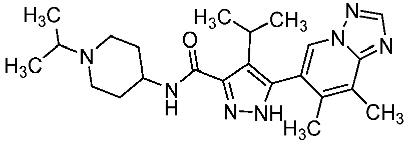 | 423.5 | 424.2 | 0.977 | E |
| 34 | 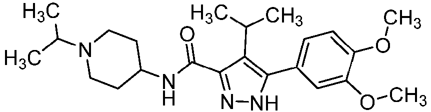 | 414.5 | 415.2 | 1.16 | E |
| 35 | 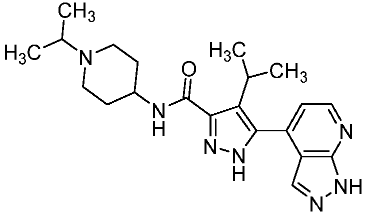 | 395.5 | 396.3 | 0.83 | E |
| 36 | 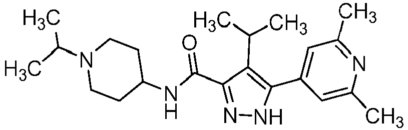 | 383.5 | 384.3 | 1.14 | E |
| 37 | 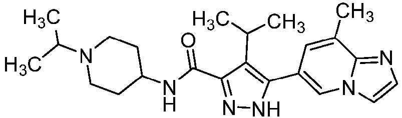 | 408.5 | 409.3 | 0.93 | E |
| 38 | 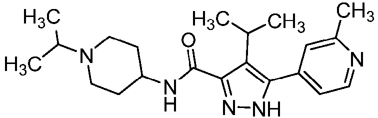 | 369.5 | 370.3 | 0.94 | E |
| 39 | 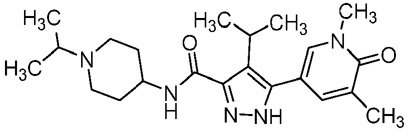 | 399.5 | 400.2 | 0.86 | E |
| 40 | 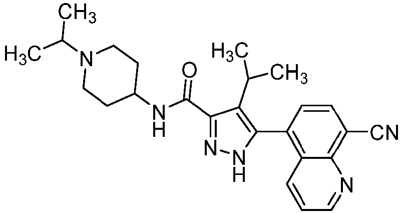 | 430.5 | 431.2 | 1.12 | E |
| 41 | 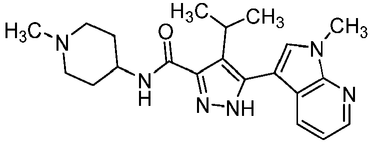 | 380.4 | 381.3 | 1.111 | E |
| 42 | 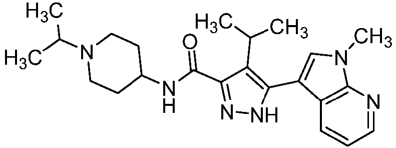 | 408.5 | 409.3 | 1.176 | E |

### EXAMPLE 43

### 4-isopropyl-5-(8-methyl-[1,2,4]triazolo[1,5-a]pyridin-6-yl)-N-(piperidin-4-yl)-1H-pyrazole-3-carboxamide

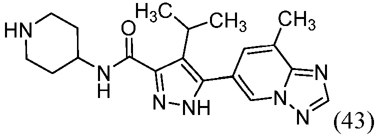

To a solution of tert-butyl 4-(4-isopropyl-5-(8-methyl-[1,2,4]triazolo[1,5-a]pyridin-6-yl)-1H-pyrazole-3-carboxamido)piperidine-1-carboxylate (0.630 g, 1.347 mmol) in dioxane (5.00 mL) was added 4 M hydrochloric acid in dioxane (5.00 mL, 20.00 mmol) at room temperature. The reaction mixture was stirred at room temperature for 3 h. The reaction mass was concentrated to yield the crude compound. The crude compound was triturated with diethyl ether (2 X 10 mL), and then dried under vacuum to yield 4-isopropyl-5-(8-methyl-[1,2,4]triazolo[1,5-a]pyridin-6-yl)-N-(piperidin-4-yl)-1H-pyrazole-3-carboxamide (0.462 g, 1.257 mmol, 93 % yield) as a white solid. LCMS Retention time: 0.92 min [G], MS (ES): m/z= 368.6 [M+H]; ¹H NMR (400 MHz, CD₃OD) δ ppm 8.74 (s, 1 H), 8.49 (s, 1 H), 7.54 (s, 1 H), 4.16-4.10 (m, 1 H), 3.46-3.38 (m, 2 H), 3.37-3.34 (m, 1 H), 3.15-3.06 (m, 2 H), 2.70 (s, 3 H), 2.23-2.12 (m, 2 H), 1.87-1.76 (m, 2 H), 1.34 (d, J = 7.2 Hz, 6 H).

The following Examples were prepared according to the general procedure described in Example 43.

**TABLE 3**

| Ex. No. | Structure | Mol Wt. | LCMS MH⁺ | RT (min) | HPLC Method |
|---|---|---|---|---|---|
| 45 | 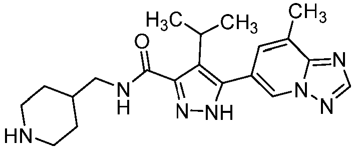 | 381.4 | 382.3 | 0.807 | F |
| 46 | 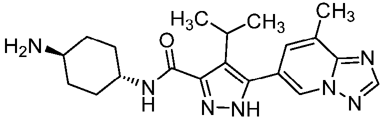 | 381.4 | 382.3 | 0.844 | E |
| 47 | 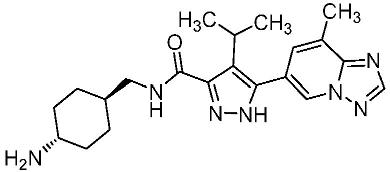 | 395.5 | 396.3 | 0.839 | E |
| 48 | 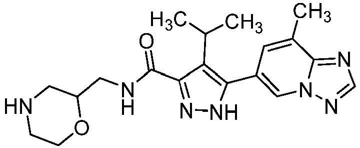 | 383.4 | 384.2 | 0.791 | E |
| 49 | 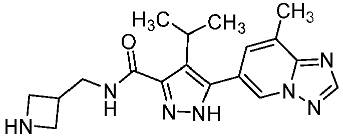 | 353.4 | 354.3 | 0.76 | F |
| 50 | 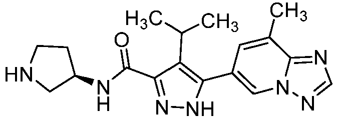 | 353.4 | 354.3 | 0.78 | F |
| 51 | 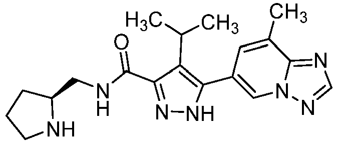 | 367.4 | 368.3 | 0.837 | F |
| 52 | 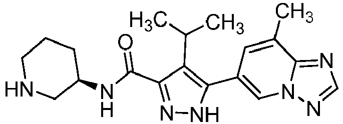 | 367.4 | 368.2 | 0.795 | E |
| 53 | 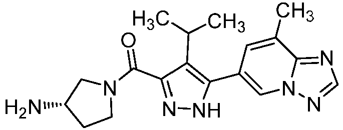 | 353.4 | 354.2 | 0.718 | E |
| 54 | 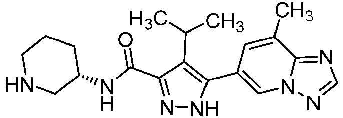 | 367.4 | 368.2 | 0.769 | E |
| 55 | 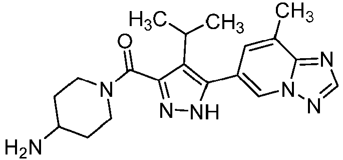 | 367.4 | 368.3 | 0.705 | F |
| 56 | 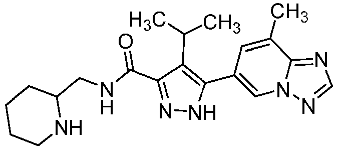 | 381.4 | 382.3 | 0.892 | F |
| 57 | 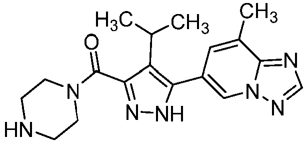 | 353.4 | 354.3 | 0.721 | E |
| 58 | 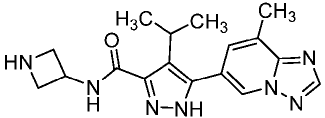 | 339.3 | 340.2 | 0.712 | E |
| 59 | 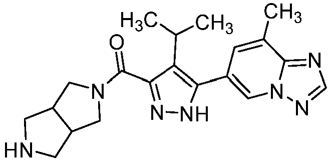 | 379.4 | 380.3 | 0.654 | E |
| 60 | 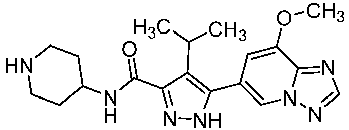 | 383.4 | 384.1 | 0.939 | E |
| 61 | 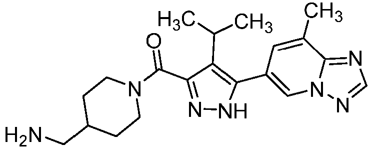 | 381.4 | 382.3 | 0.765 | F |
| 62 | 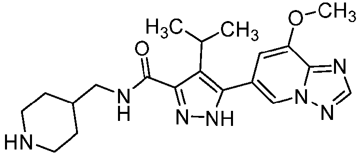 | 397.4 | 398.1 | 0.945 | E |
| 63 | 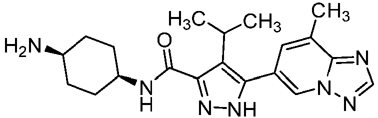 | 381.4 | 382.3 | 0.893 | E |
| 64 | 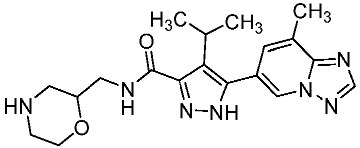 | 383.4 | 384.2 | 0.833 | E |
| 65 | 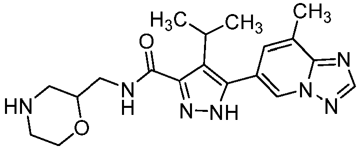 | 383.4 | 384.2 | 0.82 | E |
| 66 | 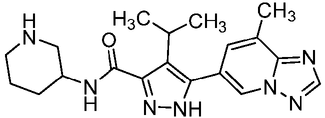 | 367.4 | 368.2 | 0.944 | E |
| 67 | 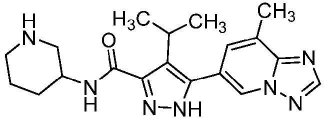 | 367.4 | 368.2 | 0.939 | E |
| 68 | 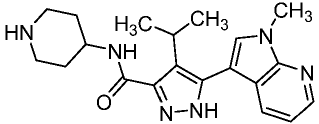 | 366.4 | 382.2 | 1.014 | E |

### EXAMPLE 69

### 4-isopropyl-5-(8-methyl-[1,2,4]triazolo[1,5-a]pyridin-6-yl)-N-(1-(3,3,3-trifluoro propyl) piperidin-4-yl)-1H-pyrazole-3-carboxamide

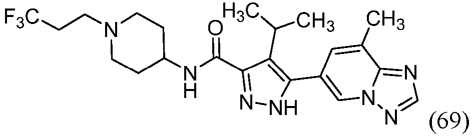

To a solution of 4-isopropyl-5-(8-methyl-[1,2,4]triazolo[1,5-a]pyridin-6-yl)-N-(piperidin-4-yl)-1H-pyrazole-3-carboxamide, HCl (22 mg, 0.054 mmol) in MeOH (2.00 mL) were added 3,3,3-trifluoropropanal (18.31 mg, 0.163 mmol) and AcOH (0.1 mL, 1.747 mmol) at room temperature. The reaction mixture was stirred at room temperature for 6 h, Sodium cyanoborohydride (17.11 mg, 0.272 mmol) was added and the reaction mixture was stirred at room temperature for 16 h. The reaction mass was purified by Prep LCMS purification using method AA, the fractions containing the product were combined and dried using a Genevac centrifugal evaporator to yield 4-isopropyl-5-(8-methyl-[1,2,4]triazolo[1,5-a]pyridin-6-yl)-N-(1-(3,3,3-trifluoropropyl)piperidin-4-yl)-1H-pyrazole-3-carboxamide (17.2 mg, 0.037 mmol, 68 % yield) as a pale solid. LCMS Retention time: 1.702 min [E], MS (ES): *m*/*z=* 464.2 [M+H]; ¹H NMR (400 MHz, METHANOL-d₄) δ ppm 8.75 (s, 1H), 8.49 (s, 1H), 7.54 (s, 1H), 3.97-3.88 (m, 1H), 2.99 (d, *J*=12.0 Hz, 2H), 2.77-2.59 (m, 4H), 2.53-2.36 (m, 2H), 2.29 (t, *J*=11.0 Hz, 2H), 2.12-1.93 (m, 2H), 1.78-1.59 (m, 2H), 1.41-1.20 (m, 6H).

The following Examples were prepared according to the general procedure described in Example 69.

**TABLE 4**

| Ex. No. | Structure | Mol Wt. | LCMS MH⁺ | RT (min) | HPLC Method |
|---|---|---|---|---|---|
| 70 | 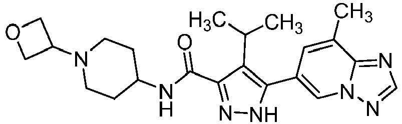 | 423.5 | 424.1 | 1.367 | E |
| 71 | 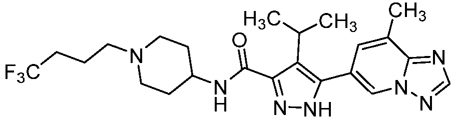 | 477.5 | 478.2 | 1.406 | C |
| 72 | 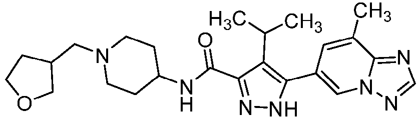 | 451.5 | 452.3 | 1.206 | E |
| 73 | 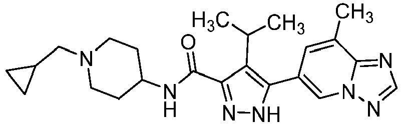 | 421.5 | 422.2 | 1.28 | E |
| 74 | 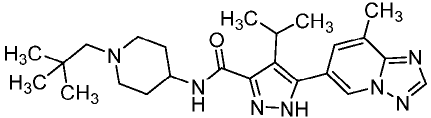 | 437.5 | 438.3 | 1.365 | E |
| 75 | 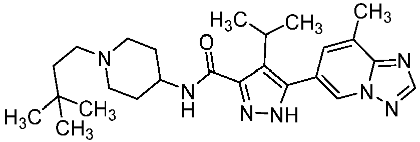 | 451.6 | 452.2 | 1.652 | C |
| 76 | 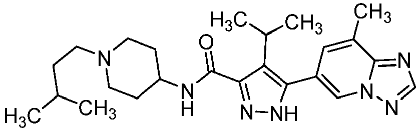 | 437.5 | 438.3 | 1.435 | D |
| 77 | 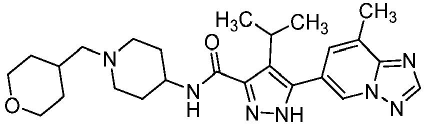 | 465.5 | 466.2 | 1.401 | C |
| 78 | 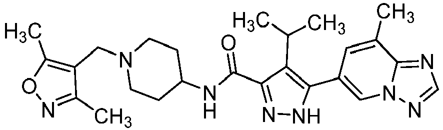 | 476.5 | 477.3 | 1.299 | D |
| 79 | 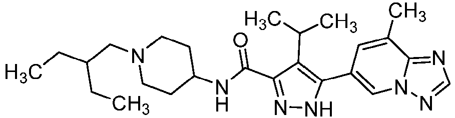 | 451.6 | 452.3 | 1.503 | D |
| 80 | 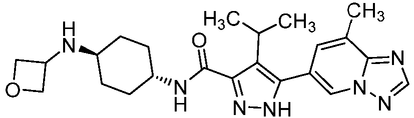 | 437.5 | 438.2 | 1.168 | E |
| 81 | 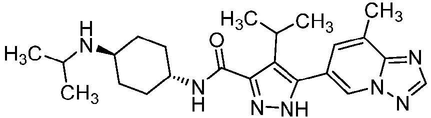 | 423.5 | 424.2 | 1.238 | E |
| 82 | 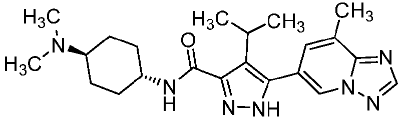 | 409.5 | 410.2 | 1.184 | E |
| 83 | 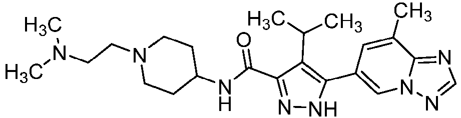 | 438.5 | 439.3 | 0.937 | E |
| 84 | 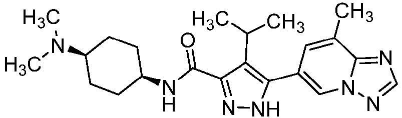 | 409.5 | 410.3 | 0.924 | E |
| 85 | 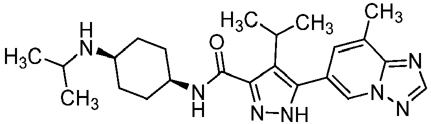 | 423.5 | 424.3 | 1.003 | E |
| 86 | 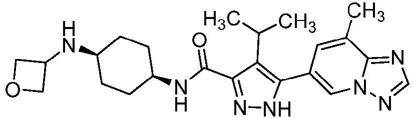 | 437.5 | 438.2 | 1.12 | E |
| 87 | 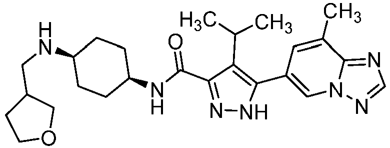 | 465.5 | 466.3 | 1.117 | C |
| 88 | 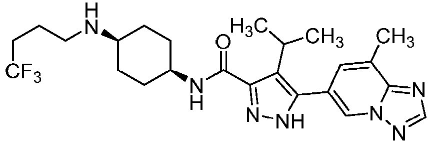 | 491.5 | 492.3 | 1.358 | C |
| 89 | 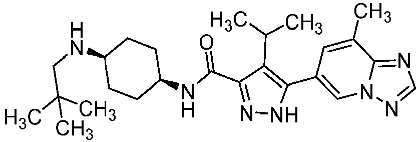 | 451.6 | 452.3 | 1.328 | C |
| 90 | 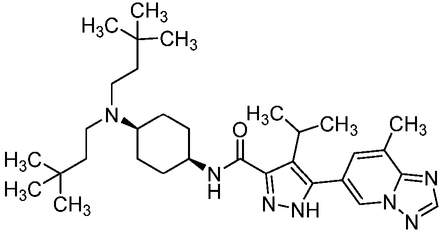 | 549.7 | 550.5 | 1.769 | D |
| 91 | 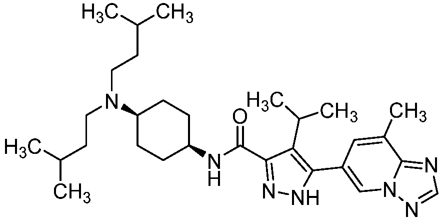 | 521.7 | 522.4 | 1.744 | C |
| 92 | 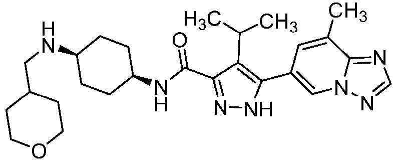 | 479.6 | 480.3 | 1.139 | D |
| 93 | 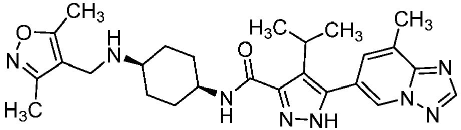 | 490.6 | 491.3 | 1.179 | D |
| 94 | 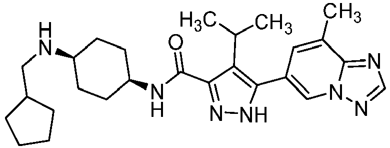 | 463.6 | 464.3 | 1.358 | C |
| 95 | 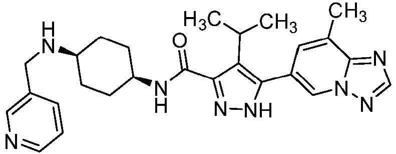 | 472.5 | 473.3 | 1.251 | C |
| 96 | 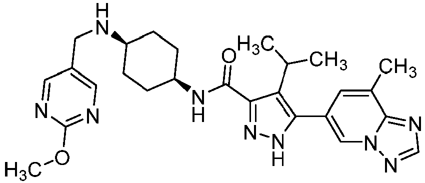 | 503.5 | 504.3 | 1.301 | C |
| 97 | 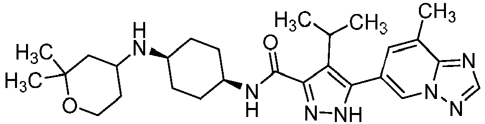 | 493.6 | 494.3 | 1.216 | D |
| 98 | 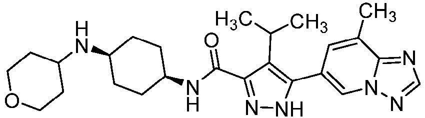 | 465.5 | 466.3 | 1.113 | D |
| 99 | 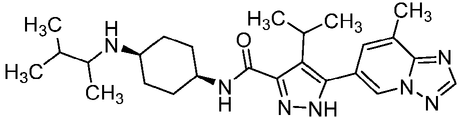 | 451.6 | 452.3 | 1.268 | D |
| 100 | 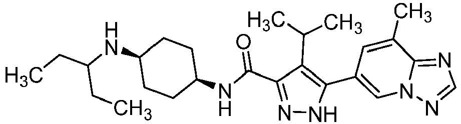 | 451.6 | 452.3 | 1.312 | C |
| 101 | 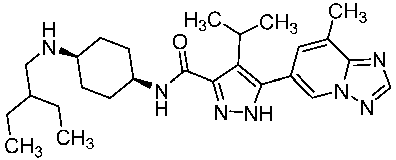 | 465.6 | 466.3 | 1.428 | C |
| 102 | 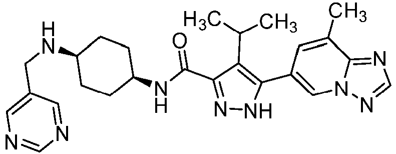 | 473.5 | 474.3 | 1.242 | C |
| 103 | 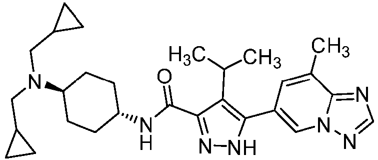 | 489.6 | 490.3 | 1.279 | D |
| 104 | 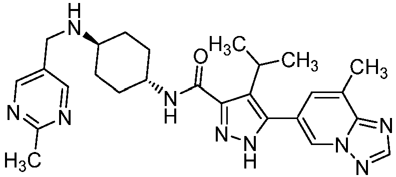 | 487.5 | 488.3 | 1.007 | D |
| 105 | 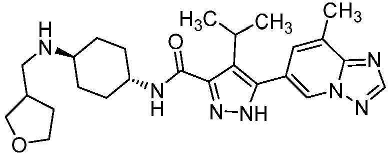 | 465.5 | 466.3 | 1.031 | D |
| 106 | 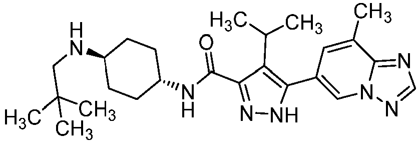 | 451.6 | 452.3 | 1.223 | D |
| 107 | 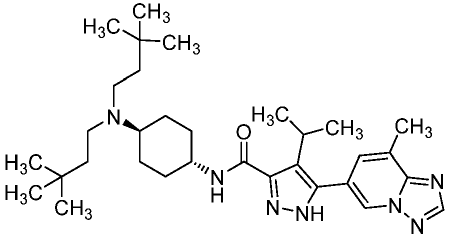 | 549.7 | 550.5 | 1.809 | D |
| 108 | 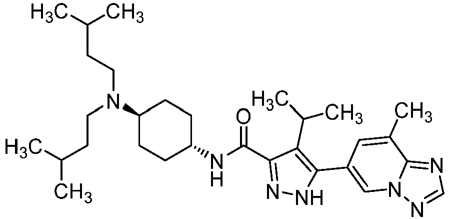 | 521.7 | 522.4 | 1.651 | D |
| 109 | 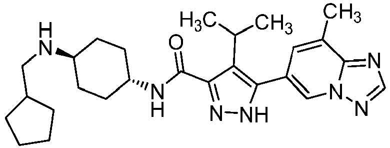 | 463.6 | 464.3 | 1.275 | D |
| 110 | 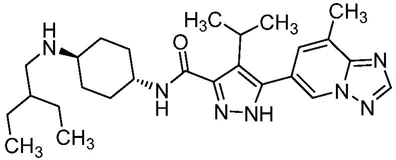 | 465.6 | 466.3 | 1.34 | D |
| 111 | 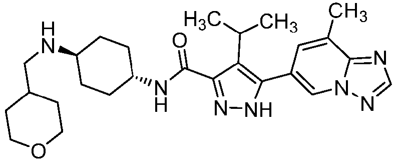 | 479.6 | 480.3 | 1.064 | D |
| 112 | 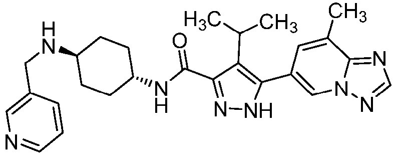 | 472.5 | 473.3 | 1.119 | C |
| 113 | 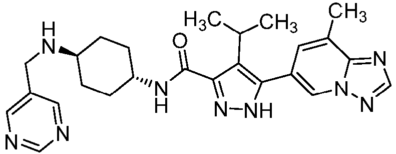 | 473.5 | 474.3 | 0.981 | D |
| 114 | 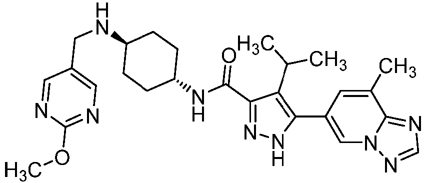 | 503.5 | 504.3 | 1.044 | D |
| 115 | 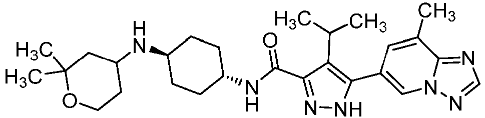 | 493.6 | 494.3 | 1.149 | C |
| 116 | 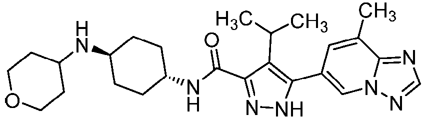 | 465.5 | 466.3 | 1.034 | C |
| 118 | 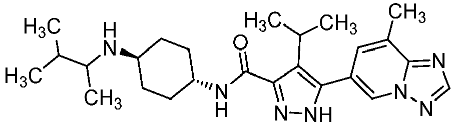 | 451.6 | 452.3 | 1.199 | C |
| 119 | 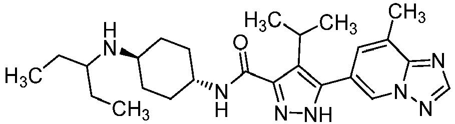 | 451.6 | 452.3 | 1.207 | C |
| 120 | 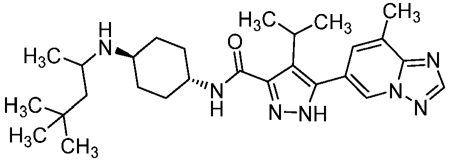 | 479.6 | 480.4 | 1.402 | D |
| 123 | 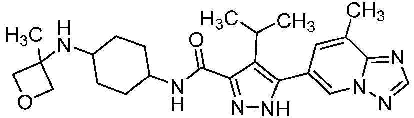 | 451.5 | 452.3 | 1.041 | E |
| 124 | 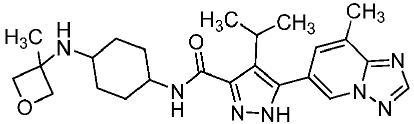 | 451.5 | 452.3 | 1.179 | E |
| 125 | 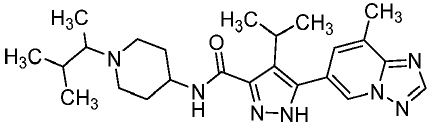 | 437.5 | 438.3 | 1.179 | E |
| 126 | 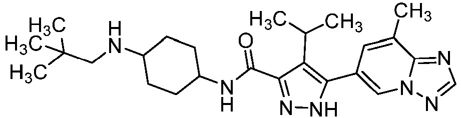 | 451.6 | 452.3 | 1.262 | C |
| 127 | 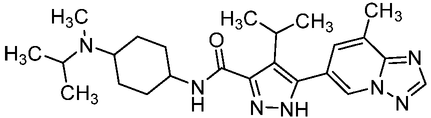 | 437.5 | 438.3 | 1.104 | D |
| 128 | 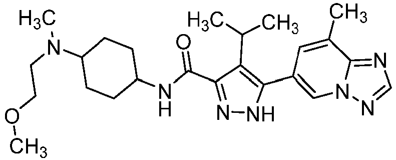 | 453.5 | 454.3 | 1.083 | D |
| 129 | 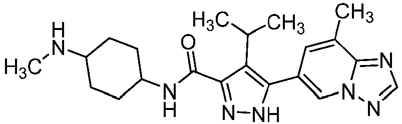 | 395.5 | 396.3 | 1.007 | D |
| 130 | 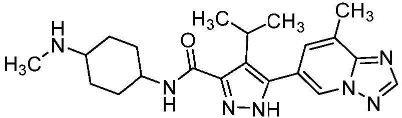 | 395.5 | 396.3 | 1.072 | D |
| 131 | 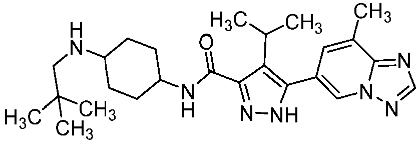 | 451.6 | 452.3 | 1.269 | D |
| 132 | 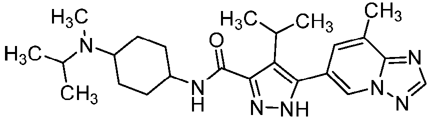 | 437.5 | 438.3 | 1.157 | D |
| 133 | 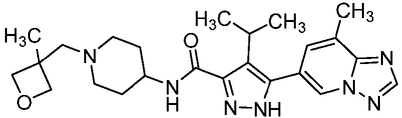 | 451.5 | 452.3 | 1.126 | E |
| 134 | 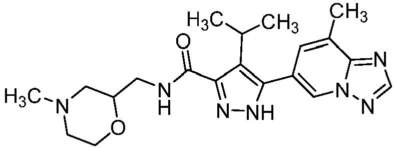 | 397.4 | 398.2 | 1.012 | E |
| 135 | 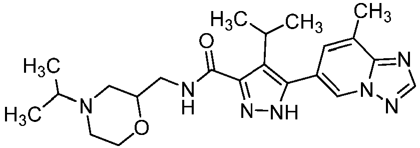 | 425.5 | 426.3 | 1.155 | E |
| 136 | 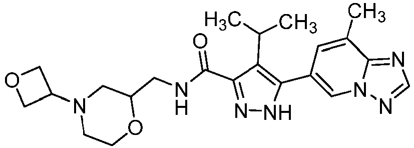 | 439.5 | 440.2 | 1.019 | E |
| 137 | 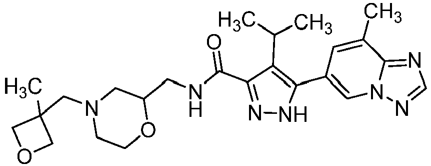 | 467.5 | 468.3 | 1.204 | E |
| 138 | 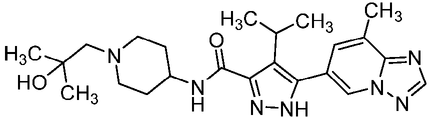 | 439.5 | 440.3 | 1.036 | E |
| 139 | 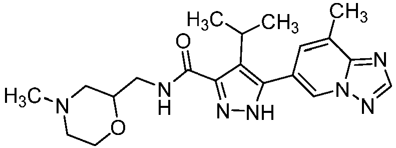 | 397.4 | 398.2 | 1.018 | E |
| 140 | 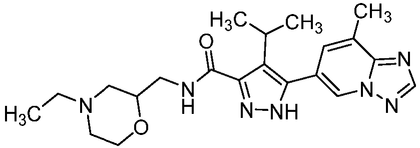 | 411.5 | 412.2 | 1.091 | E |
| 141 | 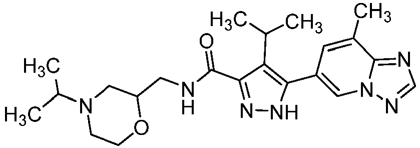 | 425.5 | 426.3 | 1.153 | E |
| 142 | 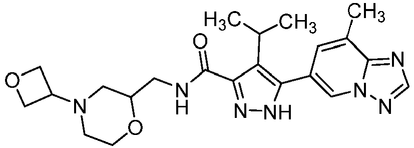 | 439.5 | 440.3 | 1.028 | E |
| 143 | 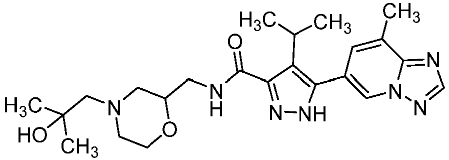 | 455.5 | 456.3 | 1.212 | E |
| 144 | 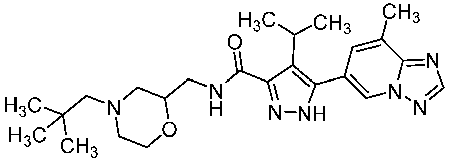 | 453.5 | 454.3 | 1.297 | E |
| 145 | 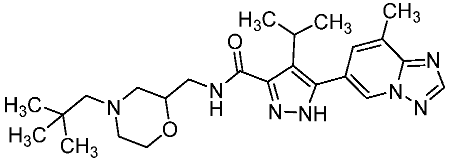 | 453.5 | 454.3 | 1.921 | E |
| 146 | 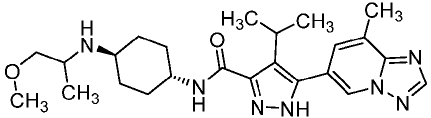 | 453.5 | 454.3 | 1.056 | E |
| 147 | 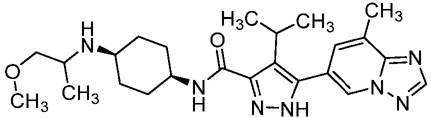 | 453.5 | 454.3 | 1.065 | E |
| 148 | 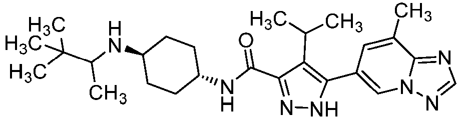 | 465.6 | 466.4 | 1.213 | E |
| 149 | 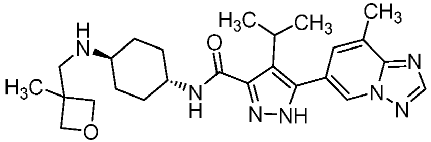 | 465.5 | 466.3 | 0.969 | E |
| 150 | 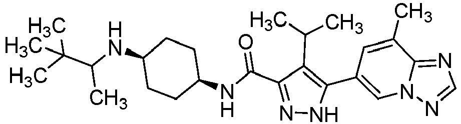 | 465.6 | 466.4 | 1.307 | E |
| 151 | 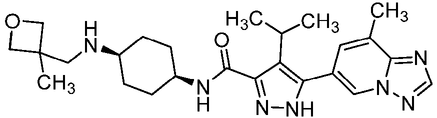 | 465.5 | 466.4 | 0.978 | F |
| 152 | 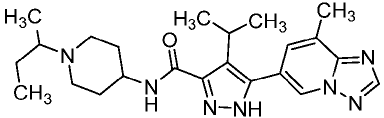 | 423.5 | 424.3 | 1.12 | E |
| 153 | 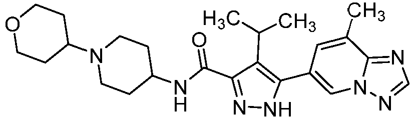 | 451.5 | 452.3 | 1.063 | E |
| 154 | 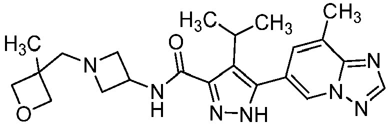 | 423.5 | 424.2 | 0.887 | E |
| 155 | 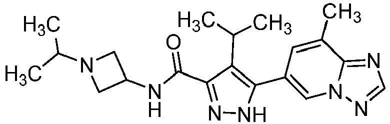 | 381.4 | 382.2 | 1.048 | E |
| 156 | 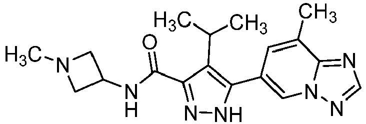 | 353.4 | 354.2 | 0.914 | E |
| 157 | 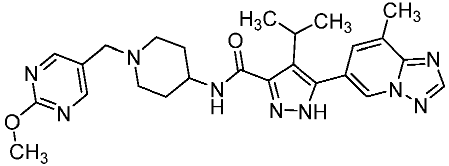 | 489.5 | 490.3 | 1.305 | E |
| 158 | 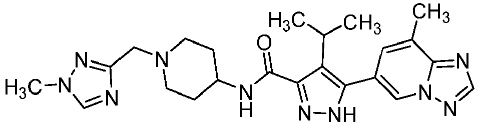 | 462.5 | 463.3 | 1.091 | E |
| 159 | 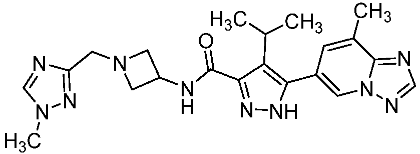 | 434.4 | 435.3 | 1.007 | E |
| 160 | 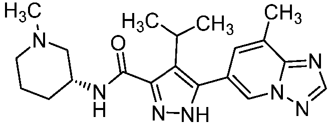 | 381.4 | 382.2 | 1.014 | E |
| 161 | 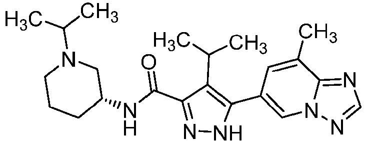 | 409.5 | 410.3 | 1.099 | E |
| 162 | 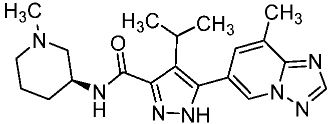 | 381.4 | 382.3 | 1.003 | E |
| 163 | 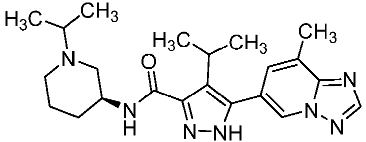 | 409.5 | 410.3 | 1.098 | E |
| 164 | 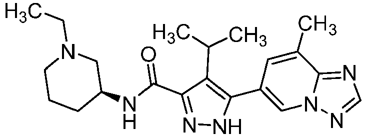 | 395.5 | 396.3 | 1.067 | E |
| 165 | 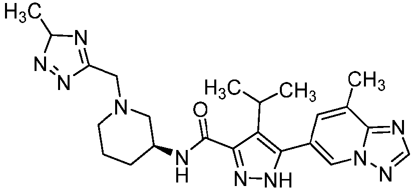 | 462.5 | 463.3 | 1.151 | E |
| 166 | 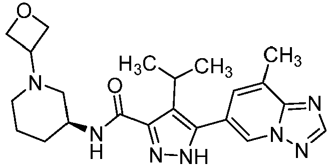 | 423.5 | 424.3 | 1.208 | E |
| 167 | 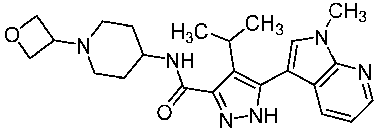 | 422.5 | 423.3 | 1.315 | E |
| 168 | 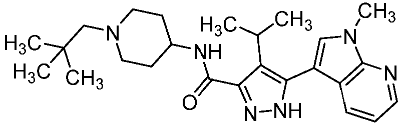 | 436.5 | 437.3 | 1.73 | E |
| 169 | 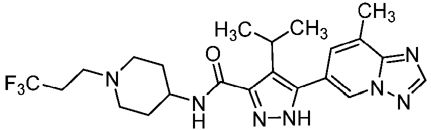 | 463.4 | 464.2 | 1.702 | E |
| 170 | 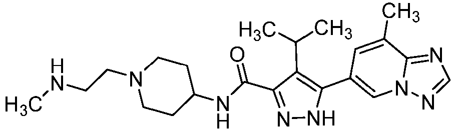 | 424.5 | 425.2 | 1.206 | E |

### EXAMPLE 171

### 4-isopropyl-N-(1-(2-methoxyethyl)piperidin-4-yl)-5-(8-methyl-[1,2,4]triazolo [1,5-a] pyridin-6-yl)-1H-pyrazole-3-carboxamide

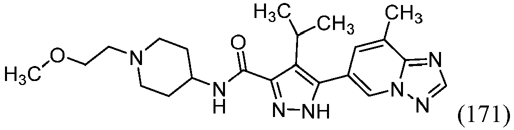

To a solution of 4-isopropyl-5-(8-methyl-[1,2,4]triazolo[1,5-a]pyridin-6-yl)-N-(piperidin-4-yl)-1H-pyrazole-3-carboxamide, HCl (0.015 g, 0.037 mmol) and 1-bromo-2-methoxyethane (10.32 mg, 0.074 mmol) in THF (1.00 mL) and DMF (0.5 mL) solvent mixture was added DIPEA (0.1 mL, 0.573 mmol) at room temperature. The mixture was stirred at 90°C for 4 h. The reaction mass purified by Prep LCMS purification using method AA, the fractions containing the product were combined and dried using Genevac centrifugal evaporator to yield 4-isopropyl-N-(1-(2-methoxyethyl)piperidin-4-yl)-5-(8-methyl-[1,2,4]triazolo[1,5-a]pyridin-6-yl)-1H-pyrazole-3-carboxamide (0.8 mg, 1.880 µmol, 5% yield) as a pale solid. LCMS Retention time: 0.92 min [E], MS (ES): *m*/*z=* 426.1 [M+H]; ¹H NMR (400 MHz, METHANOL-d₄) δ ppm 8.74 (s, 1H), 8.50 (s, 1H), 7.54 (s, 1H), 4.14 (br. s., 1H), 3.81-3.62 (m, 4H), 3.48-3.41 (m, 4H), 3.37 (d, J=6.5 Hz, 2H), 3.26-3.14 (m, 2H), 2.78-2.61 (m, 3H), 2.29 (d, J=12.5 Hz, 2H), 2.17 (br. s., 1H), 2.02-1.82 (m, 2H), 1.37-1.32 (m, 6H).

The following Examples were prepared according to the general procedure described in Example 171.

**Table 5**

| Ex. No. | Structure | Mol Wt. | LCMS MH⁺ | RT (min) | HPLC Method |
|---|---|---|---|---|---|
| 172 | 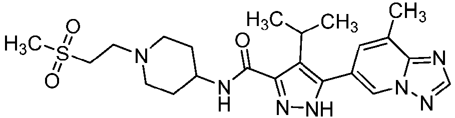 | 473.5 | 474.2 | 1.404 | E |
| 173 | 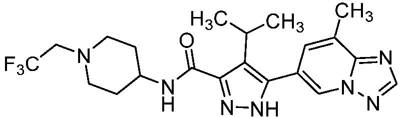 | 449.4 | 450.2 | 1.552 | E |

### EXAMPLE 174

### 4-isopropyl-5-(8-methyl-[1,2,4]triazolo[1,5-a]pyridin-6-yl)-N-(1-(1-(methyl sulfonyl)propan-2-yl)piperidin-4-yl)-1H-pyrazole-3-carboxamide

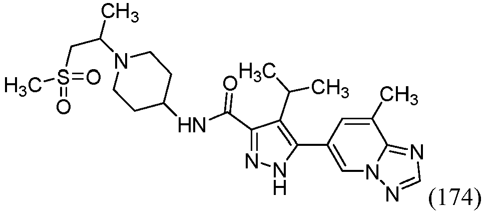

To a solution of 4-isopropyl-5-(8-methyl-[1,2,4]triazolo[1,5-a]pyridin-6-yl)-N-(piperidin-4-yl)-1H-pyrazole-3-carboxamide, HCl (0.018 g, 0.045 mmol), 2-chloro-1-(methylsulfonyl)propane (0.014 g, 0.089 mmol) and DIPEA (0.1 mL, 0.573 mmol) in dioxane (1.00 mL) was added K₂CO₃ (0.018 g, 0.134 mmol) at room temperature. The reaction mixture was stirred at 110 °C for 16 h. The reaction mixture was filtered and concentrated to afford the crude compound. The crude compound was purified by Prep LCMS using method AB, the fractions containing the product were combined and dried using Genevac centrifugal evaporator to yield 4-isopropyl-5-(8-methyl-[1,2,4]triazolo[1,5-a]pyridin-6-yl)-N-(1-(1-(methylsulfonyl)propan-2-yl)piperidin-4-yl)-1H-pyrazole-3-carboxamide (4.3 mg, 8.53 µmol, 19% yield) as a pale solid. LCMS Retention time: 1.515 min [E], MS (ES): *m*/*z=* 488.1 [M+H]; ¹H NMR (400 MHz, CD₃OD) δ ppm 8.74 (s, 1 H), 8.49 (s, 1 H), 7.54 (s, 1 H), 3.91-3.84 (m, 1 H), 3.60-3.52 (m, 1 H), 3.41-3.38 (m, 1 H), 2.96 (s, 3 H), 2.98-2.82 (m, 3 H), 2.70 (s, 3 H), 2.61-2.58 (m,1 H), 2.42-2.38 (m, 1 H), 2.06-1.95 (m, 3 H), 1.70-1.58 (m, 2 H), 1.33 (d, J = 6.8 Hz, 6 H), 1.20 (d, J = 6.8 Hz, 3 H).

### EXAMPLE 175 (REFERENCE)

### 4-isopropyl-5-(8-methyl-[1,2,4]triazolo[1,5-a]pyridin-6-yl)-1-(2,2,2-trifluoro ethyl)-N-(1-(2,2,2-trifluoroethyl)piperidin-4-yl)-1H-pyrazole-3-carboxamide

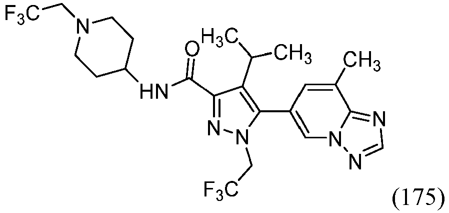

To a solution of 4-isopropyl-5-(8-methyl-[1,2,4]triazolo[1,5-a]pyridin-6-yl)-N-(piperidin-4-yl)-1H-pyrazole-3-carboxamide (0.021 g, 0.057 mmol) and 2,2,2-trifluoroethyl trifluoromethane sulfonate (0.027 g, 0.114 mmol) in DMF (1.500 mL) were added TEA (0.2 mL, 1.435 mmol) and Cs₂CO₃ (0.037 g, 0.114 mmol) at room temperature. The reaction mixture was stirred at 90 °C for 4 h. The reaction mixture was filtered, the filtrate was purified by preparative LCMS purification, the fractions containing the product were combined and dried using Genevac centrifugal evaporator to yield 4-isopropyl-5-(8-methyl-[1,2,4]triazolo[1,5-a]pyridin-6-yl)-1-(2,2,2-trifluoroethyl)-N-(1-(2,2,2-trifluoroethyl) piperidin-4-yl)-1H-pyrazole-3-carboxamide (9.6 mg, 0.018 mmol, 31% yield) as a pale solid. LCMS Retention time: 1.873 min [E], MS (ES): *m*/*z=* 532.2 [M+H]; ¹H NMR (400 MHz, METHANOL-d₄) δ ppm 8.76 (s, 1H), 8.53 (s, 1H), 7.42 (s, 1H), 4.05-3.83 (m, 1H), 3.40-3.34 (m, 2H), 3.28-3.12 (m, 3H), 2.80-2.71 (m, 2H), 2.70 (s, 3H), 2.03 (d, *J*=14.1 Hz, 2H), 1.87-1.70 (m, 2H), 1.35-1.26 (m, 1H), 1.21 (d, J=7.0 Hz, 6H).

### EXAMPLE 176

### N-(1-(2-(dimethylamino)-2-oxoethyl)piperidin-4-yl)-4-isopropyl-5-(8-methyl-[1,2,4]triazolo [1,5-a]pyridin-6-yl)-1H-pyrazole-3-carboxamide

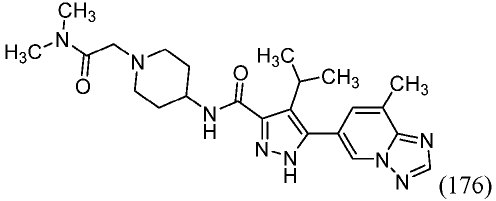

To a solution of 4-isopropyl-5-(8-methyl-[1,2,4]triazolo[1,5-a]pyridin-6-yl)-N-(piperidin-4-yl)-1H-pyrazole-3-carboxamide (0.015 g, 0.041 mmol) in THF (1.00 mL) and DMF (0.50 mL) solvent mixture were added TEA (0.2 mL, 1.435 mmol) and 2-chloro-N,N-dimethylacetamide (9.92 mg, 0.082 mmol) at room temperature. The reaction mixture was stirred at room temperature for 16 h. The reaction mass was purified by Prep LCMS purification using method AA, the fractions containing the product were combined and dried using Genevac centrifugal evaporator to yield N-(1-(2-(dimethylamino)-2-oxoethyl) piperidin-4-yl)-4-isopropyl-5-(8-methyl-[1,2,4]triazolo[1,5-a] pyridin-6-yl)-1H-pyrazole-3-carboxamide (4.8 mg, 10.08 µmol, 25% yield) as a pale solid. LCMS Retention time: 0.92 min [E], MS (ES): *m*/*z=* 453.3 [M+H]; ¹H NMR (400 MHz, METHANOL-d₄) δ ppm 8.74 (s, 1H), 8.49 (s, 1H), 7.54 (br. s., 1H), 4.95 (s, 1H), 4.00-3.85 (m, 1H), 3.81 (s, 1H), 3.16-3.09 (m, 3H), 3.08-2.99 (m, 2H), 2.97 (s, 3H), 2.76-2.64 (m, 3H), 2.36 (br. s., 2H), 2.00 (d, *J*=10.5 Hz, 2H), 1.85-1.65 (m, 2H), 1.44-1.26 (m, 7H).

The following Example was prepared according to the general procedure described in Example 176.

**TABLE 6**

| Ex. No. | Structure | Mol Wt. | LCMS MH⁺ | RT (min) | HPLC Method |
|---|---|---|---|---|---|
| 177 | 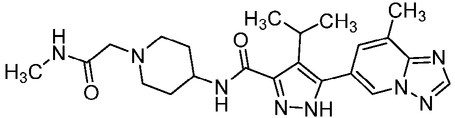 | 438.5 | 439.3 | 1.118 | E |

### EXAMPLE 178 (REFERENCE)

### N-(1-isopropylpiperidin-4-yl)-5-(8-methyl-[1,2,4]triazolo[1,5-a]pyridin-6-yl)-1H-pyrazole-3-carboxamide

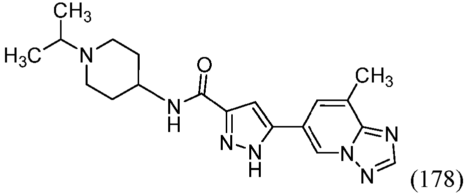

### Intermediate 178A: Methyl 4-bromo-5-iodo-1H-pyrazole-3-carboxylate

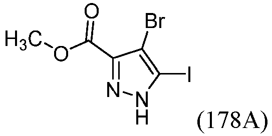

To a stirred solution of methyl 4-bromo-1H-pyrazole-3-carboxylate (1 g, 4.88 mmol) in acetonitrile (50 mL) was added NBS (5.3 mmol) at room temperature. The reaction mixture was stirred at 100 °C for 16 h. The reaction mass was diluted with ethyl acetate and washed with water, brine, dried (Na₂SO₄) and concentrated to get the crude mass. The crude mass was purified by ISCO using 24 g silica column, the compound was eluted in 80% of EA in hexanes, the fractions were collected and concentrated to yield methyl 4-bromo-5-iodo-1H-pyrazole-3-carboxylate (360 mg, 1.088 mmol, 22.30 % yield) as an off-white solid. LCMS retention time 0.98 min [B]. MS (E-) *m*/*z:* 332.9 [M+H].

### Intermediate 178B: Methyl 4-bromo-5-iodo-1-((2-(trimethylsilyl)ethoxy)methyl)-1H-pyrazole-3-carboxylate

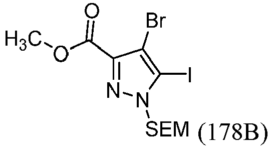

To a stirred solution of methyl 4-bromo-5-iodo-1H-pyrazole-3-carboxylate (600 mg, 1.813 mmol) in THF was added NaH (87 mg, 3.63 mmol) at 0 °C. The reaction mixture was stirred at 0 °C for 0.5 h. Next, 2-(trimethylsilyl)ethoxymethyl chloride (363 mg, 2.176 mmol) was added at 0 °C and the reaction mixture was stirred at room temperature for 3 h. The reaction was quenched with ice water. The reaction mixture was extracted with EtOAc, washed with brine, dried over sodium sulphate and concentrated to get crude compound. The crude compound was purified by ISCO using 24 g silica column, the fractions were collected and concentrated to yield methyl 4-bromo-5-iodo-1-((2-(trimethylsilyl)ethoxy) methyl)-1H-pyrazole-3-carboxylate (800 mg, 1.735 mmol, 96 % yield) as an off-white solid. LCMS retention time 1.61 min [B]. MS (E⁻) *m*/*z:* 463.1 [M+H].

### Intermediate 178C: ethyl 4-bromo-5-(8-methyl-[1,2,4]triazolo[1,5-a]pyridin-6-yl)-1-((2-(trimethylsilyl)ethoxy)methyl)-1H-pyrazole-3-carboxylate

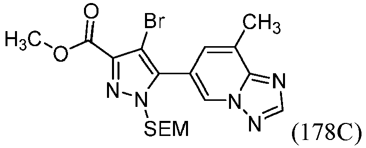

Ethyl 4-bromo-5-(8-methyl-[1,2,4]triazolo[1,5-a]pyridin-6-yl)-1-((2-(trimethylsilyl) ethoxy) methyl)-1H-pyrazole-3-carboxylate (800 mg, 1.665 mmol, 79 % yield) was prepared according to the general process described in Intermediate 1F using ethyl 4-bromo-5-iodo-1-((2-(trimethylsilyl)ethoxy) methyl)-1H-pyrazole-3-carboxylate (1 g, 2.104 mmol) as the starting intermediate. LCMS retention time 1.92 min [B]. MS (E⁻) *m*/*z:* 482.3 [M+H].

### Intermediate 178D: 4-bromo-5-(8-methyl-[1,2,4]triazolo[1,5-a]pyridin-6-yl)-1-((2-(trimethylsilyl)ethoxy)methyl)-1H-pyrazole-3-carboxylic acid

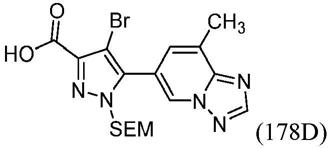

4-Bromo-5-(8-methyl-[1,2,4]triazolo[1,5-a]pyridin-6-yl)-1-((2-(trimethylsilyl)ethoxy) methyl)-1H-pyrazole-3-carboxylic acid (130 mg, 0.287 mmol, 58.3 % yield) was prepared according to the general process described in Intermediate 1Q using methyl 4-bromo-5-(8-methyl-[1,2,4]triazolo[1,5-a] pyridin-6-yl)-1-((2-(trimethylsilyl)ethoxy)methyl)-1H-pyrazole-3-carboxylate (230 mg, 0.493 mmol) as a starting intermediate to yield the title compound as an off-white solid. LCMS retention time 0.91 min [A]. MS (E⁻) *m*/*z:* 454.2 [M+H].

### Intermediate 178E: tert-butyl 4-(4-bromo-5-(8-methyl-[1,2,4]triazolo[1,5-a]pyridin-6-yl)-1-((2-(trimethylsilyl)ethoxy)methyl)-1H-pyrazole-3-carboxamido)piperidine-1-carboxylate

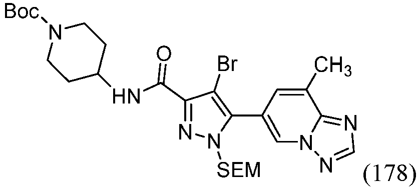

tert-Butyl 4-(4-bromo-5-(8-methyl-[1,2,4]triazolo[1,5-a]pyridin-6-yl)-1-((2-(trimethylsilyl)ethoxy)methyl)-1H-pyrazole-3-carboxamido)piperidine-1-carboxylate (160 mg, 0.252 mmol, 52 % yield) was prepared according to the procedure described in Intermediate 1H using 4-bromo-5-(8-methyl-[1,2,4]triazolo[1,5-a]pyridin-6-yl)-1-((2-(trimethylsilyl)ethoxy)methyl)-1H-pyrazole-3-carboxylic acid as the starting intermediate. LCMS retention time 1.56 min [A]. MS (E⁻) *m*/*z:* 636.4 [M+H].

### Intermediate 178F: tert-butyl 4-(5-(8-methyl-[1,2,4]triazolo[1,5-a]pyridin-6-yl)-1-((2-(trimethylsilyl)ethoxy)methyl)-1H-pyrazole-3-carboxamido)piperidine-1-carboxylate

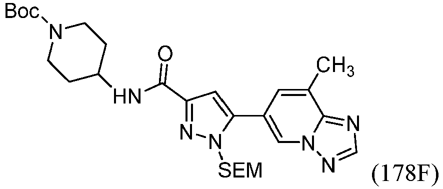

To a stirred solution of tert-butyl 4-(4-bromo-5-(8-methyl-[1,2,4]triazolo[1,5-a] pyridin-6-yl)-1-((2-(trimethylsilyl)ethoxy)methyl)-1H-pyrazole-3-carboxamido)piperidine-1-carboxylate (160 mg, 0.252 mmol) in MeOH (15 mL) was added Pd/C (26.8 mg, 0.252 mmol) at room temperature. The reaction mixture was stirred under hydrogen gas bladder pressure for 16 h. The reaction mass filtered through celite bed washed with MeOH and concentrated to yield tert-butyl 4-(5-(8-methyl-[1,2,4]triazolo[1,5-a]pyridin-6-yl)-1-((2-(trimethylsilyl)ethoxy)methyl)-1H-pyrazole-3-carboxamido)piperidine-1-carboxylate (100 mg, 0.180 mmol, 71% yield) as a brown liquid. LCMS retention time 1.94 min [A]. MS (E⁻) *m*/*z:* 556.5 [M+H].

### Intermediate 178G: 5-(8-methyl-[1,2,4]triazolo[1,5-a]pyridin-6-yl)-N-(piperidin-4-yl)-1H-pyrazole-3-carboxamide

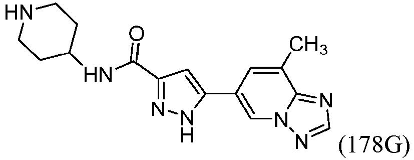

To a stirred solution of tert-butyl 4-(5-(8-methyl-[1,2,4]triazolo[1,5-a]pyridin-6-yl)-1-((2-(trimethylsilyl)ethoxy)methyl)-1H-pyrazole-3-carboxamido)piperidine-1-carboxylate (120 mg, 0.216 mmol) in DCM (10 mL) was added TFA (0.017 mL, 0.216 mmol) at room temperature. The reaction mixture was stirred at the same temperature for 3 h. The reaction mass was concentrated to get crude compound. The crude compound was purified by Prep LCMS using method AB, the fractions containing the compound was collected and dried via centrifugal evaporation to yield 5-(8-methyl-[1,2,4]triazolo[1,5-a]pyridin-6-yl)-N-(piperidin-4-yl)-1H-pyrazole-3-carboxamide (4.6 mg, 0.430 mmol, 12 % yield) as a pale solid. LCMS retention time 0.635min [D]. MS (E⁻) *m*/*z:* 326.1 (M+H). ¹H NMR (400 MHz, DMSO-d₆) δ ppm 9.19 (s, 1H), 8.50 (s, 1H), 8.42-8.28 (m, 1H), 7.96 (s, 1H),7.36 (s, 1H), 3.97 (d, J=7.8 Hz, 1H), 3.17 (d, *J*=11.7 Hz, 2H), 2.85-2.74 (m, 2H), 2.61 (s, 3H),1.95-1.83 (m, 3H), 1.69-1.55 (m, 2H).

### Example 178: N-(1-isopropylpiperidin-4-yl)-5-(8-methyl-[1,2,4]triazolo[1,5-a]pyridin -6-yl)-1H-pyrazole-3-carboxamide

To a stirred solution of 5-(8-methyl-[1,2,4]triazolo[1,5-a]pyridin-6-yl)-N-(piperidin-4-yl)-1H-pyrazole-3-carboxamide (30 mg, 0.092 mmol) in MeOH (3 mL) were added propan-2-one (5.36 mg, 0.092 mmol) and acetic acid (5.28 µl, 0.092 mmol). The reaction mixture was stirred at room temperature for 16 h. Next, NaCNBH₃ (5.79 mg, 0.092 mmol) was added and the reaction mixture was stirred at room temperature for 2 h. The reaction mass was concentrated to yield crude compound. The crude compound was purified by prep LCMS using method AA, the fractions containing the compound was collected and dried via centrifugal evaporation to yield N-(1-isopropylpiperidin-4-yl)-5-(8-methyl-[1,2,4] triazolo[1,5-a]pyridin-6-yl)-1H-pyrazole-3-carboxamide (1.1 mg, 0.252 mmol, 12 % yield) as an off-white solid. LCMS retention time 0.694 min [E]. MS (E⁻) *m*/*z:* 368.2 (M+H); ¹H NMR (400 MHz, DMSO-d₆) δ ppm 13.42 (br. s., 1H), 8.90 (s, 1H), 8.55 (s, 1H), 7.90 (d, J=8.1Hz, 1H), 7.62 (s, 1H), 3.76 (br. s., 1H), 2.88-2.73 (m, 4H), 2.63 (s, 3H), 2.23 (s, 3H), 2.09 (d, J=5.6 Hz, 2H), 1.91 (s, 2H), 1.83-1.71 (m, 2H), 1.71-1.55 (m, 2H), 1.24 (m, 3H), 1.11 (t, J=7.3Hz, 3H).

### EXAMPLE 179

### N-(1-isopropylpiperidin-4-yl)-4-methyl-5-(8-methyl-[1,2,4]triazolo[1,5-a]pyridin-6-yl)-1H-pyrazole-3-carboxamide

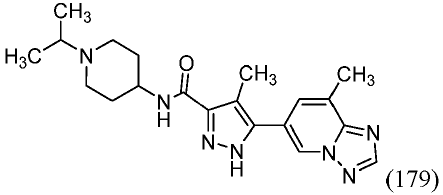

### Intermediate 179A: tert-butyl 4-(4-methyl-5-(8-methyl-[1,2,4]triazolo[1,5-a]pyridin-6-yl)-1-((2-(trimethylsilyl)ethoxy)methyl)-1H-pyrazole-3-carboxamido)piperidine-1-carboxylate

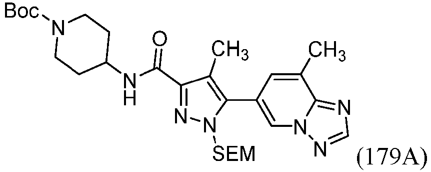

To a stirred solution of tert-butyl 4-(4-bromo-5-(8-methyl-[1,2,4]triazolo[1,5-a] pyridin-6-yl)-1-((2-(trimethylsilyl)ethoxy)methyl)-1H-pyrazole-3-carboxamido)piperidine-1-carboxylate (100 mg, 0.158 mmol), potassium phosphate tribasic (100 mg, 0.473 mmol) and methylboronic acid (18.86 mg, 0.315 mmol) in THF (50 mL) was added degassed with N₂ for 10 min. To the reaction mixture was added PdCl₂(dppf)-CH₂Cl₂ adduct (12.87 mg, 0.016 mmol). The reaction mixture was degassed for 10 min. The reaction mixture was stirred at 80 °C for 16 h. The reaction mass was diluted with ice water, extracted with EtOAc, washed with brine, dried over sodium sulphate and concentrated to yield the crude compound. The crude compound was purified by ISCO using 12 g silica column, the compound was eluted in 65% EA in hexanes the fractions containing the compound was collected and concentrated to yield tert-butyl 4-(4-methyl-5-(8-methyl-[1,2,4]triazolo[1,5-a]pyridin-6-yl)-1-((2-(trimethylsilyl)ethoxy)methyl)-1H-pyrazole-3-carboxamido)piperidine-1-carboxylate (60 mg, 0.252 mmol, 52% yield) as an oil. LCMS retention time 2.06 min [B], MS (E⁻) *m*/*z:* 570.6 (M+H).

### Intermediate 179B: 4-methyl-5-(8-methyl-[1,2,4]triazolo[1,5-a]pyridin-6-yl)-N-(piperidin-4-yl)-1H-pyrazole-3-carboxamide

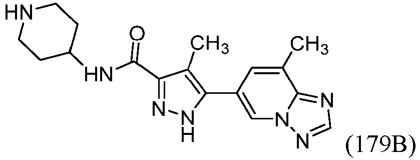

4-Methyl-5-(8-methyl-[1,2,4]triazolo[1,5-a]pyridin-6-yl)-N-(piperidin-4-yl)-1H-pyrazole-3-carboxamide (30 mg, 0.252 mmol, 49 % yield) was prepared according to the general process described in Example 43 using tert-butyl 4-(4-methyl-5-(8-methyl-[1,2,4]triazolo[1,5-a]pyridin-6-yl)-1-((2-(trimethyl silyl)ethoxy)methyl)-1H-pyrazole-3-carboxamido)piperidine-1-carboxylate (100 mg, 0.176 mmol) as the starting intermediate. LCMS retention time 0.64 min [E], MS (E⁻) *m*/*z:* 340.2 [M+H]; ¹H NMR (400 MHz, DMSO-d₆) δ ppm 13.51 (s, 1H), 8.99 (s, 1H), 8.24 (d, J=7.8 Hz, 1H), 7.67 (s, 1H), 7.14 (br. s., 1H), 7.01 (br. s., 1H),4.07 (br. s., 1H), 3.03 (br. s., 2H), 2.94 (d, J=6.8 Hz, 2H), 2.67-2.55 (m, 3H), 1.96 (d, J=13.4 Hz, 2H), 1.79 (d, *J*=11.7 Hz, 1H), 1.24 (s, 1H), 1.17 (t, J=7.3 Hz, 3H).

### Example 179: N-(1-isopropylpiperidin-4-yl)-4-methyl-5-(8-methyl-[1,2,4]triazolo[1,5-a] pyridin-6-yl)-1H-pyrazole-3-carboxamide

N-(1-isopropylpiperidin-4-yl)-4-methyl-5-(8-methyl-[1,2,4]triazolo[1,5-a]pyridin-6-yl)-1H-pyrazole-3-carboxamide (8.2 mg, 0.252 mmol, 22% yield) was prepared according to the general process described in Example 69 using 4-methyl-5-(8-methyl-[1,2,4]triazolo[1,5-a]pyridin-6-yl)-N-(piperidin-4-yl)-1H-pyrazole-3-carboxamide (30 mg, 0.088 mmol) as the starting intermediate. LCMS retention time 0.798 min [E], MS (E⁻) *m*/*z:* 382.2 (M+H); ¹H NMR (400 MHz, DMSO-d₆) δ ppm 13.53 (s, 1H), 9.01 (s, 1H), 8.57 (s, 1H), 8.32 (d, J=8.3 Hz,1H), 7.12 (s, 1H), 6.99 (s, 1H), 6.56 (s, 1H), 4.06 (br. s., 3H), 3.18-3.05 (m, 2H), 2.99 (s, 1H), 2.93 (br. s., 1H), 2.63 (s, 3H), 2.11 (d, J=19.6 Hz, 1H), 2.02 (br. s., 2H), 1.88 (d, J=12.7 Hz, 3H), 1.35-1.21 (m, 6H),1.21-1.09 (m, 2H).

The following Example was prepared according to the general procedure described in Example 179.

**TABLE 7**

| Ex. No. | Structure | Mol Wt. | LCMS MH⁺ | RT (min) | HPLC Method |
|---|---|---|---|---|---|
| 180 | 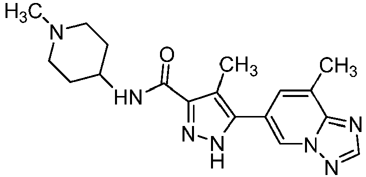 | 353.4 | 354.2 | 0.714 | C |

### EXAMPLE 181

### 4-ethyl-5-(8-methyl-[1,2,4]triazolo[1,5-a]pyridin-6-yl)-N-(1-methylpiperidin-4-yl)-1H-pyrazole-3-carboxamide

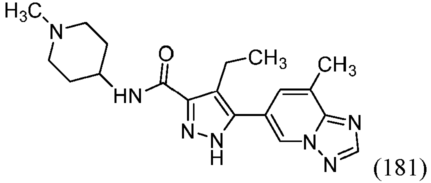

### Intermediate 181A: tert-butyl 4-(5-(8-methyl-[1,2,4]triazolo[1,5-a]pyridin-6-yl)-1-((2-(trimethylsilyl)ethoxy)methyl)-4-vinyl-1H-pyrazole-3-carboxamido)piperidine-1-carboxylate

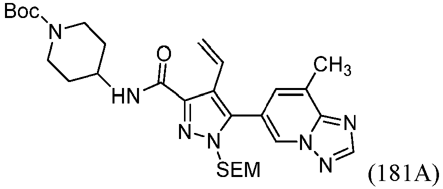

tert-butyl 4-(5-(8-methyl-[1,2,4]triazolo[1,5-a]pyridin-6-yl)-1-((2-(trimethylsilyl) ethoxy)methyl)-4-vinyl-1H-pyrazole-3-carboxamido)piperidine-1-carboxylate (250 mg, 0.430 mmol, 91 % yield) was prepared according to the process described in Intermediate 1B, using tert-butyl 4-(4-bromo-5-(8-methyl-[1,2,4]triazolo[1,5-a]pyridin-6-yl)-1-((2-(trimethylsilyl)ethoxy)methyl)-1H-pyrazole-3-carboxamido)piperidine-1-carboxylate (300 mg, 0.473 mmol) as the starting intermediate. LCMS retention time 2.07 min [C], MS (E⁻) *m*/*z:* 582.6 [M+H].

### Intermediate 181B: tert-butyl 4-(4-ethyl-5-(8-methyl-[1,2,4]triazolo[1,5-a]pyridin-6-yl)-1-((2-(trimethylsilyl)ethoxy)methyl)-1H-pyrazole-3-carboxamido)piperidine-1-carboxylate

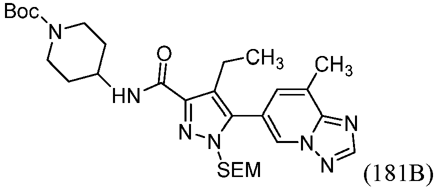

To a stirred solution of tert-butyl 4-(5-(8-methyl-[1,2,4]triazolo[1,5-a]pyridin-6-yl)-1-((2-(trimethylsilyl)ethoxy)methyl)-4-vinyl-1H-pyrazole-3-carboxamido)piperidine-1-carboxylate (200 mg, 0.344 mmol) in MeOH (125 mL) was added Pd/C (36.6 mg, 0.344 mmol) at room temperature. The reaction mixture was stirred under hydrogen with bladder pressure for 16 h. The reaction mass was filtered through a celite bed, washed with MeOH, and concentrated to yield tert-butyl 4-(4-ethyl-5-(8-methyl-[1,2,4]triazolo[1,5-a]pyridin-6-yl)-1-((2-(trimethylsilyl)ethoxy)methyl)-1H-pyrazole-3-carboxamido)piperidine-1-carboxylate (160 mg, 0.430 mmol, 68% yield) as an oil. LCMS retention time 1.53 min [A], MS (E⁻) *m*/*z:* 467.5 [M+H].

### Intermediate 181C: 4-ethyl-5-(8-methyl-[1,2,4]triazolo[1,5-a]pyridin-6-yl)-N-(piperidin-4-yl)-1H-pyrazole-3-carboxamide

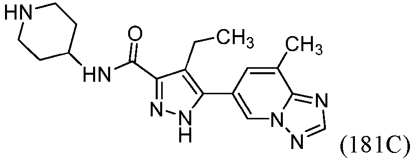

4-ethyl-5-(8-methyl-[1,2,4]triazolo[1,5-a]pyridin-6-yl)-N-(piperidin-4-yl)-1H-pyrazole-3-carboxamide (13.1 mg, 0.430 mmol, 18 % yield) was prepared according to the procedure described in Example 43 using tert-butyl 4-(4-ethyl-5-(8-methyl-[1,2,4]triazolo[1,5-a]pyridin-6-yl)-1-((2-(trimethylsilyl)ethoxy) methyl)-1H-pyrazole-3-carboxamido)piperidine-1-carboxylate (120 mg, 0.206 mmol) as the starting intermediate. LCMS retention time 0.807 min [A], MS (E⁻) *m*/*z:* 354.2 (M+H); ¹H NMR (400 MHz, DMSO-d₆) δ ppm d 13.46 (br. s., 1H), 8.92 (br. s., 1H), 8.32 (br. s.1H), 8.25 (d, J=7.8 Hz, 1H), 7.62 (br. s., 1H), 7.00 (s, 1H), 4.07 (dd, J=7.6, 3.7 Hz, 1H), 3.32(br. s., 3H), 3.09-2.99 (m, 2H), 2.99-2.89 (m, 1H), 2.68-2.58 (m, 3H), 1.97 (d, J=11.7 Hz, 2H), 1.86-1.66 (m, 2H), 1.21-1.03 (m, 3H).

### Example 181: 4-ethyl-5-(8-methyl-[1,2,4]triazolo[1,5-a]pyridin-6-yl)-N-(1-methyl piperidin-4-yl)-1H-pyrazole-3-carboxamide

4-ethyl-5-(8-methyl-[1,2,4]triazolo[1,5-a]pyridin-6-yl)-N-(1-methylpiperidin-4-yl)-1H-pyrazole-3-carboxamide (11.5 mg, 37% yield) was prepared according to the general procedure described in Example 69 using 4-ethyl-5-(8-methyl-[1,2,4]triazolo[1,5-a]pyridin-6-yl)-N-(piperidin-4-yl)-1H-pyrazole-3-carboxamide (30 mg, 0.085 mmol) as the starting intermediate. LCMS retention time 0.859 min [C]. MS (E⁻) *m*/*z:* 368.2 (M+H); ¹H NMR (400 MHz, DMSO-d₆) δ ppm 13.42 (br. s., 1H), 8.90 (s, 1H), 8.55 (s, 1H), 7.90 (d, J=8.1Hz, 1H), 7.62 (s, 1H), 3.76 (br. s., 1H), 2.88-2.73 (m, 4H), 2.63 (s, 3H), 2.23 (s, 3H), 2.09 (d, J=5.6 Hz, 2H), 1.91 (s, 2H), 1.83-1.71 (m, 2H), 1.71-1.55 (m, 2H), 1.24 (s, 1H), 1.11 (t, J=7.3Hz, 3H).

The following Example was prepared according to the general procedure described in Example 181.

**TABLE 8**

| Ex. No. | Structure | Mol Wt. | LCMS MH⁺ | RT (min) | HPLC Method |
|---|---|---|---|---|---|
| 182 | 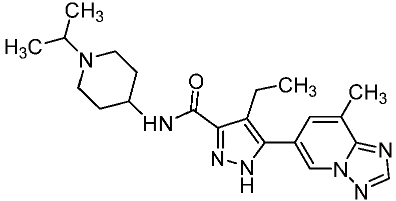 | 395.5 | 396.3 | 0.934 | E |

### EXAMPLE 183

### 2-(4-isopropyl-5-(8-methyl-[1,2,4]triazolo[1,5-a]pyridin-6-yl)-1H-pyrazol-3-yl)-N-(1-isopropylpiperidin-4-yl)-2-oxoacetamide

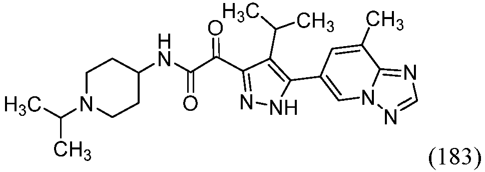

### Intermediate 183A: 2-formyl-3-methylbutanenitrile

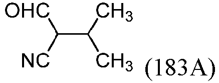

To a solution of 3-methylbutanenitrile (50.0 g, 601 mmol) in THF (1000 mL) was added dropwise LDA (2M, 391 mL, 782 mmol) at -78 °C. The reaction mixture was stirred for 20 min, and a solution of ethyl formate (66.8 g, 902 mmol) in THF (200 mL) was added. The reaction mixture was stirred for an additional 1 h at the same temperature, then brought to room temperature, and stirred room temperature at for 16 h. The reaction was quenched with saturated NH₄Cl. The mixture was stirred for 10 min. and the volatiles were evaporated. The residue was dissolved with excess EA, washed with water, brine, dried (Na₂SO₄) and concentrated to yield crude 2-formyl-3-methylbutanenitrile (55 g, 495 mmol, 82 % yield) as an oil. LCMS Retention time: 0.77 min [A], MS (E⁻) m/z: 110.2 [M-H].

### Intermediate 183B: 4-isopropyl-1H-pyrazol-3-amine

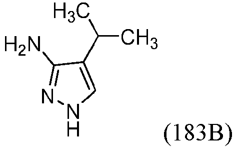

To a solution of 2-formyl-3-methylbutanenitrile (55.0 g, 495 mmol) in ethanol (600 mL) were added hydrazine (38.8 mL, 1237 mmol) and acetic acid (70.8 mL, 1237 mmol) at room temperature. The mixture was stirred at 100 °C for 16 h. The reaction mixture was cooled room temperature. The volatiles were evaporated. The residue was diluted with saturated NaHCO₃ and extracted with chloroform (3 x 500 mL). The combined organic layer was washed with water, brine, dried over sodium sulphate and concentrated to yield crude 4-isopropyl-1H-pyrazol-3-amine (45.0 g, 359 mmol, 72 % yield) as a brown color semi-solid. LCMS Retention time: 0.710min [B], MS (E⁺) *m*/*z:* 126.1 [M+H].

### Intermediate 183C: 3-bromo-4-isopropyl-1H-pyrazole

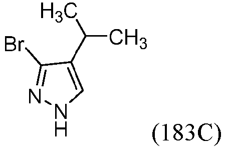

To a mixture of 4-isopropyl-1H-pyrazol-3-amine (15.0 g, 120 mmol) and p-toluene sulfonic acid monohydrate (34.2 g, 180 mmol) in acetonitrile (250 mL) were added isoamylnitrite (24.20 mL, 180 mmol), copper(II) bromide (26.8 g, 120 mmol) and tetrabutylammonium bromide (77 g, 240 mmol) at 10 °C. The mixture was stirred at room temperature for 2 h. The reaction was quenched with cold water. The reaction mixture was extracted with ethyl acetate (3 x 500 mL), the combined organic layer was washed with water, brine, dried over sodium sulphate and concentrated to get crude material. The crude material was purified by ISCO, using a silica column. The compound was eluted with 30%-90% ethyl acetate and pet ether. The fractions were collected and concentrated to yield 3-bromo-4-isopropyl-1H-pyrazole (14.0 g, 74.1 mmol, 62 % yield) as a light brown liquid. LCMS Retention time: 1.10 min [A], MS (E⁺) m/z: 191.3 [M+2H].

### Intermediate 183D: 3-bromo-5-iodo-4-isopropyl-1H-pyrazole

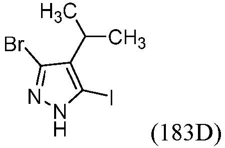

To a solution of 3-bromo-4-isopropyl-1H-pyrazole (14.0 g, 74.1 mmol) in acetonitrile (350 mL) was added NIS (33.3 g, 148 mmol) at room temperature. The reaction mixture was stirred at 80 °C for 16 h. The volatiles were evaporated. The residue was diluted with excess DCM, washed with water, brine, dried over sodium sulphate and concentrated to yield crude compound. The crude compound was purified by ISCO using 120 g silica column. The compound was eluted with 40-50% ethyl acetate in pet ether, the fractions were collected and concentrated to afford 3-bromo-5-iodo-4-isopropyl-1H-pyrazole (12.0 g, 38.1 mmol, 51 % yield) as a light brown solid. LCMS Retention time: 1.26 min [A], MS (E⁺) m/z: 317.2 [M+2H].

### Intermediate 183E: 3-bromo-5-iodo-4-isopropyl-1-((2-(trimethylsilyl)ethoxy)methyl)-1H-pyrazole

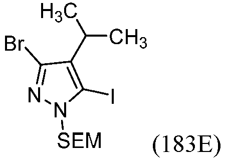

To a solution of 3-bromo-5-iodo-4-isopropyl-1H-pyrazole (40.0 g, 127 mmol) in THF (600 mL) were added NaH (7.62 g, 191 mmol) at 0 °C. The reaction mixture was stirred for 50 min. Next, SEM-Cl (31.8 g, 191 mmol) was added and the reaction mixture was stirred at room temperature for 16 h. The reaction was quenched with saturated NH₄Cl. The volatiles were evaporated, and the residue was diluted with ethyl acetate, washed with water, brine, dried over sodium sulphate and concentrated to yield crude compound. The crude compound was purified by ISCO using 120 g silica column, the compound was eluted with 25-30% ethyl acetate in pet ether, the fractions were collected and concentrated to yield 3-bromo-5-iodo-4-isopropyl-1-((2-(trimethylsilyl)ethoxy)methyl)-1H-pyrazole (30.0 g, 67.4 mmol, 53 % yield) as a light brown liquid. LCMS Retention time: 4.30 min [B] MS (E⁺) m/z: 447.0 [M+2H].

### Intermediate 183F: 6-(3-bromo-4-isopropyl-1-((2-(trimethylsilyl)ethoxy)methyl)-1H-pyrazol-5-yl)-8-methyl-[1,2,4]triazolo[1,5-a]pyridine

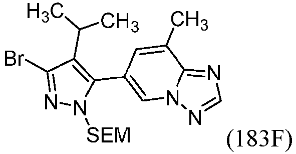

6-(3-bromo-4-isopropyl-1-((2-(trimethylsilyl)ethoxy)methyl)-1H-pyrazol-5-yl)-8-methyl-[1,2,4]triazolo[1,5-a]pyridine (12.2 g, 27.1 mmol, 86% yield) was prepared according to the general procedure described in Intermediate 1F using 3-bromo-5-iodo-4-isopropyl-1-((2-(trimethylsilyl)ethoxy)methyl)-1H-pyrazole (14 g, 31.4 mmol) and 8-methyl-6-(4,4,5,5-tetramethyl-1,3,2-dioxaborolan-2-yl)-[1,2,4]triazolo[1,5-a]pyridine (9.78 g, 37.7 mmol) as a starting intermediate to yield the title compound as a gummy solid. LCMS Retention time: 1.72 min [A] MS (E+) m/z: 452.0 [M+2H].

### Intermediate 183G: Ethyl 2-(4-isopropyl-5-(8-methyl-[1,2,4]triazolo[1,5-a]pyridin-6-yl)-1-((2-(trimethylsilyl)ethoxy)methyl)-1H-pyrazol-3-yl)-2-oxoacetate

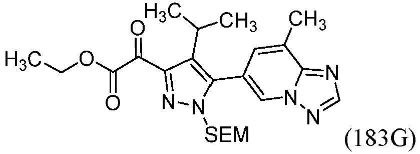

To a solution of 6-(3-bromo-4-isopropyl-1-((2-(trimethylsilyl)ethoxy)methyl)-1H-pyrazol-5-yl)-8-methyl-[1,2,4]triazolo[1,5-a]pyridine (0.070 g, 0.155 mmol) in THF (3.00 mL) was added 2.5 M nBuLi in hexanes (0.093 mL, 0.233 mmol) at -78 °C. The reaction mixture was stirred at the same temperature for 45 min. To a solution of diethyl oxalate (0.066 mL, 0.482 mmol) in THF (3.00 mL) was added dropwise the above reaction mixture at -78 °C, with stirring at the same temperature for 1 h. The reaction was quenched with the addition of water. The mixture was extracted with EtOAc (2 X 20 mL), the combined organic extracts were dried (Na₂SO₄) and concentrated to yield crude compound. The crude compound was purified by ISCO using 12 g silica column, eluted in 30% EA in hexanes, the fractions were collected and concentrated to yield ethyl 2-(4-isopropyl-5-(8-methyl-[1,2,4]triazolo[1,5-a]pyridin-6-yl)-1-((2-(trimethylsilyl)ethoxy) methyl)-1H-pyrazol-3-yl)-2-oxoacetate as a gummy solid. LCMS Retention time: 1.60 min [A], MS (ES): m/z= 472.6 [M+H].

### Intermediate 183H: 2-(4-isopropyl-5-(8-methyl-[1,2,4]triazolo[1,5-a]pyridin-6-yl)-1-((2-(trimethylsilyl)ethoxy)methyl)-1H-pyrazol-3-yl)-2-oxoacetic acid

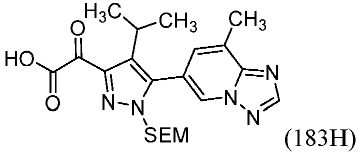

To a solution of ethyl 2-(4-isopropyl-5-(8-methyl-[1,2,4]triazolo[1,5-a]pyridin-6-yl)-1-((2-(trimethylsilyl)ethoxy)methyl)-1H-pyrazol-3-yl)-2-oxoacetate (0.060 g, 0.127 mmol) in THF (1.00 mL), MeOH (0.500 mL) and water (0.500 mL) solvent mixture was added LiOH (0.030 g, 1.272 mmol) at room temperature. The mixture was stirred at room temperature for 16 h. The reaction mixture was concentrated by removing MeOH and THF. The residue was diluted with water (5 mL) and washed with EtOAc (1 X 10 mL). The aqueous layer was brought to acidic pH with 5% HCl and extracted with 10% MeOH in DCM (2 X 20 mL). The combined organic extracts were dried (Na₂SO₄) and concentrated to afford crude 2-(4-isopropyl-5-(8-methyl-[1,2,4]triazolo[1,5-a]pyridin-6-yl)-1-((2-(trimethylsilyl)ethoxy) methyl)-1H-pyrazol-3-yl)-2-oxoacetic acid (0.042 g, 0.095 mmol, 74 % yield) as a gummy solid. LCMS Retention time: 0.88 min [A], MS (ES): m/z= 444.4 [M+H].

### Intermediate 183I: 2-(4-isopropyl-5-(8-methyl-[1,2,4]triazolo[1,5-a]pyridin-6-yl)-1-((2-(trimethylsilyl)ethoxy)methyl)-1H-pyrazol-3-yl)-N-(1-isopropylpiperidin-4-yl)-2-oxoacetamide

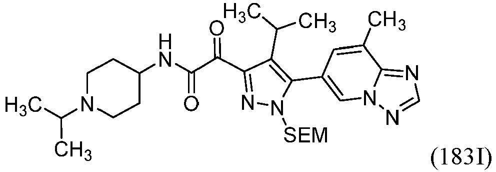

To a solution of 2-(4-isopropyl-5-(8-methyl-[1,2,4]triazolo[1,5-a]pyridin-6-yl)-1-((2-(trimethylsilyl)ethoxy)methyl)-1H-pyrazol-3-yl)-2-oxoacetic acid (0.042 g, 0.095 mmol) and 1-isopropylpiperidin-4-amine (0.013 g, 0.095 mmol) in DMF were added TEA (0.013 ml, 0.095 mmol) and HATU (0.036 g, 0.095 mmol) at room temperature. The reaction mixture was stirred at the same temperature for 1 h. The reaction was quenched with the addition of water. The reaction mixture was extracted with 10% MeOH in DCM (2 X 20 mL), the combined organic extracts were dried (Na₂SO₄) and concentrated to afford crude 2-(4-isopropyl-5-(8-methyl-[1,2,4]triazolo[1,5-a]pyridin-6-yl)-1-((2-(trimethylsilyl) ethoxy) methyl)-1H-pyrazol-3-yl)-N-(1-isopropylpiperidin-4-yl)-2-oxoacetamide (0.045 g, 0.079 mmol, 84 % yield) as a gummy solid. LCMS Retention time: 1.27 min [A], MS (ES): m/z= 568.6 [M+H].

### Example 183: 2-(4-isopropyl-5-(8-methyl-[1,2,4]triazolo[1,5-a]pyridin-6-yl)-1H-pyrazol-3-yl)-N-(1-isopropylpiperidin-4-yl)-2-oxoacetamide

To a solution of 2-(4-isopropyl-5-(8-methyl-[1,2,4]triazolo[1,5-a]pyridin-6-yl)-1-((2-(trimethylsilyl)ethoxy)methyl)-1H-pyrazol-3-yl)-N-(1-isopropylpiperidin-4-yl)-2-oxoacetamide (0.039 g, 0.068 mmol) in dioxane (0.500 mL) was added 4 M hydrochloric acid in dioxane (1.00 ml, 4.00 mmol) at room temperature. The reaction mixture was stirred at 70 °C for 16 h. The reaction mass was purified by Prep LCMS using method AB. After Preparative LCMS purification, fractions containing the product were combined and dried using Genevac centrifugal evaporator to get 2-(4-isopropyl-5-(8-methyl-[1,2,4]triazolo[1,5-a] pyridin-6-yl)-1H-pyrazol-3-yl)-N-(1-isopropyl piperidin-4-yl)-2-oxoacetamide, HCl (0.0027 g, 5.25 µmol, 8% yield) as a pale solid. LCMS Retention time: 1.285 min [F], MS (ES): *m*/*z=* 438.2 [M+H]; ¹H NMR (400 MHz, METHANOL-d₄) δ ppm 8.75 (s, 1H), 8.49 (s, 1H), 7.58 (s, 1H), 4.02-3.95 (m, 1H), 3.21-3.13 (m, 2H), 2.84-2.70 (m, 2H), 2.71 (s, 3H), 2.21 - 2.10 (m, 2H), 1.84-1.75 (m, 2H), 1.33 (d, J = 7.2 Hz, 6H), 1.24 (d, J = 6.8 Hz, 6H).

### EXAMPLE 184

### 1-(4-isopropyl-5-(8-methyl-[1,2,4]triazolo[1,5-a]pyridin-6-yl)-1H-pyrazol-3-yl)-N,N-dimethyl methanamine

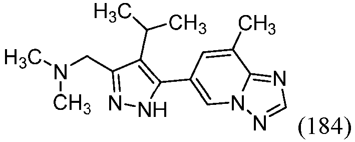

### Intermediate 184A: 4-isopropyl-5-(8-methyl-[1,2,4]triazolo[1,5-a]pyridin-6-yl)-1H-pyrazole-3-carbaldehyde

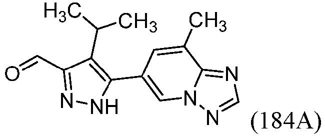

To a solution of methyl 4-isopropyl-5-(8-methyl-[1,2,4]triazolo[1,5-a]pyridin-6-yl)-1H-pyrazole-3-carboxylate (0.25 g, 0.835 mmol) in THF (10 mL) at -10 °C was added LiAlH₄ (0.383 mL, 0.919 mmol) dropwise, stirred at the same temperature for 30 min and allowed to stir at room temperature for 15 min. The reaction mass was quenched with water (5ml) and extracted with EtOAc (2 X 50 mL), separated organic layer was dried over sodium sulphate, filtered and concentrated to get crude product. The crude compound was purified by combiflash using 24 gm silica column by eluting with 2-6% MeOH/CHCl₃, the fractions were collected and concentrated to get 4-isopropyl-5-(8-methyl-[1,2,4]triazolo[1,5-a]pyridin-6-yl)-1H-pyrazole-3-carbaldehyde (0.1 g, 0.282 mmol, 34 % yield) as a yellow solid. LCMS 1.69 min [B]. MS (E⁻) *m*/*z:* 270.2 [M+H].

### Example 184: 1-(4-isopropyl-5-(8-methyl-[1,2,4]triazolo[1,5-a]pyridin-6-yl)-1H-pyrazol-3-yl)-N,N-dimethylmethanamine.

To a solution of 4-isopropyl-5-(8-methyl-[1,2,4]triazolo[1,5-a]pyridin-6-yl)-1H-pyrazole-3-carbaldehyde (0.025 g, 0.093 mmol) in MeOH (3 mL) was added TEA (0.013 mL, 0.093 mmol), dimethylamine (4.19 mg, 0.093 mmol) followed by acetic acid (5.31 µl, 0.093 mmol) dropwise, the resulting light yellow solution was stirred under nitrogen at 25°C for 12 h, to this was then added sodium cyanoborohydride (0.018 g, 0.278 mmol) and continued stirring at the same temperature for 6 h. The reaction mass was diluted with water (5 ml), extracted with EtOAc (3 X 10 mL), separated organic layer was dried over sodium sulphate, filtered and concentrated to get crude compound. The crude sample was purified by prep LCMS using method AB, the fractions were collected and concentrated to get 1-(4-isopropyl-5-(8-methyl-[1,2,4]triazolo[1,5-a]pyridin-6-yl)-1H-pyrazol-3-yl)-N,N-dimethylmethanamine (5.1 mg, 0.017 mmol, 18% yield) as a pale yellow solid. LCMS 0.97min [E]. MS (E⁻) *m*/*z:* 299.2 [M+H]; ¹H NMR (400 MHz, DMSO-d₆) δ 12.94 (s, 1H), 8.75 (br. s., 1H), 8.51 (br. s., 1H), 7.55 (br. s., 1H), 4.14-4.06(m, 1H), 3.17 (d, J=4.9 Hz, 3H), 3.03 (dt, J=14.3, 7.3 Hz, 1H), 2.60 (s, 3H), 2.33 (d, J=2.0 Hz,3H), 2.17-2.00 (m, 1H), 1.22 (d, J=7.1 Hz, 6H.)

The following Examples were prepared according to the general procedure used to prepare Example 184.

**TABLE 9**

| Ex. No. | Structure | LCMS MH⁺ | RT (min) | HPLC Method |
|---|---|---|---|---|
| 185 | 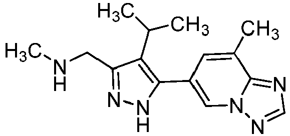 | 285.2 | 0.76 | E |
| 186 | 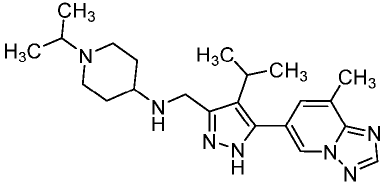 | 396.3 | 0.92 | E |
| 187 | 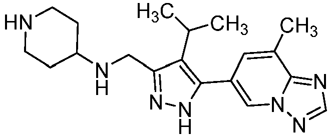 | 354.3 | 0.79 | E |

### EXAMPLES 188 AND 189 (REFERENCE)

### 4-isopropyl-N-(1-isopropylpiperidin-4-yl)-1-methyl-5-(8-methyl-[1,2,4] triazolo[1,5-a] pyridin-6-yl)-1H-pyrazole-3-carboxamide

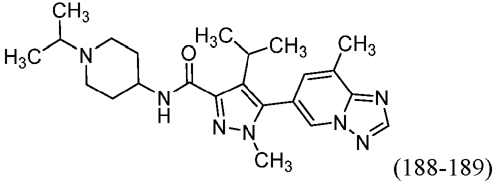

### Intermediate 188A: methyl 4-isopropyl-1-methyl-5-(8-methyl-[1,2,4]triazolo[1,5-a]pyridin-6-yl)-1H-pyrazole-3-carboxylate

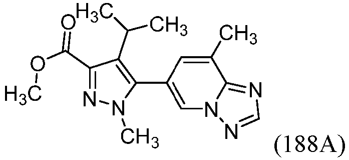

To a solution of methyl 4-isopropyl-5-(8-methyl-[1,2,4]triazolo[1,5-a]pyridin-6-yl)-1H-pyrazole-3-carboxylate (0.201 g, 0.672 mmol) in acetonitrile (12.00 mL) and DMF (1.5 mL) solvent mixture were added Cs₂CO₃ (2.188 g, 6.72 mmol) and MeI (0.210 mL, 3.36 mmol) at room temperature, then the mixture was stirred at the same temperature for 1 h. Filtered the reaction mass through celite bed, washed with EtOAc, concentrated the filtrate to get crude compound. The crude material was purified by ISCO using 24 g silica column, the mixture of regio isomer compounds was eluted in 4% MeOH in CHCl₃, the fractions were collected and concentrated to get methyl 4-isopropyl-1-methyl-5-(8-methyl-[1,2,4]triazolo[1,5-a]pyridin-6-yl)-1H-pyrazole-3-carboxylate (0.245 g, 0.782 mmol, 93% yield) as an off-white solid. LCMS Retention time: 0.98 and 1.14 min [A], MS (ES): m/z= 314.5 [M+H].

### Intermediate 188B: 4-isopropyl-1-methyl-5-(8-methyl-[1,2,4]triazolo[1,5-a]pyridin-6-yl)-1H-pyrazole-3-carboxylic acid

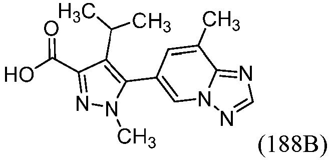

To a solution of methyl 4-isopropyl-1-methyl-5-(8-methyl-[1,2,4]triazolo[1,5-a]pyridin-6-yl)-1H-pyrazole-3-carboxylate (0.243 g, 0.775 mmol) in THF (2.00 mL) and MeOH (2.00 mL) was added 1M NaOH (1.551 mL, 1.551 mmol) at room temperature, then the mixture was stirred at 80 °C for 16 h. Concentrated the reaction mass, the residue was diluted with water (2 mL), then brought acidic using 0.5 N aqueous HCl, the precipitated out solid was filtered, washed with water dried under vacuum to get 4-isopropyl-1-methyl-5-(8-methyl-[1,2,4]triazolo[1,5-a]pyridin-6-yl)-1H-pyrazole-3-carboxylic acid (0.114 g, 0.381 mmol, 49% yield) as an off-white solid (mixture of regio isomers). LCMS Retention time: 0.40 min [A], MS (ES): m/z= 300.4 [M+H].

### Examples 188 and 189

To a solution of 4-isopropyl-1-methyl-5-(8-methyl-[1,2,4]triazolo[1,5-a]pyridin-6-yl)-1H-pyrazole-3-carboxylic acid (0.120 g, 0.401 mmol) in DMF were added TEA (0.168 ml, 1.203 mmol) and HATU (0.229 g, 0.601 mmol) at room temperature, the mixture was stirred at the same temperature for 16 h. The reaction mass purified by Prep LCMS using method AA to separate both the isomers. After purification the fractions containing the products were collected, combined and dried using Genevac centrifugal evaporator to afford Examples 188 and 189.
Example 188: (0.1055 g, 0.249 mmol, 62 % yield) as a white solid. LCMS Retention time: 0.90 min [A], MS (ES): *m*/*z=* 424.3 [M+H]; ¹H NMR (400 MHz, METHANOL-d₄) δ ppm 8.65 (s, 1H), 8.38 (s, 1H), 7.34 (s, 1H), 4.02-3.98 (m, 1H), 3.63 (s, 3H), 3.51-3.42 (m, 2H), 3.18-3.10 (m, 2H), 2.59 (s, 3H), 2.26-2.18 (m, 2H), 1.88-1.79 (m, 2H), 1.30 (d, J = 6.8 Hz, 6H), 1.11 (d, J = 7.2 Hz, 6H).
Example 189: (0.0313 g, 0.072 mmol, 18 % yield) as a white solid. LCMS Retention time: 1.098 min [A], MS (ES): *m*/*z=* 424.2 [M+H]; ¹H NMR (400 MHz, METHANOL-d₄) δ ppm 8.67 (s, 1H), 8.46 (s, 1H), 7.63 (s, 1H), 4.22-4.11 (m, 1H), 3.90 (s, 3H), 3.57-3.50 (m, 2H), 3.26-3.18 (m, 2H), 2.66 (s, 3H), 2.40-2.25 (m, 2H), 1.91-1.83 (m, 2H), 1.38 (d, J = 6.8 Hz, 6H), 1.28 (d, J = 7.2 Hz, 6H).

### EXAMPLE 190

### 5-(8-methoxy-[1,2,4]triazolo[1,5-a]pyridin-6-yl)-N-(piperidin-4-yl)-4-(2,2,2-trifluoroethyl)-1H-pyrazole-3-carboxamide

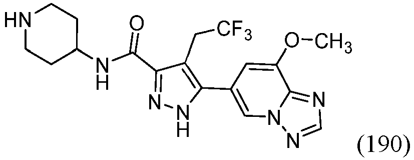

### Intermediate 190A: 4,4,4-trifluorobutanenitrile

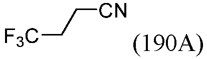

To a stirred solution of 4,4,4-trifluorobutanal (4 g, 31.7 mmol) in water (20 mL) was added hydroxylamine-o-sulfonic acid (3.95 g, 34.9 mmol) in water (20 mL) stirred at room temperature for 16 h. The reaction mass diluted with water extracted with DCM dried over sodium sulphate and concentrated to get 4,4,4-trifluorobutanenitrile (2.6 g, 21.12 mmol, 66% yield) as an oil.

### Intermediate 190B: Methyl 4-bromo-5-iodo-1-((2-(trimethylsilyl)ethoxy)methyl)-1H-pyrazole-3-carboxylate

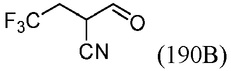

To a stirred solution of 4,4,4-trifluorobutanenitrile (2.6 g, 21.12 mmol) in THF (10 mL) was added LDA (21.12 mL, 21.12 mmol) at 0 °C, stirred for 5 min, to this was then added ethyl formate (1.565 g, 21.12 mmol), stirred room temperature 16 h. The reaction mass quenched with 1N HCl, extracted with EtOAc, dried over sodium sulphate and concentrated to get 4,4,4-trifluoro-2-formylbutanenitrile (2.5 g, 16.55 mmol, 78% yield) as a brown color oil.

### Intermediate 190C: 4-(2,2,2-trifluoroethyl)-1H-pyrazol-3-amine

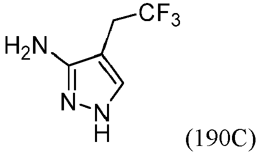

To a stirred solution of 4,4,4-trifluoro-2-formylbutanenitrile (2.5 g, 16.55 mmol) in EtOH (15 mL) were added hydrazine (1.298 mL, 41.4 mmol) and acetic acid (5 mL), then stirred at 100 ⁰C for 16 h. The reaction mass was concentrated; the residue was diluted with DCM washed with sat. NaHCO₃ solution and the aqueous layer was extracted with DCM, the combined organic extracts were dried (Na₂SO₄) and concentrated to get 4-(2,2,2-trifluoroethyl)-1H-pyrazol-3-amine (2.2 g, 13.32 mmol, 81 % yield) as a brown liquid. LCMS retention time 0.67 min [A]. MS (E⁻) *m*/*z:* 166.2 [M+H]

### Intermediate 190D: 3-bromo-4-(2,2,2-trifluoroethyl)-1H-pyrazole

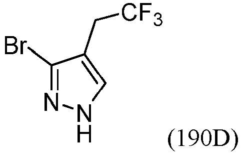

To a stirred solution of 4-(2,2,2-trifluoroethyl)-1H-pyrazol-3-amine (2.2 g, 13.32 mmol) in acetonitrile (20 mL) were added p-toluene sulfonic acid monohydrate (3.80 g, 19.99 mmol), isoamyl nitrite (2.93 mL, 19.99 mmol), cupric bromide (2.98 g, 13.32 mmol) and tetrabutylammonium bromide (8.59 g, 26.6 mmol) at room temperature, then stirred at the same temperature for 16 h. The reaction mass quenched with water extracted with EtOAc dried over sodium sulphate and concentrated to get crude. The crude mass was purified by ISCO using silica column (24 g), the fractions were collected and concentrated to get 3-bromo-4-(2,2,2-trifluoroethyl)-1H-pyrazole (1.8 g, 7.86 mmol, 59 % yield) as a brown liquid. LCMS retention time 1.30 min [A]. MS (E⁻) *m*/*z: 229.1* [M+H].

### Intermediate 190E: 3-bromo-5-iodo-4-(2,2,2-trifluoroethyl)-1H-pyrazole

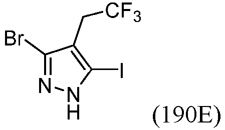

To a stirred solution of 3-bromo-4-(2,2,2-trifluoroethyl)-1H-pyrazole (1.3 g, 5.68 mmol) in acetonitrile (30 mL) was added NIS (6.39 g, 28.4 mmol) at room temperature, then stirred at 100 °C for 16 h. The reaction mass concentrated, the diluted with water, extracted with EtOAc, dried over sodium sulphate and concentrated to get crude. The crude mass was purified by ISCO silica column (24 g), the fractions were collected and concentrated to get 3-bromo-5-iodo-4-(2,2,2-trifluoroethyl)-1H-pyrazole (800 mg, 2.254 mmol, 40% yield) as a brown liquid. LCMS retention time 1.39 min [A]. MS (E⁻) *m*/*z:* 355.0 [M-H].

### Intermediate 190F: 3-bromo-5-iodo-4-(2,2,2-trifluoroethyl)-1-((2-(trimethylsilyl)ethoxy) methyl)-1H-pyrazole

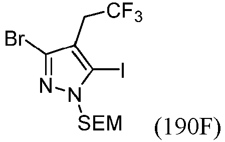

To a stirred solution of 3-bromo-5-iodo-4-(2,2,2-trifluoroethyl)-1H-pyrazole (1.2 g, 3.38 mmol) in THF (40 mL) was added NaH (0.203 g, 5.07 mmol) at 0 ⁰C, stirred at the same temperature for 30 min, to this was then added (trimethylsilyl)ethoxymethyl chloride (0.752 mL, 4.06 mmol) at the same temperature, stirred at room temperature for 3 h. The reaction mass quenched with water extracted with EtOAc, dried over sodium sulphate and concentrated to get crude. The crude mass was purified by ISCO silica column (24 g) to get 3-bromo-5-iodo-4-(2,2,2-trifluoroethyl)-1-((2-(trimethylsilyl)ethoxy) methyl)-1H-pyrazole (800 mg, 1.649 mmol, 49% yield) as brown liquid. LCMS retention time 2.41 min [A], MS (E⁻) *m*/*z* 485.0 [M+H].

### Intermediate 190G: 6-(3-bromo-4-(2,2,2-trifluoroethyl)-1-((2-(trimethylsilyl)ethoxy) methyl)-1H-pyrazol-5-yl)-8-methyl-[1,2,4]triazolo[1,5-a]pyridine

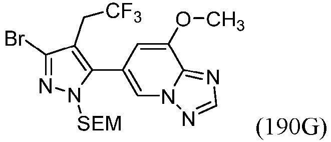

To a stirred solution of3-bromo-5-iodo-4-(2,2,2-trifluoroethyl)-1-((2-(trimethylsilyl)ethoxy)methyl)-1H-pyrazole (1.8 g, 3.71 mmol) in dioxane (100 mL) and water (3 mL) were added 8-methyl-6-(4,4,5,5-tetramethyl-1,3,2-dioxaborolan-2-yl)-[1,2,4]triazolo[1,5-a]pyridine (1.442 g, 5.57 mmol) and potassium phosphate tribasic (2.363 g, 11.13 mmol), the mixture was degassed with nitrogen for 10 min, to this was then added PdCl₂(dppf)-CH₂Cl₂adduct (0.303 g, 0.371 mmol) and the mixture was stirred at 100 ⁰C for 16 h. The reaction mass filtered through celite bed, washed with EtOAc, the filtrates were collected and concentrated to get crude. The crude mass was purified by ISCO using silica column (40 g), the fractions were collected and concentrated to get 6-(3-bromo-4-(2,2,2-trifluoroethyl)-1-((2-(trimethylsilyl) ethoxy)methyl)-1H-pyrazol-5-yl)-8-methyl-[1,2,4]triazolo[1,5-a]pyridine (1.3 g, 2.65 mmol, 71 % yield) as an oil. LCMS retention time 2.14 min [A], MS (E⁻) *m*/*z* 508.3 [M+H].

### Intermediate 190H: methyl 5-(8-methoxy-[1,2,4]triazolo[1,5-a]pyridin-6-yl)-4-(2,2,2-trifluoroethyl)-1-((2-(trimethylsilyl)ethoxy)methyl)-1H-pyrazole-3-carboxylate

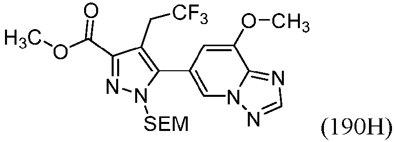

To a stirred solution of 6-(3-bromo-4-(2,2,2-trifluoroethyl)-1-((2-(trimethylsilyl)ethoxy)methyl)-1H-pyrazol-5-yl)-8-methoxy-[1,2,4]triazolo[1,5-a]pyridine (600 mg, 1.185 mmol) in DMF (10 mL) and MeOH (10 mL) were added 1,1'-bis(diphenylphosphino)ferrocene (131 mg, 0.237 mmol), palladium(ii) acetate (26.6 mg, 0.118 mmol) and TEA (0.413 mL, 2.96 mmol), degassed with nitrogen for 10 min, then was stirred at 80 ⁰C under CO in autoclave for 16 h. The reaction mass was brought to room temperature, filtered through celite, washed with MeOH, the filtrate was collected and concentrated to get crude compound. The crude mass was purified by ISCO using 24 g silica column, compound was eluted in 80% EA in hexanes, the fractions were collected and concentrated to get methyl 5-(8-methoxy-[1,2,4]triazolo[1,5-a]pyridin-6-yl)-4-(2,2,2-trifluoroethyl)-1-((2-(trimethylsilyl)ethoxy)methyl)-1H-pyrazole-3-carboxylate (460 mg, 1.088 mmol, 22% yield) as a brown color oil. LCMS retention time 1.86 min [A]. MS (E⁻) *m*/*z:* 486.3 [M+H].

### Intermediate 190I: 5-(8-methoxy-[1,2,4]triazolo[1,5-a]pyridin-6-yl)-4-(2,2,2-trifluoroethyl) -1-((2-(trimethylsilyl)ethoxy)methyl)-1H-pyrazole-3-carboxylic acid

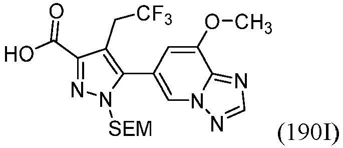

To a stirred solution of methyl 5-(8-methoxy-[1,2,4]triazolo[1,5-a]pyridin-6-yl)-4-(2,2,2-trifluoroethyl)-1-((2-(trimethylsilyl)ethoxy)methyl)-1H-pyrazole-3-carboxylate (300 mg, 0.618 mmol) in THF (10 mL), water (2 mL) and MeOH (5mL) solvent mixture was added LiOH (74.0 mg, 3.09 mmol) at room temperature, stirred 16 h. The reaction mass diluted with water extracted with EtOAc dried over sodium sulphate and concentrated to get 5-(8-methoxy-[1,2,4]triazolo[1,5-a]pyridin-6-yl)-4-(2,2,2-trifluoroethyl)-1-((2-(trimethylsilyl)ethoxy)methyl)-1H-pyrazole-3-carboxylic acid (240 mg, 0.509 mmol, 82% yield) as an off-white solid. LCMS retention time 0.96 min [A], MS (E⁻) *m*/*z:* 472.0 [M+H].

### Intermediate 190J: tert-butyl 4-(5-(8-methoxy-[1,2,4]triazolo[1,5-a]pyridin-6-yl)-4-(2,2,2-trifluoroethyl)-1-((2-(trimethylsilyl)ethoxy)methyl)-1H-pyrazole-3-carboxamido)piperidine -1-carboxylate

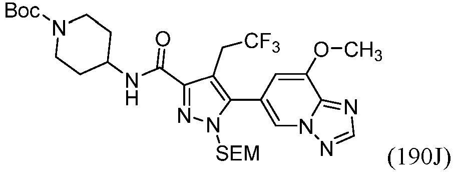

To a stirred solution of 5-(8-methoxy-[1,2,4]triazolo[1,5-a]pyridin-6-yl)-4-(2,2,2-trifluoroethyl)-1-((2-(trimethylsilyl)ethoxy)methyl)-1H-pyrazole-3-carboxylic acid (50 mg, 0.106 mmol) in DCM (15 mL) was added 1-propanephosphonic anhydride (0.062 mL, 0.212 mmol) and Et₃N (0.044 mL, 0.318 mmol) at room temperature, stirred for 16 h. The reaction mass was diluted with cool water, extracted with EtOAc, dried over sodium sulphate and concentrated to get crude compound, the crude mass was purified by ISCO using 4 g silica column, compound was eluted in EA, the fractions were collected and concentrated to get tert-butyl 4-(5-(8-methoxy-[1,2,4]triazolo[1,5-a]pyridin-6-yl)-4-(2,2,2-trifluoroethyl)-1-((2-(trimethylsilyl)ethoxy)methyl)-1H-pyrazole-3-carboxamido)piperidine-1-carboxylate (50 mg, 0.076 mmol, 72% yield) as an off-white solid. LCMS retention time 2.04 min [A]. MS (E⁻) m/z: 654.3 [M+H].

### Example 190: 5-(8-methoxy-[1,2,4]triazolo[1,5-a]pyridin-6-yl)-N-(piperidin-4-yl)-4-(2,2,2-trifluoroethyl)-1H-pyrazole-3-carboxamide

To a stirred solution of tert-butyl 4-(5-(8-methoxy-[1,2,4]triazolo[1,5-a]pyridin-6-yl)-4-(2,2,2-trifluoroethyl)-1-((2-(trimethylsilyl)ethoxy)methyl)-1H-pyrazole-3-carboxamido)piperidine-1-carboxylate (250 mg, 0.382 mmol) in DCM (2 mL) was added TFA (0.029 mL, 0.382 mmol) at room temperature, stirred for 16 h. The reaction mass was concentrated to get crude compound. The crude compound was purified by prep LCMS using method AB, fractions containing product were combined and dried using Genevac centrifugal evaporator to get 5-(8-methoxy-[1,2,4]triazolo[1,5-a]pyridin-6-yl)-N-(piperidin-4-yl)-4-(2,2,2-trifluoroethyl)-1H-pyrazole-3-carboxamide (160 mg, 0.252 mmol, 52 % yield) as an off-white solid. LCMS retention time 0.60 min [C]. MS (E⁻) m/z: 424 [M+H].

The following Examples were prepared according to the general procedure used to prepare Intermediate 190J.

**TABLE 10**

| Ex. No. | Structure | LCMS MH⁺ | RT (min) | HPLC Method |
|---|---|---|---|---|
| 191 | 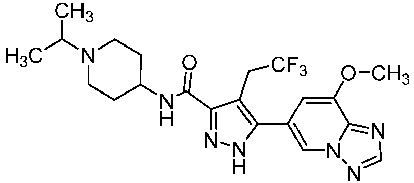 | 438.2 | 0.97 | C |
| 192 | 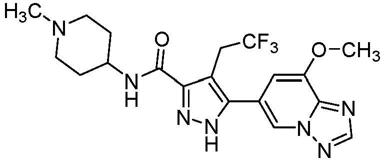 | 466.2 | 0.89 | C |
| 193 | 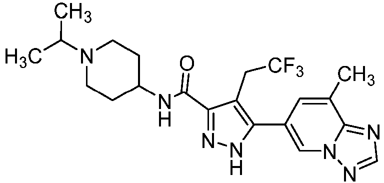 | 450.2 | 1.03 | C |

The following Example was prepared according to the general procedure used to prepare Example 190.

**TABLE 11**

| Ex. No. | Structure | LCMS MH⁺ | RT (min) | HPLC Method |
|---|---|---|---|---|
| 194 | 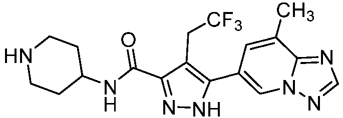 | 408.2 | 1.03 | C |

### EXAMPLE 195

### 5-(8-methoxy-[1,2,4]triazolo[1,5-a]pyridin-6-yl)-N-(1-propylpiperidin-4-yl)-4-(2,2,2-trifluoroethyl)-1H-pyrazole-3-carboxamide

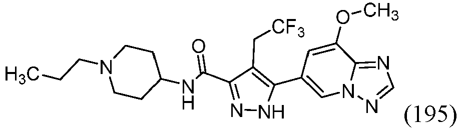

To a stirred solution of 5-(8-methoxy-[1,2,4]triazolo[1,5-a]pyridin-6-yl)-N-(piperidin-4-yl)-4-(2,2,2-trifluoroethyl)-1H-pyrazole-3-carboxamide (40 mg, 0.094 mmol) in MeOH (3 mL) was added propionaldehyde (7.13 mg, 0.123 mmol) and acetic acid (1.082 µl, 0.019 mmol) stirred at room temperature for 16 h, to this was then added NaCNBH₃ (11.87 mg, 0.189 mmol) and stirred for another 2 h. The reaction mass was purified by Prep LCMS using method AA, fractions containing product were combined and dried using Genevac centrifugal evaporator to get 5-(8-methoxy-[1,2,4]triazolo[1,5-a]pyridin-6-yl)-N-(1-propylpiperidin-4-yl)-4-(2,2,2-trifluoroethyl)-1H-pyrazole-3-carboxamide (2.3 mg, 7% yield) as an off-white solid. LCMS retention time 1.03 min [E]. MS (E⁻) m/z: 446.2 [M+H]; ¹H NMR (400 MHz, DMSO-d₆) δ = 13.97-13.83 (m, 1H), 9.27-9.09 (m, 1H), 8.93-8.80 (m, 1H), 8.64-8.49 (m, 2H), 7.28-7.19 (m, 1H), 4.21-3.95 (m,2H), 3.12-2.93 (m, 3H), 2.90 (s, 1H), 2.74 (s, 1H), 2.01 (br dd, J=1.3, 9.4 Hz, 2H), 1.94-1.78 (m, 3H), 1.74-1.59 (m, 3H), 1.03-0.80 (m, 6H).

The following Examples were prepared according to the general procedure used to prepare Example 195:

**TABLE 12**

| Ex. No. | Structure | Mol Wt. | LCMS MH⁺ | RT (min) | HPLC Method |
|---|---|---|---|---|---|
| 196 | 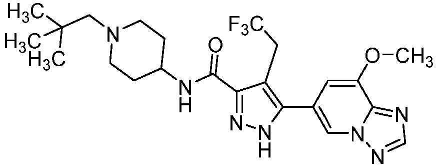 | 494.2 | 495.2 | 1.52 | C |
| 197 | 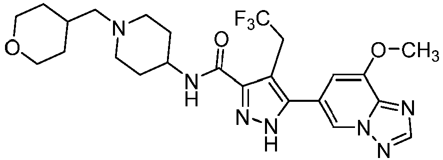 | 521.54 | 522.3 | 1.11 | C |
| 199 | 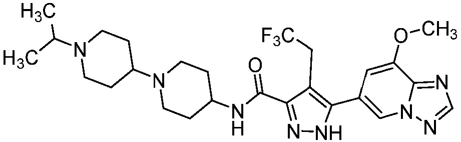 | 548.61 | 549.2 | 1.02 | C |
| 200 | 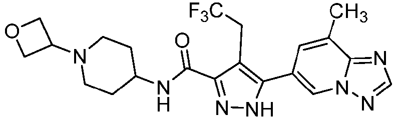 | 463.465 | 464 | 1.17 | C |
| 201 | 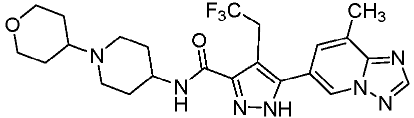 | 491.519 | 492.2 | 1.08 | C |
| 202 | 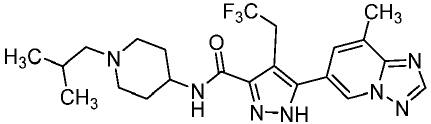 | 463.509 | 464.2 | 1.24 | C |
| 203 | 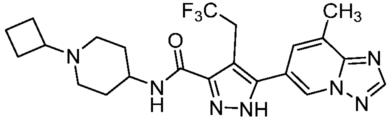 | 461.493 | 462.2 | 1.16 | C |
| 204 | 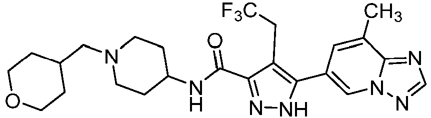 | 505.546 | 506.2 | 1.17 | C |

### EXAMPLE 205

### 5-(8-methoxy-[1,2,4]triazolo[1,5-a]pyridin-6-yl)-N-(1-(2-(methylamino)-2-oxoethyl) piperidin-4-yl)-4-(2,2,2-trifluoroethyl)-1H-pyrazole-3-carboxamide

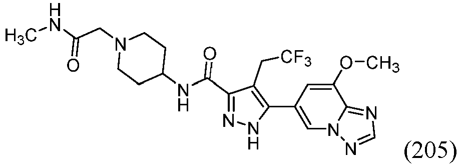

To a stirred solution of 5-(8-methoxy-[1,2,4]triazolo[1,5-a]pyridin-6-yl)-N-(piperidin-4-yl)-4-(2,2,2-trifluoroethyl)-1H-pyrazole-3-carboxamide (40 mg, 0.094 mmol) in DMF (2 mL) and THF (2 ml) was added Et₃N (0.040 mL, 0.283 mmol) at room temperature, stirred for 5 min, to this was then added 2-chloro-N-methylacetamide (12.19 mg, 0.113 mmol) and stirred for 16 h. The reaction mass was purified by Prep LCMS using method AA, fractions containing product were combined and dried using Genevac centrifugal evaporator to get 5-(8-methoxy-[1,2,4]triazolo[1,5-a]pyridin-6-yl)-N-(1-(2-(methylamino)-2-oxoethyl)piperidin-4-yl)-4-(2,2,2-trifluoroethyl)-1H-pyrazole-3-carboxamide (4.3 mg, 7 % yield). LCMS retention time 1.07 min [E]. MS (E⁻) m/z: 495.2 [M+H]; ¹H NMR (400 MHz, DMSO-d₆) δ ppm 8.91-8.78 (m, 1H), 8.54 (s, 1H), 8.19-8.00 (m, 1H), 7.62 (br d, J=3.5 Hz, 1H), 7.23 (d, *J*=1.0 Hz, 1H), 4.20-3.98 (m,5H), 3.83-3.70 (m, 1H), 3.05-2.87 (m, 4H), 2.84-2.75 (m, 2H), 2.62 (d, *J*=4.5 Hz, 5H), 2.24-2.10 (m, 2H), 1.91 (s, 2H), 1.81-1.58 (m, 5H), 1.19-1.05(m, 2H).

### EXAMPLE 206

### 6-(4-isopropyl-3-(1-methylpiperidin-4-yl)-1H-pyrazol-5-yl)-8-methyl-[1,2,4]triazolo [1,5-a]pyridine

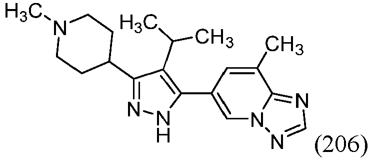

### Intermediate 206A: 6-(3-bromo-4-isopropyl-1H-pyrazol-5-yl)-8-methyl-[1,2,4]triazolo[1,5-a] pyridine

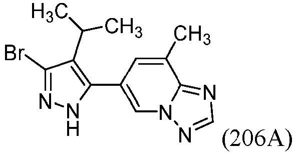

PdCl₂(dppf)-CH₂Cl₂ adduct (1.556 g, 1.905 mmol) and K₃PO₄ (9.95 g, 57.2 mmol) were added to a degassed solution of 3-bromo-5-iodo-4-isopropyl-1H-pyrazole (6.0 g, 19.05 mmol) and 8-methyl-6-(4,4,5,5-tetramethyl-1,3,2-dioxaborolan-2-yl)-[1,2,4]triazolo[1,5-a]pyridine (7.40 g, 28.6 mmol) in dioxane (150 mL) and water (35 mL) solvent mixture, then the mixture was stirred at 95 °C for 4 h in a sealed tube. The reaction mixture was diluted with ethyl acetate, filtered, the filtrate was washed water, brine, dried over sodium sulphate and concentrate to get crude compound. The crude compound was purified by ISCO using 80 g silica column, compound was eluted with 65% ethyl acetate in pet ether, the fractions were collected and concentrated to get 6-(3-bromo-4-isopropyl-1H-pyrazol-5-yl)-8-methyl-[1,2,4]triazolo[1,5-a]pyridine (3.0 g, 9.37 mmol, 49 % yield) as a light brown solid. LCMS Retention time: 1.07 min [A], MS (E⁺) m/z: 322.4 [M+2H].

### Intermediate 206B: tert-butyl 4-(4-isopropyl-5-(8-methyl-[1,2,4]triazolo[1,5-a]pyridin-6-yl)-1H-pyrazol-3-yl)-3,6-dihydropyridine-1(2H)-carboxylate

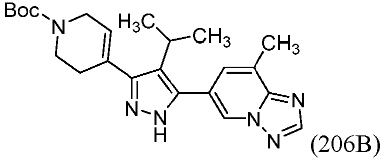

Pd₂(dba)₃ (0.057 g, 0.062 mmol) and S-Phos (0.051 g, 0.125 mmol) were added to a degassed solution of tert-butyl 4-(4,4,5,5-tetramethyl-1,3,2-dioxaborolan-2-yl)-5,6-dihydropyridine-1(2H)-carboxylate (0.579 g, 1.874 mmol), K₂CO₃ (0.518 g, 3.75 mmol) and 6-(3-bromo-4-isopropyl-1H-pyrazol-5-yl)-8-methyl-[1,2,4]triazolo[1,5-a]pyridine (0.400 g, 1.249 mmol) in mixture of acetonitrile (20 mL and water (4.0 mL), then the mixture was stirred at 110 °C for 14 h in a sealed tube. The reaction mixture was diluted with ethyl acetate, filtered, the filtrate was washed with water, brine, dried over sodium sulphate and concentrated to get crude compound. The crude compound was purified by ISCO using 40 g silica column, compound was eluted with ethyl acetate, the fractions were collected and concentrated to get tert-butyl 4-(4-isopropyl-5-(8-methyl-[1,2,4]triazolo[1,5-a]pyridin-6-yl)-1H-pyrazol-3-yl)-5,6-dihydropyridine-1(2H)-carboxylate (0.450 g, 1.065 mmol, 85% yield as an off-white solid. LCMS Retention time: 1.48 min [A], MS (E⁺) m/z: 423.6 [M+H].

### Intermediate 206C: tert-butyl 4-(4-isopropyl-5-(8-methyl-[1,2,4]triazolo[1,5-a]pyridin-6-yl)-1H-pyrazol-3-yl)piperidine-1-carboxylate

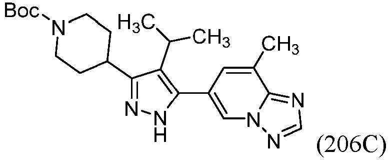

To a solution of tert-butyl 4-(4-isopropyl-5-(8-methyl-[1,2,4]triazolo[1,5-a]pyridin-6-yl)-1H-pyrazol-3-yl)-5,6-dihydropyridine-1(2H)-carboxylate (0.400 g, 0.947 mmol) in MeOH (25 mL) was added Pd/C (0.201 g, 1.893 mmol), then the slurry was stirred at room temperature for 36 h under hydrogen bladder. The reaction mixture was filtered through celite, washed with methanol, the filtrate was collected and concentrated to get tert-butyl 4-(4-isopropyl-5-(8-methyl-[1,2,4]triazolo[1,5-a]pyridin-6-yl)-1H-pyrazol-3-yl)piperidine-1-carboxylate (0.350 g, 0.824 mmol, 87 % yield) as a light brown solid. LCMS Retention time: 1.37 min [A], MS (E⁺) m/z: 425.6 [M+H].

### Intermediate 206D: 6-(4-isopropyl-3-(piperidin-4-yl)-1H-pyrazol-5-yl)-8-methyl-[1,2,4] triazolo[1,5-a]pyridine

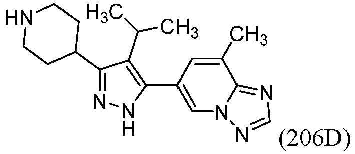

To a solution of tert-butyl 4-(4-isopropyl-5-(8-methyl-[1,2,4]triazolo[1,5-a]pyridin-6-yl)-1H-pyrazol-3-yl)piperidine-1-carboxylate (0.350 g, 0.824 mmol) in dioxane (2.0 mL) was added 4M HCl in dioxane (4.12 mL, 16.49 mmol) at room temperature, then stirred at the same temperature for 2 h. Concentrated the reaction mass and dried under vacuum to get 6-(4-isopropyl-3-(piperidin-4-yl)-1H-pyrazol-5-yl)-8-methyl-[1,2,4]triazolo[1,5-a]pyridine HCl (0.450 g) as a light yellow solid. LCM Retention time: 1.1 min [E], MS (E⁺) m/z: 325.1 [M+H]; ¹H NMR (400 MHz, METHANOL-d₄) δ ppm 8.65 (s, 1 H) 8.45 (s, 1 H) 7.54 (s, 1 H) 3.49 (s, 2 H) 3.05-3.25 (m, 4 H) 2.67 (s, 3 H) 1.86-2.10 (m, 6 H) 1.28 (d, J=7.09 Hz, 6 H).

### Example 206: 6-(4-isopropyl-3-(1-methylpiperidin-4-yl)-1H-pyrazol-5-yl)-8-methyl-[1,2,4]triazolo[1,5-a]pyridine

To a solution of 6-(4-isopropyl-3-(piperidin-4-yl)-1H-pyrazol-5-yl)-8-methyl-[1,2,4]triazolo [1,5-a]pyridine (0.040 g, 0.123 mmol), formaldehyde (0.017 mL, 0.616 mmol) and acetic acid (0.706 µl, 0.012 mmol) in MeOH (5.0 mL) was stirred at room temperature for 8 h, to this was then added sodium cyanoborohydride (0.012 g, 0.185 mmol) at 0°C and stirred at room temperature for 16 h. The reaction mass was purified via preparative LC/MS using method AA, fractions containing the product were combined and dried via centrifugal evaporation to get 6-(4-isopropyl-3-(1-methylpiperidin-4-yl)-1H-pyrazol-5-yl)-8-methyl-[1,2,4]triazolo[1,5-a]pyridine (3.8 mg) as a white solid. LCMS Retention time 1.25 min [C], MS (E⁺) m/z: 339.2 [M+H]; ¹H NMR (400 MHz, METHANOL-d₄) δ ppm 8.63 (br. s., 1 H) 8.43 (s, 1 H) 7.54 (br. s., 1 H) 3.47 (s, 1 H) 2.92-3.19 (m, 4 H) 2.66 (s, 3 H) 2.43 (br. s., 4 H) 1.83-2.07 (m, 4 H) 1.19-1.34 (m, 6 H).

The following Example was prepared according to the general procedure used to prepare Example 206.

**TABLE 13**

| Ex. No. | Structure | LCMS MH⁺ | RT (min) | HPLC Method |
|---|---|---|---|---|
| 207 | 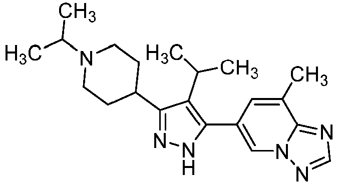 | 367.3 | 1.34 | C |

### EXAMPLE 208

### 3-(dimethylamino)-1-(4-(4-isopropyl-5-(8-methyl-[1,2,4]triazolo[1,5-a] pyridine-6-yl)-1H-pyrazol-3-yl)piperidin-1-yl)propan-1-one

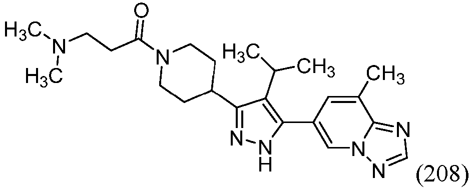

### Intermediate 208A: tert-butyl (3-(4-(4-isopropyl-5-(8-methyl-[1,2,4]triazolo[1,5-a] pyridin-6-yl)-1H-pyrazol-3-yl)piperidin-1-yl)-3-oxopropyl)carbamate

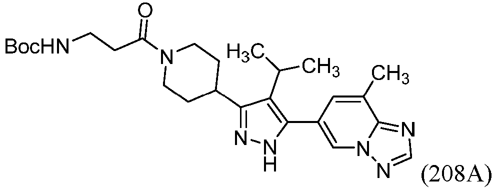

To a solution of 6-(4-isopropyl-3-(piperidin-4-yl)-1H-pyrazol-5-yl)-8-methyl-[1,2,4]triazolo[1,5-a]pyridine (0.200 g, 0.616 mmol) and 3-((tert-butoxycarbonyl)amino) propanoic acid (0.117 g, 0.616 mmol) in DMF (5.0 mL) and THF (2.0 mL) solvent mixture were added TEA (0.430 mL, 3.08 mmol) and HATU (0.234 g, 0.616 mmol) at 0 °C, the mixture was stirred at room temperature for 16 h. The reaction mixture was extracted with DCM, washed with water, brine, dried over sodium sulphate and concentrated to get crude compound. The crude compound was purified by ISCO using 24 g silica column, the compound was eluted with 5% MeOH in DCM, the fractions were collected and concentrated to get tert-butyl (3-(4-(4-isopropyl-5-(8-methyl-[1,2,4]triazolo[1,5-a]pyridin-6-yl)-1H-pyrazol-3-yl)piperidin-1-yl)-3-oxopropyl) carbamate (0.180 g, 0.363 mmol, 59 % yield) as an oil. LCMS Retention time: 1.13 min [A], MS (E⁺) m/z: 496.6 [M+H].

### Intermediate 208B: 3-Amino-1-(4-(4-isopropyl-5-(8-methyl-[1,2,4]triazolo[1,5-a]pyridin-6-yl)-1H-pyrazol-3-yl)piperidin-1-yl)propan-1-one

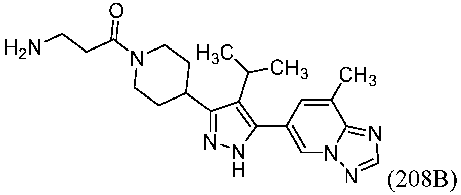

To a solution of tert-butyl (3-(4-(4-isopropyl-5-(8-methyl-[1,2,4]triazolo[1,5-a]pyridin-6-yl) -1H-pyrazol-3-yl)piperidin-1-yl)-3-oxopropyl)carbamate (0.250 g, 0.504 mmol) in dioxane (3.0 mL) was added 4M HCl in dioxane (3.78 mL, 15.13 mmol) at room temperature, stirred for 2h. The reaction mass was concentrated to get crude compound. The crude material was purified by preparative LC/MS using method AA, fractions containing the product were combined and dried via centrifugal evaporation to get 3-Amino-1-(4-(4-isopropyl-5-(8-methyl-[1,2,4]triazolo[1,5-a]pyridin-6-yl)-1H-pyrazol-3-yl)piperidin-1-yl)propan-1-one (38 mg) as an off-white solid. LCMS Retention time: 0.92 min [C], MS (E⁺) m/z: 396.3 [M+H]; ¹H NMR (400 MHz, METHANOL-d₄) δ ppm 8.66 (d, *J*=1.0 Hz, 1H), 8.46 (s, 1H), 7.64-7.42 (m,1H), 4.78-4.66 (m, 1H), 4.19-3.92 (m, 1H), 3.30-3.16 (m, 4H), 3.09 (quin, J=7.2 Hz, 1H),2.94-2.76 (m, 3H), 2.75-2.61 (m, 3H), 2.08-1.92 (m, 7H), 1.90-1.65 (m, 2H), 1.31 (d, J=7.5Hz, 6H).

### Example 208: 3-(dimethylamino)-1-(4-(4-isopropyl-5-(8-methyl-[1,2,4]triazolo[1,5-a] pyridin-6-yl)-1H-pyrazol-3-yl)piperidin-1-yl)propan-1-one

Mixture of 3-amino-1-(4-(4-isopropyl-5-(8-methyl-[1,2,4]triazolo[1,5-a]pyridin-6-yl)-1H-pyrazol-3-yl)piperidin-1-yl)propan-1-one (0.040 mg, 0.101 µmol), formaldehyde (0.014 µL, 0.506 µmol) and acetic acid (0.05 mL) in methanol (5.0 mL) was stirred at room temperature for 8 h, to this was then added sodium cyanoborohydride (0.013 mg, 0.202 µmol) at 0 °C, the resulting reaction mixture was stirred at room temperature for 16 h. The reaction mass purified by preparative LC/MS using method AB, fractions containing the product were combined and dried via centrifugal evaporation to get 3-(dimethylamino)-1-(4-(4-isopropyl-5-(8-methyl-[1,2,4] triazolo[1,5-a] pyridin-6-yl)-1H-pyrazol-3-yl)piperidin-1-yl)propan-1-one (20.2 mg) as a white solid. LCMS Retention time: 0.97 min [F], MS (E⁺) m/z: 424.3 [M+H]; ¹H NMR (400 MHz, METHANOL-d₄) δ ppm 8.67 (s, 1H), 8.48 (s, 1H), 7.56 (d, *J*=1.5 Hz, 1H), 4.79-4.69 (m, 1H), 4.07 (d, *J*=13.6 Hz, 1H), 3.52-3.39 (m, 2H), 3.31-3.17 (m, 2H), 3.09 (quin, *J*=7.2 Hz, 1H), 3.04-2.97 (m, 2H), 2.95 (d, *J*=5.5 Hz, 6H), 2.88-2.76 (m, 1H), 2.74-2.62 (m, 3H), 2.10-1.93 (m, 2H), 1.92-1.71 (m, 2H), 1.38-1.25 (m, 6H).

The following Example was prepared according to the general procedure used to prepare Example 208.

**TABLE 14**

| Ex. No. | Structure | Mol Wt. | LCMS MH⁺ | RT (min) | HPLC Method |
|---|---|---|---|---|---|
| 209 | 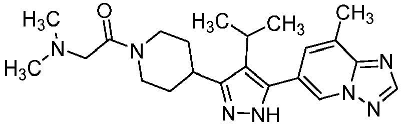 | 409.5 | 410.2 | 1.23 | C |

The following Examples were prepared according to the general procedure used to prepare Example 208.

**TABLE 15**

| Ex. No. | Structure | LCMS MH⁺ | RT (min) | HPLC Method |
|---|---|---|---|---|
| 210 | 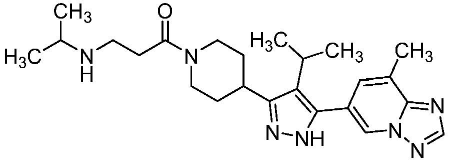 | 438.3 | 1 | C |

### EXAMPLE 211

### 2-(4-(4-isopropyl-5-(8-methyl-[1,2,4]triazolo[1,5-a]pyridin-6-yl)-1H-pyrazol-3-yl) piperidin-1-yl)-N-methylacetamide

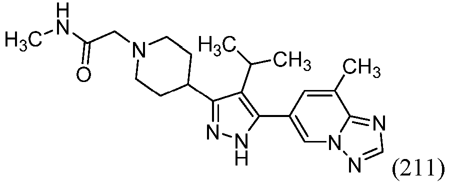

2-chloro-N,N-dimethylacetamide (0.022 g, 0.185 mmol) and TEA (0.064 mL, 0.462 mmol) were added to a solution of 6-(4-isopropyl-3-(piperidin-4-yl)-1H-pyrazol-5-yl)-8-methyl-[1,2,4]triazolo[1,5-a]pyridine (0.030 g, 0.092 mmol) in DMF (1.0 mL) and THF (1.0 mL) solvent mixture at 0 °C, then stirred at room temperature for 16 h. The reaction mass was purified via preparative LC/MS using method AA, fractions containing the product were combined and dried via centrifugal evaporation to get 2-(4-(4-isopropyl-5-(8-methyl-[1,2,4]triazolo[1,5-a]pyridin-6-yl)-1H-pyrazol-3-yl)piperidin-1-yl)-N-methylacetamide (9.2 mg) as an off-white solid. LCMS Retention time 1.50 min [C], MS (E⁺) m/z: 410.2 [M+H]; ¹H NMR (400 MHz, METHANOL-d₄) δ ppm 8.62 (br. s., 1 H) 8.43 (s, 1 H) 7.56 (br. s., 1 H) 3.40-3.53 (m, 1 H) 3.01-3.17 (m, 6 H) 2.89-2.99 (m, 4 H) 2.67 (s, 3 H) 2.29 (br. s., 2 H) 1.81-2.07 (m, 4 H) 1.17-1.32 (m, 7 H).

The following Example was prepared according to the general procedure used to prepare Example 211.

**TABLE 16**

| Ex. No. | Structure | Mol Wt. | LCMS MH⁺ | RT (min) | HPLC Method |
|---|---|---|---|---|---|
| 212 | 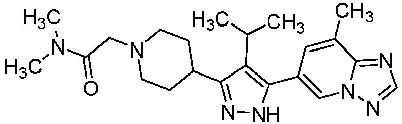 | 409.5 | 410.2 | 1.5 | C |

### EXAMPLE 213

### 6-(4-isopropyl-5-(8-methyl-[1,2,4]triazolo[1,5-a]pyridin-6-yl)-1H-pyrazol-3-yl)-1,2,3,4-tetrahydroisoquinoline, HCl

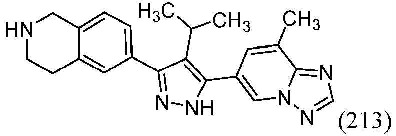

### Intermediate 213A: 3-bromo-5-iodo-4-isopropyl-1-((2-(trimethylsilyl)ethoxy)methyl)-1H-pyrazole

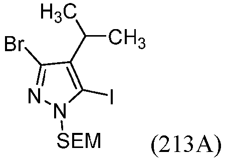

To a solution of 3-bromo-5-iodo-4-isopropyl-1H-pyrazole (40.0 g, 127 mmol) in THF (600 mL) were added NaH (7.62 g, 191 mmol) at 0 °C, stirred for 50 min, and SEM-Cl (31.8 g, 191 mmol), then the mixture was stirred at room temperature for 16 h. The reaction mixture was quenched with saturated NH₄Cl, volatiles were evaporated, and the residue was diluted with ethyl acetate, washed with water, brine, dried over sodium sulphate and concentrated to get crude compound. The crude compound was purified by ISCO using 120 g silica column, compound was eluted with 25-30% ethyl acetate in pet ether, the fractions were collected and concentrated to get 3-bromo-5-iodo-4-isopropyl-1-((2-(trimethylsilyl) ethoxy)methyl)-1H-pyrazole (30.0 g, 67.4 mmol, 53 % yield) as a light brown liquid. LCMS Retention time: 4.30 min [B] MS (E⁺) m/z: 447.0 [M+2H].

### Intermediate 213B: 6-(3-bromo-4-isopropyl-1-((2-(trimethylsilyl)ethoxy)methyl)-1H-pyrazol-5-yl)-8-methyl-[1,2,4]triazolo[1,5-a]pyridine

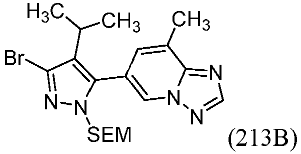

6-(3-bromo-4-isopropyl-1-((2-(trimethylsilyl)ethoxy)methyl)-1H-pyrazol-5-yl)-8-methyl-[1,2,4]triazolo[1,5-a]pyridine (12.2 g, 27.1 mmol, 86% yield) was prepared according to the general process described in Intermediate 206A using 3-bromo-5-iodo-4-isopropyl-1-((2-(trimethylsilyl) ethoxy)methyl)-1H-pyrazole (14 g, 31.4 mmol) and 8-methyl-6-(4,4,5,5-tetramethyl-1,3,2-dioxaborolan-2-yl)-[1,2,4]triazolo[1,5-a]pyridine (9.78 g, 37.7 mmol) as starting intermediate to yield the title compound as a gummy solid. LCMS Retention time: 1.72 min [A] MS (E⁺) m/z: 452.0 [M+2H].

The following Intermediates were prepared according to the general procedure used to prepare Intermediate 213B.

**TABLE 17**

| Intermediate | Structure | LCMS MH⁺ | RT (min) | HPLC Method |
|---|---|---|---|---|
| 213C | 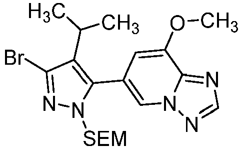 | 468.3 | 1.26 | A |
| 213D | 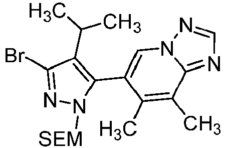 | 466.4 | 1.40 | A |
| 213E | 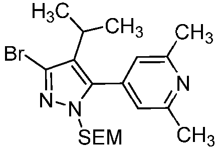 | 427.2 | 1.60 | A |
| 213F | 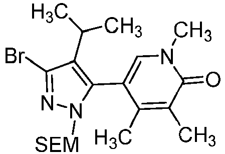 | 456.3 | 1.18 | A |
| 213G | 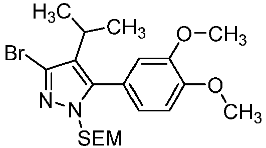 | 457.3 | 1.71 | A |

### Intermediate 213H: 6-(4-isopropyl-3-(4,4,5,5-tetramethyl-1,3,2-dioxaborolan-2-yl)-1-((2-(trimethylsilyl)ethoxy)methyl)-1H-pyrazol-5-yl)-8-methyl-[1,2,4]triazolo[1,5-a]pyridine

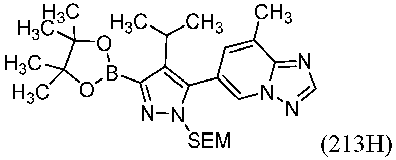

Pd₂dba₃ (0.508 g, 0.555 mmol), tricyclohexylphosphine (0.311 g, 1.110 mmol) and potassium acetate (3.27 g, 33.3 mmol) were added to a degassed solution of BISPIN (3.66 g, 14.43 mmol) and 6-(3-bromo-4-isopropyl-1-((2-(trimethylsilyl)ethoxy)methyl)-1H-pyrazol-5-yl)-8-methyl-[1,2,4]triazolo[1,5-a]pyridine (5.0 g, 11.10 mmol) in dioxane (100 mL), the mixture was stirred at 110 °C for 14 h in a sealed tube. The reaction mixture was diluted with ethyl acetate, filtered and washed with ethyl acetate. The filtrate was collected and concentrated to get crude compound. The crude compound was purified by ISCO using silica column 80 g silica column, compound was eluted with 45% ethyl acetate in Pet ether, the fractions were collected and concentrated to get 6-(4-isopropyl-3-(4,4,5,5-tetramethyl-1,3,2-dioxaborolan-2-yl)-1-((2-(trimethylsilyl)ethoxy)methyl)-1H-pyrazol-5-yl)-8-methyl-[1,2,4]triazolo[1,5-a]pyridine (4.5 g, 9.04 mmol, 81 % yield) as an off-white solid. LCMS Retention time: 1.87 min [A] MS (E⁺) m/z: 498.4 [M+H].

The following Intermediates were prepared according to the general procedure used to prepare Intermediate 213H.

**TABLE 18**

| Intermediate | Structure | LCMS MH⁺ | RT (min) | HPLC Method |
|---|---|---|---|---|
| 2311 | 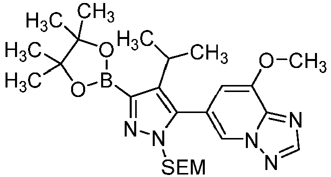 | 514.4 | 1.67 | A |
| 213J | 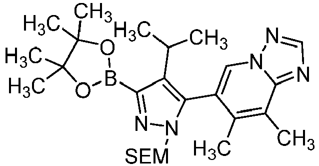 | 512.5 | 1.75 | A |
| 213K | 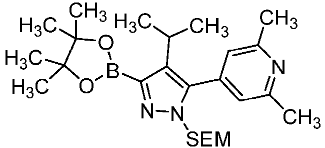 | 473.4 | 1.73 | A |
| 213L | 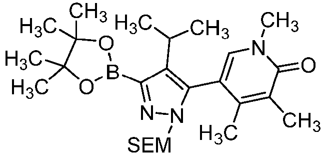 | 502.5 | 1.48 | A |
| 213M | 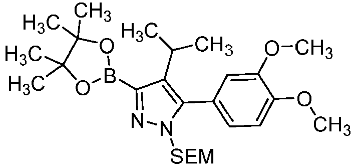 | 503.5 | 1.99 | A |

### Intermediate 213N: 6-(4-isopropyl-5-(8-methyl-[1,2,4]triazolo[1,5-a]pyridin-6-yl)-1-((2-(trimethylsilyl)ethoxy)methyl)-1H-pyrazol-3-yl)-1,2,3,4-tetrahydroisoquinoline

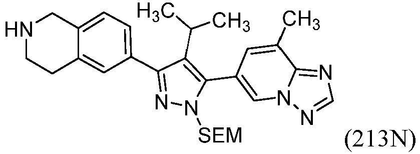

To a solution of 6-(4-isopropyl-3-(4,4,5,5-tetramethyl-1,3,2-dioxaborolan-2-yl)-1-((2-(trimethylsilyl)ethoxy)methyl)-1H-pyrazol-5-yl)-8-methyl-[1,2,4]triazolo[1,5-a]pyridine (0.600 g, 1.206 mmol), 6-bromo-1,2,3,4-tetrahydroisoquinoline (0.512 g, 2.412 mmol) and K₂CO₃ (0.500 g, 3.62 mmol) in acetonitrile (16.00 mL) and water (8.00 mL) solvent mixture was degassed for 10 min with nitrogen, to this was then added Pd₂(dba)₃ (0.055 g, 0.060 mmol) and 2-dicyclohexylphosphino-2',6'-dimethoxybiphenyl (0.050 g, 0.121 mmol), again degassed for 2 min, and stirred at 110 °C for 16 h. Separated both the layers, the aqueous layer was extracted with 10% MeOH in DCM (2 X 30 mL), the combined organic extracts were dried (Na₂SO₄) and concentrated to get crude compound. The crude compound was purified by ISCO using 24 g silica column, compound was eluted in 10% MeOH in DCM, the fractions were collected and concentrated to get 6-(4-isopropyl-5-(8-methyl-[1,2,4]triazolo[1,5-a]pyridin-6-yl)-1-((2-(trimethylsilyl) ethoxy)methyl)-1H-pyrazol-3-yl)-1,2,3,4-tetrahydroisoquinoline (0.340 g, 0.676 mmol, 56 % yield) as an off-white solid. LCMS Retention time 1.29 min [A], MS (E⁺) m/z: 503.7 [M+H].

### Example 213: 6-(4-isopropyl-5-(8-methyl-[1,2,4]triazolo[1,5-a]pyridin-6-yl)-1H-pyrazol-3-yl)-1,2,3,4-tetrahydroisoquinoline, HCl

To a solution of 6-(4-isopropyl-5-(8-methyl-[1,2,4]triazolo[1,5-a]pyridin-6-yl)-1-((2-(trimethylsilyl)ethoxy)methyl)-1H-pyrazol-3-yl)-1,2,3,4-tetrahydroisoquinoline (0.360 g, 0.716 mmol) in dioxane (3.00 mL) was added 4 M HCl in dioxane (5.00 ml, 20.00 mmol) at room temperature, then the mixture was stirred at 70 °C for 16 h. Concentrated the reaction mass to get crude compound, the crude compound was triturated with diethyl ether (2 X 10 mL), then dried under vacuum to get 6-(4-isopropyl-5-(8-methyl-[1,2,4]triazolo[1,5-a]pyridin-6-yl)-1H-pyrazol-3-yl)-1,2,3,4-tetrahydroisoquinoline, HCl (0.270 g, 0.660 mmol, 92 % yield) as a white solid. LCMS Retention time 0.76 min [A], MS (E⁺) m/z: 0.76 [M+H]; ¹H NMR (400 MHz, METHANOL-d₄) δ ppm 8.64 (s, 1H), 8.36 (s, 1H), 7.58-7.47 (m, 1H), 7.40-7.18 (m, 3H), 4.36 (s, 2H), 3.50-3.43 (m, 2H), 3.13 (t, J=6.3 Hz, 2H), 3.04-2.94 (m, 1H), 2.59 (s, 3H), 1.07 (d, J=7.0 Hz, 6H).

The following Examples were prepared according to the general procedure used to prepare Example 213.

**TABLE 19**

| Ex. No. | Structure | Mol Wt. | LCMS MH⁺ | RT (min) | HPLC Method |
|---|---|---|---|---|---|
| 214 | 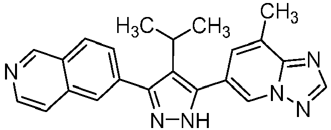 | 368.4 | 369.3 | 1.38 | C |
| 215 | 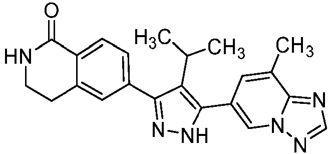 | 386.4 | 387.2 | 1.17 | E |
| 216 | 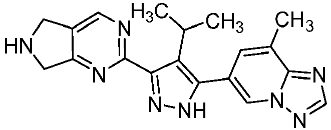 | 360.4 | 361.2 | 0.99 | C |
| 217 | 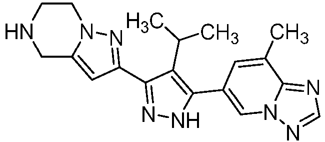 | 362.4 | 363.2 | 1.01 | E |
| 218 | 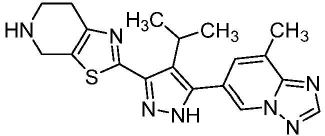 | 379.4 | 380.2 | 1.607 | B |
| 219 | 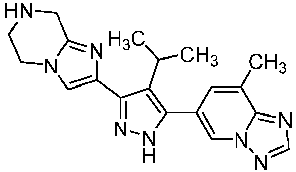 | 362.4 | 363.2 | 0.94 | E |
| 220 | 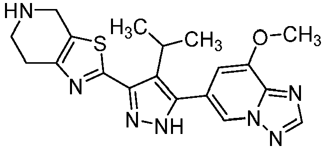 | 395.4 | 396.2 | 1.207 | B |
| 221 | 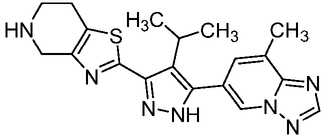 | 379.49 | 380.3 | 1.12 | E |
| 222 | 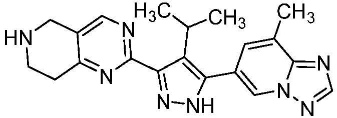 | 374.4 | 375.2 | 1 | C |
| 223 | 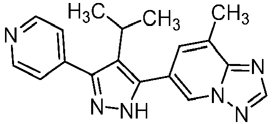 | 318.3 | 319.2 | 1.15 | C |
| 224 | 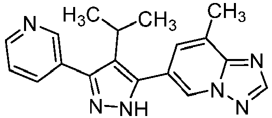 | 318.3 | 319.1 | 1.177 | E |
| 225 | 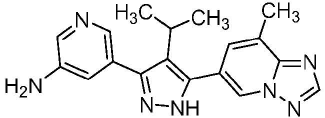 | 333.3 | 334.2 | 0.997 | E |
| 226 | 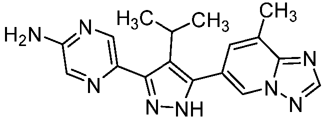 | 334.3 | 335.3 | 1.04 | C |
| 227 | 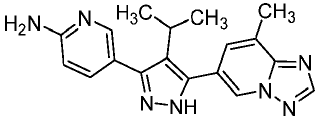 | 333.3 | 334.3 | 1.07 | C |
| 228 | 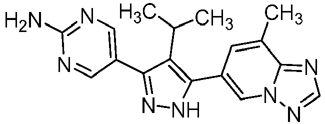 | 334.3 | 335.3 | 0.98 | C |
| 229 | 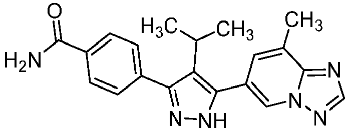 | 360.4 | 361.2 | 1.07 | C |
| 230 | 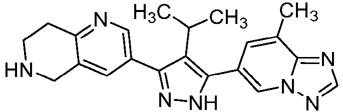 | 373.4 | 374.2 | 0.88 | C |
| 231 | 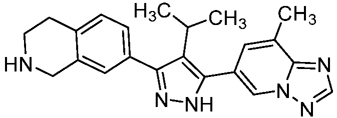 | 372.4 | 373.2 | 1.022 | E |
| 232 | 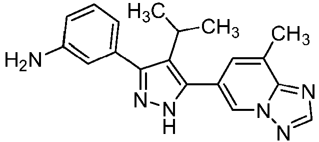 | 332.4 | 333.3 | 1.31 | C |
| 233 | 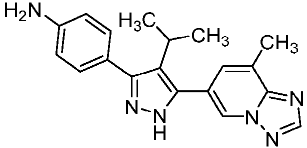 | 332.4 | 333.2 | 1.25 | C |
| 234 | 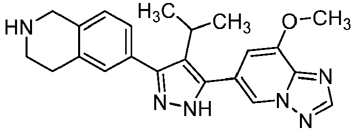 | 388.4 | 389.3 | 0.92 | C |

### EXAMPLE 235

### 6-(4-isopropyl-5-(8-methyl-[1,2,4]triazolo[1,5-a]pyridin-6-yl)-1H-pyrazol-3-yl)-2-methyl-1 ,2,3,4-tetrahydroisoquinoline

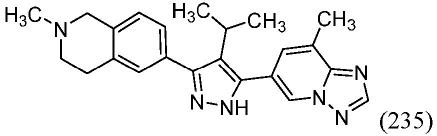

To a solution of 6-(4-isopropyl-5-(8-methyl-[1,2,4]triazolo[1,5-a]pyridin-6-yl)-1H-pyrazol-3-yl)-1,2,3,4-tetrahydroisoquinoline, HCl (0.017 g, 0.042 mmol) in MeOH (2.00 mL) were added formaldehyde (0.2 mL, 2.54 mmol) and acetic acid (0.2 mL, 3.49 mmol) at room temperature, stirred for 6 h, to this was then added sodium cyanoborohydride (7.84 mg, 0.125 mmol) and stirred at the same temperature for 16 h. The reaction mass was purified by preparative LCMS using method AA, fractions containing the product were combined and dried using Genevac centrifugal evaporator to get 6-(4-isopropyl-5-(8-methyl-[1,2,4]triazolo[1,5-a]pyridin-6-yl)-1H-pyrazol-3-yl)-2-methyl-1,2,3,4-tetrahydroisoquinoline (0.0016 g, 4.02 µmol, 10% yield) as a pale solid. LCMS Retention time 0.76 min [E], MS (E⁺) m/z: 387.2 [M+H]; ¹H NMR (400 MHz, METHANOL-d₄) δ ppm 8.75 (br. s., 1H), 8.47 (s, 1H), 7.67 (br. s., 1H), 7.40-7.28 (m, 2H), 7.27-7.18 (m, 1H), 3.90-3.68 (m, 3H), 3.18-3.02 (m, 4H), 2.91 (br. s., 2H), 2.71 (s, 3H), 2.57 (s, 3H), 1.19 (d, *J*=7.0 Hz, 6H).

The following Examples were prepared according to the general procedure used to prepare Example 235.

**TABLE 20**

| Ex. No. | Structure | LCMS MH⁺ | RT (min) | HPLC Method |
|---|---|---|---|---|
| 236 | 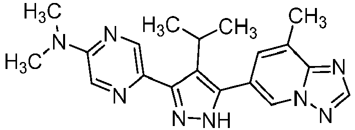 | 363.2 | 1.45 | C |
| 237 | 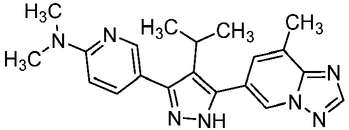 | 362.3 | 1.49 | C |
| 238 | 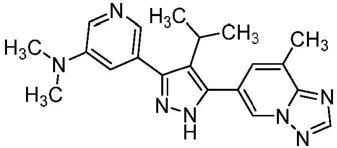 | 362.2 | 1.333 | E |
| 239 | 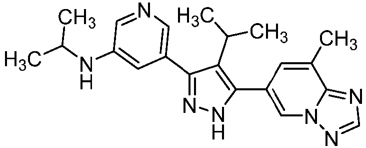 | 376.2 | 1.507 | E |
| 240 | 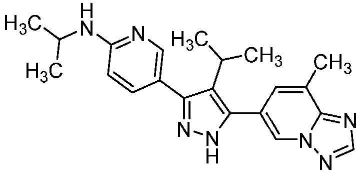 | 376.3 | 1.53 | C |
| 241 | 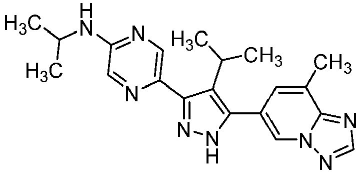 | 377.3 | 1.51 | C |
| 242 | 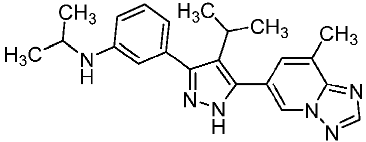 | 375.2 | 1.79 | C |
| 243 | 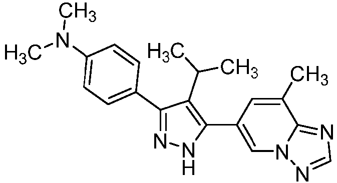 | 361.2 | 1.74 | C |
| 244 | 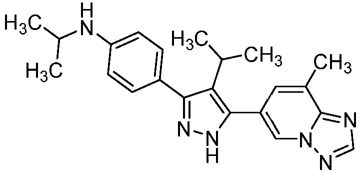 | 375.3 | 1.79 | C |
| 245 | 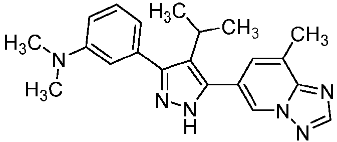 | 361.2 | 1.75 | C |
| 246 | 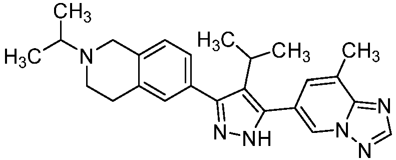 | 415.3 | 1.379 | E |
| 247 | 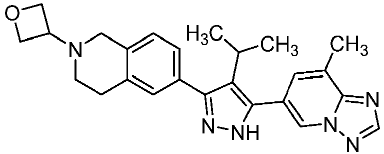 | 429.3 | 1.427 | E |
| 248 | 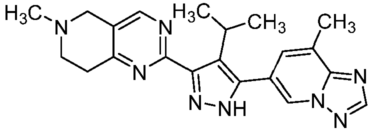 | 389.3 | 1.33 | C |
| 249 | 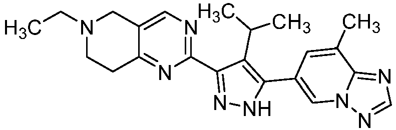 | 403.3 | 1.45 | C |
| 250 | 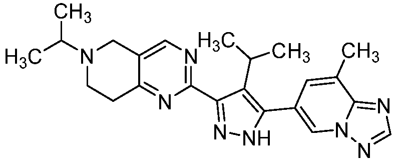 | 417.3 | 1.61 | C |
| 251 | 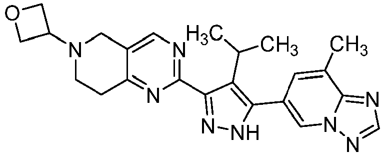 | 431.3 | 1.26 | C |
| 252 | 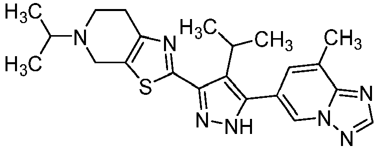 | 422.3 | 1.59 | E |
| 253 | 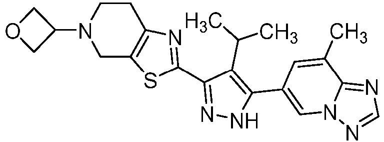 | 436.2 | 1.32 | E |
| 254 | 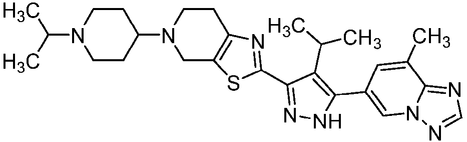 | 505.3 | 1.15 | E |
| 255 | 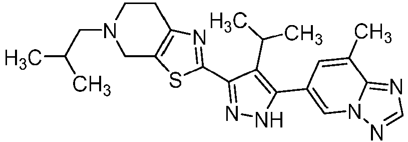 | 436.3 | 1.38 | E |
| 256 | 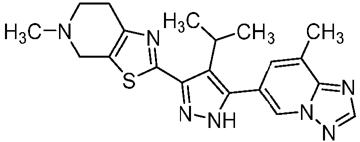 | 394.2 | 1.36 | E |
| 257 | 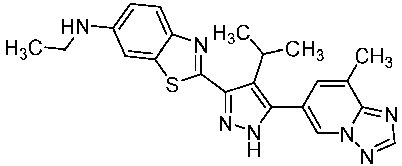 | 418.2 | 1.99 | E |
| 258 | 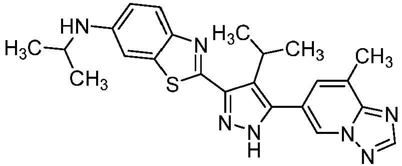 | 432.2 | 1.99 | E |
| 259 | 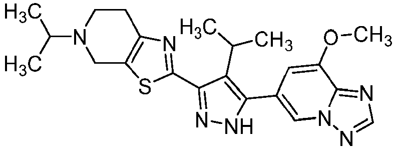 | 438.2 | 1.62 | E |
| 260 | 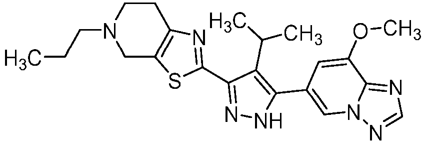 | 438.3 | 1.06 | F |
| 261 | 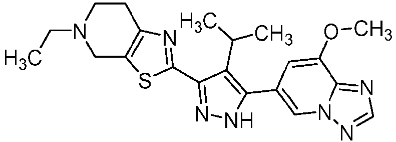 | 424.3 | 1.491 | E |
| 262 | 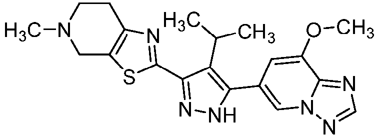 | 410.2 | 1.376 | E |

### EXAMPLE 263

### 6-(4-isopropyl-5-(8-methyl-[1,2,4]triazolo[1,5-a]pyridin-6-yl)-1H-pyrazol-3-yl)-2-(2-methoxyethyl)-1,2,3,4-tetrahydroisoquinoline

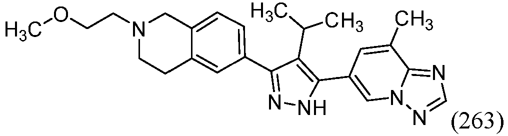

To a solution of 6-(4-isopropyl-5-(8-methyl-[1,2,4]triazolo[1,5-a]pyridin-6-yl)-1H-pyrazol-3-yl)-1,2,3,4-tetrahydroisoquinoline, HCl (0.022 g, 0.054 mmol) and 1-bromo-2-methoxyethane (0.015 g, 0.108 mmol) in THF (1.00 mL) and DMF (0.500 mL) solvent mixture was added DIPEA (0.3 mL, 1.718 mmol) at room temperature, then the mixture was stirred at 90 °C for 16 h. The reaction mass was concentrated and purified by Preparative LCMS using method AA, fractions containing the product were combined and dried using Genevac centrifugal evaporator to afford 6-(4-isopropyl-5-(8-methyl-[1,2,4]triazolo[1,5-a] pyridin-6-yl)-1H-pyrazol-3-yl)-2-(2-methoxyethyl)-1,2,3,4-tetrahydroisoquinoline (0.0094 g, 0.021 mmol, 39% yield) as a pale solid. LCMS Retention time 1.473 min [E], MS (E⁺) m/z: 429.3 [M+H]; ¹H NMR (400 MHz, METHANOL-d₄) δ ppm 8.75 (s, 1H), 8.47 (s, 1H), 7.67 (s, 1H), 7.38-7.27 (m, 2H), 7.26-7.16 (m, 1H), 3.87 (s, 2H), 3.69 (t, *J*=5.5 Hz, 2H), 3.43-3.39 (m, 3H), 3.17-3.02 (m, 4H), 3.00-2.93 (m, 2H), 2.88 (t, *J*=5.5 Hz, 2H), 2.76-2.67 (m, 3H), 1.96 (s, 5H), 1.19 (d, *J*=7.0 Hz, 6H).

The following Examples were prepared according to the general procedure used to prepare Example 263.

**TABLE 21**

| Ex. No. | Structure | LCMS MH⁺ | RT (min) | HPLC Method |
|---|---|---|---|---|
| 264 | 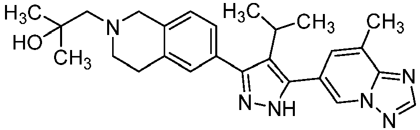 | 445.3 | 1.579 | E |
| 265 | 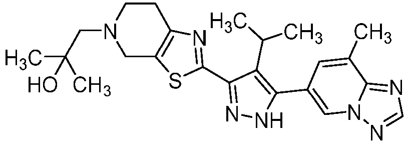 | 452.3 | 1.56 | E |
| 266 | 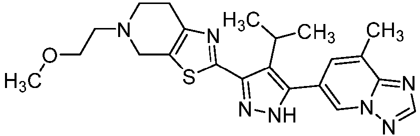 | 438.3 | 1.46 | E |
| 267 | 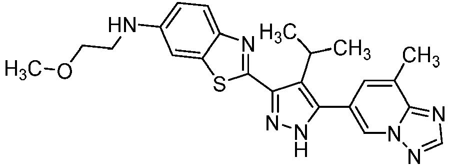 | 448.2 | 1.73 | E |

### EXAMPLE 268

### 2-(dimethylamino)-1-(6-(4-isopropyl-5-(8-methyl-[1,2,4]triazolo[1,5-a]pyridin-6-yl)-1H-pyrazol-3-yl)-3,4-dihydroisoquinolin-2(1H)-yl)ethan-1-one

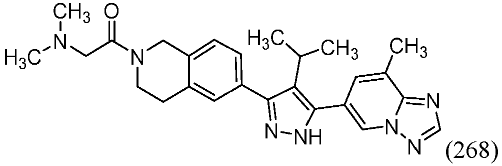

To a mixture of 6-(4-isopropyl-5-(8-methyl-[1,2,4]triazolo[1,5-a]pyridin-6-yl)-1H-pyrazol-3-yl)-1,2,3,4-tetrahydroisoquinoline, HCl (0.022 g, 0.054 mmol) and dimethylglycine (0.011 g, 0.108 mmol) in DMF (1.00 mL) were added TEA (0.2 mL, 1.435 mmol) and HATU (0.041 g, 0.108 mmol) at room temperature. The reaction mixture was stirred at the same temperature for 16 h. The reaction mass was purified by Preparative LCMS using method AA. The fractions containing the product were combined and dried using a Genevac centrifugal evaporator to yield 2-(dimethylamino)-1-(6-(4-isopropyl-5-(8-methyl-[1,2,4]triazolo[1,5-a]pyridin-6-yl)-1H-pyrazol-3-yl)-3,4-dihydroisoquinolin-2(1H)-yl)ethan-1-one (0.0034 g, 7.28 µmol, 13% yield) as a pale solid. LCMS Retention time 1.145 min [E], MS (E⁺) m/z: 458.3 [M+H]; ¹H NMR (400 MHz, METHANOL-d₄) δ ppm 8.76 (s, 1H), 8.48 (s, 1H), 7.67 (s, 1H), 7.49-7.26 (m, 3H), 4.82 (d, *J*=10.5 Hz, 2H), 3.92-3.77 (m, 2H), 3.59 (d, *J*=6.8 Hz, 2H), 3.16-2.93 (m, 3H), 2.71 (s, 3H), 2.50 (s, 6H), 1.20 (d, *J*=7.1 Hz, 6H).

The following Examples were prepared according to the general procedure used to prepare Example 268.

**TABLE 22**

| Ex. No. | Structure | LCMS MH⁺ | RT (min) | HPLC Method |
|---|---|---|---|---|
| 269 | 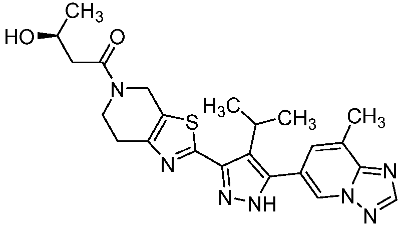 | 466.2 | 1.23 | E |
| 270 | 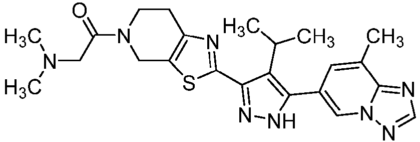 | 465.2 | 1.14 | E |

### EXAMPLE 271

### 2-(6-(4-isopropyl-5-(8-methyl-[1,2,4]triazolo[1,5-a]pyridin-6-yl)-1H-pyrazol-3-yl)-3,4-dihydroisoquinolin-2(1H)-yl)-N,N-dimethylacetamide

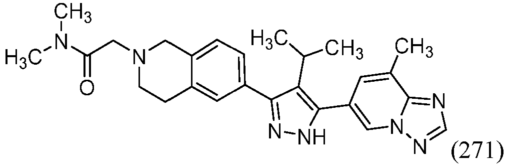

To a solution of 6-(4-isopropyl-5-(8-methyl-[1,2,4]triazolo[1,5-a]pyridin-6-yl)-1H-pyrazol-3-yl)-1,2,3,4-tetrahydroisoquinoline, HCl (0.030 g, 0.073 mmol) and 2-chloro-N,N-dimethylacetamide (0.018 g, 0.147 mmol) in THF (1.00 mL) and DMF (0.50 mL) solvent mixture was added TEA (0.2 mL, 1.435 mmol), then the mixture was stirred at the same temperature for 16 h. The reaction mixture was purified by Preparative LCMS using method AA, fractions containing product were combined and dried using Genevac centrifugal evaporator to get 2-(6-(4-isopropyl-5-(8-methyl-[1,2,4]triazolo[1,5-a]pyridin-6-yl)-1H-pyrazol-3-yl)-3,4-dihydroisoquinolin-2(1H)-yl)-N,N-dimethylacetamide (0.0022 g, 4.71 µmol, 6% yield) as a pale solid. LCMS Retention time 1.380 min [E], MS (E⁺) m/z: 458.3 [M+H]; ¹H NMR (400 MHz, METHANOL-d₄) δ ppm 8.73 (br. s., 1H), 8.46 (s, 1H), 7.66 (br. s., 1H), 7.35-7.23 (m, 2H), 7.19 (d, *J*=7.3 Hz, 1H), 3.85 (s, 2H), 3.52 (s, 2H), 3.19-3.05 (m, 4H), 3.05-2.83 (m, 7H), 2.69 (s, 3H), 1.18 (d, *J*=7.1 Hz, 6H).

The following Examples were prepared according to the general procedure used to prepare Example 271.

**TABLE 23**

| Ex. No. | Structure | LCMS MH⁺ | RT (min) | HPLC Method |
|---|---|---|---|---|
| 272 | 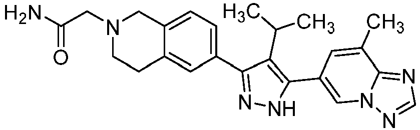 | 430.3 | 1.249 | E |
| 273 | 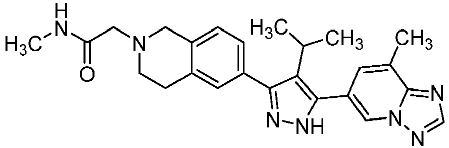 | 444.2 | 1.336 | E |
| 274 | 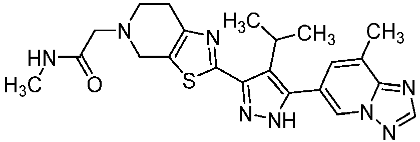 | 451.2 | 1.28 | E |
| 275 | 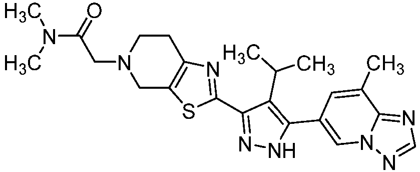 | 465.2 | 1.32 | E |
| 276 | 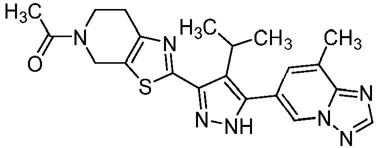 | 422.3 | 1.29 | E |

### EXAMPLE 277

### 6-(4-isopropyl-5-(8-methyl-[1,2,4]triazolo[1,5-a]pyridin-6-yl)-1H-pyrazol-3-yl)pyridin-2-ol

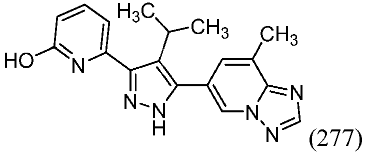

### Intermediate 277A: 6-(4-isopropyl-3-(6-methoxypyridin-2-yl)-1H-pyrazol-5-yl)-8-methyl-[1,2,4]triazolo[1,5-a]pyridine

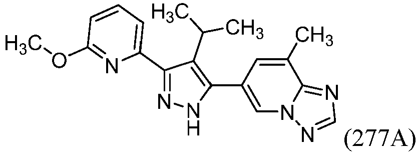

6-(4-isopropyl-3-(6-methoxypyridin-2-yl)-1H-pyrazol-5-yl)-8-methyl-[1,2,4]triazolo[1,5-a] pyridine was prepared according to the general process described in intermediate-213N using 2-bromo-6-methoxypyridine (0.041 g, 0.218 mmol) and 6-(4-isopropyl-3-(4,4,5,5-tetramethyl-1,3,2-dioxaborolan-2-yl)-1H-pyrazol--yl)-8-methyl-[1,2,4]triazolo[1,5-a]pyridine (0.080 g, 0.218 mmol) as a starting intermediate to yield the title compound as an off-white solid. LCMS Retention time 1.62 min [C] MS (E⁺) m/z: 349.1(M+H); ¹H NMR (400 MHz, METHANOL-d₄) δ 8.76 (br. s., 1H), 8.48 (s, 1H), 7.78 (br. s., 1H), 7.66 (br.s., 1H), 7.31 (br. s., 1H), 6.81 (d, *J*=7.6 Hz, 1H), 4.02 (s, 3H), 3.62-3.45 (m, 1H), 2.71 (s, 3H),1.33 (d, *J*=7.3 Hz, 6H).

### Example 277: 6-(4-isopropyl-5-(8-methyl-[1,2,4]triazolo[1,5-a]pyridin-6-yl)-1H-pyrazol-3-yl)pyridin-2-ol

BBr₃ (0.163 µL, 1.722 µmol, 1M in DCM) was added to a solution of 6-(4-isopropyl-3-(6-methoxypyridin-2-yl)-1H-pyrazol-5-yl)-8-methyl-[1,2,4]triazolo[1,5-a]pyridine (0.060 mg, 0.172 µmol) in DCM (3.0 mL), the resulting reaction mixture was stirred at room temperature for 16 h. The reaction mixture was quenched slowly with methanol and concentrated the mixture to get crude. The crude compound was purified via preparative LC/MS using method AB, the fractions containing the product were combined and dried via centrifugal evaporation to get 6-(4-isopropyl-5-(8-methyl-[1,2,4]triazolo[1,5-a]pyridin-6-yl)-1H-pyrazol-3-yl)pyridin-2-ol (12 mg) as an off-white solid. LCMS Retention time: 1.01 min [C], MS (E⁺) m/z: 335.1 [M+H]; ¹H NMR (400 MHz, METHANOL-d₄) δ 8.80 (br. s., 1H), 8.57-8.43 (m, 1H), 7.71 (dd, *J*=9.2, 7.0 Hz, 1H), 7.61 (br. s., 1H), 6.63 (br. s., 2H), 3.24-3.11 (m, 1H), 2.77-2.65 (m, 3H), 1.24 (d, *J*=7.1 Hz, 6H).

### EXAMPLE 278

### 6-(5-(2,6-dimethylpyridin-4-yl)-4-isopropyl-1H-pyrazol-3-yl)-1,2,3,4-tetrahydro isoquinoline

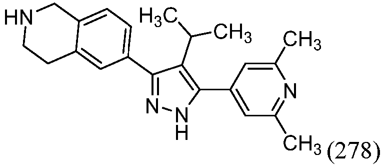

### Intermediate 278A: tert-butyl 6-bromo-3,4-dihydroisoquinoline-2(1H)-carboxylate

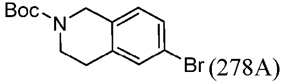

Boc-anhydride (4.34 mL, 18.69 mmol) was added to a solution of 6-bromo-1, 2, 3, 4-tetrahydro isoquinoline (3.050 g, 14.38 mmol) and TEA (6.01 mL, 43.1 mmol) in DCM (80.0 mL) at room temperature, the mixture was stirred for 14 h. The reaction mixture was diluted with DCM, washed with water, brine, dried over sodium sulphate and concentrated to get crude. The crude compound was purified by ISCO using 40 g silica column, compound was eluted with 15%-20% ethyl acetate in pet ether, the fractions were collected and concentrated to get tert-butyl 6-bromo-3,4-dihydroisoquinoline-2(1H)-carboxylate (3.5 g, 11.21 mmol, 78% yield) as a light brown solid. LCMS Retention time: 1.60 min [A], MS (E⁺) m/z: 258.3 [M+H-tBu].

### Intermediate 278B: tert-butyl 6-(4,4,5,5-tetramethyl-1,3,2-dioxaborolan-2-yl)-3,4-dihydroisoquinoline-2(1H)-carboxylate

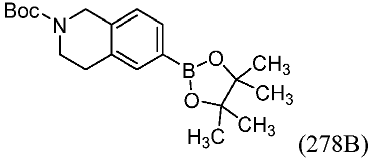

PdCl₂(dppf)-CH₂Cl₂ adduct (0.915 g, 1.121 mmol) and potassium acetate (3.30 g, 33.6 mmol) were added to a degassed solution of tert-butyl 6-bromo-3,4-dihydroisoquinoline-2(1H)-carboxylate (3.5 g, 11.21 mmol) and BISPIN (3.70 g, 14.57 mmol) in dioxane (120 mL), the resulting mixture was stirred at 95 °C for 14 h in a sealed tube. The reaction mixture was diluted with ethyl acetate, filtered and washed with excess ethyl acetate. The filtrates were dried over sodium sulphate and concentrated to get crude compound. The crude compound was purified by ISCO using 40 g silica column, compound was eluted with 45% ethyl acetate in pet ether, the fractions were collected and concentrated to get tert-butyl 6-(4,4,5,5-tetramethyl-1,3,2-dioxaborolan-2-yl)-3,4-dihydroisoquinoline-2(1H)-carboxylate (3.5 g, 9.74 mmol, 87% yield) as a light brown solid. LCMS Retention time: 1.74 min [A], MS (E⁺) m/z: 304.5 [M+H-tBu].

### Intermediate 278C: tert-butyl 6-(5-(2,6-dimethylpyridin-4-yl)-4-isopropyl-1H-pyrazol-3-yl)-3,4-dihydroisoquinoline-2(1H)-carboxylate

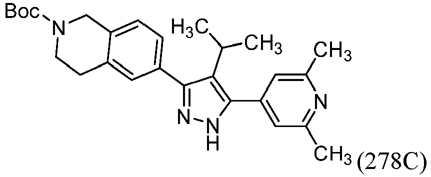

tert-butyl 6-(5-(2,6-dimethylpyridin-4-yl)-4-isopropyl-1H-pyrazol-3-yl)-3,4-dihydro isoquinoline-2(1H)-carboxylate (85 mg, 0.190 mmol, 56% yield) was prepared according to the general process described in Intermediate 213N, using tert-butyl 6-(4,4,5,5-tetramethyl-1,3,2-dioxaborolan-2-yl)-3,4-dihydroisoquinoline-2(1H)-carboxylate (0.122 g, 0.340 mmol) and 4-(3-bromo-4-isopropyl-1H-pyrazol-5-yl)-2,6-dimethylpyridine (0.100 g, 0.340 mmol) as a starting intermediate to yield the title compound as a gummy solid. LCMS Retention time: 1.49 min [A], MS (E⁺) m/z: 447.3 [M+H].

### Example 278: 6-(5-(2,6-dimethylpyridin-4-yl)-4-isopropyl-1H-pyrazol-3-yl)-1,2,3,4-tetrahydroisoquinoline

6-(5-(2,6-dimethylpyridin-4-yl)-4-isopropyl-1H-pyrazol-3-yl)-1,2,3,4-tetrahydroisoquinoline (17 mg) was prepared according to the general process described in Example 213, using tert-butyl 6-(5-(2,6-dimethylpyridin-4-yl)-4-isopropyl-1H-pyrazol-3-yl)-3,4-dihydroisoquinoline-2(1H)-carboxylate (55 mg, 0.123 mmol) as a starting intermediate to yield the title compound as an off-white solid. Retention time: 0.78 min [E] MS (E⁺) m/z: 347.6 [M+H]; ¹H NMR (400 MHz, METHANOL-d₄) δ 7.87 (s, 2H), 7.50-7.30 (m, 3H), 4.48 (s, 2H), 3.59 (t, *J*=6.3 Hz, 2H), 3.29-3.15 (m, 3H), 2.81 (s, 6H), 1.24 (d, *J*=7.0 Hz, 6H).

### EXAMPLE 279

### N-(2,2-difluoroethyl)-4-(4-isopropyl-5-(8-methyl-[1,2,4]triazolo[1,5-a]pyridin-6-yl)-1H-pyrazol-3-yl)cyclohexan-1-amine

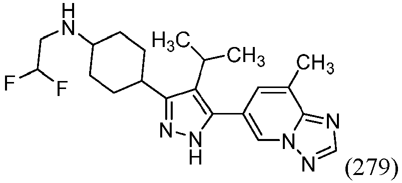

### Intermediate 279A: 6-(4-isopropyl-3-(1,4-dioxaspiro[4.5]dec-7-en-8-yl)-1-((2-(trimethylsilyl)ethoxy)methyl)-1H-pyrazol-5-yl)-8-methyl-[1,2,4]triazolo[1,5-a]pyridine

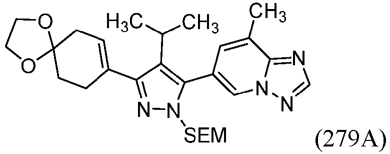

6-(4-isopropyl-3-(1,4-dioxaspiro[4.5]dec-7-en-8-yl)-1-((2-(trimethylsilyl)ethoxy) methyl)-1H-pyrazol-5-yl)-8-methyl-[1,2,4]triazolo[1,5-a]pyridine (1.1 g, 2.158 mmol, 72% yield) was prepared according to the general process described in Intermediate 213N using 4,4,5,5-tetramethyl-2-(1,4-dioxaspiro [4.5]dec-7-en-8-yl)-1,3,2-dioxaborolane (800 mg, 3.01 mmol) and 6-(3-bromo-4-isopropyl-1-((2-(trimethylsilyl)ethoxy)methyl)-1H-pyrazol-5-yl)-8-methyl-[1,2,4]triazolo[1,5-a]pyridine (1760 mg, 3.91 mmol) as starting intermediates to yield the title compound as a pale yellow solid. LCMS Retention time: 3.49 min [B], MS (E⁺) m/z: 510.4 [M+H].

### Intermediate 279B: 6-(4-isopropyl-3-(1,4-dioxaspiro[4.5]decan-8-yl)-1-((2-(trimethylsilyl) ethoxy)methyl)-1H-pyrazol-5-yl)-8-methyl-[1,2,4]triazolo[1,5-a]pyridine

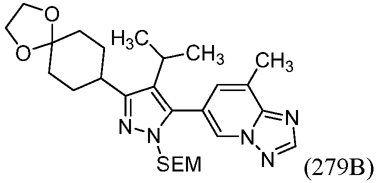

6-(4-isopropyl-3-(1,4-dioxaspiro[4. 5]decan-8-yl)-1-((2-(trimethylsilyl)ethoxy) methyl)-1H-pyrazol-5-yl)-8-methyl-[1,2,4]triazolo[1,5-a]pyridine (700 mg, 1.368 mmol, 46% yield) was prepared according to the general process described in Intermediate 206C, using 6-(4-isopropyl-3-(1,4-dioxaspiro[4.5]dec-7-en-8-yl)-1-((2-(trimethylsilyl) ethoxy)methyl)-1H-pyrazol-5-yl)-8-methyl-[1,2,4]triazolo [1,5-a]pyridine (1.5 g, 2.94 mmol) as a starting intermediate to yield the title compound as an off-white solid. LCMS Retention time: 3.79 min [B], MS (E⁺) m/z: 512.7 [M+H].

### Intermediate 279C: 4-(4-isopropyl-5-(8-methyl-[1,2,4]triazolo[1,5-a]pyridin-6-yl)-1H-pyrazol-3-yl)cyclohexan-1-one

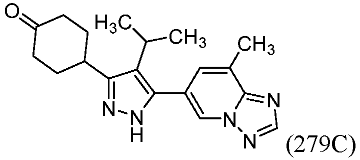

To a solution of 6-(4-isopropyl-3-(1,4-dioxaspiro[4.5]dec-7-en-8-yl)-1-((2-(trimethylsilyl)ethoxy)methyl)-1H-pyrazol-5-yl)-8-methyl-[1,2,4]triazolo[1,5-a]pyridine (500 mg, 0.981 mmol) in DCM (6.0 mL) was added TFA (1.889 mL, 24.52 mmol) at 0 °C, the mixture was stirred at room temperature for 12 h. The reaction mixture was concentrated, dried under vacuum to get 4-(4-isopropyl-5-(8-methyl-[1,2,4]triazolo[1,5-a]pyridin-6-yl)-1H-pyrazol-3-yl)cyclohexan-1-one (320 mg, 0.948 mmol, 97 % yield) as a gummy solid. LCMS Retention time: 0.92 min [A], MS (E⁺) m/z: 338.6 [M+H].

### Example 279: N-(2,2-difluoroethyl)-4-(4-isopropyl-5-(8-methyl-[1,2,4]triazolo[1,5-a] pyridin-6-yl)-1H-pyrazol-3-yl)cyclohexan-1-amine

Mixture of 4-(4-isopropyl-5-(8-methyl-[1,2,4]triazolo[1,5-a]pyridin-6-yl)-1H-pyrazol-3-yl)cyclohexan-1-one (100 mg, 0.296 mmol), 2,2-difluoroethan-1-amine (120 mg, 1.482 mmol) and acetic acid (3.39 µL, 0.059 mmol) in DMF (3.0 mL) was stirred at room temperature for 8 h, to this was then added sodium cyanoborohydride (37.2 mg, 0.593 mmol) at 0°C, stirred at room temperature for 16 h. The reaction mass was purified via preparative LC/MS using method AA, to separate the two isomers.

Isomer 1: The fractions containing the product were combined and dried via centrifugal evaporation to afford Isomer 1 as a pale solid. LCMS Retention time: 1.49 min [C], MS (E⁺) m/z: 403.2 [M+H]; ¹H NMR (400 MHz, METHANOL-d₄) δ 8.64 (br. s., 1H), 8.46 (s, 1H), 7.57 (br. s., 1H), 3.13 -2.99 (m, 3H), 2.93 (br. s., 1H), 2.69 (s, 4H), 2.15 (d, *J*=11.0 Hz, 2H), 2.00 (d, *J*=12.2 Hz, 2H),1.79-1.65 (m, 2H), 1.39-1.22 (m, 9H).

Isomer 2: The fractions containing the product were combined and dried via centrifugal evaporation to afford Isomer 1 as a pale solid. LCMS Retention time: 1.76 min [C], MS (E⁺) m/z: 403.3 [M+H]; ¹H NMR (400 MHz, METHANOL-d₄) δ 8.65 (s, 1H), 8.46 (s, 1H), 7.57 (s, 1H), 3.12-2.85 (m, 4H), 2.69 (s, 3H), 2.14-1.85 (m, 4H), 1.72 (br. s., 3H), 1.29 (d, *J*=7.1 Hz, 6H).

The following Examples were prepared according to the general procedure used to prepare Example 279.

**TABLE 24**

| Ex. No. | Structure | LCMS MH⁺ | RT (min) | HPLC Method |
|---|---|---|---|---|
| 280 | 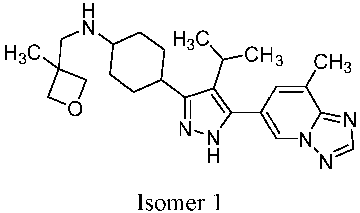 | 423.3 | 1.1 | E |
| 281 | 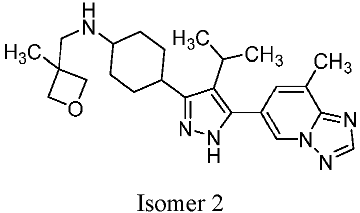 | 423.3 | 1.48 | E |
| 282 | 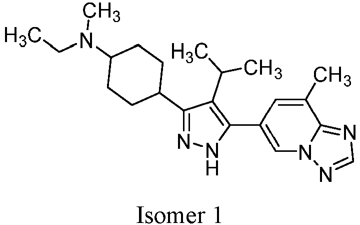 | 381.3 | 1.16 | C |
| 283 | 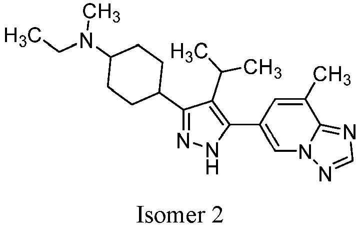 | 381.3 | 1.16 | C |
| 284 | 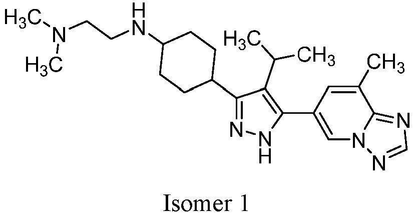 | 410.3 | 1.13 | C |
| 285 | 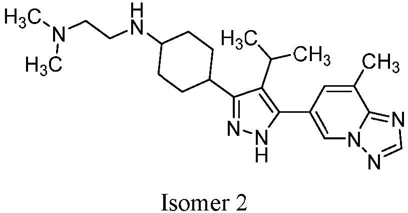 | 410.3 | 1.37 | C |
| 286 | 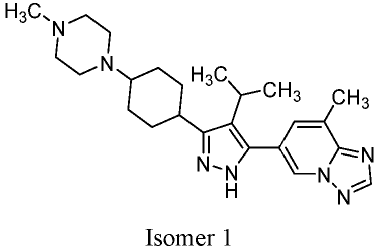 | 422.3 | 1.12 | C |
| 287 | 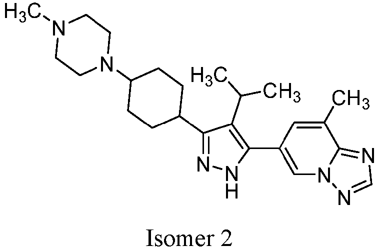 | 422.3 | 1.37 | C |
| 288 | 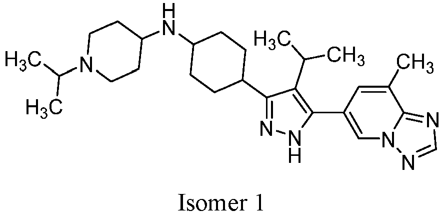 | 464.3 | 1.16 | C |
| 289 | 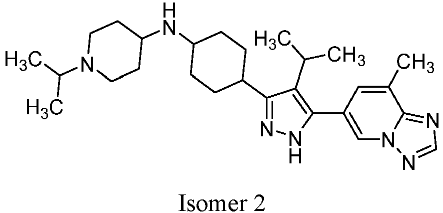 | 464.3 | 1.39 | C |
| 290 | 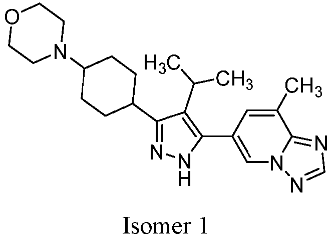 | 409.3 | 0.97 | D |
| 291 | 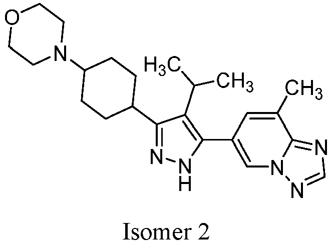 | 409.3 | 1.09 | C |
| 292 | 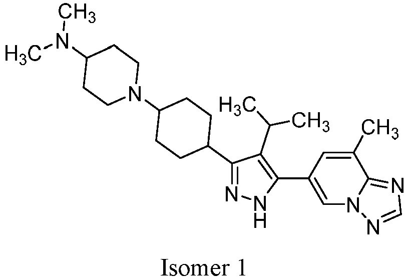 | 450.3 | 1.14 | C |
| 293 | 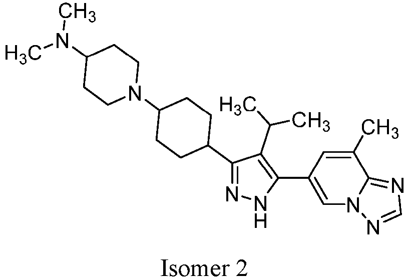 | 450.3 | 1.41 | C |
| 294 | 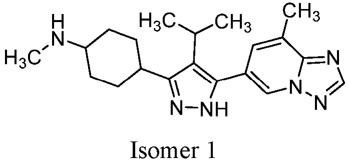 | 353.2 | 0.65 | A |
| 295 | 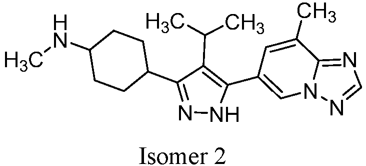 | 353.3 | 0.75 | A |

### EXAMPLE 296

### 2-(dimethylamino)-N-(4-(4-isopropyl-5-(8-methyl-[1,2,4]triazolo[1,5-a] pyridin-6-yl)-1H-pyrazol-3-yl)cyclohexyl)-N-methylacetamide

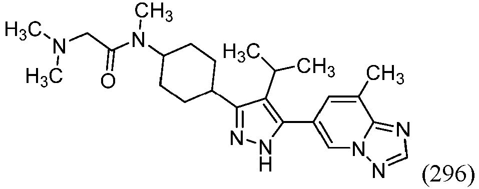

To a solution of 4-(4-isopropyl-5-(8-methyl-[1,2,4]triazolo[1,5-a]pyridin-6-yl)-1H-pyrazol-3-yl)-N-methylcyclohexan-1-amine (20.0 mg, 0.057 mmol) in DMF (2.0 mL) were added HATU (21.57 mg, 0.057 mmol), TEA (0.040 mL, 0.284 mmol) and dimethylglycine (8.78 mg, 0.085 mmol) at room temperature, stirred for 12 h. The reaction mass was purified via preparative LC/MS using method AB, the fractions containing the product were combined and dried via centrifugal evaporation to yield the title compound (12.0 mg) as an off-white solid. LCMS Retention time: 1.23 min [E], MS (E⁺) m/z: 438.3 [M+H]; ¹H NMR (400 MHz, METHANOL-d₄) δ 8.55 (br. s., 1H), 8.45 (s, 1H), 7.85 (s, 1H), 7.21 (br. s.,1H), 4.13 (s, 3H), 3.25-3.19 (m, 1H), 3.08 (d, *J*=11.5 Hz, 2H), 2.41 (s, 3H), 2.34 (t, *J*=11.7 Hz,2H), 2.24 (d, *J*=12.2 Hz, 2H), 2.03-1.89 (m, 2H), 1.28 (d, *J*=7.1 Hz, 6H).

The following Example was prepared according to the general procedure used to prepare Example 296.

**TABLE 25**

| Ex. No. | Structure | LCMS MH⁺ | RT (min) | HPLC Method |
|---|---|---|---|---|
| 297 | 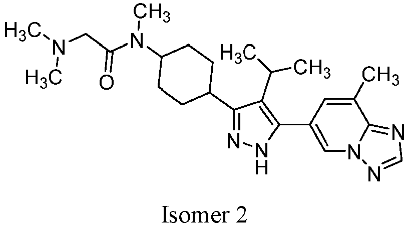 | 438.3 | 1.25 | C |

### EXAMPLE 298

### 2-((4-(4-isopropyl-5-(8-methyl-[1,2,4]triazolo[1,5-a]pyridin-6-yl)-1H-pyrazol-3-yl) cyclohexyl)(methyl)amino)-N,N-dimethylacetamide

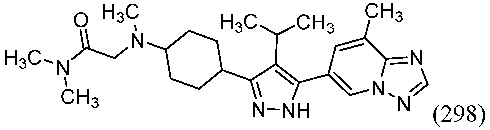

TEA (0.040 mL, 0.284 mmol) was added to a mixture of 4-(4-isopropyl-5-(8-methyl-[1,2,4] triazolo[1,5-a]pyridin-6-yl)-1H-pyrazol-3-yl)-N-methylcyclohexan-1-amine (20.0 mg, 0.057 mmol) and 2-chloro-N,N-dimethylacetamide (10.35 mg, 0.085 mmol) in DMF (2.0 mL), then stirred at room temperature for 12 h. The reaction mass was purified via preparative LC/MS using method AA, fraction collection was triggered by MS signals. Fractions containing the product were combined and dried via centrifugal evaporation to get 2-((4-(4-isopropyl-5-(8-methyl-[1,2,4]triazolo[1,5-a]pyridin-6-yl)-1H-pyrazol-3-yl)cyclohexyl)(methyl)amino)-N,N-dimethylacetamide (9.1 mg) as a pale solid. LCMS Retention time: 1.15 min [E], MS (E⁺) m/z: 438.3 [M+H]; ¹H NMR (400 MHz, METHANOL-d₄) δ 8.65 (s, 1H), 8.46 (s, 1H), 7.57 (s, 1H), 3.61 (br. s.,2H), 3.13 (s, 3H), 3.11-2.97 (m, 5H), 2.97-2.77 (m, 3H), 2.69 (s, 3H), 2.51 (br. s., 3H), 2.19 -1.95 (m, 4H), 1.81-1.67 (m, 2H), 1.65-1.46 (m, 2H), 1.29 (d, *J*=7.3 Hz, 6H).

The following Examples were prepared according to the general procedure used to prepare Example 298.

**TABLE 26**

| Ex. No. | Structure | LCMS MH⁺ | RT (min) | HPLC Method |
|---|---|---|---|---|
| 299 | 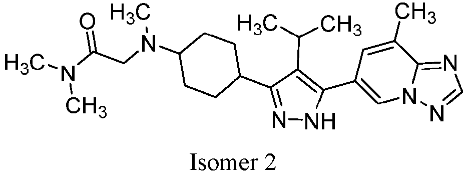 | 438.3 | 1.15 | D |

### EXAMPLE 300

### N-(4-(4-isopropyl-5-(8-methyl-[1,2,4]triazolo[1,5-a]pyridin-6-yl)-1H-pyrazol-3-yl) cyclohexyl)-N,1-dimethylazetidine-3-carboxamide

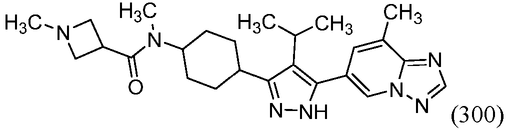

### Intermediate 300A: tert-butyl 3-((4-(4-isopropyl-5-(8-methyl-[1,2,4]triazolo[1,5-a]pyridin-6-yl)-1H-pyrazol-3-yl)cyclohexyl)(methyl)carbamoyl)azetidine-1-carboxylate

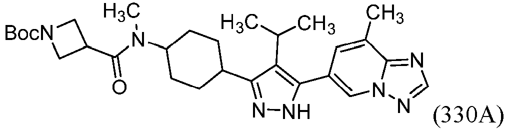

tert-Butyl 3-((4-(4-isopropyl-5-(8-methyl-[1,2,4]triazolo[1,5-a]pyridin-6-yl)-1H-pyrazol-3-yl) cyclohexyl)(methyl)carbamoyl)azetidine-1-carboxylate (150.0 mg, 0.280 mmol, 82 % yield) was prepared according to the general process described in Intermediate 296 using, 4-(4-isopropyl-5-(8-methyl-[1,2,4]triazolo[1,5-a]pyridin-6-yl)-1H-pyrazol-3-yl)-N-methylcyclohexan-1-amine (120 mg, 0.340 mmol) as a starting intermediate to yield the title compound as an off-white solid. LCMS Retention time: 1.20 min [A], MS (E⁺) m/z: 536.6 [M+H].

### Intermediate 300B: N-(4-(4-isopropyl-5-(8-methyl-[1,2,4]triazolo[1,5-a]pyridin-6-yl)-1H-pyrazol-3-yl)cyclohexyl)-N-methylazetidine-3-carboxamide

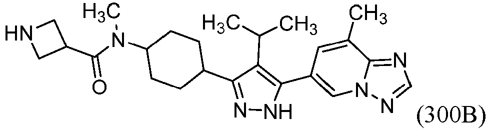

N-(4-(4-isopropyl-5-(8-methyl-[1,2,4]triazolo[1,5-a]pyridin-6-yl)-1H-pyrazol-3-yl) cyclohexyl)-N-methylazetidine-3-carboxamide (31 mg) was prepared according to the general process described in Intermediate 279C, using 3-((4-(4-isopropyl-5-(8-methyl-[1,2,4]triazolo[1,5-a]pyridin-6-yl)-1H-pyrazol-3-yl)cyclohexyl) (methyl)carbamoyl)azetidine-1-carboxylate (150 mg, 0.280 mmol) as a starting intermediate to yield the title compound as an white solid. LCMS Retention time: 1.12min [E], MS (E⁺) m/z: 436.3 [M+H]; ¹H NMR (400 MHz, METHANOL-d₄) δ 8.73-8.60 (m, 1H), 8.52-8.40 (m, 1H), 7.57 (br. s.,1H), 4.52 (d, *J*=5.4 Hz, 1H), 4.37-4.22 (m, 3H), 4.17-4.00 (m, 1H), 3.07 (quin, *J*=7.0 Hz, 2H),2.95 (s, 2H), 2.88 (s, 2H), 2.69 (s, 3H), 2.07 (d, *J*=11.7 Hz, 2H), 1.96-1.79 (m, 4H), 1.76 (br. s.,1H), 1.38-1.20 (m, 7H), 0.91 (d, *J*=9.8 Hz, 1H).

### Example 300: N-(4-(4-isopropyl-5-(8-methyl-[1,2,4]triazolo[1,5-a]pyridin-6-yl)-1H-pyrazol-3-yl)cyclohexyl)-N,1-dimethylazetidine-3-carboxamide

2-(dimethylamino)-N-(4-(4-isopropyl-5-(8-methyl-[1,2,4]triazolo[1,5-a]pyridin-6-yl)-1H-pyrazol-3-yl)cyclohexyl)-N-methylacetamide was prepared according to the general process described923 mg) in example - 307, using N-(4-(4-isopropyl-5-(8-methyl-[1,2,4]triazolo[1,5-a]pyridin-6-yl)-1H-pyrazol-3-yl)cyclohexyl)-N-methylazetidine-3-carboxamide (40.0 mg, 0.092 mmol) as a starting intermediate to yield the title compound as an white solid. LCMS Retention time: 1.13 min [E], MS (E⁺) m/z: 450.3 [M+H]; ¹H NMR (400 MHz, METHANOL-d₄) δ 8.71-8.58 (m, 1H), 8.51-8.37 (m, 1H), 7.57 (s, 1H), 4.51 (br. s., 1H), 4.05-3.89 (m, 2H), 3.82 (br. s., 2H), 3.76 (br. s., 1H), 3.50 (br. s., 1H), 3.14 -3.00 (m, 2H), 3.00-2.76 (m, 4H), 2.72-2.52 (m, 5H), 2.37 (d, *J*=7.8 Hz, 1H), 2.05 (d, *J*=5.6Hz, 2H), 2.00-1.93 (m, 2H), 1.88 (d, *J*=12.2 Hz, 2H), 1.84-1.66 (m, 4H), 1.40-1.14 (m, 7H).

The following Example was prepared according to the general procedure used to prepare Example 300.

**TABLE 27**

| Ex. No. | Structure | Mol Wt. | LCMS MH⁺ | RT (min) | HPLC Method |
|---|---|---|---|---|---|
| 301 | 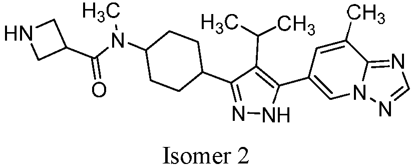 | 435.576 | 436.3 | 1.18 | E |

The following Examples were prepared according to the general procedure used to prepare Example 300.

**TABLE 28**

| Ex. No. | Structure | LCMS MH⁺ | RT (min) | HPLC Method |
|---|---|---|---|---|
| 302 | 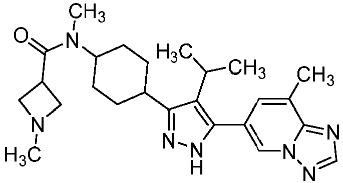 | 450.3 | 1.23 | C |
| | 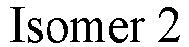 | | | |
| 303 | 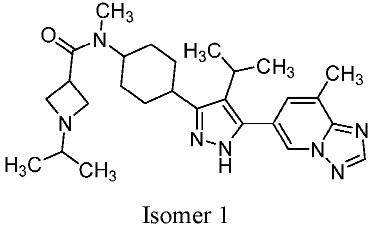 | 478.3 | 1.32 | C |
| 304 | 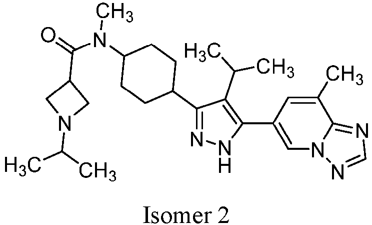 | 478.3 | 1.38 | C |
| 305 | 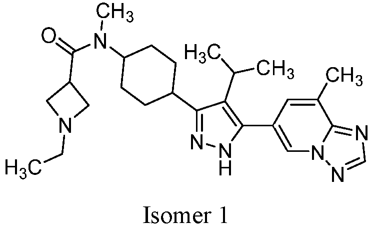 | 464.3 | 1.17 | C |
| 306 | 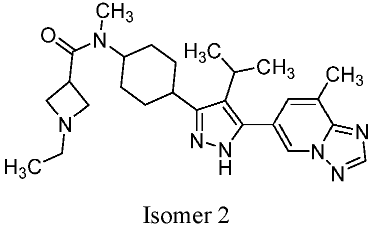 | 464.3 | 1.32 | C |

### EXAMPLE 307

### 6-(4-isopropyl-3-(4-(1-isopropylpiperidin-4-yl)phenyl)-1H-pyrazol-5-yl)-8-methyl-[1,2,4]triazolo[1,5-a]pyridine

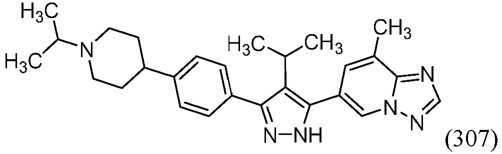

### Intermediate 307A: tert-butyl 4-(4-bromophenyl)-3,6-dihydropyridine-1(2H)-carboxylate

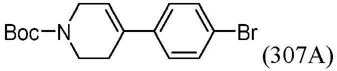

PdCl₂(dppf)-CH₂Cl₂ adduct (0.289 g, 0.353 mmol) and K₃PO₄ (1.847 g, 10.60 mmol) were added to a degassed solution of 1-bromo-4-iodobenzene (1.0 g, 3.53 mmol) and tert-butyl 4-(4,4,5,5-tetramethyl-1,3,2-dioxaborolan-2-yl)-3,6-dihydropyridine-1(2H)-carboxylate (0.656 g, 2.121 mmol) in mixture of dioxane (20.0 mL) and water (4.0 mL). The resulting reaction mixture was stirred at 95 °C for 14 h in a sealed tube. The reaction mixture was diluted with ethyl acetate, filtered and washed with excess ethyl acetate. The combined organic layers were washed with water, brine, then dried over sodium sulphate and evaporated to get crude material. The crude material was purified by ISCO using a 40 g silica column, compound was eluted with 30-55% ethyl acetate and pet ether to get tert-butyl 4-(4-bromophenyl)-3,6-dihydropyridine-1(2H)-carboxylate (850 mg, 2.51 mmol, 71 % yield) as a light brown semi-solid. LCMS Retention time: 1.71min [A], MS (E⁺) m/z: 284.3 [M-tBu].

### Intermediate 307B: tert-butyl 4-(4-(4-isopropyl-5-(8-methyl-[1,2,4]triazolo[1,5-a] pyridin-6-yl)-1-((2-(trimethylsilyl)ethoxy)methyl)-1H-pyrazol-3-yl)phenyl)-3,6-dihydro pyridine-1(2H)-carboxylate

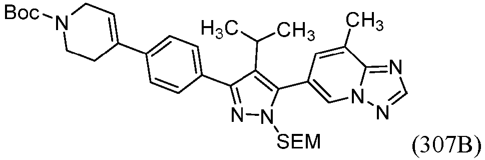

Pd₂(dba)₃ (54 mg, 0.059 mmol) and SPhos (48.5 mg, 0.118 mmol) were added to a degassed solution of tert-butyl 4-(4-bromophenyl)-3,6-dihydropyridine-1(2H)-carboxylate (400 mg, 1.183 mmol), K₂CO₃ (490 mg, 3.55 mmol) and 6-(4-isopropyl-3-(4,4,5,5-tetramethyl-1,3,2-dioxaborolan-2-yl)-1-((2-(trimethylsilyl)ethoxy)methyl)-1H-pyrazol-5-yl)-8-methyl-[1,2,4] triazolo[1,5-a] pyridine (765 mg, 1.537 mmol) in mixture of acetonitrile (20.0 mL) and water (4.0 mL). The resulting reaction mixture was stirred at 110 °C for 14 h in a sealed tube. The reaction mixture was diluted with ethyl acetate, filtered and washed with excess ethyl acetate. The combined organic layers were washed with water, brine, then dried over sodium sulphate and evaporated to get crude compound. The crude compound was purified by ISCO using a 40 g silica column, compound was eluted with 55-85% ethyl acetate and Pet ether to get tert-butyl 4-(4-(4-isopropyl-5-(8-methyl-[1,2,4]triazolo[1,5-a]pyridin-6-yl)-1-((2-(trimethylsilyl)ethoxy) methyl)-1H-pyrazol-3-yl)phenyl)-3,6-dihydropyridine-1(2H)-carboxylate (520 mg, 0.827 mmol, 70% yield) as a light yellow solid. LCMS Retention time 4.48 min [B], MS (E⁺) m/z: 629.9 [M+H].

### Intermediate 307C: tert-butyl 4-(4-(4-isopropyl-5-(8-methyl-[1,2,4]triazolo[1,5-a]pyridin-6-yl)-1-((2-(trimethylsilyl)ethoxy)methyl)-1H-pyrazol-3-yl)phenyl)piperidine-1-carboxylate

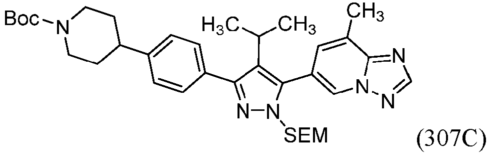

To a solution of tert-butyl 4-(4-(4-isopropyl-5-(8-methyl-[1,2,4]triazolo[1,5-a] pyridin-6-yl)-1-((2-(trimethylsilyl)ethoxy)methyl)-1H-pyrazol-3-yl)phenyl)-3,6-dihydropyridine-1(2H)-carboxylate (480 mg, 0.763 mmol) in methanol (20.0 mL) at 26 °C was added Pd-C (244 mg, 2.290 mmol). The reaction mixture was stirred at room temperature for 12 h under a hydrogen bladder. The reaction mixture was filtered and washed with excess methanol and THF. The combined organic layers were evaporated to get tert-butyl,4-(4-(4-isopropyl-5-(8-methyl-[1,2,4]triazolo[1,5-a]pyridin-6-yl)-1-((2-(trimethylsilyl)ethoxy)methyl)-1H-pyrazol-3-yl)phenyl)piperidine-1-carboxylate (330 mg, 0.523 mmol, 68% yield) as a light brown solid. LCMS Retention time 4.36 min [B], MS (E⁺) m/z: 631.4 (M+H).

### Intermediate 307D: 6-(4-isopropyl-3-(4-(piperidin-4-yl)phenyl)-1H-pyrazol-5-yl)-8-methyl-[1,2,4]triazolo[1,5-a]pyridine

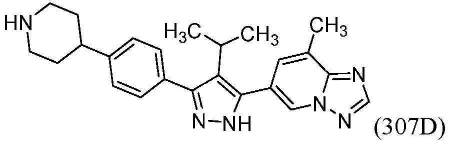

To a solution of tert-butyl 4-(4-(4-isopropyl-5-(8-methyl-[1,2,4]triazolo[1,5-a] pyridin-6-yl)-1-((2-(trimethylsilyl)ethoxy)methyl)-1H-pyrazol-3-yl)phenyl)piperidine-1-carboxylate (310 mg, 0.491 mmol) in DCM (5.0 mL) at 0 °C was added TFA (0.946 mL, 12.28 mmol). The reaction mixture was stirred for 12 h at room temperature. The volatiles were evaporated and dried under high vacuum to get 6-(4-isopropyl-3-(4-(piperidin-4-yl) phenyl)-1H-pyrazol-5-yl)-8-methyl-[1,2,4]triazolo[1,5-a] pyridine (270 mg) as an off-white solid. LCMS Retention time: 1.68 min [B], MS (E⁺) m/z: 401.4 [M+H].

### Example 307: 6-(4-isopropyl-3-(4-(1-isopropylpiperidin-4-yl)phenyl)-1H-pyrazol-5-yl)-8-methyl-[1,2,4]triazolo[1,5-a]pyridine

A solution of 6-(4-isopropyl-3-(4-(piperidin-4-yl)phenyl)-1H-pyrazol-5-yl)-8-methyl-[1,2,4] triazolo[1,5-a]pyridine (0.045 g, 0.112 mmol), acetone (0.082 mL, 1.124 mmol) and acetic acid (0.643 µl, 0.011 mmol) in MeOH (2.0 mL) was stirred at room temperature for 8 h, then sodium cyanoborohydride (10.59 mg, 0.169 mmol) was added at 0 °C. The reaction mixture was stirred at room temperature for 16 h. The reaction mass was purified via preparative LC/MS using method AA, fractions containing the product were combined and dried via centrifugal evaporation to get 6-(4-isopropyl-3-(4-(1-isopropylpiperidin-4-yl) phenyl)-1H-pyrazol-5-yl)-8-methyl-[1,2,4]triazolo[1,5-a]pyridine (2.8 mg) as a pale solid. LCMS Retention time: 1.32 min [E], MS (E⁺) m/z: 443.3 [M+H]; ¹H NMR (400 MHz, METHANOL-d₄) δ 8.76 (s, 1H), 8.48 (s, 1H), 7.71-7.63 (m, 1H), 7.60-7.49 (m, *J*=8.5 Hz, 2H), 7.49-7.37 (m, *J*=8.0 Hz, 2H), 3.70-3.56 (m, 3H), 3.30-3.23 (m, 1H),3.16-2.98 (m, 2H), 2.78-2.66 (m, 3H), 2.26 (d, *J*=14.1 Hz, 2H), 2.12-1.98 (m, 2H), 1.44 (d, *J*=7.0 Hz, 6H), 1.19 (d, *J*=7.0 Hz, 6H).

The following Example was prepared according to the general procedure used to prepare Intermediate 307D.

**TABLE 29**

| Ex. No. | Structure | LCMS MH⁺ | RT (min) | HPLC Method |
|---|---|---|---|---|
| 308 | 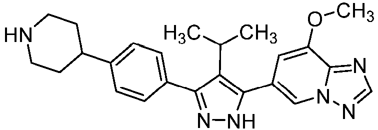 | 417.3 | 1.05 | C |

The following Examples were prepared according to the general procedure used to prepare Example 307.

**TABLE 30**

| Ex. No. | Structure | LCMS MH⁺ | RT (min) | HPLC Method |
|---|---|---|---|---|
| 309 | 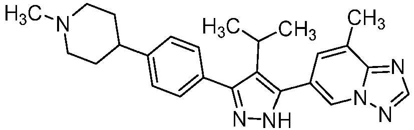 | 415.3 | 1.24 | C |
| 310 | 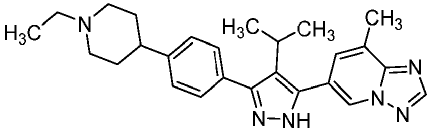 | 429.3 | 1.81 | C |
| 311 | 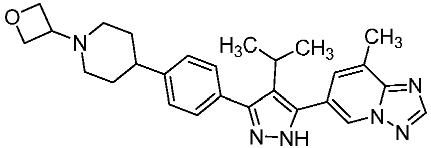 | 457.2 | 1.54 | E |
| 312 | 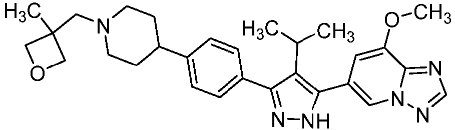 | 501.3 | 1.7 | C |
| 313 | 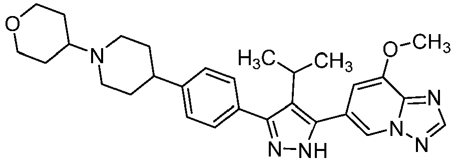 | 501.3 | 1.32 | C |
| 314 | 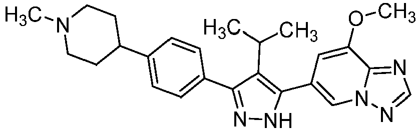 | 431.3 | 1.15 | C |
| 315 | 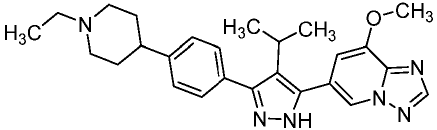 | 445.3 | 1.16 | C |
| 316 | 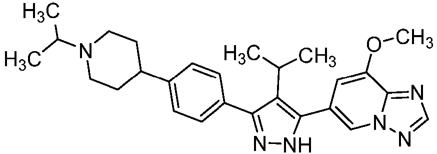 | 458.3 | 1.23 | C |
| 317 | 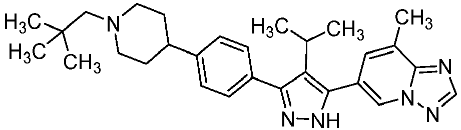 | 471.3 | 2.16 | C |

### EXAMPLE 318

### 6-(4-isopropyl-3-(4-(1-(2-methoxyethyl)piperidin-4-yl)phenyl)-1H-pyrazol-5-yl)-8-methoxy-[1,2,4]triazolo[1,5-a]pyridine

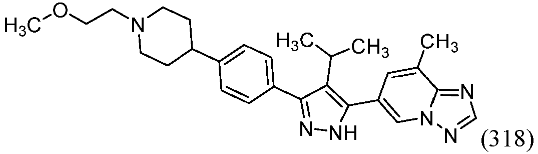

TEA (0.134 mL, 0.960 mmol) was added to a solution of 6-(4-isopropyl-3-(4-(piperidin-4-yl) phenyl)-1H-pyrazol-5-yl)-8-methoxy-[1,2,4]triazolo[1,5-a]pyridine (40.0 mg, 0.096 mmol) and 1-bromo-2-methoxyethane (66.7 mg, 0.480 mmol) in DMF (2.0 mL). The reaction mixture was stirred at 70 °C for 2 h. Volatiles were evaporated and dried in high vacuumed to get residue. The crude material was purified via preparative LC/MS using method AA, the fractions containing the product were combined and dried via centrifugal evaporation to get 6-(4-isopropyl-3-(4-(1-(2-methoxyethyl)piperidin-4-yl)phenyl)-1H-pyrazol-5-yl)-8-methoxy-[1,2,4]triazolo[1,5-a] pyridine as a white solid. LCMS Retention time: 1.65 min [E], MS (E⁺) m/z: 475.4 [M+H]; ¹H NMR (400 MHz, METHANOL-d₄) δ 8.42 (s, 1H), 8.33 (s, 1H), 7.42 (d, *J*=7.8 Hz, 2H), 7.34(d, *J*=8.1 Hz, 2H), 7.13 (s, 1H), 4.02 (s, 3H), 3.71-3.61 (m, 4H), 3.36 (s, 3H), 3.32-3.28 (m,2H), 3.14-3.02 (m, 3H), 2.95-2.88 (m, 1H), 2.60 (s, 1H), 2.10 (d, *J*=13.9 Hz, 2H), 2.03-1.94 (m, 2H), 1.19 (s, 2H), 1.10 (d, *J*=7.1 Hz, 6H).

The following Examples were prepared according to the general procedure used to prepare Example 318.

**TABLE 31**

| Ex. No. | Structure | LCMS MH⁺ | RT (min) | HPLC Method |
|---|---|---|---|---|
| 319 | 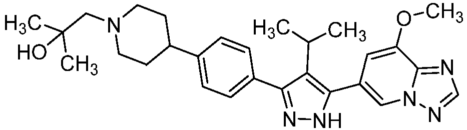 | 489.3 | 1.52 | C |
| 320 | 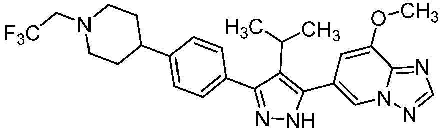 | 499.3 | 2.14 | C |

### EXAMPLE 321

### 2-(4-(4-(4-isopropyl-5-(8-methyl-[1,2,4]triazolo[1,5-a]pyridin-6-yl)-1H-pyrazol-3-yl) phenyl)piperidin-1-yl)-N,N-dimethylacetamide

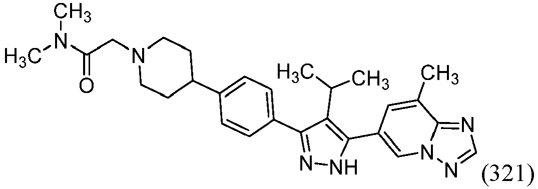

To a solution of 6-(4-isopropyl-3-(4-(piperidin-4-yl)phenyl)-1H-pyrazol-5-yl)-8-methyl-[1,2,4]triazolo[1,5-a]pyridine (0.025 g, 0.062 mmol) in DMF (2 mL) solvent were added TEA (0.026 mL, 0.187 mmol) and 2-chloro-N,N-dimethylacetamide (10.93 µL, 0.106 mmol) at room temperature. The resulting solution was stirred at room temperature for 12 h. The reaction mass was diluted with water (10 mL) and extracted with ethyl acetate (3 X 10 mL). The separated organic layer was dried over sodium sulphate, filtered and dried. The crude sample was purified by prep LCMS using method AA, the fractions containing the product were combined and dried via centrifugal evaporation to get 2-(4-(4-(4-isopropyl-5-(8-methyl-[1,2,4]triazolo[1,5-a]pyridin-6-yl)-1H-pyrazol-3-yl)phenyl) piperidin-1-yl)-N,N-dimethylacetamide (2.14 mg, 4.10 µmol, 6.57 % yield) as a pale yellow solid. LCMS retention time 1.35 min [E]. MS (E⁻) *m*/*z*: 486.2 [M+H]; ¹H NMR (400 MHz, DMSO-d₆) δ 13.04 (br. s., 1H), 8.78 (br. s., 1H), 8.51 (br. s., 1H), 7.63 (br.s., 1H), 7.55-7.38 (m, 3H), 7.35 (br. s., 1H), 3.21-3.12 (m, 2H), 3.09-3.01 (m, 4H), 2.96 (d, *J*=11.5 Hz, 2H), 2.88 (s, 1H), 2.83 (s, 3H), 2.62 (s, 3H), 2.18 (t, *J*=10.4 Hz, 2H), 1.86-1.75 (m,2H), 1.75-1.61 (m, 2H), 1.12 (d, *J*=7.1 Hz, 6H).

The following Example was prepared according to the general procedure used to prepare Example 321.

**TABLE 32**

| Ex. No. | Structure | LCMS MH⁺ | RT (min) | HPLC Method |
|---|---|---|---|---|
| 322 | 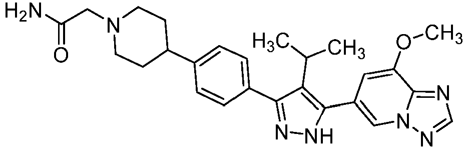 | 474.3 | 1.43 | C |

### EXAMPLE 323

### 2-(dimethylamino)-1-(4-(4-(4-isopropyl-5-(8-methyl-[1,2,4]triazolo[1,5-a]pyridin-6-yl)-1H-pyrazol-3-yl)phenyl)piperidin-1-yl)ethan-1-one

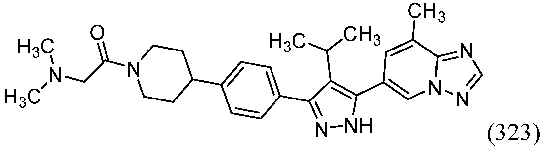

To a solution of 6-(4-isopropyl-3-(4-(piperidin-4-yl)phenyl)-1H-pyrazol-5-yl)-8-methyl-[1,2,4]triazolo[1,5-a]pyridine (175 mg, 0.436 mmol) and dimethyl glycine (45 mg, 0.436 mmol)) in DMF (1 mL) was added TEA (0.061 mL, 0.436 mmol) followed by HATU (166 mg, 0.436 mmol). The resulting reaction mixture was stirred at room temperature for 3 h. The reaction mass was diluted with water (5 ml) and extracted with ethyl acetate (3 X 10 mL). The separated organic layer was dried over sodium sulphate, filtered and dried. The crude sample was purified by prep LCMS using method AA, the fractions containing the product were combined and dried via centrifugal evaporation to get 2-(dimethylamino)-1-(4-(4-(4-isopropyl-5-(8-methyl-[1,2,4]triazolo[1,5-a]pyridin-6-yl)-1H-pyrazol-3-yl)phenyl)piperidin-1-yl)ethan-1-one (5 mg, 10.09 µmol) as a pale yellow solid. LCMS retention time 1.27 min [E], MS (E⁻) *m*/*z*: 486.2 [M+H]; ¹H NMR (400 MHz, DMSO-d₆) δ 13.05 (br. s., 1H), 8.76 (s, 1H), 8.50 (s, 1H), 7.62 (br. s., 1H),7.49-7.22 (m, 4H), 4.51 (br. s., 1H), 4.14 (d, *J*=5.4 Hz, 2H), 3.25 (d, *J*=12.7 Hz, 1H), 3.11 -2.91 (m, 2H), 2.86 (br. s., 2H), 2.61 (s, 3H), 2.25 (s, 6H), 1.91-1.77 (m, 2H), 1.62 (d, *J*=9.5 Hz,1H), 1.49 (s, 1H), 1.12 (d, *J*=7.1 Hz, 6H).

### EXAMPLE 324

### 6-(4-isopropyl-3-(4-(1-methylazetidin-3-yl)phenyl)-1H-pyrazol-5-yl)-8-methyl-[1,2,4] triazolo[1,5-a]pyridine

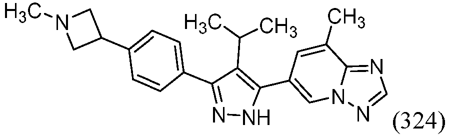

### Intermediate 324A: tert-butyl 3-(4-bromophenyl) azetidine-1-carboxylate

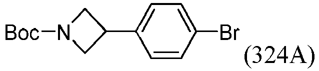

Sodium bis(trimethylsilyl)amide (3.53 mL, 7.06 mmol) was added to a degassed solution of tert-butyl 3-iodoazetidine-1-carboxylate (1.0 g, 3.53 mmol), 2-(4-bromophenyl)-4,4,5,5-tetramethyl-1,3,2-dioxaborolane (1.0 g, 3.53 mmol), nickel(ii) iodide (0.110 g, 0.353 mmol) and trans-2-aminocyclohexanol hydrochloride (0.054 g, 0.353 mmol) in 2-propanol (14.0 mL). The reaction mixture was stirred at room temperature for 30 min, and then irradiate in microwave at 80 °C for 1 h. The reaction mixture was diluted with ethyl acetate, filtered and washed with excess ethyl acetate, the filtrate was washed with water, brine, dried over sodium sulphate and concentrated to get crude compound. The crude compound was purified by ISCO using 40 g silica column, compound was eluted with 25-35% ethyl acetate in pet ether, the fractions were collected and concentrated to get tert-butyl 3-(4-bromophenyl)azetidine-1-carboxylate (650 mg, 2.082 mmol, 59 % yield) as a light brown solid. LCMS Retention time: 1.55 min [A], MS (E⁺) m/z: 256 [M-tBu].

### Intermediate 324B: tert-butyl 3-(4-(4,4,5,5-tetramethyl-1,3,2-dioxaborolan-2-yl) phenyl) azetidine-1-carboxylate

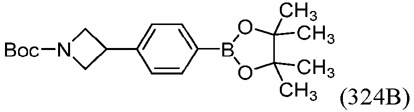

tert-Butyl 3-(4-(4,4,5,5-tetramethyl-1,3,2-dioxaborolan-2-yl)phenyl)azetidine-1-carboxylate (650 mg, 1.809 mmol, 87% yield) was prepared according to the general procedure described in Intermediate 278B using tert-butyl 3-(4-bromophenyl)azetidine-1-carboxylate (650 mg, 2.082 mmol) as a starting intermediate to yield the title compound as an off-white solid. LCMS Retention time: 1.67 min [A], MS (E⁺) m/z: 304.0 [M+H-tBu].

### Intermediate 324C: tert-butyl 3-(4-(4-isopropyl-5-(8-methyl-[1,2,4]triazolo[1,5-a] pyridin-6-yl)-1-((2-(trimethylsilyl)ethoxy)methyl)-1H-pyrazol-3-yl)phenyl)azetidine-1-carboxylate

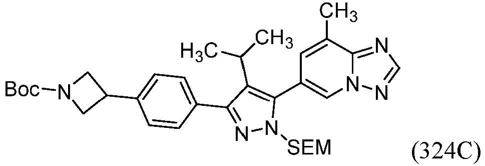

tert-butyl 3-(4-(4-isopropyl-5-(8-methyl-[1,2,4]triazolo[1,5-a]pyridin-6-yl)-1-((2-(trimethy lsilyl)ethoxy)methyl)-1H-pyrazol-3-yl)phenyl)azetidine-1-carboxylate (450 mg, 0.746 mmol, 56% yield) was prepared according to the general procedure described in Intermediate 307B, using tert-butyl 3-(4-(4,4,5,5-tetramethyl-1,3,2-dioxaborolan-2-yl)phenyl)azetidine-1-carboxylate (0.622 g, 1.732 mmol) and 6-(3-bromo-4-isopropyl-1-((2-(trimethylsilyl)ethoxy)methyl)-1H-pyrazol-5-yl)-8-methyl-[1,2,4]triazolo[1,5-a]pyridine (0.600 g, 1.332 mmol) as starting intermediates to yield the title compound as a gummy solid. LCMS Retention time: 1.91 min [A], MS (E⁺) m/z: 603.5 [M+H].

### Intermediate 324D: 6-(3-(4-(azetidin-3-yl)phenyl)-4-isopropyl-1H-pyrazol-5-yl)-8-methyl-[1,2,4]triazolo[1,5-a]pyridine

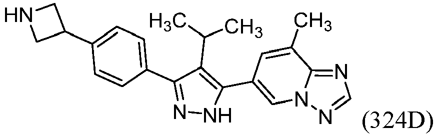

6-(3-(4-(azetidin-3-yl)phenyl)-4-isopropyl-1H-pyrazol-5-yl)-8-methyl-[1,2,4]triazolo[1,5-a] pyridine (2.6 mg) was prepared as according to the general procedure described in Intermediate 307D using tert-butyl 3-(4-(4-isopropyl-5-(8-methyl-[1,2,4]triazolo[1,5-a]pyridin-6-yl)-1-((2-(trimethylsilyl)ethoxy) methyl)-1H-pyrazol-3-yl)phenyl)azetidine-1-carboxylate (450 mg, 0.746 mmol) as a starting intermediate to yield the title compound as an off-white solid. LCMS Retention time: 1.07 min [E], MS (E⁺) m/z: 373.2 [M+H]; ¹H NMR (400 MHz, METHANOL-d₄) δ ppm 8.77 (s, 1H), 8.49 (s, 1H), 7.67 (br. s., 1H), 7.63-7.44 (m, 4H), 4.43-4.23 (m, 3H), 3.17-3.07 (m, 1H), 2.71 (s, 3H), 1.99-1.88 (m, 2H), 1.31 (s, 1H),1.20 (d, *J*=7.1 Hz, 6H).

The following Examples were prepared according to the general procedure used to prepare Example 324D.

**TABLE 33**

| Ex. No. | Structure | LCMS MH⁺ | RT (min) | HPLC Method |
|---|---|---|---|---|
| 324E | 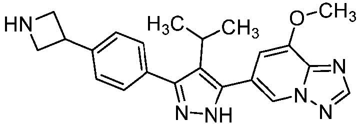 | 389.2 | 1.00 | C |

### Example 324: 6-(4-isopropyl-3-(4-(1-methylazetidin-3-yl)phenyl)-1H-pyrazol-5-yl)-8-methyl-[1,2,4]triazolo[1,5-a]pyridine

6-(4-isopropyl-3-(4-(1-methylazetidin-3-yl)phenyl)-1H-pyrazol-5-yl)-8-methyl-[1,2,4] triazolo[1,5-a]pyridine (13.6) was prepared according to the general procedure described in Example 307 using 6-(3-(4-(azetidin-3-yl)phenyl)-4-isopropyl-1H-pyrazol-5-yl)-8-methyl-[1,2,4]triazolo[1,5-a]pyridine (30.0 mg, 0.081 mmol) as an starting intermediate to yield the title compound as an off-white solid. LCMS Retention time: 1.13 min [E], MS (E⁺) m/z: 387.3 [M+H]; ¹H NMR (400 MHz, METHANOL-d₄) δ 8.77 (s, 1H), 8.49 (s, 1H), 7.66 (s, 1H), 7.65-7.48 (m,4H), 4.67 (br. s., 2H), 4.58 (br. s., 1H), 4.41 (br. s., 2H), 4.29 (br. s., 2H), 3.18-2.99 (m, 5H),2.71 (s, 3H), 1.40-1.26 (m, 1H), 1.20 (d, *J*=7.1 Hz, 6H).

The following Examples were prepared according to the general procedure used to prepare Example 324.

**TABLE 34**

| Ex. No. | Structure | LCMS MH⁺ | RT (min) | HPLC Method |
|---|---|---|---|---|
| 325 | 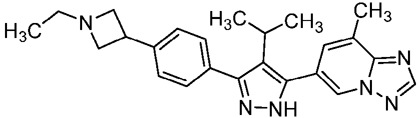 | 401.3 | 1.21 | C |
| 326 | 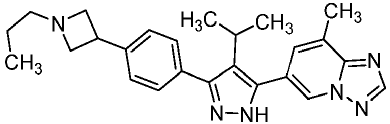 | 415.3 | 1.35 | C |
| 327 | 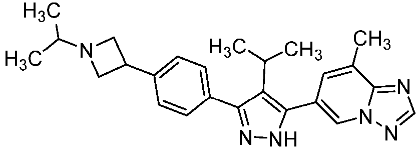 | 415.3 | 1.311 | C |
| 328 | 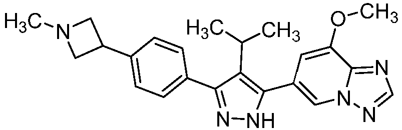 | 403.3 | 1.08 | C |
| 329 | 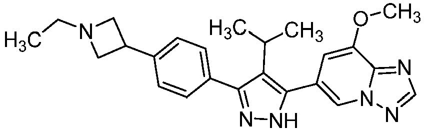 | 417.3 | 1.17 | C |
| 330 | 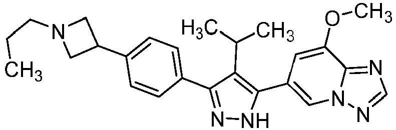 | 431.3 | 1.3 | C |
| 331 | 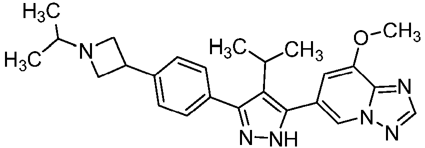 | 431.3 | 1.26 | C |
| 332 | 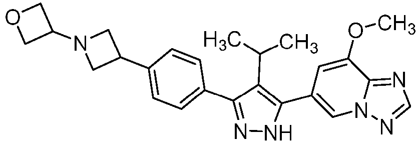 | 445.2 | 1.37 | C |
| 333 | 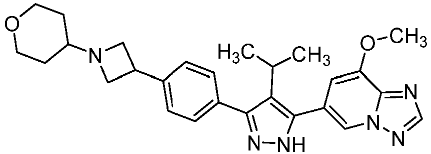 | 473.3 | 1.35 | C |

### EXAMPLE 334

### 2-(dimethylamino)-1-(3-(4-(4-isopropyl-5-(8-methyl-[1,2,4]triazolo[1,5-a]pyridin-6-yl)-1H-pyrazol-3-yl)phenyl)azetidin-1-yl)ethan-1-one

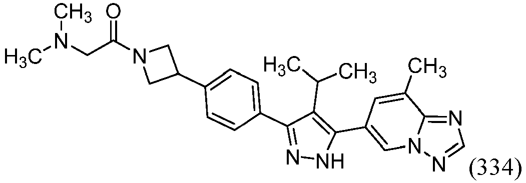

2-(dimethylamino)-1-(3-(4-(4-isopropyl-5-(8-methyl-[1,2,4]triazolo[1,5-a]pyridin-6-yl)-1H-pyrazol-3-yl)phenyl)azetidin-1-yl)ethan-1-one (28.2 mg) was prepared according to the general procedure described in Example 323 using 6-(3-(4-(azetidin-3-yl)phenyl)-4-isopropyl-1H-pyrazol-5-yl)-8-methyl-[1,2,4]triazolo [1,5-a] pyridine (40.0 mg, 0.107 mmol) as a starting intermediate to yield the title compound as an off-white solid. LCMS Retention time: 1.32 min [E], MS (E⁺) m/z: 458.3 [M+H]; ¹H NMR (400 MHz, METHANOL-d₄) δ 8.77 (s, 1H), 8.49 (s, 1H), 7.67 (s, 1H), 7.62-7.48 (m,4H), 4.69 (t, *J*=8.6 Hz, 1H), 4.62-4.55 (m, 1H), 4.39-4.32 (m, 1H), 4.23-4.11 (m, 2H), 4.09(s, 2H), 3.13 (dt, *J*=14.2, 7.1 Hz, 1H), 3.04-2.93 (m, 6H), 2.71 (s, 3H), 1.32 (d, *J*=3.7 Hz, 1H),1.20 (d, *J*=7.1 Hz, 6H).

The following Example was prepared according to the general procedure used to prepare Example 334.

**TABLE 35**

| Ex. No. | Structure | LCMS MH⁺ | RT (min) | HPLC Method |
|---|---|---|---|---|
| 335 | 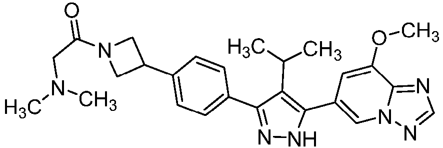 | 474.3 | 1.26 | C |

### EXAMPLE 336

### 1-(3-(4-(4-isopropyl-5-(8-methyl-[1,2,4]triazolo[1,5-a]pyridin-6-yl)-1H-pyrazol-3-yl) phenyl)azetidin-1-yl)-2-methylpropan-2-ol

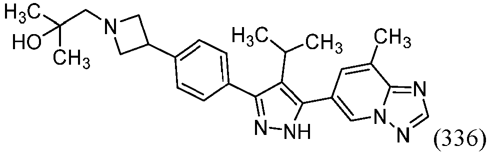

TEA (0.056 mL, 0.403 mmol) was added to a solution of 6-(3-(4-(azetidin-3-yl) phenyl)-4-isopropyl -1H-pyrazol-5-yl)-8-methyl-[1,2,4]triazolo[1,5-a]pyridine (30.0 mg, 0.081 mmol) and 1-chloro-2-methylpropan-2-ol (17.49 mg, 0.161 mmol) in DMF (2.0 mL) at room temperature. The reaction mixture was stirred at 90 °C for 16 h. The reaction mass was purified by preparative LC/MS using method AA, the fractions containing the product were combined and dried via centrifugal evaporation to get 1-(3-(4-(4-isopropyl-5-(8-methyl-[1,2,4]triazolo[1,5-a]pyridin-6-yl)-1H-pyrazol-3-yl)phenyl)azetidin-1-yl)-2-methylpropan-2-ol (4.6 mg) as a white solid. LCMS Retention time: 1.31min [E], MS (E⁺) m/z: 445.3 [M+H]; ¹H NMR (400 MHz, METHANOL-d₄) δ 8.76 (s, 1H), 8.48 (s, 1H), 7.67 (s, 1H), 7.60-7.40 (m,4H), 4.16 (d, *J*=7.6 Hz, 2H), 4.08-3.95 (m, 1H), 3.73 (br. s., 2H), 3.12 (dt, *J*=14.4, 7.1 Hz, 1H), 2.93-2.78 (m, 2H), 2.76-2.65 (m, 3H), 1.95 (s, 2H), 1.31 (br. s., 1H), 1.29-1.22 (m, 6H), 1.20(d, *J*=7.1 Hz, 6H).

The following Examples were prepared according to the general procedure used to prepare Example 336.

**TABLE 36**

| Ex. No. | Structure | LCMS MH⁺ | RT (min) | HPLC Method |
|---|---|---|---|---|
| 337 | 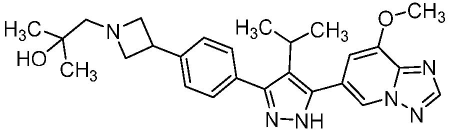 | 461.3 | 1.22 | C |
| 338 | 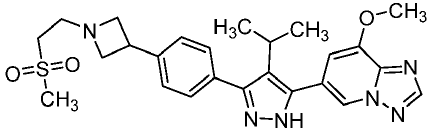 | 495.3 | 1.34 | C |

### EXAMPLE 339

### 6-(4-isopropyl-3-(5-(piperidin-4-yl)pyridin-2-yl)-1H-pyrazol-5-yl)-8-methoxy-[1,2,4] triazolo[1,5-a]pyridine

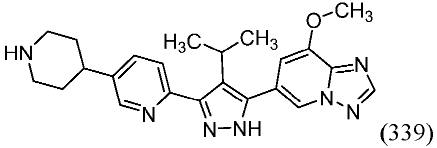

### Intermediate 339A: tert-butyl 6-bromo-3',6'-dihydro-[3,4'-bipyridine]-1'(2'H)-carboxylate

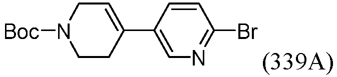

tert-Butyl 6-bromo-3',6'-dihydro-[3,4'-bipyridine]-1'(2'H)-carboxylate (1.4 g, 4.13 mmol, 85 % yield) was prepared according to the general procedure described in Intermediate 307A, using tert-butyl 4-(4,4,5,5-tetramethyl-1,3,2-dioxaborolan-2-yl)-3,6-dihydropyridine-1(2H)-carboxylate (1.5 g, 4.85 mmol) and 2-bromo-5-iodopyridine (2.066 g, 7.28 mmol) as starting intermediates to yield the title compound as a light brown semi-solid. LCMS Retention time: 1.80 min [A], MS (E⁺) m/z: 341.0 [M+2H].

### Intermediate 339B: tert-butyl 6-(4-isopropyl-5-(8-methoxy-[1,2,4]triazolo[1,5-a] pyridin-6-yl)-1-((2-(trimethylsilyl)ethoxy)methyl)-1H-pyrazol-3-yl)-3',6'-dihydro-[3,4'-bipyridine]-1'(2'H)-carboxylate

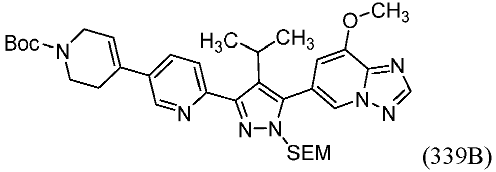

tert-butyl 6-(4-isopropyl-5-(8-methoxy-[1,2,4]triazolo[1,5-a]pyridin-6-yl)-1-((2-(trimethyl silyl)ethoxy) methyl)-1H-pyrazol-3-yl)-3',6'-dihydro-[3,4'-bipyridine]-1'(2'H)-carboxylate (700 mg, 1.084 mmol, 52.5 % yield) was prepared according to the general procedure described in Intermediate 307B using tert-butyl 6-bromo-3',6'-dihydro-[3,4'-bipyridine]-1'(2'H)-carboxylate (0.700 g, 2.063 mmol) and 6-(4-isopropyl-3-(4,4,5,5-tetramethyl-1 ,3,2-dioxaborolan-2-yl)-1-((2-(trimethylsilyl)ethoxy)methyl)-1H-pyrazol-5-yl)-8-methoxy-[1,2,4]triazolo[1,5-a]pyridine (1.695 g, 3.30 mmol) to yield the title compound as a light brown semi-solid. LCMS Retention time: 1.75 min [A], MS (E⁺) m/z: 646.6 [M+H].

### Intermediate 339C: tert-butyl 4-(6-(4-isopropyl-5-(8-methoxy-[1,2,4]triazolo[1,5-a] pyridin-6-yl)-1-((2-(trimethylsilyl)ethoxy)methyl)-1H-pyrazol-3-yl)pyridin-3-yl) piperidine-1-carboxylate

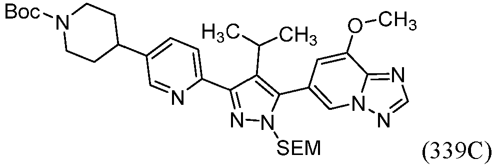

tert-Butyl 4-(6-(4-isopropyl-5-(8-methoxy-[1,2,4]triazolo[1,5-a]pyridin-6-yl)-1-((2-(trimethyl silyl)ethoxy)methyl)-1H-pyrazol-3-yl)pyridin-3-yl)piperidine-1-carboxylate (420 mg, 0.648 mmol, 59% yield) was prepared according to the general procedure described in Intermediate 307C using tert-butyl 6-(4-isopropyl-5-(8-methoxy-[1,2,4]triazolo[1,5-a] pyridin-6-yl)-1-((2-(trimethylsilyl)ethoxy) methyl)-1H-pyrazol-3-yl)-3',6'-dihydro-[3,4'-bipyridine]-1'(2'H)-carboxylate (0.700 g, 1.084 mmol) as a starting intermediate to yield the title compound as an off-white solid. LCMS Retention time: 1.67 min [A], MS (E⁺) m/z: 648.6 [M+H].

### Example 339: 6-(4-isopropyl-3-(5-(piperidin-4-yl)pyridin-2-yl)-1H-pyrazol-5-yl)-8-methoxy-[1,2,4]triazolo[1,5-a]pyridine

6-(4-Isopropyl-3-(5-(piperidin-4-yl)pyridin-2-yl)-1H-pyrazol-5-yl)-8-methoxy-[1,2,4]triazolo [1,5-a]pyridine (25 mg) was prepared according to the general procedure described in Intermediate 307D using tert-butyl 4-(6-(4-isopropyl-5-(8-methoxy-[1,2,4]triazolo[1,5-a]pyridin-6-yl)-1-((2-(trimethylsilyl) ethoxy)methyl)-1H-pyrazol-3-yl)pyridin-3-yl)piperidine-1-carboxylate (500 mg, 0.772 mmol) as a starting intermediate to yield the title compound as an off-white solid. LCMS Retention time: 0.97 min [E], MS (E⁺) m/z: 418.2 [M+H]; ¹H NMR (400 MHz, METHANOL-d₄) δ 8.65 (s, 1H), 8.54 (s, 1H), 8.45 (s, 1H), 7.86 (d, *J*=6.5Hz, 1H), 7.74 (d, *J*=8.0 Hz, 1H), 7.23 (s, 1H), 4.14 (s, 3H), 3.55-3.45 (m, 2H), 3.40 (d, *J*=6.0Hz, 1H), 3.17-3.02 (m, 3H), 2.15 (d, *J*=13.6 Hz, 2H), 2.02-1.87 (m, 4H), 1.39-1.13 (m, 6H).

The following Examples were prepared according to the general procedure used to prepare Example 339.

**TABLE 37**

| Ex. No. | Structure | LCMS MH⁺ | RT (min) | HPLC Method |
|---|---|---|---|---|
| 340 | 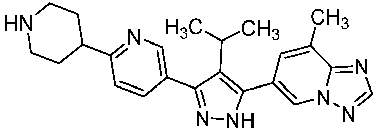 | 402.3 | 0.91 | C |
| 341 | 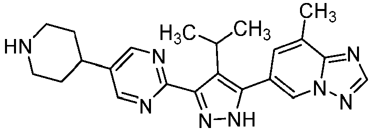 | 403.3 | 0.93 | C |
| 342 | 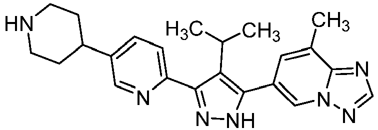 | 402.3 | 1.01 | C |
| 343 | 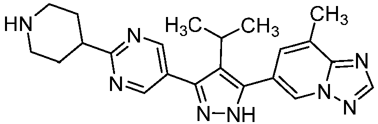 | 403.2 | 0.95 | C |
| 344 | 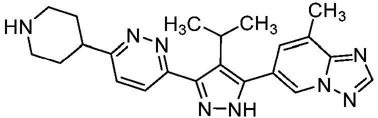 | 403.3 | 0.85 | C |
| 345 | 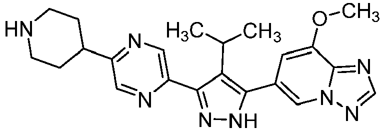 | 419.3 | 0.98 | AA |
| 346 | 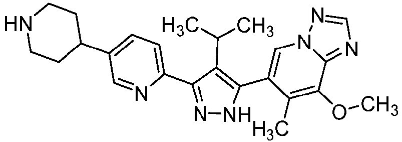 | 432.3 | 1.11 | AA |

### EXAMPLE 347

### 6-(3-(5-(1-ethylpiperidin-4-yl)pyridin-2-yl)-4-isopropyl-1H-pyrazol-5-yl)-8-methoxy-[1,2,4]triazolo[1,5-a]pyridine

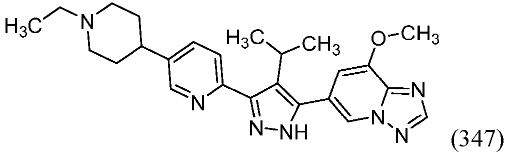

A mixture of 6-(4-isopropyl-3-(5-(piperidin-4-yl)pyridin-2-yl)-1H-pyrazol-5-yl)-8-methoxy-[1,2,4]triazolo[1,5-a]pyridine (35 mg, 0.084 mmol), acetaldehyde (0.024 mL, 0.419 mmol) and acetic acid (0.960 µL, 0.017 mmol) in MeOH (5.0 mL) was stirred at room temperature for 8 h. Sodium cyanoborohydride (10.54 mg, 0.168 mmol) was added at 0 °C, and the reaction mixture was stirred at room temperature 16 h. The reaction mass was purified via preparative LC/MS using method AA, the fractions containing product were combined and dried via centrifugal evaporation to get 6-(3-(5-(1-ethylpiperidin-4-yl)pyridin-2-yl)-4-isopropyl-1H-pyrazol-5-yl)-8-methoxy-[1,2,4]triazolo[1,5-a]pyridine (2.5 mg) as an off-white solid. LCMS Retention time: 1.11 min [E], MS (E⁺) m/z: 446.3 [M+H]; ¹H NMR (400 MHz, METHANOL-d₄) δ 8.63 (br. s., 1H), 8.54 (s, 1H), 8.44 (s, 1H), 7.85 (br. s.,1H), 7.70 (d, *J*=8.8 Hz, 1H), 7.24 (s, 1H), 4.19-4.07 (m, 3H), 3.27 (d, *J*=11.5 Hz, 2H), 2.82 (br.s., 1H), 2.67 (d, *J*=7.1 Hz, 2H), 2.43-2.29 (m, 2H), 2.09-1.98 (m, 2H), 1.98-1.85 (m, 6H),1.39-1.10 (m, 11H).

The following Examples were prepared according to the general procedure used to prepare Example 347.

**TABLE 38**

| Ex. No. | Structure | LCMS MH⁺ | RT (min) | HPLC Method |
|---|---|---|---|---|
| 348 | 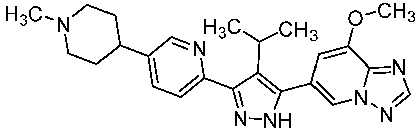 | 432.3 | 1.08 | C |
| 349 | 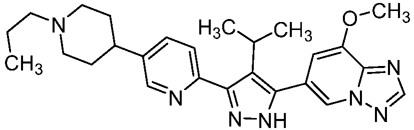 | 460.3 | 1.266 | C |
| 350 | 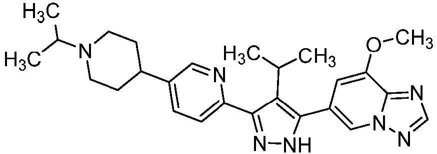 | 460.3 | 1.16 | C |
| 351 | 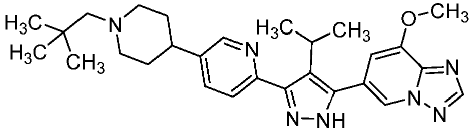 | 514.3 | 1.88 | C |
| 352 | 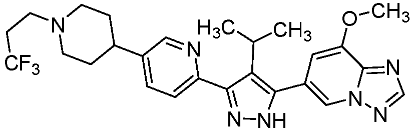 | 488.3 | 1.99 | C |
| 353 | 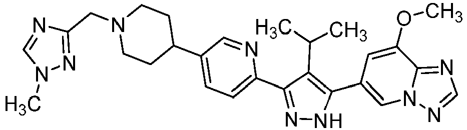 | 513.3 | 1.27 | C |
| 354 | 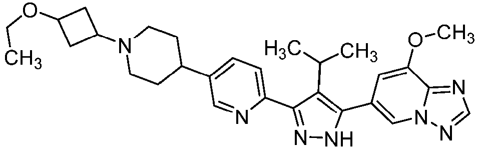 | 516.3 | 1.51 | C |
| 355 | 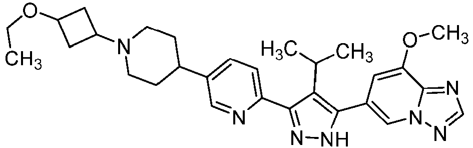 | 516.3 | 1.48 | C |
| 356 | 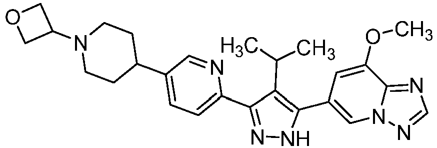 | 474.3 | 1.39 | C |
| 357 | 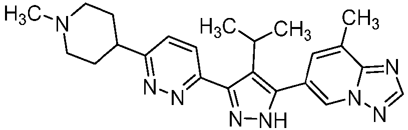 | 417.3 | 0.9 | C |
| 358 | 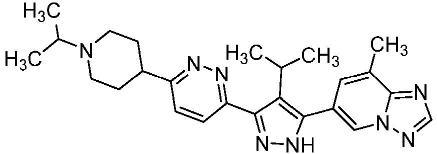 | 445.3 | 0.98 | C |
| 359 | 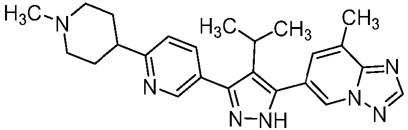 | 416.3 | 0.99 | C |
| 360 | 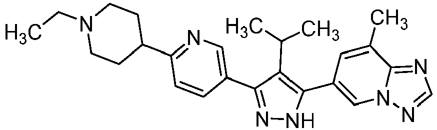 | 430.3 | 1.03 | C |
| 361 | 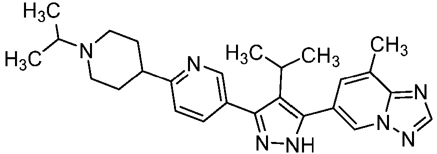 | 444.3 | 1.07 | C |
| 362 | 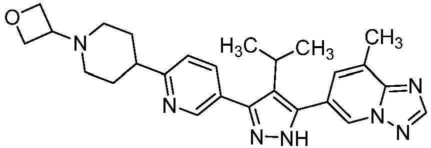 | 458.3 | 1.28 | C |
| 363 | 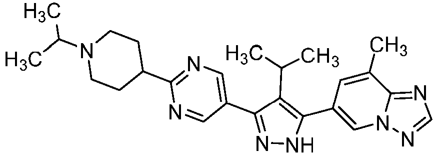 | 445.3 | 1.09 | C |
| 364 | 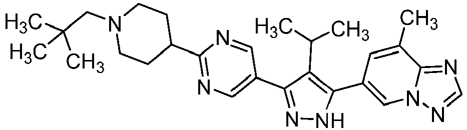 | 473.3 | 1.64 | C |
| 365 | 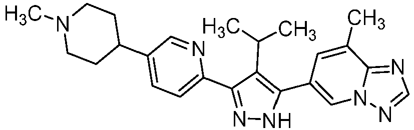 | 416.3 | 0.85 | C |
| 366 | 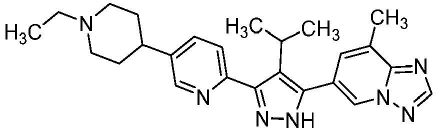 | 430.3 | 1.15 | C |
| 367 | 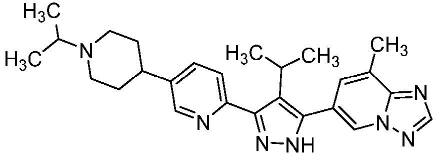 | 444.3 | 1.19 | C |
| 368 | 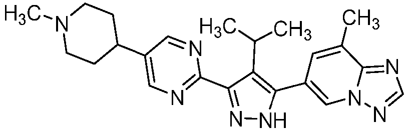 | 417.3 | 1.16 | C |
| 369 | 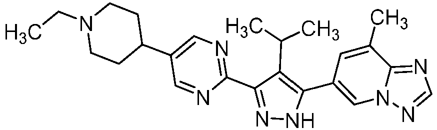 | 431.3 | 1.1 | C |
| 370 | 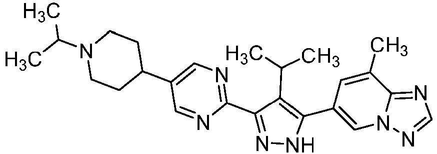 | 445.3 | 1.22 | C |
| 371 | 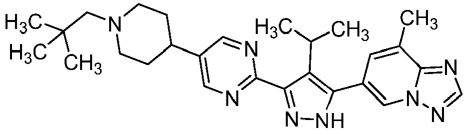 | 473.3 | 2.09 | C |
| 372 | 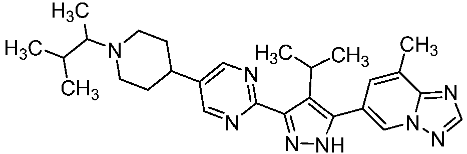 | 473.3 | 1.61 | C |
| 373 | 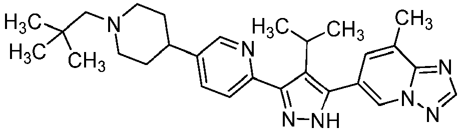 | 472.4 | 2.02 | C |
| 374 | 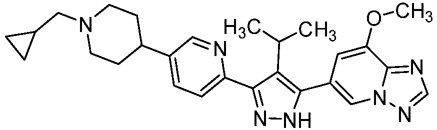 | 472.3 | 1.41 | C |
| 375 | 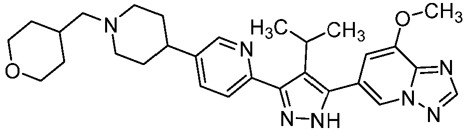 | 516.3 | 1.49 | C |
| 376 | 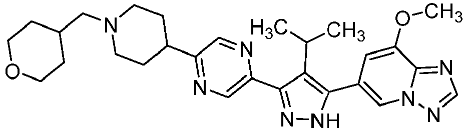 | 517.3 | 1.34 | C |
| 377 | 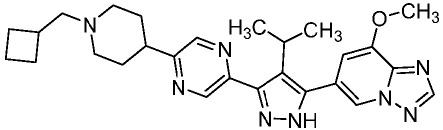 | 487.3 | 1.42 | C |
| 378 | 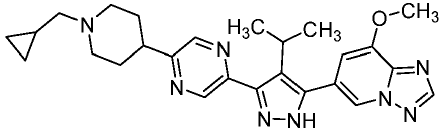 | 473.3 | 1.26 | C |
| 379 | 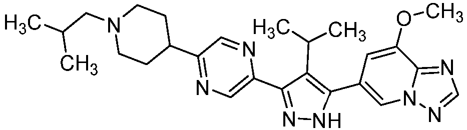 | 475.3 | 1.45 | C |
| 380 | 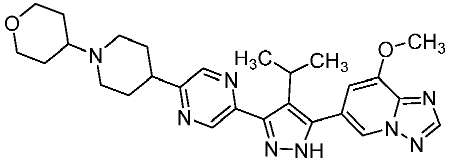 | 503.3 | 1.22 | C |
| 381 | 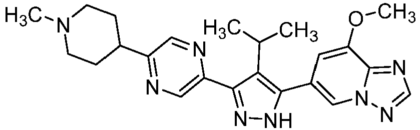 | 433.3 | 1.09 | C |
| 382 | 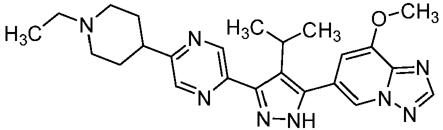 | 447.3 | 1.12 | C |
| 383 | 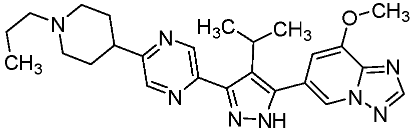 | 461.3 | 1.24 | C |
| 384 | 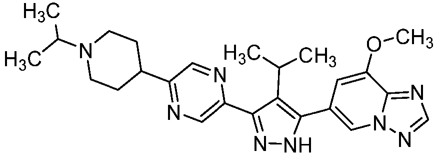 | 461.3 | 1.16 | C |
| 385 | 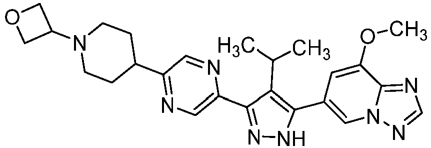 | 475.3 | 1.38 | C |
| 386 | 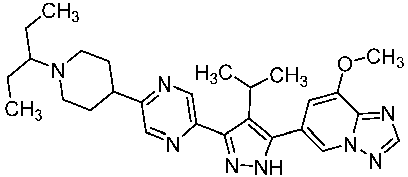 | 489.3 | 1.48 | C |
| 387 | 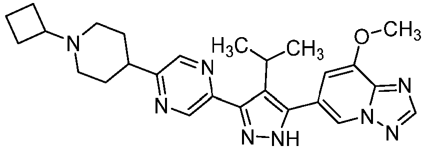 | 473.3 | 1.33 | C |
| 388 | 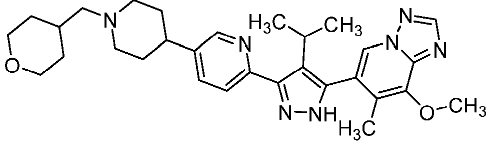 | 530.3 | 1.49 | C |

### EXAMPLE 389

### 2-(4-(6-(4-isopropyl-5-(8-methoxy-[1,2,4]triazolo[1,5-a]pyridin-6-yl)-1H-pyrazol-3-yl) pyridin-3-yl)piperidin-1-yl)-N,N-dimethylacetamide

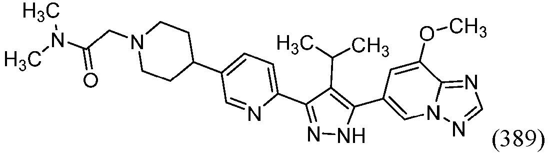

2-(4-(6-(4-Isopropyl-5-(8-methoxy-[1,2,4]triazolo[1,5-a]pyridin-6-yl)-1H-pyrazol-3-yl)pyridin-3-yl)piperidin-1-yl)-N,N-dimethylacetamide (13 mg) was prepared according to the general procedure described in Example 321, using 6-(4-isopropyl-3-(5-(piperidin-4-yl)pyridin-2-yl)-1H-pyrazol-5-yl)-8-methoxy-[1,2,4]triazolo[1,5-a]pyridine (35 mg, 0.084 mmol) as a starting intermediate to yield the title compound as a pale solid. LCMS Retention time 1.28 min [C], MS (E⁺) m/z: 503.3 [M+H]; ¹H NMR (400 MHz, METHANOL-d₄) δ 8.68 (br. s., 1H), 8.55 (s, 1H), 8.46 (s, 1H), 7.89 (d, J=8.1 Hz, 1H), 7.76 (d, *J*=8.1 Hz, 1H), 7.23 (s, 1H), 4.31 (s, 2H), 4.14 (s, 3H), 3.81 (d, *J*=12.0Hz, 2H), 3.47-3.39 (m, 1H), 3.31-3.17 (m, 2H), 3.15 (br. s., 1H), 3.12-2.89 (m, 7H), 2.24 (br.s., 3H), 2.18 (br. s., 1H), 1.38-1.19 (m, 7H).

The following Examples were prepared according to the general procedure used to prepare Example 389.

**TABLE 39**

| Ex. No. | Structure | LCMS MH⁺ | RT (min) | HPLC Method |
|---|---|---|---|---|
| 390 | 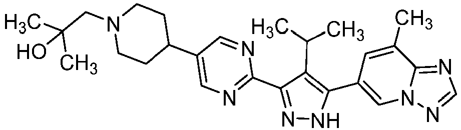 | 475.3 | 1.35 | C |
| 391 | 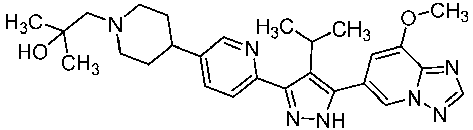 | 490.3 | 1.31 | C |
| 392 | 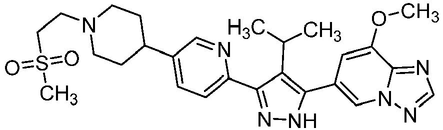 | 524.3 | 1.37 | C |
| 393 | 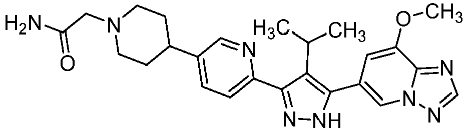 | 475.3 | 1.26 | C |
| 394 | 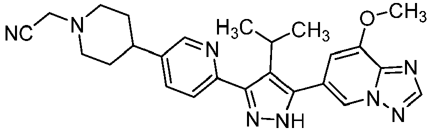 | 457.3 | 1.49 | C |
| 395 | 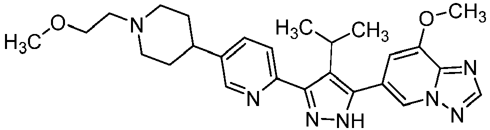 | 476.3 | 1.23 | C |
| 396 | 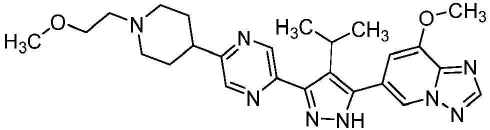 | 477.3 | 1.26 | C |
| 397 | 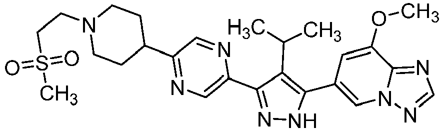 | 525.3 | 1.45 | C |
| 398 | 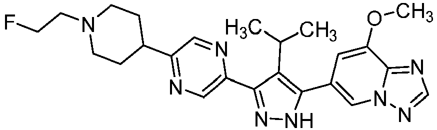 | 465.3 | 1.45 | C |
| 399 | 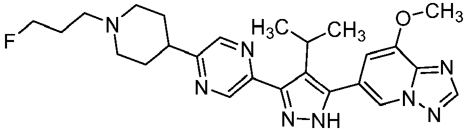 | 479.2 | 1.34 | C |
| 400 | 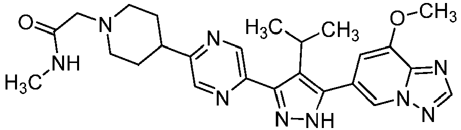 | 490.2 | 1.33 | C |
| 401 | 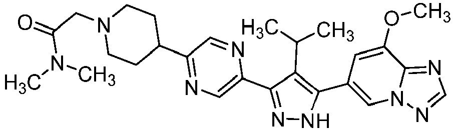 | 504.3 | 1.28 | C |

### EXAMPLE 402

### 2-(dimethylamino)-1-(4-(6-(4-isopropyl-5-(8-methoxy-[1,2,4]triazolo[1,5-a]pyridin-6-yl)-1H-pyrazol-3-yl)pyridin-3-yl)piperidin-1-yl)ethan-1-one

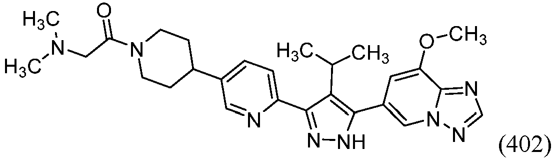

2-(dimethylamino)-1-(4-(6-(4-isopropyl-5-(8-methoxy-[1,2,4]triazolo[1,5-a] pyridin-6-yl)-1H-pyrazol-3-yl)pyridin-3-yl)piperidin-1-yl)ethan-1-one (32 mg) was prepared according to the general procedure described in Example 323, using 6-(4-isopropyl-3-(5-(piperidin-4-yl)pyridin-2-yl)-1H-pyrazol-5-yl)-8-methoxy-[1,2,4]triazolo[1,5-a]pyridine (35 mg, 0.084 mmol) as a starting intermediate to yield the title compound as an off-white solid. LCMS Retention time 1.16 min [C], MS (E⁺) m/z: 503.3 [M+H]; ¹H NMR (400 MHz, METHANOL-d₄) δ 8.64 (s, 1H), 8.55 (s, 1H), 8.45 (s, 1H), 7.86 (d, *J*=7.6Hz, 1H), 7.71 (d, *J*=8.6 Hz, 1H), 7.24 (s, 1H), 4.74 (d, *J*=10.5 Hz, 1H), 4.42-4.27 (m, 2H), 4.14(s, 3H), 3.85 (d, *J*=11.7 Hz, 1H), 3.39 (d, *J*=7.1 Hz, 2H), 3.12-2.96 (m, 16 h), 2.96-2.85 (m,1H), 2.72 (s, 3H), 2.04 (d, *J*=11.2 Hz, 2H), 1.89-1.70 (m, 2H), 1.38-1.21 (m, 6H).

The following Examples were prepared according to the general procedure used to prepare Example 402.

**TABLE 40**

| Ex. No. | Structure | LCMS MH⁺ | RT (min) | HPLC Method |
|---|---|---|---|---|
| 403 | 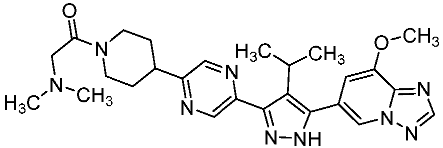 | 504.3 | 1.19 | C |
| 404 | 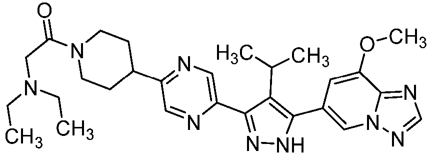 | 532.3 | 1.24 | C |

### EXAMPLE 405

### 1-(4-(4-isopropyl-5-(8-methyl-[1,2,4]triazolo[1,5-a]pyridin-6-yl)-1H-pyrazol-3-yl) phenyl)-N,N-dimethylazetidin-3-amine

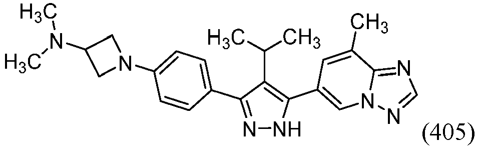

### Intermediate 405A: tert-butyl (1-(4-bromophenyl)azetidin-3-yl)carbamate

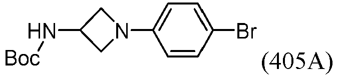

Xantphos (0.139 g, 0.240 mmol), Pd₂(dba)₃ (0.219 g, 0.240 mmol), and Cs₂CO₃ (2.342 g, 7.19 mmol) were added to a degassed solution of tert-butyl azetidin-3-ylcarbamate hydrochloride (0.500 g, 2.396 mmol) and 1-bromo-4-iodobenzene (1.356 g, 4.79 mmol) in dioxane (10.0 mL). The reaction mixture was stirred at 100 °C for 15 h. The reaction mixture was diluted with ethyl acetate, filtered and washed with excess ethyl acetate, combined organic layers were washed with water, brine, dried over sodium sulphate and concentrated to afford crude compound. The crude compound was purified by ISCO using 40 g silica column, compound was eluted with 30% ethyl acetate in pet ether, the fractions were collected and concentrated to get tert-butyl (1-(4-bromophenyl)azetidin-3-yl)carbamate (500 mg, 1.528 mmol, 64 % yield) as a light yellow solid. LCMS Retention time 1.52 min [A], MS (E⁺) m/z: 273.1 [M-tBu+H].

### Intermediate 405B: tert-butyl (1-(4-(4-isopropyl-5-(8-methyl-[1,2,4]triazolo[1,5-a] pyridin-6-yl)-1-((2-(trimethylsilyl)ethoxy)methyl)-1H-pyrazol-3-yl)phenyl)azetidin-3-yl)carbamate

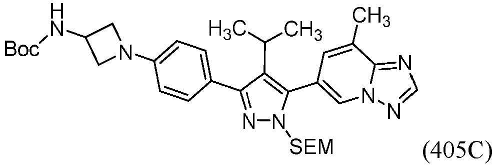

tert-butyl (1-(4-(4-isopropyl-5-(8-methyl-[1,2,4]triazolo[1,5-a]pyridin-6-yl)-1-((2-(trimethyl silyl) ethoxy)methyl)-1H-pyrazol-3-yl)phenyl)azetidin-3-yl)carbamate (260 mg, 0.421 mmol, 34% yield) was prepared according to the general process described in Intermediate 307B, using tert-butyl (1-(4-bromophenyl)azetidin-3-yl)carbamate (0.400 g, 1.222 mmol) and 6-(4-isopropyl-3-(4,4,5,5-tetramethyl-1,3,2-dioxaborolan-2-yl)-1-((2-(trimethylsilyl)ethoxy)methyl)-1H-pyrazol-5-yl)-8-methyl-[1,2,4]triazolo[1,5-a]pyridine (0.791 g, 1.589 mmol) as a starting intermediate to yield the title compound as a gummy solid. LCMS Retention time: 1.80 min [A], MS (E⁺) m/z: 618.5 [M+H].

### Intermediate 405C: 1-(4-(4-isopropyl-5-(8-methyl-[1,2,4]triazolo[1,5-a]pyridin-6-yl)-1H-pyrazol-3-yl)phenyl)azetidin-3-amine

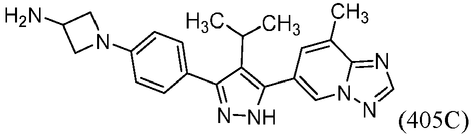

1-(4-(4-isopropyl-5-(8-methyl-[1,2,4]triazolo[1,5-a]pyridin-6-yl)-1H-pyrazol-3-yl) phenyl) azetidin-3-amine (1.2 mg) was prepared according to the general procedure described in Intermediate 307D using tert-butyl (1-(4-(4-isopropyl-5-(8-methyl-[1,2,4]triazolo[1,5-a]pyridin-6-yl)-1-((2-(trimethylsilyl)ethoxy)methyl)-1H-pyrazol-3-yl)phenyl)azetidin-3-yl)carbamate (250 mg, 0.405 mmol) as a starting intermediate to yield the title compound as an off-white solid. LCMS Retention time: 1.21 min [E], MS (E⁺) m/z: 388.3 [M+H]; ¹H NMR (400 MHz, METHANOL-d₄) δ 8.62 (s, 1H), 8.36 (s, 1H), 7.54 (s, 1H), 7.31-7.22 (m, *J*=8.6 Hz, 2H), 6.61-6.46 (m, *J*=8.6 Hz, 2H), 4.21-4.11 (m, 2H), 4.07-4.00 (m, 1H), 3.76 (dd, *J*=8.6, 4.6 Hz, 2H), 3.27-3.24 (m, 1H), 2.97 (dt, *J*=14.6, 7.5 Hz, 1H), 2.59 (s, 3H), 1.94 (s, 1H), 1.26-1.16 (m, 2H), 1.11-1.03 (m, 6H).

### Example 405: 1-(4-(4-isopropyl-5-(8-methyl-[1,2,4]triazolo[1,5-a]pyridin-6-yl)-1H-pyrazol-3-yl)phenyl)-N,N-dimethylazetidin-3-amine

1-(4-(4-isopropyl-5-(8-methyl-[1,2,4]triazolo[1,5-a]pyridin-6-yl)-1H-pyrazol-3-yl)phenyl)-N,N-dimethylazetidin-3-amine (0.9 mg) was prepared according to the general procedure described in Example 307, using 1-(4-(4-isopropyl-5-(8-methyl-[1,2,4]triazolo[1,5-a]pyridin-6-yl)-1H-pyrazol-3-yl)phenyl) azetidin-3-amine (30 mg, 0.077 mmol) as a starting intermediate to yield the title compound as a white solid. LCMS Retention time: 1.58 min [E], MS (E⁺) m/z: 416.3 [M+H]; ¹H NMR (400 MHz, DMSO-d₆) δ 7.92 (s, 1H), 7.66 (s, 1H), 6.86 (s, 1H), 6.54 (d, *J*=8.3 Hz, 2H), 5.83 (d, *J*=8.1 Hz, 2H), 3.29 (t, *J*=7.3 Hz, 2H), 2.95 (t, *J*=6.7 Hz, 2H), 2.62-2.57 (m, 1H), 2.56 (s, 2H), 2.32-2.23 (m, 1H), 1.94-1.83 (m, 4H), 1.51 (s, 6H), 1.25 (s, 1H), 1.18 (s, 1H), 0.50 (br. s., 2H), 0.45 (br. s., 1H), 0.37 (d, *J*=7.1 Hz, 7H), 0.27 (br. s., 1H), 0.09 (br. s., 1H).

The following Examples were prepared according to the general procedure used to prepare Example 405C.

**TABLE 41**

| Ex. No. | Structure | LCMS MH⁺ | RT (min) | HPLC Method |
|---|---|---|---|---|
| 406 | 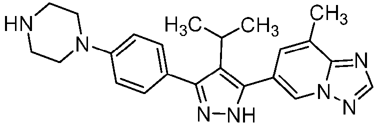 | 402.3 | 1.02 | E |
| 407 | 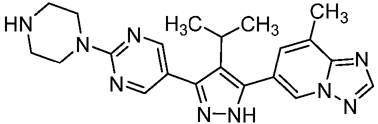 | 404.3 | 0.95 | C |

The following Examples were prepared according to the general procedure used to prepare Example 405.

**TABLE 42**

| Ex. No. | Structure | LCMS MH⁺ | RT (min) | HPLC Method |
|---|---|---|---|---|
| 408 | 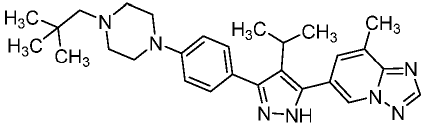 | 472.4 | 2.48 | C |
| 409 | 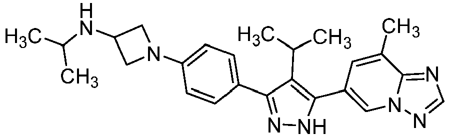 | 430.3 | 1.55 | C |
| 410 | 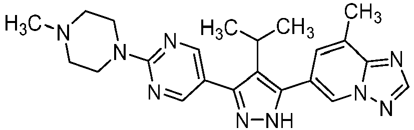 | 418.3 | 1.24 | C |
| 411 | 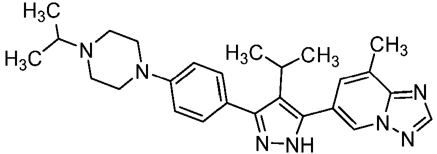 | 444.3 | 1.47 | E |
| 412 | 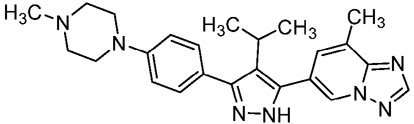 | 416.3 | 1.33 | E |
| 413 | 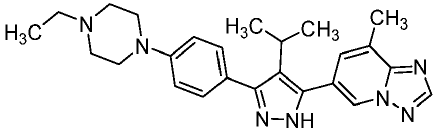 | 430.3 | 1.4 | E |
| 414 | 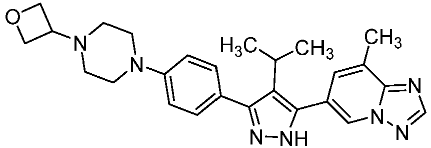 | 458.3 | 1.47 | E |
| 415 | 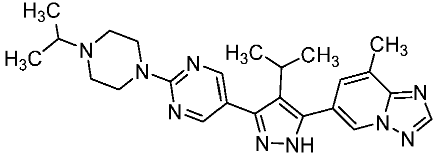 | 446.3 | 1.01 | E |
| 416 | 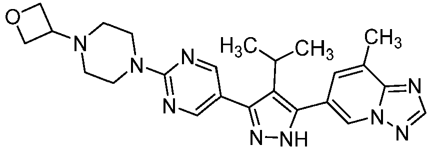 | 460.3 | 1.27 | E |

### EXAMPLE 417

### 6-(4-isopropyl-3-(4-(4-(2-(methylsulfonyl)ethyl)piperazin-1-yl)phenyl)-1H-pyrazol-5-yl)-8-methyl-[1,2,4]triazolo[1,5-a]pyridine

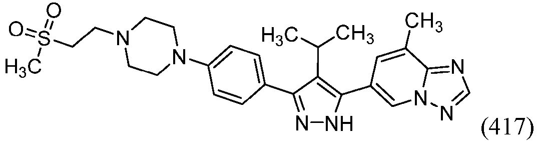

1-Chloro-2-(methylsulfonyl)ethane (0.053 g, 0.374 mmol) and DIPEA (0.065 mL, 0.374 mmol) were added to a solution of 6-(4-isopropyl-3-(4-(piperazin-1-yl)phenyl)-1H-pyrazol-5-yl)-8-methyl-[1,2,4]triazolo[1,5-a]pyridine (0.030 g, 0.075 mmol) in DMF (1.0 mL) and THF (1.0 mL) at 0 °C. The reaction mixture was stirred at 90 °C for 16 h. The reaction mass was purified by Prep LCMS using method AB, the fractions were collected and dried via centrifugal evaporation to get 6-(4-isopropyl-3-(4-(4-(2-(methylsulfonyl) ethyl)piperazin-1-yl)phenyl)-1H-pyrazol-5-yl)-8-methyl-[1,2,4]triazolo[1,5-a]pyridine (2.6 mg) as an off-white solid. LCMS Retention time: 1.03 min [D], MS (E⁺) m/z: 508.3 [M+H]; ¹H NMR (400 MHz, METHANOL-d₄) δ 8.73 (br. s., 1H), 8.48 (d, *J*=1.5 Hz, 1H), 7.66 (br. s.,1H), 7.55-7.37 (m, 2H), 7.17 (d, *J*=6.5 Hz, 2H), 3.76-3.70 (m, 2H), 3.69-3.64 (m, 2H), 3.57(br. s., 4H), 3.48 (br. s., 5H), 3.18-3.13 (m, 3H), 3.07 (d, *J*=9.5 Hz, 2H), 2.70 (br. s., 3H), 1.38(br. s., 1H), 1.35-1.27 (m, 3H), 1.20-1.12 (m, 7H), 1.08 (br. s., 1H), 0.89 (br. s., 1H).

The following Examples were prepared according to the general procedure used to prepare Example 417.

**TABLE 43**

| Ex. No. | Structure | LCMS MH⁺ | RT (min) | HPLC Method |
|---|---|---|---|---|
| 418 | 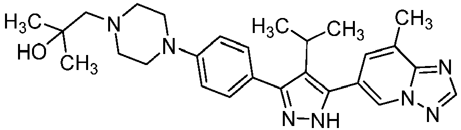 | 474.3 | 1.61 | C |
| 419 | 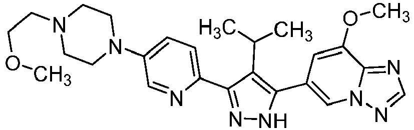 | 477.3 | 1.4 | C |

### EXAMPLE 420

### 1-(4-(4-isopropyl-5-(8-methyl-[1,2,4]triazolo[1,5-a]pyridin-6-yl)-1H-pyrazol-3-yl) phenyl)-4-methylpiperazin-2-one

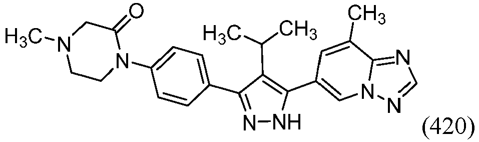

### Intermediate 420A: Tert-butyl 4-(4-bromophenyl)-3-oxopiperazine-1-carboxylate

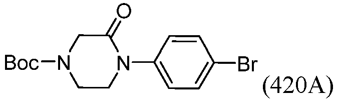

Copper (I) iodide (0.135 g, 0.707 mmol), N,N'-dimethylethylenediamine (0.062 g, 0.707 mmol) and K₃PO₄ (2.155 g, 12.37 mmol) were added to a degassed solution of 1-bromo-4-iodobenzene (1.0 g, 3.53 mmol) and tert-butyl 3-oxopiperazine-1-carboxylate (0.708 g, 3.53 mmol) in DMF (20.0 mL). The mixture was stirred at 100 °C for 14 h in a sealed tube. The reaction mixture was extracted with ethyl acetate, washed with water, brine, dried over sodium sulphate and concentrated to get crude compound. The crude compound was purified by ISCO using 40 g silica column, compound was eluted with 45% ethyl acetate in pet ether, the fractions were collected and concentrated to get tert-butyl 4-(4-bromophenyl)-3-oxopiperazine-1-carboxylate (420 mg, 1.182 mmol, 33% yield) as a light brown semi-solid. LCMS Retention time: 1.21 min [A], MS (E⁺) m/z: 357.4 [M+2H].

### Intermediate 420B: tert-butyl 4-(4-(4-isopropyl-5-(8-methyl-[1,2,4]triazolo[1,5-a] pyridin-6-yl)-1-((2-(trimethylsilyl)ethoxy)methyl)-1H-pyrazol-3-yl)phenyl)-3-oxopiperazine-1-carboxylate

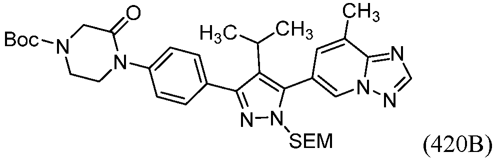

tert-Butyl 4-(4-(4-isopropyl-5-(8-methyl-[1,2,4]triazolo[1,5-a]pyridin-6-yl)-1-((2-(trimethylsilyl)ethoxy)methyl)-1H-pyrazol-3-yl)phenyl)-3-oxopiperazine-1-carboxylate (220 mg, 0.341 mmol, 34 % yield) was prepared according to the general procedure described in Intermediate 307B using tert-butyl 4-(4-bromophenyl)-3-oxopiperazine-1-carboxylate (0.357 g, 1.005 mmol) and 6-(4-isopropyl-3-(4,4,5,5-tetramethyl-1,3,2-dioxaborolan-2-yl)-1-((2-(trimethylsilyl)ethoxy) methyl)-1H-pyrazol-5-yl)-8-methyl-[1,2,4] triazolo[1,5-a]pyridine (0.500 g, 1.005 mmol) as a starting intermediate to yield the title compound as an off-white solid. LCMS Retention time: 1.58 min [A], MS (E⁺) m/z: 646.4 [M+H].

### Intermediate 420C: 1-(4-(4-isopropyl-5-(8-methyl-[1,2,4]triazolo[1,5-a]pyridin-6-yl)-1H-pyrazol-3-yl)phenyl)piperazin-2-one

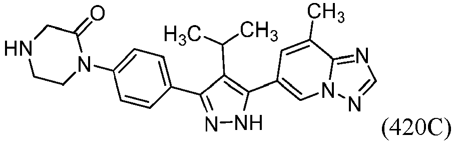

1-(4-(4-isopropyl-5-(8-methyl-[1,2,4]triazolo[1,5-a]pyridin-6-yl)-1H-pyrazol-3-yl)phenyl) piperazin-2-one (5.4 mg) was prepared according to the general procedure described in Intermediate 307D using tert-butyl 4-(4-(4-isopropyl-5-(8-methyl-[1,2,4]triazolo[1,5-a]pyridin-6-yl)-1-((2-(trimethylsilyl)ethoxy)methyl)-1H-pyrazol-3-yl)phenyl)-3-oxopiperazine-1-carboxylate (0.210 g, 0.325 mmol) as a starting intermediate to yield the title compound as a gummy solid. LCMS Retention time: 1.063 min [E], MS (E⁺) m/z: 416.2 [M+H]; ¹H NMR (400 MHz, METHANOL-d₄) δ 8.77 (br. s., 1H), 8.48 (s, 1H), 7.75-7.56 (m, 3H), 7.50 (d, *J*=8.3 Hz, 2H), 3.89-3.72 (m, 2H), 3.67 (s, 2H), 3.18-3.06 (m, 1H), 2.71 (s, 3H), 1.21 (d, *J*=7.1 Hz, 6H).

### Example 420: 1-(4-(4-isopropyl-5-(8-methyl-[1,2,4]triazolo[1,5-a]pyridin-6-yl)-1H-pyrazol-3-yl)phenyl)-4-methylpiperazin-2-one

1-(4-(4-isopropyl-5-(8-methyl-[1,2,4]triazolo[1,5-a]pyridin-6-yl)-1H-pyrazol-3-yl)phenyl)-4-methylpiperazin-2-one (2.3 mg) was prepared according to the general procedure described in Example 307, using 1-(4-(4-isopropyl-5-(8-methyl-[1,2,4]triazolo[1,5-a]pyridin-6-yl)-1H-pyrazol-3-yl)phenyl)piperazin-2-one (30 mg, 0.072 mmol) as a starting intermediate to yield the title compound as a white solid. LCMS Retention time: 1.21 min [E], MS (E⁺) m/z: 430.2 [M+H]; ¹H NMR (400 MHz, METHANOL-d₄) δ 8.77 (s, 1H), 8.49 (s, 1H), 7.77-7.59 (m, 3H), 7.54 (d, *J*=8.5 Hz, 2H), 4.24-3.98 (m, 4H), 3.76 (t, *J*=5.3 Hz, 2H), 3.18-3.05 (m, 4H), 3.01 (s, 1H),2.80-2.64 (m, 4H), 1.21 (d, *J*=7.0 Hz, 7H).

The following Examples were prepared according to the general procedure used to prepare Example 420.

**TABLE 44**

| Ex. No. | Structure | LCMS MH⁺ | RT (min) | HPLC Method |
|---|---|---|---|---|
| 421 | 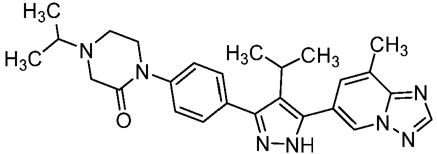 | 458.3 | 1.45 | C |
| 422 | 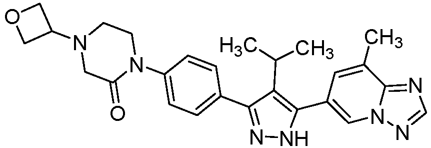 | 472.3 | 1.16 | C |

### EXAMPLE 423

### 6-(3-(5-(4-cyclobutylpiperazin-1-yl)pyridin-2-yl)-4-isopropyl-1H-pyrazol-5-yl)-8-methoxy-[1,2,4]triazolo[1,5-a]pyridine

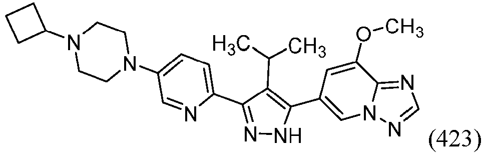

### Intermediate 423A: tert-butyl 4-(pyridin-3-yl)piperazine-1-carboxylate

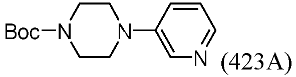

Pd₂(dba)₃ (0.869 g, 0.949 mmol) and Xantphos (1.099 g, 1.899 mmol) were added to a degassed solution of 3-bromopyridine (3.0 g, 18.99 mmol), tert-butyl piperazine-1-carboxylate (3.54 g, 18.99 mmol) and sodium tert-butoxide (5.47 g, 57.0 mmol) in dioxane (75 mL). The mixture was stirred at110 °C for 16 h in a sealed tube. The reaction mixture was diluted with ethyl acetate, washed with water, brine, dried over sodium sulphate and concentrated to afford the crude compound. The crude compound was purified by ISCO using 40 g silica column, the compound was eluted with 50-65% ethyl acetate and pet ether, the fractions were collected and concentrated to yield tert-butyl 4-(pyridin-3-yl)piperazine-1-carboxylate (3.5 g, 13.29 mmol, 70% yield) as a light brown solid. LCMS Retention time: 1.28 min [A], MS (E⁺) m/z: 264.4 [M+2H].

### Intermediate 423B: tert-butyl 4-(6-bromopyridin-3-yl)piperazine-1-carboxylate

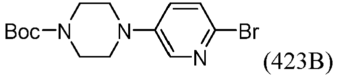

To a solution of tert-butyl 4-(pyridin-3-yl) piperazine-1-carboxylate (2.2 g, 8.35 mmol) in acetonitrile (50 mL) was added NBS (1.487 g, 8.35 mmol) at 0 °C. The mixture was stirred at the same temperature for 1 h. The reaction mixture was quenched slowly with 5% NaOH aqueous solution, extracted with ethyl acetate, washed with water, brine, dried over sodium sulphate and concentrated to yield the crude compound. The crude compound was purified by ISCO using 40 g silica column, compound was eluted with 25-30% ethyl acetate in pet ether, the fractions were collected and concentrated to afford tert-butyl 4-(6-bromopyridin-3-yl)piperazine-1-carboxylate (1.6 g, 4.68 mmol, 56.0 % yield) as a pale brown solid. LCMS Retention time: 1.66 min [A], MS (E⁺) m/z: 344.4 [M+2H].

### Intermediate 423C: tert-butyl 4-(6-(4-isopropyl-5-(8-methoxy-[1,2,4]triazolo[1,5-a]pyridin-6-yl)-1-((2-(trimethylsilyl)ethoxy)methyl)-1H-pyrazol-3-yl)pyridin-3-yl)piperazine-1-carboxylate

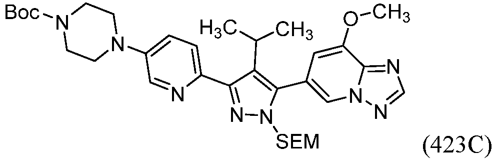

tert-butyl 4-(6-(4-isopropyl-5-(8-methoxy-[1,2,4]triazolo[1,5-a]pyridin-6-yl)-1-((2-(trimethylsilyl)ethoxy)methyl)-1H-pyrazol-3-yl)pyridin-3-yl)piperazine-1-carboxylate (620 mg, 0.955 mmol, 47% yield) was prepared according to the general procedure described in Intermediate 307B, using tert-butyl 4-(6-bromopyridin-3-yl)piperazine-1-carboxylate (0.700 g, 2.045 mmol) and 6-(4-isopropyl-3-(4,4,5,5-tetramethyl-1,3,2-dioxaborolan-2-yl)-1-((2-(trimethylsilyl)ethoxy) methyl)-1H-pyrazol-5-yl)-8-methoxy-[1,2,4]triazolo[1,5-a]pyridine (1.681 g, 3.27 mmol) as starting intermediates to yield the title compound as an off-white solid. LCMS Retention time: 1.46 min [A], MS (E⁺) m/z: 649.6 [M+H].

### Intermediate 423D: 6-(4-isopropyl-3-(5-(piperazin-1-yl)pyridin-2-yl)-1H-pyrazol-5-yl)-8-methoxy-[1,2,4]triazolo[1,5-a]pyridine

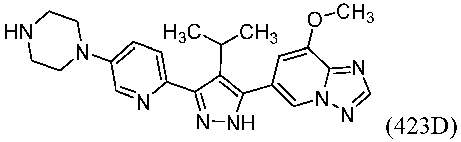

6-(4-isopropyl-3-(5-(piperazin-1-yl)pyridin-2-yl)-1H-pyrazol-5-yl)-8-methoxy-[1,2,4]triazolo [1,5-a]pyridine (18.7 mg) was prepared according to the general procedure described in Intermediate 307 B using tert-butyl 4-(6-(4-isopropyl-5-(8-methoxy-[1,2,4]triazolo[1,5-a]pyridin-6-yl)-1-((2-(trimethylsilyl) ethoxy)methyl)-1H-pyrazol-3-yl) pyridin-3-yl)piperazine-1-carboxylate (730 mg, 1.125 mmol) as a starting intermediate to yield the title compound as a white solid. LCMS Retention time: 0.96min [C] MS (E⁺) m/z: 419.3 [M+H]; ¹H NMR (400 MHz, METHANOL-d₄) δ = 8.53 (s, 1H), 8.49 (br d, J=1.2 Hz, 1H), 8.44 (s, 1H), 7.71-7.62 (m, 1H), 7.61-7.51 (m, 1H), 7.30-7.17 (m, 1H),4.14 (s, 3H), 3.64-3.54 (m, 4H), 3.49-3.40 (m, 4H), 3.39-3.34 (m, 1H), 1.27 (d, J=7.3 Hz, 6H).

### Example 423: 6-(3-(5-(4-cyclobutylpiperazin-1-yl)pyridin-2-yl)-4-isopropyl-1H-pyrazol-5-yl)-8-methoxy-[1,2,4]triazolo[1,5-a]pyridine

6-(3-(5-(4-cyclobutylpiperazin-1-yl)pyridin-2-yl)-4-isopropyl-1H-pyrazol-5-yl)-8-methoxy-[1,2,4]triazolo[1,5-a]pyridine (12.3 mg) was prepared according to the general procedure described in Example 307 using 6-(4-isopropyl-3-(5-(piperazin-1-yl)pyridin-2-yl)-1H-pyrazol-5-yl)-8-methoxy-[1,2,4]triazolo [1,5-a]pyridine (25.0 mg, 0.060 mmol) and cyclobutanone (12.56 mg, 0.179 mmol) as starting intermediates to yield the title compound as a white solid. LCMS Retention time: 1.65 min [C], MS (E⁺) m/z: 473.3 [M+H]; ¹H NMR (400 MHz, METHANOL-d₄) δ 8.52 (s, 1H), 8.46-8.34 (m, 2H), 7.61-7.54 (m, 1H), 7.53-7.45 (m, 1H), 7.24 (s, 1H), 4.14 (s, 3H), 3.44-3.38 (m,4H), 3.31-3.26 (m, 1H), 2.99-2.90 (m, 1H), 2.70-2.59 (m, 4H), 2.23-2.11 (m, 2H), 2.08-1.96 (m, 2H), 1.88-1.75 (m, 2H), 1.26 (d, J=7.1 Hz, 6H).

The following Example was prepared according to the general procedure used to prepare Example 423D.

**TABLE 45**

| Ex. No. | Structure | LCMS MH⁺ | RT (min) | HPLC Method |
|---|---|---|---|---|
| 424 | 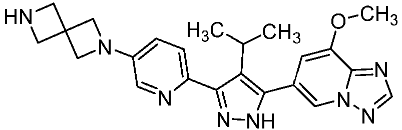 | 431.2 | 0.93 | C |

The following Examples were prepared according to the general procedure used to prepare Example 423.

**TABLE 46**

| Ex. No. | Structure | LCMS MH⁺ | RT (min) | HPLC Method |
|---|---|---|---|---|
| 425 | 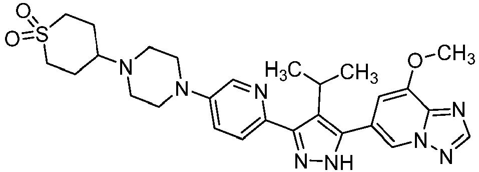 | 551.3 | 1.3 | C |
| 426 | 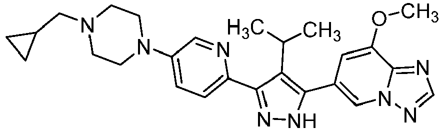 | 473.3 | 1.54 | C |
| 427 | 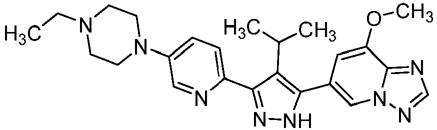 | 447.3 | 1.33 | C |
| 428 | 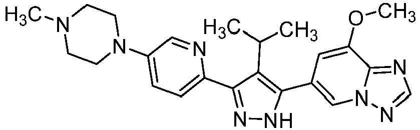 | 433.3 | 1.25 | C |
| 429 | 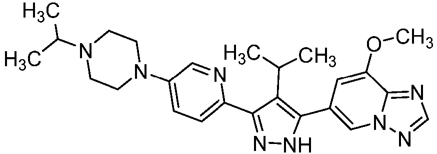 | 461.3 | 1.39 | C |
| 430 | 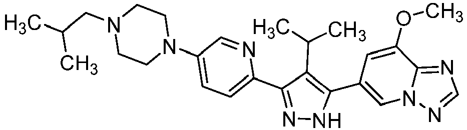 | 475.3 | 1.94 | C |
| 431 | 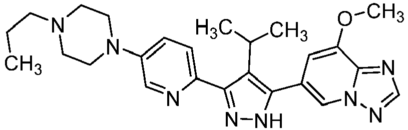 | 461.3 | 1.56 | C |
| 432 | 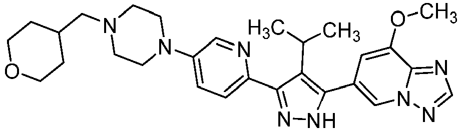 | 517.3 | 1.58 | C |
| 433 | 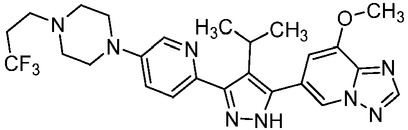 | 515.3 | 1.78 | C |
| 434 | 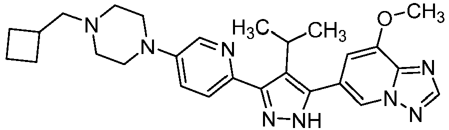 | 487.3 | 0.98 | C |
| 435 | 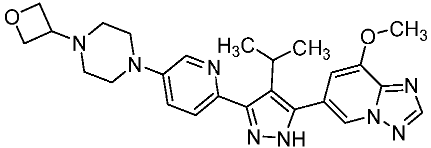 | 475.3 | 0.79 | C |
| 436 | 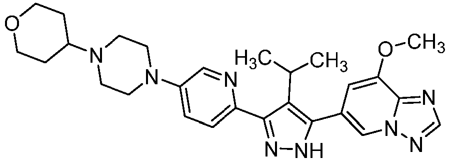 | 503.3 | 0.83 | C |
| 437 | 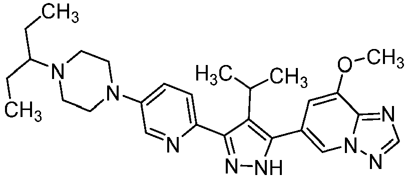 | 489.3 | 2.1 | C |
| 438 | 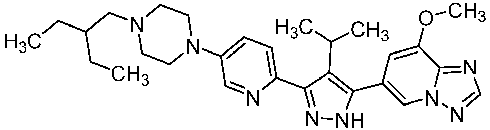 | 503.3 | 2.35 | C |
| 439 | 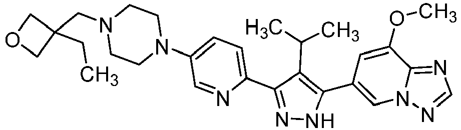 | 517.3 | 1.73 | C |
| 440 | 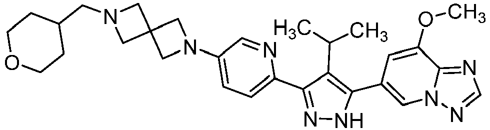 | 529.3 | 1.27 | C |
| 441 | 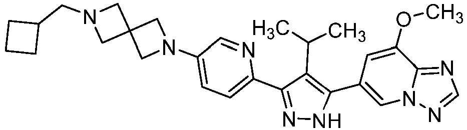 | 499.3 | 1.4 | C |
| 442 | 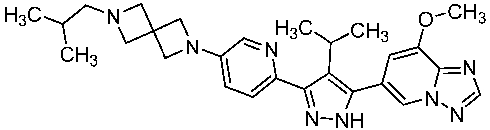 | 487.3 | 1.38 | C |
| 443 | 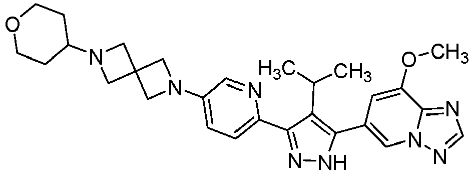 | 515.3 | 1.28 | C |
| 444 | 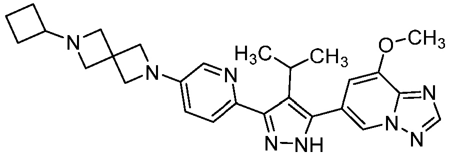 | 485.3 | 1.32 | C |
| 445 | 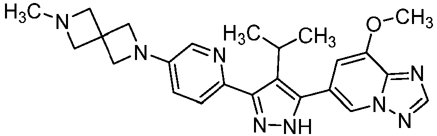 | 445.2 | 1.01 | C |
| 446 | 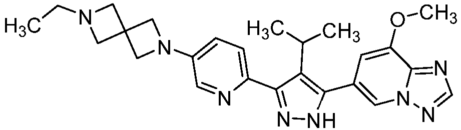 | 459.2 | 1.09 | C |
| 447 | 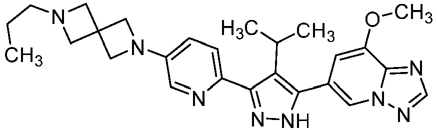 | 473.2 | 1.2 | C |
| 448 | 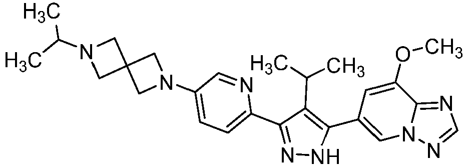 | 473.3 | 1.32 | C |
| 449 | 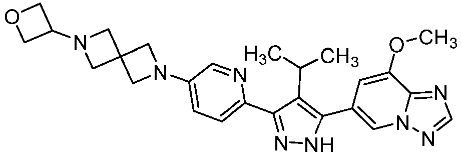 | 487.2 | 1.24 | C |
| 450 | 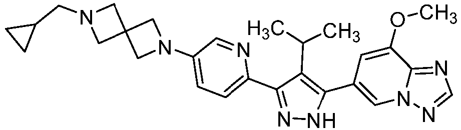 | 485.3 | 1.22 | C |

### EXAMPLE 451

### 2-(dimethylamino)-1-(4-(6-(4-isopropyl-5-(8-methoxy-[1,2,4]triazolo[1,5-a]pyridin-6-yl)-1H-pyrazol-3-yl)pyridin-3-yl)piperazin-1-yl)ethan-1-one

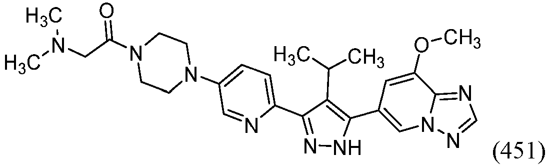

2-(Dimethylamino)-1-(4-(6-(4-isopropyl-5-(8-methoxy-[1,2,4]triazolo[1,5-a] pyridin-6-yl)-1H-pyrazol-3-yl)pyridin-3-yl)piperazin-1-yl)ethan-1-one (19.4 mg) was prepared according to the general procedure described in Example 323, using 6-(4-isopropyl-3-(5-(piperazin-1-yl)pyridin-2-yl)-1H-pyrazol-5-yl)-8-methoxy-[1,2,4]triazolo[1,5-a]pyridine (25.0 mg, 0.060 mmol) as a starting intermediate to get the title compound as an off-white solid. LCMS Retention time: 1.15 min [F], MS (E⁺) m/z: 504.3 [M+H]; ¹H NMR (400 MHz, METHANOL-d₄) δ 8.56 (d, J=1.2 Hz, 1H), 8.46 (s, 1H), 8.42 (d, *J*=2.0 Hz, 1H), 7.90-7.78 (m, 2H), 7.21 (d, *J*=1.0 Hz, 1H), 4.36 (s,2H), 4.14 (s, 3H), 3.91-3.85 (m, 2H), 3.70-3.66 (m, 2H), 3.58-3.49 (m, 4H), 3.30 (d, *J*=6.8 Hz, 1H), 3.00 (s, 6H), 1.28 (d, *J*=7.1 Hz, 6H).

The following Examples were prepared according to the general procedure used to prepare Example 451.

**TABLE 47**

| Ex. No. | Structure | LCMS MH⁺ | RT (min) | HPLC Method |
|---|---|---|---|---|
| 452 | 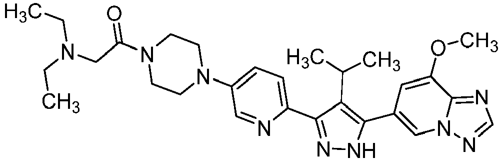 | 532.3 | 1.24 | C |
| 453 | 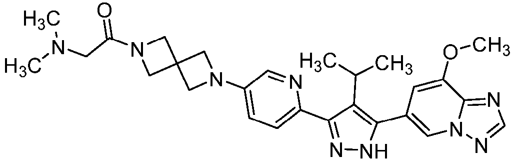 | 516.3 | 1.15 | C |

### EXAMPLE 454

### 6-(4-isopropyl-3-(5-(4-(2-(methylsulfonyl)ethyl)piperazin-1-yl)pyridin-2-yl)-1H-pyrazol-5-yl)-8-methoxy-[1,2,4]triazolo[1,5-a]pyridine

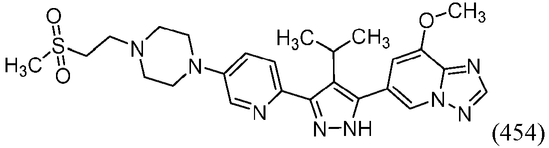

To a solution of 6-(4-isopropyl-3-(5-(piperazin-1-yl)pyridin-2-yl)-1H-pyrazol-5-yl)-8-methoxy-[1,2,4]triazolo[1,5-a]pyridine (25.0 mg, 0.060 mmol), 1-chloro-2-(methylsulfonyl)ethane (12.78 mg, 0.090 mmol) in DMF (1.0 mL) and THF (1.0 mL) was added TEA (0.042 mL, 0.299 mmol) at room temperature. The reaction mixture was stirred for 16 h. The reaction mass was purified via preparative LC/MS using method AA, the fractions containing the product were combined and dried via centrifugal evaporation to get 6-(4-isopropyl-3-(5-(4-(2-(methylsulfonyl)ethyl)piperazin-1-yl) pyridin-2-yl)-1H-pyrazol-5-yl)-8-methoxy-[1,2,4]triazolo[1,5-a]pyridine (17.6 mg) as a white solid. LCMS Retention time: 1.29 min [E], MS (E⁺) m/z: 525.3 [M+H]; ¹H NMR (400 MHz, METHANOL-d₄) δ 8.52 (s, 1H), 8.43 (s, 1H), 8.41 (br d, *J*=2.7 Hz, 1H), 7.61-7.54 (m, 1H), 7.52-7.46 (m, 1H), 7.24 (s, 1H), 4.14 (s,3H), 3.44-3.36 (m, 6H), 3.29 (br s, 1H), 3.11 (s, 3H), 2.97 (t, *J*=6.7 Hz, 2H), 2.82-2.71 (m, 4H), 2.68 (s, 1H), 1.31 (s, 1H), 1.26 (d, *J*=7.1 Hz, 6H).

The following Examples were prepared according to the general procedure used to prepare Example 454.

**TABLE 48**

| Ex. No. | Structure | LCMS MH⁺ | RT (min) | HPLC Method |
|---|---|---|---|---|
| 455 | 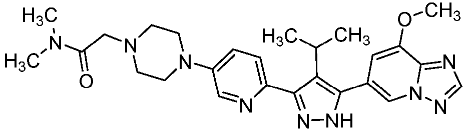 | 504.3 | 1.31 | C |
| 456 | 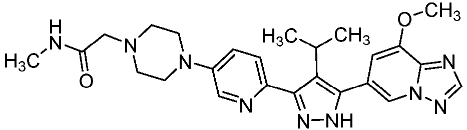 | 490.2 | 1.25 | C |
| 457 | 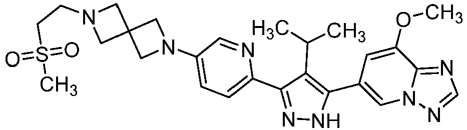 | 537.2 | 1.37 | C |
| 458 | 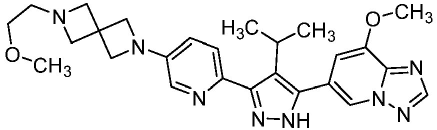 | 489.2 | 1.18 | C |

### EXAMPLE 459

### 2-(4-(4-isopropyl-5-(8-methyl-[1,2,4]triazolo[1,5-a]pyridin-6-yl)-1H-pyrazol-3-yl) phenyl)-4-methylmorpholine

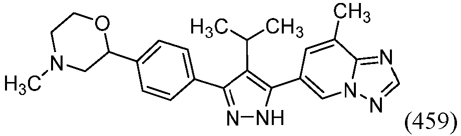

### Intermediate 459A: 2-(4-nitrophenyl)oxirane

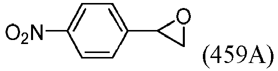

To a solution of 2-bromo-1-(4-nitrophenyl)ethanone (10.503 g, 43.0 mmol) in MeOH (100 mL) (compound not dissolved completely) was added NaBH₄ (2.035 g, 53.8 mmol) portion wise at 0 °C (observed gas evolution and then it became clear solution). The reaction mixture was stirred at the same temperature for 5 min. After stirring at room temperature for 2 h, K₂CO₃ (6.54 g, 47.3 mmol) was added portion wise. The suspension was stirred at room temperature for 6 h. The reaction mass was concentrated, the residue was diluted with water (100 mL), extracted with DCM (2 X 150 mL), the combined organic extracts were dried (Na₂SO₄) and concentrated to get 2-(4-nitrophenyl)oxirane (6.63 g, 40.1 mmol, 93 % yield) as a pale yellow solid. ¹H NMR (300 MHz, CDCl₃) δ ppm 8.23 (d, J = 9 Hz, 1 H), 7.46 (d, J = 9 Hz, 1 H), 3.99-3.97 (m, 1 H), 3.25-3.21 (m, 1 H), 2.79-2.76 (m, 1 H).

### Intermediate 459B: 2-((2-hydroxyethyl)amino)-1-(4-nitrophenyl)ethanol

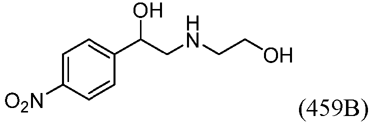

2-(4-nitrophenyl)oxirane (6.256 g, 37.9 mmol) in ethanolamine (100.00 mL) was stirred at 40 °C for 2 h. TLC showed no starting material and formation of a new polar spot. The reaction mixture was diluted with water (100 mL) and EtOAc (100 mL). The two layers were separated. The aqueous layer was extracted with EtOAc (2 X 100 mL), the combined organic extracts were washed with water (100 mL), brine (20 mL), dried (Na₂SO₄) and concentrated to get the crude compound. The crude compound was triturated with acetonitrile (3 X 20 mL) to get 2-((2-hydroxyethyl)amino)-1-(4-nitrophenyl)ethanol (4.65 g, 20.55 mmol, 54.3 % yield) as a white solid. LCMS retention time 0.48 min [G]. MS (E⁻) *m*/*z:* 227.3 [M+H].

### Intermediate 459C: tert-butyl (2-hydroxy-2-(4-nitrophenyl)ethyl)(2-hydroxyethyl) carbamate

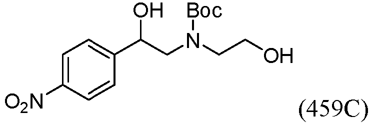

To a solution of 2-((2-hydroxyethyl)amino)-1-(4-nitrophenyl)ethanol (4.62 g, 20.42 mmol) in DCM (60.00 mL) was added TEA (3.42 mL, 24.51 mmol). The reaction mixture was stirred for 5 min. Next, Boc₂O (5.22 mL, 22.46 mmol) was added and dissolved in DCM (5 mL) dropwise at room temperature. The reaction mixture was stirred at the same temperature. Initially the compound was not dissolved completely. After the addition of Boc₂O, the compound was dissolved completely. The reaction mixture was stirred at room temperature for 2 h. The reaction was quenched with the addition of water. The reaction mixture was extracted with DCM, the organic layer was dried (Na₂SO₄) and concentrated to get crude compound. The crude compound was purified by ISCO using 80 g silica column, compound was eluted in 4% MeOH in CHCl₃, the fractions were collected and concentrated to get tert-butyl (2-hydroxy-2-(4-nitrophenyl)ethyl)(2-hydroxyethyl)carbamate (6.6 g, 20.22 mmol, 99 % yield) as a white solid. LCMS retention time 1.00 min [G]. MS (E⁻) *m*/*z:* 327.3 [M + H].

### Intermediate 459D: tert-butyl 2-(4-nitrophenyl)morpholine-4-carboxylate

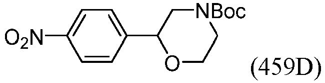

To a solution of tert-butyl (2-hydroxy-2-(4-nitrophenyl)ethyl)(2-hydroxyethyl) carbamate (6.6 g, 20.22 mmol) and triphenylphosphine (6.37 g, 24.27 mmol) in toluene (120.00 mL) was added TEA (7.33 mL, 52.6 mmol) at 0 °C. The reaction mixture was stirred for 5 min. Next, di-tert-butyl azodicarboxylate (5.59 g, 24.27 mmol) dissolved in toluene (20 mL) was added dropwise at the same temperature. The reaction mixture was stirred at room temperature for 16 h. The reaction was quenched with water (50 mL). The layers were separated, the aqueous layer was extracted with EtOAc (2 X 50 mL), the combined organic extracts were dried (Na₂SO₄) and concentrated to get crude compound. The crude compound was purified by ISCO using 80 g silica column, compound was 25% EA in hexane, the fraction was collected and concentrated to get compound as a gummy solid. The gummy solid compound was triturated with hexane (2 X 20 mL) and then the solid was dried under vacuum to get tert-butyl 2-(4-nitrophenyl)morpholine-4-carboxylate (4.2 g, 13.62 mmol, 67 % yield) as a white solid. (Product and reagent are coming in same solvent system in column purification and not able to remove from triturating with hexane also). LCMS retention time 2.804 min [H]. MS (E⁻) *m*/*z:* 253.2 [M+H-tBu].

### Intermediate 459E: tert-butyl 2-(4-aminophenyl)morpholine-4-carboxylate

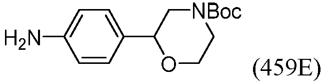

To a solution of tert-butyl 2-(4-nitrophenyl)morpholine-4-carboxylate (4.2 g, 13.62 mmol) in MeOH (75 mL) was added Pd/C (1.450 g, 13.62 mmol). The reaction mixture was stirred at room temperature under H₂ bladder for 3 h. The reaction mixture was filtered through celite and concentrated to get crude compound. The crude compound was purified by ISCO, using 80 g silica column, compound was eluted in 35% EA in hexanes, the fractions were collected and concentrated to get tert-butyl 2-(4-aminophenyl)morpholine-4-carboxylate (2.25 g, 8.08 mmol, 59 % yield) as a white solid. LCMS retention time 1.588 min [H]. MS (E⁻) *m*/*z:* 223.0 [M+H-tBu].

### Intermediate 459F: Tert-butyl 2-(4-bromophenyl)morpholine-4-carboxylate

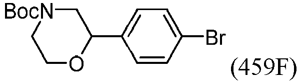

To a solution of copper(II) bromide (0.241 g, 1.078 mmol) in acetonitrile (5 mL) were added t-butyl nitrite (0.148 g, 1.437 mmol) and tert-butyl 2-(4-aminophenyl) morpholine-4-carboxylate (0.2 g, 0.719 mmol) at 0 °C. The resulting solution was stirred at room temperature for 12 h. The reaction mass was filtered through celite, washed with EtOAc (50 mL) and filtrate was concentrated to get crude product. The crude compound was purified by ISCO using 24 g silica column by eluting with 9% EtOAc\Pet ether, the fractions were collected and concentrated to get tert-butyl 2-(4-bromophenyl) morpholine-4-carboxylate (125 mg, 0.365 mmol, 50.8 % yield) as a white oil. LCMS Retention time 3.52 min [D], MS (E⁻) *m*/*z:* 243.2 [M+H-Boc].

### Intermediate 459G: tert-butyl 2-(4-(4-isopropyl-5-(8-methyl-[1,2,4]triazolo[1,5-a] pyridin-6-yl)-1-((2-(trimethylsilyl)ethoxy)methyl)-1H-pyrazol-3-yl)phenyl)morpholine-4-carboxylate

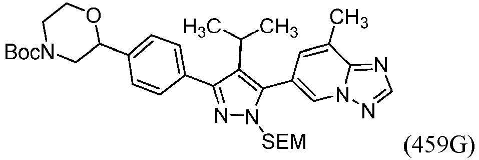

A mixture of 6-(4-isopropyl-3-(4,4,5,5-tetramethyl-1,3,2-dioxaborolan-2-yl)-1-((2-(trimethylsilyl)ethoxy)methyl)-1H-pyrazol-5-yl)-8-methyl-[1,2,4]triazolo[1,5-a] pyridine (0.872 g, 1.753 mmol), tert-butyl 2-(4-bromophenyl)morpholine-4-carboxylate (0.500 g, 1.461 mmol) and K₂CO₃ (0.606 g, 4.38 mmol) in acetonitrile (32.00 mL) and water (8.00 mL) was degassed for 10 min with nitrogen. Next, Pd₂(dba)₃ (0.067 g, 0.073 mmol) and 2-dicyclohexylphosphino-2',6'-dimethoxybiphenyl (0.060 g, 0.146 mmol) were added and the reaction mixture was stirred for 5 min. The reaction mixture was stirred at 110 °C for 16 h. The two layers were separated, the aqueous layer was extracted with DCM (2 X 30 mL), the combined organic extracts were dried (Na₂SO₄) and concentrated to get crude compound. The crude compound was purified by ISCO using 24 g silica column, compound was eluted in 50% EA in hexane, the fractions were collected and concentrated to get tert-butyl 2-(4-(4-isopropyl-5-(8-methyl-[1,2,4]triazolo[1,5-a]pyridin-6-yl)-1-((2-(trimethylsilyl)ethoxy) methyl)-1H-pyrazol-3-yl)phenyl)morpholine-4-carboxylate (0.627 g, 0.991 mmol, 68 % yield) as a gummy solid. LCMS Retention time 1.93 min [A], MS (E⁻) *m*/*z:* 633.8 [M+H].

### Intermediate 459H: 2-(4-(4-isopropyl-5-(8-methyl-[1,2,4]triazolo[1,5-a]pyridin-6-yl)-1H-pyrazol-3-yl)phenyl)morpholine.

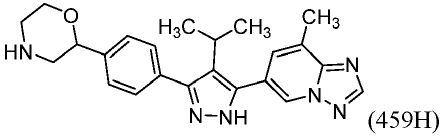

To a solution of tert-butyl 2-(4-(4-isopropyl-5-(8-methyl-[1,2,4]triazolo[1,5-a] pyridin-6-yl)-1-((2-(trimethylsilyl)ethoxy)methyl)-1H-pyrazol-3-yl)phenyl)morpholine-4-carboxylate (0.900 g, 1.422 mmol) in dioxane (5.00 mL) was added 4 M hydrochloric acid in dioxane (5.00 mL, 20.00 mmol) at room temperature. The reaction mixture was stirred at 70 °C for 16 h. The reaction mass concentrated, triturated with diethyl ether (2 X 10 mL), then dried under vacuum to get crude compound. The crude compound was purified by Prep HPLC using method AC, the fractions contained the compound was collected and concentrated to get 2-(4-(4-isopropyl-5-(8-methyl-[1,2,4]triazolo[1,5-a]pyridin-6-yl)-1H-pyrazol-3-yl)phenyl)morpholine (0.480 g, 1.193 mmol, 84 % yield) as a white solid.

The racemic 2-(4-(4-isopropyl-5-(8-methyl-[1,2,4]triazolo[1,5-a]pyridin-6-yl)-1H-pyrazol-3-yl)phenyl)morpholine was subjected to SFC for enantiomers separation. After chiral SFC purification using method AD, the fractions were collected and concentrated and lyophilized to get: Enantiomer 1, Chiral SFC RT-6.92: (0.150 g, 0.373 mmol, 31 % yield) and Enantiomer 2, Chiral SFC RT-9.69: (0.135 g, 0.335 mmol, 28 % yield) as a white solid. LCMS Retention time 0.77 min [A], MS (E⁻) *m*/*z:* 403.6 [M+H]; ¹H NMR (400 MHz, METHANOL-d₄) δ ppm 8.76 (s, 1H), 8.48 (s, 1H), 7.67 (s, 1H), 7.53 (s, 4H), 4.64 (dd, J=10.8, 2.3 Hz, 1H), 4.15-4.05 (m, 1H), 3.86 (td, J=11.3, 3.5 Hz, 1H), 3.21-3.06 (m, 2H), 3.05-2.94 (m, 2H), 2.87-2.76 (m, 1H), 2.71 (s, 3H), 1.96 (s, 1H), 1.19 (d, J=7.0 Hz, 6H).

### Example 459: 2-(4-(4-isopropyl-5-(8-methyl-[1,2,4]triazolo[1,5-a]pyridin-6-yl)-1H-pyrazol-3-yl)phenyl)-4-methylmorpholine

To a solution of 2-(4-(4-isopropyl-5-(8-methyl-[1,2,4]triazolo[1,5-a]pyridin-6-yl)-1H-pyrazol-3-yl)phenyl)morpholine (0.012 g, 0.030 mmol) in MeOH (1.5 mL) were added formaldehyde in water (0.2 mL, 2.178 mmol) and acetic acid (0.1 mL, 1.747 mmol) at room temperature. The mixture was stirred at the same temperature for 6 h. To the reaction mixture was added sodium cyanoborohydride (9.37 mg, 0.149 mmol) at room temperature. The reaction mixture was stirred at the same temperature for 16 h. The reaction mass was purified by Prep LCMS using method AA, the fractions containing product were combined and dried using Genevac centrifugal evaporator to get 2-(4-(4-isopropyl-5-(8-methyl-[1,2,4]triazolo[1,5-a]pyridin-6-yl)-1H-pyrazol-3-yl)phenyl)-4-methylmorpholine (7.2 mg, 0.016 mmol, 55% yield) as a pale solid. LCMS Retention time 1.48 min [E], MS (E⁻) *m*/*z:* 403.6 [M+H]; ¹H NMR (400 MHz, METHANOL-d₄) δ ppm 8.76 (s, 1H), 8.48 (s, 1H), 7.67 (s, 1H), 7.54 (s, 4H), 4.65 (dd, *J*=10.5, 2.5 Hz, 1H), 4.10 (dd, *J*=11.5, 2.0 Hz, 1H), 3.93-3.83 (m, 1H), 3.15-3.01 (m, 2H), 2.89 (d, *J*=13.6 Hz, 1H), 2.71 (s, 3H), 2.47-2.26 (m, 4H), 2.19 (t, *J*=11.0 Hz, 1H), 1.19 (d, *J*=7.5 Hz, 6H).

The following Examples were prepared according to the general procedure used to prepare Example 459.

**TABLE 49**

| Ex. No. | Structure | LCMS MH⁺ | RT (min) | HPLC Method |
|---|---|---|---|---|
| 460 | 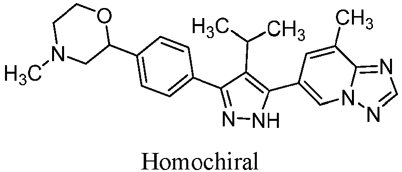 | 417.3 | 1.498 | E |
| 461 | 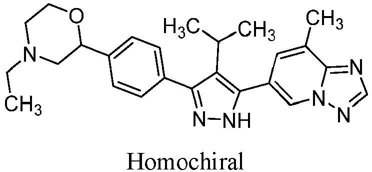 | 431.3 | 1.607 | E |
| 462 | 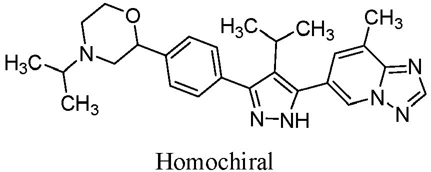 | 445.3 | 1.701 | E |
| 463 | 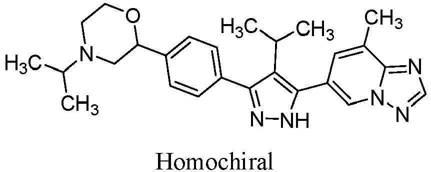 | 445.3 | 1.707 | E |
| 464 | 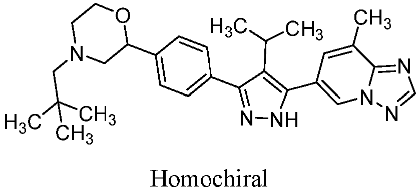 | 473.3 | 2.518 | E |
| 465 | 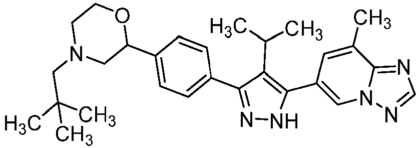 | 473.3 | 2.147 | E |
| | Homochiral | | | |

### EXAMPLE 466

### 2-(2-(4-(4-isopropyl-5-(8-methyl-[1,2,4]triazolo[1,5-a]pyridin-6-yl)-1H-pyrazol-3-yl) phenyl)morpholino)-N,N-dimethylacetamide

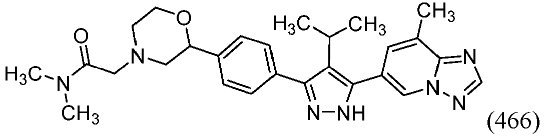

To a solution of 2-(4-(4-isopropyl-5-(8-methyl-[1,2,4]triazolo[1,5-a]pyridin-6-yl)-1H-pyrazol-3-yl)phenyl)morpholine (0.008 g, 0.020 mmol) in THF (1.00 mL) and DMF (0.50 mL) solvent mixture were added TEA (0.15 mL, 1.076 mmol) and 2-chloro-N,N-dimethylacetamide (4.83 mg, 0.040 mmol) at room temperature. The reaction mixture was stirred at the same temperature for 16 h. The reaction mass was purified by prep LCMS using method AA, the fractions containing the product were combined and dried using Genevac centrifugal evaporator to get 2-(2-(4-(4-isopropyl-5-(8-methyl-[1,2,4]triazolo[1,5-a]pyridin-6-yl)-1H-pyrazol-3-yl)phenyl) morpholino)-N,N-dimethylacetamide (8.1 mg, 0.016 mmol, 82 % yield) as a pale solid. LCMS Retention time 1.451 min [E], MS (E⁻) *m*/*z:* 488.3 [M+H]; ¹H NMR (400 MHz, METHANOL-d₄) δ ppm 8.75 (br. s., 1H), 8.47 (s, 1H), 7.68 (br. s., 1H), 7.52 (s, 4H), 4.74-4.67 (m, 1H), 4.05 (dd, *J*=11.5, 2.0 Hz, 1H), 3.91 (td, *J*=11.5, 2.5 Hz, 1H), 3.37 (s, 1H), 3.21-3.03 (m, 5H), 2.98 (s, 3H), 2.89 (d, *J*=12.5 Hz, 1H), 2.71 (s, 3H), 2.43 (td, *J*=11.5*,* 3.5 Hz, 1H), 2.24 (t, *J*=10.8 Hz, 1H).

The following Examples were prepared according to the general procedure used to prepare Example 466.

**TABLE 50**

| Ex. No. | Structure | LCMS MH⁺ | RT (min) | HPLC Method |
|---|---|---|---|---|
| 467 | 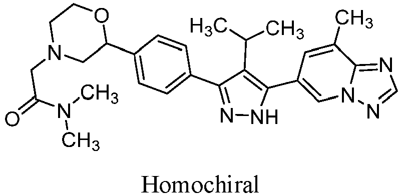 | 488.3 | 1.452 | E |
| 468 | 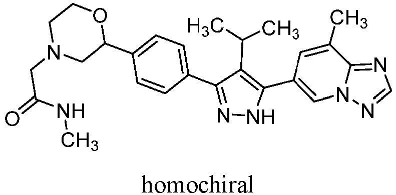 | 474.3 | 1.396 | E |

### EXAMPLE 469

### 2-(dimethylamino)-1-(2-(4-(4-isopropyl-5-(8-methyl-[1,2,4]triazolo[1,5-a]pyridin-6-yl)-1H-pyrazol-3-yl)phenyl)morpholino)ethan-1-one

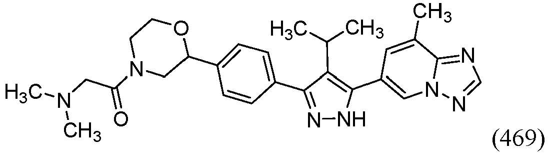

To a solution of 2-(4-(4-isopropyl-5-(8-methyl-[1,2,4]triazolo[1,5-a]pyridin-6-yl)-1H-pyrazol-3-yl)phenyl)morpholine (0.008 g, 0.020 mmol) in DMF (1.00 mL) were added TEA (0.15 mL, 1.076 mmol), dimethylglycine (4.10 mg, 0.040 mmol), and HATU (0.015 g, 0.040 mmol) at room temperature. The reaction mixture was stirred at the same temperature for 16 h. The reaction mass was purified by prep LCMS using method AA, the fractions containing the product were combined and dried using Genevac centrifugal evaporator to get 2-(dimethylamino)-1-(2-(4-(4-isopropyl-5-(8-methyl-[1,2,4]triazolo[1,5-a]pyridin-6-yl)-1H-pyrazol-3-yl)phenyl) morpholino)ethan-1-one (3.1 mg, 6.36 µmol, 32% yield) as a pale solid. LCMS Retention time 1.302 min [E], MS (E⁻) *m*/*z:* 488.3 [M+H]; ¹H NMR (400 MHz, METHANOL-d₄) δ ppm 8.77 (s, 1H), 8.48 (s, 1H), 7.67 (s, 1H), 7.63-7.48 (m, 4H), 4.63 (br. s., 1H), 4.44 (d, *J*=13.6 Hz, 1H), 4.22-4.05 (m, 2H), 3.97 (d, *J*=13.1 Hz, 1H), 3.84-3.67 (m, 1H), 3.65-3.48 (m, 1H), 3.45-3.35 (m, 2H), 3.28-3.06 (m, 2H), 3.04-2.93 (m, 1H), 2.83 (dd, *J*=13.6, 11.0 Hz, 1H), 2.71 (s, 3H), 2.46 (s, 6H), 1.95 (s, 1H), 1.20 (d, *J*=7.0 Hz, 6H).

The following Example was prepared according to the general procedure used to prepare Example 469.

**TABLE 51**

| Ex. No. | Structure | LCMS MH⁺ | RT (min) | HPLC Method |
|---|---|---|---|---|
| 470 | 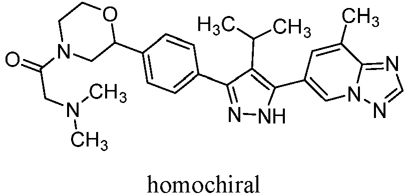 | 488.3 | 1.304 | E |

### EXAMPLE 471

### 4-(4-(4-isopropyl-5-(8-methyl-[1,2,4]triazolo[1,5-a]pyridin-6-yl)-1H-pyrazol-3-yl) phenyl)-N-methylcyclohexan-1-amine

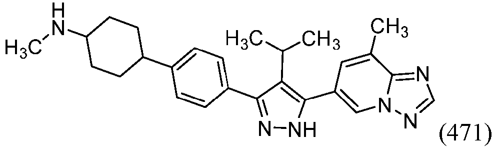

### Intermediate 471A: 8-(4-bromophenyl)-1,4-dioxaspiro[4.5]dec-7-ene

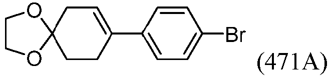

PdCl₂(dppf)-CH₂Cl₂ adduct (0.767 g, 0.939 mmol) and K₃PO₄ (4.91 g, 28.2 mmol) were added to a degassed mixture of 1-bromo-4-iodobenzene (3.99 g, 14.09 mmol) and 4,4,5,5-tetramethyl-2-(1,4-dioxaspiro[4.5]dec-7-en-8-yl)-1,3,2-dioxaborolane (2.5 g, 9.39 mmol) in dioxane (60.0 mL) and water (10.0 mL). The mixture was stirred at 100 °C for 14 h in a sealed tube. The reaction mixture was diluted with ethyl acetate, filtered and washed with excess ethyl acetate. The combined organic layers were washed with water, brine, dried over sodium sulphate and evaporated to yield crude product. The crude product was purified by ISCO using 40 g silica column, compound was eluted with 35-45% ethyl acetate in pet ether, the fractions were collected and concentrated to afford 8-(4-bromophenyl)-1,4-dioxaspiro[4.5]dec-7-ene (2.5 g, 8.47 mmol, 90% yield) as a light yellow semi-solid. LCMS Retention time: 3.13 min [B], MS (E⁺) m/z: 297.2 [M+2H].

### Intermediate 471B: 2-(4-(1,4-dioxaspiro[4.5]dec-7-en-8-yl)phenyl)-4,4,5,5-tetramethyl-1,3,2-dioxaborolane

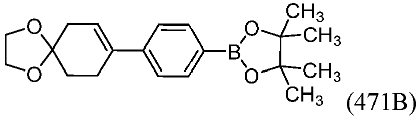

PdCl₂ (dppf)-CH₂Cl₂ adduct (0.830 g, 1.016 mmol) and potassium acetate (2.99 g, 30.5 mmol) were added to a degassed solution of BISPIN (3.36 g, 13.21 mmol) and 8-(4-bromophenyl)-1,4-dioxaspiro[4.5]dec-7-ene (3.0 g, 10.16 mmol) in dioxane (80.0 mL). The reaction mixture was stirred at 100 °C for 14 h in a sealed tube. The reaction mixture was diluted with ethyl acetate, filtered and washed with excess ethyl acetate, the filtrate was dried over sodium sulphate and evaporated to get crude compound. The crude compound was purified by ISCO using 40 g silica column, compound was eluted with 25-45% ethylacetate in pet ether, the fractions were collected and concentrated to get 2-(4-(1,4-dioxaspiro[4.5]dec-7-en-8-yl)phenyl)-4,4,5,5-tetramethyl-1,3,2-dioxaborolane (3.2 g, 9.35 mmol, 92 % yield) as a light brown semi-solid. LCMS Retention time 2.48 min [B], MS (E⁺) m/z: 343.2 [M+2H].

### Intermediate 471C: 6-(3-(4-(1,4-dioxaspiro[4.5]dec-7-en-8-yl)phenyl)-4-isopropyl-1-((2-(trimethylsilyl)ethoxy)methyl)-1H-pyrazol-5-yl)-8-methyl-[1,2,4]triazolo[1,5-a] pyridine

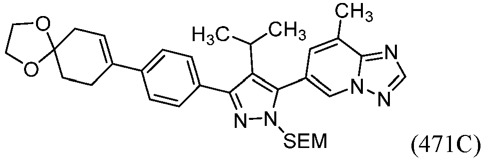

6-(3-(4-(1,4-dioxaspiro[4.5] dec-7-en-8-yl)phenyl)- 4-isopropyl-1-((2-(trimethylsilyl) ethoxy)methyl)-1H-pyrazol-5-yl)-8-methyl-[1,2,4]triazolo[1,5-a]pyridine (3.5 g, 5.97 mmol, 90 % yield) was prepared according to the general procedure described in Intermediate 307B, using 6-(3-bromo-4-isopropyl-1-((2-(trimethylsilyl)ethoxy)methyl)-1H-pyrazol-5-yl)-8-methyl-[1,2,4]triazolo[1,5-a] pyridine (3.0 g, 6.66 mmol) and 2-(4-(1,4-dioxaspiro[4.5]dec-7-en-8-yl)phenyl)-4,4,5,5-tetramethyl-1,3,2-dioxaborolane (2.74 g, 7.99 mmol) as starting intermediates to yield the title compound as a gummy solid. LCMS Retention time: 1.80 min [B], MS (E⁺) m/z: 586.4 [M+H].

### Intermediate 471D: 6-(3-(4-(1,4-dioxaspiro[4.5]decan-8-yl)phenyl)-4-isopropyl-1-((2-(trimethylsilyl)ethoxy)methyl)-1H-pyrazol-5-yl)-8-methyl-[1,2,4]triazolo[1,5-a]pyridine

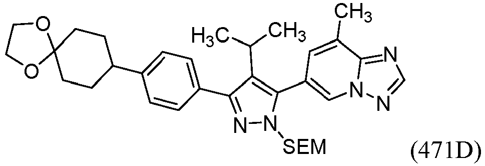

6-(3-(4-(1,4-dioxaspiro[4.5]decan-8-yl) phenyl)-4-isopropyl-1-((2-(trimethylsilyl) ethoxy)methyl)-1H-pyrazol-5-yl)-8-methyl-[1,2,4]triazolo[1,5-a]pyridine (2.0 g, 3.40 mmol, 57% yield) was prepared according to the general process described in Intermediate 307C, using 6-(3-(4-(1,4-dioxaspiro [4.5]dec-7-en-8-yl)phenyl)-4-isopropyl-1-((2-(trimethylsilyl) ethoxy)methyl)-1H-pyrazol-5-yl)-8-methyl-[1,2,4]triazolo[1,5-a]pyridine (3.5 g, 5.97 mmol) as a starting intermediate to afford the title compound as an off-white solid. LCMS Retention time: 4.53 min [B], MS (E⁺) m/z: 588.2 [M+H].

### Intermediate 471E: 4-(4-(4-isopropyl-5-(8-methyl-[1,2,4]triazolo[1,5-a]pyridin-6-yl)-1H-pyrazol-3-yl)phenyl)cyclohexan-1-one

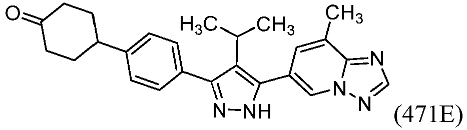

To a solution of 6-(3-(4-(1,4-dioxaspiro[4.5]decan-8-yl)phenyl)-4-isopropyl-1-((2-(trimethylsilyl)ethoxy)methyl)-1H-pyrazol-5-yl)-8-methyl-[1,2,4]triazolo[1,5-a]pyridine (2.2 g, 3.74 mmol) in DCM (25.0 mL) was added TFA (7.21 mL, 94 mmol) at 0 °C. The reaction mixture was stirred at room temperature for 12 h. The reaction mass was concentrated, triturated with diethyl ether and dried under vacuum to yield 4-(4-(4-isopropyl-5-(8-methyl-[1,2,4]triazolo[1,5-a]pyridin-6-yl)-1H-pyrazol-3-yl)phenyl)cyclohexan-1-one (1.6 g, 3.87 mmol) as an off-white solid. LCMS Retention time: 1.18 min [A], MS (E⁺) m/z: 414.4 [M+H].

### Example 471: 4-(4-(4-isopropyl-5-(8-methyl-[1,2,4]triazolo[1,5-a]pyridin-6-yl)-1H-pyrazol-3-yl)phenyl)-N-methylcyclohexan-1-amine

A mixture of 4-(4-(4-isopropyl-5-(8-methyl-[1,2,4]triazolo[1,5-a]pyridin-6-yl)-1H-pyrazol-3-yl)phenyl)cyclohexan-1-one (1.6 g, 3.87 mmol), methylamine hydrochloride (2.61 g, 38.7 mmol) and acetic acid (0.044 mL, 0.774 mmol) in DMF (20.0 mL) was stirred at room temperature for 8 h. Sodium cyanoborohydride (0.486 g, 7.74 mmol) was added at 0 °C, and the reaction mixture was stirred at room temperature for 16 h. The reaction mass was concentrated to get crude compound, the crude compound was purified by Prep LCMS using method AA to separate the isomers. The fractions were collected, concentrated and lyophilized to yield two isomers.
Intermediate 471A: Isomer 1, as a white solid. LCMS Retention time: 1.20 min [E], MS (E⁺) m/z: 429.3 [M+H]; ¹H NMR (400 MHz, METHANOL-d₄) δ 8.75 (br. s., 1H), 8.48 (s, 1H), 7.67 (s, 1H), 7.52-7.44(m, *J*=8.3 Hz, 2H), 7.44-7.34 (m, *J*=8.1 Hz, 2H), 3.80 (s, 1H), 3.13-2.99 (m, 2H), 2.77-2.65(m, 5H), 2.28 (d, *J*=9.5 Hz, 2H), 2.11 (d, *J*=13.2 Hz, 2H), 1.93 (s, 2H), 1.77-1.65 (m, 2H), 1.60- 1.50 (m, 2H), 1.31 (s, 4H), 1.19 (d, *J*=7.1 Hz, 5H), 0.91 (d, *J*=7.3 Hz, 2H); and
Intermediate 471B: Isomer 2, as a white solid. LCMS Retention time: 1.19 min [E], MS (E⁺) m/z: 429.3 [M+H]; ¹H NMR (400 MHz, METHANOL-d₄) δ 8.75 (s, 1H), 8.48 (s, 1H), 7.67 (s, 1H), 7.54-7.45 (m,2H), 7.44-7.32 (m, 2H), 3.18-2.94 (m, 3H), 2.79-2.61 (m, 7H), 2.28 (d, *J*=10.8 Hz, 2H), 2.10(d, *J*=12.5 Hz, 2H), 1.92 (s, 3H), 1.71 (qd, *J*=12.9, 3.1 Hz, 2H), 1.62-1.47 (m, 2H), 1.31 (s,1H), 1.19 (d, *J*=7.1 Hz, 6H).

The following Examples were prepared according to the general procedure used to prepare Example 471A/B.

**TABLE 52**

| Ex. No. | Structure | LCMS MH⁺ | RT (min) | HPLC Method |
|---|---|---|---|---|
| 472 | 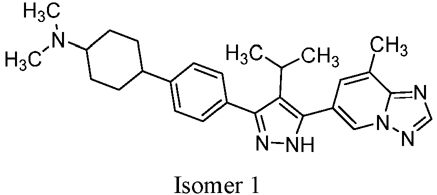 | 443.3 | 1.25 | C |
| 473 | 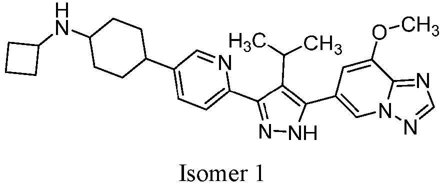 | 486.3 | 1.25 | C |
| 474 | 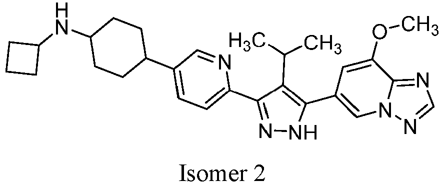 | 486.3 | 1.39 | C |
| 475 | 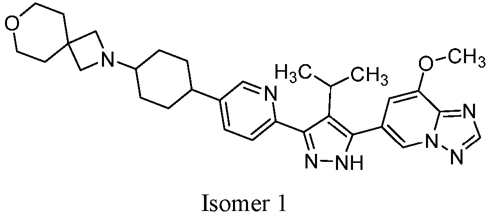 | 542.3 | 1.38 | C |
| 476 | 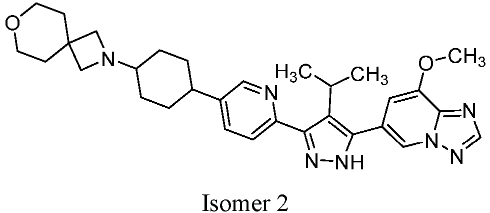 | 542.3 | 1.26 | C |
| 477 | 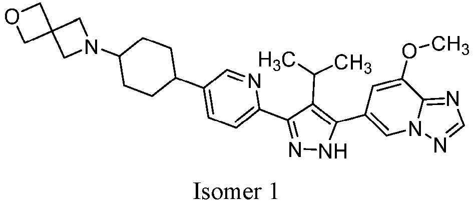 | 514.3 | 1.39 | C |
| 478 | 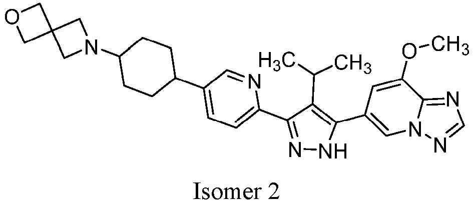 | 514.3 | 1.27 | C |
| 479 | 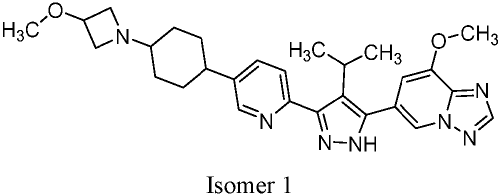 | 502.3 | 1.5 | C |
| 480 | 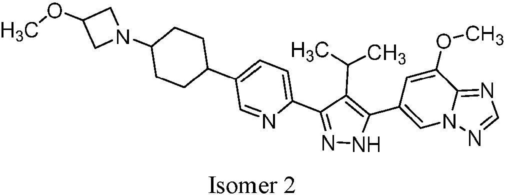 | 502.3 | 1.31 | C |
| 481 | 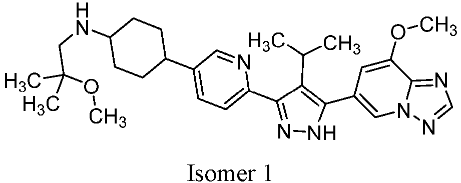 | 518.3 | 1.52 | C |
| 482 | 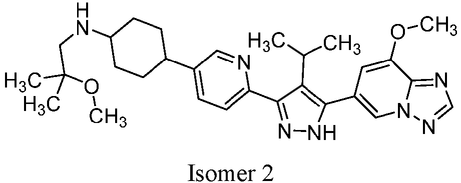 | 518.3 | 1.36 | C |
| 483 | 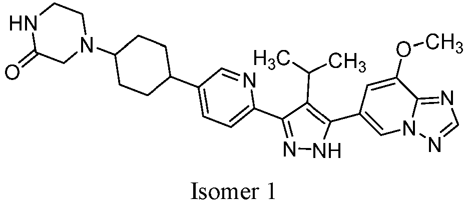 | 515.3 | 1.48 | C |
| 484 | 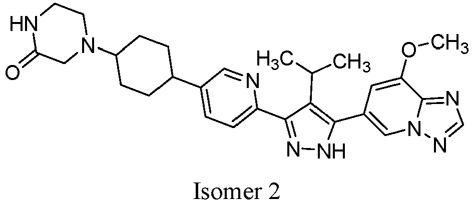 | 515.3 | 1.29 | C |
| 485 | 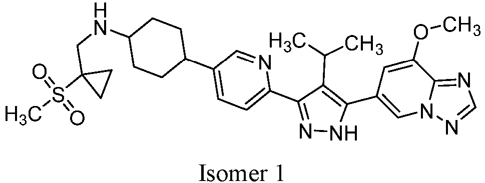 | 564.2 | 1.98 | C |
| 486 | 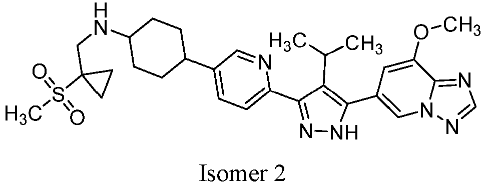 | 564.3 | 1.77 | C |
| 487 | 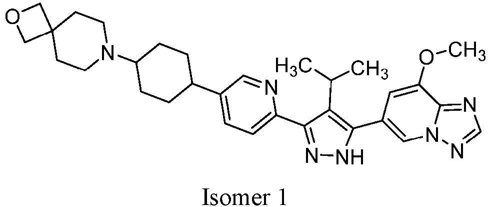 | 542.3 | 1.33 | C |
| 489 | 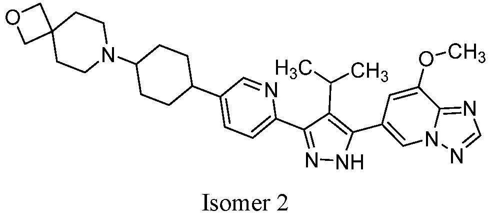 | 542.3 | 1.19 | C |
| 490 | 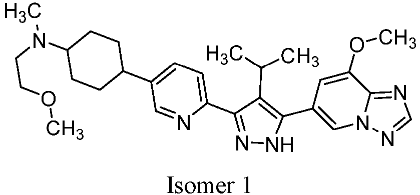 | 504.3 | 1.33 | C |
| 491 | 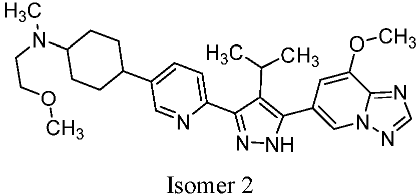 | 504.3 | 1.22 | C |
| 492 | 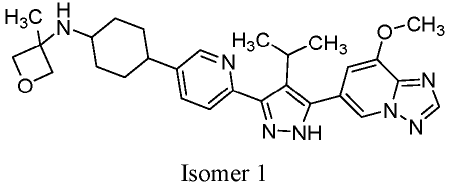 | 502.3 | 1.56 | C |
| 493 | 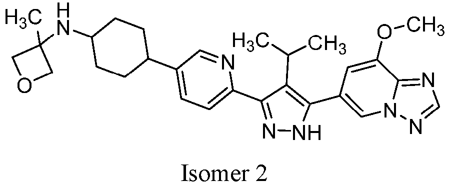 | 502.3 | 1.38 | C |
| 494 | 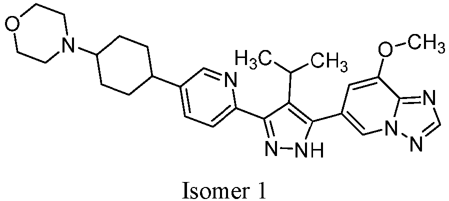 | 502.3 | 0.9 | D |
| 495 | 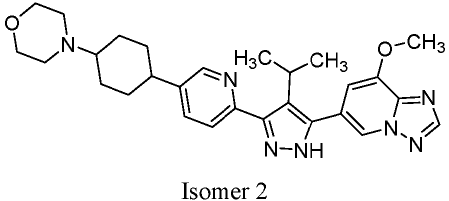 | 502.3 | 0.87 | C |
| 496 | 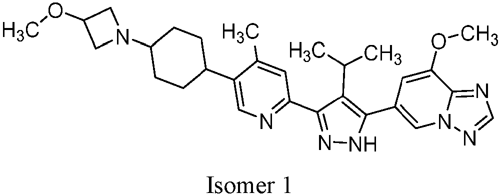 | 516.3 | 1.59 | C |
| 497 | 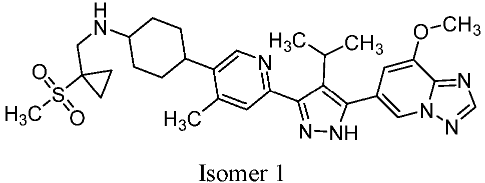 | 578.3 | 1.5 | C |
| 498 | 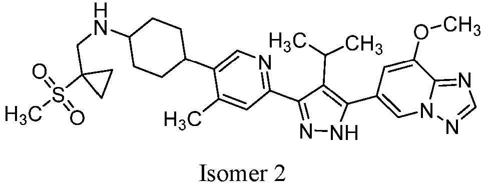 | 578.3 | 1.77 | C |
| 499 | 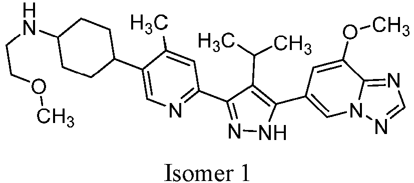 | 504.3 | 1.3 | C |
| 500 | 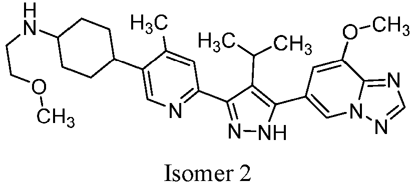 | 504.3 | 1.34 | C |
| 501 | 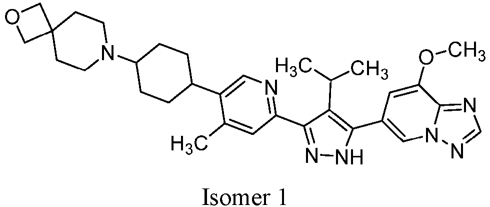 | 556.3 | 1.34 | C |
| 502 | 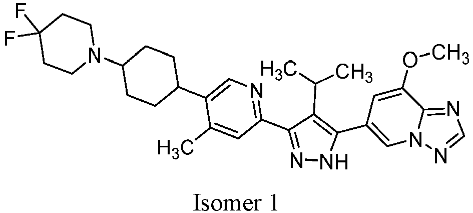 | 550.3 | 1.99 | C |
| 503 | 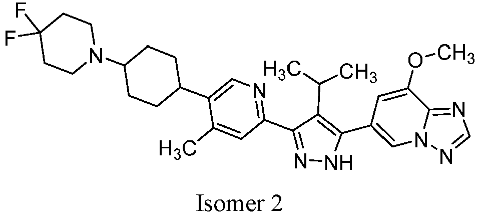 | 550.3 | 2.3 | C |
| 504 | 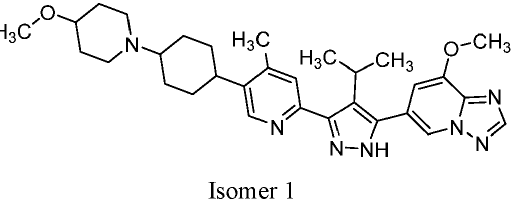 | 544.3 | 1.33 | C |
| 505 | 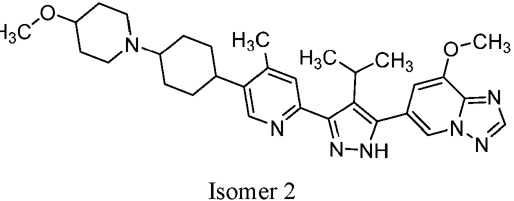 | 544.3 | 1.48 | C |
| 506 | 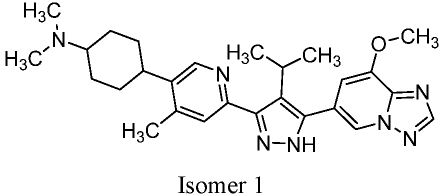 | 474.3 | 0.92 | D |
| 507 | 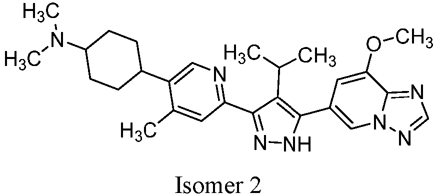 | 474.3 | 1.17 | C |
| 508 | 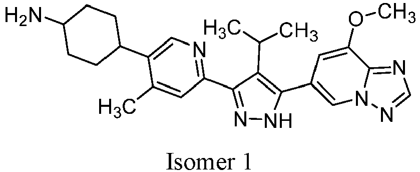 | 446.2 | 1.19 | C |
| 509 | 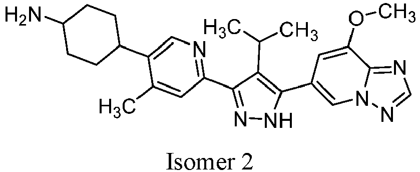 | 446.2 | 0.96 | D |

### EXAMPLE 510

### 4-(4-(4-isopropyl-5-(8-methyl-[1,2,4]triazolo[1,5-a]pyridin-6-yl)-1H-pyrazol-3-yl) phenyl)-N,N-dimethylcyclohexan-1-amine

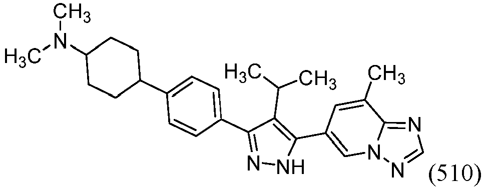

A solution of 4-(4-(4-isopropyl-5-(8-methyl-[1,2,4]triazolo[1,5-a]pyridin-6-yl)-1H-pyrazol-3-yl)phenyl)-N-methylcyclohexan-1-amine (20.0 mg, 0.047 mmol), formaldehyde (6.43 µl, 0.233 mmol) and acetic acid (0.534 µL, 9.33 µmol) in MeOH (2.0 mL) was stirred at room temperature for 8 h. Sodium cyanoborohydride (5.87 mg, 0.093 mmol) was added at 0 °C and the reaction mixture was stirred at room temperature for 16 h. The reaction mass was purified by preparative LC/MS using method AA, fractions containing the product were combined and dried via centrifugal evaporation to get 4-(4-(4-isopropyl-5-(8-methyl-[1,2,4]triazolo[1,5-a]pyridin-6-yl)-1H-pyrazol-3-yl)phenyl)-N,N-dimethylcyclohexan-1-amine (4.3 mg) as a pale solid. LCMS Retention time: 1.25 min [E], MS (E⁺) m/z: 443.3 [M+H]; ¹H NMR (400 MHz, METHANOL-d₄) δ 8.76 (s, 1H), 8.53-8.41 (m, 1H), 7.67 (s, 1H), 7.51 -7.44 (m, *J*=8.2 Hz, 2H), 7.44-7.36 (m, *J*=8.1 Hz, 2H), 3.12 (dt, *J*=14.2, 7.1 Hz, 2H), 2.77 (s,5H), 2.74-2.56 (m, 5H), 2.29-2.17 (m, 2H), 2.13 (d, *J*=10.1 Hz, 2H), 1.94 (s, 2H), 1.80-1.55(m, 4H), 1.39-1.28 (m, 1H), 1.19 (d, *J*=7.2 Hz, 6H).

The following Examples were prepared according to the general procedure used to prepare Example 510.

**TABLE 53**

| Ex. No. | Structure | LCMS MH⁺ | RT (min) | HPLC Method |
|---|---|---|---|---|
| 511 | 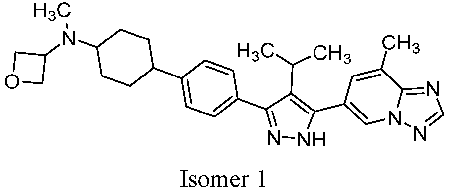 | 485.3 | 1.68 | C |
| 512 | 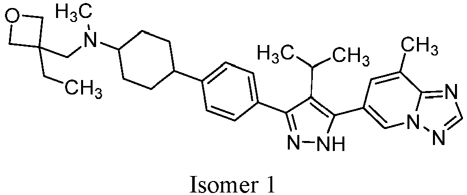 | 527.3 | 1.45 | C |
| 513 | 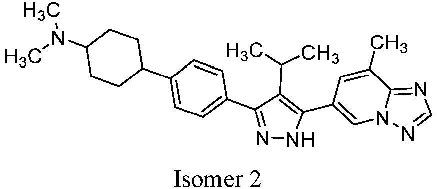 | 443.3 | 1.25 | C |
| 514 | 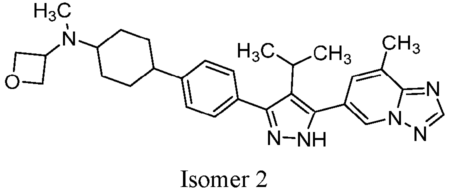 | 485.3 | 1.79 | C |
| 515 | 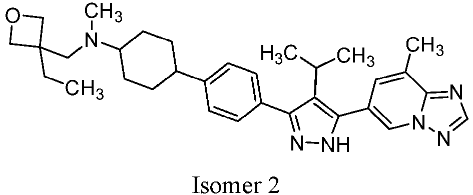 | 527.3 | 2.01 | C |
| 516 | 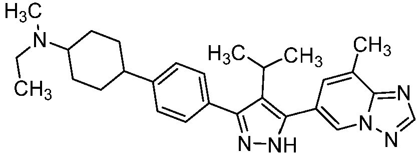 | 457.3 | 1.51 | C |
| | 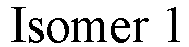 | | | |
| 517 | 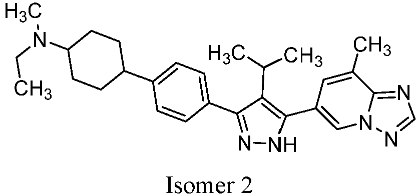 | 457.3 | 1.37 | C |

### EXAMPLE 518

### 2-(dimethylamino)-N-(4-(4-(4-isopropyl-5-(8-methyl-[1,2,4]triazolo[1,5-a]pyridin-6-yl)-1H-pyrazol-3-yl)phenyl)cyclohexyl)-N-methylacetamide

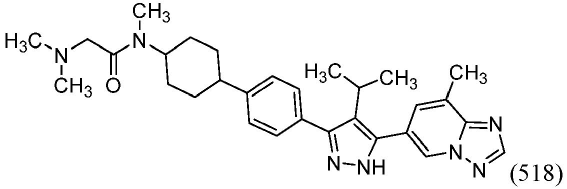

2-(dimethylamino)-N-(4-(4-(4-isopropyl-5-(8-methyl-[1,2,4]triazolo[1,5-a]pyridin-6-yl)-1H-pyrazol-3-yl)phenyl)cyclohexyl)-N-methylacetamide (3.4 mg) was prepared according to the general procedure described in Example 469, using 4-(4-(4-isopropyl-5-(8-methyl-[1,2,4]triazolo[1,5-a]pyridin-6-yl)-1H-pyrazol-3-yl)phenyl)-N-methylcyclohexan-1-amine (20.0 mg, 0.047 mmol) as a starting intermediate, to yield the title compound as an off-white solid. LCMS Retention time: 1.45 min [E], MS (E⁺) m/z: 514.3 [M+H]; ¹H NMR (400 MHz, METHANOL-d₄) δ 8.76 (s, 1H), 8.48 (s, 1H), 7.67 (s, 1H), 7.59-7.32 (m,4H), 4.50 (d, *J*=7.6 Hz, 1H), 3.62 (br. s., 1H), 3.60-3.51 (m, 1H), 3.20-3.02 (m, 1H), 3.02 -2.83 (m, 3H), 2.76-2.60 (m, 4H), 2.55 (d, *J*=3.5 Hz, 6H), 2.14-1.98 (m, 2H), 1.98-1.84 (m,2H), 1.84-1.62 (m, 4H), 1.39-1.27 (m, 1H), 1.20 (d, *J*=7.1 Hz, 6H).

The following Example was prepared according to the general procedure used to prepare Example 518.

**TABLE 54**

| Ex. No. | Structure | LCMS MH⁺ | RT (min) | HPLC Method |
|---|---|---|---|---|
| 519 | 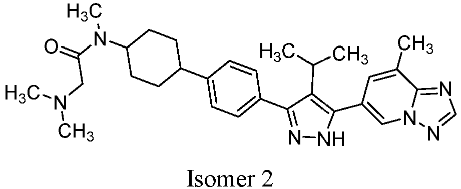 | 514.3 | 1.45 | C |

### EXAMPLE 520

### 2-((4-(4-(4-isopropyl-5-(8-methyl-[1,2,4]triazolo[1,5-a]pyridin-6-yl)-1H-pyrazol-3-yl) phenyl)cyclohexyl)(methyl)amino)-N,N-dimethylacetamide

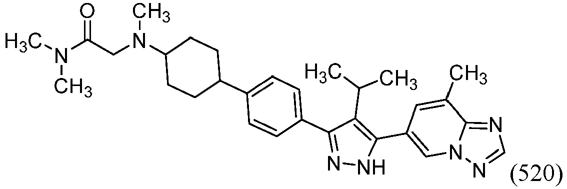

2-((4-(4-(4-isopropyl-5-(8-methyl-[1,2,4]triazolo[1,5-a]pyridin-6-yl)-1H-pyrazol-3-yl)phenyl)cyclohexyl)(methyl)amino)-N,N-dimethylacetamide (12.6 mg) was prepared according to the general procedure described in Example 466, using 4-(4-(4-isopropyl-5-(8-methyl-[1,2,4] triazolo[1,5-a]pyridin-6-yl)-1H-pyrazol-3-yl)phenyl)-N-methylcyclohexan-1-amine (20.0 mg, 0.047 mmol) as a starting intermediate to yield the title compound as an off-white solid. LCMS Retention time: 1.37 min [F], MS (E⁺) m/z: 514.4 [M+H]; ¹H NMR (400 MHz, METHANOL-d₄) δ 8.75 (br. s., 1H), 8.48 (s, 1H), 7.68 (br. s., 1H), 7.53 -7.27 (m, 4H), 3.59-3.39 (m, 2H), 3.22-3.05 (m, 4H), 2.98 (s, 3H), 2.71 (s, 4H), 2.62 (t, *J*=11.7Hz, 1H), 2.41 (br. s., 3H), 2.14-1.94 (m, 4H), 1.71-1.45 (m, 4H), 1.31 (s, 1H), 1.19 (d, J=7.2Hz, 6H), 0.91 (d, *J*=7.1 Hz, 1H).

The following Examples were prepared according to the general procedure used to prepare Example 520.

**TABLE 55**

| Ex. No. | Structure | LCMS MH⁺ | RT (min) | HPLC Method |
|---|---|---|---|---|
| 521 | 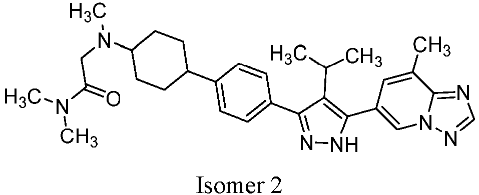 | 514.3 | 1.55 | C |
| 522 | 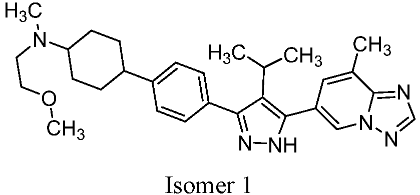 | 487.3 | 1.38 | C |
| 523 | 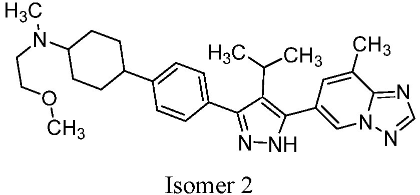 | 487.3 | 1.5 | C |

### EXAMPLE 524

### 2-(4-(4-isopropyl-5-(8-methoxy-[1,2,4]triazolo[1,5-a]pyridin-6-yl)-1H-pyrazol-3-yl) phenyl)propan-2-amine, TFA

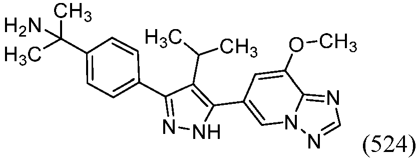

### Intermediate 524A: tert-butyl (2-(4-bromophenyl)propan-2-yl)carbamate

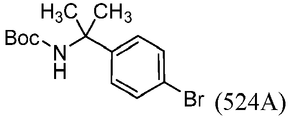

To a solution of 2-(4-bromophenyl)propan-2-amine (1.00 g, 4.67 mmol) in DCM (15.0 mL) were added TEA (1.302 mL, 9.34 mmol), and Boc-anhydride (1.627 mL, 7.01 mmol) at 0 °C. The reaction mass was concentrated to get crude compound. The crude compound was purified by ISCO using 40 g silica column, the compound was eluted in 10% EA in hexane, the fractions were collected and concentrated to get tert-butyl (2-(4-bromophenyl)propan-2-yl)carbamate (1.24 g, 3.95 mmol, 84 % yield) as a white solid. LCMS Retention time: 1.54 min [A], MS (E⁺) m/z: 260.3 [M+2H-tBu].

### Intermediate 524B: tert-butyl (2-(4-(4,4,5,5-tetramethyl-1,3,2-dioxaborolan-2-yl) phenyl) propan-2-yl)carbamate

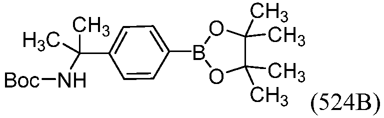

Mixture of tert-butyl (2-(4-bromophenyl)propan-2-yl)carbamate (600 mg, 1.909 mmol), BISPIN (727 mg, 2.86 mmol) and potassium acetate (562 mg, 5.73 mmol) in dioxane (18 mL) was degassed with nitrogen for 5 min. Next, PdCl₂(dppf)-CH₂Cl₂ adduct (156 mg, 0.191 mmol) was added and the reaction mixture was degassed for another 2 min. The reaction mixture was stirred at 100 °C for 16 h. The reaction mass was diluted with EtOAc (20 mL), the solids were filtered through celite, the filtrate was collected and concentrated to get crude compound. The crude compound was purified by ISCO, using 40g silica column, compound was eluted in 15% EA in hexanes, the fractions were collected and concentrated to get tert-butyl (2-(4-(4,4,5,5-tetramethyl-1,3,2-dioxaborolan-2-yl)phenyl) propan-2-yl) carbamate (600 mg, 1.661 mmol, 87% yield) as a white solid. LCMS Retention time: 1.65 min [A], MS (E⁺) m/z: 362.6 [M+H].

### Intermediate 524C: tert-butyl (2-(4-(4-isopropyl-5-(8-methoxy-[1,2,4]triazolo[1,5-a] pyridin-6-yl)-1-((2-(trimethylsilyl)ethoxy)methyl)-1H-pyrazol-3-yl)phenyl)propan-2-yl) carbamate

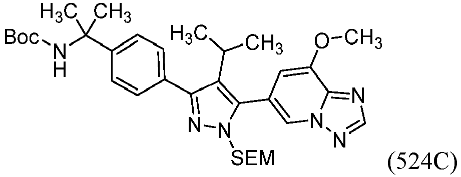

A solution of 6-(3-bromo-4-isopropyl-1-((2-(trimethylsilyl)ethoxy)methyl)-1H-pyrazol-5-yl)-8-methoxy-[1,2,4]triazolo[1,5-a]pyridine (320 mg, 0.686 mmol), tert-butyl (2-(4-(4,4,5,5-tetramethyl-1,3,2-dioxaborolan-2-yl)phenyl)propan-2-yl)carbamate (372 mg, 1.029 mmol) and K₂CO₃ (284 mg, 2.058 mmol) in acetonitrile (16.00 mL) and water (4.00 mL) solvent mixture was degassed for 10 min with nitrogen. Next, Pd₂(dba)₃ (31.4 mg, 0.034 mmol) and 2-dicyclohexylphosphino-2',6'-dimethoxybiphenyl (28.2 mg, 0.069 mmol) were added. The reaction mixture was degassed for 2 min, and stirred at 110 °C for 16 h. The reaction mixture was brought to room temperature, the two layers were separated, the aqueous layer was extracted with DCM (2 X 20 mL), the combined organic extracts were dried (Na₂SO₄) and concentrated to get crude compound. The crude compound was purified by ISCO using 24 g silica column, the compound was eluted in 50% EA in hexane, the fractions were collected and concentrated to get tert-butyl (2-(4-(4-isopropyl-5-(8-methoxy-[1,2,4]triazolo[1,5-a]pyridin-6-yl)-1-((2-(trimethylsilyl)ethoxy)methyl)-1H-pyrazol-3-yl)phenyl)propan-2-yl)carbamate (200 mg, 0.322 mmol, 47.0 % yield) as an off-white solid. LCMS Retention time: 1.87 min [A], MS (E⁺) m/z: 621.6 [M+H]·

### Intermediate 524: 2-(4-(4-isopropyl-5-(8-methoxy-[1,2,4]triazolo[1,5-a]pyridin-6-yl)-1H-pyrazol-3-yl)phenyl)propan-2-amine, TFA

To a solution of tert-butyl (2-(4-(4-isopropyl-5-(8-methoxy-[1,2,4]triazolo[1,5-a] pyridin-6-yl)-1-((2-(trimethylsilyl)ethoxy)methyl)-1H-pyrazol-3-yl)phenyl)propan-2-yl) carbamate (0.200 g, 0.322 mmol) in DCM (3.00 mL) was added TFA (1.500 mL, 19.47 mmol) at 0 °C. The reaction mixture was stirred at room temperature for 16 h. The reaction mass was concentrated, then triturated with diethyl ether (2 X 10 mL), and dried under vacuum to get 2-(4-(4-isopropyl-5-(8-methoxy-[1,2,4]triazolo[1,5-a]pyridin-6-yl)-1H-pyrazol-3-yl)phenyl)propan-2-amine, TFA (0.150 g, 0.297 mmol, 92% yield) as an off-white solid. LCMS Retention time: 0.74 min [A], MS (E⁺) m/z: 389.4 [M+H]; ¹H NMR (400 MHz, METHANOL-d₄) δ 8.43 (d, J=1.2 Hz, 1H), 8.33 (s, 1H), 7.65-7.47 (m, 4H), 7.18-7.10 (m, 1H), 4.02 (s, 3H), 3.07-2.93 (m, 2H), 1.71 (s, 6H), 1.24-1.14 (m, 3H), 1.11 (d, J=7.3 Hz, 6H).

The following Examples were prepared according to the general procedure used to prepare Example 524.

**TABLE 56**

| Ex. No. | Structure | LCMS MH⁺ | RT (min) | HPLC Method |
|---|---|---|---|---|
| 525 | 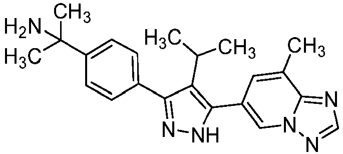 | 375.2 | 1.182 | E |
| 526 | 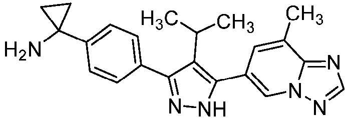 | 373.2 | 1.36 | C |
| 527 | 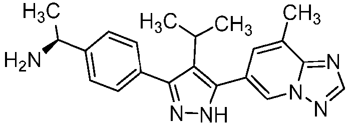 | 361.2 | 1.1 | C |
| 528 | 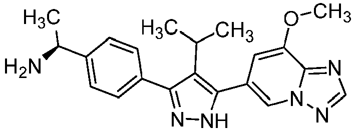 | 377.2 | 1.06 | C |
| 529 | 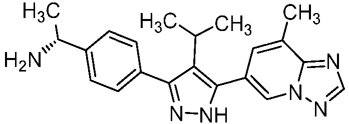 | 361.2 | 1.09 | C |
| 530 | 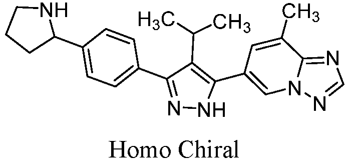 | 387.2 | 1.462 | E |
| 531 | 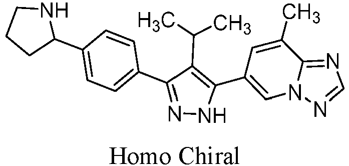 | 387.2 | 1.824 | E |

### EXAMPLE 532

### 2-(4-(4-isopropyl-5-(8-methoxy-[1,2,4]triazolo[1,5-a]pyridin-6-yl)-1H-pyrazol-3-yl) phenyl)-N,N-dimethylpropan-2-amine

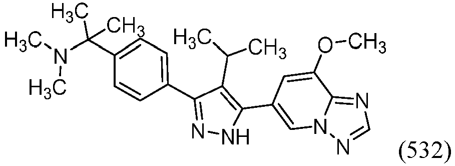

To a solution of 2-(4-(4-isopropyl-5-(8-methoxy-[1,2,4]triazolo[1,5-a]pyridin-6-yl)-1H-pyrazol-3-yl)phenyl)propan-2-amine, TFA (18 mg, 0.036 mmol) in MeOH (1.5 mL) were added formaldehyde (0.2 mL, 2.178 mmol) and acetic acid (0.2 mL, 3.49 mmol) at room temperature. The reaction mixture was stirred at the same temperature for 6 h. Next, sodium cyanoborohydride (11.21 mg, 0.178 mmol) was added at room temperature and the reaction mixture was stirred for 16 h. The reaction mass was purified by prep LCMS purification using method-AA, fractions containing product were combined and dried using Genevac centrifugal evaporator to get 2-(4-(4-isopropyl-5-(8-methoxy-[1,2,4]triazolo[1,5-a]pyridin-6-yl)-1H-pyrazol-3-yl)phenyl)-N,N-dimethylpropan-2-amine (5.1 mg, 0.012 mmol, 33 % yield) as a pale solid. LCMS Retention time: 1.289 min [E], MS (E⁺) m/z: 419.3 [M+H]; ¹H NMR (400 MHz, METHANOL-d₄) δ ppm 8.43 (s, 1H), 8.33 (s, 1H), 7.69-7.53 (m, *J*=8.3 Hz, 2H), 7.52-7.30 (m, *J*=8.1 Hz, 2H), 7.14 (s, 1H), 4.02 (s, 3H), 3.05 (quin, *J*=7.2 Hz, 1H), 2.29 (br. s., 6H), 1.84 (s, 1H), 1.52 (br. s., 6H), 1.19 (s, 1H), 1.12 (d, *J*=7.1 Hz, 6H).

The following Examples were prepared according to the general procedure used to prepare Example 532.

**TABLE 57**

| Ex. No. | Structure | LCMS MH⁺ | RT (min) | HPLC Method |
|---|---|---|---|---|
| 533 | 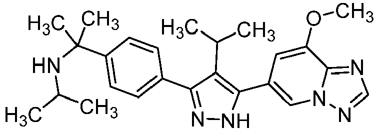 | 433.3 | 1.283 | E |
| 534 | 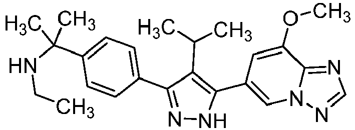 | 419.2 | 1.216 | E |
| 535 | 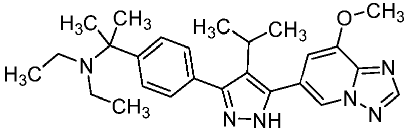 | 447.3 | 1.548 | E |
| 536 | 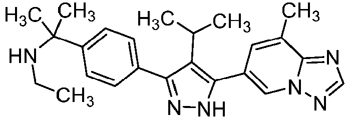 | 403.3 | 1.507 | E |
| 537 | 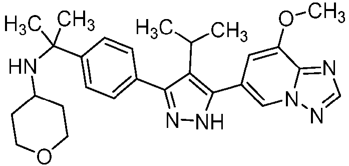 | 475.3 | 1.376 | E |
| 538 | 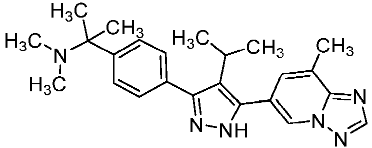 | 403.3 | 1.326 | E |
| 539 | 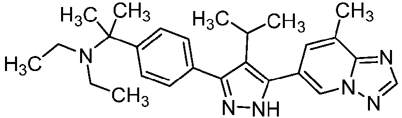 | 431.3 | 1.87 | E |
| 540 | 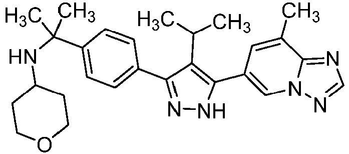 | 459.3 | 1.723 | E |
| 541 | 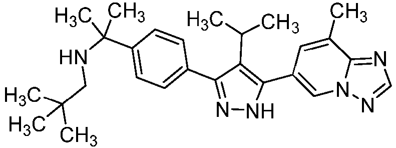 | 445.4 | 1.871 | E |
| 542 | 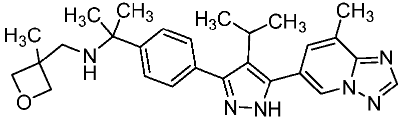 | 459.3 | 1.64 | E |
| 543 | 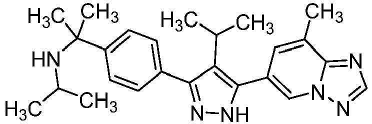 | 417.3 | 1.324 | E |
| 544 | 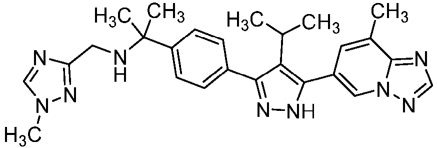 | 470.3 | 1.42 | E |
| 545 | 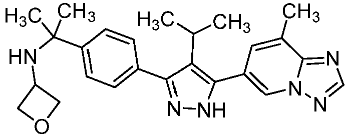 | 431.3 | 1.504 | E |
| 546 | 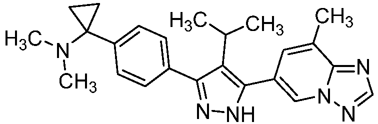 | 401.3 | 1.7 | C |
| 547 | 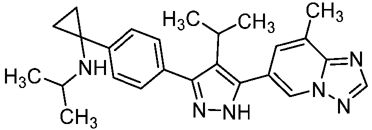 | 415.3 | 1.66 | C |
| 548 | 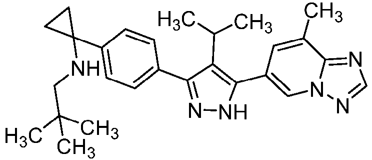 | 443.3 | 2.35 | C |
| 549 | 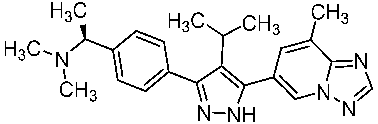 | 389.3 | 1.26 | C |
| 550 | 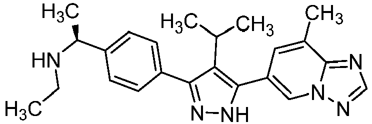 | 389.3 | 1.18 | C |
| 551 | 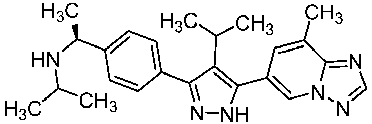 | 403.3 | 1.27 | C |
| 552 | 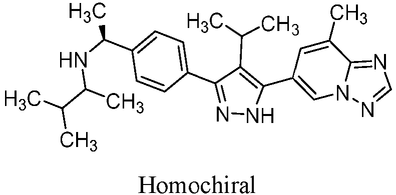 | 431.3 | 1.59 | C |
| 553 | 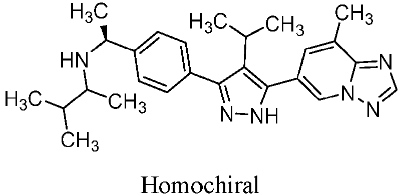 | 431.3 | 1.67 | C |
| 554 | 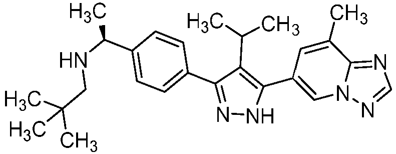 | 431.3 | 1.84 | C |
| 555 | 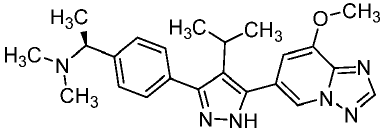 | 405.3 | 1.23 | C |
| 556 | 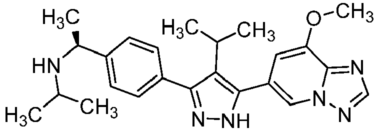 | 419.3 | 1.23 | C |
| 557 | 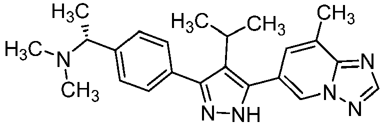 | 389.3 | 1.17 | C |
| 558 | 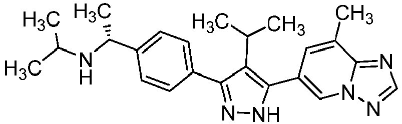 | 403.3 | 1.19 | C |
| 559 | 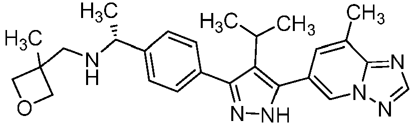 | 445.3 | 1.48 | C |
| 560 | 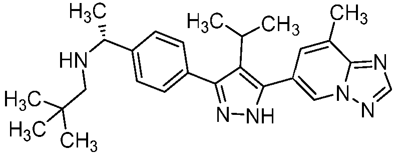 | 431.3 | 1.76 | C |
| 561 | 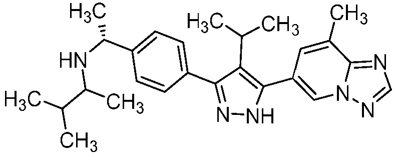 | 431.3 | 1.7 | C |
| 562 | 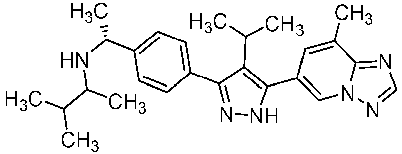 | 431.3 | 1.62 | C |
| 563 | 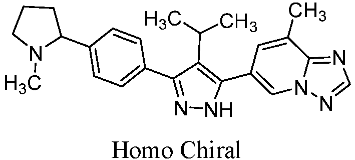 | 401.3 | 1.453 | E |
| 564 | 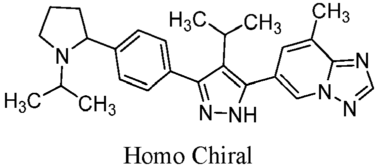 | 429.3 | 1.539 | E |
| 565 | 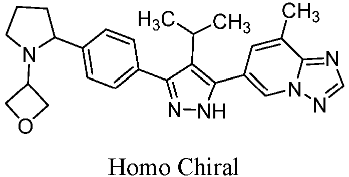 | 443.3 | 1.671 | E |
| 566 | 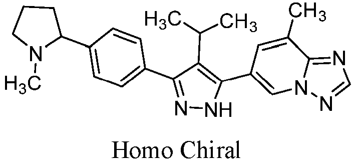 | 401.3 | 1.456 | E |
| 567 | 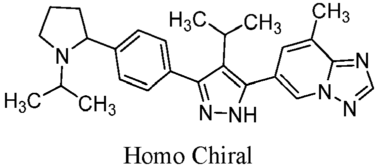 | 429.3 | 1.515 | E |
| 568 | 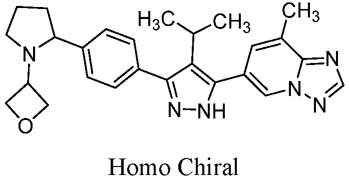 | 443.3 | 1.67 | E |
| 569 | 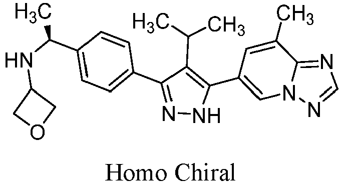 | 417.3 | 1.39 | C |

### EXAMPLE 570

### (S)-N-ethyl-1-(4-(4-isopropyl-5-(8-methoxy-[1,2,4]triazolo[1,5-a]pyridin-6-yl)-1H-pyrazol-3-yl)phenyl)-N-methylethan-1-amine

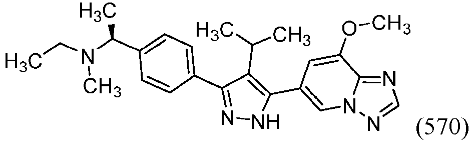

### Intermediate 570A: tert-butyl (S)-(1-(4-bromophenyl)ethyl)carbamate

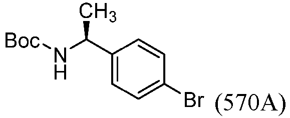

To a solution of (S)-1-(4-bromophenyl)ethan-1-amine (1.5 g, 7.50 mmol) in dichloromethane (30.0 mL) at 0 °C were added TEA (3.13 mL, 22.49 mmol) and Boc-anhydride (2.089 mL, 9.00 mmol). The reaction mixture was stirred at room temperature for 12 h. The reaction mass was concentrated, purified by ISCO using 40 g silica column, the compound was eluted with 35-40% ethyl acetate in pet ether, the fractions were collected and concentrated to get tert-butyl (S)-(1-(4-bromophenyl)ethyl)carbamate (2.0 g, 6.66 mmol, 89 % yield) as an off-white solid. LCMS Retention time: 1.46 min [A], MS (E⁺) m/z: 246.1 [M-tBu+2H].

### Intermediate 570B: Tert-butyl (S)-(1-(4-bromophenyl)ethyl)(methyl)carbamate

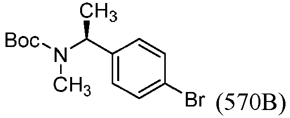

NaH (0.853 g, 21.32 mmol) was added to a solution of tert-butyl (S)-(1-(4-bromophenyl)ethyl)carbamate (3.2 g, 10.66 mmol) in THF (60.0 mL) at 0 °C. The reaction mixture was stirred for 30 min at room temperature and MeI (3.33 mL, 53.3 mmol) was added at 0 °C. The reaction mixture was stirred at room temperature for 12 h. The reaction was quenched with saturated NH₄Cl solution, extracted with ethyl acetate, washed with water, brine, dried over sodium sulphate and concentrated to get crude compound. The crude compound was purified by ISCO using 40 g silica column, compound was eluted with 25-30% ethyl acetate in pet ether, the fractions were collected and concentrated to get tert-butyl (S)-(1-(4-bromophenyl)ethyl)(methyl)carbamate (3.0 g, 9.55 mmol, 90% yield) as an oil. LCMS Retention time: 1.72 min [A], MS (E⁺) m/z: 260.1 [M-tBu+2H].

### Intermediate 570C: tert-butyl (S)-methyl(1-(4-(4,4,5,5-tetramethyl-1,3,2-dioxaborolan-2-yl)phenyl)ethyl)carbamate

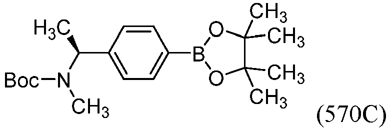

tert-Butyl (S)-methyl(1-(4-(4,4,5,5-tetramethyl-1,3,2-dioxaborolan-2-yl)phenyl) ethyl)carbamate (3.8 g, 10.52 mmol) was prepared according to the general process described in Intermediate 524B, using tert-butyl (S)-(1-(4-bromophenyl)ethyl)(methyl)carbamate (3.2 g, 10.18 mmol) as a starting intermediate to yield the title compound as an off-white solid. LCMS Retention time: 1.86 min [A], MS (E⁺) m/z: 306.3 [M-tBu].

### Intermediate 570D: tert-butyl (S)-(1-(4-(4-isopropyl-5-(8-methoxy-[1,2,4]triazolo[1,5-a] pyridin-6-yl)-1-((2-(trimethylsilyl)ethoxy)methyl)-1H-pyrazol-3-yl)phenyl)ethyl)(methyl) carbamate

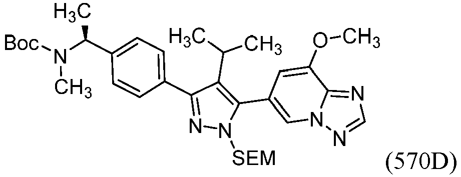

tert-Butyl (S)-(1-(4-(4-isopropyl-5-(8-methoxy-[1,2,4]triazolo[1,5-a]pyridin-6-yl)-1-((2-(trimethylsilyl)ethoxy)methyl)-1H-pyrazol-3-yl)phenyl)ethyl)(methyl)carbamate (850 mg, 1.369 mmol, 80 % yield) was prepared according to the general process described in Intermediate 524C, using 6-(3-bromo-4-isopropyl-1-((2-(trimethylsilyl)ethoxy)methyl)-1H-pyrazol-5-yl)-8-methoxy-[1,2,4]triazolo [1,5-a]pyridine (800 mg, 1.715 mmol) and tert-butyl (S)-methyl(1-(4-(4,4,5,5-tetramethyl-1,3,2-dioxaborolan-2-yl)phenyl)ethyl) carbamate (744 mg, 2.058 mmol) as starting intermediates to yield the title compound as a pale brown solid. LCMS Retention time: 4.30 min [B], MS (E⁺) m/z: 621.4 [M+H].

### Intermediate 570E: (S)-1-(4-(4-isopropyl-5-(8-methoxy-[1,2,4]triazolo[1,5-a]pyridin-6-yl)-1H-pyrazol-3-yl)phenyl)-N-methylethan-1-amine

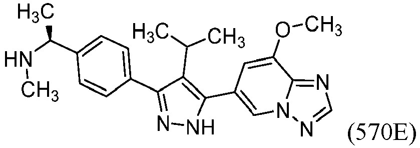

(S)-1-(4-(4-isopropyl-5-(8-methoxy-[1,2,4]triazolo[1,5-a]pyridin-6-yl)-1H-pyrazol-3-yl)phenyl)-N-methylethan-1-amine (500 mg, 1.280 mmol, 94 % yield) was prepared according to the general process described in Example 524, using tert-butyl (S)-(1-(4-(4-isopropyl-5-(8-methoxy-[1,2,4]triazolo[1,5-a]pyridin-6-yl)-1-((2-(trimethylsilyl)ethoxy) methyl)-1H-pyrazol-3-yl)phenyl)ethyl)(methyl)carbamate (850 mg, 1.369 mmol) as a starting intermediate to yield the title compound as a white solid. LCMS Retention time: 0.72 min [A], MS (E⁺) m/z: 391.4 [M+H]; ¹H NMR (400 MHz, DMSO-d₆) δ ppm 7.74 (d, J=1.0 Hz, 1H), 7.63 (s, 1H), 6.96-6.77 (m, 4H), 6.43(s, 1H), 3.68-3.56 (m, 1H), 3.33 (s, 3H), 2.35 (dt, J=14.1, 7.2 Hz, 1H), 1.85 (s, 3H), 0.94 (d, J=6.8 Hz, 3H), 0.51 (br. s., 1H), 0.42 (d, J=7.1 Hz, 6H).

### Example 570: (S)-N-ethyl-1-(4-(4-isopropyl-5-(8-methoxy-[1,2,4]triazolo[1,5-a]pyridin-6-yl)-1H-pyrazol-3-yl)phenyl)-N-methylethan-1-amine

A mixture of (S)-1-(4-(4-isopropyl-5-(8-methoxy-[1,2,4]triazolo[1,5-a]pyridin-6-yl)-1H-pyrazol -3-yl)phenyl)-N-methylethan-1-amine (40.0 mg, 0.102 mmol), acetaldehyde (0.029 mL, 0.512 mmol) and AcOH (1.173 µL, 0.020 mmol) in MeOH (3.0 mL) was stirred at room temperature for 8 h, and sodium cyanoborohydride (12.87 mg, 0.205 mmol) was added at 0 °C. The reaction mixture was stirred at room temperature for 16 h. The reaction mass was purified via preparative LC/MS using method AB, fractions containing the product were combined and dried via centrifugal evaporation to get (S)-N-ethyl-1-(4-(4-isopropyl-5-(8-methoxy-[1,2,4]triazolo[1,5-a]pyridin-6-yl)-1H-pyrazol-3-yl)phenyl)-N-methylethan-1-amine (12.4 mg) as an off-white solid. LCMS Retention time: 1.24 min [F], MS (E⁺) m/z: 419.3 [M+H]; ¹H NMR (400 MHz, METHANOL-d₄) δ 8.54 (d, *J=*1.0 Hz, 1H), 8.44 (s, 1H), 7.76-7.62 (m,4H), 7.22 (d, *J*=1.0 Hz, 1H), 4.73-4.65 (m, 2H), 4.12 (s, 3H), 3.41 (s, 1H), 3.20-3.08 (m, 2H),3.05-2.97 (m, 1H), 2.93-2.85 (m, 2H), 2.74 (s, 2H), 1.80 (dd, *J=*11.0, 7.0 Hz, 3H), 1.46-1.29(m, 4H), 1.22 (d, *J*=7.0 Hz, 6H).

The following Example was prepared according to the general procedure used to prepare Intermediate 570E.

**TABLE 58**

| Ex. No. | Structure | LCMS MH⁺ | RT (min) | HPLC Method |
|---|---|---|---|---|
| 571 | 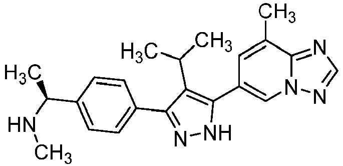 | 375.2 | 1.09 | C |

The following Examples were prepared according to the general procedure used to prepare Example 570.

**TABLE 59**

| Ex. No. | Structure | LCMS MH⁺ | RT (min) | HPLC Method |
|---|---|---|---|---|
| 572 | 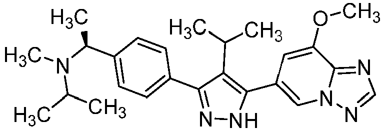 | 433.3 | 1.27 | C |
| 573 | 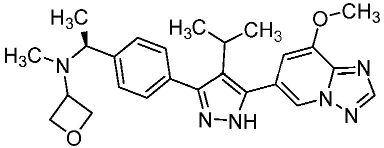 | 447.3 | 1.48 | C |
| 574 | 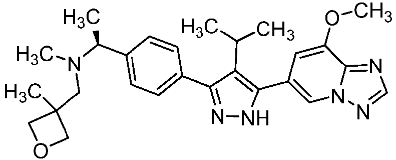 | 475.3 | 1.82 | C |
| 575 | 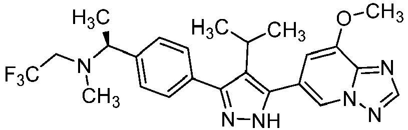 | 473.3 | 1.33 | C |
| 576 | 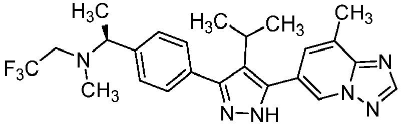 | 457.3 | 1.84 | C |
| 577 | 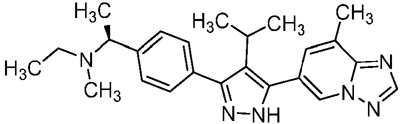 | 403.3 | 1.39 | C |
| 578 | 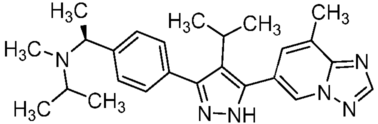 | 417.3 | 1.32 | C |
| 579 | 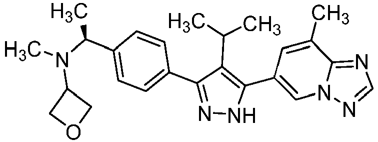 | 431.3 | 1.53 | C |
| 580 | 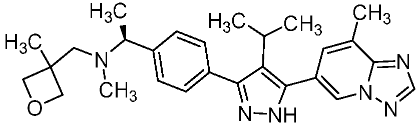 | 459.3 | 1.88 | C |

### EXAMPLE 581

### 2-(dimethylamino)-N-(2-(4-(4-isopropyl-5-(8-methyl-[1,2,4]triazolo[1,5-a]pyridin-6-yl)-1H-pyrazol-3-yl)phenyl)propan-2-yl)acetamide

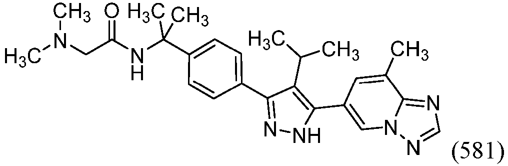

To a solution of 2-(4-(4-isopropyl-5-(8-methyl-[1,2,4]triazolo[1,5-a]pyridin-6-yl)-1H-pyrazol-3-yl)phenyl)propan-2-amine, TFA (0.014 g, 0.029 mmol) in DMF (1.00 mL) were added TEA (0.1 mL, 0.717 mmol), dimethylglycine (5.91 mg, 0.057 mmol) and HATU (0.229 g, 0.602 mmol) at room temperature. The reaction mixture was stirred at the same temperature for 2 h. The reaction mass was purified by Prep LCMS purification using method AB, the fractions containing product were combined and dried using Genevac centrifugal evaporator to get 2-(dimethylamino)-N-(2-(4-(4-isopropyl-5-(8-methyl-[1,2,4]triazolo[1,5-a]pyridin-6-yl)-1H-pyrazol-3-yl)phenyl)propan-2-yl)acetamide (6.0 mg, 0.013 mmol, 46% yield) as a pale solid. LCMS Retention time: 1.723 min [E], MS (E⁺) m/z: 460.4 [M+H]; ¹H NMR (400 MHz, METHANOL-d₄) δ ppm 8.65 (s, 1H), 8.37 (s, 1H), 7.56 (s, 1H), 7.49-7.41 (m, *J*=8.1 Hz, 2H), 7.41-7.31 (m, *J*=8.3 Hz, 2H), 3.04-2.93 (m, 3H), 2.60 (s, 3H), 2.30 (s, 6H), 1.63 (s, 6H), 1.08 (d, *J*=7.1 Hz, 6H).

The following Examples were prepared according to the general procedure used to prepare Example 581.

**TABLE 60**

| Ex. No. | Structure | LCMS MH⁺ | RT (min) | HPLC Method |
|---|---|---|---|---|
| 582 | 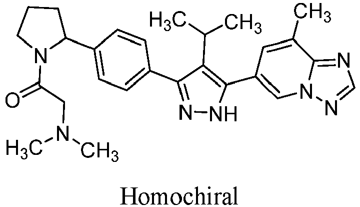 | 472.3 | 1.353 | E |
| 583 | 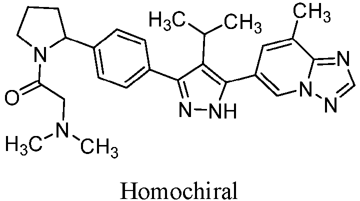 | 472.3 | 1.348 | E |

### EXAMPLE 584

### 2-(2-(4-(4-isopropyl-5-(8-methyl-[1,2,4]triazolo[1,5-a]pyridin-6-yl)-1H-pyrazol-3-yl) phenyl)pyrrolidin-1-yl)-N,N-dimethylacetamide

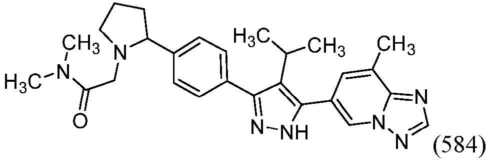

2-(2-(4-(4-isopropyl-5-(8-methyl-[1,2,4]triazolo[1,5-a]pyridin-6-yl)-1H-pyrazol-3-yl) phenyl)pyrrolidin-1-yl)-N,N-dimethylacetamide (21.5 mg) was prepared according to the general process described in Example 466, using 6-(4-isopropyl-3-(4-(pyrrolidin-2-yl)phenyl)-1H-pyrazol-5-yl)-8-methyl-[1,2,4]triazolo [1,5-a]pyridine (20 mg, 0.052 mmol) as a starting intermediate to yield the title compound as a white solid. LCMS Retention time: 1.626 min [E], MS (E⁺) m/z: 472.3 [M+H]; ¹H NMR (400 MHz, DMSO-d₆) δ 13.09 (br. s., 1H), 8.81 (br. s., 1H), 8.53 (s, 1H), 7.63 (s, 1H), 7.46 (s, 4H), 3.91 (s, 2H), 3.54 (br. s., 1H), 3.32 (br. s., 1H), 3.24 (br. s., 1H), 3.05 (dt, *J*=14.3, 7.0 Hz, 1H), 2.93 (d, *J=*11.7 Hz, 1H), 2.82 (s, 3H), 2.71 (s, 3H), 2.63 (s, 3H), 2.44 (d, *J*=8.1 Hz, 1H), 2.21 (br. s., 1H), 1.92 (s, 1H), 1.85 (br. s., 1H), 1.67 (br. s., 1H), 1.13 (dd, *J*=7.1, 3.2 Hz, 6H).

The following Example was prepared according to the general procedure used to prepare Example 584.

**TABLE 61**

| Ex. No. | Structure | LCMS MH⁺ | RT (min) | HPLC Method |
|---|---|---|---|---|
| | 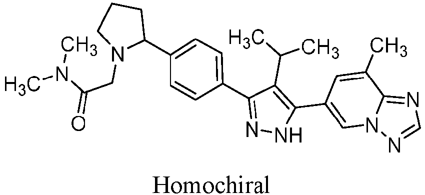 | 472.3 | 1.625 | E |

### EXAMPLE 585

### (S)-N-((S)-1-(4-(4-isopropyl-5-(8-methoxy-[1,2,4]triazolo[1,5-a]pyridin-6-yl)-1H-pyrazol-3-yl)phenyl)ethyl)-N,1-dimethylazetidine-2-carboxamide

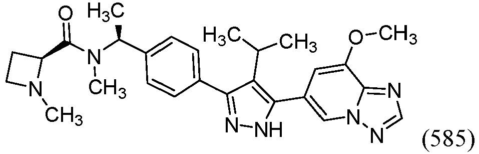

### Intermediate 585A: tert-butyl (S)-2-(((S)-1-(4-(4-isopropyl-5-(8-methoxy-[1,2,4]triazolo [1,5-a]pyridin-6-yl)-1H-pyrazol-3-yl)phenyl)ethyl)(methyl)carbamoyl)azetidine-1-carboxylate

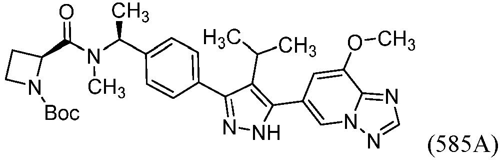

TEA (0.214 mL, 1.537 mmol) and HATU (140 mg, 0.369 mmol) were added to a solution of (S)-1-(4-(4-isopropyl-5-(8-methoxy-[1,2,4]triazolo[1,5-a]pyridin-6-yl)-1H-pyrazol-3-yl)phenyl)-N-methylethan-1-amine (120.0 mg, 0.307 mmol) and (S)-1-(tert-butoxycarbonyl)azetidine-2-carboxylic acid (61.8 mg, 0.307 mmol) in DMF (5.0 mL). The mixture was stirred at room temperature for 16 h. The reaction mass was diluted with water, extracted with 5% MeOH in DCM (2 x 50 ml), combined organic later was washed with brine, dried (Na₂SO₄) and concentrate to get tert-butyl (S)-2-(((S)-1-(4-(4-isopropyl-5-(8-methoxy-[1,2,4]triazolo[1,5-a]pyridin-6-yl)-1H-pyrazol-3-yl)phenyl)ethyl)(methyl) carbamoyl)azetidine-1-carboxylate (155 mg, 0.270 mmol, 88 % yield) as a gummy solid. LCMS Retention time: 1.14 min [A], MS (E⁺) m/z: 574.6 [M+H].

### Intermediate 585B: (S)-N-((S)-1-(4-(4-isopropyl-5-(8-methoxy-[1,2,4]triazolo[1,5-a] pyridin-6-yl)-1H-pyrazol-3-yl)phenyl)ethyl)-N-methylazetidine-2-carboxamide

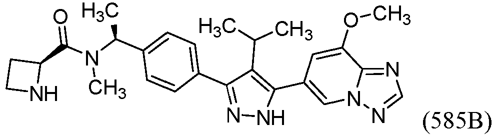

(S)-N-((S)-1-(4-(4-isopropyl-5-(8-methoxy-[1,2,4]triazolo[1,5-a]pyridin-6-yl)-1H-pyrazol-3-yl)phenyl)ethyl)-N-methylazetidine-2-carboxamide (2 mg) was prepared according to the general process described in Example 524, using tert-butyl (S)-2-(((S)-1-(4-(4-isopropyl-5-(8-methoxy-[1,2,4]triazolo[1,5-a] pyridin-6-yl)-1H-pyrazol-3-yl)phenyl)ethyl) (methyl)carbamoyl)azetidine-1-carboxylate (200 mg, 0.349 mmol) as a starting intermediate to yield the title compound as a white solid. LCMS Retention time: 1.19 min [E], MS (E⁺) m/z: 474.3 [M+H]; ¹H NMR (400 MHz, METHANOL-d₄) δ 8.54 (br. s., 1H), 8.44 (s, 1H), 7.67-7.37 (m, 4H), 7.25(br. s., 1H), 6.02 (d, *J*=6.6 Hz, 1H), 4.71 (d, *J*=7.6 Hz, 1H), 4.14 (s, 3H), 4.11-4.00 (m, 1H),3.81 (s, 1H), 3.73-3.62 (m, 1H), 3.58 (br. s., 1H), 3.16 (t, *J*=6.5 Hz, 2H), 2.88 (br. s., 2H), 2.80(s, 1H), 2.74-2.56 (m, 2H), 2.45 (br. s., 1H), 2.37 (br. s., 1H), 1.92 (s, 3H), 1.70 (d, *J*=6.6 Hz,1H), 1.66-1.53 (m, 2H), 1.31 (s, 2H), 1.22 (d, *J*=7.1 Hz, 6H).

### Example 585: (S)-N-((S)-1-(4-(4-isopropyl-5-(8-methoxy-[1,2,4]triazolo[1,5-a]pyridin-6-yl)-1H-pyrazol-3-yl)phenyl)ethyl)-N,1-dimethylazetidine-2-carboxamide

(S)-N-((S)-1-(4-(4-isopropyl-5-(8-methoxy-[1,2,4]triazolo[1,5-a]pyridin-6-yl)-1H-pyrazol-3-yl)phenyl)ethyl)-N,1-dimethylazetidine-2-carboxamide (25 mg) was prepared according to the general process described in Example 510, using (S)-N-((S)-1-(4-(4-isopropyl-5-(8-methoxy-[1,2,4]triazolo[1,5-a]pyridin-6-yl)-1H-pyrazol-3-yl)phenyl)ethyl)-N-methylazetidine-2-carboxamide (40.0 mg, 0.084 mmol) as a starting intermediate to yield the title compound as a white solid. LCMS Retention time: 1.22 min [E], MS (E⁺) m/z: 488.3 [M+H]; ¹H NMR (400 MHz, METHANOL-d₄) δ 8.61-8.50 (m, 1H), 8.44 (s, 1H), 7.62-7.33 (m, 4H),7.25 (s, 1H), 5.99 (d, *J*=7.1 Hz, 1H), 4.60 (d, *J*=18.1 Hz, 1H), 4.20-4.08 (m, 3H), 3.76 (br. s.,1H), 3.59-3.45 (m, 1H), 3.21-2.97 (m, 2H), 2.94-2.80 (m, 2H), 2.78-2.60 (m, 4H), 2.53 -2.31 (m, 2H), 2.20-2.00 (m, 2H), 1.99-1.92 (m, 3H), 1.76-1.56 (m, 3H), 1.54 (s, 2H), 1.38 -1.26 (m, 1H), 1.26-1.15 (m, 6H), 1.11 (br. s., 1H).

The following Examples were prepared according to the general procedure used to prepare Intermediate 585A.

**TABLE 62**

| Ex. No. | Structure | LCMS MH⁺ | RT (min) | HPLC Method |
|---|---|---|---|---|
| 586 | 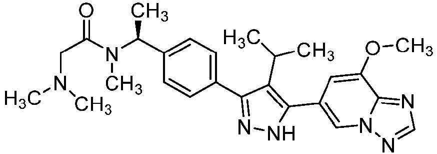 | 476.3 | 1.26 | C |
| 587 | 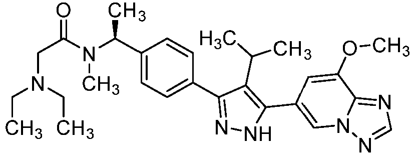 | 504.3 | 1.35 | C |
| 588 | 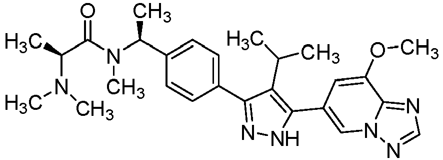 | 490.3 | 1.32 | C |
| 589 | 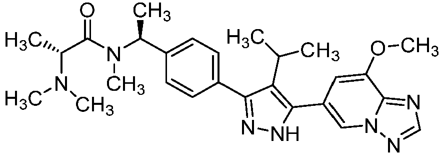 | 490.3 | 1.35 | C |
| 590 | 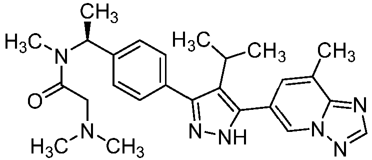 | 460.3 | 1.31 | C |
| 591 | 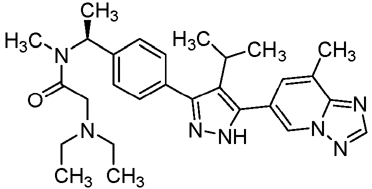 | 488.3 | 1.4 | C |
| 592 | 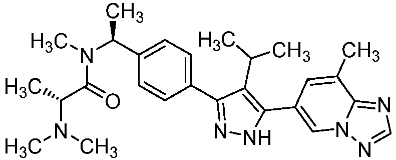 | 474.3 | 1.41 | C |
| 593 | 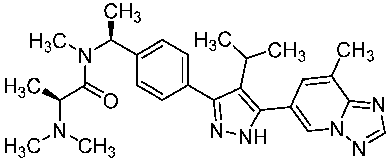 | 474.3 | 1.39 | C |

The following Examples were prepared according to the general procedure used to prepare Example 585B.

**TABLE 63**

| Ex. No. | Structure | LCMS MH⁺ | Ret Time (min) | HPLC Method |
|---|---|---|---|---|
| 594 | 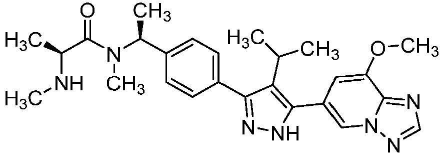 | 476.3 | 1.15 | D |
| 595 | 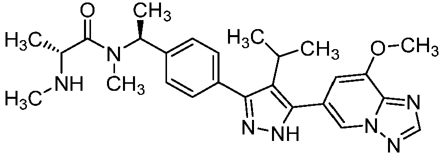 | 476.3 | 1.23 | C |
| 596 | 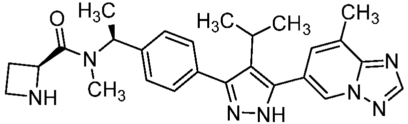 | 458.3 | 1.19 | C |
| 597 | 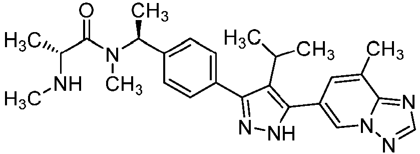 | 460.3 | 1.29 | C |
| 598 | 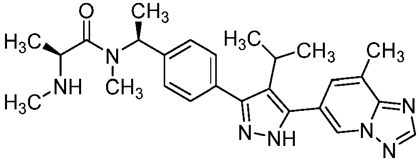 | 460.3 | 1.26 | C |
| 599 | 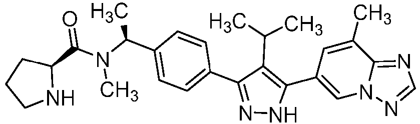 | 472.3 | 1.26 | C |
| 600 | 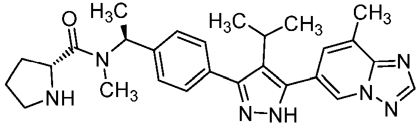 | 472.3 | 1.28 | C |

The following Examples were prepared according to the general procedure used to prepare Example 585.

**TABLE 64**

| Ex. No. | Structure | LCMS MH⁺ | RT (min) | HPLC Method |
|---|---|---|---|---|
| 601 | 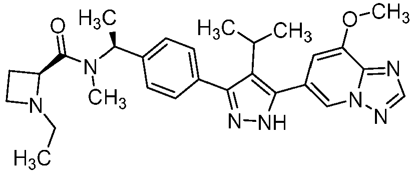 | 502.3 | 1.26 | C |
| 602 | 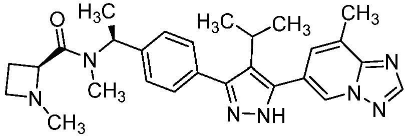 | 472.3 | 1.27 | C |
| 603 | 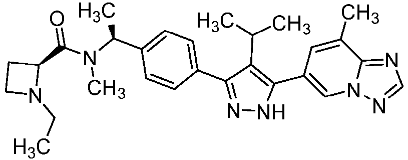 | 486.3 | 1.32 | C |
| 604 | 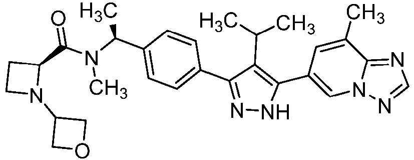 | 514.3 | 1.18 | D |
| 605 | 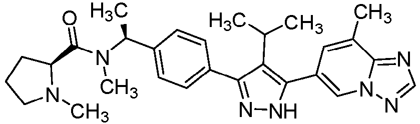 | 486.3 | 1.3 | C |
| 606 | 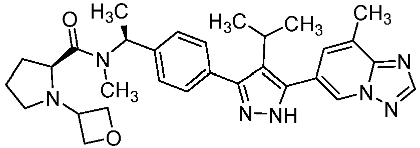 | 528.3 | 1.49 | C |
| 607 | 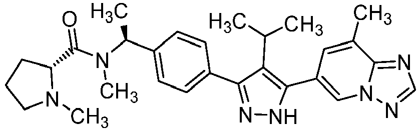 | 486.3 | 1.31 | C |
| 608 | 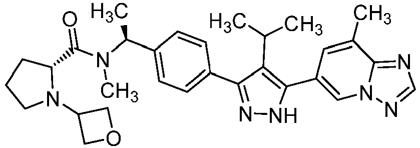 | 528.3 | 1.5 | C |
| 609 | 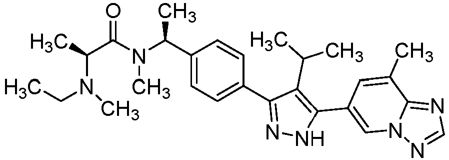 | 488.3 | 1.47 | C |
| 610 | 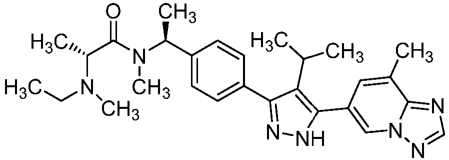 | 488.3 | 1.51 | C |
| 611 | 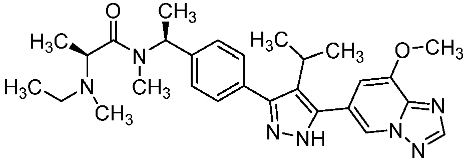 | 504.3 | 1.42 | C |
| 612 | 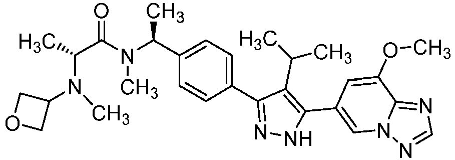 | 532.3 | 1.51 | C |
| 613 | 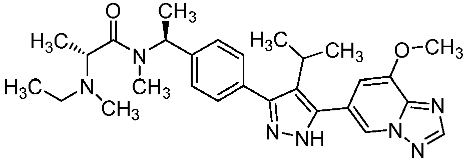 | 504.3 | 1.44 | C |
| 614 | 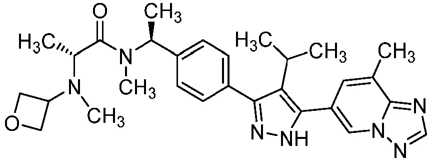 | 516.3 | 1.57 | C |
| 615 | 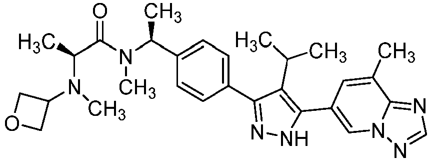 | 516.3 | 1.55 | C |

### EXAMPLE 616

### (S)-2-((1-(4-(4-isopropyl-5-(8-methyl-[1,2,4]triazolo[1,5-a]pyridin-6-yl)-1H-pyrazol-3-yl)phenyl)ethyl)(methyl)amino)-N,N-dimethylacetamide

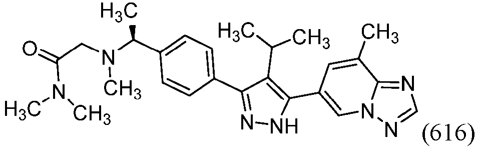

(S)-2-((1-(4-(4-isopropyl-5-(8-methyl-[1,2,4]triazolo[1,5-a]pyridin-6-yl)-1H-pyrazol-3-yl) phenyl)ethyl)(methyl)amino)-N,N-dimethylacetamide was prepared according to the general process described in Example 466 using (S)-1-(4-(4-isopropyl-5-(8-methyl-[1,2,4]triazolo[1,5-a]pyridin-6-yl)-1H-pyrazol-3-yl)phenyl)-N-methylethan-1-amine(40.0 mg, 0.107 mmol) as a starting intermediate to yield the title compound as an off-white solid. LCMS Retention time: 1.48 min.[C], MS (E⁺) m/z: 460.4 [M+H]; ¹H NMR (400 MHz, METHANOL-d₄) δ 8.76 (s, 1H), 8.48 (s, 1H), 7.68 (s, 1H), 7.53 (s, 4H),3.89 (q, *J*=6.6 Hz, 1H), 3.20-3.08 (m, 1H), 3.08-3.00 (m, 3H), 2.98-2.88 (m, 3H), 2.71 (s,3H), 2.33 (s, 3H), 2.06 (s, 2H), 1.97 (s, 1H), 1.49 (d, *J*=6.6 Hz, 3H), 1.31 (br. s., 1H), 1.20 (d, *J*=7.1 Hz, 6H).

The following Example was prepared according to the general procedure used to prepare Example 616.

**TABLE 65**

| Ex. No. | Structure | LCMS MH⁺ | Ret Time (min) | HPLC Method |
|---|---|---|---|---|
| 617 | 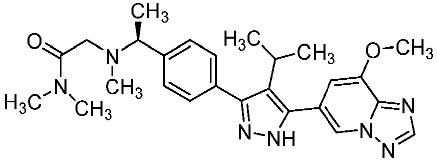 | 476.3 | 1.43 | C |

### EXAMPLE 618

### (S)-2-(dimethylamino)-N-(1-(4-(4-isopropyl-5-(8-methyl-[1,2,4]triazolo[1,5-a]pyridin-6-yl)-1H-pyrazol-3-yl)phenyl)ethyl)-N-(oxetan-3-yl)acetamide

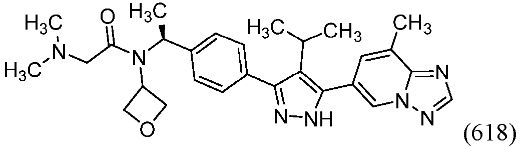

(S)-2-(dimethylamino)-N-(1-(4-(4-isopropyl-5-(8-methyl-[1,2,4]triazolo[1,5-a] pyridin-6-yl)-1H-pyrazol-3-yl)phenyl)ethyl)-N-(oxetan-3-yl)acetamide (11 mg) was prepared according to the general process described in Example 323 using (S)-N-(1-(4-(4-isopropyl-5-(8-methyl-[1,2,4]triazolo[1,5-a]pyridin-6-yl)-1H-pyrazol-3-yl)phenyl)ethyl) oxetan-3-amine (30.0 mg, 0.072 mmol) as a starting intermediate to yield the title compound as a white solid. LCMS Retention time: 1.12 min [F], MS (E⁺) m/z: 502.3 [M+H]; ¹H NMR (400 MHz, METHANOL-d₄) δ 8.77 (br. s., 1H), 8.49 (s, 1H), 7.79-7.51 (m, 5H), 5.53(d, *J*=6.8 Hz, 2H), 4.58 (br. s., 1H), 4.33 (br. s., 1H), 4.01 (dd, *J*=12.8, 6.0 Hz, 1H), 3.94-3.74(m, 3H), 3.70-3.61 (m, 1H), 3.36 (d, *J*=5.9 Hz, 3H), 3.28 (s, 2H), 3.23 (s, 2H), 3.13 (dd, *J*=13.0,7.1 Hz, 1H), 2.71 (s, 3H), 2.01-1.88 (m, 3H), 1.85 (d, *J*=7.1 Hz, 2H), 1.31 (s, 2H), 1.26-1.07(m, 6H), 0.92 (br. s., 1H).

The following Example was prepared according to the general procedure used to prepare Example 618.

**TABLE 66**

| Ex. No. | Structure | LCMS MH⁺ | RT (min) | HPLC Method |
|---|---|---|---|---|
| 619 | 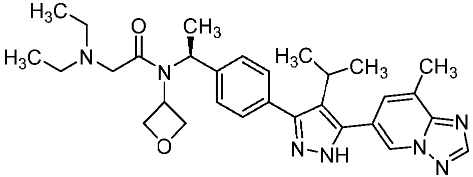 | 530.3 | 1.16 | C |

### EXAMPLE 620

### (S)-2-(ethyl(methyl)amino)-N-(1-(4-(4-isopropyl-5-(8-methyl-[1,2,4]triazolo[1,5-a] pyridin-6-yl)-1H-pyrazol-3-yl)phenyl)ethyl)-N-methylacetamide

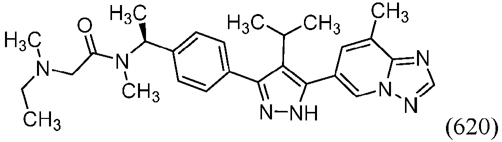

TEA (0.039 mL, 0.277 mmol) and N-methylethanamine (16.38 mg, 0.277 mmol) were added to a solution of (S)-2-chloro-N-(1-(4-(4-isopropyl-5-(8-methyl-[1,2,4]triazolo[1,5-a]pyridin-6-yl)-1H-pyrazol-3-yl)phenyl)ethyl)-N-methylacetamide (25.0 mg, 0.055 mmol) in DMF (1.5 mL) and THF (1.0 mL) solvent mixture at 0 °C. The mixture was stirred at room temperature for 16 h. The reaction mass was purified via preparative LC/MS using method AA, fractions containing the product were combined and dried via centrifugal evaporation to get (S)-2-(ethyl(methyl)amino)-N-(1-(4-(4-isopropyl-5-(8-methyl-[1,2,4]triazolo[1,5-a]pyridin-6-yl)-1H-pyrazol-3-yl)phenyl)ethyl)-N-methylacetamide (12.3 mg) as a white solid. LCMS Retention time: 1.36 min [E], MS (E⁺) m/z: 474.3 [M+H]; ¹H NMR (400 MHz, METHANOL-d₄) δ 8.77 (s, 1H), 8.49 (s, 1H), 7.67 (s, 1H), 7.62-7.41 (m,4H), 6.03 (d, *J*=7.3 Hz, 1H), 3.98-3.80 (m, 2H), 3.11 (d, *J*=7.1 Hz, 1H), 3.01 (d, *J*=6.6 Hz, 2H),2.88 (d, *J*=5.6 Hz, 1H), 2.82 (s, 2H), 2.79-2.66 (m, 6H), 2.61 (s, 1H), 1.97 (s, 3H), 1.73 (d, *J*=6.6 Hz, 1H), 1.63 (d, *J*=7.3 Hz, 2H), 1.36-1.09 (m, 10H).

The following Examples were prepared according to the general procedure used to prepare Example 620.

**TABLE 67**

| Ex. No. | Structure | LCMS MH⁺ | RT (min) | HPLC Method |
|---|---|---|---|---|
| 621 | 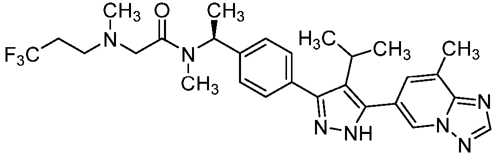 | 542.3 | 1.85 | C |
| 622 | 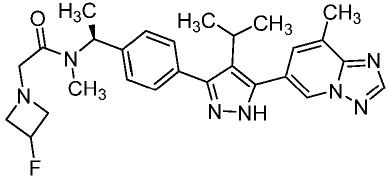 | 490.3 | 1.49 | C |
| 623 | 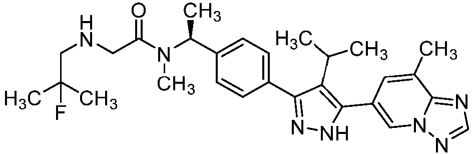 | 506.3 | 1.64 | C |

### EXAMPLE 624

### (S)-1-(2-fluoro-5-(4-isopropyl-5-(8-methyl-[1,2,4]triazolo[1,5-a]pyridin-6-yl)-1H-pyrazol-3-yl)phenyl)-N,N-dimethylethan-1-amine

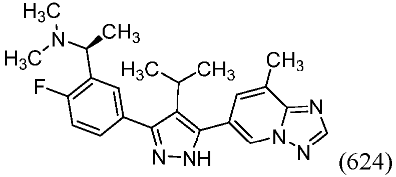

### Intermediate 624A: N-((S)-1-(2-fluoro-5-(4,4,5,5-tetramethyl-1,3,2-dioxaborolan-2-yl) phenyl)ethyl)-2-methylpropane-2-sulfinamide

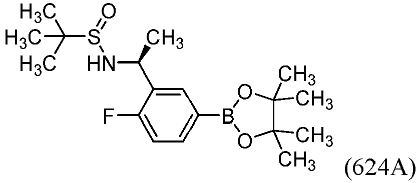

PdCl₂(dppf)-CH₂Cl₂ adduct (0.152 g, 0.186 mmol) and potassium acetate (0.548 g, 5.59 mmol) were added to a degassed solution of BISPIN (0.615 g, 2.421 mmol) and N-((S)-1-(5-bromo-2-fluorophenyl)ethyl)-2-methylpropane-2-sulfinamide (0.600 g, 1.862 mmol) in dioxane (25 mL). The reaction mixture was stirred at 100 °C for 14 h in a sealed tube. The reaction mixture was diluted with ethyl acetate, filtered and washed with excess ethyl acetate. The filtrates were collected, dried over sodium sulphate and concentrate to get crude compound. The crude compound was purified by ISCO using 40 g silica column, the compound was eluted with 45-50% ethyl acetate in pet ether, the fractions were collected and concentrated to get N-((S)-1-(2-fluoro-5-(4,4,5,5-tetramethyl-1,3,2-dioxaborolan-2-yl) phenyl)ethyl)-2-methylpropane-2-sulfinamide (680 mg, 1.841 mmol, 99% yield) as a light yellow solid. LCMS Retention time: 1.45 min [A], MS (E⁺) m/z: 370.3 [M+H].

### Intermediate 624B: N-((S)-1-(2-fluoro-5-(4-isopropyl-5-(8-methyl-[1,2,4]triazolo[1,5-a] pyridin-6-yl)-1-((2-(trimethylsilyl)ethoxy)methyl)-1H-pyrazol-3-yl)phenyl)ethyl)-2-methyl propane-2-sulfinamide

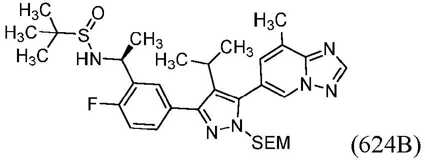

Pd₂(dba)₃ (61.0 mg, 0.067 mmol) and S-Phos (54.6 mg, 0.133 mmol) were added to a degassed solution of 6-(3-bromo-4-isopropyl-1-((2-(trimethylsilyl)ethoxy)methyl)-1H-pyrazol-5-yl)-8-methyl-[1,2,4]triazolo[1,5-a]pyridine (600 mg, 1.332 mmol), K₂CO₃ (552 mg, 4.00 mmol) and N-((S)-1-(2-fluoro-5-(4,4,5,5-tetramethyl-1,3,2-dioxaborolan-2-yl)phenyl)ethyl)-2-ethylpropane-2-sulfinamide (590 mg, 1.598 mmol) in acetonitrile (25 mL) and water (5 mL) solvent mixture. The reaction mixture was stirred at 110 °C for 14 h in a sealed tube. The reaction mixture was extracted with ethyl acetate, washed with water, brine, dried over sodium sulphate and concentrated to get crude compound. The crude compound was purified by ISCO using 40 g silica column, compound was eluted with 65-75% ethyl acetate in pet ether, the fractions were collected and concentrated to get N-((S)-1-(2-fluoro-5-(4-isopropyl-5-(8-methyl-[1,2,4]triazolo[1,5-a]pyridin-6-yl)-1-((2-(trimethylsilyl)ethoxy) methyl1H-pyrazol-3-yl)phenyl)ethyl)-2-methylpropane-2-sulfinamide (550 mg, 0.897 mmol, 67% yield) as a light brown semi-solid. LCMS Retention time: 1.75 min [A], MS (E⁺) m/z: 613.3 [M+H].

### Intermediate 624C: (S)-1-(2-fluoro-5-(4-isopropyl-5-(8-methyl-[1,2,4]triazolo[1,5-a] pyridin-6-yl)-1H-pyrazol-3-yl)phenyl)ethan-1-amine

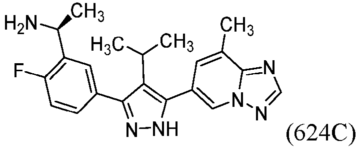

To a solution of N-((S)-1-(2-fluoro-5-(4-isopropyl-5-(8-methyl-[1,2,4]triazolo[1,5-a] pyridin-6-yl)-1-((2-(trimethylsilyl)ethoxy)methyl)-1H-pyrazol-3-yl)phenyl)ethyl)-2-methylpropane-2-sulfinamide (500 mg, 0.816 mmol) in DCM (5.0 mL) was added TFA (1.571 mL, 20.40 mmol) at 0 °C. The reaction mixture was stirred at room temperature for 12 h. The reaction mass was concentrated and dried in high vacuum to get crude compound. The crude compound was material was purified via preparative LC/MS using method AA, the fractions containing the product were combined and dried via centrifugal evaporation to get (S)-1-(2-fluoro-5-(4-isopropyl-5-(8-methyl-[1,2,4] triazolo[1,5-a]pyridin-6-yl)-1H-pyrazol-3-yl)phenyl)ethan-1-amine (8.5 mg). LCMS Retention time: 1.29 min [E], MS (E⁺) m/z: 379.2 [M+H]; ¹H NMR (400 MHz, METHANOL-d₄) δ 8.66 (s, 1H), 8.38 (s, 1H), 7.61-7.48 (m, 2H), 7.48 -7.41 (m, 1H), 7.28-7.15 (m, 1H), 4.57 (q, *J*=6.6 Hz, 1H), 3.00 (dt, *J=*14.1, 7.0 Hz, 1H), 2.66 -2.53 (m, 3H), 1.83 (s, 3H), 1.53 (d, *J*=6.7 Hz, 3H), 1.29-1.13 (m, 1H), 1.08 (d, *J*=4.6 Hz, 3H),1.10 (d, *J*=4.6 Hz, 3H).

### Example 624: (S)-1-(2-fluoro-5-(4-isopropyl-5-(8-methyl-[1,2,4]triazolo[1,5-a]pyridin-6-yl)-1H-pyrazol-3-yl)phenyl)-N,N-dimethylethan-1-amine

To a solution of (S)-1-(2-fluoro-5-(4-isopropyl-5-(8-methyl-[1,2,4]triazolo[1,5-a] pyridin-6-yl)-1H-pyrazol-3-yl)phenyl)ethan-1-amine (30.0 mg, 0.079 mmol) in MeOH (5.0 mL) were added formaldehyde (10.92 µl, 0.396 mmol) and acetic acid (0.908 µl, 0.016 mmol) at room temperature. The reaction mixture was stirred for 8 h. To this was added sodium cyanoborohydride (9.96 mg, 0.159 mmol) at 0 °C. The reaction mixture was stirred at room temperature for 16 h. The reaction mass was purified via preparative LC/MS using method AA, the fractions containing the product were combined and dried via centrifugal evaporation to get (S)-1-(2-fluoro-5-(4-isopropyl-5-(8-methyl-[1,2,4]triazolo[1,5-a]pyridin-6-yl)-1H-pyrazol-3-yl)phenyl)-N,N-dimethylethan-1-amine 10.5 mg as an off-white solid. LCMS Retention time: 1.55 min [E], MS (E⁺) m/z: 407.3 [M+H]; ¹H NMR (400 MHz, METHANOL-d₄) δ 8.65 (s, 1H), 8.37 (s, 1H), 7.55 (s, 1H), 7.50 (dd, *J*=6.8,1.8 Hz, 1H), 7.39 (td, *J*=5.4, 2.4 Hz, 1H), 7.26-7.11 (m, 1H), 3.95 (br. s., 1H), 2.99 (dt, *J*=14.2,7.2 Hz, 1H), 2.68-2.51 (m, 3H), 2.27 (s, 6H), 1.90-1.76 (m, 1H), 1.42 (d, *J*=6.7 Hz, 3H), 1.08(t, *J*=7.8 Hz, 6H).

The following Examples were prepared according to the general procedure used to prepare Example 624.

**TABLE 68**

| Ex. No. | Structure | LCMS MH⁺ | RT (min) | HPLC Method |
|---|---|---|---|---|
| 625 | 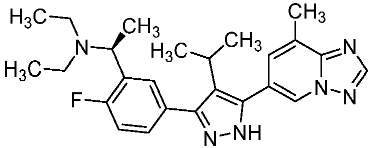 | 435.3 | 1.7 | C |
| 626 | 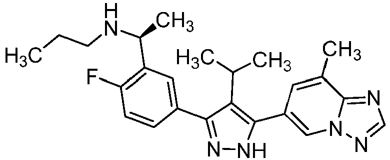 | 421.3 | 1.61 | C |
| 627 | 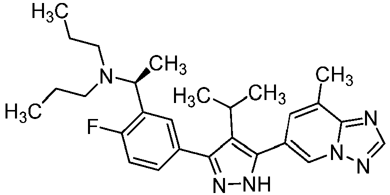 | 463.3 | 2.37 | C |
| 628 | 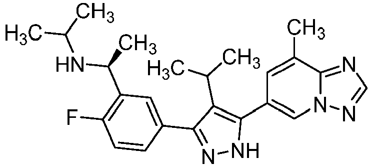 | 421.3 | 1.58 | C |

### EXAMPLE 629

### (S)-2-(dimethylamino)-N-(1-(2-fluoro-5-(4-isopropyl-5-(8-methyl-[1,2,4]triazolo[1,5-a] pyridin-6-yl)-1H-pyrazol-3-yl)phenyl)ethyl)acetamide

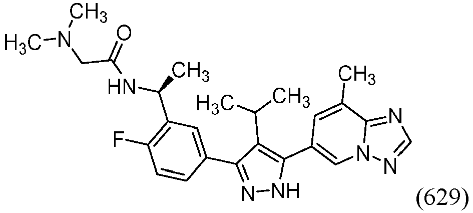

(S)-2-(dimethylamino)-N-(1-(2-fluoro-5-(4-isopropyl-5-(8-methyl-[1,2,4]triazolo[1,5-a]pyridin-6-yl)-1H-pyrazol-3-yl)phenyl)ethyl)acetamide (17.5 mg) was prepared according to the general process described in Example 469, using (S)-1-(2-fluoro-5-(4-isopropyl-5-(8-methyl-[1,2,4]triazolo[1,5-a]pyridin-6-yl)-1H-pyrazol-3-yl)phenyl)ethan-1-amine (30.0 mg, 0.079 mmol) as a starting intermediate to yield the title compound as a pale solid. LCMS Retention time: 1.52 min [E], MS (E⁺) m/z: 464.3 [M+H]; ¹H NMR (400 MHz, METHANOL-d₄) δ 8.65 (s, 1H), 8.39 (s, 1H), 7.54 (s, 1H), 7.41 (dd, *J*=7.1,1.7 Hz, 1H), 7.39-7.29 (m, 1H), 7.15 (dd, *J*=10.1, 8.6 Hz, 1H), 5.26 (q, *J*=7.0 Hz, 1H), 3.96 -3.75 (m, 2H), 2.98 (dt, *J*=14.2, 7.1 Hz, 1H), 2.89-2.79 (m, 5H), 2.76 (br. s., 1H), 2.60 (s, 3H),1.46 (d, *J*=7.0 Hz, 3H), 1.28-1.14 (m, 1H), 1.08 (t, *J*=6.6 Hz, 6H).

The following Example was prepared according to the general procedure used to prepare Example 629.

**TABLE 69**

| Ex. No. | Structure | LCMS MH⁺ | RT (min) | HPLC Method |
|---|---|---|---|---|
| 630 | 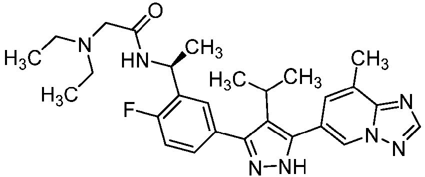 | 492.3 | 1.77 | C |

### EXAMPLE 631

### N-(1-(4-(4-isopropyl-5-(8-methyl-[1,2,4]triazolo[1,5-a]pyridin-6-yl)-1H-pyrazol-3-yl) phenyl)ethyl)propan-2-amine

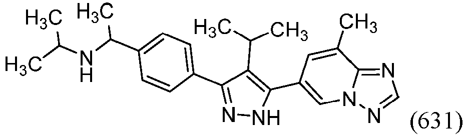

### Intermediate 631A: 1-(4-(4-isopropyl-5-(8-methyl-[1,2,4]triazolo[1,5-a]pyridin-6-yl)-1-((2-(trimethylsilyl)ethoxy)methyl)-1H-pyrazol-3-yl)phenyl)ethan-1-one

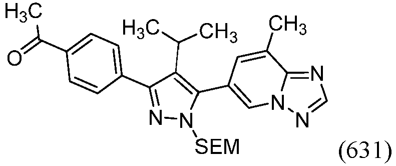

1-(4-(4-isopropyl-5-(8-methyl-[1,2,4]triazolo[1,5-a]pyridin-6-yl)-1-((2-(trimethylsilyl)ethoxy)methyl)-1H-pyrazol-3-yl)phenyl)ethan-1-one (350 mg, 0.715 mmol, 71% yield) was prepared according to the general process described in Intermediate 307B, using 6-(4-isopropyl-3-(4,4,5,5-tetramethyl-1,3,2-dioxaborolan-2-yl)-1-((2-(trimethylsilyl) ethoxy)methyl)-1H-pyrazol-5-yl)-8-methyl-[1,2,4] triazolo[1,5-a]pyridine (0.500 g, 1.005 mmol) as a starting intermediate to yield the title compound as a pale yellow solid. LCMS Retention time: 1.61 min [A], MS (E⁺) m/z: 490.6 [M+H].

### Intermediate 631B: N-(1-(4-(4-isopropyl-5-(8-methyl-[1,2,4]triazolo[1,5-a]pyridin-6-yl)-1-((2-(trimethylsilyl)ethoxy)methyl)-1H-pyrazol-3-yl)phenyl)ethyl)propan-2-amine

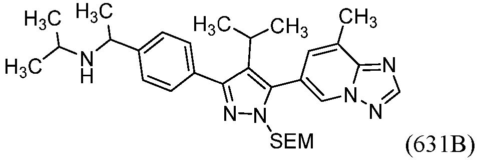

N-(1-(4-(4-isopropyl-5-(8-methyl-[1,2,4]triazolo[1,5-a]pyiridin-6-yl)-1-((2-(trimethylsilyl)ethoxy)methyl)-1H-pyrazol-3-yl)phenyl)ethyl)propan-2-amine was prepared according to the general process described in Example 279, using 1-(4-(4-isopropyl-5-(8-methyl-[1,2,4]triazolo[1,5-a]pyridin-6-yl)-1-((2-(trimethylsilyl)ethoxy)methyl)-1H-pyrazol-3-yl)phenyl)ethan-1-one (0.040 g, 0.082 mmol) and propan-2-amine (0.024 g, 0.408 mmol) as starting intermediates to yield the title compound as a pale yellow solid. LCMS Retention time: 1.62 min [A], MS (E⁺) m/z: 533.7 [M+H].

### Example 631: N-(1-(4-(4-isopropyl-5-(8-methyl-[1,2,4]triazolo[1,5-a]pyridin-6-yl)-1H-pyrazol-3-yl)phenyl)ethyl)propan-2-amine

To a solution of N-(1-(4-(4-isopropyl-5-(8-methyl-[1,2,4]triazolo[1,5-a]pyridin-6-yl)-1-((2-(trimethylsilyl)ethoxy)methyl)-1H-pyrazol-3-yl)phenyl)ethyl)propan-2-amine (0.040 g, 0.075 mmol) in CH₂Cl₂ (2.0 mL) at 0 °C was added TFA (0.145 mL, 1.877 mmol). The reaction mixture was stirred at room temperature for 12 h. The reaction mass was concentrated to get crude compound. The crude compound was purified via preparative LC/MS using method AA, the fractions containing the product were combined and dried via centrifugal evaporation to get N-(1-(4-(4-isopropyl-5-(8-methyl-[1,2,4]triazolo[1,5-a]pyridin-6-yl)-1H-pyrazol-3-yl)phenyl)ethyl)propan-2-amine (28 mg) as a white solid. LCMS Retention time: 1.18 min [E], MS (E⁺) m/z: 403.3 [M+H]; ¹H NMR (400 MHz, METHANOL-d₄) δ 8.76 (s, 1H), 8.48 (s, 1H), 7.67 (s, 1H), 7.61-7.49 (m, 4H), 4.29 (d, *J*=6.5 Hz, 1H), 3.13 (quin, *J*=7.0 Hz, 1H), 2.99-2.82 (m, 1H), 2.71 (s, 3H), 1.99-1.86 (m, 1H), 1.57 (d, *J*=7.0 Hz, 3H), 1.26-1.14 (m, 13H).

The following Examples were prepared according to the general procedure used to prepare Example 631.

**TABLE 70**

| Ex. No. | Structure | LCMS MH⁺ | RT (min) | HPLC Method |
|---|---|---|---|---|
| 632 | 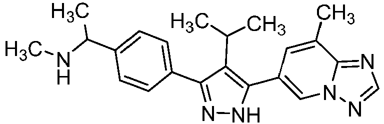 | 375.2 | 1.02 | C |
| 633 | 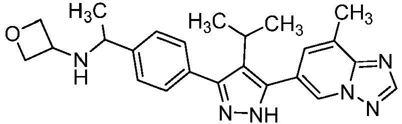 | 417.2 | 1.32 | C |
| 634 | 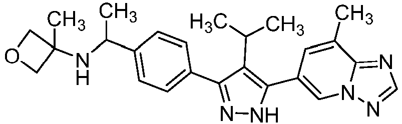 | 431.3 | 1.41 | C |
| 635 | 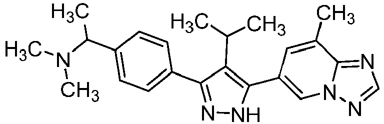 | 389.3 | 1.27 | E |
| 636 | 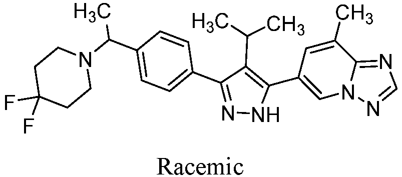 | 465.3 | 2.02 | E |
| 637 | 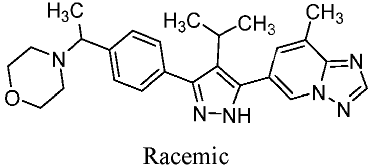 | 431.3 | 1.63 | E |
| 638 | 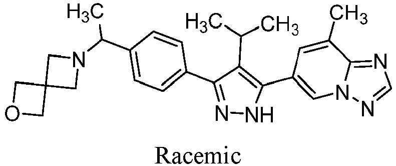 | 443.3 | 1.4 | E |
| 639 | 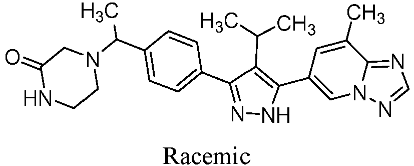 | 444.3 | 1.73 | E |
| 640 | 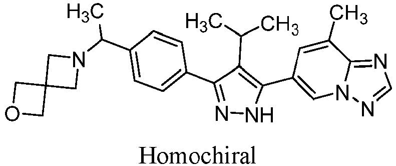 | 443.3 | 1.4 | E |
| 641 | 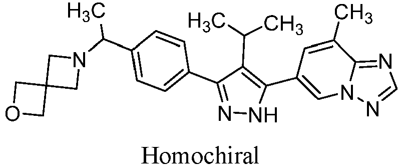 | 443.3 | 1.4 | E |
| 642 | 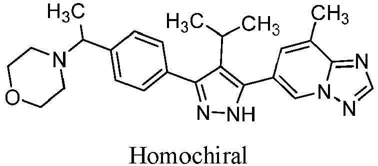 | 431.3 | 1.63 | E |
| 643 | 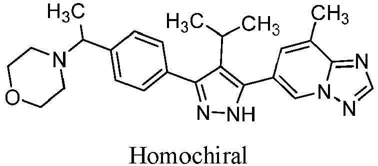 | 431.3 | 1.63 | E |
| 644 | 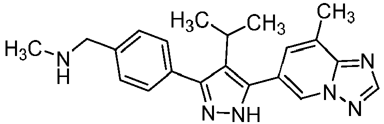 | 361.2 | 0.96 | C |
| 645 | 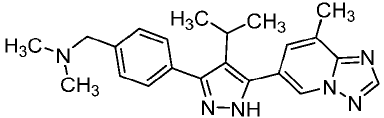 | 375.2 | 1.18 | C |
| 646 | 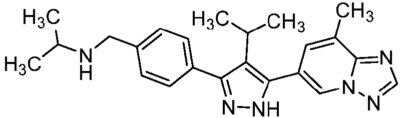 | 389.3 | 1.09 | C |
| 647 | 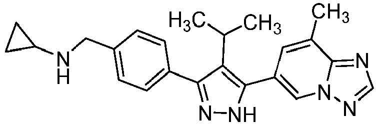 | 387.3 | 1.45 | C |
| 648 | 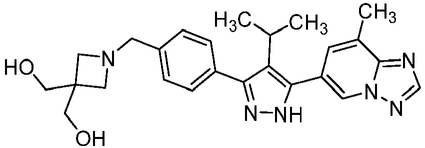 | 447.3 | 1.04 | C |
| 649 | 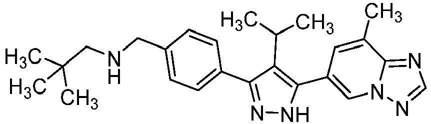 | 417.3 | 1.65 | C |
| 650 | 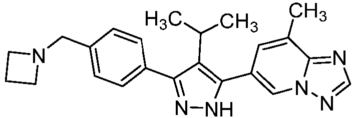 | 387.2 | 1.16 | C |
| 651 | 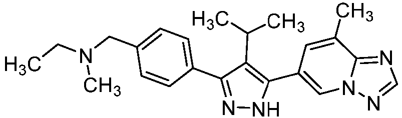 | 389.2 | 1.3 | C |
| 652 | 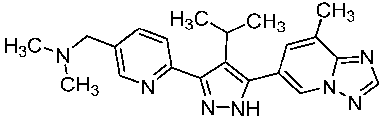 | 376.3 | 1.16 | E |
| 653 | 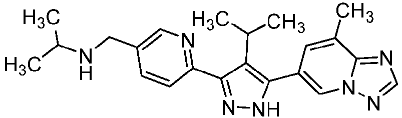 | 390.3 | 1.07 | E |
| 654 | 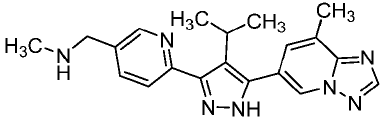 | 362.3 | 0.9 | E |
| 655 | 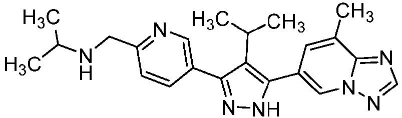 | 390.3 | 1.03 | E |
| 656 | 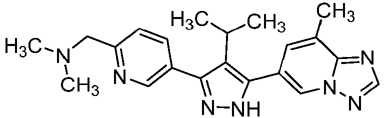 | 376.3 | 1.03 | E |
| 657 | 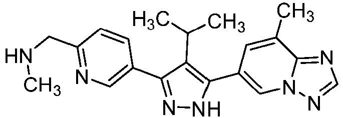 | 362.3 | 0.88 | E |
| 658 | 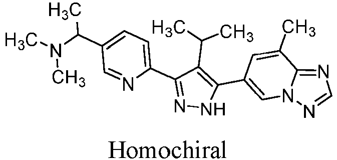 | 390.3 | 1.32 | C |
| 659 | 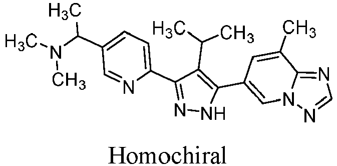 | 390.3 | 1.31 | C |
| 660 | 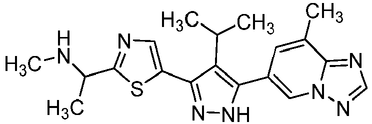 | 382.3 | 1.28 | E |
| 661 | 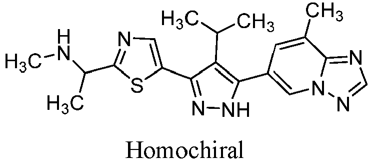 | 382.3 | 1.22 | E |
| 662 | 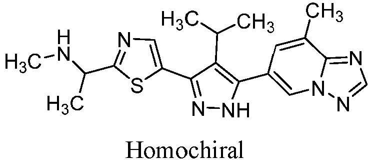 | 382.3 | 1.22 | E |
| 663 | 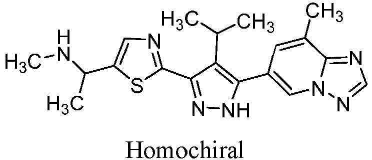 | 382.3 | 1.25 | E |
| 664 | 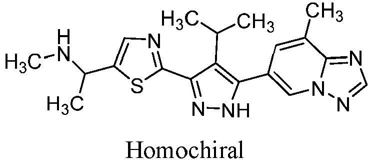 | 382.3 | 1.25 | E |
| 665 | 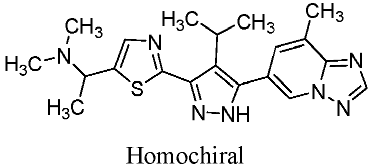 | 396.2 | 1.49 | E |
| 666 | 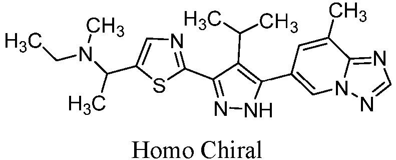 | 410.2 | 1.64 | E |
| 667 | 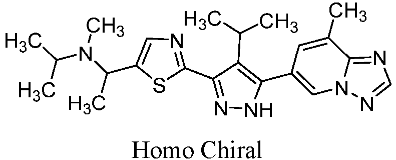 | 424.2 | 1.78 | E |
| 668 | 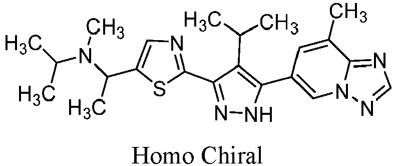 | 424.2 | 1.78 | E |
| 669 | 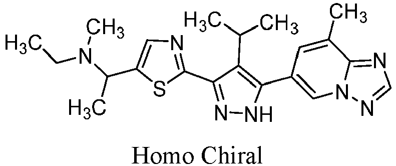 | 410.2 | 1.63 | E |
| 670 | 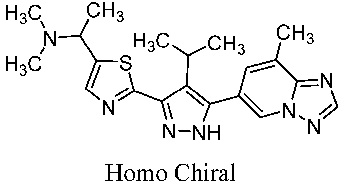 | 396.2 | 1.5 | E |
| 671 | 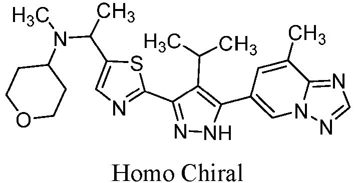 | 466.2 | 1.7 | E |

### EXAMPLE 672

### 2-(dimethylamino)-N-(1-(6-(4-isopropyl-5-(8-methyl-[1,2,4]triazolo[1,5-a]pyridin-6-yl)-1H-pyrazol-3-yl)pyridin-3-yl)ethyl)-N-methylacetamide

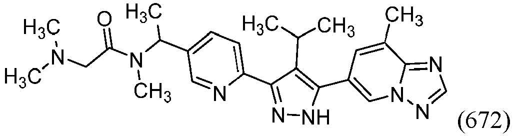

2-(Dimethylamino)-N-(1-(6-(4-isopropyl-5-(8-methyl-[1,2,4]triazolo[1,5-a] pyridin-6-yl)-1H-pyrazol-3-yl)pyridin-3-yl)ethyl)-N-methylacetamide (17 .3 mg) was prepared according to the general process described in Example 469, using 1-(6-(4-isopropyl-5-(8-methyl-[1,2,4]triazolo[1,5-a]pyridin-6-yl)-1H-pyrazol-3-yl)pyridin-3-yl)-N-methylethan-1-amine (20.0 mg, 0.053 mmol) as a starting intermediate to get the title compound as a white solid. LCMS Retention time: 1.21min [E], MS (E⁺) m/z: 461.3 [M+H]; ¹H NMR (400 MHz, METHANOL-d₄) δ 8.66 (s, 1H), 8.61-8.51 (m, 1H), 8.37 (s, 1H), 7.78 (d, *J*=9.0 Hz, 1H), 7.66 (br. s., 1H), 7.54 (s, 1H), 5.92 (q, *J*=7.1 Hz, 1H), 3.64-3.43 (m, 2H), 3.26(d, *J*=9.0 Hz, 1H), 2.82-2.69 (m, 3H), 2.68-2.55 (m, 4H), 2.48 (s, 5H), 2.39 (s, 2H), 1.84 (s,2H), 1.63 (d, *J*=6.8 Hz, 1H), 1.54 (d, *J*=7.1 Hz, 2H), 1.27-1.08 (m, 7H).

The following Examples were prepared according to the general procedure used to prepare Example 672.

**TABLE 71**

| Ex. No. | Structure | LCMS MH⁺ | RT (min) | HPLC Method |
|---|---|---|---|---|
| 673 | 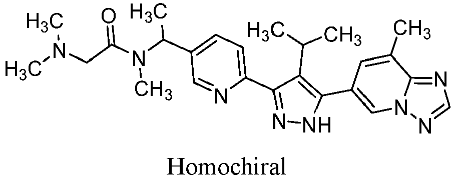 | 461.3 | 1.2 | C |
| 674 | 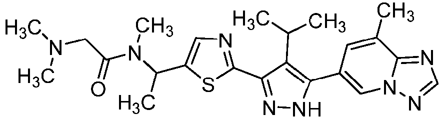 | 467.3 | 1.33 | E |
| | 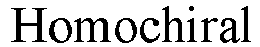 | | | |
| 675 | 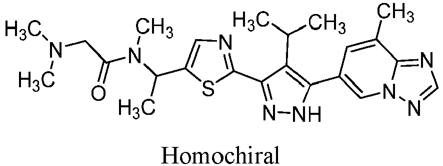 | 467.3 | 1.33 | E |

### EXAMPLE 676

### 2-((1-(2-(4-isopropyl-5-(8-methyl-[1,2,4]triazolo[1,5-a]pyridin-6-yl)-1H-pyrazol-3-yl) thiazol-5-yl)ethyl)(methyl)amino)-N,N-dimethylacetamide

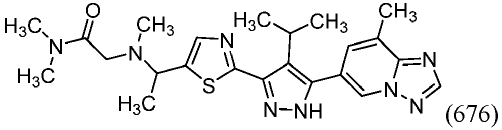

To a stirred solution of 1-(2-(4-isopropyl-5-(8-methyl-[1,2,4]triazolo[1,5-a] pyridin-6-yl)-1H-pyrazol-3-yl)thiazol-5-yl)-N-methylethan-1-amine (15 mg, 0.039 mmol) in DMF (2 mL) was added DIPEA (0.021 mL, 0.118 mmol). The reaction mixture was stirred at room temperature for 5 min, and 2-chloro-N,N-dimethylacetamide (5.74 mg, 0.047 mmol) was added. The reaction mixture was stirred for 16 h. The reaction mass was purified by Prep LCMS using method AA, the fractions containing the product were combined and dried via centrifugal evaporation to get 2-((1-(2-(4-isopropyl-5-(8-methyl-[1,2,4]triazolo[1,5-a] pyridin-6-yl)-1H-pyrazol-3-yl)thiazol-5-yl)ethyl)(methyl)amino)-N,N-dimethylacetamide (1.5 mg) as a pale solid. LCMS Retention time: 1.519 min [E], MS (E⁺) m/z: 467.3 [M+H]; ¹H NMR (400 MHz, DMSO-d₆) δ 8.94 (s, 1H), 8.56 (s, 1H), 7.71 (s, 1H), 7.56 (s, 1H), 4.22 (br. s., 1H), 3.35 (br. s., 3H), 3.05 (s, 3H), 2.83 (s, 3H), 2.65-2.60 (m, 3H), 2.16 (s, 3H), 1.86 (s, 18H), 1.76 (s, 1H), 1.41 (d, *J*=6.7 Hz, 3H), 1.34-1.16 (m, 7H).

The following Examples were prepared according to the general procedure used to prepare Example 676.

**TABLE 72**

| Ex. No. | Structure | LCMS MH⁺ | Ret Time (min) | HPLC Method |
|---|---|---|---|---|
| 677 | 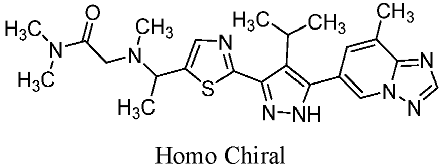 | 467.3 | 1.51 | E |
| 678 | 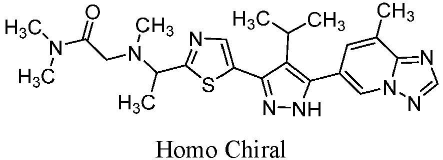 | 467.3 | 1.38 | E |

### EXAMPLE 679

### 6-(4-isopropyl-5-(8-methyl-[1,2,4]triazolo[1,5-a]pyridin-6-yl)-1H-pyrazol-3-yl)-N,N-dimethyl-1,2,3,4-tetrahydronaphthalen-2-amine

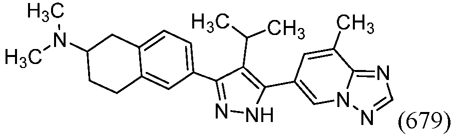

### Intermediate 679A: 6-bromo-N-methyl-1,2,3,4-tetrahydronaphthalen-2-amine

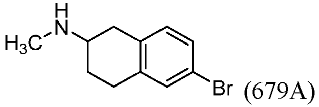

To a stirred solution of 6-bromo-3,4-dihydronaphthalen-2(1H)-one (2 g, 8.89 mmol) in MeOH (100 mL) were added methanamine (44.4 mL, 89 mmol) and AcOH (5 mL). The reaction mixture was stirred at room temperature for 16 h, and NaCNBH₃ (1.675 g, 26.7 mmol) was added. The reaction mixture was stirred at room temperature for 2 h. The reaction was quenched with water. The mixture was extracted with EtOAc, washed with water, brine, dried over sodium sulphate and concentrated to get 6-bromo-N-methyl-1,2,3,4-tetrahydronaphthalen-2-amine (1.3 g, 0.376 mmol, 63% yield) as an off-white solid. LCMS Retention time: 0.84 [A], MS (E⁺) m/z: 240.4 [M+H].

### Intermediate 679B: tert-butyl (6-bromo-1,2,3,4-tetrahydronaphthalen-2-yl)(methyl) carbamate

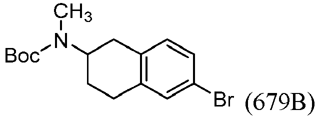

To a stirred solution of 6-bromo-N-methyl-1,2,3,4-tetrahydronaphthalen-2-amine (2 g, 8.33 mmol) in THF (50 mL) were added Boc-anhydride (1.934 mL, 8.33 mmol), TEA (1.161 mL, 8.33 mmol) and DMAP (1.017 g, 8.33 mmol) at room temperature. The reaction mixture was stirred for 3 h. The reaction mixture was extracted with EtOAc, washed with water, brine, dried over sodium sulphate and concentrated to get crude compound. The crude compound was purified by ISCO using 24 g silica column, the fractions were collected and concentrated to get 6-bromo-N-methyl-1,2,3,4-tetrahydronaphthalen-2-amine. (1.3 g, 0.376 mmol, 63 % yield) as an off-white solid. LCMS Retention time: 1.76 [A], MS (E⁻) m/z: 337.2 [M+H].

### Intermediate 679C: tert-butyl (6-(4-isopropyl-5-(8-methyl-[1,2,4]triazolo[1,5-a]pyridin-6-yl)-1-((2-(trimethylsilyl)ethoxy)methyl)-1H-pyrazol-3-yl)-1,2,3,4-tetrahydronaphthalen-2-yl) (methyl)carbamate

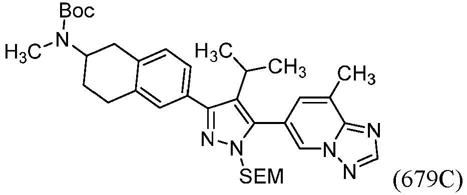

tert-Butyl (6-(4-isopropyl-5-(8-methyl-[1,2,4]triazolo[1,5-a]pyridin-6-yl)-1-((2-(trimethyl silyl)ethoxy)methyl)-1H-pyrazol-3-yl)-1,2,3,4-tetrahydronaphthalen-2-yl) (methyl)carbamate (280 mg, 0.444 mmol, 88% yield) was prepared according to the general process described Intermediate 307B using 6-(4-isopropyl-3-(4,4,5,5-tetramethyl-1,3,2-dioxaborolan-2-yl)-1-((2-(trimethylsilyl)ethoxy) methyl)-1H-pyrazol-5-yl)-8-methyl-[1,2,4]triazolo[1,5-a]pyridine (500 mg, 1.005 mmol) as a starting intermediate to yield the title compound as a brown liquid. LCMS Retention time: 2.13 [B], MS (E⁻) m/z: 631.8 [M+H].

### Intermediate 679D: 6-(4-isopropyl-5-(8-methyl-[1,2,4]triazolo[1,5-a]pyridin-6-yl)-1H-pyrazol-3-yl)-N-methyl-1,2,3,4-tetrahydronaphthalen-2-amine

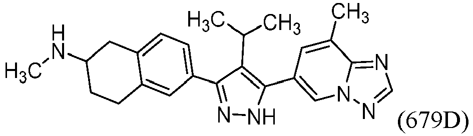

6-(4-isopropyl-5-(8-methyl-[1,2,4]triazolo[1,5-a]pyridin-6-yl)-1H-pyrazol-3-yl)-N-methyl-1,2,3,4-tetrahydronaphthalen-2-amine (8.3 mg, 0.126 mmol, 9 % yield) was prepared according to the general process described in Intermediate 307D, using tert-butyl (6-(4-isopropyl-5-(8-methyl-[1,2,4]triazolo[1,5-a]pyridin-6-yl)-1-((2-(trimethylsilyl)ethoxy) methyl)-1H-pyrazol-3-yl)-1,2,3,4-tetrahydronaphthalen-2-yl)(methyl)carbamate (230 mg, 0.365 mmol) as a starting intermediate to yield the title compound as an off-white solid. LCMS Retention time: 1.16 [E], MS (E⁻) m/z: 401.3 [M+H]; ¹H NMR (400 MHz, DMSO-d) δ ppm 8.78 (s, 1H), 8.51 (s, 1H), 7.60 (s, 1H), 7.24-7.16 (m, 3H),3.17 (s, 2H), 3.12-2.97 (m, 3H), 2.97-2.80 (m, 3H), 2.61 (s, 3H), 2.44 (s, 3H), 2.05 (s, 2H),1.80 (s, 2H), 1.16-1.05 (m, 6H).

### Example 679: 6-(4-isopropyl-5-(8-methyl-[1,2,4]triazolo[1,5-a]pyridin-6-yl)-1H-pyrazol-3-yl)-N,N-dimethyl-1,2,3,4-tetrahydronaphthalen-2-amine

6-(4-isopropyl-5-(8-methyl-[1,2,4]triazolo[1,5-a]pyridin-6-yl)-1H-pyrazol-3-yl)-N,N-dimethyl-1,2,3,4-tetrahydronaphthalen-2-amine (4.0 mg, 0.126 mmol, 8.57 % yield) was prepared according to the general process described in Intermediate 279 using 6-(4-isopropyl-5-(8-methyl-[1,2,4]triazolo[1,5-a]pyridin-6-yl)-1H-pyrazol-3-yl)-N-methyl-1,2,3,4-tetrahydronaphthalen-2-amine (30 mg, 0.075 mmol) as a starting intermediate to yield the title compound as an off-white solid. LCMS Retention time: 1.25[E], MS (E⁻) m/z: 415.3 [M+H]; ¹H NMR (400 MHz, DMSO-d₆) δ 13.03 (br. s., 1H), 8.79 (br. s., 1H), 8.52 (s, 1H), 7.61 (s, 1H),7.22 (d, J=6.8 Hz, 3H), 3.90 (s, 2H), 3.08-2.87 (m, 4H), 2.87-2.74 (m, 3H), 2.61 (s, 3H), 2.41(br. s., 5H), 2.33 (d, J=1.7 Hz, 1H), 2.09 (d, J=11.5 Hz, 1H), 1.67 (br. s., 1H), 1.12 (d, J=7.1 Hz,6H).

The following Examples were prepared according to the general procedure used to prepare Example 679D.

**TABLE 73**

| Ex. No. | Structure | LCMS MH⁺ | RT (min) | HPLC Method |
|---|---|---|---|---|
| 680 | 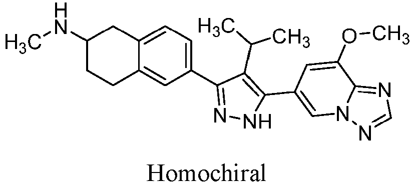 | 417.2 | 0.99 | E |
| 681 | 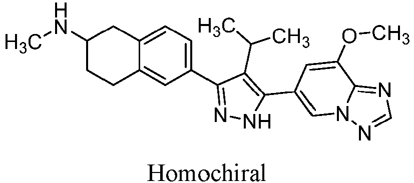 | 417.2 | 0.98 | E |

The following Examples were prepared according to the general procedure used to prepare Example 679.

**TABLE 74**

| Ex. No. | Structure | LCMS MH⁺ | RT (min) | HPLC Method |
|---|---|---|---|---|
| 682 | 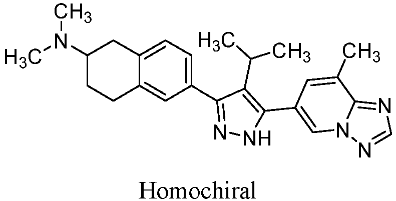 | 415.3 | 1.25 | E |
| 683 | 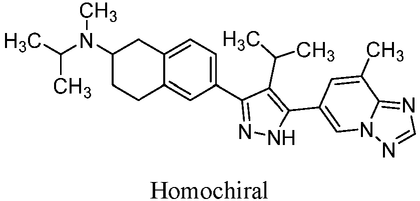 | 443.3 | 1.36 | E |
| 684 | 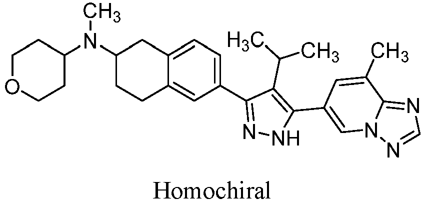 | 485.3 | 1.41 | E |
| 685 | 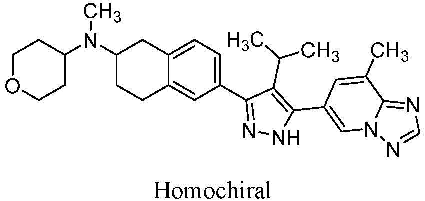 | 485.2 | 1.41 | E |
| 686 | 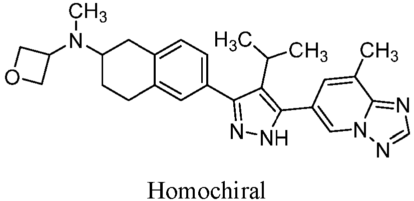 | 457.3 | 1.59 | E |
| 687 | 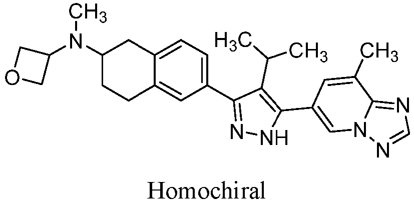 | 457.2 | 1.59 | E |
| 688 | 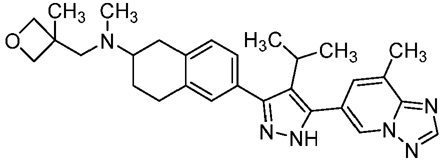 | 485.2 | 1.81 | E |
| 689 | 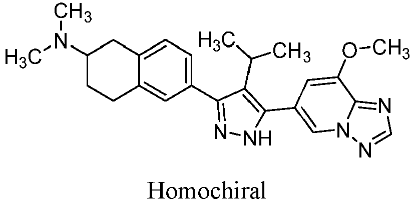 | 431.2 | 1.08 | E |

### EXAMPLE 690

### 2-(dimethylamino)-N-(6-(4-isopropyl-5-(8-methyl-[1,2,4]triazolo[1,5-a]pyridin-6-yl)-1H-pyrazol-3-yl)-1,2,3,4-tetrahydronaphthalen-2-yl)-N-methylacetamide

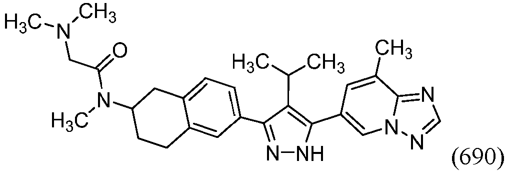

A solution of 6-(4-isopropyl-5-(8-methyl-[1,2,4]triazolo[1,5-a]pyridin-6-yl)-1H-pyrazol-3-yl)-N-methyl-1,2,3,4-tetrahydronaphthalen-2-amine (30 mg, 0.075 mmol) in DMF (2 mL) were added dimethylglycine (7.72 mg, 0.075 mmol), HATU (28.5 mg, 0.075 mmol) and DIPEA (0.013 mL, 0.075 mmol) at room temperature was stirred for 16 h. The reaction mass was purified by prep LCMS using method AA, the fractions containing the product were combined and dried via centrifugal evaporation to get 2-(dimethylamino)-N-(6-(4-isopropyl-5-(8-methyl-[1,2,4]triazolo[1,5-a]pyridin-6-yl)-1H-pyrazol-3-yl)-1,2,3,4-tetrahydronaphthalen-2-yl)-N-methylacetamide as a pale solid. LCMS Retention time: 1.37 min [E], MS (E⁺) m/z: 486.3 [M+H]; ¹H NMR (400 MHz, DMSO-d₆) δ 13.05 (br. s., 1H), 8.79 (br. s., 1H), 8.52 (s, 1H), 7.61 (s, 1H),7.24 (d, J=4.2 Hz, 3H), 4.65 (br. s., 1H), 3.90 (s, 2H), 3.26 (br. s., 2H), 3.17 (br. s., 1H), 3.10 -2.93 (m, 5H), 2.93-2.84 (m, 1H), 2.80 (s, 1H), 2.72 (dd, J=11.4, 5.0 Hz, 1H), 2.61 (s, 3H), 2.40-2.16 (m, 6H), 2.04-1.93 (m, 1H), 1.91 (s, 1H), 1.81 (br. s., 1H), 1.13 (d, J=7.1 Hz, 6H).

The following Examples were prepared according to the general procedure used to prepare Example 690.

**TABLE 75**

| Ex. No. | Structure | LCMS MH⁺ | RT (min) | HPLC Method |
|---|---|---|---|---|
| 691 | 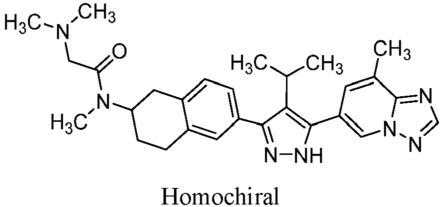 | 486.3 | 1.35 | E |

### EXAMPLE 692

### 2-((6-(4-isopropyl-5-(8-methyl-[1,2,4]triazolo[1,5-a]pyridin-6-yl)-1H-pyrazol-3-yl)-1,2,3,4-tetrahydronaphthalen-2-yl)(methyl)amino)-N,N-dimethylacetamide

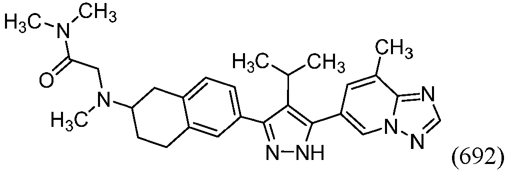

2-((6-(4-isopropyl-5-(8-methyl-[1,2,4]triazolo[1,5-a]pyridin-6-yl)-1H-pyrazol-3-yl)-1,2,3,4-tetrahydronaphthalen-2-yl)(methyl)amino)-N,N-dimethylacetamide was prepared according to the general process described in Example 466, using 6-(4-isopropyl-5-(8-methyl-[1,2,4]triazolo[1,5-a]pyridin-6-yl)-1H-pyrazol-3-yl)-N-methyl-1,2,3,4-tetrahydronaphthalen-2-amine (30 mg, 0.075 mmol) as a starting intermediate to yield the title compound as an off-white solid. LCMS Retention time: 1.46 min [E], MS (E⁺) m/z: 486.2 [M+H]; ¹H NMR (400 MHz, DMSO-d₆) δ 13.02 (br. s., 1H), 8.77 (br. s., 1H), 8.51 (br. s., 1H), 7.62 (br.s., 1H), 7.20 (d, J=8.1 Hz, 3H), 3.12-2.98 (m, 4H), 2.98-2.75 (m, 8H), 2.61 (s, 3H), 2.29 (br.s., 3H), 2.01 (d, J=11.5 Hz, 1H), 1.66 (dd, J=11.5, 4.9 Hz, 1H), 1.12 (d, J=7.1 Hz, 6H).

The following Examples were prepared according to the general procedure used to prepare Example 692.

**TABLE 76**

| Ex. No. | Structure | LCMS MH⁺ | RT (min) | HPLC Method |
|---|---|---|---|---|
| 693 | 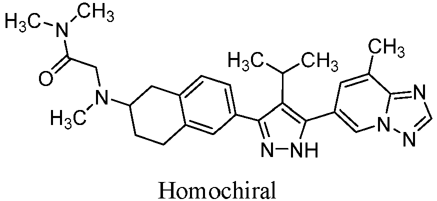 | 486.2 | 1.46 | E |

### EXAMPLE 694

### 2-(4-isopropyl-5-(8-methoxy-[1,2,4]triazolo[1,5-a]pyridin-6-yl)-1H-pyrazol-3-yl)-N,N-dimethyl-4,5,6,7-tetrahydrobenzo[d]thiazol-6-amine

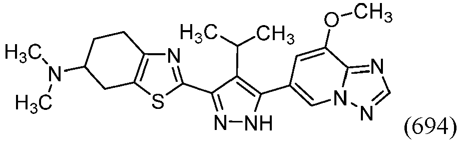

### Intermediate 694A: tert-butyl (3-bromo-4-oxocyclohexyl)carbamate

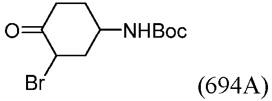

To a solution of tert-butyl (4-oxocyclohexyl)carbamate (5.00 g, 23.44 mmol) in THF (40 mL) and diethyl ether (40 mL) was added aluminum chloride (0.125 g, 0.938 mmol) at 0 °C. The reaction mixture was stirred for 5 min, bromine (1.208 mL, 23.44 mmol) was added dropwise at the same temperature. The reaction mixture was stirred for 6 h. Decolorisation occurred and solid formation was observed. The reaction mass was filtered, washed with diethyl ether, collected the filtrate, and concentrated to yield the crude compound. The crude compound was triturated with diethyl ether (3 X 30 mL), then the ether was collected and concentrated, dried under vacuum to yield tert-butyl (3-bromo-4-oxocyclohexyl)carbamate (5.1 g, 17.46 mmol, 75 % yield) as an orange color solid. LCMS Retention time: 1.14 min [A], MS (E⁺) m/z: 238.4 [M+2H-tBu].

### Intermediate 694B: tert-butyl (2-amino-4,5,6,7-tetrahydrobenzo[d]thiazol-6-yl) carbamate

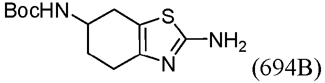

To a solution of tert-butyl (3-bromo-4-oxocyclohexyl)carbamate (5.1 g, 17.46 mmol) in acetonitrile (120 mL) were added DIPEA (6.10 mL, 34.9 mmol) and thiourea (1.462 g, 19.20 mmol) at room temperature. The reaction mixture was stirred at 90 °C for 2 h. The reaction mass was brought to room temperature and concentrated to afford tert-butyl (2-amino-4,5,6,7-tetrahydrobenzo[d]thiazol-6-yl)carbamate (4.6 g, 17.08 mmol, 98 % yield) as a gummy solid. LCMS Retention time: 1.06 min [A], MS (E⁺) m/z: 270.2 [M+H].

### Intermediate 694C: tert-butyl (2-bromo-4,5,6,7-tetrahydrobenzo[d]thiazol-6-yl)carbamate

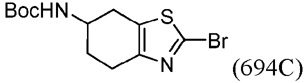

To a solution of tert-butyl (2-amino-4,5,6,7-tetrahydrobenzo[d]thiazol-6-yl)carbamate (4.6 g, 17.08 mmol) in acetonitrile (100.00 mL) were added copper(II) bromide (4.20 g, 18.79 mmol) and isoamyl nitrite (3.44 mL, 25.6 mmol) at 0 °C. The reaction was continued at the same temperature for 1.5 h. The reaction was quenched with water (100 mL). The reaction mixture was stirred for 10 min, filtered the solids, the filtrates were extracted with DCM (2 X 200 mL), the combined organic layers were collected, dried (Na₂SO₄), and concentrated under vacuum to yield the crude compound. The crude compound was purified by ISCO using 80 g column, the compound was eluted in 20% EA in hexanes, the fractions were collected and concentrated to yield tert-butyl (2-bromo-4,5,6,7-tetrahydrobenzo[d]thiazol-6-yl)carbamate (2.3 g, 6.90 mmol, 40.4 % yield) as a white solid. LCMS Retention time: 1.73 min [A], MS (E⁺) m/z: 333.0 [M].

### Intermediate 694D: tert-butyl (2-(4-isopropyl-5-(8-methoxy-[1,2,4]triazolo[1,5-a]pyridin-6-yl)-1-((2-(trimethylsilyl)ethoxy) methyl)-1H-pyrazol-3-yl)-4,5,6,7-tetrahydrobenzo[d] thiazol-6-yl)carbamate

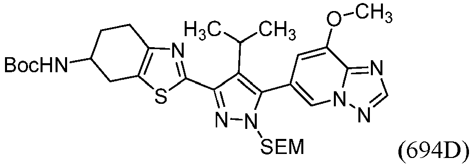

A solution of 6-(4-isopropyl-3-(4,4,5,5-tetramethyl-1,3,2-dioxaborolan-2-yl)-1-((2-(trimethylsilyl)ethoxy)methyl)-1H-pyrazol-5-yl)-8-methoxy-[1,2,4]triazolo[1,5-a]pyridine (1.15 g, 1.120 mmol), tert-butyl (2-bromo-4,5,6,7-tetrahydrobenzo[d]thiazol-6-yl)carbamate (0.410 g, 1.232 mmol) and K₂CO₃ (0.464 g, 3.36 mmol) in acetonitrile (16.00 mL) and water (4.00 mL) solvent mixture was degassed for 10 min with nitrogen. To the solution, Pd₂(dba)₃ (0.051 g, 0.056 mmol) and 2-dicyclohexylphosphino-2',6'-dimethoxybiphenyl (0.046 g, 0.112 mmol) were added. The mixture was degassed again for 2 min. The reaction mixture was stirred at 110 °C for 16 h. The reaction mixture was brought to room temperature, both the layers were separated, the aqueous layer was extracted with DCM (2 X 10 mL), the combined organic extracts were dried (Na₂SO₄) and concentrated to afford the crude compound. The crude compound was purified by ISCO using 24 g silica column, the compound was eluted in 50% EA in hexane, the fractions were collected and concentrated to yield tert-butyl (2-(4-isopropyl-5-(8-methoxy-[1,2,4]triazolo[1,5-a]pyridin-6-yl)-1-((2-(trimethylsilyl)ethoxy)methyl)-1H-pyrazol-3-yl)-4,5,6,7-tetrahydrobenzo[d]thiazol-6-yl)carbamate (750 mg, 0.586 mmol, 52.3 % yield) as an off-white solid. LCMS Retention time: 1.48 min [A], MS (E⁺) m/z: 640.5 [M+H].

### Intermediate 694E: 2-(4-isopropyl-5-(8-methoxy-[1,2,4]triazolo[1,5-a]pyridin-6-yl)-1H-pyrazol-3-yl)-4,5,6,7-tetrahydrobenzo[d]thiazol-6-amine

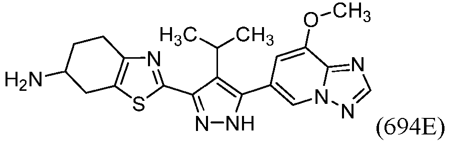

To a solution of tert-butyl (2-(4-isopropyl-5-(8-methoxy-[1,2,4]triazolo[1,5-a]pyridin-6-yl)-1-((2-(trimethylsilyl)ethoxy)methyl)-1H-pyrazol-3-yl)-4,5,6,7-tetrahydrobenzo[d]thiazol-6-yl)carbamate (0.750 g, 0.586 mmol) in DCM (5.00 mL) was added TFA (3.00 mL, 38.9 mmol) at 0 °C. The reaction mixture was stirred at room temperature for 16 h. The reaction mixture was concentrated to afford the crude compound. The crude compound was purified by Prep HPLC method AC, the fractions containing the product were combined, concentrated, and lyophilized to yield 2-(4-isopropyl-5-(8-methoxy-[1,2,4]triazolo[1,5-a]pyridin-6-yl)-1H-pyrazol-3-yl)-4,5,6,7-tetrahydrobenzo[d]thiazol-6-amine (210 mg, 0.503 mmol, 86 % yield) as a white solid. The racemic 2-(4-isopropyl-5-(8-methoxy-[1,2,4]triazolo[1,5-a]pyridin-6-yl)-1H-pyrazol-3-yl)-4,5,6,7-tetrahydrobenzo[d]thiazol-6-amine (210 mg, 0.513 mmol) was purified by SFC using method AF to separate both the enantiomers. The fractions containing the products were collected, concentrated and lyophilized to afford two isomers.

Intermediate 694E (Isomer 1): (72 mg, 0.176 mmol, 34% yield) (Peak 1, Chiral SFC RT-9.88) as a white solid. LCMS Retention time: 0.990 min [E], MS (E⁺) m/z: 410.2 [M+H]; ¹H NMR (400 MHz, METHANOL-d₄) δ ppm 8.43 (d, J = 0.8 Hz, 1H), 8.35 (s, 1H), 7.06 (s, 1H), 4.02 (s, 3 H), 3.66-3.62 (m, 1H), 3.48-3.39 (m, 1H), 3.28-3.20 (m, 1H), 2.96-2.81 (m, 2H), 2.25-2.19 (m, 1H), 2.01-1.95 (m, 2H), 1.24 (d, J = 6.8 Hz, 6 H); and

Intermediate 694E (Isomer 2): (93 mg, 0.227 mmol, 44% yield) (Peak 2, Chiral SFC RT-13.17) as a white solid. LCMS Retention time: 1.07 min [E], MS (E⁺) m/z: 410.1 [M+H]; ¹H NMR (400 MHz, METHANOL-d₄) δ ppm 8.43 (d, J = 0.8 Hz, 1H), 8.35 (s, 1H), 7.06 (s, 1H), 4.02 (s, 3 H), 3.66-3.62 (m, 1H), 3.48-3.39 (m, 1H), 3.28-3.20 (m, 1H), 2.96-2.81 (m, 2H), 2.25-2.19 (m, 1H), 2.01-1.95 (m, 2H), 1.24 (d, J = 6.8 Hz, 6 H).

### Example 694: 2-(4-isopropyl-5-(8-methoxy-[1,2,4]triazolo[1,5-a]pyridin-6-yl)-1H-pyrazol-3-yl)-N,N-dimethyl-4,5,6,7-tetrahydrobenzo[d]thiazol-6-amine

To a solution of 2-(4-isopropyl-5-(8-methoxy-[1,2,4]triazolo[1,5-a]pyridin-6-yl)-1H-pyrazol-3-yl)-4,5,6,7-tetrahydrobenzo[d]thiazol-6-amine (21 mg, 0.051 mmol) in MeOH (2.00 mL) were added formaldehyde (0.2 mL, 2.178 mmol) and AcOH (0.1 mL, 1.747 mmol) at room temperature. The mixture was stirred at the same temperature for 6 h, to this was then added sodium cyanoborohydride (16.11 mg, 0.256 mmol) at room temperature. The reaction mixture was stirred at the same temperature for 16 h. The reaction mass purified by Prep LCMS using method AA, fractions containing product were combined and dried using Genevac centrifugal evaporator to get 2-(4-isopropyl-5-(8-methoxy-[1,2,4]triazolo[1,5-a]pyridin-6-yl)-1H-pyrazol-3-yl)-N,N-dimethyl-4,5,6,7-tetrahydrobenzo[d]thiazol-6-amine (6.3 mg, 0.014 mmol, 27 % yield) as a pale solid. LCMS Retention time: 1.023 min [F], MS (E⁺) m/z: 438.2 [M+H]; ¹H NMR (400 MHz, METHANOL-d₄) δ ppm 8.54 (s, 1H), 8.45 (s, 1H), 7.18 (s, 1H), 4.13 (s, 3H), 3.81 (s, 1H), 3.50 (br. s., 1H), 3.24-3.13 (m, 2H), 3.10 (d, *J=*14.9 Hz, 1H), 3.01-2.78 (m, 2H), 2.63 (s, 6H), 2.31 (br. s., 1H), 2.05-1.87 (m, 2H), 1.43-1.21 (m, 7H).

The following Examples were prepared according to the general procedure used to prepare Example 694E.

**TABLE 77**

| Ex. No. | Structure | LCMS MH⁺ | RT (min) | HPLC Method |
|---|---|---|---|---|
| 695 | 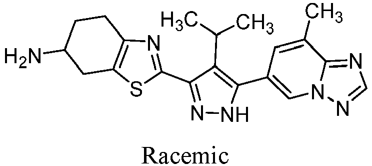 | 394.2 | 1.307 | B |
| 696 | 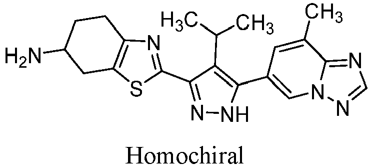 | 394.2 | 1.308 | B |
| 697 | 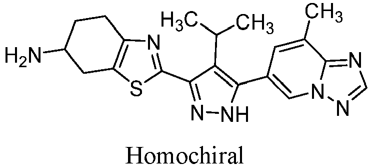 | 394.2 | 1.278 | B |

The following Examples were prepared according to the general procedure used to prepare Example 694.

**TABLE 78**

| Ex. No. | Structure | LCMS MH⁺ | RT (min) | HPLC Method |
|---|---|---|---|---|
| 698 | 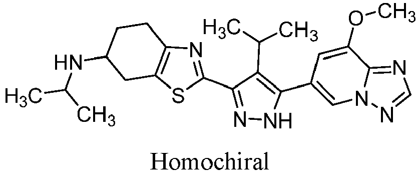 | 452.3 | 1.24 | E |
| 699 | 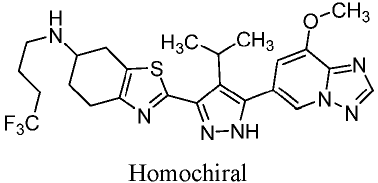 | 520.3 | 1.708 | E |
| 700 | 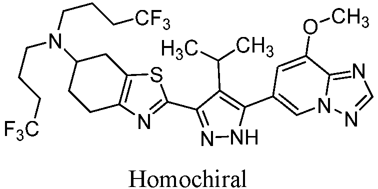 | 630.3 | 2.524 | E |
| 701 | 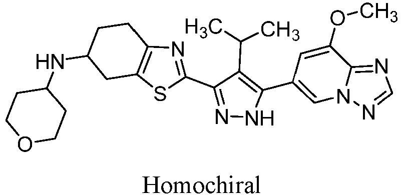 | 494.3 | 1.314 | E |
| 702 | 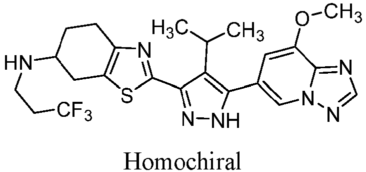 | 506.2 | 1.827 | E |
| 703 | 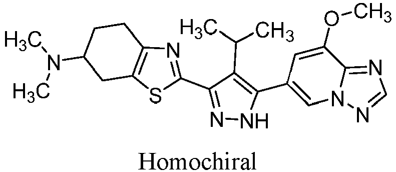 | 438.2 | 1.201 | E |
| 704 | 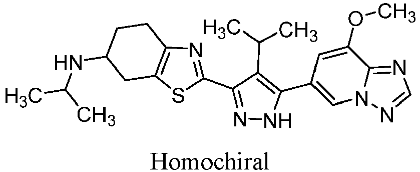 | 452.2 | 1.183 | E |
| 705 | 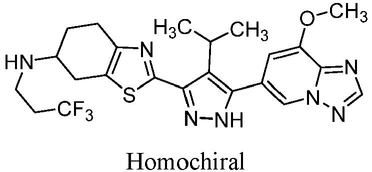 | 506.3 | 1.855 | E |
| 706 | 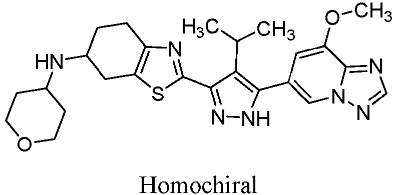 | 494.3 | 1.352 | E |
| 707 | 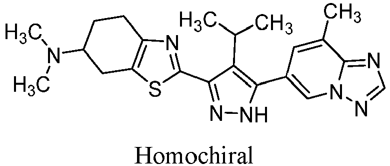 | 422.2 | 1.27 | E |
| 708 | 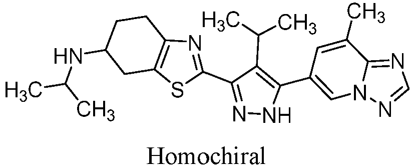 | 436.2 | 1.279 | E |
| 709 | 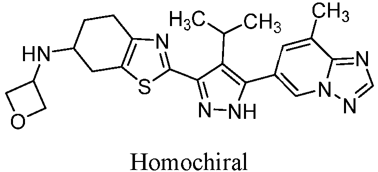 | 450.2 | 1.374 | E |
| 710 | 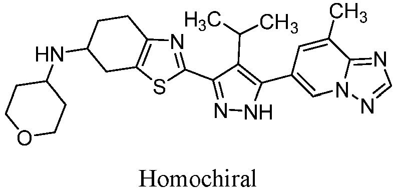 | 478.3 | 1.347 | E |
| 711 | 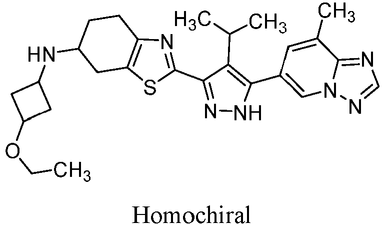 | 492.3 | 1.574 | E |
| 712 | 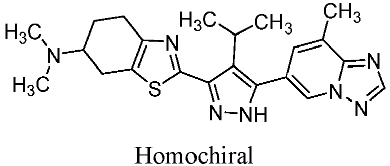 | 422.2 | 1.277 | E |
| 713 | 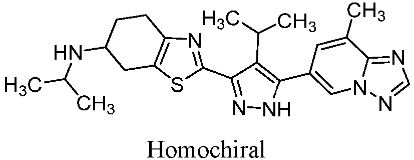 | 436.2 | 1.231 | E |
| 714 | 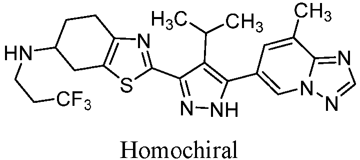 | 490.2 | 1.862 | E |
| 715 | 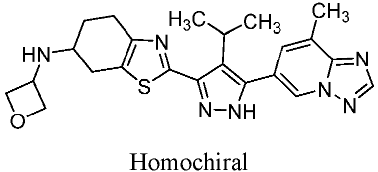 | 450.2 | 1.369 | E |
| 716 | 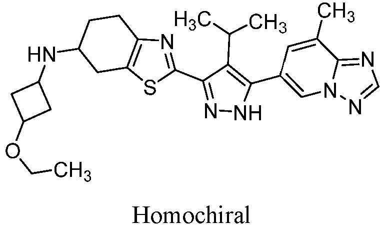 | 492.3 | 1.585 | E |
| 717 | 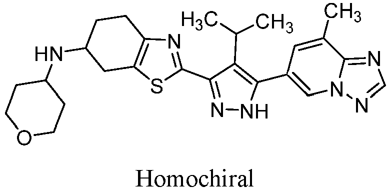 | 478.3 | 1.323 | E |

### EXAMPLE 718

### 2-(dimethylamino)-N-(2-(4-isopropyl-5-(8-methoxy-[1,2,4]triazolo[1,5-a]pyridin-6-yl)-1H-pyrazol-3-yl)-4,5,6,7-tetrahydrobenzo[d]thiazol-6-yl)acetamide

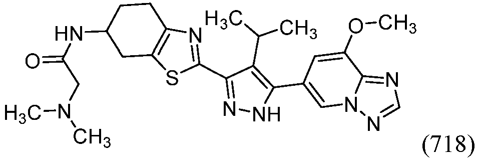

To a solution of 2-(4-isopropyl-5-(8-methoxy-[1,2,4]triazolo[1,5-a]pyridin-6-yl)-1H-pyrazol-3-yl)-4,5,6,7-tetrahydrobenzo[d]thiazol-6-amine (15 mg, 0.037 mmol) and dimethylglycine (5.67 mg, 0.055 mmol) in DMF (1.00 mL) were added TEA (0.1 mL, 0.717 mmol) and HATU (27.9 mg, 0.073 mmol) at room temperature. The reaction mixture was stirred at the same temperature for 2 h. The reaction mass was purified by Prep LCMS using method AB, the fractions containing product were combined and dried using Genevac centrifugal evaporator to get 2-(dimethylamino)-N-(2-(4-isopropyl-5-(8-methoxy-[1,2,4]triazolo[1,5-a]pyridin-6-yl)-1H-pyrazol-3-yl)-4,5,6,7-tetrahydrobenzo[d]thiazol-6-yl) acetamide (3.0 mg, 5.75 µmol, 15.70 % yield) as a pale solid. LCMS Retention time: 1.117 min [F], MS (E⁺) m/z: 495.3 [M+H]; ¹H NMR (400 MHz, METHANOL-d₄) δ ppm 8.55 (br. s., 1H), 8.45 (s, 1H), 7.17 (br. s., 1H), 4.34 (d, *J*=9.3 Hz, 1H), 4.13 (s, 3H), 3.55 (d, *J*=7.1 Hz, 1H), 3.27-3.11 (m, 3H), 3.05-2.95 (m, 2H), 2.95-2.74 (m, 2H), 2.40 (s, 5H), 2.14 (br. s., 1H), 2.08-1.91 (m, 2H), 1.39-1.17 (m, 6H).

The following Examples were prepared according to the general procedure used to prepare Example 718.

**TABLE 79**

| Ex. No. | Structure | LCMS MH⁺ | RT (min) | HPLC Method |
|---|---|---|---|---|
| 719 | 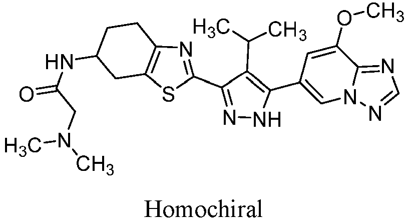 | 495.3 | 1.359 | E |
| 720 | 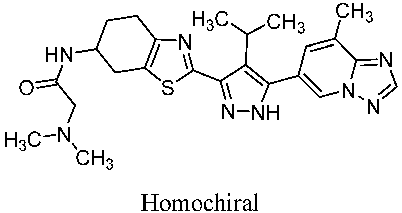 | 479.2 | 1.397 | E |
| 721 | 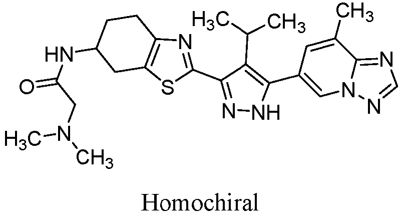 | 479.2 | 1.396 | E |

### EXAMPLE 722

### N-ethyl-2-(4-isopropyl-5-(8-methoxy-[1,2,4]triazolo[1,5-a]pyridin-6-yl)-1H-pyrazol-3-yl)-N-methyl-4,5,6,7-tetrahydrobenzo[d]thiazol-6-amine

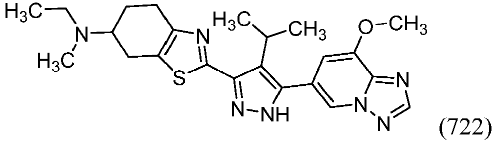

### Intermediate 722A: tert-butyl (2-bromo-4,5,6,7-tetrahydrobenzo[d]thiazol-6-yl)(methyl) carbamate

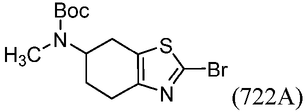

To a solution of tert-butyl (2-bromo-4,5,6,7-tetrahydrobenzo[d]thiazol-6-yl) carbamate (1.2 g, 3.60 mmol) in THF (50.00 mL) was added NaH (0.432 g, 10.80 mmol) at 0 °C. The reaction mixture was stirred for 1 h., and the reaction mixture was cooled. MeI (0.675 mL, 10.80 mmol) was added. The reaction mixture was stirred at the same temperature for 5 h. The reaction was quenched with ice at 0 °C. The mixture was extracted with EtOAc, washed with brine, and concentrated to get crude compound. The crude compound was purified by ISCO using 24 g silica column, compound was eluted in 18% EtOAc in hexane, the fractions were collected and concentrated to get tert-butyl (2-bromo-4,5,6,7-tetrahydrobenzo[d]thiazol-6-yl)(methyl)carbamate (1.15 g, 3.31 mmol, 92 % yield) as a white solid. LCMS Retention time: 1.99 min [A], MS (E⁺) m/z: 349.2 [M + 2H].

### Intermediate 722B: tert-butyl (2-(4-isopropyl-5-(8-methoxy-[1,2,4]triazolo[1,5-a]pyridin-6-yl)-1-((2-(trimethylsilyl)ethoxy) methyl)-1H-pyrazol-3-yl)-4,5,6,7-tetrahydro benzo[d]thiazol-6-yl)(methyl)carbamate

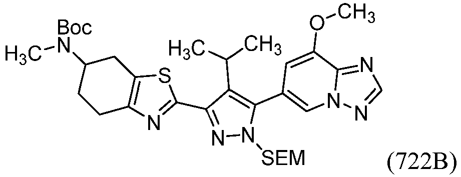

A solution of 6-(4-isopropyl-3-(4,4,5,5-tetramethyl-1,3,2-dioxaborolan-2-yl)-1-((2-(trimethylsilyl)ethoxy)methyl)-1H-pyrazol-5-yl)-8-methoxy-[1,2,4]triazolo[1,5-a]pyridine (2.90 g, 2.82 mmol), tert-butyl (2-bromo-4,5,6,7-tetrahydrobenzo[d]thiazol-6-yl)(methyl) carbamate (0.817 g, 2.353 mmol) and K₂CO₃ (0.976 g, 7.06 mmol) in acetonitrile (40.00 mL) and water (10.00 mL) solvent mixture was degassed for 10 min with nitrogen. Next, Pd₂(dba)₃ (0.108 g, 0.118 mmol) and 2-dicyclohexylphosphino-2',6'-dimethoxybiphenyl (0.097 g, 0.235 mmol) were added. The reaction mixture was degassed for 2 min, and stirred at 110 °C for 16 h. The reaction mixture was brought to room temperature, both layers were separated, the aqueous layer was extracted with DCM (2 X 20 mL), the combined organic extracts were dried (Na₂SO₄) and concentrated to get crude compound. The crude compound was purified by ISCO using 24 g silica column, compound was eluted in 50% EA in hexane, the fractions were collected and concentrated to get tert-butyl (2-(4-isopropyl-5-(8-methoxy-[1,2,4]triazolo[1,5-a]pyridin-6-yl)-1-((2-(trimethylsilyl)ethoxy)methyl)-1H-pyrazol-3-yl)-4,5,6,7-tetrahydrobenzo[d]thiazol-6-yl)(methyl)carbamate (2.75 g, 1.262 mmol, 54 % yield) as an off-white solid. LCMS Retention time: 1.85 min [A], MS (E⁺) m/z: 654.6 [M+H].

### Intermediate 722C: 2-(4-isopropyl-5-(8-methoxy-[1,2,4]triazolo[1,5-a]pyridin-6-yl)-1H-pyrazol-3-yl)-N-methyl-4,5,6,7-tetrahydrobenzo[d]thiazol-6-amine

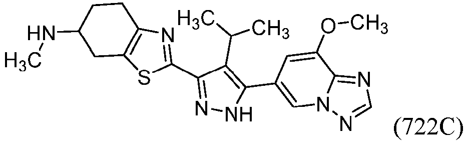

To a solution of tert-butyl (2-(4-isopropyl-5-(8-methoxy-[1,2,4]triazolo[1,5-a]pyridin-6-yl)-1-((2-(trimethylsilyl)ethoxy)methyl)-1H-pyrazol-3-yl)-4,5,6,7-tetrahydrobenzo[d]thiazol-6-yl)(methyl)carbamate (2.75 g, 1.262 mmol) in DCM (5.00 mL) was added TFA (3.00 mL, 38.9 mmol) at 0 °C. The reaction mixture was stirred at room temperature for 16 h. The reaction mass was concentrated to get crude compound, the crude compound was purified by prep HPLC using method AC, the fractions containing the compound was collected, concentrated and lyophilized to get 2-(4-isopropyl-5-(8-methoxy-[1,2,4]triazolo[1,5-a]pyridin-6-yl)-1H-pyrazol-3-yl)-N-methyl-4,5,6,7-tetrahydrobenzo[d]thiazol-6-amine, TFA (670 mg, 1.197 mmol, 95% yield) as an off-white solid.

The racemic compound was purified by SFC using method AD, to separate both the enantiomers, the desired fractions were collected, concentrated and lyophilized to yield:
Example 722Ca: Isomer 1 (270 mg, 0.631 mmol, 39.9 % yield) (Peak 1, Chiral SFC RT-11.82) as a white solid. LCMS Retention time: 1.557 min [B], MS (E⁺) m/z: 424.2 [M+H]; ¹H NMR (400 MHz, METHANOL-d₄) δ ppm 8.54 (d, J = 1.2 Hz, 1H), 8.46 (s, 1H), 7.17 (s, 1H), 4.13 (s, 3 H), 3.88-3.64 (m, 1H), 3.51 - 3.32 (m, 2H), 3.10-2.95 (m, 3 H), 2.86 (s, 3H), 2.41- 2.38 (m, 1H), 2.13-2.05 (m, 1H), 1.34 (d, J = 6.8 Hz, 6 H); and Example 722Cb: Isomer 2 (330 mg, 0.756 mmol, 47.8 % yield) (Peak 2, Chiral SFC RT-19.44) as a white solid. LCMS Retention time: 1.539 min [B], MS (E⁺) m/z: 424.2 [M+H]; ¹H NMR (400 MHz, METHANOL-d₄) δ ppm 8.54 (d, J = 1.2 Hz, 1 H), 8.45 (s, 1H), 7.17 (s, 1 H), 4.13 (s, 3 H), 3.74-3.68 (m, 1H), 3.53-3.40 (m, 2 H), 3.08-2.94 (m, 3 H), 2.85 (s, 3 H), 2.42-2.35 (m, 1H), 2.12-2.04 (m, 1H), 1.33 (d, J = 6.8 Hz, 6 H).

### Example 722: N-ethyl-2-(4-isopropyl-5-(8-methoxy-[1,2,4]triazolo[1,5-a]pyridin-6-yl)-1H-pyrazol-3-yl)-N-methyl-4,5,6,7-tetrahydrobenzo[d]thiazol-6-amine

To a solution of 2-(4-isopropyl-5-(8-methoxy-[1,2,4]triazolo[1,5-a]pyridin-6-yl)-1H-pyrazol-3-yl)-N-methyl-4,5,6,7-tetrahydrobenzo[d]thiazol-6-amine (19 mg, 0.045 mmol) and acetaldehyde (9.88 mg, 0.224 mmol) in MeOH (2.00 mL) was added AcOH (0.1 mL, 1.747 mmol) at room temperature. The reaction mixture was stirred at the same temperature for 6 h. and sodium cyanoborohydride (8.46 mg, 0.135 mmol) was added. The reaction mixture was stirred at the same temperature for 16 h. The reaction mass was purified by Prep LCMS using method AA, the fractions containing product were combined and dried using Genevac centrifugal evaporator to get N-ethyl-2-(4-isopropyl-5-(8-methoxy-[1,2,4]triazolo[1,5-a] pyridin-6-yl)-1H-pyrazol-3-yl)-N-methyl-4,5,6,7-tetrahydrobenzo[d]thiazol-6-amine (4.1 mg, 8.44 µmol, 19% yield) as a pale solid. LCMS Retention time: 0.853 min [F], MS (E⁺) m/z: 452.3 [M+H]; ¹H NMR (400 MHz, METHANOL-d₄) δ ppm 8.54 (d, *J* =1.0 Hz, 1H), 8.49-8.43 (m, 1H), 7.17 (s, 1H), 4.13 (s, 3H), 3.97-3.86 (m, 1H), 3.60-3.42 (m, 3H), 3.37 (br. s., 2H), 3.24-3.12 (m, 2H), 3.09-3.00 (m, 1H), 2.98 (s, 3H), 2.41 (br. s., 2H), 2.20 (br. s., 2H), 1.44 (t, *J*=7.2 Hz, 4H), 1.40-1.26 (m, 7H), 1.23 (br. s., 1H).

The following Examples were prepared according to the general procedure used to prepare Example 722.

**TABLE 80**

| Ex. No. | Structure | LCMS MH⁺ | RT (min) | HPLC Method |
|---|---|---|---|---|
| 723 | 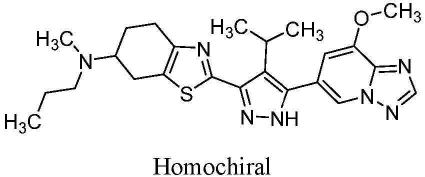 | 466.3 | 1.349 | E |
| 724 | 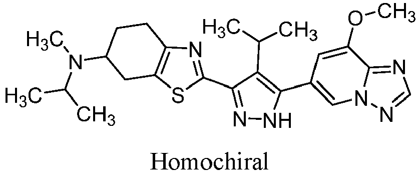 | 466.3 | 1.223 | E |
| 725 | 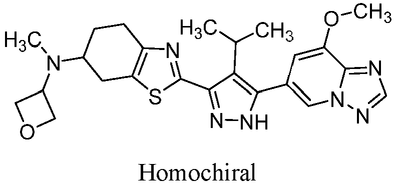 | 480.3 | 1.379 | E |
| 726 | 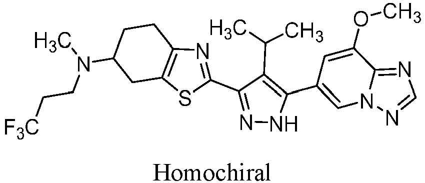 | 520.3 | 1.94 | E |
| 727 | 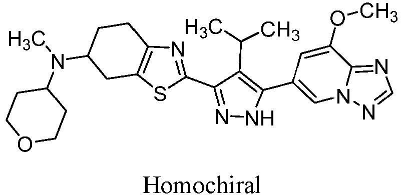 | 508.3 | 1.36 | E |
| 728 | 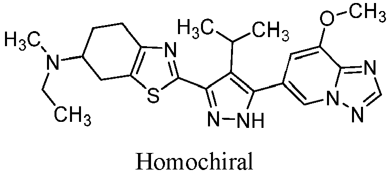 | 452.3 | 1.17 | E |
| 729 | 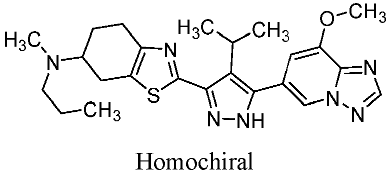 | 466.3 | 1.389 | E |
| 730 | 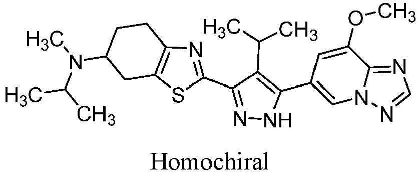 | 466.3 | 1.218 | E |
| 731 | 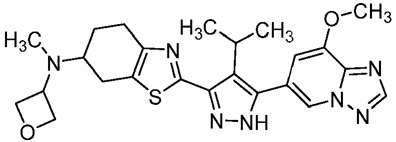 | 480.3 | 1.376 | E |
| | 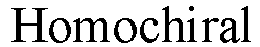 | | | |
| 732 | 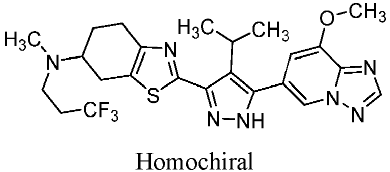 | 520.2 | 1.927 | E |
| 733 | 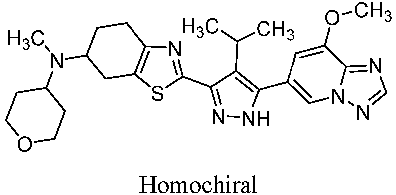 | 508.3 | 1.354 | E |

### EXAMPLE 734

### 2-(dimethylamino)-N-(2-(4-isopropyl-5-(8-methoxy-[1,2,4] triazolo[1,5-a]pyridin-6-yl)-1H-pyrazol-3-yl)-4,5,6,7-tetrahydrobenzo[d]thiazol-6-yl)-N-methylacetamide

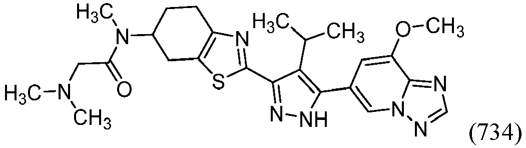

To a solution of 2-(4-isopropyl-5-(8-methoxy-[1,2,4]triazolo[1,5-a]pyridin-6-yl)-1H-pyrazol-3-yl)-N-methyl-4,5,6,7-tetrahydrobenzo[d]thiazol-6-amine (11 mg, 0.026 mmol) and dimethylglycine (4.02 mg, 0.039 mmol) in DMF (1.00 mL) were added DIPEA (0.1 mL, 0.573 mmol) and HATU (19.75 mg, 0.052 mmol) at room temperature. The reaction mixture was stirred at the same temperature for 2 h. The reaction mass was purified by Prep LCMS purification using method AB, the fractions containing the product were combined and dried using Genevac centrifugal evaporator to get 2-(dimethylamino)-N-(2-(4-isopropyl-5-(8-methoxy-[1,2,4]triazolo[1,5-a]pyridin-6-yl)-1H-pyrazol-3-yl)-4,5,6,7-tetrahydrobenzo[d]thiazol-6-yl)-N-methylacetamide (10.8 mg, 0.021 mmol, 80 % yield) as a pale solid. LCMS Retention time: 0.925 min [F], MS (E⁺) m/z: 509.2 [M+H]; ¹H NMR (400 MHz, METHANOL-d₄) δ ppm 8.55 (s, 1H), 8.46 (s, 1H), 7.18 (br. s., 1H), 4.31 (br. s., 1H), 4.13 (s, 3H), 3.70 (br. s., 2H), 3.59-3.49 (m, 1H), 3.20-2.92 (m, 8H), 2.62 (s, 3H), 2.56 (br. s., 3H), 2.32-2.16 (m, 1H), 2.12 (br. s., 1H), 2.09-1.99 (m, 1H), 1.96 (s, 2H), 1.41-1.25 (m, 8H), 0.98-0.83 (m, 1H).

The following Examples were prepared according to the general procedure used to prepare Example 734.

**TABLE 81**

| Ex. No. | Structure | LCMS MH⁺ | RT (min) | HPLC Method |
|---|---|---|---|---|
| 735 | 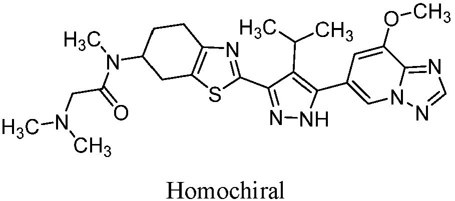 | 509.3 | 1.138 | E |

### EXAMPLE 736

### 2-((2-(4-isopropyl-5-(8-methoxy-[1,2,4]triazolo[1,5-a]pyridin-6-yl)-1H-pyrazol-3-yl)-4,5,6,7-tetrahydrobenzo[d]thiazol-6-yl)(methyl)amino)-N,N-dimethylacetamide

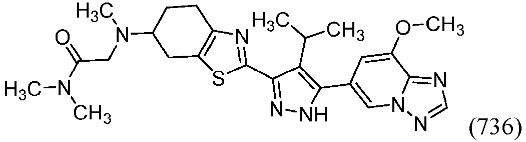

To a solution of 2-(4-isopropyl-5-(8-methoxy-[1,2,4]triazolo[1,5-a]pyridin-6-yl)-1H-pyrazol-3-yl)-N-methyl-4,5,6,7-tetrahydrobenzo[d]thiazol-6-amine (12 mg, 0.028 mmol) and 2-chloro-N,N-dimethylacetamide (5.17 mg, 0.042 mmol) in DMF (0.50 mL) and THF (1.50 mL) solvent mixture was added DIPEA (0.1 mL, 0.573 mmol) at room temperature. The reaction mixture was stirred at the same temperature for 16 h. The reaction mass was purified by prep LCMS using method AA. The fractions containing product were combined and dried using Genevac centrifugal evaporator to get 2-((2-(4-isopropyl-5-(8-methoxy-[1,2,4]triazolo[1,5-a]pyridin-6-yl)-1H-pyrazol-3-yl)-4,5,6,7-tetrahydrobenzo[d]thiazol-6-yl)(methyl)amino)-N,N-dimethylacetamide (3.6 mg, 6.68 µmol, 23% yield) as a pale solid. LCMS Retention time: 1.335 min [E], MS (E⁺) m/z: 509.3 [M+H]; ¹H NMR (400 MHz, METHANOL-d₄) δ ppm 8.55 (br. s., 1H), 8.45 (s, 1H), 7.17 (br. s., 1H), 4.20-4.10 (m, 3H), 3.80 (s, 1H), 3.63-3.44 (m, 2H), 3.37 (s, 1H), 3.19 (br. s., 1H), 3.16-3.03 (m, 4H), 3.00-2.91 (m, 3H), 2.88 (d, *J*=4.6 Hz, 1H), 2.65-2.36 (m, 3H), 2.24 (d, *J*=6.6 Hz, 1H), 1.96-1.85 (m, 1H), 1.37-1.23 (m, 5H).

The following Examples were prepared according to the general procedure used to prepare Example 736.

**TABLE 82**

| Ex. No. | Structure | LCMS MH⁺ | RT (min) | HPLC Method |
|---|---|---|---|---|
| 737 | 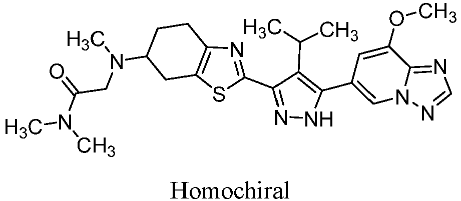 | 509.3 | 1.34 | E |
| 738 | 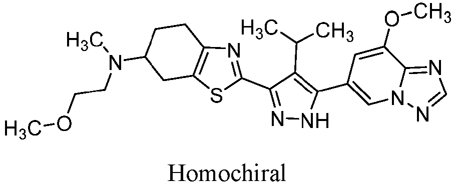 | 482.2 | 1.319 | E |
| 739 | 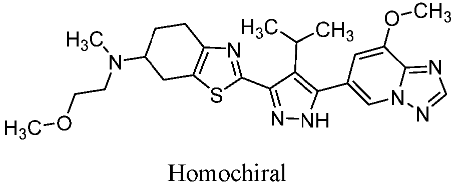 | 482.2 | 1.381 | E |
| 740 | 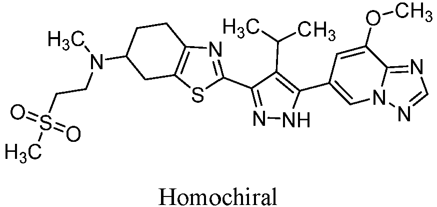 | 530.2 | 1.476 | E |
| 741 | 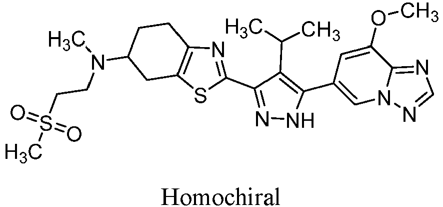 | 530.2 | 1.382 | E |

### EXAMPLE 742

### 2-(4-isopropyl-5-(8-methoxy-[1,2,4]triazolo[1,5-a]pyridin-6-yl)-1H-pyrazol-3-yl)-5-(piperidin-4-yl)thiazole

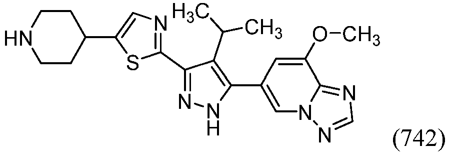

### Intermediate 742A: tert-butyl 4-(thiazol-5-yl)-3,6-dihydropyridine-1(2H)-carboxylate

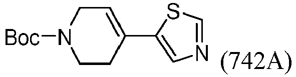

XPhos Pd G₂ (2.399 g, 3.05 mmol) and K₃PO₄ (15.93 g, 91 mmol) were added to a degassed solution of 5-bromothiazole (5.0 g, 30.5 mmol) and tert-butyl 4-(4,4,5,5-tetramethyl-1,3,2-dioxaborolan-2-yl)-3,6-dihydropyridine-1(2H)-carboxylate (11.31 g, 36.6 mmol) in THF (150 mL) and water (30.0 mL) solvent mixture. The reaction mixture was stirred at 80 °C for 14 h in a sealed tube. The reaction mixture was diluted with ethyl acetate, filtered, the filtrate was washed with water, brine, dried over sodium sulphate and concentrated to get crude compound. The crude compound was purified by ISCO using 80 g silica column, the compound was eluted with 45-65% ethyl acetate in pet ether, the fractions were collected and concentrated to get tert-butyl 4-(thiazol-5-yl)-3,6-dihydropyridine-1(2H)-carboxylate (6.2 g, 23.28 mmol, 76 % yield) as a light brown solid. LCMS Retention time: 2.49 min [B], MS (E⁺) m/z: 267.2 [M+H].

### Intermediate 742B: tert-butyl 4-(thiazol-5-yl)piperidine-1-carboxylate

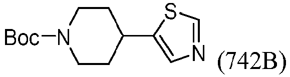

To a solution of tert-butyl 4-(thiazol-5-yl)-3,6-dihydropyridine-1(2H)-carboxylate (6.2 g, 23.28 mmol) in methanol (250 mL) was added Pd-C (2.477 g, 23.28 mmol) at room temperature. The reaction mixture was stirred at room temperature under a hydrogen bladder for 12 h. The reaction mixture was filtered and washed with excess methanol and THF, the filtrates were collected and concentrated to get crude compound. The crude compound was purified by ISCO using 80 g silica column, the compound was eluted with 20-35% ethyl acetate in pet ether, the fractions were collected and concentrated to get tert-butyl 4-(thiazol-5-yl)piperidine-1-carboxylate (5.21 g, 19.41 mmol, 83 % yield) as a brown color gummy solid. LCMS Retention time: 0.63 min [A, MS (E⁺) m/z: 269.3 [M+H].

### Intermediate 742C: tert-butyl 4-(2-bromothiazol-5-yl)piperidine-1-carboxylate

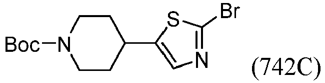

To a solution of tert-butyl 4-(thiazol-5-yl)piperidine-1-carboxylate (1.5 g, 5.59 mmol) in THF (50.0 mL) was added nBuLi (3.81 mL, 8.38 mmol) at -78 °C. The reaction mixture was stirred for 40 min, and a solution of CCl₄ (2.78 g, 8.38 mmol) in THF (1.5 mL) was added. The reaction mixture was stirred at the same temperature for 1 h. The reaction was quenched with cold water. The reaction mixture was diluted with excess ethyl acetate, washed with water, brine, dried over sodium sulphate and concentrated to get crude compound. The crude compound was purified by ISCO using 24 g silica column, compound was eluted with 25-30% ethyl acetate in pet ether, the fractions were collected and concentrated to get tert-butyl 4-(2-bromothiazol-5-yl)piperidine-1-carboxylate (1.1 g, 3.17 mmol, 57 % yield) as a light brown solid. LCMS Retention time: 0.99 min [A], MS (E⁺) m/z: 347.0 [M⁺].

### Intermediate 742D: tert-butyl4-(2-(4-isopropyl-5-(8-methoxy-[1,2,4]triazolo[1,5-a] pyridin-6-yl)-1-((2-(trimethylsilyl)ethoxy)methyl)-1H-pyrazol-3-yl)thiazol-5-yl) piperidine-1-carboxylate

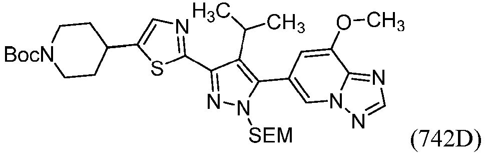

Pd₂(dba)₃ (0.145 g, 0.158 mmol) and S-Phos (0.130 g, 0.317 mmol) were added to a degassed solution of tert-butyl 4-(2-bromothiazol-5-yl)piperidine-1-carboxylate (1.1 g, 3.17 mmol), K₂CO₃ (1.313 g, 9.50 mmol) and 6-(4-isopropyl-3-(4,4,5,5-tetramethyl-1,3,2-dioxaborolan-2-yl)-1-((2-(trimethylsilyl)ethoxy)methyl)-1H-pyrazol-5-yl)-8-methoxy-[1,2,4]triazolo[1,5-a]pyridine (1.952 g, 3.80 mmol) in acetonitrile (25.0 mL) and water (5.0 mL) solvent mixture. The reaction mixture was stirred at 110 °C for 14 h in a sealed tube. The two layers were separated. The aqueous layer was extracted with ethyl acetate, combined organic extracts were dried over (Na₂SO₄) and concentrated to get crude compound. The crude compound was purified by ISCO using 40 g silica column, compound was eluted with 65-75% EtOAc in pet ether, the fractions were collected and concentrated to get tert-butyl 4-(2-(4-isopropyl-5-(8-methoxy-[1,2,4]triazolo[1,5-a]pyridin-6-yl)-1-((2-(trimethylsilyl)ethoxy)methyl)-1H-pyrazol-3-yl)thiazol-5-yl)piperidine-1-carboxylate (1.2 g, 1.835 mmol, 58% yield) as a light brown solid. LCMS Retention time: 1.75min [A], MS (E⁺) m/z: 654.4 [M+H].

### Example 742: 2-(4-isopropyl-5-(8-methoxy-[1,2,4]triazolo[1,5-a]pyridin-6-yl)-1H-pyrazol-3-yl)-5-(piperidin-4-yl)thiazole

To a solution of tert-butyl 4-(2-(4-isopropyl-5-(8-methoxy-[1,2,4]triazolo[1,5-a] pyridin-6-yl)-1-((2-(trimethylsilyl)ethoxy)methyl)-1H-pyrazol-3-yl)thiazol-5-yl)piperidine-1-carboxylate (1.2 g, 1.835 mmol) in DCM (15.0 mL) was added TFA (3.53 mL, 45.9 mmol) at 0 °C. The reaction mixture was stirred at room temperature for 16 h. The volatiles were evaporated, dried under vacuum and triturated with diethyl ether to get crude compound. The crude compound was purified by Prep LCMS purification using method AA, the fractions were collected, concentrated and lyophilized to get 2-(4-isopropyl-5-(8-methoxy-[1,2,4]triazolo[1,5-a]pyridin-6-yl)-1H-pyrazol-3-yl)-5-(piperidin-4-yl)thiazole (650 mg, 1.535 mmol, 84% yield) as an off-white solid. LCMS Retention time: 0.85 min [E], MS (E⁺) m/z: 424.3 [M+H]; ¹H NMR (400 MHz, METHANOL-d₄) δ 8.55 (d, J=1.2 Hz, 1H), 8.46 (s, 1H), 7.72 (s, 1H), 7.18 (d, J=1.0 Hz, 1H), 4.13 (s, 3H), 3.59-3.50 (m, 1H), 3.44 (d, J=13.0 Hz, 2H), 3.15-3.02 (m, 2H), 2.29 (d, J=14.9 Hz, 2H), 2.01-1.82 (m, 4H), 1.43-1.27 (m, 6H).

The following Examples were prepared according to the general procedure used to prepare Example 742.

**TABLE 83**

| Ex. No. | Structure | LCMS MH⁺ | RT (min) | HPLC Method |
|---|---|---|---|---|
| 743 | 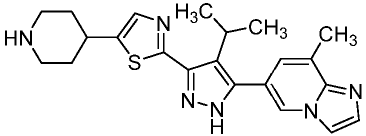 | 408.2 | 1.12 | C |
| 744 | 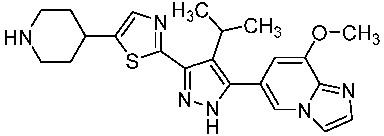 | 423.3 | 1.19 | C |
| 745 | 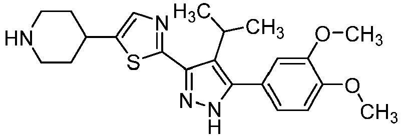 | 413.3 | 1.22 | C |
| 746 | 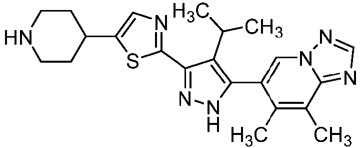 | 422.3 | 1 | C |
| 747 | 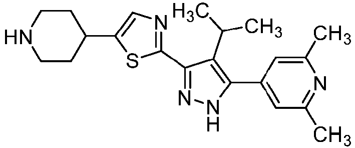 | 382.3 | 1.09 | C |
| 748 | 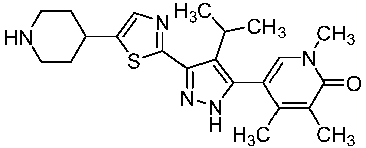 | 412.3 | 1.2 | C |
| 749 | 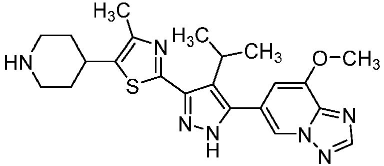 | 438.2 | 1.06 | E |
| 750 | 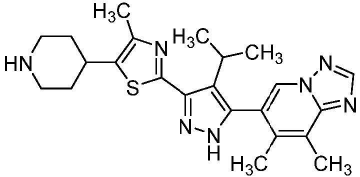 | 436.3 | 1.41 | C |
| 751 | 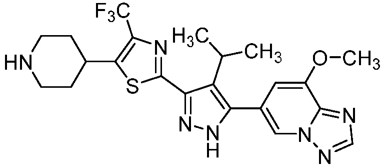 | 492.2 | 1.365 | E |

### EXAMPLE 752

### 2-(4-isopropyl-5-(8-methoxy-[1,2,4]triazolo[1,5-a]pyridin-6-yl)-1H-pyrazol-3-yl)-5-(1-((tetrahydro-2H-pyran-4-yl)methyl)piperidin-4-yl)thiazole

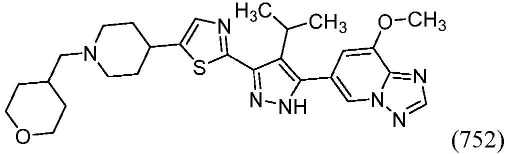

Mixture of 2-(4-isopropyl-5-(8-methoxy-[1,2,4]triazolo[1,5-a]pyridin-6-yl)-1H-pyrazol-3-yl)-5-(piperidin-4-yl)thiazole (30.0 mg, 0.071 mmol), tetrahydro-2H-pyran-4-carbaldehyde (40.4 mg, 0.354 mmol) and acetic acid (0.405 µL, 7.08 µmol) in MeOH (2.0 mL) was stirred at room temperature for 1h. Next, NaCNBH₃ (8.90 mg, 0.142 mmol) was added at 0 °C. The reaction mixture was stirred at room temperature for 16 h. The volatiles were evaporated and dried in vacuum to get crude compound. The crude was purified via preparative LC/MS using method AA, fractions containing the product were combined and dried via centrifugal evaporation to get 2-(4-isopropyl-5-(8-methoxy-[1,2,4]triazolo[1,5-a] pyridin-6-yl)-1H-pyrazol-3-yl)-5-(1-((tetrahydro-2H-pyran-4-yl)methyl)piperidin-4-yl)thiazole (15.1 mg) as a white solid. LCMS Retention time: 0.85 min [E], MS (E⁺) m/z: 424.3 [M+H]; ¹H NMR (400 MHz, METHANOL-d₄) δ 8.53 (s, 1H), 8.43 (s, 1H), 7.66 (br. s., 1H), 7.17 (s, 1H), 4.11 (s, 3H), 3.95 (d, *J*=7.3 Hz, 2H), 3.63-3.36 (m, 4H), 3.20-2.93 (m, 3H), 2.33 (d, *J*=5.1 Hz, 2H), 2.27-2.14 (m, 2H), 2.09 (d, *J*=12.5 Hz, 2H), 1.96-1.77 (m, 3H), 1.73 (d, *J*=11.5Hz, 2H), 1.39-1.10 (m, 6H).

The following Examples were prepared according to the general procedure used to prepare Example 752.

**TABLE 84**

| Ex. No. | Structure | LCMS MH⁺ | RT (min) | HPLC Method |
|---|---|---|---|---|
| 753 | 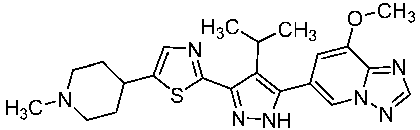 | 438.3 | 1.16 | C |
| 754 | 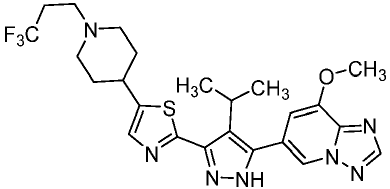 | 520.3 | 2.03 | C |
| 755 | 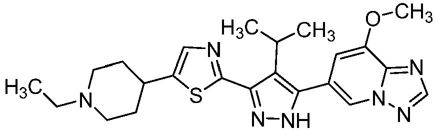 | 452.3 | 1.1 | C |
| 756 | 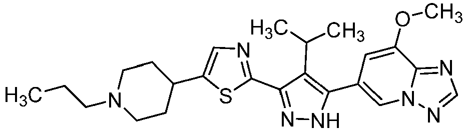 | 466.3 | 1.34 | C |
| 757 | 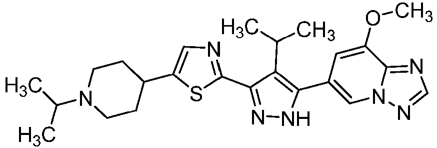 | 466.3 | 1.21 | C |
| 758 | 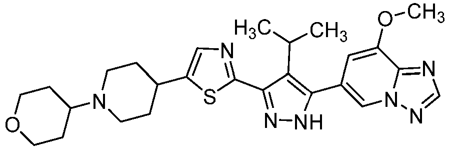 | 508.3 | 1.32 | C |
| 759 | 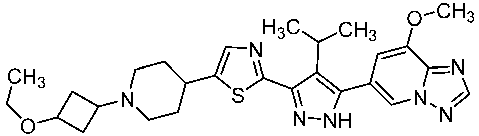 | 522.3 | 1.58 | C |
| 760 | 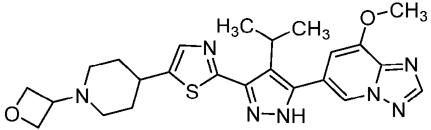 | 480.3 | 1.5 | C |
| 761 | 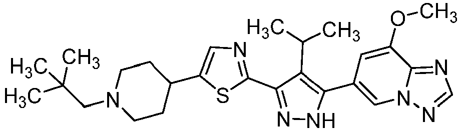 | 494.3 | 2.31 | C |
| 762 | 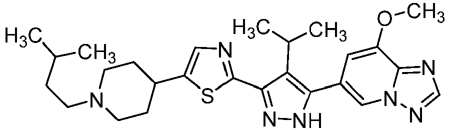 | 494.4 | 1.51 | C |
| 763 | 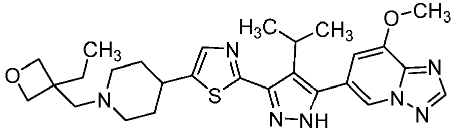 | 522.3 | 1.8 | C |
| 764 | 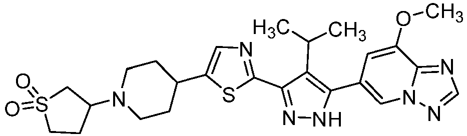 | 542.2 | 1.56 | D |
| 765 | 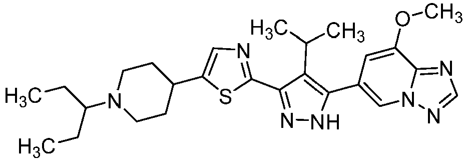 | 494.3 | 1.61 | C |
| 766 | 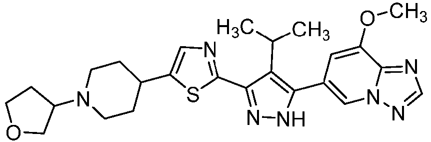 | 494.3 | 1.11 | D |
| 767 | 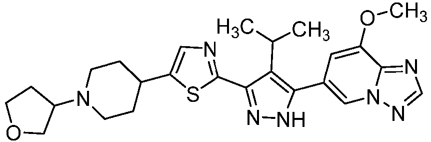 | 494.4 | 1.11 | D |
| 768 | 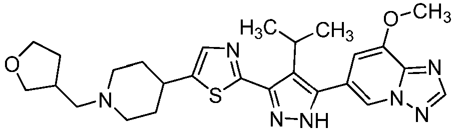 | 508.3 | 1.5 | C |
| 769 | 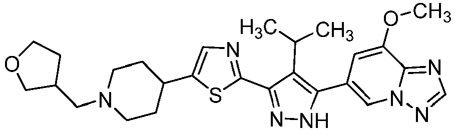 | 508.3 | 1.5 | C |
| 770 | 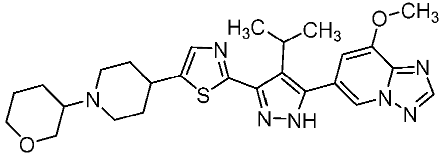 | 508.3 | 1.57 | C |
| 771 | 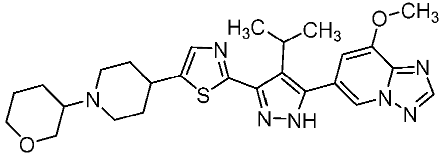 | 508.3 | 1.57 | C |
| 772 | 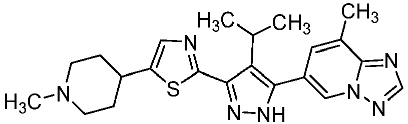 | 422.3 | 1.24 | C |
| 773 | 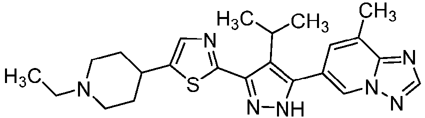 | 436.3 | 1.46 | C |
| 774 | 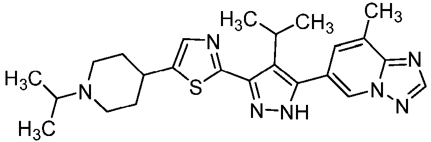 | 450.3 | 1.29 | C |
| 775 | 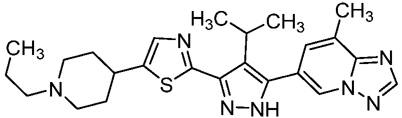 | 450.3 | 1.44 | C |
| 776 | 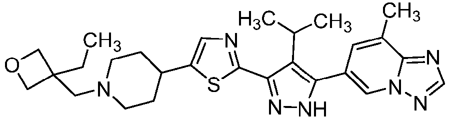 | 506.3 | 1.98 | C |
| 777 | 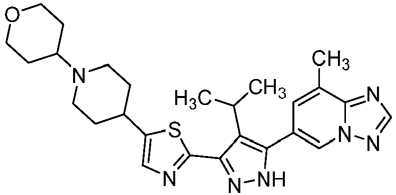 | 492.3 | 1.41 | C |
| 778 | 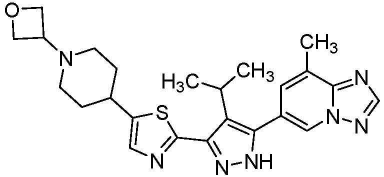 | 464.3 | 1.54 | C |
| 779 | 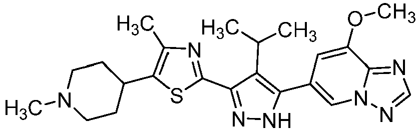 | 452.2 | 1.209 | E |
| 780 | 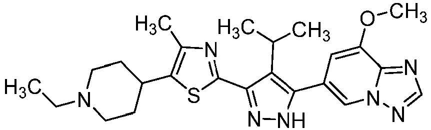 | 466.3 | 1.249 | E |
| 781 | 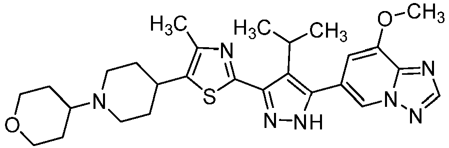 | 522.3 | 1.403 | E |
| 782 | 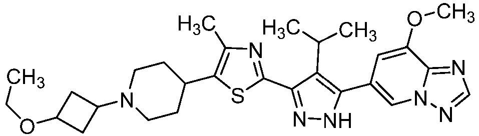 | 536.3 | 1.748 | E |
| 783 | 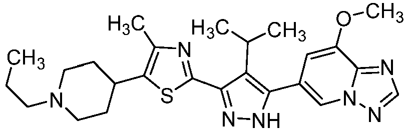 | 480.3 | 1.425 | E |
| 784 | 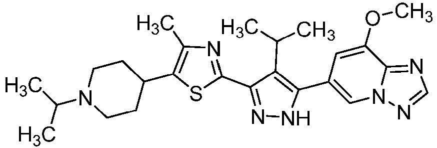 | 480.3 | 1.281 | E |
| 785 | 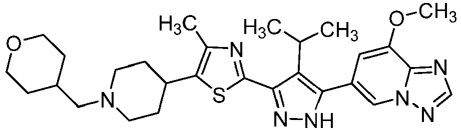 | 536.2 | 1.44 | E |
| 786 | 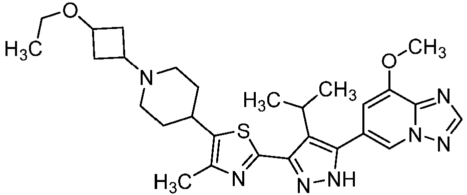 | 536.3 | 1.748 | E |
| 787 | 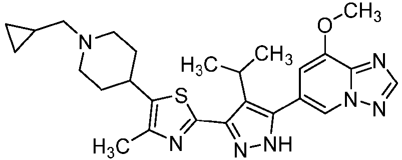 | 492.3 | 1.555 | E |
| 788 | 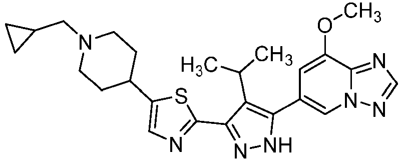 | 478.3 | 1.27 | C |
| 789 | 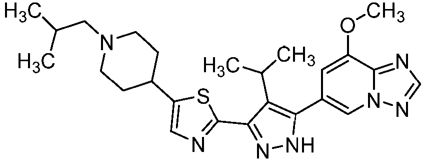 | 480.3 | 1.58 | C |
| 790 | 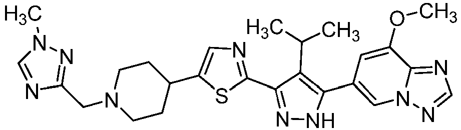 | 519.3 | 1.5 | C |
| 791 | 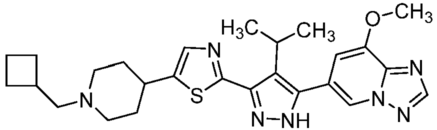 | 492.3 | 1.57 | C |
| 792 | 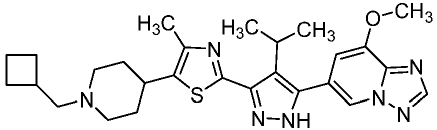 | 506.3 | 1.735 | E |
| 793 | 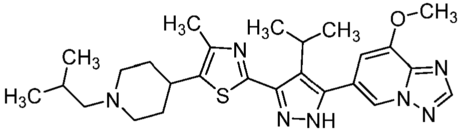 | 494.3 | 1.826 | E |
| 794 | 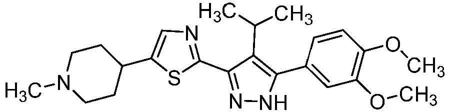 | 427.2 | 1.37 | C |
| 795 | 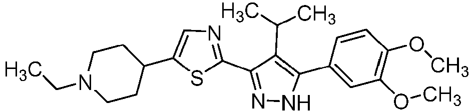 | 441.3 | 1.4 | C |
| 796 | 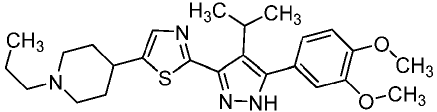 | 455.3 | 1.26 | C |
| 797 | 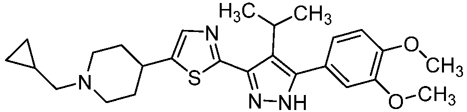 | 467.3 | 1.29 | C |
| 798 | 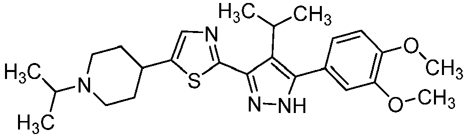 | 455.3 | 1.42 | C |
| 799 | 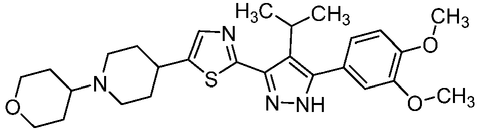 | 497.3 | 1.18 | C |
| 800 | 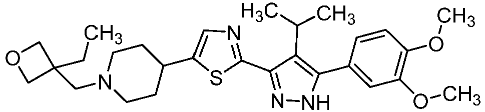 | 511.3 | 2.17 | C |
| 801 | 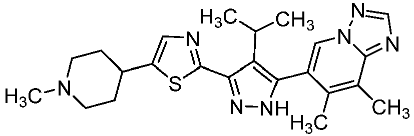 | 436.3 | 1.14 | C |
| 802 | 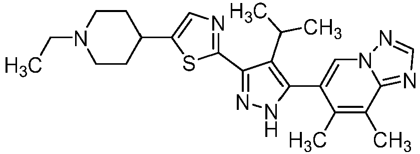 | 450.3 | 1.18 | C |
| 803 | 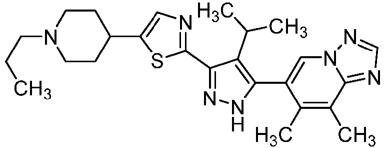 | 464.3 | 1.34 | C |
| 804 | 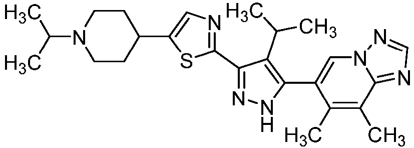 | 464.3 | 1.21 | C |
| 805 | 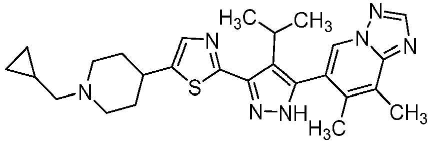 | 476.3 | 1.35 | C |
| 806 | 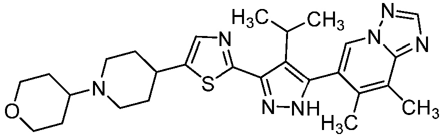 | 506.3 | 1.32 | C |
| 807 | 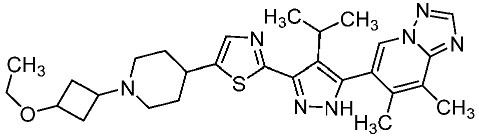 | 520.3 | 1.14 | C |
| 808 | 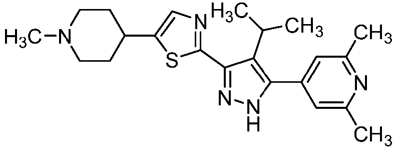 | 396.3 | 1.22 | C |
| 809 | 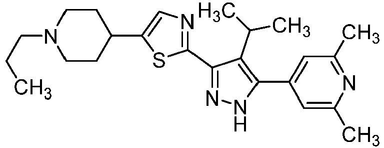 | 424.3 | 0.82 | C |
| 810 | 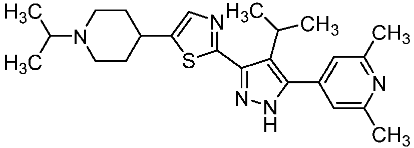 | 424.3 | 1.29 | C |
| 811 | 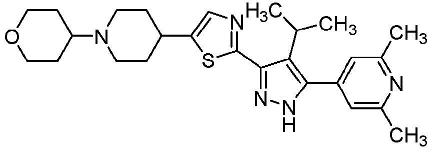 | 466.3 | 1.41 | C |
| 812 | 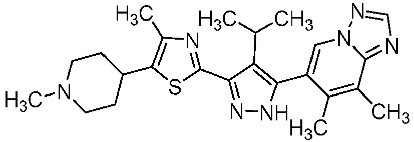 | 450.3 | 1.54 | C |
| 813 | 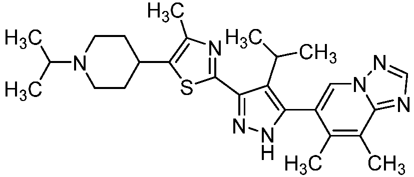 | 478.3 | 1.61 | C |
| 814 | 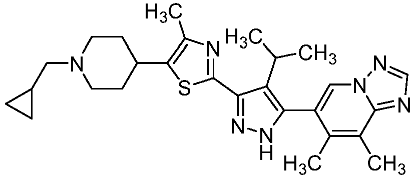 | 490.3 | 1.75 | C |
| 815 | 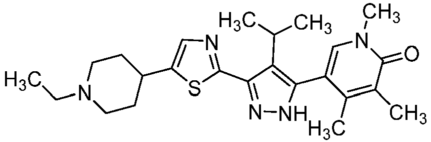 | 440.2 | 1.26 | C |
| 816 | 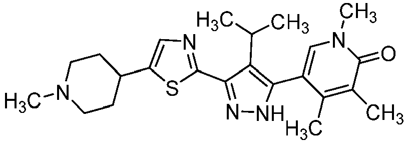 | 426.3 | 1.33 | C |
| 817 | 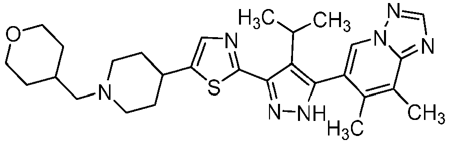 | 519.3 | 2.0 | C |
| 818 | 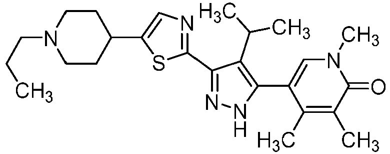 | 454.3 | 1.19 | D |
| 819 | 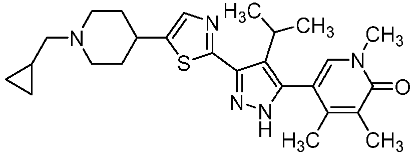 | 466.3 | 1.25 | D |
| 820 | 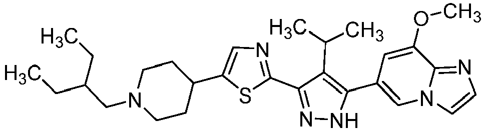 | 507.3 | 1.31 | D |
| 821 | 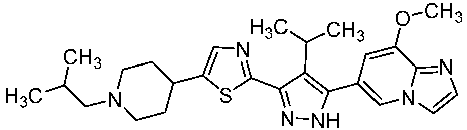 | 479.2 | 1.83 | C |
| 822 | 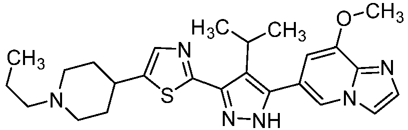 | 465.2 | 1.43 | C |
| 823 | 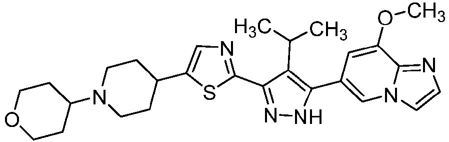 | 507.2 | 1.55 | C |
| 824 | 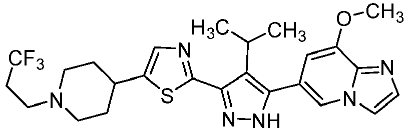 | 519.2 | 2.04 | C |
| 825 | 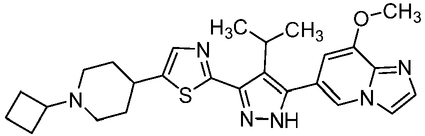 | 477.2 | 1.28 | C |
| 826 | 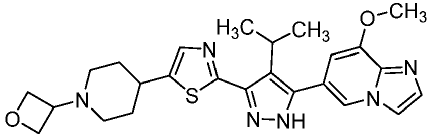 | 479.2 | 1.53 | C |
| 827 | 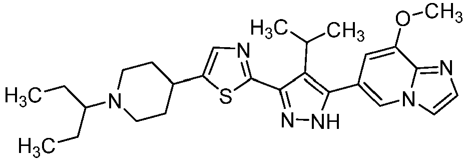 | 493.3 | 1.85 | C |
| 828 | 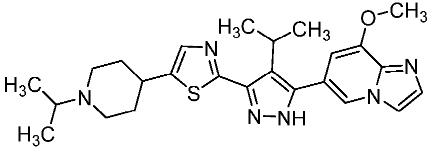 | 465.3 | 1.1 | C |
| 829 | 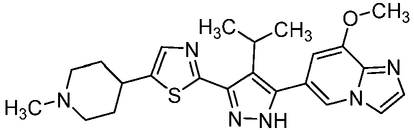 | 437.2 | 1.3 | C |
| 830 | 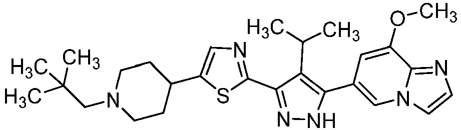 | 493.3 | 2.37 | C |
| 831 | 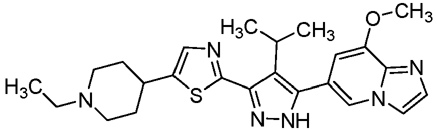 | 451.2 | 1.27 | C |
| 832 | 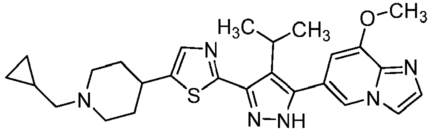 | 477.2 | 1.44 | C |
| 833 | 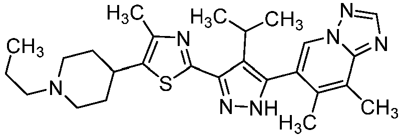 | 478.3 | 1.6 | C |
| 834 | 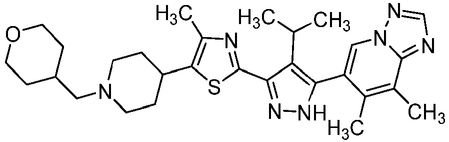 | 534.3 | 1.77 | C |
| 835 | 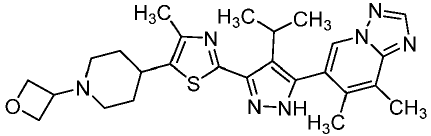 | 492.3 | 1.73 | C |
| 836 | 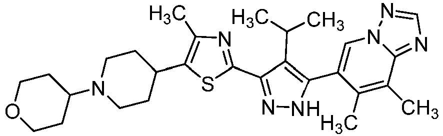 | 520.3 | 1.58 | C |
| 837 | 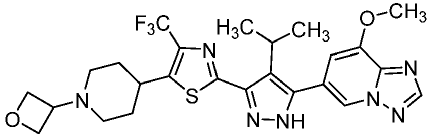 | 548.3 | 2.123 | B |
| 838 | 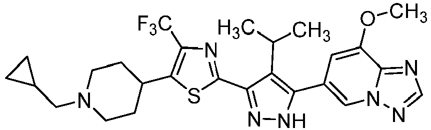 | 546.3 | 2.116 | E |
| 839 | 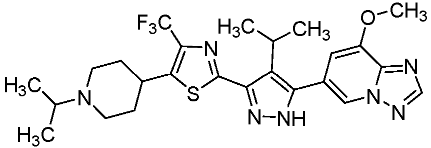 | 534.3 | 1.974 | E |
| 840 | 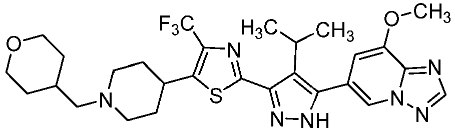 | 590.3 | 2.36 | E |
| 841 | 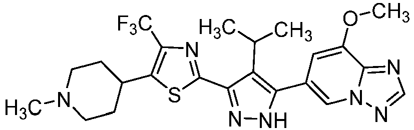 | 506.2 | 1.916 | E |
| 842 | 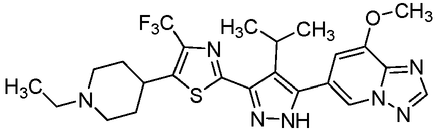 | 520.2 | 1.955 | E |
| 843 | 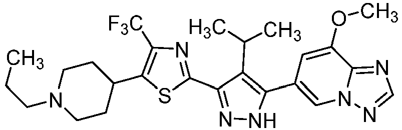 | 534.3 | 2.183 | E |

### EXAMPLE 844

### 1-(4-(2-(4-isopropyl-5-(8-methoxy-[1,2,4]triazolo[1,5-a]pyridin-6-yl)-1H-pyrazol-3-yl)thiazol-5-yl)piperidin-1-yl)-2-morpholinoethan-1-one

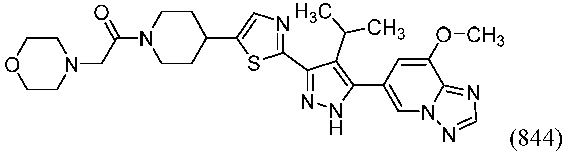

To a solution of 2-(4-isopropyl-5-(8-methoxy-[1,2,4]triazolo[1,5-a]pyridin-6-yl)-1H-pyrazol-3-yl)-5-(piperidin-4-yl)thiazole (40.0 mg, 0.094 mmol), 2-morpholinoacetic acid (68.5 mg, 0.472 mmol) in DMF (1.0 mL) were added TEA (0.132 mL, 0.944 mmol) and HATU (35.9 mg, 0.094 mmol) at 0 °C. The reaction mixture was stirred at room temperature for 16 h. The reaction mass was purified via preparative LC/MS using method AB, the fractions containing the product were combined and dried via centrifugal evaporation to get 1-(4-(2-(4-isopropyl-5-(8-methoxy-[1,2,4]triazolo[1,5-a]pyridin-6-yl)-1H-pyrazol-3-yl) thiazol-5-yl)piperidin-1-yl)-2-morpholinoethan-1-one (27 mg) as a white solid. LCMS Retention time: 1.44 min [E], MS (E⁺) m/z: 551.3 [M+H]; ¹H NMR (400 MHz, METHANOL-d₄) δ 8.55 (d, *J*=1.2 Hz, 1H), 8.46 (s, 1H), 7.71 (s, 1H), 7.17(d, *J*=1.2 Hz, 1H), 4.66 (d, *J*=13.2 Hz, 1H), 4.40-4.27 (m, 3H), 4.17-4.10 (m, 3H), 3.99 (br. s.,4H), 3.84 (d, *J*=12.2 Hz, 1H), 3.56-3.51 (m, 1H), 3.42-3.35 (m, 2H), 3.09-3.04 (m, 1H), 2.96(d, *J*=12.5 Hz, 1H), 2.19 (br. s., 2H), 1.82 (dd, *J*=12.7, 4.2 Hz, 2H), 1.77-1.67 (m, 1H), 1.40-1.27 (m, 7H).

The following Examples were prepared according to the general procedure used to prepare Example 844.

**TABLE 85**

| Ex. No. | Structure | LCMS MH⁺ | RT (min) | HPLC Method |
|---|---|---|---|---|
| 845 | 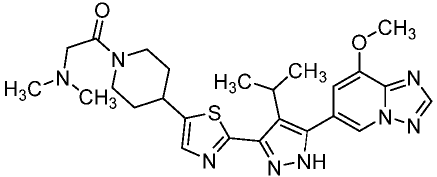 | 509.3 | 1.21 | C |
| 846 | 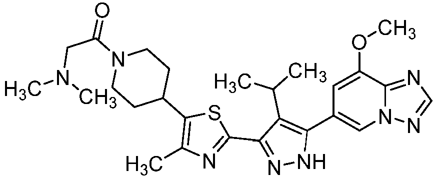 | 523.3 | 1.269 | E |
| 847 | 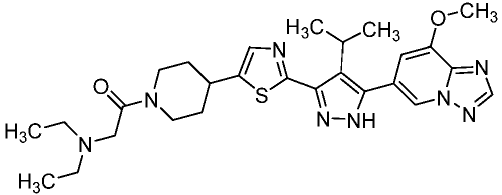 | 537.3 | 1.21 | C |
| 848 | 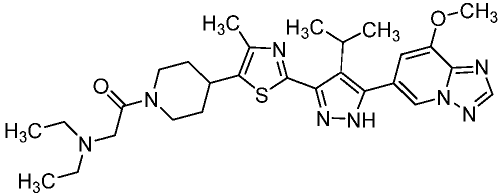 | 551.3 | 1.44 | E |
| 849 | 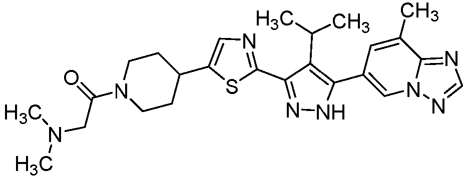 | 493.3 | 1.31 | C |
| 850 | 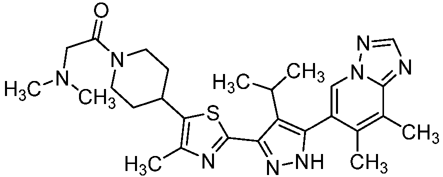 | 521.3 | 1.46 | C |
| 851 | 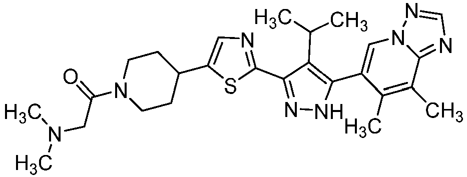 | 507.3 | 1.21 | C |
| 852 | 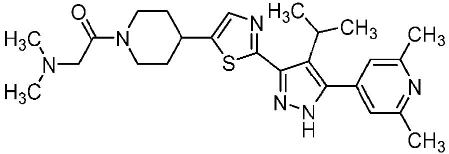 | 467.3 | 1.29 | C |
| 853 | 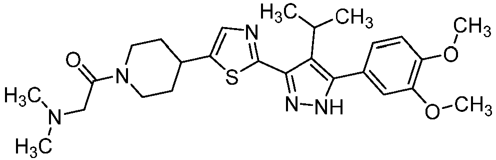 | 498.3 | 1.44 | C |
| 854 | 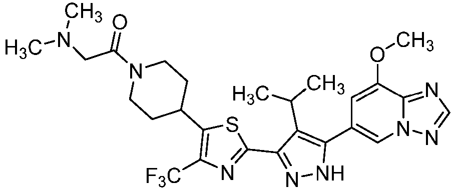 | 577.3 | 1.847 | E |

### EXAMPLE 855

### 2-(4-isopropyl-5-(8-methoxy-[1,2,4]triazolo[1,5-a]pyridin-6-yl)-1H-pyrazol-3-yl)-5-(1-(2-(methylsulfonyl)ethyl)piperidin-4-yl)thiazole

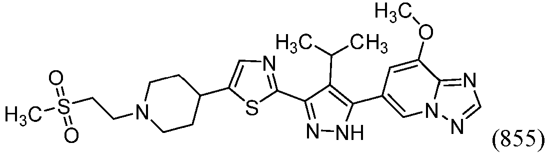

To a solution of 2-(4-isopropyl-5-(8-methoxy-[1,2,4]triazolo[1,5-a]pyridin-6-yl)-1H-pyrazol-3-yl)-5-(piperidin-4-yl)thiazole (30.0 mg, 0.071 mmol) and 1-chloro-2-(methylsulfonyl)ethane (30.3 mg, 0.212 mmol) in DMF (1.0 mL) and THF (2.0 mL) solvent mixture was added TEA (0.099 mL, 0.708 mmol) at 0 °C. The reaction mixture was stirred at room temperature for 16 h. The reaction mass was purified via preparative LC/MS using method AA, the fractions containing the product were combined and dried via centrifugal evaporation to get 2-(4-isopropyl-5-(8-methoxy-[1,2,4]triazolo[1,5-a]pyridin-6-yl)-1H-pyrazol-3-yl)-5-(1-(2-(methylsulfonyl)ethyl)piperidin-4-yl)thiazole (15.7 mg) as a pale solid. LCMS Retention time: 1.47min [E], MS (E⁺) m/z: 530.2 [M+H]; ¹H NMR (400 MHz, METHANOL-d₄) δ 8.62-8.51 (m, 1H), 8.49-8.40 (m, 1H), 7.65 (d, *J*=6.6Hz, 1H), 7.18 (br. s., 1H), 4.19-4.09 (m, 3H), 3.62-3.44 (m, 2H), 3.40-3.35 (m, 1H), 3.18 -3.07 (m, 4H), 3.07-2.96 (m, 1H), 2.93 (t, *J*=6.8 Hz, 2H), 2.31 (td, *J*=11.8, 2.3 Hz, 2H), 2.13 (d,*J*=13.0 Hz, 2H), 2.00 (s, 1H), 1.84 (qd, *J*=12.3, 3.5 Hz, 2H), 1.38-1.27 (m, 5H), 1.23 (d, *J*=7.1Hz, 1H).

The following Examples were prepared according to the general procedure used to prepare Example 855.

**TABLE 86**

| Ex. No. | Structure | LCMS MH⁺ | RT (min) | HPLC Method |
|---|---|---|---|---|
| 856 | 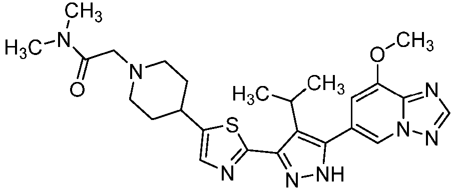 | 509.3 | 1.39 | C |
| 857 | 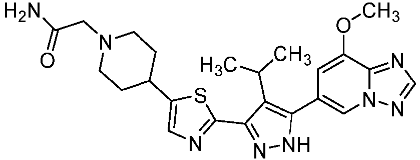 | 481.3 | 1.35 | C |
| 858 | 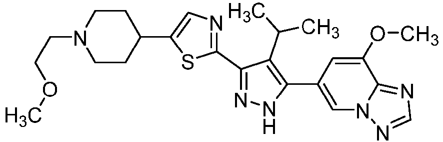 | 482.3 | 1.32 | C |
| 859 | 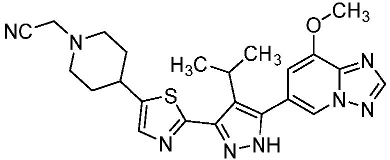 | 463.3 | 1.59 | C |
| 860 | 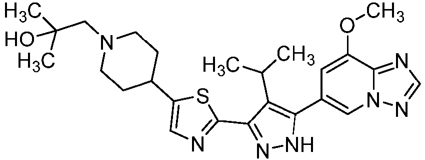 | 496.3 | 1.43 | C |
| 861 | 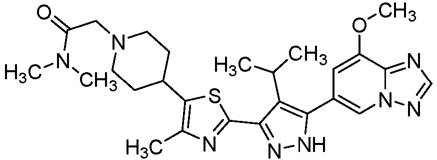 | 523.3 | 1.429 | E |
| 862 | 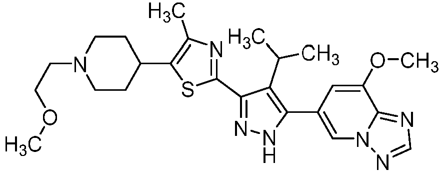 | 496.3 | 1.531 | E |
| 863 | 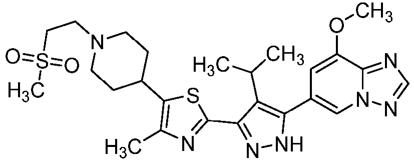 | 1.786 | 544.3 | E |
| 864 | 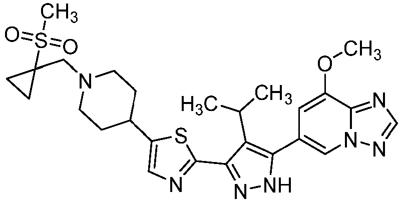 | 556.3 | 1.77 | C |
| 865 | 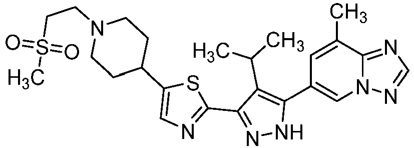 | 514.3 | 1.52 | C |
| 866 | 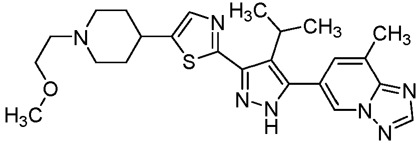 | 466.3 | 1.44 | C |
| 867 | 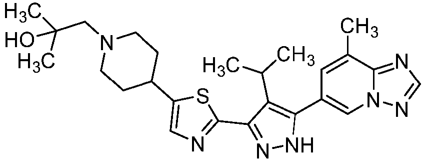 | 480.3 | 1.53 | C |
| 868 | 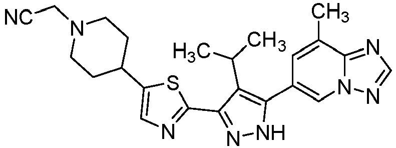 | 447.3 | 1.64 | C |
| 869 | 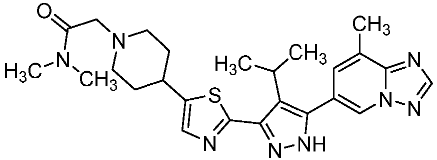 | 493.3 | 1.12 | D |
| 870 | 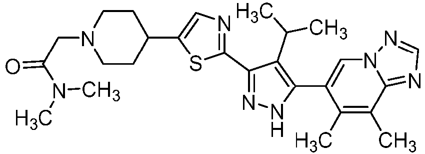 | 507.3 | 1.38 | C |
| 871 | 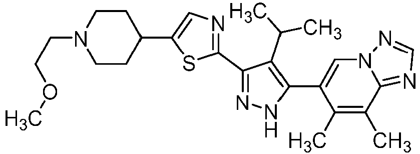 | 480.3 | 1.35 | C |
| 872 | 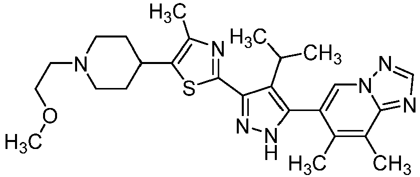 | 494.3 | 1.62 | C |
| 873 | 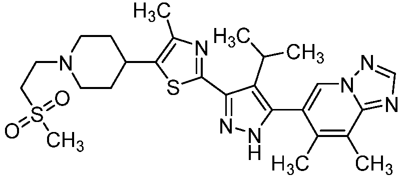 | 542.3 | 1.69 | C |
| 874 | 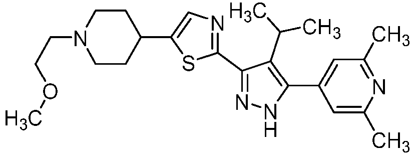 | 440.3 | 1.44 | C |
| 875 | 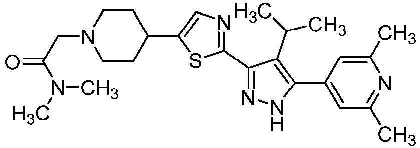 | 467.3 | 1.46 | C |
| 876 (Ref) | 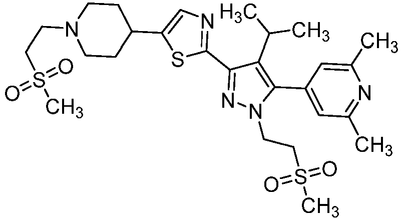 | 594.3 | 1.56 | C |
| 877 | 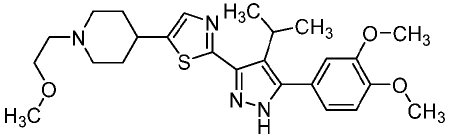 | 471.3 | 1.59 | C |
| 878 | 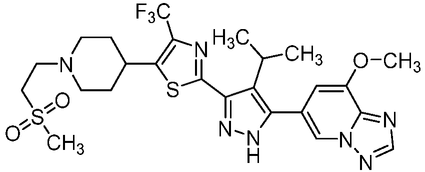 | 598.3 | 2.083 | F |
| 879 | 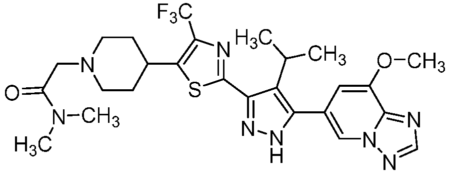 | 577.3 | 2.047 | F |
| 880 | 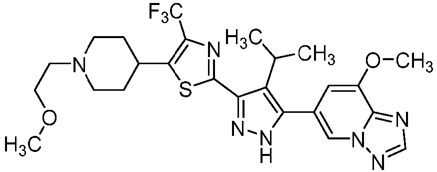 | 550.3 | 2.145 | E |
| 881 | 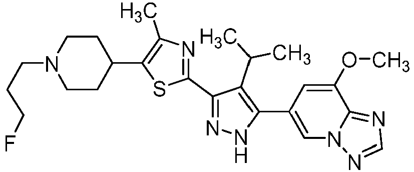 | 498.2 | 1.691 | E |
| 882 | 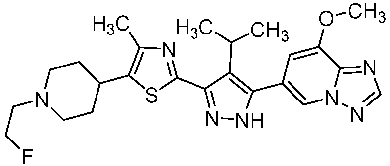 | 484.2 | 1.779 | E |

### EXAMPLE 883

### 4-(4-(2-(4-isopropyl-5-(8-methoxy-[1,2,4]triazolo[1,5-a]pyridin-6-yl)-1H-pyrazol-3-yl)-4-methylthiazol-5-yl)cyclohexyl)morpholine

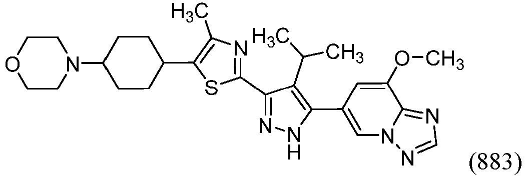

### Intermediate 883A: 4-methyl-5-(1,4-dioxaspiro[4.5]dec-7-en-8-yl)thiazole

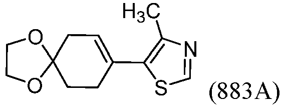

4-methyl-5-(1,4-dioxaspiro[4.5]dec-7-en-8-yl)thiazole (5.1 g, 21.49 mmol, 77 % yield) was prepared according to the general process described in Intermediate 307A using 5-bromo-4-methylthiazole (5.0 g, 28.1 mmol) and 4,4,5,5-tetramethyl-2-(1,4-dioxaspiro[4.5]dec-7-en-8-yl)-1,3,2-dioxaborolane (8.97 g, 33.7 mmol) as starting intermediates to get the title compound as a light brown solid. LCMS Retention time: 1.85 min [B], MS (E⁺) m/z: 238.2 [M+H].

### Intermediate 883B: 4-methyl-5-(1,4-dioxaspiro[4.5]decan-8-yl)thiazole

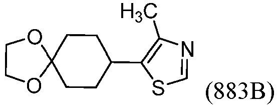

4-Methyl-5-(1,4-dioxaspiro[4.5]decan-8-yl)thiazole (5.0 g, 20.89 mmol, 90% yield) was prepared according to the general process described in Intermediate 307C using 4-methyl-5-(1,4-dioxaspiro[4.5]dec-7-en-8-yl)thiazole (5.5 g, 23.18 mmol) as a starting intermediate to get the title compound as an off-white solid. LCMS Retention time: 1.88 min [B], MS (E⁺) m/z: 240.2 [M+H].

### Intermediate 883C: 2-bromo-4-methyl-5-(1,4-dioxaspiro[4.5]decan-8-yl)thiazole

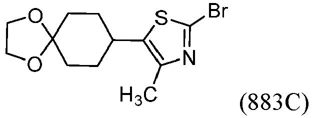

To a solution of 4-methyl-5-(1,4-dioxaspiro[4.5]decan-8-yl)thiazole (4.5 g, 18.80 mmol) in THF (100 mL) was added n-butyl lithium (1.807 g, 28.2 mmol) at -78 °C. The reaction mixture was stirred for 40 min and a solution of CCl₄ (9.35 g, 28.2 mmol) in THF (20 mL) was added. The reaction mixture was stirred at -78 °C for 1 h. The reaction was quenched slowly with cold water. The reaction mixture was stirred for 10 min at room temperature, diluted with excess ethyl acetate, washed with water, brine, dried over sodium sulphate and concentrated to get crude product. The crude product was purified by ISCO using 40 g silica column, the compound was eluted with 25-30% ethyl acetate and pet ether, the fractions were collected and concentrated to get 2-bromo-4-methyl-5-(1,4-dioxaspiro[4.5]decan-8-yl)thiazole (3.0 g, 9.43 mmol, 50.1 % yield) as a light brown semi-solid. LCMS Retention time: 2.84 min [B], MS (E⁺) m/z: 318.0 [M⁺].

### Intermediate 883D: 2-(4-isopropyl-5-(8-methoxy-[1,2,4]triazolo[1,5-a]pyridin-6-yl)-1-((2-(trimethylsilyl)ethoxy)methyl)-1H-pyrazol-3-yl)-4-methyl-5-(1,4-dioxaspiro[4.5]decan-8-yl)thiazole

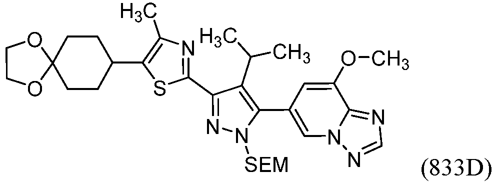

2-(4-isopropyl-5-(8-methoxy-[1,2,4]triazolo[1,5-a]pyridin-6-yl)-1-((2-(trimethylsilyl) ethoxy)methyl)-1H-pyrazol-3-yl)-4-methyl-5-(1,4-dioxaspiro[4.5]decan-8-yl)thiazole (790 mg, 1.264 mmol, 33% yield) was prepared according to the general process described in Intermediate 307B, using 6-(4-isopropyl-3-(4,4,5,5-tetramethyl-1,3,2-dioxaborolan-2-yl)-1-((2-(trimethylsilyl)ethoxy)methyl)-1H-pyrazol-5-yl)-8-methoxy-[1,2,4]triazolo[1,5-a] pyridine (3.29 g, 6.41 mmol) and 2-bromo-4-methyl-5-(1,4-dioxaspiro[4.5]decan-8-yl) thiazole (1.2 g, 3.77 mmol) as starting intermediates to get the title compound as a light brown semi-solid. LCMS Retention time: 1.64 min [A], MS (E⁺) m/z: 625.5 [M+H].

### Intermediate 883E: 4-(2-(4-isopropyl-5-(8-methoxy-[1,2,4]triazolo[1,5-a]pyridin-6-yl)-1H-pyrazol-3-yl)-4-methylthiazol-5-yl)cyclohexan-1-one

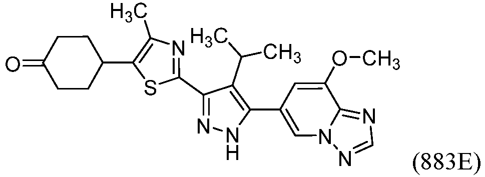

4-(2-(4-isopropyl-5-(8-methoxy-[1,2,4]triazolo[1,5-a]pyridin-6-yl)-1H-pyrazol-3-yl)-4-methyl thiazol-5-yl)cyclohexan-1-one (480 mg, 1.065 mmol, 89 % yield) was prepared according to the general process described in Intermediate 307D using 2-(4-isopropyl-5-(8-methoxy-[1,2,4]triazolo[1,5-a]pyridin-6-yl)-1-((2-(trimethylsilyl)ethoxy)methyl)-1H-pyrazol-3-yl)-4-methyl-5-(1,4-dioxaspiro[4.5]decan-8-yl)thiazole (750 mg, 1.200 mmol) as a starting intermediate to get the title compound as an off-white solid. LCMS Retention time: 1.40 min [A], MS (E⁺) m/z: 451.0 [M+H].

### Example 883: 4-(4-(2-(4-isopropyl-5-(8-methoxy-[1,2,4]triazolo[1,5-a]pyridin-6-yl)-1H-pyrazol-3-yl)-4-methylthiazol-5-yl)cyclohexyl)morpholine

A solution of 4-(2-(4-isopropyl-5-(8-methoxy-[1,2,4]triazolo[1,5-a]pyridin-6-yl)-1H-pyrazol-3-yl)-4-methylthiazol-5-yl)cyclohexan-1-one (60.0 mg, 0.133 mmol), morpholine (58.0 mg, 0.666 mmol) and acetic acid (0.762 µL, 0.013 mmol) in DMF (1.0 mL) and THF (1.5 mL) was stirred at room temperature for 8 h. Next, sodium cyanoborohydride (16.74 mg, 0.266 mmol) was added at 0 °C and the reaction mixture was stirred at room temperature for 16 h. The reaction mixture was purified by preparative LC/MS using method AA, to separate both the isomers, the fractions containing the product were combined and dried via centrifugal evaporation to get:
Example 883A: Isomer 1 (9.3 mg) as a pale solid. LCMS Retention time: 1.80 min [E], MS (E⁺) m/z: 522.3 [M+H]; ¹H NMR (400 MHz, DMSO-d₆) δ 8.54 (br s, 1H), 8.45 (br s, 1H), 7.18 (br s, 1H), 4.13 (s, 3H), 3.77 (br s, 4H), 3.59-3.38 (m, 1H), 2.97 (br s, 1H), 2.84-2.59 (m, 4H), 2.55-2.35 (m, 4H), 2.15 (br s, 4H), 1.65-1.42 (m, 4H), 1.34 (br d, *J*=7.1 Hz, 7H); and
Example 883B: Isomer 2 (8.0 mg) as a pale solid. LCMS Retention time: 2.13 min [E], MS (E⁺) m/z: 522.3 (M+H); ¹H NMR (400 MHz, DMSO-d₆) δ 8.55 (br s, 1H), 8.45 (br s, 1H), 7.30-7.05 (m, 1H), 4.14 (s, 3H), 3.86-3.66 (m, 5H), 3.62-3.37 (m, 1H), 3.21 (br s, 1H),2.68 (s, 1H), 2.58 (br s, 4H), 2.45 (br s, 3H), 2.36-2.25 (m, 1H), 2.07 (br d, J=8.1 Hz, 2H), 1.97-1.85 (m, 2H), 1.84-1.61 (m, 4H), 1.34 (br d, *J*=6.8 Hz,7H), 0.97-0.78 (m, 1H).

The following Examples were prepared according to the general procedure used to prepare Example 883.

**TABLE 87**

| Ex. No. | Structure | LCMS MH⁺ | RT (min) | HPLC Method |
|---|---|---|---|---|
| 884 | 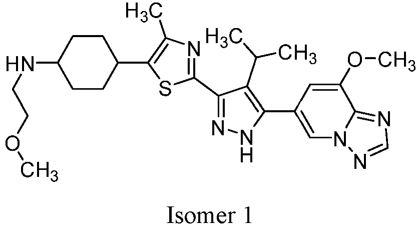 | 510.3 | 1.24 | C |
| 885 | 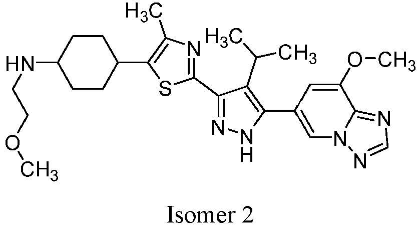 | 510.3 | 1.65 | C |
| 886 | 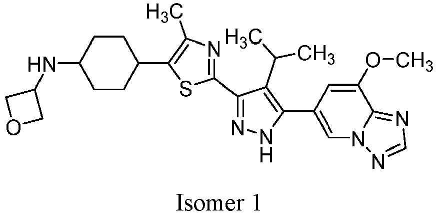 | 508.3 | 1.86 | C |
| 887 | 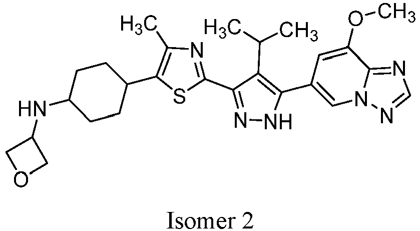 | 508.2 | 1.55 | C |
| 888 | 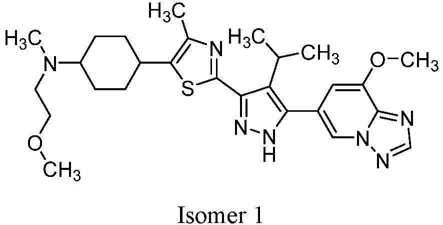 | 524.3 | 1.57 | C |
| 889 | 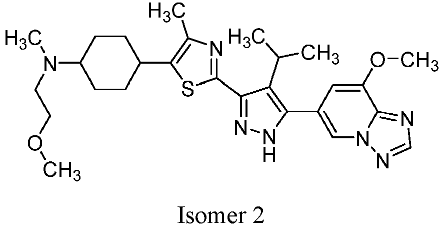 | 524.3 | 1.67 | C |
| 890 | 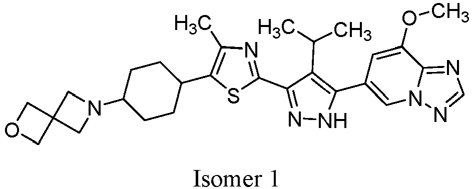 | 534.3 | 1.55 | C |
| 891 | 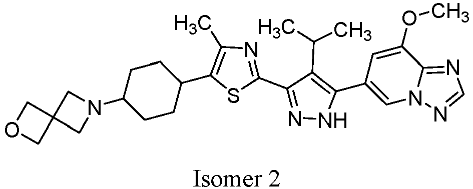 | 534.3 | 1.8 | C |
| 892 | 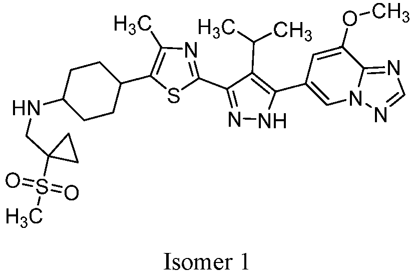 | 584.3 | 1.74 | C |
| 893 | 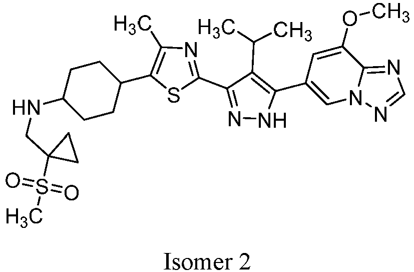 | 584.3 | 2.08 | C |
| 894 | 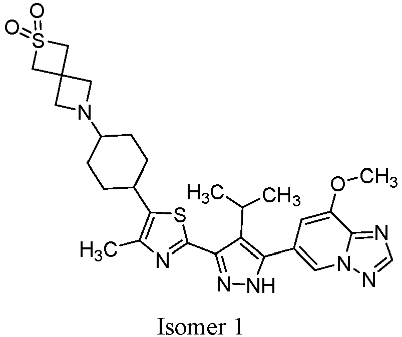 | 582.3 | 1.6 | C |
| 895 | 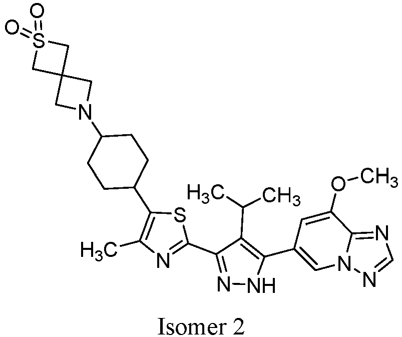 | 582.3 | 1.93 | C |
| 896 | 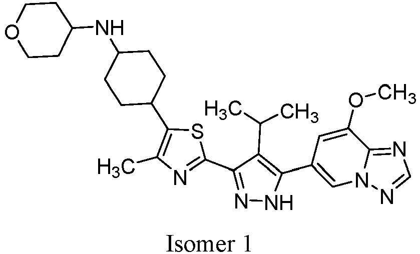 | 536.3 | 1.36 | C |
| 897 | 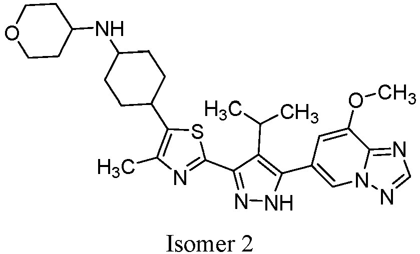 | 536.3 | 1.66 | C |
| 898 | 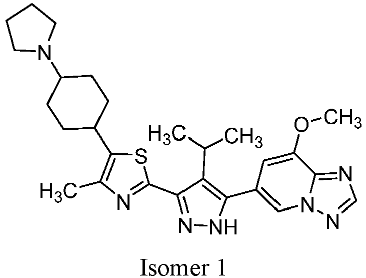 | 506.2 | 1.59 | C |
| 899 | 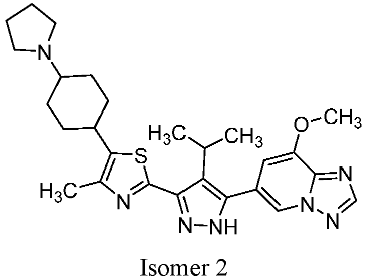 | 506.2 | 1.74 | C |
| 891B | 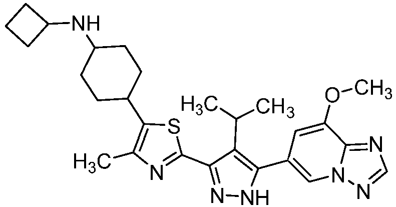 | 506.3 | 1.66 | C |
| | 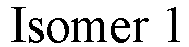 | | | |
| 892B | 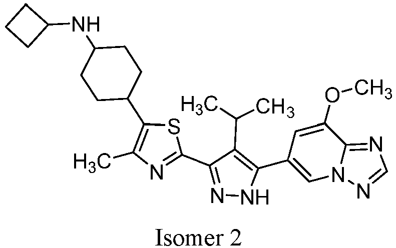 | 506.2 | 1.7 | C |
| 893B | 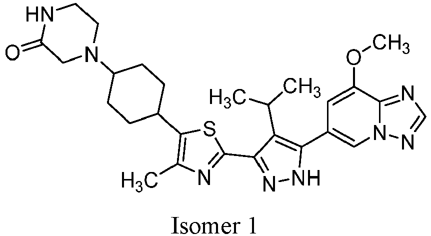 | 535.2 | 1.75 | C |
| 894B | 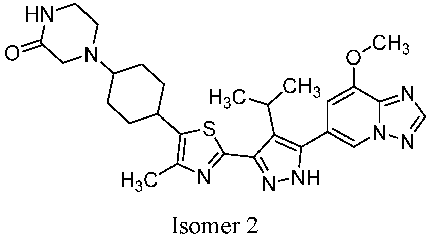 | 535.2 | 1.97 | C |
| 895B | 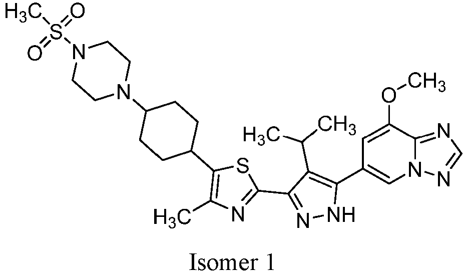 | 599.2 | 2.01 | C |
| 896B | 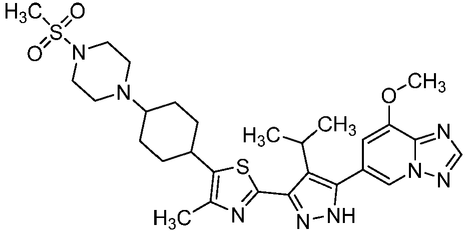 | 599.3 | 2.3 | C |
| | 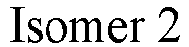 | | | |
| 897B | 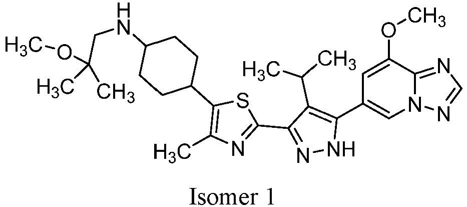 | 538.3 | 1.52 | C |
| 898B | 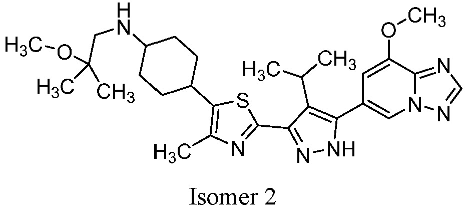 | 538.3 | 1.79 | C |
| 899B | 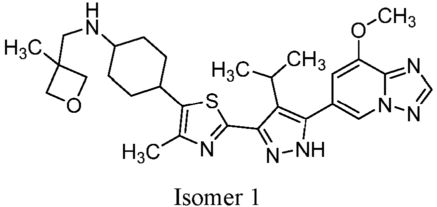 | 536.3 | 1.71 | C |
| 900 | 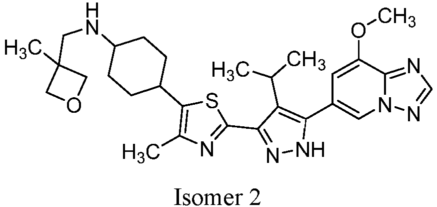 | 536.2 | 2.07 | C |
| 901 | 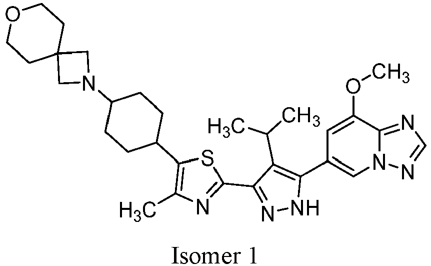 | 562.2 | 1.66 | C |
| 902 | 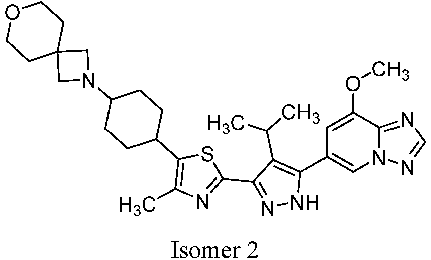 | 562.3 | 1.93 | C |
| 903 | 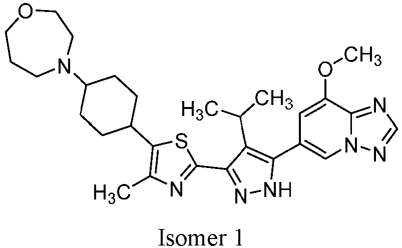 | 536.2 | 1.76 | C |
| 904 | 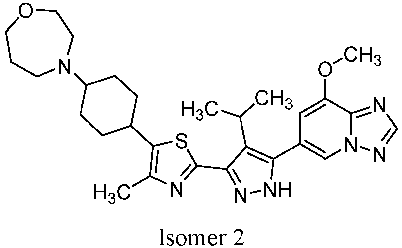 | 536.2 | 1.94 | C |
| 905 | 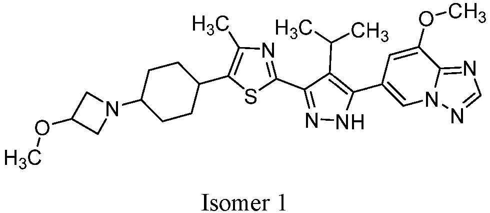 | 522.2 | 1.74 | C |
| 906 | 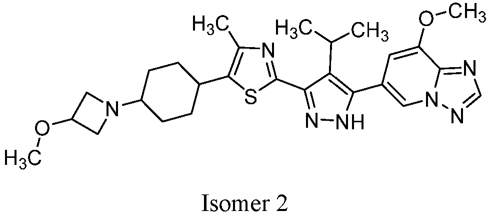 | 522.2 | 2.08 | C |
| 907 | 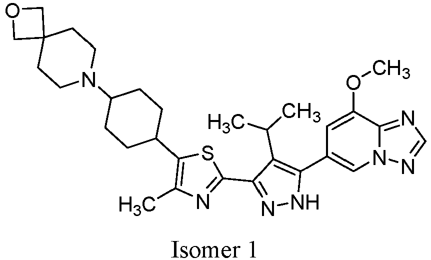 | 562.3 | 1.34 | C |
| 908 | 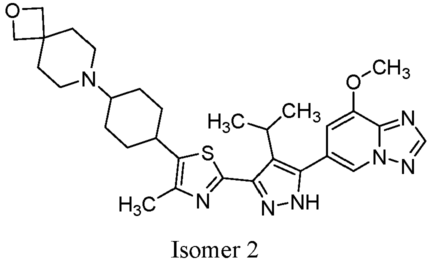 | 562.2 | 1.88 | C |
| 909 | 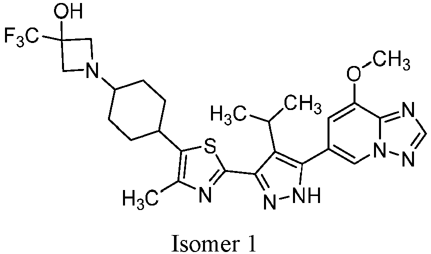 | 576.2 | 2.08 | C |
| 910 | 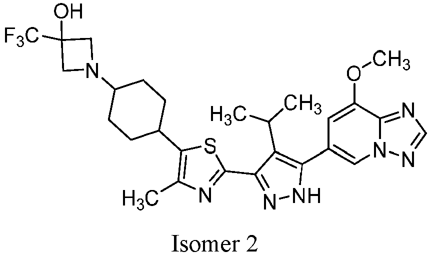 | 576.2 | 2.42 | C |
| 911 | 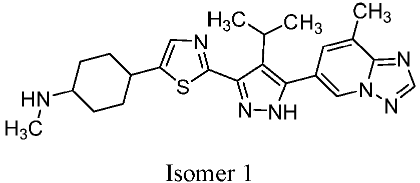 | 436.3 | 1.27 | C |
| 912 | 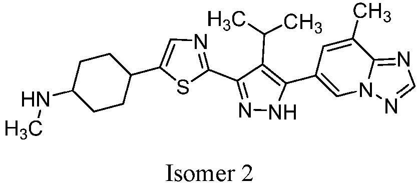 | 436.3 | 1.32 | C |
| 913 | 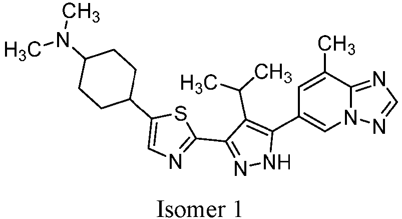 | 450.3 | 1.32 | C |
| 914 | 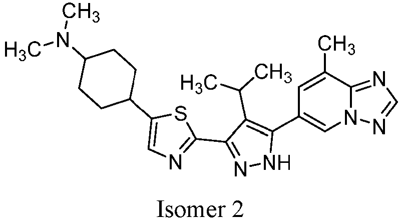 | 450.3 | 1.25 | C |
| 915 | 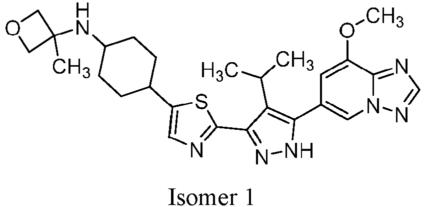 | 508.3 | 1.2 | C |
| 916 | 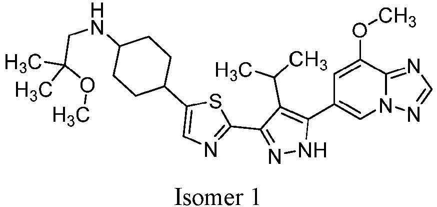 | 524.3 | 1.44 | C |
| 917 | 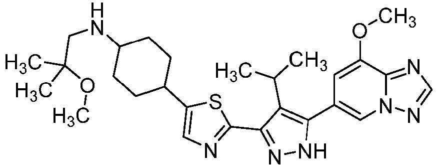 | 524.3 | 1.55 | C |
| | 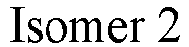 | | | |
| 918 | 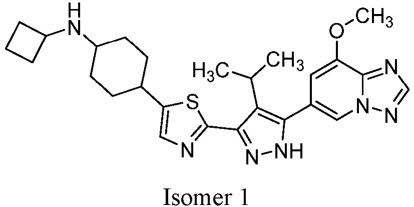 | 492.3 | 1.44 | C |
| 919 | 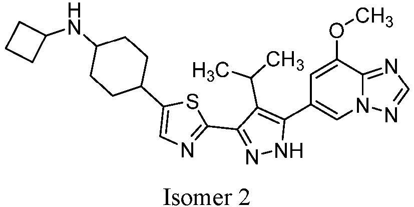 | 492.3 | 1.33 | C |
| 920 | 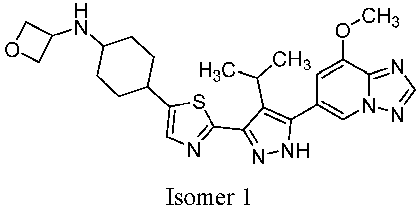 | 494.1 | 1.46 | C |
| 921 | 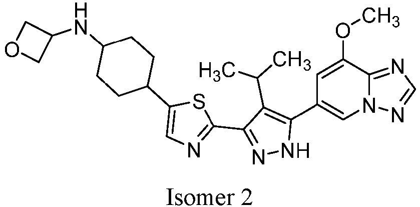 | 494.3 | 1.54 | C |
| 922 | 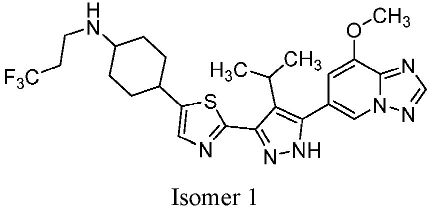 | 534.3 | 1.75 | C |
| 923 | 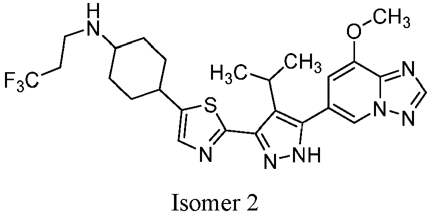 | 534.3 | 1.94 | C |
| 924 | 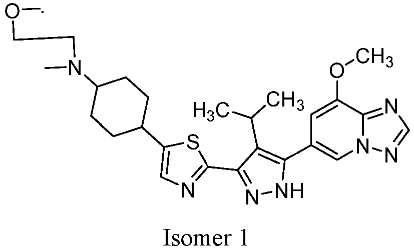 | 520.3 | 1.15 | C |
| 925 | 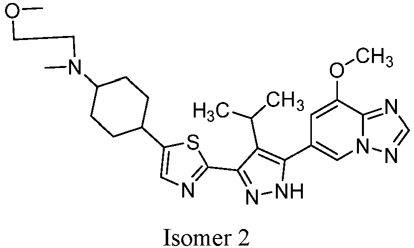 | 520.3 | 1.48 | C |

### EXAMPLE 926

### 2-(dimethylamino)-N-(4-(2-(4-isopropyl-5-(8-methyl-[1,2,4]triazolo[1,5-a]pyridin-6-yl)-1H-pyrazol-3-yl)thiazol-5-yl)cyclohexyl)-N-methylacetamide

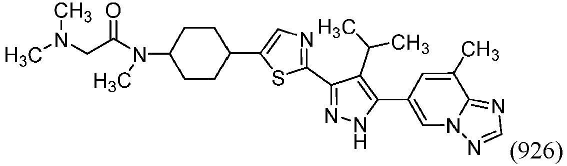

2-(dimethylamino)-N-(4-(2-(4-isopropyl-5-(8-methyl-[1,2,4]triazolo [1,5-a] pyridin-6-yl)-1H-pyrazol-3-yl)thiazol-5-yl)cyclohexyl)-N-methylacetamide (3.6 mg) was prepared according to the general process described in Example 469, using 4-(2-(4-isopropyl-5-(8-methyl-[1,2,4]triazolo[1,5-a]pyridin-6-yl)-1H-pyrazol-3-yl)thiazol-5-yl)-N-methylcyclohexan-1-amine (20.0 mg, 0.046 mmol) as a starting intermediate to get the title compound as an off-white solid. LCMS Retention time: 1.49 min [E], MS (E⁺) m/z: 521.3 [M+H]; ¹H NMR (400 MHz, METHANOL-d₄) δ 8.80 (br. s., 1H), 8.50 (s, 1H), 7.75 (br. s., 1H), 7.61 (br.s., 1H), 4.49 (t, *J*=11.7 Hz, 1H), 3.94 (br. s., 1H), 3.62-3.44 (m, 2H), 3.40 (br. s., 1H), 2.88 (s,2H), 2.78 (s, 1H), 2.72 (s, 3H), 2.41 (s, 5H), 2.27 (br. s., 2H), 2.17-2.00 (m, 2H), 2.00-1.90(m, 1H), 1.85 (d, *J*=11.2 Hz, 1H), 1.70 (d, *J*=13.7 Hz, 1H), 1.59 (d, *J*=9.8 Hz, 1H), 1.38-1.15(m, 6H).

The following Example was prepared according to the general procedure used to prepare Example 926.

**TABLE 88**

| Ex. No. | Structure | LCMS MH⁺ | RT (min) | HPLC Method |
|---|---|---|---|---|
| 927 | 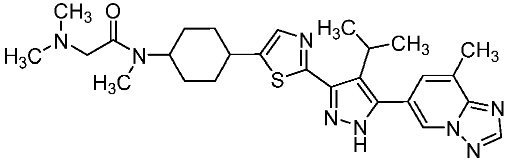 | 521.3 | 1.5 | C |

### EXAMPLE 928

### 4-(2-(4-isopropyl-5-(8-methyl-[1,2,4]triazolo[1,5-a]pyridin-6-yl)-1H-pyrazol-3-yl) thiazol-5-yl)-N-methyl-N-(3,3,3-trifluoropropyl)cyclohexan-1-amine

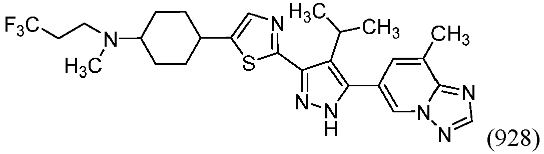

4-(2-(4-isopropyl-5-(8-methyl-[1,2,4]triazolo[1,5-a]pyridin-6-yl)-1H-pyrazol-3-yl)thiazol-5-yl)-N-methyl-N-(3,3,3-trifluoropropyl)cyclohexan-1-amine (2.5 mg) was prepared according to the general process described in Example 307, using 4-(2-(4-isopropyl-5-(8-methyl-[1,2,4]triazolo[1,5-a]pyridin-6-yl)-1H-pyrazol-3-yl)thiazol-5-yl)-N-methylcyclohexan-1-amine (20.0 mg, 0.046 mmol) as a starting intermediate to get the title compound as an off-white solid. LCMS Retention time: 2.15 min [E], MS (E⁺) m/z: 532.3 [M+H]; ¹H NMR (400 MHz, METHANOL-d₄) δ 8.79 (br. s., 1H), 8.49 (s, 1H), 7.60 (s, 1H), 7.63 (s,1H), 3.60-3.43 (m, 1H), 3.08 (qd, *J*=10.6, 6.4 Hz, 1H), 2.96 (br. s., 1H), 2.88 (br. s., 2H), 2.76 -2.55 (m, 4H), 2.55-2.30 (m, 5H), 2.25 (d, *J*=12.2 Hz, 2H), 2.04 (d, *J*=11.7 Hz, 2H), 1.72-1.47 (m, 4H), 1.31 (d, *J*=7.1 Hz, 6H).

The following Example was prepared according to the general procedure used to prepare Example 928.

**TABLE 89**

| Ex. No. | Structure | LCMS MH⁺ | RT (min) | HPLC Method |
|---|---|---|---|---|
| 929 | 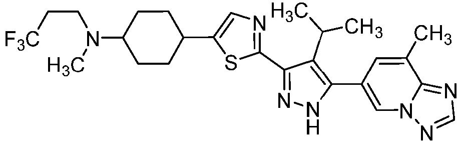 | 532.3 | 2.23 | C |

### EXAMPLE 930

### 2-(4-isopropyl-5-(8-methoxy-[1,2,4]triazolo[1,5-a]pyridin-6-yl)-1H-pyrazol-3-yl)-5-(piperazin-1-yl)thiazole

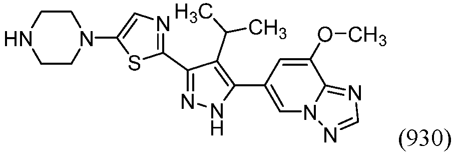

### Intermediate 930A: tert-butyl 4-(thiazol-5-yl)piperazine-1-carboxylate

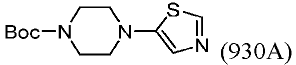

To a solution of 5-bromothiazole (2.0 g, 12.19 mmol) in acetonitrile (15.0 mL) at 0 °C were added tert-butyl piperazine-1-carboxylate (2.95 g, 15.85 mmol) and K₂CO₃ (5.06 g, 36.6 mmol) at room temperature. The reaction mixture was stirred at 120 °C for 16 h. The reaction mixture was filtered through celite, washed with ethyl acetate, the filtrate was collected and concentrated to get crude compound. The crude compound was purified by ISCO using 40 g silica column, compound was eluted with 35-45% ethyl acetate in pet ether, the fractions were collected and concentrated to get tert-butyl 4-(thiazol-5-yl)piperazine-1-carboxylate (2.2 g, 8.17 mmol, 67.0 % yield) as a light brown solid. LCMS Retention time: 2.25 min [B], MS (E⁺) m/z: 270.2 [M+H].

### Intermediate 930B: tert-butyl 4-(2-bromothiazol-5-yl)piperazine-1-carboxylate

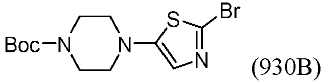

tert-Butyl 4-(2-bromothiazol-5-yl)piperazine-1-carboxylate (1.8 g, 5.17 mmol, 56 % yield) was prepared according to the general process described in Intermediate 883C, using tert-butyl 4-(thiazol-5-yl)piperazine-1-carboxylate (2.5 g, 9.28 mmol) as a starting intermediate to get the title compound as an off-white solid. LCMS Retention time: 1.35 min [B], MS (E⁺) m/z: 348.0 [M+].

### Intermediate 930C: tert-butyl 4-(2-(4-isopropyl-5-(8-methoxy-[1,2,4]triazolo[1,5-a] pyridin-6-yl)-1-((2-(trimethyl silyl)ethoxy)methyl)-1H-pyrazol-3-yl)thiazol-5-yl) piperazine-1-carboxylate

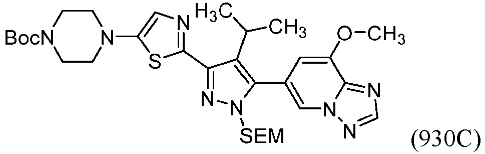

tert-butyl 4-(2-(4-isopropyl-5-(8-methoxy-[1,2,4]triazolo[ 1,5-a]pyridin-6-yl)-1-((2-(trimethyl silyl)ethoxy)methyl)-1H-pyrazol-3-yl)thiazol-5-yl)piperazine-1-carboxylate (320 mg, 0.489 mmol, 42 % yield) was prepared according to the general process described in Intermediate 307B, using 6-(4-isopropyl-3-(4,4,5,5-tetramethyl-1,3,2-dioxaborolan-2-yl)-1-((2-(trimethylsilyl)ethoxy)methyl)-1H-pyrazol-5-yl)-8-methoxy-[1,2,4]triazolo[1,5-a] pyridine (1.003 g, 1.953 mmol) as a starting intermediate to get the title compound as an off-white solid. LCMS Retention time: 1.47 min [A], MS (E⁺) m/z: 655.5 [M+H].

### Example 930: 2-(4-isopropyl-5-(8-methoxy-[1,2,4]triazolo[1,5-a]pyridin-6-yl)-1H-pyrazol-3-yl)-5-(piperazin-1-yl)thiazole

2-(4-isopropyl-5-(8-methoxy-[1,2,4]triazolo[1,5-a]pyridin-6-yl)-1H-pyrazol-3-yl)-5-(piperazin-1-yl)thiazole (200 mg, 0.471 mmol, 96 % yield) was prepared according to the general process described in Intermediate 307D using tert-butyl 4-(2-(4-isopropyl-5-(8-methoxy-[1,2,4]triazolo[1,5-a]pyridin-6-yl)-1-((2-(trimethylsilyl)ethoxy)methyl)-1H-pyrazol-3-yl)thiazol-5-yl)piperazine-1-carboxylate (0.320 g, 0.489 mmol) as a starting intermediate to get the title compound as an off-white solid. LCMS Retention time: 0.83 min [A], MS (E⁺) m/z: 425.1 [M+H]; ¹H NMR (400 MHz, METHANOL-d₄) δ 8.54 (d, J=1.2 Hz, 1H), 8.45 (s, 1H), 7.25 (s, 1H), 7.16(s, 1H), 4.13 (s, 3H), 3.58-3.41 (m, 8H), 3.06 (q, J=7.4 Hz, 4H), 1.42 (s, 1H), 1.39-1.21 (m,6H).

The following Examples were prepared according to the general procedure used to prepare Example 930.

**TABLE 90**

| Ex. No. | Structure | LCMS MH⁺ | RT (min) | HPLC Method |
|---|---|---|---|---|
| 931 | 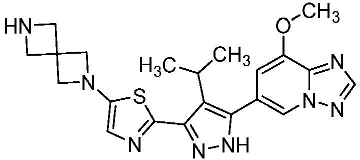 | 437.3 | 1.19 | C |
| 932 | 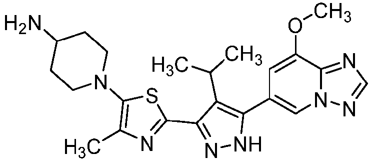 | 453.2 | 1.17 | E |
| 933 | 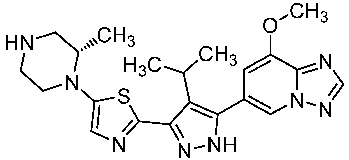 | 439.3 | 1.34 | C |
| 934 | 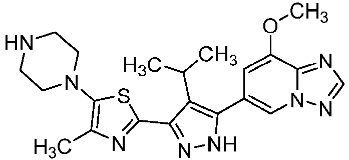 | 439.2 | 1.365 | E |
| 935 | 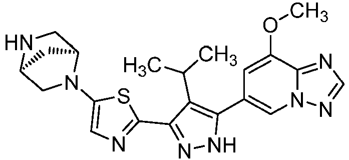 | 437.2 | 1.11 | C |
| 936 | 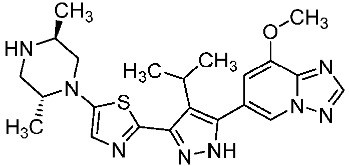 | 453.2 | 1.26 | C |
| 937 | 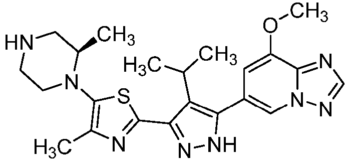 | 453.3 | 1.34 | AA |
| 938 | 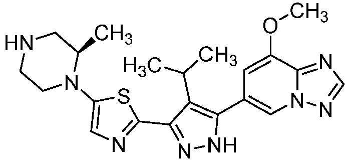 | 439.1 | 1.2 | AA |

### EXAMPLE 939

### 2-(4-isopropyl-5-(8-methoxy-[1,2,4]triazolo[1,5-a]pyridin-6-yl)-1H-pyrazol-3-yl)-5-(4-methylpiperazin-1-yl)thiazole

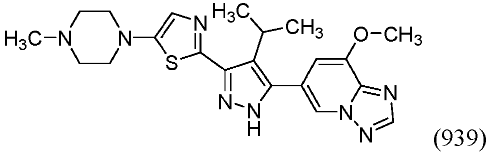

2-(4-Isopropyl-5-(8-methoxy-[1,2,4]triazolo[1,5-a]pyridin-6-yl)-1H-pyrazol-3-yl)-5-(4-methyl piperazin-1-yl)thiazole (6 mg) was prepared according to the general process described in Example 307, using 2-(4-isopropyl-5-(8-methoxy-[1,2,4]triazolo[1,5-a]pyridin-6-yl)-1H-pyrazol-3-yl)-5-(piperazin-1-yl)thiazole (20.0 mg, 0.047 mmol) as a starting intermediate to get the title compound as an off-white solid. LCMS Retention time: 1.406 min [E], MS (E⁺) m/z: 439.1 [M+H]; ¹H NMR (400 MHz, METHANOL-d₄) δ 8.52 (br s, 1H), 8.43 (s, 1H), 7.98 (s, 1H), 7.25-7.02 (m, 2H), 4.57 (s, 1H), 4.11 (s, 3H), 3.28-3.24 (m, 4H), 2.99(s, 2H), 2.86 (d, *J* =0.7 Hz, 2H), 2.76-2.61 (m, 4H), 2.40 (s, 3H), 1.39-1.16 (m, 8H).

The following Examples were prepared according to the general procedure used to prepare Example 939.

**TABLE 91**

| Ex. No. | Structure | LCMS MH⁺ | RT (min) | HPLC Method |
|---|---|---|---|---|
| 940 | 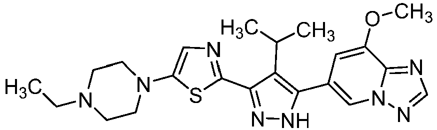 | 453.3 | 1.67 | C |
| 941 | 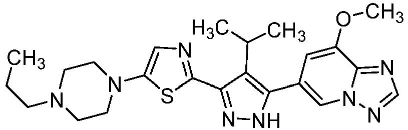 | 467.3 | 1.65 | C |
| 942 | 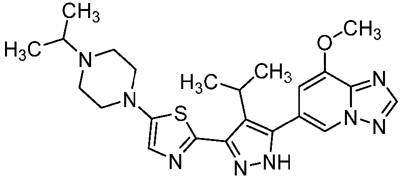 | 467.3 | 1.53 | C |
| 943 | 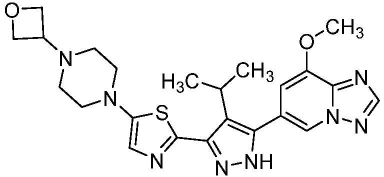 | 481.3 | 1.29 | D |
| 944 | 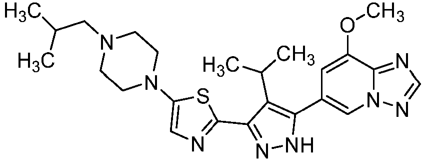 | 481.3 | 2.13 | C |
| 945 | 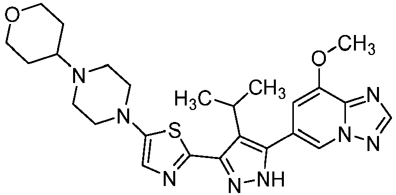 | 509.3 | 1.41 | C |
| 946 | 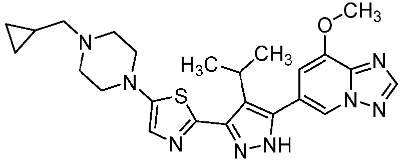 | 479.2 | 1.73 | C |
| 947 | 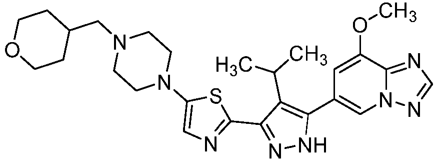 | 523.3 | 1.74 | C |
| 948 | 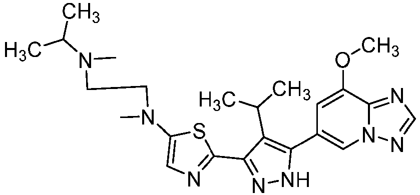 | 479.3 | 1.47 | C |
| 949 | 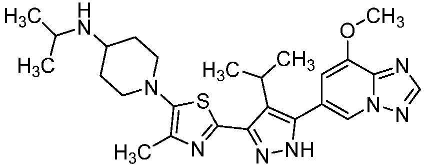 | 495.2 | 1.45 | F |
| 950 | 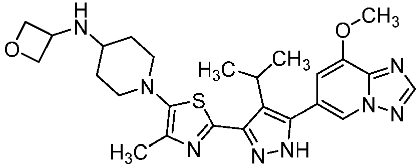 | 509.2 | 1.64 | E |
| 951 | 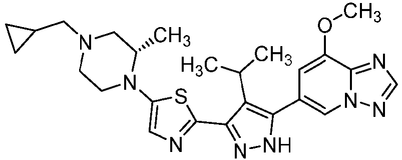 | 493.3 | 2.09 | C |
| 952 | 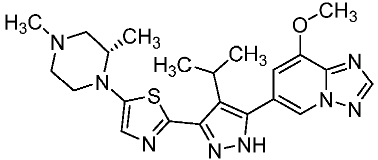 | 453.2 | 1.73 | C |
| 953 | 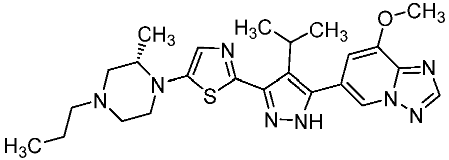 | 481.3 | 2.15 | C |
| 954 | 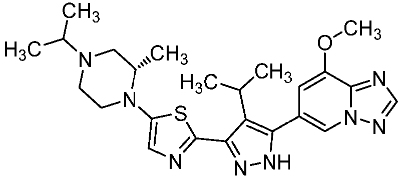 | 481.3 | 2 | C |
| 955 | 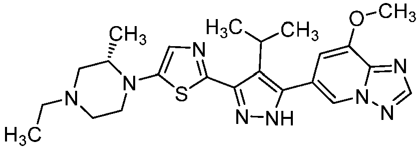 | 467.3 | 1.85 | C |
| 956 | 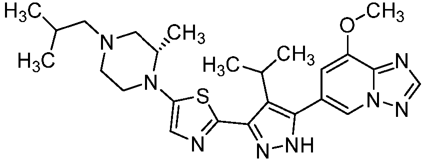 | 495.3 | 2.57 | C |
| 957 | 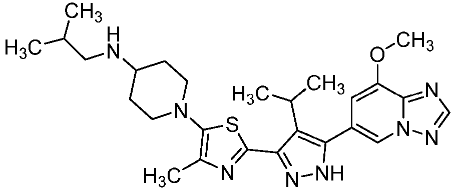 | 509.3 | 1.53 | C |
| 958 | 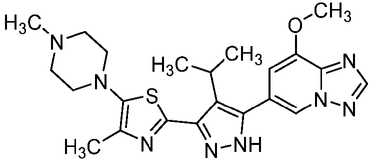 | 453.2 | 1.755 | E |
| 959 | 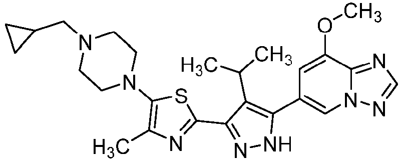 | 493.3 | 1.673 | E |
| 960 | 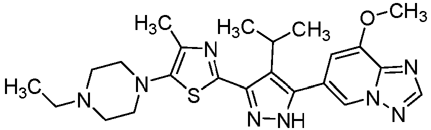 | 466.3 | 1.499 | E |
| 961 | 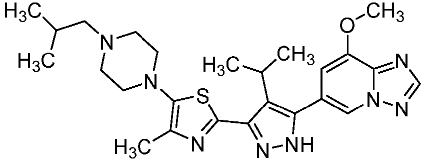 | 495.51 | 1.14 | F |
| 962 | 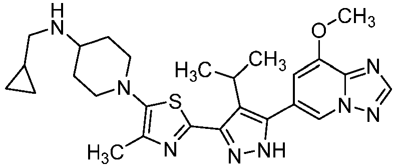 | 507.3 | 1.42 | C |
| 963 | 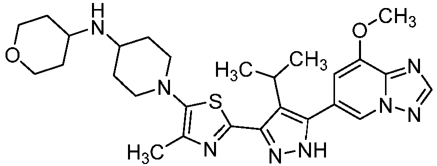 | 537.3 | 1.22 | C |
| 964 | 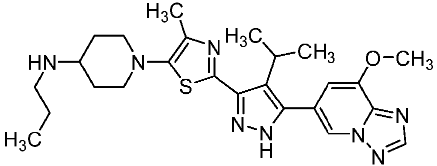 | 495.3 | 1.4 | C |
| 965 | 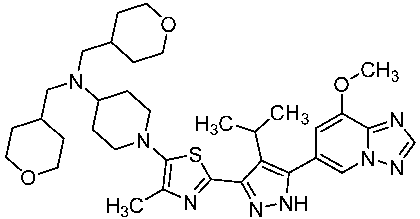 | 649.4 | 1.38 | C |
| 966 | 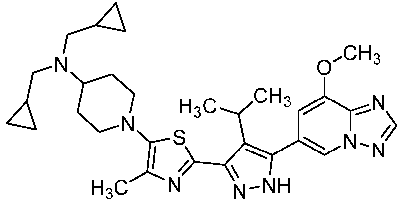 | 561.3 | 1.52 | C |
| 967 | 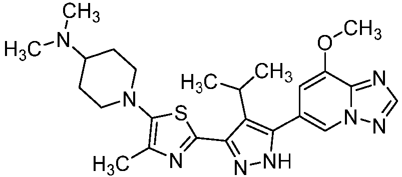 | 481.3 | 1.33 | C |
| 968 | 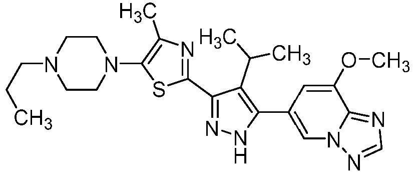 | 481.3 | 1.959 | E |
| 969 | 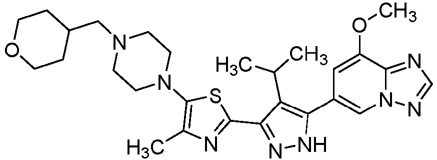 | 537.3 | 1.981 | E |
| 970 | 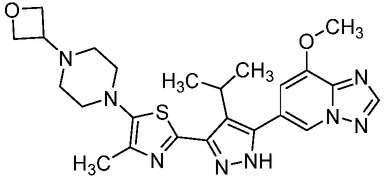 | 495.3 | 1.605 | E |
| 971 | 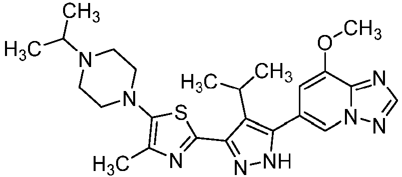 | 481.3 | 1.767 | E |
| 972 | 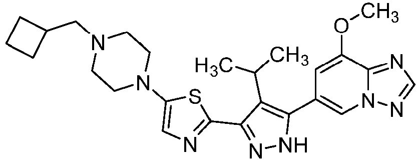 | 493.3 | 2.35 | C |
| 973 | 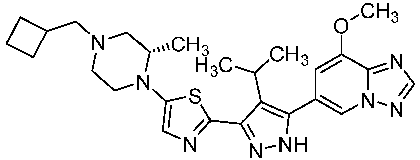 | 507.3 | 2.47 | C |
| 974 | 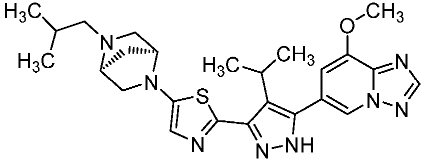 | 493.2 | 1.78 | C |
| 975 | 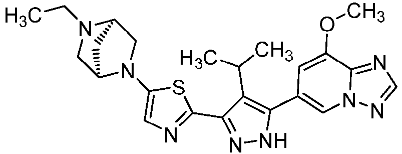 | 465.2 | 1.28 | C |
| 976 | 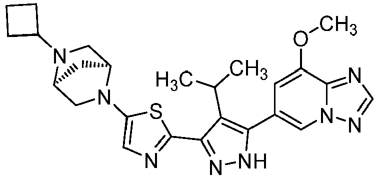 | 491.3 | 1.53 | C |
| 977 | 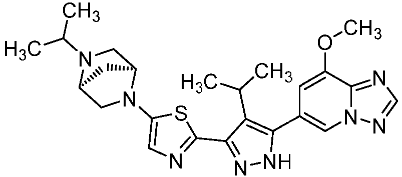 | 479.2 | 1.38 | C |
| 978 | 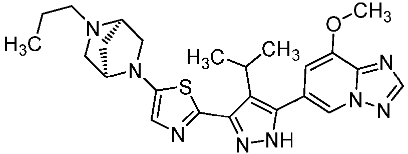 | 479.2 | 1.45 | C |
| 979 | 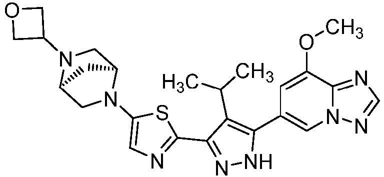 | 493.2 | 1.37 | C |
| 980 | 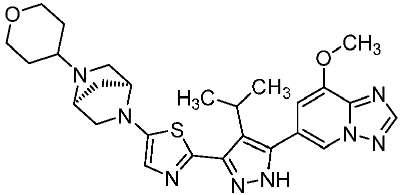 | 521.2 | 1.41 | C |
| 981 | 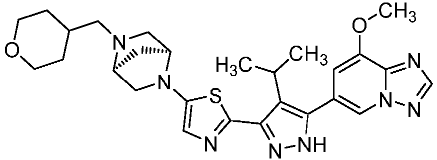 | 535.3 | 1.54 | C |
| 982 | 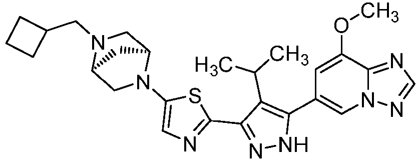 | 505.2 | 1.7 | C |
| 983 | 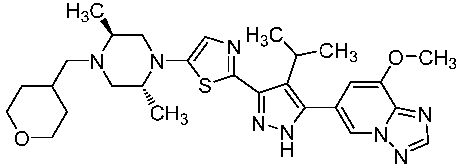 | 551.3 | 2.02 | C |
| 984 | 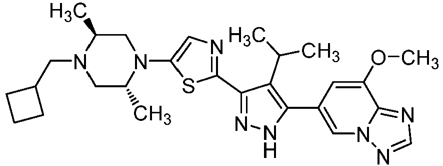 | 521.3 | 2.28 | C |
| 985 | 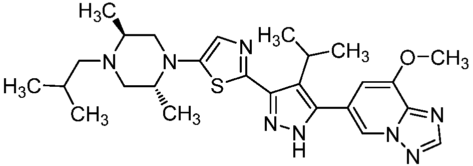 | 509.3 | 2.46 | C |
| 986 | 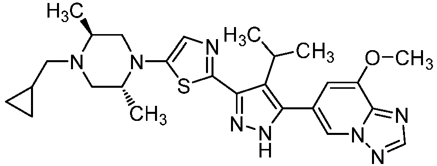 | 507.3 | 2.08 | C |
| 987 | 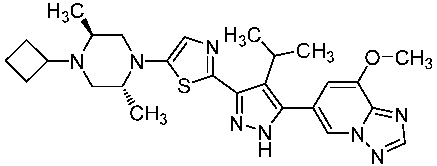 | 507.2 | 2.08 | C |
| 988 | 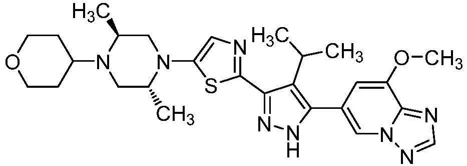 | 537.2 | 1.73 | C |
| 989 | 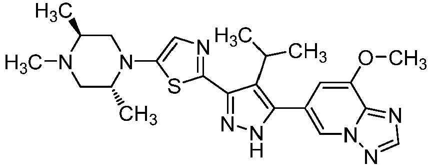 | 467.2 | 1.61 | C |
| 990 | 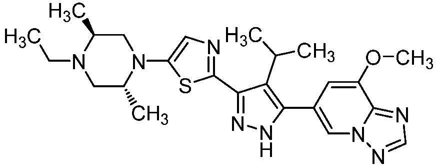 | 481.3 | 1.71 | C |
| 991 | 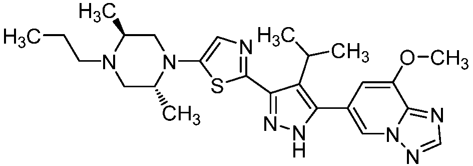 | 495.2 | 2.02 | C |
| 992 | 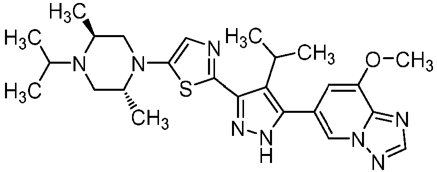 | 495.2 | 1.77 | C |
| 993 | 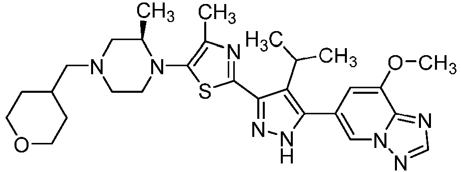 | 551.3 | 2.15 | C |
| 994 | 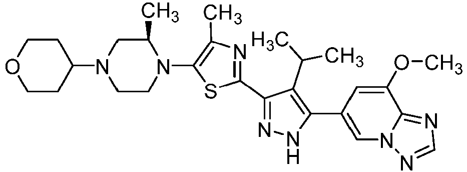 | 537.3 | 1.88 | C |
| 995 | 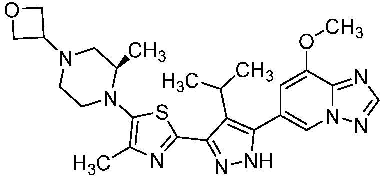 | 509.3 | 1.77 | C |
| 996 | 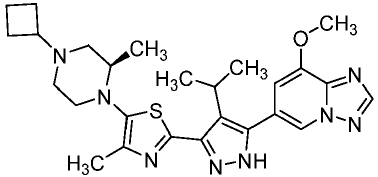 | 507.3 | 2.21 | C |
| 997 | 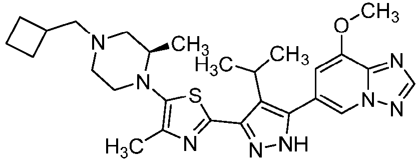 | 521.3 | 2.4 | C |
| 998 | 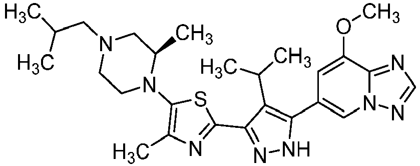 | 509.3 | 2.54 | C |
| 999 | 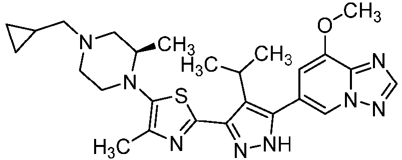 | 507.2 | 2.06 | C |
| 1000 | 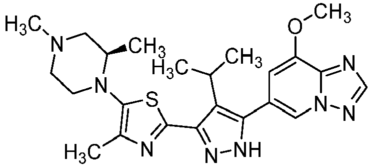 | 467.2 | 1.76 | C |
| 1001 | 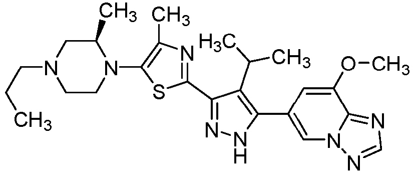 | 495.3 | 2.13 | C |
| 1002 | 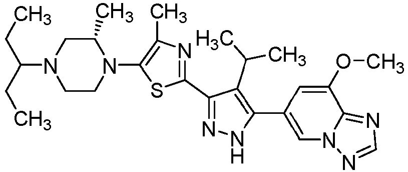 | 523.3 | 2.68 | C |
| 1003 | 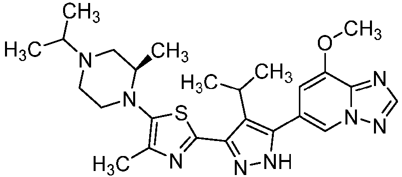 | 495.3 | 1.92 | C |
| 1004 | 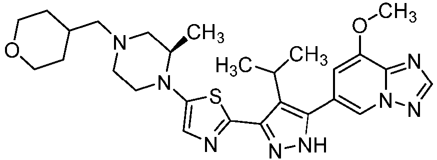 | 537.3 | 1.94 | C |
| 1005 | 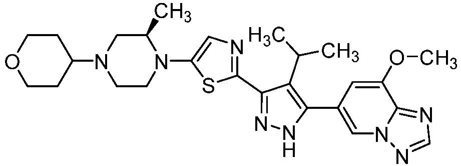 | 523.3 | 1.71 | C |
| 1006 | 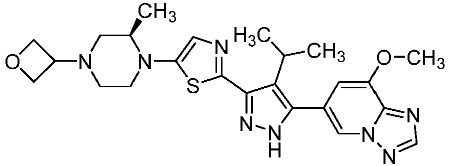 | 495.2 | 1.56 | C |
| 1007 | 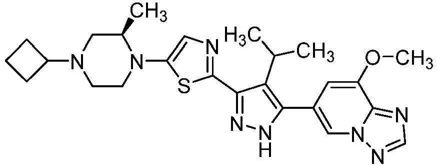 | 493.2 | 2.06 | C |
| 1008 | 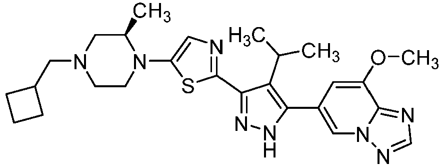 | 507.2 | 2.25 | C |
| 1009 | 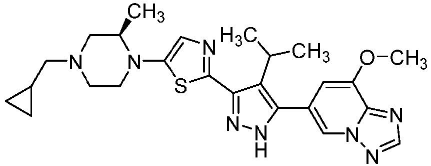 | 493.2 | 1.93 | C |
| 1010 | 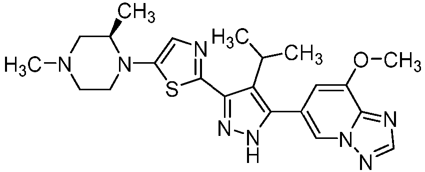 | 453.2 | 1.56 | C |
| 1011 | 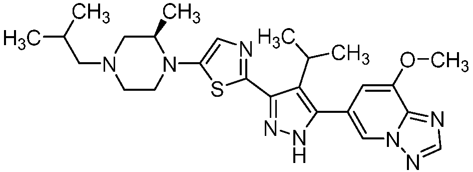 | 495.2 | 2.38 | C |
| 1012 | 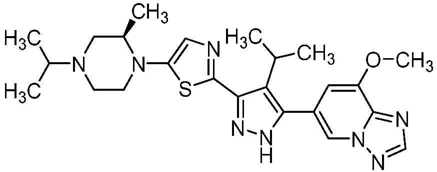 | 481.2 | 1.85 | C |
| 1013 | 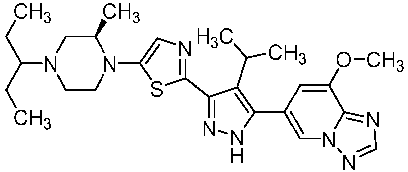 | 509.2 | 2.56 | C |
| 1014 | 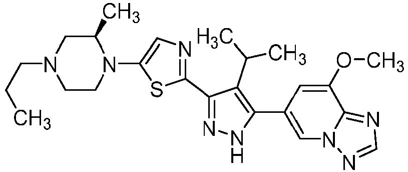 | 481.2 | 1.98 | C |

### EXAMPLE 1015

### 2-(dimethylamino)-1-(4-(2-(4-isopropyl-5-(8-methoxy-[1,2,4]triazolo[1,5-a]pyridin-6-yl)-1H-pyrazol-3-yl)thiazol-5-yl)piperazin-1-yl)ethan-1-one

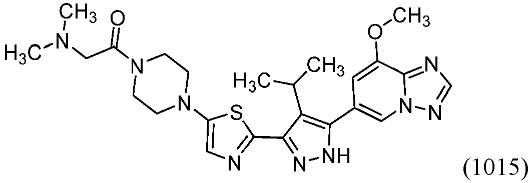

2-(Dimethylamino)-1-(4-(2-(4-isopropyl-5-(8-methoxy-[1,2,4]triazolo[1,5-a] pyridin-6-yl)-1H-pyrazol-3-yl)thiazol-5-yl)piperazin-1-yl)ethan-1-one (3.7 mg) was prepared according to the general process described in Example 323, using 2-(4-isopropyl-5-(8-methoxy-[1,2,4]triazolo[1,5-a]pyridin-6-yl)-1H-pyrazol-3-yl)-5-(piperazin-1-yl)thiazole (50.0 mg, 0.118 mmol) as a starting intermediate to get the title compound as an off-white solid. LCMS Retention time: 1.23 min [E], MS (E⁺) m/z: 510.2 [M+H]; ¹H NMR (400 MHz, METHANOL-d₄) δ 8.53 (s, 1H), 8.45 (s, 1H), 7.17 (br. s., 2H), 4.13 (s, 3H), 3.79 (br. s., 4H), 3.57-3.48 (m, 1H), 3.43 (s, 2H), 3.31-3.17 (m, 4H), 2.43 (s, 6H), 1.99-1.86 (m, 1H), 1.38-1.24 (m, 6H).

The following Examples were prepared according to the general procedure used to prepare Example 1015.

**TABLE 92**

| Ex No. | Structure | LCMS MH⁺ | RT (min) | HPLC Method |
|---|---|---|---|---|
| 1016 | 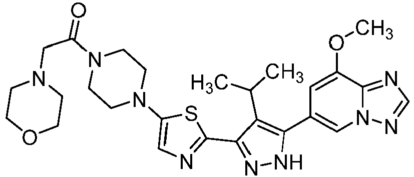 | 552.3 | 1.38 | C |
| 1017 | 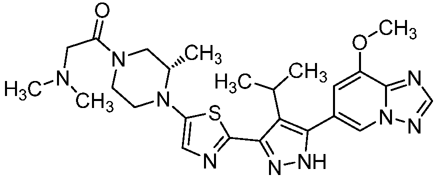 | 524.3 | 1.44 | C |
| 1018 | 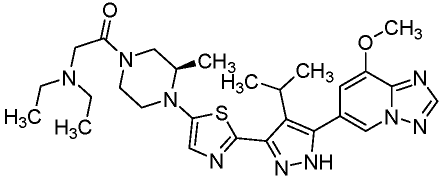 | 552.3 | 1.53 | C |
| 1018B | 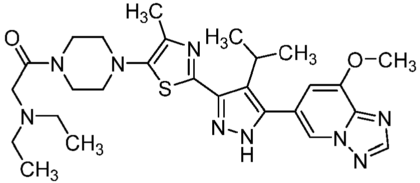 | 552.3 | 1.602 | E |
| 1019 | 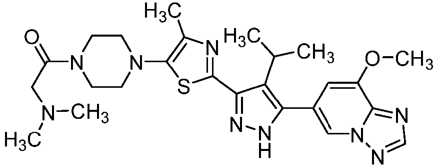 | 524.3 | 1.52 | E |
| 1020 | 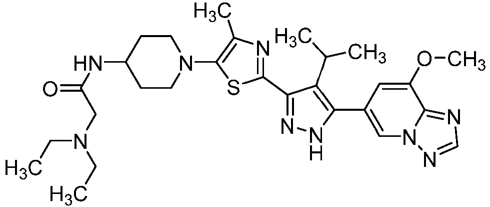 | 566.3 | 1.32 | C |
| 1021 | 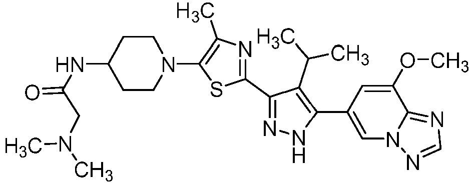 | 538.3 | 1.55 | C |
| 1022 | 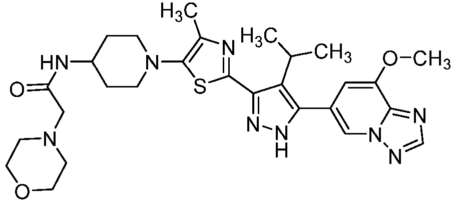 | 580.3 | 1.6 | C |
| 1023 | 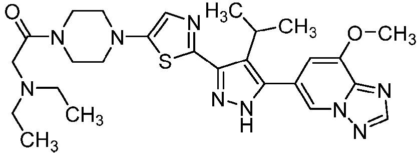 | 538.3 | 1.52 | C |
| 1024 | 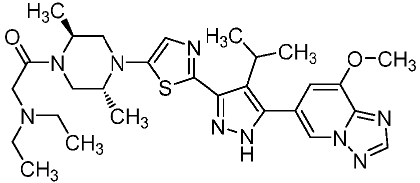 | 566.3 | 1.45 | C |
| 1025 | 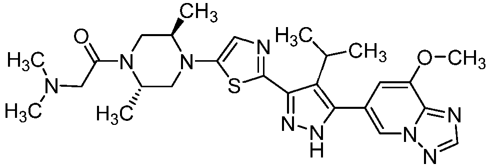 | 538.2 | 1.35 | C |
| 1026 | 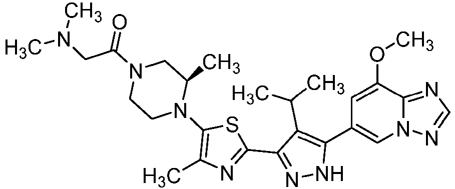 | 538.3 | 1.47 | C |
| 1027 | 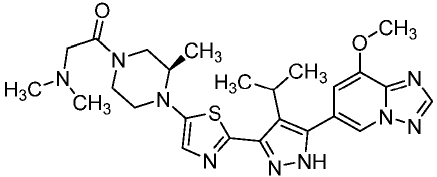 | 524.3 | 1.32 | C |

### EXAMPLE 1028

### 2-(4-isopropyl-5-(8-methoxy-[1,2,4]triazolo[1,5-a]pyridin-6-yl)-1H-pyrazol-3-yl)-5-(4-(2-methoxyethyl)piperazin-1-yl)thiazole

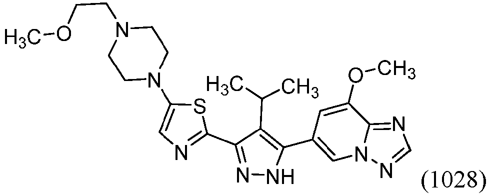

2-(4-Isopropyl-5-(8-methoxy-[1,2,4]triazolo[1,5-a]pyridin-6-yl)-1H-pyrazol-3-yl)-5-(4-(2-methoxyethyl)piperazin-1-yl)thiazole was prepared according to the general process described in Example 318, using 2-(4-isopropyl-5-(8-methoxy-[1,2,4]triazolo[1,5-a]pyridin-6-yl)-1H-pyrazol-3-yl)-5-(piperazin-1-yl)thiazole (50.0 mg, 0.118 mmol) as a starting intermediate to get the title compound as a white solid. LCMS Retention time: 1.52 min [E], MS (E⁺) m/z: 483.2 [M+H]; ¹H NMR (400 MHz, METHANOL-d₄) δ 8.54 (br. s., 1H), 8.45 (s, 1H), 7.16 (br. s., 1H), 7.08 (br.s., 1H), 4.21-4.02 (m, 3H), 3.61 (t, *J* =5.5 Hz, 2H), 3.56-3.47 (m, 1H), 3.43-3.36 (m, 2H),3.28 (br. s., 4H), 2.84-2.56 (m, 5H), 1.33 (d, *J* =7.1 Hz, 5H), 1.21 (d, *J* =16.9 Hz, 2H).

The following Examples were prepared according to the general procedure used to prepare Example 1028.

**TABLE 93**

| Ex. No. | Structure | LCMS MH⁺ | RT (min) | HPLC Method |
|---|---|---|---|---|
| 1029 | 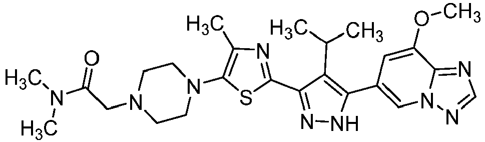 | 523.3 | 1.698 | E |
| 1030 | 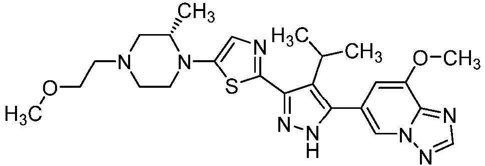 | 497.3 | 1.88 | C |
| 1031 | 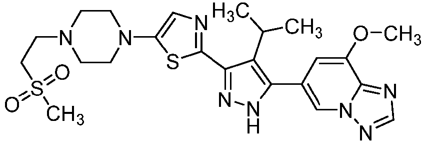 | 523.3 | 1.698 | E |
| 1032 | 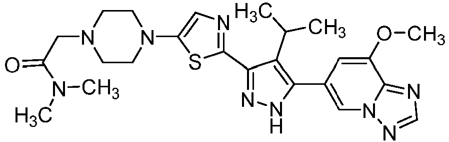 | 510.3 | 1.65 | C |
| 1033 | 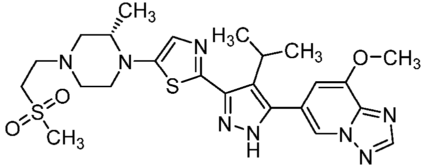 | 545.2 | 1.53 | C |
| 1034 | 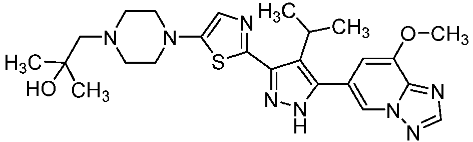 | 497.2 | 1.63 | C |
| 1035 | 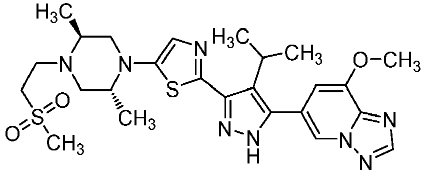 | 559.2 | 1.54 | C |
| 1036 | 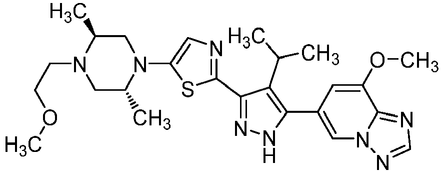 | 511.2 | 1.75 | C |
| 1037 | 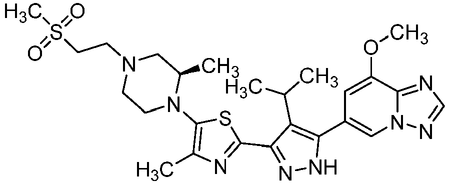 | 559.2 | 1.72 | C |
| 1038 | 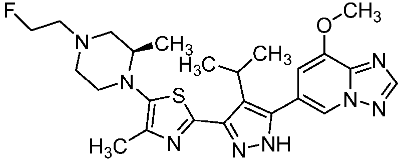 | 499.2 | 1.96 | C |
| 1039 | 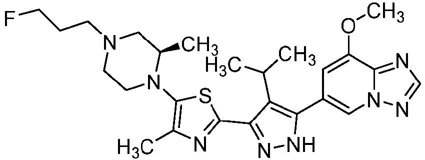 | 513.2 | 2.05 | C |
| 1040 | 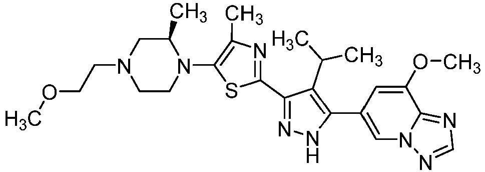 | 511.2 | 1.9 | C |
| 1041 | 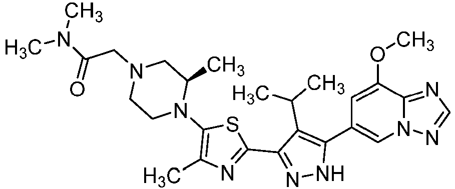 | 538.3 | 1.72 | C |
| 1042 | 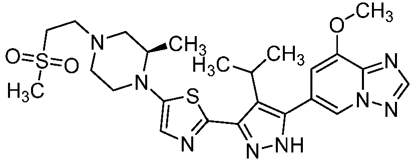 | 545.4 | 1.45 | C |
| 1043 | 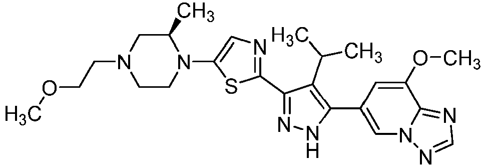 | 497.3 | 1.21 | D |
| 1044 | 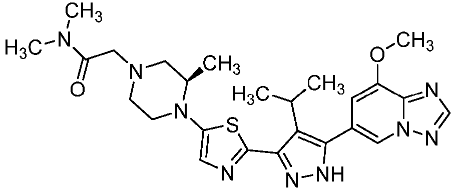 | 524.4 | 1.47 | C |
| 1045 | 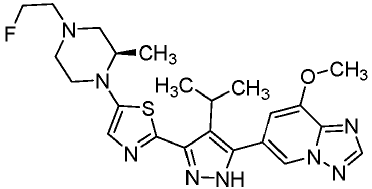 | 485.2 | 1.75 | C |
| 1046 | 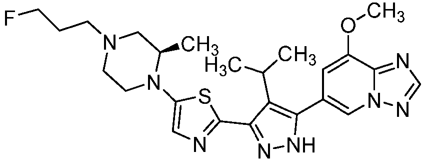 | 499.2 | 1.86 | C |
| 1047 | 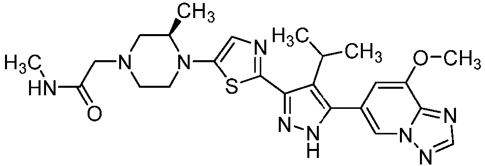 | 510.2 | 1.46 | C |
| 1048 | 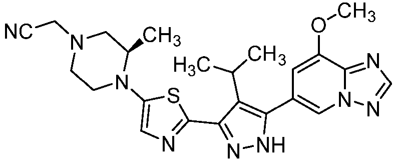 | 478.2 | 1.62 | C |

### EXAMPLE 1049

### 2-(4-isopropyl-5-(8-methoxy-[1,2,4]triazolo[1,5-a]pyridin-6-yl)-1H-pyrazol-3-yl)-4-methyl-5-(6-propyl-2,6-diazaspiro[3.3]heptan-2-yl)thiazole

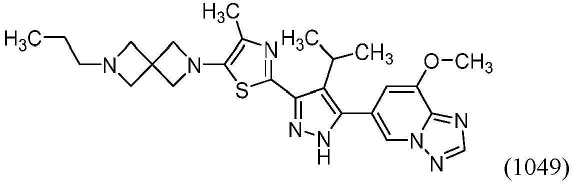

### Intermediate 1049A: 5-bromo-4-methylthiazole

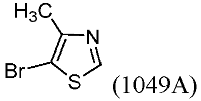

To a solution 4-methylthiazole (7.5 g, 76 mmol) in AcOH (25 mL) cooled to 0 °C, was added Br₂ (4.68 mL, 91 mmol). The reaction mixture was stirred at room temperature for 12 h. The reaction was quenched with saturated NH₄Cl (50 ml). The reaction mixture was extracted with EtOAc (100 ml), washed with water, brine, dried (Na₂SO₄) and concentrated to get crude compound. The crude compound was purified by ISCO using 80 g silica column, the compound was eluted in 14% EA in hexanes, the fractions were collected and concentrated to get 5-bromo-4-methylthiazole (7.5 g, 42.1 mmol, 56 % yield) as a yellow color liquid. LCMS retention time 1.854 min [A], MS (E⁻) *m*/*z:* 178.0 (M+2H).

### Intermediate 1049B: tert-butyl 6-(4-methylthiazol-5-yl)-2,6-diazaspiro[3.3]heptane-2-carboxylate

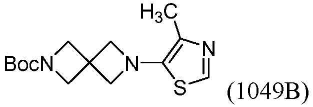

To a solution of tert-butyl 2,6-diazaspiro[3.3]heptane-2-carboxylate, oxalic acid salt (3.0 g, 10.41 mmol) and 5-bromo-4-methylthiazole (2.223 g, 12.49 mmol) in acetonitrile (100.00 mL) was added K₂CO₃ (7.19 g, 52.0 mmol). The reaction mixture was stirred at 130 °C for 3 days. The reaction mass was filtered through celite, washed with acetonitrile, the filtrate was collected and concentrated to get the crude compound. The crude compound was purified by ISCO using 40 g silica column, the compound was eluted in 40% EA in hexanes, the fractions were collected and concentrated to get tert-butyl 6-(4-methylthiazol-5-yl)-2,6-diazaspiro[3.3]heptane-2-carboxylate (2.97 g, 10.05 mmol, 97 % yield) as an off-white solid. LCMS retention time 1.49 min [A], MS (ES): m/z= 296.4 [M+H].

### Intermediate 1049C: tert-butyl 6-(2-bromo-4-methylthiazol-5-yl)-2,6-diazaspiro[3.3] heptane-2-carb oxyl ate

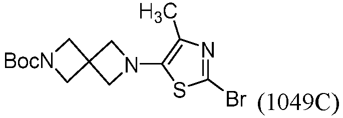

To a solution of tert-butyl 6-(4-methylthiazol-5-yl)-2,6-diazaspiro[3.3]heptane-2-carboxylate (2.230 g, 7.55 mmol) in THF (80.00 mL) was added NBS (1.478 g, 8.30 mmol) in THF (5 mL) at 0 °C. The reaction was continued for 20 minutes and quenched with water. The mixture was separated into two layers, the aqueous layer was extracted with EtOAc (2 X 20 mL), combined organic extracts were dried (Na₂SO₄) and concentrated to get crude compound. The crude compound was purified by ISCO using 24 g silica column, the compound was eluted in 30% EA in hexanes, the fractions were collected and concentrated to get tert-butyl 6-(2-bromo-4-methylthiazol-5-yl)-2,6-diazaspiro[3.3]heptane-2-carboxylate as a white solid. LCMS retention time 1.85 min [A], MS (ES): m/z= 375.9 [M+2H].

### Intermediate 1049D: tert-butyl 6-(2-(4-isopropyl-5-(8-methoxy-[1,2,4]triazolo[1,5-a] pyridin-6-yl)-1-((2-(trimethylsilyl)ethoxy) methyl)-1H-pyrazol-3-yl)-4-methylthiazol-5-yl)-2,6-diazaspiro[3.3]heptane-2-carboxylate

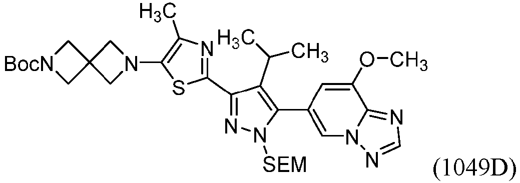

To a solution of 6-(4-isopropyl-3-(4,4,5,5-tetramethyl-1,3,2-dioxaborolan-2-yl)-1-((2-(trimethylsilyl)ethoxy)methyl)-1H-pyrazol-5-yl)-8-methoxy-[1,2,4]triazolo[1,5-a] pyridine (1.289 g, 2.509 mmol) and tert-butyl 6-(2-bromo-4-methylthiazol-5-yl)-2,6-diazaspiro[3.3]heptane-2-carboxylate (0.587 g, 1.568 mmol) in acetonitrile (32.00 mL) and water (8.00 mL) solvent mixture was added K₂CO₃ (0.650 g, 4.70 mmol). The reaction mixture was degassed with nitrogen for 5 min, then Pd₂(dba)₃ (0.072 g, 0.078 mmol) and 2-dicyclohexylphosphino-2',6'-dimethoxybiphenyl (0.064 g, 0.157 mmol) were added. The reaction mixture was degassed for 5 min. The reaction mixture was stirred in a sealed tube at 110 °C for 16 h. The reaction mass was brought to room temperature, separated both the layers, the aqueous layer was extracted with EtOAc (2 X 20 mL), the combined organic extracts were dried (Na₂SO₄) and concentrated to get crude compound. The crude compound was purified by ISCO using 40 g silica column, the compound was eluted in 50% EA in hexanes, the fraction was collected and concentrated to get tert-butyl 6-(2-(4-isopropyl-5-(8-methoxy-[1,2,4]triazolo[1,5-a]pyridin-6-yl)-1-((2-(trimethylsilyl)ethoxy)methyl)-1H-pyrazol-3-yl)-4-methylthiazol-5-yl)-2,6-diazaspiro[3.3]heptane-2-carboxylate (810 mg, 1.190 mmol, 76 % yield) as a white solid. LCMS retention time 1.69 min [B], MS (ES): m/z= 681.6 [M+H].

### Intermediate 1049E: 2-(4-isopropyl-5-(8-methoxy-[1,2,4]triazolo[1,5-a]pyridin-6-yl)-1H-pyrazol-3-yl)-4-methyl-5-(2,6-diazaspiro[3.3]heptan-2-yl)thiazole

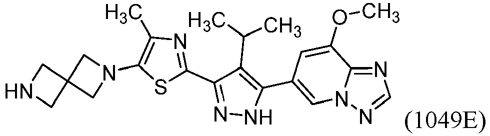

To a solution of tert-butyl 6-(2-(4-isopropyl-5-(8-methoxy-[1,2,4]triazolo[1,5-a] pyridin-6-yl)-1-((2-(trimethylsilyl)ethoxy)methyl)-1H-pyrazol-3-yl)-4-methylthiazol-5-yl)-2,6-diazaspiro [3.3]heptane-2-carboxylate (0.810 g, 1.190 mmol) in DCM (10.00 mL) was added TFA (5.00 mL, 64.9 mmol) at 0 °C. The reaction mixture was brought to room temperature and stirred at the same temperature for 16 h. The reaction mass was concentrated to get crude compound. The crude compound was triturated with diethyl ether (2 X 10 mL) to get 2-(4-isopropyl-5-(8-methoxy-[1,2,4]triazolo[1,5-a]pyridin-6-yl)-1H-pyrazol-3-yl)-4-methyl-5-(2,6-diazaspiro[3.3]heptan-2-yl)thiazole, TFA (540 mg, 0.958 mmol, 81% yield) as a white solid. LCMS retention time 1.140 min [E], MS (ES): m/z= 451.3 [M+H]⁺; ¹H NMR (400 MHz, METHANOL-d₄) δ ppm 8.50 (s, 1H), 8.43 (s, 1H), 7.15 (s, 1H), 4.33 (s, 3H), 4.11 (s, 3H), 4.13 (s, 3H), 3.53-3.38 (m, 1H), 2.31 (s, 3H), 1.46-1.21 (m, 6H).

### Example 1049: 2-(4-isopropyl-5-(8-methoxy-[1,2,4]triazolo[1,5-a]pyridin-6-yl)-1H-pyrazol-3-yl)-4-methyl-5-(6-propyl-2,6-diazaspiro[3.3]heptan-2-yl)thiazole

To a solution of 2-(4-isopropyl-5-(8-methoxy-[1,2,4]triazolo[1,5-a]pyridin-6-yl)-1H-pyrazol-3-yl)-4-methyl-5-(2,6-diazaspiro[3.3]heptan-2-yl)thiazole, TFA (27 mg, 0.048 mmol) in MeOH (1.5 mL) were added propionaldehyde (27.8 mg, 0.479 mmol), AcOH (0.1 mL, 1.747 mmol) and sodium cyanoborohydride (9.03 mg, 0.144 mmol) at room temperature. The reaction mixture was stirred at the same temperature for 16 h. The reaction mass was purified by Prep LCMS using method AA, fractions containing product were combined and dried using Genevac centrifugal evaporator to get 2-(4-isopropyl-5-(8-methoxy-[1,2,4]triazolo[1,5-a] pyridin-6-yl)-1H-pyrazol-3-yl)-4-methyl-5-(6-propyl-2,6-diazaspiro[3.3]heptan-2-yl)thiazole (1.5 mg, 3.04 µmol, 6 % yield) as a pale solid. LCMS retention time 1.682 min [E], MS (ES): m/z= 493.3 [M+H]; ¹H NMR (400 MHz, METHANOL-d₄) δ ppm 8.46-8.28 (m, 2H), 7.08-6.94 (m, 1H), 4.11-3.84 (m, 7H), 3.56-3.42 (m, 1H), 3.11-2.90 (m, 4H), 2.80-2.57 (m, 2H), 2.36-2.16 (m, 2H), 2.00-1.78 (m, 1H), 1.56-1.43 (m, 3H), 1.35-1.05 (m, 10H), 0.96-0.75 (m, 5H).

The following Examples were prepared according to the general procedure used to prepare Example 1049.

**TABLE 94**

| Ex. No. | Structure | LCMS MH⁺ | RT (min) | HPLC Method |
|---|---|---|---|---|
| 1050 | 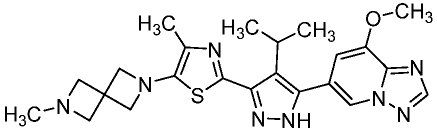 | 465.3 | 1.12 | C |
| 1051 | 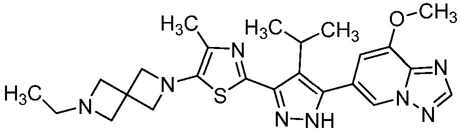 | 479.3 | 1.28 | C |
| 1052 | 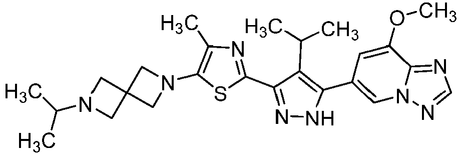 | 493.3 | 1.4 | C |
| 1053 | 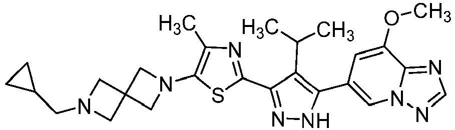 | 505.3 | 1.43 | C |
| 1054 | 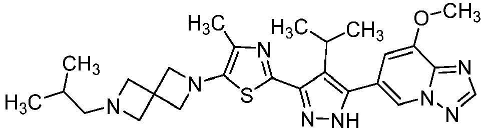 | 507.3 | 1.581 | E |
| 1055 | 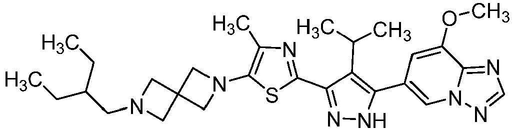 | 535.3 | 1.884 | E |
| 1056 | 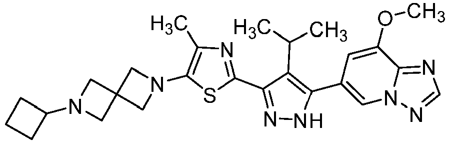 | 505.3 | 1.805 | E |
| 1057 | 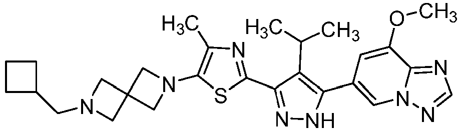 | 519.3 | 1.887 | E |
| 1058 | 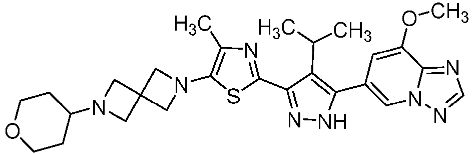 | 535.3 | 1.622 | E |
| 1059 | 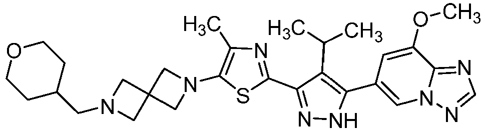 | 549.3 | 1.482 | E |

### EXAMPLE 1060

### 2-(dimethylamino)-1-(6-(2-(4-isopropyl-5-(8-methoxy-[1,2,4]triazolo[1,5-a]pyridin-6-yl)-1H-pyrazol-3-yl)-4-methylthiazol-5-yl)-2,6-diazaspiro[3.3 ]heptan-2-yl)ethan-1-one

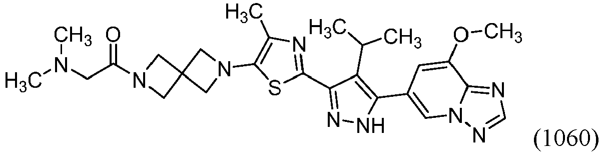

To a solution of 2-(4-isopropyl-5-(8-methoxy-[1,2,4]triazolo[1,5-a]pyridin-6-yl)-1H-pyrazol-3-yl)-4-methyl-5-(2,6-diazaspiro[3.3]heptan-2-yl)thiazole, TFA (20 mg, 0.035 mmol) in DMF (1.00 mL) were added TEA (0.1 mL, 0.717 mmol), dimethylglycine (5.49 mg, 0.053 mmol) and HATU (20.24 mg, 0.053 mmol) at room temperature. The reaction mixture was stirred at the same temperature for 16 h. The reaction mass was purified by Prep LCMS using method AB, fractions containing product were combined and dried using Genevac centrifugal evaporator to get 2-(dimethylamino)-1-(6-(2-(4-isopropyl-5-(8-methoxy-[1,2,4]triazolo [1,5-a]pyridin-6-yl)-1H-pyrazol-3-yl)-4-methylthiazol-5-yl)-2,6-diazaspiro[3.3]heptan-2-yl)ethan-1-one (2 mg, 3.73 µmol, 10 % yield) as a pale solid. LCMS retention time 1.682 min [F], MS (ES): *m*/*z=* 536.2 [M+H]⁺; ¹H NMR (400 MHz, METHANOL-d₄) δ = 8.60-8.36 (m, 3H), 7.23-7.06 (m, 1H), 4.18-4.05 (m, 8H), 4.04-3.77 (m, 8H), 3.71-3.45 (m, 3H), 3.04-2.83 (m, 11H), 2.51-2.22 (m, 1H), 1.96-1.82 (m, 1H), 1.52-1.12 (m, 12H).

### EXAMPLE 1061

### 2-(4-isopropyl-5-(8-methoxy-[1,2,4]triazolo[1,5-a]pyridin-6-yl)-1H-pyrazol-3-yl)-4-methyl-5-(6-(2-(methylsulfonyl)ethyl)-2,6-diazaspiro[3.3]heptan-2-yl)thiazole

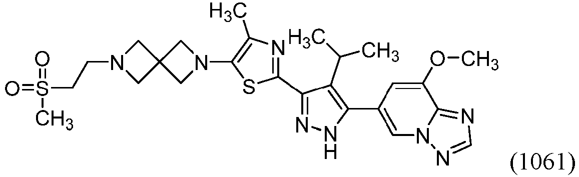

To a solution of 2-(4-isopropyl-5-(8-methoxy-[1,2,4]triazolo[1,5-a]pyridin-6-yl)-1H-pyrazol-3-yl)-4-methyl-5-(2,6-diazaspiro[3.3]heptan-2-yl)thiazole, TFA (25 mg, 0.044 mmol) in THF (1.00 mL) and DMF (0.500 mL) solvent mixture were added TEA (0.1 mL, 0.717 mmol) and 1-chloro-2-(methylsulfonyl)ethane (9.49 mg, 0.067 mmol) at room temperature. The reaction mixture was stirred at the same temperature for 16 h. The reaction mass was purified by prep LCMS using method AA, fractions containing product were combined and dried using Genevac centrifugal evaporator to get 2-(4-isopropyl-5-(8-methoxy-[1,2,4]triazolo[1,5-a]pyridin-6-yl)-1H-pyrazol-3-yl)-4-methyl-5-(6-(2-(methylsulfonyl)ethyl)-2,6-diazaspiro[3.3]heptan-2-yl)thiazole (5.2 mg, 8.78 µmol, 20 % yield) as a pale solid. LCMS retention time 1.682 min [E], MS (ES): *m*/*z= 557.2* [M+H].

The following Example was prepared according to the general procedure used to prepare Example 1061.

**TABLE 95**

| Ex. No. | Structure | LCMS MH⁺ | RT (min) | HPLC Method |
|---|---|---|---|---|
| 1062 | 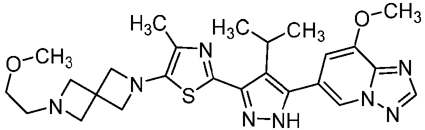 | 509.2 | 1.357 | E |

### EXAMPLE 1063

### 5-(4-isopropyl-5-(8-methoxy-[1,2,4]triazolo[1,5-a]pyridin-6-yl)-1H-pyrazol-3-yl)-2-(1-isopropylpiperidin-4-yl)thiazole

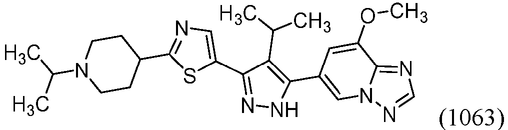

### Intermediate 1063A: 5-bromo-2-iodothiazole

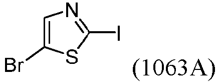

To a solution of 5-bromothiazol-2-amine hydrobromide (1.0 g, 3.85 mmol) in THF (5.0 mL) were added subsequently isoamyl nitrite (1.036 mL, 7.69 mmol) and diiodomethane (0.621 mL, 7.69 mmol) at room temperature. The reaction mixture was stirred for 12 h. The reaction mass was concentrated to get crude compound. The crude compound was purified by ISCO using 40 g silica column, the compound was eluted with 5-10% ethyl acetate in pet ether, the fractions were collected and concentrated to get 5-bromo-2-iodothiazole (500 mg, 1.725 mmol, 45% yield) as a light yellow liquid. LCMS Retention time: 2.45 min [B], MS (E⁺) *m*/*z:* 289.4 [M].

### Intermediate 1063B: Tert-butyl 4-(5-bromothiazol-2-yl)-3,6-dihydropyridine-1(2H)-carboxylate

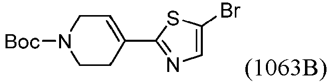

tert-Butyl 4-(5-bromothiazol-2-yl)-3,6-dihydropyridine-1(2H)-carboxylate (850 mg, 2.462 mmol, 76 % yield) was prepared according to the general process described in Intermediate 742C using 5-bromo-2-iodothiazole (1.406 g, 4.85 mmol) as a starting intermediate to get the title compound as pale brown solid. LCMS Retention time: 3.56 min [B], MS (E⁺) m/z: 347.2 [M+2H].

### Intermediate 1063C: tert-butyl 4-(5-(4-isopropyl-5-(8-methoxy-[1,2,4]triazolo[1,5-a] pyridin-6-yl)-1-((2-(trimethylsilyl)ethoxy)methyl)-1H-pyrazol-3-yl)thiazol-2-yl)-3,6-dihydropyridine-1(2H)-carboxylate

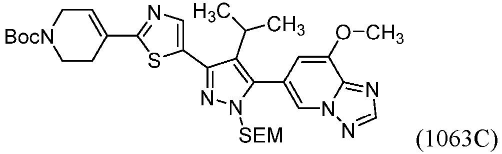

tert-Butyl 4-(5-(4-isopropyl-5-(8-methoxy-[1,2,4]triazolo[1,5-a]pyridin-6-yl)-1-((2-(trimethylsilyl)ethoxy)methyl)-1H-pyrazol-3-yl)thiazol-2-yl)-3,6-dihydropyridine-1(2H)-carboxylate (500 mg, 0.767 mmol, 41% yield) was prepared according to the general process described in Intermediate 307B, using tert-butyl 4-(5-bromothiazol-2-yl)-3,6-dihydropyridine-1(2H)-carboxylate (0.650 g, 1.883 mmol) and 6-(4-isopropyl-3-(4,4,5,5-tetramethyl-1,3,2-dioxaborolan-2-yl)-1-((2-(trimethylsilyl) ethoxy)methyl)-1H-pyrazol-5-yl)-8-methoxy-[1,2,4]triazolo[1,5-a]pyridine (1.257 g, 2.447 mmol) as starting intermediates to get the title compound as a pale brown solid. LCMS Retention time: 2.01 min [A], MS (E⁺) m/z: 652.4 [M+H].

### Intermediate 1063D: tert-butyl 4-(5-(4-isopropyl-5-(8-methoxy-[1,2,4]triazolo[1,5-a] pyridin-6-yl)-1-((2-(trimethylsilyl)ethoxy)methyl)-1H-pyrazol-3-yl)thiazol-2-yl) piperidine-1-carboxylate

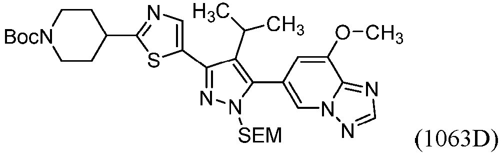

tert-Butyl 4-(5-(4-isopropyl-5-(8-methoxy-[1,2,4]triazolo[ 1,5-a]pyridin-6-yl)-1-((2-(trimethylsilyl)ethoxy)methyl)-1H-pyrazol-3-yl)thiazol-2-yl)piperidine-1-carboxylate (550 mg, 0.841 mmol, 59% yield) was prepared according to the general process described in Intermediate 307C, using of tert-butyl 4-(5-(4-isopropyl-5-(8-methoxy-[1,2,4]triazolo[1,5-a] pyridin-6-yl)-1-((2-(trimethylsilyl)ethoxy)methyl)-1H-pyrazol-3-yl)thiazol-2-yl)-3,6-dihydropyridine-1(2H)-carboxylate (1.1 g, 1.687 mmol) as a starting intermediate to get the title compound as an off-white solid. LCMS Retention time: 1.79 min [A], MS (E⁺) m/z: 654.5 [M+H].

### Example 1063E: 5-(4-isopropyl-5-(8-methoxy-[1,2,4]triazolo[1,5-a]pyridin-6-yl)-1H-pyrazol-3-yl)-2-(piperidin-4-yl)thiazole

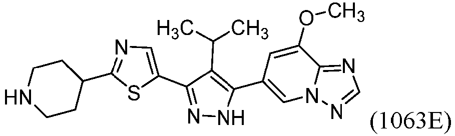

5-(4-Isopropyl-5-(8-methoxy-[1,2,4]triazolo[1,5-a]pyridin-6-yl)-1H-pyrazol-3-yl)-2-(piperidin-4-yl)thiazole (15.4 mg) was prepared according to the general process described in Intermediate 307D, using tert-butyl 4-(5-(4-isopropyl-5-(8-methoxy-[1,2,4]triazolo[1,5-a] pyridin-6-yl)-1-((2-(trimethylsilyl)ethoxy)methyl)-1H-pyrazol-3-yl)thiazol-2-yl)piperidine-1-carboxylate (250 mg, 0.382 mmol) as a starting intermediate to get the title compound as an off-white solid. LCMS Retention time: 0.97 min [E], MS (E⁺) m/z: 424.2 [M+H]; ¹H NMR (400 MHz, METHANOL-d₄) δ 8.56 (s, 1H), 8.46 (s, 1H), 7.89 (s, 1H), 7.20 (s, 1H),4.13 (s, 3H), 3.56-3.43 (m, 3H), 3.27-3.08 (m, 3H), 2.38 (d, *J* =13.2 Hz, 2H), 2.14-1.99 (m, 2H), 1.94 (s, 1H), 1.39-1.18 (m, 6H).

### Example 1063: 5-(4-isopropyl-5-(8-methoxy-[1,2,4]triazolo[1,5-a]pyridin-6-yl)-1H-pyrazol-3-yl)-2-(1-isopropylpiperidin-4-yl)thiazole

5-(4-Isopropyl-5-(8-methoxy-[1,2,4]triazolo[1,5-a]pyridin-6-yl)-1H-pyrazol-3-yl)-2-(1-isopropylpiperidin-4-yl)thiazole (16.3 mg) was prepared according to the general process described in Example 307 using 5-(4-isopropyl-5-(8-methoxy-[1,2,4]triazolo[1,5-a]pyridin-6-yl)-1H-pyrazol-3-yl)-2-(piperidin-4-yl)thiazole (35.0 mg, 0.083 mmol) as a starting intermediate to get the title compound as a white solid. LCMS Retention time: 1.37 min [E], MS (E⁺) m/z: 466.3 [M+H]; ¹H NMR (400 MHz, METHANOL-d₄) δ 8.56 (br. s., 1H), 8.46 (s, 1H), 7.88 (s, 1H), 7.20 (br. s.,1H), 4.16-4.10 (m, 5H), 3.29-3.19 (m, 2H), 3.02 (s, 1H), 2.95 (br. s., 1H), 2.88 (s, 1H), 2.39(d, *J* =13.7 Hz, 4H), 2.17-2.02 (m, 4H), 1.95 (s, 2H), 1.39 (d, *J=7.1* Hz, 2H), 1.37-1.20 (m,19H), 1.16 (d, *J* =6.6 Hz, 1H).

The following Example was prepared according to the general procedure used to prepare Example 1063.

**TABLE 96**

| Ex. No. | Structure | LCMS MH⁺ | RT (min) | HPLC Method |
|---|---|---|---|---|
| 1064 | 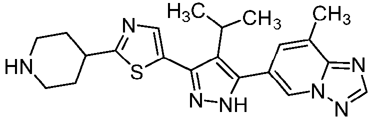 | 408.2 | 1.00 | C |

The following Examples were prepared according to the general procedure used to prepare Example 1063.

**TABLE 97**

| Ex. No. | Structure | LCMS MH⁺ | RT (min) | HPLC Method |
|---|---|---|---|---|
| 1065 | 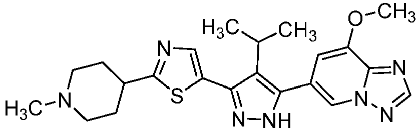 | 438.3 | 1.09 | C |
| 1066 | 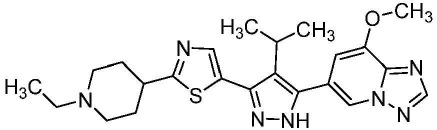 | 452.3 | 1.26 | C |
| 1067 | 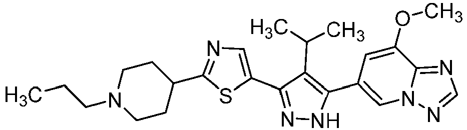 | 466.3 | 1.26 | C |
| 1068 | 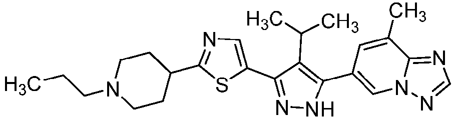 | 450.3 | 1.1 | C |
| 1069 | 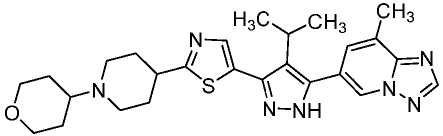 | 492.3 | 1.28 | C |
| 1070 | 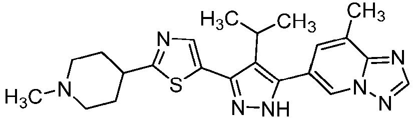 | 422.2 | 1.12 | C |
| 1071 | 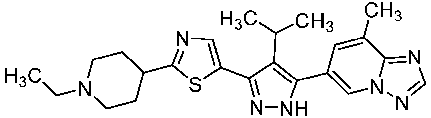 | 436.2 | 1.68 | C |
| 1072 | 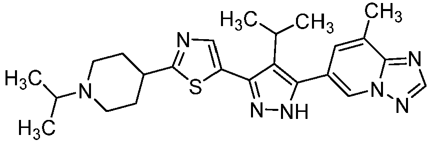 | 450.3 | 1.19 | C |
| 1073 | 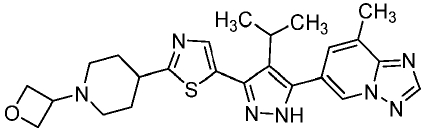 | 464.3 | 1.36 | C |
| 1074 | 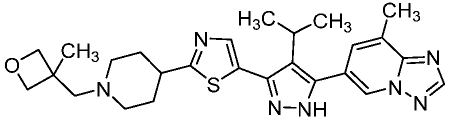 | 492.2 | 1.48 | C |
| 1075 | 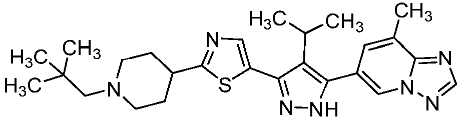 | 478.3 | 2.06 | C |

### EXAMPLE 1076

### 2-(4-(5-(4-isopropyl-5-(8-methyl-[1,2,4]triazolo[1,5-a]pyridin-6-yl)-1H-pyrazol-3-yl) thiazol-2-yl)piperidin-1-yl)-N,N-dimethylacetamide

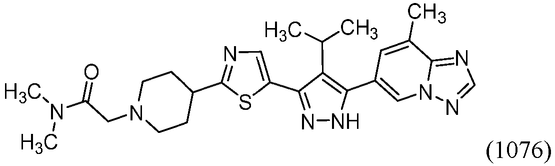

2-(4-(5-(4-Isopropyl-5-(8-methyl-[1,2,4]triazolo[1,5-a]pyridin-6-yl)-1H-pyrazol-3-yl)thiazol-2-yl)piperidin-1-yl)-N,N-dimethylacetamide (3.1 mg) was prepared according to the general process described in Example 469, using 5-(4-isopropyl-5-(8-methyl-[1,2,4]triazolo[1,5-a]pyridin-6-yl)-1H-pyrazol-3-yl)-2-(piperidin-4-yl)thiazole (40.0 mg, 0.098 mmol) as a starting intermediate to get the title compound as an off-white solid. LCMS Retention time: 1.41 min [E], MS (E⁺) m/z: 493.2 [M+H]; ¹H NMR (400 MHz, METHANOL-d₄) δ 8.83 (br. s., 1H), 8.75 (br. s., 1H), 8.50 (d, *J* =12.7 Hz,1H), 7.85 (br. s., 1H), 7.67 (br. s., 1H), 7.61 (br. s., 1H), 4.62 (s, 1H), 3.70 (d, *J* =7.3 Hz, 1H),3.47 (br. s., 1H), 3.42 (br. s., 1H), 3.26-3.10 (m, 6H), 2.98 (s, 3H), 2.71 (s, 3H), 2.45 (br. s.,3H), 2.22 (d, *J* =12.5 Hz, 2H), 2.09-1.91 (m, 2H), 1.39-1.11 (m, 8H), 0.92 (br. s., 1H).

The following Examples were prepared according to the general procedure used to prepare Intermediate 1076.

**TABLE 98**

| Ex. No. | Structure | LCMS MH⁺ | RT (min) | HPLC Method |
|---|---|---|---|---|
| 1077 | 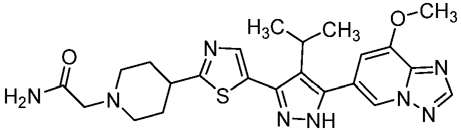 | 481.3 | 1.42 | C |
| 1078 | 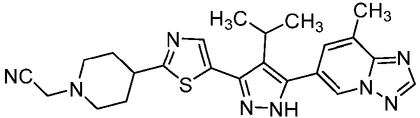 | 447.3 | 1.46 | C |
| 1079 | 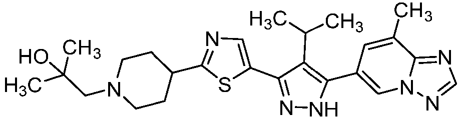 | 480.3 | 1.06 | C |
| 1080 | 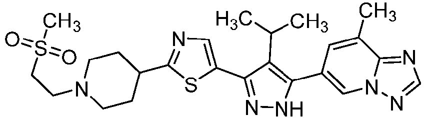 | 514.3 | 1.36 | C |
| 1081 | 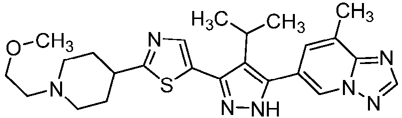 | 466.3 | 1.29 | C |

### EXAMPLE 1082

### 2-(dimethylamino)-1-(4-(5-(4-isopropyl-5-(8-methyl-[1,2,4]triazolo[1,5-a]pyridin-6-yl)-1H-pyrazol-3-yl)thiazol-2-yl)piperidin-1-yl)ethan-1-one

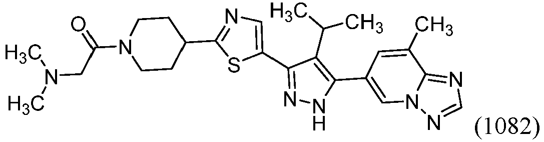

2-(Dimethylamino)-1-(4-(5-(4-isopropyl-5-(8-methyl-[1,2,4]triazolo[1,5-a] pyridin-6-yl)-1H-pyrazol-3-yl)thiazol-2-yl)piperidin-1-yl)ethan-1-one (28.5 mg) was prepared according to the general process described in Example 466, using 5-(4-isopropyl-5-(8-methyl-[1,2,4]triazolo[1,5-a]pyridin-6-yl)-1H-pyrazol-3-yl)-2-(piperidin-4-yl)thiazole (40.0 mg, 0.098 mmol) as a starting intermediate to get the title compound as a pale solid. LCMS Retention time: 1.17 min [E], MS (E⁺) m/z: 493.3 [M+H]; ¹H NMR (400 MHz, METHANOL-d₄) δ 8.79 (br. s., 1H), 8.50 (s, 1H), 7.87 (s, 1H), 7.63 (br. s.,1H), 4.63 (d, *J*=11.5 Hz, 2H), 4.08 (d, *J =13.0* Hz, 1H), 3.74-3.64 (m, 1H), 3.62 (br. s., 1H),3.42 (d, *J* =12.0 Hz, 1H), 3.26-3.13 (m, 1H), 2.94 (t, *J*=11.9 Hz, 1H), 2.71 (s, 3H), 2.57 (s, 6H),2.25 (d, *J* =13.2 Hz, 2H), 1.96 (s, 2H), 1.93-1.71 (m, 2H), 1.39-1.15 (m, 6H).

### EXAMPLE 1083

### 4-(5-(4-isopropyl-5-(8-methyl-[1,2,4]triazolo[1,5-a]pyridin-6-yl)-1H-pyrazol-3-yl) thiazol-2-yl)-N-methylcyclohexan-1-amine

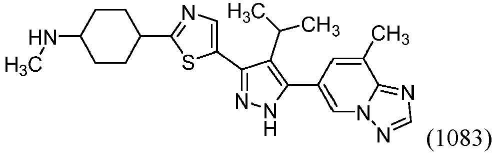

### Intermediate 1083A: 5-bromo-2-(1,4-dioxaspiro[4.5]dec-7-en-8-yl)thiazole

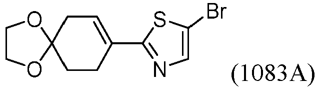

5-Bromo-2-(1,4-dioxaspiro[4.5]dec-7-en-8-yl)thiazole (1.3 g, 4.30 mmol, 64% yield) was prepared according to the general process described in Intermediate 307A using 5-bromo-2-iodothiazole (2.0 g, 6.90 mmol) as a starting intermediate to get the title compound as a pale yellow solid. LCMS Retention time: 1.31 min [A], MS (E⁺) *m*/*z:* 304.0 [M+2H].

### Intermediate 1083B: 5-(4-isopropyl-5-(8-methyl-[1,2,4]triazolo[1,5-a]pyridin-6-yl)-1-((2-(trimethylsilyl)ethoxy)methyl)-1H-pyrazol-3-yl)-2-(1,4-dioxaspiro[4.5]dec-7-en-8-yl) thiazole

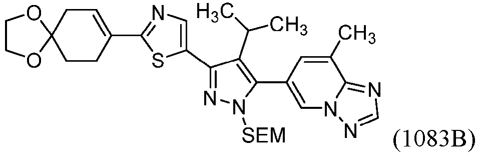

5-(4-Isopropyl-5-(8-methyl-[1,2,4]triazolo[1,5-a]pyridin-6-yl)-1-((2-(trimethylsilyl)ethoxy)methyl)-1H-pyrazol-3-yl)-2-(1,4-dioxaspiro[4.5]dec-7-en-8-yl) thiazole (900 mg, 1.518 mmol, 70% yield) was prepared according to the general process described in Intermediate 307B using 5-bromo-2-(1,4-dioxaspiro[4.5]dec-7-en-8-yl) thiazole (0.650 g, 2.151 mmol) and 6-(4-isopropyl-3-(4,4,5,5-tetramethyl-1,3,2-dioxaborolan-2-yl)-1-((2-(trimethylsilyl)ethoxy)methyl)-1H-pyrazol-5-yl)-8-methyl-[1,2,4]triazolo[1,5-a]pyridine (1.391 g, 2.80 mmol) as starting intermediates to afford the title compound as a gummy solid. LCMS Retention time: 1.69 min [A], MS (E⁺) m/z: 593.6 [M+H].

### Intermediate 1083C: 5-(4-isopropyl-5-(8-methyl-[1,2,4]triazolo[1,5-a]pyridin-6-yl)-1-((2-(trimethylsilyl)ethoxy)methyl)-1H-pyrazol-3-yl)-2-(1,4-dioxaspiro[4.5]decan-8-yl) thiazole

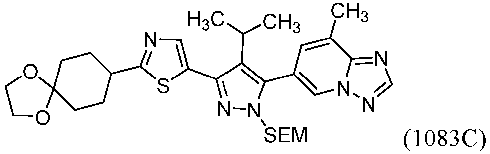

5-(4-Isopropyl-5-(8-methyl-[1,2,4]triazolo[1,5-a]pyridin-6-yl)-1-((2-(trimethylsilyl)ethoxy)methyl)-1H-pyrazol-3-yl)-2-(1,4-dioxaspiro[4.5]decan-8-yl) thiazole (550 mg, 0.925 mmol, 61 % yield) was prepared according to the general process described in Intermediate 307C, using 5-(4-isopropyl-5-(8-methyl-[1,2,4]triazolo[1,5-a] pyridin-6-yl)-1-((2-(trimethylsilyl)ethoxy)methyl)-1H-pyrazol-3-yl)-2-(1,4-dioxaspiro[4.5]dec-7-en-8-yl) thiazole (900 mg, 1.518 mmol) as a starting intermediate to get the title compound as an off-white solid. LCMS Retention time: 1.73 min [A], MS (E⁺) m/z: 595.7 [M+H].

### Intermediate 1083D: 4-(5-(4-isopropyl-5-(8-methyl-[1,2,4]triazolo[1,5-a]pyridin-6-yl)-1H-pyrazol-3-yl)thiazol-2-yl)cyclohexan-1-one

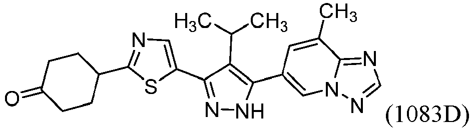

4-(4-Isopropyl-5-(8-methyl-[1,2,4]triazolo[1,5-a]pyridin-6-yl)-1H-pyrazol-3-yl) cyclohexan-1-one (600 mg, 1.778 mmol, 76 % yield) was prepared according to the general process described in Intermediate 307D using 6-(4-isopropyl-3-(1,4-dioxaspiro[4.5]decan-8-yl)-1-((2-(trimethylsilyl)ethoxy)methyl)-1H-pyrazol-5-yl)-8-methyl-[1,2,4]triazolo[1,5-a] pyridine (1.2 g, 2.345 mmol) as a starting intermediate to get the title compound as a dark brown semi-solid. LCMS Retention time: 0.96 min [A], MS (E⁺) m/z: 338.4 [M+H].

### Example 1083: 4-(5-(4-isopropyl-5-(8-methyl-[1,2,4]triazolo[1,5-a]pyridin-6-yl)-1H-pyrazol-3-yl)thiazol-2-yl)-N-methylcyclohexan-1-amine

A solution of 4-(5-(4-isopropyl-5-(8-methyl-[1,2,4]triazolo[ 1,5-a]pyridin-6-yl)-1H-pyrazol-3-yl)thiazol-2-yl)cyclohexan-1-one (300 mg, 0.713 mmol), methylamine hydrochloride (482 mg, 7.13 mmol) and acetic acid (8.17 µL, 0.143 mmol) in DMF (15.0 mL) was stirred at room temperature for 8 h. Next, sodium cyanoborohydride (90 mg, 1.427 mmol) was added at 0 °C and the reaction mixture was stirred at room temperature for 16 h. The reaction mixture was diluted with DCM, washed with water, brine, dried over sodium sulphate and concentrated to get crude compound. The crude was purified by prep HPLC using method AA to separate both the isomers, the fractions were collected, concentrated and lyophilized to yield two isomers.
Example 1083A: Isomer 1: (70 mg), LCMS Retention time: 1.09 min [E], MS (E⁺) m/z: 436.3 [M+H]; ¹H NMR (400 MHz, METHANOL-d₄) δ 8.79 (s, 1H), 8.50 (s, 1H), 7.86 (s, 1H), 7.63 (s, 1H),3.24-3.03 (m, 3H), 2.71 (s, 6H), 2.37 (d, *J* =13.2 Hz, 2H), 2.30 (d, *J* =12.7 Hz, 2H), 1.93 (s, 2H),1.85-1.68 (m, 2H), 1.66-1.46 (m, 2H), 1.25 (d, *J* =7.1 Hz, 6H); and
Example 1083B: Isomer 2: (58 mg) as a white solid. LCMS Retention time: 1.17 min [E], MS (E⁺) m/z: 436.3 [M+H]; ¹H NMR (400 MHz, METHANOL-d₄) δ 8.79 (s, 1H), 8.51 (s, 1H), 7.87 (s, 1H), 7.63 (s, 1H),3.81 (s, 1H), 3.43 (d, *J* =5.6 Hz, 1H), 3.20 (t, *J* =7.3 Hz, 2H), 2.70 (d, *J* =8.6 Hz, 6H), 2.29 (br. s.,2H), 2.15-1.96 (m, 4H), 1.96-1.78 (m, 5H), 1.26 (d, *J* =7.1 Hz, 6H).

### EXAMPLE 1084

### 4-(5-(4-isopropyl-5-(8-methyl-[1,2,4]triazolo[1,5-a]pyridin-6-yl)-1H-pyrazol-3-yl) thiazol-2-yl)-N,N-dimethylcyclohexan-1-amine

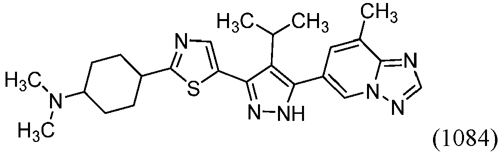

4-(5-(4-isopropyl-5-(8-methyl-[1,2,4]triazolo[1,5-a]pyridin-6-yl)-1H-pyrazol-3-yl)thiazol-2-yl)-N,N-dimethylcyclohexan-1-amine (10 mg) was prepared according to the general process described in Example 307 using 4-(5-(4-isopropyl-5-(8-methyl-[1,2,4]triazolo[1,5-a]pyridin-6-yl)-1H-pyrazol-3-yl) thiazol-2-yl)-N-methylcyclohexan-1-amine (20.0 mg, 0.046 mmol) as a starting intermediate to get the title compound as a white solid. LCMS Retention time: 1.14 min [E], MS (E⁺) m/z: 450.2 [M+H]; ¹H NMR (400 MHz, METHANOL-d₄) δ 8.79 (br. s., 1H), 8.50 (s, 1H), 7.85 (s, 1H), 7.62 (br. s.,1H), 3.81 (s, 1H), 3.26-3.07 (m, 3H), 2.77 (s, 6H), 2.71 (s, 3H), 2.40 (d, *J* =13.0 Hz, 2H), 2.23(d, *J* =11.7 Hz, 2H), 1.95 (s, 3H), 1.85-1.59 (m, 4H), 1.25 (d, *J* =7.1 Hz, 6H).

The following Examples were prepared according to the general procedure used to prepare Example 1084.

**TABLE 99**

| Ex. No. | Structure | LCMS MH⁺ | RT (min) | HPLC Method |
|---|---|---|---|---|
| 1085 | 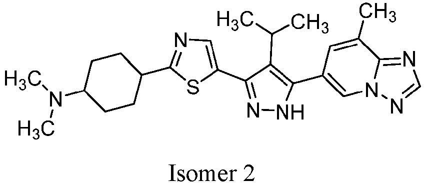 | 450.3 | 1.21 | C |
| 1086 | 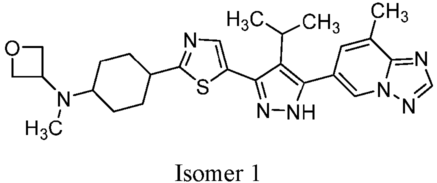 | 492.3 | 1.44 | C |
| 1087 | 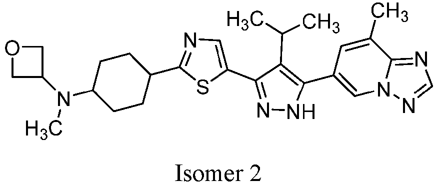 | 492.3 | 1.49 | C |
| 1088 | 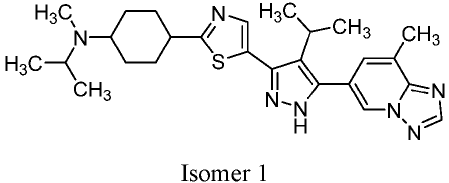 | 478.3 | 1.45 | C |
| 1089 | 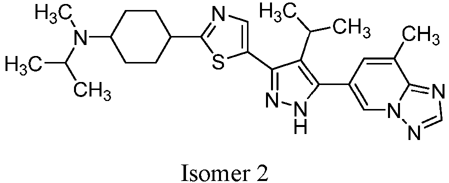 | 478.3 | 1.46 | C |

### EXAMPLE 1090

### 6-(4-isopropyl-1'-(1-methylpiperidin-4-yl)-1H,1'H-[3,4'-bipyrazol]-5-yl)-8-methyl-[1,2,4]triazolo[1,5-a]pyridine

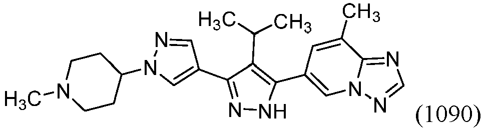

### Intermediate 1090A: tert-butyl 4-(tosyloxy)piperidine-1-carboxylate

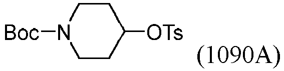

To a stirred solution of tert-butyl 4-hydroxypiperidine-1-carboxylate (7 g, 34.8 mmol) in DCM (60 mL) were added Et₃N (7.27 mL, 52.2 mmol), DMAP (0.127 g, 1.043 mmol) and tosyl-Cl (7.96 g, 41.7 mmol) at 0 °C. The reaction mixture was stirred at room temperature 16 h. The reaction mixture was diluted with excess DCM, washed with water, brine, dried (Na₂SO₄) and concentrated to get tert-butyl 4-(tosyloxy)piperidine-1-carboxylate (5 g, 14.07 mmol, 40% yield) as an off-white solid.

### Intermediate 1090B: tert-butyl 4-(4-bromo-1H-pyrazol-1-yl)piperidine-1-carboxylate

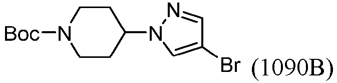

To a stirred solution of 4-bromo-1H-pyrazole (500 mg, 3.40 mmol) in DMF (10 mL) were added cesium carbonate (2217 mg, 6.80 mmol) and tert-butyl 4-(tosyloxy)piperidine-1-carboxylate (1451 mg, 4.08 mmol) at room temperature. The reaction mixture was stirred at 120 °C for 3 h. The reaction mixture was diluted with EtOAc, washed with water, brine, dried over sodium sulphate and concentrated to get crude material. The crude material was purified by ISCO using silica column 40 g, the compound was eluted with 15%-20% EtOAc in pet ether, the fractions were collected and concentrated to get tert-butyl 4-(4-bromo-1H-pyrazol-1-yl)piperidine-1-carboxylate (750 mg, 2.271 mmol, 67 % yield) as a gummy solid. LCMS Retention time: 1.24 min [A], MS (E⁺) m/z: 332.4 [M+2H].

### Intermediate 1090C: tert-butyl 4-(2-(5-(8-methoxy-[1,2,4]triazolo[1,5-a]pyridin-6-yl)-4-(2,2,2-trifluoroethyl)-1-((2-(trimethylsilyl)ethoxy)methyl)-1H-pyrazol-3-yl)-4-methyl thiazol-5-yl)piperidine-1-carboxylate

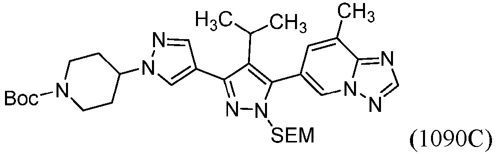

tert-butyl 4-(4-isopropyl-5-(8-methyl-[1,2,4]triazolo[1,5-a]pyridin-6-yl)-1-((2-(trimethylsilyl) ethoxy)methyl)-1H,1'H-[3,4'-bipyrazol]-1'-yl)piperidine-1-carboxylate (350 mg, 0.564 mmol, 37% yield) was prepared according to the general process described in Intermediate 307B, using 6-(4-isopropyl-3-(4,4,5,5-tetramethyl-1,3,2-dioxaborolan-2-yl)-1-((2-(trimethylsilyl)ethoxy)methyl)-1H-pyrazol-5-yl)-8-methyl-[1,2,4]triazolo[1,5-a]pyridine (1507 mg, 3.03 mmol) as a starting intermediate to get the title compound as a brown liquid. LCMS Retention time: 1.76 [A], MS (E⁻) m/z: 621.4 [M+H].

### Intermediate 1090D: 6-(4-isopropyl-1'-(piperidin-4-yl)-1H,1'H-[3,4'-bipyrazol]-5-yl)-8-methyl-[1,2,4]triazolo[1,5-a]pyridine

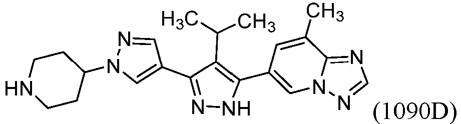

To a stirred solution of tert-butyl 4-(4-isopropyl-5-(8-methyl-[1,2,4]triazolo[1,5-a] pyridin-6-yl)-1-((2-(trimethylsilyl)ethoxy)methyl)-1H,1'H-[3,4'-bipyrazol]-1'-yl) piperidine-1-carboxylate (300 mg, 0.483 mmol) in DCM (25 mL) was added TFA (1 mL) at room temperature. The reaction mixture was stirred at room temperature for 2 h. The reaction mass was concentrated to get crude compound. The crude compound was purified by Prep LCMS using method AA, the fractions containing the product were combined and dried via centrifugal evaporation to get 6-(4-isopropyl-1'-(piperidin-4-yl)-1H,1'H-[3,4'-bipyrazol]-5-yl)-8-methyl-[1,2,4]triazolo[1,5-a]pyridine (20 mg, 0.126 mmol, 10% yield) as an off-white solid. LCMS Retention time: 0.87 [E], MS (E⁻) m/z: 391.3 [M+H]; ¹H NMR (400 MHz, DMSO-d₆) δ 12.99 (s, 1H), 8.75 (br. s., 2H), 8.51 (s, 2H), 7.75 (s, 1H), 7.56(s, 1H), 7.25 (br. s., 2H), 7.12 (br. s., 2H), 7.00 (br. s., 2H), 4.59 (br. s., 1H), 3.14-3.01 (m, 3H), 2.61 (s, 3H), 2.28-2.20 (m, 2H), 2.16 (d, J=12.7 Hz, 2H), 1.20-1.10 (m, 6H).

### Example 1090: 2-(5-(8-methoxy-[1,2,4]triazolo[1,5-a]pyridin-6-yl)-4-(2,2,2-trifluoroethyl)-1H-pyrazol-3-yl)-4-methyl-5-((1S,4S)-5-methyl-2,5-diazabicyclo[2.2.1] heptan-2-yl)thiazole

To a stirred solution of 6-(4-isopropyl-1'-(piperidin-4-yl)-1H,1'H-[3,4'-bipyrazol]-5-yl)-8-methyl-[1,2,4]triazolo[1,5-a]pyridine (30 mg, 0.077 mmol) in MeOH (5 mL) were added AcOH (0.2 mL) and formaldehyde (2.307 mg, 0.077 mmol). The reaction mixture was stirred at room temperature for 6 h. Next, NaCNBH₃ (4.83 mg, 0.077 mmol) was added and the reaction mixture was stirred for 2 h. The reaction mass was concentrated to get crude compound. The crude compound was purified by Prep LCMS using method AA, the fractions containing the product were combined and dried via centrifugal evaporation to get 6-(4-isopropyl-1'-(1-methylpiperidin-4-yl)-1H,1'H-[3,4'-bipyrazol]-5-yl)-8-methyl-[1,2,4]triazolo[1,5-a]pyridine (7.6mg, 0.136 mmol, 23 % yield) as an off-white solid. LCMS Retention time: 0.87 [E], MS (E⁻) m/z: 391.3 [M+H]; ¹H NMR (400 MHz, DMSO-d₆) δ 12.99 (s, 1H), 8.75 (br. s., 2H), 8.51 (s, 2H), 7.75 (s, 1H), 7.56(s, 1H), 7.25 (br. s., 2H), 7.12 (br. s., 2H), 7.00 (br. s., 2H), 4.59 (br. s., 1H), 3.14-3.01 (m, 3H),2.61 (s, 3H), 2.28-2.20 (m, 2H), 2.16 (d, J=12.7 Hz, 2H), 1.20-1.10 (m, 6H).

The following Examples were prepared according to the general procedure used to prepare Example 1090.

**TABLE 100**

| Ex. No. | Structure | LCMS MH⁺ | RT (min) | HPLC Method |
|---|---|---|---|---|
| 1091 | 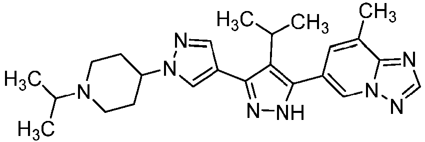 | 433.3 | 1.07 | D |
| 1092 | 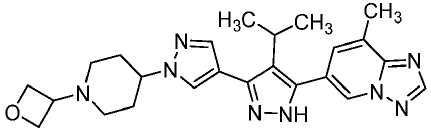 | 447.3 | 1.17 | E |

### EXAMPLE 1093

### 6-(4-isopropyl-3-(4-methyl-5-(1-propylpiperidin-4-yl)pyridin-2-yl)-1H-pyrazol-5-yl)-8-methoxy-[1,2,4]triazolo[1,5-a]pyridine

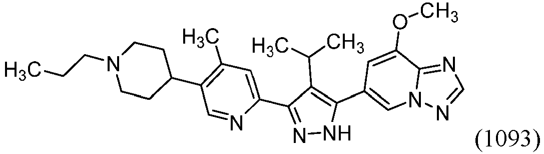

### Intermediate 1093A: tert-butyl 6-chloro-4-methyl-3',6'-dihydro-[3,4'-bipyridine]-1'(2'H)-carboxylate

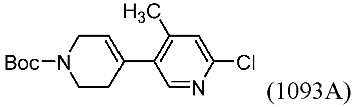

A mixture of 5-bromo-2-chloro-4-methylpyridine (1.5 g, 7.27 mmol), tert-butyl 4-(4,4,5,5-tetramethyl-1,3,2-dioxaborolan-2-yl)-3,6-dihydropyridine-1(2H)-carboxylate (1.872 g, 6.05 mmol) and potassium phosphate (3.86 g, 18.16 mmol) in dioxane (16.00 mL) and water (4.00 mL) system was degassed for 10 min. To the mixture was added PdCl₂(dppf)-CH₂Cl₂ adduct (0.494 g, 0.605 mmol). The solution was degassed for 2 min. The reaction mixture was stirred at 100 °C for 16 h. The reaction mixture was brought to room temperature, diluted with water and DCM, separated both the layers, the aqueous layer was extracted with DCM (1 X 50 mL), the combined organic extracts were dried (Na₂SO₄) and concentrated to get crude compound. The crude compound was purified by ISCO using 40 g silica column, compound was eluted in 30% EA in hexanes, the fractions were collected and concentrated to get tert-butyl 6-chloro-4-methyl-3',6'-dihydro-[3,4'-bipyridine]-1'(2'H)-carboxylate (1.62 g, 5.25 mmol, 87 % yield) as a white solid. LCMS retention time 1.92 min [A], MS (ES): *m*/*z=* 309.1 [M+H].

### Intermediate 1093B: tert-butyl 6-(4-isopropyl-5-(8-methoxy-[1,2,4]triazolo[1,5-a]pyridin-6-yl)-1-((2-(trimethylsilyl)ethoxy)methyl)-1H-pyrazol-3-yl)-4-methyl-3',6'-dihydro-[3,4'-bipyridine]-1'(2'H)-carboxylate

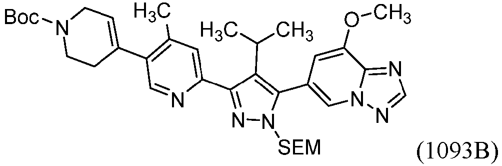

A solution of 6-(4-isopropyl-3-(4,4,5,5-tetramethyl-1,3,2-dioxaborolan-2-yl)-1-((2-(trimethylsilyl)ethoxy)methyl)-1H-pyrazol-5-yl)-8-methoxy-[1,2,4]triazolo[1,5-a] pyridine (1.846 g, 3.59 mmol), tert-butyl 6-chloro-4-methyl-3',6'-dihydro-[3,4'-bipyridine]-1'(2'H)-carboxylate (0.740 g, 2.396 mmol) and K₂CO₃ (0.994 g, 7.19 mmol) in acetonitrile (50.00 mL) and water (12.50 mL) solvent mixture was degassed for 10 min with nitrogen. To the solution were added Pd₂(dba)₃ (0.110 g, 0.120 mmol) and 2-dicyclohexylphosphino-2',6'-dimethoxybiphenyl (0.098 g, 0.240 mmol). The reaction mixture was degassed for 2 min, and stirred at 110 °C for 16 h. The reaction mixture was brought to room temperature, separated both the layers, the aqueous layer was extracted with EtOAc (2 X 20 mL), combined organic extracts were dried (Na₂SO₄) and concentrated to get crude compound. The crude was purified by ISCO using 40 g silica column, compound was eluted in 55% EA in hexane, the fractions were collected and concentrated to get tert-butyl 6-(4-isopropyl-5-(8-methoxy-[1,2,4]triazolo[1,5-a]pyridin-6-yl)-1-((2-(trimethylsilyl)ethoxy)methyl)-1H-pyrazol-3-yl)-4-methyl-3',6'-dihydro-[3,4'-bipyridine]-1'(2'H)-carboxylate (800 mg, 1.2 mmol, 51 % yield) as a gummy solid. LCMS retention time 1.85 min [A], MS (ES): *m*/*z=* 660.6 [M+H].

### Intermediate 1093C: tert-butyl 4-(6-(4-isopropyl-5-(8-methoxy-[1,2,4]triazolo[1,5-a] pyridin-6-yl)-1-((2-(trimethylsilyl)ethoxy)methyl)-1H-pyrazol-3-yl)-4-methylpyridin-3-yl)piperidine-1-carboxylate

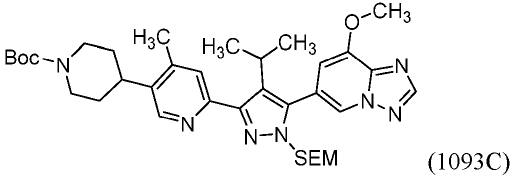

To a solution of tert-butyl 6-(4-isopropyl-5-(8-methoxy-[1,2,4]triazolo[1,5-a] pyridin-6-yl)-1-((2-(trimethylsilyl)ethoxy)methyl)-1H-pyrazol-3-yl)-4-methyl-3',6'-dihydro-[3,4'-bipyridine]-1'(2'H)-carboxylate (800 mg, 1.212 mmol) in MeOH (30 mL) was added Pd-C (129 mg, 1.212 mmol) at room temperature. The slurry was stirred at the same temperature under a hydrogen bladder for 16 h. The reaction mass was filtered and the filtrates were concentrated to get crude compound. The crude compound was purified by ISCO using 24 g silica column. The compound was eluted in 50 % EA in hexanes, the fractions were collected and concentrated to get tert-butyl 4-(6-(4-isopropyl-5-(8-methoxy-[1,2,4]triazolo[1,5-a]pyridin-6-yl)-1-((2-(trimethylsilyl)ethoxy)methyl)-1H-pyrazol-3-yl)-4-methylpyridin-3-yl)piperidine-1-carboxylate (720 mg, 1.088 mmol, 90 % yield) as a gummy solid. LCMS retention time 1.75 min [A], MS (ES): *m*/*z=* 662.6 [M+H].

### Intermediate 1093D: 6-(4-isopropyl-3-(4-methyl-5-(piperidin-4-yl)pyridin-2-yl)-1H-pyrazol-5-yl)-8-methoxy-[1,2,4]triazolo[1,5-a]pyridine, TFA

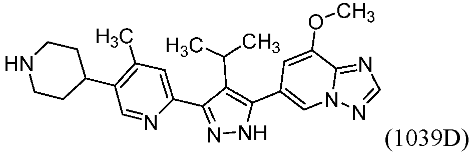

To a solution of tert-butyl 4-(6-(4-isopropyl-5-(8-methoxy-[1,2,4]triazolo[1,5-a] pyridin-6-yl)-1-((2-(trimethylsilyl)ethoxy)methyl)-1H-pyrazol-3-yl)-4-methylpyridin-3-yl)piperidine-1-carboxylate (700 mg, 1.058 mmol) in DCM (8.00 mL) was added TFA (5.00 mL, 64.9 mmol) at 0 °C. The reaction mixture was stirred at room temperature for 16 h. The solvents were removed by blowing with nitrogen gas, then dried under vacuum to get crude compound. The crude compound was triturated with diethyl ether (2 X 10 mL), and dried under vacuum to get 6-(4-isopropyl-3-(4-methyl-5-(piperidin-4-yl) pyridin-2-yl)-1H-pyrazol-5-yl)-8-methoxy-[1,2,4]triazolo[1,5-a]pyridine, TFA (450 mg, 0.826 mmol, 78 % yield) as a white solid. LCMS retention time 0.78 min [A], MS (ES): *m*/*z=* 432.2 [M+H]; ¹H NMR (400 MHz, METHANOL-d₄) δ ppm 8.64-8.49 (m, 2H), 8.45 (s, 1H), 7.64-7.48 (m, 1H), 7.31-7.15 (m, 1H), 4.18-4.11 (m, 3H), 3.62-3.53 (m, 2H), 3.37 (s, 1H), 3.31-3.17 (m, 3H), 2.54 (s, 3H), 2.19-2.01 (m, 4H), 1.36-1.18 (m, 8H).

### Example 1093: 6-(4-isopropyl-3-(4-methyl-5-(1-propylpiperidin-4-yl)pyridin-2-yl)-1H-pyrazol-5-yl)-8-methoxy-[1,2,4]triazolo[1,5-a]pyridine

To a solution of 6-(4-isopropyl-3-(4-methyl-5-(piperidin-4-yl)pyridin-2-yl)-1H-pyrazol-5-yl)-8-methoxy-[1,2,4]triazolo[1,5-a]pyridine, TFA- (25 mg, 0.046 mmol) in MeOH (1.5 mL) were added AcOH (0.1mL, 1.747 mmol), propionaldehyde (26.7 mg, 0.459 mmol) and sodium cyanoborohydride (14.42 mg, 0.230 mmol) at room temperature. The reaction mixture was stirred at the same temperature for 3 h. The reaction mass was purified by Prep LCMS using method AA, the fractions containing product were combined and dried using Genevac centrifugal evaporator to get 6-(4-isopropyl-3-(4-methyl-5-(1-propylpiperidin-4-yl)pyridin-2-yl)-1H-pyrazol-5-yl)-8-methoxy-[1,2,4]triazolo[1,5-a] pyridine (7.2 mg, 0.015 mmol, 33 % yield) as a pale solid. LCMS retention time 0.957 min [F], MS (ES): *m*/*z=* 474.3 [M+H]; ¹H NMR (400 MHz, METHANOL-d₄) δ ppm 8.54 (br d, *J* =5.6 Hz, 2H), 8.44 (s, 1H), 7.51 (s, 1H), 7.23 (s, 1H), 4.14 (s, 3H), 3.37 (s, 1H), 3.26-3.18 (m, 2H), 3.02-2.92 (m, 1H), 2.57-2.43 (m, 5H), 2.35-2.23 (m, 2H), 2.02-1.88 (m, 4H), 1.71-1.60 (m, 2H), 1.26 (d, *J* =7.1 Hz, 6H), 0.99 (t, *J* =7.3 Hz, 3H).

The following Examples were prepared according to the general procedure used to prepare Example 1093D.

**TABLE 101**

| Ex. No. | Structure | LCMS MH⁺ | Ret Time (min) | HPLC Method |
|---|---|---|---|---|
| 1094 | 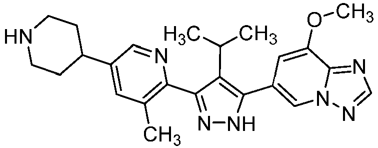 | 432.3 | 0.97 | C |
| 1095 | 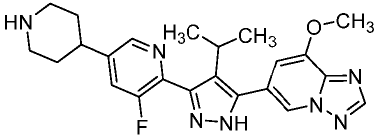 | 436.3 | 0.97 | C |
| 1096 | 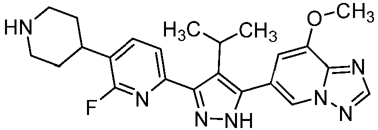 | 436.2 | 1.089 | E |

The following Examples were prepared according to the general procedure used to prepare Example 1093.

**TABLE 102**

| Ex. No. | Structure | LCMS MH⁺ | RT (min) | HPLC Method |
|---|---|---|---|---|
| 1097 | 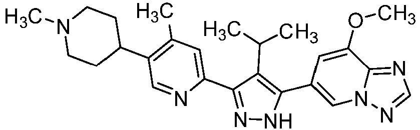 | 466.3 | 1.131 | E |
| 1098 | 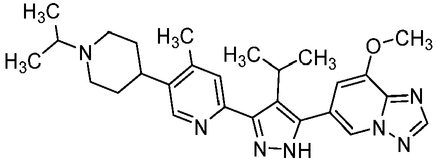 | 474.3 | 1.213 | E |
| 1099 | 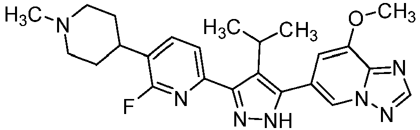 | 450.2 | 1.337 | E |
| 1100 | 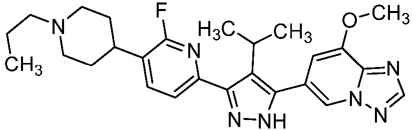 | 478.3 | 1.622 | E |
| 1101 | 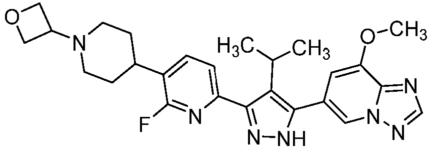 | 492.3 | 1.585 | E |
| 1102 | 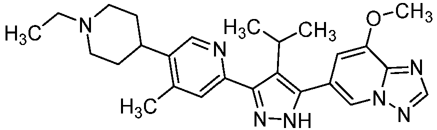 | 460.3 | 1.207 | E |
| 1103 | 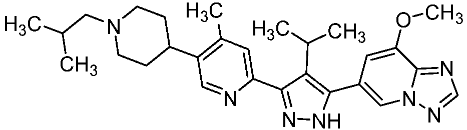 | 488.3 | 1.568 | E |
| 1104 | 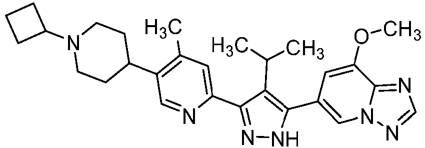 | 486.3 | 1.43 | E |
| 1105 | 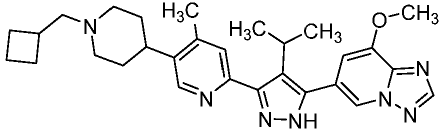 | 500.3 | 1.53 | E |
| 1106 | 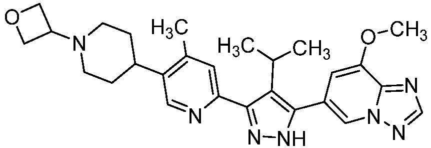 | 488.3 | 1.458 | E |
| 1107 | 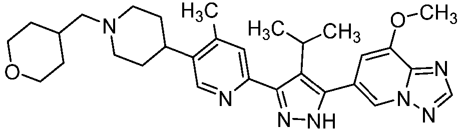 | 556.3 | 1.33 | C |
| 1108 | 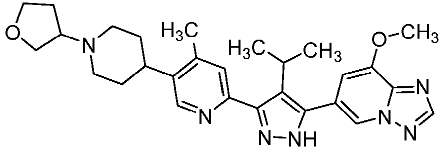 | 502.2 | 1.938 | B |
| 1109 | 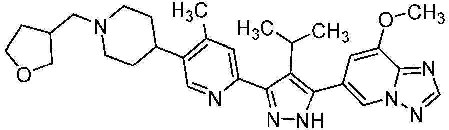 | 516.2 | 1.876 | B |
| 1110 | 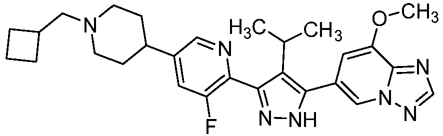 | 504.3 | 1.47 | C |
| 1111 | 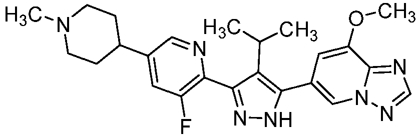 | 450.3 | 1.09 | C |
| 1112 | 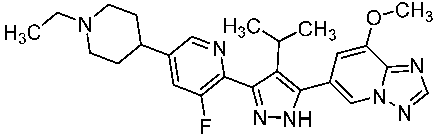 | 464.3 | 1.23 | C |
| 1113 | 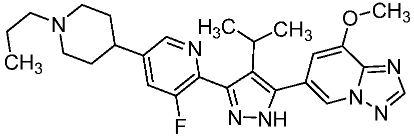 | 478.3 | 1.27 | C |
| 1114 | 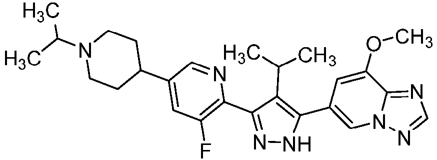 | 478.3 | 1.16 | C |
| 1115 | 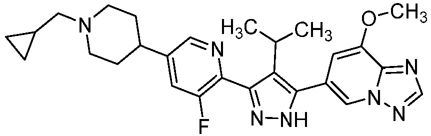 | 490.3 | 1.28 | C |
| 1116 | 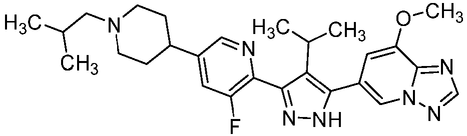 | 492.3 | 1.52 | C |
| 1117 | 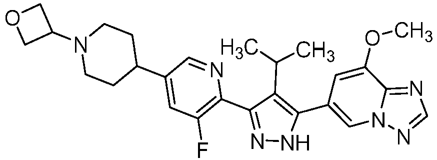 | 492.3 | 1.39 | C |
| 1118 | 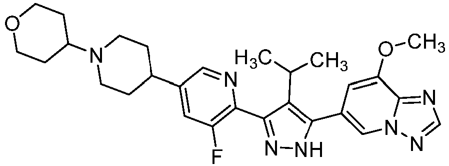 | 520.3 | 1.25 | C |
| 1119 | 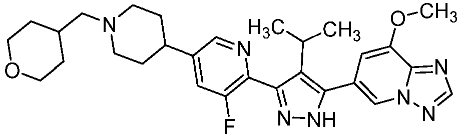 | 534.3 | 1.38 | C |
| 1120 | 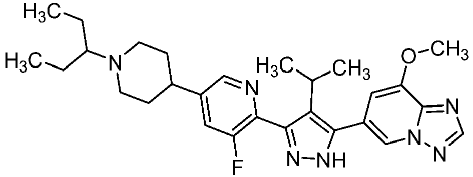 | 506.3 | 1.56 | C |
| 1121 | 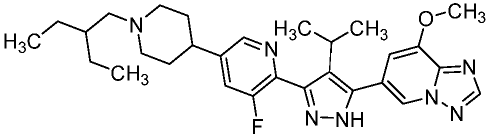 | 520.3 | 1.9 | C |
| 1123 | 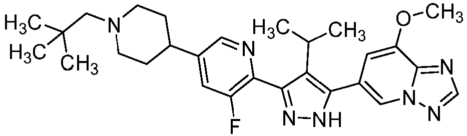 | 506.3 | 2.07 | C |
| 1124 | 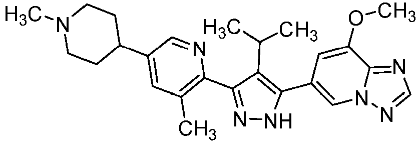 | 446.3 | 1.07 | C |
| 1125 | 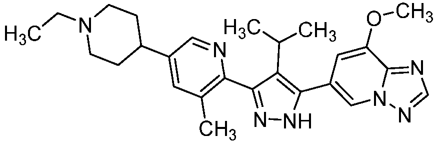 | 460.3 | 1.11 | C |
| 1126 | 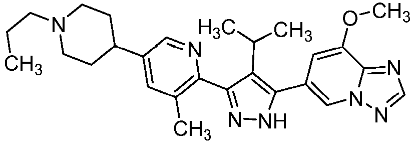 | 474.3 | 1.38 | C |
| 1127 | 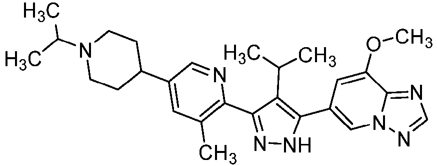 | 474.3 | 1.15 | C |
| 1128 | 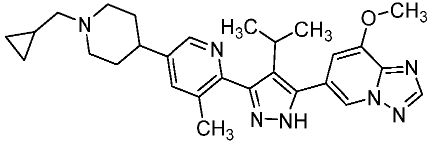 | 486.3 | 1.25 | C |
| 1129 | 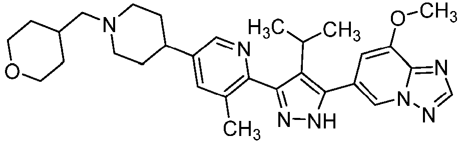 | 530.3 | 0.98 | C |
| 1130 | 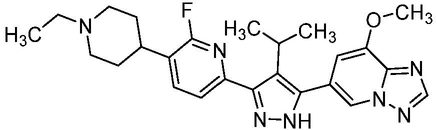 | 464.2 | 1.170 | F |
| 1131 | 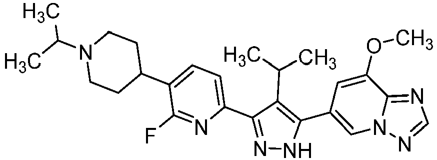 | 478.3 | 1.170 | F |
| 1132 | 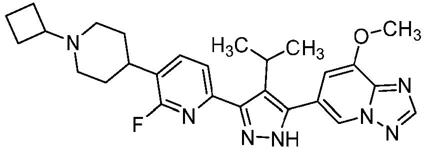 | 490.3 | 1.281 | F |
| 1133 | 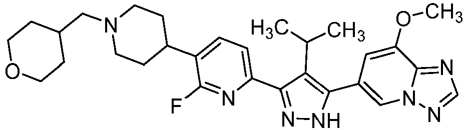 | 534.3 | 1.175 | F |

### EXAMPLE 1134

### 2-(dimethylamino)-1-(4-(6-(4-isopropyl-5-(8-methoxy-[1,2,4]triazolo[1,5-a]pyridin-6-yl)-1H-pyrazol-3-yl)-4-methylpyridin-3-yl)piperidin-1-yl)ethan-1-one

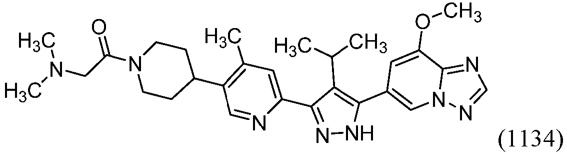

To a solution of 6-(4-isopropyl-3-(4-methyl-5-(piperidin-4-yl)pyridin-2-yl)-1H-pyrazol-5-yl)-8-methoxy-[1,2,4]triazolo[1,5-a]pyridine, TFA (19 mg, 0.035 mmol) in DMF (1.5 mL) were added TEA (0.2 mL, 1.435 mmol), dimethylglycine (17.99 mg, 0.174 mmol) and HATU (26.5 mg, 0.070 mmol) at room temperature. The reaction mixture was stirred at the same temperature for 16 h. The reaction mass purified by Prep LCMS purification using method AA, the fractions containing product were combined and dried using Genevac centrifugal evaporator to get 2-(dimethylamino)-1-(4-(6-(4-isopropyl-5-(8-methoxy-[1,2,4]triazolo[1,5-a]pyridin-6-yl)-1H-pyrazol-3-yl)-4-methylpyridin-3-yl)piperidin-1-yl)ethan-1-one (4.1 mg, 7.78 µmol, 22% yield) as a pale solid. LCMS retention time 1.247 min [E], MS (ES): *m*/*z=* 517.3 [M+H]⁺; ¹H NMR (400 MHz, METHANOL-d₄) δ ppm 8.54-8.65 (m, 2 H) 8.48 (s, 1 H) 7.92 (s, 1 H) 7.21 (d, *J*=1.22 Hz, 1 H) 4.77 (br dd, *J*=11.62, 1.83 Hz, 1 H) 4.35 (q, *J*=16.14 Hz, 2 H) 4.14 (s, 3 H) 3.83-3.93 (m, 1 H) 3.37 (br d, *J*=7.34 Hz, 2 H) 2.90-3.07 (m, 8 H) 2.74 (s, 3 H) 2.01-2.11 (m, 2 H) 1.72-1.96 (m, 2 H) 1.29 (d, *J*=7.09 Hz, 7 H).

The following Example was prepared according to the general procedure used to prepare Example 1134.

**TABLE 103**

| Ex. No. | Structure | LCMS MH⁺ | RT (min) | HPLC Method |
|---|---|---|---|---|
| 1135 | 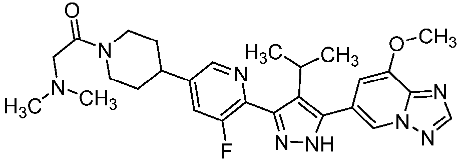 | 521.3 | 1.16 | C |

### EXAMPLE 1136

### 2-(4-(6-(4-isopropyl-5-(8-methoxy-[1,2,4]triazolo[1,5-a]pyridin-6-yl)-1H-pyrazol-3-yl)-4-methylpyridin-3-yl)piperidin-1-yl)-N,N-dimethylacetamide

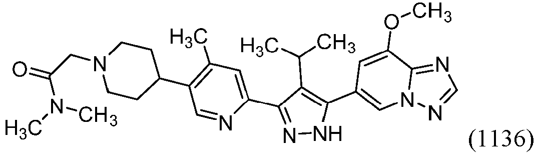

To a solution of 6-(4-isopropyl-3-(4-methyl-5-(piperidin-4-yl)pyridin-2-yl)-1H-pyrazol-5-yl)-8-methoxy-[1,2,4]triazolo[1,5-a]pyridine, TFA- (24 mg, 0.044 mmol) in DMF (0.5 mL) and THF (1.00 mL) were added TEA (0.2 mL, 1.435 mmol), and 2-chloro-N,N-dimethylacetamide (26.8 mg, 0.220 mmol) at room temperature. The reaction mixture was stirred at the same temperature for 16 h. The reaction mass was purified by Prep LCMS using method AA, the fractions containing the product were combined and dried using Genevac centrifugal evaporator to get 2-(4-(6-(4-isopropyl-5-(8-methoxy-[1,2,4]triazolo[1,5-a]pyridin-6-yl)-1H-pyrazol-3-yl)-4-methylpyridin-3-yl)piperidin-1-yl)-N,N-dimethylacetamide (11.8 mg, 0.022 mmol, 49% yield) as a pale solid. LCMS retention time 1.345 min [E], MS (ES): *m*/*z=* 517.3 [M+H]; ¹H NMR (400 MHz, METHANOL-d₄) δ ppm 8.58 (s, 1 H) 8.56 (d, *J*=1.22 Hz, 1 H) 8.46 (s, 1 H) 7.71 (s, 1 H) 7.22 (d, *J*=1.22 Hz, 1 H) 4.31 (s, 2 H) 4.14 (s, 3 H) 3.77-3.89 (m, 2 H) 3.36 (br dd, *J*=5.62, 2.20 Hz, 2 H) 3.02-3.11 (m, 7 H) 2.61 (s, 3 H) 2.14-2.34 (m, 4 H) 1.23-1.36 (m, 8 H).

The following Examples were prepared according to the general procedure used to prepare Example 1136.

**TABLE 104**

| Ex. No. | Structure | LCMS MH⁺ | RT (min) | HPLC Method |
|---|---|---|---|---|
| 1137 | 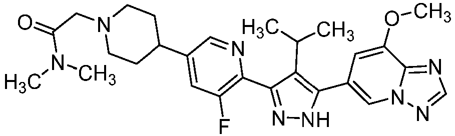 | 521.3 | 1.29 | C |
| 1138 | 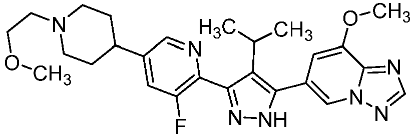 | 494.3 | 1.27 | C |
| 1139 | 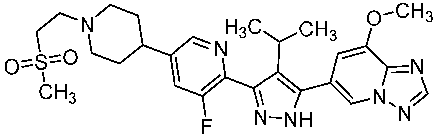 | 542.3 | 1.37 | C |
| 1140 | 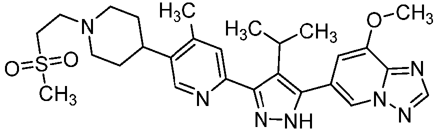 | 538.2 | 1.422 | E |

### EXAMPLE 1141

### 2-(5-(8-methoxy-[1,2,4]triazolo[1,5-a]pyridin-6-yl)-4-(2,2,2-trifluoroethyl)-1H-pyrazol-3-yl)-4-methyl-5-(1-methylpiperidin-4-yl)thiazole

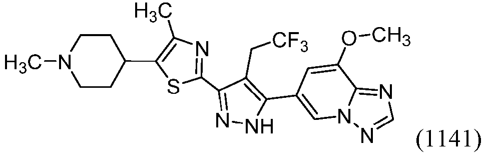

### Intermediate 1141A: 8-methoxy-6-(3-(4,4,5,5-tetramethyl-1,3,2-dioxaborolan-2-yl)-4-(2,2,2-trifluoroethyl)-1-((2-(trimethylsilyl)ethoxy)methyl)-1H-pyrazol-5-yl)-[1,2,4]triazolo[1,5-a] pyridine

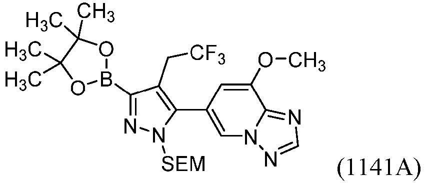

To a mixture of 6-(3-bromo-4-(2,2,2-trifluoroethyl)-1-((2-(trimethylsilyl)ethoxy) methyl)-1H-pyrazol-5-yl)-8-methoxy-[1,2,4]triazolo[1,5-a]pyridine (1.5 g, 2.96 mmol) and bis(pinacolato)diboron (1.128 g, 4.44 mmol) in dioxane (20 mL) was added potassium acetate (0.872 g, 8.89 mmol), The mixture was degassed with nitrogen for 15 min, then tricyclohexylphosphine (0.083 g, 0.296 mmol) and tris(dibenzylideneacetone) dipalladium(0) (0.136 g, 0.148 mmol) were added. The reaction mixture was degassed for 10 min. The mixture was stirred at 110 °C for 16 h. The reaction mass filtered through celite bed washed with EtOAc, the filtrates were collected and concentrated to get crude. The crude mass was purified by ISCO using silica column (40 g), the fractions were collected and concentrated to get 8-methoxy-6-(3-(4,4,5,5-tetramethyl-1,3,2-dioxaborolan-2-yl)-4-(2,2,2-trifluoroethyl)-1-((2-(trimethylsilyl)ethoxy)methyl)-1H-pyrazol-5-yl)-[1,2,4]triazolo[1,5-a]pyridine (850 mg, 1.536 mmol, 52 % yield) as a brown liquid. LCMS retention time 2.18 min [A]. MS (E⁻) *m*/*z* 554.6 [M+H].

### Intermediate 1141B: tert-butyl 4-(2-(5-(8-methoxy-[1,2,4]triazolo[1,5-a]pyridin-6-yl)-4-(2,2,2-trifluoroethyl)-1-((2-(trimethylsilyl)ethoxy)methyl)-1H-pyrazol-3-yl)-4-methyl thiazol-5-yl)piperidine-1-carboxylate

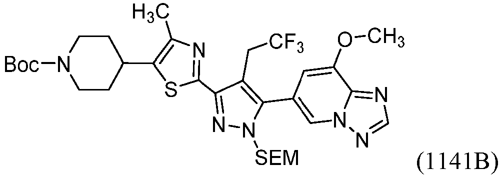

Mixture of 8-methoxy-6-(3-(4,4,5,5-tetramethyl-1,3,2-dioxaborolan-2-yl)-4-(2,2,2-trifluoroethyl)-1-((2-(trimethylsilyl)ethoxy)methyl)-1H-pyrazol-5-yl)-[1,2,4]triazolo [1,5-a] pyridine (1.5 g, 2.71 mmol), tert-butyl 4-(2-bromo-4-methylthiazol-5-yl)piperidine-1-carboxylate (1.175 g, 3.25 mmol) and potassium carbonate (1.124 g, 8.13 mmol) in acetonitrile (50 mL) was degassed with nitrogen for 10 min, Next, Pd₂(dba)₃ (0.124 g, 0.136 mmol) and 2-dicyclohexylphosphino-2',6'-dimethoxybiphenyl (0.111 g, 0.271 mmol) were added and the reaction mixture was stirred at 110 °C for 16 h. The reaction mass was filtered through a celite bed, washed with EtOAc, the filtrates were collected and concentrated to get crude compound. The crude mass was purified by ISCO silica 40 g column (50% EtOAc-Hex), the fractions were collected and concentrated to get tert-butyl 4-(2-(5-(8-methoxy-[1,2,4]triazolo[1,5-a]pyridin-6-yl)-4-(2,2,2-trifluoroethyl)-1-((2-(trimethylsilyl)ethoxy) methyl)-1H-pyrazol-3-yl)-4-methylthiazol-5-yl)piperidine-1-carboxylate (1.1 g, 1.554 mmol, 57 % yield) as a gummy solid. LCMS Retention time: 1.58 [B], MS (E⁻) m/z: 708.5 [M+H].

### Intermediate 1141C: 2-(5-(8-methoxy-[1,2,4]triazolo[1,5-a]pyridin-6-yl)-4-(2,2,2-trifluoroethyl)-1H-pyrazol-3-yl)-4-methyl-5-(piperidin-4-yl)thiazole

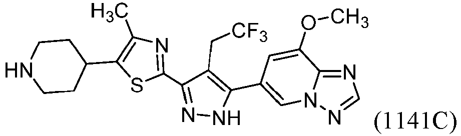

To a stirred solution of tert-butyl 4-(2-(5-(8-methoxy-[1,2,4]triazolo[1,5-a]pyridin-6-yl)-4-(2,2,2-trifluoroethyl)-1-((2-(trimethylsilyl)ethoxy)methyl)-1H-pyrazol-3-yl)-4-methylthiazol-5-yl)piperidine-1-carboxylate (220 mg, 0.311 mmol) in DCM was added TFA (23.94 µL, 0.311 mmol). The reaction mixture was stirred at room temperature for 2 h. The reaction mass was concentrated to get the crude compound. The crude compound was purified by prep LCMS using method AB, the fractions were and concentrated to get 2-(5-(8-methoxy-[1,2,4]triazolo[1,5-a]pyridin-6-yl)-4-(2,2,2-trifluoroethyl)-1H-pyrazol-3-yl)-4-methyl-5-(piperidin-4-yl)thiazole (60 mg, 0.126 mmol, 40 % yield) as an off-white solid. LCMS Retention time: 0.44 [F], MS (E⁻) m/z: 478.2 [M+H]; ¹H NMR (400 MHz, DMSO-d₆) δ 9.18 (s, 1H), 8.57 (s, 1H), 7.80-7.74 (m, 1H), 7.73 (d, J=1.0Hz, 1H), 4.34-4.25 (m, 2H), 3.91 (s, 1H), 3.25-3.12 (m, 3H), 2.88-2.74 (m, 2H), 2.63 (s, 3H),2.06 (d, J=12.5 Hz, 2H), 1.89 (s, 3H), 1.78-1.60 (m, 2H).

### Example 1141: 2-(5-(8-methoxy-[1,2,4]triazolo[1,5-a]pyridin-6-yl)-4-(2,2,2-trifluoroethyl)-1H-pyrazol-3-yl)-4-methyl-5-(1-methylpiperidin-4-yl)thiazole

To a stirred solution of 2-(5-(8-methoxy-[1,2,4]triazolo[1,5-a]pyridin-6-yl)-4-(2,2,2-trifluoroethyl)-1H-pyrazol-3-yl)-4-methyl-5-(piperidin-4-yl)thiazole (50 mg, 0.105 mmol) in MeOH (1 mL), were added formaldehyde (3.14 mg, 0.105 mmol) and acetic acid (5.99 µL, 0.105 mmol). The reaction mixture was stirred at room temperature for 16 h. Next, NaCNBH₄ (6.58 mg, 0.105 mmol) was added and the reaction mixture was stirred for another 2 h. The reaction mass was purified by Prep LCMS using method AA, the fractions were collected and concentrated to get 2-(5-(8-methoxy-[1,2,4]triazolo[1,5-a]pyridin-6-yl)-4-(2,2,2-trifluoroethyl)-1H-pyrazol-3-yl)-4-methyl-5-(1-methylpiperidin-4-yl)thiazole (4.7 mg, 0.126 mmol, 8% yield) as a white solid. LCMS Retention time: 1.44 [E], MS (E⁻) m/z: 492.2 [M+H]; ¹H NMR (400 MHz, DMSO-d₆) δ 13.87 (br. s., 1H), 8.95 (d, J=1.2 Hz, 1H), 8.55 (s, 1H), 7.72(s, 1H), 7.30 (d, J=1.2 Hz, 1H), 4.37-4.22 (m, 2H), 4.07 (s, 3H), 3.96-3.84 (m, 2H), 3.30 -3.22 (m, 2H), 3.00 (br. s., 4H), 2.37-2.16 (m, 3H), 2.08 (s, 1H), 2.02 (br. s., 1H), 1.70 (br. s.,4H), 1.49 (br. s., 2H), 1.24 (s, 2H).

The following Examples were prepared according to the general procedure used to prepare Example 1141C.

**TABLE 105**

| Ex. No. | Structure | LCMS MH⁺ | RT (min) | HPLC Method |
|---|---|---|---|---|
| 1142 | 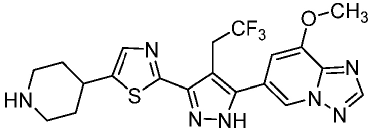 | 464.2 | 1.02 | C |
| 1143 | 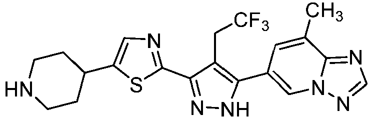 | 448.2 | 1.04 | E |
| 1144 | 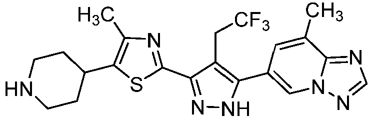 | 462.2 | 1.16 | E |
| 1145 | 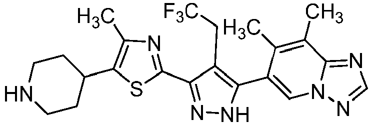 | 476.2 | 1.38 | C |

The following Examples were prepared according to the general procedure used to prepare Example 1141.

**TABLE 106**

| Ex. No. | Structure | LCMS MH⁺ | RT (min) | HPLC Method |
|---|---|---|---|---|
| 1146 | 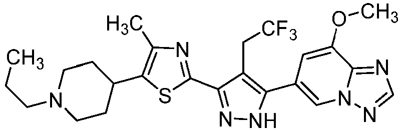 | 520.2 | 1.61 | E |
| 1147 | 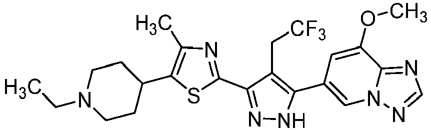 | 506.2 | 1.44 | C |
| 1148 | 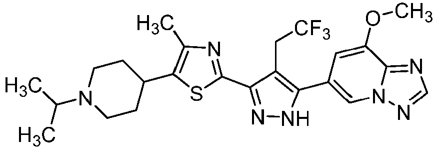 | 520.2 | 1.49 | C |
| 1149 | 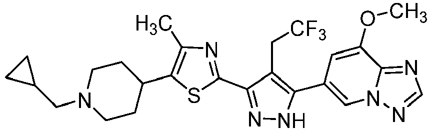 | 532.2 | 1.64 | C |
| 1150 | 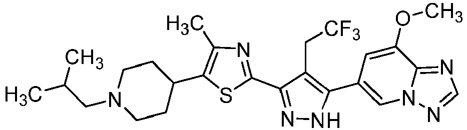 | 534.2 | 1.92 | C |
| 1151 | 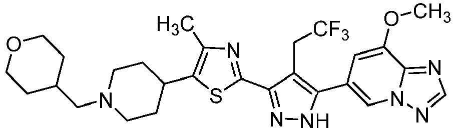 | 576.2 | 2.06 | C |
| 1152 | 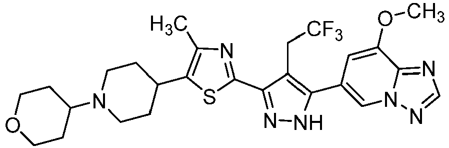 | 562.2 | 1.42 | E |
| 1153 | 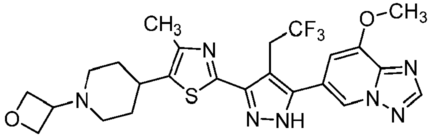 | 534.2 | 1.58 | E |
| 1154 | 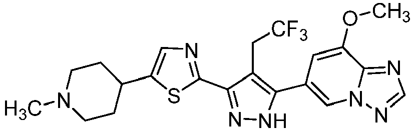 | 478.2 | 1.05 | F |
| 1155 | 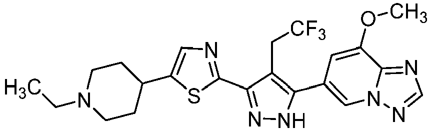 | 492.2 | 1.08 | C |
| 1156 | 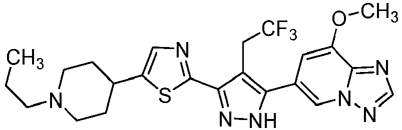 | 506.2 | 1.24 | C |
| 1157 | 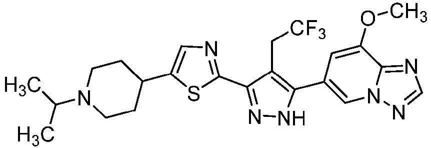 | 506.2 | 1.12 | E |
| 1158 | 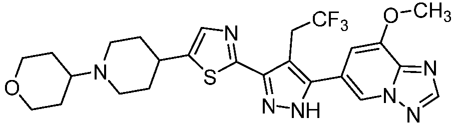 | 548.2 | 1.22 | E |
| 1159 | 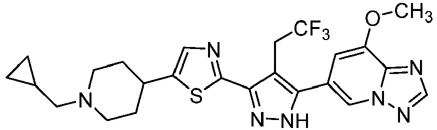 | 518.2 | 1.35 | E |
| 1160 | 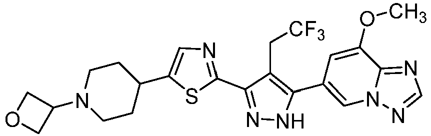 | 520.2 | 1.44 | E |
| 1161 | 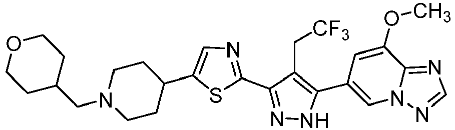 | 562.2 | 1.15 | D |
| 1162 | 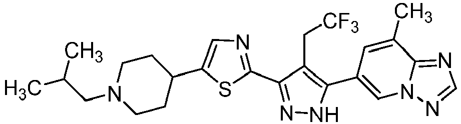 | 504.2 | 1.69 | C |
| 1163 | 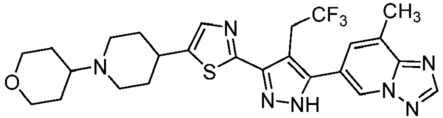 | 532.2 | 1.73 | E |
| 1164 | 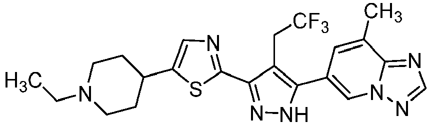 | 476.2 | 1.31 | C |
| 1165 | 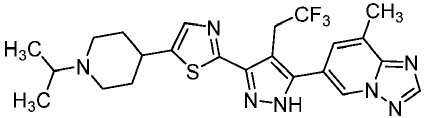 | 490.2 | 1.2 | C |
| 1166 | 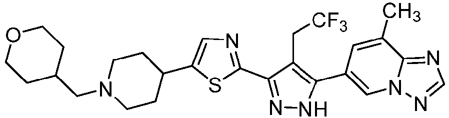 | 546.2 | 1.12 | D |
| 1167 | 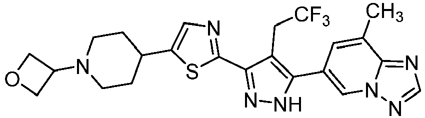 | 504.2 | 1.42 | C |
| 1168 | 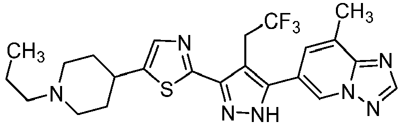 | 490.2 | 1.39 | C |
| 1169 | 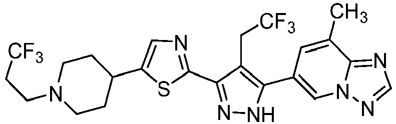 | 544.2 | 1.98 | C |
| 1170 | 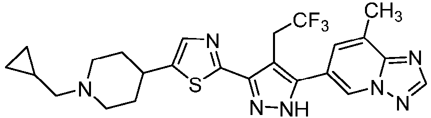 | 502.2 | 1.73 | C |
| 1171 | 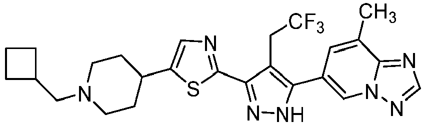 | 516.2 | 1.61 | C |
| 1172 | 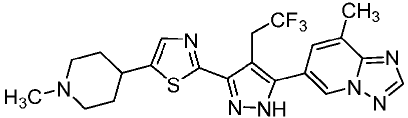 | 462.2 | 1.73 | E |
| 1173 | 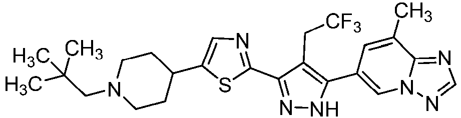 | 518.2 | 2.24 | C |
| 1174 | 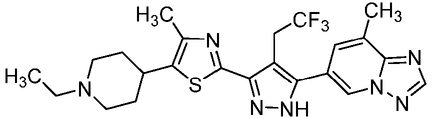 | 490.2 | 1.39 | C |
| 1175 | 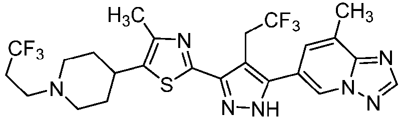 | 558.2 | 1.34 | C |
| 1176 | 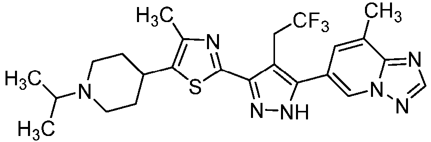 | 504.4 | 1.16 | F |
| 1177 | 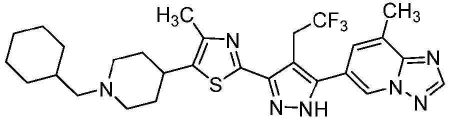 | 558.2 | 2.14 | C |
| 1179 | 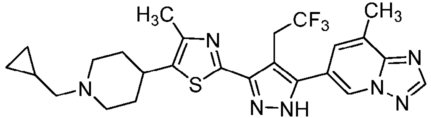 | 516.2 | 1.32 | C |
| 1180 | 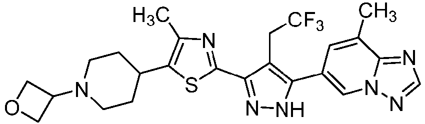 | 518.2 | 2.22 | C |
| 1181 | 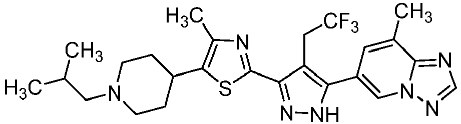 | 518.2 | 2.32 | C |
| 1182 | 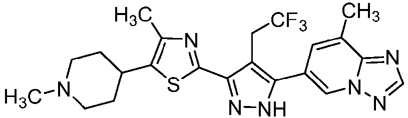 | 476.2 | 1.35 | C |
| 1183 | 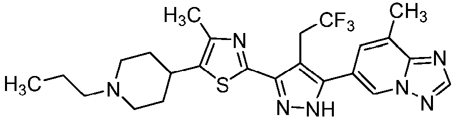 | 504.2 | 1.27 | F |
| 1184 | 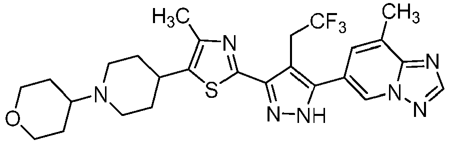 | 546.2 | 1.41 | D |
| 1185 | 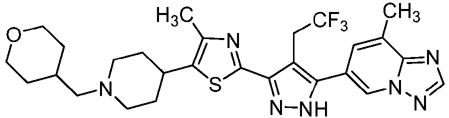 | 560.2 | 2.08 | C |
| 1186 | 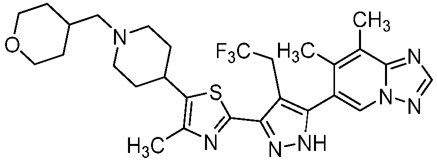 | 574.2 | 1.85 | C |
| 1187 | 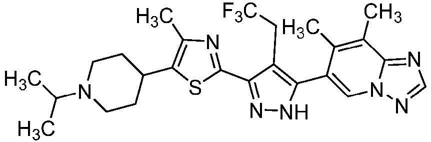 | 518.2 | 1.58 | C |
| 1188 | 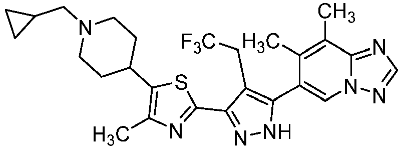 | 530.2 | 1.71 | C |
| 1189 | 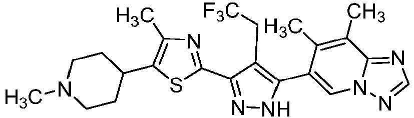 | 490.2 | 1.52 | C |
| 1190 | 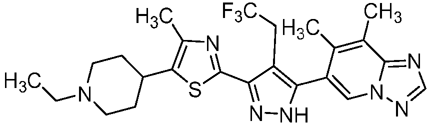 | 504.2 | 1.54 | E |
| 1191 | 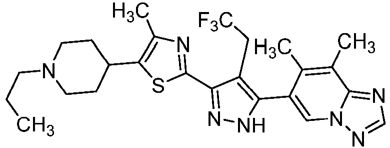 | 518.2 | 1.7 | E |

### EXAMPLE 1192

### 2-(dimethylamino)-1-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)ethan-1-one

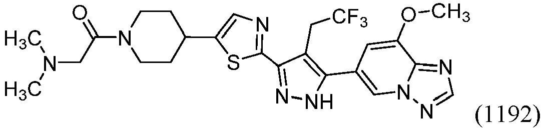

2-(Dimethylamino)-1-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)ethan-1-one was prepared according to the general process described in Example 469, using 2-(5-(8-methoxy-[1,2,4]triazolo[1,5-a]pyridin-6-yl)-4-(2,2,2-trifluoroethyl)-1H-pyrazol-3-yl)-5-(piperidin-4-yl)thiazole (30 mg, 0.065 mmol) as a starting intermediate to get the title compound as an off-white solid. LCMS Retention time 1.11 min [E], MS (E⁺) m/z: 549.2 [M+H]; ¹H NMR (400 MHz, DMSO-d₆) δ 13.89 (br. s., 1H), 8.95 (d, J=1.2 Hz, 1H), 8.55 (s, 1H), 7.74(s, 1H), 7.31 (d, J=1.2 Hz, 1H), 4.46 (d, J=11.5 Hz, 1H), 4.29 (q, J=11.2 Hz, 2H), 4.18-3.95 (m,3H), 3.24 (d, J=11.0 Hz, 1H), 3.20-3.03 (m, 2H), 2.79-2.67 (m, 1H), 2.26 (s, 4H), 2.15-1.94(m, 2H), 1.94-1.89 (m, 2H), 1.62 (d, J=9.0 Hz, 1H), 1.48 (d, J=8.8 Hz, 1H), 1.24 (s, 1H).

The following Examples were prepared according to the general procedure used to prepare Example 1192.

**TABLE 107**

| Ex. No. | Structure | LCMS MH⁺ | RT (min) | HPLC Method |
|---|---|---|---|---|
| 1193 | 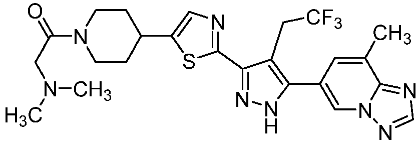 | 533.2 | 1.29 | C |
| 1194 | 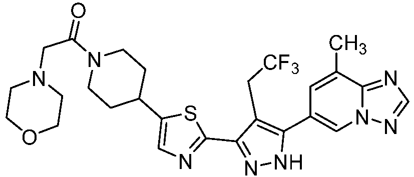 | 575.2 | 1.47 | C |
| 1195 | 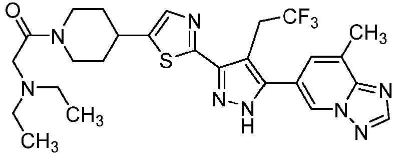 | 561.3 | 1.36 | C |

### EXAMPLE 1196

### 2-(5-(8-methoxy-[1,2,4]triazolo[1,5-a]pyridin-6-yl)-4-(2,2,2-trifluoroethyl)-1H-pyrazol-3-yl)-5-(1-(2-methoxyethyl)piperidin-4-yl)thiazole

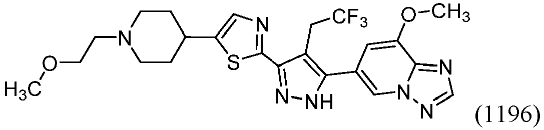

To a stirred solution of 2-(5-(8-methoxy-[1,2,4]triazolo[1,5-a]pyridin-6-yl)-4-(2,2,2-trifluoroethyl)-1H-pyrazol-3-yl)-5-(piperidin-4-yl)thiazole (30 mg, 0.065 mmol) in DMF (1 mL), THF (1 mL) were added 1-bromo-2-methoxyethane (9.00 mg, 0.065 mmol), Et₃N (9.02 µl, 0.065 mmol) at room temperature. The reaction mixture was stirred for 16 h. The reaction mass purified by Prep LCMS using method AB, the fractions containing the product were combined and dried via centrifugal evaporation to get 2-(5-(8-methoxy-[1,2,4]triazolo[1,5-a]pyridin-6-yl)-4-(2,2,2-trifluoroethyl)-1H-pyrazol-3-yl)-5-(1-(2-methoxyethyl)piperidin-4-yl)thiazole. LCMS Retention time 1.02 min [E], MS (E⁺) m/z: 522.2 [M+H]; ¹H NMR (400 MHz, DMSO-d₆) δ 13.87 (br. s., 1H), 8.95 (s, 1H), 8.55 (s, 1H), 7.73 (br. s., 1H),7.30 (s, 1H), 4.29 (d, J=11.2 Hz, 2H), 4.12-3.99 (m, 3H), 3.49 (br. s., 2H), 3.28 (br. s., 2H),2.94 (br. s., 4H), 2.08 (s, 4H), 1.92 (s, 1H), 1.76 (br. s., 3H), 1.24 (s, 3H).

The following Examples were prepared according to the general procedure used to prepare Example 1196.

**TABLE 108**

| Ex. No. | Structure | LCMS MH⁺ | RT (min) | HPLC Method |
|---|---|---|---|---|
| 1197 | 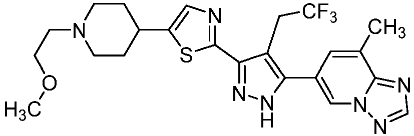 | 506.2 | 1.54 | C |
| 1198 | 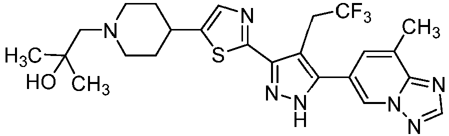 | 520.2 | 1.63 | C |
| 1199 | 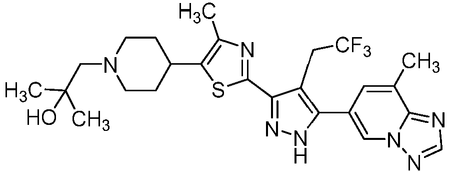 | 534.2 | 1.78 | C |
| 1200 | 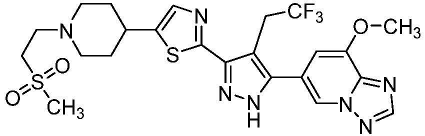 | 570.2 | 1.45 | C |
| 1201 | 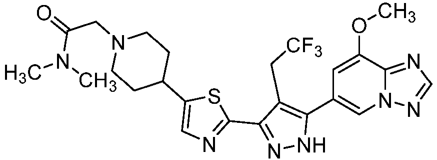 | 549.2 | 1.53 | C |
| 1202 | 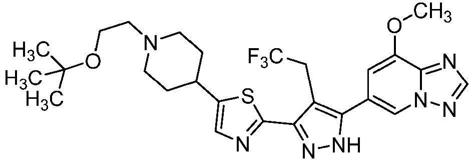 | 564.3 | 1.68 | C |
| 1203 | 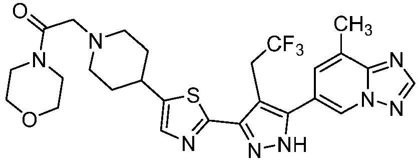 | 575.2 | 1.49 | C |
| 1204 | 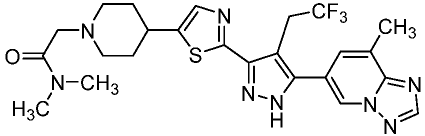 | 533.2 | 1.46 | C |
| 1205 | 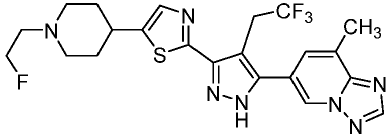 | 494.2 | 1.64 | C |
| 1206 | 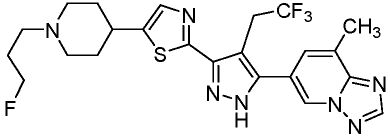 | 508.2 | 1.61 | C |

### EXAMPLE 1207

### 2-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)-1-(2-oxa-6-azaspiro[3.3] heptan-6-yl)ethan-1-one

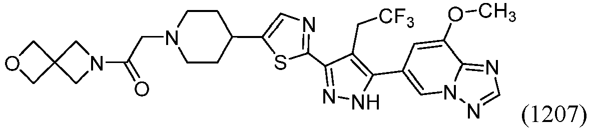

### Intermediate 1207A: tert-butyl 2-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)acetate

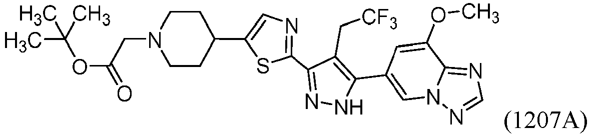

To a stirred solution of 2-(5-(8-methoxy-[1,2,4]triazolo[1,5-a]pyridin-6-yl)-4-(2,2,2-trifluoroethyl)-1H-pyrazol-3-yl)-5-(piperidin-4-yl)thiazole (200 mg, 0.432 mmol) in DCM (12 mL) were added Et₃N (0.180 mL, 1.295 mmol) and tert-butyl 2-bromoacetate (0.112 mL, 0.518 mmol) at room temperature, stirred for 16 h. The reaction mass quenched with water extracted with EtOAc, dried over sodium sulphate and concentrated to get tert-butyl 2-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)acetate (180 mg, 0.312 mmol, 72% yield) as a gummy solid. LCMS Retention time: 1.75 min [A], MS (E⁺) m/z: 578.1 [M+H].

### Intermediate 1207B: 2-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)acetic acid

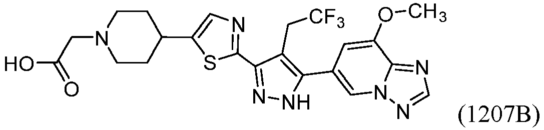

To a stirred solution of tert-butyl 2-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)acetate (250 mg, 0.433 mmol) in DCM (15 mL) was added TFA (0.033 mL, 0.433 mmol) at room temperature. The reaction mixture was stirred for 16 h. The reaction mass concentrated to get 2-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)acetic acid (160 mg, 0.307 mmol, 71 % yield) as an off-white solid. LCMS Retention time: 0.71 min [E], MS (E⁺) m/z: 522.2 [M+H].

### Example 1207: 2-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)-1-(2-oxa-6-azaspiro[3.3] heptan-6-yl)ethan-1-one

2-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)-1-(2-oxa-6-azaspiro[3.3]heptan-6-yl)ethan-1-one was prepared according to the general process described in Example 466, using 2-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)acetic acid (30 mg, 0.058 mmol) and 2-oxa-6-azaspiro[3.3]heptane (5.70 mg, 0.058 mmol) as starting intermediates to get the title compound as a white solid. LCMS Retention time: 1.38 min [E], MS (E⁺) m/z: 603.3 [M+H]. ¹H NMR (400 MHz, DMSO-d₆) δ ppm 13.97-13.75 (m, 1H), 8.95 (d, J=1.2 Hz, 1H), 8.55 (s, 1H), 7.73 (s, 1H), 7.31 (d, J=1.2 Hz, 1H), 4.72-4.62 (m, 4H), 4.38(s, 2H), 4.34-4.24 (m, 2H), 4.07 (s, 3H), 4.05-3.98 (m, 2H), 2.98 (s, 2H), 2.95-2.83 (m, 4H), 2.21-2.11 (m, 2H), 2.02-1.94 (m, 2H), 1.91 (s, 1H), 1.76 -1.63 (m, 2H).

The following Examples were prepared according to the general procedure used to prepare Example 1207.

**TABLE 109**

| Ex. No. | Structure | LCMS MH⁺ | RT (min) | HPLC Method |
|---|---|---|---|---|
| 1208 | 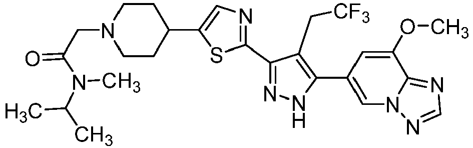 | 577.2 | 1.76 | C |
| 1209 | 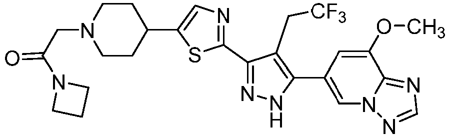 | 561.2 | 1.46 | C |

### EXAMPLE 1210

### 1-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)-2-morpholinoethan-1-one

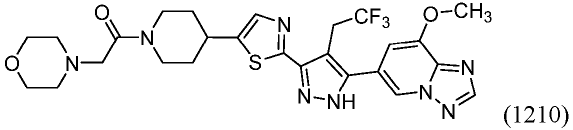

### Intermediate 1210A: 2-chloro-1-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)ethan-1-one

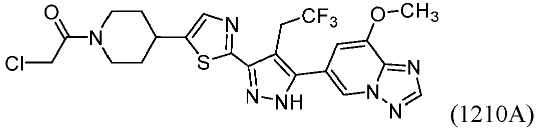

To a stirred solution of 2-(5-(8-methoxy-[1,2,4]triazolo[1,5-a]pyridin-6-yl)-4-(2,2,2-trifluoroethyl)-1H-pyrazol-3-yl)-5-(piperidin-4-yl)thiazole (230 mg, 0.496 mmol) in DCM (10 mL) were added TEA (0.208 mL, 1.489 mmol) and 2-chloroacetyl chloride (0.048 mL, 0.595 mmol) at room temperature. The reaction mixture was stirred for 16 h. The reaction mass diluted with water extracted with DCM, dried over sodium sulphate and concentrated to get 2-chloro-1-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)ethan-1-one (200 mg, 0.370 mmol, 74% yield). LCMS Retention time 1.42 min [A], MS (E⁺) m/z: 541.2 [M+H].

### Example 1210: 1-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)-2-morpholinoethan-1-one

To a stirred solution of 2-chloro-1-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)ethan-1-one (30 mg, 0.056 mmol) in THF (1 mL) and DMF (1 mL) solvent mixture were added TEA (7.74 µl, 0.056 mmol) and morpholine (4.84 mg, 0.056 mmol) at room temperature. The reaction mixture was stirred for 16 h. The reaction mass purified by Prep LCMS using method AB, the fractions containing the product were combined and dried via centrifugal evaporation to get 1-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)-2-morpholinoethan-1-one as a pale solid. LCMS Retention time 1.42 min [E], MS (E⁺) m/z: 591.2 [M+H]; ¹H NMR (400 MHz, DMSO-d₆) δ ppm 13.77-13.93 (m, 1 H) 9.88-10.11 (m, 1 H) 8.94 (br d, J=0.73 Hz, 1 H) 8.49-8.61 (m, 1 H) 7.68-7.80 (m, 1 H)7.29 (s, 1 H) 6.86-7.24 (m, 1 H) 4.20-4.53 (m, 5 H) 4.07 (s, 3 H) 3.89-4.01 (m, 2 H) 3.70-3.86 (m, 3 H) 3.22-3.30 (m, 3 H) 3.01-3.18 (m, 3 H) 2.86(br t, J=12.47 Hz, 1 H) 2.07-2.20 (m, 2 H) 1.62-1.76 (m, 1 H) 1.46-1.59 (m, 1 H) 1.21-1.29 (m, 1 H) 1.11-1.20 (m, 1 H).

The following Examples were prepared according to the general procedure used to prepare Example 1210.

**TABLE 110**

| Ex. No. | Structure | LCMS MH⁺ | RT (min) | HPLC Method |
|---|---|---|---|---|
| 1211 | 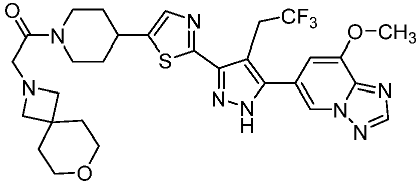 | 631.2 | 1.56 | C |
| 1212 | 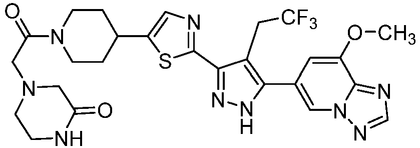 | 604.2 | 1.52 | C |
| 1213 | 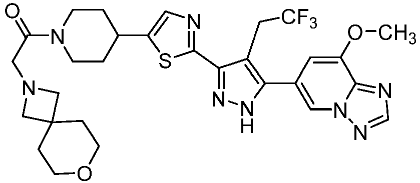 | 591.2 | 1.3 | C |
| 1214 | 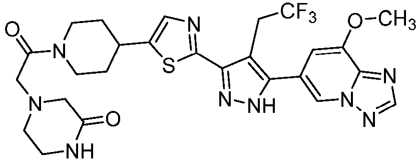 | 593.3 | 1.4 | C |

### EXAMPLE 1215

### 4-(4-(2-(5-(8-methoxy-[1,2,4]triazolo[1,5-a]pyridin-6-yl)-4-(2,2,2-trifluoroethyl)-1H-pyrazol-3-yl)-4-methylthiazol-5-yl)cyclohexyl)morpholine

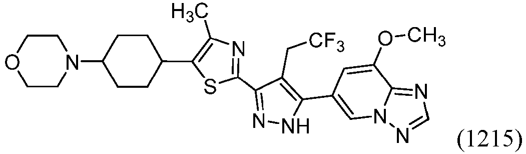

### Intermediate 1215A: 2-(5-(8-methoxy-[1,2,4]triazolo[1,5-a]pyridin-6-yl)-4-(2,2,2-trifluoroethyl)-1-((2-(trimethylsilyl)ethoxy)methyl)-1H-pyrazol-3-yl)-4-methyl-5-(1,4-dioxaspiro[4.5]decan-8-yl)thiazole

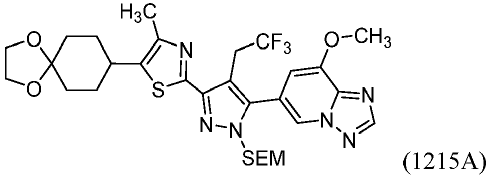

2-(5-(8-methoxy-[1,2,4]triazolo[1,5-a]pyridin-6-yl)-4-(2,2,2-trifluoroethyl)-1-((2-(trimethylsilyl)ethoxy)methyl)-1H-pyrazol-3-yl)-4-methyl-5-(1,4-dioxaspiro[4.5]decan-8-yl)thiazole (1.2 g, 1.805 mmol, 66% yield) was prepared according to the general process described in intermediate 1141B using 8-methoxy-6-(3-(4,4,5,5-tetramethyl-1,3,2-dioxaborolan-2-yl)-4-(2,2,2-trifluoroethyl)-1-((2-(trimethylsilyl)ethoxy)methyl)-1H-pyrazol-5-yl)-[1,2,4]triazolo[1,5-a]pyridine (1.5 g, 2.71 mmol) and 2-bromo-4-methyl-5-(1,4-dioxaspiro[4.5]decan-8-yl)thiazole (1.035 g, 3.25 mmol) as starting intermediates to get the title compound as a brown liquid. LCMS Retention time: 1.40 [A], MS (E⁺) m/z: 665.5 [M+H].

### Intermediate 1215B: 4-(2-(5-(8-methoxy-[1,2,4]triazolo[1,5-a]pyridin-6-yl)-4-(2,2,2-trifluoroethyl)-1H-pyrazol-3-yl)-4-methylthiazol-5-yl)cyclohexan-1-one

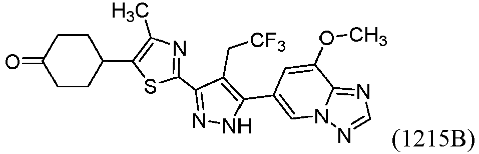

4-(2-(5-(8-methoxy-[1,2,4]triazolo[1,5-a]pyridin-6-yl)-4-(2,2,2-trifluoroethyl)-1H-pyrazol-3-yl)-4-methylthiazol-5-yl)cyclohexan-1-one (340 mg, 0.693 mmol, 92 % yield) was prepared according to the general process described in Intermediate 307D, using 2-(5-(8-methoxy-[1,2,4]triazolo[1,5-a]pyridin-6-yl)-4-(2,2,2-trifluoroethyl)-1-((2-(trimethylsilyl) ethoxy)methyl)-1H-pyrazol-3-yl)-4-methyl-5-(1,4-dioxaspiro[4.5]decan-8-yl)thiazole (500 mg, 0.752 mmol) as a starting intermediate to get the title compound as a white solid. LCMS Retention time: 2.21 min [A], MS (E⁺) m/z: 491 [M+H].

### Example 1215: 4-(4-(2-(5-(8-methoxy-[1,2,4]triazolo[1,5-a]pyridin-6-yl)-4-(2,2,2-trifluoroethyl)-1H-pyrazol-3-yl)-4-methylthiazol-5-yl)cyclohexyl)morpholine

4-(4-(2-(5-(8-methoxy-[1,2,4]triazolo[1,5-a]pyridin-6-yl)-4-(2,2,2-trifluoroethyl)-1H-pyrazol-3-yl)-4-methylthiazol-5-yl)cyclohexyl)morpholine (mixture of isomers) was prepared according to the general process described in Example 1083, using 4-(2-(5-(8-methoxy-[1,2,4]triazolo[1,5-a]pyridin-6-yl)-4-(2,2,2-trifluoroethyl)-1H-pyrazol-3-yl)-4-methylthiazol-5-yl)cyclohexan-1-one (80 mg, 0.163 mmol) as a starting intermediate to get the title compounds as a white solid.

Example 1215A: Isomer 1: LCMS Retention time: 1.61 min [E], MS (E⁺) m/z: 562.2 [M+H]; ¹H NMR (400 MHz, DMSO-d₆) δ ppm 13.90-13.72 (m, 1H), 9.03-8.87 (m, 1H), 8.55 (s, 1H), 7.30 (d, J=1.0 Hz, 1H), 4.40-4.21 (m, 2H), 4.15-3.99 (m, 3H),3.74-3.52 (m, 4H), 3.13-3.02 (m, 1H), 2.46-2.27 (m, 6H), 2.20 (br d, J=2.0 Hz, 1H), 2.05-1.93 (m, 2H), 1.83-1.62 (m, 4H), 1.62-1.45 (m, 2H).

Example 1215B: Isomer 2: LCMS Retention time: 1.89 min [E], MS (E⁺) m/z: 562.2 [M+H]; ¹H NMR (400 MHz, DMSO-d₆) δ = 13.96-13.73 (m, 1H), 8.95 (s, 1H), 8.55 (s, 1H), 7.30 (d, J=1.0 Hz, 1H), 4.39-4.23 (m, 2H), 4.13-4.01 (m, 3H), 3.70 -3.47 (m, 4H), 2.95-2.83 (m, 1H), 2.37 (s, 3H), 2.07-1.85 (m, 4H), 1.50-1.32 (m, 4H).

The following Examples were prepared according to the general procedure used to prepare Example 1215.

**TABLE 111**

| Ex. No. | Structure | LCMS MH⁺ | RT (min) | HPLC Method |
|---|---|---|---|---|
| 1216 | 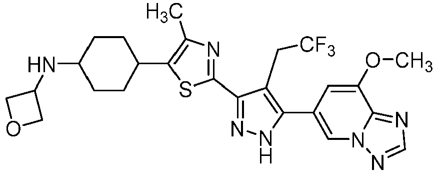 | 548.2 | 1.47 | C |
| 1217 | 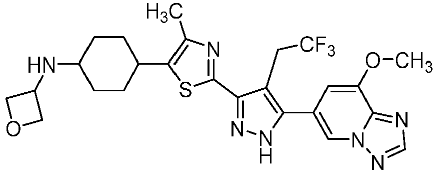 | 548.2 | 1.66 | C |
| 1218 | 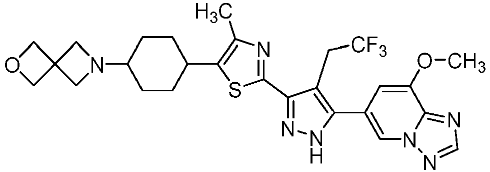 | 574.2 | 1.38 | C |
| 1219 | 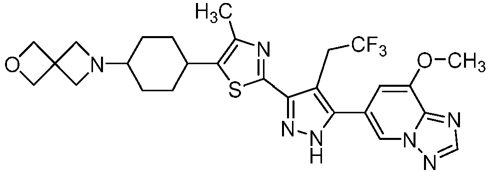 | 574.2 | 1.61 | E |
| 1220 | 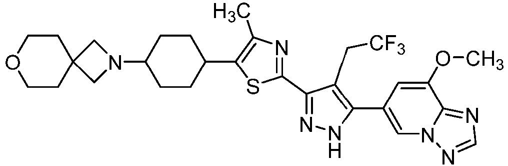 | 602.3 | 1.37 | C |
| 1221 | 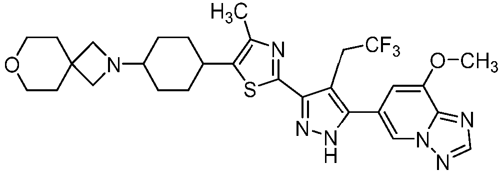 | 602.3 | 1.37 | C |
| 1222 | 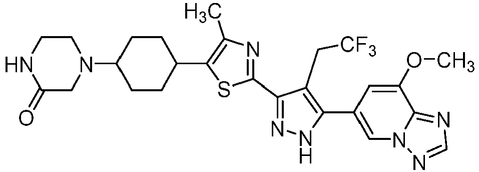 | 575.1 | 1.71 | C |
| 1223 | 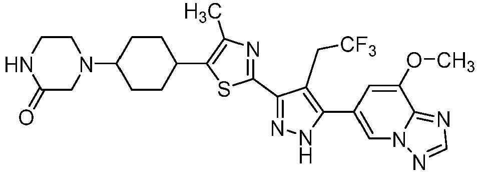 | 575.1 | 1.64 | E |
| 1224 | 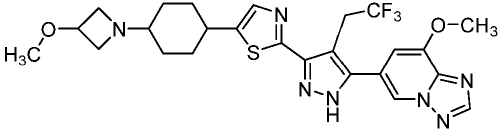 | 548.2 | 1.81 | E |
| 1225 | 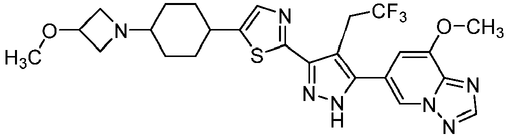 | 548.2 | 1.74 | E |
| 1226 | 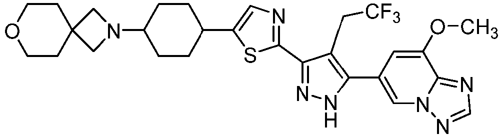 | 588.2 | 1.69 | E |
| 1227 | 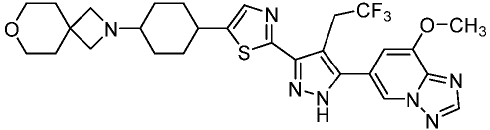 | 588.2 | 1.69 | C |
| 1228 | 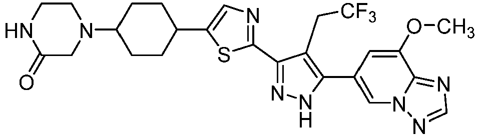 | 561.1 | 1.75 | C |
| 1229 | 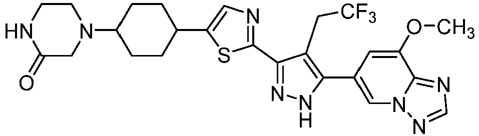 | 561.1 | 1.75 | C |
| 1230 | 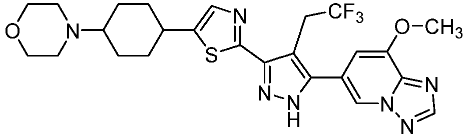 | 548.2 | 1.95 | C |
| 1231 | 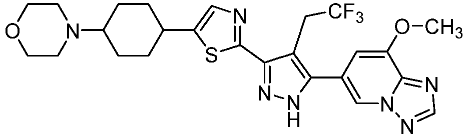 | 548.2 | 1.78 | C |

### EXAMPLE 1232

### 2-(5-(8-methoxy-[1,2,4]triazolo[1,5-a]pyridin-6-yl)-4-(2,2,2-trifluoroethyl)-1H-pyrazol-3-yl)-4-methyl-5-((1S,4S)-5-methyl-2,5-diazabicyclo[2.2.1]heptan-2-yl)thiazole

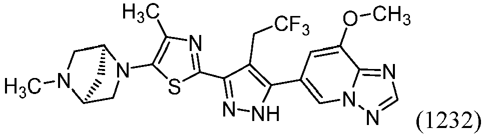

### Intermediate 1232A: tert-butyl (1S,4S)-5-(2-(5-(8-methoxy-[1,2,4]triazolo[1,5-a]pyridin-6-yl)-4-(2,2,2-trifluoroethyl)-1-((2-(trimethylsilyl)ethoxy)methyl)-1H-pyrazol-3-yl)-4-methylthiazol-5-yl)-2,5-diazabicyclo[2.2.1]heptane-2-carboxylate

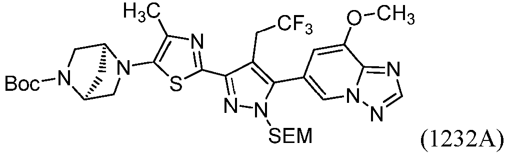

tert-butyl (1S,4S)-5-(2-(5-(8-methoxy-[1,2,4]triazolo[1,5-a]pyridin-6-yl)-4-(2,2,2-trifluoroethyl)-1-((2-(trimethylsilyl)ethoxy)methyl)-1H-pyrazol-3-yl)-4-methylthiazol-5-yl)-2,5-diazabicyclo[2.2.1]heptane-2-carboxylate (320 mg, 0.444 mmol, 49 % yield) was prepared according to the general process described in Intermediate 307B using 8-methoxy-6-(3-(4,4,5,5-tetramethyl-1,3,2-dioxaborolan-2-yl)-4-(2,2,2-trifluoroethyl)-1-((2-(trimethylsilyl)ethoxy)methyl)-1H-pyrazol-5-yl)-[1,2,4]triazolo[1,5-a]pyridine (500 mg, 0.903 mmol) and t-butyl (1S,4S)-5-(2-bromo-4-methylthiazol-5-yl)-2,5-diazabicyclo[2.2.1]heptane-2-carboxylate (406 mg, 1.084 mmol) as starting intermediates, to get the title compound as an off-white solid. LCMS Retention time: 2.38 [B], MS (E⁻) m/z: 721.6 [M+H].

### Example 1232B: 5-((1S,4S)-2,5-diazabicyclo[2.2.1]heptan-2-yl)-2-(5-(8-methoxy-[1,2,4]triazolo[1,5-a]pyridin-6-yl)-4-(2,2,2-trifluoroethyl)-1H-pyrazol-3-yl)-4-methylthiazole

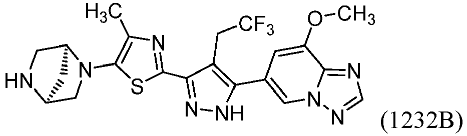

5-((1S,4S)-2,5-diazabicyclo[2.2.1]heptan-2-yl)-2-(5-(8-methoxy-[1,2,4]triazolo[1,5-a]pyridin-6-yl)-4-(2,2,2-trifluoroethyl)-1H-pyrazol-3-yl)-4-methylthiazole (1 mg, 0.126 mmol, 10 % yield) was prepared according to the general process described in Intermediate 307D using tert-butyl (1S,4S)-5-(2-(5-(8-methoxy-[1,2,4]triazolo[1,5-a]pyridin-6-yl)-4-(2,2,2-trifluoroethyl)-1-((2-(trimethylsilyl)ethoxy) methyl)-1H-pyrazol-3-yl)-4-methylthiazol-5-yl)-2,5-diazabicyclo [2.2.1]heptane-2-carboxylate (300 mg, 0.416 mmol) as a starting intermediate to get the title compound as a white solid. LCMS Retention time: 1.22 [E], MS (E⁻) m/z: 491.2 [H]; ¹H NMR (400 MHz, DMSO-d₆) δ ppm 13.71 (s, 1H), 8.92 (d, J=1.2 Hz, 1H), 8.54 (s, 1H), 7.28 (d, J=1.2 Hz, 1H), 4.32-4.23 (m, 2H), 4.12-4.03 (m, 8H), 3.53(br d, J=2.2 Hz, 1H), 3.17 (d, J=5.1 Hz, 2H), 3.06 (td, J=3.5, 5.1 Hz, 2H).

### Example 1232: 2-(5-(8-methoxy-[1,2,4]triazolo[1,5-a]pyridin-6-yl)-4-(2,2,2-trifluoroethyl)-1H-pyrazol-3-yl)-4-methyl-5-((1S,4S)-5-methyl-2,5-diazabicyclo[2.2.1] heptan-2-yl)thiazole

2-(5-(8-methoxy-[1,2,4]triazolo[1,5-a]pyridin-6-yl)-4-(2,2,2-trifluoroethyl)-1H-pyrazol-3-yl)-4-methyl-5-((1S,4S)-5-methyl-2,5-diazabicyclo[2.2.1]heptan-2-yl)thiazole (3.6mg, 0.126 mmol, 6.74 % yield) was prepared according to the general process described in Example 307 using 5-((1S,4S)-2,5-diazabicyclo[2.2.1]heptan-2-yl)-4-methyl-2-(5-(8-methyl-[1,2,4]triazolo[1,5-a]pyridin-6-yl)-4-(2,2,2-trifluoroethyl)-1H-pyrazol-3-yl)thiazole (50 mg, 0.105 mmol) as a starting intermediate to get the title compound as an off-white solid. LCMS Retention time: 1.29 [E], MS (E⁻) m/z: 505.2 [M+H]; ¹H NMR (400 MHz, DMSO-d₆) δ = 13.79 (s, 1H), 10.04-9.84 (m, 1H), 8.94 (s, 1H), 8.55 (s, 1H), 7.36-7.23 (m, 2H), 7.14 (s, 1H), 7.01 (s, 1H), 4.44-4.24(m, 3H), 4.18 (br d, J=2.0 Hz, 1H), 4.12-4.02 (m, 4H), 3.87-3.74 (m, 1H), 3.60-3.50 (m, 1H), 3.48-3.42 (m, 1H), 3.13-3.02 (m, 1H), 2.99-2.81 (m, 3H), 2.39-2.28 (m, 4H), 2.22-2.12 (m, 1H).

The following Examples were prepared according to the general procedure used to prepare Example 1232B.

**TABLE 112**

| Ex. No. | Structure | LCMS MH⁺ | RT (min) | HPLC Method |
|---|---|---|---|---|
| 1233 | 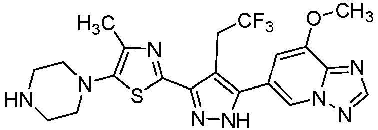 | 479.2 | 1.24 | C |
| 1234 | 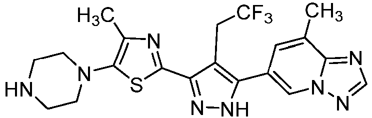 | 463.2 | 1.32 | C |
| 1235 | 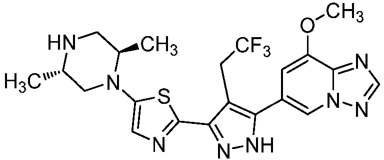 | 493.2 | 1.22 | C |
| 1236 | 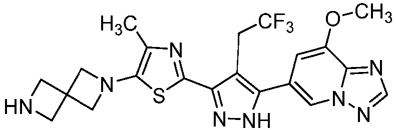 | 491.3 | 1.17 | C |

The following Examples were prepared according to the general procedure used to prepare Example 1232.

**TABLE 113**

| Ex. No. | Structure | LCMS MH⁺ | RT (min) | HPLC Method |
|---|---|---|---|---|
| 1237 | 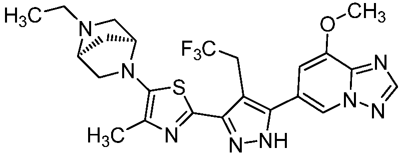 | 519.2 | 1.35 | C |
| 1238 | 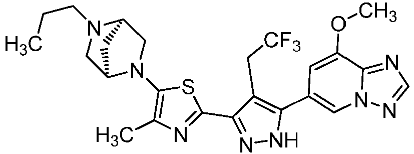 | 533.2 | 1.59 | C |
| 1239 | 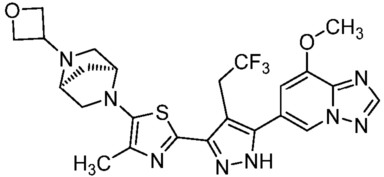 | 547.2 | 1.56 | C |
| 1240 | 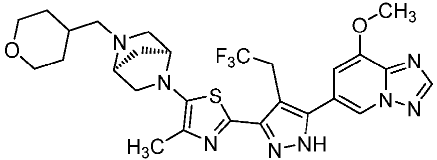 | 589.3 | 1.66 | C |
| 1241 | 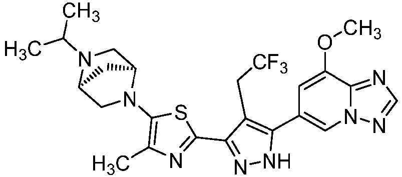 | 533.2 | 1.47 | C |
| 1242 | 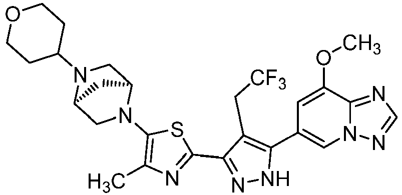 | 575.2 | 1.51 | C |
| 1243 | 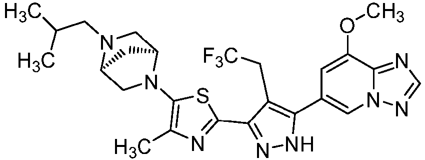 | 547.2 | 1.84 | C |
| 1244 | 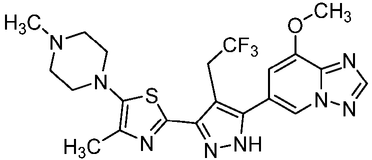 | 493.2 | 1.55 | C |
| 1245 | 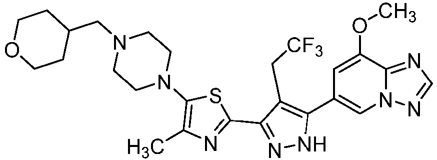 | 577.2 | 1.96 | C |
| 1246 | 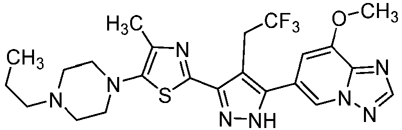 | 521.2 | 1.92 | E |
| 1247 | 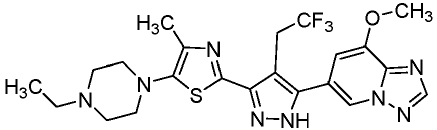 | 507.2 | 1.67 | E |
| 1248 | 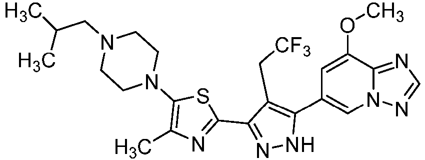 | 535.2 | 2.7 | E |
| 1249 | 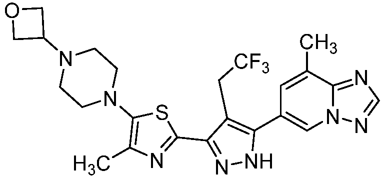 | 519.2 | 1.47 | E |
| 1250 | 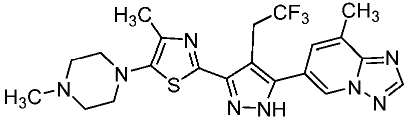 | 477.2 | 1.93 | E |
| 1251 | 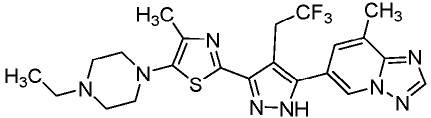 | 491.2 | 1.94 | C |
| 1252 | 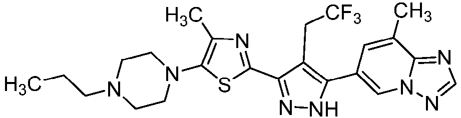 | 505.2 | 1.28 | D |
| 1253 | 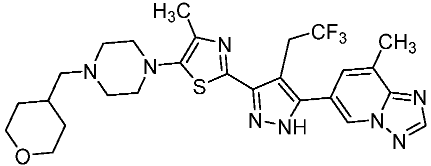 | 561.2 | 1.94 | C |
| 1254 | 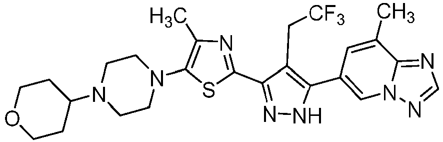 | 547.2 | 1.7 | C |
| 1256 | 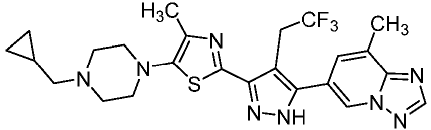 | 517.2 | 1.9 | C |
| 1257 | 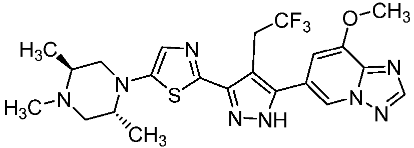 | 507.2 | 1.54 | C |
| 1258 | 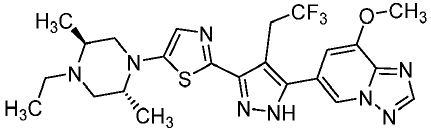 | 521.2 | 1.66 | C |
| 1259 | 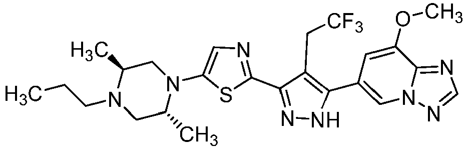 | 535.5 | 1.95 | C |
| 1260 | 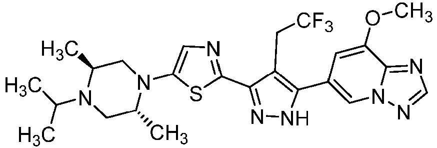 | 535.2 | 1.72 | C |
| 1261 | 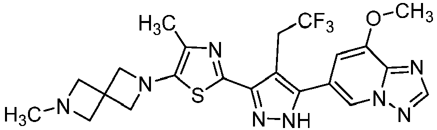 | 505.2 | 1.2 | C |
| 1262 | 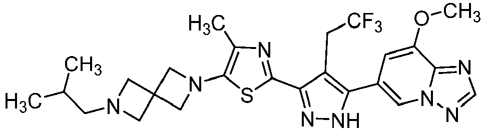 | 547.2 | 1.57 | C |
| 1263 | 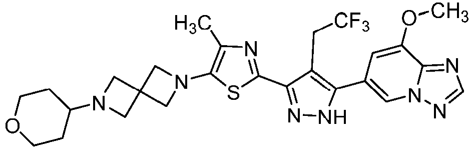 | 575.2 | 1.48 | C |
| 1264 | 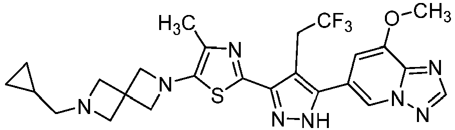 | 545.2 | 1.41 | C |
| 1265 | 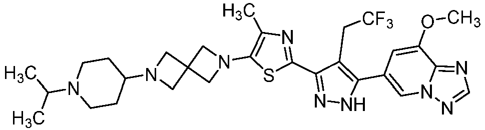 | 616.2 | 1.34 | C |
| 1266 | 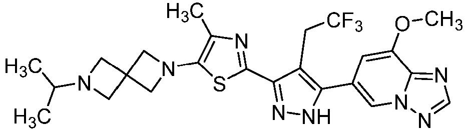 | 533.2 | 1.36 | C |
| 1267 | 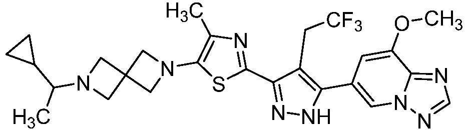 | 559.2 | 1.59 | C |
| 1268 | 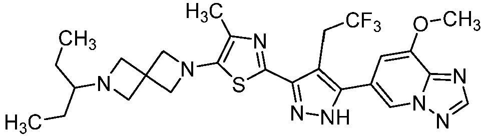 | 561.2 | 1.62 | C |

### EXAMPLE 1269

### 1-(2-(4-isopropyl-5-(8-methoxy-[1,2,4]triazolo[1,5-a]pyridin-6-yl)-1H-pyrazol-3-yl) thiazol-5-yl)-N-(3-methyloxetan-3-yl)piperidin-4-amine

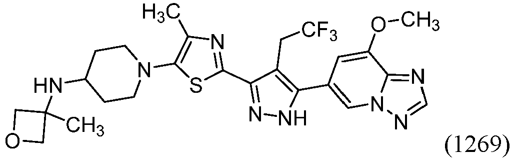

### Intermediate 1269A: 8-(2-(4-isopropyl-5-(8-methoxy-[1,2,4]triazolo[1,5-a]pyridin-6-yl)-1-((2-(trimethylsilyl)ethoxy)methyl)-1H-pyrazol-3-yl)thiazol-5-yl)-1,4-dioxa-8-azaspiro [4.5]decane

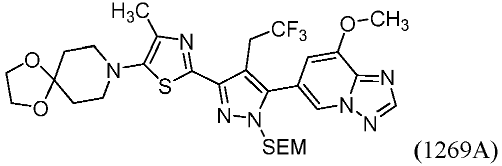

8-(2-(4-isopropyl-5-(8-methoxy-[1,2,4]triazolo[1,5-a]pyridin-6-yl)-1-((2-(trimethylsilyl)ethoxy)methyl)-1H-pyrazol-3-yl)thiazol-5-yl)-1,4-dioxa-8-azaspiro[4.5]decane (500 mg, 0.817 mmol, 52% yield) was prepared according to the general process described in Intermediate 307B, using 6-(4-isopropyl-3-(4,4,5,5-tetramethyl-1,3,2-dioxaborolan-2-yl)-1-((2-(trimethylsilyl)ethoxy) methyl)-1H-pyrazol-5-yl)-8-methoxy-[1,2,4]triazolo[1,5-a]pyridine (800 mg, 1.558 mmol) and 8-(2-bromothiazol-5-yl)-1,4-dioxa-8-azaspiro[4.5]decane (475 mg, 1.558 mmol) as starting intermediates to get the title compound as an off-white solid. LCMS Retention time: 1.40 [A], MS (E⁻) m/z: 665.5 [M+H].

### Intermediate 1269B: 1-(2-(4-isopropyl-5-(8-methoxy-[1,2,4]triazolo[1,5-a]pyridin-6-yl)-1H-pyrazol-3-yl)thiazol-5-yl)piperidin-4-one

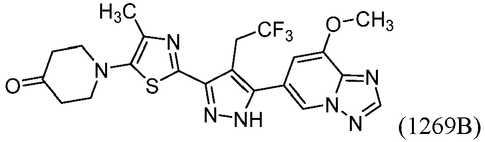

1-(2-(4-isopropyl-5-(8-methoxy-[1,2,4]triazolo[1,5-a]pyridin-6-yl)-1H-pyrazol-3-yl)thiazol-5-yl)piperidin-4-one (15 mg, 0.034 mmol, 69.9 % yield) was prepared according to the general process described in Intermediate 307D using 8-(2-(4-isopropyl-5-(8-methoxy-[1,2,4]triazolo[1,5-a]pyridin-6-yl)-1-((2-(trimethylsilyl)ethoxy)methyl)-1H-pyrazol-3-yl)thiazol-5-yl)-1,4-dioxa-8-azaspiro[4.5]decane (30 mg, 0.049 mmol) as a starting intermediate to get the title compound as an off-white solid. LCMS Retention time: 2.22 [B], MS (E⁻) m/z: 491.0 [M+H].

### Example 1269: 1-(2-(4-isopropyl-5-(8-methoxy-[1,2,4]triazolo[1,5-a]pyridin-6-yl)-1H-pyrazol-3-yl)thiazol-5-yl)-N-(3-methyloxetan-3-yl)piperidin-4-amine

1-(2-(4-isopropyl-5-(8-methoxy-[1,2,4]triazolo[1,5-a]pyridin-6-yl)-1H-pyrazol-3-yl)thiazol-5-yl)-N-(3-methyloxetan-3-yl)piperidin-4-amine (0.9 mg, 0.126 mmol, 3.57 % yield) was prepared according to the general process described in Example 1083 using 1-(2-(4-isopropyl-5-(8-methoxy-[1,2,4]triazolo[1,5-a]pyridin-6-yl)-1H-pyrazol-3-yl)thiazol-5-yl)piperidin-4-one (25 mg, 0.057 mmol) as a starting intermediate to get the title compound as an off-white solid. LCMS Retention time: 1.1 [F], MS (E⁻) m/z: 509.2 [M+H]; ¹H NMR (400 MHz, DMSO-d₆) δ 13.24 (s, 1H), 9.27 (br. s., 1H), 8.67 (s, 1H), 8.54 (s, 1H), 8.48(s, 1H), 7.22-6.98 (m, 2H), 4.73 (br. s., 2H), 4.42 (br. s., 2H), 4.16-3.97 (m, 4H), 3.93 (s, 1H), 3.54 (br. s., 3H), 3.09-2.84 (m, 4H), 2.74 (s, 1H), 1.92 (s, 1H), 1.78 (d, J=18.8 Hz, 3H), 1.67 (d, J=15.4 Hz, 4H), 1.31 (d, J=7.1 Hz, 4H), 1.27-1.12 (m, 3H), 1.12-0.94 (m, 2H).

The following Examples were prepared according to the general procedure used to prepare Example 1269.

**TABLE 114**

| Ex. No. | Structure | LCMS MH⁺ | RT (min) | HPLC Method |
|---|---|---|---|---|
| 1270 | 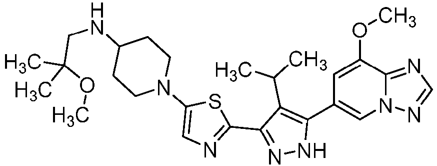 | 525.2 | 1.46 | C |
| 1271 | 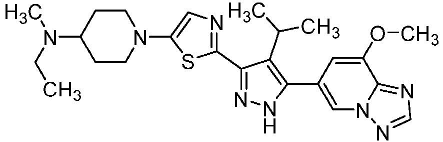 | 481.2 | 1.14 | C |

### EXAMPLE 1272

### 2-(5-(8-methoxy-[1,2,4]triazolo[1,5-a]pyridin-6-yl)-4-(2,2,2-trifluoroethyl)-1H-pyrazol-3-yl)-N,N-dimethyl-4,5,6,7-tetrahydrobenzo[d]thiazol-6-amine

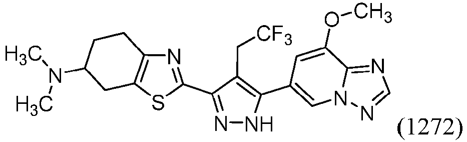

### Intermediate 1272A: tert-butyl (2-(5-(8-methoxy-[1,2,4]triazolo[1,5-a]pyridin-6-yl)-4-(2,2,2-trifluoroethyl)-1-((2-(trimethylsilyl)ethoxy)methyl)-1H-pyrazol-3-yl)-4,5,6,7-tetrahydrobenzo[d]thiazol-6-yl) (methyl)carbamate

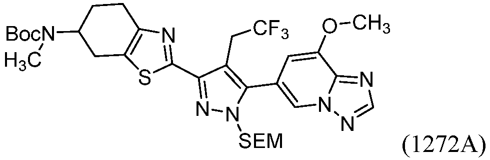

A solution of 8-methoxy-6-(3-(4,4,5,5-tetramethyl-1,3,2-dioxaborolan-2-yl)-4-(2,2,2-trifluoroethyl)-1-((2-(trimethylsilyl)ethoxy)methyl)-1H-pyrazol-5-yl)-[1,2,4]triazolo[1,5-a] pyridine (2.55 g, 1.382 mmol), tert-butyl (2-bromo-4,5,6,7-tetrahydrobenzo[d]thiazol-6-yl)(methyl)carbamate (0.400 g, 1.152 mmol) and K₂CO₃ (0.478 g, 3.46 mmol) in acetonitrile (40.00 mL) and water (10.00 mL) solvent mixture was degassed for 10 min with nitrogen. Next, Pd₂(dba)₃ (0.053 g, 0.058 mmol) and 2-dicyclohexylphosphino-2',6'-dimethoxybiphenyl (0.047 g, 0.115 mmol) were added. The reaction mixture was degassed for 2 min, and stirred at 110 °C for 16 h. The reaction mixture was brought to room temperature, the two layers were separated, the aqueous layer was extracted with EtOAc (2 X 20 mL), combined organic extracts were dried (Na₂SO₄) and concentrated to get crude compound. The crude compound was purified by ISCO using 40 g silica column, the compound was eluted in 55% EA in hexane, the fractions were collected and concentrated to get tert-butyl (2-(5-(8-methoxy-[1,2,4]triazolo[1,5-a]pyridin-6-yl)-4-(2,2,2-trifluoroethyl)-1-((2-(trimethylsilyl)ethoxy)methyl)-1H-pyrazol-3-yl)-4,5,6,7-tetrahydrobenzo[d]thiazol-6-yl) (methyl)carbamate (800 mg, 0.346 mmol, 30 % yield) as a gummy solid. LCMS Retention time: 1.53 min [A], MS (E⁺) m/z: 694.5 [M+H].

### Example 1272B: 2-(5-(8-methoxy-[1,2,4]triazolo[1,5-a]pyridin-6-yl)-4-(2,2,2-trifluoroethyl)-1H-pyrazol-3-yl)-N-methyl-4,5,6,7-tetrahydrobenzo[d]thiazol-6-amine

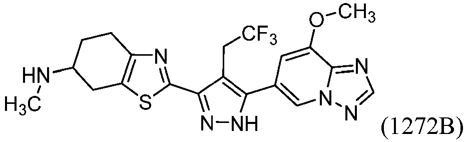

To a solution of tert-butyl (2-(5-(8-methoxy-[1,2,4]triazolo[1,5-a]pyridin-6-yl)-4-(2,2,2-trifluoroethyl)-1-((2-(trimethylsilyl)ethoxy)methyl)-1H-pyrazol-3-yl)-4,5,6,7-tetrahydro benzo[d]thiazol-6-yl)(methyl)carbamate (0.80 g, 0.346 mmol) in DCM (6.00 mL) was added TFA (3.00 ml, 38.9 mmol) at 0 °C, then the mixture was stirred at room temperature for 16 h. The reaction mass was concentrated and dried under vacuum, triturated with diethyl ether to get racemic 2-(5-(8-methoxy-[1,2,4]triazolo[1,5-a]pyridin-6-yl)-4-(2,2,2-trifluoroethyl)-1H-pyrazol-3-yl)-N-methyl-4,5,6,7-tetrahydrobenzo[d]thiazol-6-amine (0.117 g, 0.250 mmol, 72% yield) as a white solid. The racemic 2-(5-(8-methoxy-[1,2,4]triazolo[1,5-a]pyridin-6-yl)-4-(2,2,2-trifluoroethyl)-1H-pyrazol-3-yl)-N-methyl-4,5,6,7-tetrahydrobenzo[d]thiazol-6-amine (100 mg, 0.216 mmol) was purified by chiral SFC using method AF, to separate both the enantiomers. The desired fractions were collected, concentrated and lyophilized to get:
Example 1272Ba: Isomer 1. (Peak-1, Chiral SFC RT-5.77) 2-(5-(8-methoxy-[1,2,4]triazolo[1,5-a]pyridin-6-yl)-4-(2,2,2-trifluoroethyl)-1H-pyrazol-3-yl)-N-methyl-4,5,6,7-tetrahydrobenzo[d]thiazol-6-amine (42 mg, 0.090 mmol, 41% yield) as a white solid. LCMS Retention time: 1.383 min [B], MS (E⁺) m/z: 464.2 [M+H]; ¹H NMR (400 MHz, METHANOL-d₄) δ ppm 8.70 (s, 1H), 8.45 (s, 1H), 7.28 (s, 1H), 4.18-4.08 (m, 5 H), 3.26-3.20 (m, 1H), 3.04 2.96 (m, 3 H), 2.90-2.82 (m, 1 H), 2.69-2.62 (m, 1 H), 2.51 (s, 3H), 2.24-2.18 (m, 1 H), 1.82-1.75 (m, 1H); and
Example 1272Bb: Isomer 2. (Peak 2, Chiral SFC RT-19.44) 2-(5-(8-methoxy-[1,2,4]triazolo[1,5-a]pyridin-6-yl)-4-(2,2,2-trifluoroethyl)-1H-pyrazol-3-yl)-N-methyl-4,5,6,7-tetrahydrobenzo[d]thiazol-6-amine (42 mg, 0.090 mmol, 41% yield) as a white solid. LCMS Retention time: 1.391 min [B], MS (E⁺) m/z: 464.2 [M+H]; ¹H NMR (400 MHz, METHANOL-d₄) δ ppm 8.70 (s, 1 H), 8.46 (s, 1H), 7.27 (s, 1 H), 4.18-4.10 (m, 5 H), 3.28-3.24 (m, 1 H), 3.05-2.55 (m, 4 H), 2.67 (s, 3 H), 2.32 2.28 (m, 1H), 1.94-1.88 (m, 1H).

### Example 1272: 2-(5-(8-methoxy-[1,2,4]triazolo[1,5-a]pyridin-6-yl)-4-(2,2,2-trifluoro ethyl)-1H-pyrazol-3-yl)-N,N-dimethyl-4,5,6,7-tetrahydrobenzo[d]thiazol-6-amine

To a solution of 2-(5-(8-methoxy-[1,2,4]triazolo[1,5-a]pyridin-6-yl)-4-(2,2,2-trifluoroethyl)-1H-pyrazol-3-yl)-N-methyl-4,5,6,7-tetrahydrobenzo[d]thiazol-6-amine (10 mg, 0.022 mmol) in MeOH (2.00 mL) were added formaldehyde in water (0.3 mL, 3.27 mmol) and acetic acid (0.2 mL, 3.49 mmol) at room temperature, stirred for 6h, to this was then added sodium cyanoborohydride (4.07 mg, 0.065 mmol), stirred at the same temperature for 16h. The reaction mass was purified by Prep LCMS purification using method AA, the fractions containing product were combined and dried using Genevac centrifugal evaporator to get 2-(5-(8-methoxy-[1,2,4]triazolo[1,5-a]pyridin-6-yl)-4-(2,2,2-trifluoroethyl)-1H-pyrazol-3-yl)-N,N-dimethyl-4,5,6,7-tetrahydrobenzo[d]thiazol-6-amine (6.2 mg, 0.013 mmol, 60% yield) as a pale solid. LCMS Retention time: 1.318 min [E], MS (E+) m/z: 478.2 [M+H]; ¹H NMR (400 MHz, METHANOL-d4) δ ppm 8.68 (s, 1H), 8.44 (s, 1H), 7.25 (s, 1H), 4.23-4.02 (m, 4H), 3.22-3.00 (m, 3H), 2.98-2.79 (m, 2H), 2.57 (s, 5H), 2.29 (d, *J*=11.2 Hz, 1H), 1.99-1.81 (m, 2H), 1.29 (s, 1H).

The following Examples were prepared according to the general procedure used to prepare Example 1272.

**TABLE 115**

| Ex. No. | Structure | LCMS MH⁺ | RT (min) | HPLC Method |
|---|---|---|---|---|
| 1273 | 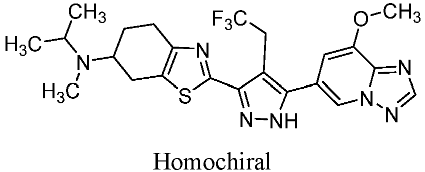 | 506.2 | 1.445 | E |
| 1274 | 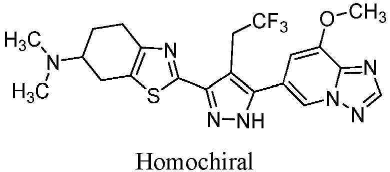 | 478.2 | 1.347 | E |

### EXAMPLE 1275

### 8-methoxy-6-(3-(4-methyl-5-(1-methylpiperidin-4-yl)pyridin-2-yl)-4-(2,2,2-trifluoroethyl)-1H-pyrazol-5-yl)-[1,2,4]triazolo[1,5-a]pyridine

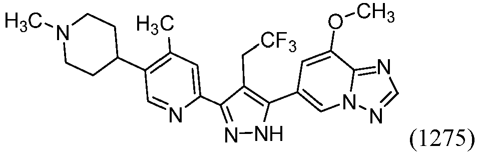

### Intermediate 1275A: tert-butyl 6-(5-(8-methoxy-[1,2,4]triazolo[1,5-a]pyridin-6-yl)-4-(2,2,2-trifluoroethyl)-1-((2-(trimethylsilyl)ethoxy)methyl)-1H-pyrazol-3-yl)-4-methyl-3',6'-dihydro-[3,4'-bipyridine]-1'(2'H)-carboxylate

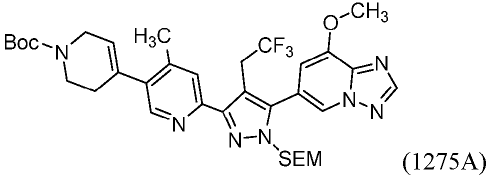

tert-Butyl 6-(5-(8-methoxy-[1,2,4]triazolo[1,5-a]pyridin-6-yl)-4-(2,2,2-trifluoroethyl)-1-((2-(trimethylsilyl)ethoxy)methyl)-1H-pyrazol-3-yl)-4-methyl-3',6'-dihydro-[3,4'-bipyridine]-1'(2'H)-carboxylate was prepared according to the general process described in Intermediate 307B, using 8-methoxy-6-(3-(4,4,5,5-tetramethyl-1,3,2-dioxaborolan-2-yl)-4-(2,2,2-trifluoroethyl)-1-((2-(trimethylsilyl) ethoxy)methyl)-1H-pyrazol-5-yl)-[1,2,4]triazolo[1,5-a]pyridine (1 g, 1.807 mmol) in acetonitrile (50 mL) and tert-butyl 6-chloro-4-methyl-3',6'-dihydro-[3,4'-bipyridine]-1'(2'H)-carboxylate (0.670 g, 2.168 mmol) as starting intermediates to get the title compound as a brown liquid. LCMS Retention time: 1.69 [A], MS (E⁺) m/z: 700.5 [M+H].

### Intermediate 1275B tert-butyl 4-(6-(5-(8-methoxy-[1,2,4]triazolo[1,5-a]pyridin-6-yl)-4-(2,2,2-trifluoroethyl)-1-((2-(trimethylsilyl)ethoxy)methyl)-1H-pyrazol-3-yl)-4-methyl pyridin-3-yl)piperidine-1-carboxylate

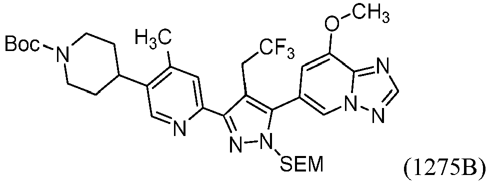

tert-Butyl 4-(6-(5-(8-methoxy-[1,2,4]triazolo[1,5-a]pyridin-6-yl)-4-(2,2,2-trifluoroethyl)-1-((2-(trimethylsilyl)ethoxy)methyl)-1H-pyrazol-3-yl)-4-methylpyridin-3-yl)piperidine-1-carboxylate (240 mg, 0.342 mmol, 36.8% yield) was prepared according to the general process described in Intermediate 307C using tert-butyl 6-(5-(8-methoxy-[1,2,4]triazolo[1,5-a]pyridin-6-yl)-4-(2,2,2-trifluoroethyl)-1-((2-(trimethylsilyl)ethoxy) methyl)-1H-pyrazol-3-yl)-4-methyl-3',6'-dihydro-[3,4'-bipyridine]-1'(2'H)-carboxylate (650 mg, 0.929 mmol) as a starting intermediate to get the title compound as an off-white solid. LCMS Retention time 1.59 min [A], MS (E⁺) m/z: 702.6 [M+H].

### Example 1275C: 8-methoxy-6-(3-(4-methyl-5-(piperidin-4-yl)pyridin-2-yl)-4-(2,2,2-trifluoroethyl)-1H-pyrazol-5-yl)-[1,2,4]triazolo[1,5-a]pyridine

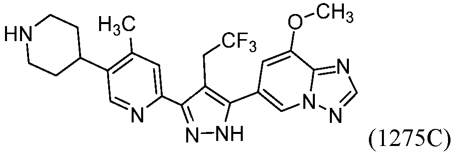

8-methoxy-6-(3-(4-methyl-5-(piperidin-4-yl)pyridin-2-yl)-4-(2,2,2-trifluoroethyl)-1H-pyrazol-5-yl)-[1,2,4]triazolo[1,5-a]pyridine was prepared according to the general process described in Intermediate 307D using tert-butyl 4-(6-(5-(8-methoxy-[1,2,4]triazolo[1,5-a]pyridin-6-yl)-4-(2,2,2-trifluoroethyl)-1-((2-(trimethylsilyl)ethoxy) methyl)-1H-pyrazol-3-yl)-4-methylpyridin-3-yl)piperidine-1-carboxylate (240 mg, 0.342 mmol) as a starting intermediate to get the title compound as an white solid. LCMS Retention time 1.09 min [E], MS (E⁺) m/z: 472.2 [M+H]; ¹H NMR (400 MHz, DMSO-d₆) δ 13.86 (br dd, J=3.06, 1.59 Hz, 1 H) 8.94 (d, J=0.98 Hz, 1 H) 8.55 (s, 1 H) 7.69 (s, 1 H) 7.30 (d, J=1.22 Hz, 1 H) 4.20- 4.37 (m, 2 H) 4.07 (s, 3 H) 3.53-3.70 (m, 4 H) 2.85-2.94 (m, 1 H) 2.27-2.45 (m, 2 H) 2.07-2.18 (m, 2 H) 1.85-2.04 (m, 3 H) 1.31-1.60 (m, 4 H).

### Example 1275: 8-methoxy-6-(3-(4-methyl-5-(1-methylpiperidin-4-yl)pyridin-2-yl)-4-(2,2,2-trifluoroethyl)-1H-pyrazol-5-yl)-[1,2,4]triazolo[1,5-a]pyridine

8-methoxy-6-(3-(4-methyl-5-(1-methylpiperidin-4-yl)pyridin-2-yl)-4-(2,2,2-trifluoroethyl)-1H-pyrazol-5-yl)-[1,2,4]triazolo[1,5-a]pyridine was prepared according to the general process described in Example 307 using 8-methoxy-6-(3-(4-methyl-5-(piperidin-4-yl)pyridin-2-yl)-4-(2,2,2-trifluoroethyl)-1H-pyrazol-5-yl)-[1,2,4]triazolo[1,5-a]pyridine (15 mg, 0.032 mmol) as a starting intermediate to get the title compound as an off-white solid. LCMS Retention time 1.15 min [E], MS (E⁺) m/z: 486.2 [M+H]; ¹H NMR (400 MHz, DMSO-d₆) δ ppm 13.60-13.85 (m, 1 H) 8.87 (s, 1 H) 8.52 (s, 1 H) 8.40-8.50 (m, 1 H) 7.70-7.83 (m, 1 H) 7.30 (d, J=1.22 Hz, 1 H) 4.35-4.46 (m, 2 H) 4.00-4.14 (m, 3 H) 3.91-3.94 (m, 1 H) 3.03-3.11 (m, 2 H) 2.78-2.86 (m, 1 H) 2.20-2.44 (m, 7 H) 1.92 (s, 1 H) 1.71-1.88 (m, 4 H)1.15-1.28 (m, 1 H).

The following Example was prepared according to the general procedure used to prepare Example 1275C.

**TABLE 116**

| Ex. No. | Structure | LCMS MH⁺ | RT (min) | HPLC Method |
|---|---|---|---|---|
| 1276 | 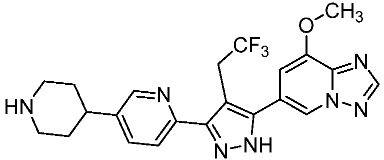 | 458.3 | 1.01 | C |

The following Examples were prepared according to the general procedure used to prepare Example 1275.

**TABLE 117**

| Ex. No. | Structure | LCMS MH⁺ | RT (min) | HPLC Method |
|---|---|---|---|---|
| 1277 | 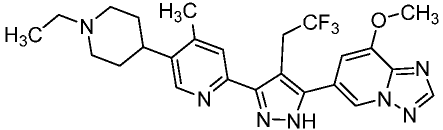 | 500.2 | 1.16 | C |
| 1278 | 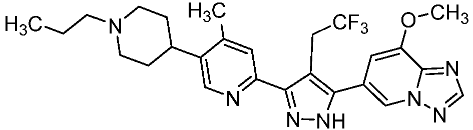 | 514.3 | 1.28 | C |
| 1279 | 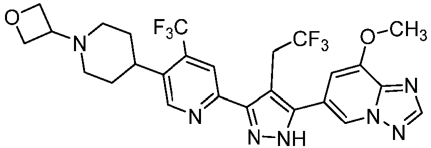 | 528.3 | 1.42 | C |
| 1280 | 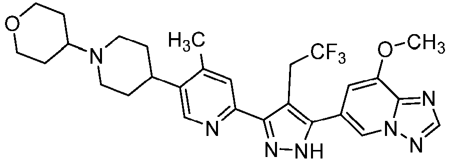 | 556.3 | 1.28 | C |
| 1281 | 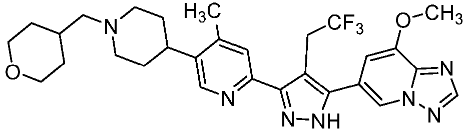 | 570.3 | 1.39 | C |
| 1282 | 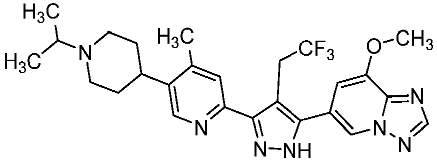 | 514.3 | 1.23 | C |
| 1283 | 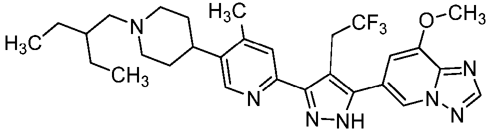 | 556.3 | 1.88 | C |
| 1284 | 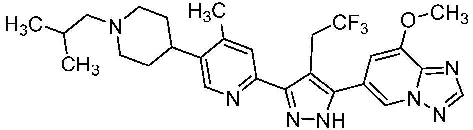 | 528.3 | 1.52 | C |
| 1285 | 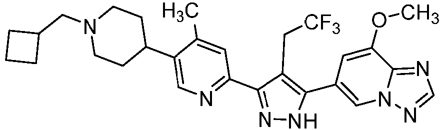 | 540.3 | 1.12 | D |
| 1286 | 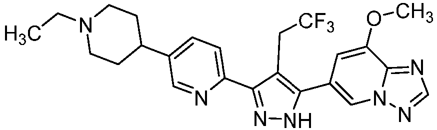 | 486.2 | 1.15 | C |
| 1287 | 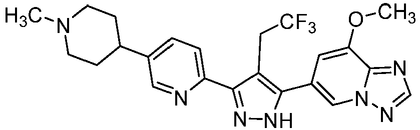 | 472.1 | 1.1 | C |
| 1288 | 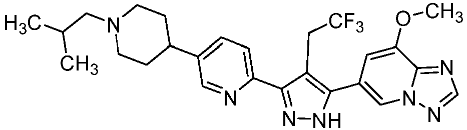 | 514.3 | 1.51 | C |
| 1289 | 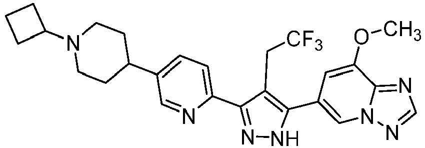 | 512.2 | 1.38 | C |
| 1290 | 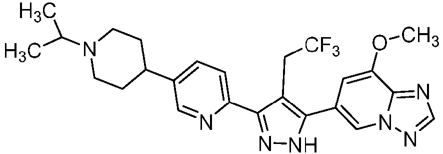 | 500.3 | 1.19 | C |
| 1291 | 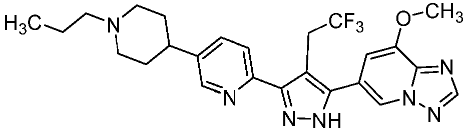 | 500.2 | 1.28 | C |
| 1292 | 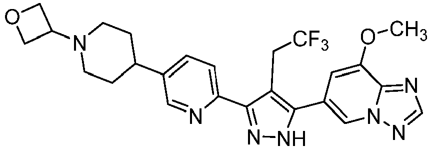 | 514.2 | 1.41 | C |
| 1293 | 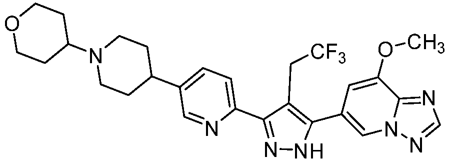 | 542.2 | 1.24 | C |
| 1294 | 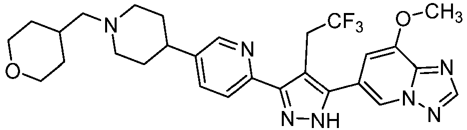 | 556.3 | 1.33 | C |
| 1295 | 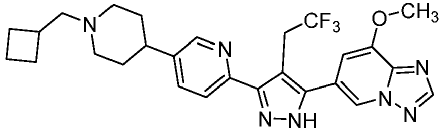 | 526.2 | 1.47 | C |

### EXAMPLE 1296

### 2-(dimethylamino)-1-(4-(6-(5-(8-methoxy-[1,2,4]triazolo[1,5-a]pyridin-6-yl)-4-(2,2,2-trifluoroethyl)-1H-pyrazol-3-yl)-4-methylpyridin-3-yl)piperidin-1-yl)ethan-1-one

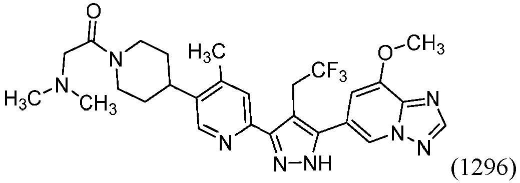

To a stirred solution of 8-methoxy-6-(3-(4-methyl-5-(piperidin-4-yl)pyridin-2-yl)-4-(2,2,2-trifluoroethyl)-1H-pyrazol-5-yl)-[1,2,4]triazolo[1,5-a]pyridine (15 mg, 0.032 mmol) in DMF (2 mL) were added dimethylglycine (3.94 mg, 0.038 mmol), Et₃N (0.013 mL, 0.095 mmol) and HATU (12.10 mg, 0.032 mmol) at room temperature. The reaction mixture was stirred for 16 h. The reaction mass concentrated to get crude product. The crude product was purified by Prep LCMS using method AA, fractions containing the product were combined and dried via centrifugal evaporation to get 2-(dimethylamino)-1-(4-(6-(5-(8-methoxy-[1,2,4]triazolo[1,5-a]pyridin-6-yl)-4-(2,2,2-trifluoroethyl)-1H-pyrazol-3-yl)-4-methylpyridin-3-yl)piperidin-1-yl)ethan-1-one as a pale solid. LCMS Retention time 1.19 min [E], MS (E⁺) m/z: 557.3 [M+H]; ¹H NMR (400 MHz, DMSO-d₆) δ ppm 13.46-13.94 (m, 1 H) 8.74-8.98 (m, 1 H) 8.28-8.64 (m, 2 H) 7.63-7.89 (m, 1 H) 7.30 (s, 1 H) 4.51-4.65 (m, 1 H)4.29-4.47 (m, 2 H) 4.12-4.21 (m, 1 H) 4.05-4.09 (m, 1 H) 4.07 (s, 2 H) 3.20-3.26 (m, 2 H) 2.98-3.18 (m, 3 H) 2.69 (br dd, J=6.48, 1.34 Hz, 1 H) 2.44(s, 3 H) 2.30 (s, 6 H) 1.92 (s, 1 H) 1.70-1.88 (m, 3 H) 1.51-1.64 (m, 1 H).

The following Examples were prepared according to the general procedure used to prepare Example 1296.

**TABLE 118**

| Ex. No. | Structure | LCMS MH⁺ | Ret Time (min) | HPLC Method |
|---|---|---|---|---|
| 1297 | 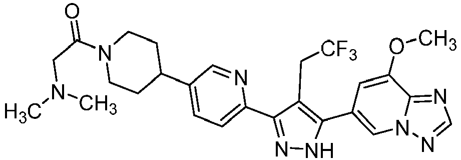 | 543.2 | 1.19 | C |
| 1298 | 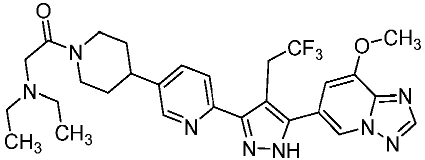 | 571.3 | 1.26 | C |

### EXAMPLE 1299

### 2-(4-(6-(5-(8-methoxy-[1,2,4]triazolo[1,5-a]pyridin-6-yl)-4-(2,2,2-trifluoroethyl)-1H-pyrazol-3-yl)-4-methylpyridin-3-yl)piperidin-1-yl)-N,N-dimethylacetamide

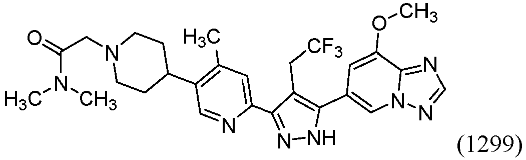

2-(4-(6-(5-(8-methoxy-[1,2,4]triazolo[1,5-a]pyridin-6-yl)-4-(2,2,2-trifluoroethyl)-1H-pyrazol-3-yl)-4-methylpyridin-3-yl)piperidin-1-yl)-N,N-dimethylacetamide was prepared according to the general process described in Example 1136 using 8-methoxy-6-(3-(4-methyl-5-(piperidin-4-yl)pyridin-2-yl)-4-(2,2,2-trifluoroethyl)-1H-pyrazol-5-yl)-[1,2,4]triazolo[1,5-a]pyridine (15 mg, 0.032 mmol) as a starting intermediate to get the title compound as an off-white solid. LCMS Retention time 0.93 min [E], MS (E⁺) m/z: 557.3 [M+H]; ¹H NMR (400 MHz, DMSO-d₆) δ ppm 13.86 (br dd, J=3.06, 1.59 Hz, 1 H) 8.94 (d, J=0.98 Hz, 1 H) 8.55 (s, 1 H) 7.69 (s, 1 H) 7.30 (d, J=1.22 Hz, 1 H) 4.20- 4.37 (m, 2 H) 4.07 (s, 3 H) 3.53-3.70 (m, 4 H) 2.85-2.94 (m, 1 H) 2.27-2.45 (m, 2 H) 2.07-2.18 (m, 2 H) 1.85-2.04 (m, 3 H) 1.31-1.60 (m, 4 H).

The following Examples were prepared according to the general procedure used to prepare Example 1299.

**TABLE 119**

| Ex. No. | Structure | LCMS MH⁺ | RT (min) | HPLC Method |
|---|---|---|---|---|
| 1300 | 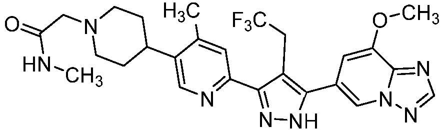 | 543.3 | 1.37 | C |
| 1301 | 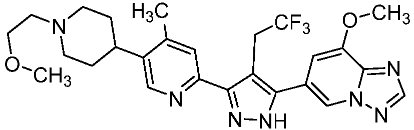 | 530.3 | 1.2 | C |
| 1302 | 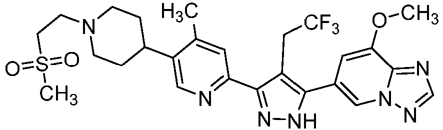 | 578.3 | 1.4 | C |

### EXAMPLE 1303

### N-(2-(4-(5-(8-methyl-[1,2,4]triazolo[1,5-a]pyridin-6-yl)-4-(2,2,2-trifluoroethyl)-1H-pyrazol-3-yl)phenyl)propan-2-yl)tetrahydro-2H-pyran-4-amine

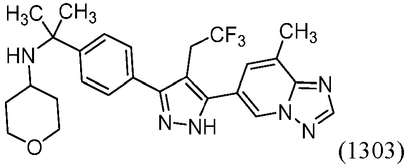

### Intermediate 1303A: tert-butyl (2-(4-(5-(8-methyl-[1,2,4]triazolo[1,5-a]pyridin-6-yl)-4-(2,2,2-trifluoroethyl)-1-((2-(trimethylsilyl)ethoxy)methyl)-1H-pyrazol-3-yl)phenyl) propan-2-yl)carbamate

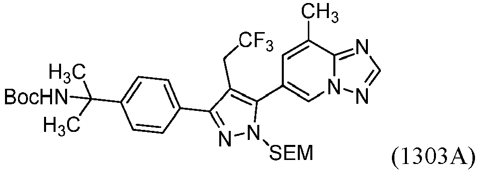

A solution of 6-(3-bromo-4-(2,2,2-trifluoroethyl)-1-((2-(trimethylsilyl)ethoxy) methyl)-1H-pyrazol-5-yl)-8-methyl-[1,2,4]triazolo[1,5-a]pyridine (180 mg, 0.367 mmol), tert-butyl (2-(4-(4,4,5,5-tetramethyl-1,3,2-dioxaborolan-2-yl)phenyl)propan-2-yl) carbamate (159 mg, 0.440 mmol) and K₂CO₃ (152 mg, 1.101 mmol) in acetonitrile (12.00 mL) and water (3.00 mL) solvent mixture was degassed for 10 min with nitrogen, Next, Pd₂(dba)₃ (16.81 mg, 0.018 mmol) and 2-dicyclohexylphosphino-2',6'-dimethoxybiphenyl (15.07 mg, 0.037 mmol) were added. The reaction mixture was degassed for 2 min., and stirred at 110 °C for 16 h. The reaction mixture was brought to room temperature, separated both the layers, the aqueous layer was extracted with DCM (2 X 20 mL), the combined organic extracts were dried (Na₂SO₄) and concentrated to get crude compound. The crude compound was purified by ISCO using 24 g silica column, the compound was eluted in 50% EA in hexane, the fractions were collected and concentrated to get tert-butyl (2-(4-(5-(8-methyl-[1,2,4]triazolo[1,5-a]pyridin-6-yl)-4-(2,2,2-trifluoroethyl)-1-((2-(trimethylsilyl)ethoxy) methyl)-1H-pyrazol-3-yl)phenyl)propan-2-yl)carbamate (180 mg, 0.279 mmol, 76 % yield) as an off-white solid. LCMS Retention time 1.38 min [B], MS (E⁺) m/z: 645.6 [M+H].

### Intermediate 1303B: 2-(4-(5-(8-methyl-[1,2,4]triazolo[1,5-a]pyridin-6-yl)-4-(2,2,2-trifluoroethyl)-1H-pyrazol-3-yl)phenyl)propan-2-amine

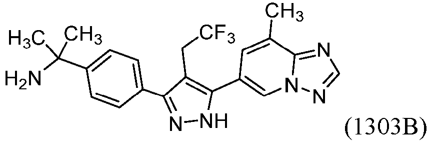

To a solution of tert-butyl (2-(4-(5-(8-methyl-[1,2,4]triazolo[1,5-a]pyridin-6-yl)-4-(2,2,2-trifluoroethyl)-1-((2-(trimethylsilyl)ethoxy)methyl)-1H-pyrazol-3-yl)phenyl) propan-2-yl)carbamate (180 mg, 0.279 mmol) in DCM (3.00 mL) was added TFA (1.5 mL, 19.47 mmol) at room temperature. The reaction mixture was stirred at the same temperature for 16 h. The reaction mass was concentrated to get crude compound, the crude was triturated with diethyl ether to get 2-(4-(5-(8-methyl-[1,2,4]triazolo[1,5-a] pyridin-6-yl)-4-(2,2,2-trifluoroethyl)-1H-pyrazol-3-yl)phenyl)propan-2-amine, TFA (117 mg, 0.221 mmol, 79% yield) as a white solid. LCMS Retention time 1.061 min [F], MS (E⁺) m/z: 415.2 [M+H]; ¹H NMR (400 MHz, METHANOL-d₄) δ ppm 8.90 (s, 1H), 8.49 (s, 1H), 7.82-7.62 (m, 5H), 3.71 (q, *J*=10.5 Hz, 2H), 3.06 (q, *J*=7.3 Hz, 1H), 2.82-2.62 (m, 3H), 2.00-1.90 (m, 2H), 1.77 (s, 6H), 1.38-1.24 (m, 2H).

The following Intermediates were prepared according to the general procedure used to prepare Intermediate 1303B.

**TABLE 120**

| Interm. No. | Structure | LCMS MH⁺ | RT (min) | HPLC Method |
|---|---|---|---|---|
| 1304B | 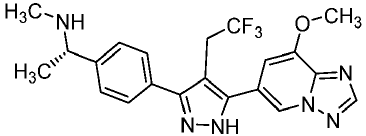 | 431.2 | 1.00 | C |
| 1305B | 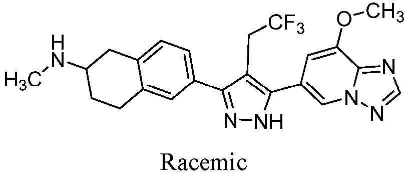 | 457.2 | 1.08 | C |
| 1306B | 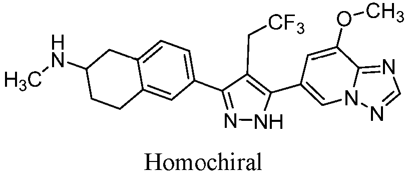 | 457.3 | 0.94 | A |
| 1307B | 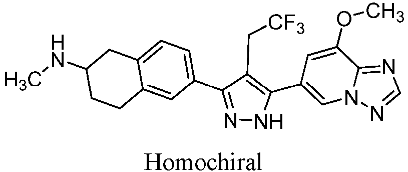 | 457.3 | 0.94 | A |

### Example 1303: N-(2-(4-(5-(8-methyl-[1,2,4]triazolo[1,5-a]pyridin-6-yl)-4-(2,2,2-trifluoroethyl)-1H-pyrazol-3-yl)phenyl)propan-2-yl)tetrahydro-2H-pyran-4-amine

To a solution of 2-(4-(5-(8-methyl-[1,2,4]triazolo[1,5-a]pyridin-6-yl)-4-(2,2,2-trifluoroethyl)-1H-pyrazol-3-yl)phenyl)propan-2-amine, TFA (31 mg, 0.059 mmol) and tetrahydro-4H-pyran-4-one (29.4 mg, 0.293 mmol) in MeOH (2.0 mL) was added TEA (0.2 mL, 1.435 mmol) at room temperature. The reaction mixture was stirred at the same temperature for 16 h. Sodium cyanoborohydride (11.06 mg, 0.176 mmol) was added at room temperature and the reaction mixture was stirred for 16 h. The reaction mass was purified by Prep LCMS using method AB, the fractions containing product were combined and dried using Genevac centrifugal evaporator to get N-(2-(4-(5-(8-methyl-[1,2,4]triazolo[1,5-a]pyridin-6-yl)-4-(2,2,2-trifluoroethyl)-1H-pyrazol-3-yl)phenyl) propan-2-yl)tetrahydro-2H-pyran-4-amine (8.7 mg, 0.016 mmol, 28 % yield) as a pale solid. LCMS Retention time 1.061 min [F], MS (E⁺) m/z: 499.3 [M+H]; ¹H NMR (400 MHz, METHANOL-d₄) δ ppm 8.90 (s, 1H), 8.56-8.44 (m, 1H), 7.90-7.65 (m, 6H), 7.65-7.52 (m, 1H), 3.93-3.77 (m, 4H), 3.72 (q, *J*=10.5 Hz, 2H), 3.47-3.37 (m, 1H), 3.27 (td, *J*=11.6, 2.6 Hz, 3H), 3.13 (d, *J*=18.1 Hz, 1H), 2.84-2.65 (m, 4H), 2.05 (s, 1H), 2.02-1.92 (m, 2H), 1.92-1.76 (m, 7H), 1.68-1.46 (m, 5H), 1.35-1.28 (m, 1H).

The following Examples were prepared according to the general procedure used to prepare Example 1303.

**TABLE 121**

| Ex. No. | Structure | LCMS MH⁺ | RT (min) | HPLC Method |
|---|---|---|---|---|
| 1308 | 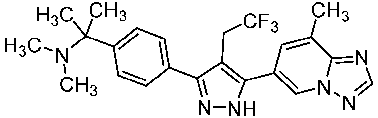 | 443.2 | 1.246 | E |
| 1309 | 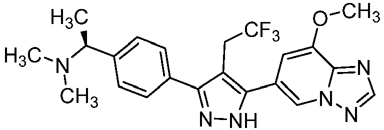 | 445.2 | 1.2 | C |
| 1310 | 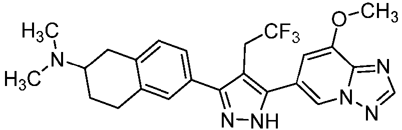 | 471.2 | 1.01 | E |
| 1311 | 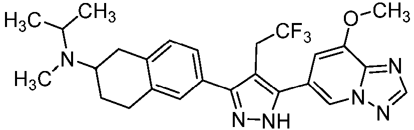 | 499.2 | 1.11 | C |
| 1312 | 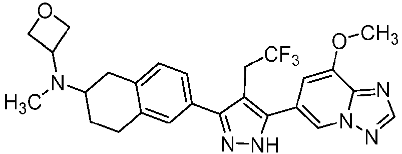 | 513.2 | 1.35 | C |

### EXAMPLE 1313

### 2-(dimethylamino)-N-(2-(4-(5-(8-methyl-[1,2,4]triazolo[1,5-a]pyridin-6-yl)-4-(2,2,2-trifluoroethyl)-1H-pyrazol-3-yl)phenyl)propan-2-yl)acetamide

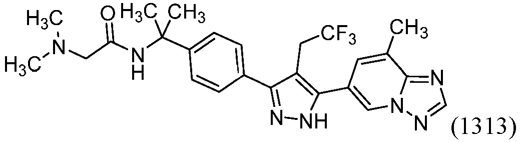

To a solution of 2-(4-(5-(8-methyl-[1,2,4]triazolo[1,5-a]pyridin-6-yl)-4-(2,2,2-trifluoroethyl)-1H-pyrazol-3-yl)phenyl)propan-2-amine, TFA (21 mg, 0.040 mmol) and dimethylglycine (8.20 mg, 0.079 mmol) in DMF (1.5 mL) were added TEA (0.2 mL, 1.435 mmol) and HATU (30.2 mg, 0.079 mmol) at room temperature. The reaction mixture was stirred at the same temperature for 16 h. The reaction mass was purified by Prep LCMS using method AA, the fractions containing product were combined and dried using Genevac centrifugal evaporator to get 2-(dimethylamino)-N-(2-(4-(5-(8-methyl-[1,2,4]triazolo[1,5-a]pyridin-6-yl)-4-(2,2,2-trifluoroethyl)-1H-pyrazol-3-yl)phenyl) propan-2-yl)acetamide (9.2 mg, 0.018 mmol, 46.0 % yield) as a pale solid. LCMS Retention time 1.403 min [E], MS (E⁺) m/z: 500.3 [M+H]; ¹H NMR (400 MHz, METHANOL-d₄) δ ppm 8.90 (s, 1H), 8.47 (s, 1H), 7.77 (br. s., 1H), 7.57 (q, *J*=8.5 Hz, 4H), 3.69 (q, *J*=10.7 Hz, 2H), 3.14-3.00 (m, 2H), 2.79-2.64 (m, 3H), 2.43 (s, 6H), 2.05-1.89 (m, 1H), 1.75 (s, 5H), 1.38-1.21 (m, 2H).

The following Examples were prepared according to the general procedure used to prepare Example 1313:

**TABLE 122**

| Ex. No. | Structure | LCMS MH⁺ | RT (min) | HPLC Method |
|---|---|---|---|---|
| 1314 | 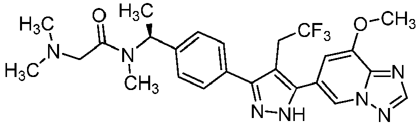 | 516.2 | 1.19 | C |
| 1315 | 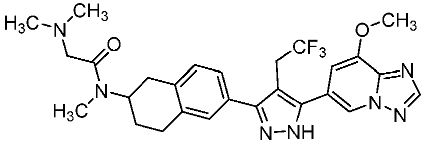 | 542.3 | 1.16 | C |

### EXAMPLE 1316

### 8-methoxy-6-(3-(5-(6-((tetrahydro-2H-pyran-4-yl)methyl)-2,6-diazaspiro[3.3]heptan-2-yl)pyridin-2-yl)-4-(2,2,2-trifluoroethyl)-1H-pyrazol-5-yl)-[1,2,4]triazolo[1,5-a]pyridine

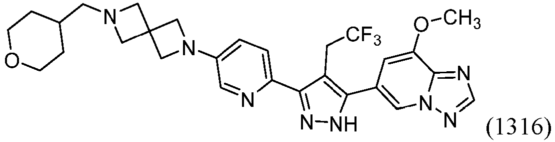

### Intermediate 1316A: tert-butyl 6-(6-(5-(8-methoxy-[1,2,4]triazolo[1,5-a]pyridin-6-yl)-4-(2,2,2-trifluoroethyl)-1-((2-(trimethylsilyl)ethoxy)methyl)-1H-pyrazol-3-yl)pyridin-3-yl)-2,6-diazaspiro[3.3]heptane-2-carboxylate

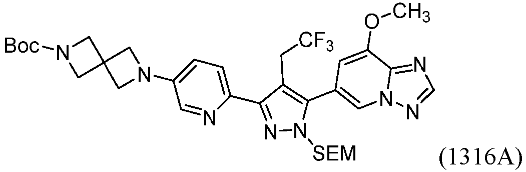

tert-butyl 6-(6-(5-(8-methoxy-[1,2,4]triazolo[1,5-a]pyridin-6-yl)-4-(2,2,2-trifluoroethyl)-1-((2-(trimethylsilyl)ethoxy)methyl)-1H-pyrazol-3-yl)pyridin-3-yl)-2,6-diazaspiro[3.3]heptan-2-carboxylate (340 mg, 0.485 mmol, 24% yield) was prepared according to the general process described in Intermediate 307B, using tert-butyl 6-(6-bromopyridin-3-yl)-2,6-diazaspiro[3.3]heptane-2-carboxylate (0.700 g, 1.976 mmol) and 8-methoxy-6-(3-(4,4,5,5-tetramethyl-1,3,2-dioxaborolan-2-yl)-4-(2,2,2-trifluoroethyl)-1-((2-(trimethylsilyl)ethoxy)methyl)-1H-pyrazol-5-yl)-[1,2,4]triazolo[1,5-a]pyridine (1.750 g, 3.16 mmol) as starting intermediates to get the title compound as an off-white solid. LCMS Retention time: 1.30 min [A], MS (E⁺) m/z: 701.3 [M+H].

### Example 1316B: 6-(3-(5-(2,6-diazaspiro[3.3]heptan-2-yl)pyridin-2-yl)-4-(2,2,2-trifluoroethyl)-1H-pyrazol-5-yl)-8-methoxy-[1,2,4]triazolo[1,5-a]pyridine

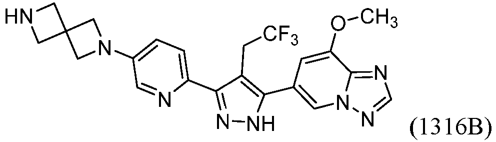

6-(3-(5-(2,6-diazaspiro[3.3]heptan-2-yl)pyridin-2-yl)-4-(2,2,2-trifluoroethyl)-1H-pyrazol-5-yl)-8-methoxy-[1,2,4]triazolo[1,5-a]pyridine (1 mg) was prepared according to the general process described in Intermediate 307D, using tert-butyl 6-(6-(5-(8-methoxy-[1,2,4]triazolo[1,5-a]pyridin-6-yl)-4-(2,2,2-trifluoroethyl)-1-((2-(trimethylsilyl)ethoxy) methyl)-1H-pyrazol-3-yl) pyridin-3-yl)-2,6-diazaspiro [3.3]heptane-2-carboxylate (320 mg, 0.457 mmol) as a starting intermediate to get the title compound as an off-white solid. LCMS Retention time: 0.96 min [F], MS (E⁺) m/z: 471.2 [M+H]; ¹H NMR (400 MHz, METHANOL-*d*4) δ ppm 8.63 (d, *J*=1.22 Hz, 1 H) 8.42 (s, 1 H) 7.94 (d, *J*=2.45 Hz, 1 H) 7.60-7.68 (m, 1 H) 7.28 (s, 1 H) 7.00 (dd, J=8.56, 2.93 Hz, 1 H) 4.14-4.21 (m, 7 H) 4.12 (s, 3 H) 4.01-4.09 (m, 2 H) 1.91 (s, 1 H) 1.21-1.36 (m, 2 H).

### Example 1316: 8-methoxy-6-(3-(5-(6-((tetrahydro-2H-pyran-4-yl)methyl)-2,6-diazaspiro[3.3]heptan-2-yl)pyridin-2-yl)-4-(2,2,2-trifluoroethyl)-1H-pyrazol-5-yl)-[1,2,4] triazolo[1,5-a]pyridine

8-methoxy-6-(3-(5-(6-((tetrahydro-2H-pyran-4-yl)methyl)-2,6-diazaspiro[3.3]heptan-2-yl)pyridin-2-yl)-4-(2,2,2-trifluoroethyl)-1H-pyrazol-5-yl)-[1,2,4] triazolo[1,5-a]pyridine (8.6 mg) was prepared according to the general process described in Example 307, using 6-(3-(5-(2,6-diazaspiro[3.3]heptan-2-yl)pyridin-2-yl)-4-(2,2,2-trifluoroethyl)-1H-pyrazol-5-yl)-8-methoxy-[1,2,4]triazolo[1,5-a]pyridine (20.0 mg, 0.043 mmol) as a starting intermediate to get the title compound as a white solid. LCMS Retention time: 1.28 min [E], MS (E⁺) m/z: 569.3 [M+H]; ¹H NMR (400 MHz, METHANOL-*d*₄) δ ppm 8.65 (s, 1 H) 8.43 (s, 1 H) 7.86-8.00 (m, 1 H) 7.54-7.72 (m, 1 H) 7.25-7.37 (m, 1 H) 7.00 (dd, *J*=8.56, 2.93Hz, 1 H) 4.13 (d, *J*=4.89 Hz, 8 H) 3.95 (br dd, *J*=11.37, 3.79 Hz, 2 H) 3.62-3.76 (m, 4 H) 3.43 (td, *J*=11.80*,* 1.83 Hz, 2 H) 2.50-2.62 (m, 2 H) 1.96 (s, 1 H)1.59-1.80 (m, 3 H) 1.25-1.37 (m, 3 H).

The following Examples were prepared according to the general procedure used to prepare Example 1316.

**TABLE 123**

| Ex. No. | Structure | LCMS MH⁺ | Ret Time (min) | HPLC Method |
|---|---|---|---|---|
| 1317 | 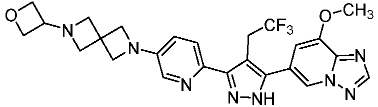 | 527.2 | 1.26 | C |
| 1318 | 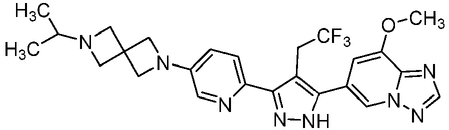 | 513.2 | 1.19 | C |
| 1319 | 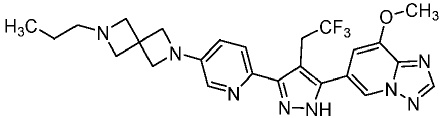 | 513.2 | 1.23 | C |
| 1320 | 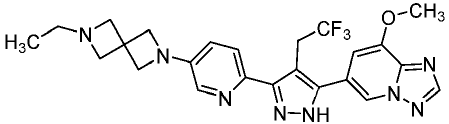 | 499.2 | 1.11 | C |
| 1321 | 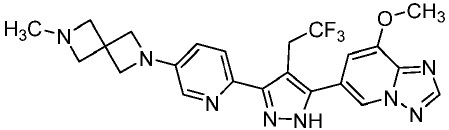 | 485.2 | 1.03 | C |
| 1322 | 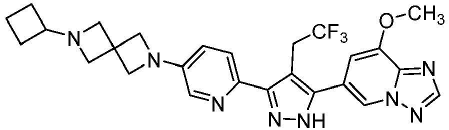 | 525.2 | 1.45 | C |
| 1323 | 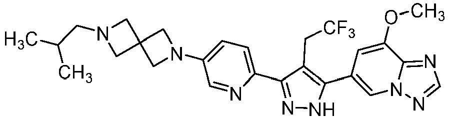 | 527.2 | 1.4 | C |
| 1324 | 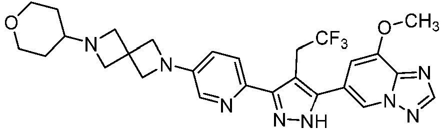 | 555.2 | 1.3 | C |
| 1325 | 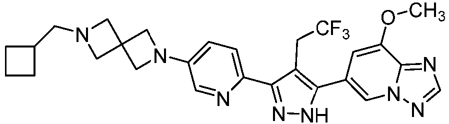 | 539.2 | 1.54 | C |

### EXAMPLE 1326

### 8-methoxy-6-(3-(5-(1-((tetrahydro-2H-pyran-4-yl)methyl)piperidin-4-yl)pyridin-2-yl)-4-(trifluoromethyl)-1H-pyrazol-5-yl)-[1,2,4]triazolo[1,5-a]pyridine

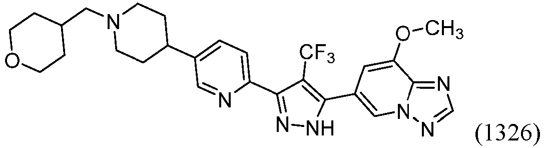

### Intermediate 1326A: tert-butyl 6-(trimethylstannyl)-3',6'-dihydro-[3,4'-bipyridine]-1'(2'H)-carboxylate

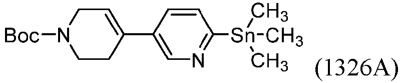

To a solution of tert-butyl 6-bromo-3',6'-dihydro-[3,4'-bipyridine]-1'(2'H)-carboxylate (100 mg, 0.295 mmol) in toluene (3 mL) was added hexamethylditin (0.073 mL, 0.354 mmol), degassed the mixture with nitrogen for 5 min, to this was then added 1,1'-bis(di-tert-butylphosphino)ferrocene palladium dichloride (15.37 mg, 0.024 mmol) and stirred in sealed tube at 90 °C for 2 h. The reaction mass was concentrated, the residue was diluted with EtOAc (20 mL), the solid was filtered and washed with toluene (50 mL), the combined filtrates were collected and concentrated to get tert-butyl 6-(trimethylstannyl)-3',6'-dihydro-[3,4'-bipyridine]-1'(2'H)-carboxylate (110 mg, 0.068 mmol, 23% yield) as an oil. LCMS retention time 0.94 min [A], MS (E⁻) *m*/*z:* 228.2 [M+H].

### Intermediate 1326B: (Z)-N-(3-(dimethylamino)-2-(trifluoromethyl)allylidene)-N-methylmethanaminium

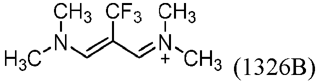

To a solution of 3,3,3-trifluoropropanoic acid (2 g, 15.62 mmol) in DMF (20 mL) was added dropwise POCl₃ (4.44 ml, 47.6 mmol) at 0 °C, brought to room temperature, then stirred at 60 °C for 16 h. The reaction mass was concentrated, the residue was triturated with diethyl ether (20 mL) and decane to get (Z)-N-(3-(dimethylamino)-2-(trifluoromethyl) allylidene)-N-methylmethanaminium (11 g, 56.3 mmol) as a gummy material, this was taken to next step without further purification. LCMS retention time 0.463 min [A], MS (E⁻) *m*/*z:* 196.0 [M+H].

### Intermediate 1326C: 4-(trifluoromethyl)-1H-pyrazole

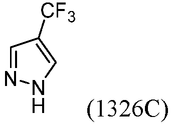

To a solution of (Z)-N-(3-(dimethylamino)-2-(trifluoromethyl)allylidene)-N-methyl methanaminium (9.3 g, 47.6 mmol) in acetonitrile (270 mL) was added hydrazine (30 mL, 956 mmol) at room temperature, then stirred at 70 °C for 1 h. The reaction mixture was concentrated and dried under vacuum to get 4-(trifluoromethyl)-1H-pyrazole (2.8 g, 20.58 mmol, 43% yield) an orange color liquid. ¹H NMR (400 MHz, CD₃OD): δ ppm 7.9 (s, 1 H); ¹⁹F NMR, δ ppm -57.2 (CF₃).

### Intermediate 1326D: 3,5-dibromo-4-(trifluoromethyl)-1H-pyrazole

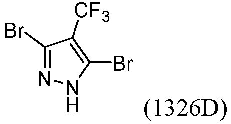

To a solution 4-(trifluoromethyl)-1H-pyrazole (150 mg, 1.102 mmol) in acetonitrile (10 mL) was added NBS (177 mg, 0.992 mmol) at room temperature, then stirred at 70 °C for 2 h. The reaction was quenched with water (20 ml). The reaction mixture was extracted with EtOAc (2 X 100 ml), dried over Na₂SO₄ and concentrated to get crude 3,5-dibromo-4-(trifluoromethyl)-1H-pyrazole (130 mg, 0.164 mmol, 14% yield) as an orange color liquid. LCMS retention time 1.23 min [A], MS (E⁻) *m*/*z:* 292.8.0 [M+H].

### Intermediate 1326E: 3,5-dibromo-4-(trifluoromethyl)-1-((2-(trimethylsilyl)ethoxy) methyl)-1H-pyrazole

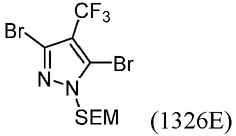

To a suspension of NaH (0.544 g, 13.61 mmol) in THF (40 mL) was cooled at 0⁰C for 5 min, then were added 3,5-dibromo-4-(trifluoromethyl)-1H-pyrazole (2 g, 6.81 mmol), stirred at the same temperature for 15 min, and SEM-Cl (2.414 mL, 13.61 mmol), stirred at room temperature for 12 h. The reaction mass was quenched with ice water, diluted with EtOAc (20 mL), separated both the layers, the organic layer was dried and concentrated to get crude compound. The crude was purified by ISCO using 40 g silica column, compound was eluted in 60% EA in hexanes, the fractions were collected and concentrated to get 3,5-dibromo-4-(trifluoromethyl)-1-((2-(trimethylsilyl)ethoxy)methyl)-1H-pyrazole (700 mg, 24%) of an oil. LCMS retention time 1.412 min [B]. MS (E⁻) *m*/*z:* 426.1 [M+H].

### Intermediate 1326F: 6-(3-bromo-4-(trifluoromethyl)-1-((2-(trimethylsilyl)ethoxy) methyl)-1H-pyrazol-5-yl)-8-methoxy-[1,2,4]triazolo[1,5-a]pyridine

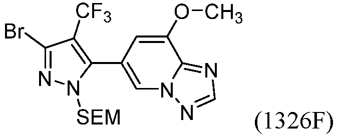

To a solution of 3,5-dibromo-4-(trifluoromethyl)-1-((2-(trimethylsilyl)ethoxy) methyl)-1H-pyrazole (1.7 g, 4.01 mmol), in 1,4-dioxane (85 mL) and water (28.3 mL) solvent mixture were added 8-methoxy-6-(4,4,5,5-tetramethyl-1,3,2-dioxaborolan-2-yl)-[1,2,4]triazolo[1,5-a] pyridine (0.992 g, 3.61 mmol) and potassium phosphate (1.702 g, 8.02 mmol), degassed the mixture with nitrogen for 5 min, to this was then added PdCl₂(dppf)-CH₂Cl₂ adduct (0.327 g, 0.401 mmol) and stirred in sealed tube at 90 °C for 16 h. The reaction mass was diluted in EtOAc (20 mL), the solid was filtered and washed with EtOAc (2 X 50 mL), the combined filtrates were collected and concentrated to get crude compound. The crude was purified by ISCO using 40 g silica column, compound was eluted in 60% EA in hexanes, the fractions were collected and concentrated to get 6-(3-bromo-4-(trifluoromethyl)-1-((2-(trimethylsilyl)ethoxy)methyl)-1H-pyrazol-5-yl)-8-methoxy-[1,2,4]triazolo[1,5-a]pyridine (450 mg, 0.832 mmol, 21% yield) as an oil. LCMS retention time 2.10 min [B], MS (E⁻) *m*/*z:* 494.1 [M+2].

### Intermediate 1326G: tert-butyl 6-(5-(8-methoxy-[1,2,4]triazolo[1,5-a]pyridin-6-yl)-4-(trifluoromethyl)-1-((2-(trimethylsilyl)ethoxy)methyl)-1H-pyrazol-3-yl)-3',6'-dihydro-[3,4'-bipyridine]-1'(2'H)-carboxylate

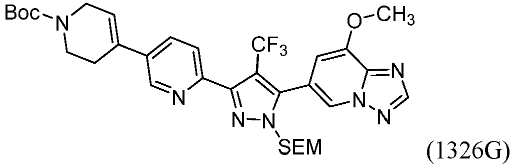

tert-butyl6-(5-(8-methoxy-[1,2,4]triazolo[1,5-a]pyridin-6-yl)-4-(trifluoromethyl)-1-((2-(trimethylsilyl)ethoxy)methyl)-1H-pyrazol-3-yl)-3',6'-dihydro-[3,4'-bipyridine]-1'(2'H)-carboxylate (350 mg, 0.406 mmol, 54% yield) was prepared according to the general process described in Intermediate 307B using tert-butyl 6-(trimethylstannyl)-3',6'-dihydro-[3,4'-bipyridine]-1'(2'H)-carboxylate (477 mg, 1.127 mmol) as a starting intermediate to get the title compound as an gummy solid. LCMS retention time 3.686 min. MS (E⁻) *m*/*z:* 672.0 [M+1].

### Intermediate 1326H: tert-butyl 4-(6-(5-(8-methoxy-[1,2,4]triazolo[1,5-a]pyridin-6-yl)-4-(trifluoromethyl)-1-((2-(trimethylsilyl)ethoxy)methyl)-1H-pyrazol-3-yl)pyridin-3-yl) piperidine-1-carboxylate

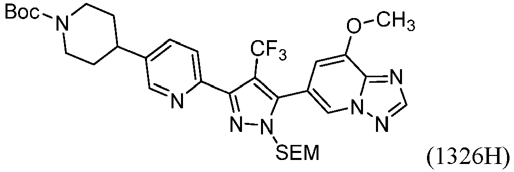

tert-butyl 4-(6-(5-(8-methoxy-[1,2,4]triazolo[1,5-a]pyridin-6-yl)-4-(trifluoromethyl)-1-((2-(trimethylsilyl)ethoxy)methyl)-1H-pyrazol-3-yl)pyridin-3-yl)piperidine-1-carboxylate (250 mg, 0.371 mmol, 100% yield) was prepared according to the general process described in Intermediate 307C, using tert-butyl 6-(5-(8-methoxy-[1,2,4]triazolo[1,5-a]pyridin-6-yl)-4-(trifluoromethyl)-1-((2-(trimethylsilyl)ethoxy)methyl)-1H-pyrazol-3-yl)-3',6'-dihydro-[3,4'-bipyridine]-1'(2'H)-carboxylate (250 mg, 0.372 mmol) as a starting intermediate to get the title compound as an orange color solid. LCMS retention time 4.16 min [B], MS (E⁻) *m*/*z:* 674.4 [M+H].

### Intermediate 13261: 8-methoxy-6-(3-(5-(piperidin-4-yl)pyridin-2-yl)-4-(trifluoromethyl)-1H-pyrazol-5-yl)-[1,2,4]triazolo[1,5-a]pyridine

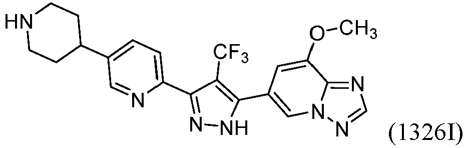

8-methoxy-6-(3-(5-(piperidin-4-yl)pyridin-2-yl)-4-(trifluoromethyl)-1H-pyrazol-5-yl)-[1,2,4]triazolo[1,5-a]pyridine (3.1 mg, 6.99 µmol, 23% yield) was prepared according to the general process described in Intermediate 307D using tert-butyl 4-(6-(5-(8-methoxy-[1,2,4]triazolo[1,5-a]pyridin-6-yl)-4-(trifluoromethyl)-1-((2-(trimethylsilyl)ethoxy)methyl)-1H-pyrazol-3-yl)pyridin-3-yl)piperidine-1-carboxylate (20 mg, 0.030 mmol) as a starting intermediate to get the title compound as a brown sloid. LCMS retention time 0.96 min [F], MS (E⁻) *m*/*z:* 444.4 [M+H]; ¹H NMR (400 MHz, DMSO-d₆) δ = 8.94-8.87 (m, 1H), 8.70 (s, 1H), 8.64-8.53 (m, 2H), 8.42-8.30 (m, 1H), 7.94-7.86 (m, 1H), 7.83-7.74 (m, 1H), 7.27 -7.20 (m, 1H), 4.06 (s, 3H), 3.47-3.39 (m, 2H), 3.12-2.99 (m, 3H), 2.98-2.89 (m, 1H), 2.1- 2.01 (m, 2H), 1.93-1.78 (m, 2H), 1.17 (t, *J=* 7.2 Hz, 1H).

### Example 1326: 8-methoxy-6-(3-(5-(1-((tetrahydro-2H-pyran-4-yl)methyl)piperidin-4-yl) pyridin-2-yl)-4-(trifluoromethyl)-1H-pyrazol-5-yl)-[1,2,4]triazolo[1,5-a]pyridine

8-methoxy-6-(3-(5-(1-((tetrahydro-2H-pyran-4-yl)methyl)piperidin-4-yl)pyridin-2-yl)-4-(trifluoromethyl)-1H-pyrazol-5-yl)-[1,2,4]triazolo[1,5-a]pyridine was prepared according to the general process described in Example 307 using 8-methoxy-6-(3-(5-(piperidin-4-yl)pyridin-2-yl)-4-(trifluoromethyl)-1H-pyrazol-5-yl)-[1,2,4]triazolo[1,5-a]pyridine as a starting intermediate to get the title compound as a white solid. LCMS retention time 1.309 min [E], MS (E⁻) *m*/*z:* 498.2 [M+H].

The following Examples were prepared according to the general procedure used to prepare Example 1326.

**TABLE 124**

| Ex. No. | Structure | **LCMS** MH⁺ | RT (min) | HPLC Method |
|---|---|---|---|---|
| 1327 | 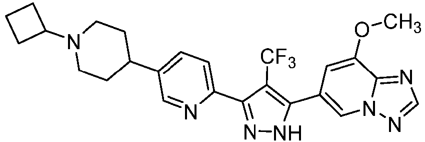 | 498.2 | 1.309 | E |
| 1328 | 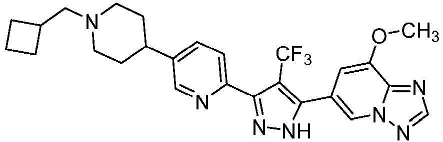 | 512.2 | 1.405 | E |

### BIOLOGICAL ASSAYS

The pharmacological properties of the compounds of this invention may be confirmed by a number of biological assays. The exemplified biological assays, which follow, have been carried out with compounds of the invention.

### TLR7/8/9 Inhibition Reporter Assays

HEK-Blue^{™}-cells (Invivogen) overexpressing human TLR7, TLR8 or TLR9 receptors were used for screening inhibitors of these receptors using an inducible SEAP (secreted embryonic alkaline phosphatase) reporter gene under the control of the IFN-β minimal promoter fused to five NF-κB and AP-1-binding sites. Briefly, cells are seeded into Greiner 384 well plates (15000 cells per well for TLR7, 20,000 for TLR8 and 25,000 for TLR9) and then treated with test compounds in DMSO to yield a final dose response concentration range of 0.05 nM-50 µM. After a 30 minute compound pre-treatment at room temperature, the cells are then stimulated with a TLR7 ligand (gardiquimod at a final concentration of 7.5 µM), TLR8 ligand (R848 at a final concentration of 15.9 µM) or TLR9 ligand (ODN2006 at a final concentration of 5 nM) to activate NF-κB and AP-1 which induce the production of SEAP. After a 22 hour incubation at 37 °C, 5% CO₂, SEAP levels are determined with the addition of HEK-Blue^{™} Detection reagent (Invivogen), a cell culture medium that allows for detection of SEAP, according to manufacturer's specifications. The percent inhibition is determined as the % reduction in the HEK-Blue signal present in wells treated with agonist plus DMSO alone compared to wells treated with a known inhibitor.

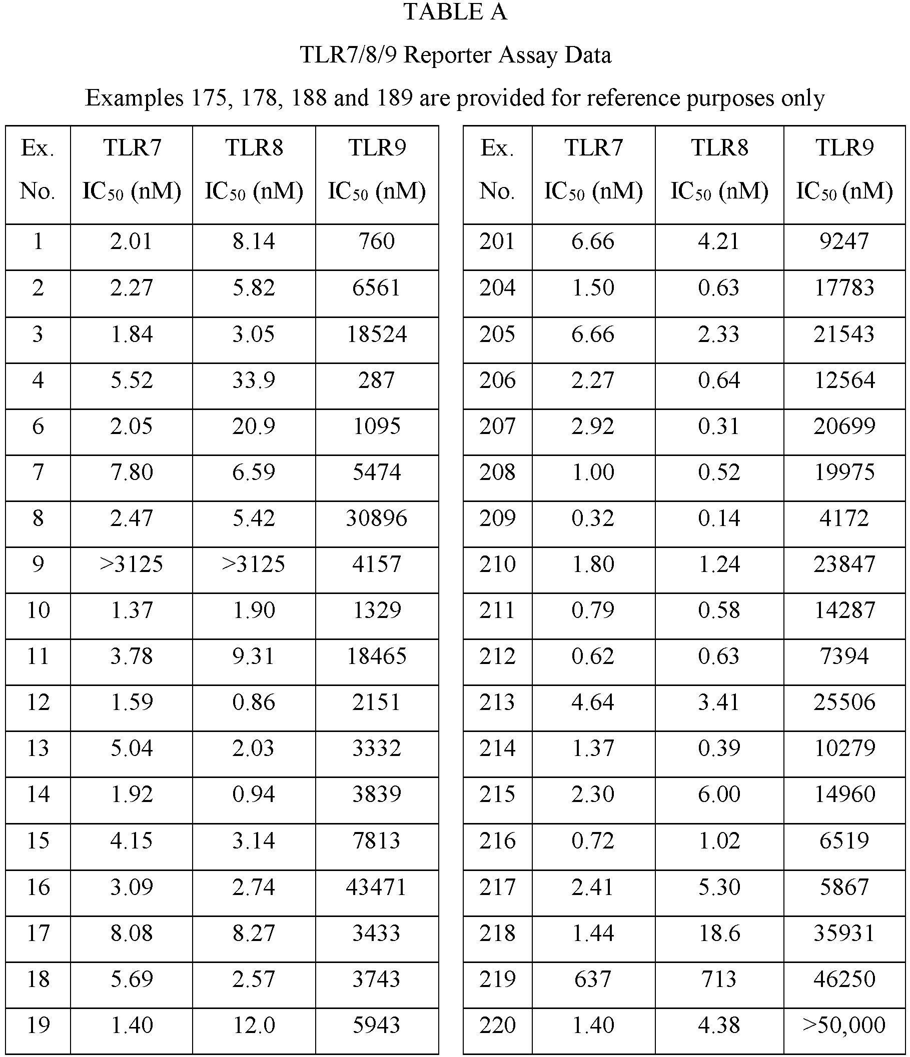

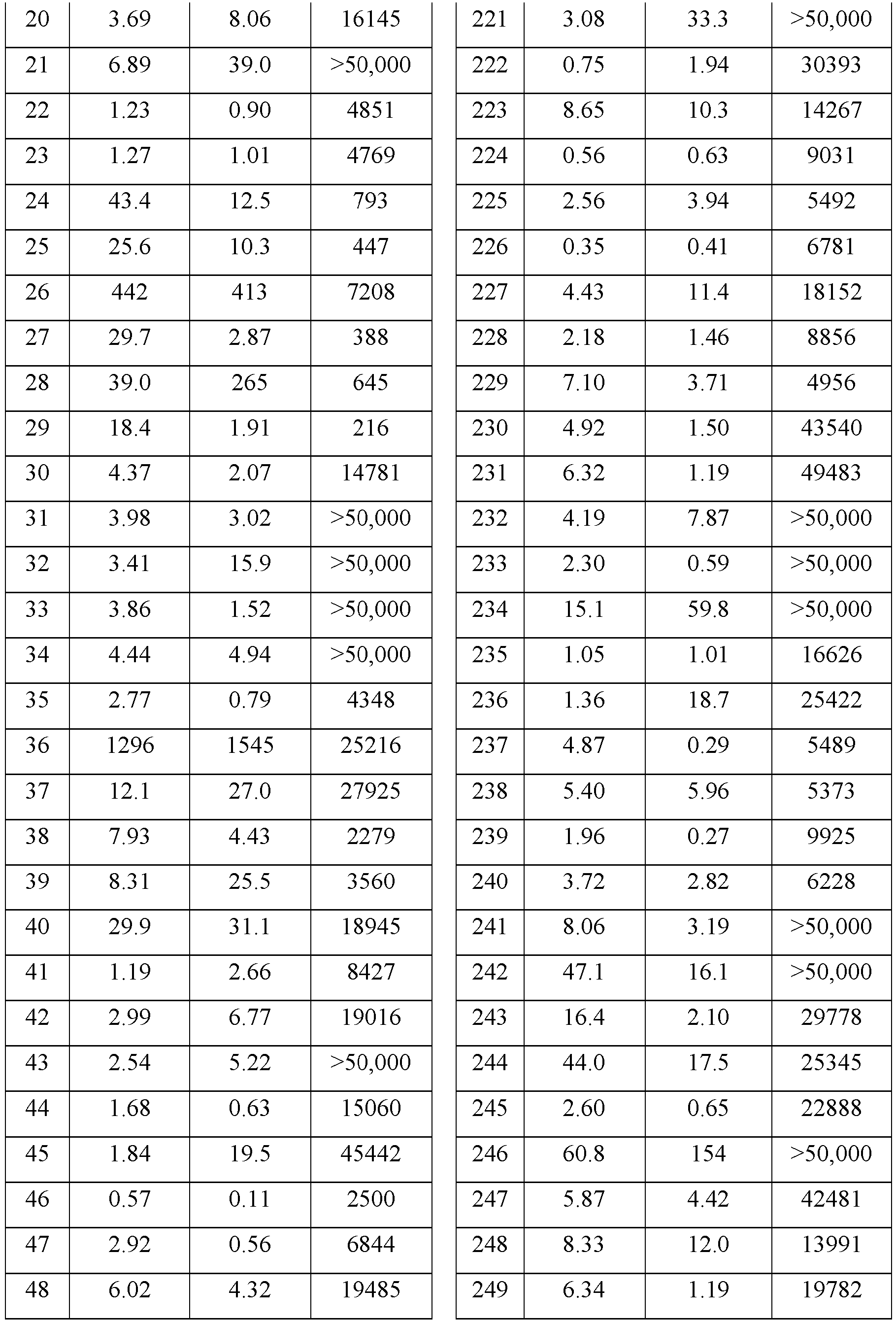

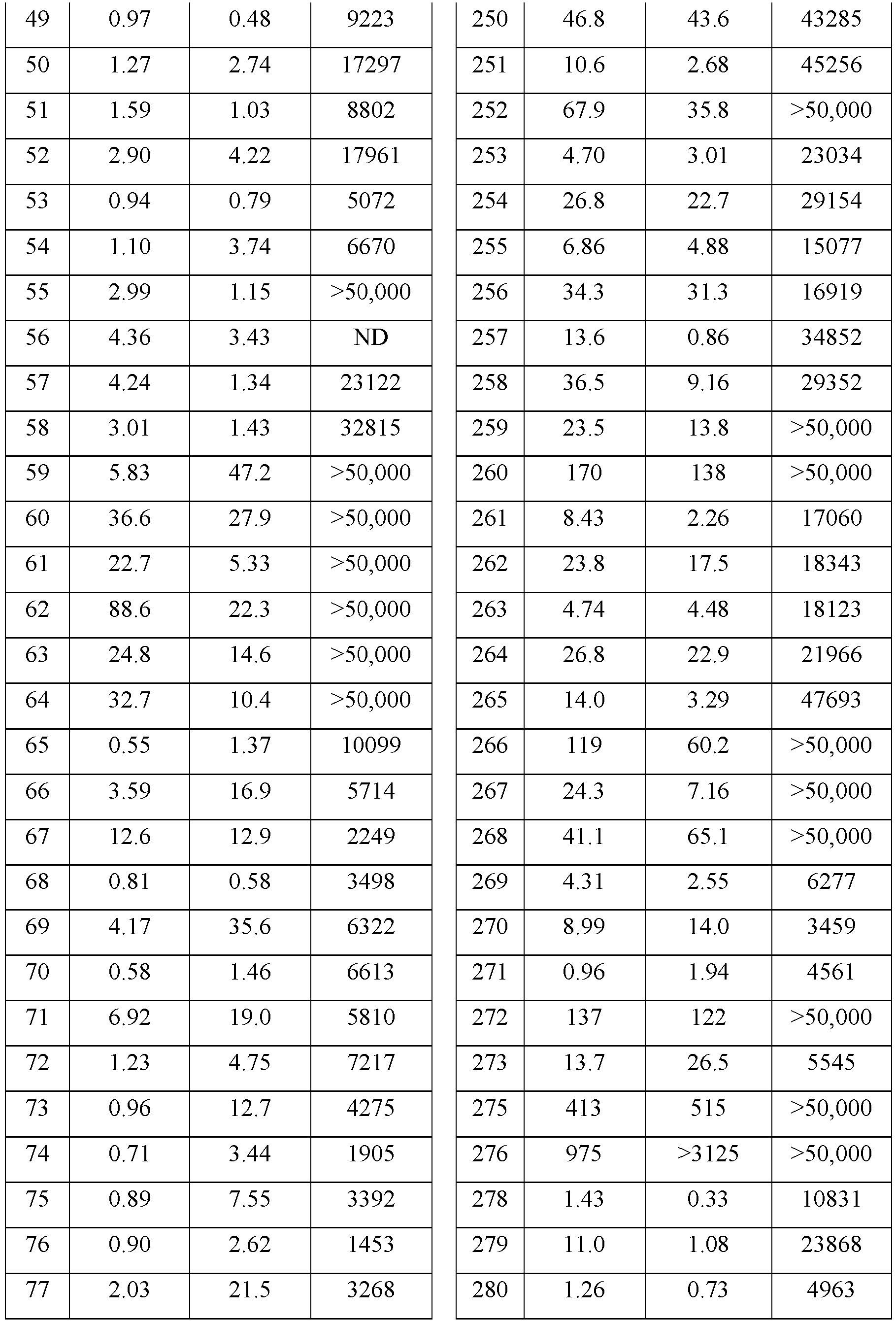

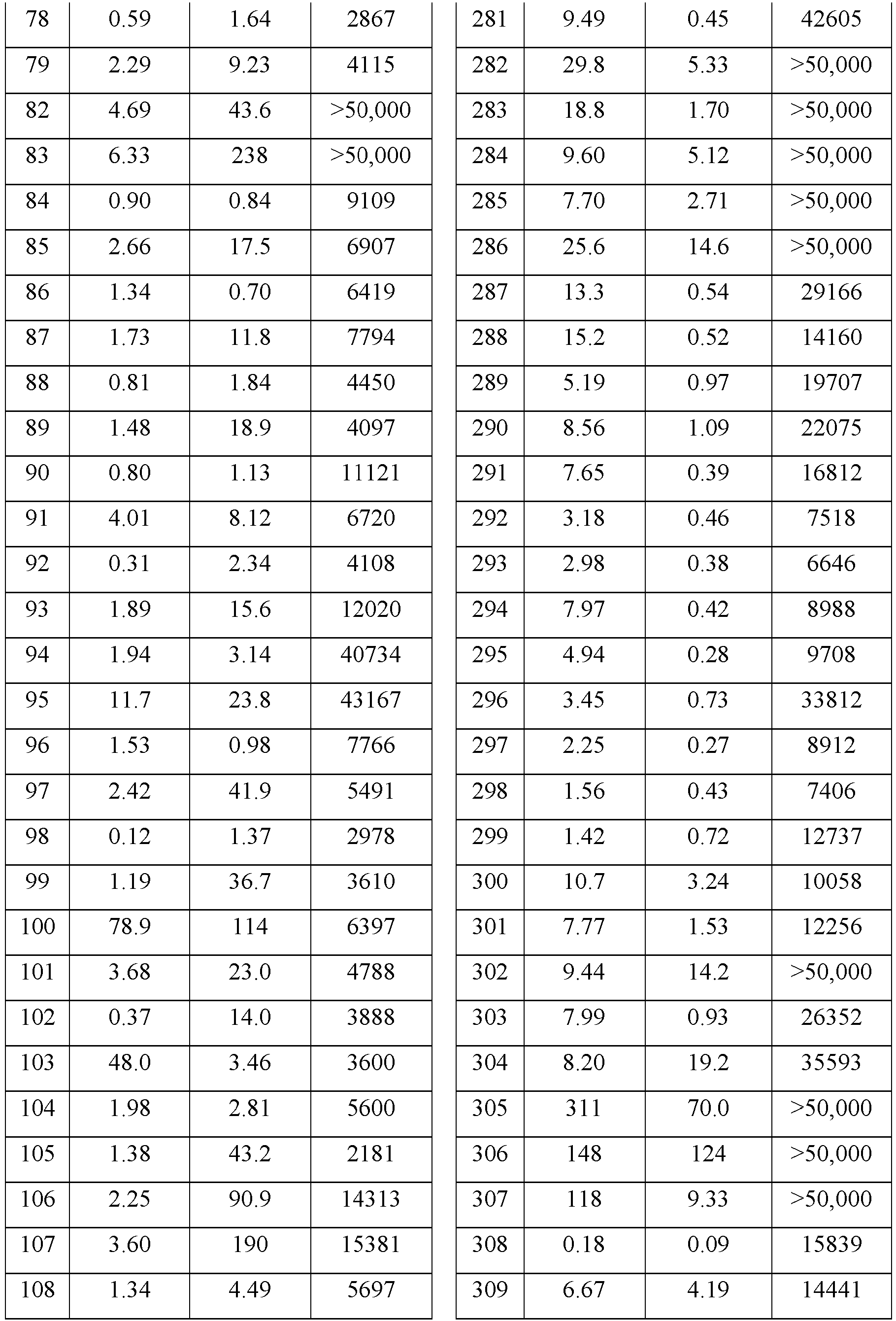

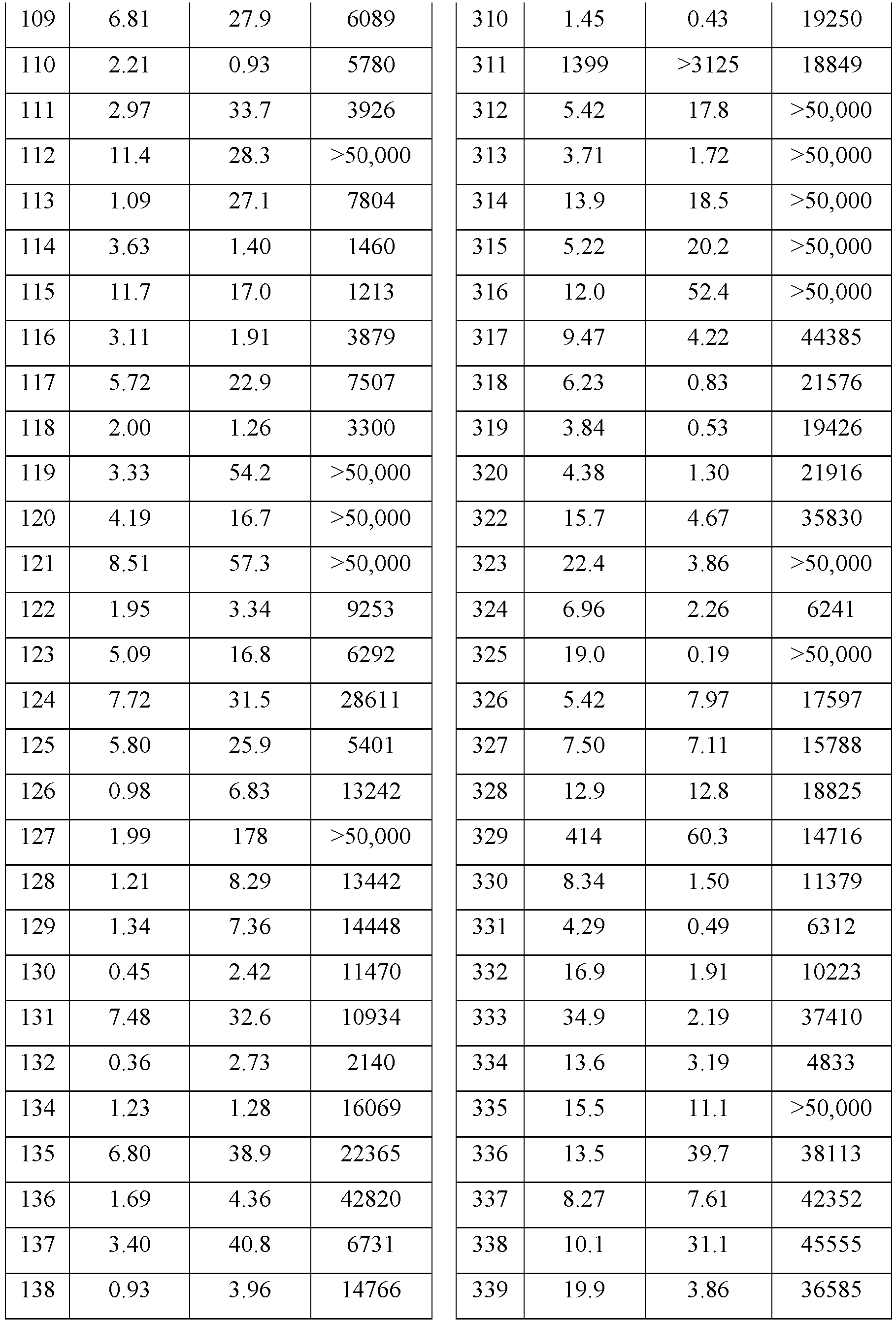

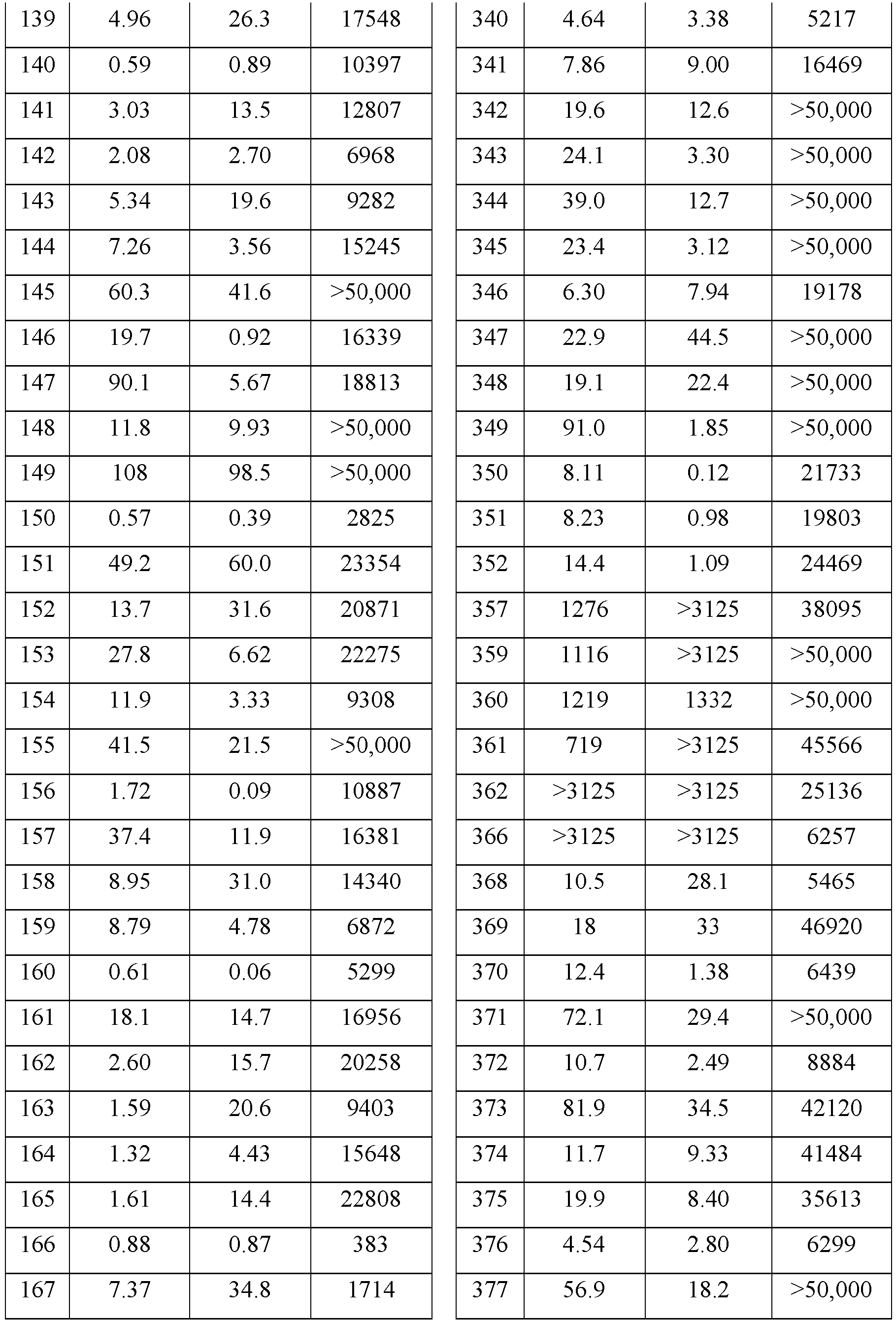

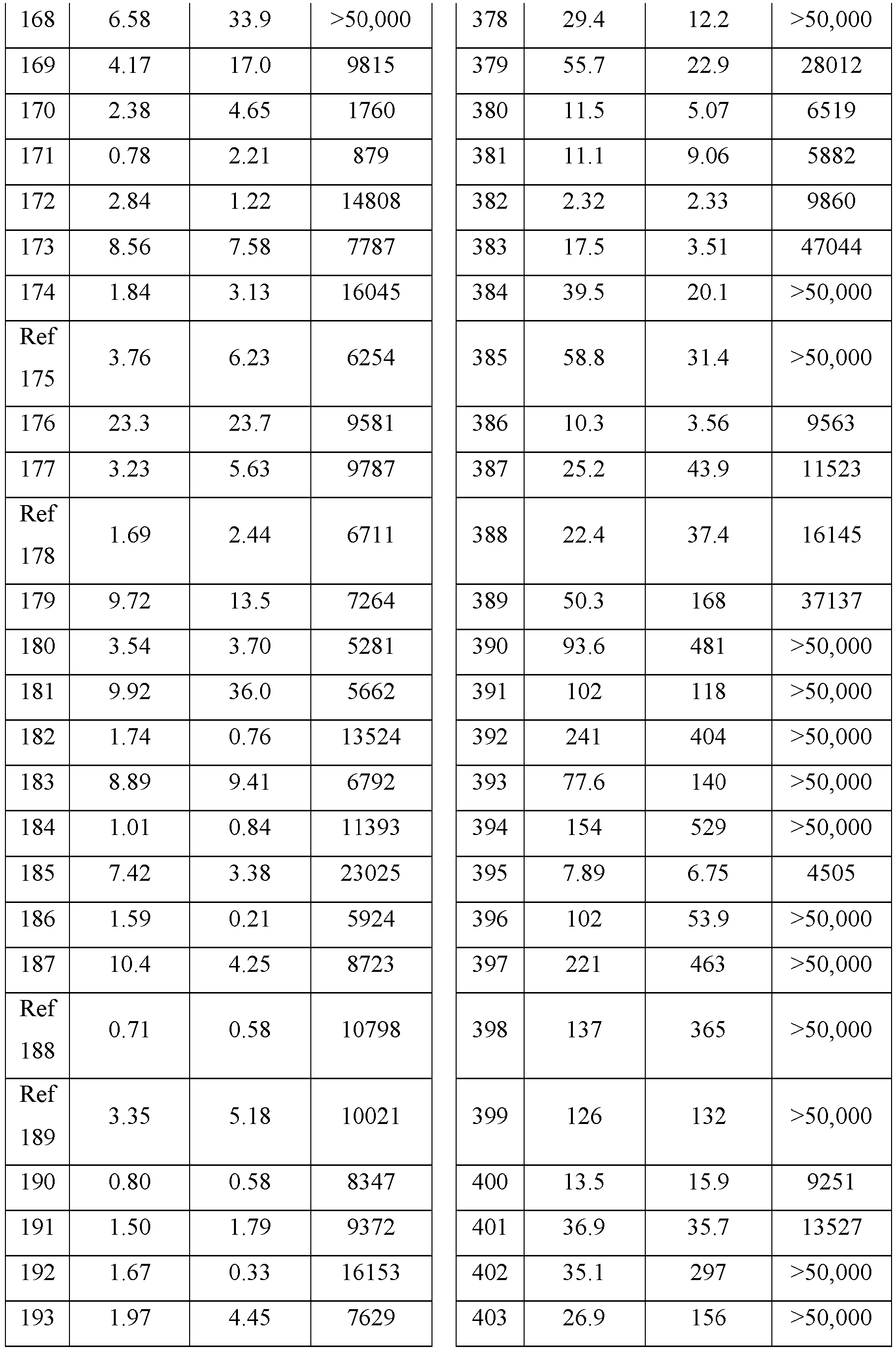

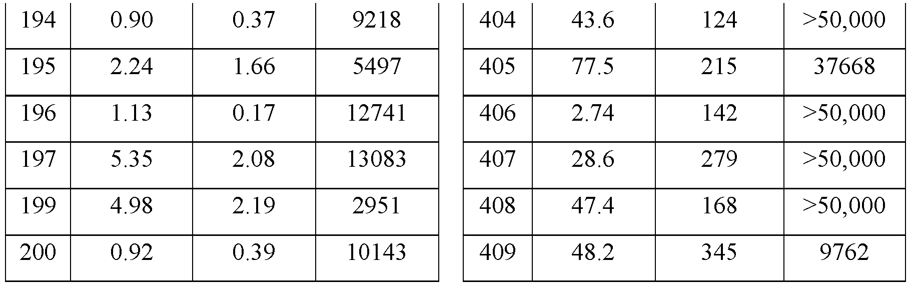

## Claims

1. A compound of Formula (I) 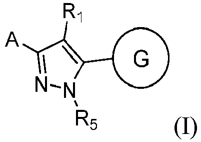 N-oxide, or a salt thereof, wherein:
G is: 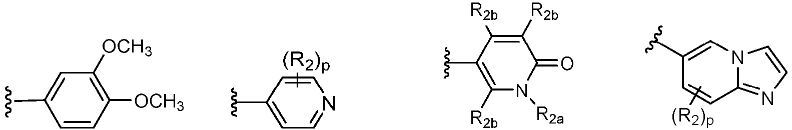 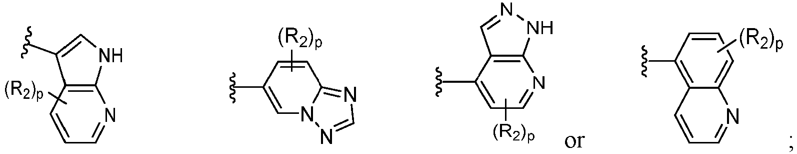
R₁ is -CH₃, -CH₂CH₃, -CH(CH₃)₂, -CF₃, or -CH₂CF₃;
each R₂ is independently -CN, -CH₃, or -OCH₃;
R₂ₐ is C₁₋₆ alkyl, C₁₋₃ fluoroalkyl, C₁₋₆ hydroxyalkyl, C₁₋₃ aminoalkyl, -(CH₂)₀₋₄O(C₁₋₃ alkyl), C₃₋₆ cycloalkyl, -(CH₂)₁₋₃C(O)NR_{y}R_{y}, -CH₂(C₃₋₆ cycloalkyl), -CH₂(phenyl), tetrahydrofuranyl, tetrahydropyranyl, or phenyl;
each R_{2b} is independently hydrogen, halo, -CN, -NRₓRₓ, C₁₋₆ alkyl, C₁₋₃ fluoroalkyl, C₁₋₃ hydroxyalkyl, C₁₋₃ fluoroalkoxy, -(CH₂)₀₋₂O(C₁₋₃ alkyl), -(CH₂)₀₋₃C(O)NRₓRₓ, -(CH₂)₁₋₃(C₃-₆ cycloalkyl), -C(O)O(C₁₋₃ alkyl), -C(O)NRₓ(C₁₋₃ alkyl), -CRₓ=CRₓRₓ, or -CRₓ=CH(C₃₋₆ cycloalkyl),
A is:
(i) -CH₂N(CH₃)Rₓ, -C(O)NRₓRₓ, -C(O)N(CH₃)(CH₂CH₂CN), -C(O)N(CH₂CH₃)(CH₂CH₂CN), or -C(O)N(CH₃)(CH₂CH₂CH₂N(CH₃)₂);
(ii) -C(O)A₁, -C(O)NRₓ(CRₓRₓ)₀₋₂A₁, -CH₂NHA₁, or -C(O)C(O)NHA₁;
(iii) cyclohexyl substituted zero to 1 R_{3b};
(iv) piperidinyl substituted with zero to 1 R_{3c};
(v) phenyl substituted with zero to 1 R_{3d} and zero to 1 R₃ₑ;
(vi) pyridinyl substituted with zero to 1 R_{3f} and zero to 1 R_{3g};
(vii) pyrazinyl or pyrimidinyl, each substituted with zero to 1 R_{3f};
(viii) thiazolyl substituted with R₃ₕ and zero to 1 R₃ᵢ;
(ix) diazabicyclo[2.2.1]heptanyl or diazaspiro[3.3]heptanyl, each substituted with zero to 1 R₃ⱼ; or
(x) benzo[d]thiazolyl, dihydroisoquinolinyl, tetrahydronaphthyridinyl, tetrahydrobenzo[d]thiazolyl, tetrahydroimidazo[1,2-a]pyrazinyl, tetrahydroisoquinolinonyl, tetrahydroisoquinolinyl, tetrahydronaphthalenyl, tetrahydropyrazolo[1,5-a]pyrazinyl, tetrahydropyrido[4,3-d]pyrimidinyl, tetrahydrothiazolo[4,5-c]pyridinyl, or tetrahydrothiazolo[5,4-c]pyridinyl, each substituted with zero to 1 R₃ₖ;
A₁ is azetidinyl, cyclohexyl, pyrrolidinyl, piperidinyl, piperazinyl, morpholinyl, tetrahydropyranyl, pyridinyl, diazepanyl, hexahydropyrrolo[3,4-c]pyrrolyl, each substituted with zero to 1 R₃ₐ;
R₃ₐ is -OH, -CH₃, -CH₂CH₃, -CH₂CH₂CH₃, -CH(CH₃)₂, -CH₂CH(CH₃)₂, -CH(CH₃)(CH₂CH₃), -CH₂C(CH₃)₃, -CH₂CH₂CH(CH₃)₂, -CH(CH₃)CH(CH₃)₂, -CH₂CH₂C(CH₃)₃, -CH₂C(CH₂CH₃)₂, -CH₂CF₃, -CH₂CH₂CF₃, -CH₂CH₂CH₂CF₃, -CH₂CH₂CN,-CH₂C(CH₃)₂OH, -CH₂CH₂OCH₃, -CH₂CH₂S(O)₂CH₃, -CH(CH₃)CH₂S(O)₂CH₃, -CH₂NH₂, -CH₂CH₂N(CH₃)₂, -CH₂C(O)NH(CH₃), -CH₂C(O)N(CH₃)₂, -NR_{y}R_{y}, -N(CH₃)(CH₂CH₂OCH₃), -NH(CH₂CH₂CH₂CF₃), -NHCH(CH₃)(CH₂OCH₃), -C(O)NH₂, -C(O)OC(CH₃)₃, -CH₂(cyclopropyl), -CH₂(methyloxetanyl), -CH₂(tetrahydrofuranyl), -CH₂(tetrahydropyranyl), -CH₂(dimethylisoxazolyl), -CH₂(methyltriazolyl), -CH₂(methoxypyrimidinyl), -NH(oxetanyl), -NH(methyloxetanyl), -NH(tetrahydropyranyl), -NH(dimethyltetrahydropyranyl), -N(cyclopropyl)₂, -NHCH₂(cyclopentyl), -NHCH₂(dimethylisoxazolyl), -NHCH₂(methyloxetanyl), -NHCH₂(pyridinyl), -NHCH₂(pyrimidinyl), -NHCH₂(methylpyrimidinyl), -NHCH₂(methoxypyrimidinyl), -NHCH₂(tetrahydrofuranyl), -NHCH₂(tetrahydropyranyl), cyclobutyl, oxetanyl, isopropylpiperidinyl, tetrahydropyranyl, dimethyltetrahydropyranyl, or pyridinyl;
R_{3b} is -NH(CH₃), -NH(CH₂CHF₂), -N(CH₃)(CH₂CH₃), -NH(CH₂CH₂N(CH₃)₂), -N(CH₃)C(O)CH₂N(CH₃)₂, -N(CH₃)CH₂C(O)N(CH₃)₂, -NH(isopropylpiperidinyl), -N(CH₃)C(O)(azetidinyl), -N(CH₃)C(O)(isopropylazetidinyl), -N(CH₃)C(O)(ethylazetidinyl), -N(CH₃)C(O)(methylazetidinyl), -NH(CH₂(methyloxetanyl), morpholinyl, methylpiperazinyl, or dimethylaminopiperidinyl;
R₃, is C₁₋₃ alkyl, -CH₂C(O)N(CH₃)Rₓ, -C(O)CH₂N(CH₃)₂, -C(O)CH₂CH₂N(CH₃)₂, or -C(O)CH₂CH₂NH(CH(CH₃)₂);
R_{3d} is:
(a) -CRₓRₓNRₓRₓ, -CRₓRₓNRₓ(C₂-₅ alkyl), -CH(CH₃)N(CH₃)(CH₂CF₃), -CH₂CH₂S(O)₂CH₃, -CH(CH₃)N(CH₃)CH₂C(O)N(CH₃)₂, -CH(CH₃)NRₓC(O)CH₂N(CH₂CH₃)₂, -CRₓRₓNRₓC(O)CHRₓNRₓR_{y}, -CH(CH₃)N(CH₃)C(O)CH₂NRₓ(C₃₋₄ fluoroalkyl), -NRₓRₓ, -NH(CH(CH₃)₂), -C(O)NH₂, -CRₓRₓQ₁, -CRₓRₓNRₓQ₁, -CRₓRₓNRₓCH₂Q₁, -CRₓRₓNRₓC(O)Q₁, -CRₓRₓNRₓC(O)CRₓRₓQ₁, -CRₓRₓNRₓC(O)CRₓRₓNRₓQ₁, or -CH(CH₃)N(oxetanyl)(C(O)CH₂N(C₁₋₂ alkyl)₂);
(b) azetidinyl substituted with zero to 1 substituent selected form C₁₋₃ alkyl, -CH₂C(CH₃)₂OH, -C(O)CH₂N(CH₃)₂, -N(CH₃)₂, -NHCH(CH₃)₂, oxetanyl, and tetrahydropyranyl;
(c) cyclopropyl or cyclohexyl, each substituted with -NRₓRₓ, -NRₓ(C₂₋₄ alkyl), -NH(oxetanyl), -N(CH₃)CH₂CH₂OCH, -N(CH₃)CH₂C(O)N(CH₃)₂, -N(CH₃)C(O)CH₂N(CH₃)₂, or -N(CH₃)CH₂(ethyloxetanyl); or
(d) morpholinyl, piperazinonyl, piperazinyl, piperidinyl, or pyrrolidinyl, each substituted with zero to 1 substituent selected from C₁₋₅ alkyl, -CH₂CF₃, -CH₂C(CH₃)₂OH, -CH₂CH₂OCH₃, -CH₂C(O)CRₓRₓ, -C(O)CH₂N(CH₃)₂, oxetanyl, methyloxetanyl, and tetrahydropyranyl;
Q₁ is azetidinyl, cyclopropyl, morpholinyl, oxetanyl, tetrahydropyranyl, triazolyl, oxaazaspiro[3.3]heptanyl, piperazinonyl, difluoropiperidinyl, or pyrrolidinyl, each substituted with zero to 2 substituents independently selected from F, -CH₃, -CH₂CH₃, -CH₂OH, and oxetanyl;
R₃ₑ is F;
R_{3f} is:
(a) -OH, -NH₂, -N(CH₃)₂, -NH(CH(CH₃)₂), -NHCH₂C(CH₃)₂OCH₃, -CH₂NH(CH₃), -CH₂N(CH₃)₂, -CH₂NH(CH(CH₃)₂), -CH(CH₃)N(CH₃)₂, or -CH(CH₃)N(CH₃)C(O)CH₂N(CH₃)₂;
(b) cyclohexyl substituted with -NH₂, -N(CH₃)₂, -NRₓ(CH₂CH₂OCH₃), -NH(cyclobutyl), -NH(methyloxetanyl), -NHCH₂(methylsulfonylcyclopropyl), morpholinyl, methoxyazetidinyl, piperazinonyl, difluoropiperidinyl, methoxypiperidinyl, oxaazaspiro[3.3]heptanyl, or oxaazaspiro[3.5]nonanyl;
(c) diazaspiro[3.3]heptanyl substituted with zero to 1 substituent selected from C₁₋₄ alkyl, -CH₂CH₂OCH₃, -CH₂CH₂S(O)₂CH₃, -CH₂(C₃₋₄ cycloalkyl), -CH₂(tetrahydropyranyl), cyclobutyl, oxetanyl, and tetrahydropyranyl;
(d) piperazinyl substituted with zero to 1 substituent selected from C₁-₆ alkyl, -CH₂CH₂CF₃, -CH₂CH₂OCH₃, -CH₂CH₂S(O)₂CH₃, -CH₂(C₃-₄ cycloalkyl), -CH₂(ethyloxetanyl), -CH₂C(O)NH(CH₃), -CH₂C(O)N(CH₃)₂, -C(O)CH₂N(CH₃)₂, -C(O)CH₂N(CH₂CH₃)₂, -CH₂(tetrahydropyranyl), cyclobutyl, oxetanyl, tetrahydropyranyl, and dioxothiotetrahydropyranyl; or
(e) piperidinyl substituted with zero to 1 substituent selected from C₁-₆ alkyl, -CH₂CN, -CH₂CH₂F, -CH₂CH₂CH₂F, -CH₂CH₂CF₃, -CH₂C(CH₃)₂OH, -CH₂CH₂OCH₃, -CH₂CH₂S(O)₂CH₃, -CH₂C(O)NRₓRₓ, -C(O)CH₂N(CH₃)₂, -C(O)CH₂N(CH₂CH₃)₂, -CH₂(C₃-₄ cycloalkyl), -CH₂(tetrahydrofuranyl), -CH₂(tetrahydropyranyl), -CH₂(methyltriazolyl), cyclobutyl, ethoxycyclobutyl, oxetanyl, tetrahydrofuranyl, and tetrahydropyranyl;
R_{3g} is F, -CH₃, or -CF₃;
R₃ₕ is:
(a) -CH(CH₃)N(CH₃)Rₓ, -CH(CH₃)N(CH₃)(C₂₋₃ alkyl), -CH(CH₃)N(CH₃)CH₂C(O)N(CH₃)₂, -CH(CH₃)N(CH₃)C(O)CH₂N(CH₃)₂, or -CH(CH₃)N(CH₃)(tetrahydropyranyl);
(b) cyclohexyl substituted with -N(CH₃)Rₓ, -N(CH₃)(CH(CH₃)₃), -N(CH₃)(CH₂CH₂CF₃), -NRₓ(CH₂CH₂OCH₃), -NH(CH₂C(CH₃)₂OCH₃), -N(CH₃)C(O)CH₂N(CH₃)₂, -NH(cyclobutyl), -NRₓ(oxetanyl), -NH(methyloxetanyl), -NH(tetrahydropyranyl), -NHCH₂(methylsulfonylcyclopropyl), -NHCH₂(methyloxetanyl), methoxyazetidinyl, (trifluoromethyl)hydroxyazetidinyl, morpholinyl, pyrrolidinyl, piperazinonyl, methylsulfonylpiperazinyl, oxazepanyl, oxaazaspiro[3.3]heptanyl, oxaazaspiro[3.5]nonanyl, or dioxothiaazaspiro[3.3]heptanyl;
(c) piperazinyl substituted with zero to two -CH₃; and zero or 1 substituent selected from C₁₋₅ alkyl, -CH₂CN, -CH₂CH₂F, -CH₂CH₂CH₂F, -CH₂C(CH₃)₂OH, -CH₂CH₂OCH₃, -CH₂CH₂S(O)₂CH₃, -CH₂C(O)N(CH₃)Rₓ, -NHC(O)CH₂N(CH₃)₂, -NHC(O)CH₂N(CH₂CH₃)₂, -C(O)CH₂N(CH₃)₂, -C(O)CH₂N(CH₂CH₃)₂, -CH₂(C₃-₄ cycloalkyl), -CH₂(tetrahydropyranyl), -C(O)CH₂(morpholinyl), cyclobutyl, oxetanyl, and tetrahydropyranyl; or
(d) piperidinyl substituted with zero or one -CH₃ and zero or 1 substituent selected from C₁-₆ alkyl, -CH₂CN, -CH₂CH₂F, -CH₂CH₂CH₂F, -CH₂CH₂CF₃, -CH₂C(CH₃)₂OH, -CH₂CH₂OCH₃, -CH₂CH₂OC(CH₃)₃, -CH₂CH₂S(O)₂CH₃, -CH₂C(O)NH₂, -CH₂C(O)N(CH₃)₂, -CH₂C(O)N(CH₃)(CH(CH₃)₂), -C(O)CH₂N(CH₃)₂, -C(O)CH₂N(CH₂CH₃)₂, -C(O)CH₂N(CH₃)(CH₂CH₂OCH₃), -NH₂, -NH(CH₂CH₂CH₃), -NH(CH(CH₃)₂), -NHCH₂CH(CH₃)₂, -N(CH₃)₂, -N(CH₃)(CH₂CH₃), -NH(CH₂C(CH₃)₂OCH₃), -CH₂(C₃₋₆ cycloalkyl), -CH₂(methylsulfonylcyclopropyl), -CH₂(oxetanyl), -CH₂(methyloxetanyl), -CH₂(ethyloxetanyl), -CH₂(tetrahydrofuranyl), -CH₂(tetrahydropyranyl), -CH₂(methyltriazolyl), -CH₂C(O)(oxetanyl), -CH₂C(O)(morpholinyl), -CH₂C(O)(oxaazaspiro[3.3]heptanyl), -C(O)CH₂(methoxyazetidinyl), -C(O)CH₂(morpholinyl), -C(O)CH₂(oxaazaspiro[3.5]nonanyl), -C(O)CH₂(piperidinonyl), -N(CH₂(cyclopropyl))₂, -N(CH₂(tetrahydropyranyl))₂, -NH(methyloxetanyl), -NH(tetrahydropyranyl), -NHC(O)CH₂(morpholinyl), -NHCH₂(cyclopropyl), cyclobutyl, ethoxycyclobutyl, ethoxycyclobutyl, oxetanyl, tetrahydrofuranyl, tetrahydropyranyl, dioxotetrahydrothiophenyl, and (oxetanylamino)piperidinyl;
R₃ᵢ is -CH₃ or -CF₃;
R₃ⱼ is C₁-₆ alkyl, -CH₂CH₂OCH₃, -CH₂CH₂S(O)₂CH₃, -C(O)CH₂N(CH₃)₂, -CH₂(C₃-₄ cycloalkyl), -CH₂(tetrahydropyranyl), -CH(CH₃)(cyclopropyl), cyclobutyl, oxetanyl, tetrahydropyranyl, or isopropylpiperidinyl;
R₃ₖ is C₁₋₄ alkyl, -CH₂C(CH₃)₂OH, -CH₂CH₂OCH₃, -CH₂C(O)NRₓRₓ, -C(O)CH₃, -C(O)CH₂CH(CH₃)OH, -C(O)CH₂N(CH₃)₂, -NRₓ(C₁₋₃ alkyl), -NRₓ(C₃₋₄ fluoroalkyl), -NRₓ(CH₂CH₂OCH₃), -N(CH₃)(CH₂CH₂S(O)₂CH₃), -N(CH₃)(CH₂C(O)N(CH₃)₂), -NRₓ(C(O)CH₂N(CH₃)₂), -N(CH₂CH₂CH₂CF₃)₂, -NRₓ(oxetanyl), -N(CH₃)(methyloxetanyl), -N(CH₃)(tetrahydropyranyl), -NH(ethoxycyclobutyl), oxetanyl, or isopropylpiperidinyl; and
R₅ is hydrogen;
each Rₓ is independently H or -CH₃;
each R_{y} is independently H or C₁-₆ alkyl; and
p is zero, 1 or 2.

2. The compound according to claim 1, N-oxide, or a salt thereof, wherein A is:
(i) -CH₂N(CH₃)Rₓ, -C(O)NRₓRₓ, -C(O)N(CH₃)(CH₂CH₂CN), -C(O)N(CH₂CH₃)(CH₂CH₂CN), or -C(O)N(CH₃)(CH₂CH₂CH₂N(CH₃)₂); or
(ii) -C(O)A₁, -C(O)NRₓ(CRₓRₓ)₀₋₂A₁, -CH₂NHA₁, or -C(O)C(O)NHA₁.

3. The compound according to claim 1, N-oxide, or a salt thereof, wherein A is:
(i) cyclohexyl substituted zero to 1 R_{3b};
(ii) piperidinyl substituted with zero to 1 R_{3c};
(iii) phenyl substituted with zero to 1 R_{3d} and zero to 1 R₃ₑ;
(iv) pyridinyl substituted with zero to 1 R_{3f} and zero to 1 R_{3g};
(v) pyrazinyl or pyrimidinyl, each substituted with zero to 1 R_{3f};
(vi) thiazolyl substituted with R₃ₕ and zero to 1 R₃ᵢ;
(vii) diazabicyclo[2.2.1]heptanyl or diazaspiro[3.3]heptanyl, each substituted with zero to 1 R₃ⱼ; or
(viii) benzo[d]thiazolyl, dihydroisoquinolinyl, tetrahydronaphthyridinyl, tetrahydrobenzo[d]thiazolyl, tetrahydroimidazo[1,2-a]pyrazinyl, tetrahydroisoquinolinonyl, tetrahydroisoquinolinyl, tetrahydronaphthalenyl, tetrahydropyrazolo[1,5-a]pyrazinyl, tetrahydropyrido[4,3-d]pyrimidinyl, tetrahydrothiazolo[4,5-c]pyridinyl, or tetrahydrothiazolo[5,4-c]pyridinyl, each substituted with zero to 1 R₃ₖ.

4. The compound according to claim 1 or a salt thereof, wherein
G is: 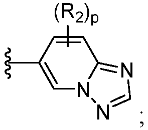
R₁ is -CH(CH₃)₂;
each R₂ is independently -CH₃ or -OCH₃; and
p is 1 or 2.

5. The compound according to claim 4 or a salt thereof, wherein A is:
(i) cyclohexyl substituted zero to 1 R_{3b};
(ii) phenyl substituted with zero to 1 R_{3d} and zero to 1 R₃ₑ;
(iii) pyridinyl substituted with zero to 1 R_{3f} and zero to 1 R_{3g}; and
(iv) thiazolyl substituted with R₃ₕ and zero to 1 R₃ᵢ.

6. The compound according to claim 4 or a salt thereof, wherein:
A is -C(O)NRₓ(CRₓRₓ)₀₋₂A₁; and
A₁ is azetidinyl, cyclohexyl, pyrrolidinyl, piperidinyl, piperazinyl, morpholinyl, tetrahydropyranyl, pyridinyl, diazepanyl, hexahydropyrrolo[3,4-c]pyrrolyl, each substituted with zero to 1 R₃ₐ.

7. The compound according to claim 1, N-oxide, or salt thereof, wherein said compound is:
tert-butyl 4-(4-isopropyl-5-(8-methyl-[1,2,4]triazolo[1,5-a]pyridin-6-yl)-1H-pyrazole-3-carboxamido)piperidine-1-carboxylate (1);
4-isopropyl-N-(1-isopropylpiperidin-4-yl)-5-(8-methyl-[1,2,4]triazolo[1,5-a]pyridin-6-yl)-1H-pyrazole-3-carboxamide (2);
4-isopropyl-5-(8-methyl-[1,2,4]triazolo[1,5-a]pyridin-6-yl)-N-(2-morpholinoethyl)-1H-pyrazole-3-carboxamide (3);
4-isopropyl-5-(8-methyl-[1,2,4]triazolo[1,5-a]pyridin-6-yl)-N-(2-(pyridin-2-yl)ethyl)-1H-pyrazole-3-carboxamide (4);
4-isopropyl-N-(2-methyl-2-morpholinopropyl)-5-(8-m=5-a]pyridin-6-yl)-1H-pyrazole-3-carboxamide (5);
4-isopropyl-N-methyl-5-(8-methyl-[1,2,4]triazolo[1,5-a]pyridin-6-yl)-N-(1-methylpiperidin-4-yl)-1H-pyrazole-3-carboxamide (6);
N-(4-(dimethylamino)cyclohexyl)-4-isopropyl-5-(8-methyl-[1,2,4]triazolo[1,5-a]pyridin-6-yl)-1H-pyrazole-3-carboxamide (7)
4-isopropyl-5-(8-methyl-[1,2,4]triazolo[1,5-a]pyridin-6-yl)-N-(1-methylpiperidin-4-yl)-1H-pyrazole-3-carboxamide (8);
4-isopropyl-5-(8-methoxy-[1,2,4]triazolo[1,5-a]pyridin-6-yl)-N-methyl-N-(1-methylpiperidin-4-yl)-1H-pyrazole-3-carboxamide (9);
(4-isopropyl-5-(8-methoxy-[1,2,4]triazolo[1,5-a]pyridin-6-yl)-1H-pyrazol-3-yl)(4-methyl-1,4-diazepan-1-yl)methanone (10);
4-isopropyl-5-(8-methoxy-[1,2,4]triazolo[1,5-a]pyridin-6-yl)-N-(1-methylpiperidin-4-yl)-1H-pyrazole-3-carboxamide (11);
4-isopropyl-N-(1-isopropylpiperidin-4-yl)-5-(8-methoxy-[1,2,4]triazolo[1,5-a]pyridin-6-yl)-1H-pyrazole-3-carboxamide 912);
(4-(dimethylamino)piperidin-1-yl)(4-isopropyl-5-(8-methyl-[1,2,4]triazolo[1,5-a]pyridin-6-yl)-1H-pyrazol-3-yl)methanone (13);
(4-isopropyl-5-(8-methyl-[1,2,4]triazolo[1,5-a]pyridin-6-yl)-1H-pyrazol-3-yl)(4-(pyridin-4-yl)piperazin-1-yl)methanone (14);
1-(4-isopropyl-5-(8-methyl-[1,2,4]triazolo[1,5-a]pyridin-6-yl)-1H-pyrazole-3-carbonyl)piperidine-4-carboxamide (15);
N-(2-cyanoethyl)-4-isopropyl-N-methyl-5-(8-methyl-[1,2,4]triazolo[1,5-a]pyridin-6-yl)-1H-pyrazole-3-carboxamide (16);
4-isopropyl-N-methyl-5-(8-methyl-[1,2,4]triazolo[1,5-a]pyridin-6-yl)-N-(pyridin-3-ylmethyl)-1H-pyrazole-3-carboxamide (17);
(R)-(3-(dimethylamino)pyrrolidin-1-yl)(4-isopropyl-5-(8-methyl-[1,2,4]triazolo[1,5-a]pyridin-6-yl)-1H-pyrazol-3-yl)methanone (18);
3-(4-(4-isopropyl-5-(8-methyl-[1,2,4]triazolo[1,5-a]pyridin-6-yl)-1H-pyrazole-3-carbonyl)piperazin-1-yl)propanenitrile (19);
4-isopropyl-5-(8-methoxy-[1,2,4]triazolo[1,5-a]pyridin-6-yl)-N-methyl-N-(1-methylpiperidin-4-yl)-1H-pyrazole-3-carboxamide (20);
(3-(dimethylamino)azetidin-1-yl)(4-isopropyl-5-(8-methyl-[1,2,4]triazolo[1,5-a]pyridin-6-yl)-1H-pyrazol-3-yl)methanone (21);
(S)-(3-(dimethylamino)pyrrolidin-1-yl)(4-isopropyl-5-(8-methyl-[1,2,4]triazolo[1,5-a]pyridin-6-yl)-1H-pyrazol-3-yl)methanone (22);
(4-isopropyl-5-(8-methyl-[1,2,4]triazolo[1,5-a]pyridin-6-yl)-1H-pyrazol-3-yl)(4-methyl-1,4-diazepan-1-yl)methanone (23);
N-(3-(dimethylamino)propyl)-4-isopropyl-N-methyl-5-(8-methyl-[1,2,4]triazolo[1,5-a]pyridin-6-yl)-1H-pyrazole-3-carboxamide (24);
4-isopropyl-N-methyl-5-(8-methyl-[1,2,4]triazolo[1,5-a]pyridin-6-yl)-N-(1-methylpyrrolidin-3-yl)-1H-pyrazole-3-carboxamide (25);
4-isopropyl-5-(8-methoxy-[1,2,4]triazolo[1,5-a]pyridin-6-yl)-N-(2-methyl-2-morpholinopropyl)-1H-pyrazole-3-carboxamide (26);
4-isopropyl-N-methyl-5-(8-methyl-[1,2,4]triazolo[1,5-a]pyridin-6-yl)-1H-pyrazole-3-carboxamide (27);
4-isopropyl-5-(8-methyl-[1,2,4]triazolo[1,5-a]pyridin-6-yl)-N-(tetrahydro-2H-pyran-4-yl)-1H-pyrazole-3-carboxamide (28);
4-isopropyl-5-(8-methyl-[1,2,4]triazolo[1,5-a]pyridin-6-yl)-1H-pyrazole-3-carboxamide (29);
(4-isopropyl-5-(8-methyl-[1,2,4]triazolo[1,5-a]pyridin-6-yl)-1H-pyrazol-3-yl)(morpholino)methanone (30);
4-isopropyl-N,N-dimethyl-5-(8-methyl-[1,2,4]triazolo[1,5-a]pyridin-6-yl)-1H-pyrazole-3-carboxamide (31);
N-(4-hydroxycyclohexyl)-4-isopropyl-5-(8-methyl-[1,2,4]triazolo[1,5-a]pyridin-6-yl)-1H-pyrazole-3-carboxamide (32);
5-(7,8-dimethyl-[1,2,4]triazolo[1,5-a]pyridin-6-yl)-4-isopropyl-N-(1-isopropylpiperidin-4-yl)-1H-pyrazole-3-carboxamide (33);
5-(3,4-dimethoxyphenyl)-4-isopropyl-N-(1-isopropylpiperidin-4-yl)-1H-pyrazole-3-carboxamide (34);
4-isopropyl-N-(1-isopropylpiperidin-4-yl)-5-(1H-pyrazolo[3,4-b]pyridin-4-yl)-1H-pyrazole-3-carboxamide (35);
5-(2,6-dimethylpyridin-4-yl)-4-isopropyl-N-(1-isopropylpiperidin-4-yl)-1H-pyrazole-3-carboxamide (36);
4-isopropyl-N-(1-isopropylpiperidin-4-yl)-5-(8-methylimidazo[1,2-a]pyridin-6-yl)-1H-pyrazole-3-carboxamide (37);
4-isopropyl-N-(1-isopropylpiperidin-4-yl)-5-(2-methylpyridin-4-yl)-1H-pyrazole-3-carboxamide (38);
5-(1,5-dimethyl-6-oxo-1,6-dihydropyridin-3-yl)-4-isopropyl-N-(1-isopropylpiperidin-4-yl)-1H-pyrazole-3-carboxamide (39);
5-(8-cyanoquinolin-5-yl)-4-isopropyl-N-(1-isopropylpiperidin-4-yl)-1H-pyrazole-3-carboxamide (40);
4-isopropyl-5-(1-methyl-1H-pyrrolo[2,3-b]pyridin-3-yl)-N-(1-methylpiperidin-4-yl)-1H-pyrazole-3-carboxamide (41);
4-isopropyl-N-(1-isopropylpiperidin-4-yl)-5-(1-methyl-1H-pyrrolo[2,3-b]pyridin-3-yl)-1H-pyrazole-3-carboxamide (42);
4-isopropyl-5-(8-methyl-[1,2,4]triazolo[1,5-a]pyridin-6-yl)-N-(piperidin-4-yl)-1H-pyrazole-3-carboxamide (43);
4-isopropyl-5-(8-methyl-[1,2,4]triazolo[1,5-a]pyridin-6-yl)-N-(piperidin-4-ylmethyl)-1H-pyrazole-3-carboxamide (45);
N-((1r,4r)-4-aminocyclohexyl)-4-isopropyl-5-(8-methyl-[1,2,4]triazolo[1,5-a]pyridin-6-yl)-1H-pyrazole-3-carboxamide (46);
N-(((1r,4r)-4-aminocyclohexyl)methyl)-4-isopropyl-5-(8-methyl-[1,2,4]triazolo[1,5-a]pyridin-6-yl)-1H-pyrazole-3-carboxamide (47);
4-isopropyl-5-(8-methyl-[1,2,4]triazolo[1,5-a]pyridin-6-yl)-N-(morpholin-2-ylmethyl)-1H-pyrazole-3-carboxamide (48);
N-(azetidin-3-ylmethyl)-4-isopropyl-5-(8-methyl-[1,2,4]triazolo[1,5-a]pyridin-6-yl)-1H-pyrazole-3-carboxamide (49);
(R)-4-isopropyl-5-(8-methyl-[1,2,4]triazolo[1,5-a]pyridin-6-yl)-N-(pyrrolidin-3-yl)-1H-pyrazole-3-carboxamide (50);
(S)-4-isopropyl-5-(8-methyl-[1,2,4]triazolo[1,5-a]pyridin-6-yl)-N-(pyrrolidin-2-ylmethyl)-1H-pyrazole-3-carboxamide (51);
(R)-4-isopropyl-5-(8-methyl-[1,2,4]triazolo[1,5-a]pyridin-6-yl)-N-(piperidin-3-yl)-1H-pyrazole-3-carboxamide (52);
(S)-(3-aminopyrrolidin-1-yl)(4-isopropyl-5-(8-methyl-[1,2,4]triazolo[1,5-a]pyridin-6-yl)-1H-pyrazol-3-yl)methanone (53);
(S)-4-isopropyl-5-(8-methyl-[1,2,4]triazolo[1,5-a]pyridin-6-yl)-N-(piperidin-3-yl)-1H-pyrazole-3-carboxamide (54);
(4-aminopiperidin-1-yl)(4-isopropyl-5-(8-methyl-[1,2,4]triazolo[1,5-a]pyridin-6-yl)-1H-pyrazol-3-yl)methanone (55);
4-isopropyl-5-(8-methyl-[1,2,4]triazolo[1,5-a]pyridin-6-yl)-N-(piperidin-2-ylmethyl)-1H-pyrazole-3-carboxamide (56);
(4-isopropyl-5-(8-methyl-[1,2,4]triazolo[1,5-a]pyridin-6-yl)-1H-pyrazol-3-yl)(piperazin-1-yl)methanone (57);
N-(azetidin-3-yl)-4-isopropyl-5-(8-methyl-[1,2,4]triazolo[1,5-a]pyridin-6-yl)-1H-pyrazole-3-carboxamide (58);
(hexahydropyrrolo[3,4-c]pyrrol-2(1H)-yl)(4-isopropyl-5-(8-methyl-[1,2,4]triazolo[1,5-a]pyridin-6-yl)-1H-pyrazol-3-yl)methanone (59);
4-isopropyl-5-(8-methoxy-[1,2,4]triazolo[1,5-a]pyridin-6-yl)-N-(piperidin-4-yl)-1H-pyrazole-3-carboxamide (60);
(4-(aminomethyl)piperidin-1-yl)(4-isopropyl-5-(8-methyl-[1,2,4]triazolo[1,5-a]pyridin-6-yl)-1H-pyrazol-3-yl)methanone (61);
4-isopropyl-5-(8-methoxy-[1,2,4]triazolo[1,5-a]pyridin-6-yl)-N-(piperidin-4-ylmethyl)-1H-pyrazole-3-carboxamide (62);
N-((1s,4s)-4-aminocyclohexyl)-4-isopropyl-5-(8-methyl-[1,2,4]triazolo[1,5-a]pyridin-6-yl)-1H-pyrazole-3-carboxamide (63);
4-isopropyl-5-(8-methyl-[1,2,4]triazolo[1,5-a]pyridin-6-yl)-N-(morpholin-2-ylmethyl)-1H-pyrazole-3-carboxamide (64);
4-isopropyl-5-(8-methyl-[1,2,4]triazolo[1,5-a]pyridin-6-yl)-N-(morpholin-2-ylmethyl)-1H-pyrazole-3-carboxamide (65);
4-isopropyl-5-(8-methyl-[1,2,4]triazolo[1,5-a]pyridin-6-yl)-N-(piperidin-3-yl)-1H-pyrazole-3-carboxamide (66);
4-isopropyl-5-(8-methyl-[1,2,4]triazolo[1,5-a]pyridin-6-yl)-N-(piperidin-3-yl)-1H-pyrazole-3-carboxamide (67);
4-isopropyl-5-(1-methyl-1H-pyrrolo[2,3-b]pyridin-3-yl)-N-(piperidin-4-yl)-1H-pyrazole-3-carboxamide (68);
4-isopropyl-5-(8-methyl-[1,2,4]triazolo[1,5-a]pyridin-6-yl)-N-(1-(3,3,3-trifluoro propyl) piperidin-4-yl)-1H-pyrazole-3-carboxamide (69);
4-isopropyl-5-(8-methyl-[1,2,4]triazolo[1,5-a]pyridin-6-yl)-N-(1-(oxetan-3-yl)piperidin-4-yl)-1H-pyrazole-3-carboxamide (70);
4-isopropyl-5-(8-methyl-[1,2,4]triazolo[1,5-a]pyridin-6-yl)-N-(1-(4,4,4-trifluorobutyl)piperidin-4-yl)-1H-pyrazole-3-carboxamide (71);
4-isopropyl-5-(8-methyl-[1,2,4]triazolo[1,5-a]pyridin-6-yl)-N-(1-((tetrahydrofuran-3-yl)methyl)piperidin-4-yl)-1H-pyrazole-3-carboxamide (72);
N-(1-(cyclopropylmethyl)piperidin-4-yl)-4-isopropyl-5-(8-methyl-[1,2,4]triazolo[1,5-a]pyridin-6-yl)-1H-pyrazole-3-carboxamide (73);
4-isopropyl-5-(8-methyl-[1,2,4]triazolo[1,5-a]pyridin-6-yl)-N-(1-neopentylpiperidin-4-yl)-1H-pyrazole-3-carboxamide (74);
N-(1-(3,3-dimethylbutyl)piperidin-4-yl)-4-isopropyl-5-(8-methyl-[1,2,4]triazolo[1,5-a]pyridin-6-yl)-1H-pyrazole-3-carboxamide (75);
N-(1-isopentylpiperidin-4-yl)-4-isopropyl-5-(8-methyl-[1,2,4]triazolo[1,5-a]pyridin-6-yl)-1H-pyrazole-3-carboxamide (76);
4-isopropyl-5-(8-methyl-[1,2,4]triazolo[1,5-a]pyridin-6-yl)-N-(1-((tetrahydro-2H-pyran-4-yl)methyl)piperidin-4-yl)-1H-pyrazole-3-carboxamide (77);
N-(1-((3,5-dimethylisoxazol-4-yl)methyl)piperidin-4-yl)-4-isopropyl-5-(8-methyl-[1,2,4]triazolo[1,5-a]pyridin-6-yl)-1H-pyrazole-3-carboxamide (78);
N-(1-(2-ethylbutyl)piperidin-4-yl)-4-isopropyl-5-(8-methyl-[1,2,4]triazolo[1,5-a]pyridin-6-yl)-1H-pyrazole-3-carboxamide (79);
4-isopropyl-5-(8-methyl-[1,2,4]triazolo[1,5-a]pyridin-6-yl)-N-((1r,4r)-4-(oxetan-3-ylamino)cyclohexyl)-1H-pyrazole-3-carboxamide (80);
4-isopropyl-N-((1r,4r)-4-(isopropylamino)cyclohexyl)-5-(8-methyl-[1,2,4]triazolo[1,5-a]pyridin-6-yl)-1H-pyrazole-3-carboxamide (81);
N-((1r,4r)-4-(dimethylamino)cyclohexyl)-4-isopropyl-5-(8-methyl-[1,2,4]triazolo[1,5-a]pyridin-6-yl)-1H-pyrazole-3-carboxamide (82);
N-(1-(2-(dimethylamino)ethyl)piperidin-4-yl)-4-isopropyl-5-(8-methyl-[1,2,4]triazolo[1,5-a]pyridin-6-yl)-1H-pyrazole-3-carboxamide (83);
N-((1s,4s)-4-(dimethylamino)cyclohexyl)-4-isopropyl-5-(8-methyl-[1,2,4]triazolo[1,5-a]pyridin-6-yl)-1H-pyrazole-3-carboxamide (84);
4-isopropyl-N-((1s,4s)-4-(isopropylamino)cyclohexyl)-5-(8-methyl-[1,2,4]triazolo[1,5-a]pyridin-6-yl)-1H-pyrazole-3-carboxamide (85);
4-isopropyl-5-(8-methyl-[1,2,4]triazolo[1,5-a]pyridin-6-yl)-N-((1s,4s)-4-(oxetan-3-ylamino)cyclohexyl)-1H-pyrazole-3-carboxamide (86);
4-isopropyl-5-(8-methyl-[1,2,4]triazolo[1,5-a]pyridin-6-yl)-N-((1s,4s)-4-(((tetrahydrofuran-3-yl)methyl)amino)cyclohexyl)-1H-pyrazole-3-carboxamide (87);
4-isopropyl-5-(8-methyl-[1,2,4]triazolo[1,5-a]pyridin-6-yl)-N-((1s,4s)-4-((4,4,4-trifluorobutyl)amino)cyclohexyl)-1H-pyrazole-3-carboxamide (88);
4-isopropyl-5-(8-methyl-[1,2,4]triazolo[1,5-a]pyridin-6-yl)-N-((1s,4s)-4-(neopentylamino)cyclohexyl)-1H-pyrazole-3-carboxamide (89);
N-((1s,4s)-4-(bis(3,3-dimethylbutyl)amino)cyclohexyl)-4-isopropyl-5-(8-methyl-[1,2,4]triazolo[1,5-a]pyridin-6-yl)-1H-pyrazole-3-carboxamide (90);
N-((1s,4s)-4-(diisopentylamino)cyclohexyl)-4-isopropyl-5-(8-methyl-[1,2,4]triazolo[1,5-a]pyridin-6-yl)-1H-pyrazole-3-carboxamide (91);
4-isopropyl-5-(8-methyl-[1,2,4]triazolo[1,5-a]pyridin-6-yl)-N-((1s,4s)-4-(((tetrahydro-2H-pyran-4-yl)methyl)amino)cyclohexyl)-1H-pyrazole-3-carboxamide (92);
N-((1s,4s)-4-(((3,5-dimethylisoxazol-4-yl)methyl)amino)cyclohexyl)-4-isopropyl-5-(8-methyl-[1,2,4]triazolo[1,5-a]pyridin-6-yl)-1H-pyrazole-3-carboxamide (93);
N-((1s,4s)-4-((cyclopentylmethyl)amino)cyclohexyl)-4-isopropyl-5-(8-methyl-[1,2,4]triazolo[1,5-a]pyridin-6-yl)-1H-pyrazole-3-carboxamide (94);
4-isopropyl-5-(8-methyl-[1,2,4]triazolo[1,5-a]pyridin-6-yl)-N-((1s,4s)-4-((pyridin-3-ylmethyl)amino)cyclohexyl)-1H-pyrazole-3-carboxamide (95);
4-isopropyl-N-((1s,4s)-4-(((2-methoxypyrimidin-5-yl)methyl)amino)cyclohexyl)-5-(8-methyl-[1,2,4]triazolo[1,5-a]pyridin-6-yl)-1H-pyrazole-3-carboxamide (96);
N-((1s,4s)-4-((2,2-dimethyltetrahydro-2H-pyran-4-yl)amino)cyclohexyl)-4-isopropyl-5-(8-methyl-[1,2,4]triazolo[1,5-a]pyridin-6-yl)-1H-pyrazole-3-carboxamide (97);
4-isopropyl-5-(8-methyl-[1,2,4]triazolo[1,5-a]pyridin-6-yl)-N-((1s,4s)-4-((tetrahydro-2H-pyran-4-yl)amino)cyclohexyl)-1H-pyrazole-3-carboxamide (98);
4-isopropyl-5-(8-methyl-[1,2,4]triazolo[1,5-a]pyridin-6-yl)-N-((1s,4s)-4-((3-methylbutan-2-yl)amino)cyclohexyl)-1H-pyrazole-3-carboxamide (99);
4-isopropyl-5-(8-methyl-[1,2,4]triazolo[1,5-a]pyridin-6-yl)-N-((1s,4s)-4-(pentan-3-ylamino)cyclohexyl)-1H-pyrazole-3-carboxamide (100);
N-((1s,4s)-4-((2-ethylbutyl)amino)cyclohexyl)-4-isopropyl-5-(8-methyl-[1,2,4]triazolo[1,5-a]pyridin-6-yl)-1H-pyrazole-3-carboxamide (101);
4-isopropyl-5-(8-methyl-[1,2,4]triazolo[1,5-a]pyridin-6-yl)-N-((1s,4s)-4-((pyrimidin-5-ylmethyl)amino)cyclohexyl)-1H-pyrazole-3-carboxamide (102);
N-((1r,4r)-4-(bis(cyclopropylmethyl)amino)cyclohexyl)-4-isopropyl-5-(8-methyl-[1,2,4]triazolo[1,5-a]pyridin-6-yl)-1H-pyrazole-3-carboxamide (103);
4-isopropyl-5-(8-methyl-[1,2,4]triazolo[1,5-a]pyridin-6-yl)-N-((1r,4r)-4-(((2-methylpyrimidin-5-yl)methyl)amino)cyclohexyl)-1H-pyrazole-3-carboxamide (104);
4-isopropyl-5-(8-methyl-[1,2,4]triazolo[1,5-a]pyridin-6-yl)-N-((1r,4r)-4-(((tetrahydrofuran-3-yl)methyl)amino)cyclohexyl)-1H-pyrazole-3-carboxamide (105);
4-isopropyl-5-(8-methyl-[1,2,4]triazolo[1,5-a]pyridin-6-yl)-N-((1r,4r)-4-(neopentylamino)cyclohexyl)-1H-pyrazole-3-carboxamide
N-((1r,4r)-4-(bis(3,3-dimethylbutyl)amino)cyclohexyl)-4-isopropyl-5-(8-methyl-[1,2,4]triazolo[1,5-a]pyridin-6-yl)-1H-pyrazole-3-carboxamide (107);
N-((1r,4r)-4-(diisopentylamino)cyclohexyl)-4-isopropyl-5-(8-methyl-[1,2,4]triazolo[1,5-a]pyridin-6-yl)-1H-pyrazole-3-carboxamide (108);
N-((1r,4r)-4-((cyclopentylmethyl)amino)cyclohexyl)-4-isopropyl-5-(8-methyl-[1,2,4]triazolo[1,5-a]pyridin-6-yl)-1H-pyrazole-3-carboxamide (109);
N-((1r,4r)-4-((2-ethylbutyl)amino)cyclohexyl)-4-isopropyl-5-(8-methyl-[1,2,4]triazolo[1,5-a]pyridin-6-yl)-1H-pyrazole-3-carboxamide (110);
4-isopropyl-5-(8-methyl-[1,2,4]triazolo[1,5-a]pyridin-6-yl)-N-((1r,4r)-4-(((tetrahydro-2H-pyran-4-yl)methyl)amino)cyclohexyl)-1H-pyrazole-3-carboxamide (111);
4-isopropyl-5-(8-methyl-[1,2,4]triazolo[1,5-a]pyridin-6-yl)-N-((1r,4r)-4-((pyridin-3-ylmethyl)amino)cyclohexyl)-1H-pyrazole-3-carboxamide (112);
4-isopropyl-5-(8-methyl-[1,2,4]triazolo[1,5-a]pyridin-6-yl)-N-((1r,4r)-4-((pyrimidin-5-ylmethyl)amino)cyclohexyl)-1H-pyrazole-3-carboxamide (113);
4-isopropyl-N-((1r,4r)-4-(((2-methoxypyrimidin-5-yl)methyl)amino)cyclohexyl)-5-(8-methyl-[1,2,4]triazolo[1,5-a]pyridin-6-yl)-1H-pyrazole-3-carboxamide (114);
N-((1r,4r)-4-((2,2-dimethyltetrahydro-2H-pyran-4-yl)amino)cyclohexyl)-4-isopropyl-5-(8-methyl-[1,2,4]triazolo[1,5-a]pyridin-6-yl)-1H-pyrazole-3-carboxamide (115);
4-isopropyl-5-(8-methyl-[1,2,4]triazolo[1,5-a]pyridin-6-yl)-N-((1r,4r)-4-((tetrahydro-2H-pyran-4-yl)amino)cyclohexyl)-1H-pyrazole-3-carboxamide (116);
4-isopropyl-5-(8-methyl-[1,2,4]triazolo[1,5-a]pyridin-6-yl)-N-((1r,4r)-4-((3-methylbutan-2-yl)amino)cyclohexyl)-1H-pyrazole-3-carboxamide (118);
4-isopropyl-5-(8-methyl-[1,2,4]triazolo[1,5-a]pyridin-6-yl)-N-((1r,4r)-4-(pentan-3-ylamino)cyclohexyl)-1H-pyrazole-3-carboxamide (119);
N-((1r,4r)-4-((4,4-dimethylpentan-2-yl)amino)cyclohexyl)-4-isopropyl-5-(8-methyl-[1,2,4]triazolo[1,5-a]pyridin-6-yl)-1H-pyrazole-3-carboxamide (120);
4-isopropyl-5-(8-methyl-[1,2,4]triazolo[1,5-a]pyridin-6-yl)-N-(4-((3-methyloxetan-3-yl)amino)cyclohexyl)-1H-pyrazole-3-carboxamide (123-124);
4-isopropyl-5-(8-methyl-[1,2,4]triazolo[1,5-a]pyridin-6-yl)-N-(1-(3-methylbutan-2-yl)piperidin-4-yl)-1H-pyrazole-3-carboxamide (125);
4-isopropyl-5-(8-methyl-[1,2,4]triazolo[1,5-a]pyridin-6-yl)-N-(4-(neopentylamino)cyclohexyl)-1H-pyrazole-3-carboxamide (126 and 131);
4-isopropyl-N-(4-(isopropyl(methyl)amino)cyclohexyl)-5-(8-methyl-[1,2,4]triazolo[1,5-a]pyridin-6-yl)-1H-pyrazole-3-carboxamide (127 and 132);
4-isopropyl-N-(4-((2-methoxyethyl)(methyl)amino)cyclohexyl)-5-(8-methyl-[1,2,4]triazolo[1,5-a]pyridin-6-yl)-1H-pyrazole-3-carboxamide (128);
4-isopropyl-5-(8-methyl-[1,2,4]triazolo[1,5-a]pyridin-6-yl)-N-(4-(methylamino)cyclohexyl)-1H-pyrazole-3-carboxamide (129-130);
4-isopropyl-5-(8-methyl-[1,2,4]triazolo[1,5-a]pyridin-6-yl)-N-(1-((3-methyloxetan-3-yl)methyl)piperidin-4-yl)-1H-pyrazole-3-carboxamide (133);
4-isopropyl-5-(8-methyl-[1,2,4]triazolo[1,5-a]pyridin-6-yl)-N-((4-methylmorpholin-2-yl)methyl)-1H-pyrazole-3-carboxamide
4-isopropyl-N-((4-isopropylmorpholin-2-yl)methyl)-5-(8-methyl-[1,2,4]triazolo[1,5-a]pyridin-6-yl)-1H-pyrazole-3-carboxamide (135);
4-isopropyl-5-(8-methyl-[1,2,4]triazolo[1,5-a]pyridin-6-yl)-N-((4-(oxetan-3-yl)morpholin-2-yl)methyl)-1H-pyrazole-3-carboxamide (136 and 142);
4-isopropyl-5-(8-methyl-[1,2,4]triazolo[1,5-a]pyridin-6-yl)-N-((4-((3-methyloxetan-3-yl)methyl)morpholin-2-yl)methyl)-1H-pyrazole-3-carboxamide (137);
N-(1-(2-hydroxy-2-methylpropyl)piperidin-4-yl)-4-isopropyl-5-(8-methyl-[1,2,4]triazolo[1,5-a]pyridin-6-yl)-1H-pyrazole-3-carboxamide (138);
4-isopropyl-5-(8-methyl-[1,2,4]triazolo[1,5-a]pyridin-6-yl)-N-((4-methylmorpholin-2-yl)methyl)-1H-pyrazole-3-carboxamide (139);
N-((4-ethylmorpholin-2-yl)methyl)-4-isopropyl-5-(8-methyl-[1,2,4]triazolo[1,5-a]pyridin-6-yl)-1H-pyrazole-3-carboxamide (140);
4-isopropyl-N-((4-isopropylmorpholin-2-yl)methyl)-5-(8-methyl-[1,2,4]triazolo[1,5-a]pyridin-6-yl)-1H-pyrazole-3-carboxamide (141);
N-((4-(2-hydroxy-2-methylpropyl)morpholin-2-yl)methyl)-4-isopropyl-5-(8-methyl-[1,2,4]triazolo[1,5-a]pyridin-6-yl)-1H-pyrazole-3-carboxamide (143);
4-isopropyl-5-(8-methyl-[1,2,4]triazolo[1,5-a]pyridin-6-yl)-N-((4-neopentylmorpholin-2-yl)methyl)-1H-pyrazole-3-carboxamide (144-145);
4-isopropyl-N-((1r,4r)-4-((1-methoxypropan-2-yl)amino)cyclohexyl)-5-(8-methyl-[1,2,4]triazolo[1,5-a]pyridin-6-yl)-1H-pyrazole-3-carboxamide (146);
4-isopropyl-N-((1s,4s)-4-((1-methoxypropan-2-yl)amino)cyclohexyl)-5-(8-methyl-[1,2,4]triazolo[1,5-a]pyridin-6-yl)-1H-pyrazole-3-carboxamide (147);
N-((1r,4r)-4-((3,3-dimethylbutan-2-yl)amino)cyclohexyl)-4-isopropyl-5-(8-methyl-[1,2,4]triazolo[1,5-a]pyridin-6-yl)-1H-pyrazole-3-carboxamide (148);
4-isopropyl-5-(8-methyl-[1,2,4]triazolo[1,5-a]pyridin-6-yl)-N-((1r,4r)-4-(((3-methyloxetan-3-yl)methyl)amino)cyclohexyl)-1H-pyrazole-3-carboxamide (149);
N-((1s,4s)-4-((3,3-dimethylbutan-2-yl)amino)cyclohexyl)-4-isopropyl-5-(8-methyl-[1,2,4]triazolo[1,5-a]pyridin-6-yl)-1H-pyrazole-3-carboxamide (150);
4-isopropyl-5-(8-methyl-[1,2,4]triazolo[1,5-a]pyridin-6-yl)-N-((1s,4s)-4-(((3-methyloxetan-3-yl)methyl)amino)cyclohexyl)-1H-pyrazole-3-carboxamide (151);
N-(1-(sec-butyl)piperidin-4-yl)-4-isopropyl-5-(8-methyl-[1,2,4]triazolo[1,5-a]pyridin-6-yl)-1H-pyrazole-3-carboxamide (152);
4-isopropyl-5-(8-methyl-[1,2,4]triazolo[1,5-a]pyridin-6-yl)-N-(1-(tetrahydro-2H-pyran-4-yl)piperidin-4-yl)-1H-pyrazole-3-carboxamide (153);
4-isopropyl-5-(8-methyl-[1,2,4]triazolo[1,5-a]pyridin-6-yl)-N-(1-((3-methyloxetan-3-yl)methyl)azetidin-3-yl)-1H-pyrazole-3-carboxamide (154);
4-isopropyl-N-(1-isopropylazetidin-3-yl)-5-(8-methyl-[1,2,4]triazolo[1,5-a]pyridin-6-yl)-1H-pyrazole-3-carboxamide (155);
4-isopropyl-5-(8-methyl-[1,2,4]triazolo[1,5-a]pyridin-6-yl)-N-(1-methylazetidin-3-yl)-1H-pyrazole-3-carboxamide (156);
4-isopropyl-N-(1-((2-methoxypyrimidin-5-yl)methyl)piperidin-4-yl)-5-(8-methyl-[1,2,4]triazolo[1,5-a]pyridin-6-yl)-1H-pyrazole-3-carboxamide (157);
4-isopropyl-N-(1-((1-methyl-1H-1,2,4-triazol-3-yl)methyl)piperidin-4-yl)-5-(8-methyl-[1,2,4]triazolo[1,5-a]pyridin-6-yl)-1H-pyrazole-3-carboxamide (158);
4-isopropyl-N-(1-((1-methyl-1H-1,2,4-triazol-3-yl)methyl)azetidin-3-yl)-5-(8-methyl-[1,2,4]triazolo[1,5-a]pyridin-6-yl)-1H-pyrazole-3-carboxamide (159);
(R)-4-isopropyl-5-(8-methyl-[1,2,4]triazolo[1,5-a]pyridin-6-yl)-N-(1-methylpiperidin-3-yl)-1H-pyrazole-3-carboxamide (160);
(R)-4-isopropyl-N-(1-isopropylpiperidin-3-yl)-5-(8-methyl-[1,2,4]triazolo[1,5-a]pyridin-6-yl)-1H-pyrazole-3-carboxamide (161);
(S)-4-isopropyl-5-(8-methyl-[1,2,4]triazolo[1,5-a]pyridin-6-yl)-N-(1-methylpiperidin-3-yl)-1H-pyrazole-3-carboxamide (162);
(S)-4-isopropyl-N-(1-isopropylpiperidin-3-yl)-5-(8-methyl-[1,2,4]triazolo[1,5-a]pyridin-6-yl)-1H-pyrazole-3-carboxamide (163);
(S)-N-(1-ethylpiperidin-3-yl)-4-isopropyl-5-(8-methyl-[1,2,4]triazolo[1,5-a]pyridin-6-yl)-1H-pyrazole-3-carboxamide (164);
4-isopropyl-N-((3S)-1-((3-methyl-3H-1,2,4-triazol-5-yl)methyl)piperidin-3-yl)-5-(8-methyl-[1,2,4]triazolo[1,5-a]pyridin-6-yl)-1H-pyrazole-3-carboxamide (165);
(S)-4-isopropyl-5-(8-methyl-[1,2,4]triazolo[1,5-a]pyridin-6-yl)-N-(1-(oxetan-3-yl)piperidin-3-yl)-1H-pyrazole-3-carboxamide (166);
4-isopropyl-5-(1-methyl-1H-pyrrolo[2,3-b]pyridin-3-yl)-N-(1-(oxetan-3-yl)piperidin-4-yl)-1H-pyrazole-3-carboxamide (167);
4-isopropyl-5-(1-methyl-1H-pyrrolo[2,3-b]pyridin-3-yl)-N-(1-neopentylpiperidin-4-yl)-1H-pyrazole-3-carboxamide (168);
4-isopropyl-5-(8-methyl-[1,2,4]triazolo[1,5-a]pyridin-6-yl)-N-(1-(3,3,3-trifluoropropyl)piperidin-4-yl)-1H-pyrazole-3-carboxamide (169);
4-isopropyl-5-(8-methyl-[1,2,4]triazolo[1,5-a]pyridin-6-yl)-N-(1-(2-(methylamino)ethyl)piperidin-4-yl)-1H-pyrazole-3-carboxamide (170);
4-isopropyl-N-(1-(2-methoxyethyl)piperidin-4-yl)-5-(8-methyl-[1,2,4]triazolo [1,5-a]pyridin-6-yl)-1H-pyrazole-3-carboxamide (171);
4-isopropyl-5-(8-methyl-[1,2,4]triazolo[1,5-a]pyridin-6-yl)-N-(1-(2-(methylsulfonyl)ethyl)piperidin-4-yl)-1H-pyrazole-3-carboxamide (172);
4-isopropyl-5-(8-methyl-[1,2,4]triazolo[1,5-a]pyridin-6-yl)-N-(1-(2,2,2-trifluoroethyl)piperidin-4-yl)-1H-pyrazole-3-carboxamide (173);
4-isopropyl-5-(8-methyl-[1,2,4]triazolo[1,5-a]pyridin-6-yl)-N-(1-(1-(methyl sulfonyl)propan-2-yl)piperidin-4-yl)-1H-pyrazole-3-carboxamide (174);
N-(1-(2-(dimethylamino)-2-oxoethyl)piperidin-4-yl)-4-isopropyl-5-(8-methyl-[1,2,4]triazolo [1,5-a]pyridin-6-yl)-1H-pyrazole-3-carboxamide (176);
4-isopropyl-5-(8-methyl-[1,2,4]triazolo[1,5-a]pyridin-6-yl)-N-(1-(2-(methylamino)-2-oxoethyl)piperidin-4-yl)-1H-pyrazole-3-carboxamide (177);
N-(1-isopropylpiperidin-4-yl)-4-methyl-5-(8-methyl-[1,2,4]triazolo[1,5-a]pyridin-6-yl)-1H-pyrazole-3-carboxamide (179);
4-methyl-5-(8-methyl-[1,2,4]triazolo[1,5-a]pyridin-6-yl)-N-(1-methylpiperidin-4-yl)-1H-pyrazole-3-carboxamide (180);
4-ethyl-5-(8-methyl-[1,2,4]triazolo[1,5-a]pyridin-6-yl)-N-(1-methylpiperidin-4-yl)-1H-pyrazole-3-carboxamide (181);
4-ethyl-N-(1-isopropylpiperidin-4-yl)-5-(8-methyl-[1,2,4]triazolo[1,5-a]pyridin-6-yl)-1H-pyrazole-3-carboxamide (182);
2-(4-isopropyl-5-(8-methyl-[1,2,4]triazolo[1,5-a]pyridin-6-yl)-1H-pyrazol-3-yl)-N-(1-isopropylpiperidin-4-yl)-2-oxoacetamide (183);
1-(4-isopropyl-5-(8-methyl-[1,2,4]triazolo[1,5-a]pyridin-6-yl)-1H-pyrazol-3-yl)-N,N-dimethyl methanamine (184);
1-(4-isopropyl-5-(8-methyl-[1,2,4]triazolo[1,5-a]pyridin-6-yl)-1H-pyrazol-3-yl)-N-methylmethanamine (185);
1-isopropyl-N-((4-isopropyl-5-(8-methyl-[1,2,4]triazolo[1,5-a]pyridin-6-yl)-1H-pyrazol-3-yl)methyl)piperidin-4-amine (186);
N-((4-isopropyl-5-(8-methyl-[1,2,4]triazolo[1,5-a]pyridin-6-yl)-1H-pyrazol-3-yl)methyl)piperidin-4-amine (187);
5-(8-methoxy-[1,2,4]triazolo[1,5-a]pyridin-6-yl)-N-(piperidin-4-yl)-4-(2,2,2-trifluoroethyl)-1H-pyrazole-3-carboxamide (190);
N-(1-isopropylpiperidin-4-yl)-5-(8-methoxy-[1,2,4]triazolo[1,5-a]pyridin-6-yl)-4-(2,2,2-trifluoroethyl)-1H-pyrazole-3-carboxamide (191);
5-(8-methoxy-[1,2,4]triazolo[1,5-a]pyridin-6-yl)-N-(1-methylpiperidin-4-yl)-4-(2,2,2-trifluoroethyl)-1H-pyrazole-3-carboxamide (192);
N-(1-isopropylpiperidin-4-yl)-5-(8-methyl-[1,2,4]triazolo[1,5-a]pyridin-6-yl)-4-(2,2,2-trifluoroethyl)-1H-pyrazole-3-carboxamide (193);
5-(8-methyl-[1,2,4]triazolo[1,5-a]pyridin-6-yl)-N-(piperidin-4-yl)-4-(2,2,2-trifluoroethyl)-1H-pyrazole-3-carboxamide (194);
5-(8-methoxy-[1,2,4]triazolo[1,5-a]pyridin-6-yl)-N-(1-propylpiperidin-4-yl)-4-(2,2,2-trifluoroethyl)-1H-pyrazole-3-carboxamide (195);
5-(8-methoxy-[1,2,4]triazolo[1,5-a]pyridin-6-yl)-N-(1-neopentylpiperidin-4-yl)-4-(2,2,2-trifluoroethyl)-1H-pyrazole-3-carboxamide (196);
5-(8-methoxy-[1,2,4]triazolo[1,5-a]pyridin-6-yl)-N-(1-((tetrahydro-2H-pyran-4-yl)methyl)piperidin-4-yl)-4-(2,2,2-trifluoroethyl)-1H-pyrazole-3-carboxamide (197);
N-(1'-isopropyl-[ 1,4'-bipiperidin]-4-yl)-5-(8-methoxy-[1,2,4]triazolo[1,5-a]pyridin-6-yl)-4-(2,2,2-trifluoroethyl)-1H-pyrazole-3-carboxamide (199);
5-(8-methyl-[1,2,4]triazolo[1,5-a]pyridin-6-yl)-N-(1-(oxetan-3-yl)piperidin-4-yl)-4-(2,2,2-trifluoroethyl)-1H-pyrazole-3-carboxamide (200);
5-(8-methyl-[1,2,4]triazolo[1,5-a]pyridin-6-yl)-N-(1-(tetrahydro-2H-pyran-4-yl)piperidin-4-yl)-4-(2,2,2-trifluoroethyl)-1H-pyrazole-3-carboxamide (201);
N-(1-isobutylpiperidin-4-yl)-5-(8-methyl-[1,2,4]triazolo[1,5-a]pyridin-6-yl)-4-(2,2,2-trifluoroethyl)-1H-pyrazole-3-carboxamide (202);
N-(1-cyclobutylpiperidin-4-yl)-5-(8-methyl-[1,2,4]triazolo[1,5-a]pyridin-6-yl)-4-(2,2,2-trifluoroethyl)-1H-pyrazole-3-carboxamide (203);
5-(8-methyl-[1,2,4]triazolo[1,5-a]pyridin-6-yl)-N-(1-((tetrahydro-2H-pyran-4-yl)methyl)piperidin-4-yl)-4-(2,2,2-trifluoroethyl)-1H-pyrazole-3-carboxamide (204);
5-(8-methoxy-[1,2,4]triazolo[1,5-a]pyridin-6-yl)-N-(1-(2-(methylamino)-2-oxoethyl) piperidin-4-yl)-4-(2,2,2-trifluoroethyl)-1H-pyrazole-3-carboxamide (205);
6-(4-isopropyl-3-(1-methylpiperidin-4-yl)-1H-pyrazol-5-yl)-8-methyl-[1,2,4]triazolo[1,5-a]pyridine (206);
6-(4-isopropyl-3-(1-isopropylpiperidin-4-yl)-1H-pyrazol-5-yl)-8-methyl-[1,2,4]triazolo[1,5-a]pyridine (207);
3-(dimethylamino)-1-(4-(4-isopropyl-5-(8-methyl-[1,2,4]triazolo[1,5-a] pyridine-6-yl)-1H-pyrazol-3-yl)piperidin-1-yl)propan-1-one (208);
2-(dimethylamino)-1-(4-(4-isopropyl-5-(8-methyl-[1,2,4]triazolo[1,5-a]pyridin-6-yl)-1H-pyrazol-3-yl)piperidin-1-yl)ethan-1-one (209);
1-(4-(4-isopropyl-5-(8-methyl-[1,2,4]triazolo[1,5-a]pyridin-6-yl)-1H-pyrazol-3-yl)piperidin-1-yl)-3-(isopropylamino)propan-1-one (210);
2-(4-(4-isopropyl-5-(8-methyl-[1,2,4]triazolo[1,5-a]pyridin-6-yl)-1H-pyrazol-3-yl)piperidin-1-yl)-N-methylacetamide (211);
2-(4-(4-isopropyl-5-(8-methyl-[1,2,4]triazolo[1,5-a]pyridin-6-yl)-1H-pyrazol-3-yl)piperidin-1-yl)-N,N-dimethylacetamide (212);
6-(4-isopropyl-5-(8-methyl-[1,2,4]triazolo[1,5-a]pyridin-6-yl)-1H-pyrazol-3-yl)-1,2,3,4-tetrahydroisoquinoline, HCl (213);
6-(4-isopropyl-5-(8-methyl-[1,2,4]triazolo[1,5-a]pyridin-6-yl)-1H-pyrazol-3-yl)isoquinoline (214);
6-(4-isopropyl-5-(8-methyl-[1,2,4]triazolo[1,5-a]pyridin-6-yl)-1H-pyrazol-3-yl)-3,4-dihydroisoquinolin-1(2H)-one (215);
6-(3-(6,7-dihydro-5H-pyrrolo[3,4-d]pyrimidin-2-yl)-4-isopropyl-1H-pyrazol-5-yl)-8-methyl-[1,2,4]triazolo[1,5-a]pyridine (216);
6-(4-isopropyl-3-(4,5,6,7-tetrahydropyrazolo[1,5-a]pyrazin-2-yl)-1H-pyrazol-5-yl)-8-methyl-[1,2,4]triazolo[1,5-a]pyridine (217);
2-(4-isopropyl-5-(8-methyl-[1,2,4]triazolo[1,5-a]pyridin-6-yl)-1H-pyrazol-3-yl)-4,5,6,7-tetrahydrothiazolo[5,4-c]pyridine (218);
6-(4-isopropyl-3-(5,6,7,8-tetrahydroimidazo[1,2-a]pyrazin-2-yl)-1H-pyrazol-5-yl)-8-methyl-[1,2,4]triazolo[1,5-a]pyridine (219);
2-(4-isopropyl-5-(8-methoxy-[1,2,4]triazolo[1,5-a]pyridin-6-yl)-1H-pyrazol-3-yl)-4,5,6,7-tetrahydrothiazolo[5,4-c]pyridine (220);
2-(4-isopropyl-5-(8-methyl-[1,2,4]triazolo[1,5-a]pyridin-6-yl)-1H-pyrazol-3-yl)-4,5,6,7-tetrahydrothiazolo[4,5-c]pyridine (221);
2-(4-isopropyl-5-(8-methyl-[1,2,4]triazolo[1,5-a]pyridin-6-yl)-1H-pyrazol-3-yl)-5,6,7,8-tetrahydropyrido[4,3-d]pyrimidine (222);
6-(4-isopropyl-3-(pyridin-4-yl)-1H-pyrazol-5-yl)-8-methyl-[1,2,4]triazolo[1,5-a]pyridine (223);
6-(4-isopropyl-3-(pyridin-3-yl)-1H-pyrazol-5-yl)-8-methyl-[1,2,4]triazolo[1,5-a]pyridine (224);
5-(4-isopropyl-5-(8-methyl-[1,2,4]triazolo[1,5-a]pyridin-6-yl)-1H-pyrazol-3-yl)pyridin-3-amine (225);
5-(4-isopropyl-5-(8-methyl-[1,2,4]triazolo[1,5-a]pyridin-6-yl)-1H-pyrazol-3-yl)pyrazin-2-amine (226);
5-(4-isopropyl-5-(8-methyl-[1,2,4]triazolo[1,5-a]pyridin-6-yl)-1H-pyrazol-3-yl)pyridin-2-amine (227);
5-(4-isopropyl-5-(8-methyl-[1,2,4]triazolo[1,5-a]pyridin-6-yl)-1H-pyrazol-3-yl)pyrimidin-2-amine (228);
4-(4-isopropyl-5-(8-methyl-[1,2,4]triazolo[1,5-a]pyridin-6-yl)-1H-pyrazol-3-yl)benzamide (229);
3-(4-isopropyl-5-(8-methyl-[1,2,4]triazolo[1,5-a]pyridin-6-yl)-1H-pyrazol-3-yl)-5,6,7,8-tetrahydro-1,6-naphthyridine (230);
7-(4-isopropyl-5-(8-methyl-[1,2,4]triazolo[1,5-a]pyridin-6-yl)-1H-pyrazol-3-yl)-1,2,3,4-tetrahydroisoquinoline (231);
3-(4-isopropyl-5-(8-methyl-[1,2,4]triazolo[1,5-a]pyridin-6-yl)-1H-pyrazol-3-yl)aniline (232);
4-(4-isopropyl-5-(8-methyl-[1,2,4]triazolo[1,5-a]pyridin-6-yl)-1H-pyrazol-3-yl)aniline (233);
6-(4-isopropyl-5-(8-methoxy-[1,2,4]triazolo[1,5-a]pyridin-6-yl)-1H-pyrazol-3-yl)-1,2,3,4-tetrahydroisoquinoline (234);
6-(4-isopropyl-5-(8-methyl-[1,2,4]triazolo[1,5-a]pyridin-6-yl)-1H-pyrazol-3-yl)-2-methyl-1,2,3,4-tetrahydroisoquinoline (235);
5-(4-isopropyl-5-(8-methyl-[1,2,4]triazolo[1,5-a]pyridin-6-yl)-1H-pyrazol-3-yl)-N,N-dimethylpyrazin-2-amine (236);
5-(4-isopropyl-5-(8-methyl-[1,2,4]triazolo[1,5-a]pyridin-6-yl)-1H-pyrazol-3-yl)-N,N-dimethylpyridin-2-amine (237);
5-(4-isopropyl-5-(8-methyl-[1,2,4]triazolo[1,5-a]pyridin-6-yl)-1H-pyrazol-3-yl)-N,N-dimethylpyridin-3-amine (238);
N-isopropyl-5-(4-isopropyl-5-(8-methyl-[1,2,4]triazolo[1,5-a]pyridin-6-yl)-1H-pyrazol-3-yl)pyridin-3-amine (239);
N-isopropyl-5-(4-isopropyl-5-(8-methyl-[1,2,4]triazolo[1,5-a]pyridin-6-yl)-1H-pyrazol-3-yl)pyridin-2-amine (240);
N-isopropyl-5-(4-isopropyl-5-(8-methyl-[1,2,4]triazolo[1,5-a]pyridin-6-yl)-1H-pyrazol-3-yl)pyrazin-2-amine (241);
N-isopropyl-3-(4-isopropyl-5-(8-methyl-[1,2,4]triazolo[1,5-a]pyridin-6-yl)-1H-pyrazol-3-yl)aniline (242);
4-(4-isopropyl-5-(8-methyl-[1,2,4]triazolo[1,5-a]pyridin-6-yl)-1H-pyrazol-3-yl)-N,N-dimethylaniline (243);
N-isopropyl-4-(4-isopropyl-5-(8-methyl-[1,2,4]triazolo[1,5-a]pyridin-6-yl)-1H-pyrazol-3-yl)aniline (244);
3-(4-isopropyl-5-(8-methyl-[1,2,4]triazolo[1,5-a]pyridin-6-yl)-1H-pyrazol-3-yl)-N,N-dimethylaniline (245);
2-isopropyl-6-(4-isopropyl-5-(8-methyl-[1,2,4]triazolo[1,5-a]pyridin-6-yl)-1H-pyrazol-3-yl)-1,2,3,4-tetrahydroisoquinoline (246);
6-(4-isopropyl-5-(8-methyl-[1,2,4]triazolo[1,5-a]pyridin-6-yl)-1H-pyrazol-3-yl)-2-(oxetan-3-yl)-1,2,3,4-tetrahydroisoquinoline (247);
2-(4-isopropyl-5-(8-methyl-[1,2,4]triazolo[1,5-a]pyridin-6-yl)-1H-pyrazol-3-yl)-6-methyl-5,6,7,8-tetrahydropyrido[4,3-d]pyrimidine (248);
6-ethyl-2-(4-isopropyl-5-(8-methyl-[1,2,4]triazolo[1,5-a]pyridin-6-yl)-1H-pyrazol-3-yl)-5,6,7,8-tetrahydropyrido[4,3-d]pyrimidine (249);
6-isopropyl-2-(4-isopropyl-5-(8-methyl-[1,2,4]triazolo[1,5-a]pyridin-6-yl)-1H-pyrazol-3-yl)-5,6,7,8-tetrahydropyrido[4,3-d]pyrimidine (250);
2-(4-isopropyl-5-(8-methyl-[1,2,4]triazolo[1,5-a]pyridin-6-yl)-1H-pyrazol-3-yl)-6-(oxetan-3-yl)-5,6,7,8-tetrahydropyrido[4,3-d]pyrimidine (251);
5-isopropyl-2-(4-isopropyl-5-(8-methyl-[1,2,4]triazolo[1,5-a]pyridin-6-yl)-1H-pyrazol-3-yl)-4,5,6,7-tetrahydrothiazolo[5,4-c]pyridine (252);
2-(4-isopropyl-5-(8-methyl-[1,2,4]triazolo[1,5-a]pyridin-6-yl)-1H-pyrazol-3-yl)-5-(oxetan-3-yl)-4,5,6,7-tetrahydrothiazolo[5,4-c]pyridine (253);
2-(4-isopropyl-5-(8-methyl-[1,2,4]triazolo[1,5-a]pyridin-6-yl)-1H-pyrazol-3-yl)-5-(1-isopropylpiperidin-4-yl)-4,5,6,7-tetrahydrothiazolo[5,4-c]pyridine (254);
5-isobutyl-2-(4-isopropyl-5-(8-methyl-[1,2,4]triazolo[1,5-a]pyridin-6-yl)-1H-pyrazol-3-yl)-4,5,6,7-tetrahydrothiazolo[5,4-c]pyridine (255);
2-(4-isopropyl-5-(8-methyl-[1,2,4]triazolo[1,5-a]pyridin-6-yl)-1H-pyrazol-3-yl)-5-methyl-4,5,6,7-tetrahydrothiazolo[5,4-c]pyridine (256);
N-ethyl-2-(4-isopropyl-5-(8-methyl-[1,2,4]triazolo[1,5-a]pyridin-6-yl)-1H-pyrazol-3-yl)benzo[d]thiazol-6-amine (257);
N-isopropyl-2-(4-isopropyl-5-(8-methyl-[1,2,4]triazolo[1,5-a]pyridin-6-yl)-1H-pyrazol-3-yl)benzo[d]thiazol-6-amine (258);
5-isopropyl-2-(4-isopropyl-5-(8-methoxy-[1,2,4]triazolo[1,5-a]pyridin-6-yl)-1H-pyrazol-3-yl)-4,5,6,7-tetrahydrothiazolo[5,4-c]pyridine (259);
2-(4-isopropyl-5-(8-methoxy-[1,2,4]triazolo[1,5-a]pyridin-6-yl)-1H-pyrazol-3-yl)-5-propyl-4,5,6,7-tetrahydrothiazolo[5,4-c]pyridine (260);
5-ethyl-2-(4-isopropyl-5-(8-methoxy-[1,2,4]triazolo[1,5-a]pyridin-6-yl)-1H-pyrazol-3-yl)-4,5,6,7-tetrahydrothiazolo[5,4-c]pyridine (261);
2-(4-isopropyl-5-(8-methoxy-[1,2,4]triazolo[1,5-a]pyridin-6-yl)-1H-pyrazol-3-yl)-5-methyl-4,5,6,7-tetrahydrothiazolo[5,4-c]pyridine (262);
6-(4-isopropyl-5-(8-methyl-[1,2,4]triazolo[1,5-a]pyridin-6-yl)-1H-pyrazol-3-yl)-2-(2-methoxyethyl)-1,2,3,4-tetrahydroisoquinoline (263);
1-(6-(4-isopropyl-5-(8-methyl-[1,2,4]triazolo[1,5-a]pyridin-6-yl)-1H-pyrazol-3-yl)-3,4-dihydroisoquinolin-2(1H)-yl)-2-methylpropan-2-ol (264);
1-(2-(4-isopropyl-5-(8-methyl-[1,2,4]triazolo[1,5-a]pyridin-6-yl)-1H-pyrazol-3-yl)-6,7-dihydrothiazolo[5,4-c]pyridin-5(4H)-yl)-2-methylpropan-2-ol (265);
2-(4-isopropyl-5-(8-methyl-[1,2,4]triazolo[1,5-a]pyridin-6-yl)-1H-pyrazol-3-yl)-5-(2-methoxyethyl)-4,5,6,7-tetrahydrothiazolo[5,4-c]pyridine (266);
2-(4-isopropyl-5-(8-methyl-[1,2,4]triazolo[1,5-a]pyridin-6-yl)-1H-pyrazol-3-yl)-N-(2-methoxyethyl)benzo[d]thiazol-6-amine (267);
2-(dimethylamino)-1-(6-(4-isopropyl-5-(8-methyl-[1,2,4]triazolo[1,5-a]pyridin-6-yl)-1H-pyrazol-3-yl)-3,4-dihydroisoquinolin-2(1H)-yl)ethan-1-one (268);
(S)-3-hydroxy-1-(2-(4-isopropyl-5-(8-methyl-[1,2,4]triazolo[1,5-a]pyridin-6-yl)-1H-pyrazol-3-yl)-6,7-dihydrothiazolo[5,4-c]pyridin-5(4H)-yl)butan-1-one (269);
2-(dimethylamino)-1-(2-(4-isopropyl-5-(8-methyl-[1,2,4]triazolo[1,5-a]pyridin-6-yl)-1H-pyrazol-3-yl)-6,7-dihydrothiazolo[5,4-c]pyridin-5(4H)-yl)ethan-1-one (270);
2-(6-(4-isopropyl-5-(8-methyl-[1,2,4]triazolo[1,5-a]pyridin-6-yl)-1H-pyrazol-3-yl)-3,4-dihydroisoquinolin-2(1H)-yl)-N,N-dimethylacetamide (271);
2-(6-(4-isopropyl-5-(8-methyl-[1,2,4]triazolo[1,5-a]pyridin-6-yl)-1H-pyrazol-3-yl)-3,4-dihydroisoquinolin-2(1H)-yl)acetamide (272);
2-(6-(4-isopropyl-5-(8-methyl-[1,2,4]triazolo[1,5-a]pyridin-6-yl)-1H-pyrazol-3-yl)-3,4-dihydroisoquinolin-2(1H)-yl)-N-methylacetamide (273);
2-(2-(4-isopropyl-5-(8-methyl-[1,2,4]triazolo[1,5-a]pyridin-6-yl)-1H-pyrazol-3-yl)-6,7-dihydrothiazolo[5,4-c]pyridin-5(4H)-yl)-N-methylacetamide (274);
2-(2-(4-isopropyl-5-(8-methyl-[1,2,4]triazolo[1,5-a]pyridin-6-yl)-1H-pyrazol-3-yl)-6,7-dihydrothiazolo[5,4-c]pyridin-5(4H)-yl)-N,N-dimethylacetamide (275);
1-(2-(4-isopropyl-5-(8-methyl-[1,2,4]triazolo[1,5-a]pyridin-6-yl)-1H-pyrazol-3-yl)-6,7-dihydrothiazolo[5,4-c]pyridin-5(4H)-yl)ethan-1-one (276);
6-(4-isopropyl-5-(8-methyl-[1,2,4]triazolo[1,5-a]pyridin-6-yl)-1H-pyrazol-3-yl)pyridin-2-ol (277);
6-(5-(2,6-dimethylpyridin-4-yl)-4-isopropyl-1H-pyrazol-3-yl)-1,2,3,4-tetrahydro isoquinoline (278);
N-(2,2-difluoroethyl)-4-(4-isopropyl-5-(8-methyl-[1,2,4]triazolo[1,5-a]pyridin-6-yl)-1H-pyrazol-3-yl)cyclohexan-1-amine (279);
4-(4-isopropyl-5-(8-methyl-[1,2,4]triazolo[1,5-a]pyridin-6-yl)-1H-pyrazol-3-yl)-N-((3-methyloxetan-3-yl)methyl)cyclohexan-1-amine (280-281);
N-ethyl-4-(4-isopropyl-5-(8-methyl-[1,2,4]triazolo[1,5-a]pyridin-6-yl)-1H-pyrazol-3-yl)-N-methylcyclohexan-1-amine (282-283);
N1-(4-(4-isopropyl-5-(8-methyl-[1,2,4]triazolo[1,5-a]pyridin-6-yl)-1H-pyrazol-3-yl)cyclohexyl)-N2,N2-dimethylethane-1,2-diamine (284-285);
6-(4-isopropyl-3-(4-(4-methylpiperazin-1-yl)cyclohexyl)-1H-pyrazol-5-yl)-8-methyl-[1,2,4]triazolo[1,5-a]pyridine (286-287);
1-isopropyl-N-(4-(4-isopropyl-5-(8-methyl-[1,2,4]triazolo[1,5-a]pyridin-6-yl)-1H-pyrazol-3-yl)cyclohexyl)piperidin-4-amine (288-289);
4-(4-(4-isopropyl-5-(8-methyl-[1,2,4]triazolo[1,5-a]pyridin-6-yl)-1H-pyrazol-3-yl)cyclohexyl)morpholine (290-291);
1-(4-(4-isopropyl-5-(8-methyl-[1,2,4]triazolo[1,5-a]pyridin-6-yl)-1H-pyrazol-3-yl)cyclohexyl)-N,N-dimethylpiperidin-4-amine (292-293);
4-(4-isopropyl-5-(8-methyl-[1,2,4]triazolo[1,5-a]pyridin-6-yl)-1H-pyrazol-3-yl)-N-methylcyclohexan-1-amine (294-295);
2-(dimethylamino)-N-(4-(4-isopropyl-5-(8-methyl-[1,2,4]triazolo[1,5-a] pyridin-6-yl)-1H-pyrazol-3-yl)cyclohexyl)-N-methylacetamide (296-297);
2-((4-(4-isopropyl-5-(8-methyl-[1,2,4]triazolo[1,5-a]pyridin-6-yl)-1H-pyrazol-3-yl)cyclohexyl)(methyl)amino)-N,N-dimethylacetamide (298-299);
N-(4-(4-isopropyl-5-(8-methyl-[1,2,4]triazolo[1,5-a]pyridin-6-yl)-1H-pyrazol-3-yl) cyclohexyl)-N,1-dimethylazetidine-3-carboxamide (300 and 302);
N-(4-(4-isopropyl-5-(8-methyl-[1,2,4]triazolo[1,5-a]pyridin-6-yl)-1H-pyrazol-3-yl)cyclohexyl)-N-methylazetidine-3-carboxamide (300B and 301);
1-isopropyl-N-(4-(4-isopropyl-5-(8-methyl-[1,2,4]triazolo[1,5-a]pyridin-6-yl)-1H-pyrazol-3-yl)cyclohexyl)-N-methylazetidine-3-carboxamide (303-304);
1-ethyl-N-(4-(4-isopropyl-5-(8-methyl-[1,2,4]triazolo[1,5-a]pyridin-6-yl)-1H-pyrazol-3-yl)cyclohexyl)-N-methylazetidine-3-carboxamide (305-306);
6-(4-isopropyl-3-(4-(1-isopropylpiperidin-4-yl)phenyl)-1H-pyrazol-5-yl)-8-methyl-[1,2,4]triazolo[1,5-a]pyridine (307);
6-(4-isopropyl-3-(4-(piperidin-4-yl)phenyl)-1H-pyrazol-5-yl)-8-methoxy-[1,2,4]triazolo[1,5-a]pyridine (308);
6-(4-isopropyl-3-(4-(1-methylpiperidin-4-yl)phenyl)-1H-pyrazol-5-yl)-8-methyl-[1,2,4]triazolo[1,5-a]pyridine (309);
6-(3-(4-(1-ethylpiperidin-4-yl)phenyl)-4-isopropyl-1H-pyrazol-5-yl)-8-methyl-[1,2,4]triazolo[1,5-a]pyridine (310);
6-(4-isopropyl-3-(4-(1-(oxetan-3-yl)piperidin-4-yl)phenyl)-1H-pyrazol-5-yl)-8-methyl-[1,2,4]triazolo[1,5-a]pyridine (311);
6-(4-isopropyl-3-(4-(1-((3-methyloxetan-3-yl)methyl)piperidin-4-yl)phenyl)-1H-pyrazol-5-yl)-8-methoxy-[1,2,4]triazolo[1,5-a]pyridine (312);
6-(4-isopropyl-3-(4-(1-(tetrahydro-2H-pyran-4-yl)piperidin-4-yl)phenyl)-1H-pyrazol-5-yl)-8-methoxy-[1,2,4]triazolo[1,5-a]pyridine (313);
6-(4-isopropyl-3-(4-(1-methylpiperidin-4-yl)phenyl)-1H-pyrazol-5-yl)-8-methoxy-[1,2,4]triazolo[1,5-a]pyridine (314);
6-(4-isopropyl-3-(4-(1-methylpiperidin-4-yl)phenyl)-1H-pyrazol-5-yl)-8-methoxy-[1,2,4]triazolo[1,5-a]pyridine (315);
6-(4-isopropyl-3-(4-(1-isopropylpiperidin-4-yl)phenyl)-1H-pyrazol-5-yl)-8-methoxy-[1,2,4]triazolo[1,5-a]pyridine (316);
6-(4-isopropyl-3-(4-(1-neopentylpiperidin-4-yl)phenyl)-1H-pyrazol-5-yl)-8-methyl-[1,2,4]triazolo[1,5-a]pyridine (317);
6-(4-isopropyl-3-(4-(1-(2-methoxyethyl)piperidin-4-yl)phenyl)-1H-pyrazol-5-yl)-8-methoxy-[1,2,4]triazolo[1,5-a]pyridine (318);
1-(4-(4-(4-isopropyl-5-(8-methoxy-[1,2,4]triazolo[1,5-a]pyridin-6-yl)-1H-pyrazol-3-yl)phenyl)piperidin-1-yl)-2-methylpropan-2-ol (319);
6-(4-isopropyl-3-(4-(1-(2,2,2-trifluoroethyl)piperidin-4-yl)phenyl)-1H-pyrazol-5-yl)-8-methoxy-[1,2,4]triazolo[1,5-a]pyridine (320);
2-(4-(4-(4-isopropyl-5-(8-methyl-[1,2,4]triazolo[1,5-a]pyridin-6-yl)-1H-pyrazol-3-yl) phenyl)piperidin-1-yl)-N,N-dimethylacetamide (321);
2-(4-(4-(4-isopropyl-5-(8-methoxy-[1,2,4]triazolo[1,5-a]pyridin-6-yl)-1H-pyrazol-3-yl)phenyl)piperidin-1-yl)acetamide (322);
2-(dimethylamino)-1-(4-(4-(4-isopropyl-5-(8-methyl-[1,2,4]triazolo[1,5-a]pyridin-6-yl)-1H-pyrazol-3-yl)phenyl)piperidin-1-yl)ethan-1-one (323);
6-(4-isopropyl-3-(4-(1-methylazetidin-3-yl)phenyl)-1H-pyrazol-5-yl)-8-methyl-[1,2,4] triazolo[1,5-a]pyridine (324);
6-(3-(4-(azetidin-3-yl)phenyl)-4-isopropyl-1H-pyrazol-5-yl)-8-methoxy-[1,2,4]triazolo[1,5-a]pyridine (324E);
6-(3-(4-(1-ethylazetidin-3-yl)phenyl)-4-isopropyl-1H-pyrazol-5-yl)-8-methyl-[1,2,4]triazolo[1,5-a]pyridine (325);
6-(4-isopropyl-3-(4-(1-propylazetidin-3-yl)phenyl)-1H-pyrazol-5-yl)-8-methyl-[1,2,4]triazolo[1,5-a]pyridine (326);
6-(4-isopropyl-3-(4-(1-isopropylazetidin-3-yl)phenyl)-1H-pyrazol-5-yl)-8-methyl-[1,2,4]triazolo[1,5-a]pyridine (327);
6-(4-isopropyl-3-(4-(1-methylazetidin-3-yl)phenyl)-1H-pyrazol-5-yl)-8-methoxy-[1,2,4]triazolo[1,5-a]pyridine (328);
6-(3-(4-(1-ethylazetidin-3-yl)phenyl)-4-isopropyl-1H-pyrazol-5-yl)-8-methoxy-[1,2,4]triazolo[1,5-a]pyridine (329);
6-(4-isopropyl-3-(4-(1-propylazetidin-3-yl)phenyl)-1H-pyrazol-5-yl)-8-methoxy-[1,2,4]triazolo[1,5-a]pyridine (330);
6-(4-isopropyl-3-(4-(1-isopropylazetidin-3-yl)phenyl)-1H-pyrazol-5-yl)-8-methoxy-[1,2,4]triazolo[1,5-a]pyridine (331);
6-(4-isopropyl-3-(4-(1-(oxetan-3-yl)azetidin-3-yl)phenyl)-1H-pyrazol-5-yl)-8-methoxy-[1,2,4]triazolo[1,5-a]pyridine (332);
6-(4-isopropyl-3-(4-(1-(tetrahydro-2H-pyran-4-yl)azetidin-3-yl)phenyl)-1H-pyrazol-5-yl)-8-methoxy-[1,2,4]triazolo[1,5-a]pyridine (333);
2-(dimethylamino)-1-(3-(4-(4-isopropyl-5-(8-methyl-[1,2,4]triazolo[1,5-a]pyridin-6-yl)-1H-pyrazol-3-yl)phenyl)azetidin-1-yl)ethan-1-one (334);
2-(dimethylamino)-1-(3-(4-(4-isopropyl-5-(8-methoxy-[1,2,4]triazolo[1,5-a]pyridin-6-yl)-1H-pyrazol-3-yl)phenyl)azetidin-1-yl)ethan-1-one (335);
1-(3-(4-(4-isopropyl-5-(8-methyl-[1,2,4]triazolo[1,5-a]pyridin-6-yl)-1H-pyrazol-3-yl)phenyl)azetidin-1-yl)-2-methylpropan-2-ol (336);
1-(3-(4-(4-isopropyl-5-(8-methoxy-[1,2,4]triazolo[1,5-a]pyridin-6-yl)-1H-pyrazol-3-yl)phenyl)azetidin-1-yl)-2-methylpropan-2-ol (337);
6-(4-isopropyl-3-(4-(1-(2-(methylsulfonyl)ethyl)azetidin-3-yl)phenyl)-1H-pyrazol-5-yl)-8-methoxy-[1,2,4]triazolo[1,5-a]pyridine (338);
6-(4-isopropyl-3-(5-(piperidin-4-yl)pyridin-2-yl)-1H-pyrazol-5-yl)-8-methoxy-[1,2,4] triazolo[1,5-a]pyridine (339);
6-(4-isopropyl-3-(6-(piperidin-4-yl)pyridin-3-yl)-1H-pyrazol-5-yl)-8-methyl-[1,2,4]triazolo[1,5-a]pyridine (340);
6-(4-isopropyl-3-(5-(piperidin-4-yl)pyrimidin-2-yl)-1H-pyrazol-5-yl)-8-methyl-[1,2,4]triazolo[1,5-a]pyridine (341);
6-(4-isopropyl-3-(5-(piperidin-4-yl)pyridin-2-yl)-1H-pyrazol-5-yl)-8-methyl-[1,2,4]triazolo[1,5-a]pyridine (342);
6-(4-isopropyl-3-(2-(piperidin-4-yl)pyrimidin-5-yl)-1H-pyrazol-5-yl)-8-methyl-[1,2,4]triazolo[1,5-a]pyridine (343);
6-(4-isopropyl-3-(6-(piperidin-4-yl)pyridazin-3-yl)-1H-pyrazol-5-yl)-8-methyl-[1,2,4]triazolo[1,5-a]pyridine (344);
6-(4-isopropyl-3-(5-(piperidin-4-yl)pyrazin-2-yl)-1H-pyrazol-5-yl)-8-methoxy-[1,2,4]triazolo[1,5-a]pyridine (345);
6-(4-isopropyl-3-(5-(piperidin-4-yl)pyridin-2-yl)-1H-pyrazol-5-yl)-8-methoxy-7-methyl-[1,2,4]triazolo[1,5-a]pyridine (346);
6-(3-(5-(1-ethylpiperidin-4-yl)pyridin-2-yl)-4-isopropyl-1H-pyrazol-5-yl)-8-methoxy-[1,2,4]triazolo[1,5-a]pyridine (347);
6-(4-isopropyl-3-(5-(1-methylpiperidin-4-yl)pyridin-2-yl)-1H-pyrazol-5-yl)-8-methoxy-[1,2,4]triazolo[1,5-a]pyridine (348);
6-(4-isopropyl-3-(5-(1-propylpiperidin-4-yl)pyridin-2-yl)-1H-pyrazol-5-yl)-8-methoxy-[1,2,4]triazolo[1,5-a]pyridine (349);
6-(4-isopropyl-3-(5-(1-isopropylpiperidin-4-yl)pyridin-2-yl)-1H-pyrazol-5-yl)-8-methoxy-[1,2,4]triazolo[1,5-a]pyridine (350);
6-(4-isopropyl-3-(5-(1-neopentylpiperidin-4-yl)pyridin-2-yl)-1H-pyrazol-5-yl)-8-methoxy-[1,2,4]triazolo[1,5-a]pyridine (351);
6-(4-isopropyl-3-(5-(1-(3,3,3-trifluoropropyl)piperidin-4-yl)pyridin-2-yl)-1H-pyrazol-5-yl)-8-methoxy-[1,2,4]triazolo[1,5-a]pyridine (352);
6-(4-isopropyl-3-(5-(1-((1-methyl-1H-1,2,4-triazol-3-yl)methyl)piperidin-4-yl)pyridin-2-yl)-1H-pyrazol-5-yl)-8-methoxy-[1,2,4]triazolo[1,5-a]pyridine (353);
6-(3-(5-(1-(3-ethoxycyclobutyl)piperidin-4-yl)pyridin-2-yl)-4-isopropyl-1H-pyrazol-5-yl)-8-methoxy-[1,2,4]triazolo[1,5-a]pyridine (354);
6-(3-(5-(1-(3-ethoxycyclobutyl)piperidin-4-yl)pyridin-2-yl)-4-isopropyl-1H-pyrazol-5-yl)-8-methoxy-[1,2,4]triazolo[1,5-a]pyridine (355);
6-(4-isopropyl-3-(5-(1-(oxetan-3-yl)piperidin-4-yl)pyridin-2-yl)-1H-pyrazol-5-yl)-8-methoxy-[1,2,4]triazolo[1,5-a]pyridine (356);
6-(4-isopropyl-3-(6-(1-methylpiperidin-4-yl)pyridazin-3-yl)-1H-pyrazol-5-yl)-8-methyl-[1,2,4]triazolo[1,5-a]pyridine (357);
6-(4-isopropyl-3-(6-(1-isopropylpiperidin-4-yl)pyridazin-3-yl)-1H-pyrazol-5-yl)-8-methyl-[1,2,4]triazolo[1,5-a]pyridine (358);
6-(4-isopropyl-3-(6-(1-methylpiperidin-4-yl)pyridin-3-yl)-1H-pyrazol-5-yl)-8-methyl-[1,2,4]triazolo[1,5-a]pyridine (359);
6-(3-(6-(1-ethylpiperidin-4-yl)pyridin-3-yl)-4-isopropyl-1H-pyrazol-5-yl)-8-methyl-[1,2,4]triazolo[1,5-a]pyridine (360);
6-(4-isopropyl-3-(6-(1-isopropylpiperidin-4-yl)pyridin-3-yl)-1H-pyrazol-5-yl)-8-methyl-[1,2,4]triazolo[1,5-a]pyridine (361);
6-(4-isopropyl-3-(6-(1-(oxetan-3-yl)piperidin-4-yl)pyridin-3-yl)-1H-pyrazol-5-yl)-8-methyl-[1,2,4]triazolo[1,5-a]pyridine (362);
6-(4-isopropyl-3-(2-(1-isopropylpiperidin-4-yl)pyrimidin-5-yl)-1H-pyrazol-5-yl)-8-methyl-[1,2,4]triazolo[1,5-a]pyridine (363);
6-(4-isopropyl-3-(2-(1-neopentylpiperidin-4-yl)pyrimidin-5-yl)-1H-pyrazol-5-yl)-8-methyl-[1,2,4]triazolo[1,5-a]pyridine (364);
6-(4-isopropyl-3-(5-(1-methylpiperidin-4-yl)pyridin-2-yl)-1H-pyrazol-5-yl)-8-methyl-[1,2,4]triazolo[1,5-a]pyridine (365);
6-(3-(5-(1-ethylpiperidin-4-yl)pyridin-2-yl)-4-isopropyl-1H-pyrazol-5-yl)-8-methyl-[1,2,4]triazolo[1,5-a]pyridine (366);
6-(4-isopropyl-3-(5-(1-isopropylpiperidin-4-yl)pyridin-2-yl)-1H-pyrazol-5-yl)-8-methyl-[1,2,4]triazolo[1,5-a]pyridine (367);
6-(4-isopropyl-3-(5-(1-methylpiperidin-4-yl)pyrimidin-2-yl)-1H-pyrazol-5-yl)-8-methyl-[1,2,4]triazolo[1,5-a]pyridine (368);
6-(3-(5-(1-ethylpiperidin-4-yl)pyrimidin-2-yl)-4-isopropyl-1H-pyrazol-5-yl)-8-methyl-[1,2,4]triazolo[1,5-a]pyridine (369);
6-(4-isopropyl-3-(5-(1-isopropylpiperidin-4-yl)pyrimidin-2-yl)-1H-pyrazol-5-yl)-8-methyl-[1,2,4]triazolo[1,5-a]pyridine (370);
6-(4-isopropyl-3-(5-(1-neopentylpiperidin-4-yl)pyrimidin-2-yl)-1H-pyrazol-5-yl)-8-methyl-[1,2,4]triazolo[1,5-a]pyridine (371);
6-(4-isopropyl-3-(5-(1-(3-methylbutan-2-yl)piperidin-4-yl)pyrimidin-2-yl)-1H-pyrazol-5-yl)-8-methyl-[1,2,4]triazolo[1,5-a]pyridine (372);
6-(4-isopropyl-3-(5-(1-neopentylpiperidin-4-yl)pyridin-2-yl)-1H-pyrazol-5-yl)-8-methyl-[1,2,4]triazolo[1,5-a]pyridine (373);
6-(3-(5-(1-(cyclopropylmethyl)piperidin-4-yl)pyridin-2-yl)-4-isopropyl-1H-pyrazol-5-yl)-8-methoxy-[1,2,4]triazolo[1,5-a]pyridine (374);
6-(4-isopropyl-3-(5-(1-((tetrahydro-2H-pyran-4-yl)methyl)piperidin-4-yl)pyridin-2-yD)-1H-pyrazol-5-yl)-8-methoxy-[1,2,4]triazolo[1,5-a]pyridine (375);
6-(4-isopropyl-3-(5-(1-((tetrahydro-2H-pyran-4-yl)methyl)piperidin-4-yl)pyrazin-2-yl)-1H-pyrazol-5-yl)-8-methoxy-[1,2,4]triazolo[1,5-a]pyridine (376);
6-(3-(5-(1-(cyclobutylmethyl)piperidin-4-yl)pyrazin-2-yl)-4-isopropyl-1H-pyrazol-5-yl)-8-methoxy-[1,2,4]triazolo[1,5-a]pyridine (377);
6-(3-(5-(1-(cyclopropylmethyl)piperidin-4-yl)pyrazin-2-yl)-4-isopropyl-1H-pyrazol-5-yl)-8-methoxy-[1,2,4]triazolo[1,5-a]pyridine (378);
6-(3-(5-(1-isobutylpiperidin-4-yl)pyrazin-2-yl)-4-isopropyl-1H-pyrazol-5-yl)-8-methoxy-[1,2,4]triazolo[1,5-a]pyridine (379);
6-(4-isopropyl-3-(5-(1-(tetrahydro-2H-pyran-4-yl)piperidin-4-yl)pyrazin-2-yl)-1H-pyrazol-5-yl)-8-methoxy-[1,2,4]triazolo[1,5-a]pyridine (380);
6-(4-isopropyl-3-(5-(1-methylpiperidin-4-yl)pyrazin-2-yl)-1H-pyrazol-5-yl)-8-methoxy-[1,2,4]triazolo[1,5-a]pyridine (381);
6-(3-(5-(1-ethylpiperidin-4-yl)pyrazin-2-yl)-4-isopropyl-1H-pyrazol-5-yl)-8-methoxy-[1,2,4]triazolo[1,5-a]pyridine (382);
6-(4-isopropyl-3-(5-(1-propylpiperidin-4-yl)pyrazin-2-yl)-1H-pyrazol-5-yl)-8-methoxy-[1,2,4]triazolo[1,5-a]pyridine (383);
6-(4-isopropyl-3-(5-(1-isopropylpiperidin-4-yl)pyrazin-2-yl)-1H-pyrazol-5-yl)-8-methoxy-[1,2,4]triazolo[1,5-a]pyridine (384);
6-(4-isopropyl-3-(5-(1-(oxetan-3-yl)piperidin-4-yl)pyrazin-2-yl)-1H-pyrazol-5-yl)-8-methoxy-[1,2,4]triazolo[1,5-a]pyridine (385);
6-(4-isopropyl-3-(5-(1-(pentan-3-yl)piperidin-4-yl)pyrazin-2-yl)-1H-pyrazol-5-yl)-8-methoxy-[1,2,4]triazolo[1,5-a]pyridine (386);
6-(3-(5-(1-cyclobutylpiperidin-4-yl)pyrazin-2-yl)-4-isopropyl-1H-pyrazol-5-yl)-8-methoxy-[1,2,4]triazolo[1,5-a]pyridine (387);
6-(4-isopropyl-3-(5-(1-((tetrahydro-2H-pyran-4-yl)methyl)piperidin-4-yl)pyridin-2-yl)-1H-pyrazol-5-yl)-8-methoxy-7-methyl-[1,2,4]triazolo[1,5-a]pyridine (388);
2-(4-(6-(4-isopropyl-5-(8-methoxy-[1,2,4]triazolo[1,5-a]pyridin-6-yl)-1H-pyrazol-3-yl)pyridin-3-yl)piperidin-1-yl)-N,N-dimethylacetamide (389);
1-(4-(2-(4-isopropyl-5-(8-methyl-[1,2,4]triazolo[1,5-a]pyridin-6-yl)-1H-pyrazol-3-yl)pyrimidin-5-yl)piperidin-1-yl)-2-methylpropan-2-ol (390);
1-(4-(6-(4-isopropyl-5-(8-methoxy-[1,2,4]triazolo[1,5-a]pyridin-6-yl)-1H-pyrazol-3-yl)pyridin-3 -yl)piperidin-1-yl)-2-methylpropan-2-ol (391);
6-(4-isopropyl-3-(5-(1-(2-(methylsulfonyl)ethyl)piperidin-4-yl)pyridin-2-yl)-1H-pyrazol-5-yl)-8-methoxy-[1,2,4]triazolo[1,5-a]pyridine (392);
2-(4-(6-(4-isopropyl-5-(8-methoxy-[1,2,4]triazolo[1,5-a]pyridin-6-yl)-1H-pyrazol-3-yl)pyridin-3-yl)piperidin-1-yl)acetamide (393);
2-(4-(6-(4-isopropyl-5-(8-methoxy-[1,2,4]triazolo[1,5-a]pyridin-6-yl)-1H-pyrazol-3-yl)pyridin-3-yl)piperidin-1-yl)acetonitrile (394);
6-(4-isopropyl-3-(5-(1-(2-methoxyethyl)piperidin-4-yl)pyridin-2-yl)-1H-pyrazol-5-yl)-8-methoxy-[1,2,4]triazolo[1,5-a]pyridine (395);
6-(4-isopropyl-3-(5-(1-(2-methoxyethyl)piperidin-4-yl)pyrazin-2-yl)-1H-pyrazol-5-yl)-8-methoxy-[1,2,4]triazolo[1,5-a]pyridine (396);
6-(4-isopropyl-3-(5-(1-(2-(methylsulfonyl)ethyl)piperidin-4-yl)pyrazin-2-yl)-1H-pyrazol-5-yl)-8-methoxy-[1,2,4]triazolo[1,5-a]pyridine (397);
6-(3-(5-(1-(2-fluoroethyl)piperidin-4-yl)pyrazin-2-yl)-4-isopropyl-1H-pyrazol-5-yl)-8-methoxy-[1,2,4]triazolo[1,5-a]pyridine (398);
6-(3-(5-(1-(3-fluoropropyl)piperidin-4-yl)pyrazin-2-yl)-4-isopropyl-1H-pyrazol-5-yl)-8-methoxy-[1,2,4]triazolo[1,5-a]pyridine (399);
2-(4-(5-(4-isopropyl-5-(8-methoxy-[1,2,4]triazolo[1,5-a]pyridin-6-yl)-1H-pyrazol-3-yl)pyrazin-2-yl)piperidin-1-yl)-N-methylacetamide (400);
2-(4-(5-(4-isopropyl-5-(8-methoxy-[1,2,4]triazolo[1,5-a]pyridin-6-yl)-1H-pyrazol-3-yl)pyrazin-2-yl)piperidin-1-yl)-N,N-dimethylacetamide (401);
2-(dimethylamino)-1-(4-(6-(4-isopropyl-5-(8-methoxy-[1,2,4]triazolo[1,5-a]pyridin-6-yl)-1H-pyrazol-3-yl)pyridin-3-yl)piperidin-1-yl)ethan-1-one (402);
2-(dimethylamino)-1-(4-(5-(4-isopropyl-5-(8-methoxy-[1,2,4]triazolo[1,5-a]pyridin-6-yl)-1H-pyrazol-3-yl)pyrazin-2-yl)piperidin-1-yl)ethan-1-one (403);
2-(diethylamino)-1-(4-(5-(4-isopropyl-5-(8-methoxy-[1,2,4]triazolo[1,5-a]pyridin-6-yl)-1H-pyrazol-3-yl)pyrazin-2-yl)piperidin-1-yl)ethan-1-one (404);
1-(4-(4-isopropyl-5-(8-methyl-[1,2,4]triazolo[1,5-a]pyridin-6-yl)-1H-pyrazol-3-yl)phenyl)-N,N-dimethylazetidin-3-amine (405);
6-(4-isopropyl-3-(4-(piperazin-1-yl)phenyl)-1H-pyrazol-5-yl)-8-methyl-[1,2,4]triazolo[1,5-a]pyridine (406);
6-(4-isopropyl-3-(2-(piperazin-1-yl)pyrimidin-5-yl)-1H-pyrazol-5-yl)-8-methyl-[1,2,4]triazolo[1,5-a]pyridine (407);
6-(4-isopropyl-3-(4-(4-neopentylpiperazin-1-yl)phenyl)-1H-pyrazol-5-yl)-8-methyl-[1,2,4]triazolo[1,5-a]pyridine (408);
N-isopropyl-1-(4-(4-isopropyl-5-(8-methyl-[1,2,4]triazolo[1,5-a]pyridin-6-yl)-1H-pyrazol-3-yl)phenyl)azetidin-3-amine (409);
6-(4-isopropyl-3-(2-(4-methylpiperazin-1-yl)pyrimidin-5-yl)-1H-pyrazol-5-yl)-8-methyl-[1,2,4]triazolo[1,5-a]pyridine (410);
6-(4-isopropyl-3-(4-(4-isopropylpiperazin-1-yl)phenyl)-1H-pyrazol-5-yl)-8-methyl-[1,2,4]triazolo[1,5-a]pyridine (411);
6-(4-isopropyl-3-(4-(4-methylpiperazin-1-yl)phenyl)-1H-pyrazol-5-yl)-8-methyl-[1,2,4]triazolo[1,5-a]pyridine (412);
6-(3-(4-(4-ethylpiperazin-1-yl)phenyl)-4-isopropyl-1H-pyrazol-5-yl)-8-methyl-[1,2,4]triazolo[1,5-a]pyridine (413);
6-(4-isopropyl-3-(4-(4-(oxetan-3-yl)piperazin-1-yl)phenyl)-1H-pyrazol-5-yl)-8-methyl-[1,2,4]triazolo[1,5-a]pyridine (414);
6-(4-isopropyl-3-(2-(4-isopropylpiperazin-1-yl)pyrimidin-5-yl)-1H-pyrazol-5-yl)-8-methyl-[1,2,4]triazolo[1,5-a]pyridine (415);
6-(4-isopropyl-3-(2-(4-(oxetan-3-yl)piperazin-1-yl)pyrimidin-5-yl)-1H-pyrazol-5-yl)-8-methyl-[1,2,4]triazolo[1,5-a]pyridine (416);
6-(4-isopropyl-3-(4-(4-(2-(methylsulfonyl)ethyl)piperazin-1-yl)phenyl)-1H-pyrazol-5-yl)-8-methyl-[1,2,4]triazolo[1,5-a]pyridine (417);
1-(4-(4-(4-isopropyl-5-(8-methyl-[1,2,4]triazolo[1,5-a]pyridin-6-yl)-1H-pyrazol-3-yl)phenyl)piperazin-1-yl)-2-methylpropan-2-ol (418);
6-(4-isopropyl-3-(5-(4-(2-methoxyethyl)piperazin-1-yl)pyridin-2-yl)-1H-pyrazol-5-yl)-8-methoxy-[1,2,4]triazolo[1,5-a]pyridine (419);
1-(4-(4-isopropyl-5-(8-methyl-[1,2,4]triazolo[1,5-a]pyridin-6-yl)-1H-pyrazol-3-yl)phenyl) -4-methylpiperazin-2-one (420);
4-isopropyl-1-(4-(4-isopropyl-5-(8-methyl-[1,2,4]triazolo[1,5-a]pyridin-6-yl)-1H-pyrazol-3-yl)phenyl)piperazin-2-one (421);
1-(4-(4-isopropyl-5-(8-methyl-[1,2,4]triazolo[1,5-a]pyridin-6-yl)-1H-pyrazol-3-yl)phenyl)-4-(oxetan-3-yl)piperazin-2-one (422);
6-(3-(5-(4-cyclobutylpiperazin-1-yl)pyridin-2-yl)-4-isopropyl-1H-pyrazol-5-yl)-8-methoxy-[1,2,4]triazolo[1,5-a]pyridine (423);
6-(3-(5-(2,6-diazaspiro[3.3]heptan-2-yl)pyridin-2-yl)-4-isopropyl-1H-pyrazol-5-yl)-8-methoxy-[1,2,4]triazolo[1,5-a]pyridine (424);
4-(4-(6-(4-isopropyl-5-(8-methoxy-[1,2,4]triazolo[1,5-a]pyridin-6-yl)-1H-pyrazol-3-yl)pyridin-3-yl)piperazin-1-yl)tetrahydro-2H-thiopyran 1,1-dioxide (425);
6-(3-(5-(4-(cyclopropylmethyl)piperazin-1-yl)pyridin-2-yl)-4-isopropyl-1H-pyrazol-5-yl)-8-methoxy-[1,2,4]triazolo[1,5-a]pyridine (426);
6-(3-(5-(4-ethylpiperazin-1-yl)pyridin-2-yl)-4-isopropyl-1H-pyrazol-5-yl)-8-methoxy-[1,2,4]triazolo[1,5-a]pyridine (427);
6-(4-isopropyl-3-(5-(4-methylpiperazin-1-yl)pyridin-2-yl)-1H-pyrazol-5-yl)-8-methoxy-[1,2,4]triazolo[1,5-a]pyridine (428);
6-(4-isopropyl-3-(5-(4-isopropylpiperazin-1-yl)pyridin-2-yl)-1H-pyrazol-5-yl)-8-methoxy-[1,2,4]triazolo[1,5-a]pyridine (429);
6-(3-(5-(4-isobutylpiperazin-1-yl)pyridin-2-yl)-4-isopropyl-1H-pyrazol-5-yl)-8-methoxy-[1,2,4]triazolo[1,5-a]pyridine (430);
6-(4-isopropyl-3-(5-(4-propylpiperazin-1-yl)pyridin-2-yl)-1H-pyrazol-5-yl)-8-methoxy-[1,2,4]triazolo[1,5-a]pyridine (431);
6-(4-isopropyl-3-(5-(4-((tetrahydro-2H-pyran-4-yl)methyl)piperazin-1-yl)pyridin-2-yl)-1H-pyrazol-5-yl)-8-methoxy-[1,2,4]triazolo[1,5-a]pyridine (432);
6-(4-isopropyl-3-(5-(4-(3,3,3-trifluoropropyl)piperazin-1-yl)pyridin-2-yl)-1H-pyrazol-5-yl)-8-methoxy-[1,2,4]triazolo[1,5-a]pyridine (433);
6-(3-(5-(4-(cyclobutylmethyl)piperazin-1-yl)pyridin-2-yl)-4-isopropyl-1H-pyrazol-5-yl)-8-methoxy-[1,2,4]triazolo[1,5-a]pyridine (434);
6-(4-isopropyl-3-(5-(4-(oxetan-3-yl)piperazin-1-yl)pyridin-2-yl)-1H-pyrazol-5-yl)-8-methoxy-[1,2,4]triazolo[1,5-a]pyridine (435);
6-(4-isopropyl-3-(5-(4-(tetrahydro-2H-pyran-4-yl)piperazin-1-yl)pyridin-2-yl)-1H-pyrazol-5-yl)-8-methoxy-[1,2,4]triazolo[1,5-a]pyridine (436);
6-(4-isopropyl-3-(5-(4-(pentan-3-yl)piperazin-1-yl)pyridin-2-yl)-1H-pyrazol-5-yl)-8-methoxy-[1,2,4]triazolo[1,5-a]pyridine (437);
6-(3-(5-(4-(2-ethylbutyl)piperazin-1-yl)pyridin-2-yl)-4-isopropyl-1H-pyrazol-5-yl)-8-methoxy-[1,2,4]triazolo[1,5-a]pyridine (438);
6-(3-(5-(4-((3-ethyloxetan-3-yl)methyl)piperazin-1-yl)pyridin-2-yl)-4-isopropyl-1H-pyrazol-5-yl)-8-methoxy-[1,2,4]triazolo[1,5-a]pyridine (439);
6-(4-isopropyl-3-(5-(6-((tetrahydro-2H-pyran-4-yl)methyl)-2,6-diazaspiro[3.3]heptan-2-yl)pyridin-2-yl)-1H-pyrazol-5-yl)-8-methoxy-[1,2,4]triazolo[1,5-a]pyridine (440);
6-(3-(5-(6-(cyclobutylmethyl)-2,6-diazaspiro[3.3]heptan-2-yl)pyridin-2-yl)-4-isopropyl-1H-pyrazol-5-yl)-8-methoxy-[1,2,4]triazolo[1,5-a]pyridine (441);
6-(3-(5-(6-isobutyl-2,6-diazaspiro[3.3]heptan-2-yl)pyridin-2-yl)-4-isopropyl-1H-pyrazol-5-yl)-8-methoxy-[1,2,4]triazolo[1,5-a]pyridine (442);
6-(4-isopropyl-3-(5-(6-(tetrahydro-2H-pyran-4-yl)-2,6-diazaspiro[3.3]heptan-2-yl)pyridin-2-yl)-1H-pyrazol-5-yl)-8-methoxy-[1,2,4]triazolo[1,5-a]pyridine (443);
6-(3-(5-(6-cyclobutyl-2,6-diazaspiro[3.3]heptan-2-yl)pyridin-2-yl)-4-isopropyl-1H-pyrazol-5-yl)-8-methoxy-[1,2,4]triazolo[1,5-a]pyridine (444);
6-(4-isopropyl-3-(5-(6-methyl-2,6-diazaspiro[3.3]heptan-2-yl)pyridin-2-yl)-1H-pyrazol-5-yl)-8-methoxy-[1,2,4]triazolo[1,5-a]pyridine (445);
6-(3-(5-(6-ethyl-2,6-diazaspiro[3.3]heptan-2-yl)pyridin-2-yl)-4-isopropyl-1H-pyrazol-5-yl)-8-methoxy-[1,2,4]triazolo[1,5-a]pyridine (446);
6-(4-isopropyl-3-(5-(6-propyl-2,6-diazaspiro[3.3]heptan-2-yl)pyridin-2-yl)-1H-pyrazol-5-yl)-8-methoxy-[1,2,4]triazolo[1,5-a]pyridine (447);
6-(4-isopropyl-3-(5-(6-isopropyl-2,6-diazaspiro[3.3]heptan-2-yl)pyridin-2-yl)-1H-pyrazol-5-yl)-8-methoxy-[1,2,4]triazolo[1,5-a]pyridine (448);
6-(4-isopropyl-3-(5-(6-(oxetan-3-yl)-2,6-diazaspiro[3.3]heptan-2-yl)pyridin-2-yl)-1H-pyrazol-5-yl)-8-methoxy-[1,2,4]triazolo[1,5-a]pyridine (449);
6-(3-(5-(6-(cyclopropylmethyl)-2,6-diazaspiro[3.3]heptan-2-yl)pyridin-2-yl)-4-isopropyl-1H-pyrazol-5-yl)-8-methoxy-[1,2,4]triazolo[1,5-a]pyridine (450);
2-(dimethylamino)-1-(4-(6-(4-isopropyl-5-(8-methoxy-[1,2,4]triazolo[1,5-a]pyridin-6-yl)-1H-pyrazol-3-yl)pyridin-3-yl)piperazin-1-yl)ethan-1-one (451);
2-(diethylamino)-1-(4-(6-(4-isopropyl-5-(8-methoxy-[1,2,4]triazolo[1,5-a]pyridin-6-yl)-1H-pyrazol-3-yl)pyridin-3-yl)piperazin-1-yl)ethan-1-one (452);
2-(dimethylamino)-1-(6-(6-(4-isopropyl-5-(8-methoxy-[1,2,4]triazolo[1,5-a]pyridin-6-yl)-1H-pyrazol-3-yl)pyridin-3-yl)-2,6-diazaspiro[3.3]heptan-2-yl)ethan-1-one (453);
6-(4-isopropyl-3-(5-(4-(2-(methylsulfonyl)ethyl)piperazin-1-yl)pyridin-2-yl)-1H-pyrazol-5-yl)-8-methoxy-[1,2,4]triazolo[1,5-a]pyridine (454);
2-(4-(6-(4-isopropyl-5-(8-methoxy-[1,2,4]triazolo[1,5-a]pyridin-6-yl)-1H-pyrazol-3-yl)pyridin-3-yl)piperazin-1-yl)-N,N-dimethylacetamide (455);
2-(4-(6-(4-isopropyl-5-(8-methoxy-[1,2,4]triazolo[1,5-a]pyridin-6-yl)-1H-pyrazol-3-yl)pyridin-3-yl)piperazin-1-yl)-N-methylacetamide (456);
6-(4-isopropyl-3-(5-(6-(2-(methylsulfonyl)ethyl)-2,6-diazaspiro[3.3]heptan-2-yl)pyridin-2-yl)-1H-pyrazol-5-yl)-8-methoxy-[1,2,4]triazolo[1,5-a]pyridine (457);
6-(4-isopropyl-3-(5-(6-(2-methoxyethyl)-2,6-diazaspiro[3.3]heptan-2-yl)pyridin-2-yl)-1H-pyrazol-5-yl)-8-methoxy-[1,2,4]triazolo[1,5-a]pyridine (458);
2-(4-(4-isopropyl-5-(8-methyl-[1,2,4]triazolo[1,5-a]pyridin-6-yl)-1H-pyrazol-3-yl)phenyl)-4-methylmorpholine (459-460);
4-ethyl-2-(4-(4-isopropyl-5-(8-methyl-[1,2,4]triazolo[1,5-a]pyridin-6-yl)-1H-pyrazol-3-yl)phenyl)morpholine (461);
4-isopropyl-2-(4-(4-isopropyl-5-(8-methyl-[1,2,4]triazolo[1,5-a]pyridin-6-yl)-1H-pyrazol-3-yl)phenyl)morpholine (462-463);
2-(4-(4-isopropyl-5-(8-methyl-[1,2,4]triazolo[1,5-a]pyridin-6-yl)-1H-pyrazol-3-yl)phenyl)-4-neopentylmorpholine (464-465);
2-(2-(4-(4-isopropyl-5-(8-methyl-[1,2,4]triazolo[1,5-a]pyridin-6-yl)-1H-pyrazol-3-yl) phenyl)morpholino)-N,N-dimethylacetamide (466-468);
2-(dimethylamino)-1-(2-(4-(4-isopropyl-5-(8-methyl-[1,2,4]triazolo[1,5-a]pyridin-6-yl)-1H-pyrazol-3-yl)phenyl)morpholino)ethan-1-one (469-470);
4-(4-(4-isopropyl-5-(8-methyl-[1,2,4]triazolo[1,5-a]pyridin-6-yl)-1H-pyrazol-3-yl)phenyl)-N-methylcyclohexan-1-amine (471);
4-(4-(4-isopropyl-5-(8-methyl-[1,2,4]triazolo[1,5-a]pyridin-6-yl)-1H-pyrazol-3-yl)phenyl)-N,N-dimethylcyclohexan-1-amine (472);
N-cyclobutyl-4-(6-(4-isopropyl-5-(8-methoxy-[1,2,4]triazolo[1,5-a]pyridin-6-yl)-1H-pyrazol-3-yl)pyridin-3-yl)cyclohexan-1-amine (473-474);
2-(4-(6-(4-isopropyl-5-(8-methoxy-[1,2,4]triazolo[1,5-a]pyridin-6-yl)-1H-pyrazol-3-yl)pyridin-3-yl)cyclohexyl)-7-oxa-2-azaspiro[3.5]nonane (475-476);
6-(4-(6-(4-isopropyl-5-(8-methoxy-[1,2,4]triazolo[1,5-a]pyridin-6-yl)-1H-pyrazol-3-yl)pyridin-3-yl)cyclohexyl)-2-oxa-6-azaspiro[3.3]heptane (477-478);
6-(4-isopropyl-3-(5-(4-(3-methoxyazetidin-1-yl)cyclohexyl)pyridin-2-yl)-1H-pyrazol-5-yl)-8-methoxy-[1,2,4]triazolo[1,5-a]pyridine (479-480);
4-(6-(4-isopropyl-5-(8-methoxy-[1,2,4]triazolo[1,5-a]pyridin-6-yl)-1H-pyrazol-3-yl)pyridin-3-yl)-N-(2-methoxy-2-methylpropyl)cyclohexan-1-amine (481-482);
4-(4-(6-(4-isopropyl-5-(8-methoxy-[1,2,4]triazolo[1,5-a]pyridin-6-yl)-1H-pyrazol-3-yl)pyridin-3-yl)cyclohexyl)piperazin-2-one (483-484);
4-(6-(4-isopropyl-5-(8-methoxy-[1,2,4]triazolo[1,5-a]pyridin-6-yl)-1H-pyrazol-3-yl)pyridin-3-yl)-N-((1-(methylsulfonyl)cyclopropyl)methyl)cyclohexan-1-amine (485-486);
7-(4-(6-(4-isopropyl-5-(8-methoxy-[1,2,4]triazolo[1,5-a]pyridin-6-yl)-1H-pyrazol-3-yl)pyridin-3-yl)cyclohexyl)-2-oxa-7-azaspiro[3.5]nonane (487-488);
4-(6-(4-isopropyl-5-(8-methoxy-[1,2,4]triazolo[1,5-a]pyridin-6-yl)-1H-pyrazol-3-yl)pyridin-3-yl)-N-(2-methoxyethyl)-N-methylcyclohexan-1-amine (490-491);
N-(4-(6-(4-isopropyl-5-(8-methoxy-[1,2,4]triazolo[1,5-a]pyridin-6-yl)-1H-pyrazol-3-yl)pyridin-3-yl)cyclohexyl)-3-methyloxetan-3-amine (492-493);
4-(4-(6-(4-isopropyl-5-(8-methoxy-[1,2,4]triazolo[1,5-a]pyridin-6-yl)-1H-pyrazol-3-yl)pyridin-3-yl)cyclohexyl)morpholine (494-495);
6-(4-isopropyl-3-(5-(4-(3-methoxyazetidin-1-yl)cyclohexyl)-4-methylpyridin-2-yl)-1H-pyrazol-5-yl)-8-methoxy-[1,2,4]triazolo[1,5-a]pyridine (496);
4-(6-(4-isopropyl-5-(8-methoxy-[1,2,4]triazolo[1,5-a]pyridin-6-yl)-1H-pyrazol-3-yl)-4-methylpyridin-3-yl)-N-((1-(methylsulfonyl)cyclopropyl)methyl)cyclohexan-1-amine (497-498);
4-(6-(4-isopropyl-5-(8-methoxy-[1,2,4]triazolo[1,5-a]pyridin-6-yl)-1H-pyrazol-3-yl)-4-methylpyridin-3-yl)-N-(2-methoxyethyl)cyclohexan-1-amine (499-500);
7-(4-(6-(4-isopropyl-5-(8-methoxy-[1,2,4]triazolo[1,5-a]pyridin-6-yl)-1H-pyrazol-3-yl)-4-methylpyridin-3-yl)cyclohexyl)-2-oxa-7-azaspiro[3.5]nonane (501);
6-(3-(5-(4-(4,4-difluoropiperidin-1-yl)cyclohexyl)-4-methylpyridin-2-yl)-4-isopropyl-1H-pyrazol-5-yl)-8-methoxy-[1,2,4]triazolo[1,5-a]pyridine (502-503);
6-(4-isopropyl-3-(5-(4-(4-methoxypiperidin-1-yl)cyclohexyl)-4-methylpyridin-2-yl)-1H-pyrazol-5-yl)-8-methoxy-[1,2,4]triazolo[1,5-a]pyridine (504-505);
4-(6-(4-isopropyl-5-(8-methoxy-[1,2,4]triazolo[1,5-a]pyridin-6-yl)-1H-pyrazol-3-yl)-4-methylpyridin-3-yl)-N,N-dimethylcyclohexan-1-amine (506-507);
4-(6-(4-isopropyl-5-(8-methoxy-[1,2,4]triazolo[1,5-a]pyridin-6-yl)-1H-pyrazol-3-yl)-4-methylpyridin-3-yl)cyclohexan-1-amine (508-509);
4-(4-(4-isopropyl-5-(8-methyl-[1,2,4]triazolo[1,5-a]pyridin-6-yl)-1H-pyrazol-3-yl)phenyl)-N,N-dimethylcyclohexan-1-amine (510 and 513);
N-(4-(4-(4-isopropyl-5-(8-methyl-[1,2,4]triazolo[1,5-a]pyridin-6-yl)-1H-pyrazol-3-yl)phenyl)cyclohexyl)-N-methyloxetan-3-amine (511 and 514);
N-((3-ethyloxetan-3-yl)methyl)-4-(4-(4-isopropyl-5-(8-methyl-[1,2,4]triazolo[1,5-a]pyridin-6-yl)-1H-pyrazol-3-yl)phenyl)-N-methylcyclohexan-1-amine (512 and 515);
N-ethyl-4-(4-(4-isopropyl-5-(8-methyl-[1,2,4]triazolo[1,5-a]pyridin-6-yl)-1H-pyrazol-3-yl)phenyl)-N-methylcyclohexan-1-amine (516-517);
2-(dimethylamino)-N-(4-(4-(4-isopropyl-5-(8-methyl-[1,2,4]triazolo[1,5-a]pyridin-6-yl)-1H-pyrazol-3-yl)phenyl)cyclohexyl)-N-methylacetamide (518-519);
2-((4-(4-(4-isopropyl-5-(8-methyl-[1,2,4]triazolo[1,5-a]pyridin-6-yl)-1H-pyrazol-3-yl)phenyl)cyclohexyl)(methyl)amino)-N,N-dimethylacetamide (520-521);
4-(4-(4-isopropyl-5-(8-methyl-[1,2,4]triazolo[1,5-a]pyridin-6-yl)-1H-pyrazol-3-yl)phenyl)-N-(2-methoxyethyl)-N-methylcyclohexan-1-amine (522-523);
2-(4-( 4-isopropyl-5-(8-methoxy-[1,2,4]triazolo[1,5-a]pyridin-6-yl)-1H-pyrazol-3-yl) phenyl)propan-2-amine, TFA (524);
2-(4-(4-isopropyl-5-(8-methyl-[1,2,4]triazolo[1,5-a]pyridin-6-yl)-1H-pyrazol-3-yl)phenyl)propan-2-amine (525);
1-(4-(4-isopropyl-5-(8-methyl-[1,2,4]triazolo[1,5-a]pyridin-6-yl)-1H-pyrazol-3-yl)phenyl)cyclopropan-1-amine (526);
(S)-1-(4-(4-isopropyl-5-(8-methyl-[1,2,4]triazolo[1,5-a]pyridin-6-yl)-1H-pyrazol-3-yl)phenyl)ethan-1-amine (527);
(S)-1-(4-(4-isopropyl-5-(8-methoxy-[1,2,4]triazolo[1,5-a]pyridin-6-yl)-1H-pyrazol-3-yl)phenyl)ethan-1-amine (528);
(R)-1-(4-(4-isopropyl-5-(8-methyl-[1,2,4]triazolo[1,5-a]pyridin-6-yl)-1H-pyrazol-3-yl)phenyl)ethan-1-amine (529);
6-(4-isopropyl-3-(4-(pyrrolidin-2-yl)phenyl)-1H-pyrazol-5-yl)-8-methyl-[1,2,4]triazolo[1,5-a]pyridine (530-531);
2-(4-(4-isopropyl-5-(8-methoxy-[1,2,4]triazolo[1,5-a]pyridin-6-yl)-1H-pyrazol-3-yl)phenyl)-N,N-dimethylpropan-2-amine (532);
N-isopropyl-2-(4-(4-isopropyl-5-(8-methoxy-[1,2,4]triazolo[1,5-a]pyridin-6-yl)-1H-pyrazol-3-yl)phenyl)propan-2-amine (533);
N-ethyl-2-(4-(4-isopropyl-5-(8-methoxy-[1,2,4]triazolo[1,5-a]pyridin-6-yl)-1H-pyrazol-3-yl)phenyl)propan-2-amine (534);
N,N-diethyl-2-(4-(4-isopropyl-5-(8-methoxy-[1,2,4]triazolo[1,5-a]pyridin-6-yl)-1H-pyrazol-3-yl)phenyl)propan-2-amine (535);
N-ethyl-2-(4-(4-isopropyl-5-(8-methyl-[1,2,4]triazolo[1,5-a]pyridin-6-yl)-1H-pyrazol-3-yl)phenyl)propan-2-amine (536);
N-(2-(4-(4-isopropyl-5-(8-methoxy-[1,2,4]triazolo[1,5-a]pyridin-6-yl)-1H-pyrazol-3-yl)phenyl)propan-2-yl)tetrahydro-2H-pyran-4-amine (537);
2-(4-(4-isopropyl-5-(8-methyl-[1,2,4]triazolo[1,5-a]pyridin-6-yl)-1H-pyrazol-3-yl)phenyl)-N,N-dimethylpropan-2-amine (538);
N,N-diethyl-2-(4-(4-isopropyl-5-(8-methyl-[1,2,4]triazolo[1,5-a]pyridin-6-yl)-1H-pyrazol-3-yl)phenyl)propan-2-amine (539);
N-(2-(4-(4-isopropyl-5-(8-methyl-[1,2,4]triazolo[1,5-a]pyridin-6-yl)-1H-pyrazol-3-yl)phenyl)propan-2-yl)tetrahydro-2H-pyran-4-amine (540);
N-(2-(4-(4-isopropyl-5-(8-methyl-[1,2,4]triazolo[1,5-a]pyridin-6-yl)-1H-pyrazol-3-yl)phenyl)propan-2-yl)-2,2-dimethylpropan-1-amine (541);
2-(4-(4-isopropyl-5-(8-methyl-[1,2,4]triazolo[1,5-a]pyridin-6-yl)-1H-pyrazol-3-yl)phenyl)-N-((3-methyloxetan-3-yl)methyl)propan-2-amine (542);
N-isopropyl-2-(4-(4-isopropyl-5-(8-methyl-[1,2,4]triazolo[1,5-a]pyridin-6-yl)-1H-pyrazol-3-yl)phenyl)propan-2-amine (543);
2-(4-(4-isopropyl-5-(8-methyl-[1,2,4]triazolo[1,5-a]pyridin-6-yl)-1H-pyrazol-3-yl)phenyl)-N-((1-methyl-1H-1,2,4-triazol-3-yl)methyl)propan-2-amine (544);
N-(2-(4-(4-isopropyl-5-(8-methyl-[1,2,4]triazolo[1,5-a]pyridin-6-yl)-1H-pyrazol-3-yl)phenyl)propan-2-yl)oxetan-3-amine (545);
1-(4-(4-isopropyl-5-(8-methyl-[1,2,4]triazolo[1,5-a]pyridin-6-yl)-1H-pyrazol-3-yl)phenyl)-N,N-dimethylcyclopropan-1-amine (546);
N-isopropyl-1-(4-(4-isopropyl-5-(8-methyl-[1,2,4]triazolo[1,5-a]pyridin-6-yl)-1H-pyrazol-3-yl)phenyl)cyclopropan-1-amine (547);
1-(4-(4-isopropyl-5-(8-methyl-[1,2,4]triazolo[1,5-a]pyridin-6-yl)-1H-pyrazol-3-yl)phenyl)-N-neopentylcyclopropan-1-amine (548);
(S)-1-(4-(4-isopropyl-5-(8-methyl-[1,2,4]triazolo[1,5-a]pyridin-6-yl)-1H-pyrazol-3-yl)phenyl)-N,N-dimethylethan-1-amine (549);
(S)-N-ethyl-1-(4-(4-isopropyl-5-(8-methyl-[1,2,4]triazolo[1,5-a]pyridin-6-yl)-1H-pyrazol-3-yl)phenyl)ethan-1-amine (550);
(S)-N-(1-(4-(4-isopropyl-5-(8-methyl-[1,2,4]triazolo[1,5-a]pyridin-6-yl)-1H-pyrazol-3-yl)phenyl)ethyl)propan-2-amine (551);
N-((S)-1-(4-(4-isopropyl-5-(8-methyl-[1,2,4]triazolo[1,5-a]pyridin-6-yl)-1H-pyrazol-3-yl)phenyl)ethyl)-3-methylbutan-2-amine (552-553);
(S)-N-(1-(4-(4-isopropyl-5-(8-methyl-[1,2,4]triazolo[1,5-a]pyridin-6-yl)-1H-pyrazol-3-yl)phenyl)ethyl)-2,2-dimethylpropan-1-amine (554);
(S)-1-(4-(4-isopropyl-5-(8-methoxy-[1,2,4]triazolo[1,5-a]pyridin-6-yl)-1H-pyrazol-3-yl)phenyl)-N,N-dimethylethan-1-amine (555);
(S)-N-(1-(4-(4-isopropyl-5-(8-methoxy-[1,2,4]triazolo[1,5-a]pyridin-6-yl)-1H-pyrazol-3-yl)phenyl)ethyl)propan-2-amine (556);
(R)-1-(4-(4-isopropyl-5-(8-methyl-[1,2,4]triazolo[1,5-a]pyridin-6-yl)-1H-pyrazol-3-yl)phenyl)-N,N-dimethylethan-1-amine (557);
(R)-N-(1-(4-(4-isopropyl-5-(8-methyl-[1,2,4]triazolo[1,5-a]pyridin-6-yl)-1H-pyrazol-3-yl)phenyl)ethyl)propan-2-amine (558);
(R)-1-(4-(4-isopropyl-5-(8-methyl-[1,2,4]triazolo[1,5-a]pyridin-6-yl)-1H-pyrazol-3-yl)phenyl)-N-((3-methyloxetan-3-yl)methyl)ethan-1-amine (559);
(R)-N-(1-(4-(4-isopropyl-5-(8-methyl-[1,2,4]triazolo[1,5-a]pyridin-6-yl)-1H-pyrazol-3-yl)phenyl)ethyl)-2,2-dimethylpropan-1-amine (560);
N-((R)-1-(4-(4-isopropyl-5-(8-methyl-[1,2,4]triazolo[1,5-a]pyridin-6-yl)-1H-pyrazol-3-yl)phenyl)ethyl)-3-methylbutan-2-amine (561);
N-((R)-1-(4-(4-isopropyl-5-(8-methyl-[1,2,4]triazolo[1,5-a]pyridin-6-yl)-1H-pyrazol-3-yl)phenyl)ethyl)-3-methylbutan-2-amine (562);
6-(4-isopropyl-3-(4-(1-methylpyrrolidin-2-yl)phenyl)-1H-pyrazol-5-yl)-8-methyl-[1,2,4]triazolo[1,5-a]pyridine (563 and 566);
6-(4-isopropyl-3-(4-(1-isopropylpyrrolidin-2-yl)phenyl)-1H-pyrazol-5-yl)-8-methyl-[1,2,4]triazolo[1,5-a]pyridine (564 and 567);
6-(4-isopropyl-3-(4-(1-(oxetan-3-yl)pyrrolidin-2-yl)phenyl)-1H-pyrazol-5-yl)-8-methyl-[1,2,4]triazolo[1,5-a]pyridine (565 and 568);
(S)-N-(1-(4-(4-isopropyl-5-(8-methyl-[1,2,4]triazolo[1,5-a]pyridin-6-yl)-1H-pyrazol-3-yl)phenyl)ethyl)oxetan-3-amine (569);
(S)-N-ethyl-1-(4-(4-isopropyl-5-(8-methoxy-[1,2,4]triazolo[1,5-a]pyridin-6-yl)-1H-pyrazol-3-yl)phenyl)-N-methylethan-1-amine (570);
(S)-1-(4-(4-isopropyl-5-(8-methyl-[1,2,4]triazolo[1,5-a]pyridin-6-yl)-1H-pyrazol-3-yl)phenyl)-N-methylethan-1-amine (571);
(S)-N-(1-(4-(4-isopropyl-5-(8-methoxy-[1,2,4]triazolo[1,5-a]pyridin-6-yl)-1H-pyrazol-3-yl)phenyl)ethyl)-N-methylpropan-2-amine (572);
(S)-N-(1-(4-(4-isopropyl-5-(8-methoxy-[1,2,4]triazolo[1,5-a]pyridin-6-yl)-1H-pyrazol-3-yl)phenyl)ethyl)-N-methyloxetan-3-amine (573);
(S)-1-(4-(4-isopropyl-5-(8-methoxy-[1,2,4]triazolo[1,5-a]pyridin-6-yl)-1H-pyrazol-3-yl)phenyl)-N-methyl-N-((3-methyloxetan-3-yl)methyl)ethan-1-amine (574);
(S)-2,2,2-trifluoro-N-(1-(4-(4-isopropyl-5-(8-methoxy-[1,2,4]triazolo[1,5-a]pyridin-6-yl)-1H-pyrazol-3-yl)phenyl)ethyl)-N-methylethan-1-amine (575);
(S)-2,2,2-trifluoro-N-(1-(4-(4-isopropyl-5-(8-methyl-[1,2,4]triazolo[1,5-a]pyridin-6-yl)-1H-pyrazol-3-yl)phenyl)ethyl)-N-methylethan-1-amine (576);
(S)-N-ethyl-1-(4-(4-isopropyl-5-(8-methyl-[1,2,4]triazolo[1,5-a]pyridin-6-yl)-1H-pyrazol-3-yl)phenyl)-N-methylethan-1-amine (577);
(S)-N-(1-(4-(4-isopropyl-5-(8-methyl-[1,2,4]triazolo[1,5-a]pyridin-6-yl)-1H-pyrazol-3-yl)phenyl)ethyl)-N-methylpropan-2-amine (578);
(S)-N-(1-(4-(4-isopropyl-5-(8-methyl-[1,2,4]triazolo[1,5-a]pyridin-6-yl)-1H-pyrazol-3-yl)phenyl)ethyl)-N-methyloxetan-3-amine (579);
(S)-1-(4-(4-isopropyl-5-(8-methyl-[1,2,4]triazolo[1,5-a]pyridin-6-yl)-1H-pyrazol-3-yl)phenyl)-N-methyl-N-((3-methyloxetan-3-yl)methyl)ethan-1-amine (580);
2-(dimethylamino)-N-(2-(4-(4-isopropyl-5-(8-methyl-[1,2,4]triazolo[1,5-a]pyridin-6-yl)-1H-pyrazol-3-yl)phenyl)propan-2-yl)acetamide (581);
2-(dimethylamino)-1-(2-(4-(4-isopropyl-5-(8-methyl-[1,2,4]triazolo[1,5-a]pyridin-6-yl)-1H-pyrazol-3-yl)phenyl)pyrrolidin-1-yl)ethan-1-one (582-583);
2-(2-(4-(4-isopropyl-5-(8-methyl-[1,2,4]triazolo[1,5-a]pyridin-6-yl)-1H-pyrazol-3-yl) phenyl)pyrrolidin-1-yl)-N,N-dimethylacetamide (584-585);
(S)-N-((S)-1-(4-(4-isopropyl-5-(8-methoxy-[1,2,4]triazolo[1,5-a]pyridin-6-yl)-1H-pyrazol-3-yl)phenyl)ethyl)-N,1-dimethylazetidine-2-carboxamide (585);
(S)-2-(dimethylamino)-N-(1-(4-(4-isopropyl-5-(8-methoxy-[1,2,4]triazolo[1,5-a]pyridin-6-yl)-1H-pyrazol-3-yl)phenyl)ethyl)-N-methylacetamide (586);
(S)-2-(diethylamino)-N-(1-(4-(4-isopropyl-5-(8-methoxy-[1,2,4]triazolo[1,5-a]pyridin-6-yl)-1H-pyrazol-3-yl)phenyl)ethyl)-N-methylacetamide (587);
(S)-2-(dimethylamino)-N-((S)-1-(4-(4-isopropyl-5-(8-methoxy-[1,2,4]triazolo[1,5-a]pyridin-6-yl)-1H-pyrazol-3-yl)phenyl)ethyl)-N-methylpropanamide (588);
(R)-2-(dimethylamino)-N-((S)-1-(4-(4-isopropyl-5-(8-methoxy-[1,2,4]triazolo[1,5-a]pyridin-6-yl)-1H-pyrazol-3-yl)phenyl)ethyl)-N-methylpropanamide (589);
(S)-2-(dimethylamino)-N-(1-(4-(4-isopropyl-5-(8-methyl-[1,2,4]triazolo[1,5-a]pyridin-6-yl)-1H-pyrazol-3-yl)phenyl)ethyl)-N-methylacetamide (590);
(S)-2-(diethylamino)-N-(1-(4-(4-isopropyl-5-(8-methyl-[1,2,4]triazolo[1,5-a]pyridin-6-yl)-1H-pyrazol-3-yl)phenyl)ethyl)-N-methylacetamide (591);
(R)-2-(dimethylamino)-N-((S)-1-(4-(4-isopropyl-5-(8-methyl-[1,2,4]triazolo[1,5-a]pyridin-6-yl)-1H-pyrazol-3-yl)phenyl)ethyl)-N-methylpropanamide (592);
(S)-2-(dimethylamino)-N-((S)-1-(4-(4-isopropyl-5-(8-methyl-[1,2,4]triazolo[1,5-a]pyridin-6-yl)-1H-pyrazol-3-yl)phenyl)ethyl)-N-methylpropanamide (593);
(S)-N-((S)-1-(4-(4-isopropyl-5-(8-methoxy-[1,2,4]triazolo[1,5-a]pyridin-6-yl)-1H-pyrazol-3-yl)phenyl)ethyl)-N-methyl-2-(methylamino)propanamide (594);
(R)-N-((S)-1-(4-(4-isopropyl-5-(8-methoxy-[1,2,4]triazolo[1,5-a]pyridin-6-yl)-1H-pyrazol-3-yl)phenyl)ethyl)-N-methyl-2-(methylamino)propanamide (595);
(S)-N-((S)-1-(4-(4-isopropyl-5-(8-methyl-[1,2,4]triazolo[1,5-a]pyridin-6-yl)-1H-pyrazol-3-yl)phenyl)ethyl)-N-methylazetidine-2-carboxamide (596);
(R)-N-((S)-1-(4-(4-isopropyl-5-(8-methyl-[1,2,4]triazolo[1,5-a]pyridin-6-yl)-1H-pyrazol-3-yl)phenyl)ethyl)-N-methyl-2-(methylamino)propanamide (597);
(S)-N-((S)-1-(4-(4-isopropyl-5-(8-methyl-[1,2,4]triazolo[1,5-a]pyridin-6-yl)-1H-pyrazol-3-yl)phenyl)ethyl)-N-methyl-2-(methylamino)propanamide (598);
(S)-N-((S)-1-(4-(4-isopropyl-5-(8-methyl-[1,2,4]triazolo[1,5-a]pyridin-6-yl)-1H-pyrazol-3-yl)phenyl)ethyl)-N-methylpyrrolidine-2-carboxamide (599);
(R)-N-((S)-1-(4-(4-isopropyl-5-(8-methyl-[1,2,4]triazolo[1,5-a]pyridin-6-yl)-1H-pyrazol-3-yl)phenyl)ethyl)-N-methylpyrrolidine-2-carboxamide (600);
(S)-1-ethyl-N-((S)-1-(4-(4-isopropyl-5-(8-methoxy-[1,2,4]triazolo[1,5-a]pyridin-6-yl)-1H-pyrazol-3-yl)phenyl)ethyl)-N-methylazetidine-2-carboxamide (601);
(S)-N-((S)-1-(4-(4-isopropyl-5-(8-methyl-[1,2,4]triazolo[1,5-a]pyridin-6-yl)-1H-pyrazol-3-yl)phenyl)ethyl)-N,1-dimethylazetidine-2-carboxamide (602);
(S)-1-ethyl-N-((S)-1-(4-(4-isopropyl-5-(8-methyl-[1,2,4]triazolo[1,5-a]pyridin-6-yl)-1H-pyrazol-3-yl)phenyl)ethyl)-N-methylazetidine-2-carboxamide (603);
(S)-N-((S)-1-(4-(4-isopropyl-5-(8-methyl-[1,2,4]triazolo[1,5-a]pyridin-6-yl)-1H-pyrazol-3-yl)phenyl)ethyl)-N-methyl-1-(oxetan-3-yl)azetidine-2-carboxamide (604);
(S)-N-((S)-1-(4-(4-isopropyl-5-(8-methyl-[1,2,4]triazolo[1,5-a]pyridin-6-yl)-1H-pyrazol-3-yl)phenyl)ethyl)-N,1-dimethylpyrrolidine-2-carboxamide (605);
(S)-N-((S)-1-(4-(4-isopropyl-5-(8-methyl-[1,2,4]triazolo[1,5-a]pyridin-6-yl)-1H-pyrazol-3-yl)phenyl)ethyl)-N-methyl-1-(oxetan-3-yl)pyrrolidine-2-carboxamide (606);
(R)-N-((S)-1-(4-(4-isopropyl-5-(8-methyl-[1,2,4]triazolo[1,5-a]pyridin-6-yl)-1H-pyrazol-3-yl)phenyl)ethyl)-N,1-dimethylpyrrolidine-2-carboxamide (607);
(R)-N-((S)-1-(4-(4-isopropyl-5-(8-methyl-[1,2,4]triazolo[1,5-a]pyridin-6-yl)-1H-pyrazol-3-yl)phenyl)ethyl)-N-methyl-1-(oxetan-3-yl)pyrrolidine-2-carboxamide (608);
(S)-2-(ethyl(methyl)amino)-N-((S)-1-(4-(4-isopropyl-5-(8-methyl-[1,2,4]triazolo[1,5-a]pyridin-6-yl)-1H-pyrazol-3-yl)phenyl)ethyl)-N-methylpropanamide (609);
(R)-2-(ethyl(methyl)amino)-N-((S)-1-(4-(4-isopropyl-5-(8-methyl-[1,2,4]triazolo[1,5-a]pyridin-6-yl)-1H-pyrazol-3-yl)phenyl)ethyl)-N-methylpropanamide (610);
(S)-2-(ethyl(methyl)amino)-N-((S)-1-(4-(4-isopropyl-5-(8-methoxy-[1,2,4]triazolo[1,5-a]pyridin-6-yl)-1H-pyrazol-3-yl)phenyl)ethyl)-N-methylpropanamide (611);
(R)-N-((S)-1-(4-(4-isopropyl-5-(8-methoxy-[1,2,4]triazolo[1,5-a]pyridin-6-yl)-1H-pyrazol-3-yl)phenyl)ethyl)-N-methyl-2-(methyl(oxetan-3-yl)amino)propanamide (612);
(S)-2-(ethyl(methyl)amino)-N-((S)-1-(4-(4-isopropyl-5-(8-methoxy-[1,2,4]triazolo[1,5-a]pyridin-6-yl)-1H-pyrazol-3-yl)phenyl)ethyl)-N-methylpropanamide (613);
(R)-N-((S)-1-(4-(4-isopropyl-5-(8-methyl-[1,2,4]triazolo[1,5-a]pyridin-6-yl)-1H-pyrazol-3-yl)phenyl)ethyl)-N-methyl-2-(methyl(oxetan-3-yl)amino)propanamide (614);
(S)-N-((S)-1-(4-(4-isopropyl-5-(8-methyl-[1,2,4]triazolo[1,5-a]pyridin-6-yl)-1H-pyrazol-3-yl)phenyl)ethyl)-N-methyl-2-(methyl(oxetan-3-yl)amino)propanamide (615);
(S)-2-((1-(4-(4-isopropyl-5-(8-methyl-[1,2,4]triazolo[1,5-a]pyridin-6-yl)-1H-pyrazol-3-yl)phenyl)ethyl)(methyl)amino)-N,N-dimethylacetamide (616);
(S)-2-((1-(4-(4-isopropyl-5-(8-methoxy-[1,2,4]triazolo[1,5-a]pyridin-6-yl)-1H-pyrazol-3-yl)phenyl)ethyl)(methyl)amino)-N,N-dimethylacetamide (617);
(S)-2-(dimethylamino)-N-(1-(4-(4-isopropyl-5-(8-methyl-[1,2,4]triazolo[1,5-a]pyridin-6-yl)-1H-pyrazol-3-yl)phenyl)ethyl)-N-(oxetan-3-yl)acetamide (618);
(S)-2-(diethylamino)-N-(1-(4-(4-isopropyl-5-(8-methyl-[1,2,4]triazolo[1,5-a]pyridin-6-yl)-1H-pyrazol-3-yl)phenyl)ethyl)-N-(oxetan-3-yl)acetamide (619);
(S)-2-(ethyl(methyl)amino)-N-(1-(4-(4-isopropyl-5-(8-methyl-[1,2,4]triazolo[1,5-a]pyridin-6-yl)-1H-pyrazol-3-yl)phenyl)ethyl)-N-methylacetamide (620);
(S)-N-(1-(4-(4-isopropyl-5-(8-methyl-[1,2,4]triazolo[1,5-a]pyridin-6-yl)-1H-pyrazol-3-yl)phenyl)ethyl)-N-methyl-2-(methyl(3,3,3-trifluoropropyl)amino)acetamide (621);
(S)-2-(3-fluoroazetidin-1-yl)-N-(1-(4-(4-isopropyl-5-(8-methyl-[1,2,4]triazolo[1,5-a]pyridin-6-yl)-1H-pyrazol-3-yl)phenyl)ethyl)-N-methylacetamide (622);
(S)-2-((2-fluoro-2-methylpropyl)amino)-N-(1-(4-(4-isopropyl-5-(8-methyl-[1,2,4]triazolo[1,5-a]pyridin-6-yl)-1H-pyrazol-3-yl)phenyl)ethyl)-N-methylacetamide (623);
(S)-1-(2-fluoro-5-(4-isopropyl-5-(8-methyl-[1,2,4]triazolo[1,5-a]pyridin-6-yl)-1H-pyrazol-3-yl)phenyl)-N,N-dimethylethan-1-amine (624);
(S)-N,N-diethyl-1-(2-fluoro-5-(4-isopropyl-5-(8-methyl-[1,2,4]triazolo[1,5-a]pyridin-6-yl)-1H-pyrazol-3-yl)phenyl)ethan-1-amine (625);
(S)-N-(1-(2-fluoro-5-(4-isopropyl-5-(8-methyl-[1,2,4]triazolo[1,5-a]pyridin-6-yl)-1H-pyrazol-3-yl)phenyl)ethyl)propan-1-amine (626);
(S)-N-(1-(2-fluoro-5-(4-isopropyl-5-(8-methyl-[1,2,4]triazolo[1,5-a]pyridin-6-yl)-1H-pyrazol-3-yl)phenyl)ethyl)-N-propylpropan-1-amine (627);
(S)-N-(1-(2-fluoro-5-(4-isopropyl-5-(8-methyl-[1,2,4]triazolo[1,5-a]pyridin-6-yl)-1H-pyrazol-3-yl)phenyl)ethyl)propan-2-amine (628);
(S)-2-(dimethylamino)-N-(1-(2-fluoro-5-(4-isopropyl-5-(8-methyl-[1,2,4]triazolo[1,5-a] pyridin-6-yl)-1H-pyrazol-3-yl)phenyl)ethyl)acetamide (629);
(S)-2-(diethylamino)-N-(1-(2-fluoro-5-(4-isopropyl-5-(8-methyl-[1,2,4]triazolo[1,5-a]pyridin-6-yl)-1H-pyrazol-3-yl)phenyl)ethyl)acetamide (630);
N-(1-(4-(4-isopropyl-5-(8-methyl-[1,2,4]triazolo[1,5-a]pyridin-6-yl)-1H-pyrazol-3-yl) phenyl)ethyl)propan-2-amine (631);
1-(4-(4-isopropyl-5-(8-methyl-[1,2,4]triazolo[1,5-a]pyridin-6-yl)-1H-pyrazol-3-yl)phenyl)-N-methylethan-1-amine (632);
N-(1-(4-(4-isopropyl-5-(8-methyl-[1,2,4]triazolo[1,5-a]pyridin-6-yl)-1H-pyrazol-3-yl)phenyl)ethyl)oxetan-3-amine (633);
N-(1-(4-(4-isopropyl-5-(8-methyl-[1,2,4]triazolo[1,5-a]pyridin-6-yl)-1H-pyrazol-3-yl)phenyl)ethyl)-3-methyloxetan-3-amine (634);
1-(4-(4-isopropyl-5-(8-methyl-[1,2,4]triazolo[1,5-a]pyridin-6-yl)-1H-pyrazol-3-yl)phenyl)-N,N-dimethylethan-1-amine (635);
6-(3-(4-(1-(4,4-difluoropiperidin-1-yl)ethyl)phenyl)-4-isopropyl-1H-pyrazol-5-yl)-8-methyl-[1,2,4]triazolo[1,5-a]pyridine (636);
4-(1-(4-(4-isopropyl-5-(8-methyl-[1,2,4]triazolo[1,5-a]pyridin-6-yl)-1H-pyrazol-3-yl)phenyl)ethyl)morpholine (637);
6-(1-(4-(4-isopropyl-5-(8-methyl-[1,2,4]triazolo[1,5-a]pyridin-6-yl)-1H-pyrazol-3-yl)phenyl)ethyl)-2-oxa-6-azaspiro[3.3]heptane (638);
4-(1-(4-(4-isopropyl-5-(8-methyl-[1,2,4]triazolo[1,5-a]pyridin-6-yl)-1H-pyrazol-3-yl)phenyl)ethyl)piperazin-2-one (639);
6-(1-(4-(4-isopropyl-5-(8-methyl-[1,2,4]triazolo[1,5-a]pyridin-6-yl)-1H-pyrazol-3-yl)phenyl)ethyl)-2-oxa-6-azaspiro[3.3]heptane (640-641);
4-(1-(4-(4-isopropyl-5-(8-methyl-[1,2,4]triazolo[1,5-a]pyridin-6-yl)-1H-pyrazol-3-yl)phenyl)ethyl)morpholine (642-643);
1-(4-(4-isopropyl-5-(8-methyl-[1,2,4]triazolo[1,5-a]pyridin-6-yl)-1H-pyrazol-3-yl)phenyl)-N-methylmethanamine (644);
1-(4-(4-isopropyl-5-(8-methyl-[1,2,4]triazolo[1,5-a]pyridin-6-yl)-1H-pyrazol-3-yl)phenyl)-N,N-dimethylmethanamine (645 and 652);
N-(4-(4-isopropyl-5-(8-methyl-[1,2,4]triazolo[1,5-a]pyridin-6-yl)-1H-pyrazol-3-yl)benzyl)propan-2-amine (646);
N-(4-(4-isopropyl-5-(8-methyl-[1,2,4]triazolo[1,5-a]pyridin-6-yl)-1H-pyrazol-3-yl)benzyl)cyclopropanamine (647);
(1-(4-(4-isopropyl-5-(8-methyl-[1,2,4]triazolo[1,5-a]pyridin-6-yl)-1H-pyrazol-3-yl)benzyl)azetidine-3,3-diyl)dimethanol (648);
N-(4-(4-isopropyl-5-(8-methyl-[1,2,4]triazolo[1,5-a]pyridin-6-yl)-1H-pyrazol-3-yl)benzyl)-2,2-dimethylpropan-1-amine (649);
6-(3-(4-(azetidin-1-ylmethyl)phenyl)-4-isopropyl-1H-pyrazol-5-yl)-8-methyl-[1,2,4]triazolo[1,5-a]pyridine (650);
N-(4-(4-isopropyl-5-(8-methyl-[1,2,4]triazolo[1,5-a]pyridin-6-yl)-1H-pyrazol-3-yl)benzyl)-N-methylethanamine (651);
N-((6-(4-isopropyl-5-(8-methyl-[1,2,4]triazolo[1,5-a]pyridin-6-yl)-1H-pyrazol-3-yl)pyridin-3-yl)methyl)propan-2-amine (653);
1-(6-(4-isopropyl-5-(8-methyl-[1,2,4]triazolo[1,5-a]pyridin-6-yl)-1H-pyrazol-3-yl)pyridin-3-yl)-N-methylmethanamine (654);
N-((5-(4-isopropyl-5-(8-methyl-[1,2,4]triazolo[1,5-a]pyridin-6-yl)-1H-pyrazol-3-yl)pyridin-2-yl)methyl)propan-2-amine (655);
1-(5-(4-isopropyl-5-(8-methyl-[1,2,4]triazolo[1,5-a]pyridin-6-yl)-1H-pyrazol-3-yl)pyridin-2-yl)-N,N-dimethylmethanamine (656);
1-(5-(4-isopropyl-5-(8-methyl-[1,2,4]triazolo[1,5-a]pyridin-6-yl)-1H-pyrazol-3-yl)pyridin-2-yl)-N-methylmethanamine (657);
1-(6-(4-isopropyl-5-(8-methyl-[1,2,4]triazolo[1,5-a]pyridin-6-yl)-1H-pyrazol-3-yl)pyridin-3-yl)-N,N-dimethylethan-1-amine (658-659);
1-(5-(4-isopropyl-5-(8-methyl-[1,2,4]triazolo[1,5-a]pyridin-6-yl)-1H-pyrazol-3-yl)thiazol-2-yl)-N-methylethan-1-amine (660-662);
1-(2-(4-isopropyl-5-(8-methyl-[1,2,4]triazolo[1,5-a]pyridin-6-yl)-1H-pyrazol-3-yl)thiazol-5-yl)-N-methylethan-1-amine (663-664);
1-(2-(4-isopropyl-5-(8-methyl-[1,2,4]triazolo[1,5-a]pyridin-6-yl)-1H-pyrazol-3-yl)thiazol-5-yl)-N,N-dimethylethan-1-amine (665 and 670);
N-ethyl-1-(2-(4-isopropyl-5-(8-methyl-[1,2,4]triazolo[1,5-a]pyridin-6-yl)-1H-pyrazol-3-yl)thiazol-5-yl)-N-methylethan-1-amine (666 and 669);
N-(1-(2-(4-isopropyl-5-(8-methyl-[1,2,4]triazolo[1,5-a]pyridin-6-yl)-1H-pyrazol-3-yl)thiazol-5-yl)ethyl)-N-methylpropan-2-amine (677-678);
N-(1-(2-(4-isopropyl-5-(8-methyl-[1,2,4]triazolo[1,5-a]pyridin-6-yl)-1H-pyrazol-3-yl)thiazol-5-yl)ethyl)-N-methyltetrahydro-2H-pyran-4-amine (671);
2-(dimethylamino)-N-(1-(6-(4-isopropyl-5-(8-methyl-[1,2,4]triazolo[1,5-a]pyridin-6-yl)-1H-pyrazol-3-yl)pyridin-3-yl)ethyl)-N-methylacetamide (672-673)
2-(dimethylamino)-N-(1-(2-(4-isopropyl-5-(8-methyl-[1,2,4]triazolo[1,5-a]pyridin-6-yl)-1H-pyrazol-3-yl)thiazol-5-yl)ethyl)-N-methylacetamide (674-675);
2-((1-(2-(4-isopropyl-5-(8-methyl-[1,2,4]triazolo[1,5-a]pyridin-6-yl)-1H-pyrazol-3-yl)thiazol-5-yl)ethyl)(methyl)amino)-N,N-dimethylacetamide (676);
2-((1-(2-(4-isopropyl-5-(8-methyl-[1,2,4]triazolo[1,5-a]pyridin-6-yl)-1H-pyrazol-3-yl)thiazol-5-yl)ethyl)(methyl)amino)-N,N-dimethylacetamide (677-678);
6-(4-isopropyl-5-(8-methyl-[1,2,4]triazolo[1,5-a]pyridin-6-yl)-1H-pyrazol-3-yl)-N,N-dimethyl-1,2,3,4-tetrahydronaphthalen-2-amine (679 and 682);
6-(4-isopropyl-5-(8-methoxy-[1,2,4]triazolo[1,5-a]pyridin-6-yl)-1H-pyrazol-3-yl)-N-methyl-1,2,3,4-tetrahydronaphthalen-2-amine (680-681);
6-(4-isopropyl-5-(8-methyl-[1,2,4]triazolo[1,5-a]pyridin-6-yl)-1H-pyrazol-3-yl)-N,N-dimethyl-1,2,3,4-tetrahydronaphthalen-2-amine (682);
N-isopropyl-6-(4-isopropyl-5-(8-methyl-[1,2,4]triazolo[1,5-a]pyridin-6-yl)-1H-pyrazol-3-yl)-N-methyl-1,2,3,4-tetrahydronaphthalen-2-amine (683);
N-(6-(4-isopropyl-5-(8-methyl-[1,2,4]triazolo[1,5-a]pyridin-6-yl)-1H-pyrazol-3-yl)-1,2,3,4-tetrahydronaphthalen-2-yl)-N-methyltetrahydro-2H-pyran-4-amine (684-685);
N-(6-(4-isopropyl-5-(8-methyl-[1,2,4]triazolo[1,5-a]pyridin-6-yl)-1H-pyrazol-3-yl)-1,2,3,4-tetrahydronaphthalen-2-yl)-N-methyloxetan-3-amine (686-687);
6-(4-isopropyl-5-(8-methyl-[1,2,4]triazolo[1,5-a]pyridin-6-yl)-1H-pyrazol-3-yl)-N-methyl-N-((3-methyloxetan-3-yl)methyl)-1,2,3,4-tetrahydronaphthalen-2-amine (688);
6-(4-isopropyl-5-(8-methoxy-[1,2,4]triazolo[1,5-a]pyridin-6-yl)-1H-pyrazol-3-yl)-N,N-dimethyl-1,2,3,4-tetrahydronaphthalen-2-amine (689);
2-(dimethylamino)-N-(6-(4-isopropyl-5-(8-methyl-[1,2,4]triazolo[1,5-a]pyridin-6-yl)-1H-pyrazol-3-yl)-1,2,3,4-tetrahydronaphthalen-2-yl)-N-methylacetamide (690-691);
2-((6-(4-isopropyl-5-(8-methyl-[1,2,4]triazolo[1,5-a]pyridin-6-yl)-1H-pyrazol-3-yl)-1,2,3,4-tetrahydronaphthalen-2-yl)(methyl)amino)-N,N-dimethylacetamide (692-693);
2-(4-isopropyl-5-(8-methoxy-[1,2,4]triazolo[1,5-a]pyridin-6-yl)-1H-pyrazol-3-yl)-N,N-dimethyl-4,5,6,7-tetrahydrobenzo[d]thiazol-6-amine (694 and 703);
2-(4-isopropyl-5-(8-methyl-[1,2,4]triazolo[1,5-a]pyridin-6-yl)-1H-pyrazol-3-yl)-4,5,6,7-tetrahydrobenzo[d]thiazol-6-amine (695-697);
N-isopropyl-2-(4-isopropyl-5-(8-methoxy-[1,2,4]triazolo[1,5-a]pyridin-6-yl)-1H-pyrazol-3-yl)-4,5,6,7-tetrahydrobenzo[d]thiazol-6-amine (698 and 704);
2-(4-isopropyl-5-(8-methoxy-[1,2,4]triazolo[1,5-a]pyridin-6-yl)-1H-pyrazol-3-yl)-N-(4,4,4-trifluorobutyl)-4,5,6,7-tetrahydrobenzo[d]thiazol-6-amine (699);
2-(4-isopropyl-5-(8-methoxy-[1,2,4]triazolo[1,5-a]pyridin-6-yl)-1H-pyrazol-3-yl)-N,N-bis(4,4,4-trifluorobutyl)-4,5,6,7-tetrahydrobenzo[d]thiazol-6-amine (700);
2-(4-isopropyl-5-(8-methoxy-[1,2,4]triazolo[1,5-a]pyridin-6-yl)-1H-pyrazol-3-yl)-N-(tetrahydro-2H-pyran-4-yl)-4,5,6,7-tetrahydrobenzo[d]thiazol-6-amine (701 and 706);
2-(4-isopropyl-5-(8-methoxy-[1,2,4]triazolo[1,5-a]pyridin-6-yl)-1H-pyrazol-3-yl)-N-(3,3,3-trifluoropropyl)-4,5,6,7-tetrahydrobenzo[d]thiazol-6-amine (702 and 705);
2-(4-isopropyl-5-(8-methyl-[1,2,4]triazolo[1,5-a]pyridin-6-yl)-1H-pyrazol-3-yl)-N,N-dimethyl-4,5,6,7-tetrahydrobenzo[d]thiazol-6-amine (707 and 712);
N-isopropyl-2-(4-isopropyl-5-(8-methyl-[1,2,4]triazolo[1,5-a]pyridin-6-yl)-1H-pyrazol-3-yl)-4,5,6,7-tetrahydrobenzo[d]thiazol-6-amine (708 and 713);
2-(4-isopropyl-5-(8-methyl-[1,2,4]triazolo[1,5-a]pyridin-6-yl)-1H-pyrazol-3-yl)-N-(oxetan-3-yl)-4,5,6,7-tetrahydrobenzo[d]thiazol-6-amine (709 and 715);
2-(4-isopropyl-5-(8-methyl-[1,2,4]triazolo[1,5-a]pyridin-6-yl)-1H-pyrazol-3-yl)-N-(tetrahydro-2H-pyran-4-yl)-4,5,6,7-tetrahydrobenzo[d]thiazol-6-amine (710 and 717);
N-(3-ethoxycyclobutyl)-2-(4-isopropyl-5-(8-methyl-[1,2,4]triazolo[1,5-a]pyridin-6-yl)-1H-pyrazol-3-yl)-4,5,6,7-tetrahydrobenzo[d]thiazol-6-amine (711-716);
2-(4-isopropyl-5-(8-methyl-[1,2,4]triazolo[1,5-a]pyridin-6-yl)-1H-pyrazol-3-yl)-N-(3,3,3-trifluoropropyl)-4,5,6,7-tetrahydrobenzo[d]thiazol-6-amine (714);
2-(4-isopropyl-5-(8-methyl-[1,2,4]triazolo[1,5-a]pyridin-6-yl)-1H-pyrazol-3-yl)-N-(oxetan-3-yl)-4,5,6,7-tetrahydrobenzo[d]thiazol-6-amine (715);
2-(dimethylamino)-N-(2-(4-isopropyl-5-(8-methoxy-[1,2,4]triazolo[1,5-a]pyridin-6-yl)-1H-pyrazol-3-yl)-4,5,6,7-tetrahydrobenzo[d]thiazol-6-yl)acetamide (718-719);
2-(dimethylamino)-N-(2-(4-isopropyl-5-(8-methyl-[1,2,4]triazolo[1,5-a]pyridin-6-yl)-1H-pyrazol-3-yl)-4,5,6,7-tetrahydrobenzo[d]thiazol-6-yl)acetamide (720-721);
N-ethyl-2-(4-isopropyl-5-(8-methoxy-[1,2,4]triazolo[1,5-a]pyridin-6-yl)-1H-pyrazol-3-yl)-N-methyl-4,5,6,7-tetrahydrobenzo[d]thiazol-6-amine (722 and 728);
2-(4-isopropyl-5-(8-methoxy-[1,2,4]triazolo[1,5-a]pyridin-6-yl)-1H-pyrazol-3-yl)-N-methyl-N-propyl-4,5,6,7-tetrahydrobenzo[d]thiazol-6-amine (723 and 729);
N-isopropyl-2-(4-isopropyl-5-(8-methoxy-[1,2,4]triazolo[1,5-a]pyridin-6-yl)-1H-pyrazol-3-yl)-N-methyl-4,5,6,7-tetrahydrobenzo[d]thiazol-6-amine (724 and 730);
2-(4-isopropyl-5-(8-methoxy-[1,2,4]triazolo[1,5-a]pyridin-6-yl)-1H-pyrazol-3-yl)-N-methyl-N-(oxetan-3-yl)-4,5,6,7-tetrahydrobenzo[d]thiazol-6-amine (725 and 731);
2-(4-isopropyl-5-(8-methoxy-[1,2,4]triazolo[1,5-a]pyridin-6-yl)-1H-pyrazol-3-yl)-N-methyl-N-(3,3,3-trifluoropropyl)-4,5,6,7-tetrahydrobenzo[d]thiazol-6-amine (726 and 732);
2-(4-isopropyl-5-(8-methoxy-[1,2,4]triazolo[1,5-a]pyridin-6-yl)-1H-pyrazol-3-yl)-N-methyl-N-(tetrahydro-2H-pyran-4-yl)-4,5,6,7-tetrahydrobenzo[d]thiazol-6-amine (727-733);
2-(dimethylamino)-N-(2-(4-isopropyl-5-(8-methoxy-[1,2,4] triazolo[1,5-a]pyridin-6-yl)-1H-pyrazol-3-yl)-4,5,6,7-tetrahydrobenzo[d]thiazol-6-yl)-N-methylacetamide (734);
2-(dimethylamino)-N-(2-(4-isopropyl-5-(8-methoxy-[1,2,4]triazolo[1,5-a]pyridin-6-yl)-1H-pyrazol-3-yl)-4,5,6,7-tetrahydrobenzo[d]thiazol-6-yl)-N-methylacetamide (735);
2-((2-(4-isopropyl-5-(8-methoxy-[1,2,4]triazolo[1,5-a]pyridin-6-yl)-1H-pyrazol-3-yl)-4,5,6,7-tetrahydrobenzo[d]thiazol-6-yl)(methyl)amino)-N,N-dimethylacetamide (736);
2-((2-(4-isopropyl-5-(8-methoxy-[1,2,4]triazolo[1,5-a]pyridin-6-yl)-1H-pyrazol-3-yl)-4,5,6,7-tetrahydrobenzo[d]thiazol-6-yl)(methyl)amino)-N,N-dimethylacetamide (737);
2-(4-isopropyl-5-(8-methoxy-[1,2,4]triazolo[1,5-a]pyridin-6-yl)-1H-pyrazol-3-yl)-N-(2-methoxyethyl)-N-methyl-4,5,6,7-tetrahydrobenzo[d]thiazol-6-amine (738-739);
2-(4-isopropyl-5-(8-methoxy-[1,2,4]triazolo[1,5-a]pyridin-6-yl)-1H-pyrazol-3-yl)-N-methyl-N-(2-(methylsulfonyl)ethyl)-4,5,6,7-tetrahydrobenzo[d]thiazol-6-amine (740-741);
2-(4-isopropyl-5-(8-methoxy-[1,2,4]triazolo[1,5-a]pyridin-6-yl)-1H-pyrazol-3-yl)-5-(piperidin-4-yl)thiazole (742);
2-(4-isopropyl-5-(8-methylimidazo[1,2-a]pyridin-6-yl)-1H-pyrazol-3-yl)-5-(piperidin-4-yl)thiazole (743);
2-(4-isopropyl-5-(8-methoxyimidazo[1,2-a]pyridin-6-yl)-1H-pyrazol-3-yl)-5-(piperidin-4-yl)thiazole (744);
2-(5-(3,4-dimethoxyphenyl)-4-isopropyl-1H-pyrazol-3-yl)-5-(piperidin-4-yl)thiazole (745);
2-(5-(7,8-dimethyl-[1,2,4]triazolo[1,5-a]pyridin-6-yl)-4-isopropyl-1H-pyrazol-3-yl)-5-(piperidin-4-yl)thiazole (746);
2-(5-(2,6-dimethylpyridin-4-yl)-4-isopropyl-1H-pyrazol-3-yl)-5-(piperidin-4-yl)thiazole (747);
5-(4-isopropyl-3-(5-(piperidin-4-yl)thiazol-2-yl)-1H-pyrazol-5-yl)-1,3,4-trimethylpyridin-2(1H)-one (748);
2-(4-isopropyl-5-(8-methoxy-[1,2,4]triazolo[1,5-a]pyridin-6-yl)-1H-pyrazol-3-yl)-4-methyl-5-(piperidin-4-yl)thiazole (749);
2-(5-(7,8-dimethyl-[1,2,4]triazolo[1,5-a]pyridin-6-yl)-4-isopropyl-1H-pyrazol-3-yl)-4-methyl-5-(piperidin-4-yl)thiazole (750);
2-(4-isopropyl-5-(8-methoxy-[1,2,4]triazolo[1,5-a]pyridin-6-yl)-1H-pyrazol-3-yl)-5-(piperidin-4-yl)-4-(trifluoromethyl)thiazole (751);
2-(4-isopropyl-5-(8-methoxy-[1,2,4]triazolo[1,5-a]pyridin-6-yl)-1H-pyrazol-3-yl)-5-(1-((tetrahydro-2H-pyran-4-yl)methyl)piperidin-4-yl)thiazole (752);
2-(4-isopropyl-5-(8-methoxy-[1,2,4]triazolo[1,5-a]pyridin-6-yl)-1H-pyrazol-3-yl)-5-(1-methylpiperidin-4-yl)thiazole (753);
2-(4-isopropyl-5-(8-methoxy-[1,2,4]triazolo[1,5-a]pyridin-6-yl)-1H-pyrazol-3-yl)-5-(1-(3,3,3-trifluoropropyl)piperidin-4-yl)thiazole (754);
5-(1-ethylpiperidin-4-yl)-2-(4-isopropyl-5-(8-methoxy-[1,2,4]triazolo[1,5-a]pyridin-6-yl)-1H-pyrazol-3-yl)thiazole (755);
2-(4-isopropyl-5-(8-methoxy-[1,2,4]triazolo[1,5-a]pyridin-6-yl)-1H-pyrazol-3-yl)-5-(1-propylpiperidin-4-yl)thiazole (756);
2-(4-isopropyl-5-(8-methoxy-[1,2,4]triazolo[1,5-a]pyridin-6-yl)-1H-pyrazol-3-yl)-5-(1-isopropylpiperidin-4-yl)thiazole (757);
2-(4-isopropyl-5-(8-methoxy-[1,2,4]triazolo[1,5-a]pyridin-6-yl)-1H-pyrazol-3-yl)-5-(1-(tetrahydro-2H-pyran-4-yl)piperidin-4-yl)thiazole (758);
5-(1-(3-ethoxycyclobutyl)piperidin-4-yl)-2-(4-isopropyl-5-(8-methoxy-[1,2,4]triazolo[1,5-a]pyridin-6-yl)-1H-pyrazol-3-yl)thiazole (759);
2-(4-isopropyl-5-(8-methoxy-[1,2,4]triazolo[1,5-a]pyridin-6-yl)-1H-pyrazol-3-yl)-5-(1-(oxetan-3-yl)piperidin-4-yl)thiazole (760);
2-(4-isopropyl-5-(8-methoxy-[1,2,4]triazolo[1,5-a]pyridin-6-yl)-1H-pyrazol-3-yl)-5-(1-neopentylpiperidin-4-yl)thiazole (761);
5-(1-isopentylpiperidin-4-yl)-2-(4-isopropyl-5-(8-methoxy-[1,2,4]triazolo[1,5-a]pyridin-6-yl)-1H-pyrazol-3-yl)thiazole (762);
5-(1-((3-ethyloxetan-3-yl)methyl)piperidin-4-yl)-2-(4-isopropyl-5-(8-methoxy-[1,2,4]triazolo[1,5-a]pyridin-6-yl)-1H-pyrazol-3-yl)thiazole (763);
3-(4-(2-(4-isopropyl-5-(8-methoxy-[1,2,4]triazolo[1,5-a]pyridin-6-yl)-1H-pyrazol-3-yl)thiazol-5-yl)piperidin-1-yl)tetrahydrothiophene 1,1-dioxide (764);
2-(4-isopropyl-5-(8-methoxy-[1,2,4]triazolo[1,5-a]pyridin-6-yl)-1H-pyrazol-3-yl)-5-(1-(pentan-3-yl)piperidin-4-yl)thiazole (765(;
2-(4-isopropyl-5-(8-methoxy-[1,2,4]triazolo[1,5-a]pyridin-6-yl)-1H-pyrazol-3-yl)-5-(1-(tetrahydrofuran-3-yl)piperidin-4-yl)thiazole (766);
2-(4-isopropyl-5-(8-methoxy-[1,2,4]triazolo[1,5-a]pyridin-6-yl)-1H-pyrazol-3-yl)-5-(1-(tetrahydrofuran-3-yl)piperidin-4-yl)thiazole (767);
2-(4-isopropyl-5-(8-methoxy-[1,2,4]triazolo[1,5-a]pyridin-6-yl)-1H-pyrazol-3-yl)-5-(1-((tetrahydrofuran-3-yl)methyl)piperidin-4-yl)thiazole (786);
2-(4-isopropyl-5-(8-methoxy-[1,2,4]triazolo[1,5-a]pyridin-6-yl)-1H-pyrazol-3-yl)-5-(1-((tetrahydrofuran-3-yl)methyl)piperidin-4-yl)thiazole (769);
2-(4-isopropyl-5-(8-methoxy-[1,2,4]triazolo[1,5-a]pyridin-6-yl)-1H-pyrazol-3-yl)-5-(1-(tetrahydro-2H-pyran-3-yl)piperidin-4-yl)thiazole (770);
2-(4-isopropyl-5-(8-methoxy-[1,2,4]triazolo[1,5-a]pyridin-6-yl)-1H-pyrazol-3-yl)-5-(1-(tetrahydro-2H-pyran-3-yl)piperidin-4-yl)thiazole (771);
2-(4-isopropyl-5-(8-methyl-[1,2,4]triazolo[1,5-a]pyridin-6-yl)-1H-pyrazol-3-yl)-5-(1-methylpiperidin-4-yl)thiazole (772);
5-(1-ethylpiperidin-4-yl)-2-(4-isopropyl-5-(8-methyl-[1,2,4]triazolo[1,5-a]pyridin-6-yl)-1H-pyrazol-3-yl)thiazole (773);
2-(4-isopropyl-5-(8-methyl-[1,2,4]triazolo[1,5-a]pyridin-6-yl)-1H-pyrazol-3-yl)-5-(1-isopropylpiperidin-4-yl)thiazole (774);
2-(4-isopropyl-5-(8-methyl-[1,2,4]triazolo[1,5-a]pyridin-6-yl)-1H-pyrazol-3-yl)-5-(1-propylpiperidin-4-yl)thiazole (775);
5-(1-((3-ethyloxetan-3-yl)methyl)piperidin-4-yl)-2-(4-isopropyl-5-(8-methyl-[1,2,4]triazolo[1,5-a]pyridin-6-yl)-1H-pyrazol-3-yl)thiazole (776);
2-(4-isopropyl-5-(8-methyl-[1,2,4]triazolo[1,5-a]pyridin-6-yl)-1H-pyrazol-3-yl)-5-(1-(tetrahydro-2H-pyran-4-yl)piperidin-4-yl)thiazole (777);
2-(4-isopropyl-5-(8-methyl-[1,2,4]triazolo[1,5-a]pyridin-6-yl)-1H-pyrazol-3-yl)-5-(1-(oxetan-3-yl)piperidin-4-yl)thiazole (778);
2-(4-isopropyl-5-(8-methoxy-[1,2,4]triazolo[1,5-a]pyridin-6-yl)-1H-pyrazol-3-yl)-4-methyl-5-(1-methylpiperidin-4-yl)thiazole (779);
5-(1-ethylpiperidin-4-yl)-2-(4-isopropyl-5-(8-methoxy-[1,2,4]triazolo[1,5-a]pyridin-6-yl)-1H-pyrazol-3-yl)-4-methylthiazole (780);
2-(4-isopropyl-5-(8-methoxy-[1,2,4]triazolo[1,5-a]pyridin-6-yl)-1H-pyrazol-3-yl)-4-methyl-5-(1-(tetrahydro-2H-pyran-4-yl)piperidin-4-yl)thiazole (781);
5-(1-(3-ethoxycyclobutyl)piperidin-4-yl)-2-(4-isopropyl-5-(8-methoxy-[1,2,4]triazolo[1,5-a]pyridin-6-yl)-1H-pyrazol-3-yl)-4-methylthiazole (782);
2-(4-isopropyl-5-(8-methoxy-[1,2,4]triazolo[1,5-a]pyridin-6-yl)-1H-pyrazol-3-yl)-4-methyl-5-(1-propylpiperidin-4-yl)thiazole (783);
2-(4-isopropyl-5-(8-methoxy-[1,2,4]triazolo[1,5-a]pyridin-6-yl)-1H-pyrazol-3-yl)-5-(1-isopropylpiperidin-4-yl)-4-methylthiazole (784);
2-(4-isopropyl-5-(8-methoxy-[1,2,4]triazolo[1,5-a]pyridin-6-yl)-1H-pyrazol-3-yl)-4-methyl-5-(1-((tetrahydro-2H-pyran-4-yl)methyl)piperidin-4-yl)thiazole (785);
5-(1-(3-ethoxycyclobutyl)piperidin-4-yl)-2-(4-isopropyl-5-(8-methoxy-[1,2,4]triazolo[1,5-a]pyridin-6-yl)-1H-pyrazol-3-yl)-4-methylthiazole (786);
5-(1-(cyclopropylmethyl)piperidin-4-yl)-2-(4-isopropyl-5-(8-methoxy-[1,2,4]triazolo[1,5-a]pyridin-6-yl)-1H-pyrazol-3-yl)-4-methylthiazole (787);
5-(1-(cyclopropylmethyl)piperidin-4-yl)-2-(4-isopropyl-5-(8-methoxy-[1,2,4]triazolo[1,5-a]pyridin-6-yl)-1H-pyrazol-3-yl)thiazole (788);
5-(1-isobutylpiperidin-4-yl)-2-(4-isopropyl-5-(8-methoxy-[1,2,4]triazolo[1,5-a]pyridin-6-yl)-1H-pyrazol-3-yl)thiazole (789);
2-(4-isopropyl-5-(8-methoxy-[1,2,4]triazolo[1,5-a]pyridin-6-yl)-1H-pyrazol-3-yl)-5-(1-((1-methyl-1H-1,2,4-triazol-3-yl)methyl)piperidin-4-yl)thiazole (790);
5-(1-(cyclobutylmethyl)piperidin-4-yl)-2-(4-isopropyl-5-(8-methoxy-[1,2,4]triazolo[1,5-a]pyridin-6-yl)-1H-pyrazol-3-yl)thiazole (791);
5-(1-(cyclobutylmethyl)piperidin-4-yl)-2-(4-isopropyl-5-(8-methoxy-[1,2,4]triazolo[1,5-a]pyridin-6-yl)-1H-pyrazol-3-yl)-4-methylthiazole (792);
5-(1-isobutylpiperidin-4-yl)-2-(4-isopropyl-5-(8-methoxy-[1,2,4]triazolo[1,5-a]pyridin-6-yl)-1H-pyrazol-3-yl)-4-methylthiazole (793);
2-(5-(3,4-dimethoxyphenyl)-4-isopropyl-1H-pyrazol-3-yl)-5-(1-methylpiperidin-4-yl)thiazole (794);
2-(5-(3,4-dimethoxyphenyl)-4-isopropyl-1H-pyrazol-3-yl)-5-(1-ethylpiperidin-4-yl)thiazole (795);
2-(5-(3,4-dimethoxyphenyl)-4-isopropyl-1H-pyrazol-3-yl)-5-(1-propylpiperidin-4-yl)thiazole (796);
5-(1-(cyclopropylmethyl)piperidin-4-yl)-2-(5-(3,4-dimethoxyphenyl)-4-isopropyl-1H-pyrazol-3-yl)thiazole (797);
2-(5-(3,4-dimethoxyphenyl)-4-isopropyl-1H-pyrazol-3-yl)-5-(1-isopropylpiperidin-4-yl)thiazole (798);
2-(5-(3,4-dimethoxyphenyl)-4-isopropyl-1H-pyrazol-3-yl)-5-(1-(tetrahydro-2H-pyran-4-yl)piperidin-4-yl)thiazole (799);
2-(5-(3,4-dimethoxyphenyl)-4-isopropyl-1H-pyrazol-3-yl)-5-(1-((3-ethyloxetan-3-yl)methyl)piperidin-4-yl)thiazole (800);
2-(5-(7,8-dimethyl-[1,2,4]triazolo[1,5-a]pyridin-6-yl)-4-isopropyl-1H-pyrazol-3-yl)-5-(1-methylpiperidin-4-yl)thiazole (801);
2-(5-(7,8-dimethyl-[1,2,4]triazolo[1,5-a]pyridin-6-yl)-4-isopropyl-1H-pyrazol-3-yl)-5-(1-ethylpiperidin-4-yl)thiazole (802);
2-(5-(7,8-dimethyl-[1,2,4]triazolo[1,5-a]pyridin-6-yl)-4-isopropyl-1H-pyrazol-3-yl)-5-(1-propylpiperidin-4-yl)thiazole (803);
2-(5-(7,8-dimethyl-[1,2,4]triazolo[1,5-a]pyridin-6-yl)-4-isopropyl-1H-pyrazol-3-yl)-5-(1-isopropylpiperidin-4-yl)thiazole (804);
5-(1-(cyclopropylmethyl)piperidin-4-yl)-2-(5-(7,8-dimethyl-[1,2,4]triazolo[1,5-a]pyridin-6-yl)-4-isopropyl-1H-pyrazol-3-yl)thiazole (805);
2-(5-(7,8-dimethyl-[1,2,4]triazolo[1,5-a]pyridin-6-yl)-4-isopropyl-1H-pyrazol-3-yl)-5-(1-(tetrahydro-2H-pyran-4-yl)piperidin-4-yl)thiazole (806);
2-(5-(7,8-dimethyl-[1,2,4]triazolo[1,5-a]pyridin-6-yl)-4-isopropyl-1H-pyrazol-3-yl)-5-(1-(3-ethoxycyclobutyl)piperidin-4-yl)thiazole (807);
2-(5-(2,6-dimethylpyridin-4-yl)-4-isopropyl-1H-pyrazol-3-yl)-5-(1-methylpiperidin-4-yl)thiazole (808);
2-(5-(2,6-dimethylpyridin-4-yl)-4-isopropyl-1H-pyrazol-3-yl)-5-(1-propylpiperidin-4-yl)thiazole (809);
2-(5-(2,6-dimethylpyridin-4-yl)-4-isopropyl-1H-pyrazol-3-yl)-5-(1-isopropylpiperidin-4-yl)thiazole (810);
2-(5-(2,6-dimethylpyridin-4-yl)-4-isopropyl-1H-pyrazol-3-yl)-5-(1-(tetrahydro-2H-pyran-4-yl)piperidin-4-yl)thiazole (811);
2-(5-(7,8-dimethyl-[1,2,4]triazolo[1,5-a]pyridin-6-yl)-4-isopropyl-1H-pyrazol-3-yl)-4-methyl-5-(1-methylpiperidin-4-yl)thiazole (812);
2-(5-(7,8-dimethyl-[1,2,4]triazolo[1,5-a]pyridin-6-yl)-4-isopropyl-1H-pyrazol-3-yl)-5-(1-isopropylpiperidin-4-yl)-4-methylthiazole (813);
5-(1-(cyclopropylmethyl)piperidin-4-yl)-2-(5-(7,8-dimethyl-[1,2,4]triazolo[1,5-a]pyridin-6-yl)-4-isopropyl-1H-pyrazol-3-yl)-4-methylthiazole (814);
5-(3-(5-(1-ethylpiperidin-4-yl)thiazol-2-yl)-4-isopropyl-1H-pyrazol-5-yl)-1,3,4-trimethylpyridin-2(1H)-one (815);
5-(4-isopropyl-3-(5-(1-methylpiperidin-4-yl)thiazol-2-yl)-1H-pyrazol-5-yl)-1,3,4-trimethylpyridin-2(1H)-one (816);
2-(5-(7,8-dimethyl-[1,2,4]triazolo[1,5-a]pyridin-6-yl)-4-isopropyl-1H-pyrazol-3-yl)-5-(1-((tetrahydro-2H-pyran-4-yl)methyl)piperidin-4-yl)thiazole (817);
5-(4-isopropyl-3-(5-(1-propylpiperidin-4-yl)thiazol-2-yl)-1H-pyrazol-5-yl)-1,3,4-trimethylpyridin-2(1H)-one (818);
5-(3-(5-(1-(cyclopropylmethyl)piperidin-4-yl)thiazol-2-yl)-4-isopropyl-1H-pyrazol-5-yl)-1,3,4-trimethylpyridin-2(1H)-one (819);
5-(1-(2-ethylbutyl)piperidin-4-yl)-2-(4-isopropyl-5-(8-methoxyimidazo[1,2-a]pyridin-6-yl)-1H-pyrazol-3-yl)thiazole (820);
5-(1-isobutylpiperidin-4-yl)-2-(4-isopropyl-5-(8-methoxyimidazo[1,2-a]pyridin-6-yl)-1H-pyrazol-3-yl)thiazole (821);
2-(4-isopropyl-5-(8-methoxyimidazo[1,2-a]pyridin-6-yl)-1H-pyrazol-3-yl)-5-(1-propylpiperidin-4-yl)thiazole (822);
2-(4-isopropyl-5-(8-methoxyimidazo[1,2-a]pyridin-6-yl)-1H-pyrazol-3-yl)-5-(1-(tetrahydro-2H-pyran-4-yl)piperidin-4-yl)thiazole (823);
2-(4-isopropyl-5-(8-methoxyimidazo[1,2-a]pyridin-6-yl)-1H-pyrazol-3-yl)-5-(1-(3,3,3-trifluoropropyl)piperidin-4-yl)thiazole (824);
5-(1-cyclobutylpiperidin-4-yl)-2-(4-isopropyl-5-(8-methoxyimidazo[1,2-a]pyridin-6-yl)-1H-pyrazol-3-yl)thiazole (825);
2-(4-isopropyl-5-(8-methoxyimidazo[1,2-a]pyridin-6-yl)-1H-pyrazol-3-yl)-5-(1-(oxetan-3-yl)piperidin-4-yl)thiazole (826);
2-(4-isopropyl-5-(8-methoxyimidazo[1,2-a]pyridin-6-yl)-1H-pyrazol-3-yl)-5-(1-(pentan-3-yl)piperidin-4-yl)thiazole (827);
2-(4-isopropyl-5-(8-methoxyimidazo[1,2-a]pyridin-6-yl)-1H-pyrazol-3-yl)-5-(1-isopropylpiperidin-4-yl)thiazole (828);
2-(4-isopropyl-5-(8-methoxyimidazo[1,2-a]pyridin-6-yl)-1H-pyrazol-3-yl)-5-(1-methylpiperidin-4-yl)thiazole (829);
2-(4-isopropyl-5-(8-methoxyimidazo[1,2-a]pyridin-6-yl)-1H-pyrazol-3-yl)-5-(1-neopentylpiperidin-4-yl)thiazole (830);
5-(1-ethylpiperidin-4-yl)-2-(4-isopropyl-5-(8-methoxyimidazo[1,2-a]pyridin-6-yl)-1H-pyrazol-3-yl)thiazole (831);
5-(1-(cyclopropylmethyl)piperidin-4-yl)-2-(4-isopropyl-5-(8-methoxyimidazo[1,2-a]pyridin-6-yl)-1H-pyrazol-3-yl)thiazole (832);
2-(5-(7,8-dimethyl-[1,2,4]triazolo[1,5-a]pyridin-6-yl)-4-isopropyl-1H-pyrazol-3-yl)-4-methyl-5-(1-propylpiperidin-4-yl)thiazole (833);
2-(5-(7,8-dimethyl-[1,2,4]triazolo[1,5-a]pyridin-6-yl)-4-isopropyl-1H-pyrazol-3-yl)-4-methyl-5-(1-((tetrahydro-2H-pyran-4-yl)methyl)piperidin-4-yl)thiazole (834);
2-(5-(7,8-dimethyl-[1,2,4]triazolo[1,5-a]pyridin-6-yl)-4-isopropyl-1H-pyrazol-3-yl)-4-methyl-5-(1-(oxetan-3-yl)piperidin-4-yl)thiazole (835);
2-(5-(7,8-dimethyl-[1,2,4]triazolo[1,5-a]pyridin-6-yl)-4-isopropyl-1H-pyrazol-3-yl)-4-methyl-5-(1-(tetrahydro-2H-pyran-4-yl)piperidin-4-yl)thiazole (836);
2-(4-isopropyl-5-(8-methoxy-[1,2,4]triazolo[1,5-a]pyridin-6-yl)-1H-pyrazol-3-yl)-5-(1-(oxetan-3-yl)piperidin-4-yl)-4-(trifluoromethyl)thiazole (837);
5-(1-(cyclopropylmethyl)piperidin-4-yl)-2-(4-isopropyl-5-(8-methoxy-[1,2,4]triazolo[1,5-a]pyridin-6-yl)-1H-pyrazol-3-yl)-4-(trifluoromethyl)thiazole (838);
2-(4-isopropyl-5-(8-methoxy-[1,2,4]triazolo[1,5-a]pyridin-6-yl)-1H-pyrazol-3-yl)-5-(1-isopropylpiperidin-4-yl)-4-(trifluoromethyl)thiazole (839);
2-(4-isopropyl-5-(8-methoxy-[1,2,4]triazolo[1,5-a]pyridin-6-yl)-1H-pyrazol-3-yl)-5-(1-((tetrahydro-2H-pyran-4-yl)methyl)piperidin-4-yl)-4-(trifluoromethyl)thiazole (840);
2-(4-isopropyl-5-(8-methoxy-[1,2,4]triazolo[1,5-a]pyridin-6-yl)-1H-pyrazol-3-yl)-5-(1-methylpiperidin-4-yl)-4-(trifluoromethyl)thiazole (841);
5-(1-ethylpiperidin-4-yl)-2-(4-isopropyl-5-(8-methoxy-[1,2,4]triazolo[1,5-a]pyridin-6-yl)-1H-pyrazol-3-yl)-4-(trifluoromethyl)thiazole (842);
2-(4-isopropyl-5-(8-methoxy-[1,2,4]triazolo[1,5-a]pyridin-6-yl)-1H-pyrazol-3-yl)-5-(1-propylpiperidin-4-yl)-4-(trifluoromethyl)thiazole (843);
1-(4-(2-(4-isopropyl-5-(8-methoxy-[1,2,4]triazolo[1,5-a]pyridin-6-yl)-1H-pyrazol-3-yl)thiazol-5-yl)piperidin-1-yl)-2-morpholinoethan-1-one (844);
2-(dimethylamino)-1-(4-(2-(4-isopropyl-5-(8-methoxy-[1,2,4]triazolo[1,5-a]pyridin-6-yl)-1H-pyrazol-3-yl)thiazol-5-yl)piperidin-1-yl)ethan-1-one (845);
2-(dimethylamino)-1-(4-(2-(4-isopropyl-5-(8-methoxy-[1,2,4]triazolo[1,5-a]pyridin-6-yl)-1H-pyrazol-3-yl)-4-methylthiazol-5-yl)piperidin-1-yl)ethan-1-one (846);
2-(diethylamino)-1-(4-(2-(4-isopropyl-5-(8-methoxy-[1,2,4]triazolo[1,5-a]pyridin-6-yl)-1H-pyrazol-3-yl)thiazol-5-yl)piperidin-1-yl)ethan-1-one (847);
2-(diethylamino)-1-(4-(2-(4-isopropyl-5-(8-methoxy-[1,2,4]triazolo[1,5-a]pyridin-6-yl)-1H-pyrazol-3-yl)-4-methylthiazol-5-yl)piperidin-1-yl)ethan-1-one (848);
2-(dimethylamino)-1-(4-(2-(4-isopropyl-5-(8-methyl-[1,2,4]triazolo[1,5-a]pyridin-6-yl)-1H-pyrazol-3-yl)thiazol-5-yl)piperidin-1-yl)ethan-1-one (849);
1-(4-(2-(5-(7,8-dimethyl-[1,2,4]triazolo[1,5-a]pyridin-6-yl)-4-isopropyl-1H-pyrazol-3-yl)-4-methylthiazol-5-yl)piperidin-1-yl)-2-(dimethylamino)ethan-1-one (850);
1-(4-(2-(5-(7,8-dimethyl-[1,2,4]triazolo[1,5-a]pyridin-6-yl)-4-isopropyl-1H-pyrazol-3-yl)thiazol-5-yl)piperidin-1-yl)-2-(dimethylamino)ethan-1-one (851);
2-(dimethylamino)-1-(4-(2-(5-(2,6-dimethylpyridin-4-yl)-4-isopropyl-1H-pyrazol-3-yl)thiazol-5-yl)piperidin-1-yl)ethan-1-one (852);
1-(4-(2-(5-(3,4-dimethoxyphenyl)-4-isopropyl-1H-pyrazol-3-yl)thiazol-5-yl)piperidin-1-yl)-2-(dimethylamino)ethan-1-one (853);
2-(dimethylamino)-1-(4-(2-(4-isopropyl-5-(8-methoxy-[1,2,4]triazolo[1,5-a]pyridin-6-yl)-1H-pyrazol-3-yl)-4-(trifluoromethyl)thiazol-5-yl)piperidin-1-yl)ethan-1-one (854);
2-(4-isopropyl-5-(8-methoxy-[1,2,4]triazolo[1,5-a]pyridin-6-yl)-1H-pyrazol-3-yl)-5-(1-(2-(methylsulfonyl)ethyl)piperidin-4-yl)thiazole (855);
2-(4-(2-(4-isopropyl-5-(8-methoxy-[1,2,4]triazolo[1,5-a]pyridin-6-yl)-1H-pyrazol-3-yl)thiazol-5-yl)piperidin-1-yl)-N,N-dimethylacetamide (856);
2-(4-(2-(4-isopropyl-5-(8-methoxy-[1,2,4]triazolo[1,5-a]pyridin-6-yl)-1H-pyrazol-3-yl)thiazol-5-yl)piperidin-1-yl)acetamide (857);
2-(4-isopropyl-5-(8-methoxy-[1,2,4]triazolo[1,5-a]pyridin-6-yl)-1H-pyrazol-3-yl)-5-(1-(2-methoxyethyl)piperidin-4-yl)thiazole (858);
2-(4-(2-(4-isopropyl-5-(8-methoxy-[1,2,4]triazolo[1,5-a]pyridin-6-yl)-1H-pyrazol-3-yl)thiazol-5-yl)piperidin-1-yl)acetonitrile (859);
1-(4-(2-(4-isopropyl-5-(8-methoxy-[1,2,4]triazolo[1,5-a]pyridin-6-yl)-1H-pyrazol-3-yl)thiazol-5-yl)piperidin-1-yl)-2-methylpropan-2-ol (860);
2-(4-(2-(4-isopropyl-5-(8-methoxy-[1,2,4]triazolo[1,5-a]pyridin-6-yl)-1H-pyrazol-3-yl)-4-methylthiazol-5-yl)piperidin-1-yl)-N,N-dimethylacetamide (861);
2-(4-isopropyl-5-(8-methoxy-[1,2,4]triazolo[1,5-a]pyridin-6-yl)-1H-pyrazol-3-yl)-5-(1-(2-methoxyethyl)piperidin-4-yl)-4-methylthiazole (862);
2-(4-isopropyl-5-(8-methoxy-[1,2,4]triazolo[1,5-a]pyridin-6-yl)-1H-pyrazol-3-yl)-4-methyl-5-(1-(2-(methylsulfonyl)ethyl)piperidin-4-yl)thiazole (863);
2-(4-isopropyl-5-(8-methoxy-[1,2,4]triazolo[1,5-a]pyridin-6-yl)-1H-pyrazol-3-yl)-5-(1-((1-(methylsulfonyl)cyclopropyl)methyl)piperidin-4-yl)thiazole (864);
2-(4-isopropyl-5-(8-methyl-[1,2,4]triazolo[1,5-a]pyridin-6-yl)-1H-pyrazol-3-yl)-5-(1-(2-(methylsulfonyl)ethyl)piperidin-4-yl)thiazole (865);
2-(4-isopropyl-5-(8-methyl-[1,2,4]triazolo[1,5-a]pyridin-6-yl)-1H-pyrazol-3-yl)-5-(1-(2-methoxyethyl)piperidin-4-yl)thiazole (866);
1-(4-(2-(4-isopropyl-5-(8-methyl-[1,2,4]triazolo[1,5-a]pyridin-6-yl)-1H-pyrazol-3-yl)thiazol-5-yl)piperidin-1-yl)-2-methylpropan-2-ol (867);
2-(4-(2-(4-isopropyl-5-(8-methyl-[1,2,4]triazolo[1,5-a]pyridin-6-yl)-1H-pyrazol-3-yl)thiazol-5-yl)piperidin-1-yl)acetonitrile (868);
2-(4-(2-(4-isopropyl-5-(8-methyl-[1,2,4]triazolo[1,5-a]pyridin-6-yl)-1H-pyrazol-3-yl)thiazol-5-yl)piperidin-1-yl)-N,N-dimethylacetamide (869);
2-(4-(2-(5-(7,8-dimethyl-[1,2,4]triazolo[1,5-a]pyridin-6-yl)-4-isopropyl-1H-pyrazol-3-yl)thiazol-5-yl)piperidin-1-yl)-N,N-dimethylacetamide (870);
2-(5-(7,8-dimethyl-[1,2,4]triazolo[1,5-a]pyridin-6-yl)-4-isopropyl-1H-pyrazol-3-yl)-5-(1-(2-methoxyethyl)piperidin-4-yl)thiazole (871);
2-(5-(7,8-dimethyl-[1,2,4]triazolo[1,5-a]pyridin-6-yl)-4-isopropyl-1H-pyrazol-3-yl)-5-(1-(2-methoxyethyl)piperidin-4-yl)-4-methylthiazole (872);
2-(5-(7,8-dimethyl-[1,2,4]triazolo[1,5-a]pyridin-6-yl)-4-isopropyl-1H-pyrazol-3-yl)-4-methyl-5-(1-(2-(methylsulfonyl)ethyl)piperidin-4-yl)thiazole (873);
2-(5-(2,6-dimethylpyridin-4-yl)-4-isopropyl-1H-pyrazol-3-yl)-5-(1-(2-methoxyethyl)piperidin-4-yl)thiazole (874);
2-(4-(2-(5-(2,6-dimethylpyridin-4-yl)-4-isopropyl-1H-pyrazol-3-yl)thiazol-5-yl)piperidin-1-yl)-N,N-dimethylacetamide (875);
2-(5-(3,4-dimethoxyphenyl)-4-isopropyl-1H-pyrazol-3-yl)-5-(1-(2-methoxyethyl)piperidin-4-yl)thiazole (877);
2-(4-isopropyl-5-(8-methoxy-[1,2,4]triazolo[1,5-a]pyridin-6-yl)-1H-pyrazol-3-yl)-5-(1-(2-(methylsulfonyl)ethyl)piperidin-4-yl)-4-(trifluoromethyl)thiazole (878);
2-(4-(2-(4-isopropyl-5-(8-methoxy-[1,2,4]triazolo[1,5-a]pyridin-6-yl)-1H-pyrazol-3-yl)-4-(trifluoromethyl)thiazol-5-yl)piperidin-1-yl)-N,N-dimethylacetamide (879);
2-(4-isopropyl-5-(8-methoxy-[1,2,4]triazolo[1,5-a]pyridin-6-yl)-1H-pyrazol-3-yl)-5-(1-(2-methoxyethyl)piperidin-4-yl)-4-(trifluoromethyl)thiazole (880);
5-(1-(3-fluoropropyl)piperidin-4-yl)-2-(4-isopropyl-5-(8-methoxy-[1,2,4]triazolo[1,5-a]pyridin-6-yl)-1H-pyrazol-3-yl)-4-methylthiazole (881);
5-(1-(2-fluoroethyl)piperidin-4-yl)-2-(4-isopropyl-5-(8-methoxy-[1,2,4]triazolo[1,5-a]pyridin-6-yl)-1H-pyrazol-3-yl)-4-methylthiazole (882);
4-(4-(2-(4-isopropyl-5-(8-methoxy-[1,2,4]triazolo[1,5-a]pyridin-6-yl)-1H-pyrazol-3-yl)-4-methylthiazol-5-yl)cyclohexyl)morpholine (883);
4-(2-(4-isopropyl-5-(8-methoxy-[1,2,4]triazolo[1,5-a]pyridin-6-yl)-1H-pyrazol-3-yl)-4-methylthiazol-5-yl)-N-(2-methoxyethyl)cyclohexan-1-amine (884-885);
N-(4-(2-(4-isopropyl-5-(8-methoxy-[1,2,4]triazolo[1,5-a]pyridin-6-yl)-1H-pyrazol-3-yl)-4-methylthiazol-5-yl)cyclohexyl)oxetan-3-amine (886-887);
4-(2-(4-isopropyl-5-(8-methoxy-[1,2,4]triazolo[1,5-a]pyridin-6-yl)-1H-pyrazol-3-yl)-4-methylthiazol-5-yl)-N-(2-methoxyethyl)-N-methylcyclohexan-1-amine (888-889);
6-(4-(2-(4-isopropyl-5-(8-methoxy-[1,2,4]triazolo[1,5-a]pyridin-6-yl)-1H-pyrazol-3-yl)-4-methylthiazol-5-yl)cyclohexyl)-2-oxa-6-azaspiro[3.3]heptane (890-891);
4-(2-(4-isopropyl-5-(8-methoxy-[1,2,4]triazolo[1,5-a]pyridin-6-yl)-1H-pyrazol-3-yl)-4-methylthiazol-5-yl)-N-((1-(methylsulfonyl)cyclopropyl)methyl)cyclohexan-1-amine (892-893);
6-(4-(2-(4-isopropyl-5-(8-methoxy-[1,2,4]triazolo[1,5-a]pyridin-6-yl)-1H-pyrazol-3-yl)-4-methylthiazol-5-yl)cyclohexyl)-2-thia-6-azaspiro[3.3]heptane 2,2-dioxide (894-895);
N-(4-(2-(4-isopropyl-5-(8-methoxy-[1,2,4]triazolo[1,5-a]pyridin-6-yl)-1H-pyrazol-3-yl)-4-methylthiazol-5-yl)cyclohexyl)tetrahydro-2H-pyran-4-amine (896-897);
2-(4-isopropyl-5-(8-methoxy-[1,2,4]triazolo[1,5-a]pyridin-6-yl)-1H-pyrazol-3-yl)-4-methyl-5-(4-(pyrrolidin-1-yl)cyclohexyl)thiazole (898);
N-cyclobutyl-4-(2-(4-isopropyl-5-(8-methoxy-[1,2,4]triazolo[1,5-a]pyridin-6-yl)-1H-pyrazol-3-yl)-4-methylthiazol-5-yl)cyclohexan-1-amine (891B and 892B);
4-(4-(2-(4-isopropyl-5-(8-methoxy-[1,2,4]triazolo[1,5-a]pyridin-6-yl)-1H-pyrazol-3-yl)-4-methylthiazol-5-yl)cyclohexyl)piperazin-2-one (893B and 894B);
2-(4-isopropyl-5-(8-methoxy-[1,2,4]triazolo[1,5-a]pyridin-6-yl)-1H-pyrazol-3-yl)-4-methyl-5-(4-(4-(methylsulfonyl)piperazin-1-yl)cyclohexyl)thiazole (895B and 896B);
4-(2-(4-isopropyl-5-(8-methoxy-[1,2,4]triazolo[1,5-a]pyridin-6-yl)-1H-pyrazol-3-yl)-4-methylthiazol-5-yl)-N-(2-methoxy-2-methylpropyl)cyclohexan-1-amine (897B and 898B);
4-(2-(4-isopropyl-5-(8-methoxy-[1,2,4]triazolo[1,5-a]pyridin-6-yl)-1H-pyrazol-3-yl)-4-methylthiazol-5-yl)-N-((3-methyloxetan-3-yl)methyl)cyclohexan-1-amine (899B and 900);
2-(4-(2-(4-isopropyl-5-(8-methoxy-[1,2,4]triazolo[1,5-a]pyridin-6-yl)-1H-pyrazol-3-yl)-4-methylthiazol-5-yl)cyclohexyl)-7-oxa-2-azaspiro[3.5]nonane (901-902);
4-(4-(2-(4-isopropyl-5-(8-methoxy-[1,2,4]triazolo[1,5-a]pyridin-6-yl)-1H-pyrazol-3-yl)-4-methylthiazol-5-yl)cyclohexyl)-1,4-oxazepane (903-904);
2-(4-isopropyl-5-(8-methoxy-[1,2,4]triazolo[1,5-a]pyridin-6-yl)-1H-pyrazol-3-yl)-5-(4-(3-methoxyazetidin-1-yl)cyclohexyl)-4-methylthiazole (905-906);
7-(4-(2-(4-isopropyl-5-(8-methoxy-[1,2,4]triazolo[1,5-a]pyridin-6-yl)-1H-pyrazol-3-yl)-4-methylthiazol-5-yl)cyclohexyl)-2-oxa-7-azaspiro[3.5]nonane (907-908);
1-(4-(2-(4-isopropyl-5-(8-methoxy-[1,2,4]triazolo[1,5-a]pyridin-6-yl)-1H-pyrazol-3-yl)-4-methylthiazol-5-yl)cyclohexyl)-3-(trifluoromethyl)azetidin-3-ol (909-910);
4-(2-(4-isopropyl-5-(8-methyl-[1,2,4]triazolo[1,5-a]pyridin-6-yl)-1H-pyrazol-3-yl)thiazol-5-yl)-N-methylcyclohexan-1-amine (911-912);
4-(2-(4-isopropyl-5-(8-methyl-[1,2,4]triazolo[1,5-a]pyridin-6-yl)-1H-pyrazol-3-yl)thiazol-5-yl)-N,N-dimethylcyclohexan-1-amine (913-914);
N-(4-(2-(4-isopropyl-5-(8-methoxy-[1,2,4]triazolo[1,5-a]pyridin-6-yl)-1H-pyrazol-3-yl)thiazol-5-yl)cyclohexyl)-3-methyloxetan-3-amine (915-916);
4-(2-(4-isopropyl-5-(8-methoxy-[1,2,4]triazolo[1,5-a]pyridin-6-yl)-1H-pyrazol-3-yl)thiazol-5-yl)-N-(2-methoxy-2-methylpropyl)cyclohexan-1-amine (916-917);
N-cyclobutyl-4-(2-(4-isopropyl-5-(8-methoxy-[1,2,4]triazolo[1,5-a]pyridin-6-yl)-1H-pyrazol-3-yl)thiazol-5-yl)cyclohexan-1-amine (918-919);
N-(4-(2-(4-isopropyl-5-(8-methoxy-[1,2,4]triazolo[1,5-a]pyridin-6-yl)-1H-pyrazol-3-yl)thiazol-5-yl)cyclohexyl)oxetan-3-amine (920-921);
4-(2-(4-isopropyl-5-(8-methoxy-[1,2,4]triazolo[1,5-a]pyridin-6-yl)-1H-pyrazol-3-yl)thiazol-5-yl)-N-(3,3,3-trifluoropropyl)cyclohexan-1-amine (922-923);
6-(4-(2-(4-isopropyl-5-(8-methoxy-[1,2,4]triazolo[1,5-a]pyridin-6-yl)-1H-pyrazol-3-yl)thiazol-5-yl)cyclohexyl)-2-oxa-6-azaspiro[3.3]heptane (924);
2-(dimethylamino)-N-(4-(2-(4-isopropyl-5-(8-methyl-[1,2,4]triazolo[1,5-a]pyridin-6-yl)-1H-pyrazol-3-yl)thiazol-5-yl)cyclohexyl)-N-methylacetamide (926-927);
4-(2-(4-isopropyl-5-(8-methyl-[1,2,4]triazolo[1,5-a]pyridin-6-yl)-1H-pyrazol-3-yl)thiazol-5-yl)-N-methyl-N-(3,3,3-trifluoropropyl)cyclohexan-1-amine (928-929);
2-(4-isopropyl-5-(8-methoxy-[1,2,4]triazolo[1,5-a]pyridin-6-yl)-1H-pyrazol-3-yl)-5-(piperazin-1-yl)thiazole (930);
2-(4-isopropyl-5-(8-methoxy-[1,2,4]triazolo[1,5-a]pyridin-6-yl)-1H-pyrazol-3-yl)-5-(2,6-diazaspiro[3.3]heptan-2-yl)thiazole (931);
1-(2-(4-isopropyl-5-(8-methoxy-[1,2,4]triazolo[1,5-a]pyridin-6-yl)-1H-pyrazol-3-yl)-4-methylthiazol-5-yl)piperidin-4-amine (932);
(S)-2-(4-isopropyl-5-(8-methoxy-[1,2,4]triazolo[1,5-a]pyridin-6-yl)-1H-pyrazol-3-yl)-5-(2-methylpiperazin-1-yl)thiazole (933);
2-(4-isopropyl-5-(8-methoxy-[1,2,4]triazolo[1,5-a]pyridin-6-yl)-1H-pyrazol-3-yl)-4-methyl-5-(piperazin-1-yl)thiazole (934);
5-((185,4S)-2,5-diazabicyclo[2.2.1]heptan-2-yl)-2-(4-isopropyl-5-(8-methoxy-[1,2,4]triazolo[1,5-a]pyridin-6-yl)-1H-pyrazol-3-yl)thiazole (935); 5-((2R,5S)-2,5-dimethylpiperazin-1-yl)-2-(4-isopropyl-5-(8-methoxy-[1,2,4]triazolo[1,5-a]pyridin-6-yl)-1H-pyrazol-3-yl)thiazole (936);
(R)-2-(4-isopropyl-5-(8-methoxy-[1,2,4]triazolo[1,5-a]pyridin-6-yl)-1H-pyrazol-3-yl)-4-methyl-5-(2-methylpiperazin-1-yl)thiazole (937);
(R)-2-(4-isopropyl-5-(8-methoxy-[1,2,4]triazolo[1,5-a]pyridin-6-yl)-1H-pyrazol-3-yl)-5-(2-methylpiperazin-1-yl)thiazole (938);
2-(4-isopropyl-5-(8-methoxy-[1,2,4]triazolo[1,5-a]pyridin-6-yl)-1H-pyrazol-3-yl)-5-(4-methyl piperazin-1-yl)thiazole (939);
5-(4-ethylpiperazin-1-yl)-2-(4-isopropyl-5-(8-methoxy-[1,2,4]triazolo[1,5-a]pyridin-6-yl)-1H-pyrazol-3-yl)thiazole (940);
2-(4-isopropyl-5-(8-methoxy-[1,2,4]triazolo[1,5-a]pyridin-6-yl)-1H-pyrazol-3-yl)-5-(4-propylpiperazin-1-yl)thiazole (941);
2-(4-isopropyl-5-(8-methoxy-[1,2,4]triazolo[1,5-a]pyridin-6-yl)-1H-pyrazol-3-yl)-5-(4-isopropylpiperazin-1-yl)thiazole (942);
2-(4-isopropyl-5-(8-methoxy-[1,2,4]triazolo[1,5-a]pyridin-6-yl)-1H-pyrazol-3-yl)-5-(4-(oxetan-3-yl)piperazin-1-yl)thiazole (943);
5-(4-isobutylpiperazin-1-yl)-2-(4-isopropyl-5-(8-methoxy-[1,2,4]triazolo[1,5-a]pyridin-6-yl)-1H-pyrazol-3-yl)thiazole (944);
2-(4-isopropyl-5-(8-methoxy-[1,2,4]triazolo[1,5-a]pyridin-6-yl)-1H-pyrazol-3-yl)-5-(4-(tetrahydro-2H-pyran-4-yl)piperazin-1-yl)thiazole (945);
5-(4-(cyclopropylmethyl)piperazin-1-yl)-2-(4-isopropyl-5-(8-methoxy-[1,2,4]triazolo[1,5-a]pyridin-6-yl)-1H-pyrazol-3-yl)thiazole (946);
2-(4-isopropyl-5-(8-methoxy-[1,2,4]triazolo[1,5-a]pyridin-6-yl)-1H-pyrazol-3-yl)-5-(4-((tetrahydro-2H-pyran-4-yl)methyl)piperazin-1-yl)thiazole (947);
2-(4-isopropyl-5-(8-methoxy-[1,2,4]triazolo[1,5-a]pyridin-6-yl)-1H-pyrazol-3-yl)-5-(6-isopropyl-2,6-diazaspiro[3.3]heptan-2-yl)thiazole (948);
N-isopropyl-1-(2-(4-isopropyl-5-(8-methoxy-[1,2,4]triazolo[1,5-a]pyridin-6-yl)-1H-pyrazol-3-yl)-4-methylthiazol-5-yl)piperidin-4-amine (949);
1-(2-(4-isopropyl-5-(8-methoxy-[1,2,4]triazolo[1,5-a]pyridin-6-yl)-1H-pyrazol-3-yl)-4-methylthiazol-5-yl)-N-(oxetan-3-yl)piperidin-4-amine (950);
(S)-5-(4-(cyclopropylmethyl)-2-methylpiperazin-1-yl)-2-(4-isopropyl-5-(8-methoxy-[1,2,4]triazolo[1,5-a]pyridin-6-yl)-1H-pyrazol-3-yl)thiazole (951);
(S)-5-(2,4-dimethylpiperazin-1-yl)-2-(4-isopropyl-5-(8-methoxy-[1,2,4]triazolo[1,5-a]pyridin-6-yl)-1H-pyrazol-3-yl)thiazole (952);
(S)-2-(4-isopropyl-5-(8-methoxy-[1,2,4]triazolo[1,5-a]pyridin-6-yl)-1H-pyrazol-3-yl)-5-(2-methyl-4-propylpiperazin-1-yl)thiazole (953);
(S)-5-(4-isopropyl-2-methylpiperazin-1-yl)-2-(4-isopropyl-5-(8-methoxy-[1,2,4]triazolo[1,5-a]pyridin-6-yl)-1H-pyrazol-3-yl)thiazole (954);
(S)-5-(4-ethyl-2-methylpiperazin-1-yl)-2-(4-isopropyl-5-(8-methoxy-[1,2,4]triazolo[1,5-a]pyridin-6-yl)-1H-pyrazol-3-yl)thiazole (955);
(S)-5-(4-isobutyl-2-methylpiperazin-1-yl)-2-(4-isopropyl-5-(8-methoxy-[1,2,4]triazolo[1,5-a]pyridin-6-yl)-1H-pyrazol-3-yl)thiazole (956);
N-isobutyl-1-(2-(4-isopropyl-5-(8-methoxy-[1,2,4]triazolo[1,5-a]pyridin-6-yl)-1H-pyrazol-3-yl)-4-methylthiazol-5-yl)piperidin-4-amine (957);
2-(4-isopropyl-5-(8-methoxy-[1,2,4]triazolo[1,5-a]pyridin-6-yl)-1H-pyrazol-3-yl)-4-methyl-5-(4-methylpiperazin-1-yl)thiazole (958);
5-(4-(cyclopropylmethyl)piperazin-1-yl)-2-(4-isopropyl-5-(8-methoxy-[1,2,4]triazolo[1,5-a]pyridin-6-yl)-1H-pyrazol-3-yl)-4-methylthiazole (959);
5-(4-ethylpiperazin-1-yl)-2-(4-isopropyl-5-(8-methoxy-[1,2,4]triazolo[1,5-a]pyridin-6-yl)-1H-pyrazol-3-yl)-4-methylthiazole (960);
5-(4-isobutylpiperazin-1-yl)-2-(4-isopropyl-5-(8-methoxy-[1,2,4]triazolo[1,5-a]pyridin-6-yl)-1H-pyrazol-3-yl)-4-methylthiazole (961);
N-(cyclopropylmethyl)-1-(2-(4-isopropyl-5-(8-methoxy-[1,2,4]triazolo[1,5-a]pyridin-6-yl)-1H-pyrazol-3-yl)-4-methylthiazol-5-yl)piperidin-4-amine (962);
1-(2-(4-isopropyl-5-(8-methoxy-[1,2,4]triazolo[1,5-a]pyridin-6-yl)-1H-pyrazol-3-yl)-4-methylthiazol-5-yl)-N-(tetrahydro-2H-pyran-4-yl)piperidin-4-amine (963);
1-(2-(4-isopropyl-5-(8-methoxy-[1,2,4]triazolo[1,5-a]pyridin-6-yl)-1H-pyrazol-3-yl)-4-methylthiazol-5-yl)-N-propylpiperidin-4-amine (964);
1-(2-(4-isopropyl-5-(8-methoxy-[1,2,4]triazolo[1,5-a]pyridin-6-yl)-1H-pyrazol-3-yl)-4-methylthiazol-5-yl)-N,N-bis((tetrahydro-2H-pyran-4-yl)methyl)piperidin-4-amine (965);
N,N-bis(cyclopropylmethyl)-1-(2-(4-isopropyl-5-(8-methoxy-[1,2,4]triazolo[1,5-a]pyridin-6-yl)-1H-pyrazol-3-yl)-4-methylthiazol-5-yl)piperidin-4-amine (966);
1-(2-(4-isopropyl-5-(8-methoxy-[1,2,4]triazolo[1,5-a]pyridin-6-yl)-1H-pyrazol-3-yl)-4-methylthiazol-5-yl)-N,N-dimethylpiperidin-4-amine (967);
2-(4-isopropyl-5-(8-methoxy-[1,2,4]triazolo[1,5-a]pyridin-6-yl)-1H-pyrazol-3-yl)-4-methyl-5-(4-propylpiperazin-1-yl)thiazole (968);
2-(4-isopropyl-5-(8-methoxy-[1,2,4]triazolo[1,5-a]pyridin-6-yl)-1H-pyrazol-3-yl)-4-methyl-5-(4-((tetrahydro-2H-pyran-4-yl)methyl)piperazin-1-yl)thiazole (969);
2-(4-isopropyl-5-(8-methoxy-[1,2,4]triazolo[1,5-a]pyridin-6-yl)-1H-pyrazol-3-yl)-4-methyl-5-(4-(oxetan-3-yl)piperazin-1-yl)thiazole (970);
2-(4-isopropyl-5-(8-methoxy-[1,2,4]triazolo[1,5-a]pyridin-6-yl)-1H-pyrazol-3-yl)-5-(4-isopropylpiperazin-1-yl)-4-methylthiazole (971);
5-(4-(cyclobutylmethyl)piperazin-1-yl)-2-(4-isopropyl-5-(8-methoxy-[1,2,4]triazolo[1,5-a]pyridin-6-yl)-1H-pyrazol-3-yl)thiazole (972);
(S)-5-(4-(cyclobutylmethyl)-2-methylpiperazin-1-yl)-2-(4-isopropyl-5-(8-methoxy-[1,2,4]triazolo[1,5-a]pyridin-6-yl)-1H-pyrazol-3-yl)thiazole (973);
5-((1S,4S)-5-isobutyl-2,5-diazabicyclo[2.2.1]heptan-2-yl)-2-(4-isopropyl-5-(8-methoxy-[1,2,4]triazolo[1,5-a]pyridin-6-yl)-1H-pyrazol-3-yl)thiazole (974);
5-((1R,4R)-5-ethyl-2,5-diazabicyclo[2.2.1]heptan-2-yl)-2-(4-isopropyl-5-(8-methoxy-[1,2,4]triazolo[1,5-a]pyridin-6-yl)-1H-pyrazol-3-yl)thiazole (975);
5-((185,4S)-5-cyclobutyl-2,5-diazabicyclo[2.2.1]heptan-2-yl)-2-(4-isopropyl-5-(8-methoxy-[1,2,4]triazolo[1,5-a]pyridin-6-yl)-1H-pyrazol-3-yl)thiazole (976);
2-(4-isopropyl-5-(8-methoxy-[1,2,4]triazolo[1,5-a]pyridin-6-yl)-1H-pyrazol-3-yl)-5-((1S,4S)-5-isopropyl-2,5-diazabicyclo[2.2.1]heptan-2-yl)thiazole (977);
2-(4-isopropyl-5-(8-methoxy-[1,2,4]triazolo[1,5-a]pyridin-6-yl)-1H-pyrazol-3-yl)-5-((1R,4R)-5-propyl-2,5-diazabicyclo[2.2.1]heptan-2-yl)thiazole (978);
2-(4-isopropyl-5-(8-methoxy-[1,2,4]triazolo[1,5-a]pyridin-6-yl)-1H-pyrazol-3-yl)-5-((1S,4S)-5-(oxetan-3-yl)-2,5-diazabicyclo[2.2.1]heptan-2-yl)thiazole (979);
2-(4-isopropyl-5-(8-methoxy-[1,2,4]triazolo[1,5-a]pyridin-6-yl)-1H-pyrazol-3-yl)-5-((1S,4S)-5-(tetrahydro-2H-pyran-4-yl)-2,5-diazabicyclo[2.2.1]heptan-2-yl)thiazole (980);
2-(4-isopropyl-5-(8-methoxy-[1,2,4]triazolo[1,5-a]pyridin-6-yl)-1H-pyrazol-3-yl)-5-((1S,4S)-5-((tetrahydro-2H-pyran-4-yl)methyl)-2,5-diazabicyclo[2.2.1]heptan-2-yl)thiazole (981);
5-((1S,4S)-5-(cyclobutylmethyl)-2,5-diazabicyclo[2.2.1]heptan-2-yl)-2-(4-isopropyl-5-(8-methoxy-[1,2,4]triazolo[1,5-a]pyridin-6-yl)-1H-pyrazol-3-yl)thiazole (982);
5-((2R,55)-2,5-dimethyl-4-((tetrahydro-2H-pyran-4-yl)methyl)piperazin-1-yl)-2-(4-isopropyl-5-(8-methoxy-[1,2,4]triazolo[1,5-a]pyridin-6-yl)-1H-pyrazol-3-yl)thiazole (983);
5-((2R,5S)-4-(cyclobutylmethyl)-2,5-dimethylpiperazin-1-yl)-2-(4-isopropyl-5-(8-methoxy-[1,2,4]triazolo[1,5-a]pyridin-6-yl)-1H-pyrazol-3-yl)thiazole (984);
5-((2R,5S)-4-isobutyl-2,5-dimethylpiperazin-1-yl)-2-(4-isopropyl-5-(8-methoxy-[1,2,4]triazolo[1,5-a]pyridin-6-yl)-1H-pyrazol-3-yl)thiazole (985);
5-((2R,55)-4-(cyclopropylmethyl)-2,5-dimethylpiperazin-1-yl)-2-(4-isopropyl-5-(8-methoxy-[1,2,4]triazolo[1,5-a]pyridin-6-yl)-1H-pyrazol-3-yl)thiazole (986);
5-((2R,5S)-4-cyclobutyl-2,5-dimethylpiperazin-1-yl)-2-(4-isopropyl-5-(8-methoxy-[1,2,4]triazolo[1,5-a]pyridin-6-yl)-1H-pyrazol-3-yl)thiazole (987);
5-((2R,55)-2,5-dimethyl-4-(tetrahydro-2H-pyran-4-yl)piperazin-1-yl)-2-(4-isopropyl-5-(8-methoxy-[1,2,4]triazolo[1,5-a]pyridin-6-yl)-1H-pyrazol-3-yl)thiazole (988);
2-(4-isopropyl-5-(8-methoxy-[1,2,4]triazolo[1,5-a]pyridin-6-yl)-1H-pyrazol-3-yl)-5-((2R,5S)-2,4,5-trimethylpiperazin-1-yl)thiazole (989);
5-((2R,5S)-4-ethyl-2,5-dimethylpiperazin-1-yl)-2-(4-isopropyl-5-(8-methoxy-[1,2,4]triazolo[1,5-a]pyridin-6-yl)-1H-pyrazol-3-yl)thiazole (990);
5-((2R,55)-2,5-dimethyl-4-propylpiperazin-1-yl)-2-(4-isopropyl-5-(8-methoxy-[1,2,4]triazolo[1,5-a]pyridin-6-yl)-1H-pyrazol-3-yl)thiazole (991);
5-((2R,5S)-4-isopropyl-2,5-dimethylpiperazin-1-yl)-2-(4-isopropyl-5-(8-methoxy-[1,2,4]triazolo[1,5-a]pyridin-6-yl)-1H-pyrazol-3-yl)thiazole (992);
(R)-2-(4-isopropyl-5-(8-methoxy-[1,2,4]triazolo[1,5-a]pyridin-6-yl)-1H-pyrazol-3-yl)-4-methyl-5-(2-methyl-4-((tetrahydro-2H-pyran-4-yl)methyl)piperazin-1-yl)thiazole (993);
(R)-2-(4-isopropyl-5-(8-methoxy-[1,2,4]triazolo[1,5-a]pyridin-6-yl)-1H-pyrazol-3-yl)-4-methyl-5-(2-methyl-4-(tetrahydro-2H-pyran-4-yl)piperazin-1-yl)thiazole (994);
(R)-2-(4-isopropyl-5-(8-methoxy-[1,2,4]triazolo[1,5-a]pyridin-6-yl)-1H-pyrazol-3-yl)-4-methyl-5-(2-methyl-4-(oxetan-3-yl)piperazin-1-yl)thiazole (995);
(R)-5-(4-cyclobutyl-2-methylpiperazin-1-yl)-2-(4-isopropyl-5-(8-methoxy-[1,2,4]triazolo[1,5-a]pyridin-6-yl)-1H-pyrazol-3-yl)-4-methylthiazole (996);
(R)-5-(4-(cyclobutylmethyl)-2-methylpiperazin-1-yl)-2-(4-isopropyl-5-(8-methoxy-[1,2,4]triazolo[1,5-a]pyridin-6-yl)-1H-pyrazol-3-yl)-4-methylthiazole (997);
(R)-5-(4-isobutyl-2-methylpiperazin-1-yl)-2-(4-isopropyl-5-(8-methoxy-[1,2,4]triazolo[1,5-a]pyridin-6-yl)-1H-pyrazol-3-yl)-4-methylthiazole (998);
(R)-5-(4-(cyclopropylmethyl)-2-methyl piperazin-1-yl)-2-(4-isopropyl-5-(8-methoxy-[1,2,4]triazolo[1,5-a]pyridin-6-yl)-1H-pyrazol-3-yl)-4-methylthiazole (999);
(R)-5-(2,4-dimethylpiperazin-1-yl)-2-(4-isopropyl-5-(8-methoxy-[1,2,4]triazolo[1,5-a]pyridin-6-yl)-1H-pyrazol-3-yl)-4-methylthiazole (1000);
(R)-2-(4-isopropyl-5-(8-methoxy-[1,2,4]triazolo[1,5-a]pyridin-6-yl)-1H-pyrazol-3-yl)-4-methyl-5-(2-methyl-4-propylpiperazin-1-yl)thiazole (1001);
(S)-2-(4-isopropyl-5-(8-methoxy-[1,2,4]triazolo[1,5-a]pyridin-6-yl)-1H-pyrazol-3-yl)-4-methyl-5-(2-methyl-4-(pentan-3-yl)piperazin-1-yl)thiazole (1002);
(R)-5-(4-isopropyl-2-methylpiperazin-1-yl)-2-(4-isopropyl-5-(8-methoxy-[1,2,4]triazolo[1,5-a]pyridin-6-yl)-1H-pyrazol-3-yl)-4-methylthiazole (1003);
(R)-2-(4-isopropyl-5-(8-methoxy-[1,2,4]triazolo[1,5-a]pyridin-6-yl)-1H-pyrazol-3-yl)-5-(2-methyl-4-((tetrahydro-2H-pyran-4-yl)methyl)piperazin-1-yl)thiazole (1004);
(R)-2-(4-isopropyl-5-(8-methoxy-[1,2,4]triazolo[1,5-a]pyridin-6-yl)-1H-pyrazol-3-yl)-5-(2-methyl-4-(tetrahydro-2H-pyran-4-yl)piperazin-1-yl)thiazole (1005);
(R)-2-(4-isopropyl-5-(8-methoxy-[1,2,4]triazolo[1,5-a]pyridin-6-yl)-1H-pyrazol-3-yl)-5-(2-methyl-4-(oxetan-3-yl)piperazin-1-yl)thiazole (1006);
(R)-5-(4-cyclobutyl-2-methylpiperazin-1-yl)-2-(4-isopropyl-5-(8-methoxy-[1,2,4]triazolo[1,5-a]pyridin-6-yl)-1H-pyrazol-3-yl)thiazole (1007);
(R)-5-(4-(cyclobutylmethyl)-2-methylpiperazin-1-yl)-2-(4-isopropyl-5-(8-methoxy-[1,2,4]triazolo[1,5-a]pyridin-6-yl)-1H-pyrazol-3-yl)thiazole (1008);
(R)-5-(4-(cyclopropylmethyl)-2-methyl piperazin-1-yl)-2-(4-isopropyl-5-(8-methoxy-[1,2,4]triazolo[1,5-a]pyridin-6-yl)-1H-pyrazol-3-yl)thiazole (1009);
(R)-5-(2,4-dimethylpiperazin-1-yl)-2-(4-isopropyl-5-(8-methoxy-[1,2,4]triazolo[1,5-a]pyridin-6-yl)-1H-pyrazol-3-yl)thiazole (1010);
(R)-5-(4-isobutyl-2-methylpiperazin-1-yl)-2-(4-isopropyl-5-(8-methoxy-[1,2,4]triazolo[1,5-a]pyridin-6-yl)-1H-pyrazol-3-yl)thiazole (1011);
(R)-5-(4-isopropyl-2-methylpiperazin-1-yl)-2-(4-isopropyl-5-(8-methoxy-[1,2,4]triazolo[1,5-a]pyridin-6-yl)-1H-pyrazol-3-yl)thiazole (1012);
(R)-2-(4-isopropyl-5-(8-methoxy-[1,2,4]triazolo[1,5-a]pyridin-6-yl)-1H-pyrazol-3-yl)-5-(2-methyl-4-(pentan-3-yl)piperazin-1-yl)thiazole (1013);
(R)-2-(4-isopropyl-5-(8-methoxy-[1,2,4]triazolo[1,5-a]pyridin-6-yl)-1H-pyrazol-3-yl)-5-(2-methyl-4-propylpiperazin-1-yl)thiazole (1014);
2-(dimethylamino)-1-(4-(2-(4-isopropyl-5-(8-methoxy-[1,2,4]triazolo[1,5-a]pyridin-6-yl)-1H-pyrazol-3-yl)thiazol-5-yl)piperazin-1-yl)ethan-1-one (1015);
1-(4-(2-(4-isopropyl-5-(8-methoxy-[1,2,4]triazolo[1,5-a]pyridin-6-yl)-1H-pyrazol-3-yl)thiazol-5-yl)piperazin-1-yl)-2-morpholinoethan-1-one (1016);
(S)-2-(dimethylamino)-1-(4-(2-(4-isopropyl-5-(8-methoxy-[1,2,4]triazolo[1,5-a]pyridin-6-yl)-1H-pyrazol-3-yl)thiazol-5-yl)-3-methylpiperazin-1-yl)ethan-1-one (1017);
(R)-2-(diethylamino)-1-(4-(2-(4-isopropyl-5-(8-methoxy-[1,2,4]triazolo[1,5-a]pyridin-6-yl)-1H-pyrazol-3-yl)thiazol-5-yl)-3-methylpiperazin-1-yl)ethan-1-one (1018);
2-(diethylamino)-1-(4-(2-(4-isopropyl-5-(8-methoxy-[1,2,4]triazolo[1,5-a]pyridin-6-yl)-1H-pyrazol-3-yl)-4-methylthiazol-5-yl)piperazin-1-yl)ethan-1-one (1018B)
2-(dimethylamino)-1-(4-(2-(4-isopropyl-5-(8-methoxy-[1,2,4]triazolo[1,5-a]pyridin-6-yl)-1H-pyrazol-3-yl)-4-methylthiazol-5-yl)piperazin-1-yl)ethan-1-one (1019);
2-(diethylamino)-N-(1-(2-(4-isopropyl-5-(8-methoxy-[1,2,4]triazolo[1,5-a]pyridin-6-yl)-1H-pyrazol-3-yl)-4-methylthiazol-5-yl)piperidin-4-yl)acetamide (1020);
2-(dimethylamino)-N-(1-(2-(4-isopropyl-5-(8-methoxy-[1,2,4]triazolo[1,5-a]pyridin-6-yl)-1H-pyrazol-3-yl)-4-methylthiazol-5-yl)piperidin-4-yl)acetamide (1021);
N-(1-(2-(4-isopropyl-5-(8-methoxy-[1,2,4]triazolo[1,5-a]pyridin-6-yl)-1H-pyrazol-3-yl)-4-methylthiazol-5-yl)piperidin-4-yl)-2-morpholinoacetamide (1022);
2-(diethylamino)-1-(4-(2-(4-isopropyl-5-(8-methoxy-[1,2,4]triazolo[1,5-a]pyridin-6-yl)-1H-pyrazol-3-yl)thiazol-5-yl)piperazin-1-yl)ethan-1-one (1023);
2-(diethylamino)-1-((2S,5R)-4-(2-(4-isopropyl-5-(8-methoxy-[1,2,4]triazolo[1,5-a]pyridin-6-yl)-1H-pyrazol-3-yl)thiazol-5-yl)-2,5-dimethylpiperazin-1-yl)ethan-1-one (1024);
2-(dimethylamino)-1-((2S,5R)-4-(2-(4-isopropyl-5-(8-methoxy-[1,2,4]triazolo[1,5-a]pyridin-6-yl)-1H-pyrazol-3-yl)thiazol-5-yl)-2, 5-dimethylpiperazin-1-yl)ethan-1-one (1025);
(R)-2-(dimethylamino)-1-(4-(2-(4-isopropyl-5-(8-methoxy-[1,2,4]triazolo[1,5-a]pyridin-6-yl)-1H-pyrazol-3-yl)-4-methylthiazol-5-yl)-3-methylpiperazin-1-yl)ethan-1-one (1026);
(R)-2-(dimethylamino)-1-(4-(2-(4-isopropyl-5-(8-methoxy-[1,2,4]triazolo[1,5-a]pyridin-6-yl)-1H-pyrazol-3-yl)thiazol-5-yl)-3-methylpiperazin-1-yl)ethan-1-one (1027);
2-(4-isopropyl-5-(8-methoxy-[1,2,4]triazolo[1,5-a]pyridin-6-yl)-1H-pyrazol-3-yl)-5-(4-(2-methoxyethyl)piperazin-1-yl)thiazole (1028);
2-(4-(2-(4-isopropyl-5-(8-methoxy-[1,2,4]triazolo[1,5-a]pyridin-6-yl)-1H-pyrazol-3-yl)-4-methylthiazol-5-yl)piperazin-1-yl)-N,N-dimethylacetamide (1029);
(S)-2-(4-isopropyl-5-(8-methoxy-[1,2,4]triazolo[1,5-a]pyridin-6-yl)-1H-pyrazol-3-yl)-5-(4-(2-methoxyethyl)-2-methylpiperazin-1-yl)thiazole (1030);
2-(4-isopropyl-5-(8-methoxy-[1,2,4]triazolo[1,5-a]pyridin-6-yl)-1H-pyrazol-3-yl)-5-(4-(2-(methylsulfonyl)ethyl)piperazin-1-yl)thiazole (1031);
2-(4-(2-(4-isopropyl-5-(8-methoxy-[1,2,4]triazolo[1,5-a]pyridin-6-yl)-1H-pyrazol-3-yl)thiazol-5-yl)piperazin-1-yl)-N,N-dimethylacetamide (1032);
(S)-2-(4-isopropyl-5-(8-methoxy-[1,2,4]triazolo[1,5-a]pyridin-6-yl)-1H-pyrazol-3-yl)-5-(2-methyl-4-(2-(methylsulfonyl)ethyl)piperazin-1-yl)thiazole (1033);
1-(4-(2-(4-isopropyl-5-(8-methoxy-[1,2,4]triazolo[1,5-a]pyridin-6-yl)-1H-pyrazol-3-yl)thiazol-5-yl)piperazin-1-yl)-2-methylpropan-2-ol (1034);
5-((2R,5S)-2,5-dimethyl-4-(2-(methylsulfonyl)ethyl)piperazin-1-yl)-2-(4-isopropyl-5-(8-methoxy-[1,2,4]triazolo[1,5-a]pyridin-6-yl)-1H-pyrazol-3-yl)thiazole (1035);
2-(4-isopropyl-5-(8-methoxy-[1,2,4]triazolo[1,5-a]pyridin-6-yl)-1H-pyrazol-3-yl)-5-((2R,5S)-4-(2-methoxyethyl)-2,5-dimethylpiperazin-1-yl)thiazole (1036);
(R)-2-(4-isopropyl-5-(8-methoxy-[1,2,4]triazolo[1,5-a]pyridin-6-yl)-1H-pyrazol-3-yl)-4-methyl-5-(2-methyl-4-(2-(methylsulfonyl)ethyl)piperazin-1-yl)thiazole (1037);
(R)-5-(4-(2-fluoroethyl)-2-methylpiperazin-1-yl)-2-(4-isopropyl-5-(8-methoxy-[1,2,4]triazolo[1,5-a]pyridin-6-yl)-1H-pyrazol-3-yl)-4-methylthiazole (1038);
(R)-5-(4-(3-fluoropropyl)-2-methylpiperazin-1-yl)-2-(4-isopropyl-5-(8-methoxy-[1,2,4]triazolo[1,5-a]pyridin-6-yl)-1H-pyrazol-3-yl)-4-methylthiazole (1039);
(R)-2-(4-isopropyl-5-(8-methoxy-[1,2,4]triazolo[1,5-a]pyridin-6-yl)-1H-pyrazol-3-yl)-5-(4-(2-methoxyethyl)-2-methylpiperazin-1-yl)-4-methylthiazole (1040);
(R)-2-(4-(2-(4-isopropyl-5-(8-methoxy-[1,2,4]triazolo[1,5-a]pyridin-6-yl)-1H-pyrazol-3-yl)-4-methylthiazol-5-yl)-3-methylpiperazin-1-yl)-N,N-dimethylacetamide (1041);
(R)-2-(4-isopropyl-5-(8-methoxy-[1,2,4]triazolo[1,5-a]pyridin-6-yl)-1H-pyrazol-3-yl)-5-(2-methyl-4-(2-(methylsulfonyl)ethyl)piperazin-1-yl)thiazole (1042);
(R)-2-(4-isopropyl-5-(8-methoxy-[1,2,4]triazolo[1,5-a]pyridin-6-yl)-1H-pyrazol-3-yl)-5-(4-(2-methoxyethyl)-2-methylpiperazin-1-yl)thiazole (1043);
(R)-2-(4-(2-(4-isopropyl-5-(8-methoxy-[1,2,4]triazolo[1,5-a]pyridin-6-yl)-1H-pyrazol-3-yl)thiazol-5-yl)-3-methylpiperazin-1-yl)-N,N-dimethylacetamide (1044);
(R)-5-(4-(2-fluoroethyl)-2-methylpiperazin-1-yl)-2-(4-isopropyl-5-(8-methoxy-[1,2,4]triazolo[1,5-a]pyridin-6-yl)-1H-pyrazol-3-yl)thiazole (1045);
(R)-5-(4-(3-fluoropropyl)-2-methylpiperazin-1-yl)-2-(4-isopropyl-5-(8-methoxy-[1,2,4]triazolo[1,5-a]pyridin-6-yl)-1H-pyrazol-3-yl)thiazole (1046);
(R)-2-(4-(2-(4-isopropyl-5-(8-methoxy-[1,2,4]triazolo[1,5-a]pyridin-6-yl)-1H-pyrazol-3-yl)thiazol-5-yl)-3-methylpiperazin-1-yl)-N-methylacetamide (1047);
(R)-2-(4-(2-(4-isopropyl-5-(8-methoxy-[1,2,4]triazolo[1,5-a]pyridin-6-yl)-1H-pyrazol-3-yl)thiazol-5-yl)-3-methylpiperazin-1-yl)acetonitrile (1048);
2-(4-isopropyl-5-(8-methoxy-[1,2,4]triazolo[1,5-a]pyridin-6-yl)-1H-pyrazol-3-yl)-4-methyl-5-(6-propyl-2,6-diazaspiro[3.3]heptan-2-yl)thiazole (1049);
2-(4-isopropyl-5-(8-methoxy-[1,2,4]triazolo[1,5-a]pyridin-6-yl)-1H-pyrazol-3-yl)-4-methyl-5-(6-methyl-2,6-diazaspiro[3.3]heptan-2-yl)thiazole (1050);
5-(6-ethyl-2,6-diazaspiro[3.3]heptan-2-yl)-2-(4-isopropyl-5-(8-methoxy-[1,2,4]triazolo[1,5-a]pyridin-6-yl)-1H-pyrazol-3-yl)-4-methylthiazole (1051);
2-(4-isopropyl-5-(8-methoxy-[1,2,4]triazolo[1,5-a]pyridin-6-yl)-1H-pyrazol-3-yl)-5-(6-isopropyl-2,6-diazaspiro[3.3]heptan-2-yl)-4-methylthiazole (1052);
5-(6-(cyclopropylmethyl)-2,6-diazaspiro[3.3]heptan-2-yl)-2-(4-isopropyl-5-(8-methoxy-[1,2,4]triazolo[1,5-a]pyridin-6-yl)-1H-pyrazol-3-yl)-4-methylthiazole (1053);
5-(6-isobutyl-2,6-diazaspiro[3.3]heptan-2-yl)-2-(4-isopropyl-5-(8-methoxy-[1,2,4]triazolo[1,5-a]pyridin-6-yl)-1H-pyrazol-3-yl)-4-methylthiazole (1054);
5-(6-(2-ethylbutyl)-2,6-diazaspiro[3.3]heptan-2-yl)-2-(4-isopropyl-5-(8-methoxy-[1,2,4]triazolo[1,5-a]pyridin-6-yl)-1H-pyrazol-3-yl)-4-methylthiazole (1055);
5-(6-cyclobutyl-2,6-diazaspiro[3.3]heptan-2-yl)-2-(4-isopropyl-5-(8-methoxy-[1,2,4]triazolo[1,5-a]pyridin-6-yl)-1H-pyrazol-3-yl)-4-methylthiazole (1056);
5-(6-(cyclobutylmethyl)-2,6-diazaspiro[3.3]heptan-2-yl)-2-(4-isopropyl-5-(8-methoxy-[1,2,4]triazolo[1,5-a]pyridin-6-yl)-1H-pyrazol-3-yl)-4-methylthiazole (1057);
2-(4-isopropyl-5-(8-methoxy-[1,2,4]triazolo[1,5-a]pyridin-6-yl)-1H-pyrazol-3-yl)-4-methyl-5-(6-(tetrahydro-2H-pyran-4-yl)-2,6-diazaspiro[3.3]heptan-2-yl)thiazole (1058);
2-(4-isopropyl-5-(8-methoxy-[1,2,4]triazolo[1,5-a]pyridin-6-yl)-1H-pyrazol-3-yl)-4-methyl-5-(6-((tetrahydro-2H-pyran-4-yl)methyl)-2,6-diazaspiro[3.3]heptan-2-yl)thiazole (1059);
2-(dimethylamino)-1-(6-(2-(4-isopropyl-5-(8-methoxy-[1,2,4]triazolo[1,5-a]pyridin-6-yl)-1H-pyrazol-3-yl)-4-methylthiazol-5-yl)-2,6-diazaspiro[3.3]heptan-2-yl)ethan-1-one (1060);
2-(4-isopropyl-5-(8-methoxy-[1,2,4]triazolo[1,5-a]pyridin-6-yl)-1H-pyrazol-3-yl)-4-methyl-5-(6-(2-(methylsulfonyl)ethyl)-2,6-diazaspiro[3.3]heptan-2-yl)thiazole (1061);
2-(4-isopropyl-5-(8-methoxy-[1,2,4]triazolo[1,5-a]pyridin-6-yl)-1H-pyrazol-3-yl)-5-(6-(2-methoxyethyl)-2,6-diazaspiro[3.3]heptan-2-yl)-4-methylthiazole (1062);
5-(4-isopropyl-5-(8-methoxy-[1,2,4]triazolo[1,5-a]pyridin-6-yl)-1H-pyrazol-3-yl)-2-(1-isopropylpiperidin-4-yl)thiazole (1063);
5-(4-isopropyl-5-(8-methyl-[1,2,4]triazolo[1,5-a]pyridin-6-yl)-1H-pyrazol-3-yl)-2-(piperidin-4-yl)thiazole (1064);
5-(4-isopropyl-5-(8-methoxy-[1,2,4]triazolo[1,5-a]pyridin-6-yl)-1H-pyrazol-3-yl)-2-(1-methylpiperidin-4-yl)thiazole (1065);
2-(1-ethylpiperidin-4-yl)-5-(4-isopropyl-5-(8-methoxy-[1,2,4]triazolo[1,5-a]pyridin-6-yl)-1H-pyrazol-3-yl)thiazole (1066);
5-(4-isopropyl-5-(8-methoxy-[1,2,4]triazolo[1,5-a]pyridin-6-yl)-1H-pyrazol-3-yl)-2-(1-propylpiperidin-4-yl)thiazole (1067);
5-(4-isopropyl-5-(8-methyl-[1,2,4]triazolo[1,5-a]pyridin-6-yl)-1H-pyrazol-3-yl)-2-(1-propylpiperidin-4-yl)thiazole (1068);
5-(4-isopropyl-5-(8-methyl-[1,2,4]triazolo[1,5-a]pyridin-6-yl)-1H-pyrazol-3-yl)-2-(1-(tetrahydro-2H-pyran-4-yl)piperidin-4-yl)thiazole (1069);
5-(4-isopropyl-5-(8-methyl-[1,2,4]triazolo[1,5-a]pyridin-6-yl)-1H-pyrazol-3-yl)-2-(1-methylpiperidin-4-yl)thiazole (1070);
2-(1-ethylpiperidin-4-yl)-5-(4-isopropyl-5-(8-methyl-[1,2,4]triazolo[1,5-a]pyridin-6-yl)-1H-pyrazol-3-yl)thiazole (1071);
5-(4-isopropyl-5-(8-methyl-[1,2,4]triazolo[1,5-a]pyridin-6-yl)-1H-pyrazol-3-yl)-2-(1-isopropylpiperidin-4-yl)thiazole (1072);
5-(4-isopropyl-5-(8-methyl-[1,2,4]triazolo[1,5-a]pyridin-6-yl)-1H-pyrazol-3-yl)-2-(1-(oxetan-3-yl)piperidin-4-yl)thiazole (1073);
5-(4-isopropyl-5-(8-methyl-[1,2,4]triazolo[1,5-a]pyridin-6-yl)-1H-pyrazol-3-yl)-2-(1-((3-methyloxetan-3-yl)methyl)piperidin-4-yl)thiazole (1074);
5-(4-isopropyl-5-(8-methyl-[1,2,4]triazolo[1,5-a]pyridin-6-yl)-1H-pyrazol-3-yl)-2-(1-neopentylpiperidin-4-yl)thiazole (1075);
2-(4-(5-(4-isopropyl-5-(8-methyl-[1,2,4]triazolo[1,5-a]pyridin-6-yl)-1H-pyrazol-3-yl) thiazol-2-yl)piperidin-1-yl)-N,N-dimethylacetamide (1076);
2-(4-(5-(4-isopropyl-5-(8-methoxy-[1,2,4]triazolo[1,5-a]pyridin-6-yl)-1H-pyrazol-3-yl)thiazol-2-yl)piperidin-1-yl)acetamide (1077);
2-(4-(5-(4-isopropyl-5-(8-methyl-[1,2,4]triazolo[1,5-a]pyridin-6-yl)-1H-pyrazol-3-yl) thiazol-2-yl)piperidin-1-yl)acetonitrile (1078);
1-(4-(5-(4-isopropyl-5-(8-methyl-[1,2,4]triazolo[1,5-a]pyridin-6-yl)-1H-pyrazol-3-yl) thiazol-2-yl)piperidin-1-yl)-2-methylpropan-2-ol (1079);
5-(4-isopropyl-5-(8-methyl-[1,2,4]triazolo[1,5-a]pyridin-6-yl)-1H-pyrazol-3-yl)-2-(1-(2-(methylsulfonyl)ethyl)piperidin-4-yl)thiazole (1080);
5-(4-isopropyl-5-(8-methyl-[1,2,4]triazolo[1,5-a]pyridin-6-yl)-1H-pyrazol-3-yl)-2-(1-(2-methoxyethyl)piperidin-4-yl)thiazole (1081);
2-(dimethylamino)-1-(4-(5-(4-isopropyl-5-(8-methyl-[1,2,4]triazolo[1,5-a]pyridin-6-yl)-1H-pyrazol-3-yl)thiazol-2-yl)piperidin-1-yl)ethan-1-one (1082);
4-(5-(4-isopropyl-5-(8-methyl-[1,2,4]triazolo[1,5-a]pyridin-6-yl)-1H-pyrazol-3-yl) thiazol-2-yl)-N-methylcyclohexan-1-amine (1083);
4-(5-(4-isopropyl-5-(8-methyl-[1,2,4]triazolo[1,5-a]pyridin-6-yl)-1H-pyrazol-3-yl) thiazol-2-yl)-N,N-dimethylcyclohexan-1-amine (1084-1085);
4-(5-(4-isopropyl-5-(8-methyl-[1,2,4]triazolo[1,5-a]pyridin-6-yl)-1H-pyrazol-3-yl) thiazol-2-yl)-N,N-dimethylcyclohexan-1-amine (1085);
N-(4-(5-(4-isopropyl-5-(8-methyl-[1,2,4]triazolo[1,5-a]pyridin-6-yl)-1H-pyrazol-3-yl)thiazol-2-yl)cyclohexyl)-N-methyloxetan-3-amine (1086-1087);
N-isopropyl-4-(5-(4-isopropyl-5-(8-methyl-[1,2,4]triazolo[1,5-a]pyridin-6-yl)-1H-pyrazol-3-yl)thiazol-2-yl)-N-methylcyclohexan-1-amine (1088-1089);
6-(4-isopropyl-1'-(1-methylpiperidin-4-yl)-1H,1'H-[3,4'-bipyrazol]-5-yl)-8-methyl-[1,2,4]triazolo[1,5-a]pyridine (1090);
6-(4-isopropyl-1'-(1-isopropylpiperidin-4-yl)-1H,1'H-[3,4'-bipyrazol]-5-yl)-8-methyl-[1,2,4]triazolo[1,5-a]pyridine (1091);
6-(4-isopropyl-1'-(1-(oxetan-3-yl)piperidin-4-yl)-1H,1'H-[3,4'-bipyrazol]-5-yl)-8-methyl-[1,2,4]triazolo[1,5-a]pyridine (1092);
6-(4-isopropyl-3-(4-methyl-5-(1-propylpiperidin-4-yl)pyridin-2-yl)-1H-pyrazol-5-yl)-8-methoxy-[1,2,4]triazolo[1,5-a]pyridine (1093);
6-(4-isopropyl-3-(3-methyl-5-(piperidin-4-yl)pyridin-2-yl)-1H-pyrazol-5-yl)-8-methoxy-[1,2,4]triazolo[1,5-a]pyridine (1094);
6-(3-(3-fluoro-5-(piperidin-4-yl)pyridin-2-yl)-4-isopropyl-1H-pyrazol-5-yl)-8-methoxy-[1,2,4]triazolo[1,5-a]pyridine (1095);
6-(3-(6-fluoro-5-(piperidin-4-yl)pyridin-2-yl)-4-isopropyl-1H-pyrazol-5-yl)-8-methoxy-[1,2,4]triazolo[1,5-a]pyridine (1096);
6-(4-isopropyl-3-(4-methyl-5-(1-methylpiperidin-4-yl)pyridin-2-yl)-1H-pyrazol-5-yl)-8-methoxy-[1,2,4]triazolo[1,5-a]pyridine (1097);
6-(4-isopropyl-3-(5-(1-isopropylpiperidin-4-yl)-4-methylpyridin-2-yl)-1H-pyrazol-5-yl)-8-methoxy-[1,2,4]triazolo[1,5-a]pyridine (1098);
6-(3-(6-fluoro-5-(1-methylpiperidin-4-yl)pyridin-2-yl)-4-isopropyl-1H-pyrazol-5-yl)-8-methoxy-[1,2,4]triazolo[1,5-a]pyridine (1099);
6-(3-(6-fluoro-5-(1-propylpiperidin-4-yl)pyridin-2-yl)-4-isopropyl-1H-pyrazol-5-yl)-8-methoxy-[1,2,4]triazolo[1,5-a]pyridine (1100);
6-(3-(6-fluoro-5-(1-(oxetan-3-yl)piperidin-4-yl)pyridin-2-yl)-4-isopropyl-1H-pyrazol-5-yl)-8-methoxy-[1,2,4]triazolo[1,5-a]pyridine (1101);
6-(3-(5-(1-ethylpiperidin-4-yl)-4-methylpyridin-2-yl)-4-isopropyl-1H-pyrazol-5-yl)-8-methoxy-[1,2,4]triazolo[1,5-a]pyridine (1102);
6-(3-(5-(1-isobutylpiperidin-4-yl)-4-methylpyridin-2-yl)-4-isopropyl-1H-pyrazol-5-yl)-8-methoxy-[1,2,4]triazolo[1,5-a]pyridine (1103);
6-(3-(5-(1-cyclobutylpiperidin-4-yl)-4-methylpyridin-2-yl)-4-isopropyl-1H-pyrazol-5-yl)-8-methoxy-[1,2,4]triazolo[1,5-a]pyridine (1104);
6-(3-(5-(1-(cyclobutylmethyl)piperidin-4-yl)-4-methylpyridin-2-yl)-4-isopropyl-1H-pyrazol-5-yl)-8-methoxy-[1,2,4]triazolo[1,5-a]pyridine (1105);
6-(4-isopropyl-3-(4-methyl-5-(1-(oxetan-3-yl)piperidin-4-yl)pyridin-2-yl)-1H-pyrazol-5-yl)-8-methoxy-[1,2,4]triazolo[1,5-a]pyridine (1106);
6-(4-isopropyl-3-(4-methyl-5-(1-((tetrahydro-2H-pyran-4-yl)methyl)piperidin-4-yl) pyridin-2-yl)-1H-pyrazol-5-yl)-8-methoxy-[1,2,4]triazolo[1,5-a]pyridine (1107);
6-(4-isopropyl-3-(4-methyl-5-(1-(tetrahydrofuran-3-yl)piperidin-4-yl)pyridin-2-yl)-1H-pyrazol-5-yl)-8-methoxy-[1,2,4]triazolo[1,5-a]pyridine (1108);
6-(4-isopropyl-3-(4-methyl-5-(1-((tetrahydrofuran-3-yl)methyl)piperidin-4-yl)pyridin-2-yl)-1H-pyrazol-5-yl)-8-methoxy-[1,2,4]triazolo[1,5-a]pyridine (1109);
6-(3-(5-(1-(cyclobutylmethyl)piperidin-4-yl)-3-fluoropyridin-2-yl)-4-isopropyl-1H-pyrazol-5-yl)-8-methoxy-[1,2,4]triazolo[1,5-a]pyridine (1110);
6-(3-(3-fluoro-5-(1-methylpiperidin-4-yl)pyridin-2-yl)-4-isopropyl-1H-pyrazol-5-yl)-8-methoxy-[1,2,4]triazolo[1,5-a]pyridine (1111);
6-(3-(5-(1-ethylpiperidin-4-yl)-3-fluoropyridin-2-yl)-4-isopropyl-1H-pyrazol-5-yl)-8-methoxy-[1,2,4]triazolo[1,5-a]pyridine (1112);
6-(3-(3-fluoro-5-(1-propylpiperidin-4-yl)pyridin-2-yl)-4-isopropyl-1H-pyrazol-5-yl)-8-methoxy-[1,2,4]triazolo[1,5-a]pyridine (1113);
6-(3-(3-fluoro-5-(1-isopropylpiperidin-4-yl)pyridin-2-yl)-4-isopropyl-1H-pyrazol-5-yl)-8-methoxy-[1,2,4]triazolo[1,5-a]pyridine (1114);
6-(3-(5-(1-(cyclopropylmethyl)piperidin-4-yl)-3-fluoropyridin-2-yl)-4-isopropyl-1H-pyrazol-5-yl)-8-methoxy-[1,2,4]triazolo[1,5-a]pyridine (1115);
6-(3-(3-fluoro-5-(1-isobutylpiperidin-4-yl)pyridin-2-yl)-4-isopropyl-1H-pyrazol-5-yl)-8-methoxy-[1,2,4]triazolo[1,5-a]pyridine (1116);
6-(3-(3-fluoro-5-(1-(oxetan-3-yl)piperidin-4-yl)pyridin-2-yl)-4-isopropyl-1H-pyrazol-5-yl)-8-methoxy-[1,2,4]triazolo[1,5-a]pyridine (1117);
6-(3-(3-fluoro-5-(1-(tetrahydro-2H-pyran-4-yl)piperidin-4-yl)pyridin-2-yl)-4-isopropyl-1H-pyrazol-5-yl)-8-methoxy-[1,2,4]triazolo[1,5-a]pyridine (1118);
6-(3-(3-fluoro-5-(1-((tetrahydro-2H-pyran-4-yl)methyl)piperidin-4-yl)pyridin-2-yl)-4-isopropyl-1H-pyrazol-5-yl)-8-methoxy-[1,2,4]triazolo[1,5-a]pyridine (1119);
6-(3-(3-fluoro-5-(1-(pentan-3-yl)piperidin-4-yl)pyridin-2-yl)-4-isopropyl-1H-pyrazol-5-yl)-8-methoxy-[1,2,4]triazolo[1,5-a]pyridine (1120);
6-(3-(5-(1-(2-ethylbutyl)piperidin-4-yl)-3-fluoropyridin-2-yl)-4-isopropyl-1H-pyrazol-5-yl)-8-methoxy-[1,2,4]triazolo[1,5-a]pyridine (1121);
6-(3-(3-fluoro-5-(1-neopentylpiperidin-4-yl)pyridin-2-yl)-4-isopropyl-1H-pyrazol-5-yl)-8-methoxy-[1,2,4]triazolo[1,5-a]pyridine (1123);
6-(4-isopropyl-3-(3-methyl-5-(1-methylpiperidin-4-yl)pyridin-2-yl)-1H-pyrazol-5-yl)-8-methoxy-[1,2,4]triazolo[1,5-a]pyridine (1124);
6-(3-(5-(1-ethylpiperidin-4-yl)-3-methylpyridin-2-yl)-4-isopropyl-1H-pyrazol-5-yl)-8-methoxy-[1,2,4]triazolo[1,5-a]pyridine (1125);
6-(4-isopropyl-3-(3-methyl-5-(1-propylpiperidin-4-yl)pyridin-2-yl)-1H-pyrazol-5-yl)-8-methoxy-[1,2,4]triazolo[1,5-a]pyridine (1126);
6-(4-isopropyl-3-(5-(1-isopropylpiperidin-4-yl)-3-methylpyridin-2-yl)-1H-pyrazol-5-yl)-8-methoxy-[1,2,4]triazolo[1,5-a]pyridine (1127);
6-(3-(5-(1-(cyclopropylmethyl)piperidin-4-yl)-3-methylpyridin-2-yl)-4-isopropyl-1H-pyrazol-5-yl)-8-methoxy-[1,2,4]triazolo[1,5-a]pyridine (1128);
6-(4-isopropyl-3-(3-methyl-5-(1-((tetrahydro-2H-pyran-4-yl)methyl)piperidin-4-yl) pyridin-2-yl)-1H-pyrazol-5-yl)-8-methoxy-[1,2,4]triazolo[1,5-a]pyridine (1129);
6-(3-(5-(1-ethylpiperidin-4-yl)-6-fluoropyridin-2-yl)-4-isopropyl-1H-pyrazol-5-yl)-8-methoxy-[1,2,4]triazolo[1,5-a]pyridine (1130);
6-(3-(6-fluoro-5-(1-isopropylpiperidin-4-yl)pyridin-2-yl)-4-isopropyl-1H-pyrazol-5-yl)-8-methoxy-[1,2,4]triazolo[1,5-a]pyridine (1131);
6-(3-(5-(1-cyclobutylpiperidin-4-yl)-6-fluoropyridin-2-yl)-4-isopropyl-1H-pyrazol-5-yl)-8-methoxy-[1,2,4]triazolo[1,5-a]pyridine (1132);
6-(3-(6-fluoro-5-(1-((tetrahydro-2H-pyran-4-yl)methyl)piperidin-4-yl)pyridin-2-yl)-4-isopropyl-1H-pyrazol-5-yl)-8-methoxy-[1,2,4]triazolo[1,5-a]pyridine (1133);
2-(dimethylamino)-1-(4-(6-(4-isopropyl-5-(8-methoxy-[1,2,4]triazolo[1,5-a]pyridin-6-yl)-1H-pyrazol-3-yl)-4-methylpyridin-3-yl)piperidin-1-yl)ethan-1-one (1134);
2-(dimethylamino)-1-(4-(5-fluoro-6-(4-isopropyl-5-(8-methoxy-[1,2,4]triazolo[1,5-a] pyridin-6-yl)-1H-pyrazol-3-yl)pyridin-3-yl)piperidin-1-yl)ethan-1-one (1135);
2-(4-(6-(4-isopropyl-5-(8-methoxy-[1,2,4]triazolo[1,5-a]pyridin-6-yl)-1H-pyrazol-3-yl)-4-methylpyridin-3-yl)piperidin-1-yl)-N,N-dimethylacetamide (1136);
2-(4-(5-fluoro-6-(4-isopropyl-5-(8-methoxy-[1,2,4]triazolo[1,5-a]pyridin-6-yl)-1H-pyrazol-3-yl)pyridin-3-yl)piperidin-1-yl)-N,N-dimethylacetamide (1137);
6-(3-(3-fluoro-5-(1-(2-methoxyethyl)piperidin-4-yl)pyridin-2-yl)-4-isopropyl-1H-pyrazol-5-yl)-8-methoxy-[1,2,4]triazolo[1,5-a]pyridine (1138);
6-(3-(3-fluoro-5-(1-(2-(methylsulfonyl)ethyl)piperidin-4-yl)pyridin-2-yl)-4-isopropyl-1H-pyrazol-5-yl)-8-methoxy-[1,2,4]triazolo[1,5-a]pyridine (1139);
6-(4-isopropyl-3-(4-methyl-5-(1-(2-(methylsulfonyl)ethyl)piperidin-4-yl)pyridin-2-yl)-1H-pyrazol-5-yl)-8-methoxy-[1,2,4]triazolo[1,5-a]pyridine (1140);
2-(5-(8-methoxy-[1,2,4]triazolo[1,5-a]pyridin-6-yl)-4-(2,2,2-trifluoroethyl)-1H-pyrazol-3-yl)-4-methyl-5-(1-methylpiperidin-4-yl)thiazole (1141);
2-(5-(8-methoxy-[1,2,4]triazolo[1,5-a]pyridin-6-yl)-4-(2,2,2-trifluoroethyl)-1H-pyrazol-3-yl)-5-(piperidin-4-yl)thiazole (1142);
2-(5-(8-methyl-[1,2,4]triazolo[1,5-a]pyridin-6-yl)-4-(2,2,2-trifluoroethyl)-1H-pyrazol-3-yl)-5-(piperidin-4-yl)thiazole (1143);
4-methyl-2-(5-(8-methyl-[1,2,4]triazolo[1,5-a]pyridin-6-yl)-4-(2,2,2-trifluoroethyl)-1H-pyrazol-3-yl)-5-(piperidin-4-yl)thiazole (1144);
2-(5-(7,8-dimethyl-[1,2,4]triazolo[1,5-a]pyridin-6-yl)-4-(2,2,2-trifluoroethyl)-1H-pyrazol-3-yl)-4-methyl-5-(piperidin-4-yl)thiazole (1145);
2-(5-(8-methoxy-[1,2,4]triazolo[1,5-a]pyridin-6-yl)-4-(2,2,2-trifluoroethyl)-1H-pyrazol-3-yl)-4-methyl-5-(1-propylpiperidin-4-yl)thiazole (1146);
5-(1-ethylpiperidin-4-yl)-2-(5-(8-methoxy-[1,2,4]triazolo[1,5-a]pyridin-6-yl)-4-(2,2,2-trifluoroethyl)-1H-pyrazol-3-yl)-4-methylthiazole (1147);
5-(1-isopropylpiperidin-4-yl)-2-(5-(8-methoxy-[1,2,4]triazolo[1,5-a]pyridin-6-yl)-4-(2,2,2-trifluoroethyl)-1H-pyrazol-3-yl)-4-methylthiazole (1148);
5-(1-(cyclopropylmethyl)piperidin-4-yl)-2-(5-(8-methoxy-[1,2,4]triazolo[1,5-a]pyridin-6-yl)-4-(2,2,2-trifluoroethyl)-1H-pyrazol-3-yl)-4-methylthiazole (1149);
5-(1-isobutylpiperidin-4-yl)-2-(5-(8-methoxy-[1,2,4]triazolo[1,5-a]pyridin-6-yl)-4-(2,2,2-trifluoroethyl)-1H-pyrazol-3-yl)-4-methylthiazole (1150);
2-(5-(8-methoxy-[1,2,4]triazolo[1,5-a]pyridin-6-yl)-4-(2,2,2-trifluoroethyl)-1H-pyrazol-3-yl)-4-methyl-5-(1-((tetrahydro-2H-pyran-4-yl)methyl)piperidin-4-yl)thiazole (1151);
2-(5-(8-methoxy-[1,2,4]triazolo[1,5-a]pyridin-6-yl)-4-(2,2,2-trifluoroethyl)-1H-pyrazol-3-yl)-4-methyl-5-(1-(tetrahydro-2H-pyran-4-yl)piperidin-4-yl)thiazole (1152);
2-(5-(8-methoxy-[1,2,4]triazolo[1,5-a]pyridin-6-yl)-4-(2,2,2-trifluoroethyl)-1H-pyrazol-3-yl)-4-methyl-5-(1-(oxetan-3-yl)piperidin-4-yl)thiazole (1153);
2-(5-(8-methoxy-[1,2,4]triazolo[1,5-a]pyridin-6-yl)-4-(2,2,2-trifluoroethyl)-1H-pyrazol-3-yl)-5-(1-methylpiperidin-4-yl)thiazole (1154);
5-(1-ethylpiperidin-4-yl)-2-(5-(8-methoxy-[1,2,4]triazolo[1,5-a]pyridin-6-yl)-4-(2,2,2-trifluoroethyl)-1H-pyrazol-3-yl)thiazole (1155);
2-(5-(8-methoxy-[1,2,4]triazolo[1,5-a]pyridin-6-yl)-4-(2,2,2-trifluoroethyl)-1H-pyrazol-3-yl)-5-(1-propylpiperidin-4-yl)thiazole (1156);
5-(1-isopropylpiperidin-4-yl)-2-(5-(8-methoxy-[1,2,4]triazolo[1,5-a]pyridin-6-yl)-4-(2,2,2-trifluoroethyl)-1H-pyrazol-3-yl)thiazole (1157);
2-(5-(8-methoxy-[1,2,4]triazolo[1,5-a]pyridin-6-yl)-4-(2,2,2-trifluoroethyl)-1H-pyrazol-3-yl)-5-(1-(tetrahydro-2H-pyran-4-yl)piperidin-4-yl)thiazole (1158);
5-(1-(cyclopropylmethyl)piperidin-4-yl)-2-(5-(8-methoxy-[1,2,4]triazolo[1,5-a] pyridin-6-yl)-4-(2,2,2-trifluoroethyl)-1H-pyrazol-3-yl)thiazole (1159);
2-(5-(8-methoxy-[1,2,4]triazolo[1,5-a]pyridin-6-yl)-4-(2,2,2-trifluoroethyl)-1H-pyrazol-3-yl)-5-(1-(oxetan-3-yl)piperidin-4-yl)thiazole (1160);
2-(5-(8-methoxy-[1,2,4]triazolo[1,5-a]pyridin-6-yl)-4-(2,2,2-trifluoroethyl)-1H-pyrazol-3-yl)-5-(1-((tetrahydro-2H-pyran-4-yl)methyl)piperidin-4-yl)thiazole (1161);
5-(1-isobutylpiperidin-4-yl)-2-(5-(8-methyl-[1,2,4]triazolo[1,5-a]pyridin-6-yl)-4-(2,2,2-trifluoroethyl)-1H-pyrazol-3-yl)thiazole (1162);
2-(5-(8-methyl-[1,2,4]triazolo[1,5-a]pyridin-6-yl)-4-(2,2,2-trifluoroethyl)-1H-pyrazol-3-yl)-5-(1-(tetrahydro-2H-pyran-4-yl)piperidin-4-yl)thiazole (1163);
5-(1-ethylpiperidin-4-yl)-2-(5-(8-methyl-[1,2,4]triazolo[1,5-a]pyridin-6-yl)-4-(2,2,2-trifluoroethyl)-1H-pyrazol-3-yl)thiazole (1164);
5-(1-isopropylpiperidin-4-yl)-2-(5-(8-methyl-[1,2,4]triazolo[1,5-a]pyridin-6-yl)-4-(2,2,2-trifluoroethyl)-1H-pyrazol-3-yl)thiazole (1165);
2-(5-(8-methyl-[1,2,4]triazolo[1,5-a]pyridin-6-yl)-4-(2,2,2-trifluoroethyl)-1H-pyrazol-3-yl)-5-(1-((tetrahydro-2H-pyran-4-yl)methyl)piperidin-4-yl)thiazole (1166);
2-(5-(8-methyl-[1,2,4]triazolo[1,5-a]pyridin-6-yl)-4-(2,2,2-trifluoroethyl)-1H-pyrazol-3-yl)-5-(1-(oxetan-3-yl)piperidin-4-yl)thiazole (1167);
2-(5-(8-methyl-[1,2,4]triazolo[1,5-a]pyridin-6-yl)-4-(2,2,2-trifluoroethyl)-1H-pyrazol-3-yl)-5-(1-propylpiperidin-4-yl)thiazole (1168);
2-(5-(8-methyl-[1,2,4]triazolo[1,5-a]pyridin-6-yl)-4-(2,2,2-trifluoroethyl)-1H-pyrazol-3-yl)-5-(1-(3,3,3-trifluoropropyl)piperidin-4-yl)thiazole (1169);
5-(1-(cyclopropylmethyl)piperidin-4-yl)-2-(5-(8-methyl-[1,2,4]triazolo[1,5-a]pyridin-6-yl)-4-(2,2,2-trifluoroethyl)-1H-pyrazol-3-yl)thiazole (1170);
5-(1-(cyclobutylmethyl)piperidin-4-yl)-2-(5-(8-methyl-[1,2,4]triazolo[1,5-a]pyridin-6-yl)-4-(2,2,2-trifluoroethyl)-1H-pyrazol-3-yl)thiazole (1171);
2-(5-(8-methyl-[1,2,4]triazolo[1,5-a]pyridin-6-yl)-4-(2,2,2-trifluoroethyl)-1H-pyrazol-3-yl)-5-(1-methylpiperidin-4-yl)thiazole (1172);
2-(5-(8-methyl-[1,2,4]triazolo[1,5-a]pyridin-6-yl)-4-(2,2,2-trifluoroethyl)-1H-pyrazol-3-yl)-5-(1-neopentylpiperidin-4-yl)thiazole (1173);
5-(1-ethylpiperidin-4-yl)-4-methyl-2-(5-(8-methyl-[1,2,4]triazolo[1,5-a]pyridin-6-yl)-4-(2,2,2-trifluoroethyl)-1H-pyrazol-3-yl)thiazole (1174);
4-methyl-2-(5-(8-methyl-[1,2,4]triazolo[1,5-a]pyridin-6-yl)-4-(2,2,2-trifluoroethyl)-1H-pyrazol-3-yl)-5-(1-(3,3,3-trifluoropropyl)piperidin-4-yl)thiazole (1175);
5-(1-isopropylpiperidin-4-yl)-4-methyl-2-(5-(8-methyl-[1,2,4]triazolo[1,5-a]pyridin-6-yl)-4-(2,2,2-trifluoroethyl)-1H-pyrazol-3-yl)thiazole (1176);
5-(1-(cyclohexylmethyl)piperidin-4-yl)-4-methyl-2-(5-(8-methyl-[1,2,4]triazolo[1,5-a]pyridin-6-yl)-4-(2,2,2-trifluoroethyl)-1H-pyrazol-3-yl)thiazole (1177);
5-(1-(cyclopropylmethyl)piperidin-4-yl)-4-methyl-2-(5-(8-methyl-[1,2,4]triazolo[1,5-a]pyridin-6-yl)-4-(2,2,2-trifluoroethyl)-1H-pyrazol-3-yl)thiazole (1179);
4-methyl-2-(5-(8-methyl-[1,2,4]triazolo[1,5-a]pyridin-6-yl)-4-(2,2,2-trifluoroethyl)-1H-pyrazol-3-yl)-5-(1-(oxetan-3-yl)piperidin-4-yl)thiazole (1180);
5-(1-isobutylpiperidin-4-yl)-4-methyl-2-(5-(8-methyl-[1,2,4]triazolo[1,5-a]pyridin-6-yl)-4-(2,2,2-trifluoroethyl)-1H-pyrazol-3-yl)thiazole (1181);
4-methyl-2-(5-(8-methyl-[1,2,4]triazolo[1,5-a]pyridin-6-yl)-4-(2,2,2-trifluoroethyl)-1H-pyrazol-3-yl)-5-(1-methylpiperidin-4-yl)thiazole (1182);
4-methyl-2-(5-(8-methyl-[1,2,4]triazolo[1,5-a]pyridin-6-yl)-4-(2,2,2-trifluoroethyl)-1H-pyrazol-3-yl)-5-(1-propylpiperidin-4-yl)thiazole (1183);
4-methyl-2-(5-(8-methyl-[1,2,4]triazolo[1,5-a]pyridin-6-yl)-4-(2,2,2-trifluoroethyl)-1H-pyrazol-3-yl)-5-(1-(tetrahydro-2H-pyran-4-yl)piperidin-4-yl)thiazole (1184);
4-methyl-2-(5-(8-methyl-[1,2,4]triazolo[1,5-a]pyridin-6-yl)-4-(2,2,2-trifluoroethyl)-1H-pyrazol-3-yl)-5-(1-((tetrahydro-2H-pyran-4-yl)methyl)piperidin-4-yl)thiazole (1185);
2-(5-(7,8-dimethyl-[1,2,4]triazolo[1,5-a]pyridin-6-yl)-4-(2,2,2-trifluoroethyl)-1H-pyrazol-3-yl)-4-methyl-5-(1-((tetrahydro-2H-pyran-4-yl)methyl)piperidin-4-yl)thiazole (1186);
2-(5-(7,8-dimethyl-[1,2,4]triazolo[1,5-a]pyridin-6-yl)-4-(2,2,2-trifluoroethyl)-1H-pyrazol-3-yl)-5-(1-isopropylpiperidin-4-yl)-4-methylthiazole (1187);
5-(1-(cyclopropylmethyl)piperidin-4-yl)-2-(5-(7,8-dimethyl-[1,2,4]triazolo[1,5-a] pyridin-6-yl)-4-(2,2,2-trifluoroethyl)-1H-pyrazol-3-yl)-4-methylthiazole (1188);
2-(5-(7,8-dimethyl-[1,2,4]triazolo[1,5-a]pyridin-6-yl)-4-(2,2,2-trifluoroethyl)-1H-pyrazol-3-yl)-4-methyl-5-(1-methylpiperidin-4-yl)thiazole (1189);
2-(5-(7,8-dimethyl-[1,2,4]triazolo[1,5-a]pyridin-6-yl)-4-(2,2,2-trifluoroethyl)-1H-pyrazol-3-yl)-5-(1-ethylpiperidin-4-yl)-4-methylthiazole (1190);
2-(5-(7,8-dimethyl-[1,2,4]triazolo[1,5-a]pyridin-6-yl)-4-(2,2,2-trifluoroethyl)-1H-pyrazol-3-yl)-4-methyl-5-(1-propylpiperidin-4-yl)thiazole (1191);
2-(dimethylamino)-1-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)ethan-1-one (1192);
2-(dimethylamino)-1-(4-(2-(5-(8-methyl-[1,2,4]triazolo[1,5-a]pyridin-6-yl)-4-(2,2,2-trifluoroethyl)-1H-pyrazol-3-yl)thiazol-5-yl)piperidin-1-yl)ethan-1-one (1193);
1-(4-(2-(5-(8-methyl-[1,2,4]triazolo[1,5-a]pyridin-6-yl)-4-(2,2,2-trifluoroethyl)-1H-pyrazol-3-yl)thiazol-5-yl)piperidin-1-yl)-2-morpholinoethan-1-one (1194);
2-(diethylamino)-1-(4-(2-(5-(8-methyl-[1,2,4]triazolo[1,2-a]pyridin-6-yl)-4-(2,2,2-trifluoroethyl)-1H-pyrazol-3-yl)thiazol-5-yl)piperidin-1-yl)ethan-1-one (1195);
2-(5-(8-methoxy-[1,2,4]triazolo[1,5-a]pyridin-6-yl)-4-(2,2,2-trifluoroethyl)-1H-pyrazol-3-yl)-5-(1-(2-methoxyethyl)piperidin-4-yl)thiazole (1196);
5-(1-(2-methoxyethyl)piperidin-4-yl)-2-(5-(8-methyl-[1,2,4]triazolo[1,5-a]pyridin-6-yl)-4-(2,2,2-trifluoroethyl)-1H-pyrazol-3-yl)thiazole (1197);
2-methyl-1-(4-(2-(5-(8-methyl-[1,2,4]triazolo[1,5-a]pyridin-6-yl)-4-(2,2,2-trifluoroethyl)-1H-pyrazol-3-yl)thiazol-5-yl)piperidin-1-yl)propan-2-ol (1198);
2-methyl-1-(4-(4-methyl-2-(5-(8-methyl-[1,2,4]triazolo[1,5-a]pyridin-6-yl)-4-(2,2,2-trifluoroethyl)-1H-pyrazol-3-yl)thiazol-5-yl)piperidin-1-yl)propan-2-ol (1199);
2-(5-(8-methoxy-[1,2,4]triazolo[1,5-a]pyridin-6-yl)-4-(2,2,2-trifluoroethyl)-1H-pyrazol-3-yl)-5-(1-(2-(methylsulfonyl)ethyl)piperidin-4-yl)thiazole (1200);
2-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)-N,N-dimethylacetamide (1201);
5-(1-(2-(tert-butoxy)ethyl)piperidin-4-yl)-2-(5-(8-methoxy-[1,2,4]triazolo[1,5-a] pyridin-6-yl)-4-(2,2,2-trifluoroethyl)-1H-pyrazol-3-yl)thiazole (1202);
2-(4-(2-(5-(8-methyl-[1,2,4]triazolo[1,5-a]pyridin-6-yl)-4-(2,2,2-trifluoroethyl)-1H-pyrazol-3-yl)thiazol-5-yl)piperidin-1-yl)-1-morpholinoethan-1-one (1203);
N,N-dimethyl-2-(4-(2-(5-(8-methyl-[1,2,4]triazolo[1,5-a]pyridin-6-yl)-4-(2,2,2-trifluoroethyl)-1H-pyrazol-3-yl)thiazol-5-yl)piperidin-1-yl)acetamide (1204);
5-(1-(2-fluoroethyl)piperidin-4-yl)-2-(5-(8-methyl-[1,2,4]triazolo[1,5-a]pyridin-6-yl)-4-(2,2,2-trifluoroethyl)-1H-pyrazol-3-yl)thiazole (1205);
5-(1-(3-fluoropropyl)piperidin-4-yl)-2-(5-(8-methyl-[1,2,4]triazolo[1,5-a]pyridin-6-yl)-4-(2,2,2-trifluoroethyl)-1H-pyrazol-3-yl)thiazole (1206);
2-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)-1-(2-oxa-6-azaspiro[3.3] heptan-6-yl)ethan-1-one (1207);
N-isopropyl-2-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)-N-methylacetamide (1208);
1-(azetidin-1-yl)-2-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)ethan-1-one (1209);
1-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)-2-morpholinoethan-1-one (1210);
1-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)-2-(7-oxa-2-azaspiro[3.5]nonan-2-yl)ethan-1-one (1211);
4-(2-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)-2-oxoethyl)piperazin-2-one (1212);
1-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)-2-(3-methoxyazetidin-1-yl)ethan-1-one (1213);
1-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)piperidin-1-yl)-2-((2-methoxyethyl)(methyl)amino)ethan-1-one (1214);
4-(4-(2-(5-(8-methoxy-[1,2,4]triazolo[1,5-a]pyridin-6-yl)-4-(2,2,2-trifluoroethyl)-1H-pyrazol-3-yl)-4-methylthiazol-5-yl)cyclohexyl)morpholine (1215);
N-(4-(2-(5-(8-methoxy-[1,2,4]triazolo[1,5-a]pyridin-6-yl)-4-(2,2,2-trifluoroethyl)-1H-pyrazol-3-yl)-4-methylthiazol-5-yl)cyclohexyl)oxetan-3-amine (1216-1217);
6-(4-(2-(5-(8-methoxy-[1,2,4]triazolo[1,5-a]pyridin-6-yl)-4-(2,2,2-trifluoroethyl)-1H-pyrazol-3-yl)-4-methylthiazol-5-yl)cyclohexyl)-2-oxa-6-azaspiro[3.3]heptane (1218-1219);
2-(4-(2-(5-(8-methoxy-[1,2,4]triazolo[1,5-a]pyridin-6-yl)-4-(2,2,2-trifluoroethyl)-1H-pyrazol-3-yl)-4-methylthiazol-5-yl)cyclohexyl)-7-oxa-2-azaspiro[3.5]nonane (1220-1221);
4-(4-(2-(5-(8-methoxy-[1,2,4]triazolo[1,5-a]pyridin-6-yl)-4-(2,2,2-trifluoroethyl)-1H-pyrazol-3-yl)-4-methylthiazol-5-yl)cyclohexyl)piperazin-2-one (1222-1223);
2-(5-(8-methoxy-[1,2,4]triazolo[1,5-a]pyridin-6-yl)-4-(2,2,2-trifluoroethyl)-1H-pyrazol-3-yl)-5-(4-(3-methoxyazetidin-1-yl)cyclohexyl)thiazole (1224-1225);
2-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)cyclohexyl)-7-oxa-2-azaspiro[3.5]nonane (1226-1227);
4-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)cyclohexyl)piperazin-2-one (1228-1229);
4-(4-(2-(5-(8-methoxy-[1,2,4]triazolo[1,5-a]pyridin-6-yl)-4-(2,2,2-trifluoroethyl)-1H-pyrazol-3-yl)thiazol-5-yl)cyclohexyl)morpholine (1230-1231);
2-(5-(8-methoxy-[1,2,4]triazolo[1,5-a]pyridin-6-yl)-4-(2,2,2-trifluoroethyl)-1H-pyrazol-3-yl)-4-methyl-5-((1S,4S)-5-methyl-2,5-diazabicyclo[2.2.1]heptan-2-yl)thiazole (1232);
2-(5-(8-methoxy-[1,2,4]triazolo[1,5-a]pyridin-6-yl)-4-(2,2,2-trifluoroethyl)-1H-pyrazol-3-yl)-4-methyl-5-(piperazin-1-yl)thiazole (1233);
4-methyl-2-(5-(8-methyl-[1,2,4]triazolo[1,5-a]pyridin-6-yl)-4-(2,2,2-trifluoroethyl)-1H-pyrazol-3-yl)-5-(piperazin-1-yl)thiazole (1234);
5-((2R,5S)-2,5-dimethylpiperazin-1-yl)-2-(5-(8-methoxy-[1,2,4]triazolo[1,5-a] pyridin-6-yl)-4-(2,2,2-trifluoroethyl)-1H-pyrazol-3-yl)thiazole (1235);
2-(5-(8-methoxy-[1,2,4]triazolo[1,5-a]pyridin-6-yl)-4-(2,2,2-trifluoroethyl)-1H-pyrazol-3-yl)-4-methyl-5-(2,6-diazaspiro[3.3]heptan-2-yl)thiazole (1236);
5-((1S,4S)-5-ethyl-2,5-diazabicyclo[2.2.1]heptan-2-yl)-2-(5-(8-methoxy-[1,2,4]triazolo[1,5-a]pyridin-6-yl)-4-(2,2,2-trifluoroethyl)-1H-pyrazol-3-yl)-4-methylthiazole (1237);
2-(5-(8-methoxy-[1,2,4]triazolo[1,5-a]pyridin-6-yl)-4-(2,2,2-trifluoroethyl)-1H-pyrazol-3-yl)-4-methyl-5-((1R,4R)-5-propyl-2,5-diazabicyclo[2.2.1]heptan-2-yl)thiazole (1238);
2-(5-(8-methoxy-[1,2,4]triazolo[1,5-a]pyridin-6-yl)-4-(2,2,2-trifluoroethyl)-1H-pyrazol-3-yl)-4-methyl-5-((1S,4S)-5-(oxetan-3-yl)-2,5-diazabicyclo[2.2.1]heptan-2-yl) thiazole (1239);
2-(5-(8-methoxy-[1,2,4]triazolo[1,5-a]pyridin-6-yl)-4-(2,2,2-trifluoroethyl)-1H-pyrazol-3-yl)-4-methyl-5-((1S,4S)-5-((tetrahydro-2H-pyran-4-yl)methyl)-2,5-diazabicyclo[2.2.1]heptan-2-yl)thiazole (1240);
5-((1S,4S)-5-isopropyl-2,5-diazabicyclo[2.2.1]heptan-2-yl)-2-(5-(8-methoxy-[1,2,4]triazolo[1,5-a]pyridin-6-yl)-4-(2,2,2-trifluoroethyl)-1H-pyrazol-3-yl)-4-methylthiazole (1241);
2-(5-(8-methoxy-[1,2,4]triazolo[1,5-a]pyridin-6-yl)-4-(2,2,2-trifluoroethyl)-1H-pyrazol-3-yl)-4-methyl-5-((1S,4S)-5-(tetrahydro-2H-pyran-4-yl)-2,5-diazabicyclo[2.2.1]heptan-2-yl)thiazole (1242);
5-((1S,4S)-5-isobutyl-2,5-diazabicyclo[2.2.1]heptan-2-yl)-2-(5-(8-methoxy-[1,2,4]triazolo[1,5-a]pyridin-6-yl)-4-(2,2,2-trifluoroethyl)-1H-pyrazol-3-yl)-4-methylthiazole (1243);
2-(5-(8-methoxy-[1,2,4]triazolo[1,5-a]pyridin-6-yl)-4-(2,2,2-trifluoroethyl)-1H-pyrazol-3-yl)-4-methyl-5-(4-methylpiperazin-1-yl)thiazole (1244);
2-(5-(8-methoxy-[1,2,4]triazolo[1,5-a]pyridin-6-yl)-4-(2,2,2-trifluoroethyl)-1H-pyrazol-3-yl)-4-methyl-5-(4-((tetrahydro-2H-pyran-4-yl)methyl)piperazin-1-yl)thiazole (1245);
2-(5-(8-methoxy-[1,2,4]triazolo[1,5-a]pyridin-6-yl)-4-(2,2,2-trifluoroethyl)-1H-pyrazol-3-yl)-4-methyl-5-(4-propylpiperazin-1-yl)thiazole (1246);
5-(4-ethylpiperazin-1-yl)-2-(5-(8-methoxy-[1,2,4]triazolo[1,5-a]pyridin-6-yl)-4-(2,2,2-trifluoroethyl)-1H-pyrazol-3-yl)-4-methylthiazole (1247);
5-(4-isobutylpiperazin-1-yl)-2-(5-(8-methoxy-[1,2,4]triazolo[1,5-a]pyridin-6-yl)-4-(2,2,2-trifluoroethyl)-1H-pyrazol-3-yl)-4-methylthiazole (1248);
4-methyl-2-(5-(8-methyl-[1,2,4]triazolo[1,5-a]pyridin-6-yl)-4-(2,2,2-trifluoroethyl)-1H-pyrazol-3-yl)-5-(4-(oxetan-3-yl)piperazin-1-yl)thiazole (1249);
4-methyl-2-(5-(8-methyl-[1,2,4]triazolo[1,5-a]pyridin-6-yl)-4-(2,2,2-trifluoroethyl)-1H-pyrazol-3-yl)-5-(4-methylpiperazin-1-yl)thiazole (1250);
5-(4-ethylpiperazin-1-yl)-4-methyl-2-(5-(8-methyl-[1,2,4]triazolo[1,5-a]pyridin-6-yl)-4-(2,2,2-trifluoroethyl)-1H-pyrazol-3-yl)thiazole (1251);
4-methyl-2-(5-(8-methyl-[1,2,4]triazolo[1,5-a]pyridin-6-yl)-4-(2,2,2-trifluoroethyl)-1H-pyrazol-3-yl)-5-(4-propylpiperazin-1-yl)thiazole (1252);
4-methyl-2-(5-(8-methyl-[1,2,4]triazolo[1,5-a]pyridin-6-yl)-4-(2,2,2-trifluoroethyl)-1H-pyrazol-3-yl)-5-(4-((tetrahydro-2H-pyran-4-yl)methyl)piperazin-1-yl)thiazole (1253);
4-methyl-2-(5-(8-methyl-[1,2,4]triazolo[1,5-a]pyridin-6-yl)-4-(2,2,2-trifluoroethyl)-1H-pyrazol-3-yl)-5-(4-(tetrahydro-2H-pyran-4-yl)piperazin-1-yl)thiazole (1254);
5-(4-(cyclopropylmethyl)piperazin-1-yl)-4-methyl-2-(5-(8-methyl-[1,2,4]triazolo[1,5-a]pyridin-6-yl)-4-(2,2,2-trifluoroethyl)-1H-pyrazol-3-yl)thiazole (1256);
2-(5-(8-methoxy-[1,2,4]triazolo[1,5-a]pyridin-6-yl)-4-(2,2,2-trifluoroethyl)-1H-pyrazol-3-yl)-5-((2R,5S)-2,4,5-trimethylpiperazin-1-yl)thiazole (1257);
5-((2R,5S)-4-ethyl-2,5-dimethylpiperazin-1-yl)-2-(5-(8-methoxy-[1,2,4]triazolo[1,5-a]pyridin-6-yl)-4-(2,2,2-trifluoroethyl)-1H-pyrazol-3-yl)thiazole (1258);
5-((2R,5S)-2,5-dimethyl-4-propylpiperazin-1-yl)-2-(5-(8-methoxy-[1,2,4]triazolo[1,5-a]pyridin-6-yl)-4-(2,2,2-trifluoroethyl)-1H-pyrazol-3-yl)thiazole (1259);
5-((2R,5S)-4-isopropyl-2,5-dimethylpiperazin-1-yl)-2-(5-(8-methoxy-[1,2,4]triazolo[1,5-a]pyridin-6-yl)-4-(2,2,2-trifluoroethyl)-1H-pyrazol-3-yl)thiazole (1260);
2-(5-(8-methoxy-[1,2,4]triazolo[1,5-a]pyridin-6-yl)-4-(2,2,2-trifluoroethyl)-1H-pyrazol-3-yl)-4-methyl-5-(6-methyl-2,6-diazaspiro[3.3]heptan-2-yl)thiazole (1261);
5-(6-isobutyl-2,6-diazaspiro[3.3]heptan-2-yl)-2-(5-(8-methoxy-[1,2,4]triazolo[1,5-a] pyridin-6-yl)-4-(2,2,2-trifluoroethyl)-1H-pyrazol-3-yl)-4-methylthiazole (1262);
2-(5-(8-methoxy-[1,2,4]triazolo[1,5-a]pyridin-6-yl)-4-(2,2,2-trifluoroethyl)-1H-pyrazol-3-yl)-4-methyl-5-(6-(tetrahydro-2H-pyran-4-yl)-2,6-diazaspiro[3.3]heptan-2-yl) thiazole (1263);
5-(6-(cyclopropylmethyl)-2,6-diazaspiro[3.3]heptan-2-yl)-2-(5-(8-methoxy-[1,2,4]triazolo[1,5-a]pyridin-6-yl)-4-(2,2,2-trifluoroethyl)-1H-pyrazol-3-yl)-4-methylthiazole (1264);
5-(6-(1-isopropylpiperidin-4-yl)-2,6-diazaspiro[3.3]heptan-2-yl)-2-(5-(8-methoxy-[1,2,4]triazolo[1,5-a]pyridin-6-yl)-4-(2,2,2-trifluoroethyl)-1H-pyrazol-3-yl)-4-methylthiazole (1265);
5-(6-isopropyl-2,6-diazaspiro[3.3]heptan-2-yl)-2-(5-(8-methoxy-[1,2,4]triazolo[1,5-a] pyridin-6-yl)-4-(2,2,2-trifluoroethyl)-1H-pyrazol-3-yl)-4-methylthiazole (1266);
5-(6-(1-cyclopropylethyl)-2,6-diazaspiro[3.3]heptan-2-yl)-2-(5-(8-methoxy-[1,2,4]triazolo[1,5-a]pyridin-6-yl)-4-(2,2,2-trifluoroethyl)-1H-pyrazol-3-yl)-4-methylthiazole (1267);
2-(5-(8-methoxy-[1,2,4]triazolo[1,5-a]pyridin-6-yl)-4-(2,2,2-trifluoroethyl)-1H-pyrazol-3-yl)-4-methyl-5-(6-(pentan-3-yl)-2,6-diazaspiro[3.3]heptan-2-yl)thiazole (1268);
1-(2-(4-isopropyl-5-(8-methoxy-[1,2,4]triazolo[1,5-a]pyridin-6-yl)-1H-pyrazol-3-yl) thiazol-5-yl)-N-(3-methyloxetan-3-yl)piperidin-4-amine (1269);
1-(2-(4-isopropyl-5-(8-methoxy-[1,2,4]triazolo[1,5-a]pyridin-6-yl)-1H-pyrazol-3-yl) thiazol-5-yl)-N-(2-methoxy-2-methylpropyl)piperidin-4-amine (1270);
N-ethyl-1-(2-(4-isopropyl-5-(8-methoxy-[1,2,4]triazolo[1,5-a]pyridin-6-yl)-1H-pyrazol-3-yl)thiazol-5-yl)-N-methylpiperidin-4-amine (1271);
2-(5-(8-methoxy-[1,2,4]triazolo[1,5-a]pyridin-6-yl)-4-(2,2,2-trifluoroethyl)-1H-pyrazol-3-yl)-N,N-dimethyl-4,5,6,7-tetrahydrobenzo[d]thiazol-6-amine (1272);
N-isopropyl-2-(5-(8-methoxy-[1,2,4]triazolo[1,5-a]pyridin-6-yl)-4-(2,2,2-trifluoroethyl)-1H-pyrazol-3-yl)-N-methyl-4,5,6,7-tetrahydrobenzo[d]thiazol-6-amine (1273);
2-(5-(8-methoxy-[1,2,4]triazolo[1,5-a]pyridin-6-yl)-4-(2,2,2-trifluoroethyl)-1H-pyrazol-3-yl)-N,N-dimethyl-4,5,6,7-tetrahydrobenzo[d]thiazol-6-amine (1274);
8-methoxy-6-(3-(4-methyl-5-(1-methylpiperidin-4-yl)pyridin-2-yl)-4-(2,2,2-trifluoroethyl)-1H-pyrazol-5-yl)-[1,2,4]triazolo[1,5-a]pyridine (1275);
8-methoxy-6-(3-(5-(piperidin-4-yl)pyridin-2-yl)-4-(2,2,2-trifluoroethyl)-1H-pyrazol-5-yl)-[1,2,4]triazolo[1,5-a]pyridine (1276);
6-(3-(5-(1-ethylpiperidin-4-yl)-4-methylpyridin-2-yl)-4-(2,2,2-trifluoroethyl)-1H-pyrazol-5-yl)-8-methoxy-[1,2,4]triazolo[1,5-a]pyridine (1277);
8-methoxy-6-(3-(4-methyl-5-(1-propylpiperidin-4-yl)pyridin-2-yl)-4-(2,2,2-trifluoroethyl)-1H-pyrazol-5-yl)-[1,2,4]triazolo[1,5-a]pyridine (1278);
8-methoxy-6-(3-(5-(1-(oxetan-3-yl)piperidin-4-yl)-4-(trifluoromethyl)pyridin-2-yl)-4-(2,2,2-trifluoroethyl)-1H-pyrazol-5-yl)-[1,2,4]triazolo[1,5-a]pyridine (1279);
8-methoxy-6-(3-(4-methyl-5-(1-(tetrahydro-2H-pyran-4-yl)piperidin-4-yl)pyridin-2-yl)-4-(2,2,2-trifluoroethyl)-1H-pyrazol-5-yl)-[1,2,4]triazolo[1,5-a]pyridine (1280);
8-methoxy-6-(3-(4-methyl-5-(1-((tetrahydro-2H-pyran-4-yl)methyl)piperidin-4-yl) pyridin-2-yl)-4-(2,2,2-trifluoroethyl)-1H-pyrazol-5-yl)-[1,2,4]triazolo[1,5-a]pyridine (1281);
6-(3-(5-(1-isopropylpiperidin-4-yl)-4-methylpyridin-2-yl)-4-(2,2,2-trifluoroethyl)-1H-pyrazol-5-yl)-8-methoxy-[1,2,4]triazolo[1,5-a]pyridine (1282);
6-(3-(5-(1-(2-ethylbutyl)piperidin-4-yl)-4-methylpyridin-2-yl)-4-(2,2,2-trifluoroethyl)-1H-pyrazol-5-yl)-8-methoxy-[1,2,4]triazolo[1,5-a]pyridine (1283);
6-(3-(5-(1-isobutylpiperidin-4-yl)-4-methylpyridin-2-yl)-4-(2,2,2-trifluoroethyl)-1H-pyrazol-5-yl)-8-methoxy-[1,2,4]triazolo[1,5-a]pyridine (1284);
6-(3-(5-(1-(cyclobutylmethyl)piperidin-4-yl)-4-methylpyridin-2-yl)-4-(2,2,2-trifluoroethyl)-1H-pyrazol-5-yl)-8-methoxy-[1,2,4]triazolo[1,5-a]pyridine (1285);
6-(3-(5-(1-ethylpiperidin-4-yl)pyridin-2-yl)-4-(2,2,2-trifluoroethyl)-1H-pyrazol-5-yl)-8-methoxy-[1,2,4]triazolo[1,5-a]pyridine (1286);
8-methoxy-6-(3-(5-(1-methylpiperidin-4-yl)pyridin-2-yl)-4-(2,2,2-trifluoroethyl)-1H-pyrazol-5-yl)-[1,2,4]triazolo[1,5-a]pyridine (1287);
6-(3-(5-(1-isobutylpiperidin-4-yl)pyridin-2-yl)-4-(2,2,2-trifluoroethyl)-1H-pyrazol-5-yl)-8-methoxy-[1,2,4]triazolo[1,5-a]pyridine (1288);
6-(3-(5-(1-cyclobutylpiperidin-4-yl)pyridin-2-yl)-4-(2,2,2-trifluoroethyl)-1H-pyrazol-5-yl)-8-methoxy-[1,2,4]triazolo[1,5-a]pyridine (1289);
6-(3-(5-(1-isopropylpiperidin-4-yl)pyridin-2-yl)-4-(2,2,2-trifluoroethyl)-1H-pyrazol-5-yl)-8-methoxy-[1,2,4]triazolo[1,5-a]pyridine (1290);
8-methoxy-6-(3-(5-(1-propylpiperidin-4-yl)pyridin-2-yl)-4-(2,2,2-trifluoroethyl)-1H-pyrazol-5-yl)-[1,2,4]triazolo[1,5-a]pyridine (1291);
8-methoxy-6-(3-(5-(1-(oxetan-3-yl)piperidin-4-yl)pyridin-2-yl)-4-(2,2,2-trifluoroethyl)-1H-pyrazol-5-yl)-[1,2,4]triazolo[1,5-a]pyridine (1292);
8-methoxy-6-(3-(5-(1-(tetrahydro-2H-pyran-4-yl)piperidin-4-yl)pyridin-2-yl)-4-(2,2,2-trifluoroethyl)-1H-pyrazol-5-yl)-[1,2,4]triazolo[1,5-a]pyridine (1293);
8-methoxy-6-(3-(5-(1-((tetrahydro-2H-pyran-4-yl)methyl)piperidin-4-yl)pyridin-2-yl)-4-(2,2,2-trifluoroethyl)-1H-pyrazol-5-yl)-[1,2,4]triazolo[1,5-a]pyridine (1294);
6-(3-(5-(1-(cyclobutylmethyl)piperidin-4-yl)pyridin-2-yl)-4-(2,2,2-trifluoroethyl)-1H-pyrazol-5-yl)-8-methoxy-[1,2,4]triazolo[1,5-a]pyridine (1295);
2-(dimethylamino)-1-(4-(6-(5-(8-methoxy-[1,2,4]triazolo[1,5-a]pyridin-6-yl)-4-(2,2,2-trifluoroethyl)-1H-pyrazol-3-yl)-4-methylpyridin-3-yl)piperidin-1-yl)ethan-1-one (1296);
2-(dimethylamino)-1-(4-(6-(5-(8-methoxy-[1,2,4]triazolo[1,5-a]pyridin-6-yl)-4-(2,2,2-trifluoroethyl)-1H-pyrazol-3-yl)pyridin-3-yl)piperidin-1-yl)ethan-1-one (1297);
2-(diethylamino)-1-(4-(6-(5-(8-methoxy-[1,2,4]triazolo[1,5-a]pyridin-6-yl)-4-(2,2,2-trifluoroethyl)-1H-pyrazol-3-yl)pyridin-3-yl)piperidin-1-yl)ethan-1-one (1298);
2-(4-(6-(5-(8-methoxy-[1,2,4]triazolo[1,5-a]pyridin-6-yl)-4-(2,2,2-trifluoroethyl)-1H-pyrazol-3-yl)-4-methylpyridin-3-yl)piperidin-1-yl)-N,N-dimethylacetamide (1299);
2-(4-(6-(5-(8-methoxy-[1,2,4]triazolo[1,5-a]pyridin-6-yl)-4-(2,2,2-trifluoroethyl)-1H-pyrazol-3-yl)-4-methylpyridin-3-yl)piperidin-1-yl)-N-methylacetamide (1300);
8-methoxy-6-(3-(5-(1-(2-methoxyethyl)piperidin-4-yl)-4-methylpyridin-2-yl)-4-(2,2,2-trifluoroethyl)-1H-pyrazol-5-yl)-[1,2,4]triazolo[1,5-a]pyridine (1301);
8-methoxy-6-(3-(4-methyl-5-(1-(2-(methylsulfonyl)ethyl)piperidin-4-yl)pyridin-2-yl)-4-(2,2,2-trifluoroethyl)-1H-pyrazol-5-yl)-[1,2,4]triazolo[1,5-a]pyridine (1302);
N-(2-(4-(5-(8-methyl-[1,2,4]triazolo[1,5-a]pyridin-6-yl)-4-(2,2,2-trifluoroethyl)-1H-pyrazol-3-yl)phenyl)propan-2-yl)tetrahydro-2H-pyran-4-amine (1303);
(S)-1-(4-(5-(8-methoxy-[1,2,4]triazolo[1,5-a]pyridin-6-yl)-4-(2,2,2-trifluoroethyl)-1H-pyrazol-3-yl)phenyl)-N-methylethan-1-amine (1304B);
6-(5-(8-methoxy-[1,2,4]triazolo[1,5-a]pyridin-6-yl)-4-(2,2,2-trifluoroethyl)-1H-pyrazol-3-yl)-N-methyl-1,2,3,4-tetrahydronaphthalen-2-amine (1305B);
6-(5-(8-methoxy-[1,2,4]triazolo[1,5-a]pyridin-6-yl)-4-(2,2,2-trifluoroethyl)-1H-pyrazol-3-yl)-N-methyl-1,2,3,4-tetrahydronaphthalen-2-amine (1306B);
6-(5-(8-methoxy-[1,2,4]triazolo[1,5-a]pyridin-6-yl)-4-(2,2,2-trifluoroethyl)-1H-pyrazol-3-yl)-N-methyl-1,2,3,4-tetrahydronaphthalen-2-amine (1307B);
N,N-dimethyl-2-(4-(5-(8-methyl-[1,2,4]triazolo[1,5-a]pyridin-6-yl)-4-(2,2,2-trifluoroethyl)-1H-pyrazol-3-yl)phenyl)propan-2-amine (1308);
(S)-1-(4-(5-(8-methoxy-[1,2,4]triazolo[1,5-a]pyridin-6-yl)-4-(2,2,2-trifluoroethyl)-1H-pyrazol-3-yl)phenyl)-N,N-dimethylethan-1-amine (1309);
6-(5-(8-methoxy-[1,2,4]triazolo[1,5-a]pyridin-6-yl)-4-(2,2,2-trifluoroethyl)-1H-pyrazol-3-yl)-N,N-dimethyl-1,2,3,4-tetrahydronaphthalen-2-amine (1310);
N-isopropyl-6-(5-(8-methoxy-[1,2,4]triazolo[1,5-a]pyridin-6-yl)-4-(2,2,2-trifluoroethyl)-1H-pyrazol-3-yl)-N-methyl-1,2,3,4-tetrahydronaphthalen-2-amine (1311);
N-(6-(5-(8-methoxy-[1,2,4]triazolo[1,5-a]pyridin-6-yl)-4-(2,2,2-trifluoroethyl)-1H-pyrazol-3-yl)-1,2,3,4-tetrahydronaphthalen-2-yl)-N-methyloxetan-3-amine (1312);
2-(dimethylamino)-N-(2-(4-(5-(8-methyl-[1,2,4]triazolo[1,5-a]pyridin-6-yl)-4-(2,2,2-trifluoroethyl)-1H-pyrazol-3-yl)phenyl)propan-2-yl)acetamide (1313);
(S)-2-(dimethylamino)-N-(1-(4-(5-(8-methoxy-[1,2,4]triazolo[1,5-a]pyridin-6-yl)-4-(2,2,2-trifluoroethyl)-1H-pyrazol-3-yl)phenyl)ethyl)-N-methylacetamide (1314);
2-(dimethylamino)-N-(6-(5-(8-methoxy-[1,2,4]triazolo[1,5-a]pyridin-6-yl)-4-(2,2,2-trifluoroethyl)-1H-pyrazol-3-yl)-1,2,3,4-tetrahydronaphthalen-2-yl)-N-methylacetamide (1315);
8-methoxy-6-(3-(5-(6-((tetrahydro-2H-pyran-4-yl)methyl)-2,6-diazaspiro[3.3]heptan-2-yl)pyridin-2-yl)-4-(2,2,2-trifluoroethyl)-1H-pyrazol-5-yl)-[1,2,4]triazolo[1,5-a]pyridine (1316);
8-methoxy-6-(3-(5-(6-(oxetan-3-yl)-2,6-diazaspiro[3.3]heptan-2-yl)pyridin-2-yl)-4-(2,2,2-trifluoroethyl)-1H-pyrazol-5-yl)-[1,2,4]triazolo[1,5-a]pyridine (1317);
6-(3-(5-(6-isopropyl-2,6-diazaspiro[3.3]heptan-2-yl)pyridin-2-yl)-4-(2,2,2-trifluoroethyl)-1H-pyrazol-5-yl)-8-methoxy-[1,2,4]triazolo[1,5-a]pyridine (1318);
8-methoxy-6-(3-(5-(6-propyl-2,6-diazaspiro[3.3]heptan-2-yl)pyridin-2-yl)-4-(2,2,2-trifluoroethyl)-1H-pyrazol-5-yl)-[1,2,4]triazolo[1,5-a]pyridine (1319);
6-(3-(5-(6-ethyl-2,6-diazaspiro[3.3]heptan-2-yl)pyridin-2-yl)-4-(2,2,2-trifluoroethyl)-1H-pyrazol-5-yl)-8-methoxy-[1,2,4]triazolo[1,5-a]pyridine (1320);
8-methoxy-6-(3-(5-(6-methyl-2,6-diazaspiro[3.3]heptan-2-yl)pyridin-2-yl)-4-(2,2,2-trifluoroethyl)-1H-pyrazol-5-yl)-[1,2,4]triazolo[1,5-a]pyridine (1321);
6-(3-(5-(6-cyclobutyl-2,6-diazaspiro[3.3]heptan-2-yl)pyridin-2-yl)-4-(2,2,2-trifluoroethyl)-1H-pyrazol-5-yl)-8-methoxy-[1,2,4]triazolo[1,5-a]pyridine (1322);
6-(3-(5-(6-isobutyl-2,6-diazaspiro[3.3]heptan-2-yl)pyridin-2-yl)-4-(2,2,2-trifluoroethyl)-1H-pyrazol-5-yl)-8-methoxy-[1,2,4]triazolo[1,5-a]pyridine (1323);
8-methoxy-6-(3-(5-(6-(tetrahydro-2H-pyran-4-yl)-2,6-diazaspiro[3.3]heptan-2-yl) pyridin-2-yl)-4-(2,2,2-trifluoroethyl)-1H-pyrazol-5-yl)-[1,2,4]triazolo[1,5-a]pyridine (1324);
6-(3-(5-(6-(cyclobutylmethyl)-2,6-diazaspiro[3.3]heptan-2-yl)pyridin-2-yl)-4-(2,2,2-trifluoroethyl)-1H-pyrazol-5-yl)-8-methoxy-[1,2,4]triazolo[1,5-a]pyridine (1325);
8-methoxy-6-(3-(5-(1-((tetrahydro-2H-pyran-4-yl)methyl)piperidin-4-yl)pyridin-2-yl)-4-(trifluoromethyl)-1H-pyrazol-5-yl)-[1,2,4]triazolo[1,5-a]pyridine (1326);
6-(3-(5-(1-cyclobutylpiperidin-4-yl)pyridin-2-yl)-4-(trifluoromethyl)-1H-pyrazol-5-yl)-8-methoxy-[1,2,4]triazolo[1,5-a]pyridine (1327); or
6-(3-(5-(1-(cyclobutylmethyl)piperidin-4-yl)pyridin-2-yl)-4-(trifluoromethyl)-1H-pyrazol-5-yl)-8-methoxy-[1,2,4]triazolo[1,5-a]pyridine (1328).

8. A pharmaceutical composition comprising a compound according to any one of claims 1-7 or a pharmaceutically-acceptable salt thereof; and a pharmaceutically acceptable carrier.

9. A compound according to any one of claims 1-7 or a pharmaceutically-acceptable salt thereof, or a pharmaceutical compositions according to claim 8, for use in therapy.

10. The compound or a pharmaceutical acceptable salt thereof or a pharmaceutical composition for use according to claim 9, for use in treating autoimmune disease or chronic inflammatory disease.

11. The compound or a pharmaceutically acceptable salt thereof or a erpharmaceutical composition for use according to claim 10, wherein said autoimmune disease or chronic inflammatory disease is selected from systemic lupus erythematosus (SLE), rheumatoid arthritis, multiple sclerosis (MS), and Sjögren's syndrome.

## Patentansprüche

1. Verbindung der Formel (I) 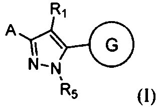 N-Oxid oder ein Salz davon, wobei:
G Folgendes ist: 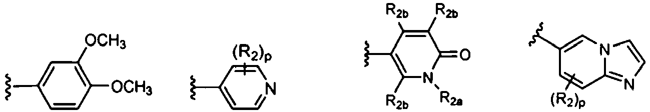 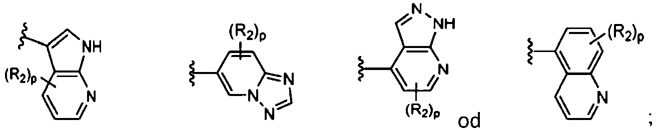
R₁ -CH₃, -CH₂CH₃, -CH(CH₃)₂, -CF₃ oder -CH₂CF₃ ist;
jedes R₂ unabhängig -CN, -CH₃ oder -OCH₃ ist;
R₂ₐ C₁₋₆-Alkyl, C₁₋₃-Fluoralkyl, C₁₋₆-Hydroxyalkyl, C₁₋₃-Aminoalkyl, - (CH₂)₀₋₄O(C₁₋₃-Alkyl), C₃₋₆-Cycloalkyl, -(CH₂)₁₋₃C(O)NR_{y}R_{y}, -CH₂(C₃₋₆-Cycloalkyl), -CH₂(phenyl), Tetrahydrofuranyl, Tetrahydropyranyl oder Phenyl ist;
jedes R_{2b} unabhängig Wasserstoff, Halogen, -CN, -NRₓRₓ, C₁₋₆-Alkyl, C₁₋₃-Fluoralkyl, C₁₋₃-Hydroxyalkyl, C₁₋₃-Fluoralkoxy, -(CH₂)₀₋₂0(C₁₋₃-Alkyl), -(CH₂)₀₋₃C(O)NRₓRₓ, -(CH₂)₁₋₃(C₃₋₆-Cycloalkyl), -C(O)O(C₁₋₃-Alkyl-), -C(O)NRₓ(C₁₋₃-Alkyl), -CR_{y} = CRₓRₓ, oder -CRₓ = CH(C₃₋₆-Cycloalkyl) ist;
A ist:
(i) - CH₂ N(CH₃)Rₓ, -C(O)NRₓRₓ, -C(O)N(CH₃)(CH₂CH₂CN), -C(O)N(CH₂CH₃)(CH₂CH₂CN) oder -C(O)N(CH₃)(CH₂CH₂CH₂ N(CH₃)₂);
(ii) -C(O)A₁, -C(O)NRₓ(CRₓRₓ)₀₋₂A₁, -CH₂NHA₁ oder -C(O)C(O)NHA₁;
(iii) Cyclohexyl, durch null bis 1R_{3b} ersetzt;
(iv) Piperidinyl, durch null bis 1R_{3b} ersetzt;
(v) Phenyl, durch null bis 1R_{3d} und null bis 1R_{3b} ersetzt;
(vi) Pyridinyl, durch null bis 1R_{3f} und null bis 1R_{3b} ersetzt;
(vii) Pyrazinyl oder Pyrimidinyl, jeweils durch null bis 1R_{3f} ersetzt,
(viii) Thiazolyl, durch R₃ₕ und null bis 1R₃ᵢ ersetzt;
(ix) Diazabicyclo[2.2.1]heptanyl oder Diazaspiro[3.3]heptanyl, jeweils durch null bis 1R₃ⱼ ersetzt; oder
(x) Benzo[d]thiazolyl, Dihydroisochinolinyl, Tetrahydronaphthyridinyl, Tetrahydrobenzo[d]thiazolyl, Tetrahydroimidazo[1,2-a]pyrazinyl, Tetrahydroisochinolinyl, Tetrahydroisochinolinyl, Tetrahydronaphthalenyl, Tetrahydropyrazolo[1,5-a]pyrazinyl, Tetrahydropyrido[4,3-d]pyrimidinyl, Tetrahydrothiazolo[4,5-c]pyridinyl oder Tetrahydrothiazolo[5,4-c]pyridinyl, jeweils durch null bis 1 R₃ₖ ersetzt;
A₁ Azetidinyl, Cyclohexyl, Pyrrolidinyl, Piperidinyl, Piperazinyl, Morpholinyl, Tetrahydropyranyl, Pyridinyl, Diazepanyl, Hexahydropyrrolo[3,4-c]pyrrolyl ist, jeweils durch null bis 1 R₃ₐ ersetzt;
R₃ₐ -OH, -CH₃, -CH₂CH₃, -CH,CH,CH₃, -CH(CH₃)₂, -CH_{2CH}(CH₃)₂, -CH(CH₃)(CH₂CH₃), -CH₂C(CH₃)₃, -CH₂CH₂CH(CH₃)₂, -CH(CH₃)CH(CH₃)₂, -CH₂CH₂C(CH₃)₃, -CH₂C(CH₂CH₃)₂, -CH₂CF₃, -CH₂CH₂CF₃, -CH₂CH₂CH₂CF₃, CH₂CH₂CN,-CH₂C(CH₃)₂OH,-CH₂CH₂OCH₃,-CH₂CH₂ S(O)₂CH₃, -CH(CH₃)CH₂ S(O)₂CH₃, -CH₂NH₂, -CH₂CH₂ N(CH₃)₂,-CH₂C(O)NH(CH₃), -CH₂C(O)N(CH₃)₂, -NR_{y}R_{y}, -N(CH₃)(CH₂CH₂OCH₃), -NH(CH₂CH₂CH₂CF₃), -NHCH(CH₃)(CH₂OCH₃), -C(O)NH₂, -C(O)OC(CH₃)₃, -CH₂(Cyclopropyl), -CH₂(Methyloxetanyl), -CH₂(Tetrahydrofuranyl), -CH₂(Tetrahydropyranyl), -CH₂(Dimethylisoxazolyl), -CH₂(Methyltriazolyl), -CH₂(Methoxypyrimidinyl), - NH(Oxetanyl), -NH(Methyloxetanyl), -NH(Tetrahydropyranyl), - NH(Dimethyltetrahydropyranyl), -N(Cyclopropyl)₂, -NHCH₂(cyclopentyl), -NHCH₂(Dimethylisoxazolyl), -NHCH₂(Methyloxetanyl), -NHCH₂(Pyridinyl), -NHCH₂(Pyrimidinyl), -NHCH₂(Methylpyrimidinyl), -NHCH₂(Methoxypyrimidinyl), -NHCH₂(Tetrahydrofuranyl), -NHCH₂(Tetrahydropyranyl), Cyclobutyl, Oxetanyl, Isopropylpiperidinyl, Tetrahydropyranyl, Dimethyltetrahydropyranyl oder Pyridinyl ist;
R_{3b} -NH(CH₃), -NH(CH₂CHF₂),-N(CH₃)(CH₂CH₃),-NH(CH₂CH₂ N(CH₃)₂), -N(CH₃)C(O)CH₂ N(CH₃)₂, -N(CH₃)CH₂C(O)N(CH₃)₂,-NH(-Isopropylpiperidinyl), -N(CH₃)C(O)(Azetidinyl), -N(CH₃)C(O)(Isopropylazetidinyl), -N(CH₃)C(O)(Ethylazetidinyl), -N(CH₃)C(O)(Methylazetidinyl), -NH(CH₂(Methyloxetanyl), Morpholinyl, Methylpiperazinyl oder Dimethylaminopiperidinyl ist;
R_{3c} C₁₋₃-Alkyl, -CH₂C(O)N(CH₃)Rₓ,-C(O)CH₂ N(CH₃)₂, - C(O)CH₂CH₂ N(CH₃)₂ oder -C(O)CH₂CH₂NH(CH(CH₃)₂) ist;
R_{3 d} ist:
(a) -CRₓRₓNRₓRₓ, -CRₓRₓNRₓ(C₂₋₅-Alkyl), -CH(CH₃)N(CH₃)(CH₂CF₃), -CH₂CH₂ S(O)₂CH₃, -CH(CH₃)N(CH₃)CH₂C(O)N(CH₃)₂, -CH(CH₃)NRₓC(O)CH₂ N(CH₂CH₃)₂,-CRₓRₓNRₓC(O)CHRₓNRₓR_{y}, -CH(CH₃)N(CH₃)C(O)CH₂NRₓ(C₃₋₄-Fluoralkyl), -NRₓRₓ, -NH(CH(CH₃)₂), -C(O)NH₂, -CRₓRₓQ₁, -CRₓRₓNRₓQ₁, CRₓRₓNRₓCH₂Q₁, -CRₓRₓNRₓC(O)Q₁, -CRₓRₓNRₓC(O)CRₓRₓQ₁, -CRₓRₓNRₓC(O)CRₓRₓNRₓQ₁ oder -CH(CH₃)N(-Oxetanyl)(C(O)CH₂ N(C₁₋₂ Alkyl)₂);
(b) Azetidinyl, durch null bis einen Substituenten ersetzt, der aus C₁₋₃-Alkyl, -CH₂C(CH₃)₂OH, -C(O)CH₂ N(CH₃)₂, -N(CH₃)₂,-NHCH(CH₃)₂, Oxetanyl und Tetrahydropyranyl ausgewählt ist;
(c) Cyclopropyl oder Cyclohexyl, jeweils durch -NRₓRₓ, -NRₓ(C₂₋₄-Alkyl), -NH(-Oxetanyl), -N(CH₃)CH₂CH₂OCH, -N(CH₃)CH₂C(O)N(CH₃)₂, -N(CH₃)C(O)CH₂ N(CH₃)₂ oder -N(CH₃)CH₂(Ethyloxetanyl) ersetzt; oder
(d) Morpholinyl, Piperazinonyl, Piperazinyl, Piperidinyl oder Pyrrolidinyl, jeweils durch null bis einen Substituenten ersetzt, der aus C₁₋₅-Alkyl, -CH₂CF₃, -CH₂C(CH₃)₂OH, -CH₂CH₂OCH₃, -CH₂C(O)CRₓRₓ, -C(O)CH₂N(CH₃)₂, Oxetanyl, Methyloxetanyl und Tetrahydropyranyl ausgewählt ist;
Q₁ Azetidinyl, Cyclopropyl, Morpholinyl, Oxetanyl, Tetrahydropyranyl, Triazolyl, Oxaazaspiro[3.3]heptanyl, Piperazinonyl, Difluorpiperidinyl oder Pyrrolidinyl ist, jeweils durch null bis 2 Substituenten ersetzt, die unabhängig aus F, -CH₃, -CH₂CH₃, -CH₂OH und Oxetanyl ausgewählt sind;
R_{3c} F ist;
R_{3f} ist:
(a) -OH, -NH₂, -N(CH₃)₂, -NH(CH(CH₃)₂), -NHCH₂C(CH₃)₂OCH₃, -CH₂NH(CH₃), -CH₂ N(CH₃)₂, -CH₂NH(CH(CH₃)₂), -CH(CH₃)N(CH₃)₂, oder -CH(CH₃)N(CH₃)C(O)CH₂ N(CH₃)₂;
(b) Cyclohexyl, durch -NH₂, -N(CH₃)₂, -NRₓ(CH₂CH₂OCH₃), - NH(Cyclobutyl), -NH(Methyloxetanyl), -NHCH₂(Methylsulfonylcyclopropyl), Morpholinyl, Methoxyazetidinyl, Piperazinonyl, Difluorpiperidinyl, Methoxypiperidinyl, Oxaazaspiro[3.3]heptanyl oder Oxaazaspiro[3.5]nonanyl ersetzt;
(c) Diazaspiro[3.3]heptanyl, durch null bis 1 Substituenten ersetzt, der aus C₁₋₄-Alkyl, -CH₂CH₂OCH₃,-CH₂CH₂ S(O)₂CH₃,-CH₂(C₃₋₄-Cycloalkyl), -CH₂(Tetrahydropyranyl), Cyclobutyl, Oxetanyl und Tetrahydropyranyl ausgewählt ist;
(d) Piperazinyl, durch null bis 1 Substituenten ersetzt, der aus C₁₋₆-Alkyl, -CH₂CH₂CF₃,-CH₂CH₂OCH₃, -CH₂CH₂ S(O)₂CH₃, -CH₂(C₃₋₄-Cycloalkyl), -CH₂(Ethyloxetanyl), -CH₂C(O)NH(CH₃), -CH₂C(O)N(CH₃)₂, -C(O)CH₂ N(CH₃)₂, -C(O)CH₂ N(CH₂CH₃)₂, -CH₂(Tetrahydropyranyl), Cyclobutyl, Oxetanyl, Tetrahydropyranyl und Dioxothiotetrahydropyranyl ausgewählt ist; oder
(e) Piperidinyl, durch null bis 1 Substituenten ersetzt, der aus C₁₋₆-Alkyl, -CH₂CN, -CH₂CH₂ F, -CH₂CH₂CH₂ F, -CH₂CH₂CF₃, -CH₂C(CH₃)₂OH, -CH₂CH₂OCH₃, -CH₂CH₂ S(O)₂CH₃, -CH₂C(O)NRₓRₓ, -C(O)CH₂ N(CH₃)₂, -C(O)CH₂ N(CH₂CH₃)₂, -CH₂(C₃₋₄-Cycloalkyl), -CH₂(Tetrahydrofuranyl), -CH₂(Tetahydropyranyl), -CH₂(Methyltriazolyl), Cyclobutyl, Ethoxycyclobutyl, Oxetanyl, Tetrahydrofuranyl und Tetrahydropyranyl ausgewählt ist;
R_{3g} F, -CH₃ oder -CF₃ ist;
R₃ₕ ist:
(a) -CH(CH₃)N(CH₃)Rₓ,-CH(CH₃)N(CH₃)(C₂₋₃ Alkyl), -CH(CH₃)N(CH₃)CH₂C(O)N(CH₃)₂, -CH(CH₃)N(CH₃)C(O)CH₂ N(CH₃)₂ oder -CH(CH₃)N(CH₃)(Tetrahydropyranyl);
(b) Cyclohexyl, durch -N(CH₃)Rₓ, -N(CH₃)(CH(CH₃)₃), -N(CH₃)(CH₂CH₂CF₃), -NRₓ(CH₂CH₂OCH₃), -NH(CH₂C(CH₃)₂OCH₃), -N(CH₃)C(O)CH₂ N(CH₃)₂,-NH(-Cyclobutyl-), -NRₓ(-oxetanyl-), - NH(Methyloxetanyl), -NH(Tetrahydropyranyl), -NHCH₂(Methylsulfonylcyclopropyl), -NHCH₂(Methyloxetanyl), Methoxyazetidinyl, (Trifluormethyl)hydroxyazetidinyl, Morpholinyl, Pyrrolidinyl, Piperazinonyl, Methylsulfonylpiperazinyl, Oxazepanyl, Oxaazaspiro[3.3]heptanyl, Oxaazaspiro[3.5]nonanyl oder Dioxothiaazaspiro[3.3]heptanyl ersetzt;
(c) Piperazinyl, durch null bis zwei -CH₃ ersetzt; und null oder 1 Substituenten, der aus C₁₋₅-Alkyl, -CH₂CN, -CH₂CH₂ F, -CH₂CH₂CH₂ F, -CH₂C(CH₃)₂OH, -CH₂CH₂OCH₃, -CH₂CH₂ S(O)₂CH₃, -CH₂c(O)N(CH₃)Rₓ, -NHC(O)CH₂ N(CH₃)₂, -NHC(O)CH₂ N(CH,CH₃)₂, -C(O)CH₂ N(CH₃)₂, -C(O)CH₂ N(CH₂CH₃)₂,-CH₂(C₃₋₄ -Cycloalkyl), -CH₂(Tetrahydropyranyl), -C(O)CH₂(Morpholinyl), Cyclobutyl, Oxetanyl und Tetrahydropyranyl ausgewählt ist; oder
(d) Piperidinyl, durch null oder ein -CH₃ und null oder 1 Substituenten ersetzt, der aus C₁₋₆-Alkyl, -CH₂CN,-CH₂CH₂ F, - CH₂CH₂CH₂ F,-CH₂CH₂CF₃, -CH₂C(CH₃)₂OH, -CH₂CH₂OCH₃, -CH₂CH₂OC(CH₃)₃, -CH₂CH₂ S(O)₂CH₃, -CH₂C(O)NH₂, -CH₂C(O)N(CH₃)₂, -CH₂C(O)N(CH₃)(CH(CH₃)₂), -C(O)CH₂ N(CH₃)₂, -C(O)CH₂ N(CH₂CH₃)₂, -C(O)CH₂ N(CH₃)(CH₂CH₂OCH₃), -NH₂, -NH(CH₂CH_{2C}H₃), -NH(CH(CH₃)₂), -NHCH₂CH(CH₃)₂, -N(CH₃)₂, -N(CH₃)(CH₂CH₃), -NH(CH₂C(CH₃)₂OCH₃), -CH₂(C₃₋₆-Cycloalkyl), -CH₂(Methylsulfonylcyclopropyl), -CH₂(Oxetanyl), -CH₂(Methyloxetanyl), -CH₂(Ethyloxetanyl), -CH₂(Tetrahydrofuranyl), -CH₂(Tetrahydropyranyl), -CH₂(Methyltriazolyl), -CH₂C(O)(Oxetanyl), -CH₂C(O)(Morpholinyl), -CH₂C(O)(Oxaazaspiro[3.3]heptanyl), -C(O)CH₂(Methoxyazetidinyl), -C(O)CH₂(Morpholinyl), -C(O)CH₂(Oxaazaspiro[3.5]nonanyl), -C(O)CH₂(Piperidinyl), -N(CH₂ (Cyclopropyl))₂, -N(CH₂ (Tetrahydropyranyl) )₂, -NH(Methyloxetanyl), - NH(Tetrahydropyranyl), -NHC(O)CH₂(Morpholinyl), -NHCH₂(Cyclopropyl), Cyclobutyl, Ethoxycyclobutyl, Ethoxycyclobutyl, Oxetanyl, Tetrahydrofuranyl, Tetrahydropyranyl, Dioxotetrahydrothiophenyl und (Oxetanylamino)piperidinyl ausgewählt;
R₃ᵢ -CH₃ oder -CF₃ ist;
R₃ⱼ C₁₋₆-Alkyl, -CH₂CH₂OCH₃, -CH₂CH₂ S(O)₂CH₃, -C(O)CH₂ N(CH₃)₂, -CH₂(C₃₋₄-Cycloalkyl), -CH₂(Tetrahydropyranyl), -CH(CH₃)(Cyclopropyl), Cyclobutyl, Oxetanyl, Tetrahydropyranyl oder Isopropylpiperidinyl ist;
R₃ₖ C₁₋₄-Alkyl, -CH₂C(CH₃)₂OH, -CH₂CH₂OCH₃, -CH₂C(O)NRₓRₓ, -C(O)CH₃, -C(O)CH,CH(CH₃)OH, -C(O)CH, N(CH₃)₂,-NRₓ(C₁₋₃-Alkyl), -NRₓ(C₃₋₄-Fluoralkyl), -NRₓ(CH₂CH₂OCH₃), -N(CH₃)(CH₂CH₂S(O)₂CH₃), -N(CH₃)(CH₂C(O)N(CH₃)₂), -NRₓ(C(O)CH₂ N(CH₃)₂), -N(CH₂CH₂CH₂CF₃)₂, -NRₓ(Oxetanyl), -N(CH₃)(Methyloxetanyl), -N(CH₃)(Tetrahydropyranyl), - NH(Ethoxycyclobutyl), Oxetanyl oder Isopropylpiperidinyl ist; und
R₅ Wasserstoff ist;
jedes Rₓ unabhängig H oder -CH₃ ist;
jedes R_{y} unabhängig H oder C₁₋₆-Alkyl ist; und
p Null, 1 oder 2 ist.

2. Verbindung nach Anspruch 1, N-Oxid oder ein Salz davon, wobei A Folgendes ist:
(i) -CH₂ N(CH₃)Rₓ, -C(O)NRₓRₓ, -C(O)N(CH₃)(CH₂CH₂CN), -C(O)N(CH₂CH₃)(CH₂CH₂CN) oder -C(O)N(CH₃)(CH₂CH₂CH₂ N(CH₃)₂); oder
(ii) -C(O)A₁, -C(O)NRₓ(CRₓRₓ)₀₋₂A₁, -CH₂NHA₁ oder -C(O)C(O)NHA₁.

3. Verbindung nach Anspruch 1, N-Oxid oder ein Salz davon, wobei X A Folgendes ist:
(i) Cyclohexyl, durch bis 1 R_{3b} ersetzt;
(ii) Piperidinyl, durch null bis 1 R_{3c} ersetzt;
(iii) Phenyl, durch null bis 1 R_{3d} und null bis 1 R₃ₑ ersetzt;
(iv) Pyridinyl, durch null bis 1 R_{3f} und null bis 1 R_{3g} ersetzt;
(v) Pyrazinyl oder Pyrimidinyl, jeweils durch null bis 1 R_{3f} ersetzt,
(vi) Thiazolyl, durch R₃ₕ und null bis 1 R₃ᵢ ersetzt;
(vii) Diazabicyclo[2.2.1]heptanyl oder Diazaspiro[3.3]heptanyl, jeweils durch null bis 1 R₃ⱼ ersetzt; oder
(viii) Benzo[d]thiazolyl, Dihydroisochinolinyl, Tetrahydronaphthyridinyl, Tetrahydrobenzo[d]thiazolyl, Tetrahydroimidazo[1,2-a]pyrazinyl, Tetrahydroisochinolinyl, Tetrahydroisochinolinyl, Tetrahydronaphthalenyl, Tetrahydropyrazolo[1,5-a]pyrazinyl, Tetrahydropyrido[4,3-d]pyrimidinyl, Tetrahydrothiazolo[4,5-c]pyridinyl oder Tetrahydrothiazolo[5,4-c]pyridinyl, jeweils durch null bis 1 R₃ₖ ersetzt.

4. Verbindung nach Anspruch 1 oder ein Salz davon, wobei
G Folgendes ist: 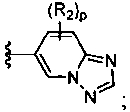
R₁ -CH(CH₃)₂ ist;
jedes R₂ unabhängig -CH₃ oder -OCH₃ ist; und
p 1 oder 2 ist.

5. Verbindung nach Anspruch 4 oder ein Salz davon, wobei A Folgendes ist:
(i) Cyclohexyl, durch bis 1 R_{3b} ersetzt;
(ii) Phenyl, durch null bis 1 R_{3d} und null bis 1 R₃ₑ ersetzt;
(iii) Pyridinyl, durch null bis 1 R_{3f} und null bis 1 R_{3g} ersetzt; und
(iv) Thiazolyl, durch R₃ₕ und null bis 1 R₃ᵢ ersetzt;

6. Verbindung nach Anspruch 4 oder ein Salz davon, wobei:
A -C(O)NRₓ(CRₓRₓ)₀₋₂A₁ ist; und
A₁ Azetidinyl, Cyclohexyl, Pyrrolidinyl, Piperidinyl, Piperazinyl, Morpholinyl, Tetrahydropyranyl, Pyridinyl, Diazepanyl, Hexahydropyrrolo[3,4-c]pyrrolyl ist, jeweils durch null bis 1 R₃ₐ ersetzt.

7. Verbindung nach Anspruch 1, N-Oxid oder ein Salz davon, wobei die Verbindung Folgendes ist:
tert-Butyl 4-(4-isopropyl-5-(8-methyl-[1,2,4]triazolo[1,5-a]pyridin-6-yl)-1H-pyrazol-3-carboxamido)piperidin-1-carboxylat (1);
4-Isopropyl-N-(1-isopropylpiperidin-4-yl)-5-(8-methyl-[1,2,4]triazolo[1,5-a]pyridin-6-yl)-1H-pyrazol-3-carboxamid (2);
4-Isopropyl-5-(8-methyl-[1,2,4]triazolo[1,5-a]pyridin-6-yl)-N-(2-morpholinoethyl)-1H-pyrazol-3-carboxamid (3);
4-Isopropyl-5-(8-methyl-[1,2,4]triazolo[1,5-a]pyridin-6-yl)-N-(2-(pyridin-2-yl)ethyl)-1H-pyrazol-3-carboxamid (4);
4-Isopropyl-N-(2-methyl-2-morpholinopropyl)-5-(8-methyl-[1,2,4]triazolo[1,5-a]pyridin-6-yl)-1H-pyrazol-3-carboxamid (5);
4-Isopropyl-N-methyl-5-(8-methyl-[1,2,4]triazolo[1,5-a]pyridin-6-yl)-N-(1-methylpiperidin-4-yl)-1H-pyrazol-3-carboxamid (6);
N-(4-(Dimethylamino)cyclohexyl)-4-isopropyl-5-(8-methyl-[1,2,4]triazolo[1,5-a]pyridin-6-yl)-1H-pyrazol-3-carboxamid (7)
4-Isopropyl-5-(8-methyl-[1,2,4]triazolo[1,5-a]pyridin-6-yl)-N-(1-methylpiperidin-4-yl)-1H-pyrazol-3-carboxamid (8);
4-Isopropyl-5-(8-methoxy-[1,2,4]triazolo[1,5-a]pyridin-6-yl)-N-methyl-N-(1-methylpiperidin-4-yl)-1H-pyrazol-3-carboxamid (9);
(4-Isopropyl-5-(8-methoxy-[1,2,4]triazolo[1,5-a]pyridin-6-yl)-1H-pyrazol-3-yl)(4-methyl-1,4-diazepan-1-yl)methanon (10);
4-Isopropyl-5-(8-methoxy-[1,2,4]triazolo[1,5-a]pyridin-6-yl)-N-(1-methylpiperidin-4-yl)-1H-pyrazol-3-carboxamid (11);
4-Isopropyl-N-(1-isopropylpiperidin-4-yl)-5-(8-methoxy-[1,2,4]triazolo[1,5-a]pyridin-6-yl)-1H-pyrazol-3-carboxamid (12);
(4-(Dimethylamino)piperidin-1-yl)(4-isopropyl-5-(8-methyl-[1,2,4]triazolo[1,5-a]pyridin-6-yl)-1H-pyrazol-3-yl)methanon (13);
(4-Isopropyl-5-(8-methyl-[1,2,4]triazolo[1,5-a]pyridin-6-yl)-1H-pyrazol-3-yl)(4-(pyridin-4-yl)piperazin-1-yl)methanon (14);
1-(4-Isopropyl-5-(8-methyl-[1,2,4]triazolo[1,5-a]pyridin-6-yl)-1H-pyrazol-3-carbonyl)piperidin-4-carboxamid (15);
N-(2-Cyanoethyl)-4-isopropyl-N-methyl-5-(8-methyl-[1,2,4]triazolo[1,5-a]pyridin-6-yl)-1H-pyrazol-3-carboxamid (16);
4-Isopropyl-N-methyl-5-(8-methyl-[1,2,4]triazolo[1,5-a]pyridin-6-yl)-N-(pyridin-3-ylmethyl)-1H-pyrazol-3-carboxamid (17);
(R)-(3-(Dimethylamino)pyrrolidin-1-yl)(4-isopropyl-5-(8-methyl-[1,2,4]triazolo[1,5-a]pyridin-6-yl)-1H-pyrazol-3-yl)methanon (18);
3-(4-(4-Isopropyl-5-(8-methyl-[1,2,4]triazolo[1,5-a]pyridin-6-yl)-1H-pyrazol-3-carbonyl)piperazin-1-yl)propanenitril (19);
4-Isopropyl-5-(8-methoxy-[1,2,4]triazolo[1,5-a]pyridin-6-yl)-N-methyl-N-(1-methylpiperidin-4-yl)-1H-pyrazol-3-carboxamid (20);
(3-(Dimethylamino)azetidin-1-yl)(4-isopropyl-5-(8-methyl-[1,2,4]triazolo[1,5-a]pyridin-6-yl)-1H-pyrazol-3-yl)methanon (21);
(S)-(3-(Dimethylamino)pyrrolidin-1-yl)(4-isopropyl-5-(8-methyl-[1,2,4]triazolo[1,5-a]pyridin-6-yl)-1H-pyrazol-3-yl)methanon (22);
(4-Isopropyl-5-(8-methyl-[1,2,4]triazolo[1,5-a]pyridin-6-yl)-1H-pyrazol-3-yl)(4-methyl-1,4-diazepan-1-yl)methanon (23);
N-(3-(Dimethylamino)propyl)-4-isopropyl-N-methyl-5-(8-methyl-[1,2,4]triazolo[1,5-a]pyridin-6-yl)-1H-pyrazol-3-carboxamid (24);
4-Isopropyl-N-methyl-5-(8-methyl-[1,2,4]triazolo[1,5-a]pyridin-6-yl)-N-(1-methylpyrrolidin-3-yl)-1H-pyrazol-3-carboxamid (25);
4-Isopropyl-5-(8-methoxy-[1,2,4]triazolo[1,5-a]pyridin-6-yl)-N-(2-methyl-2-morpholinopropyl)-1H-pyrazol-3-carboxamid (26);
4-Isopropyl-N-methyl-5-(8-methyl-[1,2,4]triazolo[1,5-a]pyridin-6-yl)-1H-pyrazol-3-carboxamid (27);
4-Isopropyl-5-(8-methyl-[1,2,4]triazolo[1,5-a]pyridin-6-yl)-N-(tetrahydro-2H-pyran-4-yl)-1H-pyrazol-3-carboxamid (28);
4-Isopropyl-5-(8-methyl-[1,2,4]triazolo[1,5-a]pyridin-6-yl)-1H-pyrazol-3-carboxamid (29);
(4-Isopropyl-5-(8-methyl-[1,2,4]triazolo[1,5-a]pyridin-6-yl)-1H-pyrazol-3-yl)(morpholino)methanon (30);
4-Isopropyl-N,N-dimethyl-5-(8-methyl-[1,2,4]triazolo[1,5-a]pyridin-6-yl)-1H-pyrazol-3-carboxamid (31);
N-(4-Hydroxycyclohexyl)-4-isopropyl-5-(8-methyl-[1,2,4]triazolo[1,5-a]pyridin-6-yl)-1H-pyrazol-3-carboxamid (32);
5-(7,8-Dimethyl-[1,2,4]triazolo[1,5-a]pyridin-6-yl)-4-isopropyl-N-(1-isopropylpiperidin-4-yl)-1H-pyrazol-3-carboxamid (33);
5-(3,4-Dimethoxyphenyl)-4-isopropyl-N-(1-isopropylpiperidin-4-yl)-1H-pyrazol-3-carboxamid (34);
4-Isopropyl-N-(1-isopropylpiperidin-4-yl)-5-(1H-pyrazolo[3,4-b]pyridin-4-yl)-1H-pyrazol-3-carboxamid (35);
5-(2,6-Dimethylpyridin-4-yl)-4-isopropyl-N-(1-isopropylpiperidin-4-yl)-1H-pyrazol-3-carboxamid (36);
4-Isopropyl-N-(1-isopropylpiperidin-4-yl)-5-(8-methylimidazo[1,2-a]pyridin-6-yl)-1H-pyrazol-3-carboxamid (37);
4-Isopropyl-N-(1-isopropylpiperidin-4-yl)-5-(2-methylpyridin-4-yl)-1H-pyrazol-3-carboxamid (38);
5-(1,5-Dimethyl-6-oxo-1,6-dihydropyridin-3-yl)-4-isopropyl-N-(1-isopropylpiperidin-4-yl)-1H-pyrazol-3-carboxamid (39);
5-(8-Cyanochinolin-5-yl)-4-isopropyl-N-(1-isopropylpiperidin-4-yl)-1H-pyrazol-3-carboxamid (40);
4-Isopropyl-5-(1-methyl-1H-pyrrolo[2,3-b]pyridin-3-yl)-N-(1-methylpiperidin-4-yl)-1H-pyrazol-3-carboxamid (41);
4-Isopropyl-N-(1-isopropylpiperidin-4-yl)-5-(1-methyl-1H-pyrrolo[2,3-b]pyridin-3-yl)-1H-pyrazol-3-carboxamid (42);
4-Isopropyl-5-(8-methyl-[1,2,4]triazolo[1,5-a]pyridin-6-yl)-N-(piperidin-4-yl)-1H-pyrazol-3-carboxamid (43);
4-Isopropyl-5-(8-methyl-[1,2,4]triazolo[1,5-a]pyridin-6-yl)-N-(piperidin-4-ylmethyl)-1H-pyrazol-3-carboxamid (45);
N-((1r,4r)-4-Aminocyclohexyl)-4-isopropyl-5-(8-methyl-[1,2,4]triazolo[1,5-a]pyridin-6-yl)-1H-pyrazol-3-carboxamid (46);
N-(((1r,4r)-4-Aminocyclohexyl)methyl)-4-isopropyl-5-(8-methyl-[1,2,4]triazolo[1,5-a]pyridin-6-yl)-1H-pyrazol-3-carboxamid (47);
4-Isopropyl-5-(8-methyl-[1,2,4]triazolo[1,5-a]pyridin-6-yl)-N-(morpholin-2-ylmethyl)-1H-pyrazol-3-carboxamid (48);
N-(Azetidin-3-ylmethyl)-4-isopropyl-5-(8-methyl-[1,2,4]triazolo[1,5-a]pyridin-6-yl)-1H-pyrazol-3-carboxamid (49);
(R)-4-Isopropyl-5-(8-methyl-[1,2,4]triazolo[1,5-a]pyridin-6-yl)-N-(pyrrolidin-3-yl)-1H-pyrazol-3-carboxamid (50);
(S)-4-Isopropyl-5-(8-methyl-[1,2,4]triazolo[1,5-a]pyridin-6-yl)-N-(pyrrolidin-2-ylmethyl)-1H-pyrazol-3-carboxamid (51);
(R)-4-Isopropyl-5-(8-methyl-[1,2,4]triazolo[1,5-a]pyridin-6-yl)-N-(piperidin-3-yl)-1H-pyrazol-3-carboxamid (52);
(S)-(3-Aminopyrrolidin-1-yl)(4-isopropyl-5-(8-methyl-[1,2,4]triazolo[1,5-a]pyridin-6-yl)-1H-pyrazol-3-yl)methanon (53);
(S)-4-Isopropyl-5-(8-methyl-[1,2,4]triazolo[1,5-a]pyridin-6-yl)-N-(piperidin-3-yl)-1H-pyrazol-3-carboxamid (54);
(4-Aminopiperidin-1-yl)(4-isopropyl-5-(8-methyl-[1,2,4]triazolo[1,5-a]pyridin-6-yl)-1H-pyrazol-3-yl)methanon (55);
4-Isopropyl-5-(8-methyl-[1,2,4]triazolo[1,5-a]pyridin-6-yl)-N-(piperidin-2-ylmethyl)-1H-pyrazol-3-carboxamid (56);
(4-Isopropyl-5-(8-methyl-[1,2,4]triazolo[1,5-a]pyridin-6-yl)-1H-pyrazol-3-yl)(piperazin-1-yl)methanon (57);
N-(Azetidin-3-yl)-4-isopropyl-5-(8-methyl-[1,2,4]triazolo[1,5-a]pyridin-6-yl)-1H-pyrazol-3-carboxamid (58);
(Hexahydropyrrolo[3,4-c]pyrrol-2(1H)-yl)(4-isopropyl-5-(8-methyl-[1,2,4]triazolo[1,5-a]pyridin-6-yl)-1H-pyrazol-3-yl)methanon (59);
4-Isopropyl-5-(8-methoxy-[1,2,4]triazolo[1,5-a]pyridin-6-yl)-N-(piperidin-4-yl)-1H-pyrazol-3-carboxamid (60);
(4-(Aminomethyl)piperidin-1-yl)(4-isopropyl-5-(8-methyl-[1,2,4]triazolo[1,5-a]pyridin-6-yl)-1H-pyrazol-3-yl)methanon (61);
4-Isopropyl-5-(8-methoxy-[1,2,4]triazolo[1,5-a]pyridin-6-yl)-N-(piperidin-4-ylmethyl)-1H-pyrazol-3-carboxamid (62);
N-((1s,4s)-4-Aminocyclohexyl)-4-isopropyl-5-(8-methyl-[1,2,4]triazolo[1,5-a]pyridin-6-yl)-1H-pyrazol-3-carboxamid (63);
4-Isopropyl-5-(8-methyl-[1,2,4]triazolo[1,5-a]pyridin-6-yl)-N-(morpholin-2-ylmethyl)-1H-pyrazol-3-carboxamid (64);
4-Isopropyl-5-(8-methyl-[1,2,4]triazolo[1,5-a]pyridin-6-yl)-N-(morpholin-2-ylmethyl)-1H-pyrazol-3-carboxamid (65);
4-Isopropyl-5-(8-methyl-[1,2,4]triazolo[1,5-a]pyridin-6-yl)-N-(piperidin-3-yl)-1H-pyrazol-3-carboxamid (66);
4-Isopropyl-5-(8-methyl-[1,2,4]triazolo[1,5-a]pyridin-6-yl)-N-(piperidin-3-yl)-1H-pyrazol-3-carboxamid (67);
4-Isopropyl-5-(1-methyl-1H-pyrrolo[2,3-b]pyridin-3-yl)-N-(piperidin-4-yl)-1H-pyrazol-3-carboxamid (68);
4-Isopropyl-5-(8-methyl-[1,2,4]triazolo[1,5-a]pyridin-6-yl)-N-(1-(3,3,3-trifluorpropyl)piperidin-4-yl)-1H-pyrazol-3-carboxamid (69);
4-Isopropyl-5-(8-methyl-[1,2,4]triazolo[1,5-a]pyridin-6-yl)-N-(1-(oxetan-3-yl)piperidin-4-yl)-1H-pyrazol-3-carboxamid (70);
4-Isopropyl-5-(8-methyl-[1,2,4]triazolo[1,5-a]pyridin-6-yl)-N-(1-(4,4,4-trifluorbutyl)piperidin-4-yl)-1H-pyrazol-3-carboxamid (71);
4-Isopropyl-5-(8-methyl-[1,2,4]triazolo[1,5-a]pyridin-6-yl)-N-(1-((tetrahydrofuran-3-yl)methyl)piperidin-4-yl)-1H-pyrazol-3-carboxamid (72);
N-(1-(Cyclopropylmethyl)piperidin-4-yl)-4-isopropyl-5-(8-methyl-[1,2,4]triazolo[1,5-a]pyridin-6-yl)-1H-pyrazol-3-carboxamid (73);
4-Isopropyl-5-(8-methyl-[1,2,4]triazolo[1,5-a]pyridin-6-yl)-N-(1-neopentylpiperidin-4-yl)-1H-pyrazol-3-carboxamid (74);
N-(1-(3,3-Dimethylbutyl)piperidin-4-yl)-4-isopropyl-5-(8-methyl-[1,2,4]triazolo[1,5-a]pyridin-6-yl)-1H-pyrazol-3-carboxamid (75);
N-(1-Isopentylpiperidin-4-yl)-4-isopropyl-5-(8-methyl-[1,2,4]triazolo[1,5-a]pyridin-6-yl)-1H-pyrazol-3-carboxamid (76);
4-Isopropyl-5-(8-methyl-[1,2,4]triazolo[1,5-a]pyridin-6-yl)-N-(1-((tetrahydro-2H-pyran-4-yl)methyl)piperidin-4-yl)-1H-pyrazol-3-carboxamid (77);
N-(1-((3,5-Dimethylisoxazol-4-yl)methyl)piperidin-4-yl)-4-isopropyl-5-(8-methyl-[1,2,4]triazolo[1,5-a]pyridin-6-yl)-1H-pyrazol-3-carboxamid (78);
N-(1-(2-Ethylbutyl)piperidin-4-yl)-4-isopropyl-5-(8-methyl-[1,2,4]triazolo[1,5-a]pyridin-6-yl)-1H-pyrazol-3-carboxamid (79);
4-Isopropyl-5-(8-methyl-[1,2,4]triazolo[1,5-a]pyridin-6-yl)-N-((1r,4r)-4-(oxetan-3-ylamino)cyclohexyl)-1H-pyrazol-3-carboxamid (80);
4-Isopropyl-N-((1r,4r)-4-(isopropylamino)cyclohexyl)-5-(8-methyl-[1,2,4]triazolo[1,5-a]pyridin-6-yl)-1H-pyrazol-3-carboxamid (81);
N-((1r,4r)-4-(Dimethylamino)cyclohexyl)-4-isopropyl-5-(8-methyl-[1,2,4]triazolo[1,5-a]pyridin-6-yl)-1H-pyrazol-3-carboxamid (82);
N-(1-(2-(Dimethylamino)ethyl)piperidin-4-yl)-4-isopropyl-5-(8-methyl-[1,2,4]triazolo[1,5-a]pyridin-6-yl)-1H-pyrazol-3-carboxamid (83);
N-((1s,4s)-4-(Dimethylamino)cyclohexyl)-4-isopropyl-5-(8-methyl-[1,2,4]triazolo[1,5-a]pyridin-6-yl)-1H-pyrazol-3-carboxamid (84);
4-Isopropyl-N-((1s,4s)-4-(isopropylamino)cyclohexyl)-5-(8-methyl-[1,2,4]triazolo[1,5-a]pyridin-6-yl)-1H-pyrazol-3-carboxamid (85);
4-Isopropyl-5-(8-methyl-[1,2,4]triazolo[1,5-a]pyridin-6-yl)-N-((1s,4s)-4-(oxetan-3-ylamino)cyclohexyl)-1H-pyrazol-3-carboxamid (86);
4-Isopropyl-5-(8-methyl-[1,2,4]triazolo[1,5-a]pyridin-6-yl)-N-((1s,4s)-4-(((tetrahydrofuran-3-yl)methyl)amino)cyclohexyl)-1H-pyrazol-3-carboxamid (87);
4-Isopropyl-5-(8-methyl-[1,2,4]triazolo[1,5-a]pyridin-6-yl)-N-((1s,4s)-4-((4,4,4-trifluorbutyl)amino)cyclohexyl)-1H-pyrazol-3-carboxamid (88);
4-Isopropyl-5-(8-methyl-[1,2,4]triazolo[1,5-a]pyridin-6-yl)-N-((1s,4s)-4-(neopentylamino)cyclohexyl)-1H-pyrazol-3-carboxamid (89);
N-((1s,4s)-4-(bis(3,3-Dimethylbutyl)amino)cyclohexyl)-4-isopropyl-5-(8-methyl-[1,2,4]triazolo[1,5-a]pyridin-6-yl)-1H-pyrazol-3-carboxamid (90);
N-((1s,4s)-4-(Diisopentylamino)cyclohexyl)-4-isopropyl-5-(8-methyl-[1,2,4]triazolo[1,5-a]pyridin-6-yl)-1H-pyrazol-3-carboxamid (91);
4-Isopropyl-5-(8-methyl-[1,2,4]triazolo[1,5-a]pyridin-6-yl)-N-((1s,4s)-4-(((tetrahydro-2H-pyran-4-yl)methyl)amino)cyclohexyl)-1H-pyrazol-3-carboxamid (92);
N-((1s,4s)-4-(((3,5-Dimethylisoxazol-4-yl)methyl)amino)cyclohexyl)-4-isopropyl-5-(8-methyl-[1,2,4]triazolo[1,5-a]pyridin-6-yl)-1H-pyrazol-3-carboxamid (93);
N-((1s,4s)-4-((Cyclopentylmethyl)amino)cyclohexyl)-4-isopropyl-5-(8-methyl-[1,2,4]triazolo[1,5-a]pyridin-6-yl)-1H-pyrazol-3-carboxamid (94);
4-isopropyl-5-(8-methyl-[1,2,4]triazolo[1,5-a]pyridin-6-yl)-N-((1s,4s)-4-((pyridin-3-ylmethyl)amino)cyclohexyl)-1H-pyrazol-3-carboxamid (95);
4-Isopropyl-N-((1s,4s)-4-(((2-methoxypyrimidin-5-yl)methyl)amino)cyclohexyl)-5-(8-methyl-[1,2,4]triazolo[1,5-a]pyridin-6-yl)-1H-pyrazol-3-carboxamid (96);
N-((1s,4s)-4-((2,2-Dimethyltetrahydro-2H-pyran-4-yl)amino)cyclohexyl)-4-isopropyl-5-(8-methyl-[1,2,4]triazolo[1,5-a]pyridin-6-yl)-1H-pyrazol-3-carboxamid (97);
4-Isopropyl-5-(8-methyl-[1,2,4]triazolo[1,5-a]pyridin-6-yl)-N-((1s,4s)-4-((tetrahydro-2H-pyran-4-yl)amino)cyclohexyl)-1H-pyrazol-3-carboxamid (98);
4-Isopropyl-5-(8-methyl-[1,2,4]triazolo[1,5-a]pyridin-6-yl)-N-((1s,4s)-4-((3-methylbutan-2-yl)amino)cyclohexyl)-1H-pyrazol-3-carboxamid (99);
4-Isopropyl-5-(8-methyl-[1,2,4]triazolo[1,5-a]pyridin-6-yl)-N-((1s,4s)-4-(pentan-3-ylamino)cyclohexyl)-1H-pyrazol-3-carboxamid (100);
N-((1s,4s)-4-((2-Ethylbutyl)amino)cyclohexyl)-4-isopropyl-5-(8-methyl-[1,2,4]triazolo[1,5-a]pyridin-6-yl)-1H-pyrazol-3-carboxamid (101);
4-Isopropyl-5-(8-methyl-[1,2,4]triazolo[1,5-a]pyridin-6-yl)-N-((1s,4s)-4-((pyrimidin-5-ylmethyl)amino)cyclohexyl)-1H-pyrazol-3-carboxamid (102);
N-((1r,4r)-4-(bis(Cyclopropylmethyl)amino)cyclohexyl)-4-isopropyl-5-(8-methyl-[1,2,4]triazolo[1,5-a]pyridin-6-yl)-1H-pyrazol-3-carboxamid (103);
4-Isopropyl-5-(8-methyl-[1,2,4]triazolo[1,5-a]pyridin-6-yl)-N-((1r,4r)-4-(((2-methylpyrimidin-5-yl)methyl)amino)cyclohexyl)-1H-pyrazol-3-carboxamid (104);
4-Isopropyl-5-(8-methyl-[1,2,4]triazolo[1,5-a]pyridin-6-yl)-N-((1r,4r)-4-(((tetrahydrofuran-3-yl)methyl)amino)cyclohexyl)-1H-pyrazol-3-carboxamid (105);
4-Isopropyl-5-(8-methyl-[1,2,4]triazolo[1,5-a]pyridin-6-yl)-N-((1r,4r)-4-(neopentylamino)cyclohexyl)-1H-pyrazol-3-carboxamid
N-((1r,4r)-4-(bis(3,3-Dimethylbutyl)amino)cyclohexyl)-4-isopropyl-5-(8-methyl-[1,2,4]triazolo[1,5-a]pyridin-6-yl)-1H-pyrazol-3-carboxamid (107);
N-((1r,4r)-4-(Diisopentylamino)cyclohexyl)-4-isopropyl-5-(8-methyl-[1,2,4]triazolo[1,5-a]pyridin-6-yl)-1H-pyrazol-3-carboxamid (108);
N-((1r,4r)-4-((Cyclopentylmethyl)amino)cyclohexyl)-4-isopropyl-5-(8-methyl-[1,2,4]triazolo[1,5-a]pyridin-6-yl)-1H-pyrazol-3-carboxamid (109);
N-((1r,4r)-4-((2-Ethylbutyl)amino)cyclohexyl)-4-isopropyl-5-(8-methyl-[1,2,4]triazolo[1,5-a]pyridin-6-yl)-1H-pyrazol-3-carboxamid (110);
4-Isopropyl-5-(8-methyl-[1,2,4]triazolo[1,5-a]pyridin-6-yl)-N-((1r,4r)-4-(((tetrahydro-2H-pyran-4-yl)methyl)amino)cyclohexyl)-1H-pyrazol-3-carboxamid (111);
4-Isopropyl-5-(8-methyl-[1,2,4]triazolo[1,5-a]pyridin-6-yl)-N-((1r,4r)-4-((pyridin-3-ylmethyl)amino)cyclohexyl)-1H-pyrazol-3-carboxamid (112);
4-Isopropyl-5-(8-methyl-[1,2,4]triazolo[1,5-a]pyridin-6-yl)-N-((1r,4r)-4-((pyrimidin-5-ylmethyl)amino)cyclohexyl)-1H-pyrazol-3-carboxamid (113);
4-Isopropyl-N-((1r,4r)-4-(((2-methoxypyrimidin-5-yl)methyl)amino)cyclohexyl)-5-(8-methyl-[1,2,4]triazolo[1,5-a]pyridin-6-yl)-1H-pyrazol-3-carboxamid (114);
N -((1r,4r)-4-((2,2-Dimethyltetrahydro-2H-pyran-4-yl)amino)cyclohexyl)-4-isopropyl-5-(8-methyl-[1,2,4]triazolo[1,5-a]pyridin-6-yl)-1H-pyrazol-3-carboxamid (115);
4-Isopropyl-5-(8-methyl-[1,2,4]triazolo[1,5-a]pyridin-6-yl)-N-((1r,4r)-4-((tetrahydro-2H-pyran-4-yl)amino)cyclohexyl)-1H-pyrazol-3-carboxamid (116);
4-Isopropyl-5-(8-methyl-[1,2,4]triazolo[1,5-a]pyridin-6-yl)-N-((1r,4r)-4-((3-methylbutan-2-yl)amino)cyclohexyl)-1H-pyrazol-3-carboxamid (118);
4-Isopropyl-5-(8-methyl-[1,2,4]triazolo[1,5-a]pyridin-6-yl)-N-((1r,4r)-4-(pentan-3-ylamino)cyclohexyl)-1H-pyrazol-3-carboxamid (119);
N-((1r,4r)-4-((4,4-Dimethylpentan-2-yl)amino)cyclohexyl)-4-isopropyl-5-(8-methyl-[1,2,4]triazolo[1,5-a]pyridin-6-yl)-1H-pyrazol-3-carboxamid (120);
4-Isopropyl-5-(8-methyl-[1,2,4]triazolo[1,5-a]pyridin-6-yl)-N-(4-((3-methyloxetan-3-yl)amino)cyclohexyl)-1H-pyrazol-3-carboxamid (123-124);
4-Isopropyl-5-(8-methyl-[1,2,4]triazolo[1,5-a]pyridin-6-yl)-N-(1-(3-methylbutan-2-yl)piperidin-4-yl)-1H-pyrazol-3-carboxamid (125);
4-Isopropyl-5-(8-methyl-[1,2,4]triazolo[1,5-a]pyridin-6-yl)-N-(4-(neopentylamino)cyclohexyl)-1H-pyrazol-3-carboxamid (126 und 131);
4-Isopropyl-N-(4-(isopropyl(methyl)amino)cyclohexyl)-5-(8-methyl-[1,2,4]triazolo[1,5-a]pyridin-6-yl)-1H-pyrazol-3-carboxamid (127 und 132);
4-Isopropyl-N-(4-((2-methoxyethyl)(methyl)amino)cyclohexyl)-5-(8-methyl-[1,2,4]triazolo[1,5-a]pyridin-6-yl)-1H-pyrazol-3-carboxamid (128);
4-Isopropyl-5-(8-methyl-[1,2,4]triazolo[1,5-a]pyridin-6-yl)-N-(4-(methylamino)cyclohexyl)-1H-pyrazol-3-carboxamid (129-130);
4-Isopropyl-5-(8-methyl-[1,2,4]triazolo[1,5-a]pyridin-6-yl)-N-(1-((3-methyloxetan-3-yl)methyl)piperidin-4-yl)-1H-pyrazol-3-carboxamid (133);
4-Isopropyl-5-(8-methyl-[1,2,4]triazolo[1,5-a]pyridin-6-yl)-N-((4-methylmorpholin-2-yl)methyl)-1H-pyrazol-3-carboxamid
4-Isopropyl-N-((4-isopropylmorpholin-2-yl)methyl)-5-(8-methyl-[1,2,4]triazolo[1,5-a]pyridin-6-yl)-1H-pyrazol-3-carboxamid (135);
4-Isopropyl-5-(8-methyl-[1,2,4]triazolo[1,5-a]pyridin-6-yl)-N-((4-(oxetan-3-yl)morpholin-2-yl)methyl)-1H-pyrazol-3-carboxamid (136 und 142);
4-Isopropyl-5-(8-methyl-[1,2,4]triazolo[1,5-a]pyridin-6-yl)-N-((4-((3-methyloxetan-3-yl)methyl)morpholin-2-yl)methyl)-1H-pyrazol-3-carboxamid (137);
N-(1-(2-Hydroxy-2-methylpropyl)piperidin-4-yl)-4-isopropyl-5-(8-methyl-[1,2,4]triazolo[1,5-a]pyridin-6-yl)-1H-pyrazol-3-carboxamid (138);
4-Isopropyl-5-(8-methyl-[1,2,4]triazolo[1,5-a]pyridin-6-yl)-N-((4-methylmorpholin-2-yl)methyl)-1H-pyrazol-3-carboxamid (139);
N-((4-Ethylmorpholin-2-yl)methyl)-4-isopropyl-5-(8-methyl-[1,2,4]triazolo[1,5-a]pyridin-6-yl)-1H-pyrazol-3-carboxamid (140);
4-Isopropyl-N-((4-isopropylmorpholin-2-yl)methyl)-5-(8-methyl-[1,2,4]triazolo[1,5-a]pyridin-6-yl)-1H-pyrazol-3-carboxamid (141);
N-((4-(2-Hydroxy-2-methylpropyl)morpholin-2-yl)methyl)-4-isopropyl-5-(8-methyl-[1,2,4]triazolo[1,5-a]pyridin-6-yl)-1H-pyrazol-3-carboxamid (143);
4-Isopropyl-5-(8-methyl-[1,2,4]triazolo[1,5-a]pyridin-6-yl)-N-((4-neopentylmorpholin-2-yl)methyl)-1H-pyrazol-3-carboxamid (144-145);
4-Isopropyl-N-((1r,4r)-4-((1-methoxypropan-2-yl)amino)cyclohexyl)-5-(8-methyl-[1,2,4]triazolo[1,5-a]pyridin-6-yl)-1H-pyrazol-3-carboxamid (146);
4-Isopropyl-N-((1s,4s)-4-((1-methoxypropan-2-yl)amino)cyclohexyl)-5-(8-methyl-[1,2,4]triazolo[1,5-a]pyridin-6-yl)-1H-pyrazol-3-carboxamid (147);
N-((1r,4r)-4-((3,3-Dimethylbutan-2-yl)amino)cyclohexyl)-4-isopropyl-5-(8-methyl-[1,2,4]triazolo[1,5-a]pyridin-6-yl)-1H-pyrazol-3-carboxamid (148);
4-Isopropyl-5-(8-methyl-[1,2,4]triazolo[1,5-a]pyridin-6-yl)-N-((1r,4r)-4-(((3-methyloxetan-3-yl)methyl)amino)cyclohexyl)-1H-pyrazol-3-carboxamid (149);
N-((1s,4s)-4-((3,3-Dimethylbutan-2-yl)amino)cyclohexyl)-4-isopropyl-5-(8-methyl-[1,2,4]triazolo[1,5-a]pyridin-6-yl)-1H-pyrazol-3-carboxamid (150);
4-Isopropyl-5-(8-methyl-[1,2,4]triazolo[1,5-a]pyridin-6-yl)-N-((1s,4s)-4-(((3-methyloxetan-3-yl)methyl)amino)cyclohexyl)-1H-pyrazol-3-carboxamid (151);
N-(1-(sec-Butyl)piperidin-4-yl)-4-isopropyl-5-(8-methyl-[1,2,4]triazolo[1,5-a]pyridin-6-yl)-1H-pyrazol-3-carboxamid (152);
4-Isopropyl-5-(8-methyl-[1,2,4]triazolo[1,5-a]pyridin-6-yl)-N-(1-(tetrahydro-2H-pyran-4-yl)piperidin-4-yl)-1H-pyrazol-3-carboxamid (153);
4-Isopropyl-5-(8-methyl-[1,2,4]triazolo[1,5-a]pyridin-6-yl)-N-(1-((3-methyloxetan-3-yl)methyl)azetidin-3-yl)-1H-pyrazol-3-carboxamid (154);
4-Isopropyl-N-(1-isopropylazetidin-3-yl)-5-(8-methyl-[1,2,4]triazolo[1,5-a]pyridin-6-yl)-1H-pyrazol-3-carboxamid (155);
4-Isopropyl-5-(8-methyl-[1,2,4]triazolo[1,5-a]pyridin-6-yl)-N-(1-methylazetidin-3-yl)-1H-pyrazol-3-carboxamid (156);
4-Isopropyl-N-(1-((2-methoxypyrimidin-5-yl)methyl)piperidin-4-yl)-5-(8-methyl-[1,2,4]triazolo[1,5-a]pyridin-6-yl)-1H-pyrazol-3-carboxamid (157);
4-Isopropyl-N-(1-((1-methyl-1H-1,2,4-triazol-3-yl)methyl)piperidin-4-yl)-5-(8-methyl-[1,2,4]triazolo[1,5-a]pyridin-6-yl)-1H-pyrazol-3-carboxamid (158);
4-Isopropyl-N-(1-((1-methyl-1H-1,2,4-triazol-3-yl)methyl)azetidin-3-yl)-5-(8-methyl-[1,2,4]triazolo[1,5-a]pyridin-6-yl)-1H-pyrazol-3-carboxamid (159);
(R)-4-Isopropyl-5-(8-methyl-[1,2,4]triazolo[1,5-a]pyridin-6-yl)-N-(1-methylpiperidin-3-yl)-1H-pyrazol-3-carboxamid (160);
(R)-4-Isopropyl-N-(1-isopropylpiperidin-3-yl)-5-(8-methyl-[1,2,4]triazolo[1,5-a]pyridin-6-yl)-1H-pyrazol-3-carboxamid (161);
(S)-4-Isopropyl-5-(8-methyl-[1,2,4]triazolo[1,5-a]pyridin-6-yl)-N-(1-methylpiperidin-3-yl)-1H-pyrazol-3-carboxamid (162);
(S)-4-Isopropyl-N-(1-isopropylpiperidin-3-yl)-5-(8-methyl-[1,2,4]triazolo[1,5-a]pyridin-6-yl)-1H-pyrazol-3-carboxamid (163);
(S)-N-(1-Ethylpiperidin-3-yl)-4-isopropyl-5-(8-methyl-[1,2,4]triazolo[1,5-a]pyridin-6-yl)-1H-pyrazol-3-carboxamid (164);
4-Isopropyl-N-((3S)-1-((3-methyl-3H-1,2,4-triazol-5-yl)methyl)piperidin-3-yl)-5-(8-methyl-[1,2,4]triazolo[1,5-a]pyridin-6-yl)-1H-pyrazol-3-carboxamid (165);
(S)-4-Isopropyl-5-(8-methyl-[1,2,4]triazolo[1,5-a]pyridin-6-yl)-N-(1-(oxetan-3-yl)piperidin-3-yl)-1H-pyrazol-3-carboxamid (166);
4-Isopropyl-5-(1-methyl-1H-pyrrolo[2,3-b]pyridin-3-yl)-N-(1-(oxetan-3-yl)piperidin-4-yl)-1H-pyrazol-3-carboxamid (167);
4-Isopropyl-5-(1-methyl-1H-pyrrolo[2,3-b]pyridin-3-yl)-N-(1-neopentylpiperidin-4-yl)-1H-pyrazol-3-carboxamid (168);
4-Isopropyl-5-(8-methyl-[1,2,4]triazolo[1,5-a]pyridin-6-yl)-N-(1-(3,3,3-trifluorpropyl)piperidin-4-yl)-1H-pyrazol-3-carboxamid (169);
4-Isopropyl-5-(8-methyl-[1,2,4]triazolo[1,5-a]pyridin-6-yl)-N-(1-(2-(methylamino)ethyl)piperidin-4-yl)-1H-pyrazol-3-carboxamid (170);
4-Isopropyl-N-(1-(2-methoxyethyl)piperidin-4-yl)-5-(8-methyl-[1,2,4]triazolo [1,5-a]pyridin-6-yl)-1H-pyrazol-3-carboxamid (171);
4-Isopropyl-5-(8-methyl-[1,2,4]triazolo[1,5-a]pyridin-6-yl)-N-(1-(2-(methylsulfonyl)ethyl)piperidin-4-yl)-1H-pyrazol-3-carboxamid (172);
4-Isopropyl-5-(8-methyl-[1,2,4]triazolo[1,5-a]pyridin-6-yl)-N-(1-(2,2,2-trifluorethyl)piperidin-4-yl)-1H-pyrazol-3-carboxamid (173);
4-Isopropyl-5-(8-methyl-[1,2,4]triazolo[1,5-a]pyridin-6-yl)-N-(1-(1-(methylsulfonyl)propan-2-yl)piperidin-4-yl)-1H-pyrazol-3-carboxamid (174);
N-(1-(2-(Dimethylamino)-2-oxoethyl)piperidin-4-yl)-4-isopropyl-5-(8-methyl[1,2,4]triazolo[1,5-a]pyridin-6-yl)-1H-pyrazol-3-carboxamid (176);
4-Isopropyl-5-(8-methyl-[1,2,4]triazolo[1,5-a]pyridin-6-yl)-N-(1-(2-(methylamino)-2-oxoethyl)piperidin-4-yl)-1H-pyrazol-3-carboxamid (177);
N-(1-Isopropylpiperidin-4-yl)-4-methyl-5-(8-methyl-[1,2,4]triazolo[1,5-a]pyridin-6-yl)-1H-pyrazol-3-carboxamid (179);
4-Methyl-5-(8-methyl-[1,2,4]triazolo[1,5-a]pyridin-6-yl)-N-(1-methylpiperidin-4-yl)-1H-pyrazol-3-carboxamid (180);
4-Ethyl-5-(8-methyl-[1,2,4]triazolo[1,5-a]pyridin-6-yl)-N-(1-methylpiperidin-4-yl)-1H-pyrazol-3-carboxamid (181);
4-Ethyl-N-(1-isopropylpiperidin-4-yl)-5-(8-methyl-[1,2,4]triazolo[1,5-a]pyridin-6-yl)-1H-pyrazol-3-carboxamid (182);
2-(4-Isopropyl-5-(8-methyl-[1,2,4]triazolo[1,5-a]pyridin-6-yl)-1H-pyrazol-3-yl)-N-(1-isopropylpiperidin-4-yl)-2-oxoacetamid (183);
1-(4-Isopropyl-5-(8-methyl-[1,2,4]triazolo[1,5-a]pyridin-6-yl)-1H-pyrazol-3-yl)-N,N-dimethylmethanamin (184);
1-(4-Isopropyl-5-(8-methyl-[1,2,4]triazolo[1,5-a]pyridin-6-yl)-1H-pyrazol-3-yl)-N-methylmethanamin (185);
1-Isopropyl-N-((4-isopropyl-5-(8-methyl-[1,2,4]triazolo[1,5-a]pyridin-6-yl)-1H-pyrazol-3-yl)methyl)piperidin-4-amin (186);
N-((4-Isopropyl-5-(8-methyl-[1,2,4]triazolo[1,5-a]pyridin-6-yl)-1H-pyrazol-3-yl)methyl)piperidin-4-amin (187);
5-(8-Methoxy-[1,2,4]triazolo[1,5-a]pyridin-6-yl)-N-(piperidin-4-yl)-4-(2,2,2-trifluorethyl)-1H-pyrazol-3-carboxamid (190);
N-(1-Isopropylpiperidin-4-yl)-5-(8-methoxy-[1,2,4]triazolo[1,5-a]pyridin-6-yl)-4-(2,2,2-trifluorethyl)-1H-pyrazol-3-carboxamid (191);
5-(8-Methoxy-[1,2,4]triazolo[1,5-a]pyridin-6-yl)-N-(1-methylpiperidin-4-yl)-4-(2,2,2-trifluorethyl)-1H-pyrazol-3-carboxamid (192);
N-(1-Isopropylpiperidin-4-yl)-5-(8-methyl-[1,2,4]triazolo[1,5-a]pyridin-6-yl)-4-(2,2,2-trifluorethyl)-1H-pyrazol-3-carboxamid (193);
5-(8-Methyl-[1,2,4]triazolo[1,5-a]pyridin-6-yl)-N-(piperidin-4-yl)-4-(2,2,2-trifluorethyl)-1H-pyrazol-3-carboxamid (194);
5-(8-Methoxy-[1,2,4]triazolo[1,5-a]pyridin-6-yl)-N-(1-propylpiperidin-4-yl)-4-(2,2,2-trifluorethyl)-1H-pyrazol-3-carboxamid (195);
5-(8-Methoxy-[1,2,4]triazolo[1,5-a]pyridin-6-yl)-N-(1-neopentylpiperidin-4-yl)-4-(2,2,2-trifluorethyl)-1H-pyrazol-3-carboxamid (196);
5-(8-Methoxy-[1,2,4]triazolo[1,5-a]pyridin-6-yl)-N-(1-((tetrahydro-2H-pyran-4-yl)methyl)piperidin-4-yl)-4-(2,2,2-trifluorethyl)-1H-pyrazol-3-carboxamid (197);
N-(1'-Isopropyl-[1,4'-bipiperidin]-4-yl)-5-(8-methoxy-[1,2,4]triazolo[1,5-a]pyridin-6-yl)-4-(2,2,2-trifluorethyl)-1H-pyrazol-3-carboxamid (199);
5-(8-Methyl-[1,2,4]triazolo[1,5-a]pyridin-6-yl)-N-(1-(oxetan-3-yl)piperidin-4-yl)-4-(2,2,2-trifluorethyl)-1H-pyrazol-3-carboxamid (200);
5-(8-Methyl-[1,2,4]triazolo[1,5-a]pyridin-6-yl)-N-(1-(tetrahydro-2H-pyran-4-yl)piperidin-4-yl)-4-(2,2,2-trifluorethyl)-1H-pyrazol-3-carboxamid (201);
N-(1-Isobutylpiperidin-4-yl)-5-(8-methyl-[1,2,4]triazolo[1,5-a]pyridin-6-yl)-4-(2,2,2-trifluorethyl)-1H-pyrazol-3-carboxamid (202);
N-(1-Cyclobutylpiperidin-4-yl)-5-(8-methyl-[1,2,4]triazolo[1,5-a]pyridin-6-yl)-4-(2,2,2-trifluorethyl)-1H-pyrazol-3-carboxamid (203);
5-(8-Methyl-[1,2,4]triazolo[1,5-a]pyridin-6-yl)-N-(1-((tetrahydro-2H-pyran-4-yl)methyl)piperidin-4-yl)-4-(2,2,2-trifluorethyl)-1H-pyrazol-3-carboxamid (204);
5-(8-Methoxy-[1,2,4]triazolo[1,5-a]pyridin-6-yl)-N-(1-(2-(methylamino)-2-oxoethyl)piperidin-4-yl)-4-(2,2,2-trifluorethyl)-1H-pyrazol-3-carboxamid (205);
6-(4-Isopropyl-3-(1-methylpiperidin-4-yl)-1H-pyrazol-5-yl)-8-methyl-[1,2,4]triazolo[1,5-a]pyridin (206);
6-(4-Isopropyl-3-(1-isopropylpiperidin-4-yl)-1H-pyrazol-5-yl)-8-methyl-[1,2,4]triazolo[1,5-a]pyridin (207);
3-(Dimethylamino)-1-(4-(4-isopropyl-5-(8-methyl-[1,2,4]triazolo[1,5-a]pyridine-6-yl)-1H-pyrazol-3-yl)piperidin-1-yl)propan-1-on (208);
2-(Dimethylamino)-1-(4-(4-isopropyl-5-(8-methyl-[1,2,4]triazolo[1,5-a]pyridin-6-yl)-1H-pyrazol-3-yl)piperidin-1-yl)ethan-1-on (209);
1-(4-(4-Isopropyl-5-(8-methyl-[1,2,4]triazolo[1,5-a]pyridin-6-yl)-1H-pyrazol-3-yl)piperidin-1-yl)-3-(isopropylamino)propan-1-on (210);
2-(4-(4-Isopropyl-5-(8-methyl-[1,2,4]triazolo[1,5-a]pyridin-6-yl)-1H-pyrazol-3-yl)piperidin-1-yl)-N-methylacetamid (211);
2-(4-(4-Isopropyl-5-(8-methyl-[1,2,4]triazolo[1,5-a]pyridin-6-yl)-1H-pyrazol-3-yl)piperidin-1-yl)-N,N-dimethylacetamid (212);
6-(4-Isopropyl-5-(8-methyl-[1,2,4]triazolo[1,5-a]pyridin-6-yl)-1H-pyrazol-3-yl)-1,2,3,4-tetrahydroisochinolin, HCl(213);
6-(4-Isopropyl-5-(8-methyl-[1,2,4]triazolo[1,5-a]pyridin-6-yl)-1H-pyrazol-3-yl)isochinolin (214);
6-(4-Isopropyl-5-(8-methyl-[1,2,4]triazolo[1,5-a]pyridin-6-yl)-1H-pyrazol-3-yl)-3,4-dihydroisochinolin-1(2H)-on (215);
6-(3-(6,7-Dihydro-5H-pyrrolo[3,4-d]pyrimidin-2-yl)-4-isopropyl-1H-pyrazol-5-yl)-8-methyl-[1,2,4]triazolo[1,5-a]pyridin (216);
6-(4-Isopropyl-3-(4,5,6,7-tetrahydropyrazolo[1,5-a]pyrazin-2-yl)-1H-pyrazol-5-yl)-8-methyl-[1,2,4]triazolo[1,5-a]pyridin (217);
2-(4-Isopropyl-5-(8-methyl-[1,2,4]triazolo[1,5-a]pyridin-6-yl)-1H-pyrazol-3-yl)-4,5,6,7-tetrahydrothiazolo[5,4-c]pyridin (218);
6-(4-Isopropyl-3-(5,6,7,8-tetrahydroimidazo[1,2-a]pyrazin-2-yl)-1H-pyrazol-5-yl)-8-methyl-[1,2,4]triazolo[1,5-a]pyridin (219);
2-(4-Isopropyl-5-(8-methoxy-[1,2,4]triazolo[1,5-a]pyridin-6-yl)-1H-pyrazol-3-yl)-4,5,6,7-tetrahydrothiazolo[5,4-c]pyridin (220);
2-(4-Isopropyl-5-(8-methyl-[1,2,4]triazolo[1,5-a]pyridin-6-yl)-1H-pyrazol-3-yl)-4,5,6,7-tetrahydrothiazolo[4,5-c]pyridin (221);
2-(4-Isopropyl-5-(8-methyl-[1,2,4]triazolo[1,5-a]pyridin-6-yl)-1H-pyrazol-3-yl)-5,6,7,8-tetrahydropyrido[4,3-d]pyrimidin (222);
6-(4-Isopropyl-3-(pyridin-4-yl)-1H-pyrazol-5-yl)-8-methyl-[1,2,4]triazolo[1,5a]pyridin (223);
6-(4-Isopropyl-3-(pyridin-3-yl)-1H-pyrazol-5-yl)-8-methyl-[1,2,4]triazolo[1,5a]pyridin (224);
5-(4-Isopropyl-5-(8-methyl-[1,2,4]triazolo[1,5-a]pyridin-6-yl)-1H-pyrazol-3-yl)pyridin-3-amin (225);
5-(4-Isopropyl-5-(8-methyl-[1,2,4]triazolo[1,5-a]pyridin-6-yl)-1H-pyrazol-3-yl)pyrazin-2-amin (226);
5-(4-Isopropyl-5-(8-methyl-[1,2,4]triazolo[1,5-a]pyridin-6-yl)-1H-pyrazol-3-yl)pyridin-2-amin (227);
5-(4-Isopropyl-5-(8-methyl-[1,2,4]triazolo[1,5-a]pyridin-6-yl)-1H-pyrazol-3-yl)pyrimidin-2-amin (228);
4-(4-Isopropyl-5-(8-methyl-[1,2,4]triazolo[1,5-a]pyridin-6-yl)-1H-pyrazol-3-yl)benzamid (229);
3-(4-Isopropyl-5-(8-methyl-[1,2,4]triazolo[1,5-a]pyridin-6-yl)-1H-pyrazol-3-yl)5,6,7,8-tetrahydro-1,6-naphthyridin (230);
7-(4-Isopropyl-5-(8-methyl-[1,2,4]triazolo[1,5-a]pyridin-6-yl)-1H-pyrazol-3-yl)-1,2,3,4-tetrahydroisochinolin (231);
3-(4-Isopropyl-5-(8-methyl-[1,2,4]triazolo[1,5-a]pyridin-6-yl)-1H-pyrazol-3-yl)anilin (232);
4-(4-Isopropyl-5-(8-methyl-[1,2,4]triazolo[1,5-a]pyridin-6-yl)-1H-pyrazol-3-yl)anilin (233);
6-(4-Isopropyl-5-(8-methoxy-[1,2,4]triazolo[1,5-a]pyridin-6-yl)-1H-pyrazol-3-yl)-1,2,3,4-tetrahydroisochinolin (234);
6-(4-Isopropyl-5-(8-methyl-[1,2,4]triazolo[1,5-a]pyridin-6-yl)-1H-pyrazol-3-yl)-2-methyl-1,2,3,4-tetrahydroisochinolin (235);
5-(4-Isopropyl-5-(8-methyl-[1,2,4]triazolo[1,5-a]pyridin-6-yl)-1H-pyrazol-3-yl)-N,N-dimethylpyrazin-2-amin (236);
5-(4-Isopropyl-5-(8-methyl-[1,2,4]triazolo[1,5-a]pyridin-6-yl)-1H-pyrazol-3-yl)-N,N-dimethylpyridin-2-amin (237);
5-(4-Isopropyl-5-(8-methyl-[1,2,4]triazolo[1,5-a]pyridin-6-yl)-1H-pyrazol-3-yl)-N,N-dimethylpyridin-3-amin (238);
N-Isopropyl-5-(4-isopropyl-5-(8-methyl-[1,2,4]triazolo[1,5-a]pyridin-6-yl)-1H-pyrazol-3-yl)pyridin-3-amin (239);
N-Isopropyl-5-(4-isopropyl-5-(8-methyl-[1,2,4]triazolo[1,5-a]pyridin-6-yl)-1H-pyrazol-3-yl)pyridin-2-amin (240);
N-Isopropyl-5-(4-isopropyl-5-(8-methyl-[1,2,4]triazolo[1,5-a]pyridin-6-yl)-1H-pyrazol-3-yl)pyrazin-2-amin (241);
N-Isopropyl-3-(4-isopropyl-5-(8-methyl-[1,2,4]triazolo[1,5-a]pyridin-6-yl)-1H-pyrazol-3-yl)anilin (242);
4-(4-Isopropyl-5-(8-methyl-[1,2,4]triazolo[1,5-a]pyridin-6-yl)-1H-pyrazol-3-yl)-N,N-dimethylanilin (243);
N-Isopropyl-4-(4-isopropyl-5-(8-methyl-[1,2,4]triazolo[1,5-a]pyridin-6-yl)-1H-pyrazol-3-yl)anilin (244);
3-(4-Isopropyl-5-(8-methyl-[1,2,4]triazolo[1,5-a]pyridin-6-yl)-1H-pyrazol-3-yl)-N,N-dimethylanilin (245);
2-Isopropyl-6-(4-isopropyl-5-(8-methyl-[1,2,4]triazolo[1,5-a]pyridin-6-yl)-1H-pyrazol-3-yl)-1,2,3,4-tetrahydroisochinolin (246);
6-(4-Isopropyl-5-(8-methyl-[1,2,4]triazolo[1,5-a]pyridin-6-yl)-1H-pyrazol-3-yl)-2-(oxetan-3-yl)-1,2,3,4-tetrahydroisochinolin (247);
2-(4-Isopropyl-5-(8-methyl-[1,2,4]triazolo[1,5-a]pyridin-6-yl)-1H-pyrazol-3-yl)-6-methyl-5,6,7,8-tetrahydropyrido[4,3-d]pyrimidin (248);
6-Ethyl-2-(4-isopropyl-5-(8-methyl-[1,2,4]triazolo[1,5-a]pyridin-6-yl)-1H-pyrazol-3-yl)-5,6,7,8-tetrahydropyrido[4,3-d]pyrimidin (249);
6-Isopropyl-2-(4-isopropyl-5-(8-methyl-[1,2,4]triazolo[1,5-a]pyridin-6-yl)-1H-pyrazol-3-yl)-5,6,7,8-tetrahydropyrido[4,3-d]pyrimidin (250);
2-(4-Isopropyl-5-(8-methyl-[1,2,4]triazolo[1,5-a]pyridin-6-yl)-1H-pyrazol-3-yl)-6-(oxetan-3-yl)-5,6,7,8-tetrahydropyrido[4,3-d]pyrimidin (251);
5-Isopropyl-2-(4-isopropyl-5-(8-methyl-[1,2,4]triazolo[1,5-a]pyridin-6-yl)-1H-pyrazol-3-yl)-4,5,6,7-tetrahydrothiazolo[5,4-c]pyridin (252);
2-(4-Isopropyl-5-(8-methyl-[1,2,4]triazolo[1,5-a]pyridin-6-yl)-1H-pyrazol-3-yl)-5-(oxetan-3-yl)-4,5,6,7-tetrahydrothiazolo[5,4-c]pyridin (253);
2-(4-Isopropyl-5-(8-methyl-[1,2,4]triazolo[1,5-a]pyridin-6-yl)-1H-pyrazol-3-yl)-5-(1-isopropylpiperidin-4-yl)-4,5,6,7-tetrahydrothiazolo[5,4-c]pyridin (254);
5-Isobutyl-2-(4-isopropyl-5-(8-methyl-[1,2,4]triazolo[1,5-a]pyridin-6-yl)-1H-pyrazol-3-yl)-4,5,6,7-tetrahydrothiazolo[5,4-c]pyridin (255);
2-(4-Isopropyl-5-(8-methyl-[1,2,4]triazolo[1,5-a]pyridin-6-yl)-1H-pyrazol-3-yl)-5-methyl-4,5,6,7-tetrahydrothiazolo[5,4-c]pyridin (256);
N-Ethyl-2-(4-isopropyl-5-(8-methyl-[1,2,4]triazolo[1,5-a]pyridin-6-yl)-1H-pyrazol-3-yl)benzo[d]thiazol-6-amin (257);
N-Isopropyl-2-(4-isopropyl-5-(8-methyl-[1,2,4]triazolo[1,5-a]pyridin-6-yl)-1H-pyrazol-3-yl)benzo[d]thiazol-6-amin (258);
5-Isopropyl-2-(4-isopropyl-5-(8-methoxy-[1,2,4]triazolo[1,5-a]pyridin-6-yl)-1H-pyrazol-3-yl)-4,5,6,7-tetrahydrothiazolo[5,4-c]pyridin (259);
2-(4-Isopropyl-5-(8-methoxy-[1,2,4]triazolo[1,5-a]pyridin-6-yl)-1H-pyrazol-3-yl)-5-propyl-4,5,6,7-tetrahydrothiazolo[5,4-c]pyridin (260);
5-Ethyl-2-(4-isopropyl-5-(8-methoxy-[1,2,4]triazolo[1,5-a]pyridin-6-yl)-1H-pyrazol-3-yl)-4,5,6,7-tetrahydrothiazolo[5,4-c]pyridin (261);
2-(4-Isopropyl-5-(8-methoxy-[1,2,4]triazolo[1,5-a]pyridin-6-yl)-1H-pyrazol-3-yl)-5-methyl-4,5,6,7-tetrahydrothiazolo[5,4-c]pyridin (262);
6-(4-Isopropyl-5-(8-methyl-[1,2,4]triazolo[1,5-a]pyridin-6-yl)-1H-pyrazol-3-yl)-2-(2-methoxyethyl)-1,2,3,4-tetrahydroisochinolin (263);
1-(6-(4-Isopropyl-5-(8-methyl-[1,2,4]triazolo[1,5-a]pyridin-6-yl)-1H-pyrazol-3-yl)-3,4-dihydroisochinolin-2(1H)-yl)-2-methylpropan-2-ol (264);
1-(2-(4-Isopropyl-5-(8-methyl-[1,2,4]triazolo[1,5-a]pyridin-6-yl)-1H-pyrazol-3-yl)-6,7-dihydrothiazolo[5,4-c]pyridin-5(4H)-yl)-2-methylpropan-2-ol (265);
2-(4-Isopropyl-5-(8-methyl-[1,2,4]triazolo[1,5-a]pyridin-6-yl)-1H-pyrazol-3-yl)-5-(2-methoxyethyl)-4,5,6,7-tetrahydrothiazolo[5,4-c]pyridin (266);
2-(4-Isopropyl-5-(8-methyl-[1,2,4]triazolo[1,5-a]pyridin-6-yl)-1H-pyrazol-3-yl)-N-(2-methoxyethyl)benzo[d]thiazol-6-amin (267);
2-(Dimethylamino)-1-(6-(4-isopropyl-5-(8-methyl-[1,2,4]triazolo[1,5-a]pyridin-6-yl)-1H-pyrazol-3-yl)-3,4-dihydroisochinolin-2(1H)-yl)ethan-1-on (268);
(S)-3-Hydroxy-1-(2-(4-isopropyl-5-(8-methyl-[1,2,4]triazolo[1,5-a]pyridin-6-yl)-1H-pyrazol-3-yl)-6,7-dihydrothiazolo[5,4-c]pyridin-5(4H)-yl)butan-1-on (269);
2-(Dimethylamino)-1-(2-(4-isopropyl-5-(8-methyl-[1,2,4]triazolo[1,5-a]pyridin-6-yl)-1H-pyrazol-3-yl)-6,7-dihydrothiazolo[5,4-c]pyridin-5(4H)-yl)ethan-1-on (270);
2-(6-(4-Isopropyl-5-(8-methyl-[1,2,4]triazolo[1,5-a]pyridin-6-yl)-1H-pyrazol-3-yl)-3,4-dihydroisochinolin-2(1H)-yl)-N,N-dimethylacetamid (271);
2-(6-(4-Isopropyl-5-(8-methyl-[1,2,4]triazolo[1,5-a]pyridin-6-yl)-1H-pyrazol-3-yl)-3,4-dihydroisochinolin-2(1H)-yl)acetamid (272);
2-(6-(4-Isopropyl-5-(8-methyl-[1,2,4]triazolo[1,5-a]pyridin-6-yl)-1H-pyrazol-3-yl)-3,4-dihydroisochinolin-2(1H)-yl)-N-methylacetamid (273);
2-(2-(4-Isopropyl-5-(8-methyl-[1,2,4]triazolo[1,5-a]pyridin-6-yl)-1H-pyrazol-3-yl)-6,7-dihydrothiazolo[5,4-c]pyridin-5(4H)-yl)-N-methylacetamid (274);
2-(2-(4-Isopropyl-5-(8-methyl-[1,2,4]triazolo[1,5-a]pyridin-6-yl)-1H-pyrazol-3-yl)-6,7-dihydrothiazolo[5,4-c]pyridin-5(4H)-yl)-N,N-dimethylacetamid (275);
1-(2-(4-Isopropyl-5-(8-methyl-[1,2,4]triazolo[1,5-a]pyridin-6-yl)-1H-pyrazol-3-yl)-6,7-dihydrothiazolo[5,4-c]pyridin-5(4H)-yl)ethan-1-on (276);
6-(4-Isopropyl-5-(8-methyl-[1,2,4]triazolo[1,5-a]pyridin-6-yl)-1H-pyrazol-3-yl)pyridin-2-ol (277);
6-(5-(2,6-Dimethylpyridin-4-yl)-4-isopropyl-1H-pyrazol-3-yl)-1,2,3,4-tetrahydroisochinolin (278);
N-(2,2-Difluorethyl)-4-(4-isopropyl-5-(8-methyl-[1,2,4]triazolo[1,5-a]pyridin-6-yl)-1H-pyrazol-3-yl)cyclohexan-1-amin (279);
4-(4-Isopropyl-5-(8-methyl-[1,2,4]triazolo[1,5-a]pyridin-6-yl)-1H-pyrazol-3-yl)-N-((3-methyloxetan-3-yl)methyl)cyclohexan-1-amin (280-281);
N-Ethyl-4-(4-isopropyl-5-(8-methyl-[1,2,4]triazolo[1,5-a]pyridin-6-yl)-1H-pyrazol-3-yl)-N-methylcyclohexan-1-amin (282-283);
N1-(4-(4-Isopropyl-5-(8-methyl-[1,2,4]triazolo[1,5-a]pyridin-6-yl)-1H-pyrazol-3-yl)cyclohexyl)-N2,N2-dimethylethan-1,2-diamin (284-285);
6-(4-Isopropyl-3-(4-(4-methylpiperazin-1-yl)cyclohexyl)-1H-pyrazol-5-yl)-8-methyl-[1,2,4]triazolo[1,5-a]pyridin (286-287);
1-Isopropyl-N-(4-(4-isopropyl-5-(8-methyl-[1,2,4]triazolo[1,5-a]pyridin-6-yl)-1H-pyrazol-3-yl)cyclohexyl)piperidin-4-amin (288-289);
4-(4-(4-Isopropyl-5-(8-methyl-[1,2,4]triazolo[1,5-a]pyridin-6-yl)-1H-pyrazol-3-yl)cyclohexyl)morpholin (290-291);
1-(4-(4-Isopropyl-5-(8-methyl-[1,2,4]triazolo[1,5-a]pyridin-6-yl)-1H-pyrazol-3-yl)cyclohexyl)-N,N-dimethylpiperidin-4-amin (292-293);
4-(4-Isopropyl-5-(8-methyl-[1,2,4]triazolo[1,5-a]pyridin-6-yl)-1H-pyrazol-3-yl)-N-methylcyclohexan-1-amin (294-295);
2-(Dimethylamino)-N-(4-(4-isopropyl-5-(8-methyl-[1,2,4]triazolo[1,5-a]pyridin-6-yl)-1H-pyrazol-3-yl)cyclohexyl)-N-methylacetamid (296-297);
2-((4-(4-Isopropyl-5-(8-methyl-[1,2,4]triazolo[1,5-a]pyridin-6-yl)-1H-pyrazol-3-yl)cyclohexyl)(methyl)amino)-N,N-dimethylacetamid (298-299);
N-(4-(4-Isopropyl-5-(8-methyl-[1,2,4]triazolo[1,5-a]pyridin-6-yl)-1H-pyrazol-3-yl)cyclohexyl)-N,1-dimethylazetidin-3-carboxamid (300 und 302);
N-(4-(4-Isopropyl-5-(8-methyl-[1,2,4]triazolo[1,5-a]pyridin-6-yl)-1H-pyrazol-3-yl)cyclohexyl)-N-methylazetidin-3-carboxamid (300B und 301);
1-Isopropyl-N-(4-(4-isopropyl-5-(8-methyl-[1,2,4]triazolo[1,5-a]pyridin-6-yl)-1H-pyrazol-3-yl)cyclohexyl)-N-methylazetidin-3-carboxamid (303-304);
1-Ethyl-N-(4-(4-isopropyl-5-(8-methyl-[1,2,4]triazolo[1,5-a]pyridin-6-yl)-1H-pyrazol-3-yl)cyclohexyl)-N-methylazetidin-3-carboxamid (305-306);
6-(4-Isopropyl-3-(4-(1-isopropylpiperidin-4-yl)phenyl)-1H-pyrazol-5-yl)-8-methyl-[1,2,4]triazolo[1,5-a]pyridin (307);
6-(4-Isopropyl-3-(4-(piperidin-4-yl)phenyl)-1H-pyrazol-5-yl)-8-methoxy-[1,2,4]triazolo[1,5-a]pyridin (308);
6-(4-Isopropyl-3-(4-(1-methylpiperidin-4-yl)phenyl)-1H-pyrazol-5-yl)-8-methyl-[1,2,4]triazolo[1,5-a]pyridin (309);
6-(3-(4-(1-Ethylpiperidin-4-yl)phenyl)-4-isopropyl-1H-pyrazol-5-yl)-8-methyl-[1,2,4]triazolo[1,5-a]pyridin (310);
6-(4-Isopropyl-3-(4-(1-(oxetan-3-yl)piperidin-4-yl)phenyl)-1H-pyrazol-5-yl)-8-methyl-[1,2,4]triazolo[1,5-a]pyridin (311);
6-(4-Isopropyl-3-(4-(1-((3-methyloxetan-3-yl)methyl)piperidin-4-yl)phenyl)-1H-pyrazol-5-yl)-8-methoxy-[1,2,4]triazolo[1,5-a]pyridin (312);
6-(4-Isopropyl-3-(4-(1-(tetrahydro-2H-pyran-4-yl)piperidin-4-yl)phenyl)-1H-pyrazol-5-yl)-8-methoxy-[1,2,4]triazolo[1,5-a]pyridin (313);
6-(4-Isopropyl-3-(4-(1-methylpiperidin-4-yl)phenyl)-1H-pyrazol-5-yl)-8-methoxy-[1,2,4]triazolo[1,5-a]pyridin (314);
6-(4-Isopropyl-3-(4-(1-methylpiperidin-4-yl)phenyl)-1H-pyrazol-5-yl)-8-methoxy-[1,2,4]triazolo[1,5-a]pyridin (315);
6-(4-Isopropyl-3-(4-(1-isopropylpiperidin-4-yl)phenyl)-1H-pyrazol-5-yl)-8-methoxy-[1,2,4]triazolo[1,5-a]pyridin (316);
6-(4-Isopropyl-3-(4-(1-neopentylpiperidin-4-yl)phenyl)-1H-pyrazol-5-yl)-8-methyl-[1,2,4]triazolo[1,5-a]pyridin (317);
6-(4-Isopropyl-3-(4-(1-(2-methoxyethyl)piperidin-4-yl)phenyl)-1H-pyrazol-5-yl)-8-methoxy-[1,2,4]triazolo[1,5-a]pyridin (318);
1-(4-(4-(4-Isopropyl-5-(8-methoxy-[1,2,4]triazolo[1,5-a]pyridin-6-yl)-1H-pyrazol-3-yl)phenyl)piperidin-1-yl)-2-methylpropan-2-ol (319);
6-(4-Isopropyl-3-(4-(1-(2,2,2-trifluorethyl)piperidin-4-yl)phenyl)-1H-pyrazol-5-yl)-8-methoxy-[1,2,4]triazolo[1,5-a]pyridin (320);
2-(4-(4-(4-Isopropyl-5-(8-methyl-[1,2,4]triazolo[1,5-a]pyridin-6-yl)-1H-pyrazol-3-yl)phenyl)piperidin-1-yl)-N,N-dimethylacetamid (321);
2-(4-(4-(4-Isopropyl-5-(8-methoxy-[1,2,4]triazolo[1,5-a]pyridin-6-yl)-1H-pyrazol-3-yl)phenyl)piperidin-1-yl)acetamid (322);
2-(Dimethylamino)-1-(4-(4-(4-isopropyl-5-(8-methyl-[1,2,4]triazolo[1,5-a]pyridin-6-yl)-1H-pyrazol-3-yl)phenyl)piperidin-1-yl)ethan-1-on (323);
6-(4-Isopropyl-3-(4-(1-methylazetidin-3-yl)phenyl)-1H-pyrazol-5-yl)-8-methyl[1,2,4]triazolo[1,5-a]pyridin (324);
6-(3-(4-(Azetidin-3-yl)phenyl)-4-isopropyl-1H-pyrazol-5-yl)-8-methoxy-[1,2,4]triazolo[1,5-a]pyridin (324E);
6-(3-(4-(1-Ethylazetidin-3-yl)phenyl)-4-isopropyl-1H-pyrazol-5-yl)-8-methyl-[1,2,4]triazolo[1,5-a]pyridin (325);
6-(4-Isopropyl-3-(4-(1-propylazetidin-3-yl)phenyl)-1H-pyrazol-5-yl)-8-methyl-[1,2,4]triazolo[1,5-a]pyridin (326);
6-(4-Isopropyl-3-(4-(1-isopropylazetidin-3-yl)phenyl)-1H-pyrazol-5-yl)-8-methyl-[1,2,4]triazolo[1,5-a]pyridin (327);
6-(4-Isopropyl-3-(4-(1-methylazetidin-3-yl)phenyl)-1H-pyrazol-5-yl)-8-methoxy-[1,2,4]triazolo[1,5-a]pyridin (328);
6-(3-(4-(1-Ethylazetidin-3-yl)phenyl)-4-isopropyl-1H-pyrazol-5-yl)-8-methoxy-[1,2,4]triazolo[1,5-a]pyridin (329);
6-(4-Isopropyl-3-(4-(1-propylazetidin-3-yl)phenyl)-1H-pyrazol-5-yl)-8-methoxy-[1,2,4]triazolo[1,5-a]pyridin (330);
6-(4-Isopropyl-3-(4-(1-isopropylazetidin-3-yl)phenyl)-1H-pyrazol-5-yl)-8-methoxy-[1,2,4]triazolo[1,5-a]pyridin (331);
6-(4-Isopropyl-3-(4-(1-(oxetan-3-yl)azetidin-3-yl)phenyl)-1H-pyrazol-5-yl)-8-methoxy-[1,2,4]triazolo[1,5-a]pyridin (332);
6-(4-Isopropyl-3-(4-(1-(tetrahydro-2H-pyran-4-yl)azetidin-3-yl)phenyl)-1H-pyrazol-5-yl)-8-methoxy-[1,2,4]triazolo[1,5-a]pyridin (333);
2-(Dimethylamino)-1-(3-(4-(4-isopropyl-5-(8-methyl-[1,2,4]triazolo[1,5-a]pyridin-6-yl)-1H-pyrazol-3-yl)phenyl)azetidin-1-yl)ethan-1-on (334);
2-(Dimethylamino)-1-(3-(4-(4-isopropyl-5-(8-methoxy-[1,2,4]triazolo[1,5-a]pyridin-6-yl)-1H-pyrazol-3-yl)phenyl)azetidin-1-yl)ethan-1-on (335);
1-(3-(4-(4-Isopropyl-5-(8-methyl-[1,2,4]triazolo[1,5-a]pyridin-6-yl)-1H-pyrazol-3-yl)phenyl)azetidin-1-yl)-2-methylpropan-2-ol (336);
1-(3-(4-(4-Isopropyl-5-(8-methoxy-[1,2,4]triazolo[1,5-a]pyridin-6-yl)-1H-pyrazol-3-yl)phenyl)azetidin-1-yl)-2-methylpropan-2-ol (337);
6-(4-Isopropyl-3-(4-(1-(2-(methylsulfonyl)ethyl)azetidin-3-yl)phenyl)-1H-pyrazol-5-yl)-8-methoxy-[1,2,4]triazolo[1,5-a]pyridin (338);
6-(4-Isopropyl-3-(5-(piperidin-4-yl)pyridin-2-yl)-1H-pyrazol-5-yl)-8-methoxy-[1,2,4]triazolo[1,5-a]pyridin (339);
6-(4-Isopropyl-3-(6-(piperidin-4-yl)pyridin-3-yl)-1H-pyrazol-5-yl)-8-methyl-[1,2,4]triazolo[1,5-a]pyridin (340);
6-(4-Isopropyl-3-(5-(piperidin-4-yl)pyrimidin-2-yl)-1H-pyrazol-5-yl)-8-methyl-[1,2,4]triazolo[1,5-a]pyridin (341);
6-(4-Isopropyl-3-(5-(piperidin-4-yl)pyridin-2-yl)-1H-pyrazol-5-yl)-8-methyl-[1,2,4]triazolo[1,5-a]pyridin (342);
6-(4-Isopropyl-3-(2-(piperidin-4-yl)pyrimidin-5-yl)-1H-pyrazol-5-yl)-8-methyl-[1,2,4]triazolo[1,5-a]pyridin (343);
6-(4-Isopropyl-3-(6-(piperidin-4-yl)pyridazin-3-yl)-1H-pyrazol-5-yl)-8-methyl-[1,2,4]triazolo[1,5-a]pyridin (344);
6-(4-Isopropyl-3-(5-(piperidin-4-yl)pyrazin-2-yl)-1H-pyrazol-5-yl)-8-methoxy-[1,2,4]triazolo[1,5-a]pyridin (345);
6-(4-Isopropyl-3-(5-(piperidin-4-yl)pyridin-2-yl)-1H-pyrazol-5-yl)-8-methoxy-7-methyl-[1,2,4]triazolo[1,5-a]pyridin (346);
6-(3-(5-(1-Ethylpiperidin-4-yl)pyridin-2-yl)-4-isopropyl-1H-pyrazol-5-yl)-8-methoxy-[1,2,4]triazolo[1,5-a]pyridin (347);
6-(4-Isopropyl-3-(5-(1-methylpiperidin-4-yl)pyridin-2-yl)-1H-pyrazol-5-yl)-8-methoxy-[1,2,4]triazolo[1,5-a]pyridin (348);
6-(4-Isopropyl-3-(5-(1-propylpiperidin-4-yl)pyridin-2-yl)-1H-pyrazol-5-yl)-8-methoxy-[1,2,4]triazolo[1,5-a]pyridin (349);
6-(4-Isopropyl-3-(5-(1-isopropylpiperidin-4-yl)pyridin-2-yl)-1H-pyrazol-5-yl)-8-methoxy-[1,2,4]triazolo[1,5-a]pyridin (350);
6-(4-Isopropyl-3-(5-(1-neopentylpiperidin-4-yl)pyridin-2-yl)-1H-pyrazol-5-yl)-8-methoxy-[1,2,4]triazolo[1,5-a]pyridin (351);
6-(4-Isopropyl-3-(5-(1-(3,3,3-trifluorpropyl)piperidin-4-yl)pyridin-2-yl)-1H-pyrazol-5-yl)-8-methoxy-[1,2,4]triazolo[1,5-a]pyridin (352);
6-(4-Isopropyl-3-(5-(1-((1-methyl-1H-1,2,4-triazol-3-yl)methyl)piperidin-4-yl)pyridin-2-yl)-1H-pyrazol-5-yl)-8-methoxy-[1,2,4]triazolo[1,5-a]pyridin (353);
6-(3-(5-(1-(3-Ethoxycyclobutyl)piperidin-4-yl)pyridin-2-yl)-4-isopropyl-1H-pyrazol-5-yl)-8-methoxy-[1,2,4]triazolo[1,5-a]pyridin (354);
6-(3-(5-(1-(3-Ethoxycyclobutyl)piperidin-4-yl)pyridin-2-yl)-4-isopropyl-1H-pyrazol-5-yl)-8-methoxy-[1,2,4]triazolo[1,5-a]pyridin (355);
6-(4-Isopropyl-3-(5-(1-(oxetan-3-yl)piperidin-4-yl)pyridin-2-yl)-1H-pyrazol-5-yl)-8-methoxy-[1,2,4]triazolo[1,5-a]pyridin (356);
6-(4-Isopropyl-3-(6-(1-methylpiperidin-4-yl)pyridazin-3-yl)-1H-pyrazol-5-yl)-8-methyl-[1,2,4]triazolo[1,5-a]pyridin (357);
6-(4-Isopropyl-3-(6-(1-isopropylpiperidin-4-yl)pyridazin-3-yl)-1H-pyrazol-5-yl)-8-methyl-[1,2,4]triazolo[1,5-a]pyridin (358);
6-(4-Isopropyl-3-(6-(1-methylpiperidin-4-yl)pyridin-3-yl)-1H-pyrazol-5-yl)-8-methyl-[1,2,4]triazolo[1,5-a]pyridin (359);
6-(3-(6-(1-Ethylpiperidin-4-yl)pyridin-3-yl)-4-isopropyl-1H-pyrazol-5-yl)-8-methyl-[1,2,4]triazolo[1,5-a]pyridin (360);
6-(4-Isopropyl-3-(6-(1-isopropylpiperidin-4-yl)pyridin-3-yl)-1H-pyrazol-5-yl)-8-methyl-[1,2,4]triazolo[1,5-a]pyridin (361);
6-(4-Isopropyl-3-(6-(1-(oxetan-3-yl)piperidin-4-yl)pyridin-3-yl)-1H-pyrazol-5-yl)-8-methyl-[1,2,4]triazolo[1,5-a]pyridin (362);
6-(4-Isopropyl-3-(2-(1-isopropylpiperidin-4-yl)pyrimidin-5-yl)-1H-pyrazol-5-yl)-8-methyl-[1,2,4]triazolo[1,5-a]pyridin (363);
6-(4-Isopropyl-3-(2-(1-neopentylpiperidin-4-yl)pyrimidin-5-yl)-1H-pyrazol-5-yl)-8-methyl-[1,2,4]triazolo[1,5-a]pyridin (364);
6-(4-Isopropyl-3-(5-(1-methylpiperidin-4-yl)pyridin-2-yl)-1H-pyrazol-5-yl)-8-methyl-[1,2,4]triazolo[1,5-a]pyridin (365);
6-(3-(5-(1-Ethylpiperidin-4-yl)pyridin-2-yl)-4-isopropyl-1H-pyrazol-5-yl)-8-methyl-[1,2,4]triazolo[1,5-a]pyridin (366);
6-(4-Isopropyl-3-(5-(1-isopropylpiperidin-4-yl)pyridin-2-yl)-1H-pyrazol-5-yl)-8-methyl-[1,2,4]triazolo[1,5-a]pyridin (367);
6-(4-Isopropyl-3-(5-(1-methylpiperidin-4-yl) pyrimidin-2-yl)-1H-pyrazol-5-yl)-8-methyl-[1,2,4]triazolo[1,5-a]pyridin (368);
6-(3-(5-(1-Ethylpiperidin-4-yl)pyrimidin-2-yl)-4-isopropyl-1H-pyrazol-5-yl)-8-methyl-[1,2,4]triazolo[1,5-a]pyridin (369);
6-(4-Isopropyl-3-(5-(1-isopropylpiperidin-4-yl)pyrimidin-2-yl)-1H-pyrazol-5-yl)-8-methyl-[1,2,4]triazolo[1,5-a]pyridin (370);
6-(4-Isopropyl-3-(5-(1-neopentylpiperidin-4-yl)pyrimidin-2-yl)-1H-pyrazol-5-yl)-8-methyl-[1,2,4]triazolo[1,5-a]pyridin (371);
6-(4-Isopropyl-3-(5-(1-(3-methylbutan-2-yl)piperidin-4-yl)pyrimidin-2-yl)-1H-pyrazol-5-yl)-8-methyl-[1,2,4]triazolo[1,5-a]pyridin (372);
6-(4-Isopropyl-3-(5-(1-neopentylpiperidin-4-yl)pyridin-2-yl)-1H-pyrazol-5-yl)-8-methyl-[1,2,4]triazolo[1,5-a]pyridin (373);
6-(3-(5-(1-(Cyclopropylmethyl)piperidin-4-yl)pyridin-2-yl)-4-isopropyl-1H-pyrazol-5-yl)-8-methoxy-[1,2,4]triazolo[1,5-a]pyridin (374);
6-(4-Isopropyl-3-(5-(1-((tetrahydro-2H-pyran-4-yl)methyl)piperidin-4-yl)pyridin-2-yl)-1H-pyrazol-5-yl)-8-methoxy-[1,2,4]triazolo[1,5-a]pyridin (375);
6-(4-Isopropyl-3-(5-(1-((tetrahydro-2H-pyran-4-yl)methyl)piperidin-4-yl)pyrazin-2-yl)-1H-pyrazol-5-yl)-8-methoxy-[1,2,4]triazolo[1,5-a]pyridin (376);
6-(3-(5-(1-(Cyclobutylmethyl)piperidin-4-yl)pyrazin-2-yl)-4-isopropyl-1H-pyrazol-5-yl)-8-methoxy-[1,2,4]triazolo[1,5-a]pyridin (377);
6-(3-(5-(1-(Cyclopropylmethyl)piperidin-4-yl)pyrazin-2-yl)-4-isopropyl-1H-pyrazol-5-yl)-8-methoxy-[1,2,4]triazolo[1,5-a]pyridin (378);
6-(3-(5-(1-Isobutylpiperidin-4-yl)pyrazin-2-yl)-4-isopropyl-1H-pyrazol-5-yl)-8-methoxy-[1,2,4]triazolo[1,5-a]pyridin (379);
6-(4-Isopropyl-3-(5-(1-(tetrahydro-2H-pyran-4-yl)piperidin-4-yl)pyrazin-2-yl)-1H-pyrazol-5-yl)-8-methoxy-[1,2,4]triazolo[1,5-a]pyridin (380);
6-(4-Isopropyl-3-(5-(1-methylpiperidin-4-yl)pyrazin-2-yl)-1H-pyrazol-5-yl)-8-methoxy-[1,2,4]triazolo[1,5-a]pyridin (381);
6-(3-(5-(1-Ethylpiperidin-4-yl)pyrazin-2-yl)-4-isopropyl-1H-pyrazol-5-yl)-8-methoxy-[1,2,4]triazolo[1,5-a]pyridin (382);
6-(4-Isopropyl-3-(5-(1-propylpiperidin-4-yl)pyrazin-2-yl)-1H-pyrazol-5-yl)-8-methoxy-[1,2,4]triazolo[1,5-a]pyridin (383);
6-(4-Isopropyl-3-(5-(1-isopropylpiperidin-4-yl)pyrazin-2-yl)-1H-pyrazol-5-yl)-8-methoxy-[1,2,4]triazolo[1,5-a]pyridin (384);
6-(4-Isopropyl-3-(5-(1-(oxetan-3-yl)piperidin-4-yl)pyrazin-2-yl)-1H-pyrazol-5-yl)-8-methoxy-[1,2,4]triazolo[1,5-a]pyridin (385);
6-(4-Isopropyl-3-(5-(1-(pentan-3-yl)piperidin-4-yl)pyrazin-2-yl)-1H-pyrazol-5-yl)-8-methoxy-[1,2,4]triazolo[1,5-a]pyridin (386);
6-(3-(5-(1-Cyclobutylpiperidin-4-yl)pyrazin-2-yl)-4-isopropyl-1H-pyrazol-5-yl)-8-methoxy-[1,2,4]triazolo[1,5-a]pyridin (387);
6-(4-Isopropyl-3-(5-(1-((tetrahydro-2H-pyran-4-yl)methyl)piperidin-4-yl)pyridin-2-yl)-1H-pyrazol-5-yl)-8-methoxy-7-methyl-[1,2,4]triazolo[1,5-a]pyridin (388);
2-(4-(6-(4-Isopropyl-5-(8-methoxy-[1,2,4]triazolo[1,5-a]pyridin-6-yl)-1H-pyrazol-3-yl)pyridin-3-yl)piperidin-1-yl)-N,N-dimethylacetamid (389);
1-(4-(2-(4-Isopropyl-5-(8-methyl-[1,2,4]triazolo[1,5-a]pyridin-6-yl)-1H-pyrazol-3-yl)pyrimidin-5-yl)piperidin-1-yl)-2-methylpropan-2-ol (390);
1-(4-(6-(4-Isopropyl-5-(8-methoxy-[1,2,4]triazolo[1,5-a]pyridin-6-yl)-1H-pyrazol-3-yl)pyridin-3-yl)piperidin-1-yl)-2-methylpropan-2-ol (391);
6-(4-Isopropyl-3-(5-(1-(2-(methylsulfonyl)ethyl)piperidin-4-yl)pyridin-2-yl)-1H-pyrazol-5-yl)-8-methoxy-[1,2,4]triazolo[1,5-a]pyridin (392);
2-(4-(6-(4-Isopropyl-5-(8-methoxy-[1,2,4]triazolo[1,5-a]pyridin-6-yl)-1H-pyrazol-3-yl)pyridin-3-yl)piperidin-1-yl)acetamid (393);
2-(4-(6-(4-Isopropyl-5-(8-methoxy-[1,2,4]triazolo[1,5-a]pyridin-6-yl)-1H-pyrazol-3-yl)pyridin-3-yl)piperidin-1-yl)acetonitril (394);
6-(4-Isopropyl-3-(5-(1-(2-methoxyethyl)piperidin-4-yl)pyridin-2-yl)-1H-pyrazol-5-yl)-8-methoxy-[1,2,4]triazolo[1,5-a]pyridin (395);
6-(4-Isopropyl-3-(5-(1-(2-methoxyethyl)piperidin-4-yl)pyrazin-2-yl)-1H-pyrazol-5-yl)-8-methoxy-[1,2,4]triazolo[1,5-a]pyridin (396);
6-(4-Isopropyl-3-(5-(1-(2-(methylsulfonyl)ethyl)piperidin-4-yl)pyrazin-2-yl)-1H-pyrazol-5-yl)-8-methoxy-[1,2,4]triazolo[1,5-a]pyridin (397);
6-(3-(5-(1-(2-Fluorethyl)piperidin-4-yl)pyrazin-2-yl)-4-isopropyl-1H-pyrazol-5-yl)-8-methoxy-[1,2,4]triazolo[1,5-a]pyridin (398);
6-(3-(5-(1-(3-Fluorpropyl)piperidin-4-yl)pyrazin-2-yl)-4-isopropyl-1H-pyrazol-5-yl)-8-methoxy-[1,2,4]triazolo[1,5-a]pyridin (399);
2-(4-(5-(4-Isopropyl-5-(8-methoxy-[1,2,4]triazolo[1,5-a]pyridin-6-yl)-1H-pyrazol-3-yl)pyrazin-2-yl)piperidin-1-yl)-N-methylacetamid (400);
2-(4-(5-(4-Isopropyl-5-(8-methoxy-[1,2,4]triazolo[1,5-a]pyridin-6-yl)-1H-pyrazol-3-yl)pyrazin-2-yl)piperidin-1-yl)-N,N-dimethylacetamid (401);
2-(Dimethylamino)-1-(4-(6-(4-isopropyl-5-(8-methoxy-[1,2,4]triazolo[1,5-a]pyridin-6-yl)-1H-pyrazol-3-yl)pyridin-3-yl)piperidin-1-yl)ethan-1-on (402);
2-(Dimethylamino)-1-(4-(5-(4-isopropyl-5-(8-methoxy-[1,2,4]triazolo[1,5-a]pyridin-6-yl)-1H-pyrazol-3-yl)pyrazin-2-yl)piperidin-1-yl)ethan-1-on (403);
2-(Diethylamino)-1-(4-(5-(4-isopropyl-5-(8-methoxy-[1,2,4]triazolo[1,5-a]pyridin-6-yl)-1H-pyrazol-3-yl)pyrazin-2-yl)piperidin-1-yl)ethan-1-on (404);
1-(4-(4-Isopropyl-5-(8-methyl-[1,2,4]triazolo[1,5-a]pyridin-6-yl)-1H-pyrazol-3-yl)phenyl)-N,N-dimethylazetidin-3-amin (405);
6-(4-Isopropyl-3-(4-(piperazin-1-yl)phenyl)-1H-pyrazol-5-yl)-8-methyl-[1,2,4]triazolo[1,5-a]pyridin (406);
6-(4-Isopropyl-3-(2-(piperazin-1-yl)pyrimidin-5-yl)-1H-pyrazol-5-yl)-8-methyl-[1,2,4]triazolo[1,5-a]pyridin (407);
6-(4-Isopropyl-3-(4-(4-neopentylpiperazin-1-yl)phenyl)-1H-pyrazol-5-yl)-8-methyl-[1,2,4]triazolo[1,5-a]pyridin (408);
N-Isopropyl-1-(4-(4-isopropyl-5-(8-methyl-[1,2,4]triazolo[1,5-a]pyridin-6-yl)-1H-pyrazol-3-yl)phenyl)azetidin-3-amin (409);
6-(4-Isopropyl-3-(2-(4-methylpiperazin-1-yl)pyrimidin-5-yl)-1H-pyrazol-5-yl)-8-methyl-[1,2,4]triazolo[1,5-a]pyridin (410);
6-(4-Isopropyl-3-(4-(4-isopropylpiperazin-1-yl)phenyl)-1H-pyrazol-5-yl)-8-methyl-[1,2,4]triazolo[1,5-a]pyridin (411);
6-(4-Isopropyl-3-(4-(4-methylpiperazin-1-yl)phenyl)-1H-pyrazol-5-yl)-8-methyl-[1,2,4]triazolo[1,5-a]pyridin (412);
6-(3-(4-(4-Ethylpiperazin-1-yl)phenyl)-4-isopropyl-1H-pyrazol-5-yl)-8-methyl-[1,2,4]triazolo[1,5-a]pyridin (413);
6-(4-Isopropyl-3-(4-(4-(oxetan-3-yl)piperazin-1-yl)phenyl)-1H-pyrazol-5-yl)-8-methyl-[1,2,4]triazolo[1,5-a]pyridin (414);
6-(4-Isopropyl-3-(2-(4-isopropylpiperazin-1-yl)pyrimidin-5-yl)-1H-pyrazol-5-yl)-8-methyl-[1,2,4]triazolo[1,5-a]pyridin (415);
6-(4-Isopropyl-3-(2-(4-(oxetan-3-yl)piperazin-1-yl)pyrimidin-5-yl)-1H-pyrazol-5-yl)-8-methyl-[1,2,4]triazolo[1,5-a]pyridin (416);
6-(4-Isopropyl-3-(4-(4-(2-(methylsulfonyl)ethyl)piperazin-1-yl)phenyl)-1H-pyrazol-5-yl)-8-methyl-[1,2,4]triazolo[1,5-a]pyridin (417);
1-(4-(4-(4-Isopropyl-5-(8-methyl-[1,2,4]triazolo[1,5-a]pyridin-6-yl)-1H-pyrazol-3-yl)phenyl)piperazin-1-yl)-2-methylpropan-2-ol (418);
6-(4-Isopropyl-3-(5-(4-(2-methoxyethyl)piperazin-1-yl)pyridin-2-yl)-1H-pyrazol-5-yl)-8-methoxy-[1,2,4]triazolo[1,5-a]pyridin (419);
1-(4-(4-Isopropyl-5-(8-methyl-[1,2,4]triazolo[1,5-a]pyridin-6-yl)-1H-pyrazol-3-yl)phenyl)-4-methylpiperazin-2-on (420);
4-Isopropyl-1-(4-(4-isopropyl-5-(8-methyl-[1,2,4]triazolo[1,5-a]pyridin-6-yl)-1H-pyrazol-3-yl)phenyl)piperazin-2-on (421);
1-(4-(4-Isopropyl-5-(8-methyl-[1,2,4]triazolo[1,5-a]pyridin-6-yl)-1H-pyrazol-3-yl)phenyl)-4-(oxetan-3-yl)piperazin-2-on (422);
6-(3-(5-(4-Cyclobutylpiperazin-1-yl)pyridin-2-yl)-4-isopropyl-1H-pyrazol-5-yl)-8-methoxy-[1,2,4]triazolo[1,5-a]pyridin (423);
6-(3-(5-(2,6-Diazaspiro[3.3]heptan-2-yl)pyridin-2-yl)-4-isopropyl-1H-pyrazol-5-yl)-8-methoxy-[1,2,4]triazolo[1,5-a]pyridin (424);
4-(4-(6-(4-Isopropyl-5-(8-methoxy-[1,2,4]triazolo[1,5-a]pyridin-6-yl)-1H-pyrazol-3-yl)pyridin-3-yl)piperazin-1-yl)tetrahydro-2H-thiopyran 1,1-dioxid (425);
6-(3-(5-(4-(Cyclopropylmethyl)piperazin-1-yl)pyridin-2-yl)-4-isopropyl-1H-pyrazol-5-yl)-8-methoxy-[1,2,4]triazolo[1,5-a]pyridin (426);
6-(3-(5-(4-Ethylpiperazin-1-yl)pyridin-2-yl)-4-isopropyl-1H-pyrazol-5-yl)-8-methoxy-[1,2,4]triazolo[1,5-a]pyridin (427);
6-(4-Isopropyl-3-(5-(4-methylpiperazin-1-yl)pyridin-2-yl)-1H-pyrazol-5-yl)-8-methoxy-[1,2,4]triazolo[1,5-a]pyridin (428);
6-(4-Isopropyl-3-(5-(4-isopropylpiperazin-1-yl)pyridin-2-yl)-1H-pyrazol-5-yl)-8-methoxy-[1,2,4]triazolo[1,5-a]pyridin (429);
6-(3-(5-(4-Isobutylpiperazin-1-yl)pyridin-2-yl)-4-isopropyl-1H-pyrazol-5-yl)-8-methoxy-[1,2,4]triazolo[1,5-a]pyridin (430);
6-(4-Isopropyl-3-(5-(4-propylpiperazin-1-yl)pyridin-2-yl)-1H-pyrazol-5-yl)-8-methoxy-[1,2,4]triazolo[1,5-a]pyridin (431);
6-(4-Isopropyl-3-(5-(4-((tetrahydro-2H-pyran-4-yl)methyl)piperazin-1-yl)pyridin-2-yl)-1H-pyrazol-5-yl)-8-methoxy-[1,2,4]triazolo[1,5-a]pyridin (432);
6-(4-Isopropyl-3-(5-(4-(3,3,3-trifluorpropyl)piperazin-1-yl)pyridin-2-yl)-1H-pyrazol-5-yl)-8-methoxy-[1,2,4]triazolo[1,5-a]pyridin (433);
6-(3-(5-(4-(Cyclobutylmethyl)piperazin-1-yl)pyridin-2-yl)-4-isopropyl-1H-pyrazol-5-yl)-8-methoxy-[1,2,4]triazolo[1,5-a]pyridin (434);
6-(4-Isopropyl-3-(5-(4-(oxetan-3-yl)piperazin-1-yl)pyridin-2-yl)-1H-pyrazol-5-yl)-8-methoxy-[1,2,4]triazolo[1,5-a]pyridin (435);
6-(4-Isopropyl-3-(5-(4-(tetrahydro-2H-pyran-4-yl)piperazin-1-yl)pyridin-2-yl)-1H-pyrazol-5-yl)-8-methoxy-[1,2,4]triazolo[1,5-a]pyridin (436);
6-(4-Isopropyl-3-(5-(4-(pentan-3-yl)piperazin-1-yl)pyridin-2-yl)-1H-pyrazol-5-yl)-8-methoxy-[1,2,4]triazolo[1,5-a]pyridin (437);
6-(3-(5-(4-(2-Ethylbutyl)piperazin-1-yl)pyridin-2-yl)-4-isopropyl-1H-pyrazol-5-yl)-8-methoxy-[1,2,4]triazolo[1,5-a]pyridin (438);
6-(3-(5-(4-((3-Ethyloxetan-3-yl)methyl)piperazin-1-yl)pyridin-2-yl)-4-isopropyl-1H-pyrazol-5-yl)-8-methoxy-[1,2,4]triazolo[1,5-a]pyridin (439);
6-(4-Isopropyl-3-(5-(6-((tetrahydro-2H-pyran-4-yl)methyl)-2,6-diazaspiro[3.3]heptan-2-yl)pyridin-2-yl)-1H-pyrazol-5-yl)-8-methoxy-[1,2,4]triazolo[1,5-a]pyridin (440);
6-(3-(5-(6-(Cyclobutylmethyl)-2,6-diazaspiro[3.3]heptan-2-yl)pyridin-2-yl)-4-isopropyl-1H-pyrazol-5-yl)-8-methoxy-[1,2,4]triazolo[1,5-a]pyridin (441);
6-(3-(5-(6-Isobutyl-2,6-diazaspiro[3.3]heptan-2-yl)pyridin-2-yl)-4-isopropyl-1H-pyrazol-5-yl)-8-methoxy-[1,2,4]triazolo[1,5-a]pyridin (442);
6-(4-Isopropyl-3-(5-(6-(tetrahydro-2H-pyran-4-yl)-2,6-diazaspiro[3.3]heptan-2-yl)pyridin-2-yl)-1H-pyrazol-5-yl)-8-methoxy-[1,2,4]triazolo[1,5-a]pyridin (443);
6-(3-(5-(6-Cyclobutyl-2,6-diazaspiro[3.3]heptan-2-yl)pyridin-2-yl)-4-isopropyl-1H-pyrazol-5-yl)-8-methoxy-[1,2,4]triazolo[1,5-a]pyridin (444);
6-(4-Isopropyl-3-(5-(6-methyl-2,6-diazaspiro[3.3]heptan-2-yl)pyridin-2-yl)-1H-pyrazol-5-yl)-8-methoxy-[1,2,4]triazolo[1,5-a]pyridin (445);
6-(3-(5-(6-Ethyl-2,6-diazaspiro[3.3]heptan-2-yl)pyridin-2-yl)-4-isopropyl-1H-pyrazol-5-yl)-8-methoxy-[1,2,4]triazolo[1,5-a]pyridin (446);
6-(4-Isopropyl-3-(5-(6-propyl-2,6-diazaspiro[3.3]heptan-2-yl)pyridin-2-yl)-1H-pyrazol-5-yl)-8-methoxy-[1,2,4]triazolo[1,5-a]pyridin (447);
6-(4-Isopropyl-3-(5-(6-isopropyl-2,6-diazaspiro[3.3]heptan-2-yl)pyridin-2-yl)-1H-pyrazol-5-yl)-8-methoxy-[1,2,4]triazolo[1,5-a]pyridin (448);
6-(4-Isopropyl-3-(5-(6-(oxetan-3-yl)-2,6-diazaspiro[3.3]heptan-2-yl)pyridin-2-yl)-1H-pyrazol-5-yl)-8-methoxy-[1,2,4]triazolo[1,5-a]pyridin (449);
6-(3-(5-(6-(Cyclopropylmethyl)-2,6-diazaspiro[3.3]heptan-2-yl)pyridin-2-yl)-4-isopropyl-1H-pyrazol-5-yl)-8-methoxy-[1,2,4]triazolo[1,5-a]pyridin (450);
2-(Dimethylamino)-1-(4-(6-(4-isopropyl-5-(8-methoxy-[1,2,4]triazolo[1,5-a]pyridin-6-yl)-1H-pyrazol-3-yl)pyridin-3-yl)piperazin-1-yl)ethan-1-on (451);
2-(Diethylamino)-1-(4-(6-(4-isopropyl-5-(8-methoxy-[1,2,4]triazolo[1,5-a]pyridin-6-yl)-1H-pyrazol-3-yl)pyridin-3-yl)piperazin-1-yl)ethan-1-on (452);
2-(Dimethylamino)-1-(6-(6-(4-isopropyl-5-(8-methoxy-[1,2,4]triazolo[1,5-a]pyridin-6-yl)-1H-pyrazol-3-yl)pyridin-3-yl)-2,6-diazaspiro[3.3]heptan-2-yl)ethan-1-on (453);
6-(4-Isopropyl-3-(5-(4-(2-(methylsulfonyl)ethyl)piperazin-1-yl)pyridin-2-yl)-1H-pyrazol-5-yl)-8-methoxy-[1,2,4]triazolo[1,5-a]pyridin (454);
2-(4-(6-(4-Isopropyl-5-(8-methoxy-[1,2,4]triazolo[1,5-a]pyridin-6-yl)-1H-pyrazol-3-yl)pyridin-3-yl)piperazin-1-yl)-N,N-dimethylacetamid (455);
2-(4-(6-(4-Isopropyl-5-(8-methoxy-[1,2,4]triazolo[1,5-a]pyridin-6-yl)-1H-pyrazol-3-yl)pyridin-3-yl)piperazin-1-yl)-N-methylacetamid (456);
6-(4-Isopropyl-3-(5-(6-(2-(methylsulfonyl)ethyl)-2,6-diazaspiro[3.3]heptan-2-yl)pyridin-2-yl)-1H-pyrazol-5-yl)-8-methoxy-[1,2,4]triazolo[1,5-a]pyridin (457);
6-(4-Isopropyl-3-(5-(6-(2-methoxyethyl)-2,6-diazaspiro[3.3]heptan-2-yl)pyridin-2-yl)-1H-pyrazol-5-yl)-8-methoxy-[1,2,4]triazolo[1,5-a]pyridin (458);
2-(4-(4-Isopropyl-5-(8-methyl-[1,2,4]triazolo[1,5-a]pyridin-6-yl)-1H-pyrazol-3-yl)phenyl)-4-methylmorpholin (459-460);
4-Ethyl-2-(4-(4-isopropyl-5-(8-methyl-[1,2,4]triazolo[1,5-a]pyridin-6-yl)-1H-pyrazol-3-yl)phenyl)morpholin (461);
4-Isopropyl-2-(4-(4-isopropyl-5-(8-methyl-[1,2,4]triazolo[1,5-a]pyridin-6-yl)-1H-pyrazol-3-yl)phenyl)morpholin (462-463);
2-(4-(4-Isopropyl-5-(8-methyl-[1,2,4]triazolo[1,5-a]pyridin-6-yl)-1H-pyrazol-3-yl)phenyl)-4-neopentylmorpholin (464-465);
2-(2-(4-(4-Isopropyl-5-(8-methyl-[1,2,4]triazolo[1,5-a]pyridin-6-yl)-1H-pyrazol-3-yl)phenyl)morpholino)-N,N-dimethylacetamid (466-468);
2-(Dimethylamino)-1-(2-(4-(4-isopropyl-5-(8-methyl-[1,2,4]triazolo[1,5-a]pyridin-6-yl)-1H-pyrazol-3-yl)phenyl)morpholino)ethan-1-on (469-470);
4-(4-(4-Isopropyl-5-(8-methyl-[1,2,4]triazolo[1,5-a]pyridin-6-yl)-1H-pyrazol-3-yl)phenyl)-N-methylcyclohexan-1-amin (471);
4-(4-(4-Isopropyl-5-(8-methyl-[1,2,4]triazolo[1,5-a]pyridin-6-yl)-1H-pyrazol-3-yl)phenyl)-N,N-dimethylcyclohexan-1-amin (472);
N-Cyclobutyl-4-(6-(4-isopropyl-5-(8-methoxy-[1,2,4]triazolo[1,5-a]pyridin-6-yl)-1H-pyrazol-3-yl)pyridin-3-yl)cyclohexan-1-amin (473-474);
2-(4-(6-(4-Isopropyl-5-(8-methoxy-[1,2,4]triazolo[1,5-a]pyridin-6-yl)-1H-pyrazol-3-yl)pyridin-3-yl)cyclohexyl)-7-oxa-2-azaspiro[3.5]nonan (475-476);
6-(4-(6-(4-Isopropyl-5-(8-methoxy-[1,2,4]triazolo[1,5-a]pyridin-6-yl)-1H-pyrazol-3-yl)pyridin-3-yl)cyclohexyl)-2-oxa-6-azaspiro[3.3]heptan (477-478);
6-(4-Isopropyl-3-(5-(4-(3-methoxyazetidin-1-yl)cyclohexyl)pyridin-2-yl)-1H-pyrazol-5-yl)-8-methoxy-[1,2,4]triazolo[1,5-a]pyridin (479-480);
4-(6-(4-Isopropyl-5-(8-methoxy-[1,2,4]triazolo[1,5-a]pyridin-6-yl)-1H-pyrazol-3-yl)pyridin-3-yl)-N-(2-methoxy-2-methylpropyl)cyclohexan-1-amin (481-482);
4-(4-(6-(4-Isopropyl-5-(8-methoxy-[1,2,4]triazolo[1,5-a]pyridin-6-yl)-1H-pyrazol-3-yl)pyridin-3-yl)cyclohexyl)piperazin-2-on (483-484);
4-(6-(4-Isopropyl-5-(8-methoxy-[1,2,4]triazolo[1,5-a]pyridin-6-yl)-1H-pyrazol-3-yl)pyridin-3-yl)-N-((1-(methylsulfonyl)cyclopropyl)methyl)cyclohexan-1-amin (485-486);
7-(4-(6-(4-Isopropyl-5-(8-methoxy-[1,2,4]triazolo[1,5-a]pyridin-6-yl)-1H-pyrazol-3-yl)pyridin-3-yl)cyclohexyl)-2-oxa-7-azaspiro[3.5]nonan (487-488);
4-(6-(4-Isopropyl-5-(8-methoxy-[1,2,4]triazolo[1,5-a]pyridin-6-yl)-1H-pyrazol-3-yl)pyridin-3-yl)-N-(2-methoxyethyl)-N-methylcyclohexan-1-amin (490-491);
N -(4-(6-(4-Isopropyl-5-(8-methoxy-[1,2,4]triazolo[1,5-a]pyridin-6-yl)-1H-pyrazol-3-yl)pyridin-3-yl)cyclohexyl)-3-methyloxetan-3-amin (492-493);
4-(4-(6-(4-Isopropyl-5-(8-methoxy-[1,2,4]triazolo[1,5-a]pyridin-6-yl)-1H-pyrazol-3-yl)pyridin-3-yl)cyclohexyl)morpholin (494-495);
6-(4-Isopropyl-3-(5-(4-(3-methoxyazetidin-1-yl)cyclohexyl)-4-methylpyridin-2-yl)-1H-pyrazol-5-yl)-8-methoxy-[1,2,4]triazolo[1,5-a]pyridin (496);
4-(6-(4-Isopropyl-5-(8-methoxy-[1,2,4]triazolo[1,5-a]pyridin-6-yl)-1H-pyrazol-3-yl)-4-methylpyridin-3-yl)-N-((1-(methylsulfonyl)cyclopropyl)methyl)cyclohexan-1-amin (497-498);
4-(6-(4-Isopropyl-5-(8-methoxy-[1,2,4]triazolo[1,5-a]pyridin-6-yl)-1H-pyrazol-3-yl)-4-methylpyridin-3-yl)-N-(2-methoxyethyl)cyclohexan-1-amin (499-500);
7-(4-(6-(4-Isopropyl-5-(8-methoxy-[1,2,4]triazolo[1,5-a]pyridin-6-yl)-1H-pyrazol-3-yl)-4-methylpyridin-3-yl)cyclohexyl)-2-oxa-7-azaspiro[3.5]nonan (501);
6-(3-(5-(4-(4,4-Difluorpiperidin-1-yl)cyclohexyl)-4-methylpyridin-2-yl)-4-isopropyl-1H-pyrazol-5-yl)-8-methoxy-[1,2,4]triazolo[1,5-a]pyridin (502-503);
6-(4-Isopropyl-3-(5-(4-(4-methoxypiperidin-1-yl)cyclohexyl)-4-methylpyridin-2-yl)-1H-pyrazol-5-yl)-8-methoxy-[1,2,4]triazolo[1,5-a]pyridin (504-505);
4-(6-(4-Isopropyl-5-(8-methoxy-[1,2,4]triazolo[1,5-a]pyridin-6-yl)-1H-pyrazol-3-yl)-4-methylpyridin-3-yl)-N,N-dimethylcyclohexan-1-amin (506-507);
4-(6-(4-Isopropyl-5-(8-methoxy-[1,2,4]triazolo[1,5-a]pyridin-6-yl)-1H-pyrazol-3-yl)-4-methylpyridin-3-yl)cyclohexan-1-amin (508-509);
4-(4-(4-Isopropyl-5-(8-methyl-[1,2,4]triazolo[1,5-a]pyridin-6-yl)-1H-pyrazol-3-yl)phenyl)-N,N-dimethylcyclohexan-1-amin (510 und 513);
N-(4-(4-(4-Isopropyl-5-(8-methyl-[1,2,4]triazolo[1,5-a]pyridin-6-yl)-1H-pyrazol-3-yl)phenyl)cyclohexyl)-N-methyloxetan-3-amin (511 und 514);
N-((3-Ethyloxetan-3-yl)methyl)-4-(4-(4-isopropyl-5-(8-methyl-[1,2,4]triazolo[1,5-a]pyridin-6-yl)-1H-pyrazol-3-yl)phenyl)-N-methylcyclohexan-1-amin (512 und 515);
N-Ethyl-4-(4-(4-isopropyl-5-(8-methyl-[1,2,4]triazolo[1,5-a]pyridin-6-yl)-1H-pyrazol-3-yl)phenyl)-N-methylcyclohexan-1-amin (516-517);
2-(Dimethylamino)-N-(4-(4-(4-isopropyl-5-(8-methyl-[1,2,4]triazolo[1,5-a]pyridin-6-yl)-1H-pyrazol-3-yl)phenyl)cyclohexyl)-N-methylacetamid (518-519);
2-((4-(4-(4-Isopropyl-5-(8-methyl-[1,2,4]triazolo[1,5-a]pyridin-6-yl)-1H-pyrazol-3-yl)phenyl)cyclohexyl)(methyl)amino)-N,N-dimethylacetamid (520-521);
4-(4-(4-Isopropyl-5-(8-methyl-[1,2,4]triazolo[1,5-a]pyridin-6-yl)-1H-pyrazol-3-yl)phenyl)-N-(2-methoxyethyl)-N-methylcyclohexan-1-amin (522-523);
2-(4-(4-Isopropyl-5-(8-methoxy-[1,2,4]triazolo[1,5-a]pyridin-6-yl)-1H-pyrazol-3-yl)phenyl)propan-2-amin, TFA (524);
2-(4-(4-Isopropyl-5-(8-methyl-[1,2,4]triazolo[1,5-a]pyridin-6-yl)-1H-pyrazol-3-yl)phenyl)propan-2-amin (525);
1-(4-(4-Isopropyl-5-(8-methyl-[1,2,4]triazolo[1,5-a]pyridin-6-yl)-1H-pyrazol-3-yl)phenyl)cyclopropan-1-amin (526);
(S)-1-(4-(4-Isopropyl-5-(8-methyl-[1,2,4]triazolo[1,5-a]pyridin-6-yl)-1H-pyrazol-3-yl)phenyl)ethan-1-amin (527);
(S)-1-(4-(4-Isopropyl-5-(8-methoxy-[1,2,4]triazolo[1,5-a]pyridin-6-yl)-1H-pyrazol-3-yl)phenyl)ethan-1-amin (528);
(R)-1-(4-(4-Isopropyl-5-(8-methyl-[1,2,4]triazolo[1,5-a]pyridin-6-yl)-1H-pyrazol-3-yl)phenyl)ethan-1-amin (529);
6-(4-Isopropyl-3-(4-(pyrrolidin-2-yl)phenyl)-1H-pyrazol-5-yl)-8-methyl-[1,2,4]triazolo[1,5-a]pyridin (530-531);
2-(4-(4-Isopropyl-5-(8-methoxy-[1,2,4]triazolo[1,5-a]pyridin-6-yl)-1H-pyrazol-3-yl)phenyl)-N,N-dimethylpropan-2-amin (532);
N-Isopropyl-2-(4-(4-isopropyl-5-(8-methoxy-[1,2,4]triazolo[1,5-a]pyridin-6-yl)-1H-pyrazol-3-yl)phenyl)propan-2-amin (533);
N-Ethyl-2-(4-(4-isopropyl-5-(8-methoxy-[1,2,4]triazolo[1,5-a]pyridin-6-yl)-1H-pyrazol-3-yl)phenyl)propan-2-amin (534);
N,N-Diethyl-2-(4-(4-isopropyl-5-(8-methoxy-[1,2,4]triazolo[1,5-a]pyridin-6-yl)-1H-pyrazol-3-yl)phenyl)propan-2-amin (535);
N-Ethyl-2-(4-(4-isopropyl-5-(8-methyl-[1,2,4]triazolo[1,5-a]pyridin-6-yl)-1H-pyrazol-3-yl)phenyl)propan-2-amin (536);
N-(2-(4-(4-Isopropyl-5-(8-methoxy-[1,2,4]triazolo[1,5-a]pyridin-6-yl)-1H-pyrazol-3-yl)phenyl)propan-2-yl)tetrahydro-2H-pyran-4-amin (537),
2-(4-(4-Isopropyl-5-(8-methyl-[1,2,4]triazolo[1,5-a]pyridin-6-yl)-1H-pyrazol-3-yl)phenyl)-N,N-dimethylpropan-2-amin (538);
N,N-Diethyl-2-(4-(4-isopropyl-5-(8-methyl-[1,2,4]triazolo[1,5-a]pyridin-6-yl)-1H-pyrazol-3-yl)phenyl)propan-2-amin (539);
N-(2-(4-(4-Isopropyl-5-(8-methyl-[1,2,4]triazolo[1,5-a]pyridin-6-yl)-1H-pyrazol-3-yl)phenyl)propan-2-yl)tetrahydro-2H-pyran-4-amin (540);
N-(2-(4-(4-Isopropyl-5-(8-methyl-[1,2,4]triazolo[1,5-a]pyridin-6-yl)-1H-pyrazol-3-yl)phenyl)propan-2-yl)-2,2-dimethylpropan-1-amin (541);
2-(4-(4-Isopropyl-5-(8-methyl-[1,2,4]triazolo[1,5-a]pyridin-6-yl)-1H-pyrazol-3-yl)phenyl)-N-((3-methyloxetan-3-yl)methyl)propan-2-amin (542);
N-Isopropyl-2-(4-(4-isopropyl-5-(8-methyl-[1,2,4]triazolo[1,5-a]pyridin-6-yl)-1H-pyrazol-3-yl)phenyl)propan-2-amin (543);
2-(4-(4-Isopropyl-5-(8-methyl-[1,2,4]triazolo[1,5-a]pyridin-6-yl)-1H-pyrazol-3-yl)phenyl)-N-((1-methyl-1H-1,2,4-triazol-3-yl)methyl)propan-2-amin (544);
N-(2-(4-(4-Isopropyl-5-(8-methyl-[1,2,4]triazolo[1,5-a]pyridin-6-yl)-1H-pyrazol-3-yl)phenyl)propan-2-yl)oxetan-3-amin (545);
1-(4-(4-Isopropyl-5-(8-methyl-[1,2,4]triazolo[1,5-a]pyridin-6-yl)-1H-pyrazol-3-yl)phenyl)-N,N-dimethylcyclopropan-1-amin (546);
N-Isopropyl-1-(4-(4-isopropyl-5-(8-methyl-[1,2,4]triazolo[1,5-a]pyridin-6-yl)-1H-pyrazol-3-yl)phenyl)cyclopropan-1-amin (547);
1-(4-(4-Isopropyl-5-(8-methyl-[1,2,4]triazolo[1,5-a]pyridin-6-yl)-1H-pyrazol-3-yl)phenyl)-N-neopentylcyclopropan-1-amin (548);
(S)-1-(4-(4-Isopropyl-5-(8-methyl-[1,2,4]triazolo[1,5-a]pyridin-6-yl)-1H-pyrazol-3-yl)phenyl)-N,N-dimethylethan-1-amin (549);
(S)-N-Ethyl-1-(4-(4-isopropyl-5-(8-methyl-[1,2,4]triazolo[1,5-a]pyridin-6-yl)-1H-pyrazol-3-yl)phenyl)ethan-1-amin (550);
(S)-N-(1-(4-(4-Isopropyl-5-(8-methyl-[1,2,4]triazolo[1,5-a]pyridin-6-yl)-1H-pyrazol-3-yl)phenyl)ethyl)propan-2-amin (551);
N-((S)-1-(4-(4-Isopropyl-5-(8-methyl-[1,2,4]triazolo[1,5-a]pyridin-6-yl)-1H-pyrazol-3-yl)phenyl)ethyl)-3-methylbutan-2-amin (552-553);
(S)-N-(1-(4-(4-Isopropyl-5-(8-methyl-[1,2,4]triazolo[1,5-a]pyridin-6-yl)-1H-pyrazol-3-yl)phenyl)ethyl)-2,2-dimethylpropan-1-amin (554);
(S)-1-(4-(4-Isopropyl-5-(8-methoxy-[1,2,4]triazolo[1,5-a]pyridin-6-yl)-1H-pyrazol-3-yl)phenyl)-N,N-dimethylethan-1-amin (555)
(S)-N-(1-(4-(4-Isopropyl-5-(8-methoxy-[1,2,4]triazolo[1,5-a]pyridin-6-yl)-1H-pyrazol-3-yl)phenyl)ethyl)propan-2-amin (556);
(R)-1-(4-(4-Isopropyl-5-(8-methyl-[1,2,4]triazolo[1,5-a]pyridin-6-yl)-1H-pyrazol-3-yl)phenyl)-N,N-dimethylethan-1-amin (557);
(R)-N-(1-(4-(4-Isopropyl-5-(8-methyl-[1,2,4]triazolo[1,5-a]pyridin-6-yl)-1H-pyrazol-3-yl)phenyl)ethyl)propan-2-amin (558);
(R)-1-(4-(4-Isopropyl-5-(8-methyl-[1,2,4]triazolo[1,5-a]pyridin-6-yl)-1H-pyrazol-3-yl)phenyl)-N-((3-methyloxetan-3-yl)methyl)ethan-1-amin (559);
(R)-N-(1-(4-(4-Isopropyl-5-(8-methyl-[1,2,4]triazolo[1,5-a]pyridin-6-yl)-1H-pyrazol-3-yl)phenyl)ethyl)-2,2-dimethylpropan-1-amin (560);
N-((R)-1-(4-(4-Isopropyl-5-(8-methyl-[1,2,4]triazolo[1,5-a]pyridin-6-yl)-1H-pyrazol-3-yl)phenyl)ethyl)-3-methylbutan-2-amin (561);
N-((R)-1-(4-(4-Isopropyl-5-(8-methyl-[1,2,4]triazolo[1,5-a]pyridin-6-yl)-1H-pyrazol-3-yl)phenyl)ethyl)-3-methylbutan-2-amin (562);
6-(4-Isopropyl-3-(4-(1-methylpyrrolidin-2-yl)phenyl)-1H-pyrazol-5-yl)-8-methyl-[1,2,4]triazolo[1,5-a]pyridin (563 und 566);
6-(4-Isopropyl-3-(4-(1-isopropylpyrrolidin-2-yl)phenyl)-1H-pyrazol-5-yl)-8-methyl-[1,2,4]triazolo[1,5-a]pyridin (564 und 567);
6-(4-Isopropyl-3-(4-(1-(oxetan-3-yl)pyrrolidin-2-yl)phenyl)-1H-pyrazol-5-yl)-8-methyl-[1,2,4]triazolo[1,5-a]pyridin (565 und 568);
(S)-N-(1-(4-(4-Isopropyl-5-(8-methyl-[1,2,4]triazolo[1,5-a]pyridin-6-yl)-1H-pyrazol-3-yl)phenyl)ethyl)oxetan-3-amin (569);
(S)-N-Ethyl-1-(4-(4-isopropyl-5-(8-methoxy-[1,2,4]triazolo[1,5-a]pyridin-6-yl)-1H-pyrazol-3-yl)phenyl)-N-methylethan-1-amin (570);
(S)-1-(4-(4-Isopropyl-5-(8-methyl-[1,2,4]triazolo[1,5-a]pyridin-6-yl)-1H-pyrazol-3-yl)phenyl)-N-methylethan-1-amin (571);
(S)-N-(1-(4-(4-Isopropyl-5-(8-methoxy-[1,2,4]triazolo[1,5-a]pyridin-6-yl)-1H-pyrazol-3-yl)phenyl)ethyl)-N-methylpropan-2-amin (572);
(S)-N-(1-(4-(4-Isopropyl-5-(8-methoxy-[1,2,4]triazolo[1,5-a]pyridin-6-yl)-1H-pyrazol-3-yl)phenyl)ethyl)-N-methyloxetan-3-amin (573);
(S)-1-(4-(4-Isopropyl-5-(8-methoxy-[1,2,4]triazolo[1,5-a]pyridin-6-yl)-1H-pyrazol-3-yl)phenyl)-N-methyl-N-((3-methyloxetan-3-yl)methyl)ethan-1-amin (574);
(S)-2,2,2-Trifluor-N-(1-(4-(4-isopropyl-5-(8-methoxy-[1,2,4]triazolo[1,5-a]pyridin-6-yl)-1H-pyrazol-3-yl)phenyl)ethyl)-N-methylethan-1-amin (575);
(S)-2,2,2-Trifluor-N-(1-(4-(4-isopropyl-5-(8-methyl-[1,2,4]triazolo[1,5-a]pyridin-6-yl)-1H-pyrazol-3-yl)phenyl)ethyl)-N-methylethan-1-amin (576);
(S)-N-Ethyl-1-(4-(4-isopropyl-5-(8-methyl-[1,2,4]triazolo[1,5-a]pyridin-6-yl)-1H-pyrazol-3-yl)phenyl)-N-methylethan-1-amin (577);
(S)-N-(1-(4-(4-Isopropyl-5-(8-methyl-[1,2,4]triazolo[1,5-a]pyridin-6-yl)-1H-pyrazol-3-yl)phenyl)ethyl)-N-methylpropan-2-amin (578);
(S)-N-(1-(4-(4-Isopropyl-5-(8-methyl-[1,2,4]triazolo[1,5-a]pyridin-6-yl)-1H-pyrazol-3-yl)phenyl)ethyl)-N-methyloxetan-3-amin (579);
(S)-1-(4-(4-Isopropyl-5-(8-methyl-[1,2,4]triazolo[1,5-a]pyridin-6-yl)-1H-pyrazol-3-yl)phenyl)-N-methyl-N-((3-methyloxetan-3-yl)methyl)ethan-1-amin (580);
2-(Dimethylamino)-N-(2-(4-(4-isopropyl-5-(8-methyl-[1,2,4]triazolo[1,5-a]pyridin-6-yl)-1H-pyrazol-3-yl)phenyl)propan-2-yl)acetamid (581);
2-(Dimethylamino)-1-(2-(4-(4-isopropyl-5-(8-methyl-[1,2,4]triazolo[1,5-a]pyridin-6-yl)-1H-pyrazol-3-yl)phenyl)pyrrolidin-1-yl)ethan-1-on (582-583);
2-(2-(4-(4-Isopropyl-5-(8-methyl-[1,2,4]triazolo[1,5-a]pyridin-6-yl)-1H-pyrazol-3-yl)phenyl)pyrrolidin-1-yl)-N,N-dimethylacetamid (584-585);
(S)-N-((S)-1-(4-(4-Isopropyl-5-(8-methoxy-[1,2,4]triazolo[1,5-a]pyridin-6-yl)-1H-pyrazol-3-yl)phenyl)ethyl)-N,1-dimethylazetidin-2-carboxamid (585);
(S)-2-(Dimethylamino)-N-(1-(4-(4-isopropyl-5-(8-methoxy-[1,2,4]triazolo[1,5-a]pyridin-6-yl)-1H-pyrazol-3-yl)phenyl)ethyl)-N-methylacetamid (586);
(S)-2-(Diethylamino)-N-(1-(4-(4-isopropyl-5-(8-methoxy-[1,2,4]triazolo[1,5-a]pyridin-6-yl)-1H-pyrazol-3-yl)phenyl)ethyl)-N-methylacetamid (587);
(S)-2-(Dimethylamino)-N-((S)-1-(4-(4-isopropyl-5-(8-methoxy-[1,2,4]triazolo[1,5-a]pyridin-6-yl)-1H-pyrazol-3-yl)phenyl)ethyl)-N-methylpropanamid (588);
(R)-2-(Dimethylamino)-N-((S)-1-(4-(4-isopropyl-5-(8-methoxy-[1,2,4]triazolo[1,5-a]pyridin-6-yl)-1H-pyrazol-3-yl)phenyl)ethyl)-N-methylpropanamid (589);
(S)-2-(Dimethylamino)-N-(1-(4-(4-isopropyl-5-(8-methyl-[1,2,4]triazolo[1,5-a]pyridin-6-yl)-1H-pyrazol-3-yl)phenyl)ethyl)-N-methylacetamid (590);
(S)-2-(Diethylamino)-N-(1-(4-(4-isopropyl-5-(8-methyl-[1,2,4]triazolo[1,5-a]pyridin-6-yl)-1H-pyrazol-3-yl)phenyl)ethyl)-N-methylacetamid (591);
(R)-2-(Dimethylamino)-N-((S)-1-(4-(4-isopropyl-5-(8-methyl-[1,2,4]triazolo[1,5-a]pyridin-6-yl)-1H-pyrazol-3-yl)phenyl)ethyl)-N-methylpropanamid (592);
(S)-2-(Dimethylamino)-N-((S)-1-(4-(4-isopropyl-5-(8-methyl-[1,2,4]triazolo[1,5-a]pyridin-6-yl)-1H-pyrazol-3-yl)phenyl)ethyl)-N-methylpropanamid (593);
(S)-N-((S)-1-(4-(4-Isopropyl-5-(8-methoxy-[1,2,4]triazolo[1,5-a]pyridin-6-yl)-1H-pyrazol-3-yl)phenyl)ethyl)-N-methyl-2-(methylamino)propanamid (594);
(R)-N-((S)-1-(4-(4-Isopropyl-5-(8-methoxy-[1,2,4]triazolo[1,5-a]pyridin-6-yl)-1H-pyrazol-3-yl)phenyl)ethyl)-N-methyl-2-(methylamino)propanamid (595);
(S)-N-((S)-1-(4-(4-Isopropyl-5-(8-methyl-[1,2,4]triazolo[1,5-a]pyridin-6-yl)-1H-pyrazol-3-yl)phenyl)ethyl)-N-methylazetidin-2-carboxamid (596);
(R)-N-((S)-1-(4-(4-Isopropyl-5-(8-methyl-[1,2,4]triazolo[1,5-a]pyridin-6-yl)-1H-pyrazol-3-yl)phenyl)ethyl)-N-methyl-2-(methylamino)propanamid (597);
(S)-N-((S)-1-(4-(4-Isopropyl-5-(8-methyl-[1,2,4]triazolo[1,5-a]pyridin-6-yl)-1H-pyrazol-3-yl)phenyl)ethyl)-N-methyl-2-(methylamino)propanamid (598);
(S)-N-((S)-1-(4-(4-Isopropyl-5-(8-methyl-[1,2,4]triazolo[1,5-a]pyridin-6-yl)-1H-pyrazol-3-yl)phenyl)ethyl)-N-methylpyrrolidin-2-carboxamid (599);
(R)-N-((S)-1-(4-(4-Isopropyl-5-(8-methyl-[1,2,4]triazolo[1,5-a]pyridin-6-yl)-1H-pyrazol-3-yl)phenyl)ethyl)-N-methylpyrrolidin-2-carboxamid (600);
(S)-1-Ethyl-N-((S)-1-(4-(4-isopropyl-5-(8-methoxy-[1,2,4]triazolo[1,5-a]pyridin-6-yl)-1H-pyrazol-3-yl)phenyl)ethyl)-N-methylazetidin-2-carboxamid (601);
(S)-N-((S)-1-(4-(4-Isopropyl-5-(8-methyl-[1,2,4]triazolo[1,5-a]pyridin-6-yl)-1H-pyrazol-3-yl)phenyl)ethyl)-N,1-dimethylazetidin-2-carboxamid (602);
(S)-1-Ethyl-N-((S)-1-(4-(4-isopropyl-5-(8-methyl-[1,2,4]triazolo[1,5-a]pyridin-6-yl)-1H-pyrazol-3-yl)phenyl)ethyl)-N-methylazetidin-2-carboxamid (603);
(S)-N-((S)-1-(4-(4-Isopropyl-5-(8-methyl-[1,2,4]triazolo[1,5-a]pyridin-6-yl)-1H-pyrazol-3-yl)phenyl)ethyl)-N-methyl-1-(oxetan-3-yl)azetidin-2-carboxamid (604);
(S)-N-((S)-1-(4-(4-Isopropyl-5-(8-methyl-[1,2,4]triazolo[1,5-a]pyridin-6-yl)-1H-pyrazol-3-yl)phenyl)ethyl)-N,1-dimethylpyrrolidin-2-carboxamid (605);
(S)-N-((S)-1-(4-(4-Isopropyl-5-(8-methyl-[1,2,4]triazolo[1,5-a]pyridin-6-yl)-1H-pyrazol-3-yl)phenyl)ethyl)-N-methyl-1-(oxetan-3-yl)pyrrolidin-2-carboxamid (606);
(R)-N-((S)-1-(4-(4-Isopropyl-5-(8-methyl-[1,2,4]triazolo[1,5-a]pyridin-6-yl)-1H-pyrazol-3-yl)phenyl)ethyl)-N,1-dimethylpyrrolidin-2-carboxamid (607);
(R)-N-((S)-1-(4-(4-Isopropyl-5-(8-methyl-[1,2,4]triazolo[1,5-a]pyridin-6-yl)-1H-pyrazol-3-yl)phenyl)ethyl)-N-methyl-1-(oxetan-3-yl)pyrrolidin-2-carboxamid (608);
(S)-2-(Ethyl(methyl)amino)-N-((S)-1-(4-(4-isopropyl-5-(8-methyl-[1,2,4]triazolo[1,5-a]pyridin-6-yl)-1H-pyrazol-3-yl)phenyl)ethyl)-N-methylpropanamid (609);
(R)-2-(Ethyl(methyl)amino)-N-((S)-1-(4-(4-isopropyl-5-(8-methyl-[1,2,4]triazolo[1,5-a]pyridin-6-yl)-1H-pyrazol-3-yl)phenyl)ethyl)-N-methylpropanamid (610);
(S)-2-(Ethyl(methyl)amino)-N-((S)-1-(4-(4-isopropyl-5-(8-methoxy-[1,2,4]triazolo[1,5-a]pyridin-6-yl)-1H-pyrazol-3-yl)phenyl)ethyl)-N-methylpropanamid (611);
(R)-N-((S)-1-(4-(4-Isopropyl-5-(8-methoxy-[1,2,4]triazolo[1,5-a]pyridin-6-yl)-1H-pyrazol-3-yl)phenyl)ethyl)-N-methyl-2-(methyl(oxetan-3-yl)amino)propanamid (612);
(S)-2-(Ethyl(methyl)amino)-N-((S)-1-(4-(4-isopropyl-5-(8-methoxy-[1,2,4]triazolo[1,5-a]pyridin-6-yl)-1H-pyrazol-3-yl)phenyl)ethyl)-N-methylpropanamid (613);
(R)-N-((S)-1-(4-(4-Isopropyl-5-(8-methyl-[1,2,4]triazolo[1,5-a]pyridin-6-yl)-1H-pyrazol-3-yl)phenyl)ethyl)-N-methyl-2-(methyl(oxetan-3-yl)amino)propanamid (614);
(S)-N-((S)-1-(4-(4-Isopropyl-5-(8-methyl-[1,2,4]triazolo[1,5-a]pyridin-6-yl)-1H-pyrazol-3-yl)phenyl)ethyl)-N-methyl-2-(methyl(oxetan-3-yl)amino)propanamid (615);
(S)-2-((1-(4-(4-Isopropyl-5-(8-methyl-[1,2,4]triazolo[1,5-a]pyridin-6-yl)-1H-pyrazol-3-yl)phenyl)ethyl)(methyl)amino)-N,N-dimethylacetamid (616);
(S)-2-((1-(4-(4-Isopropyl-5-(8-methoxy-[1,2,4]triazolo[1,5-a]pyridin-6-yl)-1H-pyrazol-3-yl)phenyl)ethyl)(methyl)amino)-N,N-dimethylacetamid (617);
(S)-2-(Dimethylamino)-N-(1-(4-(4-isopropyl-5-(8-methyl-[1,2,4]triazolo[1,5-a]pyridin-6-yl)-1H-pyrazol-3-yl)phenyl)ethyl)-N-(oxetan-3-yl)acetamid (618);
(S)-2-(Diethylamino)-N-(1-(4-(4-isopropyl-5-(8-methyl-[1,2,4]triazolo[1,5-a]pyridin-6-yl)-1H-pyrazol-3-yl)phenyl)ethyl)-N-(oxetan-3-yl)acetamid (619);
(S)-2-(Ethyl(methyl)amino)-N-(1-(4-(4-isopropyl-5-(8-methyl-[1,2,4]triazolo[1,5-a]pyridin-6-yl)-1H-pyrazol-3-yl)phenyl)ethyl)-N-methylacetamid (620);
(S)-N-(1-(4-(4-Isopropyl-5-(8-methyl-[1,2,4]triazolo[1,5-a]pyridin-6-yl)-1H-pyrazol-3-yl)phenyl)ethyl)-N-methyl-2-(methyl(3,3,3-trifluorpropyl)amino)acetamid (621);
(S)-2-(3-Fluorazetidin-1-yl)-N-(1-(4-(4-isopropyl-5-(8-methyl-[1,2,4]triazolo[1,5-a]pyridin-6-yl)-1H-pyrazol-3-yl)phenyl)ethyl)-N-methylacetamid (622);
(S)-2-((2-Fluor-2-methylpropyl)amino)-N-(1-(4-(4-isopropyl-5-(8-methyl-[1,2,4]triazolo[1,5-a]pyridin-6-yl)-1H-pyrazol-3-yl)phenyl)ethyl)-N-methylacetamid (623);
(S)-1-(2-Fluor-5-(4-isopropyl-5-(8-methyl-[1,2,4]triazolo[1,5-a]pyridin-6-yl)-1H-pyrazol-3-yl)phenyl)-N,N-dimethylethan-1-amin (624);
(S)-N,N-Diethyl-1-(2-fluor-5-(4-isopropyl-5-(8-methyl-[1,2,4]triazolo[1,5-a]pyridin-6-yl)-1H-pyrazol-3-yl)phenyl)ethan-1-amin (625);
(S)-N-(1-(2-Fluor-5-(4-isopropyl-5-(8-methyl-[1,2,4]triazolo[1,5-a]pyridin-6-yl)-1H-pyrazol-3-yl)phenyl)ethyl)propan-1-amin (626);
(S)-N-(1-(2-Fluor-5-(4-isopropyl-5-(8-methyl-[1,2,4]triazolo[1,5-a]pyridin-6-yl)-1H-pyrazol-3-yl)phenyl)ethyl)-N-propylpropan-1-amin (627);
(S)-N-(1-(2-Fluor-5-(4-isopropyl-5-(8-methyl-[1,2,4]triazolo[1,5-a]pyridin-6-yl)-1H-pyrazol-3-yl)phenyl)ethyl)propan-2-amin (628);
(S)-2-(Dimethylamino)-N-(1-(2-fluor-5-(4-isopropyl-5-(8-methyl-[1,2,4]triazolo[1,5a] Pyridin-6-yl)-1H-pyrazol-3-yl)phenyl)ethyl)acetamid (629);
(S)-2-(Diethylamino)-N-(1-(2-fluor-5-(4-isopropyl-5-(8-methyl-[1,2,4]triazolo[1,5-a]pyridin-6-yl)-1H-pyrazol-3-yl)phenyl)ethyl)acetamid (630);
N-(1-(4-(4-Isopropyl-5-(8-methyl-[1,2,4]triazolo[1,5-a]pyridin-6-yl)-1H-pyrazol-3-yl)phenyl)ethyl)propan-2-amin (631);
1-(4-(4-Isopropyl-5-(8-methyl-[1,2,4]triazolo[1,5-a]pyridin-6-yl)-1H-pyrazol-3-yl)phenyl)-N-methylethan-1-amin (632);
N-(1-(4-(4-Isopropyl-5-(8-methyl-[1,2,4]triazolo[1,5-a]pyridin-6-yl)-1H-pyrazol-3-yl)phenyl)ethyl)oxetan-3-amin (633);
N-(1-(4-(4-Isopropyl-5-(8-methyl-[1,2,4]triazolo[1,5-a]pyridin-6-yl)-1H-pyrazol-3-yl)phenyl)ethyl)-3-methyloxetan-3-amin (634);
1-(4-(4-Isopropyl-5-(8-methyl-[1,2,4]triazolo[1,5-a]pyridin-6-yl)-1H-pyrazol-3-yl)phenyl)-N,N-dimethylethan-1-amin (635);
6-(3-(4-(1-(4,4-Difluorpiperidin-1-yl)ethyl)phenyl)-4-isopropyl-1H-pyrazol-5-yl)-8-methyl-[1,2,4]triazolo[1,5-a]pyridin (636);
4-(1-(4-(4-Isopropyl-5-(8-methyl-[1,2,4]triazolo[1,5-a]pyridin-6-yl)-1H-pyrazol-3-yl)phenyl)ethyl)morpholin (637);
6-(1-(4-(4-Isopropyl-5-(8-methyl-[1,2,4]triazolo[1,5-a]pyridin-6-yl)-1H-pyrazol-3-yl)phenyl)ethyl)-2-oxa-6-azaspiro[3.3]heptan (638);
4-(1-(4-(4-Isopropyl-5-(8-methyl-[1,2,4]triazolo[1,5-a]pyridin-6-yl)-1H-pyrazol-3-yl)phenyl)ethyl)piperazin-2-on (639);
6-(1-(4-(4-Isopropyl-5-(8-methyl-[1,2,4]triazolo[1,5-a]pyridin-6-yl)-1H-pyrazol-3-yl)phenyl)ethyl)-2-oxa-6-azaspiro[3.3]heptan (640-641);
4-(1-(4-(4-Isopropyl-5-(8-methyl-[1,2,4]triazolo[1,5-a]pyridin-6-yl)-1H-pyrazol-3-yl)phenyl)ethyl)morpholin (642-643);
1-(4-(4-Isopropyl-5-(8-methyl-[1,2,4]triazolo[1,5-a]pyridin-6-yl)-1H-pyrazol-3-yl)phenyl)-N-methylmethanamin (644);
1-(4-(4-Isopropyl-5-(8-methyl-[1,2,4]triazolo[1,5-a]pyridin-6-yl)-1H-pyrazol-3-yl)phenyl)-N,N-dimethylmethanamin (645 und 652);
N-(4-(4-Isopropyl-5-(8-methyl-[1,2,4]triazolo[1,5-a]pyridin-6-yl)-1H-pyrazol-3-yl)benzyl)propan-2-amin (646);
N-(4-(4-Isopropyl-5-(8-methyl-[1,2,4]triazolo[1,5-a]pyridin-6-yl)-1H-pyrazol-3-yl)benzyl)cyclopropanamin (647);
(1-(4-(4-Isopropyl-5-(8-methyl-[1,2,4]triazolo[1,5-a]pyridin-6-yl)-1H-pyrazol-3-yl)benzyl)azetidin-3,3-diyl)dimethanol (648);
N-(4-(4-Isopropyl-5-(8-methyl-[1,2,4]triazolo[1,5-a]pyridin-6-yl)-1H-pyrazol-3-yl)benzyl)-2,2-dimethylpropan-1-amin (649);
6-(3-(4-(Azetidin-1-ylmethyl)phenyl)-4-isopropyl-1H-pyrazol-5-yl)-8-methyl-[1,2,4]triazolo[1,5-a]pyridin (650);
N-(4-(4-Isopropyl-5-(8-methyl-[1,2,4]triazolo[1,5-a]pyridin-6-yl)-1H-pyrazol-3-yl)benzyl)-N-methylethanamin (651);
N-((6-(4-Isopropyl-5-(8-methyl-[1,2,4]triazolo[1,5-a]pyridin-6-yl)-1H-pyrazol-3-yl)pyridin-3-yl)methyl)propan-2-amin (653);
1-(6-(4-Isopropyl-5-(8-methyl-[1,2,4]triazolo[1,5-a]pyridin-6-yl)-1H-pyrazol-3-yl)pyridin-3-yl)-N-methylmethanamin (654);
N-((5-(4-Isopropyl-5-(8-methyl-[1,2,4]triazolo[1,5-a]pyridin-6-yl)-1H-pyrazol-3-yl)pyridin-2-yl)methyl)propan-2-amin (655);
1-(5-(4-Isopropyl-5-(8-methyl-[1,2,4]triazolo[1,5-a]pyridin-6-yl)-1H-pyrazol-3-yl)pyridin-2-yl)-N,N-dimethylmethanamin (656);
1-(5-(4-Isopropyl-5-(8-methyl-[1,2,4]triazolo[1,5-a]pyridin-6-yl)-1H-pyrazol-3-yl)pyridin-2-yl)-N-methylmethanamin (657);
1-(6-(4-Isopropyl-5-(8-methyl-[1,2,4]triazolo[1,5-a]pyridin-6-yl)-1H-pyrazol-3-yl)pyridin-3-yl)-N,N-dimethylethan-1-amin (658-659);
1-(5-(4-Isopropyl-5-(8-methyl-[1,2,4]triazolo[1,5-a]pyridin-6-yl)-1H-pyrazol-3-yl)thiazol-2-yl)-N-methylethan-1-amin (660-662);
1-(2-(4-Isopropyl-5-(8-methyl-[1,2,4]triazolo[1,5-a]pyridin-6-yl)-1H-pyrazol-3-yl)thiazol-5-yl)-N-methylethan-1-amin (663-664);
1-(2-(4-Isopropyl-5-(8-methyl-[1,2,4]triazolo[1,5-a]pyridin-6-yl)-1H-pyrazol-3-yl)thiazol-5-yl)-N,N-dimethylethan-1-amin (665 und 670);
N-Ethyl-1-(2-(4-isopropyl-5-(8-methyl-[1,2,4]triazolo[1,5-a]pyridin-6-yl)-1H-pyrazol-3-yl)thiazol-5-yl)-N-methylethan-1-amin (666 und 669);
N-(1-(2-(4-Isopropyl-5-(8-methyl-[1,2,4]triazolo[1,5-a]pyridin-6-yl)-1H-pyrazol-3-yl)thiazol-5-yl)ethyl)-N-methylpropan-2-amin (677-678);
N-(1-(2-(4-Isopropyl-5-(8-methyl-[1,2,4]triazolo[1,5-a]pyridin-6-yl)-1H-pyrazol-3-yl)thiazol-5-yl)ethyl)-N-methyltetrahydro-2H-pyran-4-amin (671);
2-(Dimethylamino)-N-(1-(6-(4-isopropyl-5-(8-methyl-[1,2,4]triazolo[1,5-a]pyridin-6-yl)-1H-pyrazol-3-yl)pyridin-3-yl)ethyl)-N-methylacetamid (672-673)
2-(Dimethylamino)-N-(1-(2-(4-isopropyl-5-(8-methyl-[1,2,4]triazolo[1,5-a]pyridin-6-yl)-1H-pyrazol-3-yl)thiazol-5-yl)ethyl)-N-methylacetamid (674-675);
2-((1-(2-(4-Isopropyl-5-(8-methyl-[1,2,4]triazolo[1,5-a]pyridin-6-yl)-1H-pyrazol-3-yl)thiazol-5-yl)ethyl)(methyl)amino)-N,N-dimethylacetamid (676);
2-((1-(2-(4-Isopropyl-5-(8-methyl-[1,2,4]triazolo[1,5-a]pyridin-6-yl)-1H-pyrazol-3-yl)thiazol-5-yl)ethyl)(methyl)amino)-N,N-dimethylacetamid (677-678);
6-(4-Isopropyl-5-(8-methyl-[1,2,4]triazolo[1,5-a]pyridin-6-yl)-1H-pyrazol-3-yl)-N,N-dimethyl-1,2,3,4-tetrahydronaphthalen-2-amin (679 und 682);
6-(4-Isopropyl-5-(8-methoxy-[1,2,4]triazolo[1,5-a]pyridin-6-yl)-1H-pyrazol-3-yl)-N-methyl-1,2,3,4-tetrahydronaphthalen-2-amin (680-681);
6-(4-Isopropyl-5-(8-methyl-[1,2,4]triazolo[1,5-a]pyridin-6-yl)-1H-pyrazol-3-yl)-N,N-dimethyl-1,2,3,4-tetrahydronaphthalen-2-amin (682);
N-Isopropyl-6-(4-isopropyl-5-(8-methyl-[1,2,4]triazolo[1,5-a]pyridin-6-yl)-1H-pyrazol-3-yl)-N-methyl-1,2,3,4-tetrahydronaphthalen-2-amin (683);
N-(6-(4-Isopropyl-5-(8-methyl-[1,2,4]triazolo[1,5-a]pyridin-6-yl)-1H-pyrazol-3-yl)-1,2,3,4-tetrahydronaphthalen-2-yl)-N-methyltetrahydro-2H-pyran-4-amin (684-685);
N-(6-(4-Isopropyl-5-(8-methyl-[1,2,4]triazolo[1,5-a]pyridin-6-yl)-1H-pyrazol-3-yl)-1,2,3,4-tetrahydronaphthalen-2-yl)-N-methyloxetan-3-amin (686-687);
6-(4-Isopropyl-5-(8-methyl-[1,2,4]triazolo[1,5-a]pyridin-6-yl)-1H-pyrazol-3-yl)-N-methyl-N-((3-methyloxetan-3-yl)methyl)-1,2,3,4-tetrahydronaphthalen-2-amin (688);
6-(4-Isopropyl-5-(8-methoxy-[1,2,4]triazolo[1,5-a]pyridin-6-yl)-1H-pyrazol-3-yl)-N,N-dimethyl-1,2,3,4-tetrahydronaphthalen-2-amin (689);
2-(Dimethylamino)-N-(6-(4-isopropyl-5-(8-methyl-[1,2,4]triazolo[1,5-a]pyridin-6-yl)-1H-pyrazol-3-yl)-1,2,3,4-tetrahydronaphthalen-2-yl)-N-methylacetamid (690-691);
2-((6-(4-Isopropyl-5-(8-methyl-[1,2,4]triazolo[1,5-a]pyridin-6-yl)-1H-pyrazol-3-yl)-1,2,3,4-tetrahydronaphthalen-2-yl)(methyl)amino)-N,N-dimethylacetamid (692-693);
2-(4-Isopropyl-5-(8-methoxy-[1,2,4]triazolo[1,5-a]pyridin-6-yl)-1H-pyrazol-3-yl)-N,N-dimethyl-4,5,6,7-tetrahydrobenzo[d]thiazol-6-amin (694 und 703);
2-(4-Isopropyl-5-(8-methyl-[1,2,4]triazolo[1,5-a]pyridin-6-yl)-1H-pyrazol-3-yl)-4,5,6,7-tetrahydrobenzo[d]thiazol-6-amin (695-697);
N-Isopropyl-2-(4-isopropyl-5-(8-methoxy-[1,2,4]triazolo[1,5-a]pyridin-6-yl)-1H-pyrazol-3-yl)-4,5,6,7-tetrahydrobenzo[d]thiazol-6-amin (698 und 704);
2-(4-Isopropyl-5-(8-methoxy-[1,2,4]triazolo[1,5-a]pyridin-6-yl)-1H-pyrazol-3-yl)-N-(4,4,4-trifluorbutyl)-4,5,6,7-tetrahydrobenzo[d]thiazol-6-amin (699);
2-(4-Isopropyl-5-(8-methoxy-[1,2,4]triazolo[1,5-a]pyridin-6-yl)-1H-pyrazol-3-yl)-N,N-bis(4,4,4-trifluorbutyl)-4,5,6,7-tetrahydrobenzo[d]thiazol-6-amin (700);
2-(4-Isopropyl-5-(8-methoxy-[1,2,4]triazolo[1,5-a]pyridin-6-yl)-1H-pyrazol-3-yl)-N-(tetrahydro-2H-pyran-4-yl)-4,5,6,7-tetrahydrobenzo[d]thiazol-6-amin (701 und 706);
2-(4-Isopropyl-5-(8-methoxy-[1,2,4]triazolo[1,5-a]pyridin-6-yl)-1H-pyrazol-3-yl)-N-(3,3,3-trifluorpropyl)-4,5,6,7-tetrahydrobenzo[d]thiazol-6-amin (702 und 705);
2-(4-Isopropyl-5-(8-methyl-[1,2,4]triazolo[1,5-a]pyridin-6-yl)-1H-pyrazol-3-yl)-N,N-dimethyl-4,5,6,7-tetrahydrobenzo[d]thiazol-6-amin (707 und 712);
N-Isopropyl-2-(4-isopropyl-5-(8-methyl-[1,2,4]triazolo[1,5-a]pyridin-6-yl)-1H-pyrazol-3-yl)-4,5,6,7-tetrahydrobenzo[d]thiazol-6-amin (708 und 713);
2-(4-Isopropyl-5-(8-methyl-[1,2,4]triazolo[1,5-a]pyridin-6-yl)-1H-pyrazol-3-yl)-N-(oxetan-3-yl)-4,5,6,7-tetrahydrobenzo[d]thiazol-6-amin (709 und 715);
2-(4-Isopropyl-5-(8-methyl-[1,2,4]triazolo[1,5-a]pyridin-6-yl)-1H-pyrazol-3-yl)-N-(tetrahydro-2H-pyran-4-yl)-4,5,6,7-tetrahydrobenzo[d]thiazol-6-amin (710 und 717);
N-(3-Ethoxycyclobutyl)-2-(4-isopropyl-5-(8-methyl-[1,2,4]triazolo[1,5-a]pyridin-6-yl)-1H-pyrazol-3-yl)-4,5,6,7-tetrahydrobenzo[d]thiazol-6-amin (711-716);
2-(4-Isopropyl-5-(8-methyl-[1,2,4]triazolo[1,5-a]pyridin-6-yl)-1H-pyrazol-3-yl)-N-(3,3,3-trifluorpropyl)-4,5,6,7-tetrahydrobenzo[d]thiazol-6-amin (714);
2-(4-Isopropyl-5-(8-methyl-[1,2,4]triazolo[1,5-a]pyridin-6-yl)-1H-pyrazol-3-yl)-N-(oxetan-3-yl)-4,5,6,7-tetrahydrobenzo[d]thiazol-6-amin (715);
2-(Dimethylamino)-N-(2-(4-isopropyl-5-(8-methoxy-[1,2,4]triazolo[1,5-a]pyridin-6-yl)-1H-pyrazol-3-yl)-4,5,6,7-tetrahydrobenzo[d]thiazol-6-yl)acetamid (718-719);
2-(Dimethylamino)-N-(2-(4-isopropyl-5-(8-methyl-[1,2,4]triazolo[1,5-a]pyridin-6-yl)-1H-pyrazol-3-yl)-4,5,6,7-tetrahydrobenzo[d]thiazol-6-yl)acetamid (720-721);
N-Ethyl-2-(4-isopropyl-5-(8-methoxy-[1,2,4]triazolo[1,5-a]pyridin-6-yl)-1H-pyrazol-3-yl)-N-methyl-4,5,6,7-tetrahydrobenzo[d]thiazol-6-amin (722 und 728);
2-(4-Isopropyl-5-(8-methoxy-[1,2,4]triazolo[1,5-a]pyridin-6-yl)-1H-pyrazol-3-yl)-N-methyl-N-propyl-4,5,6,7-tetrahydrobenzo[d]thiazol-6-amin (723 und 729);
N-Isopropyl-2-(4-isopropyl-5-(8-methoxy-[1,2,4]triazolo[1,5-a]pyridin-6-yl)-1H-pyrazol-3-yl)-N-methyl-4,5,6,7-tetrahydrobenzo[d]thiazol-6-amin (724 und 730);
2-(4-Isopropyl-5-(8-methoxy-[1,2,4]triazolo[1,5-a]pyridin-6-yl)-1H-pyrazol-3-yl)-N-methyl-N-(oxetan-3-yl)-4,5,6,7-tetrahydrobenzo[d]thiazol-6-amin (725 und 731);
2-(4-Isopropyl-5-(8-methoxy-[1,2,4]triazolo[1,5-a]pyridin-6-yl)-1H-pyrazol-3-yl)-N-methyl-N-(3,3,3-trifluorpropyl)-4,5,6,7-tetrahydrobenzo[d]thiazol-6-amin (726 und 732);
2-(4-Isopropyl-5-(8-methoxy-[1,2,4]triazolo[1,5-a]pyridin-6-yl)-1H-pyrazol-3-yl)-N-methyl-N-(tetrahydro-2H-pyran-4-yl)-4,5,6,7-tetrahydrobenzo[d]thiazol-6-amin (727 und 733);
2-(Dimethylamino)-N-(2-(4-isopropyl-5-(8-methoxy-[1,2,4] triazolo[1,5-a]pyridin-6-yl)-1H-pyrazol-3-yl)-4,5,6,7-tetrahydrobenzo[d]thiazol-6-yl)-N-methylacetamid (734);
2-(Dimethylamino)-N-(2-(4-isopropyl-5-(8-methoxy-[1,2,4]triazolo[1,5-a]pyridin-6-yl)-1H-pyrazol-3-yl)-4,5,6,7-tetrahydrobenzo[d]thiazol-6-yl)-N-methylacetamid (735);
2-((2-(4-Isopropyl-5-(8-methoxy-[1,2,4]triazolo[1,5-a]pyridin-6-yl)-1H-pyrazol-3-yl)-4,5,6,7-tetrahydrobenzo[d]thiazol-6-yl)(methyl)amino)-N,N-dimethylacetamid (736);
2-((2-(4-Isopropyl-5-(8-methoxy-[1,2,4]triazolo[1,5-a]pyridin-6-yl)-1H-pyrazol-3-yl)-4,5,6,7-tetrahydrobenzo[d]thiazol-6-yl)(methyl)amino)-N,N-dimethylacetamid (737);
2-(4-Isopropyl-5-(8-methoxy-[1,2,4]triazolo[1,5-a]pyridin-6-yl)-1H-pyrazol-3-yl)-N-(2-methoxyethyl)-N-methyl-4,5,6,7-tetrahydrobenzo[d]thiazol-6-amin (738-739);
2-(4-Isopropyl-5-(8-methoxy-[1,2,4]triazolo[1,5-a]pyridin-6-yl)-1H-pyrazol-3-yl)-N-methyl-N-(2-(methylsulfonyl)ethyl)-4,5,6,7-tetrahydrobenzo[d]thiazol-6-amin (740-741);
2-(4-Isopropyl-5-(8-methoxy-[1,2,4]triazolo[1,5-a]pyridin-6-yl)-1H-pyrazol-3-yl)-5-(piperidin-4-yl)thiazol (742),
2-(4-Isopropyl-5-(8-methylimidazo[1,2-a]pyridin-6-yl)-1H-pyrazol-3-yl)-5-(piperidin-4-yl)thiazol (743),
2-(4-Isopropyl-5-(8-methoxyimidazo[1,2-a]pyridin-6-yl)-1H-pyrazol-3-yl)-5-(piperidin-4-yl)thiazol (744);
2-(5-(3,4-Dimethoxyphenyl)-4-isopropyl-1H-pyrazol-3-yl)-5-(piperidin-4-yl)thiazol (745);
2-(5-(7,8-Dimethyl-[1,2,4]triazolo[1,5-a]pyridin-6-yl)-4-isopropyl-1H-pyrazol-3-yl)-5-(piperidin-4-yl)thiazol (746);
2-(5-(2,6-Dimethylpyridin-4-yl)-4-isopropyl-1H-pyrazol-3-yl)-5-(piperidin-4-yl)thiazol (747);
5-(4-Isopropyl-3-(5-(piperidin-4-yl)thiazol-2-yl)-1H-pyrazol-5-yl)-1,3,4-trimethylpyridin-2(1H)-on (748);
2-(4-Isopropyl-5-(8-methoxy-[1,2,4]triazolo[1,5-a]pyridin-6-yl)-1H-pyrazol-3-yl)-4-methyl-5-(piperidin-4-yl)thiazol (749);
2-(5-(7,8-Dimethyl-[1,2,4]triazolo[1,5-a]pyridin-6-yl)-4-isopropyl-1H-pyrazol-3-yl)-4-methyl-5-(piperidin-4-yl)thiazol (750);
2-(4-Isopropyl-5-(8-methoxy-[1,2,4]triazolo[1,5-a]pyridin-6-yl)-1H-pyrazol-3-yl)-5-(piperidin-4-yl)-4-(trifluormethyl)thiazol (751);
2-(4-Isopropyl-5-(8-methoxy-[1,2,4]triazolo[1,5-a]pyridin-6-yl)-1H-pyrazol-3-yl)-5-(1-((tetrahydro-2H-pyran-4-yl)methyl)piperidin-4-yl)thiazol (752);
2-(4-Isopropyl-5-(8-methoxy-[1,2,4]triazolo[1,5-a]pyridin-6-yl)-1H-pyrazol-3-yl)-5-(1-methylpiperidin-4-yl)thiazol (753);
2-(4-Isopropyl-5-(8-methoxy-[1,2,4]triazolo[1,5-a]pyridin-6-yl)-1H-pyrazol-3-yl)-5-(1-(3,3,3-trifluorpropyl)piperidin-4-yl)thiazol (754);
5-(1-Ethylpiperidin-4-yl)-2-(4-isopropyl-5-(8-methoxy-[1,2,4]triazolo[1,5-a]pyridin-6-yl)-1H-pyrazol-3-yl)thiazol (755);
2-(4-Isopropyl-5-(8-methoxy-[1,2,4]triazolo[1,5-a]pyridin-6-yl)-1H-pyrazol-3-yl)-5-(1-propylpiperidin-4-yl)thiazol (756);
2-(4-Isopropyl-5-(8-methoxy-[1,2,4]triazolo[1,5-a]pyridin-6-yl)-1H-pyrazol-3-yl)-5-(1-isopropylpiperidin-4-yl)thiazol (757);
2-(4-Isopropyl-5-(8-methoxy-[1,2,4]triazolo[1,5-a]pyridin-6-yl)-1H-pyrazol-3-yl)-5-(1-(tetrahydro-2H-pyran-4-yl)piperidin-4-yl)thiazol (758);
5-(1-(3-Ethoxycyclobutyl)piperidin-4-yl)-2-(4-isopropyl-5-(8-methoxy-[1,2,4]triazolo[1,5-a]pyridin-6-yl)-1H-pyrazol-3-yl)thiazol (759);
2-(4-Isopropyl-5-(8-methoxy-[1,2,4]triazolo[1,5-a]pyridin-6-yl)-1H-pyrazol-3-yl)-5-(1-(oxetan-3-yl)piperidin-4-yl)thiazol (760);
2-(4-Isopropyl-5-(8-methoxy-[1,2,4]triazolo[1,5-a]pyridin-6-yl)-1H-pyrazol-3-yl)-5-(1-neopentylpiperidin-4-yl)thiazol (761);
5-(1-Isopentylpiperidin-4-yl)-2-(4-isopropyl-5-(8-methoxy-[1,2,4]triazolo[1,5-a]pyridin-6-yl)-1H-pyrazol-3-yl)thiazol (762);
5-(1-((3-Ethyloxetan-3-yl)methyl)piperidin-4-yl)-2-(4-isopropyl-5-(8-methoxy-[1,2,4]triazolo[1,5-a]pyridin-6-yl)-1H-pyrazol-3-yl)thiazol (763);
3-(4-(2-(4-Isopropyl-5-(8-methoxy-[1,2,4]triazolo[1,5-a]pyridin-6-yl)-1H-pyrazol-3-yl)thiazol-5-yl)piperidin-1-yl)tetrahydrothiophen 1,1-dioxid (764);
2-(4-Isopropyl-5-(8-methoxy-[1,2,4]triazolo[1,5-a]pyridin-6-yl)-1H-pyrazol-3-yl)-5-(1-(pentan-3-yl)piperidin-4-yl)thiazol (765);
2-(4-Isopropyl-5-(8-methoxy-[1,2,4]triazolo[1,5-a]pyridin-6-yl)-1H-pyrazol-3-yl)-5-(1-(oxetan-3-yl)piperidin-4-yl)thiazol (766);
2-(4-Isopropyl-5-(8-methoxy-[1,2,4]triazolo[1,5-a]pyridin-6-yl)-1H-pyrazol-3-yl)-5-(1-(oxetan-3-yl)piperidin-4-yl)thiazol (767);
2-(4-Isopropyl-5-(8-methoxy-[1,2,4]triazolo[1,5-a]pyridin-6-yl)-1H-pyrazol-3-yl)-5-(1-((tetrahydrofuran-3-yl)methyl)piperidin-4-yl)thiazol (786);
2-(4-Isopropyl-5-(8-methoxy-[1,2,4]triazolo[1,5-a]pyridin-6-yl)-1H-pyrazol-3-yl)-5-(1-(oxetan-3-yl)piperidin-4-yl)thiazol (769);
2-(4-Isopropyl-5-(8-methoxy-[1,2,4]triazolo[1,5-a]pyridin-6-yl)-1H-pyrazol-3-yl)-5-(1-(tetrahydro-2H-pyran-3-yl)piperidin-4-yl)thiazol (770);
2-(4-Isopropyl-5-(8-methoxy-[1,2,4]triazolo[1,5-a]pyridin-6-yl)-1H-pyrazol-3-yl)-5-(1-(tetrahydro-2H-pyran-4-yl)piperidin-4-yl)thiazol (771);
2-(4-Isopropyl-5-(8-methyl-[1,2,4]triazolo[1,5-a]pyridin-6-yl)-1H-pyrazol-3-yl)-5-(1-methylpiperidin-4-yl)thiazol (772);
5-(1-Ethylpiperidin-4-yl)-2-(4-isopropyl-5-(8-methyl-[1,2,4]triazolo[1,5-a]pyridin-6-yl)-1H-pyrazol-3-yl)thiazol (773);
2-(4-Isopropyl-5-(8-methyl-[1,2,4]triazolo[1,5-a]pyridin-6-yl)-1H-pyrazol-3-yl)-5-(1-isopropylpiperidin-4-yl)thiazol (774);
2-(4-Isopropyl-5-(8-methyl-[1,2,4]triazolo[1,5-a]pyridin-6-yl)-1H-pyrazol-3-yl)-5-(1-propylpiperidin-4-yl)thiazol (775);
5-(1-((3-Ethyloxetan-3-yl)methyl)piperidin-4-yl)-2-(4-isopropyl-5-(8-methyl-[1,2,4]triazolo[1,5-a]pyridin-6-yl)-1H-pyrazol-3-yl)thiazol (776);
2-(4-Isopropyl-5-(8-methyl-[1,2,4]triazolo[1,5-a]pyridin-6-yl)-1H-pyrazol-3-yl)-5-(1-(tetrahydro-2H-pyran-4-yl)piperidin-4-yl)thiazol (777);
2-(4-Isopropyl-5-(8-methyl-[1,2,4]triazolo[1,5-a]pyridin-6-yl)-1H-pyrazol-3-yl)-5-(1-(oxetan-3-yl)piperidin-4-yl)thiazol (778);
2-(4-Isopropyl-5-(8-methoxy-[1,2,4]triazolo[1,5-a]pyridin-6-yl)-1H-pyrazol-3-yl)-4-methyl-5-(1-methylpiperidin-4-yl)thiazol (779);
5-(1-Ethylpiperidin-4-yl)-2-(4-isopropyl-5-(8-methoxy-[1,2,4]triazolo[1,5-a]pyridin-6-yl)-1H-pyrazol-3-yl)-4-methylthiazol (780);
2-(4-Isopropyl-5-(8-methoxy-[1,2,4]triazolo[1,5-a]pyridin-6-yl)-1H-pyrazol-3-yl)-4-methyl-5-(1-(tetrahydro-2H-pyran-4-yl)piperidin-4-yl)thiazol (781);
5-(1-(3-Ethoxycyclobutyl)piperidin-4-yl)-2-(4-isopropyl-5-(8-methoxy-[1,2,4]triazolo[1,5-a]pyridin-6-yl)-1H-pyrazol-3-yl)-4-methylthiazol (782);
2-(4-Isopropyl-5-(8-methoxy-[1,2,4]triazolo[1,5-a]pyridin-6-yl)-1H-pyrazol-3-yl)-4-methyl-5-(1-propylpiperidin-4-yl)thiazol (783);
2-(4-Isopropyl-5-(8-methoxy-[1,2,4]triazolo[1,5-a]pyridin-6-yl)-1H-pyrazol-3-yl)-5-(1-isopropylpiperidin-4-yl)-4-methylthiazol (784);
2-(4-Isopropyl-5-(8-methoxy-[1,2,4]triazolo[1,5-a]pyridin-6-yl)-1H-pyrazol-3-yl)-4-methyl-5-(1-((tetrahydro-2H-pyran-4-yl)methyl)piperidin-4-yl)thiazol (785);
5-(1-(3-Ethoxycyclobutyl)piperidin-4-yl)-2-(4-isopropyl-5-(8-methoxy-[1,2,4]triazolo[1,5-a]pyridin-6-yl)-1H-pyrazol-3-yl)-4-methylthiazol (786);
5-(1-(Cyclopropylmethyl)piperidin-4-yl)-2-(4-isopropyl-5-(8-methoxy-[1,2,4]triazolo[1,5-a]pyridin-6-yl)-1H-pyrazol-3-yl)-4-methylthiazol (787);
5-(1-(Cyclopropylmethyl)piperidin-4-yl)-2-(4-isopropyl-5-(8-methoxy-[1,2,4]triazolo[1,5-a]pyridin-6-yl)-1H-pyrazol-3-yl)thiazol (788);
5-(1-Isobutylpiperidin-4-yl)-2-(4-isopropyl-5-(8-methoxy-[1,2,4]triazolo[1,5-a]pyridin-6-yl)-1H-pyrazol-3-yl)thiazol (789);
2-(4-Isopropyl-5-(8-methoxy-[1,2,4]triazolo[1,5-a]pyridin-6-yl)-1H-pyrazol-3-yl)-5-(1-((1-methyl-1H-1,2,4-triazol-3-yl)methyl)piperidin-4-yl)thiazol (790);
5-(1-(Cyclobutylmethyl)piperidin-4-yl)-2-(4-isopropyl-5-(8-methoxy-[1,2,4]triazolo[1,5-a]pyridin-6-yl)-1H-pyrazol-3-yl)thiazol (791);
5-(1-(Cyclobutylmethyl)piperidin-4-yl)-2-(4-isopropyl-5-(8-methoxy-[1,2,4]triazolo[1,5-a]pyridin-6-yl)-1H-pyrazol-3-yl)-4-methylthiazol (792);
5-(1-Isobutylpiperidin-4-yl)-2-(4-isopropyl-5-(8-methoxy-[1,2,4]triazolo[1,5-a]pyridin-6-yl)-1H-pyrazol-3-yl)-4-methylthiazol (793);
2-(5-(3,4-Dimethoxyphenyl)-4-isopropyl-1H-pyrazol-3-yl)-5-(1-methylpiperidin-4-yl)thiazol (794);
2-(5-(3,4-Dimethoxyphenyl)-4-isopropyl-1H-pyrazol-3-yl)-5-(1-ethylpiperidin-4-yl)thiazol (795);
2-(5-(3,4-Dimethoxyphenyl)-4-isopropyl-1H-pyrazol-3-yl)-5-(1-propylpiperidin-4-yl)thiazol (796),
5-(1-(Cyclopropylmethyl)piperidin-4-yl)-2-(5-(3,4-Dimethoxyphenyl)-4-isopropyl-1H-pyrazol-3-yl)thiazol (797);
2-(5-(3,4-Dimethoxyphenyl)-4-isopropyl-1H-pyrazol-3-yl)-5-(1-isopropylpiperidin-4-yl)thiazol (798);
2-(5-(3,4-Dimethoxyphenyl)-4-isopropyl-1H-pyrazol-3-yl)-5-(1-(tetrahydro-2H-pyran-4-yl)piperidin-4-yl)thiazol (799);
2-(5-(3,4-Dimethoxyphenyl)-4-isopropyl-1H-pyrazol-3-yl)-5-(1-((3-ethyloxetan-3-yl)methyl)piperidin-4-yl)thiazol (800);
2-(5-(7,8-Dimethyl-[1,2,4]triazolo[1,5-a]pyridin-6-yl)-4-isopropyl-1H-pyrazol-3-yl)-5-(1-methylpiperidin-4-yl)thiazol (801);
2-(5-(7,8-Dimethyl-[1,2,4]triazolo[1,5-a]pyridin-6-yl)-4-isopropyl-1H-pyrazol-3-yl)-5-(1-ethylpiperidin-4-yl)thiazol (802);
2-(5-(7,8-Dimethyl-[1,2,4]triazolo[1,5-a]pyridin-6-yl)-4-isopropyl-1H-pyrazol-3-yl)-5-(1-propylpiperidin-4-yl)thiazol (803);
2-(5-(7,8-Dimethyl-[1,2,4]triazolo[1,5-a]pyridin-6-yl)-4-isopropyl-1H-pyrazol-3-yl)-5-(1-isopropylpiperidin-4-yl)thiazol (804);
5-(1-(Cyclopropylmethyl)piperidin-4-yl)-2-(5-(7,8-dimethyl-[1,2,4]triazolo[1,5-a]pyridin-6-yl)-4-isopropyl-1H-pyrazol-3-yl)thiazol (805);
2-(5-(7,8-Dimethyl-[1,2,4]triazolo[1,5-a]pyridin-6-yl)-4-isopropyl-1H-pyrazol-3-yl)-5-(1-(tetrahydro-2H-pyran-4-yl)piperidin-4-yl)thiazol (806);
2-(5-(7,8-Dimethyl-[1,2,4]triazolo[1,5-a]pyridin-6-yl)-4-isopropyl-1H-pyrazol-3-yl)-5-(1-(3-ethoxycyclobutyl)piperidin-4-yl)thiazol (807);
2-(5-(2,6-Dimethylpyridin-4-yl)-4-isopropyl-1H-pyrazol-3-yl)-5-(1-methylpiperidin-4-yl)thiazol (808);
2-(5-(2,6-Dimethylpyridin-4-yl)-4-isopropyl-1H-pyrazol-3-yl)-5-(1-propylpiperidin-4-yl)thiazol (809);
2-(5-(2,6-Dimethylpyridin-4-yl)-4-isopropyl-1H-pyrazol-3-yl)-5-(1-isopropylpiperidin-4-yl)thiazol (810);
2-(5-(2,6-Dimethylpyridin-4-yl)-4-isopropyl-1H-pyrazol-3-yl)-5-(1-(Tetrahydro-2H-pyran-4-yl)piperidin-4-yl)thiazol (811);
2-(5-(7,8-Dimethyl-[1,2,4]triazolo[1,5-a]pyridin-6-yl)-4-isopropyl-1H-pyrazol-3-yl)-4-methyl-5-(1-methylpiperidin-4-yl)thiazol (812);
2-(5-(7,8-Dimethyl-[1,2,4]triazolo[1,5-a]pyridin-6-yl)-4-isopropyl-1H-pyrazol-3-yl)-5-(1-isopropylpiperidin-4-yl)-4-methylthiazol (813);
5-(1-(Cyclopropylmethyl)piperidin-4-yl)-2-(5-(7,8-dimethyl-[1,2,4]triazolo[1,5-a]pyridin-6-yl)-4-isopropyl-1H-pyrazol-3-yl)-4-methylthiazol (814);
5-(3-(5-(1-Ethylpiperidin-4-yl)thiazol-2-yl)-4-isopropyl-1H-pyrazol-5-yl)-1,3,4-trimethylpyridin-2(1H)-on (815);
5-(4-Isopropyl-3-(5-(1-methylpiperidin-4-yl)thiazol-2-yl)-1H-pyrazol-5-yl)-1,3,4-trimethylpyridin-2(1H)-on (816);
2-(5-(7,8-Dimethyl-[1,2,4]triazolo[1,5-a]pyridin-6-yl)-4-isopropyl-1H-pyrazol-3-yl)-5-(1-((tetrahydro-2H-pyran-4-yl)methyl)piperidin-4-yl)thiazol (817);
5-(4-Isopropyl-3-(5-(1-propylpiperidin-4-yl)thiazol-2-yl)-1H-pyrazol-5-yl)-1,3,4-trimethylpyridin-2(1H)-on (818);
5-(3-(5-(1-(Cyclopropylmethyl)piperidin-4-yl)thiazol-2-yl)-4-isopropyl-1H-pyrazol-5-yl)-1,3,4-trimethylpyridin-2(1H)-on (819);
5-(1-(2-Ethylbutyl)piperidin-4-yl)-2-(4-isopropyl-5-(8-methoxyimidazo[1,2-a]pyridin-6-yl)-1H-pyrazol-3-yl)thiazol (820);
5-(1-Isobutylpiperidin-4-yl)-2-(4-isopropyl-5-(8-methoxyimidazo[1,2-a]pyridin-6-yl)-1H-pyrazol-3-yl)thiazol (821);
2-(4-Isopropyl-5-(8-methoxyimidazo[1,2-a]pyridin-6-yl)-1H-pyrazol-3-yl)-5-(1-propylpiperidin-4-yl)thiazol (822);
2-(4-Isopropyl-5-(8-methoxyimidazo[1,2-a]pyridin-6-yl)-1H-pyrazol-3-yl)-5-(1-(tetrahydro-2H-pyran-4-yl)piperidin-4-yl)thiazol (823);
2-(4-Isopropyl-5-(8-methoxyimidazo[1,2-a]pyridin-6-yl)-1H-pyrazol-3-yl)-5-(1-(3,3,3-trifluorpropyl)piperidin-4-yl)thiazol (824);
5-(1-Cyclobutylpiperidin-4-yl)-2-(4-isopropyl-5-(8-methoxyimidazo[1,2-a]pyridin-6-yl)-1H-pyrazol-3-yl)thiazol (825);
2-(4-Isopropyl-5-(8-methoxyimidazo[1,2-a]pyridin-6-yl)-1H-pyrazol-3-yl)-5-(1-(oxetan-3-yl)piperidin-4-yl)thiazol (826);
2-(4-Isopropyl-5-(8-methoxyimidazo[1,2-a]pyridin-6-yl)-1H-pyrazol-3-yl)-5-(1-(pentan-3-yl)piperidin-4-yl)thiazol (827);
2-(4-Isopropyl-5-(8-methoxyimidazo[1,2-a]pyridin-6-yl)-1H-pyrazol-3-yl)-5-(1-isopropylpiperidin-4-yl)thiazol (828);
2-(4-Isopropyl-5-(8-methoxyimidazo[1,2-a]pyridin-6-yl)-1H-pyrazol-3-yl)-5-(1-methylpiperidin-4-yl)thiazol (829);
2-(4-Isopropyl-5-(8-methoxyimidazo[1,2-a]pyridin-6-yl)-1H-pyrazol-3-yl)-5-(1-neopentylpiperidin-4-yl)thiazol (830);
5-(1-Ethylpiperidin-4-yl)-2-(4-isopropyl-5-(8-methoxyimidazo[1,2-a]pyridin-6-yl)-1H-pyrazol-3-yl)thiazol (831);
5-(1-(Cyclopropylmethyl)piperidin-4-yl)-2-(4-isopropyl-5-(8-methoxyimidazo[1,2-a]pyridin-6-yl)-1H-pyrazol-3-yl)thiazol (832);
2-(5-(7,8-Dimethyl-[1,2,4]triazolo[1,5-a]pyridin-6-yl)-4-isopropyl-1H-pyrazol-3-yl)-4-methyl-5-(1-propylpiperidin-4-yl)thiazol (833);
2-(5-(7,8-Dimethyl-[1,2,4]triazolo[1,5-a]pyridin-6-yl)-4-isopropyl-1H-pyrazol-3-yl)-4-methyl-5-(1-((tetrahydro-2H-pyran-4-yl)methyl)piperidin-4-yl)thiazol (834);
2-(5-(7,8-Dimethyl-[1,2,4]triazolo[1,5-a]pyridin-6-yl)-4-isopropyl-1H-pyrazol-3-yl)-4-methyl-5-(1-(oxetan-3-yl)piperidin-4-yl)thiazol (835);
2-(5-(7,8-Dimethyl-[1,2,4]triazolo[1,5-a]pyridin-6-yl)-4-isopropyl-1H-pyrazol-3-yl)-4-methyl-5-(1-(tetrahydro-2H-pyran-4-yl)piperidin-4-yl)thiazol (836);
2-(4-Isopropyl-5-(8-methoxy-[1,2,4]triazolo[1,5-a]pyridin-6-yl)-1H-pyrazol-3-yl)-5-(1-(oxetan-3-yl)piperidin-4-yl)-4-(trifluormethyl)thiazol (837);
5-(1-(Cyclopropylmethyl)piperidin-4-yl)-2-(4-isopropyl-5-(8-methoxy-[1,2,4]triazolo[1,5-a]pyridin-6-yl)-1H-pyrazol-3-yl)-4-(trifluormethyl)thiazol (838);
2-(4-Isopropyl-5-(8-methoxy-[1,2,4]triazolo[1,5-a]pyridin-6-yl)-1H-pyrazol-3-yl)-5-(1-isopropylpiperidin-4-yl)-4-(trifluormethyl)thiazol (839);
2-(4-Isopropyl-5-(8-methoxy-[1,2,4]triazolo[1,5-a]pyridin-6-yl)-1H-pyrazol-3-yl)-5-(1-((tetrahydro-2H-pyran-4-yl)methyl)piperidin-4-yl)-4-(trifluormethyl)thiazol (840);
2-(4-Isopropyl-5-(8-methoxy-[1,2,4]triazolo[1,5-a]pyridin-6-yl)-1H-pyrazol-3-yl)-5-(1-methylpiperidin-4-yl)-4-(trifluormethyl)thiazol (841);
5-(1-Ethylpiperidin-4-yl)-2-(4-isopropyl-5-(8-methoxy-[1,2,4]triazolo[1,5-a]pyridin-6-yl)-1H-pyrazol-3-yl)-4-(trifluormethyl)thiazol (842);
2-(4-Isopropyl-5-(8-methoxy-[1,2,4]triazolo[1,5-a]pyridin-6-yl)-1H-pyrazol-3-yl)-5-(1-propylpiperidin-4-yl)-4-(trifluormethyl)thiazol (843);
1-(4-(2-(4-Isopropyl-5-(8-methoxy-[1,2,4]triazolo[1,5-a]pyridin-6-yl)-1H-pyrazol-3-yl)thiazol-5-yl)piperidin-1-yl)-2-morpholinoethan-1-on (844);
2-(Dimethylamino)-1-(4-(2-(4-isopropyl-5-(8-methoxy-[1,2,4]triazolo[1,5-a]pyridin-6-yl)-1H-pyrazol-3-yl)thiazol-5-yl)piperidin-1-yl)ethan-1-on (845);
2-(Dimethylamino)-1-(4-(2-(4-isopropyl-5-(8-methoxy-[1,2,4]triazolo[1,5-a]pyridin-6-yl)-1H-pyrazol-3-yl)-4-methylthiazol-5-yl)piperidin-1-yl)ethan-1-on (846);
2-(Diethylamino)-1-(4-(2-(4-isopropyl-5-(8-methoxy-[1,2,4]triazolo[1,5-a]pyridin-6-yl)-1H-pyrazol-3-yl)thiazol-5-yl)piperidin-1-yl)ethan-1-on (847);
2-(Diethylamino)-1-(4-(2-(4-isopropyl-5-(8-methoxy-[1,2,4]triazolo[1,5-a]pyridin-6-yl)-1H-pyrazol-3-yl)-4-methylthiazol-5-yl)piperidin-1-yl)ethan-1-on (848);
2-(Dimethylamino)-1-(4-(2-(4-isopropyl-5-(8-methyl-[1,2,4]triazolo[1,5-a]pyridin-6-yl)-1H-pyrazol-3-yl)thiazol-5-yl)piperidin-1-yl)ethan-1-on (849);
1-(4-(2-(5-(7,8-Dimethyl-[1,2,4]triazolo[1,5-a]pyridin-6-yl)-4-isopropyl-1H-pyrazol-3-yl)-4-methylthiazol-5-yl)piperidin-1-yl)-2-(dimethylamino)ethan-1-on (850);
1-(4-(2-(5-(7,8-Dimethyl-[1,2,4]triazolo[1,5-a]pyridin-6-yl)-4-isopropyl-1H-pyrazol-3-yl)thiazol-5-yl)piperidin-1-yl)-2-(dimethylamino)ethan-1-on (851);
2-(Dimethylamino)-1-(4-(2-(5-(2,6-Dimethylpyridin-4-yl)-4-isopropyl-1H-pyrazol-3-yl)thiazol-5-yl)piperidin-1-yl)ethan-1-on (852);
1-(4-(2-(5-(3,4-Dimethoxyphenyl)-4-isopropyl-1H-pyrazol-3-yl)thiazol-5-yl)piperidin-1-yl)-2-(dimethylamino)ethan-1-on (853);
2-(Dimethylamino)-1-(4-(2-(4-isopropyl-5-(8-methoxy-[1,2,4]triazolo[1,5-a]pyridin-6-yl)-1H-pyrazol-3-yl)-4-(trifluormethyl)thiazol-5-yl)piperidin-1-yl)ethan-1-on (854);
2-(4-Isopropyl-5-(8-methoxy-[1,2,4]triazolo[1,5-a]pyridin-6-yl)-1H-pyrazol-3-yl)-5-(1-(2-(methylsulfonyl)ethyl)piperidin-4-yl)thiazol (855);
2-(4-(2-(4-Isopropyl-5-(8-methoxy-[1,2,4]triazolo[1,5-a]pyridin-6-yl)-1H-pyrazol-3-yl)thiazol-5-yl)piperidin-1-yl)-N,N-dimethylacetamid (856);
2-(4-(2-(4-Isopropyl-5-(8-methoxy-[1,2,4]triazolo[1,5-a]pyridin-6-yl)-1H-pyrazol-3-yl)thiazol-5-yl)piperidin-1-yl)acetamid (857);
2-(4-Isopropyl-5-(8-methoxy-[1,2,4]triazolo[1,5-a]pyridin-6-yl)-1H-pyrazol-3-yl)-5-(1-(2-methoxyethyl)piperidin-4-yl)thiazol (858);
2-(4-(2-(4-Isopropyl-5-(8-methoxy-[1,2,4]triazolo[1,5-a]pyridin-6-yl)-1H-pyrazol-3-yl)thiazol-5-yl)piperidin-1-yl)acetonitril (859);
1-(4-(2-(4-Isopropyl-5-(8-methoxy-[1,2,4]triazolo[1,5-a]pyridin-6-yl)-1H-pyrazol-3-yl)thiazol-5-yl)piperidin-1-yl)-2-methylpropan-2-ol (860);
2-(4-(2-(4-Isopropyl-5-(8-methoxy-[1,2,4]triazolo[1,5-a]pyridin-6-yl)-1H-pyrazol-3-yl)-4-methylthiazol-5-yl)piperidin-1-yl)-N,N-dimethylacetamid (861);
2-(4-Isopropyl-5-(8-methoxy-[1,2,4]triazolo[1,5-a]pyridin-6-yl)-1H-pyrazol-3-yl)-5-(1-(2-methoxyethyl)piperidin-4-yl)-4-methylthiazol (862);
2-(4-Isopropyl-5-(8-methoxy-[1,2,4]triazolo[1,5-a]pyridin-6-yl)-1H-pyrazol-3-yl)-4-methyl-5-(1-(2-(methylsulfonyl)ethyl)piperidin-4-yl)thiazol (863);
2-(4-Isopropyl-5-(8-methoxy-[1,2,4]triazolo[1,5-a]pyridin-6-yl)-1H-pyrazol-3-yl)-5-(1-((1-(methylsulfonyl)cyclopropyl)methyl)piperidin-4-yl)thiazol (864);
2-(4-Isopropyl-5-(8-methyl-[1,2,4]triazolo[1,5-a]pyridin-6-yl)-1H-pyrazol-3-yl)-5-(1-(2-(methylsulfonyl)ethyl)piperidin-4-yl)thiazol (865);
2-(4-Isopropyl-5-(8-methyl-[1,2,4]triazolo[1,5-a]pyridin-6-yl)-1H-pyrazol-3-yl)-5-(1-(2-methoxyethyl)piperidin-4-yl)thiazol (866);
1-(4-(2-(4-Isopropyl-5-(8-methyl-[1,2,4]triazolo[1,5-a]pyridin-6-yl)-1H-pyrazol-3-yl)thiazol-5-yl)piperidin-1-yl)-2-methylpropan-2-ol (867);
2-(4-(2-(4-Isopropyl-5-(8-methyl-[1,2,4]triazolo[1,5-a]pyridin-6-yl)-1H-pyrazol-3-yl)thiazol-5-yl)piperidin-1-yl)acetonitril (868);
2-(4-(2-(4-Isopropyl-5-(8-methyl-[1,2,4]triazolo[1,5-a]pyridin-6-yl)-1H-pyrazol-3-yl)thiazol-5-yl)piperidin-1-yl)-N,N-dimethylacetamid (869);
2-(4-(2-(5-(7,8-Dimethyl-[1,2,4]triazolo[1,5-a]pyridin-6-yl)-4-isopropyl-1H-pyrazol-3-yl)thiazol-5-yl)piperidin-1-yl)-N,N-dimethylacetamid (870);
2-(5-(7,8-Dimethyl-[1,2,4]triazolo[1,5-a]pyridin-6-yl)-4-isopropyl-1H-pyrazol-3-yl)-5-(1-(2-methoxyethyl)piperidin-4-yl)thiazol (871);
2-(5-(7,8-Dimethyl-[1,2,4]triazolo[1,5-a]pyridin-6-yl)-4-isopropyl-1H-pyrazol-3-yl)-5-(1-(2-methoxyethyl)piperidin-4-yl)-4-methylthiazol (872);
2-(5-(7,8-Dimethyl-[1,2,4]triazolo[1,5-a]pyridin-6-yl)-4-isopropyl-1H-pyrazol-3-yl)-4-methyl-5-(1-(2-(methylsulfonyl)ethyl)piperidin-4-yl)thiazol (873);
2-(5-(2,6-Dimethylpyridin-4-yl)-4-isopropyl-1H-pyrazol-3-yl)-5-(1-(2-Methoxyethyl)piperidin-4-yl)thiazol (874);
2-(4-(2-(5-(2,6-Dimethylpyridin-4-yl)-4-isopropyl-1H-pyrazol-3-yl)thiazol-5-yl)piperidin-1-yl)-N,N-dimethylacetamid (875);
2-(5-(3,4-Dimethoxyphenyl)-4-isopropyl-1H-pyrazol-3-yl)-5-(1-(2-methoxyethyl)piperidin-4-yl)thiazol (877);
2-(4-Isopropyl-5-(8-methoxy-[1,2,4]triazolo[1,5-a]pyridin-6-yl)-1H-pyrazol-3-yl)-5-(1-(2-(methylsulfonyl)ethyl)piperidin-4-yl)-4-(trifluormethyl)thiazol (878);
2-(4-(2-(4-Isopropyl-5-(8-methoxy-[1,2,4]triazolo[1,5-a]pyridin-6-yl)-1H-pyrazol-3-yl)-4-(trifluormethyl)thiazol-5-yl)piperidin-1-yl)-N,N-dimethylacetamid (879);
2-(4-Isopropyl-5-(8-methoxy-[1,2,4]triazolo[1,5-a]pyridin-6-yl)-1H-pyrazol-3-yl)-5-(1-(2-methoxyethyl)piperidin-4-yl)-4-(trifluormethyl)thiazol (880);
5-(1-(3-Fluorpropyl)piperidin-4-yl)-2-(4-isopropyl-5-(8-methoxy-[1,2,4]triazolo[1,5-a]pyridin-6-yl)-1H-pyrazol-3-yl)-4-methylthiazol (881);
5-(1-(2-Fluorethyl)piperidin-4-yl)-2-(4-isopropyl-5-(8-methoxy-[1,2,4]triazolo[1,5-a]pyridin-6-yl)-1H-pyrazol-3-yl)-4-methylthiazol (882);
4-(4-(2-(4-Isopropyl-5-(8-methoxy-[1,2,4]triazolo[1,5-a]pyridin-6-yl)-1H-pyrazol-3-yl)-4-methylthiazol-5-yl)cyclohexyl)morpholin (883);
4-(2-(4-Isopropyl-5-(8-methoxy-[1,2,4]triazolo[1,5-a]pyridin-6-yl)-1H-pyrazol-3-yl)-4-methylthiazol-5-yl)-N-(2-methoxyethyl)cyclohexan-1-amin (884-885);
N-(4-(2-(4-Isopropyl-5-(8-methoxy-[1,2,4]triazolo[1,5-a]pyridin-6-yl)-1H-pyrazol-3-yl)-4-methylthiazol-5-yl)cyclohexyl)oxetan-3-amin (886-887);
4-(2-(4-Isopropyl-5-(8-methoxy-[1,2,4]triazolo[1,5-a]pyridin-6-yl)-1H-pyrazol-3-yl)-4-methylthiazol-5-yl)-N-(2-methoxyethyl)-N-methylcyclohexan-1-amin (888-889);
6-(4-(2-(4-Isopropyl-5-(8-methoxy-[1,2,4]triazolo[1,5-a]pyridin-6-yl)-1H-pyrazol-3-yl)-4-methylthiazol-5-yl)cyclohexyl)-2-oxa-6-azaspiro[3.3]heptan (890-891);
4-(2-(4-Isopropyl-5-(8-methoxy-[1,2,4]triazolo[1,5-a]pyridin-6-yl)-1H-pyrazol-3-yl)-4-methylthiazol-5-yl)-N-((1-(methylsulfonyl)cyclopropyl)methyl)cyclohexan-1-amin (892-893);
6-(4-(2-(4-Isopropyl-5-(8-methoxy-[1,2,4]triazolo[1,5-a]pyridin-6-yl)-1H-pyrazol-3-yl)-4-methylthiazol-5-yl)cyclohexyl)-2-thia-6-azaspiro[3.3]heptane 2,2-dioxid (894-895);
N-(4-(2-(4-Isopropyl-5-(8-methoxy-[1,2,4]triazolo[1,5-a]pyridin-6-yl)-1H-pyrazol-3-yl)-4-methylthiazol-5-yl)cyclohexyl)tetrahydro-2H-pyran-4-amin (896-897);
2-(4-Isopropyl-5-(8-methoxy-[1,2,4]triazolo[1,5-a]pyridin-6-yl)-1H-pyrazol-3-yl)-4-methyl-5-(4-(pyrrolidin-1-yl)cyclohexyl)thiazol (898);
N-Cyclobutyl-4-(2-(4-isopropyl-5-(8-methoxy-[1,2,4]triazolo[1,5-a]pyridin-6-yl)-1H-pyrazol-3-yl)-4-methylthiazol-5-yl)cyclohexan-1-amin (891B und 892B);
4-(4-(2-(4-Isopropyl-5-(8-methoxy-[1,2,4]triazolo[1,5-a]pyridin-6-yl)-1H-pyrazol-3-yl)-4-methylthiazol-5-yl)cyclohexyl)piperazin-2-on (893B und 894B);
2-(4-Isopropyl-5-(8-methoxy-[1,2,4]triazolo[1,5-a]pyridin-6-yl)-1H-pyrazol-3-yl)-4-methyl-5-(4-(4-(methylsulfonyl)piperazin-1-yl)cyclohexyl)thiazol (895B und 896B);
4-(2-(4-Isopropyl-5-(8-methoxy-[1,2,4]triazolo[1,5-a]pyridin-6-yl)-1H-pyrazol-3-yl)-4-methylthiazol-5-yl)-N-(2-methoxy-2-methylpropyl)cyclohexan-1-amin (897B und 898B);
4-(2-(4-Isopropyl-5-(8-methoxy-[1,2,4]triazolo[1,5-a]pyridin-6-yl)-1H-pyrazol-3-yl)-4-methylthiazol-5-yl)-N-((3-methyloxetan-3-yl)methyl)cyclohexan-1-amin (899B und 900);
2-(4-(2-(4-Isopropyl-5-(8-methoxy-[1,2,4]triazolo[1,5-a]pyridin-6-yl)-1H-pyrazol-3-yl)-4-methylthiazol-5-yl)cyclohexyl)-7-oxa-2-azaspiro[3.5]nonan (901-902);
4-(4-(2-(4-Isopropyl-5-(8-methoxy-[1,2,4]triazolo[1,5-a]pyridin-6-yl)-1H-pyrazol-3-yl)-4-methylthiazol-5-yl)cyclohexyl)-1,4-oxazepan (903-904);
2-(4-Isopropyl-5-(8-methoxy-[1,2,4]triazolo[1,5-a]pyridin-6-yl)-1H-pyrazol-3-yl)-5-(4-(3-methoxyazetidin-1-yl)cyclohexyl)-4-methylthiazol (905-906);
7-(4-(2-(4-Isopropyl-5-(8-methoxy-[1,2,4]triazolo[1,5-a]pyridin-6-yl)-1H-pyrazol-3-yl)-4-methylthiazol-5-yl)cyclohexyl)-2-oxa-7-azaspiro[3.5]nonan (907-908);
1-(4-(2-(4-Isopropyl-5-(8-methoxy-[1,2,4]triazolo[1,5-a]pyridin-6-yl)-1H-pyrazol-3-yl)-4-methylthiazol-5-yl)cyclohexyl)-3-(trifluormethyl)azetidin-3-ol (909-910);
4-(2-(4-Isopropyl-5-(8-methyl-[1,2,4]triazolo[1,5-a]pyridin-6-yl)-1H-pyrazol-3-yl)thiazol-5-yl)-N-methylcyclohexan-1-amin (911-912);
4-(2-(4-Isopropyl-5-(8-methyl-[1,2,4]triazolo[1,5-a]pyridin-6-yl)-1H-pyrazol-3-yl)thiazol-5-yl)-N,N-dimethylcyclohexan-1-amin (913-914);
N-(4-(2-(4-Isopropyl-5-(8-methoxy-[1,2,4]triazolo[1,5-a]pyridin-6-yl)-1H-pyrazol-3-yl)thiazol-5-yl)cyclohexyl)-3-methyloxetan-3-amin (915-916);
4-(2-(4-Isopropyl-5-(8-methoxy-[1,2,4]triazolo[1,5-a]pyridin-6-yl)-1H-pyrazol-3-yl)thiazol-5-yl)-N-(2-methoxy-2-methylpropyl)cyclohexan-1-amin (916-917);
N-Cyclobutyl-4-(2-(4-isopropyl-5-(8-methoxy-[1,2,4]triazolo[1,5-a]pyridin-6-yl)-1H-pyrazol-3-yl)thiazol-5-yl)cyclohexan-1-amin (918-919);
N -(4-(2-(4-Isopropyl-5-(8-methoxy-[1,2,4]triazolo[1,5-a]pyridin-6-yl)-1H-pyrazol-3-yl)thiazol-5-yl)cyclohexyl)oxetan-3-amin (920-921);
4-(2-(4-Isopropyl-5-(8-methoxy-[1,2,4]triazolo[1,5-a]pyridin-6-yl)-1H-pyrazol-3-yl)thiazol-5-yl)-N-(3,3,3-trifluorpropyl)cyclohexan-1-amin (922-923);
6-(4-(2-(4-Isopropyl-5-(8-methoxy-[1,2,4]triazolo[1,5-a]pyridin-6-yl)-1H-pyrazol-3-yl)thiazol-5-yl)cyclohexyl)-2-oxa-6-azaspiro[3.3]heptan (924);
2-(Dimethylamino)-N-(4-(2-(4-isopropyl-5-(8-methyl-[1,2,4]triazolo[1,5-a]pyridin-6-yl)-1H-pyrazol-3-yl)thiazol-5-yl)cyclohexyl)-N-methylacetamid (926-927);
4-(2-(4-Isopropyl-5-(8-methyl-[1,2,4]triazolo[1,5-a]pyridin-6-yl)-1H-pyrazol-3-yl)thiazol-5-yl)-N-methyl-N-(3,3,3-trifluorpropyl)cyclohexan-1-amin (928-929);
2-(4-Isopropyl-5-(8-methoxy-[1,2,4]triazolo[1,5-a]pyridin-6-yl)-1H-pyrazol-3-yl)-5-(piperazin-1-yl)thiazol (930);
2-(4-Isopropyl-5-(8-methoxy-[1,2,4]triazolo[1,5-a]pyridin-6-yl)-1H-pyrazol-3-yl)-5-(2,6-diazaspiro[3.3]heptan-2-yl)thiazol (931);
1-(2-(4-Isopropyl-5-(8-methoxy-[1,2,4]triazolo[1,5-a]pyridin-6-yl)-1H-pyrazol-3-yl)-4-methylthiazol-5-yl)piperidin-4-amin (932);
(S)-2-(4-Isopropyl-5-(8-methoxy-[1,2,4]triazolo[1,5-a]pyridin-6-yl)-1H-pyrazol-3-yl)-5-(2-methylpiperazin-1-yl)thiazol (933);
2-(4-Isopropyl-5-(8-methoxy-[1,2,4]triazolo[1,5-a]pyridin-6-yl)-1H-pyrazol-3-yl)-4-methyl-5-(piperazin-1-yl)thiazol (934);
5-((1S,4S)-2,5-Diazabicyclo[2.2.1]heptan-2-yl)-2-(4-isopropyl-5-(8-methoxy-[1,2,4]triazolo[1,5-a]pyridin-6-yl)-1H-pyrazol-3-yl)thiazol (935); 5-((2R,5S)-2,5-Dimethylpiperazin-1-yl)-2-(4-isopropyl-5-(8-methoxy-[1,2,4]triazolo[1,5-a]pyridin-6-yl)-1H-pyrazol-3-yl)thiazol (936);
(R)-2-(4-Isopropyl-5-(8-methoxy-[1,2,4]triazolo[1,5-a]pyridin-6-yl)-1H-pyrazol-3-yl)-4-methyl-5-(2-methylpiperazin-1-yl)thiazol (937);
(R)-2-(4-Isopropyl-5-(8-methoxy-[1,2,4]triazolo[1,5-a]pyridin-6-yl)-1H-pyrazol-3-yl)-5-(2-methylpiperazin-1-yl)thiazol (938);
2-(4-Isopropyl-5-(8-methoxy-[1,2,4]triazolo[1,5-a]pyridin-6-yl)-1H-pyrazol-3-yl)-5-(4-methyl Piperazin-1-yl)thiazol (939);
5-(4-Ethylpiperazin-1-yl)-2-(4-isopropyl-5-(8-methoxy-[1,2,4]triazolo[1,5-a]pyridin-6-yl)-1H-pyrazol-3-yl)thiazol (940);
2-(4-Isopropyl-5-(8-methoxy-[1,2,4]triazolo[1,5-a]pyridin-6-yl)-1H-pyrazol-3-yl)-5-(4-propylpiperazin-1-yl)thiazol (941);
2-(4-Isopropyl-5-(8-methoxy-[1,2,4]triazolo[1,5-a]pyridin-6-yl)-1H-pyrazol-3-yl)-5-(4-isopropylpiperazin-1-yl)thiazol (942);
2-(4-Isopropyl-5-(8-methoxy-[1,2,4]triazolo[1,5-a]pyridin-6-yl)-1H-pyrazol-3-yl)-5-(4-(oxetan-3-yl)piperazin-1-yl)thiazol (943);
5-(4-Isobutylpiperazin-1-yl)-2-(4-isopropyl-5-(8-methoxy-[1,2,4]triazolo[1,5-a]pyridin-6-yl)-1H-pyrazol-3-yl)thiazol (944);
2-(4-Isopropyl-5-(8-methoxy-[1,2,4]triazolo[1,5-a]pyridin-6-yl)-1H-pyrazol-3-yl)-5-(4-(tetrahydro-2H-pyran-4-yl)piperazin-1-yl)thiazol (945);
5-(4-(Cyclopropylmethyl)piperazin-1-yl)-2-(4-isopropyl-5-(8-methoxy-[1,2,4]triazolo[1,5-a]pyridin-6-yl)-1H-pyrazol-3-yl)thiazol (946);
2-(4-Isopropyl-5-(8-methoxy-[1,2,4]triazolo[1,5-a]pyridin-6-yl)-1H-pyrazol-3-yl)-5-(4-((tetrahydro-2H-pyran-4-yl)methyl)piperazin-1-yl)thiazol (947);
2-(4-Isopropyl-5-(8-methoxy-[1,2,4]triazolo[1,5-a]pyridin-6-yl)-1H-pyrazol-3-yl)-5-(6-isopropyl-2,6-diazaspiro[3.3]heptan-2-yl)thiazol (948);
N-Isopropyl-1-(2-(4-isopropyl-5-(8-methoxy-[1,2,4]triazolo[1,5-a]pyridin-6-yl)-1H-pyrazol-3-yl)-4-methylthiazol-5-yl)piperidin-4-amin (949);
1-(2-(4-Isopropyl-5-(8-methoxy-[1,2,4]triazolo[1,5-a]pyridin-6-yl)-1H-pyrazol-3-yl)-4-methylthiazol-5-yl)-N-(oxetan-3-yl)piperidin-4-amin (950);
(S)-5-(4-(Cyclopropylmethyl)-2-methylpiperazin-1-yl)-2-(4-isopropyl-5-(8-methoxy-[1,2,4]triazolo[1,5-a]pyridin-6-yl)-1H-pyrazol-3-yl)thiazol (951);
(S)-5-(2,4-Dimethylpiperazin-1-yl)-2-(4-isopropyl-5-(8-methoxy-[1,2,4]triazolo[1,5-a]pyridin-6-yl)-1H-pyrazol-3-yl)thiazol (952);
(S)-2-(4-Isopropyl-5-(8-methoxy-[1,2,4]triazolo[1,5-a]pyridin-6-yl)-1H-pyrazol-3-yl)-5-(2-methyl-4-propylpiperazin-1-yl)thiazol (953);
(S)-5-(4-Isopropyl-2-methylpiperazin-1-yl)-2-(4-isopropyl-5-(8-methoxy-[1,2,4]triazolo[1,5-a]pyridin-6-yl)-1H-pyrazol-3-yl)thiazol (954);
(S)-5-(4-Ethyl-2-methylpiperazin-1-yl)-2-(4-isopropyl-5-(8-methoxy-[1,2,4]triazolo[1,5-a]pyridin-6-yl)-1H-pyrazol-3-yl)thiazol (955);
(S)-5-(4-Isobutyl-2-methylpiperazin-1-yl)-2-(4-isopropyl-5-(8-methoxy-[1,2,4]triazolo[1,5-a]pyridin-6-yl)-1H-pyrazol-3-yl)thiazol (956);
N-Isobutyl-1-(2-(4-isopropyl-5-(8-methoxy-[1,2,4]triazolo[1,5-a]pyridin-6-yl)-1H-pyrazol-3-yl)-4-methylthiazol-5-yl)piperidin-4-amin (957);
2-(4-Isopropyl-5-(8-methoxy-[1,2,4]triazolo[1,5-a]pyridin-6-yl)-1H-pyrazol-3-yl)-4-methyl-5-(4-methylpiperazin-1-yl)thiazol (958);
5-(4-(Cyclopropylmethyl)piperazin-1-yl)-2-(4-isopropyl-5-(8-methoxy-[1,2,4]triazolo[1,5-a]pyridin-6-yl)-1H-pyrazol-3-yl)-4-methylthiazol (959);
5-(4-Ethylpiperazin-1-yl)-2-(4-isopropyl-5-(8-methoxy-[1,2,4]triazolo[1,5-a]pyridin-6-yl)-1H-pyrazol-3-yl)-4-methylthiazol (960);
5-(4-Isobutylpiperazin-1-yl)-2-(4-isopropyl-5-(8-methoxy-[1,2,4]triazolo[1,5-a]pyridin-6-yl)-1H-pyrazol-3-yl)-4-methylthiazol (961);
N-(Cyclopropylmethyl)-1-(2-(4-isopropyl-5-(8-methoxy-[1,2,4]triazolo[1,5-a]pyridin-6-yl)-1H-pyrazol-3-yl)-4-methylthiazol-5-yl)piperidin-4-amin (962);
1-(2-(4-Isopropyl-5-(8-methoxy-[1,2,4]triazolo[1,5-a]pyridin-6-yl)-1H-pyrazol-3-yl)-4-methylthiazol-5-yl)-N-(tetrahydro-2H-pyran-4-yl)piperidin-4-amin (963);
1-(2-(4-Isopropyl-5-(8-methoxy-[1,2,4]triazolo[1,5-a]pyridin-6-yl)-1H-pyrazol-3-yl)-4-methylthiazol-5-yl)-N-propylpiperidin-4-amin (964);
1-(2-(4-Isopropyl-5-(8-methoxy-[1,2,4]triazolo[1,5-a]pyridin-6-yl)-1H-pyrazol-3-yl)-4-methylthiazol-5-yl)-N,N-bis((tetrahydro-2H-pyran-4-yl)methyl)piperidin-4-amin (965);
N,N-bis(Cyclopropylmethyl)-1-(2-(4-isopropyl-5-(8-methoxy-[1,2,4]triazolo[1,5-a]pyridin-6-yl)-1H-pyrazol-3-yl)-4-methylthiazol-5-yl)piperidin-4-amin (966);
1-(2-(4-Isopropyl-5-(8-methoxy-[1,2,4]triazolo[1,5-a]pyridin-6-yl)-1H-pyrazol-3-yl)-4-methylthiazol-5-yl)-N,N-dimethylpiperidin-4-amin (967);
2-(4-Isopropyl-5-(8-methoxy-[1,2,4]triazolo[1,5-a]pyridin-6-yl)-1H-pyrazol-3-yl)-4-methyl-5-(4-propylpiperazin-1-yl)thiazol (968);
2-(4-Isopropyl-5-(8-methoxy-[1,2,4]triazolo[1,5-a]pyridin-6-yl)-1H-pyrazol-3-yl)-4-methyl-5-(4-((tetrahydro-2H-pyran-4-yl)methyl)piperazin-1-yl)thiazol (969);
2-(4-Isopropyl-5-(8-methoxy-[1,2,4]triazolo[1,5-a]pyridin-6-yl)-1H-pyrazol-3-yl)-4-methyl-5-(4-(oxetan-3-yl)piperazin-1-yl)thiazol (970);
2-(4-Isopropyl-5-(8-methoxy-[1,2,4]triazolo[1,5-a]pyridin-6-yl)-1H-pyrazol-3-yl)-5-(4-isopropylpiperazin-1-yl)-4-methylthiazol (971);
5-(4-(Cyclobutylmethyl)piperazin-1-yl)-2-(4-isopropyl-5-(8-methoxy-[1,2,4]triazolo[1,5-a]pyridin-6-yl)-1H-pyrazol-3-yl)thiazol (972);
(S)-5-(4-(Cyclobutylmethyl)-2-methylpiperazin-1-yl)-2-(4-isopropyl-5-(8-methoxy-[1,2,4]triazolo[1,5-a]pyridin-6-yl)-1H-pyrazol-3-yl)thiazol (973);
5-((1S,4S)-5-Isobutyl-2,5-diazabicyclo[2.2.1]heptan-2-yl)-2-(4-isopropyl-5-(8-methoxy-[1,2,4]triazolo[1,5-a]pyridin-6-yl)-1H-pyrazol-3-yl)thiazol (974);
5-((1R,4R)-5-Ethyl-2,5-diazabicyclo[2.2.1]heptan-2-yl)-2-(4-isopropyl-5-(8-methoxy-[1,2,4]triazolo[1,5-a]pyridin-6-yl)-1H-pyrazol-3-yl)thiazol (975);
5-((1S,4S)-5-Cyclobutyl-2,5-diazabicyclo[2.2.1]heptan-2-yl)-2-(4-isopropyl-5-(8-methoxy-[1,2,4]triazolo[1,5-a]pyridin-6-yl)-1H-pyrazol-3-yl)thiazol (976);
2-(4-Isopropyl-5-(8-methoxy-[1,2,4]triazolo[1,5-a]pyridin-6-yl)-1H-pyrazol-3-yl)-5-((1S,4S)-5-isopropyl-2,5-diazabicyclo[2.2.1]heptan-2-yl)thiazol (977);
2-(4-Isopropyl-5-(8-methoxy-[1,2,4]triazolo[1,5-a]pyridin-6-yl)-1H-pyrazol-3-yl)-5-((1R,4R)-5-propyl-2,5-diazabicyclo[2.2.1]heptan-2-yl)thiazol (978);
2-(4-Isopropyl-5-(8-methoxy-[1,2,4]triazolo[1,5-a]pyridin-6-yl)-1H-pyrazol-3-yl)-5-((1S,4S)-5-(oxetan-3-yl)-2,5-diazabicyclo[2.2.1]heptan-2-yl)thiazol (979);
2-(4-Isopropyl-5-(8-methoxy-[1,2,4]triazolo[1,5-a]pyridin-6-yl)-1H-pyrazol-3-yl)-5-((1S, 4S)-5-(tetrahydro-2H-pyran-4-yl)-2,5-diazabicyclo[2.2.1]heptan-2-yl)thiazol (980);
2-(4-Isopropyl-5-(8-methoxy-[1,2,4]triazolo[1,5-a]pyridin-6-yl)-1H-pyrazol-3-yl)-5-((1S,4S)-5-((tetrahydro-2H-pyran-4-yl)methyl)-2,5-diazabicyclo[2.2.1]heptan-2-yl)thiazol (981);
5-((1S,4S)-5-(Cyclobutylmethyl)-2,5-diazabicyclo[2.2.1]heptan-2-yl)-2-(4-isopropyl-5-(8-methoxy-[1,2,4]triazolo[1,5-a]pyridin-6-yl)-1H-pyrazol-3-yl)thiazol (982);
5-((2R,5S)-2,5-Dimethyl-4-((tetrahydro-2H-pyran-4-yl)methyl)piperazin-1-yl)-2-(4-isopropyl-5-(8-methoxy-[1,2,4]triazolo[1,5-a]pyridin-6-yl)-1H-pyrazol-3-yl)thiazol (983);
5-((2R,5S)-4-(Cyclobutylmethyl)-2,5-dimethylpiperazin-1-yl)-2-(4-isopropyl-5-(8-methoxy-[1,2,4]triazolo[1,5-a]pyridin-6-yl)-1H-pyrazol-3-yl)thiazol (984);
5-((2R,5S)-4-Isobutyl-2,5-dimethylpiperazin-1-yl)-2-(4-isopropyl-5-(8-methoxy-[1,2,4]triazolo[1,5-a]pyridin-6-yl)-1H-pyrazol-3-yl)thiazol (985);
5-((2R,5S)-4-(Cyclopropylmethyl)-2,5-dimethylpiperazin-1-yl)-2-(4-isopropyl-5-(8-methoxy-[1,2,4]triazolo[1,5-a]pyridin-6-yl)-1H-pyrazol-3-yl)thiazol (986);
5-((2R,5S)-4-Cyclobutyl-2,5-dimethylpiperazin-1-yl)-2-(4-isopropyl-5-(8-methoxy-[1,2,4]triazolo[1,5-a]pyridin-6-yl)-1H-pyrazol-3-yl)thiazol (987);
5-((2R,5S)-2,5-Dimethyl-4-(tetrahydro-2H-pyran-4-yl)piperazin-1-yl)-2-(4-isopropyl-5-(8-methoxy-[1,2,4]triazolo[1,5-a]pyridin-6-yl)-1H-pyrazol-3-yl)thiazol (988);
2-(4-Isopropyl-5-(8-methoxy-[1,2,4]triazolo[1,5-a]pyridin-6-yl)-1H-pyrazol-3-yl)-5-((2R,5S)-2,4,5-trimethylpiperazin-1-yl)thiazol (989);
5-((2R,5S)-4-Ethyl-2,5-dimethylpiperazin-1-yl)-2-(4-isopropyl-5-(8-methoxy-[1,2,4]triazolo[1,5-a]pyridin-6-yl)-1H-pyrazol-3-yl)thiazol (990);
5-((2R,5S)-2,5-Dimethyl-4-propylpiperazin-1-yl)-2-(4-isopropyl-5-(8-methoxy-[1,2,4]triazolo[1,5-a]pyridin-6-yl)-1H-pyrazol-3-yl)thiazol (991);
5-((2R,5S)-4-Isopropyl-2,5-dimethylpiperazin-1-yl)-2-(4-isopropyl-5-(8-methoxy-[1,2,4]triazolo[1,5-a]pyridin-6-yl)-1H-pyrazol-3-yl)thiazol (992);
(R)-2-(4-Isopropyl-5-(8-methoxy-[1,2,4]triazolo[1,5-a]pyridin-6-yl)-1H-pyrazol-3-yl)-4-methyl-5-(2-methyl-4-((tetrahydro-2H-pyran-4-yl)methyl)piperazin-1-yl)thiazol (993);
(R)-2-(4-Isopropyl-5-(8-methoxy-[1,2,4]triazolo[1,5-a]pyridin-6-yl)-1H-pyrazol-3-yl)-4-methyl-5-(2-methyl-4-(tetrahydro-2H-pyran-4-yl)piperazin-1-yl)thiazol (994);
(R)-2-(4-Isopropyl-5-(8-methoxy-[1,2,4]triazolo[1,5-a]pyridin-6-yl)-1H-pyrazol-3-yl)-4-methyl-5-(2-methyl-4-(oxetan-3-yl)piperazin-1-yl)thiazol (995);
(R)-5-(4-Cyclobutyl-2-methylpiperazin-1-yl)-2-(4-isopropyl-5-(8-methoxy-[1,2,4]triazolo[1,5-a]pyridin-6-yl)-1H-pyrazol-3-yl)-4-methylthiazol (996);
(R)-5-(4-(Cyclobutylmethyl)-2-methylpiperazin-1-yl)-2-(4-isopropyl-5-(8-methoxy-[1,2,4]triazolo[1,5-a]pyridin-6-yl)-1H-pyrazol-3-yl)-4-methylthiazol (997);
(R)-5-(4-Isobutyl-2-methylpiperazin-1-yl)-2-(4-isopropyl-5-(8-methoxy-[1,2,4]triazolo[1,5-a]pyridin-6-yl)-1H-pyrazol-3-yl)-4-methylthiazol (998);
(R)-5-(4-(Cyclopropylmethyl)-2-methylpiperazin-1-yl)-2-(4-isopropyl-5-(8-methoxy-[1,2,4]triazolo[1,5-a]pyridin-6-yl)-1H-pyrazol-3-yl)-4-methylthiazol (999);
(R)-5-(2,4-Dimethylpiperazin-1-yl)-2-(4-isopropyl-5-(8-methoxy-[1,2,4]triazolo[1,5-a]pyridin-6-yl)-1H-pyrazol-3-yl)-4-methylthiazol (1000);
(R)-2-(4-Isopropyl-5-(8-methoxy-[1,2,4]triazolo[1,5-a]pyridin-6-yl)-1H-pyrazol-3-yl)-4-methyl-5-(2-methyl-4-propylpiperazin-1-yl)thiazol (1001);
(S)-2-(4-Isopropyl-5-(8-methoxy-[1,2,4]triazolo[1,5-a]pyridin-6-yl)-1H-pyrazol-3-yl)-4-methyl-5-(2-methyl-4-(pentan-3-yl)piperazin-1-yl)thiazol (1002);
(R)-5-(4-Isopropyl-2-methylpiperazin-1-yl)-2-(4-isopropyl-5-(8-methoxy-[1,2,4]triazolo[1,5-a]pyridin-6-yl)-1H-pyrazol-3-yl)-4-methylthiazol (1003);
(R)-2-(4-Isopropyl-5-(8-methoxy-[1,2,4]triazolo[1,5-a]pyridin-6-yl)-1H-pyrazol-3-yl)-5-(2-methyl-4-((tetrahydro-2H-pyran-4-yl)methyl)piperazin-1-yl)thiazol (1004);
(R)-2-(4-Isopropyl-5-(8-methoxy-[1,2,4]triazolo[1,5-a]pyridin-6-yl)-1H-pyrazol-3-yl)-5-(2-methyl-4-(tetrahydro-2H-pyran-4-yl)piperazin-1-yl)thiazol (1005);
(R)-2-(4-Isopropyl-5-(8-methoxy-[1,2,4]triazolo[1,5-a]pyridin-6-yl)-1H-pyrazol-3-yl)-5-(2-methyl-4-(oxetan-3-yl)piperazin-1-yl)thiazol (1006);
(R)-5-(4-Cyclobutyl-2-methylpiperazin-1-yl)-2-(4-isopropyl-5-(8-methoxy-[1,2,4]triazolo[1,5-a]pyridin-6-yl)-1H-pyrazol-3-yl)thiazol (1007);
(R)-5-(4-(Cyclobutylmethyl)-2-methylpiperazin-1-yl)-2-(4-isopropyl-5-(8-methoxy-[1,2,4]triazolo[1,5-a]pyridin-6-yl)-1H-pyrazol-3-yl)thiazol (1008);
(R)-5-(4-(Cyclopropylmethyl)-2-methylpiperazin-1-yl)-2-(4-isopropyl-5-(8-methoxy-[1,2,4]triazolo[1,5-a]pyridin-6-yl)-1H-pyrazol-3-yl)thiazol (1009);
(R)-5-(2,4-Dimethylpiperazin-1-yl)-2-(4-isopropyl-5-(8-methoxy-[1,2,4]triazolo[1,5-a]pyridin-6-yl)-1H-pyrazol-3-yl)thiazol (1010);
(R)-5-(4-Isobutyl-2-methylpiperazin-1-yl)-2-(4-isopropyl-5-(8-methoxy-[1,2,4]triazolo[1,5-a]pyridin-6-yl)-1H-pyrazol-3-yl)thiazol (1011);
(R)-5-(4-Isopropyl-2-methylpiperazin-1-yl)-2-(4-isopropyl-5-(8-methoxy-[1,2,4]triazolo[1,5-a]pyridin-6-yl)-1H-pyrazol-3-yl)thiazol (1012);
(R)-2-(4-Isopropyl-5-(8-methoxy-[1,2,4]triazolo[1,5-a]pyridin-6-yl)-1H-pyrazol-3-yl)-5-(2-methyl-4-(pentan-3-yl)piperazin-1-yl)thiazol (1013);
(R)-2-(4-Isopropyl-5-(8-methoxy-[1,2,4]triazolo[1,5-a]pyridin-6-yl)-1H-pyrazol-3-yl)-5-(2-methyl-4-propylpiperazin-1-yl)thiazol (1014);
2-(Dimethylamino)-1-(4-(2-(4-isopropyl-5-(8-methoxy-[1,2,4]triazolo[1,5-a]pyridin-6-yl)-1H-pyrazol-3-yl)thiazol-5-yl)piperazin-1-yl)ethan-1-on (1015);
1-(4-(2-(4-Isopropyl-5-(8-methoxy-[1,2,4]triazolo[1,5-a]pyridin-6-yl)-1H-pyrazol-3-yl)thiazol-5-yl)piperazin-1-yl)-2-morpholinoethan-1-on (1016);
(S)-2-(Dimethylamino)-1-(4-(2-(4-isopropyl-5-(8-methoxy-[1,2,4]triazolo[1,5-a]pyridin-6-yl)-1H-pyrazol-3-yl)thiazol-5-yl)-3-methylpiperazin-1-yl)ethan-1-on (1017);
(R)-2-(Diethylamino)-1-(4-(2-(4-isopropyl-5-(8-methoxy-[1,2,4]triazolo[1,5-a]pyridin-6-yl)-1H-pyrazol-3-yl)thiazol-5-yl)-3-methylpiperazin-1-yl)ethan-1-on (1018);
2-(Diethylamino)-1-(4-(2-(4-isopropyl-5-(8-methoxy-[1,2,4]triazolo[1,5-a]pyridin-6-yl)-1H-pyrazol-3-yl)-4-methylthiazol-5-yl)piperazin-1-yl)ethan-1-on (1018B)
2-(Dimethylamino)-1-(4-(2-(4-isopropyl-5-(8-methoxy-[1,2,4]triazolo[1,5-a]pyridin-6-yl)-1H-pyrazol-3-yl)-4-methylthiazol-5-yl)piperazin-1-yl)ethan-1-on (1019);
2-(Diethylamino)- N -(1-(2-(4-isopropyl-5-(8-methoxy-[1,2,4]triazolo[1,5-a]pyridin-6-yl)-1H-pyrazol-3-yl)-4-methylthiazol-5-yl)piperidin-4-yl)acetamid (1020);
2-(Dimethylamino)-N-(1-(2-(4-isopropyl-5-(8-methoxy-[1,2,4]triazolo[1,5-a]pyridin-6-yl)-1H-pyrazol-3-yl)-4-methylthiazol-5-yl)piperidin-4-yl)acetamid (1021);
N-(1-(2-(4-Isopropyl-5-(8-methoxy-[1,2,4]triazolo[1,5-a]pyridin-6-yl)-1H-pyrazol-3-yl)-4-methylthiazol-5-yl)piperidin-4-yl)-2-morpholinoacetamid (1022);
2-(Diethylamino)-1-(4-(2-(4-isopropyl-5-(8-methoxy-[1,2,4]triazolo[1,5-a]pyridin-6-yl)-1H-pyrazol-3-yl)thiazol-5-yl)piperazin-1-yl)ethan-1-on (1023);
2-(Diethylamino)-1-((2S,5R)-4-(2-(4-isopropyl-5-(8-methoxy-[1,2,4]triazolo[1,5-a]pyridin-6-yl)-1H-pyrazol-3-yl)thiazol-5-yl)-2,5-dimethylpiperazin-1-yl)ethan-1-on (1024);
2-(Dimethylamino)-1-((2S,5R)-4-(2-(4-isopropyl-5-(8-methoxy-[1,2,4]triazolo[1,5-a]pyridin-6-yl)-1H-pyrazol-3-yl)thiazol-5-yl)-2,5-dimethylpiperazin-1-yl)ethan-1-on (1025);
(R)-2-(Dimethylamino)-1-(4-(2-(4-isopropyl-5-(8-methoxy-[1,2,4]triazolo[1,5-a]pyridin-6-yl)-1H-pyrazol-3-yl)-4-methylthiazol-5-yl)-3-methylpiperazin-1-yl)ethan-1-on (1026);
(R)-2-(Dimethylamino)-1-(4-(2-(4-isopropyl-5-(8-methoxy-[1,2,4]triazolo[1,5-a]pyridin-6-yl)-1H-pyrazol-3-yl)thiazol-5-yl)-3-methylpiperazin-1-yl)ethan-1-on (1027);
2-(4-Isopropyl-5-(8-methoxy-[1,2,4]triazolo[1,5-a]pyridin-6-yl)-1H-pyrazol-3-yl)-5-(4-(2-methoxyethyl)piperazin-1-yl)thiazol (1028);
2-(4-(2-(4-Isopropyl-5-(8-methoxy-[1,2,4]triazolo[1,5-a]pyridin-6-yl)-1H-pyrazol-3-yl)-4-methylthiazol-5-yl)piperazin-1-yl)-N,N-dimethylacetamid (1029);
(S)-2-(4-Isopropyl-5-(8-methoxy-[1,2,4]triazolo[1,5-a]pyridin-6-yl)-1H-pyrazol-3-yl)-5-(4-(2-methoxyethyl)-2-methylpiperazin-1-yl)thiazol (1030);
2-(4-Isopropyl-5-(8-methoxy-[1,2,4]triazolo[1,5-a]pyridin-6-yl)-1H-pyrazol-3-yl)-5-(4-(2-(methylsulfonyl)ethyl)piperazin-1-yl)thiazol (1031);
2-(4-(2-(4-Isopropyl-5-(8-methoxy-[1,2,4]triazolo[1,5-a]pyridin-6-yl)-1H-pyrazol-3-yl)thiazol-5-yl)piperazin-1-yl)-N,N-dimethylacetamid (1032);
(S)-2-(4-Isopropyl-5-(8-methoxy-[1,2,4]triazolo[1,5-a]pyridin-6-yl)-1H-pyrazol-3-yl)-5-(2-methyl-4-(2-(methylsulfonyl)ethyl)piperazin-1-yl)thiazol (1033);
1-(4-(2-(4-Isopropyl-5-(8-methoxy-[1,2,4]triazolo[1,5-a]pyridin-6-yl)-1H-pyrazol-3-yl)thiazol-5-yl)piperazin-1-yl)-2-methylpropan-2-ol (1034);
5-((2R,5S)-2,5-Dimethyl-4-(2-(methylsulfonyl)ethyl)piperazin-1-yl)-2-(4-isopropyl-5-(8-methoxy-[1,2,4]triazolo[1,5-a]pyridin-6-yl)-1H-pyrazol-3-yl)thiazol (1035);
2-(4-Isopropyl-5-(8-methoxy-[1,2,4]triazolo[1,5-a]pyridin-6-yl)-1H-pyrazol-3-yl)-5-((2R,5S)-4-(2-methoxyethyl)-2,5-dimethylpiperazin-1-yl)thiazol (1036);
(R)-2-(4-Isopropyl-5-(8-methoxy-[1,2,4]triazolo[1,5-a]pyridin-6-yl)-1H-pyrazol-3-yl)-4-methyl-5-(2-methyl-4-(2-(methylsulfonyl)ethyl)piperazin-1-yl)thiazol (1037);
(R)-5-(4-(2-Fluorethyl)-2-methylpiperazin-1-yl)-2-(4-isopropyl-5-(8-methoxy-[1,2,4]triazolo[1,5-a]pyridin-6-yl)-1H-pyrazol-3-yl)-4-methylthiazol (1038);
(R)-5-(4-(3-Fluorpropyl)-2-methylpiperazin-1-yl)-2-(4-isopropyl-5-(8-methoxy-[1,2,4]triazolo[1,5-a]pyridin-6-yl)-1H-pyrazol-3-yl)-4-methylthiazol (1039);
(R)-2-(4-Isopropyl-5-(8-methoxy-[1,2,4]triazolo[1,5-a]pyridin-6-yl)-1H-pyrazol-3-yl)-5-(4-(2-methoxyethyl)-2-methylpiperazin-1-yl)-4-methylthiazol (1040);
(R)-2-(4-(2-(4-Isopropyl-5-(8-methoxy-[1,2,4]triazolo[1,5-a]pyridin-6-yl)-1H-pyrazol-3-yl)-4-methylthiazol-5-yl)-3-methylpiperazin-1-yl)-N,N-dimethylacetamid (1041);
(R)-2-(4-Isopropyl-5-(8-methoxy-[1,2,4]triazolo[1,5-a]pyridin-6-yl)-1H-pyrazol-3-yl)-5-(2-methyl-4-(2-(methylsulfonyl)ethyl)piperazin-1-yl)thiazol (1042);
(R)-2-(4-Isopropyl-5-(8-methoxy-[1,2,4]triazolo[1,5-a]pyridin-6-yl)-1H-pyrazol-3-yl)-5-(4-(2-methoxyethyl)-2-methylpiperazin-1-yl)thiazol (1043);
(R)-2-(4-(2-(4-Isopropyl-5-(8-methoxy-[1,2,4]triazolo[1,5-a]pyridin-6-yl)-1H-pyrazol-3-yl)thiazol-5-yl)-3-methylpiperazin-1-yl)-N,N-dimethylacetamid (1044);
(R)-5-(4-(2-Fluorethyl)-2-methylpiperazin-1-yl)-2-(4-isopropyl-5-(8-methoxy-[1,2,4]triazolo[1,5-a]pyridin-6-yl)-1H-pyrazol-3-yl)thiazol (1045);
(R)-5-(4-(3-Fluorpropyl)-2-methylpiperazin-1-yl)-2-(4-isopropyl-5-(8-methoxy-[1,2,4]triazolo[1,5-a]pyridin-6-yl)-1H-pyrazol-3-yl)thiazol (1046);
(R)-2-(4-(2-(4-Isopropyl-5-(8-methoxy-[1,2,4]triazolo[1,5-a]pyridin-6-yl)-1H-pyrazol-3-yl)thiazol-5-yl)-3-methylpiperazin-1-yl)-N-methylacetamid (1047);
(R)-2-(4-(2-(4-Isopropyl-5-(8-methoxy-[1,2,4]triazolo[1,5-a]pyridin-6-yl)-1H-pyrazol-3-yl)thiazol-5-yl)-3-methylpiperazin-1-yl)acetonitril (1048);
2-(4-Isopropyl-5-(8-methoxy-[1,2,4]triazolo[1,5-a]pyridin-6-yl)-1H-pyrazol-3-yl)-4-methyl-5-(6-propyl-2,6-diazaspiro[3.3]heptan-2-yl)thiazol (1049);
2-(4-Isopropyl-5-(8-methoxy-[1,2,4]triazolo[1,5-a]pyridin-6-yl)-1H-pyrazol-3-yl)-4-methyl-5-(6-methyl-2,6-diazaspiro[3.3]heptan-2-yl)thiazol (1050);
5-(6-Ethyl-2,6-diazaspiro[3.3]heptan-2-yl)-2-(4-isopropyl-5-(8-methoxy-[1,2,4]triazolo[1,5-a]pyridin-6-yl)-1H-pyrazol-3-yl)-4-methylthiazol (1051);
2-(4-Isopropyl-5-(8-methoxy-[1,2,4]triazolo[1,5-a]pyridin-6-yl)-1H-pyrazol-3-yl)-5-(6-isopropyl-2,6-diazaspiro[3.3]heptan-2-yl)-4-methylthiazol (1052);
5-(6-(Cyclopropylmethyl)-2,6-diazaspiro[3.3]heptan-2-yl)-2-(4-isopropyl-5-(8-methoxy-[1,2,4]triazolo[1,5-a]pyridin-6-yl)-1H-pyrazol-3-yl)-4-methylthiazol (1053);
5-(6-Isobutyl-2,6-diazaspiro[3.3]heptan-2-yl)-2-(4-isopropyl-5-(8-methoxy-[1,2,4]triazolo[1,5-a]pyridin-6-yl)-1H-pyrazol-3-yl)-4-methylthiazol (1054);
5-(6-(2-Ethylbutyl)-2,6-diazaspiro[3.3]heptan-2-yl)-2-(4-isopropyl-5-(8-methoxy-[1,2,4]triazolo[1,5-a]pyridin-6-yl)-1H-pyrazol-3-yl)-4-methylthiazol (1055);
5-(6-Cyclobutyl-2,6-diazaspiro[3.3]heptan-2-yl)-2-(4-isopropyl-5-(8-methoxy-[1,2,4]triazolo[1,5-a]pyridin-6-yl)-1H-pyrazol-3-yl)-4-methylthiazol (1056);
5-(6-(Cyclobutylmethyl)-2,6-diazaspiro[3.3]heptan-2-yl)-2-(4-isopropyl-5-(8-methoxy-[1,2,4]triazolo[1,5-a]pyridin-6-yl)-1H-pyrazol-3-yl)-4-methylthiazol (1057);
2-(4-Isopropyl-5-(8-methoxy-[1,2,4]triazolo[1,5-a]pyridin-6-yl)-1H-pyrazol-3-yl)-4-methyl-5-(6-(tetrahydro-2H-pyran-4-yl)-2,6-diazaspiro[3.3]heptan-2-yl)thiazol (1058);
2-(4-Isopropyl-5-(8-methoxy-[1,2,4]triazolo[1,5-a]pyridin-6-yl)-1H-pyrazol-3-yl)-4-methyl-5-(6-((tetrahydro-2H-pyran-4-yl)methyl)-2,6-diazaspiro[3.3]heptan-2-yl)thiazol (1059);
2-(Dimethylamino)-1-(6-(2-(4-isopropyl-5-(8-methoxy-[1,2,4]triazolo[1,5-a]pyridin-6-yl)-1H-pyrazol-3-yl)-4-methylthiazol-5-yl)-2,6-diazaspiro[3.3]heptan-2-yl)ethan-1-on (1060);
2-(4-Isopropyl-5-(8-methoxy-[1,2,4]triazolo[1,5-a]pyridin-6-yl)-1H-pyrazol-3-yl)-4-methyl-5-(6-(2-(methylsulfonyl)ethyl)-2,6-diazaspiro[3.3]heptan-2-yl)thiazol (1061);
2-(4-Isopropyl-5-(8-methoxy-[1,2,4]triazolo[1,5-a]pyridin-6-yl)-1H-pyrazol-3-yl)-5-(6-(2-methoxyethyl)-2,6-diazaspiro[3.3]heptan-2-yl)-4-methylthiazol (1062);
5-(4-Isopropyl-5-(8-methoxy-[1,2,4]triazolo[1,5-a]pyridin-6-yl)-1H-pyrazol-3-yl)-2-(1-isopropylpiperidin-4-yl)thiazol (1063);
5-(4-Isopropyl-5-(8-methyl-[1,2,4]triazolo[1,5-a]pyridin-6-yl)-1H-pyrazol-3-yl)-2-(piperidin-4-yl)thiazol (1064);
5-(4-Isopropyl-5-(8-methoxy-[1,2,4]triazolo[1,5-a]pyridin-6-yl)-1H-pyrazol-3-yl)-2-(1-methylpiperidin-4-yl)thiazol (1065);
2-(1-Ethylpiperidin-4-yl)-5-(4-isopropyl-5-(8-methoxy-[1,2,4]triazolo[1,5-a]pyridin-6-yl)-1H-pyrazol-3-yl)thiazol (1066);
5-(4-Isopropyl-5-(8-methoxy-[1,2,4]triazolo[1,5-a]pyridin-6-yl)-1H-pyrazol-3-yl)-2-(1-propylpiperidin-4-yl)thiazol (1067);
5-(4-Isopropyl-5-(8-methyl-[1,2,4]triazolo[1,5-a]pyridin-6-yl)-1H-pyrazol-3-yl)-2-(1-propylpiperidin-4-yl)thiazol (1068);
5-(4-Isopropyl-5-(8-methyl-[1,2,4]triazolo[1,5-a]pyridin-6-yl)-1H-pyrazol-3-yl)-2-(1-(tetrahydro-2H-pyran-4-yl)piperidin-4-yl)thiazol (1069);
5-(4-Isopropyl-5-(8-methyl-[1,2,4]triazolo[1,5-a]pyridin-6-yl)-1H-pyrazol-3-yl)-2-(1-methylpiperidin-4-yl)thiazol (1070);
2-(1-Ethylpiperidin-4-yl)-5-(4-isopropyl-5-(8-methyl-[1,2,4]triazolo[1,5-a]pyridin-6-yl)-1H-pyrazol-3-yl)thiazol (1071);
5-(4-Isopropyl-5-(8-methyl-[1,2,4]triazolo[1,5-a]pyridin-6-yl)-1H-pyrazol-3-yl)-2-(1-isopropylpiperidin-4-yl)thiazol (1072);
5-(4-Isopropyl-5-(8-methyl-[1,2,4]triazolo[1,5-a]pyridin-6-yl)-1H-pyrazol-3-yl)-2-(1-(oxetan-3-yl)piperidin-4-yl)thiazol (1073);
5-(4-Isopropyl-5-(8-methyl-[1,2,4]triazolo[1,5-a]pyridin-6-yl)-1H-pyrazol-3-yl)-2-(1-((3-methyloxetan-3-yl)methyl)piperidin-4-yl)thiazol (1074);
5-(4-Isopropyl-5-(8-methyl-[1,2,4]triazolo[1,5-a]pyridin-6-yl)-1H-pyrazol-3-yl)-2-(1-neopentylpiperidin-4-yl)thiazol (1075);
2-(4-(5-(4-Isopropyl-5-(8-methyl-[1,2,4]triazolo[1,5-a]pyridin-6-yl)-1H-pyrazol-3-yl) Thiazol-2-yl)piperidin-1-yl)-N,N-dimethylacetamid (1076);
2-(4-(5-(4-Isopropyl-5-(8-methoxy-[1,2,4]triazolo[1,5-a]pyridin-6-yl)-1H-pyrazol-3-yl)thiazol-2-yl)piperidin-1-yl)acetamid (1077);
2-(4-(5-(4-Isopropyl-5-(8-methyl-[1,2,4]triazolo[1,5-a]pyridin-6-yl)-1H-pyrazol-3-yl) Thiazol-2-yl)piperidin-1-yl)acetonitril (1078);
1-(4-(5-(4-Isopropyl-5-(8-methyl-[1,2,4]triazolo[1,5-a]pyridin-6-yl)-1H-pyrazol-3-yl) Thiazol-2-yl)piperidin-1-yl)-2-methylpropan-2-ol (1079);
5-(4-Isopropyl-5-(8-methyl-[1,2,4]triazolo[1,5-a]pyridin-6-yl)-1H-pyrazol-3-yl)-2-(1-(2-(methylsulfonyl)ethyl)piperidin-4-yl)thiazol (1080);
5-(4-Isopropyl-5-(8-methyl-[1,2,4]triazolo[1,5-a]pyridin-6-yl)-1H-pyrazol-3-yl)-2-(1-(2-methoxyethyl)piperidin-4-yl)thiazol (1081);
2-(Dimethylamino)-1-(4-(5-(4-isopropyl-5-(8-methyl-[1,2,4]triazolo[1,5-a]pyridin-6-yl)-1H-pyrazol-3-yl)thiazol-2-yl)piperidin-1-yl)ethan-1-on (1082);
4-(5-(4-Isopropyl-5-(8-methyl-[1,2,4]triazolo[1,5-a]pyridin-6-yl)-1H-pyrazol-3-yl) Thiazol-2-yl)-N-methylcyclohexan-1-amin (1083);
4-(5-(4-Isopropyl-5-(8-methyl-[1,2,4]triazolo[1,5-a]pyridin-6-yl)-1H-pyrazol-3-yl) Thiazol-2-yl)-N,N-dimethylcyclohexan-1-amin (1084-1085);
4-(5-(4-Isopropyl-5-(8-methyl-[1,2,4]triazolo[1,5-a]pyridin-6-yl)-1H-pyrazol-3-yl) Thiazol-2-yl)-N,N-dimethylcyclohexan-1-amin (1085);
N-(4-(5-(4-Isopropyl-5-(8-methyl-[1,2,4]triazolo[1,5-a]pyridin-6-yl)-1H-pyrazol-3-yl)thiazol-2-yl)cyclohexyl)-N-methyloxetan-3-amin (1086-1087);
N-Isopropyl-4-(5-(4-isopropyl-5-(8-methyl-[1,2,4]triazolo[1,5-a]pyridin-6-yl)-1H-pyrazol-3-yl)thiazol-2-yl)-N-methylcyclohexan-1-amin (1088-1089);
6-(4-Isopropyl-1'-(1-methylpiperidin-4-yl)-1H, 1'H-[3,4'-bipyrazol]-5-yl)-8-methyl-[1,2,4]triazolo[1,5-a]pyridin (1090);
6-(4-Isopropyl-1'-(1-Isopropylpiperidin-4-yl)-1H, 1'H-[3,4'-bipyrazol]-5-yl)-8-methyl-[1,2,4]triazolo[1,5-a]pyridin (1091);
6-(4-Isopropyl-1'-(1-(oxetan-3-yl)piperidin-4-yl)-1H, 1'H-[3,4'-bipyrazol]-5-yl)-8-methyl-[1,2,4]triazolo[1,5-a]pyridin (1092);
6-(4-Isopropyl-3-(4-methyl-5-(1-propylpiperidin-4-yl)pyridin-2-yl)-1H-pyrazol-5-yl)-8-methoxy-[1,2,4]triazolo[1,5-a]pyridin (1093);
6-(4-Isopropyl-3-(3-methyl-5-(piperidin-4-yl)pyridin-2-yl)-1H-pyrazol-5-yl)-8-methoxy-[1,2,4]triazolo[1,5-a]pyridin (1094);
6-(3-(3-Fluor-5-(piperidin-4-yl)pyridin-2-yl)-4-isopropyl-1H-pyrazol-5-yl)-8-methoxy-[1,2,4]triazolo[1,5-a]pyridin (1095);
6-(3-(6-Fluor-5-(piperidin-4-yl)pyridin-2-yl)-4-isopropyl-1H-pyrazol-5-yl)-8-methoxy-[1,2,4]triazolo[1,5-a]pyridin (1096);
6-(4-Isopropyl-3-(4-methyl-5-(1-methylpiperidin-4-yl)pyridin-2-yl)-1H-pyrazol-5-yl)-8-methoxy-[1,2,4]triazolo[1,5-a]pyridin (1097);
6-(4-Isopropyl-3-(5-(1-isopropylpiperidin-4-yl)-4-methylpyridin-2-yl)-1H-pyrazol-5-yl)-8-methoxy-[1,2,4]triazolo[1,5-a]pyridin (1098);
6-(3-(6-Fluor-5-(1-methylpiperidin-4-yl)pyridin-2-yl)-4-isopropyl-1H-pyrazol-5-yl)-8-methoxy-[1,2,4]triazolo[1,5-a]pyridin (1099);
6-(3-(6-Fluor-5-(1-propylpiperidin-4-yl) pyridin-2-yl)-4-isopropyl-1H-pyrazol-5-yl)-8-methoxy-[1,2,4]triazolo[1,5-a]pyridin (1100);
6-(3-(6-Fluor-5-(1-(oxetan-3-yl)piperidin-4-yl)pyridin-2-yl)-4-isopropyl-1H-pyrazol-5-yl)-8-methoxy-[1,2,4]triazolo[1,5-a]pyridin (1101);
6-(3-(5-(1-Ethylpiperidin-4-yl)-4-methylpyridin-2-yl)-4-isopropyl-1H-pyrazol-5-yl)-8-methoxy-[1,2,4]triazolo[1,5-a]pyridin (1102);
6-(3-(5-(1-Isobutylpiperidin-4-yl)-4-methylpyridin-2-yl)-4-isopropyl-1H-pyrazol-5-yl)-8-methoxy-[1,2,4]triazolo[1,5-a]pyridin (1103);
6-(3-(5-(1-Cyclobutylpiperidin-4-yl)-4-methylpyridin-2-yl)-4-isopropyl-1H-pyrazol-5-yl)-8-methoxy-[1,2,4]triazolo[1,5-a]pyridin (1104);
6-(3-(5-(1-(Cyclobutylmethyl)piperidin-4-yl)-4-methylpyridin-2-yl)-4-isopropyl-1H-pyrazol-5-yl)-8-methoxy-[1,2,4]triazolo[1,5-a]pyridin (1105);
6-(4-Isopropyl-3-(4-methyl-5-(1-(oxetan-3-yl)piperidin-4-yl)pyridin-2-yl)-1H-pyrazol-5-yl)-8-methoxy-[1,2,4]triazolo[1,5-a]pyridin (1106);
6-(4-Isopropyl-3-(4-methyl-5-(1-((tetrahydro-2H-pyran-4-yl)methyl)piperidin-4-yl) pyridin-2-yl)-1H-pyrazol-5-yl)-8-methoxy-[1,2,4]triazolo[1,5-a]pyridin (1107);
6-(4-Isopropyl-3-(4-methyl-5-(1-(tetrahydrofuran-3-yl)piperidin-4-yl)pyridin-2-yl)-1H-pyrazol-5-yl)-8-methoxy-[1,2,4]triazolo[1,5-a]pyridin (1108);
6-(4-Isopropyl-3-(4-methyl-5-(1-((tetrahydrofuran-3-yl)methyl)piperidin-4-yl)pyridin-2-yl)-1H-pyrazol-5-yl)-8-methoxy-[1,2,4]triazolo[1,5-a]pyridin (1109);
6-(3-(5-(1-(Cyclobutylmethyl)piperidin-4-yl)-3-fluorpyridin-2-yl)-4-isopropyl-1H-pyrazol-5-yl)-8-methoxy-[1,2,4]triazolo[1,5-a]pyridin (1110);
6-(3-(3-Fluor-5-(1-methylpiperidin-4-yl)pyridin-2-yl)-4-isopropyl-1H-pyrazol-5-yl)-8-methoxy-[1,2,4]triazolo[1,5-a]pyridin (1111);
6-(3-(5-(1-Ethylpiperidin-4-yl)-3-fluorpyridin-2-yl)-4-isopropyl-1H-pyrazol-5-yl)-8-methoxy-[1,2,4]triazolo[1,5-a]pyridin (1112);
6-(3-(3-Fluor-5-(1-propylpiperidin-4-yl) pyridin-2-yl)-4-isopropyl-1H-pyrazol-5-yl)-8-methoxy-[1,2,4]triazolo[1,5-a]pyridin (1113);
6-(3-(3-Fluor-5-(1-isopropylpiperidin-4-yl) pyridin-2-yl)-4-isopropyl-1H-pyrazol-5-yl)-8-methoxy-[1,2,4]triazolo[1,5-a]pyridin (1114);
6-(3-(5-(1-(Cyclopropylmethyl)piperidin-4-yl)-3-fluorpyridin-2-yl)-4-isopropyl-1H-pyrazol-5-yl)-8-methoxy-[1,2,4]triazolo[1,5-a]pyridin (1115);
6-(3-(3-Fluor-5-(1-isobutylpiperidin-4-yl) pyridin-2-yl)-4-isopropyl-1H-pyrazol-5-yl)-8-methoxy-[1,2,4]triazolo[1,5-a]pyridin (1116);
6-(3-(3-Fluor-5-(1-(oxetan-3-yl)piperidin-4-yl)pyridin-2-yl)-4-isopropyl-1H-pyrazol-5-yl)-8-methoxy-[1,2,4]triazolo[1,5-a]pyridin (1117);
6-(3-(3-Fluor-5-(1-(tetrahydro-2H-pyran-4-yl)piperidin-4-yl)pyridin-2-yl)-4-isopropyl-1H-pyrazol-5-yl)-8-methoxy-[1,2,4]triazolo[1,5-a]pyridin (1118);
6-(3-(3-Fluor-5-(1-((tetrahydro-2H-pyran-4-yl)methyl)piperidin-4-yl)pyridin-2-yl)-4-isopropyl-1H-pyrazol-5-yl)-8-methoxy-[1,2,4]triazolo[1,5-a]pyridin (1119);
6-(3-(3-Fluor-5-(1-(pentan-3-yl)piperidin-4-yl)pyridin-2-yl)-4-isopropyl-1H-pyrazol-5-yl)-8-methoxy-[1,2,4]triazolo[1,5-a]pyridin (1120);
6-(3-(5-(1-(2-Ethylbutyl)piperidin-4-yl)-3-fluorpyridin-2-yl)-4-isopropyl-1H-pyrazol-5-yl)-8-methoxy-[1,2,4]triazolo[1,5-a]pyridin (1121);
6-(3-(3-Fluor-5-(1-neopentylpiperidin-4-yl)pyridin-2-yl)-4-isopropyl-1H-pyrazol-5-yl)-8-methoxy-[1,2,4]triazolo[1,5-a]pyridin (1123);
6-(4-Isopropyl-3-(3-methyl-5-(1-methylpiperidin-4-yl)pyridin-2-yl)-1H-pyrazol-5-yl)-8-methoxy-[1,2,4]triazolo[1,5-a]pyridin (1124);
6-(3-(5-(1-Ethylpiperidin-4-yl)-3-methylpyridin-2-yl)-4-isopropyl-1H-pyrazol-5-yl)-8-methoxy-[1,2,4]triazolo[1,5-a]pyridin (1125);
6-(4-Isopropyl-3-(3-methyl-5-(1-propylpiperidin-4-yl)pyridin-2-yl)-1H-pyrazol-5-yl)-8-methoxy-[1,2,4]triazolo[1,5-a]pyridin (1126);
6-(4-Isopropyl-3-(5-(1-isopropylpiperidin-4-yl)-3-methylpyridin-2-yl)-1H-pyrazol-5-yl)-8-methoxy-[1,2,4]triazolo[1,5-a]pyridin (1127);
6-(3-(5-(1-(Cyclopropylmethyl)piperidin-4-yl)-3-methylpyridin-2-yl)-4-isopropyl-1H-pyrazol-5-yl)-8-methoxy-[1,2,4]triazolo[1,5-a]pyridin (1128);
6-(4-Isopropyl-3-(3-methyl-5-(1-((tetrahydro-2H-pyran-4-yl)methyl)piperidin-4-yl) pyridin-2-yl)-1H-pyrazol-5-yl)-8-methoxy-[1,2,4]triazolo[1,5-a]pyridin (1129);
6-(3-(5-(1-Ethylpiperidin-4-yl)-6-fluorpyridin-2-yl)-4-isopropyl-1H-pyrazol-5-yl)-8-methoxy-[1,2,4]triazolo[1,5-a]pyridin (1130);
6-(3-(6-Fluor-5-(1-isopropylpiperidin-4-yl) pyridin-2-yl)-4-isopropyl-1H-pyrazol-5-yl)-8-methoxy-[1,2,4]triazolo[1,5-a]pyridin (1131);
6-(3-(5-(1-Cyclobutylpiperidin-4-yl)-6-fluorpyridin-2-yl)-4-isopropyl-1H-pyrazol-5-yl)-8-methoxy-[1,2,4]triazolo[1,5-a]pyridin (1132);
6-(3-(6-Fluor-5-(1-((tetrahydro-2H-pyran-4-yl)methyl)piperidin-4-yl)pyridin-2-yl)-4-isopropyl-1H-pyrazol-5-yl)-8-methoxy-[1,2,4]triazolo[1,5-a]pyridin (1133);
2-(Dimethylamino)-1-(4-(6-(4-isopropyl-5-(8-methoxy-[1,2,4]triazolo[1,5-a]pyridin-6-yl)-1H-pyrazol-3-yl)-4-methylpyridin-3-yl)piperidin-1-yl)ethan-1-on (1134);
2-(Dimethylamino)-1-(4-(5-fluor-6-(4-isopropyl-5-(8-methoxy-[1,2,4]triazolo[1,5-a] Pyridin-6-yl)-1H-pyrazol-3-yl)pyridin-3-yl)piperidin-1-yl)ethan-1-on (1135);
2-(4-(6-(4-Isopropyl-5-(8-methoxy-[1,2,4]triazolo[1,5-a]pyridin-6-yl)-1H-pyrazol-3-yl)-4-methylpyridin-3-yl)piperidin-1-yl)-N,N-dimethylacetamid (1136);
2-(4-(5-Fluor-6-(4-isopropyl-5-(8-methoxy-[1,2,4]triazolo[1,5-a]pyridin-6-yl)-1H-pyrazol-3-yl)pyridin-3-yl)piperidin-1-yl)-N,N-dimethylacetamid (1137);
6-(3-(3-Fluor-5-(1-(2-methoxyethyl)piperidin-4-yl)pyridin-2-yl)-4-isopropyl-1H-pyrazol-5-yl)-8-methoxy-[1,2,4]triazolo[1,5-a]pyridin (1138);
6-(3-(3-Fluor-5-(1-(2-(methylsulfonyl)ethyl)piperidin-4-yl)pyridin-2-yl)-4-isopropyl-1H-pyrazol-5-yl)-8-methoxy-[1,2,4]triazolo[1,5-a]pyridin (1139);
6-(4-Isopropyl-3-(4-methyl-5-(1-(2-(methylsulfonyl)ethyl)piperidin-4-yl)pyridin-2-yl)-1H-pyrazol-5-yl)-8-methoxy-[1,2,4]triazolo[1,5-a]pyridin (1140);
2-(5-(8-Methoxy-[1,2,4]triazolo[1,5-a]pyridin-6-yl)-4-(2,2,2-trifluorethyl)-1H-pyrazol-3-yl)-4-methyl-5-(1-methylpiperidin-4-yl)thiazol (1141);
2-(5-(8-Methoxy-[1,2,4]triazolo[1,5-a]pyridin-6-yl)-4-(2,2,2-trifluorethyl)-1H-pyrazol-3-yl)-5-(piperidin-4-yl)thiazol (1142);
2-(5-(8-Methyl-[1,2,4]triazolo[1,5-a]pyridin-6-yl)-4-(2,2,2-trifluorethyl)-1H-pyrazol-3-yl)-5-(piperidin-4-yl)thiazol (1143);
4-Methyl-2-(5-(8-methyl-[1,2,4]triazolo[1,5-a]pyridin-6-yl)-4-(2,2,2-trifluorethyl)-1H-pyrazol-3-yl)-5-(piperidin-4-yl)thiazol (1144);
2-(5-(7,8-Dimethyl-[1,2,4]triazolo[1,5-a]pyridin-6-yl)-4-(2,2,2-trifluorethyl)-1H-pyrazol-3-yl)-4-methyl-5-(piperidin-4-yl)thiazol (1145);
2-(5-(8-Methoxy-[1,2,4]triazolo[1,5-a]pyridin-6-yl)-4-(2,2,2-trifluorethyl)-1H-pyrazol-3-yl)-4-methyl-5-(1-propylpiperidin-4-yl)thiazol (1146);
5-(1-Ethylpiperidin-4-yl)-2-(5-(8-methoxy-[1,2,4]triazolo[1,5-a]pyridin-6-yl)-4-(2,2,2-trifluorethyl)-1H-pyrazol-3-yl)-4-methylthiazol (1147);
5-(1-Isopropylpiperidin-4-yl)-2-(5-(8-methoxy-[1,2,4]triazolo[1,5-a]pyridin-6-yl)-4-(2,2,2-trifluorethyl)-1H-pyrazol-3-yl)-4-methylthiazol (1148);
5-(1-(Cyclopropylmethyl)piperidin-4-yl)-2-(5-(8-methoxy-[1,2,4]triazolo[1,5-a]pyridin-6-yl)-4-(2,2,2-trifluorethyl)-1H-pyrazol-3-yl)-4-methylthiazol (1149);
5-(1-Isobutylpiperidin-4-yl)-2-(5-(8-methoxy-[1,2,4]triazolo[1,5-a]pyridin-6-yl)-4-(2,2,2-trifluorethyl)-1H-pyrazol-3-yl)-4-methylthiazol (1150);
2-(5-(8-Methoxy-[1,2,4]triazolo[1,5-a]pyridin-6-yl)-4-(2,2,2-trifluorethyl)-1H-pyrazol-3-yl)-4-methyl-5-(1-((tetrahydro-2H-pyran-4-yl)methyl)piperidin-4-yl)thiazol (1151);
2-(5-(8-Methoxy-[1,2,4]triazolo[1,5-a]pyridin-6-yl)-4-(2,2,2-trifluorethyl)-1H-pyrazol-3-yl)-4-methyl-5-(1-(tetrahydro-2H-pyran-4-yl)piperidin-4-yl)thiazol (1152);
2-(5-(8-Methoxy-[1,2,4]triazolo[1,5-a]pyridin-6-yl)-4-(2,2,2-trifluorethyl)-1H-pyrazol-3-yl)-4-methyl-5-(1-(oxetan-3-yl)piperidin-4-yl)thiazol (1153);
2-(5-(8-Methoxy-[1,2,4]triazolo[1,5-a]pyridin-6-yl)-4-(2,2,2-trifluorethyl)-1H-pyrazol-3-yl)-5-(1-methylpiperidin-4-yl)thiazol (1154);
5-(1-Ethylpiperidin-4-yl)-2-(5-(8-methoxy-[1,2,4]triazolo[1,5-a]pyridin-6-yl)-4-(2,2,2-trifluorethyl)-1H-pyrazol-3-yl)thiazol (1155);
2-(5-(8-Methoxy-[1,2,4]triazolo[1,5-a]pyridin-6-yl)-4-(2,2,2-trifluorethyl)-1H-pyrazol-3-yl)-5-(1-propylpiperidin-4-yl)thiazol (1156);
5-(1-Isopropylpiperidin-4-yl)-2-(5-(8-methoxy-[1,2,4]triazolo[1,5-a]pyridin-6-yl)-4-(2,2,2-trifluorethyl)-1H-pyrazol-3-yl)thiazol (1157);
2-(5-(8-Methoxy-[1,2,4]triazolo[1,5-a]pyridin-6-yl)-4-(2,2,2-trifluorethyl)-1H-pyrazol-3-yl)-5-(1-(tetrahydro-2H-pyran-4-yl)piperidin-4-yl)thiazol (1158);
5-(1-(Cyclopropylmethyl)piperidin-4-yl)-2-(5-(8-methoxy-[1,2,4]triazolo[1,5-a]pyridin-6-yl)-4-(2,2,2-trifluorethyl)-1H-pyrazol-3-yl)thiazol (1159);
2-(5-(8-Methoxy-[1,2,4]triazolo[1,5-a]pyridin-6-yl)-4-(2,2,2-trifluorethyl)-1H-pyrazol-3-yl)-5-(1-(oxetan-3-yl)piperidin-4-yl)thiazol (1160);
2-(5-(8-Methoxy-[1,2,4]triazolo[1,5-a]pyridin-6-yl)-4-(2,2,2-trifluorethyl)-1H-pyrazol-3-yl)-5-(1-((tetrahydro-2H-pyran-4-yl)methyl)piperidin-4-yl)thiazol (1161);
5-(1-Isobutylpiperidin-4-yl)-2-(5-(8-methyl-[1,2,4]triazolo[1,5-a]pyridin-6-yl)-4-(2,2,2-trifluorethyl)-1H-pyrazol-3-yl)thiazol (1162);
2-(5-(8-Methyl-[1,2,4]triazolo[1,5-a]pyridin-6-yl)-4-(2,2,2-trifluorethyl)-1H-pyrazol-3-yl)-5-(1-(tetrahydro-2H-pyran-4-yl)piperidin-4-yl)thiazol (1163);
5-(1-Ethylpiperidin-4-yl)-2-(5-(8-methyl-[1,2,4]triazolo[1,5-a]pyridin-6-yl)-4-(2,2,2-trifluorethyl)-1H-pyrazol-3-yl)thiazol (1164);
5-(1-Isopropylpiperidin-4-yl)-2-(5-(8-methyl-[1,2,4]triazolo[1,5-a]pyridin-6-yl)-4-(2,2,2-trifluorethyl)-1H-pyrazol-3-yl)thiazol (1165);
2-(5-(8-Methyl-[1,2,4]triazolo[1,5-a]pyridin-6-yl)-4-(2,2,2-trifluorethyl)-1H-pyrazol-3-yl)-5-(1-((tetrahydro-2H-pyran-4-yl)methyl)piperidin-4-yl)thiazol (1166);
2-(5-(8-Methyl-[1,2,4]triazolo[1,5-a]pyridin-6-yl)-4-(2,2,2-trifluorethyl)-1H-pyrazol-3-yl)-5-(1-(oxetan-3-yl)piperidin-4-yl)thiazol (1167);
2-(5-(8-Methyl-[1,2,4]triazolo[1,5-a]pyridin-6-yl)-4-(2,2,2-trifluorethyl)-1H-pyrazol-3-yl)-5-(1-propylpiperidin-4-yl)thiazol (1168);
2-(5-(8-Methyl-[1,2,4]triazolo[1,5-a]pyridin-6-yl)-4-(2,2,2-trifluorethyl)-1H-pyrazol-3-yl)-5-(1-(3,3,3-trifluorpropyl)piperidin-4-yl)thiazol (1169);
5-(1-(Cyclopropylmethyl)piperidin-4-yl)-2-(5-(8-methyl-[1,2,4]triazolo[1,5-a]pyridin-6-yl)-4-(2,2,2-trifluorethyl)-1H-pyrazol-3-yl)thiazol (1170);
5-(1-(Cyclobutylmethyl)piperidin-4-yl)-2-(5-(8-methyl-[1,2,4]triazolo[1,5-a]pyridin-6-yl)-4-(2,2,2-trifluorethyl)-1H-pyrazol-3-yl)thiazol (1171);
2-(5-(8-Methyl-[1,2,4]triazolo[1,5-a]pyridin-6-yl)-4-(2,2,2-trifluorethyl)-1H-pyrazol-3-yl)-5-(1-methylpiperidin-4-yl)thiazol (1172);
2-(5-(8-Methyl-[1,2,4]triazolo[1,5-a]pyridin-6-yl)-4-(2,2,2-trifluorethyl)-1H-pyrazol-3-yl)-5-(1-neopentylpiperidin-4-yl)thiazol (1173);
5-(1-Ethylpiperidin-4-yl)-4-methyl-2-(5-(8-methyl-[1,2,4]triazolo[1,5-a]pyridin-6-yl)-4-(2,2,2-trifluorethyl)-1H-pyrazol-3-yl)thiazol (1174);
4-Methyl-2-(5-(8-methyl-[1,2,4]triazolo[1,5-a]pyridin-6-yl)-4-(2,2,2-trifluorethyl)-1H-pyrazol-3-yl)-5-(1-(3,3,3-trifluorpropyl)piperidin-4-yl)thiazol (1175);
5-(1-Isopropylpiperidin-4-yl)-4-methyl-2-(5-(8-methyl-[1,2,4]triazolo[1,5-a]pyridin-6-yl)-4-(2,2,2-trifluorethyl)-1H-pyrazol-3-yl)thiazol (1176);
5-(1-(Cyclohexylmethyl)piperidin-4-yl)-4-methyl-2-(5-(8-methyl-[1,2,4]triazolo[1,5-a]pyridin-6-yl)-4-(2,2,2-trifluorethyl)-1H-pyrazol-3-yl)thiazol (1177);
5-(1-(Cyclopropylmethyl)piperidin-4-yl)-4-methyl-2-(5-(8-methyl-[1,2,4]triazolo[1,5-a]pyridin-6-yl)-4-(2,2,2-trifluorethyl)-1H-pyrazol-3-yl)thiazol (1179);
4-Methyl-2-(5-(8-methyl-[1,2,4]triazolo[1,5-a]pyridin-6-yl)-4-(2,2,2-trifluorethyl)-1H-pyrazol-3-yl)-5-(1-(oxetan-3-yl)piperidin-4-yl)thiazol (1180);
5-(1-Isobutylpiperidin-4-yl)-4-methyl-2-(5-(8-methyl-[1,2,4]triazolo[1,5-a]pyridin-6-yl)-4-(2,2,2-trifluorethyl)-1H-pyrazol-3-yl)thiazol (1181);
4-Methyl-2-(5-(8-methyl-[1,2,4]triazolo[1,5-a]pyridin-6-yl)-4-(2,2,2-trifluorethyl)-1H-pyrazol-3-yl)-5-(1-methylpiperidin-4-yl)thiazol (1182);
4-Methyl-2-(5-(8-methyl-[1,2,4]triazolo[1,5-a]pyridin-6-yl)-4-(2,2,2-trifluorethyl)-1H-pyrazol-3-yl)-5-(1-propylpiperidin-4-yl)thiazol (1183);
4-Methyl-2-(5-(8-methyl-[1,2,4]triazolo[1,5-a]pyridin-6-yl)-4-(2,2,2-trifluorethyl)-1H-pyrazol-3-yl)-5-(1-(tetrahydro-2H-pyran-4-yl)piperidin-4-yl)thiazol (1184);
4-Methyl-2-(5-(8-methyl-[1,2,4]triazolo[1,5-a]pyridin-6-yl)-4-(2,2,2-trifluorethyl)-1H-pyrazol-3-yl)-5-(1-((tetrahydro-2H-pyran-4-yl)methyl)piperidin-4-yl)thiazol (1185);
2-(5-(7,8-Dimethyl-[1,2,4]triazolo[1,5-a]pyridin-6-yl)-4-(2,2,2-trifluorethyl)-1H-pyrazol-3-yl)-4-methyl-5-(1-((tetrahydro-2H-pyran-4-yl)methyl)piperidin-4-yl)thiazol (1186);
2-(5-(7,8-Dimethyl-[1,2,4]triazolo[1,5-a]pyridin-6-yl)-4-(2,2,2-trifluorethyl)-1H-pyrazol-3-yl)-5-(1-isopropylpiperidin-4-yl)-4-methylthiazol (1187);
5-(1-(Cyclopropylmethyl)piperidin-4-yl)-2-(5-(7,8-dimethyl-[1,2,4]triazolo[1,5-a]pyridin-6-yl)-4-(2,2,2-trifluorethyl)-1H-pyrazol-3-yl)-4-methylthiazol (1188);
2-(5-(7,8-Dimethyl-[1,2,4]triazolo[1,5-a]pyridin-6-yl)-4-(2,2,2-trifluorethyl)-1H-pyrazol-3-yl)-4-methyl-5-(1-methylpiperidin-4-yl)thiazol (1189);
2-(5-(7,8-Dimethyl-[1,2,4]triazolo[1,5-a]pyridin-6-yl)-4-(2,2,2-trifluorethyl)-1H-pyrazol-3-yl)-5-(1-ethylpiperidin-4-yl)-4-methylthiazol (1190);
2-(5-(7,8-Dimethyl-[1,2,4]triazolo[1,5-a]pyridin-6-yl)-4-(2,2,2-trifluorethyl)-1H-pyrazol-3-yl)-4-methyl-5-(1-propylpiperidin-4-yl)thiazol (1191);
2-(Dimethylamino)-1-(4-(2-(5-(8-methoxy-[1,2,4]triazolo[1,5-a]pyridin-6-yl)-4-(2,2,2-trifluorethyl)-1H-pyrazol-3-yl)thiazol-5-yl)piperidin-1-yl)ethan-1-on (1192);
2-(Dimethylamino)-1-(4-(2-(5-(8-methyl-[1,2,4]triazolo[1,5-a]pyridin-6-yl)-4-(2,2,2-trifluorethyl)-1H-pyrazol-3-yl)thiazol-5-yl)piperidin-1-yl)ethan-1-on (1193);
1-(4-(2-(5-(8-Methyl-[1,2,4]triazolo[1,5-a]pyridin-6-yl)-4-(2,2,2-trifluorethyl)-1H-pyrazol-3-yl)thiazol-5-yl)piperidin-1-yl)-2-morpholinoethan-1-on (1194);
2-(Diethylamino)-1-(4-(2-(5-(8-methyl-[1,2,4]triazolo[1,5-a]pyridin-6-yl)-4-(2,2,2-trifluorethyl)-1H-pyrazol-3-yl)thiazol-5-yl)piperidin-1-yl)ethan-1-on (1195);
2-(5-(8-Methoxy-[1,2,4]triazolo[1,5-a]pyridin-6-yl)-4-(2,2,2-trifluorethyl)-1H-pyrazol-3-yl)-5-(1-(2-methoxyethyl)piperidin-4-yl)thiazol (1196);
5-(1-(2-Methoxyethyl)piperidin-4-yl)-2-(5-(8-methyl-[1,2,4]triazolo[1,5-a]pyridin-6-yl)-4-(2,2,2-trifluorethyl)-1H-pyrazol-3-yl)thiazol (1197);
2-Methyl-1-(4-(2-(5-(8-methyl-[1,2,4]triazolo[1,5-a]pyridin-6-yl)-4-(2,2,2-trifluorethyl)-1H-pyrazol-3-yl)thiazol-5-yl)piperidin-1-yl)propan-2-ol (1198);
2-Methyl-1-(4-(4-methyl-2-(5-(8-methyl-[1,2,4]triazolo[1,5-a]pyridin-6-yl)-4-(2,2,2-trifluorethyl)-1H-pyrazol-3-yl)thiazol-5-yl)piperidin-1-yl)propan-2-ol (1199);
2-(5-(8-Methoxy-[1,2,4]triazolo[1,5-a]pyridin-6-yl)-4-(2,2,2-trifluorethyl)-1H-pyrazol-3-yl)-5-(1-(2-(methylsulfonyl)ethyl)piperidin-4-yl)thiazol (1200);
2-(4-(2-(5-(8-Methoxy-[1,2,4]triazolo[1,5-a]pyridin-6-yl)-4-(2,2,2-trifluorethyl)-1H-pyrazol-3-yl)thiazol-5-yl)piperidin-1-yl)-N,N-dimethylacetamid (1201);
5-(1-(2-(tert-Butoxy)ethyl)piperidin-4-yl)-2-(5-(8-methoxy-[1,2,4]triazolo[1,5-a] Pyridin-6-yl)-4-(2,2,2-trifluorethyl)-1H-pyrazol-3-yl)thiazol (1202);
2-(4-(2-(5-(8-Methyl-[1,2,4]triazolo[1,5-a]pyridin-6-yl)-4-(2,2,2-trifluorethyl)-1H-pyrazol-3-yl)thiazol-5-yl)piperidin-1-yl)-1-morpholinoethan-1-on (1203);
N,N-Dimethyl-2-(4-(2-(5-(8-methyl-[1,2,4]triazolo[1,5-a]pyridin-6-yl)-4-(2,2,2-trifluorethyl)-1H-pyrazol-3-yl)thiazol-5-yl)piperidin-1-yl)acetamid (1204);
5-(1-(2-Fluorethyl)piperidin-4-yl)-2-(5-(8-methyl-[1,2,4]triazolo[1,5-a]pyridin-6-yl)-4-(2,2,2-trifluorethyl)-1H-pyrazol-3-yl)thiazol (1205);
5-(1-(3-Fluorpropyl)piperidin-4-yl)-2-(5-(8-methyl-[1,2,4]triazolo[1,5-a]pyridin-6-yl)-4-(2,2,2-trifluorethyl)-1H-pyrazol-3-yl)thiazol (1206);
2-(4-(2-(5-(8-Methoxy-[1,2,4]triazolo[1,5-a]pyridin-6-yl)-4-(2,2,2-trifluorethyl)-1H-pyrazol-3-yl)thiazol-5-yl)piperidin-1-yl)-1-(2-oxa-6-azaspiro[3.3] Heptan-6-yl)ethan-1on (1207);
N-Isopropyl-2-(4-(2-(5-(8-methoxy-[1,2,4]triazolo[1,5-a]pyridin-6-yl)-4-(2,2,2-trifluorethyl)-1H-pyrazol-3-yl)thiazol-5-yl)piperidin-1-yl)-N-methylacetamid (1208);
1-(Azetidin-1-yl)-2-(4-(2-(5-(8-methoxy-[1,2,4]triazolo[1,5-a]pyridin-6-yl)-4-(2,2,2-trifluorethyl)-1H-pyrazol-3-yl)thiazol-5-yl)piperidin-1-yl)ethan-1-on (1209);
1-(4-(2-(5-(8-Methoxy-[1,2,4]triazolo[1,5-a]pyridin-6-yl)-4-(2,2,2-trifluorethyl)-1H-pyrazol-3-yl)thiazol-5-yl)piperidin-1-yl)-2-morpholinoethan-1-on (1210);
1-(4-(2-(5-(8-Methoxy-[1,2,4]triazolo[1,5-a]pyridin-6-yl)-4-(2,2,2-trifluorethyl)-1H-pyrazol-3-yl)thiazol-5-yl)piperidin-1-yl)-2-(7-oxa-2-azaspiro[3.5]nonan-2-yl)ethan-1-on (1211);
4-(2-(4-(2-(5-(8-Methoxy-[1,2,4]triazolo[1,5-a]pyridin-6-yl)-4-(2,2,2-trifluorethyl)-1H-pyrazol-3-yl)thiazol-5-yl)piperidin-1-yl)-2-oxoethyl)piperazin-2-on (1212);
1-(4-(2-(5-(8-Methoxy-[1,2,4]triazolo[1,5-a]pyridin-6-yl)-4-(2,2,2-trifluorethyl)-1H-pyrazol-3-yl)thiazol-5-yl)piperidin-1-yl)-2-(3-methoxyazetidin-1-yl)ethan-1-on (1213);
1-(4-(2-(5-(8-Methoxy-[1,2,4]triazolo[1,5-a]pyridin-6-yl)-4-(2,2,2-trifluorethyl)-1H-pyrazol-3-yl)thiazol-5-yl)piperidin-1-yl)-2-((2-methoxyethyl)(methyl)amino)ethan-1-on (1214);
4-(4-(2-(5-(8-Methoxy-[1,2,4]triazolo[1,5-a]pyridin-6-yl)-4-(2,2,2-trifluorethyl)-1H-pyrazol-3-yl)-4-methylthiazol-5-yl)cyclohexyl)morpholin (1215);
N-(4-(2-(5-(8-Methoxy-[1,2,4]triazolo[1,5-a]pyridin-6-yl)-4-(2,2,2-trifluorethyl)-1H-pyrazol-3-yl)-4-methylthiazol-5-yl)cyclohexyl)oxetan-3-amin (1216-1217);
6-(4-(2-(5-(8-Methoxy-[1,2,4]triazolo[1,5-a]pyridin-6-yl)-4-(2,2,2-trifluorethyl)-1H-pyrazol-3-yl)-4-methylthiazol-5-yl)cyclohexyl)-2-oxa-6-azaspiro[3.3]heptan (12181219);
2-(4-(2-(5-(8-Methoxy-[1,2,4]triazolo[1,5-a]pyridin-6-yl)-4-(2,2,2-trifluorethyl)-1H-pyrazol-3-yl)-4-methylthiazol-5-yl)cyclohexyl)-7-oxa-2-azaspiro[3.5]nonan (1220- 1221);
4-(4-(2-(5-(8-Methoxy-[1,2,4]triazolo[1,5-a]pyridin-6-yl)-4-(2,2,2-trifluorethyl)-1H-pyrazol-3-yl)-4-methylthiazol-5-yl)cyclohexyl)piperazin-2-on (1222-1223);
2-(5-(8-Methoxy-[1,2,4]triazolo[1,5-a]pyridin-6-yl)-4-(2,2,2-trifluorethyl)-1H-pyrazol-3-yl)-5-(4-(3-methoxyazetidin-1-yl)cyclohexyl)thiazol (1224-1225);
2-(4-(2-(5-(8-Methoxy-[1,2,4]triazolo[1,5-a]pyridin-6-yl)-4-(2,2,2-trifluorethyl)-1H-pyrazol-3-yl)thiazol-5-yl)cyclohexyl)-7-oxa-2-azaspiro[3.5]nonan (1226-1227);
4-(4-(2-(5-(8-Methoxy-[1,2,4]triazolo[1,5-a]pyridin-6-yl)-4-(2,2,2-trifluorethyl)-1H-pyrazol-3-yl)thiazol-5-yl)cyclohexyl)piperazin-2-on (1228-1229);
4-(4-(2-(5-(8-Methoxy-[1,2,4]triazolo[1,5-a]pyridin-6-yl)-4-(2,2,2-trifluorethyl)-1H-pyrazol-3-yl)thiazol-5-yl)cyclohexyl)morpholin (1230-1231);
2-(5-(8-Methoxy-[1,2,4]triazolo[1,5-a]pyridin-6-yl)-4-(2,2,2-trifluorethyl)-1H-pyrazol-3-yl)-4-methyl-5-((1S,4S)-5-methyl-2,5-diazabicyclo[2.2.1]heptan-2-yl)thiazol (1232);
2-(5-(8-Methoxy-[1,2,4]triazolo[1,5-a]pyridin-6-yl)-4-(2,2,2-trifluorethyl)-1H-pyrazol-3-yl)-4-methyl-5-(piperazin-1-yl)thiazol (1233);
4-Methyl-2-(5-(8-methyl-[1,2,4]triazolo[1,5-a]pyridin-6-yl)-4-(2,2,2-trifluorethyl)-1H-pyrazol-3-yl)-5-(piperazin-1-yl)thiazol (1234);
5-((2R,5S)-2,5-Dimethylpiperazin-1-yl)-2-(5-(8-methoxy-[1,2,4]triazolo[1,5-a]pyridin-6-yl)-4-(2,2,2-trifluorethyl)-1H-pyrazol-3-yl)thiazol (1235);
2-(5-(8-Methoxy-[1,2,4]triazolo[1,5-a]pyridin-6-yl)-4-(2,2,2-trifluorethyl)-1H-pyrazol-3-yl)-4-methyl-5-(2,6-diazaspiro[3.3]heptan-2-yl)thiazol (1236);
5-((15,4S)-5-Ethyl-2,5-diazabicyclo[2.2.1]heptan-2-yl)-2-(5-(8-methoxy-[1,2,4]triazolo[1,5-a]pyridin-6-yl)-4-(2,2,2-trifluorethyl)-1H-pyrazol-3-yl)-4methylthiazol (1237);
2-(5-(8-Methoxy-[1,2,4]triazolo[1,5-a]pyridin-6-yl)-4-(2,2,2-trifluorethyl)-1H-pyrazol-3-yl)-4-methyl-5-((1R,4R)-5-propyl-2,5-diazabicyclo[2.2.1]heptan-2-yl)thiazol (1238);
2-(5-(8-Methoxy-[1,2,4]triazolo[1,5-a]pyridin-6-yl)-4-(2,2,2-trifluorethyl)-1H-pyrazol-3-yl)-4-methyl-5-((1S,4S)-5-(oxetan-3-yl)-2,5-diazabicyclo[2.2.1]heptan-2-yl)thiazol (1239);
2-(5-(8-Methoxy-[1,2,4]triazolo[1,5-a]pyridin-6-yl)-4-(2,2,2-trifluorethyl)-1H-pyrazol-3-yl)-4-methyl-5-((1S,4S)-5-((tetrahydro-2H-pyran-4-yl)methyl)-2,5-diazabicyclo[2.2.1]heptan-2-yl)thiazol (1240);
5-((15,45)-5-Isopropyl-2,5-diazabicyclo[2.2.1]heptan-2-yl)-2-(5-(8-methoxy-[1,2,4]triazolo[1,5-a]pyridin-6-yl)-4-(2,2,2-trifluorethyl)-1H-pyrazol-3-yl)-4methylthiazol (1241);
2-(5-(8-Methoxy-[1,2,4]triazolo[1,5-a]pyridin-6-yl)-4-(2,2,2-trifluorethyl)-1H-pyrazol-3-yl)-4-methyl-5-((15,45)-5-(tetrahydro-2H-pyran-4-yl)-2,5-diazabicyclo[2.2.1]heptan-2-yl)thiazol (1242);
5-((1S,4S)-5-Isobutyl-2,5-diazabicyclo[2.2.1]heptan-2-yl)-2-(5-(8-methoxy-[1,2,4]triazolo[1,5-a]pyridin-6-yl)-4-(2,2,2-trifluorethyl)-1H-pyrazol-3-yl)-4methylthiazol (1243);
2-(5-(8-Methoxy-[1,2,4]triazolo[1,5-a]pyridin-6-yl)-4-(2,2,2-trifluorethyl)-1H-pyrazol-3-yl)-4-methyl-5-(4-methylpiperazin-1-yl)thiazol (1244);
2-(5-(8-Methoxy-[1,2,4]triazolo[1,5-a]pyridin-6-yl)-4-(2,2,2-trifluorethyl)-1H-pyrazol-3-yl)-4-methyl-5-(4-((tetrahydro-2H-pyran-4-yl)methyl)piperazin-1-yl)thiazol (1245);
2-(5-(8-Methoxy-[1,2,4]triazolo[1,5-a]pyridin-6-yl)-4-(2,2,2-trifluorethyl)-1H-pyrazol-3-yl)-4-methyl-5-(4-propylpiperazin-1-yl)thiazol (1246);
5-(4-Ethylpiperazin-1-yl)-2-(5-(8-methoxy-[1,2,4]triazolo[1,5-a]pyridin-6-yl)-4-(2,2,2-trifluorethyl)-1H-pyrazol-3-yl)-4-methylthiazol (1247);
5-(4-Isobutylpiperazin-1-yl)-2-(5-(8-methoxy-[1,2,4]triazolo[1,5-a]pyridin-6-yl)-4-(2,2,2-trifluorethyl)-1H-pyrazol-3-yl)-4-methylthiazol (1248);
4-Methyl-2-(5-(8-methyl-[1,2,4]triazolo[1,5-a]pyridin-6-yl)-4-(2,2,2-trifluorethyl)-1H-pyrazol-3-yl)-5-(4-(oxetan-3-yl)piperazin-1-yl)thiazol (1249);
4-Methyl-2-(5-(8-methyl-[1,2,4]triazolo[1,5-a]pyridin-6-yl)-4-(2,2,2-trifluorethyl)-1H-pyrazol-3-yl)-5-(4-methylpiperazin-1-yl)thiazol (1250);
5-(4-Ethylpiperazin-1-yl)-4-methyl-2-(5-(8-methyl-[1,2,4]triazolo[1,5-a]pyridin-6-yl)-4-(2,2,2-trifluorethyl)-1H-pyrazol-3-yl)thiazol (1251);
4-Methyl-2-(5-(8-methyl-[1,2,4]triazolo[1,5-a]pyridin-6-yl)-4-(2,2,2-trifluorethyl)-1H-pyrazol-3-yl)-5-(4-propylpiperazin-1-yl)thiazol (1252);
4-Methyl-2-(5-(8-methyl-[1,2,4]triazolo[1,5-a]pyridin-6-yl)-4-(2,2,2-trifluorethyl)-1H-pyrazol-3-yl)-5-(4-((tetrahydro-2H-pyran-4-yl)methyl)piperazin-1-yl)thiazol (1253);
4-Methyl-2-(5-(8-methyl-[1,2,4]triazolo[1,5-a]pyridin-6-yl)-4-(2,2,2-trifluorethyl)-1H-pyrazol-3-yl)-5-(4-(tetrahydro-2H-pyran-4-yl)piperazin-1-yl)thiazol (1254);
5-(4-(Cyclopropylmethyl)piperazin-1-yl)-4-methyl-2-(5-(8-methyl-[1,2,4]triazolo[1,5-a]pyridin-6-yl)-4-(2,2,2-trifluorethyl)-1H-pyrazol-3-yl)thiazol (1256);
2-(5-(8-Methoxy-[1,2,4]triazolo[1,5-a]pyridin-6-yl)-4-(2,2,2-trifluorethyl)-1H-pyrazol-3-yl)-5-((2R,5S)-2,4,5-trimethylpiperazin-1-yl)thiazol (1257);
5-((2R,5S)-4-Ethyl-2,5-dimethylpiperazin-1-yl)-2-(5-(8-methoxy-[1,2,4]triazolo[1,5-a]pyridin-6-yl)-4-(2,2,2-trifluorethyl)-1H-pyrazol-3-yl)thiazol (1258);
5-((2R,5S)-2,5-Dimethyl-4-propylpiperazin-1-yl)-2-(5-(8-methoxy-[1,2,4]triazolo[1,5-a]pyridin-6-yl)-4-(2,2,2-trifluorethyl)-1H-pyrazol-3-yl)thiazol (1259);
5-((2R,5S)-4-Isopropyl-2,5-dimethylpiperazin-1-yl)-2-(5-(8-methoxy-[1,2,4]triazolo[1,5-a]pyridin-6-yl)-4-(2,2,2-trifluorethyl)-1H-pyrazol-3-yl)thiazol (1260);
2-(5-(8-Methoxy-[1,2,4]triazolo[1,5-a]pyridin-6-yl)-4-(2,2,2-trifluorethyl)-1H-pyrazol-3-yl)-4-methyl-5-(6-methyl-2,6-diazaspiro[3.3]heptan-2-yl)thiazol (1261);
5-(6-Isobutyl-2,6-diazaspiro[3.3]heptan-2-yl)-2-(5-(8-methoxy-[1,2,4]triazolo[1,5-a]pyridin-6-yl)-4-(2,2,2-trifluorethyl)-1H-pyrazol-3-yl)-4-methylthiazol (1262);
2-(5-(8-Methoxy-[1,2,4]triazolo[1,5-a]pyridin-6-yl)-4-(2,2,2-trifluorethyl)-1H-pyrazol-3-yl)-4-methyl-5-(6-(tetrahydro-2H-pyran-4-yl)-2,6-diazaspiro[3.3]heptan-2-yl)thiazol (1263);
5-(6-(Cyclopropylmethyl)-2,6-diazaspiro[3.3]heptan-2-yl)-2-(5-(8-methoxy-[1,2,4]triazolo[1,5-a]pyridin-6-yl)-4-(2,2,2-trifluorethyl)-1H-pyrazol-3-yl)-4methylthiazol (1264);
5-(6-(1-Isopropylpiperidin-4-yl)-2,6-diazaspiro[3.3]heptan-2-yl)-2-(5-(8-methoxy-[1,2,4]triazolo[1,5-a]pyridin-6-yl)-4-(2,2,2-trifluorethyl)-1H-pyrazol-3-yl)-4methylthiazol (1265);
5-(6-Isopropyl-2,6-diazaspiro[3.3]heptan-2-yl)-2-(5-(8-methoxy-[1,2,4]triazolo[1,5-a]pyridin-6-yl)-4-(2,2,2-trifluorethyl)-1H-pyrazol-3-yl)-4-methylthiazol (1266);
5-(6-(1-Cyclopropylethyl)-2,6-diazaspiro[3.3]heptan-2-yl)-2-(5-(8-methoxy-[1,2,4]triazolo[1,5-a]pyridin-6-yl)-4-(2,2,2-trifluorethyl)-1H-pyrazol-3-yl)-4methylthiazol (1267);
2-(5-(8-Methoxy-[1,2,4]triazolo[1,5-a]pyridin-6-yl)-4-(2,2,2-trifluorethyl)-1H-pyrazol-3-yl)-4-methyl-5-(6-(pentan-3-yl)-2,6-diazaspiro[3.3]heptan-2-yl)thiazol (1268);
1-(2-(4-Isopropyl-5-(8-methoxy-[1,2,4]triazolo[1,5-a]pyridin-6-yl)-1H-pyrazol-3-yl)thiazol-5-yl)-N-(3-methyloxetan-3-yl)piperidin-4-amin (1269);
1-(2-(4-Isopropyl-5-(8-methoxy-[1,2,4]triazolo[1,5-a]pyridin-6-yl)-1H-pyrazol-3-yl)thiazol-5-yl)-N-(2-methoxy-2-methylpropyl)piperidin-4-amin (1270);
N-Ethyl-1-(2-(4-isopropyl-5-(8-methoxy-[1,2,4]triazolo[1,5-a]pyridin-6-yl)-1H-pyrazol-3-yl)thiazol-5-yl)-N-methylpiperidin-4-amin (1271);
2-(5-(8-Methoxy-[1,2,4]triazolo[1,5-a]pyridin-6-yl)-4-(2,2,2-trifluorethyl)-1H-pyrazol-3-yl)-N,N-dimethyl-4,5,6,7-tetrahydrobenzo[d]thiazol-6-amin (1272);
N-Isopropyl-2-(5-(8-methoxy-[1,2,4]triazolo[1,5-a]pyridin-6-yl)-4-(2,2,2-trifluorethyl)-1H-pyrazol-3-yl)-N-methyl-4,5,6,7-tetrahydrobenzo[d]thiazol-6-amin (1273);
2-(5-(8-Methoxy-[1,2,4]triazolo[1,5-a]pyridin-6-yl)-4-(2,2,2-trifluorethyl)-1H-pyrazol-3-yl)-N,N-dimethyl-4,5,6,7-tetrahydrobenzo[d]thiazol-6-amin (1274);
8-Methoxy-6-(3-(4-methyl-5-(1-methylpiperidin-4-yl)pyridin-2-yl)-4-(2,2,2-trifluorethyl)-1H-pyrazol-5-yl)-[1,2,4]triazolo[1,5-a]pyridin (1275);
8-Methoxy-6-(3-(5-(piperidin-4-yl)pyridin-2-yl)-4-(2,2,2-trifluorethyl)-1H-pyrazol-5-yl)-[1,2,4]triazolo[1,5-a]pyridin (1276);
6-(3-(5-(1-Ethylpiperidin-4-yl)-4-methylpyridin-2-yl)-4-(2,2,2-trifluorethyl)-1H-pyrazol-5-yl)-8-methoxy-[1,2,4]triazolo[1,5-a]pyridin (1277);
8-Methoxy-6-(3-(4-methyl-5-(1-propylpiperidin-4-yl)pyridin-2-yl)-4-(2,2,2-trifluorethyl)-1H-pyrazol-5-yl)-[1,2,4]triazolo[1,5-a]pyridin (1278);
8-Methoxy-6-(3-(5-(1-(oxetan-3-yl)piperidin-4-yl)-4-(trifluormethyl)pyridin-2-yl)-4-(2,2,2-trifluorethyl)-1H-pyrazol-5-yl)-[1,2,4]triazolo[1,5-a]pyridin (1279);
8-Methoxy-6-(3-(4-methyl-5-(1-(tetrahydro-2H-pyran-4-yl)piperidin-4-yl)pyridin-2-yl)-4-(2,2,2-trifluorethyl)-1H-pyrazol-5-yl)-[1,2,4]triazolo[1,5-a]pyridin (1280);
8-Methoxy-6-(3-(4-methyl-5-(1-((tetrahydro-2H-pyran-4-yl)methyl)piperidin-4-yl) pyridin-2-yl)-4-(2,2,2-trifluorethyl)-1H-pyrazol-5-yl)-[1,2,4]triazolo[1,5-a]pyridin (1281);
6-(3-(5-(1-Isopropylpiperidin-4-yl)-4-methylpyridin-2-yl)-4-(2,2,2-trifluorethyl)-1H-pyrazol-5-yl)-8-methoxy-[1,2,4]triazolo[1,5-a]pyridin (1282);
6-(3-(5-(1-(2-Ethylbutyl)piperidin-4-yl)-4-methylpyridin-2-yl)-4-(2,2,2-trifluorethyl)-1H-pyrazol-5-yl)-8-methoxy-[1,2,4]triazolo[1,5-a]pyridin (1283);
6-(3-(5-(1-Isobutylpiperidin-4-yl)-4-methylpyridin-2-yl)-4-(2,2,2-trifluorethyl)-1H-pyrazol-5-yl)-8-methoxy-[1,2,4]triazolo[1,5-a]pyridin (1284);
6-(3-(5-(1-(Cyclobutylmethyl)piperidin-4-yl)-4-methylpyridin-2-yl)-4-(2,2,2-trifluorethyl)-1H-pyrazol-5-yl)-8-methoxy-[1,2,4]triazolo[1,5-a]pyridin (1285);
6-(3-(5-(1-Ethylpiperidin-4-yl)pyridin-2-yl)-4-(2,2,2-trifluorethyl)-1H-pyrazol-5-yl)-8-methoxy-[1,2,4]triazolo[1,5-a]pyridin (1286);
8-Methoxy-6-(3-(5-(1-methylpiperidin-4-yl)pyridin-2-yl)-4-(2,2,2-trifluorethyl)-1H-pyrazol-5-yl)-[1,2,4]triazolo[1,5-a]pyridin (1287);
6-(3-(5-(1-Isobutylpiperidin-4-yl)pyridin-2-yl)-4-(2,2,2-trifluorethyl)-1H-pyrazol-5-yl)-8-methoxy-[1,2,4]triazolo[1,5-a]pyridin (1288);
6-(3-(5-(1-Cyclobutylpiperidin-4-yl)pyridin-2-yl)-4-(2,2,2-trifluorethyl)-1H-pyrazol-5-yl)-8-methoxy-[1,2,4]triazolo[1,5-a]pyridin (1289);
6-(3-(5-(1-Isopropylpiperidin-4-yl)pyridin-2-yl)-4-(2,2,2-trifluorethyl)-1H-pyrazol-5-yl)-8-methoxy-[1,2,4]triazolo[1,5-a]pyridin (1290);
8-Methoxy-6-(3-(5-(1-propylpiperidin-4-yl)pyridin-2-yl)-4-(2,2,2-trifluorethyl)-1H-pyrazol-5-yl)-[1,2,4]triazolo[1,5-a]pyridin (1291);
8-Methoxy-6-(3-(5-(1-(oxetan-3-yl)piperidin-4-yl)pyridin-2-yl)-4-(2,2,2-trifluorethyl)-1H-pyrazol-5-yl)-[1,2,4]triazolo[1,5-a]pyridin (1292);
8-Methoxy-6-(3-(5-(1-(tetrahydro-2H-pyran-4-yl)piperidin-4-yl)pyridin-2-yl)-4-(2,2,2-trifluorethyl)-1H-pyrazol-5-yl)-[1,2,4]triazolo[1,5-a]pyridin (1293);
8-Methoxy-6-(3-(5-(1-((tetrahydro-2H-pyran-4-yl)methyl)piperidin-4-yl)pyridin-2-yl)-4-(2,2,2-trifluorethyl)-1H-pyrazol-5-yl)-[1,2,4]triazolo[1,5-a]pyridin (1294);
6-(3-(5-(1-(Cyclobutylmethyl)piperidin-4-yl)pyridin-2-yl)-4-(2,2,2-trifluorethyl)-1H-pyrazol-5-yl)-8-methoxy-[1,2,4]triazolo[1,5-a]pyridin (1295);
2-(Dimethylamino)-1-(4-(6-(5-(8-methoxy-[1,2,4]triazolo[1,5-a]pyridin-6-yl)-4-(2,2,2-trifluorethyl)-1H-pyrazol-3-yl)-4-methylpyridin-3-yl)piperidin-1-yl)ethan-1-on (1296);
2-(Dimethylamino)-1-(4-(6-(5-(8-methoxy-[1,2,4]triazolo[1,5-a]pyridin-6-yl)-4-(2,2,2-trifluorethyl)-1H-pyrazol-3-yl)pyridin-3-yl)piperidin-1-yl)ethan-1-on (1297);
2-(Diethylamino)-1-(4-(6-(5-(8-methoxy-[1,2,4]triazolo[1,5-a]pyridin-6-yl)-4-(2,2,2-trifluorethyl)-1H-pyrazol-3-yl)pyridin-3-yl)piperidin-1-yl)ethan-1-on (1298);
2-(4-(6-(5-(8-Methoxy-[1,2,4]triazolo[1,5-a]pyridin-6-yl)-4-(2,2,2-trifluorethyl)-1H-pyrazol-3-yl)-4-methylpyridin-3-yl)piperidin-1-yl)-N,N-dimethylacetamid (1299);
2-(4-(6-(5-(8-Methoxy-[1,2,4]triazolo[1,5-a]pyridin-6-yl)-4-(2,2,2-trifluorethyl)-1H-pyrazol-3-yl)-4-methylpyridin-3-yl)piperidin-1-yl)-N-methylacetamid (1300);
8-Methoxy-6-(3-(5-(1-(2-methoxyethyl)piperidin-4-yl)-4-methylpyridin-2-yl)-4-(2,2,2-trifluorethyl)-1H-pyrazol-5-yl)-[1,2,4]triazolo[1,5-a]pyridin (1301);
8-Methoxy-6-(3-(4-methyl-5-(1-(2-(methylsulfonyl)ethyl)piperidin-4-yl)pyridin-2-yl)-4-(2,2,2-trifluorethyl)-1H-pyrazol-5-yl)-[1,2,4]triazolo[1,5-a]pyridin (1302);
N-(2-(4-(5-(8-Methyl-[1,2,4]triazolo[1,5-a]pyridin-6-yl)-4-(2,2,2-trifluorethyl)-1H-pyrazol-3-yl)phenyl)propan-2-yl)tetrahydro-2H-pyran-4-amin (1303);
(S)-1-(4-(5-(8-Methoxy-[1,2,4]triazolo[1,5-a]pyridin-6-yl)-4-(2,2,2-trifluorethyl)-1H-pyrazol-3-yl)phenyl)-N-methylethan-1-amin (1304B);
6-(5-(8-Methoxy-[1,2,4]triazolo[1,5-a]pyridin-6-yl)-4-(2,2,2-trifluorethyl)-1H-pyrazol-3-yl)-N-methyl-1,2,3,4-tetrahydronaphthalen-2-amin (1305B);
6-(5-(8-Methoxy-[1,2,4]triazolo[1,5-a]pyridin-6-yl)-4-(2,2,2-trifluorethyl)-1H-pyrazol-3-yl)-N-methyl-1,2,3,4-tetrahydronaphthalen-2-amin (1306B);
6-(5-(8-Methoxy-[1,2,4]triazolo[1,5-a]pyridin-6-yl)-4-(2,2,2-trifluorethyl)-1H-pyrazol-3-yl)-N-methyl-1,2,3,4-tetrahydronaphthalen-2-amin (1307B);
N,N-Dimethyl-2-(4-(5-(8-methyl-[1,2,4]triazolo[1,5-a]pyridin-6-yl)-4-(2,2,2-trifluorethyl)-1H-pyrazol-3-yl)phenyl)propan-2-amin (1308);
(S)-1-(4-(5-(8-Methoxy-[1,2,4]triazolo[1,5-a]pyridin-6-yl)-4-(2,2,2-trifluorethyl)-1H-pyrazol-3-yl)phenyl)-N,N-dimethylethan-1-amin (1309);
6-(5-(8-Methoxy-[1,2,4]triazolo[1,5-a]pyridin-6-yl)-4-(2,2,2-trifluorethyl)-1H-pyrazol-3-yl)-N,N-dimethyl-1,2,3,4-tetrahydronaphthalen-2-amin (1310);
N-Isopropyl-6-(5-(8-methoxy-[1,2,4]triazolo[1,5-a]pyridin-6-yl)-4-(2,2,2-trifluorethyl)-1H-pyrazol-3-yl)-N-methyl-1,2,3,4-tetrahydronaphthalen-2-amin (1311);
N-(6-(5-(8-Methoxy-[1,2,4]triazolo[1,5-a]pyridin-6-yl)-4-(2,2,2-trifluorethyl)-1H-pyrazol-3-yl)-1,2,3,4-tetrahydronaphthalen-2-yl)-N-methyloxetan-3-amin (1312);
2-(Dimethylamino)- N -(2-(4-(5-(8-methyl-[1,2,4]triazolo[1,5-a]pyridin-6-yl)-4-(2,2,2-trifluorethyl)-1H-pyrazol-3-yl)phenyl)propan-2-yl)acetamid (1313);
(S)-2-(Dimethylamino)-N -(1-(4-(5-(8-methoxy-[1,2,4]triazolo[1,5-a]pyridin-6-yl)-4-(2,2,2-trifluorethyl)-1H-pyrazol-3-yl)phenyl)ethyl)-N-methylacetamid (1314);
2-(Dimethylamino)- N -(6-(5-(8-methoxy-[1,2,4]triazolo[1,5-a]pyridin-6-yl)-4-(2,2,2-trifluorethyl)-1H-pyrazol-3-yl)-1,2,3,4-tetrahydronaphthalen-2-yl)-N-methylacetamid (1315);
8-Methoxy-6-(3-(5-(6-((tetrahydro-2H-pyran-4-yl)methyl)-2,6-diazaspiro[3.3]heptan-2-yl)pyridin-2-yl)-4-(2,2,2-trifluorethyl)-1H-pyrazol-5-yl)-[1,2,4]triazolo[1,5-a]pyridin (1316);
8-Methoxy-6-(3-(5-(6-(oxetan-3-yl)-2,6-diazaspiro[3.3]heptan-2-yl)pyridin-2-yl)-4-(2,2,2-trifluorethyl)-1H-pyrazol-5-yl)-[1,2,4]triazolo[1,5-a]pyridin (1317);
6-(3-(5-(6-Isopropyl-2,6-diazaspiro[3.3]heptan-2-yl)pyridin-2-yl)-4-(2,2,2-trifluorethyl)-1H-pyrazol-5-yl)-8-methoxy-[1,2,4]triazolo[1,5-a]pyridin (1318);
8-Methoxy-6-(3-(5-(6-propyl-2,6-diazaspiro[3.3]heptan-2-yl)pyridin-2-yl)-4-(2,2,2-trifluorethyl)-1H-pyrazol-5-yl)-[1,2,4]triazolo[1,5-a]pyridin (1319);
6-(3-(5-(6-Ethyl-2,6-diazaspiro[3.3]heptan-2-yl)pyridin-2-yl)-4-(2,2,2-trifluorethyl)-1H-pyrazol-5-yl)-8-methoxy-[1,2,4]triazolo[1,5-a]pyridin (1320);
8-Methoxy-6-(3-(5-(6-methyl-2,6-diazaspiro[3.3]heptan-2-yl)pyridin-2-yl)-4-(2,2,2-trifluorethyl)-1H-pyrazol-5-yl)-[1,2,4]triazolo[1,5-a]pyridin (1321);
6-(3-(5-(6-Cyclobutyl-2,6-diazaspiro[3.3]heptan-2-yl)pyridin-2-yl)-4-(2,2,2-trifluorethyl)-1H-pyrazol-5-yl)-8-methoxy-[1,2,4]triazolo[1,5-a]pyridin (1322);
6-(3-(5-(6-Isobutyl-2,6-diazaspiro[3.3]heptan-2-yl)pyridin-2-yl)-4-(2,2,2-trifluorethyl)-1H-pyrazol-5-yl)-8-methoxy-[1,2,4]triazolo[1,5-a]pyridin (1323);
8-Methoxy-6-(3-(5-(6-(tetrahydro-2H-pyran-4-yl)-2,6-diazaspiro[3.3]heptan-2-yl) pyridin-2-yl)-4-(2,2,2-trifluorethyl)-1H-pyrazol-5-yl)-[1,2,4]triazolo[1,5-a]pyridin (1324);
6-(3-(5-(6-(Cyclobutylmethyl)-2,6-diazaspiro[3.3]heptan-2-yl)pyridin-2-yl)-4-(2,2,2-trifluorethyl)-1H-pyrazol-5-yl)-8-methoxy-[1,2,4]triazolo[1,5-a]pyridin (1325);
8-Methoxy-6-(3-(5-(1-((tetrahydro-2H-pyran-4-yl)methyl)piperidin-4-yl)pyridin-2-yl)-4-(trifluormethyl)-1H-pyrazol-5-yl)-[1,2,4]triazolo[1,5-a]pyridin (1326);
6-(3-(5-(1-Cyclobutylpiperidin-4-yl)pyridin-2-yl)-4-(trifluormethyl)-1H-pyrazol-5-yl)-8-methoxy-[1,2,4]triazolo[1,5-a]pyridin (1327); oder
6-(3-(5-(1-(Cyclobutylmethyl)piperidin-4-yl)pyridin-2-yl)-4-(trifluormethyl)-1H-pyrazol-5-yl)-8-methoxy-[1,2,4]triazolo[1,5-a]pyridin (1328).

8. Pharmazeutische Zusammensetzung, umfassend eine Verbindung nach einem der Ansprüche 1-7 oder ein pharmazeutisch akzeptables Salz davon; und einen pharmazeutisch akzeptablen Träger.

9. Verbindung nach einem der Ansprüche 1-7 oder ein pharmazeutisch akzeptables Salz davon oder eine pharmazeutische Zusammensetzung nach Anspruch 8 zur Verwendung in der Therapie.

10. Verbindung oder ein pharmazeutisch akzeptables Salz davon oder eine pharmazeutische Zusammensetzung zur Verwendung nach Anspruch 9, zur Verwendung bei der Behandlung einer Autoimmunerkrankung oder chronischen Entzündungskrankheit.

11. Verbindung oder ein pharmazeutisch akzeptables Salz davon oder pharmazeutische Zusammensetzung zur Verwendung nach Anspruch 10, wobei die Autoimmunerkrankung oder chronische Entzündungskrankheit aus systemischem Lupus erythematodes (SLE), rheumatoider Arthritis, Multipler Sklerose (MS) und Sjögren-Syndrom ausgewählt ist.

## Revendications

1. Composé de formule (I) 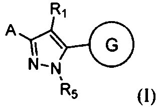 N-oxyde, ou un sel de celui-ci, dans lequel :
G est : 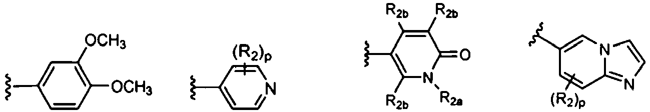 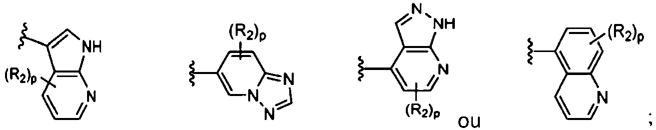
R₁ est -CH₃, -CH₂CH₃, -CH(CH₃)₂, -CF₃, ou -CH₂CF₃ ;
chaque R₂ est indépendamment -CN, -CH₃ ou -OCH₃ ;
R₂ₐ est alkyle en C₁₋₆, fluoroalkyle en C₁₋₃, hydroxyalkyle en C₁₋₆, aminoalkyleC₁₋₃, (alkyle en (CH₂)₀₋₄O(C₁₋₃), cycloalkyle en C₃₋₆, (cycloalkyle en-(CH₂)₁₋₃C(O)NR_{y}R_{y}, -CH₂(C₃₋₆), (phenyle en )-CH₂, tétrahydrofuranyle, tétrahydropyranyle ou phényle ;
chaque R_{2b} est indépendamment hydrogène, halo, -CN, -NRₓRₓ, -C₁₋₆ alkyle, C₁₋₃ fluoroalkyle, C₁₋₃ ] hydroxyalkyle, C₁₋₃ fluoroalcoxy, (CH₂)₀₋₂O(C₁₋₃ alkyle), -(CH₂)₀₋₃C(O)NRₓRₓ, -(CH₂)₁₋₃(C₃₋₆cycloalkyle), -C(O)O(C₁₋₃alkyle), -C(O)NRₓ(C₁₋₃alkyle), -CRₓ = CRₓRₓ, ou -CRₓ = CH(C₃₋₆ cycloalkyle);
A est :
(i) - CH₂ N(CH₃)Rₓ, -C(O)NRₓRₓ, -C(O)N(CH₃)(CH₂CH₂CN), -C(O)N(CH₂CH₃)(CH₂CH₂CN) ou -C(O)N(CH₃)(CH₂CH₂CH₂N(CH₃)₂) ;
(ii) -C(O)A₁, -C(O)NRₓ(CRₓRₓ)₀₋₂A₁, -CH₂NHA₁, ou -C(O)C(O)NHA₁ ;
(iii) cyclohexyle substitué par zéro jusqu'à 1R_{3b} ;
(iv) pipéridinyle substitué par zéro jusqu'à 1R_{3c} ;
(v) phényle substitué par zéro jusqu'à 1R_{3d} et zéro jusqu'à 1R₃ₑ ;
(vi) pyridinyle substitué par zéro jusqu'à 1R_{3f} et zéro jusqu'à 1R_{3g} ;
(vii) pyrazinyle ou pyrimidinyle, chacun substitué par zéro jusqu'à 1R_{3f},
(viii) thiazolyle substitué par R₃ₕ et zéro jusqu'à 1R₃ᵢ ;
(ix) diazabicyclo[2.2.1]heptanyle ou diazaspiro[3.3]heptanyle, chacun substitué par zéro jusqu'à 1R₃ⱼ ; ou
(x) benzo[d]thiazolyle, dihydroisoquinoléinyle, tétrahydronaphtyridinyle, tétrahydrobenzo[d]thiazolyle, tétrahydroimidazo[1,2-a]pyrazinyle, tétrahydroisoquinoléinyle, tétrahydroisoquinoléinyle, tétrahydronaphtalényle, tétrahydropyrazolo[1,5-a]pyrazinyle, tétrahydropyrido[4,3-d]pyrimidinyle, tétrahydrothiazolo[4,5-c]pyridinyle ou tétrahydrothiazolo[5,4-c]pyridinyle, chacun substitué par zéro jusqu'à 1R₃ₖ ;
A₁ est azétidinyle, cyclohexyle, pyrrolidinyle, pipéridinyle, pipérazinyle, morpholinyle, tétrahydropyranyle, pyridinyle, diazépanyle, hexahydropyrrolo[3,4-c]pyrrolyle, chacun substitué par zéro jusqu'à 1R₃ₐ ;
R₃ₐ est -OH, -CH₃, -CH₂CH₃, -CH₂CH₂CH₃,-CH(CH₃)₂, -CH₂CH(CH₃)₂, -CH(CH₃)(CH₂CH₃), -CH₂C(CH₃)₃, -CH₂CH₂CH(CH₃)₂, -CH(CH₃)CH(CH₃)₂, -CH₂CH₂C(CH₃)₃, -CH₂C(CH₂CH₃)₂, -CH₂CF₃, -CH₂CH₂CF₃, -CH₂CH₂CH₂CF₃, CH₂CH₂CN, -CH₂C(CH₃)₂OH, -CH₂CH₂OCH₃, -CH₂CH₂ S(O)₂CH₃, -CH(CH₃)CH₂ S(O)₂CH₃, -CH₂NH₂, -CH₂CH₂ N(CH₃)₂, -CH₂C(O)NH(CH₃), -CH₂C(O)N(CH₃)₂, -NR_{y}R_{y}, -N(CH₃)(CH₂CH₂OCH₃), -NH(CH₂CH₂CH₂CF₃), -NHCH(CH₃)(CH₂OCH₃), -C(O)NH₂, -C(O)OC(CH₃)₃, -CH₂ (cyclopropyle), -CH₂(méthyloxétanyle), -CH₂(tétrahydrofuranyle), -CH₂(tétrahydropyranyle), -CH₂(diméthylisoxazolyle), -CH₂(méthyltriazolyle), -CH₂(méthoxypyrimidinyle), -NH (oxétanyle), -NH (méthyloxétanyle), -NH (tétrahydropyranyle), -NH (diméthyltétrahydropyranyle), -N (cyclopropyle) 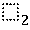 , -NHCH₂(cyclopentyle), -NHCH₂(diméthylisoxazolyle), - NHCH₂(méthyloxétanyle), -NHCH₂(pyridinyle), - NHCH₂(pyrimidinyle), - NHCH₂(méthylpyrimidinyle), -NHCH₂(méthoxypyrimidinyle), -NHCH₂(tétrahydrofuranyle), -NHCH₂(tétrahydropyranyle), cyclobutyle, oxétanyle, isopropylpipéridinyle, tétrahydropyranyle, diméthyltétrahydropyranyle ou pyridinyle ;
R_{3b} est -NH(CH₃), -NH(CH₂CHF₂), -N(CH₃)(CH₂CH₃), -NH(CH₂CH₂N(CH₃)₂), -N(CH₃)C(O)CH₂ N(CH₃)₂,-N(CH₃)CH₂C(O)N(CH₃)₂, -NH(isopropylpipéridinyle), -N(CH₃)C(O)(azétidinyel), -N(CH₃)C(O)(isopropylazétidinyle), - N(CH₃)C(O)(éthylazétidinyle), -N(CH₃)C(O)(méthylazétidinyle), -NH(CH₂(méthyloxétanyle), morpholinyle, méthylpipérazinyle ou diméthylaminopipéridinyle ;
R_{3c} est C₁₋₃alkyle, -CH₂C(O)N(CH₃)Rₓ, -C(O)CH₂ N(CH₃)₂, - C(O)CH₂CH₂ N(CH₃)₂ ou -C(O)CH₂CH₂NH(CH(CH₃)₂) ;
R_{3d} est :
(a) -CRₓRₓNRₓRₓ, -CRₓRₓNRₓ(C₂₋₅ alkyle), -CH(CH₃)N(CH₃)(CH₂CF₃), -CH₂CH₂ S(O)₂CH₃, -CH(CH₃)N(CH₃)CH₂C(O)N(CH₃)₂, -CH(CH₃)NRₓC(O)CH₂ N(CH₂CH₃)₂, -CRₓRₓNRₓC(O)CHRₓNRₓR_{y}, -CH(CH₃)N(CH₃)C(O)CH₂NRₓ(C₃₋₄ fluoroalkyle), -NRₓRₓ, -NH(CH(CH₃)₂), -C(O)NH₂, -CRₓRₓQ₁, -CRₓRₓNRₓQ₁, -CRₓRₓNRₓCH₂Q₁, -CRₓRₓNRₓC(O)Q₁, -CRₓRₓNRₓC(O)CRₓRₓQ₁, -CRₓRₓNRₓC(O)CRₓRₓNRₓQ₁ ou -CH(CH₃)N(oxétanyle)(C(O)CH₂ N(C₁₋₂ alkyle)₂) ;
(b) azétidinyle substitué par zéro jusqu'à 1 substituant sélectionné parmi C₁₋₃ alkyle, -CH₂C(CH₃)₂OH, -C(O)CH₂ N(CH₃)₂, -N(CH₃)₂, -NHCH(CH₃)₂, oxétanyle et tétrahydropyranyle ;
(c) cyclopropyle ou cyclohexyle, chacun substitué par -NRₓRₓ, -NRₓ(C₂₋₄ alkyle), -NH(oxétanyle), -N(CH₃)CH₂CH₂OCH, -N(CH₃)CH₂C(O)N(CH₃)₂, -N(CH₃)C(O)CH₂ N(CH₃)₂, ou -N(CH₃)CH₂(éthyloxétanyle) ; ou
(d) morpholinyle, pipérazinonyle, pipérazinyle, pipéridinyle ou pyrrolidinyle, chacun substitué par zéro jusqu'à 1 substituant sélectionné parmi C₁₋₅ alkyle, -CH₂CF₃, -CH₂C(CH₃)₂OH, -CH₂CH₂OCH₃, -CH₂C(O)CRₓRₓ, -C(O)CH₂ N(CH₃)₂, oxétanyle, méthyloxétanyle et tétrahydropyranyle ;
Q₁ est azétidinyle, cyclopropyle, morpholinyle, oxétanyle, tétrahydropyranyle, triazolyle, oxa-azaspiro[3.3]heptanyle, pipérazinonyle, difluoropipéridinyle ou pyrrolidinyle, chacun substitué par zéro jusqu'à 2 substituants sélectionnés indépendamment parmi F, -CH₃, -CH₂CH₃, -CH₂OH, et oxétanyle ;
R_{3c} est F ;
R_{3f} est :
(a) -OH, -NH₂, -N(CH₃)₂, -NH(CH(CH₃)₂), -NHCH₂C(CH₃)₂OCH₃, -CH₂NH(CH₃), -CH₂ N(CH₃)₂, -CH₂NH(CH(CH₃)₂), -CH(CH₃)N(CH₃)₂ ou -CH(CH₃)N(CH₃)C(O)CH₂ N(CH₃)₂ ;
(b) cyclohexyle substitué par -NH₂, -N(CH₃)₂, -NRₓ(CH₂CH₂OCH₃), - NH(cyclobutyle), -NH(méthyloxétanyle), -NHCH₂(méthylsulfonylcyclopropyle), morpholinyle, méthoxyazétidinyle, pipérazinonyle, difluoropipéridinyle, méthoxypipéridinyle, oxa-azaspiro[3.3]heptanyle ou oxa-azaspiro[3.5]nonanyle ;
(c) diazaspiro[3.3]heptanyle substitué par zéro jusqu'à 1 substituant sélectionné parmi C₁₋₄ alkyle, -CH₂CH₂OCH₃, -CH₂CH₂ S(O)₂CH₃, -CH₂(C₃₋₄ cycloalkyle), -CH₂ (tétrahydropyranyle), cyclobutyle, oxétanyle et tétrahydropyranyle ;
d) pipérazinyle substitué par zéro jusqu'à 1 substituant sélectionné parmi C₁₋₆ alkyle, -CH₂CH₂CF₃, -CH₂CH₂OCH₃, -CH₂CH₂ S(O)₂CH₃, -CH₂(C₃₋₄ cycloalkyle), -CH₂(éthyloxétanyle), -CH₂C(O)NH(CH₃), -CH₂C(O)N(CH₃)₂, -C(O)CH₂ N(CH₃)₂, -C(O)CH₂ N(CH₂CH₃)₂, -CH₂(tétrahydropyranyle), cyclobutyle, oxétanyle, tétrahydropyranyle et dioxothiotétrahydropyranyle ; ou
(e) pipéridinyle substitué par zéro jusqu'à 1 substituant sélectionné parmi C₁₋₆ alkyle, -CH₂CN, -CH₂CH₂ F ,-CH₂CH₂CH₂ F, -CH₂CH₂CF₃, -CH₂C(CH₃)₂OH, -CH₂CH₂OCH₃, -CH₂CH₂ S(O)₂CH₃, -CH₂C(O)NRₓRₓ, -C(O)CH₂ N(CH₃)₂, -C(O)CH₂ N(CH₂CH₃)₂, -CH₂(C₃₋₄ cycloalkyle), -CH₂(tétrahydrofuranyle), -CH₂(tétahydropyranyle), -CH₂(méthyltriazolyle), cyclobutyle, éthoxycyclobutyle, oxétanyle, tétrahydrofuranyle et tétrahydropyranyle ;
R_{3g} est F, -CH₃ ou -CF₃ ;
R₃ₕ est:
(a) -CH(CH₃)N(CH₃)Rₓ, -CH(CH₃)N(CH₃)(C₂₋₃ alkyle), -CH(CH₃)N(CH₃)CH₂C(O)N(CH₃)₂, -CH(CH₃)N(CH₃)C(O)CH₂ N(CH₃)₂ ou -CH(CH₃)N(CH₃)(tétrahydropyranyle) ;
(b) cyclohexyle substitué par -N(CH₃)Rₓ, -N(CH₃)(CH(CH₃)₃), -N(CH₃)(CH₂CH₂CF₃), -NRₓ(CH₂CH₂OCH₃), -NH(CH₂C(CH₃)₂OCH₃), -N(CH₃)C(O)CH₂ N(CH₃)₂, -NH(cyclobutyle), -NRₓ(oxétanyle), - NH(méthyloxétanyle), -NH(tétrahydropyranyle), -NHCH₂(méthylsulfonylcyclopropyle), -NHCH₂(méthyloxétanyle), méthoxyazétidinyle, (trifluorométhyl)hydroxyazétidinyle, morpholinyle, pyrrolidinyle, pipérazinonyle, méthylsulfonylpipérazinyle, oxazépanyle, oxa-azaspiro[3.3]heptanyle, oxa-azaspiro[3.5]nonanyle ou dioxothia-azaspiro[3.3]heptanyle ;
(c) pipérazinyle substitué par zéro jusqu'à deux -CH₃ ; et zéro ou 1 substituant sélectionné parmi C₁₋₅ alkyle, -CH₂CN, -CH₂CH₂ F, -CH₂CH₂CH₂ F, -CH₂C(CH₃)₂OH, -CH₂CH₂OCH₃, -CH₂CH₂ S(O)₂CH₃, -CH₂C(O)N(CH₃)Rₓ, -NHC(O)CH₂ N(CH₃)₂, -NHC(O)CH₂ N(CH₂CH₃)₂, -C(O)CH₂ N(CH₃)₂, -C(O)CH₂ N(CH₂CH₃)₂, -CH₂(C₃₋₄ cycloalkyle), -CH₂(tétrahydropyranyle), -C(O)CH₂(morpholinyle), cyclobutyle, oxétanyle et tétrahydropyranyle ; ou
d) pipéridinyle substitué par zéro ou un -CH₃ et zéro ou 1 substituant sélectionné parmi C₁₋₆ alkyle, -CH₂CN, -CH₂CH₂ F, -CH₂CH₂CH₂ F, -CH₂CH₂CF₃, -CH₂C(CH₃)₂OH, -CH₂CH₂OCH₃, -CH₂CH₂OC(CH₃)₃, -CH₂CH₂ S(O)₂CH₃, -CH₂C(O)NH₂, -CH₂C(O)N(CH₃)₂, -CH₂C(O)N(CH₃)(CH(CH₃)₂), -C(O)CH₂ N(CH₃)₂, -C(O)CH₂ N(CH₂CH₃)₂, -C(O)CH₂ N(CH₃)(CH₂CH₂OCH₃), -NH₂, -NH(CH₂CH₂CH₃), -NH(CH(CH₃)₂), -NHCH₂CH(CH₃)₂, -N(CH₃)₂, -N(CH₃)(CH₂CH₃), -NH(CH₂C(CH₃)₂OCH₃), -CH₂(C₃₋₆ cycloalkyle, -CH₂(méthylsulfonylcyclopropyle) , -CH₂(oxétanyle), -CH₂(méthyloxétanyle), -CH₂(éthyloxétanyle), -CH₂(tétrahydrofuranyle), -CH₂(tétrahydropyranyle , -CH₂(méthyltriazolyle), CH₂C(O)(oxétanyle), -CH₂C(O)(morpholinyle), -CH₂C(O)(oxa-azaspiro[3.3]heptanyle), -C(O)CH₂(méthoxyazétidinyle), -C(CH₂(morpholinyle), O)CH₂(oxa-azaspiro[3.5]nonanyle), -C(O)CH₂(pipéridinonyle), -N(CH₂ (cyclopropyle) )₂, -N(CH₂ (tétrahydropyranyle) )₂, -NH(méthyloxétanyle), - NH(tétrahydropyranyle), -NHC(O)CH₂(morpholinyle), -NHCH₂(cyclopropyle), cyclobutyle, éthoxycyclobutyle, éthoxycyclobutyle, oxétanyle, tétrahydrofuranyle, tétrahydropyranyle, dioxotétrahydrothiophényle et (oxétanylamino)pipéridinyle ;
R₃ᵢ est -CH₃ ou -CF₃;
R₃ⱼ est C₁₋₆ alkyle, -CH₂CH₂OCH₃, -CH₂CH₂ S(O)₂CH₃, -C(O)CH₂ N(CH₃)₂, -CH₂(C₃₋₄ cycloalkyle), -CH₂(tétrahydropyranyle), -CH(CH₃)(cyclopropyle), cyclobutyle, oxétanyle, tétrahydropyranyle ou isopropylpipéridinyle ;
R₃ₖ est C₁₋₄ alkyle, -CH₂C(CH₃)₂OH, -CH₂CH₂OCH₃, -CH₂C(O)NRₓRₓ, -C(O)CH₃, -C(O)CH₂CH(CH₃)OH, -C(O)CH₂ N(CH₃)₂, -NRₓ(C₁₋₃ alkyle), -NRₓ(C₃₋₄fluoroalkyle), NRₓ(CH₂CH₂OCH₃), -N(CH₃)(CH₂CH₂S(O)₂CH₃ -N(CH₃)(CH₂C(O)N(CH₃)₂), -NRₓ(C(O)CH₂ N(CH₃)₂), -N(CH₂CH₂CH₂CF₃)₂, -NRₓ(oxétanyle), -N(CH₃)(méthyloxétanyle), -N(CH₃)(tétrahydropyranyle), - NH(éthoxycyclobutyle), oxétanyle ou isopropylpipéridinyle ; et
R₅ est l'hydrogène ;
chaque Rₓ est indépendamment H ou -CH₃ ;
chaque R_{y} est indépendamment H ou C₁₋₆ alkyle ; et
p est zéro, 1 ou 2.

2. Composé selon la revendication 1, N-oxyde ou sel de celui-ci, dans lequel A est :
(i) -CH₂ N(CH₃)Rₓ, -C(O)NRₓRₓ, -C(O)N(CH₃)(CH₂CH₂CN), -C(O)N(CH₂CH₃)(CH₂CH₂CN), ou -C(O)N(CH₃)(CH₂CH₂CH₂N(CH₃)₂) ; ou
(ii) -C(O)A₁, -C(O)NRₓ(CRₓRₓ)₀₋₂A₁, -CH₂NHA₁ ou -C(O)C(O)NHA₁.

3. Composé selon la revendication 1, N-oxyde ou sel de celui-ci, dans lequel Aest :
(i) cyclohexyle substitué par zéro jusqu'à 1R_{3b} ;
(ii) pipéridinyle substitué par zéro jusqu'à 1R_{3c} ;
(iii) phényle substitué par zéro jusqu'à 1R_{3 d} et zéro jusqu'à 1R₃ₑ ;
(iv) pyridinyle substitué par zéro jusqu'à 1R_{3f} et zéro jusqu'à 1R_{3g} ;
(v) pyrazinyle ou pyrimidinyle, chacun substitué par zéro jusqu'à 1R_{3f},
(vi) thiazolyle substitué par R₃ₕ et zéro jusqu'à 1R₃ᵢ ;
(vii) diazabicyclo[2.2.1]heptanyle ou diazaspiro[3.3]heptanyle, chacun substitué par zéro jusqu'à 1R₃ⱼ ; ou
(viii) benzo[d]thiazolyle, dihydroisoquinoléinyle, tétrahydronaphtyridinyle, tétrahydrobenzo[d]thiazolyle, tétrahydroimidazo[1,2-a]pyrazinyle, tétrahydroisoquinoléinyle, tétrahydroisoquinoléinyle, tétrahydronaphtalényle, tétrahydropyrazolo[1,5-a]pyrazinyl, tétrahydropyrido[4,3-d]pyrimidinyle, tétrahydrothiazolo[4,5-c]pyridinyle ou tétrahydrothiazolo[5,4-c]pyridinyle, chacun substitué par zéro jusqu'à 1R₃ₖ.

4. Composé selon la revendication 1 ou sel de celui-ci, dans lequel
G est : 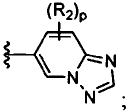
R₁ est -CH(CH₃)₂ ;
chaque R₂ est indépendamment -CH₃ ou -OCH₃ ; et
p est 1 ou 2.

5. Composé selon la revendication 4 ou sel de celui-ci, dans lequel A est :
(i) cyclohexyle substitué par zéro jusqu'à 1R_{3b} ;
(ii) phényle substitué par zéro jusqu'à 1R_{3d} et zéro jusqu'à 1R₃ₑ ;
(iii) pyridinyle substitué par zéro jusqu'à 1R_{3f} et zéro jusqu'à 1R_{3g} ; et
(iv) thiazolyle substitué par R₃ₕ et zéro jusqu'à 1R₃ᵢ.

6. Composé selon la revendication 4 ou sel de celui-ci, dans lequel :
A est -NRₓ(CRₓRₓ)₀₋₂C(O)A₁; et
A₁ est azétidinyle, cyclohexyle, pyrrolidinyle, pipéridinyle, pipérazinyle, morpholinyle, tétrahydropyranyle, pyridinyle, diazépanyle, hexahydropyrrolo[3,4-c]pyrrolyle, chacun substitué par zéro jusqu'à 1R₃ₐ.

7. Composé selon la revendication 1, N-oxyde ou sel de celui-ci, dans lequel est :
tert-butyl 4-(4-isopropyl-5-(8-méthyl-[1,2,4]triazolo[1,5-a]pyridin-6-yl)-1H-pyrazole-3-carboxamido)piperidine-1-carboxylate (1) ;
4-isopropyl-N-(1-isopropylpipéridin-4-yl)-5-(8-méthyl-[1,2,4]triazolo[1,5-a]pyridin-6-yl)-1H-pyrazole-3-carboxamide (2) ;
4-isopropyl-5-(8-méthyl-[1,2,4]triazolo[1,5-a]pyridin-6-yl)-N-(2-morpholinoéthyl)-1H-pyrazole-3-carboxamide (3) ;
4-isopropyl-5-(8-méthyl-[1,2,4]triazolo[1,5-a]pyridin-6-yl)-N-(2-(pyridin-2-yl)éthyl)-1H-pyrazole-3-carboxamide (4) ;
4-isopropyl-N-(2-méthyl-2-morpholinopropyl)-5-(8-méthyl-[1,2,4]triazolo[1,5-a]pyridin-6-yl)-1H-pyrazole-3-carboxamide (5) ;
4-isopropyl-N-méthyl-5-(8-méthyl-[1,2,4]triazolo[1,5-a]pyridin-6-yl)-N-(1-méthylpipéridin-4-yl)-1H-pyrazole-3-carboxamide (6) ;
N-(4-(diméthylamino)cyclohexyl)-4-isopropyl-5-(8-méthyl-[1,2,4]triazolo[1,5-a]pyridin-6-yl)-1H-pyrazole-3-carboxamide (7) ;
4-isopropyl-5-(8-méthyl-[1,2,4]triazolo[1,5-a]pyridin-6-yl)-N-(1-méthylpipéridin-4-yl)-1H-pyrazole-3-carboxamide (8) ;
4-isopropyl-5-(8-méthoxy-[1,2,4]triazolo[1,5-a]pyridin-6-yl)-N-méthyl-N-(1-méthylpipéridin-4-yl)-1H-pyrazole-3-carboxamide (9) ;
(4-isopropyl-5-(8-méthoxy-[1,2,4]triazolo[1,5-a]pyridin-6-yl)-1H-pyrazol-3-yl)(4-méthyl-1,4-diazépan-1-yl)méthanone (10) ;
4-isopropyl-5-(8-méthoxy-[1,2,4]triazolo[1,5-a]pyridin-6-yl)-N-méthyl-N-(1-méthylpipéridin-4-yl)-1H-pyrazole-3-carboxamide (11) ;
4-isopropyl-N-(1-isopropylpipéridin-4-yl)-5-(8-méthoxy-[1,2,4]triazolo[1,5-a]pyridin-6-yl)-1H-pyrazole-3-carboxamide (12) ;
(4-(diméthylamino)pipéridin-1-yl)(4-isopropyl-5-(8-méthyl-[1,2,4]triazolo[1,5-a]pyridin-6-yl)-1H-pyrazol-3-yl)méthanone (13) ;
(4-isopropyl-5-(8-méthyl-[1,2,4]triazolo[1,5-a]pyridin-6-yl)-1H-pyrazol-3-yl)(4-(pyridin-4-yl)pipérazin-1-yl)méthanone (14) ;
1-(4-isopropyl-5-(8-méthyl-[1,2,4]triazolo[1,5-a]pyridin-6-yl)-1H-pyrazole-3-carbonyl)pipéridine-4-carboxamide (15) ;
N-(2-cyanoéthyl)-4-isopropyl-N-méthyl-5-(8-méthyl-[1,2,4]triazolo[1,5-a]pyridin-6-yl)-1H-pyrazole-3-carboxamide (16) ;
4-isopropyl-N-méthyl-5-(8-méthyl-[1,2,4]triazolo[1,5-a]pyridin-6-yl)-N-(pyridin-3-ylméthyl)-1H-pyrazole-3-carboxamide (17) ;
(R)-(3-(diméthylamino)pyrrolidin-1-yl)(4-isopropyl-5-(8-méthyl-[1,2,4]triazolo[1,5-a]pyridin-6-yl)-1H-pyrazol-3-yl)méthanone (18) ;
3-(4-(4-isopropyl-5-(8-méthyl-[1,2,4]triazolo[1,5-a]pyridin-6-yl)-1H-pyrazole-3-carbonyl)pipérazin-1-yl)propanenitrile (19) ;
4-isopropyl-5-(8-méthoxy-[1,2,4]triazolo[1,5-a]pyridin-6-yl)-N-méthyl-N-(1-méthylpipéridin-4-yl)-1H-pyrazole-3-carboxamide (20) ;
(3-(diméthylamino)azétidin-1-yl)(4-isopropyl-5-(8-méthyl-[1,2,4]triazolo[1,5-a]pyridin-6-yl)-1H-pyrazol-3-yl)méthanone (21) ;
(S)-(3-(diméthylamino)pyrrolidin-1-yl)(4-isopropyl-5-(8-méthyl-[1,2,4]triazolo[1,5-a]pyridin-6-yl)-1H-pyrazol-3-yl)méthanone (22) ;
(4-isopropyl-5-(8-méthyl-[1,2,4]triazolo[1,5-a]pyridin-6-yl)-1H-pyrazol-3-yl)(4-méthyl-1,4-diazépan-1-yl)méthanone (23) ;
N-(3-(diméthylamino)propyl)-4-isopropyl-N-méthyl-5-(8-méthyl-[1,2,4]triazolo[1,5-a]pyridin-6-yl)-1H-pyrazole-3-carboxamide (24) ;
4-isopropyl-N-méthyl-5-(8-méthyl-[1,2,4]triazolo[1,5-a]pyridin-6-yl)-N-(1-méthylpyrrolidin-3-yl)-1H-pyrazole-3-carboxamide (25) ;
4-isopropyl-5-(8-méthoxy-[1,2,4]triazolo[1,5-a]pyridin-6-yl)-N-(2-méthyl-2-morpholinopropyl)-1H-pyrazole-3-carboxamide (26) ;
4-isopropyl-N-méthyl-5-(8-méthyl-[1,2,4]triazolo[1,5-a]pyridin-6-yl)-1H-pyrazole-3-carboxamide (27) ;
4-isopropyl-5-(8-méthyl-[1,2,4]triazolo[1,5-a]pyridin-6-yl)-N-(tétrahydro-2H-pyran-4-yl)-1H-pyrazole-3-carboxamide (28) ;
4-isopropyl-5-(8-méthyl-[1,2,4]triazolo[1,5-a]pyridin-6-yl)-1H-pyrazole-3-carboxamide (29) ;
(4-isopropyl-5-(8-méthyl-[1,2,4]triazolo[1,5-a]pyridin-6-yl)-1H-pyrazol-3-yl)(morpholino)méthanone (30) ;
4-isopropyl-N,N-diméthyl-5-(8-méthyl-[1,2,4]triazolo[1,5-a]pyridin-6-yl)-1H-pyrazole-3-carboxamide (31) ;
N-(4-hydroxycyclohexyl)-4-isopropyl-5-(8-méthyl-[1,2,4]triazolo[1,5-a]pyridin-6-yl)-1H-pyrazole-3-carboxamide (32) ;
5-(7,8-diméthyl-[1,2,4]triazolo[1,5-a]pyridin-6-yl)-4-isopropyl-N-(1-isopropylpipéridin-4-yl)-1H-pyrazole-3-carboxamide (33) ;
5-(3,4-diméthoxyphényl)-4-isopropyl-N-(1-isopropylpipéridin-4-yl)-1H-pyrazole-3-carboxamide (34) ;
4-isopropyl-N-(1-isopropylpipéridin-4-yl)-5-(1H-pyrazolo[3,4-b]pyridin-4-yl)-1H-pyrazole-3-carboxamide (35) ;
5-(2,6-diméthylpyridin-4-yl)-4-isopropyl-N-(1-isopropylpipéridin-4-yl)-1H-pyrazole-3-carboxamide (36) ;
4-isopropyl-N-(1-isopropylpipéridin-4-yl)-5(8-méthylimidazo[1,2-a]pyridin-6-yl)-1H-pyrazole-3-carboxamide (37) ;
4-isopropyl-N-(1-isopropylpipéridin-4-yl)-5-(2-méthylpyridin-4-yl)-1H-pyrazole-3-carboxamide (38) ;
5-(1,5-diméthyl-6-oxo-1,6-dihydropyridin-3-yl)-4-isopropyl-N-(1-isopropylpipéridin-4-yl)-1H-pyrazole-3-carboxamide (39) ;
5-(8-cyanoquinolin-5-yl)-4-isopropyl-N-(1-isopropylpipéridin-4-yl)-1H-pyrazole-3-carboxamide (40) ;
4-isopropyl-5-(1-méthyl-1H-pyrrolo[2,3-b]pyridin-3-yl)-N-(1-méthylpipéridin-4-yl)-1H-pyrazole-3-carboxamide (41) ;
4-isopropyl-N-(1-isopropylpipéridin-4-yl)-5-(1-méthyl-1H-pyrrolo[2,3-b]pyridin-3-yl)-1H-pyrazole-3-carboxamide (42);
4-isopropyl-5-(8-méthyl-[1,2,4]triazolo[1,5-a]pyridin-6-yl)-N-(pipéridin-4-yl)-1H-pyrazole-3-carboxamide (43) ;
4-isopropyl-5-(8-méthyl-[1,2,4]triazolo[1,5-a]pyridin-6-yl)-N-(pipéridin-4-ylméthyl)-1H-pyrazole-3-carboxamide (45) ;
N-((1r,4r)-4-aminocyclohexyl)-4-isopropyl-5-(8-méthyl-[1,2,4]triazolo[1,5-a]pyridin-6-yl)-1H-pyrazole-3-carboxamide (46) ;
N-(((1r,4r)-4-aminocyclohexyl)méthyl)-4-isopropyl-5-(8-méthyl-[1,2,4]triazolo[1,5-a]pyridin-6-yl)-1H-pyrazole-3-carboxamide (47) ;
4-isopropyl-5-(8-méthyl-[1,2,4]triazolo[1,5-a]pyridin-6-yl)-N-(morpholin-2-ylméthyl)-1H-pyrazole-3-carboxamide (48) ;
N-(azétidin-3-ylméthyl)-4-isopropyl-5-(8-méthyl-[1,2,4]triazolo[1,5-a]pyridin-6-yl)-1H-pyrazole-3-carboxamide (49) ;
(R)-4-isopropyl-5-(8-méthyl-[1,2,4]triazolo[1,5-a]pyridin-6-yl)-N-(pyrrolidin-3-yl)-1H-pyrazole-3-carboxamide (50) ;
(S)-4-isopropyl-5-(8-méthyl-[1,2,4]triazolo[1,5-a]pyridin-6-yl)-N-(pyrrolidin-2-ylméthyl)-1H-pyrazole-3-carboxamide (51) ;
(R)-4-isopropyl-5-(8-méthyl-[1,2,4]triazolo[1,5-a]pyridin-6-yl)-N-(pipéridin-3-yl)-1H-pyrazole-3-carboxamide (52) ;
(S)-(3-aminopyrrolidin-1-yl)(4-isopropyl-5-(8-méthyl-[1,2,4]triazolo[1,5-a]pyridin-6-yl)-1H-pyrazol-3-yl)méthanone (53) ;
(S)-4-isopropyl-5-(8-méthyl-[1,2,4]triazolo[1,5-a]pyridin-6-yl)-N-(pipéridin-3-yl)-1H-pyrazole-3-carboxamide (54) ;
(4-aminopipéridin-1-yl)(4-isopropyl-5-(8-méthyl-[1,2,4]triazolo[1,5-a]pyridin-6-yl)-1H-pyrazol-3-yl)methanone (55) ;
4-isopropyl-5-(8-méthyl-[1,2,4]triazolo[1,5-a]pyridin-6-yl)-N-(pipéridin-2-ylméthyl)-1H-pyrazole-3-carboxamide (56) ;
(4-isopropyl-5-(8-méthyl-[1,2,4]triazolo[1,5-a]pyridin-6-yl)-1H-pyrazol-3-yl)(pipérazin-1-yl)méthanone (57) ;
N-(azétidin-3-yl)-4-isopropyl-5-(8-méthyl-[1,2,4]triazolo[1,5-a]pyridin-6-yl)-1H-pyrazole-3-carboxamide (58) ;
(hexahydropyrrolo[3,4-c]pyrrol-2(1H)-yl)(4-isopropyl-5-(8-méthyl-[1,2,4]triazolo[1,5-a]pyridin-6-yl)-1H-pyrazol-3-yl)méthanone (59) ;
4-isopropyl-5-(8-méthoxy-[1,2,4]triazolo[1,5-a]pyridin-6-yl)-N-(pipéridin-4-yl)-1H-pyrazole-3-carboxamide (60) ;
(4-(aminométhyl)pipéridin-1-yl)(4-isopropyl-5-(8-méthyl-[1,2,4]triazolo[1,5-a]pyridin-6-yl)-1H-pyrazol-3-yl)méthanone (61) ;
4-isopropyl-5-(8-méthoxy-[1,2,4]triazolo[1,5-a]pyridin-6-yl)-N-(pipéridin-4-ylméthyl)-1H-pyrazole-3-carboxamide (62) ;
N-((1s,4s)-4-aminocyclohexyl)-4-isopropyl-5-(8-méthyl-[1,2,4]triazolo[1,5-a]pyridin-6-yl)-1H-pyrazole-3-carboxamide (63) ;
4-isopropyl-5-(8-méthyl-[1,2,4]triazolo[1,5-a]pyridin-6-yl)-N-(morpholin-2-ylméthyl)-1H-pyrazole-3-carboxamide (64) ;
4-isopropyl-5-(8-méthyl-[1,2,4]triazolo[1,5-a]pyridin-6-yl)-N-(morpholin-2-ylméthyl)-1H-pyrazole-3-carboxamide (65) ;
4-isopropyl-5-(8-méthyl-[1,2,4]triazolo[1,5-a]pyridin-6-yl)-N-(pipéridin-3-yl)-1H-pyrazole-3-carboxamide (66) ;
4-isopropyl-5-(8-méthyl-[1,2,4]triazolo[1,5-a]pyridin-6-yl)-N-(pipéridin-3-yl)-1H-pyrazole-3-carboxamide (67) ;
4-isopropyl-5-(1-méthyl-1H-pyrrolo[2,3-b]pyridin-3-yl)-N-(pipéridin-4-yl)-1H-pyrazole-3-carboxamide (68) ;
4-isopropyl-5-(8-méthyl-[1,2,4]triazolo[1,5-a]pyridin-6-yl)-N-(1-(3,3,3-trifluoropropyl) pipéridin-4-yl)-1H-pyrazole-3-carboxamide (69) ;
4-isopropyl-5-(8-méthyl-[1,2,4]triazolo[1,5-a]pyridin-6-yl)-N-(1-(oxétan-3-yl)pipéridin-4-yl)-1H-pyrazole-3-carboxamide (70) ;
4-isopropyl-5-(8-méthyl-[1,2,4]triazolo[1,5-a]pyridin-6-yl)-N-(1-(4,4,4-trifluorobutyl)pipéridin-4-yl)-1H-pyrazole-3-carboxamide (71) ;
4-isopropyl-5-(8-méthyl-[1,2,4]triazolo[1,5-a]pyridin-6-yl)-N-(1-((tétrahydrofuran-3-yl)méthyl)pipéridin-4-yl)-1H-pyrazole-3-carboxamide (72) ;
N-(1-(cyclopropylméthyl)pipéridin-4-yl)-4-isopropyl-5-(8-méthyl-[1,2,4]triazolo[1,5-a]pyridin-6-yl)-1H-pyrazole-3-carboxamide (73) ;
4-isopropyl-5-(8-méthyl-[1,2,4]triazolo[1,5-a]pyridin-6-yl)-N-(1-néopentylpipéridin-4-yl)-1H-pyrazole-3-carboxamide (74) ;
N-(1-(3,3-diméthylbutyl)pipéridin-4-yl)-4-isopropyl-5-(8-éethyl-[1,2,4]triazolo[1,5-a]pyridin-6-yl)-1H-pyrazole-3-carboxamide (75) ;
N-(1-isopentylpipéridin-4-yl)-4-isopropyl-5-(8-méthyl-[1,2,4]triazolo[1,5-a]pyridin-6-yl)-1H-pyrazole-3-carboxamide (76) ;
4-isopropyl-5-(8-méthyl-[1,2,4]triazolo[1,5-a]pyridin-6-yl)-N-(1-((tétrahydro-2H-pyran-4-yl)méthyl)pipéridin-4-yl)-1H-pyrazole-3-carboxamide (77) ;
N-(1-((3,5-diméthylisoxazol-4-yl)méthyl)pipéridin-4-yl)-4-isopropyl-5-(8-méthyl-[1,2,4]triazolo[1,5-a]pyridin-6-yl)-1H-pyrazole-3-carboxamide (78) ;
N-(1-(2-éthylbutyl)pipéridin-4-yl)-4-isopropyl-5-(8-méthyl-[1,2,4]triazolo[1,5-a]pyridin-6-yl)-1H-pyrazole-3-carboxamide (79) ;
4-isopropyl-5-(8-méthyl-[1,2,4]triazolo[1,5-a]pyridin-6-yl)-N-((1r,4r)-4-(oxétan-3-ylamino)cyclohexyl)-1H-pyrazole-3-carboxamide (80) ;
4-isopropyl-N-((1r,4r)-4-(isopropylamino)cyclohexyl)-5-(8-méthyl-[1,2,4]triazolo[1,5-a]pyridin-6-yl)-1H-pyrazole-3-carboxamide (81) ;
N-((1r,4r)-4-(diméthylamino)cyclohexyl)-4-isopropyl-5-(8-méthyl-[1,2,4]triazolo[1,5-a]pyridin-6-yl)-1H-pyrazole-3-carboxamide (82) ;
N-(1-(2-(diméthylamino)éthyl)pipéridin-4-yl)-4-isopropyl-5-(8-méthyl-[1,2,4]triazolo[1,5-a]pyridin-6-yl)-1H-pyrazole-3-carboxamide (83) ;
N-((1s,4s)-4-(diméthylamino)cyclohexyl)-4-isopropyl-5-(8-méthyl-[1,2,4]triazolo[1,5-a]pyridin-6-yl)-1H-pyrazole-3-carboxamide (84) ;
4-isopropyl-N-((1s,4s)-4-(isopropylamino)cyclohexyl)-5-(8-méthyl-[1,2,4]triazolo[1,5-a]pyridin-6-yl)-1H-pyrazole-3-carboxamide (85) ;
4-isopropyl-5-(8-méthyl-[1,2,4]triazolo[1,5-a]pyridin-6-yl)-N-((1s,4s)-4-(oxétan-3-ylamino)cyclohexyl)-1H-pyrazole-3-carboxamide (86) ;
4-isopropyl-5-(8-méthyl-[1,2,4]triazolo[1,5-a]pyridin-6-yl)-N-((1s,4s)-4-(((tétrahydrofuran-3-yl)méthyl)amino)cyclohexyl)-1H-pyrazole-3-carboxamide (87) ;
4-isopropyl-5-(8-méthyl-[1,2,4]triazolo[1,5-a]pyridin-6-yl)-N-((1s,4s)-4-((4,4,4-trifluorobutyl)amino)cyclohexyl)-1H-pyrazole-3-carboxamide (88) ;
4-isopropyl-5-(8-méthyl-[1,2,4]triazolo[1,5-a]pyridin-6-yl)-N-((1s,4s)-4-(neopentylamino)cyclohexyl)-1H-pyrazole-3-carboxamide (89) ;
N-((1s, 4s)-4-(bis(3,3-diméthylbutyl)amino)cyclohexyl)-4-isopropyl-5-(8-méthyl-[1,2,4]triazolo[1,5-a]pyridin-6-yl)-1H-pyrazole-3-carboxamide (90) ;
N-((1s,4s)-4-(diisopentylamino)cyclohexyl)-4-isopropyl-5-{8-méthyl-[1,2,4]triazolo[1,5-a]pyridin-6-yl)-1H-pyrazole-3-carboxamide (91) ;
4-isopropyl-5-(8-méthyl-[1,2,4]triazolo[1,5-a]pyridin-6-yl)-N-((1s,4s)-4-(((tétrahydro-2H-pyran-4-yl)méthyl)amino)cyclohexyl)-1H-pyrazole-3-carboxamide (92) ;
N-((1s,4s)-4-(((3,5-diméthylisoxazol-4-yl)méthyl)amino)cyclohexyl)-4-isopropyl-5-(8-méthyl-[1,2,4]triazolo[1,5-a]pyridin-6-yl)-1H-pyrazole-3-carboxamide (93) ;
N-((1s,4s)-4-((cyclopentylméthyl)amino)cyclohexyl)-4-isopropyl-5-(8-méthyl-[1,2,4]triazolo[1,5-a]pyridin-6-yl)-1H-pyrazole-3-carboxamide (94) ;
4-isopropyl-5-(8-méthyl-[1,2,4]triazolo[1,5-a]pyridin-6-yl)-N-((1s,4s)-4-((pyridin-3-ylméthyl)amino)cyclohexyl)-1H-pyrazole-3-carboxamide (95) ;
4-isopropyl-N-((1s,4s)-4-(((2-méthoxypyrimidin-5-yl)méthyl)amino)cyclohexyl)-5-(8-méthyl-[1,2,4]triazolo[1,5-a]pyridin-6-yl)-1H-pyrazole-3-carboxamide (96) ;
N-((1s,4s)-4-((2,2-diméthyltétrahydro-2H-pyran-4-yl)amino)cyclohexyl)-4-isopropyl-5-(8-méthyl-[1,2,4]triazolo[1,5-a]pyridin-6-yl)-1H-pyrazole-3-carboxamide (97) ;
4-isopropyl-5-(8-méthyl-[1,2,4]triazolo[1,5-a]pyridin-6-yl)-N-((1s,4s)-4-((tétrahydro-2H-pyran-4-yl)amino)cyclohexyl)-1H-pyrazole-3-carboxamide (98) ;
4-isopropyl-5-(8-méthyl-[1,2,4]triazolo[1,5-a]pyridin-6-yl)-N-((1s,4s)-4-((3-méthylbutan-2-yl)amino)cyclohexyl)-1H-pyrazole-3-carboxamide (99) ;
4-isopropyl-5-(8-méthyl-[1,2,4]triazolo[1,5-a]pyridin-6-yl)-N-((1s,4s)-4-(pentan-3-ylamino)cyclohexyl)-1H-pyrazole-3-carboxamide (100) ;
N-((1s,4s)-4-((2-éthylbutyl)amino)cyclohexyl)-4-isopropyl-5-(8-méthyl-[1,2,4]triazolo[1,5-a]pyridin-6-yl)-1H-pyrazole-3-carboxamide (101) ;
4-isopropyl-5-(8-méthyl-[1,2,4]triazolo[1,5-a]pyridin-6-yl)-N-((1s,4s)-4-((pyrimidin-5-ylméthyl)amino)cyclohexyl)-1H-pyrazole-3-carboxamide (102) ;
N-((1r,4r)-4-(bis(cyclopropylméthyl)amino)cyclohexyl)-4-isopropyl-5-(8-méthyl-[1,2,4]triazolo[1,5-a]pyridin-6-yl)-1H-pyrazole-3-carboxamide (103) ;
4-isopropyl-5-(8-méthyl-[1,2,4]triazolo[1,5-a]pyridin-6-yl)-N-((1r,4r)-4-(((2-méthylpyrimidin-5-yl)méthyl)amino)cyclohexyl)-1H-pyrazole-3-carboxamide (104) ;
4-isopropyl-5-(8-méthyl-[1,2,4]triazolo[1,5-a]pyridin-6-yl)-N-((1r,4r)-4-(((tétrahydrofuran-3-yl)méthyl)amino)cyclohexyl)-1H-pyrazole-3-carboxamide (105) ;
4-isopropyl-5-(8-méthyl-[1,2,4]triazolo[1,5-a]pyridin-6-yl)-N-((1r,4r)-4-(néopentylamino)cyclohexyl)-1H-pyrazole-3-carboxamide
N-((1r,4r)-4-(bis(3,3-diméthylbutyl)amino)cyclohexyl)-4-isopropyl-5-(8-méthyl-[1,2,4]triazolo[1,5-a]pyridin-6-yl)-1H-pyrazole-3-carboxamide (107) ;
N-((1r,4r)-4-(diisopentylamino)cyclohexyl)-4-isopropyl-5-(8-méthyl-[1,2,4]triazolo[1,5-a]pyridin-6-yl)-1H-pyrazole-3-carboxamide (108) ;
N-((1r,4r)-4-((cyclopentylméthyl)amino)cyclohexyl)-4-isopropyl-5-(8-méthyl-[1,2,4]triazolo[1,5-a]pyridin-6-yl)-1H-pyrazole-3-carboxamide (109) ;
N-((1r,4r)-4-((2-éthylbutyl)amino)cyclohexyl)-4-isopropyl-5-(8-méthyl-[1,2,4]triazolo[1,5-a]pyridin-6-yl)-1H-pyrazole-3-carboxamide (110) ;
4-isopropyl-5-(8-méthyl-[1,2,4]triazolo[1,5-a]pyridin-6-yl)-N-((1r,4r)-4-(((tétrahydro-2H-pyran-4-yl)méthyl)amino)cyclohexyl)-1H-pyrazole-3-carboxamide (111) ;
4-isopropyl-5-(8-méthyl-[1,2,4]triazolo[1,5-a]pyridin-6-yl)-N-((1r,4r)-4-((pyridin-3-ylméthyl)amino)cyclohexyl)-1H-pyrazole-3-carboxamide (112) ;
4-isopropyl-5-(8-méthyl-[1,2,4]triazolo[1,5-a]pyridin-6-yl)-N-((1r,4r)-4-((pyrimidin-5-ylméthyl)amino)cyclohexyl)-1H-pyrazole-3-carboxamide (113) ;
4-isopropyl-N-((1r,4r)-4-(((2-méthoxypyrimidin-5-yl)méthyl)amino)cyclohexyl)-5-(8-méthyl-[1,2,4]triazolo[1,5-a]pyridin-6-yl)-1H-pyrazole-3-carboxamide (114) ;
N-((1r,4r)-4-((2,2-diméthyltétrahydro-2H-pyran-4-yl)amino)cyclohexyl)-4-isopropyl-5-(8-méthyl-[1,2,4]triazolo[1,5-a]pyridin-6-yl)-1H-pyrazole-3-carboxamide (115) ;
4-isopropyl-5-(8-méthyl-[1,2,4]triazolo[1,5-a]pyridin-6-yl)-N-((1r,4r)-4-((tétrahydro2 H -pyran-4-yl)amino)cyclohexyl)-1H-pyrazole-3-carboxamide (116) ;
4-isopropyl-5-(8-méthyl-[1,2,4]triazolo[1,5-a]pyridin-6-yl)-N-((1r,4r)-4-((3-méthylbutan-2-yl)amino)cyclohexyl)-1H-pyrazole-3-carboxamide (118) ;
4-isopropyl-5-(8-méthyl-[1,2,4]triazolo[1,5-a]pyridin-6-yl)-N-((1r,4r)-4-(pentan-3-ylamino)cyclohexyl)-1H-pyrazole-3-carboxamide (119) ;
N-((1r,4r)-4-((4,4-diméthylpentan-2-yl)amino)cyclohexyl)-4-isopropyl-5-(8-méthyl-[1,2,4]triazolo[1,5-a]pyridin-6-yl)-1H-pyrazole-3-carboxamide (120) ;
4-isopropyl-5-(8-méthyl-[1,2,4]triazolo[1,5-a]pyridin-6-yl)-N-(4-((3-m"thyloxétan-3-yl)amino)cyclohexyl)-1H-pyrazole-3-carboxamide (123-124 ;
4-isopropyl-5-(8-méthyl-[1,2,4]triazolo[1,5-a]pyridin-6-yl)-N-(1-(3-méthylbutan-2-yl)pipéridin-4-yl)-1H-pyrazole-3-carboxamide (125) ;
4-isopropyl-5-(8-méthyl-[1,2,4]triazolo[1,5-a]pyridin-6-yl)-N-(4-(neopentylamino)cyclohexyl)-1H-pyrazole-3-carboxamide (126 et 131) ;
4-isopropyl-N-(4-(isopropyl(méthyl)amino)cyclohexyl)-5-(8-méthyl-[1,2,4]triazolo[1,5-a]pyridin-6-yl)-1H-pyrazole-3-carboxamide (127 et 132) ;
4-isopropyl-N-(4-((2-méthoxyethyl)(méthyl)amino)cyclohexyl)-5-(8-méthyl-[1,2,4]triazolo[1,5-a]pyridin-6-yl)-1H-pyrazole-3-carboxamide (128) ;
4-isopropyl-5-(8-méthyl-[1,2,4]triazolo[1,5-a]pyridin-6-yl)-N-(4-(méthylamino)cyclohexyl)-1H-pyrazole-3-carboxamide (129-130) ;
4-isopropyl-5-(8-méthyl-[1,2,4]triazolo[1,5-a]pyridin-6-yl)-N-(1-((3-méthyloxetan-3-yl)méthyl)pipéridin-4-yl)-1H-pyrazole-3-carboxamide (133) ;
4-isopropyl-5-(8-méthyl-[1,2,4]triazolo[1,5-a]pyridin-6-yl)-N-((4-méthylmorpholin-2-yl)méthyl)-1H-pyrazole-3-carboxamide
4-isopropyl-N-((4-isopropylmorpholin-2-yl)méthyl)-5-(8-méthyl-[1,2,4]triazolo[1,5-a]pyridin-6-yl)-1H-pyrazole-3-carboxamide (135) ;
4-isopropyl-5-(8-méthyl-[1,2,4]triazolo[1,5-a]pyridin-6-yl)-N-((4-(oxétan-3-yl)morpholin-2-yl)méthyl)-1H-pyrazole-3-carboxamide (136 et 142) ;
4-isopropyl-5-(8-méthyl-[1,2,4]triazolo[1,5-a]pyridin-6-yl)-N-((4-((3-méthyloxétan-3-yl)méthyl)morpholin-2-yl)méthyl)-1H-pyrazole-3-carboxamide (137) ;
N-(1-(2-hydroxy-2-méthylpropyl)pipéridin-4-yl)-4-isopropyl-5-(8-méthyl-[1,2,4]triazolo[1,5-a]pyridin-6-yl)-1H-pyrazole-3-carboxamide (138) ;
4-isopropyl-5-(8-méthyl-[1,2,4]triazolo[1,5-a]pyridin-6-yl)-N-((4-méthylmorpholin-2-yl)méthyl)-1H-pyrazole-3-carboxamide (139) ;
N-((4-éthylmorpholin-2-yl)méthyl)-4-isopropyl-5-(8-méthyl-[1,2,4]triazolo[1,5-a]pyridin-6-yl)-1H-pyrazole-3-carboxamide (140) ;
4-isopropyl-N-((4-isopropylmorpholin-2-yl)méthyl)-5-(8-méthyl-[1,2,4]triazolo[1,5-a]pyridin-6-yl)-1H-pyrazole-3-carboxamide (141) ;
N-((4-(2-hydroxy-2-méthylpropyl)morpholin-2-yl)méthyl)-4-isopropyl-5-(8-méthyl-[1,2,4]triazolo[1,5-a]pyridin-6-yl)-1H-pyrazole-3-carboxamide (143) ;
4-isopropyl-5-(8-méthyl-[1,2,4]triazolo[1,5-a]pyridin-6-yl)-N-((4-néopentylmorpholin-2-yl)méthyl)-1H-pyrazole-3-carboxamide (144-145) ;
4-isopropyl-N-((1r,4r)-4-((1-méthoxypropan-2-yl)amino)cyclohexyl)-5-(8-méthyl-[1,2,4]triazolo[1,5-a]pyridin-6-yl)-1H-pyrazole-3-carboxamide (146) ;
4-isopropyl-N-((1s,4s)-4-((1-méthoxypropan-2-yl)amino)cyclohexyl)-5-(8-méthyl-[1,2,4]triazolo[1,5-a]pyridin-6-yl)-1H-pyrazole-3-carboxamide (147) ;
N-((1r,4r)-4-((3,3-diméthylbutan-2-yl)amino)cyclohexyl)-4-isopropyl-5-(8-méthyl-[1,2,4]triazolo[1,5-a]pyridin-6-yl)-1H-pyrazole-3-carboxamide (148) ;
4-isopropyl-5-(8-méthyl-[1,2,4]triazolo[1,5-a]pyridin-6-yl)-N-((1r,4r)-4-(((3-méthyloxétan-3-yl)méthyl)amino)cyclohexyl)-1H-pyrazole-3-carboxamide (149) ;
N-((1s,4s)-4-((3,3-diméthylbutan-2-yl)amino)cyclohéxyl)-4-isopropyl-5-(8-méthyl-[1,2,4]triazolo[1,5-a]pyridin-6-yl)-1H-pyrazole-3-carboxamide (150);
4-isopropyl-5-(8-méthyl-[1,2,4]triazolo[1,5-a]pyridin-6-yl)-N-((1s,4s)-4-(((3-méthyloxétan-3-yl)méthyl)amino)cyclohexyl)-1H-pyrazole-3-carboxamide (151) ;
N-(1-(sec-butyl)pipéridin-4-yl)-4-isopropyl-5-(8-méthyl-[1,2,4]triazolo[1,5-a]pyridin-6-yl)-1H-pyrazole-3-carboxamide (152) ;
4-isopropyl-5-(8-méthyl-[1,2,4]triazolo[1,5-a]pyridin-6-yl)-N-(1-(tétrahydro-2H-pyran-4-yl)pipéridin-4-yl)-1H-pyrazole-3-carboxamide (153) ;
4-isopropyl-5-(8-méthyl-[1,2,4]triazolo[1,5-a]pyridin-6-yl)-N-(1-((3-méthyloxétan-3-yl)méthyl)azétidin-3-yl)-1H-pyrazole-3-carboxamide (154) ;
4-isopropyl-N-(1-isopropylazétidin-3-yl)-5-(8-méthyl-[1,2,4]triazolo[1,5-a]pyridin-6-yl)-1H-pyrazole-3-carboxamide (155) ;
4-isopropyl-5-(8-méthyl-[1,2,4]triazolo[1,5-a]pyridin-6-yl)-N-(1-méthylazétidin-3-yl)-1H-pyrazole-3-carboxamide (156) ;
4-isopropyl-N-(1-((2-méthoxypyrimidin-5-yl)méthyl)pipéridin-4-yl)-5-(8-méthyl-[1,2,4]triazolo[1,5-a]pyridin-6-yl)-1H-pyrazole-3-carboxamide (157) ;
4-isopropyl-N-(1-((1-méthyl-1H-1,2,4-triazol-3-yl)méthyl)pipéridin-4-yl)-5-(8-méthyl-[1,2,4]triazolo[1,5-a]pyridin-6-yl)-1H-pyrazole-3-carboxamide (158) ;
4-isopropyl-N-(1-((1-méthyl-1H-1,2,4-triazol-3-yl)méthyl)azétidin-3-yl)-5-(8-méthyl-[1,2,4]triazolo[1,5-a]pyridin-6-yl)-1H-pyrazole-3-carboxamide (159) ;
(R)-4-isopropyl-5-(8-méthyl-[1,2,4]triazolo[1,5-a]pyridin-6-yl)-N-(1-méthylpiperidin-3-yl)-1H-pyrazole-3-carboxamide (160) ;
(R)-4-isopropyl-N-(1-isopropylpipéridin-3-yl)-5-(8-méthyl-[1,2,4]triazolo[1,5-a]pyridin-6-yl)-1H-pyrazole-3-carboxamide (161) ;
(S)-4-isopropyl-5-(8-méthyl-[1,2,4]triazolo[1,5-a]pyridin-6-yl)-N-(1-méthylpipéridin-3-yl)-1H-pyrazole-3-carboxamide (162) ;
(S)-4-isopropyl-N-(1-isopropylpipéridin-3-yl)-5-(8-méthyl-[1,2,4]triazolo[1,5-a]pyridin-6-yl)-1H-pyrazole-3-carboxamide (163) ;
(S)-N-(1-éthylpipéridin-3-yl)-4-isopropyl-5-(8-méthyl-[1,2,4]triazolo[1,5-a]pyridin-6-yl)-1H-pyrazole-3-carboxamide (164) ;
4-isopropyl-N-((3S)-1-((3-méthyl-3H-1,2,4-triazol-5-yl)méthyl)pipéridin-3-yl)-5-(8-méthyl-[1,2,4]triazolo[1,5-a]pyridin-6-yl)-1H-pyrazole-3-carboxamide (165) ;
(S)-4-isopropyl-5-(8-méthyl-[1,2,4]triazolo[1,5-a]pyridin-6-yl)-N-(1-(oxétan-3-yl)pipéridin-3-yl)-1H-pyrazole-3-carboxamide (166) ;
4-isopropyl-5-(1-méthyl-1H-pyrrolo[2,3-b]pyridin-3-yl)-N-(1-(oxétan-3-yl)pipéridin-4-yl)-1H-pyrazole-3-carboxamide (167) ;
4-isopropyl-5-(1-méthyl-1H-pyrrolo[2,3-b]pyridin-3-yl)-N-(1-néopentylpipéridin-4-yl)-1H-pyrazole-3-carboxamide (168) ;
4-isopropyl-5-(8-méthyl-[1,2,4]triazolo[1,5-a]pyridin-6-yl)-N-(1-(3,3,3-trifluoropropyl)pipéridin-4-yl)-1H-pyrazole-3-carboxamide (169) ;
4-isopropyl-5-(8-méthyl-[1,2,4]triazolo[1,5-a]pyridin-6-yl)-N-(1-(2-(méthylamino)ethyl)pipéridin-4-yl)-1H-pyrazole-3-carboxamide (170) ;
4-isopropyl-N-(1-(2-méthoxyethyl)pipéridin-4-yl)-5-(8-méthyl-[1,2,4]triazolo [1,5-a]pyridin-6-yl)-1H-pyrazole-3-carboxamide (171) ;
4-isopropyl-5-(8-méthyl-[1,2,4]triazolo[1,5-a]pyridin-6-yl)-N-(1-(2-(méthylsulfonyl)éthyl)pipéridin-4-yl)-1H-pyrazole-3-carboxamide (172) ;
4-isopropyl-5-(8-méthyl-[1,2,4]triazolo[1,5-a]pyridin-6-yl)-N-(1-(2,2,2-trifluoroéthyl)pipéridin-4-yl)-1H-pyrazole-3-carboxamide (173) ;
4-isopropyl-5-(8-méthyl-[1,2,4]triazolo[1,5-a]pyridin-6-yl)-N-(1-(1-(méthylsulfonyl)propan-2-yl)pipéridin-4-yl)-1H-pyrazole-3-carboxamide (174) ;
N-(1-(2-(diméthylamino)-2-oxoéthyl)pipéridin-4-yl)-4-isopropyl-5-(8-méthyl[1,2,4]triazolo [1,5-a]pyridin-6-yl)-1H-pyrazole-3-carboxamide (176) ;
4-isopropyl-5-(8-méthyl-[1,2,4]triazolo[1,5-a]pyridin-6-yl)-N-(1-(2-(méthylamino)-2-oxoéthyl)pipéridin-4-yl)-1H-pyrazole-3-carboxamide (177) ;
N-(1-isopropylpipéridin-4-yl)-4-méthyl-5-(8-méthyl-[1,2,4]triazolo[1,5-a]pyridin-6-yl)-1H-pyrazole-3-carboxamide (179) ;
4-méthyl-5-(8-méthyl-[1,2,4]triazolo[1,5-a]pyridin-6-yl)-N-(1-méthylpipéridin-4-yl)-1H-pyrazole-3-carboxamide (180) ;
4-éthyl-5-(8-méthyl-[1,2,4]triazolo[1,5-a]pyridin-6-yl)-N-(1-méthylpipéridin-4-yl)-1H-pyrazole-3-carboxamide (181) ;
4-éthyl-N-(1-isopropylpipéridin-4-yl)-5-(8-méthyl-[1,2,4]triazolo[1,5-a]pyridin-6-yl)-1H-pyrazole-3-carboxamide (182) ;
2-(4-isopropyl-5-(8-méthyl-[1,2,4]triazolo[1,5-a]pyridin-6-yl)-1H-pyrazol-3-yl)-N-(1-isopropylpipéridin-4-yl)-2-oxoacétamide (183) ;
1-(4-isopropyl-5-(8-méthyl-[1,2,4]triazolo[1,5-a]pyridin-6-yl)-1H-pyrazol-3-yl)-N,N-diméthylméthanamine (184) ;
1-(4-isopropyl-5-(8-méthyl-[1,2,4]triazolo[1,5-a]pyridin-6-yl)-1H-pyrazol-3-yl)-Nméthylméthanamine (185) ;
1-isopropyl-N-((4-isopropyl-5-(8-méthyl-[1,2,4]triazolo[1,5-a]pyridin-6-yl)-1H-pyrazol-3-yl)méthyl)pipéridin-4-amine (186) ;
N-((4-isopropyl-5-(8-méthyl-[1,2,4]triazolo[1,5-a]pyridin-6-yl)-1H-pyrazol-3-yl)méthyl)pipéridin-4-amine (187) ;
5-(8-méthoxy-[1,2,4]triazolo[1,5-a]pyridin-6-yl)-N-(pipéridin-4-yl)-4-(2,2,2-trifluoroéthyl)-1H-pyrazole-3-carboxamide (190) ;
N-(1-isopropylpipéridin-4-yl)-5-(8-méthoxy-[1,2,4]triazolo[1,5-a]pyridin-6-yl)-4-(2,2,2-trifluoroéthyl)-1H-pyrazole-3-carboxamide (191) ;
5-(8-méthoxy-[1,2,4]triazolo[1,5-a]pyridin-6-yl)-N-(1-méthylpipéridin-4-yl)-4-(2,2,2-trifluoroéthyl)-1H-pyrazole-3-carboxamide (192) ;
N-(1-isopropylpipéridin-4-yl)-5-(8-méthyl-[1,2,4]triazolo[1,5-a]pyridin-6-yl)-4-(2,2,2-trifluoroéthyl)-1H-pyrazole-3-carboxamide (193) ;
5-(8-méthyl-[1,2,4]triazolo[1,5-a]pyridin-6-yl)-N-(pipéridin-4-yl)-4-(2,2,2-trifluoroéthyl)-1H-pyrazole-3-carboxamide (194) ;
5-(8-méthoxy-[1,2,4]triazolo[1,5-a]pyridin-6-yl)-N-(1-propylpipéridin-4-yl)-4-(2,2,2-trifluoroéthyl)-1H-pyrazole-3-carboxamide (195) ;
5-(8-méthoxy-[1,2,4]triazolo[1,5-a]pyridin-6-yl)-N-(1-néopentylpipéridin-4-yl)-4-(2,2,2-trifluoroéthyl)-1H-pyrazole-3-carboxamide (196) ;
5-(8-méthoxy-[1,2,4]triazolo[1,5-a]pyridin-6-yl)-N-(1-((tétrahydro-2H-pyran-4-yl)méthyl)pipéridin-4-yl)-4-(2,2,2-trifluoroéthyl)-1H-pyrazole-3-carboxamide (197) ;
N-(1'-isopropyl-[1,4'-bipipéridin]-4-yl)-5-(8-méthoxy-[1,2,4]triazolo[1,5-a]pyridin-6-yl)-4-(2,2,2-trifluoroéthyl)-1H-pyrazole-3-carboxamide (199) ;
5-(8-méthyl-[1,2,4]triazolo[1,5-a]pyridin-6-yl)-N-(1-(oxétan-3-yl)pipéridin-4-yl)-4-(2,2,2-trifluoroéthyl)-1H-pyrazole-3-carboxamide (200) ;
5-(8-méthyl-[1,2,4]triazolo[1,5-a]pyridin-6-yl)-N-(1-(tétrahydro-2H-pyran-4-yl)pipéridin-4-yl)-4-(2,2,2-trifluoroéthyl)-1H-pyrazole-3-carboxamide (201) ;
N-(1-isobutylpipéridin-4-yl)-5-(8-méthyl-[1,2,4]triazolo[1,5-a]pyridin-6-yl)-4-(2,2,2-trifluoroéthyl)-1H-pyrazole-3-carboxamide (202) ;
N-(1-cyclobutylpipéridin-4-yl)-5-(8-méthyl-[1,2,4]triazolo[1,5-a]pyridin-6-yl)-4-(2,2,2-trifluoroéthyl)-1H-pyrazole-3-carboxamide (203) ;
5-(8-méthyl-[1,2,4]triazolo[1,5-a]pyridin-6-yl)-N-(1-((tétrahydro-2H-pyran-4-yl)méthyl)pipéridin-4-yl)-4-(2,2,2-trifluoroéthyl)-1H-pyrazole-3-carboxamide (204) ;
5-(8-méthoxy-[1,2,4]triazolo[1,5-a]pyridin-6-yl)-N-(1-(2-(méthylamino)-2-oxoéthyl)pipéridin-4-yl)-4-(2,2,2-trifluoroéthyl)-1H-pyrazole-3-carboxamide (205) ;
6-(4-isopropyl-3-(1-méthylpipéridin-4-yl)-1H-pyrazol-5-yl)-8-méthyl-[1,2,4]triazolo[1,5-a]pyridine (206) ;
6-(4-isopropyl-3-(1-isopropylpipéridin-4-yl)-1H-pyrazol-5-yl)-8-méthyl-[1,2,4]triazolo[1,5-a]pyridine (207) ;
3-(diméhylamino)-1-(4-(4-isopropyl-5-(8-méthyl-[1,2,4]triazolo[1,5-a]pyridine-6-yl)-1H-pyrazol-3-yl)pipéridin-1-yl)propan-1-one (208) ;
2-(diméthylamino)-1-(4-(4-isopropyl-5-(8-méthyl-[1,2,4]triazolo[1,5-a]pyridin-6-yl)-1H-pyrazol-3-yl)pipéridin-1-yl)éthan-1-one (209) ;
1-(4-(4-isopropyl-5-(8-méthyl-[1,2,4]triazolo[1,5-a]pyridin-6-yl)-1H-pyrazol-3-yl)pipéridin-1-yl)-3-(isopropylamino)propan-1-one (210) ;
2-(4-(4-isopropyl-5-(8-méthyl-[1,2,4]triazolo[1,5-a]pyridin-6-yl)-1H-pyrazol-3-yl)pipéridin-1-yl)-N-méthylacétamide (211) ;
2-(4-(4-isopropyl-5-(8-méthyl-[1,2,4]triazolo[1,5-a]pyridin-6-yl)-1H-pyrazol-3-yl)pipéridin-1-yl)-N,N-diméthylacétamide (212) ;
6-(4-isopropyl-5-(8-méthyl-[1,2,4]triazolo[1,5-a]pyridin-6-yl)-1H-pyrazol-3-yl)-1,2,3,4-tétrahydroisoquinoline HCl(213);
6-(4-isopropyl-5-(8-méthyl-[1,2,4]triazolo[1,5-a]pyridin-6-yl)-1H-pyrazol-3-yl)isoquinoline (214) ;
6-(4-isopropyl-5-(8-méthyl-[1,2,4]triazolo[1,5-a]pyridin-6-yl)-1H-pyrazol-3-yl)-3,4-dihydroisoquinolin-1 (2H)-one (215) ;
6-(3-(6,7-dihydro-5H-pyrrolo[3,4-d]pyrimidin-2-yl)-4-isopropyl-1H-pyrazol-5-yl)-8-méthyl-[1,2,4]triazolo[1,5-a]pyridine (216) ;
6-(4-isopropyl-3-(4,5,6,7-tetrahydropyrazolo[1,5-a]pyrazin-2-yl)-1H-pyrazol-5-yl)-8-methyl-[1,2,4]triazolo[1,5-a]pyridine (217);
2-(4-isopropyl-5-(8-méhyl-[1,2,4]triazolo[1,5-a]pyridin-6-yl)-1H-pyrazol-3-yl)-4,5,6,7-tétrahydrothiazolo[5,4-c]pyridine (218) ;
6-(4-isopropyl-3-(5,6,7,8-tétrahydroimidazo[1,2-a]pyrazin-2-yl)-1H-pyrazol-5-yl)-8-méthyl-[1,2,4]triazolo[1,5-a]pyridine (219) ;
2-(4-isopropyl-5-(8-méthoxy-[1,2,4]triazolo[1,5-a]pyridin-6-yl)-1H-pyrazol-3-yl)-4,5,6,7-tétrahydrothiazolo[5,4-c]pyridine (220) ;
2-(4-isopropyl-5-(8-méthyl-[1,2,4]triazolo[1,5-a]pyridin-6-yl)-1H-pyrazol-3-yl)-4,5,6,7-tétrahydrothiazolo[4,5-c]pyridine (221) ;
2-(4-isopropyl-5-(8-méthyl-[1,2,4]triazolo[1,5-a]pyridin-6-yl)-1H-pyrazol-3-yl)-5,6,7,8-tétrahydropyrido[4,3-d]pyrimidine (222) ;
6-(4-isopropyl-3-(pyridin-4-yl)-1H-pyrazol-5-yl)-8-méthyl-[1,2,4]triazolo[1,5a]pyridine (223) ;
6-(4-isopropyl-3-(pyridin-3-yl)-1H-pyrazol-5-yl)-8-méthyl-[1,2,4]triazolo[1,5a]pyridine (224) ;
5-(4-isopropyl-5-(8-méthyl-[1,2,4]triazolo[1,5-a]pyridin-6-yl)-1H-pyrazol-3-yl)pyridin-3-amine (225) ;
5-(4-isopropyl-5-(8-méthyl-[1,2,4]triazolo[1,5-a]pyridin-6-yl)-1H-pyrazol-3-yl)pyrazin-2-amine (226) ;
5-(4-isopropyl-5-(8-méthyl-[1,2,4]triazolo[1,5-a]pyridin-6-yl)-1H-pyrazol-3-yl)pyridin-2-amine (227) ;
5-(4-isopropyl-5-(8-méthyl-[1,2,4]triazolo[1,5-a]pyridin-6-yl)-1H-pyrazol-3-yl)pyridin-2-amine (228) ;
4-(4-isopropyl-5-(8-méthyl-[1,2,4]triazolo[1,5-a]pyridin-6-yl)-1H-pyrazol-3-yl)benzamide (229) ;
3-(4-isopropyl-5-(8-méthyl-[1,2,4]triazolo[1,5-a]pyridin-6-yl)-1H-pyrazol-3-yl)5,6,7,8-tétrahydro-1,6-naphtyridine (230) ;
7-(4-isopropyl-5-(8-méthyl-[1,2,4]triazolo[1,5-a]pyridin-6-yl)-1H-pyrazol-3-yl)-1,2,3,4-tétrahydroisoquinoline (231) ;
3-(4-isopropyl-5-(8-méthyl-[1,2,4]triazolo[1,5-a]pyridin-6-yl)-1H-pyrazol-3-yl)aniline (232) ;
4-(4-isopropyl-5-(8-meéhyl-[1,2,4]triazolo[1,5-a]pyridin-6-yl)-1H-pyrazol-3-yl)aniline (233) ;
6-(4-isopropyl-5-(8-méthoxy-[1,2,4]triazolo[1,5-a]pyridin-6-yl)-1H-pyrazol-3-yl)-1,2,3,4-tétrahydroisoquinoline (234) ;
6-(4-isopropyl-5-(8-méthyl-[1,2,4]triazolo[1,5-a]pyridin-6-yl)-1H-pyrazol-3-yl)-2-méthyl-1,2,3,4-tétrahydroisoquinoline (235) ;
5-(4-isopropyl-5-(8-méthyl-[1,2,4]triazolo[1,5-a]pyridin-6-yl)-1H-pyrazol-3-yl)-N,N-diméthylpyrazin-2-amine (236) ;
5-(4-isopropyl-5-(8-méthyl-[1,2,4]triazolo[1,5-a]pyridin-6-yl)-1H-pyrazol-3-yl)-N,N-diméthylpyridin-2-amine (237) ;
5-(4-isopropyl-5-(8-méthyl-[1,2,4]triazolo[1,5-a]pyridin-6-yl)-1H-pyrazol-3-yl)-N,N-diméthylpyridin-3-amine (238) ;
N-isopropyl-5-(4-isopropyl-5-(8-méthyl-[1,2,4]triazolo[1,5-a]pyridin-6-yl)-1H-pyrazol-3-yl)pyridin-3-amine (239) ;
N-isopropyl-5-(4-isopropyl-5-(8-méthyl-[1,2,4]triazolo[1,5-a]pyridin-6-yl)-1H-pyrazol-3-yl)pyridin-2-amine (240) ;
N-isopropyl-5-(4-isopropyl-5-(8-méthyl-[1,2,4]triazolo[1,5-a]pyridin-6-yl)-1H-pyrazol-3-yl)pyrazin-2-amine (241) ;
N-isopropyl-3-(4-isopropyl-5-(8-méthyl-[1,2,4]triazolo[1,5-a]pyridin-6-yl)-1H-pyrazol-3-yl)aniline (242) ;
4-(4-isopropyl-5-(8-méthyl-[1,2,4]triazolo[1,5-a]pyridin-6-yl)-1H-pyrazol-3-yl)-N,N-diméthylaniline (243) ;
N-isopropyl-4-(4-isopropyl-5-(8-méthyl-[1,2,4]triazolo[1,5-a]pyridin-6-yl)-1H-pyrazol-3-yl)aniline (244) ;
3-(4-isopropyl-5-(8-méthyl-[1,2,4]triazolo[1,5-a]pyridin-6-yl)-1H-pyrazol-3-yl)-N,N-diméthylaniline (245) ;
2-isopropyl-6-(4-isopropyl-5-(8-méthyl-[1,2,4]triazolo[1,5-a]pyridin-6-yl)-1H-pyrazol-3-yl)-1,2,3,4-tétrahydroisoquinoline (246) ;
6-(4-isopropyl-5-(8-méthyl-[1,2,4]triazolo[1,5-a]pyridin-6-yl)-1H-pyrazol-3-yl)-2-(oxétan-3-yl)-1,2,3,4-tétrahydroisoquinoline (247) ;
2-(4-isopropyl-5-(8-méthyl-[1,2,4]triazolo[1,5-a]pyridin-6-yl)-1H-pyrazol-3-yl)-6-méthyl-5,6,7,8-tétrahydropyrido[4,3-d]pyrimidine (248) ;
6-éthyl-2-(4-isopropyl-5-(8-méthyl-[1,2,4]triazolo[1,5-a]pyridin-6-yl)-1H-pyrazol-3-yl)-5,6,7,8-tétrahydropyrido[4,3-d]pyrimidine (249) ;
6-isopropyl-2-(4-isopropyl-5-(8-méthyl-[1,2,4]triazolo[1,5-a]pyridin-6-yl)-1H-pyrazol-3-yl)-5,6,7,8-tétrahydropyrido[4,3-d]pyrimidine (250) ;
2-(4-isopropyl-5-(8-méthyl-[1,2,4]triazolo[1,5-a]pyridin-6-yl)-1H-pyrazol-3-yl)-6-(oxétan-3-yl)-5,6,7,8-tétrahydropyrido[4,3-d]pyrimidine (251) ;
5-isopropyl-2-(4-isopropyl-5-(8-méthyl-[1,2,4]triazolo[1,5-a]pyridin-6-yl)-1H-pyrazol-3-yl)-4,5,6,7-tétrahydrothiazolo[5,4-c]pyridine (252) ;
2-(4-isopropyl-5-(8-méthyl-[1,2,4]triazolo[1,5-a]pyridin-6-yl)-1H-pyrazol-3-yl)-5-(oxétan-3-yl)-4,5,6,7-tétrahydrothiazolo[5,4-c]pyridine (253) ;
2-(4-isopropyl-5-(8-méthyl-[1,2,4]triazolo[1,5-a]pyridin-6-yl)-1H-pyrazol-3-yl)-5-(1-isopropylpipéridin-4-yl)-4,5,6,7-tétrahydrothiazolo[5,4-c]pyridine (254) ;
5-isobutyl-2-(4-isopropyl-5-(8-méthyl-[1,2,4]triazolo[1,5-a]pyridin-6-yl)-1H-pyrazol-3-yl)-4,5,6,7-tétrahydrothiazolo[5,4-c]pyridine (255) ;
2-(4-isopropyl-5-(8-méthyl-[1,2,4]triazolo[1,5-a]pyridin-6-yl)-1H-pyrazol-3-yl)-5-méthyl-4,5,6,7-tétrahydrothiazolo[5,4-c]pyridine (256) ;
N-éthyl-2-(4-isopropyl-5-(8-méthyl-[1,2,4]triazolo[1,5-a]pyridin-6-yl)-1H-pyrazol-3-yl)benzo[d]thiazol-6-amine (257) ;
N-isopropyl-2-(4-isopropyl-5-(8-méthyl-[1,2,4]triazolo[1,5-a]pyridin-6-yl)-1H-pyrazol-3-yl)benzo[d]thiazol-6-amine (258) ;
5-isopropyl-2-(4-isopropyl-5-(8-méthoxy-[1,2,4]triazolo[1,5-a]pyridin-6-yl)-1H-pyrazol-3-yl)-4,5,6,7-tétrahydrothiazolo[5,4-c]pyridine (259) ;
2-(4-isopropyl-5-(8-méthoxy-[1,2,4]triazolo[1,5-a]pyridin-6-yl)-1H-pyrazol-3-yl)-5-propyl-4,5,6,7-tétrahydrothiazolo[5,4-c]pyridine (260) ;
5-éthyl-2-(4-isopropyl-5-(8-méthoxy-[1,2,4]triazolo[1,5-a]pyridin-6-yl)-1H-pyrazol-3-yl)-4,5,6,7-tétrahydrothiazolo[5,4-c]pyridine (261) ;
2-(4-isopropyl-5-(8-méthoxy-[1,2,4]triazolo[1,5-a]pyridin-6-yl)-1H-pyrazol-3-yl)-5-méthyl-4,5,6,7-tétrahydrothiazolo[5,4-c]pyridine (262) ;
6-(4-isopropyl-5-(8-méthyl-[1,2,4]triazolo[1,5-a]pyridin-6-yl)-1H-pyrazol-3-yl)-2-(2-méthoxyéthyl)-1,2,3,4-tétrahydroisoquinoline (263) ;
1-(6-(4-isopropyl-5-(8-méthyl-[1,2,4]triazolo[1,5-a]pyridin-6-yl)-1H-pyrazol-3-yl)-3,4-dihydroisoquinolin-2(1H)-yl)-2-méthylpropan-2-ol (264) ;
1-(2-(4-isopropyl-5-(8-méthyl-[1,2,4]triazolo[1,5-a]pyridin-6-yl)-1H-pyrazol-3-yl)-6,7-dihydrothiazolo[5,4-c]pyridin-5(4H)-yl)-2-méthylpropan-2-ol (265) ;
2-(4-isopropyl-5-(8-méthyl-[1,2,4]triazolo[1,5-a]pyridin-6-yl)-1H-pyrazol-3-yl)-5-(2-méthoxyéthyl)-4,5,6,7-tétrahydrothiazolo[5,4-c]pyridine (266) ;
2-(4-isopropyl-5-(8-méthyl-[1,2,4]triazolo[1,5-a]pyridin-6-yl)-1H-pyrazol-3-yl)-N-(2-méthoxyethyl)benzo[d]thiazol-6-amine (267) ;
2-(diméthylamino)-1-(6-(4-isopropyl-5-(8-méthyl-[1,2,4]triazolo[1,5-a]pyridin-6-yl)-1H-pyrazol-3-yl)-3,4-dihydroisoquinolin-2(1H)-yl)éthan-1-one (268) ;
(S)-3-hydroxy-1-(2-(4-isopropyl-5-(8-méthyl-[1,2,4]triazolo[1,5-a]pyridin-6-yl)-1H-pyrazol-3-yl)-6,7-dihydrothiazolo[5,4-c]pyridin-5(4H)-yl)butan-1-one (269) ;
2-(diméthylamino)-1-(2-(4-isopropyl-5-(8-méthyl-[1,2,4]triazolo[1,5-a]pyridin-6-yl)-1H-pyrazol-3-yl)-6,7-dihydrothiazolo[5,4-c]pyridin-5(4H)-yl)éthan-1-one (270) ;
2-(6-(4-isopropyl-5-(8-méthyl-[1,2,4]triazolo[1,5-a]pyridin-6-yl)-1H-pyrazol-3-yl)-3,4-dihydroisoquinolin-2(1H)-yl)-N,N-diméthylacétamide (271) ;
2-(6-(4-isopropyl-5-(8-méthyl-[1,2,4]triazolo[1,5-a]pyridin-6-yl)-1H-pyrazol-3-yl)-3,4-dihydroisoquinolin-2(1H)-yl)acétamide (272) ;
2-(6-(4-isopropyl-5-(8-méthyl-[1,2,4]triazolo[1,5-a]pyridin-6-yl)-1H-pyrazol-3-yl)-3,4-dihydroisoquinolin-2(1H)-yl)-N-méthylacétamide (273) ;
2-(2-(4-isopropyl-5-(8-méthyl-[1,2,4]triazolo[1,5-a]pyridin-6-yl)-1H-pyrazol-3-yl)-6,7-dihydrothiazolo[5,4-c]pyridin-5(4H)-yl)-N-méthylacétamide (274) ;
2-(2-(4-isopropyl-5-(8-méthyl-[1,2,4]triazolo[1,5-a]pyridin-6-yl)-1H-pyrazol-3-yl)-6,7-dihydrothiazolo[5,4-c]pyridin-5(4H)-yl)-N,N-diméthylacétamide (275) ;
1-(2-(4-isopropyl-5-(8-méthyl-[1,2,4]triazolo[1,5-a]pyridin-6-yl)-1H-pyrazol-3-yl)-6,7-dihydrothiazolo[5,4-c]pyridin-5(4H)-yl)éthan-1-one (276) ;
6-(4-isopropyl-5-(8-méthyl-[1,2,4]triazolo[1,5-a]pyridin-6-yl)-1H-pyrazol-3-yl)pyridin-2-ol (277) ;
6-(5-(2,6-diméthylpyridin-4-yl)-4-isopropyl-1H-pyrazol-3-yl)-1,2,3,4-tétrahydroisoquinoline (278) ;
N-(2,2-difluoroéthyl)-4-(4-isopropyl-5-(8-méthyl-[1,2,4]triazolo[1,5-a]pyridin-6-yl)-1H-pyrazol-3-yl)cyclohexan-1-amine (279) ;
4-(4-isopropyl-5-(8-méthyl-[1,2,4]triazolo[1,5-a]pyridin-6-yl)-1H-pyrazol-3-yl)-N-((3-méthyloxétan-3-yl)méthyl)cyclohexan-1-amine (280-281 ;
N-éthyl-4-(4-isopropyl-5-(8-méthyl-[1,2,4]triazolo[1,5-a]pyridin-6-yl)-1H-pyrazol-3-yl)- N -méthylcyclohexan-1-amine (282-283) ;
N1-(4-(4-isopropyl-5-(8-méthyl-[1,2,4]triazolo[1,5-a]pyridin-6-yl)-1H-pyrazol-3-yl)cyclohexyl)-N2,N2-diméthyléthane-1,2-diamine (284-285) ;
6-(4-isopropyl-3-(4-(4-méthylpipérazin-1-yl)cyclohexyl)-1H-pyrazol-5-yl)-8-méthyl-[1,2,4]triazolo[1,5-a]pyridine (286-287) ;
1-isopropyl-N-(4-(4-isopropyl-5-(8-méthyl-[1,2,4]triazolo[1,5-a]pyridin-6-yl)-1H-pyrazol-3-yl)cyclohexyl)pipéridin-4-amine (288-289) ;
4-(4-(4-isopropyl-5-(8-méthyl-[1,2,4]triazolo[1,5-a]pyridin-6-yl)-1H-pyrazol-3-yl)cyclohexyl)morpholine (290-291) ;
1-(4-(4-isopropyl-5-(8-méthyl-[1,2,4]triazolo[1,5-a]pyridin-6-yl)-1H-pyrazol-3-yl)cyclohexyl)-N,N-diméthylpipéridin-4-amine (292-293) ;
4-(4-isopropyl-5-(8-méthyl-[1,2,4]triazolo[1,5-a]pyridin-6-yl)-1H-pyrazol-3-yl)-N-méthylcyclohexan-1-amine (294-295) ;
2-(diméthylamino)-N-(4-(4-isopropyl-5-(8-méthyl-[1,2,4]triazolo[1,5-a]pyridin-6-yl)-1H-pyrazol-3-yl)cyclohexyl)-N-méthylacétamide (296-297) ;
2-((4-(4-isopropyl-5-(8-méthyl-[1,2,4]triazolo[1,5-a]pyridin-6-yl)-1H-pyrazol-3-yl)cyclohexyl)(méthyl)amino)-N,N-diméthylacétamide (298-299) ;
N-(4-(4-isopropyl-5-(8-méthyl-[1,2,4]triazolo[1,5-a]pyridin-6-yl)-1H-pyrazol-3-yl)cyclohexyl)-N,1-diméthylazétidine-3-carboxamide (300 et 302) ;
N-(4-(4-isopropyl-5-(8-méthyl-[1,2,4]triazolo[1,5-a]pyridin-6-yl)-1H-pyrazol-3-yl)cyclohexyl)-N-méthylazétidine-3-carboxamide (300B et 301) ;
le 1-isopropyl-N-(4-(4-isopropyl-5-(8-méthyl-[1,2,4]triazolo[1,5-a]pyridin-6-yl)-1H-pyrazol-3-yl)cyclohexyl)-N-méthylazétidine-3-carboxamide (303-304 ;
1-éthyl-N-(4-(4-isopropyl-5-(8-méthyl-[1,2,4]triazolo[1,5-a]pyridin-6-yl)-1H-pyrazol-3-yl)cyclohexyl)-N-méthylazetidine-3-carboxamide (305-306 ;
6-(4-isopropyl-3-(4-(1-isopropylpipéridin-4-yl)phényl)-1H-pyrazol-5-yl)-8-méthyl-[1,2,4]triazolo[1,5-a]pyridine (307) ;
6-(4-isopropyl-3-(4-(pipéridin-4-yl)phényl)-1H-pyrazol-5-yl)-8-méthoxy-[1,2,4]triazolo[1,5-a]pyridine (308) ;
6-(4-isopropyl-3-(4-(1-méthylpipéridin-4-yl)phényl)-1H-pyrazol-5-yl)-8-méthyl-[1,2,4]triazolo[1,5-a]pyridine (309) ;
6-(3-(4-(1-éthylpipéridin-4-yl)phényl)-4-isopropyl-1H-pyrazol-5-yl)-8-méthyl-[1,2,4]triazolo[1,5-a]pyridine (310) ;
6-(4-isopropyl-3-(4-(1-(oxétan-3-yl)pipéridin-4-yl)phényl)-1H-pyrazol-5-yl)-8-méthyl-[1,2,4]triazolo[1,5-a]pyridine (311) ;
6-(4-isopropyl-3-(4-(1-((3-méthyloxétan-3-yl)méthyl)pipéridin-4-yl)phényl)-1H-pyrazol-5-yl)-8-méthoxy-[1,2,4]triazolo[1,5-a]pyridine (312) ;
6-(4-isopropyl-3-(4-(1-(tétrahydro-2H-pyran-4-yl)pipéridin-4-yl)phényl)-1H-pyrazol-5-yl)-8-méthoxy-[1,2,4]triazolo[1,5-a]pyridine (313) ;
6-(4-isopropyl-3-(4-(1-méthylpipéridin-4-yl)phényl)-1H-pyrazol-5-yl)-8-méthoxy-[1,2,4]triazolo[1,5-a]pyridine (314) ;
6-(4-isopropyl-3-(4-(1-méthylpipéridin-4-yl)phényl)-1H-pyrazol-5-yl)-8-méthoxy-[1,2,4]triazolo[1,5-a]pyridine (315) ;
6-(4-isopropyl-3-(4-(1-isopropylpipéridin-4-yl)phényl)-1H-pyrazol-5-yl)-8-méthoxy-[1,2,4]triazolo[1,5-a]pyridine (316) ;
6-(4-isopropyl-3-(4-(1-néopentylpipéridin-4-yl)phényl)-1H-pyrazol-5-yl)-8-méthyl-[1,2,4]triazolo[1,5-a]pyridine (317) ;
6-(4-isopropyl-3-(4-(1-(2-méthoxyéthyl)pipéridin-4-yl)phényl)-1H-pyrazol-5-yl)-8-méthoxy-[1,2,4]triazolo[1,5-a]pyridine (318) ;
1-(4-(4-(4-isopropyl-5-(8-méthoxy-[1,2,4]triazolo[1,5-a]pyridin-6-yl)-1H-pyrazol-3-yl)phényl)pipéridin-1-yl)-2-méthylpropan-2-ol (319) ;
6-(4-isopropyl-3-(4-(1-(2,2,2-trifluoroéthyl)pipéridin-4-yl)phényl)-1H-pyrazol-5-yl)-8-méthoxy-[1,2,4]triazolo[1,5-a]pyridine (320) ;
2-(4-(4-(4-isopropyl-5-(8-méthyl-[1,2,4]triazolo[1,5-a]pyridin-6-yl)-1H-pyrazol-3-yl)phényl)pipéridin-1-yl)-N,N-diméthylacétamide (321) ;
2-(4-(4-(4-isopropyl-5-(8-méthoxy-[1,2,4]triazolo[1,5-a]pyridin-6-yl)-1H-pyrazol-3-yl)phényl)pipéridin-1-yl)acétamide (322) ;
2-(diméthylamino)-1-(4-(4-(4-isopropyl-5-(8-méthyl-[1,2,4]triazolo[1,5-a]pyridin-6-yl)-1H-pyrazol-3-yl)phényl)pipéridin-1-yl)éthan-1-one (323) ;
6-(4-isopropyl-3-(4-(1-méthylazétidin-3-yl)phényl)-1H-pyrazol-5-yl)-8-méthyl[1,2,4]triazolo[1,5-a]pyridine (324) ;
6-(3-(4-(azétidin-3-yl)phenyl)-4-isopropyl-1H-pyrazol-5-yl)-8-méthoxy-[1,2,4]triazolo[1,5-a]pyridine (324E) ;
6-(3-(4-(1-éthylazétidin-3-yl)phényl)-4-isopropyl-1H-pyrazol-5-yl)-8-méthyl-[1,2,4]triazolo[1,5-a]pyridine (325) ;
6-(4-isopropyl-3-(4-(1-propylazétidin-3-yl)phényl)-1H-pyrazol-5-yl)-8-méthyl-[1,2,4]triazolo[1,5-a]pyridine (326) ;
6-(4-isopropyl-3-(4-(1-isopropylazétidin-3-yl)phényl)-1H-pyrazol-5-yl)-8-méthyl-[1,2,4]triazolo[1,5-a]pyridine (327) ;
6-(4-isopropyl-3-(4-(1-méthylazétidin-3-yl)phényl)-1H-pyrazol-5-yl)-8-méthoxy-[1,2,4]triazolo[1,5-a]pyridine (328) ;
6-(3-(4-(1-éthylazétidin-3-yl)phényl)-4-isopropyl-1H-pyrazol-5-yl)-8-méthoxy-[1,2,4]triazolo[1,5-a]pyridine (329) ;
6-(4-isopropyl-3-(4-(1-propylazétidin-3-yl)phényl)-1H-pyrazol-5-yl)-8-méthoxy-[1,2,4]triazolo[1,5-a]pyridine (330) ;
6-(4-isopropyl-3-(4-(1-isopropylazétidin-3-yl)phényl)-1H-pyrazol-5-yl)-8-méthoxy-[1,2,4]triazolo[1,5-a]pyridine (331) ;
6-(4-isopropyl-3-(4-(1-(oxétan-3-yl)azétidin-3-yl)phényl)-1H-pyrazol-5-yl)-8-méthoxy-[1,2,4]triazolo[1,5-a]pyridine (332) ;
6-(4-isopropyl-3-(4-(1-(tétrahydro-2H-pyran-4-yl)azétidin-3-yl)phényl)-1H-pyrazol-5-yl)-8-méthoxy-[1,2,4]triazolo[1,5-a]pyridine (333) ;
2-(diméthylamino)-1-(3-(4-(4-isopropyl-5-(8-méthyl-[1,2,4]triazolo[1,5-a]pyridin-6-yl)-1H-pyrazol-3-yl)phényl)azétidin-1-yl)éthan-1-one (334) ;
2-(diméthylamino)-1-(3-(4-(4-isopropyl-5-(8-méthoxy-[1,2,4]triazolo[1,5-a]pyridin-6-yl)-1H-pyrazol-3-yl)phényl)azétidin-1-yl)éthan-1-one (335) ;
1-(3-(4-(4-isopropyl-5-(8-méthyl-[1,2,4]triazolo[1,5-a]pyridin-6-yl)-1H-pyrazol-3-yl)phényl)azétidin-1-yl)-2-méthylpropan-2-ol (336) ;
1-(3-(4-(4-isopropyl-5-(8-méthoxy-[1,2,4]triazolo[1,5-a]pyridin-6-yl)-1H-pyrazol-3-yl)phényl)azétidin-1-yl)-2-méthylpropan-2-ol (337) ;
6-(4-isopropyl-3-(4-(1-(2-(méthylsulfonyl)éthyl)azétidin-3-yl)phényl)-1H-pyrazol-5-yl)-8-méthoxy-[1,2,4]triazolo[1,5-a]pyridine (338) ;
6-(4-isopropyl-3-(5-(pipéridin-4-yl)pyridin-2-yl)-1H-pyrazol-5-yl)-8-méthoxy-[1,2,4]triazolo[1,5-a]pyridine (339) ;
6-(4-isopropyl-3-(6-(pipéridin-4-yl)pyridin-3-yl)-1H-pyrazol-5-yl)-8-méthyl-[1,2,4]triazolo[1,5-a]pyridine (340) ;
6-(4-isopropyl-3-(5-(pipéridin-4-yl)pyrimidin-2-yl)-1H-pyrazol-5-yl)-8-méthyl-[1,2,4]triazolo[1,5-a]pyridine (341) ;
6-(4-isopropyl-3-(5-(pipéridin-4-yl)pyridin-2-yl)-1H-pyrazol-5-yl)-8-méthyl-[1,2,4]triazolo[1,5-a]pyridine (342) ;
6-(4-isopropyl-3-(2-(pipéridin-4-yl)pyrimidin-5-yl)-1H-pyrazol-5-yl)-8-méthyl-[1,2,4]triazolo[1,5-a]pyridine (343) ;
6-(4-isopropyl-3-(6-(pipéridin-4-yl)pyridazin-3-yl)-1H-pyrazol-5-yl)-8-méthyl-[1,2,4]triazolo[1,5-a]pyridine (344 );
6-(4-isopropyl-3-(5-(pipéridin-4-yl)pyrazin-2-yl)-1H-pyrazol-5-yl)-8-méthoxy-[1,2,4]triazolo[1,5-a]pyridine (345) ;
6-(4-isopropyl-3-(5-(pipéridin-4-yl)pyridin-2-yl)-1H-pyrazol-5-yl)-8-méthoxy-7-méthyl-[1,2,4]triazolo[1,5-a]pyridine (346) ;
6-(3-(5-(1-éthylpiperidin-4-yl)pyridin-2-yl)-4-isopropyl-1H-pyrazol-5-yl)-8-méthoxy-[1,2,4]triazolo[1,5-a]pyridine (347) ;
6-(4-isopropyl-3-(5-(1-méthylpipéridin-4-yl)pyridin-2-yl)-1H-pyrazol-5-yl)-8-méthoxy-[1,2,4]triazolo[1,5-a]pyridine (348) ;
6-(4-isopropyl-3-(5-(1-propylpipéridin-4-yl)pyridin-2-yl)-1H-pyrazol-5-yl)-8-méthoxy-[1,2,4]triazolo[1,5-a]pyridine (349) ;
6-(4-isopropyl-3-(5-(1-isopropylpipéridin-4-yl)pyridin-2-yl)-1H-pyrazol-5-yl)-8-méthoxy-[1,2,4]triazolo[1,5-a]pyridine (350) ;
6-(4-isopropyl-3-(5-(1-néopentylpipéridin-4-yl)pyridin-2-yl)-1H-pyrazol-5-yl)-8-méthoxy-[1,2,4]triazolo[1,5-a]pyridine (351) ;
6-(4-isopropyl-3-(5-(1-(3,3,3-trifluoropropyl)pipéridin-4-yl)pyridin-2-yl)-1H-pyrazol-5-yl)-8-méthoxy-[1,2,4]triazolo[1,5-a]pyridine (352) ;
6-(4-isopropyl-3-(5-(1-((1-méthyl-1H-1,2,4-triazol-3-yl)méthyl)pipéridin-4-yl)pyridin-2-yl)-1H-pyrazol-5-yl)-8-méthoxy-[1,2,4]triazolo[1,5-a]pyridine (353) ;
6-(3-(5-(1-(3-éthoxycyclobutyl)pipéridin-4-yl)pyridin-2-yl)-4-isopropyl-1H-pyrazol-5-yl)-8-méthoxy-[1,2,4]triazolo[1,5-a]pyridine (354) ;
6-(3-(5-(1-(3-éthoxycyclobutyl)pipéridin-4-yl)pyridin-2-yl)-4-isopropyl-1H-pyrazol-5-yl)-8-méthoxy-[1,2,4]triazolo[1,5-a]pyridine (355) ;
6-(4-isopropyl-3-(5-(1-(oxétan-3-yl)pipéridin-4-yl)pyridin-2-yl)-1H-pyrazol-5-yl)-8-méthoxy-[1,2,4]triazolo[1,5-a]pyridine (356) ;
6-(4-isopropyl-3-(6-(1-méthylpipéridin-4-yl)pyridazin-3-yl)-1H-pyrazol-5-yl)-8-méthyl-[1,2,4]triazolo[1,5-a]pyridine (357) ;
6-(4-isopropyl-3-(6-(1-isopropylpipéridin-4-yl)pyridazin-3-yl)-1H-pyrazol-5-yl)-8-méthyl-[1,2,4]triazolo[1,5-a]pyridine (358) ;
6-(4-isopropyl-3-(6-(1-méthylpipéridin-4-yl)pyridin-3-yl)-1H-pyrazol-5-yl)-8-méthyl-[1,2,4]triazolo[1,5-a]pyridine (359) ;
6-(3-(6-(1-éthylpipéridin-4-yl)pyridin-3-yl)-4-isopropyl-1H-pyrazol-5-yl)-8-méthyl-[1,2,4]triazolo[1,5-a]pyridine (360) ;
6-(4-isopropyl-3-(6-(1-isopropylpipéridin-4-yl)pyridin-3-yl)-1H-pyrazol-5-yl)-8-méthyl-[1,2,4]triazolo[1,5-a]pyridine (361) ;
6-(4-isopropyl-3-(6-(1-(oxétan-3-yl)pipéridin-4-yl)pyridin-3-yl)-1H-pyrazol-5-yl)-8-méthyl-[1,2,4]triazolo[1,5-a]pyridine (362) ;
6-(4-isopropyl-3-(2-(1-isopropylpipéridin-4-yl)pyrimidin-5-yl)-1H-pyrazol-5-yl)-8-méthyl-[1,2,4]triazolo[1,5-a]pyridine (363 ;
6-(4-isopropyl-3-(2-(1-néopentylpipéridin-4-yl)pyrimidin-5-yl)-1H-pyrazol-5-yl)-8-méthyl-[1,2,4]triazolo[1,5-a]pyridine (364) ;
6-(4-isopropyl-3-(5-(1-méthylpipéridin-4-yl)pyridin-2-yl)-1H-pyrazol-5-yl)-8-méthyl-[1,2,4]triazolo[1,5-a]pyridine (365) ;
6-(3-(5-(1-éthylpipéridin-4-yl)pyridin-2-yl)-4-isopropyl-1H-pyrazol-5-yl)-8-méthyl-[1,2,4]triazolo[1,5-a]pyridine (366) ;
6-(4-isopropyl-3-(5-(1-isopropylpipéridin-4-yl)pyridin-2-yl)-1H-pyrazol-5-yl)-8-méthyl-[1,2,4]triazolo[1,5-a]pyridine (367) ;
6-(4-isopropyl-3-(5-(1-méthylpipéridin-4-yl)pyrimidin-2-yl)-1H-pyrazol-5-yl)-8-méthyl-[1,2,4]triazolo[1,5-a]pyridine (368) ;
6-(3-(5-(1-éthylpipéridin-4-yl)pyrimidin-2-yl)-4-isopropyl-1H-pyrazol-5-yl)-8-méthyl-[1,2,4]triazolo[1,5-a]pyridine (369) ;
6-(4-isopropyl-3-(5-(1-isopropylpipéridin-4-yl)pyrimidin-2-yl)-1H-pyrazol-5-yl)-8-méthyl-[1,2,4]triazolo[1,5-a]pyridine (370) ;
6-(4-isopropyl-3-(5-(1-néopentylpipéridin-4-yl)pyrimidin-2-yl)-1H-pyrazol-5-yl)-8-méthyl-[1,2,4]triazolo[1,5-a]pyridine (371) ;
6-(4-isopropyl-3-(5-(1-(3-méthylbutan-2-yl)pipéridin-4-yl)pyrimidin-2-yl)-1H-pyrazol-5-yl)-8-méthyl-[1,2,4]triazolo[1,5-a]pyridine (372) ;
6-(4-isopropyl-3-(5-(1-neopentylpipéridin-4-yl)pyridin-2-yl)-1H-pyrazol-5-yl)-8-méthyl-[1,2,4]triazolo[1,5-a]pyridine (373) ;
6-(3-(5-(1-(cyclopropylméthyl)pipéridin-4-yl)pyridin-2-yl)-4-isopropyl-1H-pyrazol-5-yl)-8-méthoxy-[1,2,4]triazolo[1,5-a]pyridine (374) ;
6-(4-isopropyl-3-(5-(1-((tétrahydro-2H-pyran-4-yl)méthyl)pipéridin-4-yl)pyridin-2-yl)-1H-pyrazol-5-yl)-8-méthoxy-[1,2,4]triazolo[1,5-a]pyridine (375) ;
6-(4-isopropyl-3-(5-(1-((tétrahydro-2H-pyran-4-yl)méthyl)pipéridin-4-yl)pyrazin-2-yl)-1H-pyrazol-5-yl)-8-méthoxy-[1,2,4]triazolo[1,5-a]pyridine (376) ;
6-(3-(5-(1-(cyclobutylméthyl)pipéridin-4-yl)pyrazin-2-yl)-4-isopropyl-1H-pyrazol-5-yl)-8-méthoxy-[1,2,4]triazolo[1,5-a]pyridine (377) ;
6-(3-(5-(1-(cyclopropylméthyl)pipéridin-4-yl)pyrazin-2-yl)-4-isopropyl-1H-pyrazol-5-yl)-8-méthoxy-[1,2,4]triazolo[1,5-a]pyridine (378) ;
6-(3-(5-(1-isobutylpipéridin-4-yl)pyrazin-2-yl)-4-isopropyl-1H-pyrazol-5-yl)-8-méthoxy-[1,2,4]triazolo[1,5-a]pyridine (379) ;
6-(4-isopropyl-3-(5-(1-(tétrahydro-2H-pyran-4-yl)pipéridin-4-yl)pyrazin-2-yl)-1H-pyrazol-5-yl)-8-méthoxy-[1,2,4]triazolo[1,5-a]pyridine (380) ;
6-(4-isopropyl-3-(5-(1-méthylpipéridin-4-yl)pyrazin-2-yl)-1H-pyrazol-5-yl)-8-méthoxy-[1,2,4]triazolo[1,5-a]pyridine (381) ;
6-(3-(5-(1-éthylpipéridin-4-yl)pyrazin-2-yl)-4-isopropyl-1H-pyrazol-5-yl)-8-méthoxy-[1,2,4]triazolo[1,5-a]pyridine (382) ;
6-(4-isopropyl-3-(5-(1-propylpipéridin-4-yl)pyrazin-2-yl)-1H-pyrazol-5-yl)-8-méthoxy-[1,2,4]triazolo[1,5-a]pyridine (383) ;
6-(4-isopropyl-3-(5-(1-isopropylpipéridin-4-yl)pyrazin-2-yl)-1H-pyrazol-5-yl)-8-méthoxy-[1,2,4]triazolo[1,5-a]pyridine (384) ;
6-(4-isopropyl-3-(5-(1-(oxétan-3-yl)pipéridin-4-yl)pyrazin-2-yl)-1H-pyrazol-5-yl)-8-méthoxy-[1,2,4]triazolo[1,5-a]pyridine (385) ;
6-(4-isopropyl-3-(5-(1-(pentan-3-yl)pipéridin-4-yl)pyrazin-2-yl)-1H-pyrazol-5-yl)-8-méthoxy-[1,2,4]triazolo[1,5-a]pyridine (386) ;
6-(3-(5-(1-cyclobutylpipéridin-4-yl)pyrazin-2-yl)-4-isopropyl-1H-pyrazol-5-yl)-8-méthoxy-[1,2,4]triazolo[1,5-a]pyridine (387) ;
6-(4-isopropyl-3-(5-(1-((tétrahydro-2H-pyran-4-yl)méthyl)pipéridin-4-yl)pyridin-2-yl)-1H-pyrazol-5-yl)-8-méthoxy-7-méthyl-[1,2,4]triazolo[1,5-a]pyridine (388) ;
2-(4-(6-(4-isopropyl-5-(8-méthoxy-[1,2,4]triazolo[1,5-a]pyridin-6-yl)-1H-pyrazol-3-yl)pyridin-3-yl)pipéridin-1-yl)-N,N-diméthylacétamide (389) ;
1-(4-(2-(4-isopropyl-5-(8-méthyl-[1,2,4]triazolo[1,5-a]pyridin-6-yl)-1H-pyrazol-3-yl)pyrimidin-5-yl)pipéridin-1-yl)-2-méthylpropan-2-ol (390) ;
1-(4-(6-(4-isopropyl-5-(8-méthoxy-[1,2,4]triazolo[1,5-a]pyridin-6-yl)-1H-pyrazol-3-yl)pyridin-3-yl)pipéridin-1-yl)-2-méthylpropan-2-ol (391) ;
6-(4-isopropyl-3-(5-(1-(2-(méthylsulfonyl)éthyl)pipéridin-4-yl)pyridin-2-yl)-1H-pyrazol-5-yl)-8-méthoxy-[1,2,4]triazolo[1,5-a]pyridine (392) ;
2-(4-(6-(4-isopropyl-5-(8-méthoxy-[1,2,4]triazolo[1,5-a]pyridin-6-yl)-1H-pyrazol-3-yl)pyridin-3-yl)pipéridin-1-yl)acétamide (393) ;
2-(4-(6-(4-isopropyl-5-(8-méthoxy-[1,2,4]triazolo[1,5-a]pyridin-6-yl)-1H-pyrazol-3-yl)pyridin-3-yl)pipéridin-1-yl)acétonitri(394) ;
6-(4-isopropyl-3-(5-(1-(2-méthoxyéthyl)pipéridin-4-yl)pyridin-2-yl)-1H-pyrazol-5-yl)-8-méthoxy-[1,2,4]triazolo[1,5-a]pyridine (395) ;
6-(4-isopropyl-3-(5-(1-(2-méthoxyéthyl)pipéridin-4-yl)pyrazin-2-yl)-1H-pyrazol-5-yl)-8-méthoxy-[1,2,4]triazolo[1,5-a]pyridine (396) ;
6-(4-isopropyl-3-(5-(1-(2-(méthylsulfonyl)éthyl)pipéridin-4-yl)pyrazin-2-yl)-1H-pyrazol-5-yl)-8-méthoxy-[1,2,4]triazolo[1,5-a]pyridine (397) ;
6-(3-(5-(1-(2-fluoroéthyl)pipéridin-4-yl)pyrazin-2-yl)-4-isopropyl-1H-pyrazol-5-yl)-8-méthoxy-[1,2,4]triazolo[1,5-a]pyridine (398) ;
6-(3-(5-(1-(3-fluoropropyl)pipéridin-4-yl)pyrazin-2-yl)-4-isopropyl-1H-pyrazol-5-yl)-8-méthoxy-[1,2,4]triazolo[1,5-a]pyridine (399) ;
2-(4-(5-(4-isopropyl-5-(8-méthoxy-[1,2,4]triazolo[1,5-a]pyridin-6-yl)-1H-pyrazol-3-yl)pyrazin-2-yl)pipéridin-1-yl)-N-méthylacétamide (400) ;
2-(4-(5-(4-isopropyl-5-(8-méthoxy-[1,2,4]triazolo[1,5-a]pyridin-6-yl)-1H-pyrazol-3-yl)pyrazin-2-yl)pipéridin-1-yl)-N,N-diméthylacétamide (401) ;
2-(diméthylamino)-1-(4-(6-(4-isopropyl-5-(8-méthoxy-[1,2,4]triazolo[1,5-a]pyridin-6-yl)-1H-pyrazol-3-yl)pyridin-3-yl)pipéridin-1-yl)éthan-1-one (402) ;
2-(diméthylamino)-1-(4-(5-(4-isopropyl-5-(8-méthoxy-[1,2,4]triazolo[1,5-a]pyridin-6-yl)-1H-pyrazol-3-yl)pyrazin-2-yl)pipéridin-1-yl)éthan-1-one (403) ;
2-(diéthylamino)-1-(4-(5-(4-isopropyl-5-(8-méthoxy-[1,2,4]triazolo[1,5-a]pyridin-6-yl)-1H-pyrazol-3-yl)pyrazin-2-yl)pipéridin-1-yl)éthan-1-one (404) ;
1-(4-(4-isopropyl-5-(8-méthyl-[1,2,4]triazolo[1,5-a]pyridin-6-yl)-1H-pyrazol-3-yl)phényl)-N,N-diméthylazétidin-3-amine (405) ;
6-(4-isopropyl-3-(4-(pipérazin-1-yl)phényl)-1H-pyrazol-5-yl)-8-méthyl-[1,2,4]triazolo[1,5-a]pyridine (406) ;
6-(4-isopropyl-3-(2-(pipérazin-1-yl)pyrimidin-5-yl)-1H-pyrazol-5-yl)-8-méthyl-[1,2,4]triazolo[1,5-a]pyridine (407) ;
6-(4-isopropyl-3-(4-(4-néopentylpipérazin-1-yl)phényl)-1H-pyrazol-5-yl)-8-méthyl-[1,2,4]triazolo[1,5-a]pyridine (408) ;
N-isopropyl-1-(4-(4-isopropyl-5-(8-méthyl-[1,2,4]triazolo[1,5-a]pyridin-6-yl)-1H-pyrazol-3-yl)phényl)azétidin-3-amine (409) ;
6-(4-isopropyl-3-(2-(4-méthylpipérazin-1-yl)pyrimidin-5-yl)-1H-pyrazol-5-yl)-8-méthyl-[1,2,4]triazolo[1,5-a]pyridine (410) ;
6-(4-isopropyl-3-(4-(4-isopropylpipérazin-1-yl)phényl)-1H-pyrazol-5-yl)-8-méthyl-[1,2,4]triazolo[1,5-a]pyridine (411) );
6-(4-isopropyl-3-(4-(4-méthylpipérazin-1-yl)phényl)-1H-pyrazol-5-yl)-8-méthyl-[1,2,4]triazolo[1,5-a]pyridine (412) ;
6-(3-(4-(4-éthylpipérazin-1-yl)phényl)-4-isopropyl-1H-pyrazol-5-yl)-8-méthyl-[1,2,4]triazolo[1,5-a]pyridine (413) ;
6-(4-isopropyl-3-(4-(4-(oxétan-3-yl)pipérazin-1-yl)phényl)-1H-pyrazol-5-yl)-8-méthyl-[1,2,4]triazolo[1,5-a]pyridine (414) ;
6-(4-isopropyl-3-(2-(4-isopropylpipérazin-1-yl)pyrimidin-5-yl)-1H-pyrazol-5-yl)-8-méthyl-[1,2,4]triazolo[1,5-a]pyridine (415) ;
6-(4-isopropyl-3-(2-(4-(oxétan-3-yl)pipérazin-1-yl)pyrimidin-5-yl)-1H-pyrazol-5-yl)-8-méthyl-[1,2,4]triazolo[1,5-a]pyridine (416) ;
6-(4-isopropyl-3-(4-(4-(2-(méthylsulfonyl)éthyl)pipérazin-1-yl)phényl)-1H-pyrazol-5-yl)-8-méthyl-[1,2,4]triazolo[1,5-a]pyridine (417) ;
1-(4-(4-(4-isopropyl-5-(8-méthyl-[1,2,4]triazolo[1,5-a]pyridin-6-yl)-1H-pyrazol-3-yl)phényl)pipérazin-1-yl)-2-méthylpropan-2-ol (418) ;
6-(4-isopropyl-3-(5-(4-(2-méthoxyéthyl)pipérazin-1-yl)pyridin-2-yl)-1H-pyrazol-5-yl)-8-méthoxy-[1,2,4]triazolo[1,5-a]pyridine (419) ;
1-(4-(4-isopropyl-5-(8-méthyl-[1,2,4]triazolo[1,5-a]pyridin-6-yl)-1H-pyrazol-3-yl)phényl)-4-méthylpipérazin-2-one (420) ;
4-isopropyl-1-(4-(4-isopropyl-5-(8-méthyl-[1,2,4]triazolo[1,5-a]pyridin-6-yl)-1H-pyrazol-3-yl)phényl)pipérazin-2-one (421) ;
1-(4-(4-isopropyl-5-(8-méthyl-[1,2,4]triazolo[1,5-a]pyridin-6-yl)-1H-pyrazol-3-yl)phényl)-4-(oxétan-3-yl)pipérazin-2-one (422) ;
6-(3-(5-(4-cyclobutylpipérazin-1-yl)pyridin-2-yl)-4-isopropyl-1H-pyrazol-5-yl)-8-méthoxy-[1,2,4]triazolo[1,5-a]pyridine (423) ;
6-(3-(5-(2,6-diazaspiro[3.3]heptan-2-yl)pyridin-2-yl)-4-isopropyl-1H-pyrazol-5-yl)-8-méthoxy-[1,2,4]triazolo[1,5-a]pyridine (424) ;
4-(4-(6-(4-isopropyl-5-(8-éethoxy-[1,2,4]triazolo[1,5-a]pyridin-6-yl)-1H-pyrazol-3-yl)pyridin-3-yl)pipérazin-1-yl)tétrahydro-2H-thiopyran 1,1-dioxide (425) ;
6-(3-(5-(4-(cyclopropylméthyl)pipérazin-1-yl)pyridin-2-yl)-4-isopropyl-1H-pyrazol-5-yl)-8-méthoxy-[1,2,4]triazolo[1,5-a]pyridine (426) ;
6-(3-(5-(4-éthylpipérazin-1-yl)pyridin-2-yl)-4-isopropyl-1H-pyrazol-5-yl)-8-méthoxy-[1,2,4]triazolo[1,5-a]pyridine (427) ;
6-(4-isopropyl-3-(5-(4-méthylpipérazin-1-yl)pyridin-2-yl)-1H-pyrazol-5-yl)-8-méthoxy-[1,2,4]triazolo[1,5-a]pyridine (428) ;
6-(4-isopropyl-3-(5-(4-isopropylpipérazin-1-yl)pyridin-2-yl)-1H-pyrazol-5-yl)-8-méthoxy-[1,2,4]triazolo[1,5-a]pyridine (429) ;
6-(3-(5-(4-isobutylpipérazin-1-yl)pyridin-2-yl)-4-isopropyl-1H-pyrazol-5-yl)-8-méthoxy-[1,2,4]triazolo[1,5-a]pyridine (430) ;
6-(4-isopropyl-3-(5-(4-propylpipérazin-1-yl)pyridin-2-yl)-1H-pyrazol-5-yl)-8-méthoxy-[1,2,4]triazolo[1,5-a]pyridine (431) ;
6-(4-isopropyl-3-(5-(4-((tétrahydro-2H-pyran-4-yl)méthyl)pipérazin-1-yl)pyridin-2-yl)-1H-pyrazol-5-yl)-8-méthoxy-[1,2,4]triazolo[1,5-a]pyridine (432) ;
6-(4-isopropyl-3-(5-(4-(3,3,3-trifluoropropyl)pipérazin-1-yl)pyridin-2-yl)-1H-pyrazol-5-yl)-8-méthoxy-[1,2,4]triazolo[1,5-a]pyridine (433) ;
6-(3-(5-(4-(cyclobutylméthyl)pipérazin-1-yl)pyridin-2-yl)-4-isopropyl-1H-pyrazol-5-yl)-8-méthoxy-[1,2,4]triazolo[1,5-a]pyridine (434) ;
6-(4-isopropyl-3-(5-(4-(oxétan-3-yl)pipérazin-1-yl)pyridin-2-yl)-1H-pyrazol-5-yl)-8-méthoxy-[1,2,4]triazolo[1,5-a]pyridine (435) ;
6-(4-isopropyl-3-(5-(4-(tétrahydro-2H-pyran-4-yl)pipérazin-1-yl)pyridin-2-yl)-1H-pyrazol-5-yl)-8-méthoxy-[1,2,4]triazolo[1,5-a]pyridine (436) ;
6-(4-isopropyl-3-(5-(4-(pentan-3-yl)pipérazin-1-yl)pyridin-2-yl)-1H-pyrazol-5-yl)-8-méthoxy-[1,2,4]triazolo[1,5-a]pyridine (437) ;
6-(3-(5-(4-(2-éthylbutyl)pipérazin-1-yl)pyridin-2-yl)-4-isopropyl-1H-pyrazol-5-yl)-8-méthoxy-[1,2,4]triazolo[1,5-a]pyridine (438) ;
6-(3-(5-(4-((3-éthyloxétan-3-yl)méthyl)pipérazin-1-yl)pyridin-2-yl)-4-isopropyl-1H-pyrazol-5-yl)-8-méthoxy-[1,2,4]triazolo[1,5-a]pyridine (439) ;
6-(4-isopropyl-3-(5-(6-((tétrahydro-2H-pyran-4-yl)méthyl)-2,6-diazaspiro[3.3]heptan-2-yl)pyridin-2-yl)-1H-pyrazol-5-yl)-8-méthoxy-[1,2,4]triazolo[1,5-a]pyridine (440) ;
6-(3-(5-(6-(cyclobutylméthyl)-2,6-diazaspiro[3.3]heptan-2-yl)pyridin-2-yl)-4-isopropyl-1H-pyrazol-5-yl)-8-méthoxy-[1,2,4]triazolo[1,5-a]pyridine (441) ;
6-(3-(5-(6-isobutyl-2,6-diazaspiro[3.3]heptan-2-yl)pyridin-2-yl)-4-isopropyl-1H-pyrazol-5-yl)-8-méthoxy-[1,2,4]triazolo[1,5-a]pyridine (442) ;
6-(4-isopropyl-3-(5-(6-(tétrahydro-2H-pyran-4-yl)-2,6-diazaspiro[3.3]heptan-2-yl)pyridin-2-yl)-1H-pyrazol-5-yl)-8-méthoxy-[1,2,4]triazolo[1,5-a]pyridine (443) ;
6-(3-(5-(6-cyclobutyl-2,6-diazaspiro[3.3]heptan-2-yl)pyridin-2-yl)-4-isopropyl-1H-pyrazol-5-yl)-8-méthoxy-[1,2,4]triazolo[1,5-a]pyridine (444) ;
6-(4-isopropyl-3-(5-(6-méthyl-2,6-diazaspiro[3.3]heptan-2-yl)pyridin-2-yl)-1H-pyrazol-5-yl)-8-méthoxy-[1,2,4]triazolo[1,5-a]pyridine (445) ;
6-(3-(5-(6-éthyl-2,6-diazaspiro[3.3]heptan-2-yl)pyridin-2-yl)-4-isopropyl-1H-pyrazol-5-yl)-8-méthoxy-[1,2,4]triazolo[1,5-a]pyridine (446) ;
6-(4-isopropyl-3-(5-(6-propyl-2,6-diazaspiro[3.3]heptan-2-yl)pyridin-2-yl)-1H-pyrazol-5-yl)-8-méthoxy-[1,2,4]triazolo[1,5-a]pyridine (447) ;
6-(4-isopropyl-3-(5-(6-isopropyl-2,6-diazaspiro[3.3]heptan-2-yl)pyridin-2-yl)-1H-pyrazol-5-yl)-8-méthoxy-[1,2,4]triazolo[1,5-a]pyridine (448) ;
6-(4-isopropyl-3-(5-(6-(oxétan-3-yl)-2,6-diazaspiro[3.3]heptan-2-yl)pyridin-2-yl)-1H-pyrazol-5-yl)-8-méthoxy-[1,2,4]triazolo[1,5-a]pyridine (449) ;
6-(3-(5-(6-(cyclopropylméthyl)-2,6-diazaspiro[3.3]heptan-2-yl)pyridin-2-yl)-4-isopropyl-1H-pyrazol-5-yl)-8-méthoxy-[1,2,4]triazolo[1,5-a]pyridine (450) ;
2-(diméthylamino)-1-(4-(6-(4-isopropyl-5-(8-méthoxy-[1,2,4]triazolo[1,5-a]pyridin-6-yl)-1H-pyrazol-3-yl)pyridin-3-yl)pipérazin-1-yl)éthan-1-one (451) ;
2-(diéthylamino)-1-(4-(6-(4-isopropyl-5-(8-méthoxy-[1,2,4]triazolo[1,5-a]pyridin-6-yl)-1H-pyrazol-3-yl)pyridin-3-yl)pipérazin-1-yl)éthan-1-one (452) ;
2-(diméthylamino)-1-(6-(6-(4-isopropyl-5-(8-méthoxy-[1,2,4]triazolo[1,5-a]pyridin-6-yl)-1H-pyrazol-3-yl)pyridin-3-yl)-2,6-diazaspiro[3.3]heptan-2-yl)éthan-1-one (453) ;
6-(4-isopropyl-3-(5-(4-(2-(méthylsulfonyl)éthyl)pipérazin-1-yl)pyridin-2-yl)-1H-pyrazol-5-yl)-8-méthoxy-[1,2,4]triazolo[1,5-a]pyridine (454) ;
2-(4-(6-(4-isopropyl-5-(8-méthoxy-[1,2,4]triazolo[1,5-a]pyridin-6-yl)-1H-pyrazol-3-yl)pyridin-3-yl)pipérazin-1-yl)-N,N-diméthylacétamide (455) ;
2-(4-(6-(4-isopropyl-5-(8-méthoxy-[1,2,4]triazolo[1,5-a]pyridin-6-yl)-1H-pyrazol-3-yl)pyridin-3-yl)pipérazin-1-yl)-N-méthylacétamide (456) ;
6-(4-isopropyl-3-(5-(6-(2-(méthylsulfonyl)éthyl)-2,6-diazaspiro[3.3]heptan-2-yl)pyridin-2-yl)-1H-pyrazol-5-yl)-8-méthoxy-[1,2,4]triazolo[1,5-a]pyridine (457) ;
6-(4-isopropyl-3-(5-(6-(2-méthoxyéthyl)-2,6-diazaspiro[3.3]heptan-2-yl)pyridin-2-yl)-1H-pyrazol-5-yl)-8-méthoxy-[1,2,4]triazolo[1,5-a]pyridine (458) ;
2-(4-(4-isopropyl-5-(8-méthyl-[1,2,4]triazolo[1,5-a]pyridin-6-yl)-1H-pyrazol-3-yl)phényl)-4-méthylmorpholine (459-460) ;
4-éthyl-2-(4-(4-isopropyl-5-(8-méthyl-[1,2,4]triazolo[1,5-a]pyridin-6-yl)-1H-pyrazol-3-yl)phényl)morpholine (461) ;
4-isopropyl-2-(4-(4-isopropyl-5-(8-méthyl-[1,2,4]triazolo[1,5-a]pyridin-6-yl)-1H-pyrazol-3-yl)phényl)morpholine (462-463) ;
2-(4-(4-isopropyl-5-(8-méthyl-[1,2,4]triazolo[1,5-a]pyridin-6-yl)-1H-pyrazol-3-yl)phényl)-4-néopentylmorpholine (464-465) ;
2-(2-(4-(4-isopropyl-5-(8-méthyl-[1,2,4]triazolo[1,5-a]pyridin-6-yl)-1H-pyrazol-3-yl)phényl)morpholino)-N,N-diméthylacétamide (466-468) ;
2-(diméthylamino)-1-(2-(4-(4-isopropyl-5-(8-méthyl-[1,2,4]triazolo[1,5-a]pyridin-6-yl)-1H-pyrazol-3-yl)phényl)morpholino)éthan-1-one (469-470) ;
4-(4-(4-isopropyl-5-(8-méthyl-[1,2,4]triazolo[1,5-a]pyridin-6-yl)-1H-pyrazol-3-yl)phényl)-N-méthylcyclohexan-1-amine (471) ;
4-(4-(4-isopropyl-5-(8-méthyl-[1,2,4]triazolo[1,5-a]pyridin-6-yl)-1H-pyrazol-3-yl)phényl)-N,N-diméthylcyclohexan-1-amine (472) ;
N-cyclobutyl-4-(6-(4-isopropyl-5-(8-méthoxy-[1,2,4]triazolo[1,5-a]pyridin-6-yl)-1H-pyrazol-3-yl)pyridin-3-yl)cyclohexan-1-amine (473-474) ;
2-(4-(6-(4-isopropyl-5-(8-méthoxy-[1,2,4]triazolo[1,5-a]pyridin-6-yl)-1H-pyrazol-3-yl)pyridin-3-yl)cyclohexyl)-7-oxa-2-azaspiro[3.5]nonane (475-476) ;
6-(4-(6-(4-isopropyl-5-(8-méthoxy-[1,2,4]triazolo[1,5-a]pyridin-6-yl)-1H-pyrazol-3-yl)pyridin-3-yl)cyclohexyl)-2-oxa-6-azaspiro[3.3]heptane (477-478) ;
6-(4-isopropyl-3-(5-(4-(3-méthoxyazétidin-1-yl)cyclohexyl)pyridin-2-yl)-1H-pyrazol-5-yl)-8-méthoxy-[1,2,4]triazolo[1,5-a]pyridine (479-480) ;
4-(6-(4-isopropyl-5-(8-méthoxy-[1,2,4]triazolo[1,5-a]pyridin-6-yl)-1H-pyrazol-3-yl)pyridin-3-yl)-N-(2-méthoxy-2-méthylpropyl)cyclohexan-1-amine (481-482) ;
4-(4-(6-(4-isopropyl-5-(8-méthoxy-[1,2,4]triazolo[1,5-a]pyridin-6-yl)-1H-pyrazol-3-yl)pyridin-3-yl)cyclohexyl)pipérazin-2-one (483-484) ;
4-(6-(4-isopropyl-5-(8-méthoxy-[1,2,4]triazolo[1,5-a]pyridin-6-yl)-1H-pyrazol-3-yl)pyridin-3-yl)-N-((1-(méthylsulfonyl)cyclopropyl)méthyl)cyclohexan-1-amine (485-486) ;
7-(4-(6-(4-isopropyl-5-(8-méthoxy-[1,2,4]triazolo[1,5-a]pyridin-6-yl)-1H-pyrazol-3-yl)pyridin-3-yl)cyclohexyl)-2-oxa-7-azaspiro[3.5]nonane (487-488) ;
4-(6-(4-isopropyl-5-(8-méthoxy-[1,2,4]triazolo[1,5-a]pyridin-6-yl)-1H-pyrazol-3-yl)pyridin-3-yl)-N-(2-méthoxyéthyl)-N-méthylcyclohexan-1-amine (490-491) ;
N -(4-(6-(4-isopropyl-5-(8-méthoxy-[1,2,4]triazolo[1,5-a]pyridin-6-yl)-1H-pyrazol-3-yl)pyridin-3-yl)cyclohexyl)-3-méthyloxétan-3-amine (492-493) ;
4-(4-(6-(4-isopropyl-5-(8-méthoxy-[1,2,4]triazolo[1,5-a]pyridin-6-yl)-1H-pyrazol-3-yl)pyridin-3-yl)cyclohexyl)morpholine (494-495) ;
6-(4-isopropyl-3-(5-(4-(3-méthoxyazétidin-1-yl)cyclohexyl)-4-méthylpyridin-2-yl)-1H-pyrazol-5-yl)-8-méthoxy-[1,2,4]triazolo[1,5-a]pyridine (496) ;
4-(6-(4-isopropyl-5-(8-méthoxy-[1,2,4]triazolo[1,5-a]pyridin-6-yl)-1H-pyrazol-3-yl)-4-méthylpyridin-3-yl)-N-((1-(méthylsulfonyl)cyclopropyl)méthyl)cyclohexan-1-amine (497-498) ;
4-(6-(4-isopropyl-5-(8-méthoxy-[1,2,4]triazolo[1,5-a]pyridin-6-yl)-1H-pyrazol-3-yl)-4-méthylpyridin-3-yl)-N-(2-méthoxyethyl)cyclohexan-1-amine (499-500) ;
7-(4-(6-(4-isopropyl-5-(8-méthoxy-[1,2,4]triazolo[1,5-a]pyridin-6-yl)-1H-pyrazol-3-yl)-4-méthylpyridin-3-yl)cyclohexyl)-2-oxa-7-azaspiro[3.5]nonane (501) ;
6-(3-(5-(4-(4,4-difluoropipéridin-1-yl)cyclohexyl)-4-méthylpyridin-2-yl)-4-isopropyl-1H-pyrazol-5-yl)-8-méthoxy-[1,2,4]triazolo[1,5-a]pyridine (502-503) ;
6-(4-isopropyl-3-(5-(4-(4-méthoxypipéridin-1-yl)cyclohexyl)-4-méthylpyridin-2-yl)-1H-pyrazol-5-yl)-8-méthoxy-[1,2,4]triazolo[1,5-a]pyridine (504-505) ;
4-(6-(4-isopropyl-5-(8-méthoxy-[1,2,4]triazolo[1,5-a]pyridin-6-yl)-1H-pyrazol-3-yl)-4-méthylpyridin-3-yl)-N,N-diméthylcyclohexan-1-amine (506-507) ;
4-(6-(4-isopropyl-5-(8-méthoxy-[1,2,4]triazolo[1,5-a]pyridin-6-yl)-1H-pyrazol-3-yl)-4-méthylpyridin-3-yl)cyclohexan-1-amine (508-509) ;
4-(4-(4-isopropyl-5-(8-méthyl-[1,2,4]triazolo[1,5-a]pyridin-6-yl)-1H-pyrazol-3-yl)phényl)-N,N-diméthylcyclohexan-1-amine (510 et 513) ;
N-(4-(4-(4-isopropyl-5-(8-méthyl-[1,2,4]triazolo[1,5-a]pyridin-6-yl)-1H-pyrazol-3-yl)phényl)cyclohexyl)-N-méthyloxétan-3-amine (511 et 514) ;
N-((3-éthyloxétan-3-yl)méthyl)-4-(4-(4-isopropyl-5-(8-méthyl-[1,2,4]triazolo[1,5-a]pyridin-6-yl)-1H-pyrazol-3-yl)phényl)-N-méthylcyclohexan-1-amine (512 et 515) ;
N-éthyl-4-(4-(4-isopropyl-5-(8-méthyl-[1,2,4]triazolo[1,5-a]pyridin-6-yl)-1H-pyrazol-3-yl)phényl)-N-méthylcyclohexan-1-amine (516-517) ;
2-(diméthylamino)-N-(4-(4-(4-isopropyl-5-(8-méthyl-[1,2,4]triazolo[1,5-a]pyridin-6-yl)-1H-pyrazol-3-yl)phényl)cyclohexyl)-N-méthylacétamide (518-519) ;
2-((4-(4-(4-isopropyl-5-(8-méthyl-[1,2,4]triazolo[1,5-a]pyridin-6-yl)-1H-pyrazol-3-yl)phényl)cyclohexyl)(méthyl)amino)-N,N-diméthylacétamide (520-521) ;
4-(4-(4-isopropyl-5-(8-méthyl-[1,2,4]triazolo[1,5-a]pyridin-6-yl)-1H-pyrazol-3-yl)phényl)-N-(2-méthoxyéthyl)-N-méthylcyclohexan-1-amine (522-523) ;
2-(4-(4-isopropyl-5-(8-méthoxy-[1,2,4]triazolo[1,5-a]pyridin-6-yl)-1H-pyrazol-3-yl)phényl)propan-2-amine, TFA (524) ;
2-(4-(4-isopropyl-5-(8-méthyl-[1,2,4]triazolo[1,5-a]pyridin-6-yl)-1H-pyrazol-3-yl)phényl)propan-2-amine (525) ;
1-(4-(4-isopropyl-5-(8-méthyl-[1,2,4]triazolo[1,5-a]pyridin-6-yl)-1H-pyrazol-3-yl)phényl)cyclopropan-1-amine (526) ;
(S)-1-(4-(4-isopropyl-5-(8-méthyl-[1,2,4]triazolo[1,5-a]pyridin-6-yl)-1H-pyrazol-3-yl)phényl)éthan-1-amine (527) ;
(S)-1-(4-(4-isopropyl-5-(8-méthoxy-[1,2,4]triazolo[1,5-a]pyridin-6-yl)-1H-pyrazol-3-yl)phényl)éthan-1-amine (528) ;
(R)-1-(4-(4-isopropyl-5-(8-méthyl-[1,2,4]triazolo[1,5-a]pyridin-6-yl)-1H-pyrazol-3-yl)phényl)éthan-1-amine (529) ;
6-(4-isopropyl-3-(4-(pyrrolidin-2-yl)phényl)-1H-pyrazol-5-yl)-8-méthyl-[1,2,4]triazolo[1,5-a]pyridine (530-531) ;
2-(4-(4-isopropyl-5-(8-méthoxy-[1,2,4]triazolo[1,5-a]pyridin-6-yl)-1H-pyrazol-3-yl)phényl)-N,N-diméthylpropan-2-amine (532) ;
N-isopropyl-2-(4-(4-isopropyl-5-(8-méthoxy-[1,2,4]triazolo[1,5-a]pyridin-6-yl)-1H-pyrazol-3-yl)phényl)propan-2-amine (533) ;
N-éthyl-2-(4-(4-isopropyl-5-(8-méthoxy-[1,2,4]triazolo[1,5-a]pyridin-6-yl)-1H-pyrazol-3-yl)phényl)propan-2-amine (534) ;
N,N-diéthyl-2-(4-(4-isopropyl-5-(8-méthoxy-[1,2,4]triazolo[1,5-a]pyridin-6-yl)-1H-pyrazol-3-yl)phényl)propan-2-amine (535) ;
N-éthyl-2-(4-(4-isopropyl-5-(8-méthyl-[1,2,4]triazolo[1,5-a]pyridin-6-yl)-1H-pyrazol-3-yl)phényl)propan-2-amine (536) ;
N-(2-(4-(4-isopropyl-5-(8-méthoxy-[1,2,4]triazolo[1,5-a]pyridin-6-yl)-1H-pyrazol-3-yl)phényl)propan-2-yl)tétrahydro-2H-pyran-4-amine (537) ;
2-(4-(4-isopropyl-5-(8-méthyl-[1,2,4]triazolo[1,5-a]pyridin-6-yl)-1H-pyrazol-3-yl)phényl)-N,N-diméthylpropan-2-amine (538) ;
N,N-diéthyl-2-(4-(4-isopropyl-5-(8-méthyl-[1,2,4]triazolo[1,5-a]pyridin-6-yl)-1H-pyrazol-3-yl)phényl)propan-2-amine (539) ;
N-(2-(4-(4-isopropyl-5-(8-méthyl-[1,2,4]triazolo[1,5-a]pyridin-6-yl)-1H-pyrazol-3-yl)phényl)propan-2-yl)tétrahydro-2H-pyran-4-amine (540) ;
N-(2-(4-(4-isopropyl-5-(8-méthyl-[1,2,4]triazolo[1,5-a]pyridin-6-yl)-1H-pyrazol-3-yl)phényl)propan-2-yl)-2,2-diméthylpropan-1-amine (541) ;
2-(4-(4-isopropyl-5-(8-méthyl-[1,2,4]triazolo[1,5-a]pyridin-6-yl)-1H-pyrazol-3-yl)phényl)-N-((3-méthyloxétan-3-yl)méthyl)propan-2-amine (542) ;
N-isopropyl-2-(4-(4-isopropyl-5-(8-méthyl-[1,2,4]triazolo[1,5-a]pyridin-6-yl)-1H-pyrazol-3-yl)phényl)propan-2-amine (543) ;
2-(4-(4-isopropyl-5-(8-méthyl-[1,2,4]triazolo[1,5-a]pyridin-6-yl)-1H-pyrazol-3-yl)phényl)-N-((1-méthyl-1H-1,2,4-triazol-3-yl)méthyl)propan-2-amine (544) ;
N-(2-(4-(4-isopropyl-5-(8-méthyl-[1,2,4]triazolo[1,5-a]pyridin-6-yl)-1H-pyrazol-3-yl)phényl)propan-2-yl)oxetan-3-amine (545) ;
1-(4-(4-isopropyl-5-(8-méthyl-[1,2,4]triazolo[1,5-a]pyridin-6-yl)-1H-pyrazol-3-yl)phényl)-N,N-diméthylcyclopropan-1-amine (546) ;
N-isopropyl-1-(4-(4-isopropyl-5-(8-méthyl-[1,2,4]triazolo[1,5-a]pyridin-6-yl)-1H-pyrazol-3-yl)phényl)cyclopropan-1-amine (547) ;
1-(4-(4-isopropyl-5-(8-méthyl-[1,2,4]triazolo[1,5-a]pyridin-6-yl)-1H-pyrazol-3-yl)phényl)-N-néopentylcyclopropan-1-amine (548) ;
(S)-1-(4-(4-isopropyl-5-(8-méthyl-[1,2,4]triazolo[1,5-a]pyridin-6-yl)-1H-pyrazol-3-yl)phényl)-N,N-diméthyléthan-1-amine (549) ;
(S)-N-éthyl-1-(4-(4-isopropyl-5-(8-méthyl-[1,2,4]triazolo[1,5-a]pyridin-6-yl)-1H-pyrazol-3-yl)phényl)éthan-1-amine (550) ;
(S)-N-(1-(4-(4-isopropyl-5-(8-méthyl-[1,2,4]triazolo[1,5-a]pyridin-6-yl)-1H-pyrazol-3-yl)phényl)éthyl)propan-2-amine (551) ;
N-((S)-1-(4-(4-isopropyl-5-(8-méthyl-[1,2,4]triazolo[1,5-a]pyridin-6-yl)-1H-pyrazol-3-yl)phényl)éthyl)-3-méthylbutan-2-amine (552-553) ;
(S)-N-(1-(4-(4-isopropyl-5-(8-méthyl-[1,2,4]triazolo[1,5-a]pyridin-6-yl)-1H-pyrazol-3-yl)phényl)éthyl)-2,2-diméthylpropan-1-amine (554);
(S)-1-(4-(4-isopropyl-5-(8-méthoxy-[1,2,4]triazolo[1,5-a]pyridin-6-yl)-1H-pyrazol-3-yl)phényl)-N,N-diméthyléthan-1-amine (555) ;
(S)-N-(1-(4-(4-isopropyl-5-(8-méthoxy-[1,2,4]triazolo[1,5-a]pyridin-6-yl)-1H-pyrazol-3-yl)phényl)ethyl)propan-2-amine (556) ;
(R)-1-(4-(4-isopropyl-5-(8-méthyl-[1,2,4]triazolo[1,5-a]pyridin-6-yl)-1H-pyrazol-3-yl)phényl)-N,N-diméthyléthan-1-amine (557) ;
(R)-N-(1-(4-(4-isopropyl-5-(8-méthyl-[1,2,4]triazolo[1,5-a]pyridin-6-yl)-1H-pyrazol-3-yl)phényl)éthyl)propan-2-amine (558) ;
(R)-1-(4-(4-isopropyl-5-(8-méthyl-[1,2,4]triazolo[1,5-a]pyridin-6-yl)-1H-pyrazol-3-yl)phényl)-N-((3-méthyloxétan-3-yl)méthyl)éthan-1-amine (559) ;
(R)-N-(1-(4-(4-isopropyl-5-(8-méthyl-[1,2,4]triazolo[1,5-a]pyridin-6-yl)-1H-pyrazol-3-yl)phényl)éthyl)-2,2-diméthylpropan-1-amine (560) ;
N-((R)-1-(4-(4-isopropyl-5-(8-méthyl-[1,2,4]triazolo[1,5-a]pyridin-6-yl)-1H-pyrazol-3-yl)phényl)éthyl)-3-méthylbutan-2-amine (561) ;
N-((R)-1-(4-(4-isopropyl-5-(8-méthyl-[1,2,4]triazolo[1,5-a]pyridin-6-yl)-1H-pyrazol-3-yl)phényl)éthyl)-3-méthylbutan-2-amine (562) ;
6-(4-isopropyl-3-(4-(1-méthylpyrrolidin-2-yl)phényl)-1H-pyrazol-5-yl)-8-méthyl-[1,2,4]triazolo[1,5-a]pyridine (563 et 566) ;
6-(4-isopropyl-3-(4-(1-isopropylpyrrolidin-2-yl)ph"nyl)-1H-pyrazol-5-yl)-8-méthyl-[1,2,4]triazolo[1,5-a]pyridine (564 et 567) ;
6-(4-isopropyl-3-(4-(1-(oxétan-3-yl)pyrrolidin-2-yl)phényl)-1H-pyrazol-5-yl)-8-méthyl-[1,2,4]triazolo[1,5-a]pyridine (565 et 568) ;
(S)-N-(1-(4-(4-isopropyl-5-(8-méthyl-[1,2,4]triazolo[1,5-a]pyridin-6-yl)-1H-pyrazol-3-yl)phényl)éthyl)oxétan-3-amine (569) ;
(S)-N-éthyl-1-(4-(4-isopropyl-5-(8-méthoxy-[1,2,4]triazolo[1,5-a]pyridin-6-yl)-1H-pyrazol-3-yl)phényl)-N-méthyléthan-1-amine (570) ;
(S)-1-(4-(4-isopropyl-5-(8-méthyl-[1,2,4]triazolo[1,5-a]pyridin-6-yl)-1H-pyrazol-3-yl)phényl)-N-méthyléthan-1-amine (571) ;
(S)-N-(1-(4-(4-isopropyl-5-(8-méthoxy-[1,2,4]triazolo[1,5-a]pyridin-6-yl)-1H-pyrazol-3-yl)phényl)éthyl)-N-méthylpropan-2-amine (572) ;
(S)-N-(1-(4-(4-isopropyl-5-(8-méthoxy-[1,2,4]triazolo[1,5-a]pyridin-6-yl)-1H-pyrazol-3-yl)phényl)éthyl)-N-méthyloxétan-3-amine (573) ;
(S)-1-(4-(4-isopropyl-5-(8-méthoxy-[1,2,4]triazolo[1,5-a]pyridin-6-yl)-1H-pyrazol-3-yl)phényl)-N-méthyl-N-((3-méthyloxétan-3-yl)méthyl)éthan-1-amine (574) ;
(S)-2,2,2-trifluoro-N-(1-(4-(4-isopropyl-5-(8-méthoxy-[1,2,4]triazolo[1,5-a]pyridin-6-yl)-1H-pyrazol-3-yl)phényl)éthyl)-N-méthyléthan-1-amine (575) ;
(S)-2,2,2-trifluoro-N-(1-(4-(4-isopropyl-5-(8-méthyl-[1,2,4]triazolo[1,5-a]pyridin-6-yl)-1H-pyrazol-3-yl)phényl)éthyl)-N-méthyléthan-1-amine (576) ;
(S)-N-éthyl-1-(4-(4-isopropyl-5-(8-méthyl-[1,2,4]triazolo[1,5-a]pyridin-6-yl)-1H-pyrazol-3-yl)phényl)-N-méthyléthan-1-amine (577) ;
(S)-N-(1-(4-(4-isopropyl-5-(8-méthyl-[1,2,4]triazolo[1,5-a]pyridin-6-yl)-1H-pyrazol-3-yl)phényl)éthyl)-N-méthylpropan-2-amine (578) ;
(S)-N-(1-(4-(4-isopropyl-5-(8-méthyl-[1,2,4]triazolo[1,5-a]pyridin-6-yl)-1H-pyrazol-3-yl)phényl)éthyl)-N-méthyloxétan-3-amine (579) ;
(S)-1-(4-(4-isopropyl-5-(8-méthyl-[1,2,4]triazolo[1,5-a]pyridin-6-yl)-1H-pyrazol-3-yl)phényl)-N-méthyl-N-((3-méthyloxétan-3-yl)méthyl)éthan-1-amine (580) ;
2-(diméthylamino)-N-(2-(4-(4-isopropyl-5-(8-méthyl-[1,2,4]triazolo[1,5-a]pyridin-6-yl)-1H-pyrazol-3-yl)phényl)propan-2-yl)acétamide (581) ;
2-(diméthylamino)-1-(2-(4-(4-isopropyl-5-(8-méthyl-[1,2,4]triazolo[1,5-a]pyridin-6-yl)-1H-pyrazol-3-yl)phényl)pyrrolidin-1-yl)éthan-1-one (582-583) ;
2-(2-(4-(4-isopropyl-5-(8-méthyl-[1,2,4]triazolo[1,5-a]pyridin-6-yl)-1H-pyrazol-3-yl)phényl)pyrrolidin-1-yl)-N,N-diméthylacétamide (584-585) ;
(S)-N-((S)-1-(4-(4-isopropyl-5-(8-méthoxy-[1,2,4]triazolo[1,5-a]pyridin-6-yl)-1H-pyrazol-3-yl)phényl)éthyl)-N,1-diméthylazétidine-2-carboxamide (585) ;
(S)-2-(diméthylamino)-N-(1-(4-(4-isopropyl-5-(8-méthoxy-[1,2,4]triazolo[1,5-a]pyridin-6-yl)-1H-pyrazol-3-yl)phényl)ethyl)-N-méthylacétamide (586) ;
(S)-2-(diéthylamino)-N-(1-(4-(4-isopropyl-5-(8-méthoxy-[1,2,4]triazolo[1,5-a]pyridin-6-yl)-1H-pyrazol-3-yl)phényl)éthyl)-N-méthylacétamide (587) ;
(S)-2-(diméthylamino)-N-((S)-1-(4-(4-isopropyl-5-(8-méthoxy-[1,2,4]triazolo[1,5-a]pyridin-6-yl)-1H-pyrazol-3-yl)phényl)éthyl)-N-méthylpropanamide (588) ;
(R)-2-(diméthylamino)-N-((S)-1-(4-(4-isopropyl-5-(8-méthoxy-[1,2,4]triazolo[1,5-a]pyridin-6-yl)-1H-pyrazol-3-yl)phényl)éthyl)-N-méthylpropanamide (589) ;
(S)-2-(diméthylamino)-N-(1-(4-(4-isopropyl-5-(8-méthyl-[1,2,4]triazolo[1,5-a]pyridin-6-yl)-1H-pyrazol-3-yl)phényl)éthyl)-N-méthylacétamide (590) ;
(S)-2-(diéthylamino)-N-(1-(4-(4-isopropyl-5-(8-méthyl-[1,2,4]triazolo[1,5-a]pyridin-6-yl)-1H-pyrazol-3-yl)phényl)éthyl)-N-méthylacétamide (591) ;
(R)-2-(diméthylamino)-N-((S)-1-(4-(4-isopropyl-5-(8-méthyl-[1,2,4]triazolo[1,5-a]pyridin-6-yl)-1H-pyrazol-3-yl)phényl)éthyl)-N-méthylpropanamide (592) ;
(S)-2-(diméthylamino)-N-((S)-1-(4-(4-isopropyl-5-(8-méthyl-[1,2,4]triazolo[1,5-a]pyridin-6-yl)-1H-pyrazol-3-yl)phényl)éthyl)-N-méthylpropanamide (593) ;
(S)-N-((S)-1-(4-(4-isopropyl-5-(8-méthoxy-[1,2,4]triazolo[1,5-a]pyridin-6-yl)-1H-pyrazol-3-yl)phényl)éthyl)-N-méthyl-2-(méthylamino)propanamide (594) ;
(R)-N-((S)-1-(4-(4-isopropyl-5-(8-méthoxy-[1,2,4]triazolo[1,5-a]pyridin-6-yl)-1H-pyrazol-3-yl)phényl)éthyl)-N-méthyl-2-(méthylamino)propanamide (595) ;
(S)-N-((S)-1-(4-(4-isopropyl-5-(8-méthyl-[1,2,4]triazolo[1,5-a]pyridin-6-yl)-1H-pyrazol-3-yl)phényl)éthyl)-N-méthylazétidine-2-carboxamide (596) ;
(R)-N-((S)-1-(4-(4-isopropyl-5-(8-méthyl-[1,2,4]triazolo[1,5-a]pyridin-6-yl)-1H-pyrazol-3-yl)phényl)éthyl)-N-méthyl-2-(méthylamino)propanamide (597) ;
(S)-N-((S)-1-(4-(4-isopropyl-5-(8-méthyl-[1,2,4]triazolo[1,5-a]pyridin-6-yl)-1H-pyrazol-3-yl)phényl)éthyl)-N-méthyl-2-(méthylamino)propanamide (598) ;
(S)-N-((S)-1-(4-(4-isopropyl-5-(8-méthyl-[1,2,4]triazolo[1,5-a]pyridin-6-yl)-1H-pyrazol-3-yl)phényl)éthyl)-N-méthylpyrrolidine-2-carboxamide (599) ;
(R)-N-((S)-1-(4-(4-isopropyl-5-(8-méthyl-[1,2,4]triazolo[1,5-a]pyridin-6-yl)-1H-pyrazol-3-yl)phényl)éthyl)-N-méthylpyrrolidine-2-carboxamide (600) ;
(S)-1-éthyl-N-((S)-1-(4-(4-isopropyl-5-(8-méthoxy-[1,2,4]triazolo[1,5-a]pyridin-6-yl)-1H-pyrazol-3-yl)phényl)éthyl)-N-méthylazétidine-2-carboxamide (601) ;
S)-N-((S)-1-(4-(4-isopropyl-5-(8-méthyl-[1,2,4]triazolo[1,5-a]pyridin-6-yl)-1H-pyrazol-3-yl)phényl)éthyl)-N,1-diméthylazétidine-2-carboxamide (602) ;
(S)-1-éthyl-N-((S)-1-(4-(4-isopropyl-5-(8-méthyl-[1,2,4]triazolo[1,5-a]pyridin-6-yl)-1H-pyrazol-3-yl)phényl)éthyl)-N-méthylazétidine-2-carboxamide (603) ;
(S)-N-((S)-1-(4-(4-isopropyl-5-(8-méthyl-[1,2,4]triazolo[1,5-a]pyridin-6-yl)-1H-pyrazol-3-yl)phényl)éthyl)-N-méthyl-1-(oxétan-3-yl)azétidine-2-carboxamide (604) ;
(S)-N-((S)-1-(4-(4-isopropyl-5-(8-méthyl-[1,2,4]triazolo[1,5-a]pyridin-6-yl)-1H-pyrazol-3-yl)phényl)éthyl)-N,1-diméthylpyrrolidine-2-carboxamide (605) ;
(S)-N-((S)-1-(4-(4-isopropyl-5-(8-méthyl-[1,2,4]triazolo[1,5-a]pyridin-6-yl)-1H-pyrazol-3-yl)phényl)éthyl)-N-méthyl-1-(oxétan-3-yl)pyrrolidine-2-carboxamide (606) ;
(R)-N-((S)-1-(4-(4-isopropyl-5-(8-méthyl-[1,2,4]triazolo[1,5-a]pyridin-6-yl)-1H-pyrazol-3-yl)phényl)éthyl)-N,1-diméthylpyrrolidine-2-carboxamide (607) ;
(R)-N-((S)-1-(4-(4-isopropyl-5-(8-méthyl-[1,2,4]triazolo[1,5-a]pyridin-6-yl)-1H-pyrazol-3-yl)phényl)éthyl)-N-méthyl-1-(oxétan-3-yl)pyrrolidine-2-carboxamide (608) ;
(S)-2-(éthyl(méthyl)amino)-N-((S)-1-(4-(4-isopropyl-5-(8-méthyl-[1,2,4]triazolo[1,5-a]pyridin-6-yl)-1H-pyrazol-3-yl)phényl)éthyl)-N-méthylpropanamide (609) ;
(R)-2-(éthyl(méthyl)amino)-N-((S)-1-(4-(4-isopropyl-5-(8-méthyl-[1,2,4]triazolo[1,5-a]pyridin-6-yl)-1H-pyrazol-3-yl)phényl)éthyl)-N-méthylpropanamide (610) ;
(S)-2-(éthyl(méthyl)amino)-N-((S)-1-(4-(4-isopropyl-5-(8-méthoxy-[1,2,4]triazolo[1,5-a]pyridin-6-yl)-1H-pyrazol-3-yl)phényl)éthyl)-N-méthylpropanamide (611) ;
(R)-N-((S)-1-(4-(4-isopropyl-5-(8-méthoxy-[1,2,4]triazolo[1,5-a]pyridin-6-yl)-1H-pyrazol-3-yl)phényl)éthyl)-N-méthyl-2-(méthyl(oxétan-3-yl)amino)propanamide (612) ;
(S)-2-(éthyl(méthyl)amino)-N-((S)-1-(4-(4-isopropyl-5-(8-méthoxy-[1,2,4]triazolo[1,5-a]pyridin-6-yl)-1H-pyrazol-3-yl)phényl)éthyl)-N-méthylpropanamide (613) ;
(R)-N-((S)-1-(4-(4-isopropyl-5-(8-méthyl-[1,2,4]triazolo[1,5-a]pyridin-6-yl)-1H-pyrazol-3-yl)phényl)éthyl)-N-méthyl-2-(méthyl(oxétan-3-yl)amino)propanamide (614) ;
(S)-N-((S)-1-(4-(4-isopropyl-5-(8-méthyl-[1,2,4]triazolo[1,5-a]pyridin-6-yl)-1H-pyrazol-3-yl)phényl)éthyl)-N-méthyl-2-(méthyl(oxétan-3-yl)amino)propanamide (615) ;
(S)-2-((1-(4-(4-isopropyl-5-(8-méthyl-[1,2,4]triazolo[1,5-a]pyridin-6-yl)-1H-pyrazol-3-yl)phényl)éthyl)(méthyl)amino)-N,N-diméthylacétamide (616) ;
(S)-2-((1-(4-(4-isopropyl-5-(8-méthoxy-[1,2,4]triazolo[1,5-a]pyridin-6-yl)-1H-pyrazol-3-yl)phényl)éthyl)(méthyl)amino)-N,N-diméthylacétamide (617) ;
(S)-2-(diméthylamino)-N-(1-(4-(4-isopropyl-5-(8-méthyl-[1,2,4]triazolo[1,5-a]pyridin-6-yl)-1H-pyrazol-3-yl)phényl)éthyl)-N-(oxétan-3-yl)acétamide (618) ;
(S)-2-(diéthylamino)-N-(1-(4-(4-isopropyl-5-(8-méthyl-[1,2,4]triazolo[1,5-a]pyridin-6-yl)-1H-pyrazol-3-yl)phényl)éthyl)-N-(oxétan-3-yl)acétamide (619) ;
(S)-2-(éthyl(méthyl)amino)-N-(1-(4-(4-isopropyl-5-(8-méthyl-[1,2,4]triazolo[1,5-a]pyridin-6-yl)-1H-pyrazol-3-yl)phényl)éthyl)-N-méthylacétamide (620) ;
(S)-N-(1-(4-(4-isopropyl-5-(8-méthyl-[1,2,4]triazolo[1,5-a]pyridin-6-yl)-1H-pyrazol-3-yl)phényl)éthyl)-N-méthyl-2-(méthyl(3,3,3-trifluoropropyl)amino)acétamide (621) ;
(S)-2-(3-fluoroazétidin-1-yl)-N-(1-(4-(4-isopropyl-5-(8-méthyl-[1,2,4]triazolo[1,5-a]pyridin-6-yl)-1H-pyrazol-3-yl)phényl)éthyl)-N-méthylacétamide (622) ;
(S)-2-((2-fluoro-2-méthylpropyl)amino)-N-(1-(4-(4-isopropyl-5-(8-méthyl-[1,2,4]triazolo[1,5-a]pyridin-6-yl)-1H-pyrazol-3-yl)phényl)éthyl)-N-méthylacétamide (623) ;
(S)-1-(2-fluoro-5-(4-isopropyl-5-(8-méthyl-[1,2,4]triazolo[1,5-a]pyridin-6-yl)-1H-pyrazol-3-yl)phényl)-N,N-diméthyléthan-1-amine (624) ;
(S)-N,N-diéthyl-1-(2-fluoro-5-(4-isopropyl-5-(8-méthyl-[1,2,4]triazolo[1,5-a]pyridin-6-yl)-1H-pyrazol-3-yl)phényl)ethan-1-amine (625) ;
(S)-N-(1-(2-fluoro-5-(4-isopropyl-5-(8-méthyl-[1,2,4]triazolo[1,5-a]pyridin-6-yl)-1H-pyrazol-3-yl)phényl)éthyl)propan-1-amine (626) ;
(S)-N-(1-(2-fluoro-5-(4-isopropyl-5-(8-méthyl-[1,2,4]triazolo[1,5-a]pyridin-6-yl)-1H-pyrazol-3-yl)phényl)éthyl)-N-propylpropan-1-amine (627) ;
(S)-N-(1-(2-fluoro-5-(4-isopropyl-5-(8-méthyl-[1,2,4]triazolo[1,5-a]pyridin-6-yl)-1H-pyrazol-3-yl)phényl)éthyl)propan-2-amine (628) ;
(S)-2-(dimethylamino)-N-(1-(2-fluoro-5-(4-isopropyl-5-(8-methyl-[1,2,4]triazolo[1,5a] pyridin-6-yl)-1H-pyrazol-3-yl)phenyl)ethyl)acetamide (629) ;
(S)-2-(diéthylamino)-N-(1-(2-fluoro-5-(4-isopropyl-5-(8-méthyl-[1,2,4]triazolo[1,5-a]pyridin-6-yl)-1H-pyrazol-3-yl)phényl)éthyl)acétamide (630) ;
N-(1-(4-(4-isopropyl-5-(8-méthyl-[1,2,4]triazolo[1,5-a]pyridin-6-yl)-1H-pyrazol-3-yl)phényl)éthyl)propan-2-amine (631) ;
1-(4-(4-isopropyl-5-(8-méthyl-[1,2,4]triazolo[1,5-a]pyridin-6-yl)-1H-pyrazol-3-yl)phényl)-N-méthyléthan-1-amine (632) ;
N-(1-(4-(4-isopropyl-5-(8-méthyl-[1,2,4]triazolo[1,5-a]pyridin-6-yl)-1H-pyrazol-3-yl)phényl)éthyl)oxétan-3-amine (633) ;
N-(1-(4-(4-isopropyl-5-(8-méthyl-[1,2,4]triazolo[1,5-a]pyridin-6-yl)-1H-pyrazol-3-yl)phényl)éthyl)-3-méthyloxétan-3-amine (634) ;
1-(4-(4-isopropyl-5-(8-méthyl-[1,2,4]triazolo[1,5-a]pyridin-6-yl)-1H-pyrazol-3-yl)phényl)-N,N-diméthyléthan-1-amine (635) ;
6-(3-(4-(1-(4,4-difluoropipéridin-1-yl)éthyl)phényl)-4-isopropyl-1H-pyrazol-5-yl)-8-méthyl-[1,2,4]triazolo[1,5-a]pyridine (636) ;
4-(1-(4-(4-isopropyl-5-(8-méthyl-[1,2,4]triazolo[1,5-a]pyridin-6-yl)-1H-pyrazol-3-yl)phényl)éthyl)morpholine (637) ;
6-(1-(4-(4-isopropyl-5-(8-méthyl-[1,2,4]triazolo[1,5-a]pyridin-6-yl)-1H-pyrazol-3-yl)phényl)éthyl)-2-oxa-6-azaspiro[3.3]heptane (638) ;
4-(1-(4-(4-isopropyl-5-(8-méthyl-[1,2,4]triazolo[1,5-a]pyridin-6-yl)-1H-pyrazol-3-yl)phényl)éthyl)pipérazin-2-one (639) ;
6-(1-(4-(4-isopropyl-5-(8-méthyl-[1,2,4]triazolo[1,5-a]pyridin-6-yl)-1H-pyrazol-3-yl)phényl)éthyl)-2-oxa-6-azaspiro[3.3]heptane (640-641) ;
4-(1-(4-(4-isopropyl-5-(8-méthyl-[1,2,4]triazolo[1,5-a]pyridin-6-yl)-1H-pyrazol-3-yl)phényl)éthyl)morpholine (642-643) ;
1-(4-(4-isopropyl-5-(8-méthyl-[1,2,4]triazolo[1,5-a]pyridin-6-yl)-1H-pyrazol-3-yl)phényl)-N-méthylméthanamine (644) ;
1-(4-(4-isopropyl-5-(8-méthyl-[1,2,4]triazolo[1,5-a]pyridin-6-yl)-1H-pyrazol-3-yl)phényl)-N,N-diméthylméthanamine (645 et 652) ;
N-(4-(4-isopropyl-5-(8-méthyl-[1,2,4]triazolo[1,5-a]pyridin-6-yl)-1H-pyrazol-3-yl)benzyl)propan-2-amine (646) ;
N-(4-(4-isopropyl-5-(8-méthyl-[1,2,4]triazolo[1,5-a]pyridin-6-yl)-1H-pyrazol-3-yl)benzyl)cyclopropanamine (647) ;
(1-(4-(4-isopropyl-5-(8-méthyl-[1,2,4]triazolo[1,5-a]pyridin-6-yl)-1H-pyrazol-3-yl)benzyl)azétidine-3,3-diyl)diméthanol (648) ;
N-(4-(4-isopropyl-5-(8-méthyl-[1,2,4]triazolo[1,5-a]pyridin-6-yl)-1H-pyrazol-3-yl)benzyl)-2,2-diméthylpropan-1-amine (649) ;
6-(3-(4-(azétidin-1-ylmethyl)phényl)-4-isopropyl-1H-pyrazol-5-yl)-8-méthyl-[1,2,4]triazolo[1,5-a]pyridine (650);
N-(4-(4-isopropyl-5-(8-méthyl-[1,2,4]triazolo[1,5-a]pyridin-6-yl)-1H-pyrazol-3-yl)benzyl)-N-méthyléthanamine (651) ;
N-((6-(4-isopropyl-5-(8-méthyl-[1,2,4]triazolo[1,5-a]pyridin-6-yl)-1H-pyrazol-3-yl)pyridin-3-yl)méthyl)propan-2-amine (653) ;
1-(6-(4-isopropyl-5-(8-méthyl-[1,2,4]triazolo[1,5-a]pyridin-6-yl)-1H-pyrazol-3-yl)pyridin-3-yl)-N-méthylméthanamine (654) ;
N-((5-(4-isopropyl-5-(8-méthyl-[1,2,4]triazolo[1,5-a]pyridin-6-yl)-1H-pyrazol-3-yl)pyridin-2-yl)méthyl)propan-2-amine (655) ;
1-(5-(4-isopropyl-5-(8-méthyl-[1,2,4]triazolo[1,5-a]pyridin-6-yl)-1H-pyrazol-3-yl)pyridin-2-yl)-N,N-diméthylméthanamine (656) ;
1-(5-(4-isopropyl-5-(8-méthyl-[1,2,4]triazolo[1,5-a]pyridin-6-yl)-1H-pyrazol-3-yl)pyridin-2-yl)-N-méthylméthanamine (657) ;
1-(6-(4-isopropyl-5-(8-méthyl-[1,2,4]triazolo[1,5-a]pyridin-6-yl)-1H-pyrazol-3-yl)pyridin-3-yl)-N,N-diméthyléthan-1-amine (658-659) ;
1-(5-(4-isopropyl-5-(8-méthyl-[1,2,4]triazolo[1,5-a]pyridin-6-yl)-1H-pyrazol-3-yl)thiazol-2-yl)-N-méthyléthan-1-amine (660-662) ;
1-(2-(4-isopropyl-5-(8-méthyl-[1,2,4]triazolo[1,5-a]pyridin-6-yl)-1H-pyrazol-3-yl)thiazol-5-yl)-N-méthyléthan-1-amine (663-664) ;
1-(2-(4-isopropyl-5-(8-méthyl-[1,2,4]triazolo[1,5-a]pyridin-6-yl)-1H-pyrazol-3-yl)thiazol-5-yl)-N,N-diméthyléthan-1-amine (665 et 670) ;
N-éthyl-1-(2-(4-isopropyl-5-(8-méthyl-[1,2,4]triazolo[1,5-a]pyridin-6-yl)-1H-pyrazol-3-yl)thiazol-5-yl)-N-méthyléthan-1-amine (666 et 669) ;
N-(1-(2-(4-isopropyl-5-(8-méthyl-[1,2,4]triazolo[1,5-a]pyridin-6-yl)-1H-pyrazol-3-yl)thiazol-5-yl)éthyl)-N-méthylpropan-2-amine (677-678) ;
N-(1-(2-(4-isopropyl-5-(8-méthyl-[1,2,4]triazolo[1,5-a]pyridin-6-yl)-1H-pyrazol-3-yl)thiazol-5-yl)éthyl)-N-méthyltétrahydro-2H-pyran-4-amine (671) ;
2-(diméthylamino)-N-(1-(6-(4-isopropyl-5-(8-méthyl-[1,2,4]triazolo[1,5-a]pyridin-6-yl)-1H-pyrazol-3-yl)pyridin-3-yl)éthyl)-N-méthylacétamide (672-673) ;
2-(diméthylamino)-N-(1-(2-(4-isopropyl-5-(8-méthyl-[1,2,4]triazolo[1,5-a]pyridin-6-yl)-1H-pyrazol-3-yl)thiazol-5-yl)éthyl)-N-méthylacétamide (674-675) ;
2-((1-(2-(4-isopropyl-5-(8-méthyl-[1,2,4]triazolo[1,5-a]pyridin-6-yl)-1H-pyrazol-3-yl)thiazol-5-yl)éthyl)(méthyl)amino)-N,N-diméthylacétamide (676) ;
2-((1-(2-(4-isopropyl-5-(8-méthyl-[1,2,4]triazolo[1,5-a]pyridin-6-yl)-1H-pyrazol-3-yl)thiazol-5-yl)éthyl)(méthyl)amino)-N,N-diméthylacétamide (677-678) ;
6-(4-isopropyl-5-(8-méthyl-[1,2,4]triazolo[1,5-a]pyridin-6-yl)-1H-pyrazol-3-yl)-N,N-diméthyl-1,2,3,4-tétrahydronaphtalènee-2-amine (679 et 682) ;
6-(4-isopropyl-5-(8-méthoxy-[1,2,4]triazolo[1,5-a]pyridin-6-yl)-1H-pyrazol-3-yl)-N-méthyl-1,2,3,4-tétrahydronaphthalène-2-amine (680-681) ;
6-(4-isopropyl-5-(8-méthyl-[1,2,4]triazolo[1,5-a]pyridin-6-yl)-1H-pyrazol-3-yl)-N,N-diméthyl-1,2,3,4-tétrahydronaphtalènee-2-amine (682) ;
N-isopropyl-6-(4-isopropyl-5-(8-méthyl-[1,2,4]triazolo[1,5-a]pyridin-6-yl)-1H-pyrazol-3-yl)-N-méthyl-1,2,3,4-tétrahydronaphtalènee-2-amine (683) ;
N-(6-(4-isopropyl-5-(8-méthyl-[1,2,4]triazolo[1,5-a]pyridin-6-yl)-1H-pyrazol-3-yl)-1,2,3,4-tétrahydronaphtalènee-2-yl)-N-méthyltétrahydro-2H-pyran-4-amine (684-685) ;
N-(6-(4-isopropyl-5-(8-méthyl-[1,2,4]triazolo[1,5-a]pyridin-6-yl)-1H-pyrazol-3-yl)-1,2,3,4-tétrahydronaphtalènee-2-yl)-N-méthyloxétan-3-amine (686-687) ;
6-(4-isopropyl-5-(8-méthyl-[1,2,4]triazolo[1,5-a]pyridin-6-yl)-1H-pyrazol-3-yl)-N-méthyl-N-((3-méthyloxétan-3-yl)méthyl)-1,2,3,4-tétrahydronaphtalène-2-amine (688) ;
6-(4-isopropyl-5-(8-méthoxy-[1,2,4]triazolo[1,5-a]pyridin-6-yl)-1H-pyrazol-3-yl)-N,N-diméthyl-1,2,3,4-tétrahydronaphtalènee-2-amine (689) ;
2-(diméthylamino)-N-(6-(4-isopropyl-5-(8-méthyl-[1,2,4]triazolo[1,5-a]pyridin-6-yl)-1H-pyrazol-3-yl)-1,2,3,4-tétrahydronaphtalènee-2-yl)-N-méthylacétamide (690-691) ;
2-((6-(4-isopropyl-5-(8-méthyl-[1,2,4]triazolo[1,5-a]pyridin-6-yl)-1H-pyrazol-3-yl)-1,2,3,4-tétrahydronaphtalènee-2-yl)(méthyl)amino)-N,N-diméthylacétamide (692-693) ;
2-(4-isopropyl-5-(8-méthoxy-[1,2,4]triazolo[1,5-a]pyridin-6-yl)-1H-pyrazol-3-yl)-N,N-diméthyl-4,5,6,7-tétrahydrobenzo[d]thiazol-6-amine (694 et 703) ;
2-(4-isopropyl-5-(8-méthyl-[1,2,4]triazolo[1,5-a]pyridin-6-yl)-1H-pyrazol-3-yl)-4,5,6,7-tétrahydrobenzo[d]thiazol-6-amine (695-697) ;
N-isopropyl-2-(4-isopropyl-5-(8-méthoxy-[1,2,4]triazolo[1,5-a]pyridin-6-yl)-1H-pyrazol-3-yl)-4,5,6,7-tétrahydrobenzo[d]thiazol-6-amine (698 et 704) ;
2-(4-isopropyl-5-(8-méthoxy-[1,2,4]triazolo[1,5-a]pyridin-6-yl)-1H-pyrazol-3-yl)-N-(4,4,4-trifluorobutyl)-4,5,6,7-tétrahydrobenzo[d]thiazol-6-amine (699) ;
2-(4-isopropyl-5-(8-méthoxy-[1,2,4]triazolo[1,5-a]pyridin-6-yl)-1H-pyrazol-3-yl)-N,N-bis(4,4,4-trifluorobutyl)-4,5,6,7-tétrahydrobenzo[d]thiazol-6-amine (700) ;
2-(4-isopropyl-5-(8-méthoxy-[1,2,4]triazolo[1,5-a]pyridin-6-yl)-1H-pyrazol-3-yl)-N-(tétrahydro-2H-pyran-4-yl)-4,5,6,7-tétrahydrobenzo[d]thiazol-6-amine (701 et 706) ;
2-(4-isopropyl-5-(8-méthoxy-[1,2,4]triazolo[1,5-a]pyridin-6-yl)-1H-pyrazol-3-yl)-N-(3,3,3-trifluoropropyl)-4,5,6,7-tétrahydrobenzo[d]thiazol-6-amine (702 et 705) ;
2-(4-isopropyl-5-(8-méthyl-[1,2,4]triazolo[1,5-a]pyridin-6-yl)-1H-pyrazol-3-yl)-N,N-diméthyl-4,5,6,7-tétrahydrobenzo[d]thiazol-6-amine (707 et 712) ;
N-isopropyl-2-(4-isopropyl-5-(8-méthyl-[1,2,4]triazolo[1,5-a]pyridin-6-yl)-1H-pyrazol-3-yl)-4,5,6,7-tétrahydrobenzo[d]thiazol-6-amine (708 et 713) ;
2-(4-isopropyl-5-(8-méthyl-[1,2,4]triazolo[1,5-a]pyridin-6-yl)-1H-pyrazol-3-yl)-N-(oxétan-3-yl)-4,5,6,7-tétrahydrobenzo[d]thiazol-6-amine (709 et 715) ;
2-(4-isopropyl-5-(8-méthyl-[1,2,4]triazolo[1,5-a]pyridin-6-yl)-1H-pyrazol-3-yl)-N-(tétrahydro-2H-pyran-4-yl)-4,5,6,7-tétrahydrobenzo[d]thiazol-6-amine (710 et 717) ;
N-(3-éthoxycyclobutyl)-2-(4-isopropyl-5-(8-méthyl-[1,2,4]triazolo[1,5-a]pyridin-6-yl)-1H-pyrazol-3-yl)-4,5,6,7-tétrahydrobenzo[d]thiazol-6-amine (711-716) ;
2-(4-isopropyl-5-(8-méthyl-[1,2,4]triazolo[1,5-a]pyridin-6-yl)-1H-pyrazol-3-yl)-N-(3,3,3-trifluoropropyl)-4,5,6,7-tétrahydrobenzo[d]thiazol-6-amine (714) ;
2-(4-isopropyl-5-(8-méthyl-[1,2,4]triazolo[1,5-a]pyridin-6-yl)-1H-pyrazol-3-yl)-N-(oxétan-3-yl)-4,5,6,7-tétrahydrobenzo[d]thiazol-6-amine (715) ;
2-(diméthylamino)-N-(2-(4-isopropyl-5-(8-méthoxy-[1,2,4]triazolo[1,5-a]pyridin-6-yl)-1H-pyrazol-3-yl)-4,5,6,7-tétrahydrobenzo[d]thiazol-6-yl)acétamide (718-719) ;
2-(diméthylamino)-N-(2-(4-isopropyl-5-(8-méthyl-[1,2,4]triazolo[1,5-a]pyridin-6-yl)-1H-pyrazol-3-yl)-4,5,6,7-tétrahydrobenzo[d]thiazol-6-yl)acétamide (720-721) ;
N-éthyl-2-(4-isopropyl-5-(8-méthoxy-[1,2,4]triazolo[1,5-a]pyridin-6-yl)-1H-pyrazol-3-yl)-N-méthyl-4,5,6,7-tétrahydrobenzo[d]thiazol-6-amine (722 et 728) ;
2-(4-isopropyl-5-(8-méthoxy-[1,2,4]triazolo[1,5-a]pyridin-6-yl)-1H-pyrazol-3-yl)-N-méthyl-N-propyl-4,5,6,7-tétrahydrobenzo[d]thiazol-6-amine (723 et 729) ;
N-isopropyl-2-(4-isopropyl-5-(8-méthoxy-[1,2,4]triazolo[1,5-a]pyridin-6-yl)-1H-pyrazol-3-yl)-N-méthyl-4,5,6,7-tétrahydrobenzo[d]thiazol-6-amine (724 et 730) ;
2-(4-isopropyl-5-(8-méthoxy-[1,2,4]triazolo[1,5-a]pyridin-6-yl)-1H-pyrazol-3-yl)-N-méthyl-N-(oxétan-3-yl)-4,5,6,7-tétrahydrobenzo[d]thiazol-6-amine (725 et 731) ;
2-(4-isopropyl-5-(8-méthoxy-[1,2,4]triazolo[1,5-a]pyridin-6-yl)-1H-pyrazol-3-yl)-N-méthyl-N-(3,3,3-trifluoropropyl)-4,5,6,7-tétrahydrobenzo[d]thiazol-6-amine (726 et 732) ;
2-(4-isopropyl-5-(8-méthoxy-[1,2,4]triazolo[1,5-a]pyridin-6-yl)-1H-pyrazol-3-yl)-N-méthyl-N-(tétrahydro-2H-pyran-4-yl)-4,5,6,7-tétrahydrobenzo[d]thiazol-6-amine (727-733) ;
2-(diméthylamino)-N-(2-(4-isopropyl-5-(8-méthoxy-[1,2,4] triazolo[1,5-a]pyridin-6-yl)-1H-pyrazol-3-yl)-4,5,6,7-tétrahydrobenzo[d]thiazol-6-yl)-N-méthylacétamide (734) ;
2-(diméthylamino)-N-(2-(4-isopropyl-5-(8-méthoxy-[1,2,4]triazolo[1,5-a]pyridin-6-yl)-1H-pyrazol-3-yl)-4,5,6,7-tétrahydrobenzo[d]thiazol-6-yl)-N-méthylacétamide (735) ;
2-((2-(4-isopropyl-5-(8-méthoxy-[1,2,4]triazolo[1,5-a]pyridin-6-yl)-1H-pyrazol-3-yl)-4,5,6,7-tétrahydrobenzo[d]thiazol-6-yl)(méthyl)amino)-N,N-diméthylacétamide (736) ;
2-((2-(4-isopropyl-5-(8-méthoxy-[1,2,4]triazolo[1,5-a]pyridin-6-yl)-1H-pyrazol-3-yl)-4,5,6,7-tétrahydrobenzo[d]thiazol-6-yl)(méthyl)amino)-N,N-diméthylacétamide (737) ;
2-(4-isopropyl-5-(8-méthoxy-[1,2,4]triazolo[1,5-a]pyridin-6-yl)-1H-pyrazol-3-yl)-N-(2-méthoxyéthyl)-N-méthyl-4,5,6,7-tétrahydrobenzo[d]thiazol-6-amine (738-739) ;
2-(4-isopropyl-5-(8-méthoxy-[1,2,4]triazolo[1,5-a]pyridin-6-yl)-1H-pyrazol-3-yl)-N-méthyl-N-(2-(méthylsulfonyl)éthyl)-4,5,6,7-tétrahydrobenzo[d]thiazol-6-amine (740-741) ;
2-(4-isopropyl-5-(8-méthoxy-[1,2,4]triazolo[1,5-a]pyridin-6-yl)-1H-pyrazol-3-yl)-5-(pipéridin-4-yl)thiazole (742) ;
2-(4-isopropyl-5-(8-méthylimidazo[1,2-a]pyridin-6-yl)-1H-pyrazol-3-yl)-5-(pipéridin-4-yl)thiazole (743) ;
2-(4-isopropyl-5-(8-méthoxyimidazo[1,2-a]pyridin-6-yl)-1H-pyrazol-3-yl)-5-(pipéridin-4-yl)thiazole (744) ;
2-(5-(3,4-diméthoxyphényl)-4-isopropyl-1H-pyrazol-3-yl)-5-(pipéridin-4-yl)thiazole (745) ;
2-(5-(7,8-diméthyl-[1,2,4]triazolo[1,5-a]pyridin-6-yl)-4-isopropyl-1H-pyrazol-3-yl)-5-(pipéridin-4-yl)thiazole (746) ;
2-(5-(2,6-diméthylpyridin-4-yl)-4-isopropyl-1H-pyrazol-3-yl)-5-(pipéridin-4-yl)thiazole (747) ;
5-(4-isopropyl-3-(5-(pipéridin-4-yl)thiazol-2-yl)-1H-pyrazol-5-yl)-1,3,4-triméthylpyridin-2(1H)-one (748) ;
2-(4-isopropyl-5-(8-méthoxy-[1,2,4]triazolo[1,5-a]pyridin-6-yl)-1H-pyrazol-3-yl)-4-méthyl-5-(pipéridin-4-yl)thiazole (749) ;
2-(5-(7,8-diméthyl-[1,2,4]triazolo[1,5-a]pyridin-6-yl)-4-isopropyl-1H-pyrazol-3-yl)-4-méthyl-5-(pipéridin-4-yl)thiazole (750) ;
2-(4-isopropyl-5-(8-méthoxy-[1,2,4]triazolo[1,5-a]pyridin-6-yl)-1H-pyrazol-3-yl)-5-(pipéridin-4-yl)-4-(trifluorométhyl)thiazole (751) ;
2-(4-isopropyl-5-(8-méthoxy-[1,2,4]triazolo[1,5-a]pyridin-6-yl)-1H-pyrazol-3-yl)-5-(1-((tétrahydro-2H-pyran-4-yl)méthyl)pipéridin-4-yl)thiazole (752) ;
2-(4-isopropyl-5-(8-méthoxy-[1,2,4]triazolo[1,5-a]pyridin-6-yl)-1H-pyrazol-3-yl)-5-(1-méthylpipéridin-4-yl)thiazole (753) ;
2-(4-isopropyl-5-(8-méthoxy-[1,2,4]triazolo[1,5-a]pyridin-6-yl)-1H-pyrazol-3-yl)-5-(1-(3,3,3-trifluoropropyl)pipéridin-4-yl)thiazole (754) ;
5-(1-éthylpipéridin-4-yl)-2-(4-isopropyl-5-(8-méthoxy-[1,2,4]triazolo[1,5-a]pyridin-6-yl)-1H-pyrazol-3-yl)thiazole (755) ;
2-(4-isopropyl-5-(8-méthoxy-[1,2,4]triazolo[1,5-a]pyridin-6-yl)-1H-pyrazol-3-yl)-5-(1-propylpipéridin-4-yl)thiazole (756) ;
2-(4-isopropyl-5-(8-méthoxy-[1,2,4]triazolo[1,5-a]pyridin-6-yl)-1H-pyrazol-3-yl)-5-(1-isopropylpipéridin-4-yl)thiazole (757) ;
2-(4-isopropyl-5-(8-méthoxy-[1,2,4]triazolo[1,5-a]pyridin-6-yl)-1H-pyrazol-3-yl)-5-(1-(tétrahydro-2H-pyran-4-yl)pipéridin-4-yl)thiazole (758) ;
5-(1-(3-éthoxycyclobutyl)pipéridin-4-yl)-2-(4-isopropyl-5-(8-méthoxy-[1,2,4]triazolo[1,5-a]pyridin-6-yl)-1H-pyrazol-3-yl)thiazole (759) ;
2-(4-isopropyl-5-(8-méthoxy-[1,2,4]triazolo[1,5-a]pyridin-6-yl)-1H-pyrazol-3-yl)-5-(1-(oxétan-3-yl)pipéridin-4-yl)thiazole (760) ;
2-(4-isopropyl-5-(8-méthoxy-[1,2,4]triazolo[1,5-a]pyridin-6-yl)-1H-pyrazol-3-yl)-5-(1-néopentylpipéridin-4-yl)thiazole (761) ;
5-(1-isopentylpipéridin-4-yl)-2-(4-isopropyl-5-(8-méthoxy-[1,2,4]triazolo[1,5-a]pyridin-6-yl)-1H-pyrazol-3-yl)thiazole (762) ;
5-(1-((3-éthyloxétan-3-yl)méthyl)pipéridin-4-yl)-2-(4-isopropyl-5-(8-méthoxy-[1,2,4]triazolo[1,5-a]pyridin-6-yl)-1H-pyrazol-3-yl)thiazole (763) ;
3-(4-(2-(4-isopropyl-5-(8-méthoxy-[1,2,4]triazolo[1,5-a]pyridin-6-yl)-1H-pyrazol-3-yl)thiazol-5-yl)pipéridin-1-yl)tétrahydrothiophène 1,1-dioxide (764) ;
2-(4-isopropyl-5-(8-méthoxy-[1,2,4]triazolo[1,5-a]pyridin-6-yl)-1H-pyrazol-3-yl)-5-(1-(pentan-3-yl)pipéridin-4-yl)thiazole (765) ;
2-(4-isopropyl-5-(8-méthoxy-[1,2,4]triazolo[1,5-a]pyridin-6-yl)-1H-pyrazol-3-yl)-5-(1-(tétrahydrofuran-3-yl)pipéridin-4-yl)thiazole (766) ;
2-(4-isopropyl-5-(8-méthoxy-[1,2,4]triazolo[1,5-a]pyridin-6-yl)-1H-pyrazol-3-yl)-5-(1-(tétrahydrofuran-3-yl)pipéridin-4-yl)thiazole (767) ;
2-(4-isopropyl-5-(8-méthoxy-[1,2,4]triazolo[1,5-a]pyridin-6-yl)-1H-pyrazol-3-yl)-5-(1-((tétrahydrofuran-3-yl)méthyl)pipéridin-4-yl)thiazole (786) ;
2-(4-isopropyl-5-(8-méthoxy-[1,2,4]triazolo[1,5-a]pyridin-6-yl)-1H-pyrazol-3-yl)-5-(1-((tétrahydrofuran-3-yl)méthyl)pipéridin-4-yl)thiazole (769) ;
2-(4-isopropyl-5-(8-méthoxy-[1,2,4]triazolo[1,5-a]pyridin-6-yl)-1H-pyrazol-3-yl)-5-(1-(tétrahydro-2H-pyran-3-yl)pipéridin-4-yl)thiazole (770) ;
2-(4-isopropyl-5-(8-méthoxy-[1,2,4]triazolo[1,5-a]pyridin-6-yl)-1H-pyrazol-3-yl)-5-(1-(tétrahydro-2H-pyran-3-yl)pipéridin-4-yl)thiazole (771) ;
2-(4-isopropyl-5-(8-méthyl-[1,2,4]triazolo[1,5-a]pyridin-6-yl)-1H-pyrazol-3-yl)-5-(1-méthylpipéridin-4-yl)thiazole (772) ;
5-(1-éthylpipéridin-4-yl)-2-(4-isopropyl-5-(8-méthyl-[1,2,4]triazolo[1,5-a]pyridin-6-yl)-1H-pyrazol-3-yl)thiazole (773) ;
2-(4-isopropyl-5-(8-méthyl-[1,2,4]triazolo[1,5-a]pyridin-6-yl)-1H-pyrazol-3-yl)-5-(1-isopropylpipéridin-4-yl)thiazole (774) ;
2-(4-isopropyl-5-(8-méthyl-[1,2,4]triazolo[1,5-a]pyridin-6-yl)-1H-pyrazol-3-yl)-5-(1-propylpipéridin-4-yl)thiazole (775) ;
5-(1-((3-éthyloxétan-3-yl)méthyl)pipéridin-4-yl)-2-(4-isopropyl-5-(8-méthyl-[1,2,4]triazolo[1,5-a]pyridin-6-yl)-1H-pyrazol-3-yl)thiazole (776) ;
2-(4-isopropyl-5-(8-méthyl-[1,2,4]triazolo[1,5-a]pyridin-6-yl)-1H-pyrazol-3-yl)-5-(1-(tétrahydro-2H-pyran-4-yl)pipéridin-4-yl)thiazole (777) ;
2-(4-isopropyl-5-(8-méthyl-[1,2,4]triazolo[1,5-a]pyridin-6-yl)-1H-pyrazol-3-yl)-5-(1-(oxétan-3-yl)pipéridin-4-yl)thiazole (778) ;
2-(4-isopropyl-5-(8-méthoxy-[1,2,4]triazolo[1,5-a]pyridin-6-yl)-1H-pyrazol-3-yl)-4-méthyl-5-(1-méthylpipéridin-4-yl)thiazole (779) ;
5-(1-éthylpipéridin-4-yl)-2-(4-isopropyl-5-(8-méthoxy-[1,2,4]triazolo[1,5-a]pyridin-6-yl)-1H-pyrazol-3-yl)-4-méthylthiazole (780) ;
2-(4-isopropyl-5-(8-méthoxy-[1,2,4]triazolo[1,5-a]pyridin-6-yl)-1H-pyrazol-3-yl)-4-méthyl-5-(1-(tétrahydro-2H-pyran-4-yl)pipéridin-4-yl)thiazole (781) ;
5-(1-(3-éthoxycyclobutyl)pipéridin-4-yl)-2-(4-isopropyl-5-(8-méthoxy-[1,2,4]triazolo[1,5-a]pyridin-6-yl)-1H-pyrazol-3-yl)-4-méthylthiazole (782) ;
2-(4-isopropyl-5-(8-méthoxy-[1,2,4]triazolo[1,5-a]pyridin-6-yl)-1H-pyrazol-3-yl)-4-méthyl-5-(1-propylpipéridin-4-yl)thiazole (783) ;
2-(4-isopropyl-5-(8-méthoxy-[1,2,4]triazolo[1,5-a]pyridin-6-yl)-1H-pyrazol-3-yl)-5-(1-isopropylpipéridin-4-yl)-4-méthylthiazole (784) ;
2-(4-isopropyl-5-(8-méthoxy-[1,2,4]triazolo[1,5-a]pyridin-6-yl)-1H-pyrazol-3-yl)-4-méthyl-5-(1-((tétrahydro-2H-pyran-4-yl)méthyl)pipéridin-4-yl)thiazole (785) ;
5-(1-(3-éthoxycyclobutyl)pipéridin-4-yl)-2-(4-isopropyl-5-(8-méthoxy-[1,2,4]triazolo[1,5-a]pyridin-6-yl)-1H-pyrazol-3-yl)-4-méthylthiazole (786);
5-(1-(cyclopropylméthyl)pipéridin-4-yl)-2-(4-isopropyl-5-(8-méthoxy-[1,2,4]triazolo[1,5-a]pyridin-6-yl)-1H-pyrazol-3-yl)-4-méthylthiazole (787) ;
5-(1-(cyclopropylméthyl)pipéridin-4-yl)-2-(4-isopropyl-5-(8-méthoxy-[1,2,4]triazolo[1,5-a]pyridin-6-yl)-1H-pyrazol-3-yl)thiazole (788) ;
5-(1-isobutylpipéridin-4-yl)-2-(4-isopropyl-5-(8-méthoxy-[1,2,4]triazolo[1,5-a]pyridin-6-yl)-1H-pyrazol-3-yl)thiazole (789) ;
2-(4-isopropyl-5-(8-méthoxy-[1,2,4]triazolo[1,5-a]pyridin-6-yl)-1H-pyrazol-3-yl)-5-(1-((1-méthyl-1H-1,2,4-triazol-3-yl)méthyl)pipéridin-4-yl)thiazole (790) ;
5-(1-(cyclobutylméthyl)pipéridin-4-yl)-2-(4-isopropyl-5-(8-méthoxy-[1,2,4]triazolo[1,5-a]pyridin-6-yl)-1H-pyrazol-3-yl)thiazole (791) ;
5-(1-(cyclobutylméthyl)pipéridin-4-yl)-2-(4-isopropyl-5-(8-méthoxy-[1,2,4]triazolo[1,5-a]pyridin-6-yl)-1H-pyrazol-3-yl)-4-méthylthiazole (792) ;
5-(1-isobutylpipéridin-4-yl)-2-(4-isopropyl-5-(8-méthoxy-[1,2,4]triazolo[1,5-a]pyridin-6-yl)-1H-pyrazol-3-yl)-4-méthylthiazole (793) ;
2-(5-(3,4-diméthoxyphényl)-4-isopropyl-1H-pyrazol-3-yl)-5-(1-méthylpipéridin-4-yl)thiazole (794) ;
2-(5-(3,4-diméthoxyphényl)-4-isopropyl-1H-pyrazol-3-yl)-5-(1-éthylpipéridin-4-yl)thiazole (795) ;
2-(5-(3,4-diméthoxyphényl)-4-isopropyl-1H-pyrazol-3-yl)-5-(1-propylpipéridin-4-yl)thiazole (796) ;
5-(1-(cyclopropylméthyl)pipéridin-4-yl)-2-(5-(3,4-diméthoxyphényl)-4-isopropyl-1H-pyrazol-3-yl)thiazole (797) ;
2-(5-(3,4-diméthoxyphényl)-4-isopropyl-1H-pyrazol-3-yl)-5-(1-isopropylpipéridin-4-yl)thiazole (798) ;
2-(5-(3,4-diméthoxyphényl)-4-isopropyl-1H-pyrazol-3-yl)-5-(1-(tétrahydro-2H-pyran-4-yl)pipéridin-4-yl)thiazole (799) ;
2-(5-(3,4-diméthoxyphényl)-4-isopropyl-1H-pyrazol-3-yl)-5-(1-((3-éthyloxétan-3-yl)méthyl)pipéridin-4-yl)thiazole (800) ;
2-(5-(7,8-diméthyl-[1,2,4]triazolo[1,5-a]pyridin-6-yl)-4-isopropyl-1H-pyrazol-3-yl)-5-(1-méthylpipéridin-4-yl)thiazole (801) ;
2-(5-(7,8-diméthyl-[1,2,4]triazolo[1,5-a]pyridin-6-yl)-4-isopropyl-1H-pyrazol-3-yl)-5-(1-éthylpipéridin-4-yl)thiazole (802) ;
2-(5-(7,8-dim"thyl-[1,2,4]triazolo[1,5-a]pyridin-6-yl)-4-isopropyl-1H-pyrazol-3-yl)-5-(1-propylpip"ridin-4-yl)thiazole (803) ;
2-(5-(7,8-diméthyl-[1,2,4]triazolo[1,5-a]pyridin-6-yl)-4-isopropyl-1H-pyrazol-3-yl)-5-(1-isopropylpipéridin-4-yl)thiazole (804) ;
5-(1-(cyclopropylméthyl)pipéridin-4-yl)-2-(5-(7,8-diméthyl-[1,2,4]triazolo[1,5-a]pyridin-6-yl)-4-isopropyl-1H-pyrazol-3-yl)thiazole (805) ;
2-(5-(7,8-diméthyl-[1,2,4]triazolo[1,5-a]pyridin-6-yl)-4-isopropyl-1H-pyrazol-3-yl)-5-(1-(tétrahydro-2H-pyran-4-yl)pipéridin-4-yl)thiazole (806) ;
2-(5-(7,8-diméthyl-[1,2,4]triazolo[1,5-a]pyridin-6-yl)-4-isopropyl-1H-pyrazol-3-yl)-5-(1-(3-éthoxycyclobutyl)pipéridin-4-yl)thiazole (807) ;
2-(5-(2,6-diméthylpyridin-4-yl)-4-isopropyl-1H-pyrazol-3-yl)-5-(1-méthylpipéridin-4-yl)thiazole (808) ;
2-(5-(2,6-diméthylpyridin-4-yl)-4-isopropyl-1H-pyrazol-3-yl)-5-(1-propylpipéridin-4-yl)thiazole (809) ;
2-(5-(2,6-diméthylpyridin-4-yl)-4-isopropyl-1H-pyrazol-3-yl)-5-(1-isopropylpipéridin-4-yl)thiazole (810) ;
2-(5-(2,6-diméthylpyridin-4-yl)-4-isopropyl-1H-pyrazol-3-yl)-5-(1-(tétrahydro-2H-pyran-4-yl)pipéridin-4-yl)thiazole (811) ;
2-(5-(7,8-diméthyl-[1,2,4]triazolo[1,5-a]pyridin-6-yl)-4-isopropyl-1H-pyrazol-3-yl)-4-méthyl-5-(1-méthylpipéridin-4-yl)thiazole (812) ;
2-(5-(7,8-diméthyl-[1,2,4]triazolo[1,5-a]pyridin-6-yl)-4-isopropyl-1H-pyrazol-3-yl)-5-(1-isopropylpipéridin-4-yl)thiazole (813) ;
5-(1-(cyclopropylmethyl)pipéridin-4-yl)-2-(5-(7,8-diméthyl-[1,2,4]triazolo[1,5-a]pyridin-6-yl)-4-isopropyl-1H-pyrazol-3-yl)-4-méthylthiazole (814) ;
5-(3-(5-(1-éthylpipéridin-4-yl)thiazol-2-yl)-4-isopropyl-1H-pyrazol-5-yl)-1,3,4-triméthylpyridin-2(1H)-one (815) ;
5-(4-isopropyl-3-(5-(1-méthylpipéridin-4-yl)thiazol-2-yl)-1H-pyrazol-5-yl)-1,3,4-triméthylpyridin-2(1H)-one (816) ;
2-(5-(7,8-diméthyl-[1,2,4]triazolo[1,5-a]pyridin-6-yl)-4-isopropyl-1H-pyrazol-3-yl)-5-(1-((tétrahydro-2H-pyran-4-yl)méthyl)pipéridin-4-yl)thiazole (817) ;
5-(4-isopropyl-3-(5-(1-propylpipéridin-4-yl)thiazol-2-yl)-1H-pyrazol-5-yl)-1,3,4-triméthylpyridin-2(1H)-one (818 );
5-(3-(5-(1-(cyclopropylméthyl)pipéridin-4-yl)thiazol-2-yl)-4-isopropyl-1H-pyrazol-5-yl)-1,3,4-triméthylpyridin-2(1H)-one (819) ;
5-(1-(2-éthylbutyl)pipéridin-4-yl)-2-(4-isopropyl-5-(8-méthoxyimidazo[1,2-a]pyridin-6-yl)-1H-pyrazol-3-yl)thiazole (820) ;
5-(1-isobutylpipéridin-4-yl)-2-(4-isopropyl-5-(8-méthoxyimidazo[1,2-a]pyridin-6-yl)-1H-pyrazol-3-yl)thiazole (821) ;
2-(4-isopropyl-5-(8-méthoxyimidazo[1,2-a]pyridin-6-yl)-1H-pyrazol-3-yl)-5-(1-propylpipéridin-4-yl)thiazole (822) ;
2-(4-isopropyl-5-(8-méthoxyimidazo[1,2-a]pyridin-6-yl)-1H-pyrazol-3-yl)-5-(1-(tétrahydro-2H-pyran-4-yl)pipéridin-4-yl)thiazole (823) ;
2-(4-isopropyl-5-(8-méthoxyimidazo[1,2-a]pyridin-6-yl)-1H-pyrazol-3-yl)-5-(1-(3,3,3-trifluoropropyl)pipéridin-4-yl)thiazole (824) ;
5-(1-cyclobutylpipéridin-4-yl)-2-(4-isopropyl-5-(8-méthoxyimidazo[1,2-a]pyridin-6-yl)-1H-pyrazol-3-yl)thiazole (825) ;
2-(4-isopropyl-5-(8-méthoxyimidazo[1,2-a]pyridin-6-yl)-1H-pyrazol-3-yl)-5-(1-(oxétan-3-yl)pipéridin-4-yl)thiazole (826) ;
2-(4-isopropyl-5-(8-méthoxyimidazo[1,2-a]pyridin-6-yl)-1H-pyrazol-3-yl)-5-(1-(pentan-3-yl)pipéridin-4-yl)thiazole (827) ;
2-(4-isopropyl-5-(8-méthoxyimidazo[1,2-a]pyridin-6-yl)-1H-pyrazol-3-yl)-5-(1-isopropylpipéridin-4-yl)thiazole (828) ;
2-(4-isopropyl-5-(8-méthoxyimidazo[1,2-a]pyridin-6-yl)-1H-pyrazol-3-yl)-5-(1-méthylpipéridin-4-yl)thiazole (829) ;
2-(4-isopropyl-5-(8-méthoxyimidazo[1,2-a]pyridin-6-yl)-1H-pyrazol-3-yl)-5-(1-néopentylpipéridin-4-yl)thiazole (830) ;
5-(1-éthylpipéridin-4-yl)-2-(4-isopropyl-5-(8-méthoxyimidazo[1,2-a]pyridin-6-yl)-1H-pyrazol-3-yl)thiazole (831) ;
5-(1-(cyclopropylméthyl)pipéridin-4-yl)-2-(4-isopropyl-5-(8-méthoxyimidazo[1,2-a]pyridin-6-yl)-1H-pyrazol-3-yl)thiazole (832) ;
2-(5-(7,8-diméthyl-[1,2,4]triazolo[1,5-a]pyridin-6-yl)-4-isopropyl-1H-pyrazol-3-yl)-4-méthyl-5-(1-propylpipéridin-4-yl)thiazole (833) ;
2-(5-(7,8-diméthyl-[1,2,4]triazolo[1,5-a]pyridin-6-yl)-4-isopropyl-1H-pyrazol-3-yl)-4-méthyl-5-(1-((tétrahydro-2H-pyran-4-yl)méthyl)pipéridin-4-yl)thiazole (834) ;
2-(5-(7,8-diméthyl-[1,2,4]triazolo[1,5-a]pyridin-6-yl)-4-isopropyl-1H-pyrazol-3-yl)-4-méthyl-5-(1-(oxétan-3-yl)pipéridin-4-yl)thiazole (835) ;
2-(5-(7,8-diméthyl-[1,2,4]triazolo[1,5-a]pyridin-6-yl)-4-isopropyl-1H-pyrazol-3-yl)-4-méthyl-5-(1-(tétrahydro-2H-pyran-4-yl)pipéridin-4-yl)thiazole (836) ;
2-(4-isopropyl-5-(8-méthoxy-[1,2,4]triazolo[1,5-a]pyridin-6-yl)-1H-pyrazol-3-yl)-5-(1-(oxétan-3-yl)pipéridin-4-yl)-4-(trifluorométhyl)thiazole (837) ;
5-(1-(cyclopropylméthyl)pipéridin-4-yl)-2-(4-isopropyl-5-(8-méthoxy-[1,2,4]triazolo[1,5-a]pyridin-6-yl)-1H-pyrazol-3-yl)-4-(trifluorométhyl)thiazole (838) ;
2-(4-isopropyl-5-(8-méthoxy-[1,2,4]triazolo[1,5-a]pyridin-6-yl)-1H-pyrazol-3-yl)-5-(1-isopropylpipéridin-4-yl)-4-(trifluorométhyl)thiazole (839) ;
2-(4-isopropyl-5-(8-méthoxy-[1,2,4]triazolo[1,5-a]pyridin-6-yl)-1H-pyrazol-3-yl)-5-(1-((tétrahydro-2H-pyran-4-yl)méthyl)pipéridin-4-yl)-4-(trifluorométhyl)thiazole (840) ;
2-(4-isopropyl-5-(8-méthoxy-[1,2,4]triazolo[1,5-a]pyridin-6-yl)-1H-pyrazol-3-yl)-5-(1-méthylpipéridin-4-yl)-4-(trifluorométhyl)thiazole (841) ;
5-(1-éthylpipéridin-4-yl)-2-(4-isopropyl-5-(8-méthoxy-[1,2,4]triazolo[1,5-a]pyridin-6-yl)-1H-pyrazol-3-yl)-4-(trifluorométhyl)thiazole (842) ;
2-(4-isopropyl-5-(8-méthoxy-[1,2,4]triazolo[1,5-a]pyridin-6-yl)-1H-pyrazol-3-yl)-5-(1-propylpipéridin-4-yl)-4-(trifluorométhyl)thiazole (843) ;
1-(4-(2-(4-isopropyl-5-(8-méthoxy-[1,2,4]triazolo[1,5-a]pyridin-6-yl)-1H-pyrazol-3-yl)thiazol-5-yl)pipéridin-1-yl)-2-morpholinoéthan-1-one (844) ;
2-(diméthylamino)-1-(4-(2-(4-isopropyl-5-(8-méthoxy-[1,2,4]triazolo[1,5-a]pyridin-6-yl)-1H-pyrazol-3-yl)thiazol-5-yl)pipéridin-1-yl)éthan-1-one (845) ;
2-(dim"thylamino)-1-(4-(2-(4-isopropyl-5-(8-m"thoxy-[1,2,4]triazolo[1,5-a]pyridin-6-yl)-1H-pyrazol-3-yl)-4-m"thylthiazol-5-yl)pipéridin-1-yl)"than-1-one (846) ;
2-(diéthylamino)-1-(4-(2-(4-isopropyl-5-(8-méthoxy-[1,2,4]triazolo[1,5-a]pyridin-6-yl)-1H-pyrazol-3-yl)thiazol-5-yl)pipéridin-1-yl)éthan-1-one (847) ;
2-(diéthylamino)-1-(4-(2-(4-isopropyl-5-(8-méthoxy-[1,2,4]triazolo[1,5-a]pyridin-6-yl)-1H-pyrazol-3-yl)-4-méthylthiazol-5-yl)pipéridin-1-yl)éthan-1-one (848) ;
2-(diméthylamino)-1-(4-(2-(4-isopropyl-5-(8-méthyl-[1,2,4]triazolo[1,5-a]pyridin-6-yl)-1H-pyrazol-3-yl)thiazol-5-yl)pipéridin-1-yl)éthan-1-one (849) ;
1-(4-(2-(5-(7,8-diméthyl-[1,2,4]triazolo[1,5-a]pyridin-6-yl)-4-isopropyl-1H-pyrazol-3-yl)-4-méthylthiazol-5-yl)pipéridin-1-yl)-2-(diméthylamino)éthan-1-one (850) ;
1-(4-(2-(5-(7,8-diméthyl-[1,2,4]triazolo[1,5-a]pyridin-6-yl)-4-isopropyl-1H-pyrazol-3-yl)thiazol-5-yl)pipéridin-1-yl)-2-(diméthylamino)éthan-1-one (851) ;
2-(diméthylamino)-1-(4-(2-(5-(2,6-diméthylpyridin-4-yl)-4-isopropyl-1H-pyrazol-3-yl)thiazol-5-yl)pipéridin-1-yl)éthan-1-one (852) ;
1-(4-(2-(5-(3,4-diméthoxyphenyl)-4-isopropyl-1H-pyrazol-3-yl)thiazol-5-yl)pipéridin-1-yl)-2-(diméthylamino)éthan-1-one (853) ;
2-(diméthylamino)-1-(4-(2-(4-isopropyl-5-(8-méthoxy-[1,2,4]triazolo[1,5-a]pyridin-6-yl)-1H-pyrazol-3-yl)-4-(trifluorométhyl)thiazol-5-yl)pipéridin-1-yl)éthan-1-one (854) ;
2-(4-isopropyl-5-(8-méthoxy-[1,2,4]triazolo[1,5-a]pyridin-6-yl)-1H-pyrazol-3-yl)-5-(1-(2-(méthylsulfonyl)éthyl)pipéridin-4-yl)thiazole (855) ;
2-(4-(2-(4-isopropyl-5-(8-méthoxy-[1,2,4]triazolo[1,5-a]pyridin-6-yl)-1H-pyrazol-3-yl)thiazol-5-yl)pipéridin-1-yl)-N,N-diméthylacétamide (856) ;
2-(4-(2-(4-isopropyl-5-(8-méthoxy-[1,2,4]triazolo[1,5-a]pyridin-6-yl)-1H-pyrazol-3-yl)thiazol-5-yl)pipéridin-1-yl)acétamide (857) ;
2-(4-isopropyl-5-(8-méthoxy-[1,2,4]triazolo[1,5-a]pyridin-6-yl)-1H-pyrazol-3-yl)-5-(1-(2-méthoxyéthyl)pipéridin-4-yl)thiazole (858) ;
2-(4-(2-(4-isopropyl-5-(8-méthoxy-[1,2,4]triazolo[1,5-a]pyridin-6-yl)-1H-pyrazol-3-yl)thiazol-5-yl)pipéridin-1-yl)acétonitrile (859) ;
1-(4-(2-(4-isopropyl-5-(8-méthoxy-[1,2,4]triazolo[1,5-a]pyridin-6-yl)-1H-pyrazol-3-yl)thiazol-5-yl)pipéridin-1-yl)-2-méthylpropan-2-ol (860) ;
2-(4-(2-(4-isopropyl-5-(8-méthoxy-[1,2,4]triazolo[1,5-a]pyridin-6-yl)-1H-pyrazol-3-yl)-4-méthylthiazol-5-yl)pipéridin-1-yl)-N,N-diméthylacétamide (861) ;
2-(4-isopropyl-5-(8-méthoxy-[1,2,4]triazolo[1,5-a]pyridin-6-yl)-1H-pyrazol-3-yl)-5-(1-(2-méthoxyéthyl)pipéridin-4-yl)-4-méthylthiazole (862) ;
2-(4-isopropyl-5-(8-méthoxy-[1,2,4]triazolo[1,5-a]pyridin-6-yl)-1H-pyrazol-3-yl)-4-méthyl-5-(1-(2-(méthylsulfonyl)éthyl)pipéridin-4-yl)thiazole (863) ;
2-(4-isopropyl-5-(8-méthoxy-[1,2,4]triazolo[1,5-a]pyridin-6-yl)-1H-pyrazol-3-yl)-5-(1-((1-(méthylsulfonyl)cyclopropyl)méthyl)pipéridin-4-yl)thiazole (864) ;
2-(4-isopropyl-5-(8-méthyl-[1,2,4]triazolo[1,5-a]pyridin-6-yl)-1H-pyrazol-3-yl)-5-(1-(2-(méthylsulfonyl)ethyl)pipéridin-4-yl)thiazole (865) ;
2-(4-isopropyl-5-(8-méthyl-[1,2,4]triazolo[1,5-a]pyridin-6-yl)-1H-pyrazol-3-yl)-5-(1-(2-méthoxyéthyl)pipéridin-4-yl)thiazole (866) ;
1-(4-(2-(4-isopropyl-5-(8-méthoxy-[1,2,4]triazolo[1,5-a]pyridin-6-yl)-1H-pyrazol-3-yl)thiazol-5-yl)pipéridin-1-yl)-2-méthylpropan-2-ol (867) ;
2-(4-(2-(4-isopropyl-5-(8-méthyl-[1,2,4]triazolo[1,5-a]pyridin-6-yl)-1H-pyrazol-3-yl)thiazol-5-yl)pipéridin-1-yl)acétonitrile (868) ;
2-(4-(2-(4-isopropyl-5-(8-méthyl-[1,2,4]triazolo[1,5-a]pyridin-6-yl)-1H-pyrazol-3-yl)thiazol-5-yl)pipéridin-1-yl)-N,N-diméthylacétamide (869) ;
2-(4-(2-(5-(7,8-diméthyl-[1,2,4]triazolo[1,5-a]pyridin-6-yl)-4-isopropyl-1H-pyrazol-3-yl)thiazol-5-yl)pipéridin-1-yl)-N,N-diméthylacétamide (870) ;
2-(5-(7,8-diméthyl-[1,2,4]triazolo[1,5-a]pyridin-6-yl)-4-isopropyl-1H-pyrazol-3-yl)-5-(1-(2-méthoxyéthyl)pipéridin-4-yl)thiazole (871) ;
2-(5-(7,8-diméthyl-[1,2,4]triazolo[1,5-a]pyridin-6-yl)-4-isopropyl-1H-pyrazol-3-yl)-5-(1-(2-méthoxyéthyl)pipéridin-4-yl)-4-méthylthiazole (872) ;
2-(5-(7,8-diméthyl-[1,2,4]triazolo[1,5-a]pyridin-6-yl)-4-isopropyl-1H-pyrazol-3-yl)-4-méthyl-5-(1-(2-(méthylsulfonyl)éthyl)pipéridin-4-yl)thiazole (873) ;
2-(5-(2,6-diméthylpyridin-4-yl)-4-isopropyl-1H-pyrazol-3-yl)-5-(1-(2-méthoxyéthyl)pipéridin-4-yl)thiazole (874) ;
2-(4-(2-(5-(2,6-diméthylpyridin-4-yl)-4-isopropyl-1H-pyrazol-3-yl)thiazol-5-yl)pipéridin-1-yl)-N,N-diméthylacétamide (875) ;
2-(5-(3,4-diméthoxyphényl)-4-isopropyl-1H-pyrazol-3-yl)-5-(1-(2-méthoxyéthyl)pipéridin-4-yl)thiazole (877) ;
2-(4-isopropyl-5-(8-méthoxy-[1,2,4]triazolo[1,5-a]pyridin-6-yl)-1H-pyrazol-3-yl)-5-(1-(2-(méthylsulfonyl)éthyl)pipéridin-4-yl)-4-(trifluorométhyl)thiazole (878) ;
2-(4-(2-(4-isopropyl-5-(8-méthoxy-[1,2,4]triazolo[1,5-a]pyridin-6-yl)-1H-pyrazol-3-yl)-4-(trifluorométhyl)thiazol-5-yl)pipéridin-1-yl)-N,N-diméthylacétamide (879) ;
2-(4-isopropyl-5-(8-méthoxy-[1,2,4]triazolo[1,5-a]pyridin-6-yl)-1H-pyrazol-3-yl)-5-(1-(2-méthoxyéthyl)pipéridin-4-yl)-4-(trifluorométhyl)thiazole (880) ;
5-(1-(3-fluoropropyl)pipéridin-4-yl)-2-(4-isopropyl-5-(8-méthoxy-[1,2,4]triazolo[1,5-a]pyridin-6-yl)-1H-pyrazol-3-yl)-4-méthylthiazole (881) ;
5-(1-(2-fluoroéthyl)pipéridin-4-yl)-2-(4-isopropyl-5-(8-méthoxy-[1,2,4]triazolo[1,5-a]pyridin-6-yl)-1H-pyrazol-3-yl)-4-méthylthiazole (882) ;
4-(4-(2-(4-isopropyl-5-(8-méthoxy-[1,2,4]triazolo[1,5-a]pyridin-6-yl)-1H-pyrazol-3-yl)-4-méthylthiazol-5-yl)cyclohexyl)morpholine (883) ;
4-(2-(4-isopropyl-5-(8-méthoxy-[1,2,4]triazolo[1,5-a]pyridin-6-yl)-1H-pyrazol-3-yl)-4-méthylthiazol-5-yl)-N-(2-méthoxyéthyl)cyclohexan-1-amine (884-885) ;
N-(4-(2-(4-isopropyl-5-(8-méthoxy-[1,2,4]triazolo[1,5-a]pyridin-6-yl)-1H-pyrazol-3-yl)-4-méthylthiazol-5-yl)cyclohexyl)oxétan-3-amine (886-887) ;
4-(2-(4-isopropyl-5-(8-méthoxy-[1,2,4]triazolo[1,5-a]pyridin-6-yl)-1H-pyrazol-3-yl)-4-méthylthiazol-5-yl)-N-(2-méthoxyéthyl)-N-méthylcyclohexan-1-amine (888-889) ;
6-(4-(2-(4-isopropyl-5-(8-méthoxy-[1,2,4]triazolo[1,5-a]pyridin-6-yl)-1H-pyrazol-3-yl)-4-méthylthiazol-5-yl)cyclohexyl)-2-oxa-6-azaspiro[3.3]heptane (890-891) ;
4-(2-(4-isopropyl-5-(8-méthoxy-[1,2,4]triazolo[1,5-a]pyridin-6-yl)-1H-pyrazol-3-yl)-4-méthylthiazol-5-yl)-N-((1-(méthylsulfonyl)cyclopropyl)méthyl)cyclohexan-1-amine (892-893) ;
6-(4-(2-(4-isopropyl-5-(8-méthoxy-[1,2,4]triazolo[1,5-a]pyridin-6-yl)-1H-pyrazol-3-yl)-4-méthylthiazol-5-yl)cyclohexyl)-2-thia-6-azaspiro[3.3]heptane 2,2-dioxide (894-895) ;
N-(4-(2-(4-isopropyl-5-(8-méthoxy-[1,2,4]triazolo[1,5-a]pyridin-6-yl)-1H-pyrazol-3-yl)-4-méthylthiazol-5-yl)cyclohexyl)tétrahydro-2H-pyran-4-amine (896-897) ;
2-(4-isopropyl-5-(8-méthoxy-[1,2,4]triazolo[1,5-a]pyridin-6-yl)-1H-pyrazol-3-yl)-4-méthyl-5-(4-(pyrrolidin-1-yl)cyclohexyl)thiazole (898) ;
N-cyclobutyl-4-(2-(4-isopropyl-5-(8-méthoxy-[1,2,4]triazolo[1,5-a]pyridin-6-yl)-1H-pyrazol-3-yl)-4-méthylthiazol-5-yl)cyclohexan-1-amine (891B et 892B) ;
4-(4-(2-(4-isopropyl-5-(8-méthoxy-[1,2,4]triazolo[1,5-a]pyridin-6-yl)-1H-pyrazol-3-yl)-4-méthylthiazol-5-yl)cyclohexyl)pipérazin-2-one (893B et 894B) ;
2-(4-isopropyl-5-(8-méthoxy-[1,2,4]triazolo[1,5-a]pyridin-6-yl)-1H-pyrazol-3-yl)-4-méthyl-5-(4-(4-(méthylsulfonyl)pipérazin-1-yl)cyclohexyl)thiazole (895B et 896B) ;
4-(2-(4-isopropyl-5-(8-méthoxy-[1,2,4]triazolo[1,5-a]pyridin-6-yl)-1H-pyrazol-3-yl)-4-méthylthiazol-5-yl)-N-(2-méthoxy-2-méthylpropyl)cyclohexan-1-amine (897B et 898B) ;
4-(2-(4-isopropyl-5-(8-méthoxy-[1,2,4]triazolo[1,5-a]pyridin-6-yl)-1H-pyrazol-3-yl)-4-méthylthiazol-5-yl)-N-((3-méthyloxetan-3-yl)méthyl)cyclohexan-1-amine (899B et 900) ;
2-(4-(2-(4-isopropyl-5-(8-méthoxy-[1,2,4]triazolo[1,5-a]pyridin-6-yl)-1H-pyrazol-3-yl)-4-méthylthiazol-5-yl)cyclohexyl)-7-oxa-2-azaspiro[3.5]nonane (901-902) ;
4-(4-(2-(4-isopropyl-5-(8-méthoxy-[1,2,4]triazolo[1,5-a]pyridin-6-yl)-1H-pyrazol-3-yl)-4-méthylthiazol-5-yl)cyclohexyl)-1,4-oxazépane (903-904) ;
2-(4-isopropyl-5-(8-méthoxy-[1,2,4]triazolo[1,5-a]pyridin-6-yl)-1H-pyrazol-3-yl)-5-(4-(3-méthoxyazétidin-1-yl)cyclohexyl)-4-méthylthiazole (905-906) ;
7-(4-(2-(4-isopropyl-5-(8-méthoxy-[1,2,4]triazolo[1,5-a]pyridin-6-yl)-1H-pyrazol-3-yl)-4-méthylthiazol-5-yl)cyclohexyl)-2-oxa-7-azaspiro[3.5]nonane (907-908) ;
1-(4-(2-(4-isopropyl-5-(8-méthoxy-[1,2,4]triazolo[1,5-a]pyridin-6-yl)-1H-pyrazol-3-yl)-4-méthylthiazol-5-yl)cyclohexyl)-3-(trifluorométhyl)azétidin-3-ol (909-910) ;
4-(2-(4-isopropyl-5-(8-méthyl-[1,2,4]triazolo[1,5-a]pyridin-6-yl)-1H-pyrazol-3-yl)thiazol-5-yl)-N-méthylcyclohexan-1-amine (911-912) ;
4-(2-(4-isopropyl-5-(8-méthyl-[1,2,4]triazolo[1,5-a]pyridin-6-yl)-1H-pyrazol-3-yl)thiazol-5-yl)-N,N-diméthylcyclohexan-1-amine (913-914) ;
N-(4-(2-(4-isopropyl-5-(8-méthoxy-[1,2,4]triazolo[1,5-a]pyridin-6-yl)-1H-pyrazol-3-yl)thiazol-5-yl)cyclohexyl)-3-méthyloxétan-3-amine (915-916) ;
4-(2-(4-isopropyl-5-(8-méthoxy-[1,2,4]triazolo[1,5-a]pyridin-6-yl)-1H-pyrazol-3-yl)thiazol-5-yl)-N-(2-méthoxy-2-méthylpropyl)cyclohexan-1-amine (916-917) ;
N-cyclobutyl-4-(2-(4-isopropyl-5-(8-méthoxy-[1,2,4]triazolo[1,5-a]pyridin-6-yl)-1H-pyrazol-3-yl)thiazol-5-yl)cyclohexan-1-amine (918-919) ;
la N -(4-(2-(4-isopropyl-5-(8-méthoxy-[1,2,4]triazolo[1,5-a]pyridin-6-yl)-1H-pyrazol-3-yl)thiazol-5-yl)cyclohexyl)oxétan-3-amine (920-921) ;
4-(2-(4-isopropyl-5-(8-méthoxy-[1,2,4]triazolo[1,5-a]pyridin-6-yl)-1H-pyrazol-3-yl)thiazol-5-yl)-N-(3,3,3-trifluoropropyl)cyclohexan-1-amine (922-923) ;
6-(4-(2-(4-isopropyl-5-(8-méthoxy-[1,2,4]triazolo[1,5-a]pyridin-6-yl)-1H-pyrazol-3-yl)thiazol-5-yl)cyclohexyl)-2-oxa-6-azaspiro[3.3]heptane (924) ;
2-(diméthylamino)-N-(4-(2-(4-isopropyl-5-(8-meéhyl-[1,2,4]triazolo[1,5-a]pyridin-6-yl)-1H-pyrazol-3-yl)thiazol-5-yl)cyclohexyl)-N-méthylacétamide (926-927) ;
4-(2-(4-isopropyl-5-(8-méthyl-[1,2,4]triazolo[1,5-a]pyridin-6-yl)-1H-pyrazol-3-yl)thiazol-5-yl)-N-méthyl-N-(3,3,3-trifluoropropyl)cyclohexan-1-amine (928-929) ;
2-(4-isopropyl-5-(8-méthoxy-[1,2,4]triazolo[1,5-a]pyridin-6-yl)-1H-pyrazol-3-yl)-5-(pipérazin-1-yl)thiazole (930) ;
2-(4-isopropyl-5-(8-méthoxy-[1,2,4]triazolo[1,5-a]pyridin-6-yl)-1H-pyrazol-3-yl)-5-(2,6-diazaspiro[3.3]heptan-2-yl)thiazole (931) ;
1-(2-(4-isopropyl-5-(8-méthoxy-[1,2,4]triazolo[1,5-a]pyridin-6-yl)-1H-pyrazol-3-yl)-4-méthylthiazol-5-yl)pipéridin-4-amine (932) ;
(S)-2-(4-isopropyl-5-(8-méthoxy-[1,2,4]triazolo[1,5-a]pyridin-6-yl)-1H-pyrazol-3-yl)-5-(2-méthylpipérazin-1-yl)thiazole (933) ;
2-(4-isopropyl-5-(8-méthoxy-[1,2,4]triazolo[1,5-a]pyridin-6-yl)-1H-pyrazol-3-yl)-4-méthyl-5-(pipérazin-1-yl)thiazole (934) ;
5-((15,45)-2,5-diazabicyclo[2.2.1]heptan-2-yl)-2-(4-isopropyl-5-(8-méthoxy-[1,2,4]triazolo[1,5-a]pyridin-6-yl)-1H-pyrazol-3-yl)thiazole (935) ; 5-((2R,5S)-2,5-diméthylpipérazin-1-yl)-2-(4-isopropyl-5-(8-méthoxy-[1,2,4]triazolo[1,5-a]pyridin-6-yl)-1H-pyrazol-3-yl)thiazole (936) ;
(R)-2-(4-isopropyl-5-(8-méthoxy-[1,2,4]triazolo[1,5-a]pyridin-6-yl)-1H-pyrazol-3-yl)-4-méthyl-5-(2-méthylpipérazin-1-yl)thiazole (937) ;
(R)-2-(4-isopropyl-5-(8-méthoxy-[1,2,4]triazolo[1,5-a]pyridin-6-yl)-1H-pyrazol-3-yl)-5-(2-méthylpipérazin-1-yl)thiazole (938) ;
2-(4-isopropyl-5-(8-méthoxy-[1,2,4]triazolo[1,5-a]pyridin-6-yl)-1H-pyrazol-3-yl)-5-(4-méthyl pipérazin-1-yl)thiazole (939) ;
5-(4-éthylpipérazin-1-yl)-2-(4-isopropyl-5-(8-méthoxy-[1,2,4]triazolo[1,5-a]pyridin-6-yl)-1H-pyrazol-3-yl)thiazole (940) ;
2-(4-isopropyl-5-(8-méthoxy-[1,2,4]triazolo[1,5-a]pyridin-6-yl)-1H-pyrazol-3-yl)-5-(4-propylpipérazin-1-yl)thiazole (941) ;
2-(4-isopropyl-5-(8-méthoxy-[1,2,4]triazolo[1,5-a]pyridin-6-yl)-1H-pyrazol-3-yl)-5-(4-isopropylpipérazin-1-yl)thiazole (942) ;
2-(4-isopropyl-5-(8-méthoxy-[1,2,4]triazolo[1,5-a]pyridin-6-yl)-1H-pyrazol-3-yl)-5-(4-(oxétan-3-yl)piperazin-1-yl)thiazole (943) ;
5-(4-isobutylpipérazin-1-yl)-2-(4-isopropyl-5-(8-méthoxy-[1,2,4]triazolo[1,5-a]pyridin-6-yl)-1H-pyrazol-3-yl)thiazole (944) ;
2-(4-isopropyl-5-(8-méthoxy-[1,2,4]triazolo[1,5-a]pyridin-6-yl)-1H-pyrazol-3-yl)-5-(4-(tétrahydro-2H-pyran-4-yl)pipérazin-1-yl)thiazole (945) ;
5-(4-(cyclopropylméthyl)pipérazin-1-yl)-2-(4-isopropyl-5-(8-méthoxy-[1,2,4]triazolo[1,5-a]pyridin-6-yl)-1H-pyrazol-3-yl)thiazole (946) ;
2-(4-isopropyl-5-(8-méthoxy-[1,2,4]triazolo[1,5-a]pyridin-6-yl)-1H-pyrazol-3-yl)-5-(4-((tétrahydro-2H-pyran-4-yl)méthyl)pipérazin-1-yl)thiazole (947) ;
2-(4-isopropyl-5-(8-méthoxy-[1,2,4]triazolo[1,5-a]pyridin-6-yl)-1H-pyrazol-3-yl)-5-(6-isopropyl-2,6-diazaspiro[3.3]heptan-2-yl)thiazole (948) ;
N-isopropyl-1-(2-(4-isopropyl-5-(8-méthoxy-[1,2,4]triazolo[1,5-a]pyridin-6-yl)-1H-pyrazol-3-yl)-4-méthylthiazol-5-yl)pipéridin-4-amine (949) ;
1-(2-(4-isopropyl-5-(8-méthoxy-[1,2,4]triazolo[1,5-a]pyridin-6-yl)-1H-pyrazol-3-yl)-4-méthylthiazol-5-yl)-N-(oxétan-3-yl)pipéridin-4-amine (950) ;
(S)-5-(4-(cyclopropylméthyl)-2-méthylpipérazin-1-yl)-2-(4-isopropyl-5-(8-méthoxy-[1,2,4]triazolo[1,5-a]pyridin-6-yl)-1H-pyrazol-3-yl)thiazole (951) ;
(S)-5-(2,4-dimethylpipérazin-1-yl)-2-(4-isopropyl-5-(8-méthoxy-[1,2,4]triazolo[1,5-a]pyridin-6-yl)-1H-pyrazol-3-yl)thiazole (952) ;
(S)-2-(4-isopropyl-5-(8-méthoxy-[1,2,4]triazolo[1,5-a]pyridin-6-yl)-1H-pyrazol-3-yl)-5-(2-méthyl-4-propylpipérazin-1-yl)thiazole (953) ;
(S)-5-(4-isopropyl-2-méthylpipérazin-1-yl)-2-(4-isopropyl-5-(8-méthoxy-[1,2,4]triazolo[1,5-a]pyridin-6-yl)-1H-pyrazol-3-yl)thiazole (954) ;
(S)-5-(4-éthyl-2-méthylpipérazin-1-yl)-2-(4-isopropyl-5-(8-méthoxy-[1,2,4]triazolo[1,5-a]pyridin-6-yl)-1H-pyrazol-3-yl)thiazole (955) ;
(S)-5-(4-isobutyl-2-méthylpipérazin-1-yl)-2-(4-isopropyl-5-(8-méthoxy-[1,2,4]triazolo[1,5-a]pyridin-6-yl)-1H-pyrazol-3-yl)thiazole (956) ;
N-isobutyl-1-(2-(4-isopropyl-5-(8-méthoxy-[1,2,4]triazolo[1,5-a]pyridin-6-yl)-1H-pyrazol-3-yl)-4-méthylthiazol-5-yl)pipéridin-4-amine (957) ;
2-(4-isopropyl-5-(8-méthoxy-[1,2,4]triazolo[1,5-a]pyridin-6-yl)-1H-pyrazol-3-yl)-4-méthyl-5-(4-méthylpiperazin-1-yl)thiazole (958) ;
5-(4-(cyclopropylméthyl)pipérazin-1-yl)-2-(4-isopropyl-5-(8-méthoxy-[1,2,4]triazolo[1,5-a]pyridin-6-yl)-1H-pyrazol-3-yl)-4-méthylthiazole (959) ;
5-(4-éthylpipérazin-1-yl)-2-(4-isopropyl-5-(8-méthoxy-[1,2,4]triazolo[1,5-a]pyridin-6-yl)-1H-pyrazol-3-yl)-4-méthylthiazole (960) ;
5-(4-isobutylpipérazin-1-yl)-2-(4-isopropyl-5-(8-méthoxy-[1,2,4]triazolo[1,5-a]pyridin-6-yl)-1H-pyrazol-3-yl)-4-méthylthiazole (961) ;
N-(cyclopropylméthyl)-1-(2-(4-isopropyl-5-(8-méthoxy-[1,2,4]triazolo[1,5-a]pyridin-6-yl)-1H-pyrazol-3-yl)-4-méthylthiazol-5-yl)pipéridin-4-amine (962) ;
1-(2-(4-isopropyl-5-(8-méthoxy-[1,2,4]triazolo[1,5-a]pyridin-6-yl)-1H-pyrazol-3-yl)-4-méthylthiazol-5-yl)-N-(tétrahydro-2H-pyran-4-yl)pipéridin-4-amine (963) ;
1-(2-(4-isopropyl-5-(8-méthoxy-[1,2,4]triazolo[1,5-a]pyridin-6-yl)-1H-pyrazol-3-yl)-4-méthylthiazol-5-yl)-N-propylpipéridin-4-amine (964) ;
1-(2-(4-isopropyl-5-(8-méthoxy-[1,2,4]triazolo[1,5-a]pyridin-6-yl)-1H-pyrazol-3-yl)-4-méthylthiazol-5-yl)-N,N-bis((tétrahydro-2H-pyran-4-yl)méthyl)pipéridin-4-amine (965) ;
N,N-bis(cyclopropylméthyl)-1-(2-(4-isopropyl-5-(8-méthoxy-[1,2,4]triazolo[1,5-a]pyridin-6-yl)-1H-pyrazol-3-yl)-4-méthylthiazol-5-yl)pipéridin-4-amine (966) ;
1-(2-(4-isopropyl-5-(8-méthoxy-[1,2,4]triazolo[1,5-a]pyridin-6-yl)-1H-pyrazol-3-yl)-4-méthylthiazol-5-yl)-N,N-diméthylpipéridin-4-amine (967) ;
2-(4-isopropyl-5-(8-méthoxy-[1,2,4]triazolo[1,5-a]pyridin-6-yl)-1H-pyrazol-3-yl)-4-méthyl-5-(4-propylpipérazin-1-yl)thiazole (968) ;
2-(4-isopropyl-5-(8-méthoxy-[1,2,4]triazolo[1,5-a]pyridin-6-yl)-1H-pyrazol-3-yl)-4-méthyl-5-(4-((tétrahydro-2H-pyran-4-yl)méthyl)pipérazin-1-yl)thiazole (969) ;
2-(4-isopropyl-5-(8-méthoxy-[1,2,4]triazolo[1,5-a]pyridin-6-yl)-1H-pyrazol-3-yl)-4-méthyl-5-(4-(oxétan-3-yl)pipérazin-1-yl)thiazole (970) ;
2-(4-isopropyl-5-(8-méthoxy-[1,2,4]triazolo[1,5-a]pyridin-6-yl)-1H-pyrazol-3-yl)-5-(4-isopropylpipérazin-1-yl)-4-méthylthiazole (971) ;
5-(4-(cyclobutylméthyl)pipérazin-1-yl)-2-(4-isopropyl-5-(8-méthoxy-[1,2,4]triazolo[1,5-a]pyridin-6-yl)-1H-pyrazol-3-yl)thiazole (972) ;
(S)-5-(4-(cyclobutylméthyl)-2-méthylpipérazin-1-yl)-2-(4-isopropyl-5-(8-méthoxy-[1,2,4]triazolo[1,5-a]pyridin-6-yl)-1H-pyrazol-3-yl)thiazole (973) ;
5-((1S,4S)-5-isobutyl-2,5-diazabicyclo[2.2.1]heptan-2-yl)-2-(4-isopropyl-5-(8-méthoxy-[1,2,4]triazolo[1,5-a]pyridin-6-yl)-1H-pyrazol-3-yl)thiazole (974) ;
5-((1R,4R)-5-éthyl-2,5-diazabicyclo[2.2.1]heptan-2-yl)-2-(4-isopropyl-5-(8-méthoxy-[1,2,4]triazolo[1,5-a]pyridin-6-yl)-1H-pyrazol-3-yl)thiazole (975) ;
5-((1S,4S)-5-cyclobutyl-2,5-diazabicyclo[2.2.1]heptan-2-yl)-2-(4-isopropyl-5-(8-méthoxy-[1,2,4]triazolo[1,5-a]pyridin-6-yl)-1H-pyrazol-3-yl)thiazole (976) ;
2-(4-isopropyl-5-(8-méthoxy-[1,2,4]triazolo[1,5-a]pyridin-6-yl)-1H-pyrazol-3-yl)-5-((1S,4S)-5-isopropyl-2,5-diazabicyclo[2.2.1]heptan-2-yl)thiazole (977) ;
2-(4-isopropyl-5-(8-thoméxy-[1,2,4]triazolo[1,5-a]pyridin-6-yl)-1H-pyrazol-3-yl)-5-((1R,4R)-5-propyl-2,5-diazabicyclo[2.2.1]heptan-2-yl)thiazole (978) ;
2-(4-isopropyl-5-(8-méthoxy-[1,2,4]triazolo[1,5-a]pyridin-6-yl)-1H-pyrazol-3-yl)-5-((1S,4S)-5-(oxétan-3-yl)-2,5-diazabicyclo[2.2.1]heptan-2-yl)thiazole (979) ;
2-(4-isopropyl-5-(8-méthoxy-[1,2,4]triazolo[1,5-a]pyridin-6-yl)-1H-pyrazol-3-yl)-5-((15, 4S)-5-(tétrahydro-2H-pyran-4-yl)-2,5-diazabicyclo[2.2.1]heptan-2-yl)thiazole (980) ;
2-(4-isopropyl-5-(8-méthoxy-[1,2,4]triazolo[1,5-a]pyridin-6-yl)-1H-pyrazol-3-yl)-5-((1S,4S)-5-((tétrahydro-2H-pyran-4-yl)méthyl)-2,5-diazabicyclo[2.2.1]heptan-2-yl)thiazole (981) ;
5-((1S,4S)-5-(cyclobutylméthyl)-2,5-diazabicyclo[2.2.1]heptan-2-yl)-2-(4-isopropyl-5-(8-méthoxy-[1,2,4]triazolo[1,5-a]pyridin-6-yl)-1H-pyrazol-3-yl)thiazole (982) ;
5-((2R,5S)-2,5-diméthyl-4-((tétrahydro-2H-pyran-4-yl)méthyl)pipérazin-1-yl)-2-(4-isopropyl-5-(8-méthoxy-[1,2,4]triazolo[1,5-a]pyridin-6-yl)-1H-pyrazol-3-yl)thiazole (983) ;
5-((2R,5S)-4-(cyclobutylméthyl)-2,5-diméthylpipérazin-1-yl)-2-(4-isopropyl-5-(8-méthoxy-[1,2,4]triazolo[1,5-a]pyridin-6-yl)-1H-pyrazol-3-yl)thiazole (984) ;
5-((2R,5S)-4-isobutyl-2,5-diméthylpipérazin-1-yl)-2-(4-isopropyl-5-(8-méthoxy-[1,2,4]triazolo[1,5-a]pyridin-6-yl)-1H-pyrazol-3-yl)thiazole (985) ;
5-((2R,55)-4-(cyclopropylméthyl)-2,5-diméthylpipérazin-1-yl)-2-(4-isopropyl-5-(8-méthoxy-[1,2,4]triazolo[1,5-a]pyridin-6-yl)-1H-pyrazol-3-yl)thiazole (986) ;
5-((2R,5S)-4-cyclobutyl-2,5-diméthylpipérazin-1-yl)-2-(4-isopropyl-5-(8-méthoxy-[1,2,4]triazolo[1,5-a]pyridin-6-yl)-1H-pyrazol-3-yl)thiazole (987) ;
5-((2R,5S)-2,5-diméthyl-4-(tétrahydro-2H-pyran-4-yl)pipérazin-1-yl)-2-(4-isopropyl-5-(8-méthoxy-[1,2,4]triazolo[1,5-a]pyridin-6-yl)-1H-pyrazol-3-yl)thiazole (988) ;
2-(4-isopropyl-5-(8-méthoxy-[1,2,4]triazolo[1,5-a]pyridin-6-yl)-1H-pyrazol-3-yl)-5-((2R,5S)-2,4,5-triméthylpipérazin-1-yl)thiazole (989) ;
5-((2R,5S)-4-éthyl-2,5-diméthylpipérazin-1-yl)-2-(4-isopropyl-5-(8-méthoxy-[1,2,4]triazolo[1,5-a]pyridin-6-yl)-1H-pyrazol-3-yl)thiazole (990) ;
5-((2R,5S)-2,5-diméthyl-4-propylpipérazin-1-yl)-2-(4-isopropyl-5-(8-méthoxy-[1,2,4]triazolo[1,5-a]pyridin-6-yl)-1H-pyrazol-3-yl)thiazole (991) ;
5-((2R,5S)-4-isopropyl-2,5-diméthylpipérazin-1-yl)-2-(4-isopropyl-5-(8-méthoxy-[1,2,4]triazolo[1,5-a]pyridin-6-yl)-1H-pyrazol-3-yl)thiazole (992) ;
(R)-2-(4-isopropyl-5-(8-méthoxy-[1,2,4]triazolo[1,5-a]pyridin-6-yl)-1H-pyrazol-3-yl)-4-méthyl-5-(2-méthyl-4-((tétrahydro-2H-pyran-4-yl)méthyl)pipérazin-1-yl)thiazole (993) ;
(R)-2-(4-isopropyl-5-(8-méthoxy-[1,2,4]triazolo[1,5-a]pyridin-6-yl)-1H-pyrazol-3-yl)-4-méthyl-5-(2-méthyl-4-(tétrahydro-2H-pyran-4-yl)pipérazin-1-yl)thiazole (994) ;
(R)-2-(4-isopropyl-5-(8-méthoxy-[1,2,4]triazolo[1,5-a]pyridin-6-yl)-1H-pyrazol-3-yl)-4-méthyl-5-(2-méthyl-4-(oxétan-3-yl)pipérazin-1-yl)thiazole (995) ;
(R)-5-(4-cyclobutyl-2-méthylpipérazin-1-yl)-2-(4-isopropyl-5-(8-méthoxy-[1,2,4]triazolo[1,5-a]pyridin-6-yl)-1H-pyrazol-3-yl)-4-méthylthiazole (996) ;
(R)-5-(4-(cyclobutylméthyl)-2-méthylpipérazin-1-yl)-2-(4-isopropyl-5-(8-méthoxy-[1,2,4]triazolo[1,5-a]pyridin-6-yl)-1H-pyrazol-3-yl)-4-méthylthiazole (997) ;
(R)-5-(4-isobutyl-2-méthylpipérazin-1-yl)-2-(4-isopropyl-5-(8-méthoxy-[1,2,4]triazolo[1,5-a]pyridin-6-yl)-1H-pyrazol-3-yl)-4-méthylthiazole (998) ;
(R)-5-(4-(cyclopropylméthyl)-2-méthylpipérazin-1-yl)-2-(4-isopropyl-5-(8-méthoxy-[1,2,4]triazolo[1,5-a]pyridin-6-yl)-1H-pyrazol-3-yl)-4-méthylthiazole (999) ;
(R)-5-(2,4-diméthylpipérazin-1-yl)-2-(4-isopropyl-5-(8-méthoxy-[1,2,4]triazolo[1,5-a]pyridin-6-yl)-1H-pyrazol-3-yl)-4-méthylthiazole (1000) ;
(R)-2-(4-isopropyl-5-(8-méthoxy-[1,2,4]triazolo[1,5-a]pyridin-6-yl)-1H-pyrazol-3-yl)-4-méthyl-5-(2-méthyl-4-propylpipérazin-1-yl)thiazole (1001) ;
(S)-2-(4-isopropyl-5-(8-méthoxy-[1,2,4]triazolo[1,5-a]pyridin-6-yl)-1H-pyrazol-3-yl)-4-méthyl-5-(2-méthyl-4-(pentan-3-yl)pipérazin-1-yl)thiazole (1002) ;
(R)-5-(4-isopropyl-2-méthylpipérazin-1-yl)-2-(4-isopropyl-5-(8-méthoxy-[1,2,4]triazolo[1,5-a]pyridin-6-yl)-1H-pyrazol-3-yl)-4-méthylthiazole (1003) ;
(R)-2-(4-isopropyl-5-(8-méthoxy-[1,2,4]triazolo[1,5-a]pyridin-6-yl)-1H-pyrazol-3-yl)-5-(2-méthyl-4-((tétrahydro-2H-pyran-4-yl)méthyl)pipérazin-1-yl)thiazole (1004) ;
(R)-2-(4-isopropyl-5-(8-méthoxy-[1,2,4]triazolo[1,5-a]pyridin-6-yl)-1H-pyrazol-3-yl)-5-(2-méthyl-4-(tétrahydro-2H-pyran-4-yl)pipérazin-1-yl)thiazole (1005) ;
(R)-2-(4-isopropyl-5-(8-méthoxy-[1,2,4]triazolo[1,5-a]pyridin-6-yl)-1H-pyrazol-3-yl)-5-(2-méthyl-4-(oxétan-3-yl)pipérazin-1-yl)thiazole (1006) ;
(R)-5-(4-cyclobutyl-2-méthylpipérazin-1-yl)-2-(4-isopropyl-5-(8-méthoxy-[1,2,4]triazolo[1,5-a]pyridin-6-yl)-1H-pyrazol-3-yl)thiazole (1007) ;
(R)-5-(4-(cyclobutylméthyl)-2-méthylpipérazin-1-yl)-2-(4-isopropyl-5-(8-méthoxy-[1,2,4]triazolo[1,5-a]pyridin-6-yl)-1H-pyrazol-3-yl)thiazole (1008) ;
(R)-5-(4-(cyclopropylméthyl)-2-méthylpipérazin-1-yl)-2-(4-isopropyl-5-(8-méthoxy-[1,2,4]triazolo[1,5-a]pyridin-6-yl)-1H-pyrazol-3-yl)thiazole (1009) ;
(R)-5-(2,4-diméthylpipérazin-1-yl)-2-(4-isopropyl-5-(8-méthoxy-[1,2,4]triazolo[1,5-a]pyridin-6-yl)-1H-pyrazol-3-yl)thiazole (1010) ;
(R)-5-(4-isobutyl-2-méthylpipérazin-1-yl)-2-(4-isopropyl-5-(8-méthoxy-[1,2,4]triazolo[1,5-a]pyridin-6-yl)-1H-pyrazol-3-yl)thiazole (1011) ;
(R)-5-(4-isopropyl-2-méthylpipérazin-1-yl)-2-(4-isopropyl-5-(8-méthoxy-[1,2,4]triazolo[1,5-a]pyridin-6-yl)-1H-pyrazol-3-yl)thiazole (1012) ;
(R)-2-(4-isopropyl-5-(8-méthoxy-[1,2,4]triazolo[1,5-a]pyridin-6-yl)-1H-pyrazol-3-yl)-5-(2-méthyl-4-(pentan-3-yl)pipérazin-1-yl)thiazole (1013) ;
(R)-2-(4-isopropyl-5-(8-méthoxy-[1,2,4]triazolo[1,5-a]pyridin-6-yl)-1H-pyrazol-3-yl)-5-(2-méthyl-4-propylpipérazin-1-yl)thiazole (1014) ;
2-(diméthylamino)-1-(4-(2-(4-isopropyl-5-(8-méthoxy-[1,2,4]triazolo[1,5-a]pyridin-6-yl)-1H-pyrazol-3-yl)thiazol-5-yl)pipérazin-1-yl)éthan-1-one (1015) ;
1-(4-(2-(4-isopropyl-5-(8-méthoxy-[1,2,4]triazolo[1,5-a]pyridin-6-yl)-1H-pyrazol-3-yl)thiazol-5-yl)pipérazin-1-yl)-2-morpholinoéthan-1-one (1016) ;
(S)-2-(diméthylamino)-1-(4-(2-(4-isopropyl-5-(8-méthoxy-[1,2,4]triazolo[1,5-a]pyridin-6-yl)-1H-pyrazol-3-yl)thiazol-5-yl)-3-methylpipérazin-1-yl)éthan-1-one (1017) ;
(R)-2-(diéthylamino)-1-(4-(2-(4-isopropyl-5-(8-méthoxy-[1,2,4]triazolo[1,5-a]pyridin-6-yl)-1H-pyrazol-3-yl)thiazol-5-yl)-3-méthylpipérazin-1-yl)éthan-1-one (1018) ;
2-(diéthylamino)-1-(4-(2-(4-isopropyl-5-(8-méthoxy-[1,2,4]triazolo[1,5-a]pyridin-6-yl)-1H-pyrazol-3-yl)-4-méthylthiazol-5-yl)pipérazin-1-yl)éthan-1-one (1018B) ;
2-(diméthylamino)-1-(4-(2-(4-isopropyl-5-(8-méthoxy-[1,2,4]triazolo[1,5-a]pyridin-6-yl)-1H-pyrazol-3-yl)-4-méthylthiazol-5-yl)pipérazin-1-yl)éthan-1-one (1019) ;
2-(diéthylamino)-N -(1-(2-(4-isopropyl-5-(8-méthoxy-[1,2,4]triazolo[1,5-a]pyridin-6-yl)-1H-pyrazol-3-yl)-4-méthylthiazol-5-yl)pipéridin-4-yl)acétamide (1020) ;
2-(diméthylamino)-N-(1-(2-(4-isopropyl-5-(8-méthoxy-[1,2,4]triazolo[1,5-a]pyridin-6-yl)-1H-pyrazol-3-yl)-4-méthylthiazol-5-yl)pipéridin-4-yl)acétamide (1021) ;
N-(1-(2-(4-isopropyl-5-(8-méthoxy-[1,2,4]triazolo[1,5-a]pyridin-6-yl)-1H-pyrazol-3-yl)-4-méthylthiazol-5-yl)pipéridin-4-yl)-2-morpholinoacétamide (1022) ;
2-(diéthylamino)-1-(4-(2-(4-isopropyl-5-(8-méthoxy-[1,2,4]triazolo[1,5-a]pyridin-6-yl)-1H-pyrazol-3-yl)thiazol-5-yl)pipérazin-1-yl)éthan-1-one (1023) ;
2-(diéthylamino)-1-((2S,5R)-4-(2-(4-isopropyl-5-(8-méthoxy-[1,2,4]triazolo[1,5-a]pyridin-6-yl)-1H-pyrazol-3-yl)thiazol-5-yl)-2,5-diméthylpipérazin-1-yl)éthan-1-one (1024) ;
2-(diméthylamino)-1-((2S,5R)-4-(2-(4-isopropyl-5-(8-méthoxy-[1,2,4]triazolo[1,5-a]pyridin-6-yl)-1H-pyrazol-3-yl)thiazol-5-yl)-2,5-diméthylpipérazin-1-yl)éthan-1-one (1025) ;
(R)-2-(diméthylamino)-1-(4-(2-(4-isopropyl-5-(8-méthoxy-[1,2,4]triazolo[1,5-a]pyridin-6-yl)-1H-pyrazol-3-yl)-4-méthylthiazol-5-yl)-3-méthylpipérazin-1-yl)éthan-lone (1026) ;
(R)-2-(diméthylamino)-1-(4-(2-(4-isopropyl-5-(8-méthoxy-[1,2,4]triazolo[1,5-a]pyridin-6-yl)-1H-pyrazol-3-yl)thiazol-5-yl)-3-méthylpipérazin-1-yl)éthan-1-one (1027) ;
2-(4-isopropyl-5-(8-méthoxy-[1,2,4]triazolo[1,5-a]pyridin-6-yl)-1H-pyrazol-3-yl)-5-(4-(2-méthoxyéthyl)pipérazin-1-yl)thiazole (1028) ;
2-(4-(2-(4-isopropyl-5-(8-méthoxy-[1,2,4]triazolo[1,5-a]pyridin-6-yl)-1H-pyrazol-3-yl)-4-méthylthiazol-5-yl)pipérazin-1-yl)-N,N-diméthylacétamide (1029) ;
(S)-2-(4-isopropyl-5-(8-méthoxy-[1,2,4]triazolo[1,5-a]pyridin-6-yl)-1H-pyrazol-3-yl)-5-(4-(2-méthoxyéthyl)-2-méthylpipérazin-1-yl)thiazole (1030) ;
2-(4-isopropyl-5-(8-méthoxy-[1,2,4]triazolo[1,5-a]pyridin-6-yl)-1H-pyrazol-3-yl)-5-(4-(2-(méthylsulfonyl)ethyl)pipérazin-1-yl)thiazole (1031) ;
2-(4-(2-(4-isopropyl-5-(8-méthoxy-[1,2,4]triazolo[1,5-a]pyridin-6-yl)-1H-pyrazol-3-yl)thiazol-5-yl)pipérazin-1-yl)-N,N-diméthylacétamide (1032) ;
(S)-2-(4-isopropyl-5-(8-méthoxy-[1,2,4]triazolo[1,5-a]pyridin-6-yl)-1H-pyrazol-3-yl)-5-(2-méthyl-4-(2-(méthylsulfonyl)éthyl)pipérazin-1-yl)thiazole (1033) ;
1-(4-(2-(4-isopropyl-5-(8-méthoxy-[1,2,4]triazolo[1,5-a]pyridin-6-yl)-1H-pyrazol-3-yl)thiazol-5-yl)pipérazin-1-yl)-2-méthylpropan-2-ol (1034) ;
5-((2R,5S)-2,5-diméthyl-4-(2-(méthylsulfonyl)éthyl)pipérazin-1-yl)-2-(4-isopropyl-5-(8-méthoxy-[1,2,4]triazolo[1,5-a]pyridin-6-yl)-1H-pyrazol-3-yl)thiazole (1035) ;
2-(4-isopropyl-5-(8-méthoxy-[1,2,4]triazolo[1,5-a]pyridin-6-yl)-1H-pyrazol-3-yl)-5-((2R,5S)-4-(2-méthoxyéthyl)-2,5-diméthylpipérazin-1-yl)thiazole (1036) ;
(R)-2-(4-isopropyl-5-(8-méthoxy-[1,2,4]triazolo[1,5-a]pyridin-6-yl)-1H-pyrazol-3-yl)-4-méthyl-5-(2-méthyl-4-(2-(méthylsulfonyl)éthyl)pipérazin-1-yl)thiazole (1037) ;
(R)-5-(4-(2-fluoroéthyl)-2-méthylpipérazin-1-yl)-2-(4-isopropyl-5-(8-méthoxy-[1,2,4]triazolo[1,5-a]pyridin-6-yl)-1H-pyrazol-3-yl)-4-méthylthiazole (1038) ;
(R)-5-(4-(3-fluoropropyl)-2-méthylpipérazin-1-yl)-2-(4-isopropyl-5-(8-méthoxy-[1,2,4]triazolo[1,5-a]pyridin-6-yl)-1H-pyrazol-3-yl)-4-méthylthiazole (1039) ;
(R)-2-(4-isopropyl-5-(8-méthoxy-[1,2,4]triazolo[1,5-a]pyridin-6-yl)-1H-pyrazol-3-yl)-5-(4-(2-méthoxyéthyl)-2-méthylpipérazin-1-yl)-4-méthylthiazole (1040) ;
(R)-2-(4-(2-(4-isopropyl-5-(8-méthoxy-[1,2,4]triazolo[1,5-a]pyridin-6-yl)-1H-pyrazol-3-yl)-4-méthylthiazol-5-yl)-3-méthylpipérazin-1-yl)-N,N-diméthylacétamide (1041) ;
(R)-2-(4-isopropyl-5-(8-méthoxy-[1,2,4]triazolo[1,5-a]pyridin-6-yl)-1H-pyrazol-3-yl)-5-(2-méthyl-4-(2-(méthylsulfonyl)éthyl)pipérazin-1-yl)thiazole (1042) ;
(R)-2-(4-isopropyl-5-(8-méthoxy-[1,2,4]triazolo[1,5-a]pyridin-6-yl)-1H-pyrazol-3-yl)-5-(4-(2-méthoxyethyl)-2-méthylpipérazin-1-yl)thiazole (1043) ;
(R)-2-(4-(2-(4-isopropyl-5-(8-méthoxy-[1,2,4]triazolo[1,5-a]pyridin-6-yl)-1H-pyrazol-3-yl)thiazol-5-yl)-3-méthylpipérazin-1-yl)-N,N-diméthylacétamide (1044) ;
(R)-5-(4-(2-fluoroéthyl)-2-méthylpipérazin-1-yl)-2-(4-isopropyl-5-(8-méthoxy-[1,2,4]triazolo[1,5-a]pyridin-6-yl)-1H-pyrazol-3-yl)thiazole (1045) ;
(R)-5-(4-(3-fluoropropyl)-2-méthylpipérazin-1-yl)-2-(4-isopropyl-5-(8-méthoxy-[1,2,4]triazolo[1,5-a]pyridin-6-yl)-1H-pyrazol-3-yl)thiazole (1046) ;
(R)-2-(4-(2-(4-isopropyl-5-(8-méthoxy-[1,2,4]triazolo[1,5-a]pyridin-6-yl)-1H-pyrazol-3-yl)thiazol-5-yl)-3-méthylpipérazin-1-yl)-N-méthylacétamide (1047) ;
(R)-2-(4-(2-(4-isopropyl-5-(8-méthoxy-[1,2,4]triazolo[1,5-a]pyridin-6-yl)-1H-pyrazol-3-yl)thiazol-5-yl)-3-méthylpipérazin-1-yl)acétonitrile (1048) ;
2-(4-isopropyl-5-(8-méthoxy-[1,2,4]triazolo[1,5-a]pyridin-6-yl)-1H-pyrazol-3-yl)-4-méthyl-5-(6-propyl-2,6-diazaspiro[3.3]heptan-2-yl)thiazole (1049) ;
2-(4-isopropyl-5-(8-méthoxy-[1,2,4]triazolo[1,5-a]pyridin-6-yl)-1H-pyrazol-3-yl)-4-méthyl-5-(6-méthyl-2,6-diazaspiro[3.3]heptan-2-yl)thiazole (1050) ;
5-(6-éthyl-2,6-diazaspiro[3.3]heptan-2-yl)-2-(4-isopropyl-5-(8-méthoxy-[1,2,4]triazolo[1,5-a]pyridin-6-yl)-1H-pyrazol-3-yl)-4-méthylthiazole (1051) ;
2-(4-isopropyl-5-(8-méthoxy-[1,2,4]triazolo[1,5-a]pyridin-6-yl)-1H-pyrazol-3-yl)-5-(6-isopropyl-2,6-diazaspiro[3.3]heptan-2-yl)-4-méthylthiazole (1052) ;
5-(6-(cyclopropylméthyl)-2,6-diazaspiro[3.3]heptan-2-yl)-2-(4-isopropyl-5-(8-méthoxy-[1,2,4]triazolo[1,5-a]pyridin-6-yl)-1H-pyrazol-3-yl)-4-méthylthiazole (1053) ;
5-(6-isobutyl-2,6-diazaspiro[3.3]heptan-2-yl)-2-(4-isopropyl-5-(8-méthoxy-[1,2,4]triazolo[1,5-a]pyridin-6-yl)-1H-pyrazol-3-yl)-4-méthylthiazole (1054) ;
5-(6-(2-éthylbutyl)-2,6-diazaspiro[3.3]heptan-2-yl)-2-(4-isopropyl-5-(8-méthoxy-[1,2,4]triazolo[1,5-a]pyridin-6-yl)-1H-pyrazol-3-yl)-4-méthylthiazole (1055) ;
5-(6-cyclobutyl-2,6-diazaspiro[3.3]heptan-2-yl)-2-(4-isopropyl-5-(8-méthoxy-[1,2,4]triazolo[1,5-a]pyridin-6-yl)-1H-pyrazol-3-yl)-4-méthylthiazole (1056) ;
5-(6-(cyclobutylméthyl)-2,6-diazaspiro[3.3]heptan-2-yl)-2-(4-isopropyl-5-(8-méthoxy-[1,2,4]triazolo[1,5-a]pyridin-6-yl)-1H-pyrazol-3-yl)-4-méthylthiazole (1057) ;
2-(4-isopropyl-5-(8-méthoxy-[1,2,4]triazolo[1,5-a]pyridin-6-yl)-1H-pyrazol-3-yl)-4-méthyl-5-(6-(tétrahydro-2H-pyran-4-yl)-2,6-diazaspiro[3.3]heptan-2-yl)thiazole (1058) ;
2-(4-isopropyl-5-(8-méthoxy-[1,2,4]triazolo[1,5-a]pyridin-6-yl)-1H-pyrazol-3-yl)-4-méthyl-5-(6-((tétrahydro-2H-pyran-4-yl)méthyl)-2,6-diazaspiro[3.3]heptan-2-yl)thiazole (1059) ;
2-(diméthylamino)-1-(6-(2-(4-isopropyl-5-(8-méthoxy-[1,2,4]triazolo[1,5-a]pyridin-6-yl)-1H-pyrazol-3-yl)-4-méthylthiazol-5-yl)-2,6-diazaspiro[3.3]heptan-2-yl)éthan-1-one (1060) ;
2-(4-isopropyl-5-(8-méthoxy-[1,2,4]triazolo[1,5-a]pyridin-6-yl)-1H-pyrazol-3-yl)-4-méthyl-5-(6-(2-(méthylsulfonyl)éthyl)-2,6-diazaspiro[3.3]heptan-2-yl)thiazole (1061) ;
2-(4-isopropyl-5-(8-méthoxy-[1,2,4]triazolo[1,5-a]pyridin-6-yl)-1H-pyrazol-3-yl)-5-(6-(2-méthoxyéthyl)-2,6-diazaspiro[3.3]heptan-2-yl)-4-méthylthiazole (1062) ;
5-(4-isopropyl-5-(8-méthoxy-[1,2,4]triazolo[1,5-a]pyridin-6-yl)-1H-pyrazol-3-yl)-2-(1-isopropylpipéridin-4-yl)thiazole (1063) ;
5-(4-isopropyl-5-(8-méthyl-[1,2,4]triazolo[1,5-a]pyridin-6-yl)-1H-pyrazol-3-yl)-2-(pipéridin-4-yl)thiazole (1064) ;
5-(4-isopropyl-5-(8-méthoxy-[1,2,4]triazolo[1,5-a]pyridin-6-yl)-1H-pyrazol-3-yl)-2-(1-méthylpipéridin-4-yl)thiazole (1065) ;
2-(1-éthylpipéridin-4-yl)-5-(4-isopropyl-5-(8-méthoxy-[1,2,4]triazolo[1,5-a]pyridin-6-yl)-1H-pyrazol-3-yl)thiazole (1066) ;
5-(4-isopropyl-5-(8-méthoxy-[1,2,4]triazolo[1,5-a]pyridin-6-yl)-1H-pyrazol-3-yl)-2-(1-propylpipéridin-4-yl)thiazole (1067) ;
5-(4-isopropyl-5-(8-méthyl-[1,2,4]triazolo[1,5-a]pyridin-6-yl)-1H-pyrazol-3-yl)-2-(1-propylpipéridin-4-yl)thiazole (1068) ;
5-(4-isopropyl-5-(8-méthyl-[1,2,4]triazolo[1,5-a]pyridin-6-yl)-1H-pyrazol-3-yl)-2-(1-(tétrahydro-2H-pyran-4-yl)pipéridin-4-yl)thiazole (1069);
5-(4-isopropyl-5-(8-méthyl-[1,2,4]triazolo[1,5-a]pyridin-6-yl)-1H-pyrazol-3-yl)-2-(1-méthylpipéridin-4-yl)thiazole (1070) ;
2-(1-éthylpipéridin-4-yl)-5-(4-isopropyl-5-(8-méthyl-[1,2,4]triazolo[1,5-a]pyridin-6-yl)-1H-pyrazol-3-yl)thiazole (1071) ;
5-(4-isopropyl-5-(8-méthyl-[1,2,4]triazolo[1,5-a]pyridin-6-yl)-1H-pyrazol-3-yl)-2-(1-isopropylpipéridin-4-yl)thiazole (1072) ;
5-(4-isopropyl-5-(8-méthyl-[1,2,4]triazolo[1,5-a]pyridin-6-yl)-1H-pyrazol-3-yl)-2-(1-(oxétan-3-yl)pipéridin-4-yl)thiazole (1073) ;
5-(4-isopropyl-5-(8-méthyl-[1,2,4]triazolo[1,5-a]pyridin-6-yl)-1H-pyrazol-3-yl)-2-(1-((3-méthyloxétan-3-yl)méthyl)pipéridin-4-yl)thiazole (1074) ;
5-(4-isopropyl-5-(8-méthyl-[1,2,4]triazolo[1,5-a]pyridin-6-yl)-1H-pyrazol-3-yl)-2-(1-néopentylpipéridin-4-yl)thiazole (1075) ;
2-(4-(5-(4-isopropyl-5-(8-méthyl-[1,2,4]triazolo[1,5-a]pyridin-6-yl)-1H-pyrazol-3-yl) thiazol-2-yl)pipéridin-1-yl)-N,N-diméthylacétamide (1076) ;
2-(4-(5-(4-isopropyl-5-(8-méthoxy-[1,2,4]triazolo[1,5-a]pyridin-6-yl)-1H-pyrazol-3-yl)thiazol-2-yl)pipéridin-1-yl)acétamide (1077) ;
2-(4-(5-(4-isopropyl-5-(8-méthyl-[1,2,4]triazolo[1,5-a]pyridin-6-yl)-1H-pyrazol-3-yl) thiazol-2-yl)pipéridin-1-yl)acétonitrile (1078) ;
1-(4-(5-(4-isopropyl-5-(8-méthyl-[1,2,4]triazolo[1,5-a]pyridin-6-yl)-1H-pyrazol-3-yl) thiazol-2-yl)pipéridin-1-yl)-2-méthylpropan-2-ol (1079) ;
5-(4-isopropyl-5-(8-méthyl-[1,2,4]triazolo[1,5-a]pyridin-6-yl)-1H-pyrazol-3-yl)-2-(1-(2-(méthylsulfonyl)éthyl)pipéridin-4-yl)thiazole (1080) ;
5-(4-isopropyl-5-(8-méthyl-[1,2,4]triazolo[1,5-a]pyridin-6-yl)-1H-pyrazol-3-yl)-2-(1-(2-méthoxyéthyl)pipéridin-4-yl)thiazole (1081) ;
2-(diméthylamino)-1-(4-(5-(4-isopropyl-5-(8-méthyl-[1,2,4]triazolo[1,5-a]pyridin-6-yl)-1H-pyrazol-3-yl)thiazol-2-yl)pipéridin-1-yl)éthan-1-one (1082) ;
4-(5-(4-isopropyl-5-(8-méthyl-[1,2,4]triazolo[1,5-a]pyridin-6-yl)-1H-pyrazol-3-yl) thiazol-2-yl)-N-méthylcyclohexan-1-amine (1083) ;
4-(5-(4-isopropyl-5-(8-méthyl-[1,2,4]triazolo[1,5-a]pyridin-6-yl)-1H-pyrazol-3-yl) thiazol-2-yl)-N,N-diméthylcyclohexan-1-amine (1084-1085) ;
4-(5-(4-isopropyl-5-(8-méthyl-[1,2,4]triazolo[1,5-a]pyridin-6-yl)-1H-pyrazol-3-yl) thiazol-2-yl)-N,N-diméthylcyclohexan-1-amine (1085) ;
N-(4-(5-(4-isopropyl-5-(8-méthyl-[1,2,4]triazolo[1,5-a]pyridin-6-yl)-1H-pyrazol-3-yl)thiazol-2-yl)cyclohexyl)-N-méthyloxétan-3-amine (1086-1087) ;
N-isopropyl-4-(5-(4-isopropyl-5-(8-méthyl-[1,2,4]triazolo[1,5-a]pyridin-6-yl)-1H-pyrazol-3-yl)thiazol-2-yl)-N-méthylcyclohexan-1-amine (1088-1089) ;
6-(4-isopropyl-1'-(1-méthylpipéridin-4-yl)-1H, 1'H-[3,4'-bipyrazol]-5-yl)-8-méthyl-[1,2,4]triazolo[1,5-a]pyridine (1090) ;
6-(4-isopropyl-1'-(1-isopropylpipéridin-4-yl)-1H, 1'H-[3,4'-bipyrazol]-5-yl)-8-méthyl-[1,2,4]triazolo[1,5-a]pyridine (1091) ;
6-(4-isopropyl-1'-(1-(oxétan-3-yl)pipéridin-4-yl)-1H, 1'H-[3,4'-bipyrazol]-5-yl)-8-méthyl-[1,2,4]triazolo[1,5-a]pyridine (1092) ;
6-(4-isopropyl-3-(4-méthyl-5-(1-propylpipéridin-4-yl)pyridin-2-yl)-1H-pyrazol-5-yl)-8-méthoxy-[1,2,4]triazolo[1,5-a]pyridine (1093) ;
6-(4-isopropyl-3-(3-méthyl-5-(pipéridin-4-yl)pyridin-2-yl)-1H-pyrazol-5-yl)-8-méthoxy-[1,2,4]triazolo[1,5-a]pyridine (1094) ;
6-(3-(3-fluoro-5-(pipéridin-4-yl)pyridin-2-yl)-4-isopropyl-1H-pyrazol-5-yl)-8-méthoxy-[1,2,4]triazolo[1,5-a]pyridine (1095) ;
6-(3-(6-fluoro-5-(pipéridin-4-yl)pyridin-2-yl)-4-isopropyl-1H-pyrazol-5-yl)-8-méthoxy-[1,2,4]triazolo[1,5-a]pyridine (1096) ;
6-(4-isopropyl-3-(4-méthyl-5-(1-méthylpipéridin-4-yl)pyridin-2-yl)-1H-pyrazol-5-yl)-8-méthoxy-[1,2,4]triazolo[1,5-a]pyridine (1097) ;
6-(4-isopropyl-3-(5-(1-isopropylpipéridin-4-yl)-4-méthylpyridin-2-yl)-1H-pyrazol-5-yl)-8-méthoxy-[1,2,4]triazolo[1,5-a]pyridine (1098) ;
6-(3-(6-fluoro-5-(1-méthylpiperidin-4-yl)pyridin-2-yl)-4-isopropyl-1H-pyrazol-5-yl)-8-méthoxy-[1,2,4]triazolo[1,5-a]pyridine (1099) ;
6-(3-(6-fluoro-5-(1-propylpipéridin-4-yl)pyridin-2-yl)-4-isopropyl-1H-pyrazol-5-yl)-8-méthoxy-[1,2,4]triazolo[1,5-a]pyridine (1100) ;
6-(3-(6-fluoro-5-(1-(oxétan-3-yl)pipéridin-4-yl)pyridin-2-yl)-4-isopropyl-1H-pyrazol-5-yl)-8-méthoxy-[1,2,4]triazolo[1,5-a]pyridine (1101) ;
6-(3-(5-(1-éthylpipéridin-4-yl)-4-méthylpyridin-2-yl)-4-isopropyl-1H-pyrazol-5-yl)-8-méthoxy-[1,2,4]triazolo[1,5-a]pyridine (1102) ;
6-(3-(5-(1-isobutylpipéridin-4-yl)-4-méthylpyridin-2-yl)-4-isopropyl-1H-pyrazol-5-yl)-8-méthoxy-[1,2,4]triazolo[1,5-a]pyridine (1103) ;
6-(3-(5-(1-cyclobutylpipéridin-4-yl)-4-méthylpyridin-2-yl)-4-isopropyl-1H-pyrazol-5-yl)-8-méthoxy-[1,2,4]triazolo[1,5-a]pyridine (1104) ;
6-(3-(5-(1-(cyclobutylméthyl)pipéridin-4-yl)-4-méthylpyridin-2-yl)-4-isopropyl-1H-pyrazol-5-yl)-8-méthoxy-[1,2,4]triazolo[1,5-a]pyridine (1105) ;
6-(4-isopropyl-3-(4-méthyl-5-(1-(oxétan-3-yl)pipéridin-4-yl)pyridin-2-yl)-1H-pyrazol-5-yl)-8-méthoxy-[1,2,4]triazolo[1,5-a]pyridine (1106) ;
6-(4-isopropyl-3-(4-méthyl-5-(1-((tétrahydro-2H-pyran-4-yl)méthyl)pipéridin-4-yl)pyridin-2-yl)-1H-pyrazol-5-yl)-8-méthoxy-[1,2,4]triazolo[1,5-a]pyridine (1107) ;
6-(4-isopropyl-3-(4-méthyl-5-(1-(tétrahydrofuran-3-yl)pipéridin-4-yl)pyridin-2-yl)-1H-pyrazol-5-yl)-8-méthoxy-[1,2,4]triazolo[1,5-a]pyridine (1108) ;
6-(4-isopropyl-3-(4-méthyl-5-(1-((tétrahydrofuran-3-yl)méthyl)pipéridin-4-yl)pyridin-2-yl)-1H-pyrazol-5-yl)-8-méthoxy-[1,2,4]triazolo[1,5-a]pyridine (1109) ;
6-(3-(5-(1-(cyclobutylméthyl)pipéridin-4-yl)-3-fluoropyridin-2-yl)-4-isopropyl-1H-pyrazol-5-yl)-8-méthoxy-[1,2,4]triazolo[1,5-a]pyridine (1110) ;
6-(3-(3-fluoro-5-(1-méthylpipéridin-4-yl)pyridin-2-yl)-4-isopropyl-1H-pyrazol-5-yl)-8-méthoxy-[1,2,4]triazolo[1,5-a]pyridine (1111) ;
6-(3-(5-(1-éthylpipéridin-4-yl)-3-fluoropyridin-2-yl)-4-isopropyl-1H-pyrazol-5-yl)-8-méthoxy-[1,2,4]triazolo[1,5-a]pyridine (1112) ;
6-(3-(3-fluoro-5-(1-propylpipéridin-4-yl)pyridin-2-yl)-4-isopropyl-1H-pyrazol-5-yl)-8-méthoxy-[1,2,4]triazolo[1,5-a]pyridine (1113) ;
6-(3-(3-fluoro-5-(1-isopropylpipéridin-4-yl)pyridin-2-yl)-4-isopropyl-1H-pyrazol-5-yl)-8-méthoxy-[1,2,4]triazolo[1,5-a]pyridine (1114) ;
6-(3-(5-(1-(cyclopropylméthyl)pipéridin-4-yl)-3-fluoropyridin-2-yl)-4-isopropyl-1H-pyrazol-5-yl)-8-méthoxy-[1,2,4]triazolo[1,5-a]pyridine (1115) ;
6-(3-(3-fluoro-5-(1-isobutylpipéridin-4-yl)pyridin-2-yl)-4-isopropyl-1H-pyrazol-5-yl)-8-méthoxy-[1,2,4]triazolo[1,5-a]pyridine (1116) ;
6-(3-(3-fluoro-5-(1-(oxétan-3-yl)pipéridin-4-yl)pyridin-2-yl)-4-isopropyl-1H-pyrazol-5-yl)-8-méthoxy-[1,2,4]triazolo[1,5-a]pyridine (1117) ;
6-(3-(3-fluoro-5-(1-(tétrahydro-2H-pyran-4-yl)pipéridin-4-yl)pyridin-2-yl)-4-isopropyl-1H-pyrazol-5-yl)-8-méthoxy-[1,2,4]triazolo[1,5-a]pyridine (1118) ;
6-(3-(3-fluoro-5-(1-((tétrahydro-2H-pyran-4-yl)méthyl)pipéridin-4-yl)pyridin-2-yl)-4-isopropyl-1H-pyrazol-5-yl)-8-méthoxy-[1,2,4]triazolo[1,5-a]pyridine (1119) ;
6-(3-(3-fluoro-5-(1-(pentan-3-yl)pipéridin-4-yl)pyridin-2-yl)-4-isopropyl-1H-pyrazol-5-yl)-8-méthoxy-[1,2,4]triazolo[1,5-a]pyridine (1120) ;
6-(3-(5-(1-(2-éthylbutyl)pipéridin-4-yl)-3-fluoropyridin-2-yl)-4-isopropyl-1H-pyrazol-5-yl)-8-méthoxy-[1,2,4]triazolo[1,5-a]pyridine (1121) ;
6-(3-(3-fluoro-5-(1-néopentylpipéridin-4-yl)pyridin-2-yl)-4-isopropyl-1H-pyrazol-5-yl)-8-méthoxy-[1,2,4]triazolo[1,5-a]pyridine (1123) ;
6-(4-isopropyl-3-(3-méthyl-5-(1-méthylpipéridin-4-yl)pyridin-2-yl)-1H-pyrazol-5-yl)-8-méthoxy-[1,2,4]triazolo[1,5-a]pyridine (1124) ;
6-(3-(5-(1-éthylpipéridin-4-yl)-3-méthylpyridin-2-yl)-4-isopropyl-1H-pyrazol-5-yl)-8-méthoxy-[1,2,4]triazolo[1,5-a]pyridine (1125) ;
6-(4-isopropyl-3-(3-méthyl-5-(1-propylpipéridin-4-yl)pyridin-2-yl)-1H-pyrazol-5-yl)-8-méthoxy-[1,2,4]triazolo[1,5-a]pyridine (1126) ;
6-(4-isopropyl-3-(5-(1-isopropylpipéridin-4-yl)-3-méthylpyridin-2-yl)-1H-pyrazol-5-yl)-8-méthoxy-[1,2,4]triazolo[1,5-a]pyridine (1127) ;
6-(3-(5-(1-(cyclopropylméthyl)pipéridin-4-yl)-3-méthylpyridin-2-yl)-4-isopropyl-1H-pyrazol-5-yl)-8-méthoxy-[1,2,4]triazolo[1,5-a]pyridine (1128) ;
6-(4-isopropyl-3-(3-méthyl-5-(1-((tétrahydro-2H-pyran-4-yl)méthyl)pipéridin-4-yl) pyridin-2-yl)-1H-pyrazol-5-yl)-8-méthoxy-[1,2,4]triazolo[1,5-a]pyridine (1129) ;
6-(3-(5-(1-éthylpipéridin-4-yl)-6-fluoropyridin-2-yl)-4-isopropyl-1H-pyrazol-5-yl)-8-méthoxy-[1,2,4]triazolo[1,5-a]pyridine (1130) ;
6-(3-(6-fluoro-5-(1-isopropylpipéridin-4-yl)pyridin-2-yl)-4-isopropyl-1H-pyrazol-5-yl)-8-méthoxy-[1,2,4]triazolo[1,5-a]pyridine (1131) ;
6-(3-(5-(1-cyclobutylpipéridin-4-yl)-6-fluoropyridin-2-yl)-4-isopropyl-1H-pyrazol-5-yl)-8-méthoxy-[1,2,4]triazolo[1,5-a]pyridine (1132) ;
6-(3-(6-fluoro-5-(1-((tétrahydro-2H-pyran-4-yl)méthyl)pipéridin-4-yl)pyridin-2-yl)-4-isopropyl-1H-pyrazol-5-yl)-8-méthoxy-[1,2,4]triazolo[1,5-a]pyridine (1133) ;
2-(diméthylamino)-1-(4-(6-(4-isopropyl-5-(8-méthoxy-[1,2,4]triazolo[1,5-a]pyridin-6-yl)-1H-pyrazol-3-yl)-4-méthylpyridin-3-yl)pipéridin-1-yl)éthan-1-one (1134) ;
2-(diméthylamino)-1-(4-(5-fluoro-6-(4-isopropyl-5-(8-méthoxy-[1,2,4]triazolo[1,5-a] pyridin-6-yl)-1H-pyrazol-3-yl)pyridin-3-yl)pipéridin-1-yl)éthan-1-one (1135) ;
2-(4-(6-(4-isopropyl-5-(8-méthoxy-[1,2,4]triazolo[1,5-a]pyridin-6-yl)-1H-pyrazol-3-yl)-4-méthylpyridin-3-yl)pipéridin-1-yl)-N,N-diméthylacétamide (1136) ;
2-(4-(5-fluoro-6-(4-isopropyl-5-(8-méthoxy-[1,2,4]triazolo[1,5-a]pyridin-6-yl)-1H-pyrazol-3-yl)pyridin-3-yl)pipéridin-1-yl)-N,N-diméthylacétamide (1137) ;
6-(3-(3-fluoro-5-(1-(2-méthoxyéthyl)pipéridin-4-yl)pyridin-2-yl)-4-isopropyl-1H-pyrazol-5-yl)-8-méthoxy-[1,2,4]triazolo[1,5-a]pyridine (1138) ;
6-(3-(3-fluoro-5-(1-(2-(méthylsulfonyl)éthyl)pipéridin-4-yl)pyridin-2-yl)-4-isopropyl-1H-pyrazol-5-yl)-8-méthoxy-[1,2,4]triazolo[1,5-a]pyridine (1139) ;
6-(4-isopropyl-3-(4-méthyl-5-(1-(2-(méthylsulfonyl)éthyl)pipéridin-4-yl)pyridin-2-yl)-1H-pyrazol-5-yl)-8-méthoxy-[1,2,4]triazolo[1,5-a]pyridine (1140) ;
2-(5-(8-méthoxy-[1,2,4]triazolo[1,5-a]pyridin-6-yl)-4-(2,2,2-trifluoroéthyl)-1H-pyrazol-3-yl)-4-méthyl-5-(1-méthylpipéridin-4-yl)thiazole (1141) ;
2-(5-(8-méthoxy-[1,2,4]triazolo[1,5-a]pyridin-6-yl)-4-(2,2,2-trifluoroéthyl)-1H-pyrazol-3-yl)-5-(pipéridin-4-yl)thiazole (1142) ;
2-(5-(8-méthyl-[1,2,4]triazolo[1,5-a]pyridin-6-yl)-4-(2,2,2-trifluoroéthyl)-1H-pyrazol-3-yl)-5-(pipéridin-4-yl)thiazole (1143) ;
4-méthyl-2-(5-(8-méthyl-[1,2,4]triazolo[1,5-a]pyridin-6-yl)-4-(2,2,2-trifluoroéthyl)-1H-pyrazol-3-yl)-5-(pipéridin-4-yl)thiazole (1144) ;
2-(5-(7,8-diméthyl-[1,2,4]triazolo[1,5-a]pyridin-6-yl)-4-(2,2,2-trifluoroéthyl)-1H-pyrazol-3-yl)-4-méthyl-5-(pipéridin-4-yl)thiazole (1145) ;
2-(5-(8-méthoxy-[1,2,4]triazolo[1,5-a]pyridin-6-yl)-4-(2,2,2-trifluoroéthyl)-1H-pyrazol-3-yl)-4-méthyl-5-(1-propylpipéridin-4-yl)thiazole (1146) ;
5-(1-éthylpipéridin-4-yl)-2-(5-(8-méthoxy-[1,2,4]triazolo[1,5-a]pyridin-6-yl)-4-(2,2,2-trifluoroéthyl)-1H-pyrazol-3-yl)-4-méthylthiazole (1147) ;
5-(1-isopropylpipéridin-4-yl)-2-(5-(8-méthoxy-[1,2,4]triazolo[1,5-a]pyridin-6-yl)-4-(2,2,2-trifluoroéthyl)-1H-pyrazol-3-yl)-4-méthylthiazole (1148) ;
5-(1-(cyclopropylméthyl)pipéridin-4-yl)-2-(5-(8-méthoxy-[1,2,4]triazolo[1,5-a]pyridin-6-yl)-4-(2,2,2-trifluoroéthyl)-1H-pyrazol-3-yl)-4-méthylthiazole (1149) ;
5-(1-isobutylpipéridin-4-yl)-2-(5-(8-méthoxy-[1,2,4]triazolo[1,5-a]pyridin-6-yl)-4-(2,2,2-trifluoroéthyl)-1H-pyrazol-3-yl)-4-méthylthiazole (1150) ;
2-(5-(8-méthoxy-[1,2,4]triazolo[1,5-a]pyridin-6-yl)-4-(2,2,2-trifluoroéthyl)-1H-pyrazol-3-yl)-4-méthyl-5-(1-((tétrahydro-2H-pyran-4-yl)méthyl)pipéridin-4-yl)thiazole (1151) ;
2-(5-(8-méthoxy-[1,2,4]triazolo[1,5-a]pyridin-6-yl)-4-(2,2,2-trifluoroéthyl)-1H-pyrazol-3-yl)-4-méthyl-5-(1-(tétrahydro-2H-pyran-4-yl)pipéridin-4-yl)thiazole (1152) ;
2-(5-(8-méthoxy-[1,2,4]triazolo[1,5-a]pyridin-6-yl)-4-(2,2,2-trifluoroéthyl)-1H-pyrazol-3-yl)-4-méthyl-5-(1-(oxétan-3-yl)pipéridin-4-yl)thiazole (1153) ;
2-(5-(8-méthoxy-[1,2,4]triazolo[1,5-a]pyridin-6-yl)-4-(2,2,2-trifluoroéthyl)-1H-pyrazol-3-yl)-5-(1-méthylpipéridin-4-yl)thiazole (1154) ;
5-(1-éthylpipéridin-4-yl)-2-(5-(8-méthoxy-[1,2,4]triazolo[1,5-a]pyridin-6-yl)-4-(2,2,2-trifluoroéthyl)-1H-pyrazol-3-yl)thiazole (1155) ;
2-(5-(8-méthoxy-[1,2,4]triazolo[1,5-a]pyridin-6-yl)-4-(2,2,2-trifluoroéthyl)-1H-pyrazol-3-yl)-5-(1-propylpipéridin-4-yl)thiazole (1156) ;
5-(1-isopropylpipéridin-4-yl)-2-(5-(8-méthoxy-[1,2,4]triazolo[1,5-a]pyridin-6-yl)-4-(2,2,2-trifluoroéthyl)-1H-pyrazol-3-yl)thiazole (1157) ;
2-(5-(8-méthoxy-[1,2,4]triazolo[1,5-a]pyridin-6-yl)-4-(2,2,2-trifluoroéthyl)-1H-pyrazol-3-yl)-5-(1-(tétrahydro-2H-pyran-4-yl)pipéridin-4-yl)thiazole (1158) ;
5-(1-(cyclopropylméthyl)pipéridin-4-yl)-2-(5-(8-méthoxy-[1,2,4]triazolo[1,5-a] pyridin-6-yl)-4-(2,2,2-trifluoroéthyl)-1H-pyrazol-3-yl)thiazole (1159) ;
2-(5-(8-méthoxy-[1,2,4]triazolo[1,5-a]pyridin-6-yl)-4-(2,2,2-trifluoroéthyl)-1H-pyrazol-3-yl)-5-(1-(oxétan-3-yl)pipéridin-4-yl)thiazole (1160) ;
2-(5-(8-méthoxy-[1,2,4]triazolo[1,5-a]pyridin-6-yl)-4-(2,2,2-trifluoroéthyl)-1H-pyrazol-3-yl)-5-(1-((tétrahydro-2H-pyran-4-yl)méthyl)pipéridin-4-yl)thiazole (1161) ;
5-(1-isobutylpipéridin-4-yl)-2-(5-(8-méthyl-[1,2,4]triazolo[1,5-a]pyridin-6-yl)-4-(2,2,2-trifluoroéthyl)-1H-pyrazol-3-yl)thiazole (1162) ;
2-(5-(8-méthyl-[1,2,4]triazolo[1,5-a]pyridin-6-yl)-4-(2,2,2-trifluoroéthyl)-1H-pyrazol-3-yl)-5-(1-(tétrahydro-2H-pyran-4-yl)pipéridin-4-yl)thiazole (1163) ;
5-(1-éthylpipéridin-4-yl)-2-(5-(8-méthyl-[1,2,4]triazolo[1,5-a]pyridin-6-yl)-4-(2,2,2-trifluoroéthyl)-1H-pyrazol-3-yl)thiazole (1164) ;
5-(1-isopropylpipéridin-4-yl)-2-(5-(8-méthyl-[1,2,4]triazolo[1,5-a]pyridin-6-yl)-4-(2,2,2-trifluoroéthyl)-1H-pyrazol-3-yl)thiazole (1165) ;
2-(5-(8-méthyl-[1,2,4]triazolo[1,5-a]pyridin-6-yl)-4-(2,2,2-trifluoroéthyl)-1H-pyrazol-3-yl)-5-(1-((tétrahydro-2H-pyran-4-yl)méthyl)pipéridin-4-yl)thiazole (1166) ;
2-(5-(8-méthyl-[1,2,4]triazolo[1,5-a]pyridin-6-yl)-4-(2,2,2-trifluoroéthyl)-1H-pyrazol-3-yl)-5-(1-(oxétan-3-yl)pipéridin-4-yl)thiazole (1167) ;
2-(5-(8-méthyl-[1,2,4]triazolo[1,5-a]pyridin-6-yl)-4-(2,2,2-trifluoroéthyl)-1H-pyrazol-3-yl)-5-(1-propylpipéridin-4-yl)thiazole (1168) ;
2-(5-(8-méthyl-[1,2,4]triazolo[1,5-a]pyridin-6-yl)-4-(2,2,2-trifluoroéthyl)-1H-pyrazol-3-yl)-5-(1-(3,3,3-trifluoropropyl)pipéridin-4-yl)thiazole (1169) ;
5-(1-(cyclopropylméthyl)pipéridin-4-yl)-2-(5-(8-méthyl-[1,2,4]triazolo[1,5-a]pyridin-6-yl)-4-(2,2,2-trifluoroéthyl)-1H-pyrazol-3-yl)thiazole (1170) ;
5-(1-(cyclobutylméthyl)pipéridin-4-yl)-2-(5-(8-méthyl-[1,2,4]triazolo[1,5-a]pyridin-6-yl)-4-(2,2,2-trifluoroéthyl)-1H-pyrazol-3-yl)thiazole (1171) ;
2-(5-(8-méthyl-[1,2,4]triazolo[1,5-a]pyridin-6-yl)-4-(2,2,2-trifluoroéthyl)-1H-pyrazol- 3-yl)-5-(1-méthylpipéridin-4-yl)thiazole (1172) ;
2-(5-(8-méthyl-[1,2,4]triazolo[1,5-a]pyridin-6-yl)-4-(2,2,2-trifluoroéthyl)-1H-pyrazol-3-yl)-5-(1-néopentylpipéridin-4-yl)thiazole (1173) ;
5-(1-éthylpipéridin-4-yl)-4-méthyl-2-(5-(8-méthyl-[1,2,4]triazolo[1,5-a]pyridin-6-yl)-4-(2,2,2-trifluoroéthyl)-1H-pyrazol-3-yl)thiazole (1174) ;
4-méthyl-2-(5-(8-méthyl-[1,2,4]triazolo[1,5-a]pyridin-6-yl)-4-(2,2,2-trifluoroéthyl)-1H-pyrazol-3-yl)-5-(1-(3,3,3-trifluoropropyl)pipéridin-4-yl)thiazole (1175) ;
5-(1-isopropylpipéridin-4-yl)-4-méthyl-2-(5-(8-méthyl-[1,2,4]triazolo[1,5-a]pyridin-6-yl)-4-(2,2,2-trifluoroéthyl)-1H-pyrazol-3-yl)thiazole (1176) ;
5-(1-(cyclohexylméthyl)pipéridin-4-yl)-4-méthyl-2-(5-(8-méthyl-[1,2,4]triazolo[1,5-a]pyridin-6-yl)-4-(2,2,2-trifluoroéthyl)-1H-pyrazol-3-yl)thiazole (1177) ;
5-(1-(cyclopropylméthyl)pipéridin-4-yl)-4-méthyl-2-(5-(8-méthyl-[1,2,4]triazolo[1,5-a]pyridin-6-yl)-4-(2,2,2-trifluoroéthyl)-1H-pyrazol-3-yl)thiazole (1179) ;
4-méthyl-2-(5-(8-méthyl-[1,2,4]triazolo[1,5-a]pyridin-6-yl)-4-(2,2,2-trifluoroéthyl)-1H-pyrazol-3-yl)-5-(1-(oxétan-3-yl)pipéridin-4-yl)thiazole (1180) ;
5-(1-isobutylpipéridin-4-yl)-4-méthyl-2-(5-(8-méthyl-[1,2,4]triazolo[1,5-a]pyridin-6-yl)-4-(2,2,2-trifluoroéthyl)-1H-pyrazol-3-yl)thiazole (1181) ;
4-méthyl-2-(5-(8-méthyl-[1,2,4]triazolo[1,5-a]pyridin-6-yl)-4-(2,2,2-trifluoroéthyl)-1H-pyrazol-3-yl)-5-(1-méthylpipéridin-4-yl)thiazole (1182) ;
4-méthyl-2-(5-(8-méthyl-[1,2,4]triazolo[1,5-a]pyridin-6-yl)-4-(2,2,2-trifluoroéthyl)-1H-pyrazol-3-yl)-5-(1-propylpipéridin-4-yl)thiazole (1183) ;
4-méthyl-2-(5-(8-méthyl-[1,2,4]triazolo[1,5-a]pyridin-6-yl)-4-(2,2,2-trifluoroéthyl)-1H-pyrazol-3-yl)-5-(1-(tétrahydro-2H-pyran-4-yl)pipéridin-4-yl)thiazole (1184) ;
4-méthyl-2-(5-(8-méthyl-[1,2,4]triazolo[1,5-a]pyridin-6-yl)-4-(2,2,2-trifluoroéthyl)-1H-pyrazol-3-yl)-5-(1-((tétrahydro-2H-pyran-4-yl)méthyl)pipéridin-4-yl)thiazole (1185) ;
2-(5-(7,8-diméthyl-[1,2,4]triazolo[1,5-a]pyridin-6-yl)-4-(2,2,2-trifluoroéthyl)-1H-pyrazol-3-yl)-4-méthyl-5-(1-((tétrahydro-2H-pyran-4-yl)méthyl)pipéridin-4-yl)thiazole (1186) ;
2-(5-(7,8-diméthyl-[1,2,4]triazolo[1,5-a]pyridin-6-yl)-4-(2,2,2-trifluoroéthyl)-1H-pyrazol-3-yl)-5-(1-isopropylpipéridin-4-yl)-4-méthylthiazole (1187) ;
5-(1-(cyclopropylméthyl)pipéridin-4-yl)-2-(5-(7,8-diméthyl-[1,2,4]triazolo[1,5-a] pyridin-6-yl)-4-(2,2,2-trifluoroéthyl)-1H-pyrazol-3-yl)-4-méthylthiazole (1188) ;
2-(5-(7,8-diméthyl-[1,2,4]triazolo[1,5-a]pyridin-6-yl)-4-(2,2,2-trifluoroéthyl)-1H-pyrazol-3-yl)-4-méthyl-5-(1-méthylpipéridin-4-yl)thiazole (1189) ;
2-(5-(7,8-diméthyl-[1,2,4]triazolo[1,5-a]pyridin-6-yl)-4-(2,2,2-trifluoroéthyl)-1H-pyrazol-3-yl)-5-(1-éthylpipéridin-4-yl)-4-méthylthiazole (1190) ;
2-(5-(7,8-diméthyl-[1,2,4]triazolo[1,5-a]pyridin-6-yl)-4-(2,2,2-trifluoroéthyl)-1H-pyrazol-3-yl)-4-méthyl-5-(1-propylpipéridin-4-yl)thiazole (1191) ;
2-(diméthylamino)-1-(4-(2-(5-(8-méthoxy-[1,2,4]triazolo[1,5-a]pyridin-6-yl)-4-(2,2,2-trifluoroéthyl)-1H-pyrazol-3-yl)thiazol-5-yl)pipéridin-1-yl)éthan-1-one (1192) ;
2-(diméthylamino)-1-(4-(2-(5-(8-méthyl-[1,2,4]triazolo[1,5-a]pyridin-6-yl)-4-(2,2,2-trifluoroéthyl)-1H-pyrazol-3-yl)thiazol-5-yl)pipéridin-1-yl)éthan-1-one (1193) ;
1-(4-(2-(5-(8-méthyl-[1,2,4]triazolo[1,5-a]pyridin-6-yl)-4-(2,2,2-trifluoroéthyl)-1H-pyrazol-3-yl)thiazol-5-yl)pipéridin-1-yl)-2-morpholinoéthan-1-one (1194) ;
2-(diéthylamino)-1-(4-(2-(5-(8-méthyl-[1,2,4]triazolo[1,5-a]pyridin-6-yl)-4-(2,2,2-trifluoroéthyl)-1H-pyrazol-3-yl)thiazol-5-yl)pipéridin-1-yl)éthan-1-one (1195) ;
2-(5-(8-méthoxy-[1,2,4]triazolo[1,5-a]pyridin-6-yl)-4-(2,2,2-trifluoroéthyl)-1H-pyrazol-3-yl)-5-(1-(2-méthoxyéthyl)pipéridin-4-yl)thiazole (1196) ;
5-(1-(2-méthoxyethyl)pipéridin-4-yl)-2-(5-(8-méthyl-[1,2,4]triazolo[1,5-a]pyridin-6-yl)-4-(2,2,2-trifluoroéthyl)-1H-pyrazol-3-yl)thiazole (1197) ;
2-méthyl-1-(4-(2-(5-(8-méthyl-[1,2,4]triazolo[1,5-a]pyridin-6-yl)-4-(2,2,2-trifluoroéthyl)-1H-pyrazol-3-yl)thiazol-5-yl)pipéridin-1-yl)propan-2-ol (1198) ;
2-méthyl-1-(4-(4-méthyl-2-(5-(8-méthyl-[1,2,4]triazolo[1,5-a]pyridin-6-yl)-4-(2,2,2-trifluoroéthyl)-1H-pyrazol-3-yl)thiazol-5-yl)pipéridin-1-yl)propan-2-ol (1199) ;
2-(5-(8-méthoxy-[1,2,4]triazolo[1,5-a]pyridin-6-yl)-4-(2,2,2-trifluoroéthyl)-1H-pyrazol-3-yl)-5-(1-(2-(méthylsulfonyl)éthyl)pipéridin-4-yl)thiazole (1200) ;
2-(4-(2-(5-(8-méthoxy-[1,2,4]triazolo[1,5-a]pyridin-6-yl)-4-(2,2,2-trifluoroéthyl)-1H-pyrazol-3-yl)thiazol-5-yl)pipéridin-1-yl)-N,N-diméthylacétamide (1201) ;
5-(1-(2-(tert-butoxy)éthyl)pipéridin-4-yl)-2-(5-(8-méthoxy-[1,2,4]triazolo[1,5-a] pyridin-6-yl)-4-(2,2,2-trifluoroéthyl)-1H-pyrazol-3-yl)thiazole (1202) ;
2-(4-(2-(5-(8-méthyl-[1,2,4]triazolo[1,5-a]pyridin-6-yl)-4-(2,2,2-trifluoroéthyl)-1H-pyrazol-3-yl)thiazol-5-yl)pipéridin-1-yl)-1-morpholinoéthan-1-one (1203) ;
N,N-diméthyl-2-(4-(2-(5-(8-méthyl-[1,2,4]triazolo[1,5-a]pyridin-6-yl)-4-(2,2,2-trifluoroéthyl)-1H-pyrazol-3-yl)thiazol-5-yl)pipéridin-1-yl)acétamide (1204) ;
5-(1-(2-fluoroéthyl)pipéridin-4-yl)-2-(5-(8-méthyl-[1,2,4]triazolo[1,5-a]pyridin-6-yl)-4-(2,2,2-trifluoroéthyl)-1H-pyrazol-3-yl)thiazole (1205) ;
5-(1-(3-fluoropropyl)pipéridin-4-yl)-2-(5-(8-méthyl-[1,2,4]triazolo[1,5-a]pyridin-6-yl)-4-(2,2,2-trifluoroéthyl)-1H-pyrazol-3-yl)thiazole (1206) ;
2-(4-(2-(5-(8-méthoxy-[1,2,4]triazolo[1,5-a]pyridin-6-yl)-4-(2,2,2-trifluoroéthyl)-1H-pyrazol-3-yl)thiazol-5-yl)pipéridin-1-yl)-1-(2-oxa-6-azaspiro[3.3] heptan-6-yl)éthan-1one (1207) ;
N-isopropyl-2-(4-(2-(5-(8-méthoxy-[1,2,4]triazolo[1,5-a]pyridin-6-yl)-4-(2,2,2-trifluoroéthyl)-1H-pyrazol-3-yl)thiazol-5-yl)pipéridin-1-yl)-N-méthylacétamide (1208) ;
1-(azétidin-1-yl)-2-(4-(2-(5-(8-méthoxy-[1,2,4]triazolo[1,5-a]pyridin-6-yl)-4-(2,2,2-trifluoroéthyl)-1H-pyrazol-3-yl)thiazol-5-yl)pipéridin-1-yl)éthan-1-one (1209) ;
1-(4-(2-(5-(8-méthoxy-[1,2,4]triazolo[1,5-a]pyridin-6-yl)-4-(2,2,2-trifluoroéthyl)-1H-pyrazol-3-yl)thiazol-5-yl)pipéridin-1-yl)-2-morpholinoéthan-1-one (1210) ;
1-(4-(2-(5-(8-méthoxy-[1,2,4]triazolo[1,5-a]pyridin-6-yl)-4-(2,2,2-trifluoroéthyl)-1H-pyrazol-3-yl)thiazol-5-yl)pipéridin-1-yl)-2-(7-oxa-2-azaspiro[3.5]nonan-2-yl)éthan-1-one (1211) ;
4-(2-(4-(2-(5-(8-méthoxy-[1,2,4]triazolo[1,5-a]pyridin-6-yl)-4-(2,2,2-trifluoroéthyl)-1H-pyrazol-3-yl)thiazol-5-yl)pipéridin-1-yl)-2-oxoéthyl)pipérazin-2-one (1212) ;
1-(4-(2-(5-(8-méthoxy-[1,2,4]triazolo[1,5-a]pyridin-6-yl)-4-(2,2,2-trifluoroéthyl)-1H-pyrazol-3-yl)thiazol-5-yl)pipéridin-1-yl)-2-(3-méthoxyazétidin-1-yl)éthan-1-one (1213) ;
1-(4-(2-(5-(8-méthoxy-[1,2,4]triazolo[1,5-a]pyridin-6-yl)-4-(2,2,2-trifluoroéthyl)-1H-pyrazol-3-yl)thiazol-5-yl)pipéridin-1-yl)-2-((2-méthoxyéthyl)(méthyl)amino)éthan-1-one (1214) ;
4-(4-(2-(5-(8-méthoxy-[1,2,4]triazolo[1,5-a]pyridin-6-yl)-4-(2,2,2-trifluoroéthyl)-1H-pyrazol-3-yl)-4-méthylthiazol-5-yl)cyclohexyl)morpholine (1215) ;
N-(4-(2-(5-(8-méthoxy-[1,2,4]triazolo[1,5-a]pyridin-6-yl)-4-(2,2,2-trifluoroéthyl)-1H-pyrazol-3-yl)-4-méthylthiazol-5-yl)cyclohexyl)oxétan-3-amine (1216-1217) ;
6-(4-(2-(5-(8-méthoxy-[1,2,4]triazolo[1,5-a]pyridin-6-yl)-4-(2,2,2-trifluoroéthyl)-1H-pyrazol-3-yl)-4-méthylthiazol-5-yl)cyclohexyl)-2-oxa-6-azaspiro[3.3]heptane (1218-1219) ;
2-(4-(2-(5-(8-méthoxy-[1,2,4]triazolo[1,5-a]pyridin-6-yl)-4-(2,2,2-trifluoroéthyl)-1H-pyrazol-3-yl)-4-méthylthiazol-5-yl)cyclohexyl)-7-oxa-2-azaspiro[3.5]nonane (1220-1221) ;
4-(4-(2-(5-(8-méthoxy-[1,2,4]triazolo[1,5-a]pyridin-6-yl)-4-(2,2,2-trifluoroéthyl)-1H-pyrazol-3-yl)-4-méthylthiazol-5-yl)cyclohexyl)pipérazin-2-one (1222-1223) ;
2-(5-(8-méthoxy-[1,2,4]triazolo[1,5-a]pyridin-6-yl)-4-(2,2,2-trifluoroéthyl)-1H-pyrazol-3-yl)-5-(4-(3-méthoxyazétidin-1-yl)cyclohexyl)thiazole (1224-1225) ;
2-(4-(2-(5-(8-méthoxy-[1,2,4]triazolo[1,5-a]pyridin-6-yl)-4-(2,2,2-trifluoroéthyl)-1H-pyrazol-3-yl)thiazol-5-yl)cyclohexyl)-7-oxa-2-azaspiro[3.5]nonane (1226-1227) ;
4-(4-(2-(5-(8-méthoxy-[1,2,4]triazolo[1,5-a]pyridin-6-yl)-4-(2,2,2-trifluoroéthyl)-1H-pyrazol-3-yl)thiazol-5-yl)cyclohexyl)pipérazin-2-one (1228-1229) ;
4-(4-(2-(5-(8-méthoxy-[1,2,4]triazolo[1,5-a]pyridin-6-yl)-4-(2,2,2-trifluoroéthyl)-1H-pyrazol-3-yl)thiazol-5-yl)cyclohexyl)morpholine (1230-1231) ;
2-(5-(8-méthoxy-[1,2,4]triazolo[1,5-a]pyridin-6-yl)-4-(2,2,2-trifluoroéthyl)-1H-pyrazol-3-yl)-4-méthyl-5-((1S,4S)-5-méthyl-2,5-diazabicyclo[2.2.1]heptan-2-yl)thiazole (1232) ;
2-(5-(8-méthoxy-[1,2,4]triazolo[1,5-a]pyridin-6-yl)-4-(2,2,2-trifluoroéthyl)-1H-pyrazol-3-yl)-4-méthyl-5-(pipérazin-1-yl)thiazole (1233) ;
4-méthyl-2-(5-(8-méthyl-[1,2,4]triazolo[1,5-a]pyridin-6-yl)-4-(2,2,2-trifluoroéthyl)-1H-pyrazol-3-yl)-5-(pipérazin-1-yl)thiazole (1234) ;
5-((2R,5S)-2,5-diméthylpipérazin-1-yl)-2-(5-(8-méthoxy-[1,2,4]triazolo[1,5-a] pyridin-6-yl)-4-(2,2,2-trifluoroéthyl)-1H-pyrazol-3-yl)thiazole (1235) ;
2-(5-(8-méthoxy-[1,2,4]triazolo[1,5-a]pyridin-6-yl)-4-(2,2,2-trifluoroéthyl)-1H-pyrazol-3-yl)-4-méthyl-5-(2,6-diazaspiro[3.3]heptan-2-yl)thiazole (1236) ;
5-((1S,4S)-5-éthyl-2,5-diazabicyclo[2.2.1]heptan-2-yl)-2-(5-(8-méthoxy-[1,2,4]triazolo[1,5-a]pyridin-6-yl)-4-(2,2,2-trifluoroéthyl)-1H-pyrazol-3-yl)-4méthylthiazole (1237) ;
2-(5-(8-méthoxy-[1,2,4]triazolo[1,5-a]pyridin-6-yl)-4-(2,2,2-trifluoroéthyl)-1H-pyrazol-3-yl)-4-méthyl-5-((1R,4R)-5-propyl-2,5-diazabicyclo[2.2.1]heptan-2-yl)thiazole (1238) ;
2-(5-(8-méthoxy-[1,2,4]triazolo[1,5-a]pyridin-6-yl)-4-(2,2,2-trifluoroéthyl)-1H-pyrazol-3-yl)-4-méthyl-5-((1S,4S)-5-(oxétan-3-yl)-2,5-diazabicyclo[2.2.1]heptan-2-yl) thiazole (1239) ;
2-(5-(8-méthoxy-[1,2,4]triazolo[1,5-a]pyridin-6-yl)-4-(2,2,2-trifluoroéthyl)-1H-pyrazol-3-yl)-4-méthyl-5-((1S,4S)-5-((tétrahydro-2H-pyran-4-yl)méthyl)-2,5-diazabicyclo[2.2.1]heptan-2-yl)thiazole (1240) ;
5-((1S,4S)-5-isopropyl-2,5-diazabicyclo[2.2.1]heptan-2-yl)-2-(5-(8-méthoxy-[1,2,4]triazolo[1,5-a]pyridin-6-yl)-4-(2,2,2-trifluoroéthyl)-1H-pyrazol-3-yl)-4méthylthiazole (1241) ;
2-(5-(8-méthoxy-[1,2,4]triazolo[1,5-a]pyridin-6-yl)-4-(2,2,2-trifluoroéthyl)-1H-pyrazol-3-yl)-4-méthyl-5-((1S,4S)-5-(tétrahydro-2H-pyran-4-yl)-2,5-diazabicyclo[2.2.1]heptan-2-yl)thiazole (1242) ;
5-((1S,4S)-5-isobutyl-2,5-diazabicyclo[2.2.1]heptan-2-yl)-2-(5-(8-méthoxy-[1,2,4]triazolo[1,5-a]pyridin-6-yl)-4-(2,2,2-trifluoroéthyl)-1H-pyrazol-3-yl)-4méthylthiazole (1243) ;
2-(5-(8-méthoxy-[1,2,4]triazolo[1,5-a]pyridin-6-yl)-4-(2,2,2-trifluoroéthyl)-1H-pyrazol-3-yl)-4-méthyl-5-(4-méthylpipérazin-1-yl)thiazole (1244) ;
2-(5-(8-méthoxy-[1,2,4]triazolo[1,5-a]pyridin-6-yl)-4-(2,2,2-trifluoroéthyl)-1H-pyrazol-3-yl)-4-méthyl-5-(4-((tétrahydro-2H-pyran-4-yl)méthyl)pipérazin-1-yl)thiazole (1245) ;
2-(5-(8-méthoxy-[1,2,4]triazolo[1,5-a]pyridin-6-yl)-4-(2,2,2-trifluoroéthyl)-1H-pyrazol-3-yl)-4-méthyl-5-(4-propylpipérazin-1-yl)thiazole (1246) ;
5-(4-éthylpipérazin-1-yl)-2-(5-(8-méthoxy-[1,2,4]triazolo[1,5-a]pyridin-6-yl)-4-(2,2,2-trifluoroéthyl)-1H-pyrazol-3-yl)-4-méthylthiazole (1247) ;
5-(4-isobutylpipérazin-1-yl)-2-(5-(8-méthoxy-[1,2,4]triazolo[1,5-a]pyridin-6-yl)-4-(2,2,2-trifluoroéthyl)-1H-pyrazol-3-yl)-4-méthylthiazole (1248) ;
4-méthyl-2-(5-(8-méthyl-[1,2,4]triazolo[1,5-a]pyridin-6-yl)-4-(2,2,2-trifluoroéthyl)-1H-pyrazol-3-yl)-5-(4-(oxétan-3-yl)pipérazin-1-yl)thiazole (1249) ;
4-méthyl-2-(5-(8-méthyl-[1,2,4]triazolo[1,5-a]pyridin-6-yl)-4-(2,2,2-trifluoroéthyl)-1H-pyrazol-3-yl)-5-(4-methylpipérazin-1-yl)thiazole (1250) ;
5-(4-éthylpipérazin-1-yl)-4-méthyl-2-(5-(8-méthyl-[1,2,4]triazolo[1,5-a]pyridin-6-yl)-4-(2,2,2-trifluoroéthyl)-1H-pyrazol-3-yl)thiazole (1251) ;
4-méthyl-2-(5-(8-méthyl-[1,2,4]triazolo[1,5-a]pyridin-6-yl)-4-(2,2,2-trifluoroéthyl)-1H-pyrazol-3-yl)-5-(4-propylpipérazin-1-yl)thiazole (1252) ;
4-méthyl-2-(5-(8-méthyl-[1,2,4]triazolo[1,5-a]pyridin-6-yl)-4-(2,2,2-trifluoroéthyl)-1H-pyrazol-3-yl)-5-(4-((tétrahydro-2H-pyran-4-yl)methyl)pipérazin-1-yl)thiazole (1253) ;
4-méthyl-2-(5-(8-méthyl-[1,2,4]triazolo[1,5-a]pyridin-6-yl)-4-(2,2,2-trifluoroéthyl)-1H-pyrazol-3-yl)-5-(4-(tétrahydro-2H-pyran-4-yl)pipérazin-1-yl)thiazole (1254) ;
5-(4-(cyclopropylméthyl)pipérazin-1-yl)-4-méthyl-2-(5-(8-méthyl-[1,2,4]triazolo[1,5-a]pyridin-6-yl)-4-(2,2,2-trifluoroéthyl)-1H-pyrazol-3-yl)thiazole (1256) ;
2-(5-(8-méthoxy-[1,2,4]triazolo[1,5-a]pyridin-6-yl)-4-(2,2,2-trifluoroéthyl)-1H-pyrazol-3-yl)-5-((2R,5S)-2,4,5-triméthylpipérazin-1-yl)thiazole (1257) ;
5-((2R,5S)-4-éthyl-2,5-diméthylpipérazin-1-yl)-2-(5-(8-méthoxy-[1,2,4]triazolo[1,5-a]pyridin-6-yl)-4-(2,2,2-trifluoroéthyl)-1H-pyrazol-3-yl)thiazole (1258) ;
5-((2R,5S)-2,5-diméthyl-4-propylpipérazin-1-yl)-2-(5-(8-méthoxy-[1,2,4]triazolo[1,5-a]pyridin-6-yl)-4-(2,2,2-trifluoroéthyl)-1H-pyrazol-3-yl)thiazole (1259) ;
5-((2R,5S)-4-isopropyl-2,5-diméthylpipérazin-1-yl)-2-(5-(8-méthoxy-[1,2,4]triazolo[1,5-a]pyridin-6-yl)-4-(2,2,2-trifluoroéthyl)-1H-pyrazol-3-yl)thiazole (1260) ;
2-(5-(8-méthoxy-[1,2,4]triazolo[1,5-a]pyridin-6-yl)-4-(2,2,2-trifluoroéthyl)-1H-pyrazol-3-yl)-4-méthyl-5-(6-méthyl-2,6-diazaspiro[3.3]heptan-2-yl)thiazole (1261) ;
5-(6-isobutyl-2,6-diazaspiro[3.3]heptan-2-yl)-2-(5-(8-méthoxy-[1,2,4]triazolo[1,5-a] pyridin-6-yl)-4-(2,2,2-trifluoroéthyl)-1H-pyrazol-3-yl)-4-méthylthiazole (1262) ;
2-(5-(8-méthoxy-[1,2,4]triazolo[1,5-a]pyridin-6-yl)-4-(2,2,2-trifluoroéthyl)-1H-pyrazol-3-yl)-4-méthyl-5-(6-(tétrahydro-2H-pyran-4-yl)-2,6-diazaspiro[3.3]heptan-2-yl) thiazole (1263) ;
5-(6-(cyclopropylméthyl)-2,6-diazaspiro[3.3]heptan-2-yl)-2-(5-(8-méthoxy-[1,2,4]triazolo[1,5-a]pyridin-6-yl)-4-(2,2,2-trifluoroéthyl)-1H-pyrazol-3-yl)-4-méthylthiazole (1264) ;
5-(6-(1-isopropylpipéridin-4-yl)-2,6-diazaspiro[3.3]heptan-2-yl)-2-(5-(8-méthoxy-[1,2,4]triazolo[1,5-a]pyridin-6-yl)-4-(2,2,2-trifluoroéthyl)-1H-pyrazol-3-yl)-4-méthylthiazole (1265) ;
5-(6-isopropyl-2,6-diazaspiro[3.3]heptan-2-yl)-2-(5-(8-méthoxy-[1,2,4]triazolo[1,5-a] pyridin-6-yl)-4-(2,2,2-trifluoroéthyl)-1H-pyrazol-3-yl)-4-méthylthiazole (1266) ;
5-(6-(1-cyclopropyléthyl)-2,6-diazaspiro[3.3]heptan-2-yl)-2-(5-(8-méthoxy-[1,2,4]triazolo[1,5-a]pyridin-6-yl)-4-(2,2,2-trifluoroéthyl)-1H-pyrazol-3-yl)-4-méthylthiazole (1267) ;
2-(5-(8-méthoxy-[1,2,4]triazolo[1,5-a]pyridin-6-yl)-4-(2,2,2-trifluoroéthyl)-1H-pyrazol-3-yl)-4-méthyl-5-(6-(pentan-3-yl)-2,6-diazaspiro[3.3]heptan-2-yl)thiazole (1268) ;
1-(2-(4-isopropyl-5-(8-méthoxy-[1,2,4]triazolo[1,5-a]pyridin-6-yl)-1H-pyrazol-3-yl) thiazol-5-yl)-N-(3-méthyloxétan-3-yl)pipéridin-4-amine (1269) ;
1-(2-(4-isopropyl-5-(8-méthoxy-[1,2,4]triazolo[1,5-a]pyridin-6-yl)-1H-pyrazol-3-yl) thiazol-5-yl)-N-(2-méthoxy-2-méthylpropyl)pipéridin-4-amine (1270) ;
N-éthyl-1-(2-(4-isopropyl-5-(8-méthoxy-[1,2,4]triazolo[1,5-a]pyridin-6-yl)-1H-pyrazol-3-yl)thiazol-5-yl)-N-méthylpipéridin-4-amine (1271) ;
2-(5-(8-méthoxy-[1,2,4]triazolo[1,5-a]pyridin-6-yl)-4-(2,2,2-trifluoroéthyl)-1H-pyrazol-3-yl)-N,N-diméthyl-4,5,6,7-tétrahydrobenzo[d]thiazol-6-amine (1272) ;
N-isopropyl-2-(5-(8-méthoxy-[1,2,4]triazolo[1,5-a]pyridin-6-yl)-4-(2,2,2-trifluoroéthyl)-1H-pyrazol-3-yl)-N-méthyl-4,5,6,7-tétrahydrobenzo[d]thiazol-6-amine (1273) ;
2-(5-(8-méthoxy-[1,2,4]triazolo[1,5-a]pyridin-6-yl)-4-(2,2,2-trifluoroéthyl)-1H-pyrazol-3-yl)-N,N-diméthyl-4,5,6,7-tétrahydrobenzo[d]thiazol-6-amine (1274) ;
la 8-méthoxy-6-(3-(4-méthyl-5-(1-méthylpipéridin-4-yl)pyridin-2-yl)-4-(2,2,2-trifluoroéthyl)-1H-pyrazol-5-yl)-[1,2,4]triazolo[1,5-a]pyridine (1275) ;
la 8-méthoxy-6-(3-(5-(pipéridin-4-yl)pyridin-2-yl)-4-(2,2,2-trifluoroéthyl)-1H-pyrazol-5-yl)-[1,2,4]triazolo[1,5-a]pyridine (1276) ;
6-(3-(5-(1-éthylpipéridin-4-yl)-4-méthylpyridin-2-yl)-4-(2,2,2-trifluoroéthyl)-1H-pyrazol-5-yl)-8-méthoxy-[1,2,4]triazolo[1,5-a]pyridine (1277) ;
8-méthoxy-6-(3-(4-méthyl-5-(1-propylpipéridin-4-yl)pyridin-2-yl)-4-(2,2,2-trifluoroéthyl)-1H-pyrazol-5-yl)-[1,2,4]triazolo[1,5-a]pyridine (1278) ;
8-méthoxy-6-(3-(5-(1-(oxétan-3-yl)pipéridin-4-yl)-4-(trifluorométhyl)pyridin-2-yl)-4-(2,2,2-trifluoroéthyl)-1H-pyrazol-5-yl)-[1,2,4]triazolo[1,5-a]pyridine (1279) ;
8-méthoxy-6-(3-(4-méthyl-5-(1-(tétrahydro-2H-pyran-4-yl)pipéridin-4-yl)pyridin-2-yl)-4-(2,2,2-trifluoroéthyl)-1H-pyrazol-5-yl)-[1,2,4]triazolo[1,5-a]pyridine (1280) ;
8-méthoxy-6-(3-(4-méthyl-5-(1-((tétrahydro-2H-pyran-4-yl)méthyl)pipéridin-4-yl)pyridin-2-yl)-4-(2,2,2-trifluoroéthyl)-1H-pyrazol-5-yl)-[1,2,4]triazolo[1,5-a]pyridine (1281) ;
6-(3-(5-(1-isopropylpipéridin-4-yl)-4-méthylpyridin-2-yl)-4-(2,2,2-trifluoroéthyl)-1H-pyrazol-5-yl)-8-méthoxy-[1,2,4]triazolo[1,5-a]pyridine (1282) ;
6-(3-(5-(1-(2-éthylbutyl)pipéridin-4-yl)-4-méthylpyridin-2-yl)-4-(2,2,2-trifluoroéthyl)-1H-pyrazol-5-yl)-8-méthoxy-[1,2,4]triazolo[1,5-a]pyridine (1283) ;
6-(3-(5-(1-isobutylpipéridin-4-yl)-4-méthylpyridin-2-yl)-4-(2,2,2-trifluoroéthyl)-1H-pyrazol-5-yl)-8-méthoxy-[1,2,4]triazolo[1,5-a]pyridine (1284) ;
6-(3-(5-(1-(cyclobutylméthyl)pipéridin-4-yl)-4-méthylpyridin-2-yl)-4-(2,2,2-trifluoroéthyl)-1H-pyrazol-5-yl)-8-méthoxy-[1,2,4]triazolo[1,5-a]pyridine (1285) ;
6-(3-(5-(1-éthylpipéridin-4-yl)pyridin-2-yl)-4-(2,2,2-trifluoroéthyl)-1H-pyrazol-5-yl)-8-méthoxy-[1,2,4]triazolo[1,5-a]pyridine (1286) ;
8-méthoxy-6-(3-(5-(1-méthylpipéridin-4-yl)pyridin-2-yl)-4-(2,2,2-trifluoroéthyl)-1H-pyrazol-5-yl)-[1,2,4]triazolo[1,5-a]pyridine (1287) ;
6-(3-(5-(1-isobutylpipéridin-4-yl)pyridin-2-yl)-4-(2,2,2-trifluoroéthyl)-1H-pyrazol-5-yl)-8-méthoxy-[1,2,4]triazolo[1,5-a]pyridine (1288) ;
6-(3-(5-(1-cyclobutylpipéridin-4-yl)pyridin-2-yl)-4-(2,2,2-trifluoroéthyl)-1H-pyrazol-5-yl)-8-méthoxy-[1,2,4]triazolo[1,5-a]pyridine (1289) ;
6-(3-(5-(1-isopropylpipéridin-4-yl)pyridin-2-yl)-4-(2,2,2-trifluoroéthyl)-1H-pyrazol-5-yl)-8-méthoxy-[1,2,4]triazolo[1,5-a]pyridine (1290) ;
8-méthoxy-6-(3-(5-(1-propylpipéridin-4-yl)pyridin-2-yl)-4-(2,2,2-trifluoroéthyl)-1H-pyrazol-5-yl)-[1,2,4]triazolo[1,5-a]pyridine (1291) ;
8-méthoxy-6-(3-(5-(1-(oxétan-3-yl)pipéridin-4-yl)pyridin-2-yl)-4-(2,2,2-trifluoroéthyl)-1H-pyrazol-5-yl)-[1,2,4]triazolo[1,5-a]pyridine (1292) ;
8-méthoxy-6-(3-(5-(1-(tétrahydro-2H-pyran-4-yl)pipéridin-4-yl)pyridin-2-yl)-4-(2,2,2-trifluoroéthyl)-1H-pyrazol-5-yl)-[1,2,4]triazolo[1,5-a]pyridine (1293) ;
8-méthoxy-6-(3-(5-(1-((tétrahydro-2H-pyran-4-yl)méthyl)pipéridin-4-yl)pyridin-2-yl)-4-(2,2,2-trifluoroéthyl)-1H-pyrazol-5-yl)-[1,2,4]triazolo[1,5-a]pyridine (1294) ;
6-(3-(5-(1-(cyclobutylméthyl)pipéridin-4-yl)pyridin-2-yl)-4-(2,2,2-trifluoroéthyl)-1H-pyrazol-5-yl)-8-méthoxy-[1,2,4]triazolo[1,5-a]pyridine (1295) ;
2-(diméthylamino)-1-(4-(6-(5-(8-méthoxy-[1,2,4]triazolo[1,5-a]pyridin-6-yl)-4-(2,2,2-trifluoroéthyl)-1H-pyrazol-3-yl)-4-méthylpyridin-3-yl)pipéridin-1-yl)éthan-1-one (1296) ;
2-(diméthylamino)-1-(4-(6-(5-(8-méthoxy-[1,2,4]triazolo[1,5-a]pyridin-6-yl)-4-(2,2,2-trifluoroéthyl)-1H-pyrazol-3-yl)pyridin-3-yl)pipéridin-1-yl)éthan-1-one (1297) ;
2-(diéthylamino)-1-(4-(6-(5-(8-méthoxy-[1,2,4]triazolo[1,5-a]pyridin-6-yl)-4-(2,2,2-trifluoroéthyl)-1H-pyrazol-3-yl)pyridin-3-yl)pipéridin-1-yl)éthan-1-one (1298) ;
2-(4-(6-(5-(8-méthoxy-[1,2,4]triazolo[1,5-a]pyridin-6-yl)-4-(2,2,2-trifluoroéthyl)-1H-pyrazol-3-yl)-4-méthylpyridin-3-yl)pipéridin-1-yl)-N,N-diméthylacétamide (1299) ;
2-(4-(6-(5-(8-méthoxy-[1,2,4]triazolo[1,5-a]pyridin-6-yl)-4-(2,2,2-trifluoroéthyl)-1H-pyrazol-3-yl)-4-méthylpyridin-3-yl)pipéridin-1-yl)-N-méthylacétamide (1300) ;
8-méthoxy-6-(3-(5-(1-(2-méthoxyéthyl)pipéridin-4-yl)-4-méthylpyridin-2-yl)-4-(2,2,2-trifluoroéthyl)-1H-pyrazol-5-yl)-[1,2,4]triazolo[1,5-a]pyridine (1301) ;
8-méthoxy-6-(3-(4-méthyl-5-(1-(2-(méthylsulfonyl)éthyl)pipéridin-4-yl)pyridin-2-yl)-4-(2,2,2-trifluoroéthyl)-1H-pyrazol-5-yl)-[1,2,4]triazolo[1,5-a]pyridine (1302) ;
N-(2-(4-(5-(8-méthyl-[1,2,4]triazolo[1,5-a]pyridin-6-yl)-4-(2,2,2-trifluoroéthyl)-1H-pyrazol-3-yl)phényl)propan-2-yl)tétrahydro-2H-pyran-4-amine (1303) ;
(S)-1-(4-(5-(8-méthoxy-[1,2,4]triazolo[1,5-a]pyridin-6-yl)-4-(2,2,2-trifluoroéthyl)-1H-pyrazol-3-yl)phényl)-N-méthyléthan-1-amine (1304B) ;
6-(5-(8-méthoxy-[1,2,4]triazolo[1,5-a]pyridin-6-yl)-4-(2,2,2-trifluoroéthyl)-1H-pyrazol-3-yl)-N-méthyl-1,2,3,4-tétrahydronaphtalène-2-amine (1305B) ;
6-(5-(8-méthoxy-[1,2,4]triazolo[1,5-a]pyridin-6-yl)-4-(2,2,2-trifluoroéthyl)-1H-pyrazol-3-yl)-N-méthyl-1,2,3,4-tétrahydronaphtalène-2-amine (1306B) ;
6-(5-(8-méthoxy-[1,2,4]triazolo[1,5-a]pyridin-6-yl)-4-(2,2,2-trifluoroéthyl)-1H-pyrazol-3-yl)-N-méthyl-1,2,3,4-tétrahydronaphtalène-2-amine (1307B) ;
N,N-diméthyl-2-(4-(5-(8-méthyl-[1,2,4]triazolo[1,5-a]pyridin-6-yl)-4-(2,2,2-trifluoroéthyl)-1H-pyrazol-3-yl)phényl)propan-2-amine (1308) ;
(S)-1-(4-(5-(8-méthoxy-[1,2,4]triazolo[1,5-a]pyridin-6-yl)-4-(2,2,2-trifluoroéthyl)-1H-pyrazol-3-yl)phényl)-N,N-dimethyléthan-1-amine (1309) ;
6-(5-(8-méthoxy-[1,2,4]triazolo[1,5-a]pyridin-6-yl)-4-(2,2,2-trifluoroéthyl)-1H-pyrazol-3-yl)-N,N-diméthyl-1,2,3,4-tétrahydronaphtalène-2-amine (1310) ;
N-isopropyl-6-(5-(8-méthoxy-[1,2,4]triazolo[1,5-a]pyridin-6-yl)-4-(2,2,2-trifluoroéthyl)-1H-pyrazol-3-yl)-N-méthyl-1,2,3,4-tétrahydronaphtalène-2-amine (1311) ;
N-(6-(5-(8-méthoxy-[1,2,4]triazolo[1,5-a]pyridin-6-yl)-4-(2,2,2-trifluoroéthyl)-1H-pyrazol-3-yl)-1,2,3,4-tétrahydronaphthalèn-2-yl)-N-méthyloxétan-3-amine (1312) ;
2-(diméthylamino)-N -(2-(4-(5-(8-méthyl-[1,2,4]triazolo[1,5-a]pyridin-6-yl)-4-(2,2,2-trifluoroéthyl)-1H-pyrazol-3-yl)phényl)propan-2-yl)acétamide (1313) ;
(S)-2-(diméthylamino)-N -(1-(4-(5-(8-méthoxy-[1,2,4]triazolo[1,5-a]pyridin-6-yl)-4-(2,2,2-trifluoroéthyl)-1H-pyrazol-3-yl)phényl)éthyl)-N-méthylacétamide (1314) ;
2-(diméthylamino)-N -(6-(5-(8-méthoxy-[1,2,4]triazolo[1,5-a]pyridin-6-yl)-4-(2,2,2-trifluoroéthyl)-1H-pyrazol-3-yl)-1,2,3,4-tétrahydronaphtalène-2-yl)-N-méthylacétamide (1315) ;
8-méthoxy-6-(3-(5-(6-((tétrahydro-2H-pyran-4-yl)méthyl)-2,6-diazaspiro[3.3]heptan-2-yl)pyridin-2-yl)-4-(2,2,2-trifluoroéthyl)-1H-pyrazol-5-yl)-[1,2,4]triazolo[1,5-a]pyridine (1316) ;
8-méthoxy-6-(3-(5-(6-(oxétan-3-yl)-2,6-diazaspiro[3.3]heptan-2-yl)pyridin-2-yl)-4-(2,2,2-trifluoroéthyl)-1H-pyrazol-5-yl)-[1,2,4]triazolo[1,5-a]pyridine (1317) ;
6-(3-(5-(6-isopropyl-2,6-diazaspiro[3.3]heptan-2-yl)pyridin-2-yl)-4-(2,2,2-trifluoroéthyl)-1H-pyrazol-5-yl)-8-méthoxy-[1,2,4]triazolo[1,5-a]pyridine (1318) ;
8-méthoxy-6-(3-(5-(6-propyl-2,6-diazaspiro[3.3]heptan-2-yl)pyridin-2-yl)-4-(2,2,2-trifluoroéthyl)-1H-pyrazol-5-yl)-[1,2,4]triazolo[1,5-a]pyridine (1319) ;
6-(3-(5-(6-éthyl-2,6-diazaspiro[3.3]heptan-2-yl)pyridin-2-yl)-4-(2,2,2-trifluoroéthyl)-1H-pyrazol-5-yl)-8-méthoxy-[1,2,4]triazolo[1,5-a]pyridine (1320) ;
8-méthoxy-6-(3-(5-(6-méthyl-2,6-diazaspiro[3.3]heptan-2-yl)pyridin-2-yl)-4-(2,2,2-trifluoroéthyl)-1H-pyrazol-5-yl)-[1,2,4]triazolo[1,5-a]pyridine (1321) ;
6-(3-(5-(6-cyclobutyl-2,6-diazaspiro[3.3]heptan-2-yl)pyridin-2-yl)-4-(2,2,2-trifluoroéthyl)-1H-pyrazol-5-yl)-8-méthoxy-[1,2,4]triazolo[1,5-a]pyridine (1322) ;
6-(3-(5-(6-isobutyl-2,6-diazaspiro[3.3]heptan-2-yl)pyridin-2-yl)-4-(2,2,2-trifluoroéthyl)-1H-pyrazol-5-yl)-8-méthoxy-[1,2,4]triazolo[1,5-a]pyridine (1323) ;
8-méthoxy-6-(3-(5-(6-(tétrahydro-2H-pyran-4-yl)-2,6-diazaspiro[3.3]heptan-2-yl) pyridin-2-yl)-4-(2,2,2-trifluoroéthyl)-1H-pyrazol-5-yl)-[1,2,4]triazolo[1,5-a]pyridine (1324) ;
6-(3-(5-(6-(cyclobutylméthyl)-2,6-diazaspiro[3.3]heptan-2-yl)pyridin-2-yl)-4-(2,2,2-trifluoroéthyl)-1H-pyrazol-5-yl)-8-méthoxy-[1,2,4]triazolo[1,5-a]pyridine (1325) ;
8-méthoxy-6-(3-(5-(1-((tétrahydro-2H-pyran-4-yl)méthyl)pipéridin-4-yl)pyridin-2-yl)-4-(trifluorométhyl)-1H-pyrazol-5-yl)-[1,2,4]triazolo[1,5-a]pyridine (1326) ;
6-(3-(5-(1-cyclobutylpipéridin-4-yl)pyridin-2-yl)-4-(trifluorométhyl)-1H-pyrazol-5-yl)-8-méthoxy-[1,2,4]triazolo[1,5-a]pyridine (1327) ; ou
6-(3-(5-(1-(cyclobutylméthyl)pipéridin-4-yl)pyridin-2-yl)-4-(trifluorométhyl)-1H-pyrazol-5-yl)-8-méthoxy-[1,2,4]triazolo[1,5-a]pyridine (1328).

8. Composition pharmaceutique comprenant un composé selon l'une quelconque des revendications 1-7 ou un sel pharmaceutiquement acceptable de celui-ci ; et un excipient pharmaceutiquement acceptable.

9. Composé selon l'une quelconque des revendications 1-7 ou sel pharmaceutiquement acceptable de celui-ci ou composition pharmaceutique selon la revendication 8, destinés à être utilisés en thérapie.

10. Composé ou sel pharmaceutiquement acceptable de celui-ci ou composition pharmaceutique destinés à être utilisés selon la revendication 9, destinés à être utilisés dans le traitement d'une maladie auto-immune ou d'une maladie inflammatoire chronique.

11. Composé ou sel pharmaceutiquement acceptable de celui-ci ou composition pharmaceutique destinés à être utilisés selon la revendication 10, dans lequel ladite maladie auto-immune ou maladie inflammatoire chronique est sélectionnée parmi le lupus érythémateux disséminé (LED), la polyarthrite rhumatoïde, la sclérose en plaques (SEP) et le syndrome de Sjögren.
